(19) Europäisches Patentamt
European Patent Office
Office européen des brevets

(11) EP 4 667 458 A1

(12) EUROPEAN PATENT APPLICATION
published in accordance with Art. 153(4) EPC

(43) Date of publication:
**24.12.2025 Bulletin 2025/52**

(21) Application number: **24757341.3**

(22) Date of filing: **16.02.2024**

(51) International Patent Classification (IPC):
***C07D 237/24*** (2006.01) ***A61K 31/50*** (2006.01)
***A61K 31/501*** (2006.01) ***A61K 31/5377*** (2006.01)
***A61P 35/00*** (2006.01) ***C07D 405/04*** (2006.01)
***C07D 401/04*** (2006.01) ***C07D 409/04*** (2006.01)
***C07D 401/10*** (2006.01) ***C07D 417/10*** (2006.01)

(52) Cooperative Patent Classification (CPC):
**A61K 31/4412; A61K 31/4439; A61K 31/444; A61K 31/4545; A61K 31/50; A61K 31/501; A61K 31/513; A61K 31/5377; A61K 45/06; A61P 35/00; C07D 237/24; C07D 401/04; C07D 401/10; C07D 405/04; C07D 409/04;** (Cont.)

(86) International application number:
**PCT/KR2024/095358**

(87) International publication number:
**WO 2024/172631 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.02.2023 KR 20230020968**

(71) Applicants:
- **Kanaph Therapeutics Inc.**
  **Seoul 04348 (KR)**
- **Cyrus Therapeutics Inc.**
  **Seoul 05836 (KR)**

(72) Inventors:
- **YU, Ha Na**
  **Gwacheon-si Gyeonggi-do 13826 (KR)**
- **JO, Seongin**
  **Gwangmyeong-si Gyeonggi-do 14202 (KR)**
- **JEON, Yeejin**
  **Gwangmyeong-si Gyeonggi-do 14295 (KR)**
- **LEE, Yeri**
  **Seoul 05629 (KR)**
- **KIM, Donggeon**
  **Seoul 05629 (KR)**
- **CHOI, Sungpil**
  **Anyang-si Gyeonggi-do 14074 (KR)**
- **HAN, Wooseok**
  **Hanam-si Gyeonggi-do 13015 (KR)**
- **NAM, Joonwoo**
  **Seongnam-si Gyeonggi-do 13527 (KR)**
- **YU, Jihyun**
  **Yongin-si Gyeonggi-do 16830 (KR)**
- **KIM, Ji Eun**
  **Anyang-si Gyeonggi-do 14044 (KR)**
- **KI, Dong Hyuk**
  **Gwacheon-si Gyeonggi-do 13839 (KR)**
- **KIM, Eun-Jung**
  **Seoul 06788 (KR)**
- **KIM, Sungeun**
  **Seoul 04385 (KR)**
- **CHOI, Hunmi**
  **Seoul 04712 (KR)**

(74) Representative: **HGF**
**HGF Limited**
**4th Floor, 1 City Square**
**Leeds LS1 2AL (GB)**

# (54) PHARMACEUTICAL COMPOSITION FOR TREATMENT OF CANCER, COMPRISING SOS1 INHIBITOR AND ANTICANCER DRUG

(57) There is provided a pharmaceutical composition for preventing or treating cancer, comprising a novel compound having SOS1 inhibitory activity and an anticancer agent as active ingredients. The novel compound according to the present invention inhibits the interaction between SOS1 and RAS family proteins, or between SOS1 and RAC1, thereby exhibiting synergistic effects in the anticancer activity when combined with other antic-

ancer agents. Accordingly, a pharmaceutical composition for treating cancer comprising the novel compound and an anticancer agent as active ingredients may be usefully used for preventing or treating cancer.

【FIG. 3】

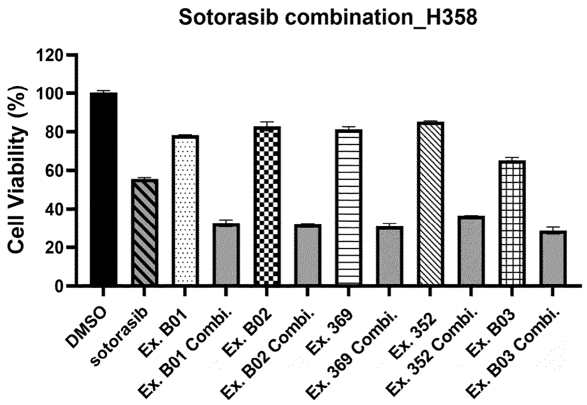


(52) Cooperative Patent Classification (CPC): (Cont.)
**C07D 417/10**

## Description

### Technical Field

The present invention relates to a pharmaceutical composition for preventing or treating cancer, comprising a novel compound having SOS1 inhibitory activity, a solvate, stereoisomer or pharmaceutically acceptable salt thereof and an anticancer agent as active ingredients, and a medicinal use thereof.

### Background Art

Mutations in the RAS gene are a major oncogene with a high incidence in human cancers, and are observed in 20 to 30% of human cancers, particularly in lung cancer, colon cancer, rectal cancer, and pancreatic cancer at high rates. RAS-family proteins include KRAS, NRAS or HRAS.

RAS proteins are small GTPases that exist in cells in either a GTP-bound or GDP-bound state, and are molecular switches that cycle between an active GTP-bound state and an inactive GDP-bound state. Mutations in the RAS gene reduce the ability of the RAS, a GTPase to hydrolyze GTP, leaving this molecular switch to maintain a constitutively active GTP-bound conformation, thereby inducing oncogenic downstream signaling (for example, RAF-MEK-ERK pathway or PI3K-PDK1-AKT pathway).

Meanwhile, binding of GTPase activating protein (GAP) such as NF1 accelerates the weak intrinsic GTPase activity of RAS proteins, thereby downregulating active RAS and returning it to an inactive form. On the other hand, binding of guanine nucleotide exchange factors (GEF) such as SOS1 promotes the release of GDP from RAS proteins and increases the GTP-bound active state.

Various studies on methods for directly or indirectly inhibiting RAS have been conducted in the prior art. However, it has been found that direct inhibition of RAS is extremely difficult due to the picomolar level of affinity of GTP for the binding site, lack of other well-defined pockets, and the fact that RAS interacts with GEFs, GAPs and effectors through the wide and flat proteinprotein interaction surface, which makes difficult to apply small molecule drugs, and the like. In addition, a method of indirectly inhibiting RAS by targeting farnesyl transferase has also been attempted, but an approved drug has not yet been prepared. In view of this failure to directly or indirectly inhibit RAS, it has been generally considered to be difficult to target RAS for drug development.

Under these circumstances, a method of inhibiting RAS by inhibiting the interaction between RAS and GEF to prevent the reloading of GTP has emerged.

SOS1 (Son of Sevenless 1) is a type of guanine nucleotide exchange factor (GEF), which promotes the release of GDP from RAS family proteins to allow GTP binding, thereby regulating RAS family protein signaling. Son of Sevenless (SOS) protein exists in two isoforms, SOS1 and SOS2, and only SOS1 is phosphorylated by ERK. Growth factor-induced phosphorylation of SOS1 is mostly mediated by ERK, which phosphorylates at least four serine residues in the C-terminal region of SOS1. This suggests that SOS1 plays an important role in the regulation of negative feedback of the KRAS pathway. The SOS1 protein consists of 1333 amino acids (150 kDa). SOS1 is a multi-domain protein having two tandem N-terminal histone domains (HD) followed by a Dbl homology domain (DH), a plextrin homology domain (PH), a helical linker (HL), a RAS exchange motif (REM), a CDC25 homology domain and a C-terminal proline rich domain (PR). SOS1 has two binding sites for RAS family proteins (i.e., a catalytic site that binds GDP-binding RAS family proteins and promotes exchange of guanine nucleotides, and an allosteric site that binds GTP-binding RAS family proteins and up-regulates a catalytic site activity of SOS1) (J. Med. Chem. 2021, 64, 10, 6569-6580). Selective pharmacological inhibition of catalytic site binding of SOS1 to RAS family proteins is expected to prevent SOS1-mediated activation of RAS-family proteins in a GTP-bound form.

Therefore, SOS1 inhibitor compounds are expected to inhibit signaling (for example, ERK phosphorylation) in cells downstream of RAS-family proteins, and thus novel SOS1 inhibitor compounds that bind to the SOS1 catalytic site and prevent binding and activation of RAS family proteins are being developed.

It has been reported that SOS1 is critically involved in mutant KRAS activation and oncogenic signaling in cancer (Current Opinion in Chemical Biology, 2021, 62: 109-118). Depletion of SOS1 levels reduced the survival of tumor cells with KRAS mutations, but no such effect was observed in KRAS wild-type cell lines. The SOS1 depletion effect cannot be rescued by the $SOS1^{F929A}$ mutation in which the catalytic site is damaged or the SOS1 mutation ($SOS1^{L687E/R688A}$) in which the GTP-KRAS binding is defective at the allosteric site, which suggests that targeting the catalytic site or the allosteric site of SOS1 may be an effective option for the treatment of KRAS mutation cancers.

In addition, SOS1 is critically involved in the activation of RAS family protein signaling in cancer through mechanisms other than mutation of RAS family proteins. SOS1 interacts with the adapter protein Grb2 to form the SOS1/Grb2 complex. The complex binds to an activated/phosphorylated receptor tyrosine kinase (for example, EGFR, ErbB2, ErbB3, ErbB4, PDGFR-A/B, FGFR1/2/3, IGF1R, INSR, ALK, ROS, TrkA, TrkB, TrkC, RET, c-MET, VEGFR1/2/3, AXL). In addition, it has been reported that SOS1 is localized to other phosphorylated cell surface receptors such as T cell

receptor (TCR), B cell receptor (BCR) and monocyte colony stimulating factor receptor, resulting in activating RAS family proteins.

Furthermore, SOS1 is a GEF for activation of the GTPase RAC1 (Ras-associated C3 botulinum toxin substrate 1). RAC1, like the RAS-family protein, is known to be involved in the pathogenesis of various cancers and other diseases.

Currently, BI3406, BI1701963, MRTX0902, and the like are being developed as inhibitors of SOS1 activity, but they are still in the early stages of development. Therefore, there is still a need in the art for the development of a novel compound for treating cancer by inhibiting SOS1 and a pharmaceutical composition comprising the same.

## Detailed Description of Invention

## Technical Problem

An object of the present invention is to provide a pharmaceutical composition comprising a compound of Formula I, or a solvate, stereoisomer or pharmaceutically acceptable salt thereof and an anticancer agent.

An object of the present invention is to provide a use of a pharmaceutical composition comprising a compound of Formula I, or a solvate, stereoisomer or pharmaceutically acceptable salt thereof and an anticancer agent, for the prevention or treatment of cancer.

An object of the present invention is to provide a method for preventing or treating cancer by administering a pharmaceutical composition comprising a compound of Formula I, or a solvate, stereoisomer or pharmaceutically acceptable salt thereof and an anticancer agent.

An object of the present invention is to provide a use of a pharmaceutical composition comprising a compound of Formula I, or a solvate, stereoisomer or pharmaceutically acceptable salt thereof and an anticancer agent, for manufacturing a medicament for the prevention or treatment of cancer.

## Solution to Problem

Each description and embodiment disclosed herein may also apply to each other description and embodiment. That is, all combinations of the various elements disclosed herein fall within the scope of the present application. In addition, it should not be construed that the scope of the present application is limited by the specific description set forth below.

In one aspect of the present invention, there is provided a pharmaceutical composition or a kit for preventing or treating of cancer, comprising a compound of Formula 1 below, a solvate, stereoisomer or pharmaceutically acceptable salt thereof; and an anticancer agent as active ingredients.

[Formula 1]

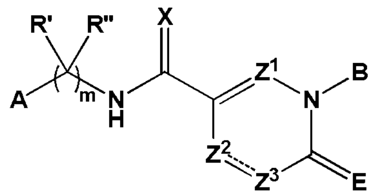

in Formula 1,

------ is a single bond or a double bond;

E is O or S; and X is O or S;

$Z^1$ is N or CH, $Z^2$ is N, NH, $CR^1$ or $CHR^1$, and $Z^3$ is $CR^1$ or $CHR^1$, provided that at most one of $Z^1$, $Z^2$ and $Z^3$ is N or NH,

each $R^1$ is independently selected from the group consisting of H, halogen, OH, CN, $NR^bR^c$, $C_1$-$C_6$ alkyl optionally interrupted by 1 to 3 oxygen atoms or nitrogen atoms and/or optionally substituted, optionally substituted $C_2$-$C_6$ alkenyl, optionally substituted $C_2$-$C_6$ alkynyl, optionally substituted $C_1$-$C_6$ alkoxy, optionally substituted $C_1$-$C_6$ acylamino, optionally substituted ($C_1C_6$ alkyl)sulfonylamino, optionally substituted $C_3$-$C_6$ cycloalkyl, optionally substituted 4- to 7-membered heterocycloalkyl, optionally substituted $C_6$-$C_{10}$ aryl, optionally substituted $C_6$-$C_{10}$ aryloxy, optionally substituted ($C_6$-$C_{10}$ aryl)-($C_1$-$C_6$ alkyl)oxy-, optionally substituted ($C_6$-$C_{10}$ aryl)amino and optionally substituted 5- to 10-membered heteroaryl; or

when $Z^1$ is N, ------ is a double bond, and both of $Z^2$ and $Z^3$ are $CR^1$, then two $R^1$ are optionally linked to each other together with the carbon atom to which they are attached to form 5-membered heteroaryl containing one N, O or S;

R' and R" are each independently H or $C_1$-$C_3$ alkyl, or R' and R" bonded to the same carbon or adjacent carbons may

be taken together with the carbon atom to which they are attached to form $C_3$-$C_4$ cycloalkyl, and said $C_1$-$C_3$ alkyl and $C_3$-$C_4$ cycloalkyl may be optionally substituted with at least one halogen, OH, CN, $C_1$-$C_3$ alkoxy or $NR^bR^c$;
m is an integer of 1 to 3;
A is $Cy_1$ or $Cy_1$-Y-$Cy_2$;
Y is $NR^d$, $CR^dR^e$, O, S, or a direct bond;
$Cy_1$ and $Cy_2$ are each independently $C_6$-$C_{10}$ aryl optionally fused with $C_3$-$C_8$ cycloalkyl, or 5- to 10-membered heteroaryl;
said $Cy_1$ and $Cy_2$ may be each optionally substituted with 1 to 3 $R^2$;
$R^2$ is selected from the group consisting of H, halogen, OH, CN, oxo, amino, $-NR^bR^C$,$-N=S(O)R^b$, $-N=S(O)NR^bR^c$, $-SF_5$, $-Si(C_1$-$C_3$ alkyl$)_3$, $-SO_2R^b$, $-C(O)R^b$, $C_1$-$C_6$ alkyl optionally interrupted by 1 to 3 oxygen atoms or nitrogen atoms and/or optionally substituted, optionally substituted $C_1$-$C_6$ alkoxy and optionally substituted $C_3$-$C_6$ cycloalkyl;
B is H, optionally substituted $C_{1-}C_6$ alkyl, $-(CH_2)_o$-$Cy_3$ or $-(CH_2)_o$-$Cy_3$-W-$Cy_4$;
o is an integer of 0 to 3;
W is $NR^d$, $CR^dR^e$, C(O), O, S, or a direct bond;
$Cy_3$ and $Cy_4$ are each independently selected from the group consisting of $C_3$-$C_6$ monocyclic cycloalkyl or $C_3$-$C_6$ monocyclic cycloalkenyl, optionally fused with 5- to 10-membered heterocycloalkyl or 5- to 10-membered heteroaryl; bicyclic, tricyclic or tetracyclic bridged, fused or spiro $C_5$-$C_{20}$ cycloalkyl or $C_5$-$C_{20}$ cycloalkenyl; $C_6$-$C_{10}$ aryl optionally fused with 5- to 10-membered heterocycloalkyl; 5- to 10-membered monocyclic heteroaryl optionally fused with $C_3$-$C_6$ cycloalkyl; 5- to 10-membered bicyclic heteroaryl; 4- to 10-membered saturated or partially unsaturated monocyclic heterocycloalkyl optionally fused with $C_3$-$C_6$ cycloalkyl; and 5-to 10-membered bicyclic bridged, fused or spiro heterocycloalkyl;
said $Cy_3$ and $Cy_4$ may be each independently optionally substituted with 1 to 3 $R^3$;
$R^3$ is selected from the group consisting of H, deuterium, halogen, OH, CN, oxo, $-NR^bR^c$, $-N=S(O)R^b$, $-N=S(O)NR^bR^c$, $-SO_2R^b$, $-C(O)R^b$, $-C(O)OR^b$, $-CONR^bR^c$, $-NR^bCOR^c$, $-NR^bC(O)OR^c$, $-NR^bSO_2R^c$, -NHCO-($C_3$-$C_6$ cycloalkyl), optionally substituted $C_1$-$C_6$ alkyl, optionally substituted $C_1$-$C_6$ alkoxy and optionally substituted $C_3$-$C_6$ cycloalkyl;
$R^b$ and $R^c$ are each independently H or optionally substituted $C_1$-$C_6$ alkyl; and
$R^d$ and $R^e$ are each independently H or optionally substituted $C_1$-$C_6$ alkyl.

In the present disclosure, "optionally substituted" as used in the definition of substituents may mean that the structure is unsubstituted or substituted with at least one substituent selected from the group consisting of:

(i) halogen, OH, CN, oxo, $NH_2$, $NH(C_{1-}C_6$ alkyl), or $N(C_{1-}C_6$ alkyl$)_2$;
(ii) $C_1$-$C_3$ alkyl optionally substituted with at least one substituent selected from the group consisting of halogen, OH, CN, oxo, $NH_2$, $NH(C_{1-}C_6$ alkyl) and $N(C_{1-}C_6$ alkyl$)_2$;
(iii) $C_1$-$C_3$ alkoxy optionally substituted with at least one substituent selected from the group consisting of halogen, OH, CN, oxo, $NH_2$, $NH(C_{1-}C_6$ alkyl) and $N(C_{1-}C_6$ alkyl$)_2$; and
(iv) $C_3$-$C_6$ cycloalkyl optionally substituted with at least one substituent selected from the group consisting of halogen, OH, CN, oxo, $NH_2$, $NH(C_{1-}C_6$ alkyl) and $N(C_{1-}C_6$ alkyl$)_2$.

In one embodiment, the optionally substituted moiety may be substituted with one or more identical or different substituents selected from the group consisting of halogen, OH, CN, $NH_2$, $NH(C_{1-}C_6$ alkyl), $N(C_{1-}C_6$ alkyl$)_2$ and $C_1$-$C_3$ alkoxy.

In one embodiment, an "optionally substituted" group may be unsubstituted or substituted with at least one substituent selected from the group consisting of deuterium, halogen, OH, CN, oxo, amino, $C_{1-}C_6$ alkylamino, di($C_{1-}C_6$ alkyl)amino, $C_{1-}C_6$ haloalkyl, $C_{1-}C_6$ hydroxyalkyl, $C_{1-}C_6$ cyanoalkyl, $C_{1-}C_6$ aminoalkyl and $C_{1-}C_6$ alkoxy. In this case, two or more substituents may be substituted on the same atom or different atoms. For example, 1-fluoro-2-oxopropyl is an alkyl group substituted with oxo and fluoro, respectively, on different carbon atoms of the propyl group, and is encompassed by "optionally substituted alkyl" in the present disclosure. In the present specification, when two or more substituents are substituted on the same moiety, they may be substituted on the same atom or different atoms of the moiety.

In Formula 1 above, E may be O or S. For example, E may be O.

In Formula 1, X may be O or S. For example, X may be O.

In Formula 1, $Z^1$ may be N or CH, $Z^2$ may be N, NH, $CR^1$ or $CHR^1$, and $Z^3$ may be $CR^1$ or $CHR^1$. ------ may be a single bond or a double bond. However, at most one of $Z^1$, $Z^2$ and $Z^3$ is N or NH.

Alternatively, when $Z^1$ is N, ------ is a double bond, and both of $Z^2$ and $Z^3$ are $CR^1$, then two $R^1$ may be optionally linked to each other together with the carbon atom to which they are attached to form a 5-membered heteroaryl ring containing one N, O or S.

In Formula 1 above, m may be an integer of 1 to 3. For example, m may be 1 or 2. In one embodiment, m may be 1. When m is 2 or 3, R' bonded to each carbon of the alkylene chain may be the same or different from each other. When m is 2

or 3, R" bonded to each carbon of the alkylene chain may be the same or different from each other.

In one embodiment, R' and R" may be each independently H or $C_{1-3}$ alkyl, for example, - $CH_3$, $-CH_2CH_3$, $-CH_2CH_2CH_3$, $-CH(CH_3)_2$. Said $C_1$-$C_3$ alkyl may be optionally substituted with at least one halogen, OH, CN, $C_1$-$C_3$ alkoxy or $NR^bR^c$. In this case, $R^b$ and $R^c$ may be each independently H or optionally substituted $C_1$-$C_3$ alkyl. In one embodiment, both R' and R" may be $C_{1-3}$ alkyl. In one embodiment, both R' and R" may be H. In one embodiment, one of R' and R" may be H, and the other may be $C_{1-3}$ alkyl. For example, one of R' and R" may be H, and the other may be methyl, ethyl, difluoromethyl, fluoromethyl, hydroxymethyl, aminomethyl, and the like, but is not limited thereto.

In some embodiments, R' and R" bonded to the same carbon or adjacent carbons may be taken together with the carbon atom to which they are attached to form a cyclopropyl or cyclobutyl ring. The cyclopropyl or cyclobutyl ring may be optionally substituted with at least one halogen, OH, CN, $C_1$-$C_3$ alkoxy or $NR^bR^c$. In this case, $R^b$ and $R^c$ may be each independently H or optionally substituted $C_1$-$C_3$ alkyl. For example, R' and R" may be taken together with the alkylene chain to which they are attached to form the following structures, but not limited to:

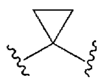, 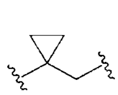, 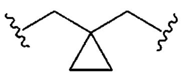, 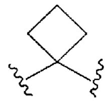, 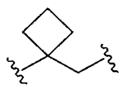, 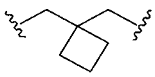, 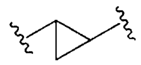,

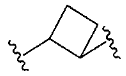, 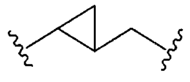, or 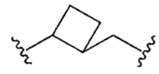.

In Formula 1 above, $R^1$ may be H, halogen, OH, CN, $NR^bR^c$, $C_1$-$C_6$ alkyl optionally interrupted by 1 to 3 oxygen atoms or nitrogen atoms, optionally substituted $C_1$-$C_6$ alkyl, optionally substituted $C_2$-$C_6$ alkenyl, optionally substituted $C_2$-$C_6$ alkynyl, optionally substituted $C_1$-$C_6$ alkoxy, optionally substituted $C_1$-$C_6$ acylamino, optionally substituted ($C_1$-$C_6$ alkyl)sulfonylamino, or $C_3$-$C_6$ cycloalkyl. In one embodiment, $R^1$ may be H, OH, $CH_3$, $-CH=CH_2$, $-C\equiv CH$, CN, or optionally substituted cyclopropyl.

In one embodiment, $R^1$ may be optionally substituted $C_1$-$C_6$ alkyl, optionally substituted $C_2$-$C_6$ alkenyl, or optionally substituted $C_2$-$C_6$ alkynyl, preferably optionally substituted $C_1$-$C_3$ alkyl. In this case, optionally substituted $C_1$-$C_6$ alkyl or $C_1$-$C_3$ alkyl, optionally substituted $C_2$-$C_6$ alkenyl, or optionally substituted $C_2$-$C_6$ alkynyl may be substituted with at least one substituent selected from the group consisting of substituents (i) to (iv) described above. In this case, at least one substituent may include a combination of two or more substituents selected from any one of (i), (ii), (iii) and (iv), or a combination of two or more substituents each selected from two or more of (i), (ii), (iii) and (iv), or a combination thereof. When there are two or more substituents, they may be the same or different from each other. For example, optionally substituted $C_1$-$C_6$ alkyl or $C_1$-$C_3$ alkyl may be substituted with 1 to 5, 1 to 4, 1 to 3, 1, 2 or 3 substituents. For example, they may include $-CF_2CH_2OH$ substituted with two F and one OH, $-CF_3$ substituted with three F, and the like.

Said $C_1$-$C_6$ alkyl may be optionally interrupted by 1 to 3 oxygen atoms or nitrogen atoms, and it may include, for example, methoxymethyl, methoxymethoxymethyl, ethoxymethyl, ethoxyethoxymethyl, methylaminomethyl, methylaminoethyl, dimethylaminomethyl, dimethylaminoethyl, and the like, but is not limited thereto.

In some embodiments, $R^1$ may be optionally substituted 4- to 7-membered heterocycloalkyl, optionally substituted $C_6$-$C_{10}$ aryl, optionally substituted $C_6$-$C_{10}$ aryloxy, optionally substituted ($C_6$-$C_{10}$ aryl)-($C_1$-$C_6$ alkyl)oxy-, optionally substituted ($C_6$-$C_{10}$ aryl)amino or optionally substituted 5- to 10-membered heteroaryl. In this case, the substituents that may be optionally substituted are as described above. In one embodiment, $R^1$ may be 4- to 7-membered heterocycloalkyl containing 1 or 2 heteroatoms selected from N, O and S, and may be, for example, azetidinyl, oxetanyl, pyrrolidinyl, tetrahydrofuranyl, piperidinyl or piperazinyl, but is not limited thereto. In one embodiment, $R^1$ may include 5- to 10-membered heteroaryl containing 1 or 2 heteroatoms selected from N, O and S, for example, but not limited to, indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl. In one embodiment, $R^1$ may be phenyl or naphthyl.

In Formula 1 of the present invention, A may be $Cy_1$ or $Cy_1$-Y-$Cy_2$. In this case, Y may be $NR^d$, $CR^dR^e$, O, S, or a direct bond. $R^d$ and $R^e$ may be each H or optionally substituted $C_1$-$C_6$ alkyl, preferably H or optionally substituted $C_1$-$C_3$ alkyl. In this case, the substituents that may be optionally substituted are as described above.

In A in Formula 1 above, $Cy_1$ and $Cy_2$ may be each independently $C_6$-$C_{10}$ aryl, $C_6$-$C_{10}$ aryl fused with $C_3$-$C_8$ cycloalkyl, or 5- to 10-membered heteroaryl.

In one embodiment, said $Cy_1$ may be $C_6$-$C_{10}$ aryl, or 5- to 6-membered heteroaryl containing 1 to 3 heteroatoms selected from N, O or S. In one embodiment, $Cy_1$ may be $C_6$-$C_{10}$ aryl. In another embodiment, $Cy_1$ may be 5- to 6-membered heteroaryl containing one or two N or S. For example, $Cy_1$ may include phenyl, naphthalenyl, thiazolyl, thiophenyl or pyrazolyl.

In one embodiment, said $Cy_2$ may be $C_6$-$C_{10}$ aryl fused with $C_3$-$C_6$ cycloalkyl, $C_6$-$C_{10}$ aryl, or 5- to 6-membered

heteroaryl containing 1 to 3 heteroatoms selected from N, O or S. In one embodiment, $Cy_2$ may be $C_6$-$C_{10}$ aryl fused with $C_3$-$C_5$ cycloalkyl, or $C_6$-$C_{10}$ aryl. In another embodiment, $Cy_2$ may be 5- to 6-membered heteroaryl containing one or two N or S. For example, $Cy_2$ may include phenyl, 2,3-dihydroindenyl or bicyclo[4.2.0]octa-1,3,5-trienyl, pyrazolyl, thiophenyl, pyridinyl, 2-oxo-1,2-dihydropyridinyl or pyrrolyl.

In some embodiments, A may be $Cy_1$, wherein $Cy_1$ may be $C_6$-$C_{10}$ aryl, such as phenyl or naphthyl. In some embodiments, A may be $Cy_1$, wherein $Cy_1$ may be 5- to 10-membered heteroaryl. In some embodiments, A may be $Cy_1$-Y-$Cy_2$, wherein $Cy_1$ and $Cy_2$ may be each $C_6$-$C_{10}$ aryl, and Y may be O. For example, A may be phenyl-O-phenyl. In some embodiments, A may be $Cy_1$-Y-$Cy_2$, wherein $Cy_1$ may be $C_6$-$C_{10}$ aryl, and $Cy_2$ may be 5- to 10-membered heteroaryl. In some embodiments, A may be $Cy_1$-Y-$Cy_2$, wherein $Cy_1$ may be 5- to 10-membered heteroaryl, and $Cy_2$ is $C_6$-$C_{10}$ aryl.

In some specific embodiments, said 5- to 10-membered heteroaryl of $Cy_1$ or $Cy_2$ may be indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl, but is not limited thereto.

In addition, said $Cy_1$ and $Cy_2$ may be each optionally substituted with 1 to 3 $R^2$. $R^2$ is halogen, OH, CN, oxo, amino, $-NR^bR^C$, $-N=S(O)R^b$, $-N=S(O)NR^bR^c$, $-SF_5$, $-Si(C_1$-$C_3$ alkyl$)_3$, - $SO_2R^b$, $-C(O)R^b$, $C_1$-$C_6$ alkyl optionally interrupted by 1 to 3 oxygen atoms or nitrogen atoms, optionally substituted $C_1$-$C_6$ alkyl, optionally substituted $C_1$-$C_6$ alkoxy and optionally substituted $C_3$-$C_6$ cycloalkyl. In this case, the substituents that may be optionally substituted are as described above. In addition, specific substituents of $R^2$ are as described in Formula I below.

In Formula 1 of the present invention, when A is $Cy_1$-Y-$Cy_2$, then $Cy_1$ may be optionally substituted with 1 to 3 $R^{2a}$, and $Cy_2$ may be optionally substituted with 1 to 3 $R^{2b}$. $R^{2a}$ and $R^{2b}$ are as described in Formula I below.

In Formula 1 of the present invention, B may be H, optionally substituted $C_1$-$C_6$ alkyl, - $(CH_2)_o$-$Cy_3$ or $-(CH_2)_o$-$Cy_3$-W-$Cy_4$. In this case, o may be an integer of 0 to 3. In addition, o may be 0 or 1.

In some embodiments, B may be $-(CH_2)_o$-$Cy_3$. In some embodiments, B may be $-(CH_2)_o$-$Cy_3$-W-$Cy_4$. In this case, W may be $NR^d$, $CR^dR^e$, O, S, or a direct bond, and $R^d$ and $R^e$ may be each H or optionally substituted $C_1$-$C_6$ alkyl, preferably H or optionally substituted $C_1$-$C_3$ alkyl. In this case, the substituents that may be optionally substituted are as described above.

In Formula 1 of the present invention, $Cy_3$ and $Cy_4$ are each independently $C_3$-$C_6$ monocyclic cycloalkyl or $C_3$-$C_6$ monocyclic cycloalkenyl, wherein the cycloalkyl or cycloalkenyl may be optionally fused with 5- to 10-membered heterocycloalkyl or 5- to 10-membered heteroaryl. In one embodiment, $Cy_3$ and $Cy_4$ may be each independently cyclopropyl; cyclobutyl, cyclopentyl, cyclohexyl; cyclobutenyl; cyclopentenyl, cyclohexenyl; cyclohexyl or cyclopentyl fused with pyrazole, piperazine or tetrahydropyran. In one embodiment, said 5- to 10-membered heteroaryl fused with cycloalkyl or cycloalkenyl may include indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl, but is not limited thereto. In one embodiment, said 5- to 10-membered heterocycloalkyl fused with cycloalkyl or cycloalkenyl may include tetrahydropyranyl, piperidinyl, tetrahydrofuranyl, or tetrahydro 2H-thiopyranyl, but is not limited thereto.

In some embodiments, $Cy_3$ and $Cy_4$ may be each independently bicyclic, tricyclic or tetracyclic bridged, fused or spiro $C_5$-$C_{20}$ cycloalkyl or $C_5$-$C_{20}$ cycloalkenyl. In one embodiment, $Cy_3$ and $Cy_4$ may be each independently bicyclic or tricyclic bridged or fused $C_5$-$C_{15}$ cycloalkyl or $C_5$-$C_{15}$ cycloalkenyl. In one embodiment, $Cy_3$ may be bicyclic or tricyclic bridged $C_5$-$C_{10}$ cycloalkyl or $C_5$-$C_{10}$ cycloalkenyl. In one embodiment, said $Cy_3$ may be bicyclo[2.2.2]octanyl, adamantyl, bicyclo[2.2.1]heptanyl, or bicyclo[2.2.1]hept-2-enyl, bicyclo[1.1.1]pentanyl, but is not limited thereto.

In some embodiments, $Cy_3$ and $Cy_4$ may be each independently $C_6$-$C_{10}$ aryl. In one embodiment, $Cy_3$ may be phenyl or naphthyl.

In some embodiments, $Cy_3$ and $Cy_4$ may be each independently 5- to 10-membered monocyclic or bicyclic heteroaryl, 5- to 10-membered monocyclic heterocycloalkyl, or 5- to 10-membered bicyclic bridged, fused or spiro heterocycloalkyl, wherein said 5- to 10-membered monocyclic heteroaryl and 5- to 10-membered monocyclic heterocycloalkyl may be optionally fused with $C_3$-$C_6$ cycloalkyl. In one embodiment, said 5- to 10-membered heteroaryl may contain 1 or 2 heteroatoms selected from N, O or S, and said 5- to 10-membered heterocycloalkyl may be 5- or 6-membered heterocycloalkyl containing 1 heteroatom selected from N, O or S. In one embodiment, said 5- to 10-membered heteroaryl may include indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl, but is not limited thereto. In one embodiment, said 5- or 6-membered heterocycloalkyl may include tetrahydropyranyl, piperidinyl, tetrahydrofuranyl, or tetrahydro 2H-thiopyranyl, but is not limited thereto. In one embodiment, said 5- to 10-membered heteroaryl or 5- to 10-membered heterocycloalkyl may be optionally fused with $C_3$-$C_6$ cycloalkyl or $C_3$-$C_6$ cycloalkenyl, and may form, for example, pyrazolyl, piperazine or tetrahydropyran fused with cyclohexyl. In one embodiment, said 5- to 10-membered heterocycloalkyl may be bicyclic bridged, fused or spiro heterocycloalkyl, which may be, for example, 3-oxabicyclo[2.1.1]hexanyl or 2-oxabicyclo [2.1.1]hexanyl, but is not limited thereto. In one embodiment, $Cy_3$ may be phenyl, naphthyl, pyridinyl, thiophenyl, tetrahydropyranyl or piperidinyl.

In some embodiments, B may be $-(CH_2)_o$-$Cy_3$-W-$Cy_4$, wherein $Cy_3$ and W may be as described above, and $Cy_4$ may be phenyl or naphthyl. In one embodiment, $Cy_3$ and $Cy_4$ may be each phenyl, and W may be a direct bond.

In one embodiment, B may be -$(CH_2)_o$-$Cy_3$-W-$Cy_4$, wherein $Cy_3$ may be selected from the group consisting of $C_3$-$C_6$ cycloalkyl, $C_3$-$C_6$ cycloalkenyl, 5- or 6-membered saturated or partially unsaturated heterocycloalkyl containing 1 or 2 heteroatoms selected from N, O or S, bridged bicyclic $C_{5-10}$ cycloalkyl, $C_6$-$C_{10}$ aryl optionally fused with 5- or 6-membered heterocycloalkyl containing 1 or 2 heteroatoms selected from N, O or S, and 5- or 6-membered monocyclic heteroaryl or 5- to 10-membered bicyclic heteroaryl containing 1 or 2 heteroatoms selected from N, O or S. In this case, W may be NH, C(O) or a direct bond. In addition, $Cy_4$ may be selected from the group consisting of saturated or partially unsaturated 4- to 10-membered heterocycloalkyl containing 1 or 2 heteroatoms selected from N, O or S, $C_6$-$C_{10}$ aryl, and 5- or 6-membered monocyclic heteroaryl containing 1 to 4 heteroatoms selected from N, O or S.

In B in Formula 1 of the present invention, said $Cy_3$ and $Cy_4$ may be each independently optionally substituted with 1 to 3 $R^3$. $R^3$ may be halogen, OH, CN, oxo, amino, -$NR^bR^c$, -N=S(O)$R^b$, -N=S(O)$NR^bR^c$, -$SO_2R^b$, -C(O)$R^b$, -$CONR^bR^c$, -$NR^bCOR^c$, $NR^bSO_2R^c$, optionally substituted $C_1$-$C_6$ alkyl, optionally substituted $C_1$-$C_6$ alkoxy and optionally substituted $C_3$-$C_6$ cycloalkyl, wherein $R^b$ and $R^c$ may be H or optionally substituted $C_1$-$C_6$ alkyl, preferably optionally substituted $C_1$-$C_3$ alkyl. In this case, the substituents that may be optionally substituted are as described above.

In Formula 1 of the present invention, when B is -$(CH_2)_o$-$Cy_3$-W-$Cy_2$, then $Cy_3$ may be optionally substituted with 1 to 3 $R^{3a}$, and $Cy_4$ may be optionally substituted with 1 to 3 $R^{3b}$. $R^{3a}$ and $R^{3b}$ are as described in Formula I below.

Limitations on each structure and substituent of Formula 1 above may be equally applied to Formula I below, if applicable. Likewise, limitations on each structure and substituent of Formula I below may be equally applied to Formula 1 above, if applicable.

In one aspect of the present invention, there is provided a pharmaceutical composition and kit for preventing or treating cancer, comprising a compound of Formula I below, a solvate, stereoisomer or pharmaceutically acceptable salt thereof; and an anticancer agent as active ingredients.

[Formula I]

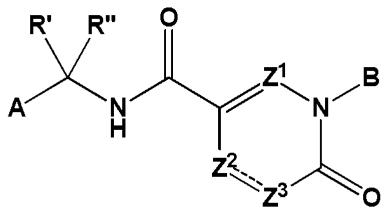

in Formula I,

------ is a single bond or a double bond;

$Z^1$ is N or CH;

when $Z^1$ is N, then both of $Z^2$ and $Z^3$ are $CHR^1$ and ------ is a single bond, or both of $Z^2$ and $Z^3$ are $CR^1$ and ------ is a double bond;

when $Z^1$ is CH, then $Z^2$ is N or $CR^1$, $Z^3$ is $CR^1$, and ------ is a double bond; or

when $Z^1$ is N, both of $Z^2$ and $Z^3$ are $CR^1$, and ------ is a double bond, then two $R^1$ may be optionally linked to each other together with the carbon atom to which they are attached to form a thiophene or pyrrole ring;

each $R^1$ is independently selected from the group consisting of H, halogen, CN, OH, $NR^bR^c$, $C_1$-$C_6$ alkoxy, $C_1$-$C_6$ acylamino, $C_1$-$C_6$ alkylsulfonylamino, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, $C_6$-$C_{10}$ aryloxy, ($C_6$-$C_{10}$ aryl)-($C_1$-$C_6$ alkyl)oxy and $C_6$-$C_{10}$ arylamino;

R' and R" are each independently H or $C_1$-$C_3$ alkyl, or R' and R" may be taken together with the carbon atom to which they are attached to form $C_3$-$C_4$ cycloalkyl, and said $C_1$-$C_3$ alkyl and $C_3$-$C_4$ cycloalkyl may be optionally substituted with at least one halogen, OH, CN, $C_1$-$C_3$ alkoxy or $NR^bR^c$;

A is $Cy_1$ or $Cy_1$-Y-$Cy_2$;

Y is O, S, or a direct bond;

$Cy_1$ is $C_6$-$C_{10}$ aryl or 5- or 6-membered heteroaryl containing 1 or 2 heteroatoms selected from N, O and S;

$Cy_1$ may be optionally substituted with 1 to 3 $R^{2a}$;

$R^{2a}$ is selected from the group consisting of H, halogen, OH, CN, oxo, $SF_5$, $NR^bR^c$, -Si($C_{1-3}$ alkyl)$_3$, -$SO_2R^b$, -C(O)$R^b$, $C_1$-$C_6$ alkyl, $C_1$-$C_6$ haloalkyl, $C_1$-$C_6$ alkoxy, $C_1$-$C_6$ haloalkoxy, $C_3$-$C_6$ cycloalkyl and

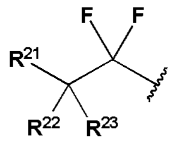
,

$R^{21}$ is H, halogen, OH, $NR^bR^c$, $C_1$-$C_6$ alkoxy or $C_1$-$C_6$ acyloxy, and $R^{22}$ and $R^{23}$ are each independently H, halogen or $C_1$-$C_2$ alkyl;
$Cy_2$ is $C_6$-$C_{10}$ aryl, phenyl fused with $C_3$-$C_6$ cycloalkyl, or 5- or 6-membered heteroaryl containing 1 or 2 heteroatoms selected from N, O and S;
$Cy_2$ may be optionally substituted with 1 to 3 $R^{2b}$;
$R^{2b}$ is selected from the group consisting of H, halogen, OH, CN, oxo, $NR^bR^c$; $C_1$-$C_6$ alkyl; $C_1$-$C_6$ alkyl substituted with halogen, CN, OH, $NR^bR^c$ or $C_1$-$C_6$ alkoxy; $C_1$-$C_6$ alkyl optionally interrupted by 1 to 3 oxygen atoms and/or nitrogen atoms; and $C_1$-$C_6$ alkyl substituted with hydroxy-($C_1$-$C_6$ alkyl)amino-;
B is H, $C_1$-$C_6$ alkyl, $C_1$-$C_6$ haloalkyl, $C_1$-$C_6$ hydroxyalkyl, $C_1$-$C_6$ alkoxy-$C_1$-$C_6$ alkyl, $C_1$-$C_6$ alkyl substituted with $NR^bR^c$, $-(CH_2)_o$-$Cy_3$ or $-(CH_2)_o$-$Cy_3$-W-$Cy_4$;
W is NH, C(O) or a direct bond;
o is an integer of 0 or 1;
$Cy_3$ is selected from the group consisting of $C_3$-$C_8$ cycloalkyl, $C_3$-$C_8$ cycloalkenyl, 5- or 6-membered saturated or partially unsaturated heterocycloalkyl containing 1 or 2 heteroatoms selected from N, O and S, bridged bicyclic $C_5$-$C_{10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms selected from N, O and S;
$Cy_3$ may be optionally substituted with 1 to 3 $R^{3a}$,
$R^{3a}$ is selected from the group consisting of H, halogen, OH, CN, oxo, $C_1$-$C_6$ alkyl; $C_1$-$C_6$ alkyl substituted with halogen, OH, CN or $C_1$-$C_6$ alkoxy; $C_3$-$C_6$ cycloalkyl, $C_1$-$C_6$ alkoxy, $C_1$-$C_6$ haloalkoxy, $C_1$-$C_6$ haloalkylamino, $C_1$-$C_6$ hydroxyalkylamino, ($C_3$-$C_6$ cycloalkyl)carbonylamino, - $NR^bR^c$, $-NR^bCOR^c$, $-NR^bC(O)OR^c$, $-SO_2R^b$, $-C(O)R^b$, $-C(O)OR^b$, $-NR^bSO_2R^c$ and $-CONR^{b1}R^{c1}$;
$Cy_4$ is selected from the group consisting of saturated or partially unsaturated 4- to 10-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S;
$Cy_4$ may be optionally substituted with 1 to 3 $R^{3b}$,
$R^{3b}$ is H, deuterium, halogen, OH, CN, oxo, $NR^bR^c$, $C_1$-$C_6$ alkyl, $C_1$-$C_6$ alkyl substituted with deuterium, $C_1$-$C_6$ haloalkyl, $C_1$-$C_6$ hydroxyalkyl, $C_1$-$C_6$ alkoxy-$C_1$-$C_6$ alkyl, $C_1$-$C_6$ alkoxy or $C_1$-$C_6$ haloalkoxy;
$R^b$ and $R^c$ are each independently H or $C_1$-$C_6$ alkyl; and
one of $R^{b1}$ and $R^{c1}$ is H or $C_1$-$C_6$ alkyl, and the other of $R^{b1}$ and $R^{c1}$ is H, $C_1$-$C_6$ alkyl, $C_1$-$C_6$ alkyl substituted with $NR^bR^c$, or $C_1$-$C_6$ alkyl substituted with $C_1$-$C_6$ alkoxy.

In Formula I above of the present invention, when $Z^1$ is N, then both of $Z^2$ and $Z^3$ may be $CR^1$, and ------ may be a double bond. In addition, both of $Z^2$ and $Z^3$ may be $CHR^1$, andmay be a single bond.

Alternatively, when $Z^1$ is N, both of $Z^2$ and $Z^3$ are $CR^1$, and ------ is a double bond, then two $R^1$ may be optionally linked to each other together with the carbon atom to which they are attached to form a thiophene or pyrrole ring.

In Formula I above, when $Z^1$ is CH, then $Z^2$ may be N or $CR^1$, $Z^3$ may be $CR^1$, and-may be a double bond.

In Formula I above of the present invention,

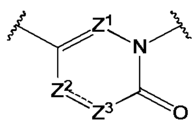

may be selected from the following structures:

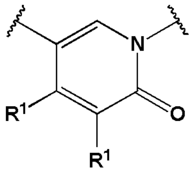
,
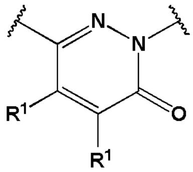
,
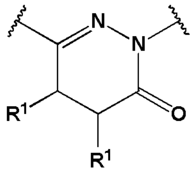
,
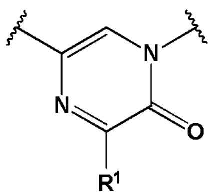
,
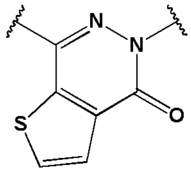
,

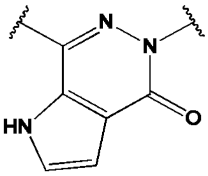
, and
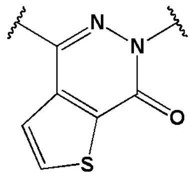
.

(In the above structures, two $R^1$ substituted on the same ring are the same or different from each other.)

In one embodiment, in Formula I,

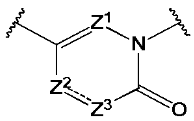

may be

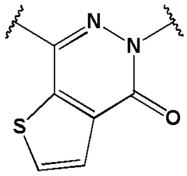
,

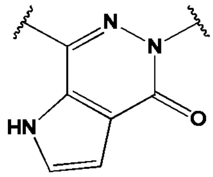
, or
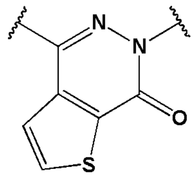
.

In one embodiment, in Formula I,

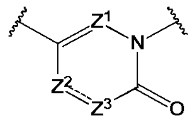

may be

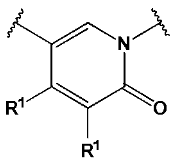
or
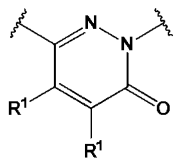
.

In Formula I above, each $R^1$ may be independently selected from the group consisting of H, halogen, CN, OH, $NR^bR^c$, $C_1$-$C_6$ alkoxy, $C_1$-$C_6$ acylamino, $C_1$-$C_6$ alkylsulfonylamino, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, $C_6$-$C_{10}$ aryloxy, ($C_6$-$C_{10}$ aryl)-($C_1C_6$ alkyl)oxy and $C_6$-$C_{10}$ arylamino. In this case, $R^b$ and $R^c$ are each independently H or $C_1$-$C_6$ alkyl. For example, each $R^1$ may be independently H, halogen, CN, OH or $C_1$-$C_6$ alkoxy. For example, $R^1$ may be an unsubstituted or substituted amino group such as $NR^bR^c$, $C_1$-$C_6$ acylamino, $C_1$-$C_6$ alkylsulfonylamino or $C_6$-$C_{10}$ arylamino. For example, $R^1$ may be a hydrocarbon group such as $C_1$-$C_6$ alkyl, $C_2$-$C_6$ alkenyl or $C_2$-$C_6$ alkynyl. For example, $R^1$ may be a ring substituent such as $C_3$-$C_6$ cycloalkyl, $C_6$-$C_{10}$ aryl, $C_6$-$C_{10}$ aryloxy or ($C_6$-$C_{10}$ aryl)-($C_1$-$C_6$ alkyl)oxy.

In one embodiment, when two $R^1$ substituted on the same ring are present, then one may be H, and the other may be not H. In another embodiment, two $R^1$ substituted on the same ring may be both H.

For example, $R^1$ may include H, F, Br, Cl, I, CN, OH, $OCH_3$, amino, methylamino, dimethylamino, ethylamino, acetylamino, methylsulfonylamino, ethylsulfonylamino, methyl, ethyl, ethenyl, ethynyl, cyclopropyl, cyclobutyl, cyclopentyl, phenyl, phenoxy, benzyloxy or phenylamino, but is not limited thereto.

In Formula I above of the present invention, R' and R" may be each independently H or $C_1$-$C_3$ alkyl, or R' and R" may be taken together with the carbon atom to which they are attached to form $C_3$-$C_4$ cycloalkyl. Optionally, said $C_1$-$C_3$ alkyl and $C_3$-$C_4$ cycloalkyl may be substituted with at least one halogen, OH, CN, $C_1$-$C_3$ alkoxy or $NR^bR^c$. In this case, $R^b$ and $R^c$ are each independently H or $C_1$-$C_6$ alkyl.

For example, R' and R" may be each independently H or $C_1$-$C_3$ alkyl. For example, R' and R" may be optionally taken together with the carbon atom to which they are attached to form a cyclopropane ring, wherein in Formula A,

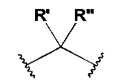

may be

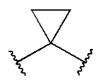.

In one embodiment, R' and R" may be the same or different from each other. When R' and R" are different, then the carbon atom to which they are attached is a chiral center, and a compound of Formula I has stereoisomers, and any such stereoisomers are also included within the scope of the present invention.

For example, when any one of R' and R" is H,

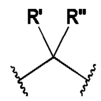

in Formula I has the steric structure of

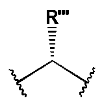 or 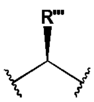

(R‴ is $C_1$-$C_3$ alkyl, such as methyl or ethyl).

In one embodiment, Formula I of the present invention may be a compound represented by Formula IA below, or a solvate, stereoisomer or pharmaceutically acceptable salt thereof:

[Formula IA]

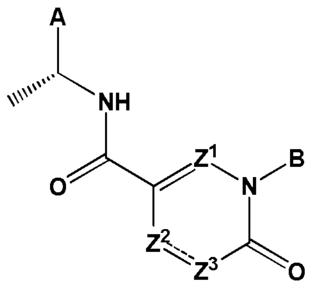

(in Formula IA, A, $Z^1$, $Z^2$, $Z^3$ and B are as defined in Formula I.)

In Formula I above of the present invention, A may be $Cy_1$. In this case, $Cy_1$ may be $C_6$-$C_{10}$ aryl, or 5- or 6-membered heteroaryl containing 1 or 2 heteroatoms selected from N, O and S.

In one embodiment, $Cy_1$ may be $C_6$-$C_{10}$ aryl, or 5- or 6-membered heteroaryl containing 1 or 2 heteroatoms selected from N and S. In one embodiment, $Cy_1$ may be phenyl, naphthalenyl, thiophenyl or pyridinyl.

For example, $Cy_1$ may have any one of the following ring structures optionally substituted with 1 to 3 $R^{2a}$:

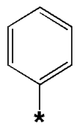 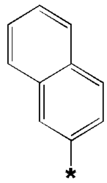 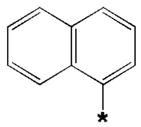 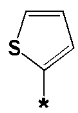 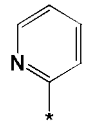.

In Formula I above of the present invention, when A is $Cy_1$, then $Cy_1$ may be optionally substituted with 1 to 3 $R^{2a}$. For example, $Cy_1$ may be substituted with 1, 2 or 3 $R^{2a}$.

$R^{2a}$ may be selected from the group consisting of H, halogen, OH, CN, oxo, $SF_5$, $NR^bR^c$, $-Si(C_{1-3}\ alkyl)_3$, $-SO_2R^b$, $-C(O)R^b$, $C_{1-}C_6$ alkyl, $C_{1-}C_6$ haloalkyl, $C_{1-}C_6$ alkoxy, $C_{1-}C_6$ haloalkoxy, $C_{3-}C_6$ cycloalkyl and

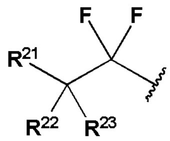,

wherein $R^{21}$ may be H, halogen, OH, $NR^bR^c$, $C_{1-}C_6$ alkoxy or $C_{1-}C_6$ acyloxy, and $R^{22}$ and $R^{23}$ may be each independently H, halogen or $C_{1-}C_2$ alkyl. In this case, $R^b$ and $R^c$ may be each independently H or $C_1$-$C_6$ alkyl.

In one embodiment, each $R^{2a}$ may be independently selected from the group consisting of H, F, Cl, Br, I, OH, CN, $SF_5$, $-Si(CH_3)_3$, $CH_3SO_2$-, methyl, ethyl, propyl, isopropyl, $CF_3$, $CHF_2$, $CH_2F$, $NH_2$, $CH_3NH$-, $(CH_3)_2N$-, methoxy, ethoxy, $OCF_3$, $OCHF_2$, $OCH_2F$, cyclopropyl, cyclobutyl, cyclopentyl, $-CF_2CH_2F$,

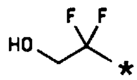, 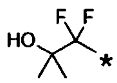, 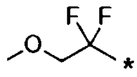, 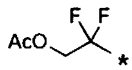,

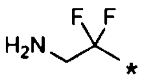, 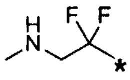 and 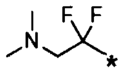,

but is not limited thereto.

When A is $Cy_1$, in Formula I of the present invention, A may be selected from the following structures:

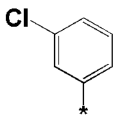 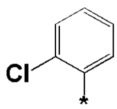 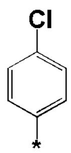 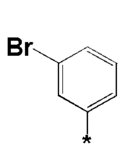 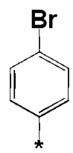 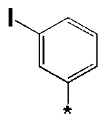 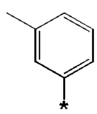 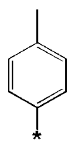 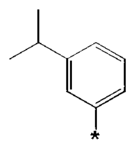

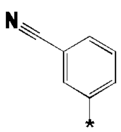 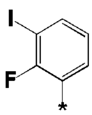 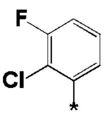 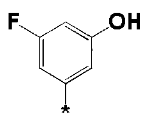 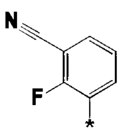 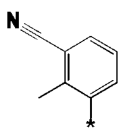 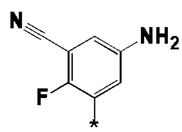 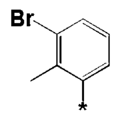

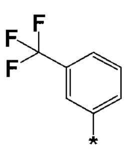 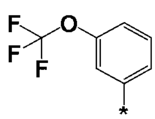 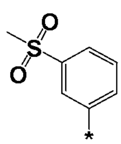 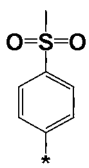 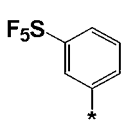 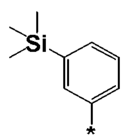 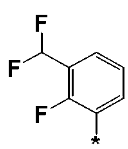

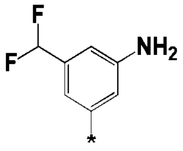 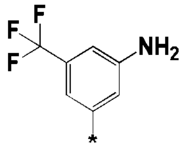 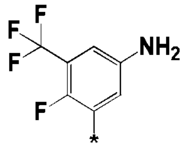 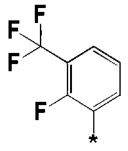 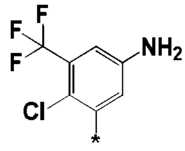 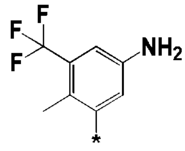

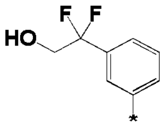 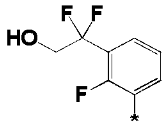 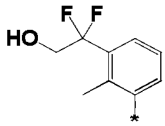 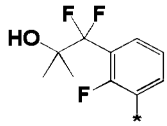 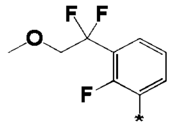 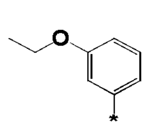

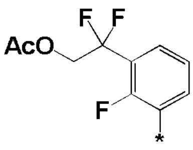 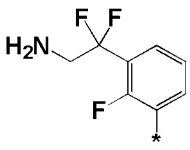 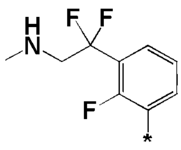 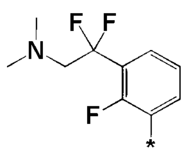 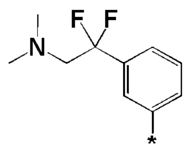 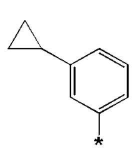

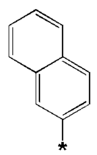 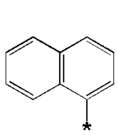 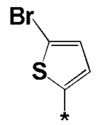 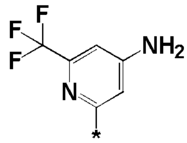 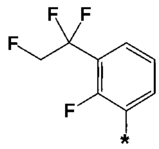 .

For example, in Formula I, A may be selected from the following structures:

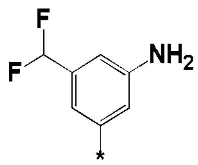 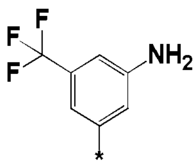 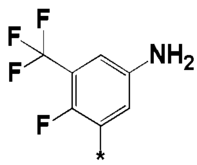 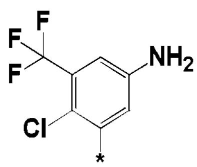 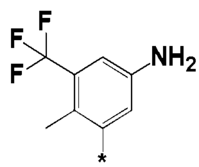

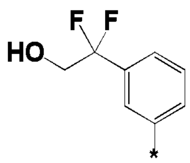 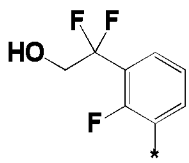 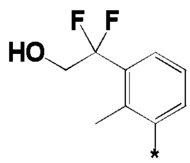 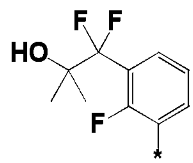 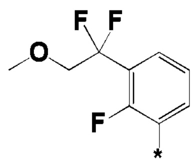 .

For example, in Formula I, A may be

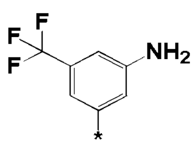 or 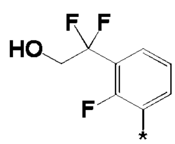 .

In Formula I above of the present invention, A may be $Cy_1$-Y-$Cy_2$. In this case, Y may be O, S, or a direct bond. For example, Y may be O or a direct bond. For example, Y may be a direct bond.

In Formula I, when A is $Cy_1$-Y-$Cy_2$, then $Cy_1$ may be $C_6$-$C_{10}$ aryl, or 5- to 6-membered heteroaryl containing 1 to 3 heteroatoms selected from N, O or S. In one embodiment, $Cy_1$ may be $C_6$-$C_{10}$ aryl, or 5- or 6-membered heteroaryl containing 1 or 2 heteroatoms selected from N, O and S. In one embodiment, it may be $C_6$-$C_{10}$ aryl, or 5- or 6-membered heteroaryl containing 1 or 2 heteroatoms selected from N and S. For example, $Cy_1$ may include phenyl, naphthalenyl, thiazolyl, thiophenyl or pyrazolyl.

In Formula I, when A is $Cy_1$-Y-$Cy_2$, then $Cy_2$ may be $C_6$-$C_{10}$ aryl fused with $C_3$-$C_6$ cycloalkyl, $C_6$-$C_{10}$ aryl, or 5- to 6-membered heteroaryl containing 1 to 3 heteroatoms selected from N, O or S. In one embodiment, $Cy_2$ may be $C_6$-$C_{10}$ aryl, phenyl fused with $C_3$-$C_6$ cycloalkyl, or 5- or 6-membered heteroaryl containing 1 or 2 heteroatoms selected from N, O and S. In another embodiment, $Cy_2$ may be $C_6$-$C_{10}$ aryl, phenyl fused with $C_3$-$C_5$ cycloalkyl, or 5- or 6-membered heteroaryl containing 1 or 2 heteroatoms selected from N and S. For example, $Cy_2$ may include phenyl, 2,3-dihydroindenyl or bicyclo[4.2.0]octa-1,3,5-trienyl, pyrazolyl, thiophenyl, pyridinyl, 2-oxo-1,2-dihydropyridinyl or pyrrolyl.

In one embodiment, $Cy_1$ may be $C_6$-$C_{10}$ aryl, or 5- or 6-membered heteroaryl containing 1 or 2 heteroatoms selected from N and S; Y may be O or a direct bond; and $Cy_2$ may be $C_6$-$C_{10}$ aryl, phenyl fused with $C_3$-$C_5$ cycloalkyl, or 5- or 6-membered heteroaryl containing 1 or 2 heteroatoms selected from N and S.

For example, in Formula I, when A is $Cy_1$-Y-$Cy_2$, then $Cy_1$ may be phenyl, and $Cy_2$ may be phenyl, pyrrolyl, pyrazolyl, thiophenyl, pyridinyl, or 2-oxo-1,2-dihydropyridinyl. In this case, Y may be O or a direct bond. In one embodiment, Y may be a direct bond. In one embodiment, $Cy_1$ may be phenyl, Y may be O, and $Cy_2$ may be phenyl or pyridinyl.

In another embodiment, $Cy_1$ may be thiazolyl, thiophenyl or pyrazolyl, and $Cy_2$ may be phenyl, 2,3-dihydroindenyl or bicyclo[4.2.0]octa-1,3,5-trienyl. For example, $Cy_1$ may be thiophenyl, and $Cy_2$ may be phenyl.

In one embodiment, $Cy_1$-Y-$Cy_2$ may have any one of the following ring structures optionally substituted with $R^{2a}$ and $R^{2b}$:

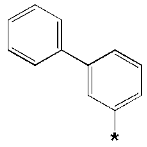 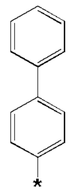 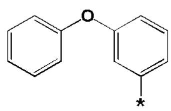 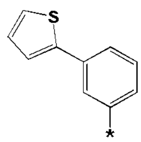 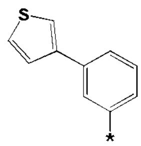 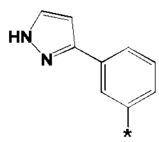 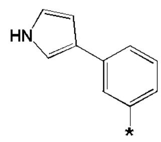

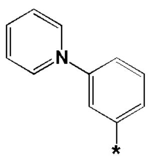 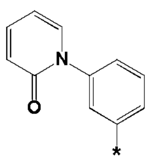 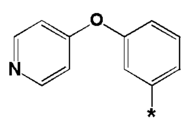 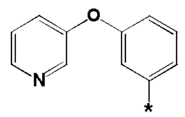 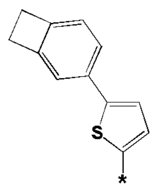 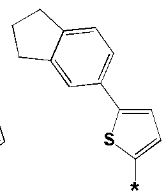

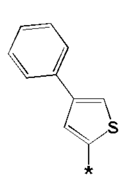 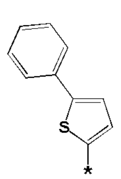 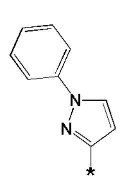 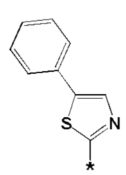 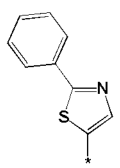 .

In Formula I, when A is $Cy_1$-Y-$Cy_2$, then said $Cy_1$ and $Cy_2$ may be each optionally substituted with 1 to 3 $R^2$. In this case, each $R^2$ may be independently selected from the group consisting of H, halogen, OH, CN, oxo, $SF_5$, -Si($C_1$-$C_3$ alkyl)$_3$, $C_1$-$C_6$ alkylsulfonyl, $C_1$-$C_6$ alkylcarbonyl, amino, $C_1$-$C_6$ alkylamino, di($C_1$-$C_6$ alkyl)amino; $C_1$-$C_6$ alkyl optionally substituted with halogen, CN, OH, $C_1$-$C_6$ alkoxy, amino, $C_1$-$C_6$ alkylamino, di($C_1$-$C_6$ alkyl)amino or hydroxy-($C_1C_6$ alkyl)amino-; $C_1$-$C_6$ alkoxy, $C_1$-$C_6$ haloalkoxy, $C_3$-$C_6$ cycloalkyl and

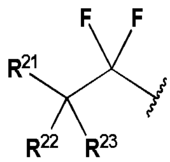 .

In this case, $R^{21}$ may be H, halogen, OH, $C_1$-$C_6$ alkoxy, $C_1$-$C_6$ acyloxy, amino, $C_1$-$C_6$ alkylamino or di($C_1$- $C_6$ alkyl)amino, and $R^{22}$ and $R^{23}$ may be each independently H, halogen or $C_1$-$C_2$ alkyl.

In Formula I, when A is $Cy_1$-Y-$Cy_2$, then said $Cy_1$ may be optionally substituted with 1 to 3 $R^{2a}$. In this case, $R^{2a}$ may be selected from the group consisting of H, halogen, OH, CN, oxo, $SF_5$, $NR^bR^c$, -Si($C_{1-3}$ alkyl)$_3$, -$SO_2R^b$, -C(O)$R^b$, $C_1$-$C_6$ alkyl, $C_1$-$C_6$ haloalkyl, $C_1$-$C_6$ alkoxy, $C_1$-$C_6$ haloalkoxy, $C_3$-$C_6$ cycloalkyl and

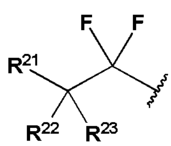 .

Said $R^{21}$ may be H, halogen, OH, $NR^bR^c$, $C_1$-$C_6$ alkoxy or $C_1$-$C_6$ acyloxy, and $R^{22}$ and $R^{23}$ may be each independently H, halogen or $C_1$-$C_2$ alkyl. In one embodiment, $Cy_1$ may be optionally substituted with one $R^{2a}$, and $R^{2a}$ may be H, halogen, OH, CN, amino, $C_1$-$C_6$ alkyl, $C_1$-$C_6$ haloalkyl, $C_1$-$C_6$ alkoxy or $C_1$-$C_6$ haloalkoxy. For example, $R^{2a}$ may include H, halogen, OH or CN. For example, $R^{2a}$ may be H or halogen. For example, $R^{2a}$ may be H.

In Formula I, when A is $Cy_1$-Y-$Cy_2$, then said $Cy_2$ may be optionally substituted with 1 to 3 $R^{2b}$. For example, $Cy_2$ may be optionally substituted with 1 to 3 $R^{2b}$.

In this case, $R^{2b}$ may be selected from the group consisting of H, halogen, OH, CN, oxo, $NR^bR^c$; $C_1$-$C_6$ alkyl; $C_1$-$C_6$ alkyl substituted with halogen, CN, OH, $NR^bR^c$ or $C_1$-$C_6$ alkoxy; $C_1$-$C_6$ alkyl optionally interrupted by 1 to 3 oxygen atoms and/or nitrogen atoms; and $C_1$-$C_6$ alkyl substituted with hydroxy-($C_1$-$C_6$ alkyl)amino-.

For example, each $R^{2b}$ may be independently H, F, Cl, Br, I, OH, CN, oxo, amino, $CH_3NH$-, $(CH_3)_2N$-, $(CH_3)_2NCH_2$- methyl, ethyl, cyanomethyl, hydroxymethyl, aminomethyl, $CH_3NHCH_2$-, $C_2H_5NHCH_2$- or $HOC_2H_4NHCH_2$-, but is not limited thereto. For example, $R^{2b}$ may be H, halogen, $C_1$-$C_6$ alkyl; or $C_1$-$C_6$ alkyl substituted with amino, $C_1$-$C_6$ alkylamino or di($C_1$-$C_6$ alkyl)amino.

When A is $Cy_1$-Y-$Cy_2$, in Formula I of the present invention, A may be selected from the following structures:

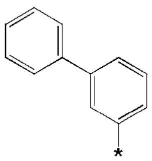
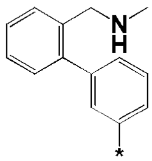
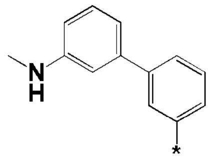
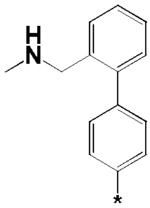
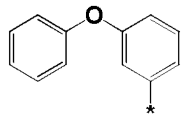
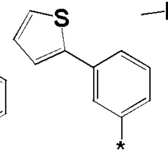
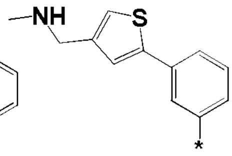

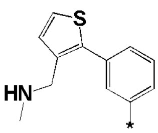
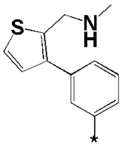
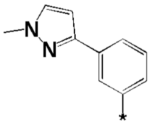
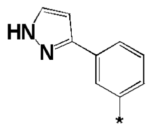
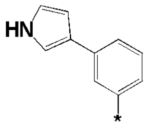
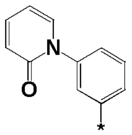
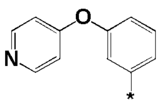
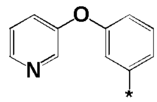

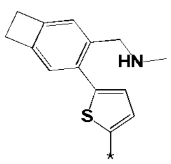
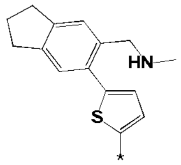
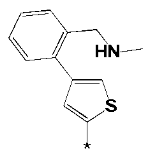
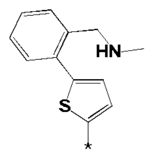
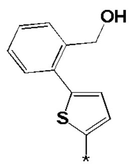
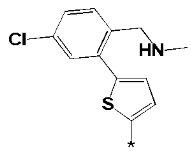
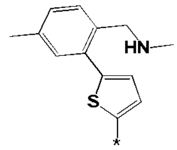

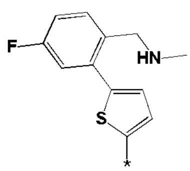
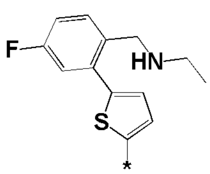
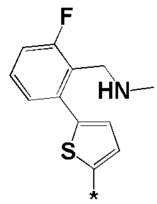
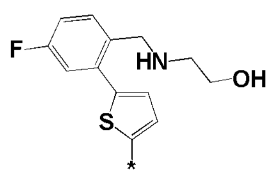
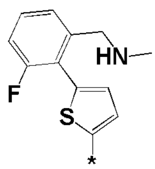
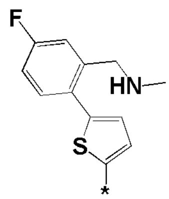

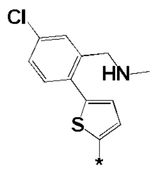
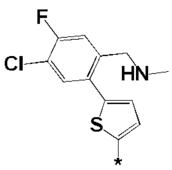
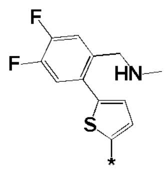
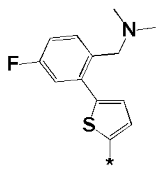
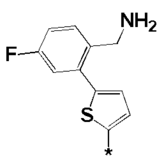
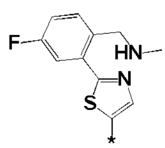
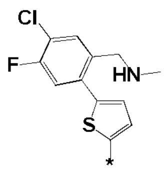

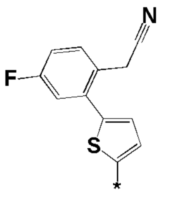 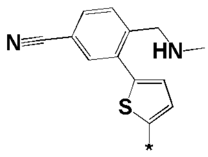 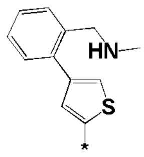 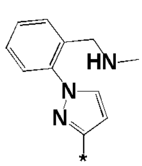 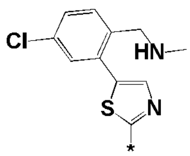 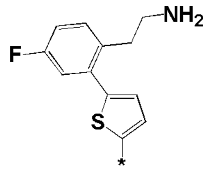 .

For example, in Formula I, A may be selected from the following structures:

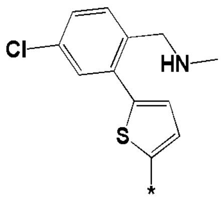 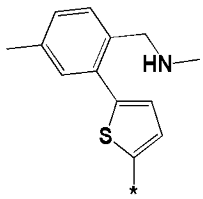 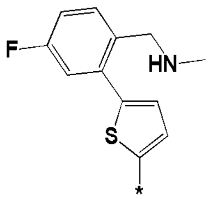 .

In Formula I above of the present invention, B may be H, $C_{1-}C_6$ alkyl, $C_{1-}C_6$ haloalkyl, $C_{1-}$ $C_6$ hydroxyalkyl, $C_{1-}C_6$ alkoxy-$C_{1-}C_6$ alkyl, or $C_{1-}C_6$ alkyl substituted with $NR^bR^c$. In this case, $R^b$ and $R^c$ are each H or $C_1$-$C_6$ alkyl. For example, B may be H, $CH_3$,

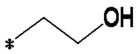 , 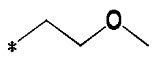 , 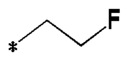 , or 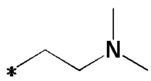 .

In Formula I above of the present invention, B may be $-(CH_2)_o$-$Cy_3$. In this case, o may be 0 or 1.

In Formula I above of the present invention, when B is $-(CH_2)_o$-$Cy_3$, then $Cy_3$ may be selected from the group consisting of $C_{3-}C_8$ cycloalkyl, $C_3$-$C_8$ cycloalkenyl, 5- or 6-membered saturated or partially unsaturated heterocycloalkyl containing 1 or 2 heteroatoms selected from N, O and S, bridged bicyclic $C_{5-10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms selected from N, O and S.

In one embodiment, $Cy_3$ may be selected from the group consisting of $C_3$-$C_8$ cycloalkyl, $C_{3-}C_8$ cycloalkenyl, 6-membered saturated or partially unsaturated heterocycloalkyl containing one N, O or S, bridged bicyclic $C_{5-8}$ cycloalkyl, $C_6$-$C_{10}$ aryl, phenyl fused with 5-membered heterocycloalkyl containing one N, O or S, 5- or 6-membered heteroaryl containing 1 or 2 heteroatoms selected from N or S, and 9- or 10-membered bicyclic heteroaryl containing 1 to 3 N.

For example, $Cy_3$ may be $C_{3-}C_6$ cycloalkyl, $C_{3-}C_6$ cycloalkenyl, tetrahydropyranyl, dihydropyranyl, thianyl, 1,1-dioxothianyl, piperidinyl, dihydropyridinyl, tetrahydropyridinyl, bicyclo[1.1.1]pentanyl, bicyclo[2.2.1]heptanyl, $C_{6-10}$ aryl, thiophenyl, thiazolyl, pyrazolyl, pyridinyl, pyrimidinyl, dihydroisobenzofuranyl, indolyl, indazolyl or benzotriazolyl, but is not limited thereto.

Said $Cy_3$ may include any one of the following ring structures, which may be optionally substituted with $R^{3a}$:

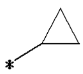 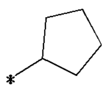 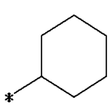 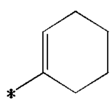 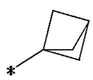 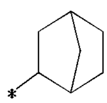 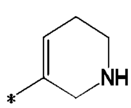 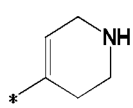

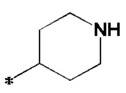 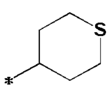 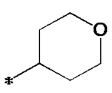 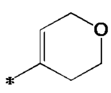 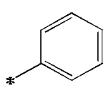 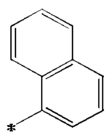 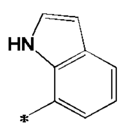 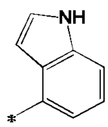

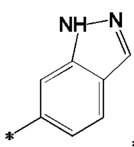 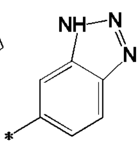 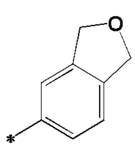 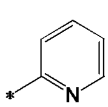 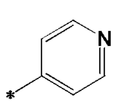 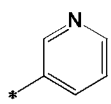 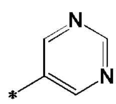 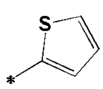

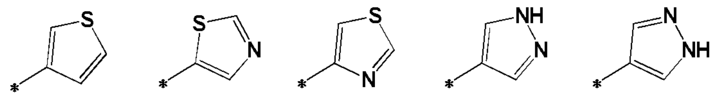

Said $Cy_3$ may be optionally substituted with 1 to 3 $R^{3a}$, wherein $R^{3a}$ may be selected from the group consisting of H, halogen, OH, CN, oxo, $C_{1-}C_6$ alkyl; $C_{1-}C_6$ alkyl substituted with halogen, OH, CN or $C_{1-}C_6$ alkoxy; $C_3$-$C_6$ cycloalkyl, $C_{1-}C_6$ alkoxy, $C_{1-}C_6$ haloalkoxy, $C_{1-}C_6$ haloalkylamino, $C_{1-}C_6$ hydroxyalkylamino, ($C_3$-$C_6$ cycloalkyl)carbonylamino, $-NR^bR^c$, $-NR^bCOR^c$, $-NR^bC(O)OR^c$, $-SO_2R^b$, $-C(O)R^b$, $-C(O)OR^b$, $-NR^bSO_2R^c$ and $-CONR^{b1}R^{c1}$. In this case, $R^b$ and $R^c$ may be each independently H or $C_1$-$C_6$ alkyl. In addition, one of $R^{b1}$ and $R^{c1}$ may be H or $C_{1-}C_6$ alkyl, and the other of $R^{b1}$ and $R^{c1}$ may be H, $C_{1-}C_6$ alkyl, $C_{1-}C_6$ alkyl substituted with $NR^bR^c$, or $C_{1-}C_6$ alkyl substituted with $C_{1-}C_6$ alkoxy.

For example, $R^{3a}$ may include H, F, Cl, Br, I, OH, CN, oxo, methyl, ethyl, amino, $CH_3NH$-, $(CH_3)_2NH$-, 1,1,1-trifluoropropan-2-ylamino, $CH_3CONH$-, $(CH_3CO)(CH_3)N$-, $CH_3OCONH$-, cyclopropylcarbonylamino, hydroxymethyl, 1-hydroxyethyl, 2-hydroxypropan-2-yl, methoxy, ethoxy, isopropoxy, methoxymethyl, 2-methoxyethyl, $OCHF_2$, $OCF_3$, $CH_3SO_2$-, $CH_3CO$-, $CH_3SO_2NH$-, -COOH, $-COOC(CH_3)_3$, $-CONH_2$, $-CONHCH_3$, $-CONHC_2H_5$, $-CON(CH_3)_2$, - $CONHC_2H_4OCH_3$ or $-CONHC_2H_4N(CH_3)_2$, but is not limited thereto.

When B is $-(CH_2)_o$-$Cy_3$, in Formula I of the present invention, B may be selected from the following structures:

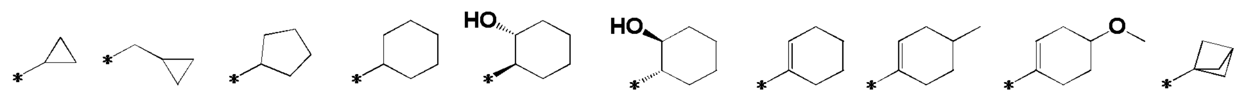

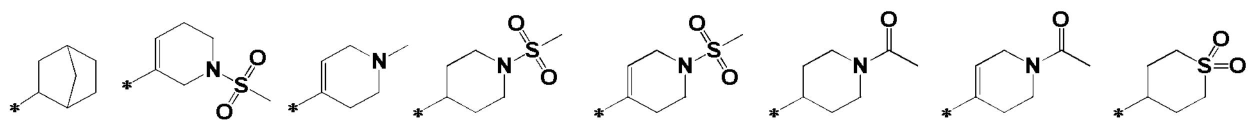

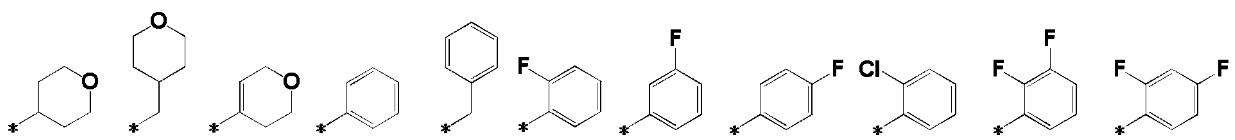

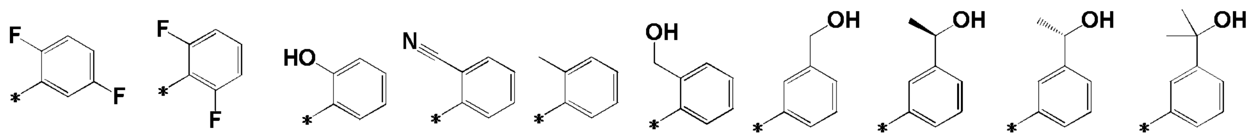

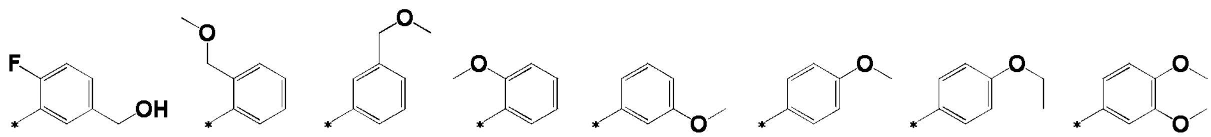

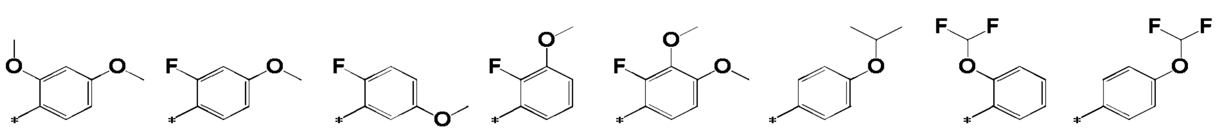

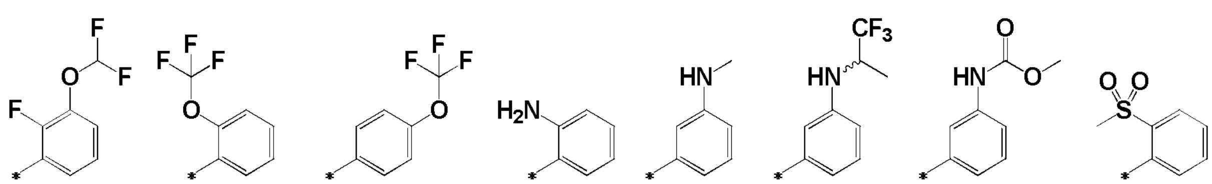

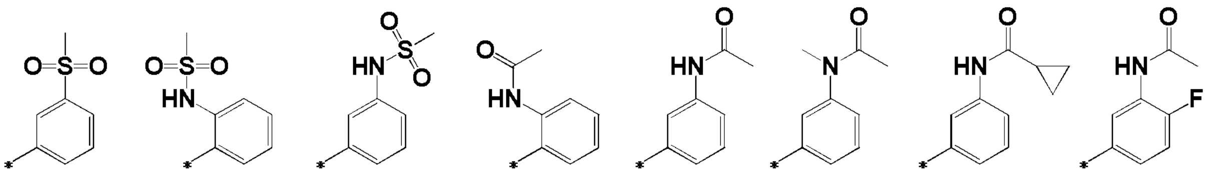

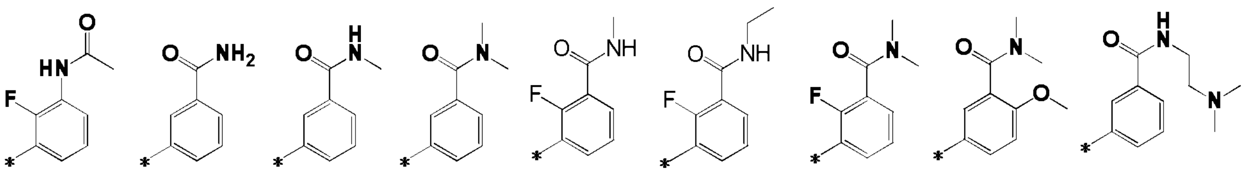

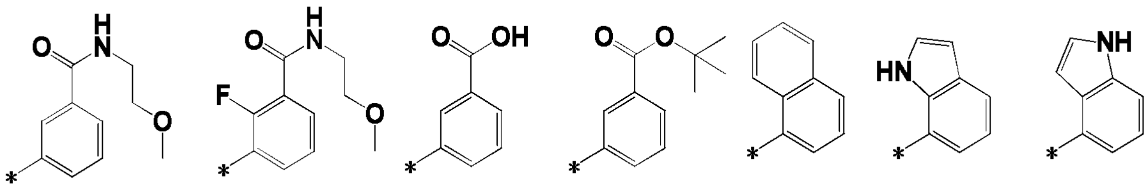

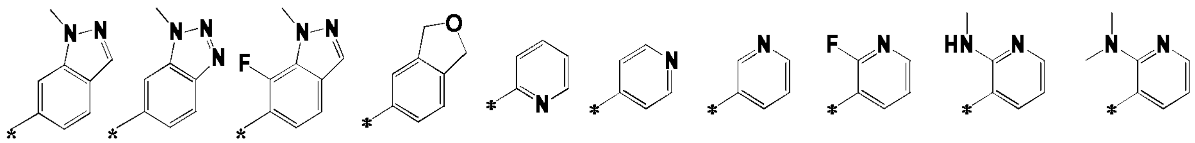

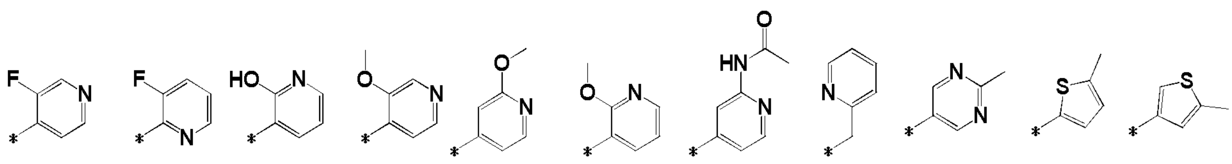

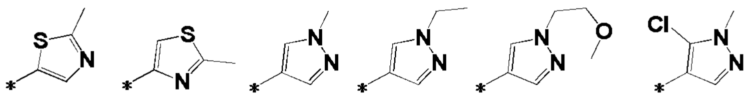

For example, B may be selected from

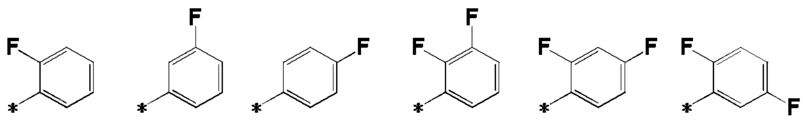 ,

but is not limited thereto. For example, B may be

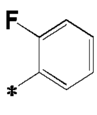 or 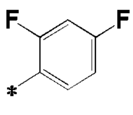 ,

but is not limited thereto.

In Formula I above of the present invention, B may be $-(CH_2)_o-Cy_3-W-Cy_4$. In this case, o may be 0 or 1. For example, o may be 0. In addition, W may be NH, C(O) or a direct bond.

When B is $-(CH_2)_o-Cy_3-W-Cy_4$, then $Cy_3$ may be selected from the group consisting of $C_3$-$C_8$ cycloalkyl, $C_3$-$C_8$ cycloalkenyl, 5- or 6-membered saturated or partially unsaturated heterocycloalkyl containing 1 or 2 heteroatoms selected from N, O and S, bridged bicyclic $C_{5-10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms

selected from N, O and S.

In one embodiment, $Cy_3$ may be $C_6$-$C_{10}$ aryl, or 5- or 6-membered heteroaryl containing 1 or 2 heteroatoms selected from N or S.

When B is $-(CH_2)_o$-$Cy_3$-W-$Cy_4$, then $Cy_4$ may be selected from the group consisting of saturated or partially unsaturated 4- to 10-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S.

In one embodiment, $Cy_4$ may be selected from the group consisting of saturated or partially unsaturated 4- to 7-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S.

In one embodiment, B may be $-(CH_2)_o$-$Cy_3$-W-$Cy_4$, and $Cy_3$ may be phenyl or pyridinyl. In addition, $Cy_4$ may be oxetanyl, tetrahydrofuranyl, pyrrolidinyl, 2-oxo-pyrrolidinyl, piperidinyl, morpholinyl, imidazolidinyl, 2-oxo-imidazolidinyl, piperazinyl, 2-oxo-piperazinyl, hexahydropyrimidinyl, 2-oxo-hexahydropyrimidinyl, phenyl, oxazolyl, isoxazolyl, thiazolyl, pyrazolyl, imidazolyl, oxadiazolyl, triazolyl, tetrazolyl, pyridinyl or 2-oxo-pyridinyl. For example, $Cy_3$ may be phenyl, and $Cy_4$ may be pyrazolyl, imidazolyl, triazolyl or tetrazolyl. For example, $Cy_3$ may be phenyl, and $Cy_4$ may be triazolyl. For example, $Cy_3$ may be pyridinyl, and $Cy_4$ may be triazolyl.

In one embodiment, W may be NH, C(O) or a direct bond. For example, W may be a direct bond.

In one embodiment, $Cy_3$ may be $C_6$-$C_{10}$ aryl, $Cy_4$ may be saturated or partially unsaturated 4- to 7-membered heterocycloalkyl containing 1 or 2 heteroatoms selected from N, O or S, and W may be NH or C(O).

Said $Cy_3$-W-$Cy_4$ may include any one of the following ring structures, and the rings corresponding to $Cy_3$ and $Cy_4$ may be optionally substituted with $R^{3a}$ and $R^{3b}$, respectively:

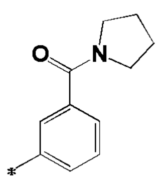 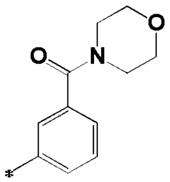 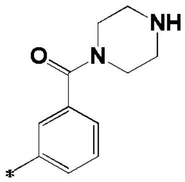 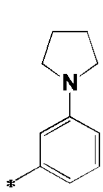 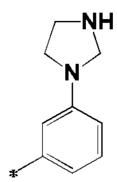 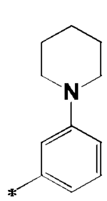 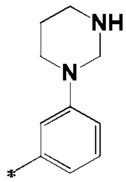 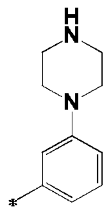

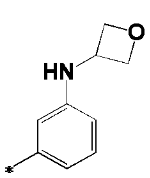 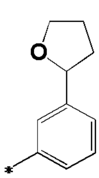 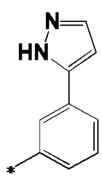 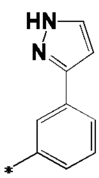 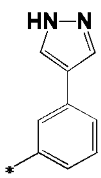 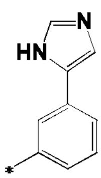 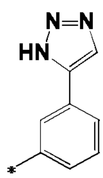 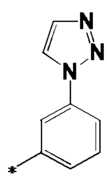 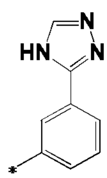

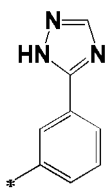 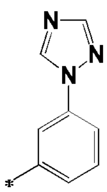 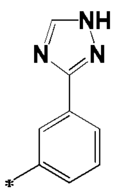 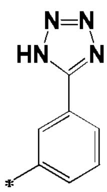 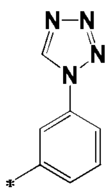 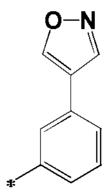 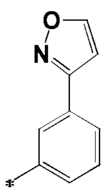 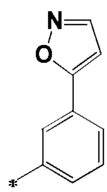 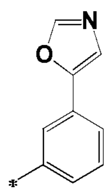

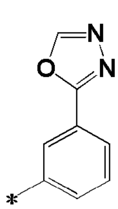 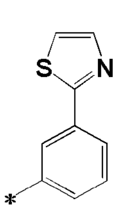 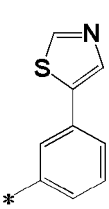 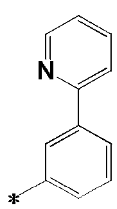 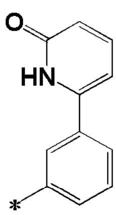 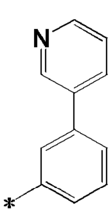 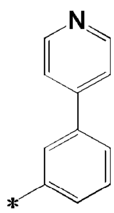 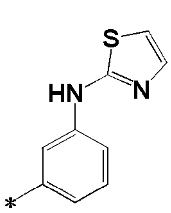

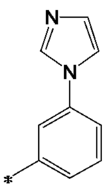 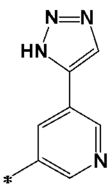 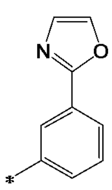 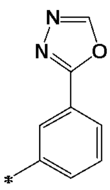 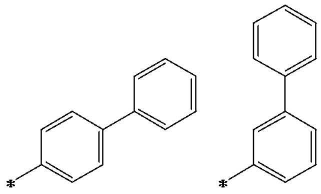 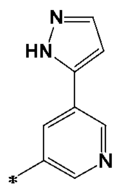 .

In one embodiment, said $Cy_3$ and $Cy_4$ may be each independently optionally substituted with 1 to 3 $R^3$.

When B is $-(CH_2)_o-Cy_3-W-Cy_4$, then said $R^3$, which is each independently substituted on $Cy_3$ and $Cy_4$, may be H, deuterium, halogen, OH, CN, oxo, $C_{1-}C_6$ alkyl, $C_{1-}C_6$ alkyl substituted with deuterium, $C_{1-}C_6$ haloalkyl, $C_{1-}C_6$ hydroxyalkyl, $C_{1-}C_6$ alkoxy-$C_{1-}C_6$ alkyl, $C_{1-}C_6$ alkoxy, $C_{1-}$ $C_6$ haloalkoxy, $C_{1-}C_6$ haloalkylamino, ($C_3$-$C_6$ cycloalkyl)carbonylamino, $-NR^bR^c$, $-NR^bCOR^c$, - $NR^bC(O)OR^c$, $-SO_2R^b$, $-C(O)R^b$, $-C(O)OR^b$, $-NR^bSO_2R^c$ or $-CONR^{b1}R^{c1}$. In this case, $R^b$ and $R^c$ may be each independently H or $C_1$-$C_6$ alkyl. In addition, one of $R^{b1}$ and $R^{c1}$ may be H or $C_{1-}C_6$ alkyl, and the other may be H, $C_{1-}C_6$ alkyl; or $C_{1-}C_6$ alkyl substituted with amino, $C_{1-}C_6$ alkylamino, di($C_{1-}C_6$ alkyl)amino or $C_{1-}C_6$ alkoxy.

In one embodiment, $Cy_3$ may be optionally substituted with 1 to 3 $R^{3a}$. $R^{3a}$ may be selected from the group consisting of H, halogen, OH, CN, oxo, $C_{1-}C_6$ alkyl; $C_{1-}C_6$ alkyl substituted with halogen, OH, CN or $C_{1-}C_6$ alkoxy; $C_3$-$C_6$ cycloalkyl, $C_{1-}C_6$ alkoxy, $C_{1-}C_6$ haloalkoxy, $C_{1-}C_6$ haloalkylamino, $C_{1-}C_6$ hydroxyalkylamino, ($C_3$-$C_6$ cycloalkyl)carbonylamino, $-NR^bR^c$, $-NR^bCOR^c$, $-NR^bC(O)OR^c$, $-SO_2R^b$, $-C(O)R^b$, $-C(O)OR^b$, $-NR^bSO_2R^c$ and $-CONR^{b1}R^{c1}$. In this case, $R^b$ and $R^c$ may be each independently H or $C_1$-$C_6$ alkyl; and one of $R^{b1}$ and $R^{c1}$ may be H or $C_{1-}C_6$ alkyl, and the other of $R^{b1}$ and $R^{c1}$ may be H, $C_{1-}C_6$ alkyl, $C_{1-}C_6$ alkyl substituted with $NR^bR^c$, or $C_{1-}C_6$ alkyl substituted with $C_{1-}C_6$ alkoxy.

In one embodiment, said $Cy_3$ may be optionally substituted with one or two $R^{3a}$. In this case, $R^{3a}$ may be H, halogen, OH, CN, oxo, amino, $C_{1-}C_6$ alkyl, $C_{1-}C_6$ haloalkyl or $C_{1-}C_6$ haloalkoxy. For example, $R^{3a}$ may include H, halogen, OH or CN, but is not limited thereto. For example, $R^{3a}$ may be H or F, but is not limited thereto. For example, $R^{3a}$ may be H.

Said $Cy_4$ may be optionally substituted with 1 to 3 $R^{3b}$. In this case, $R^{3b}$ may be H, deuterium, halogen, OH, CN, oxo, $NR^bR^c$, $C_{1-}C_6$ alkyl, $C_{1-}C_6$ alkyl substituted with deuterium, $C_{1-}$ $C_6$ haloalkyl, $C_{1-}C_6$ hydroxyalkyl, $C_{1-}C_6$ alkoxy-$C_{1-}C_6$ alkyl, $C_{1-}C_6$ alkoxy or $C_{1-}C_6$ haloalkoxy. In this case, $R^b$ and $R^c$ are H or $C_{1-}C_6$ alkyl. For example, $R^{3b}$ may include H, deuterium, halogen, OH, CN, oxo, $C_{1-}C_6$ alkyl, $C_{1-}C_6$ alkyl substituted with deuterium or $C_{1-}C_6$ haloalkyl, but is not limited thereto. For example, $R^{3b}$ may be H or $C_{1-}C_6$ alkyl. For example, $R^{3b}$ may include H, F, oxo, methyl, ethyl, $CHF_2$ and $CD_3$, but is not limited thereto. For example, $R^{3b}$ may be H or methyl.

In one embodiment, $Cy_3$ may be optionally substituted with one or two $R^{3a}$, wherein $R^{3a}$ may be H, halogen, OH or CN; and $Cy_4$ may be optionally substituted with 1 to 3 $R^{3b}$, wherein $R^{3b}$ may be H, deuterium, halogen, OH, CN, oxo, $C_{1-}C_6$ alkyl, $C_{1-}C_6$ alkyl substituted with deuterium or $C_{1-}C_6$ haloalkyl.

When B is $-(CH_2)_o-Cy_3-W-Cy_4$, then in Formula I, B may be selected from the following structures:

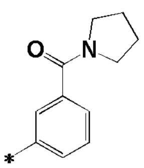 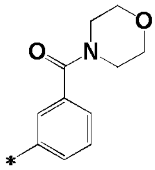 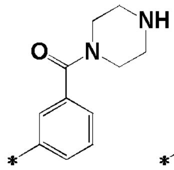 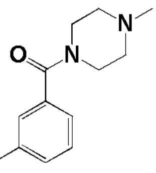 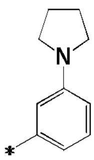 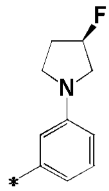 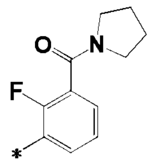 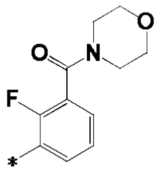

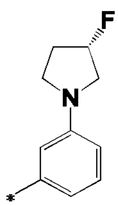 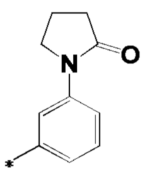 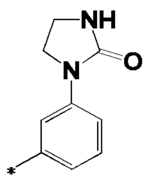 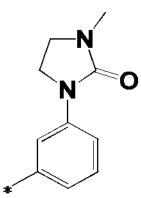 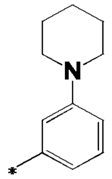 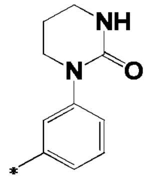 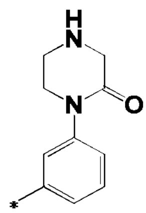 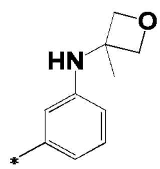

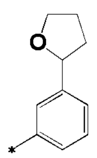 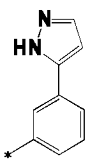 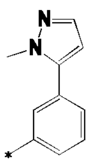 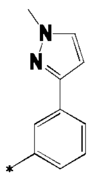 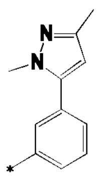 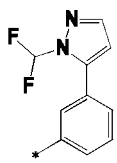 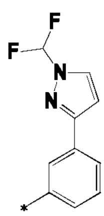 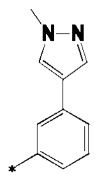 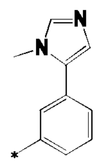 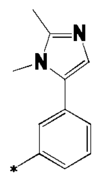

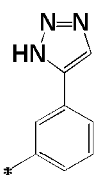 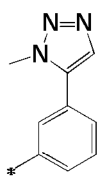 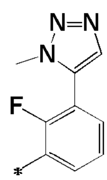 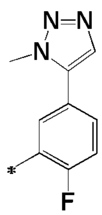 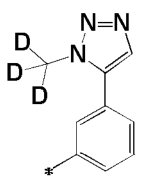 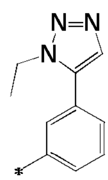 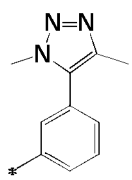 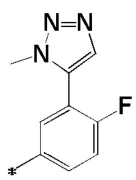

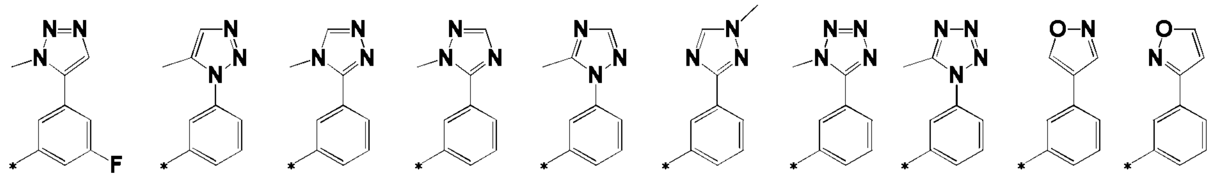

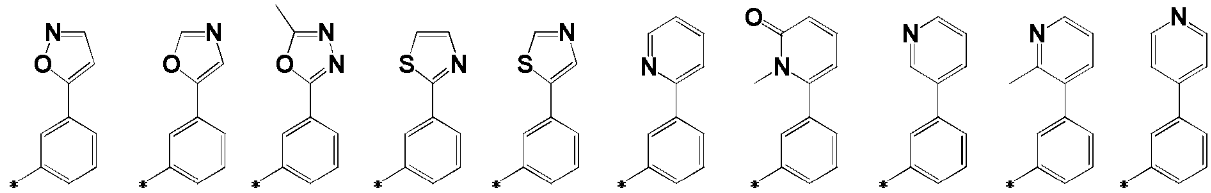

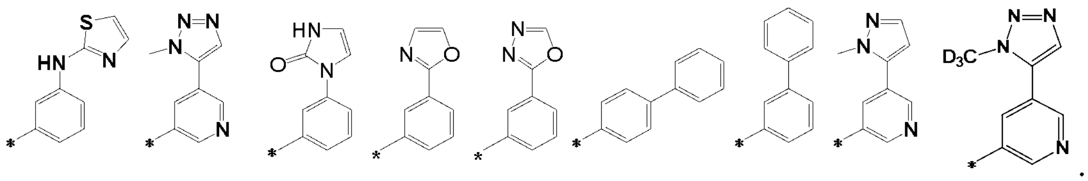

For example, B may be selected from the following structures:

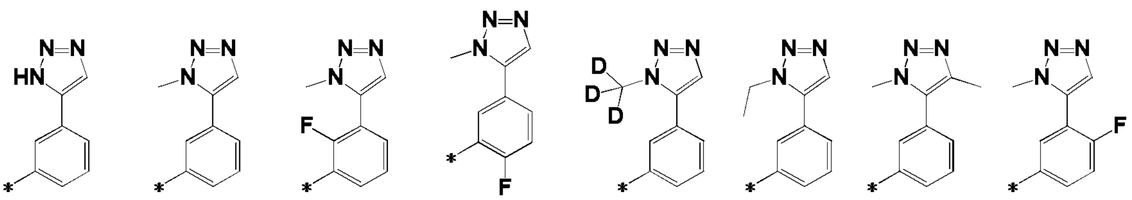

For example, B may be

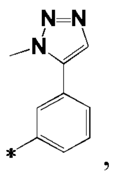

but is not limited thereto.

In one embodiment, Formula I of the present invention may be represented by any one of Formulas I-1, I-2, I-3, I-4, I-5, I-6, and I-7:

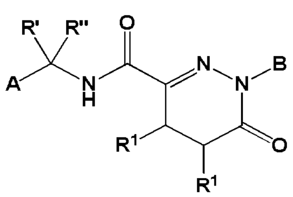

**Formula I-1**

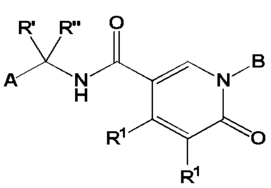

**Formula I-2**

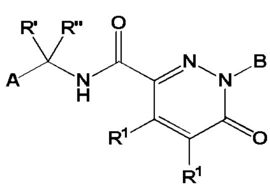

**Formula I-3**

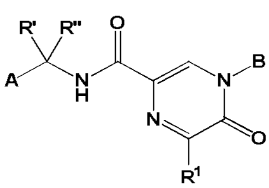

**Formula I-4**

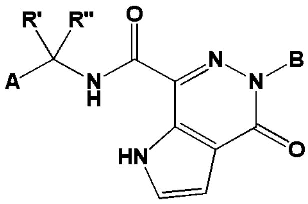
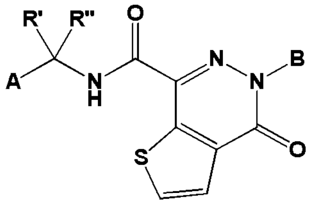
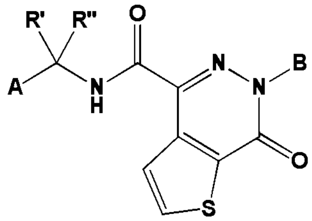

Formula I-5 Formula I-6 Formula I-7

(In Formulas I-1, I-2, I-3, I-4, I-5, I-6, and I-7, A, R', R", $R^1$ and B are as defined in Formula I, and each $R^1$ may be the same or different from each other.)

In some embodiments, the compound of Formula I may be a compound selected from the group consisting of the following compounds:

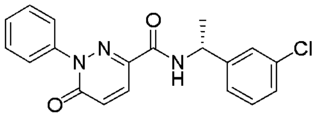 , 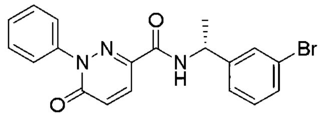 , 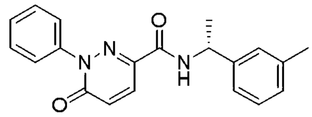 ,

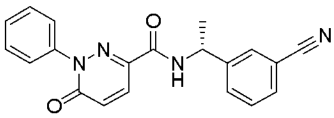 , 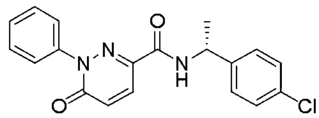 , 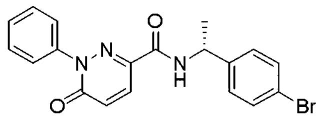 ,

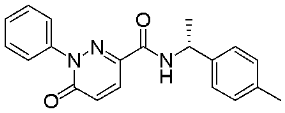 , 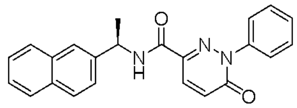 , 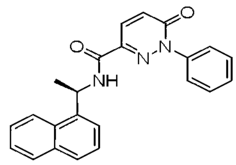 , 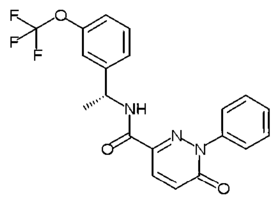 ,

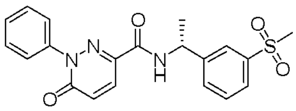 , 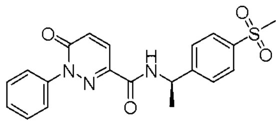 , 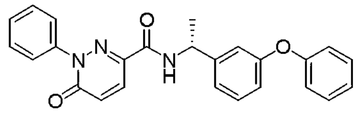 ,

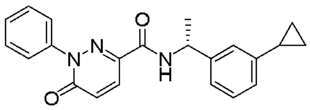 , 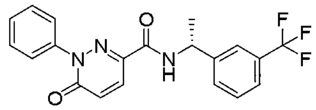 , 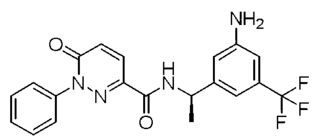 ,

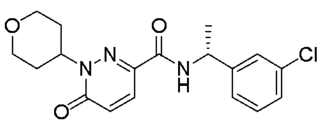 , 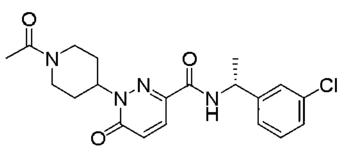 , 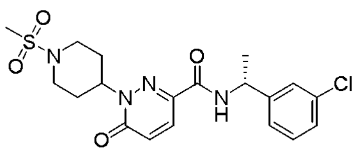 ,

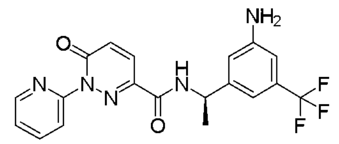,

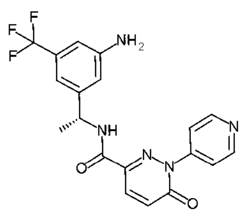,

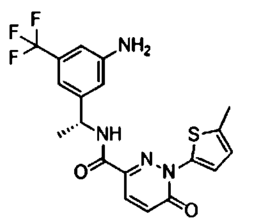,

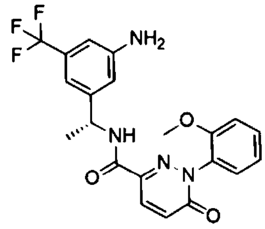,

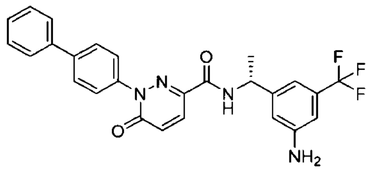,

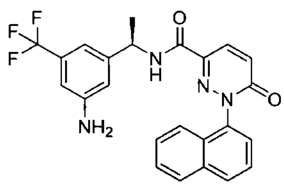,

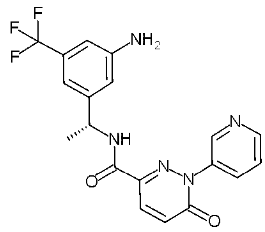,

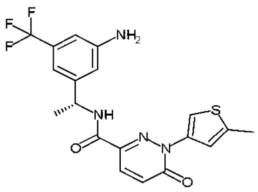,

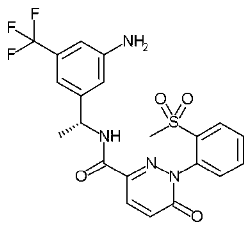,

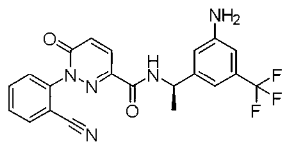,

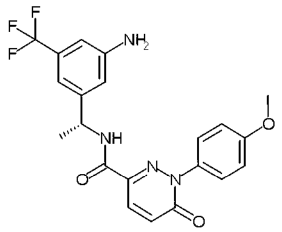,

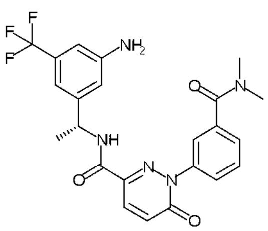,

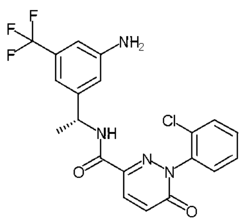,

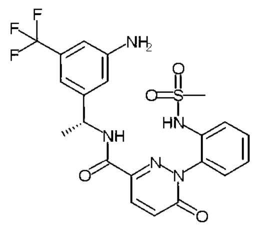,

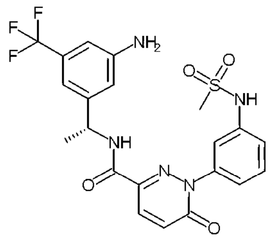,

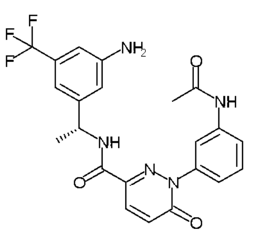,

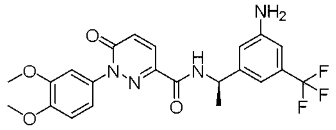,

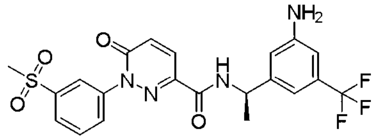,

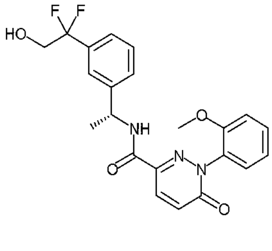,

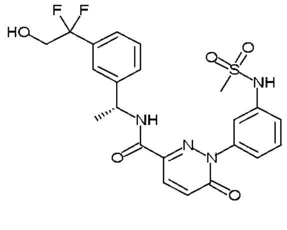,

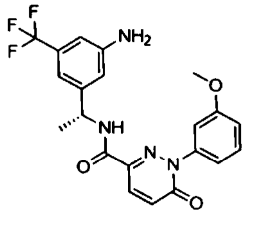,

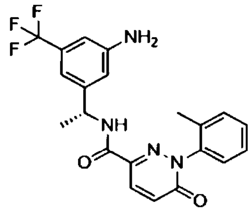,

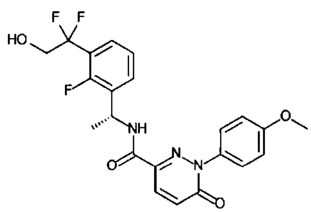
,
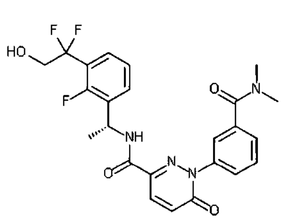
,
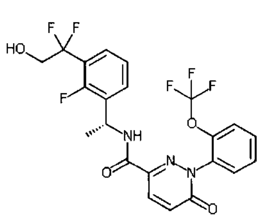
,
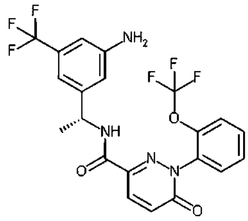
,

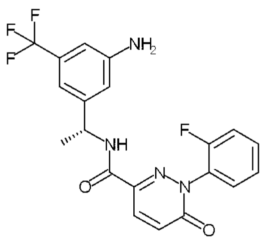
,
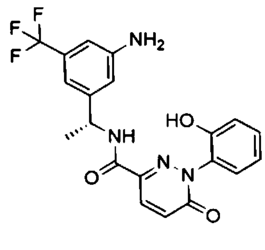
,
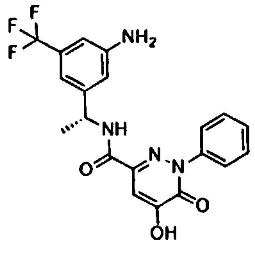
,
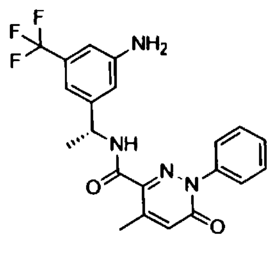
,

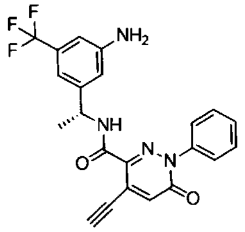
,
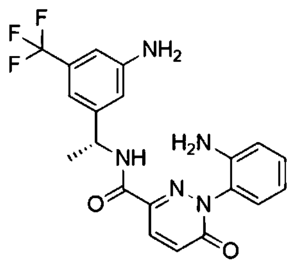
,
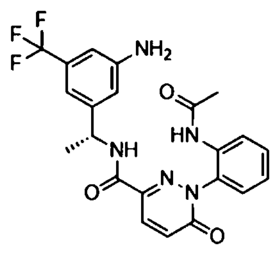
,
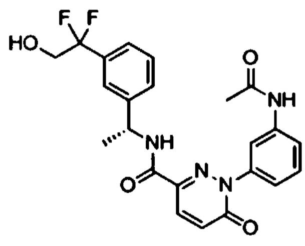
,

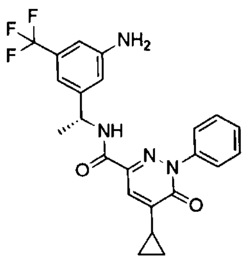
,
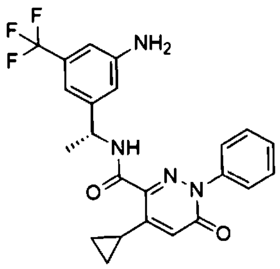
,
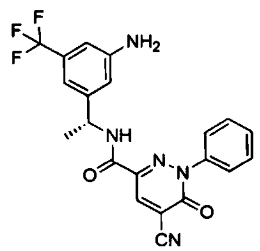
,
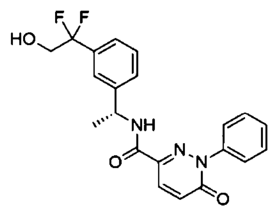
,

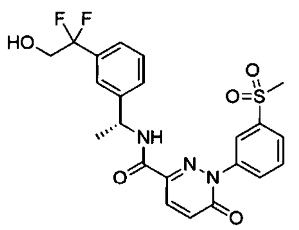
,
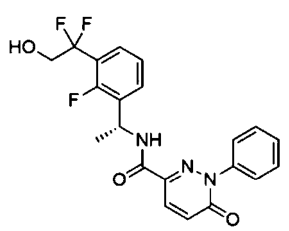
,
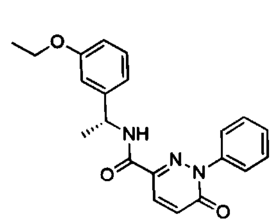
,
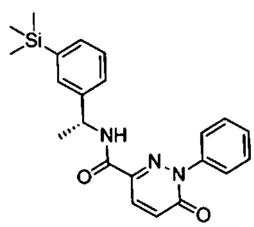
,

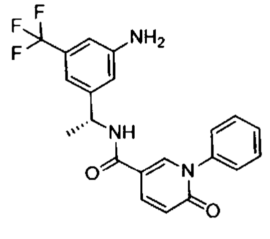
,
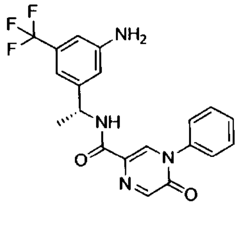
,
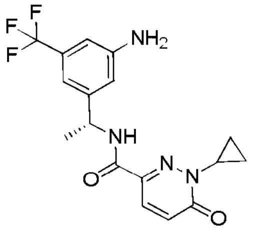
,
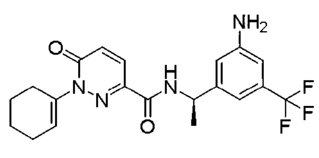
,

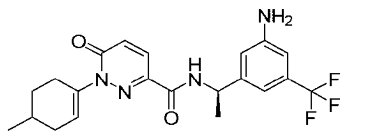, 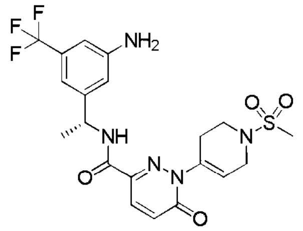, 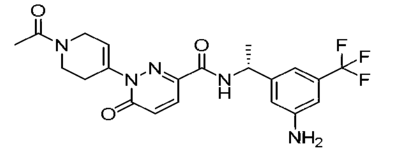,

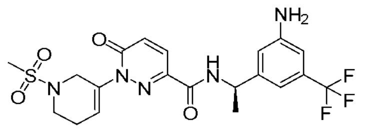, 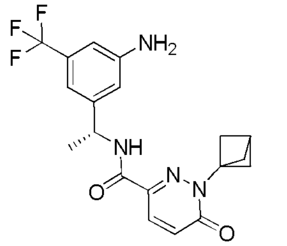, 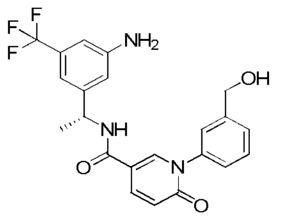,

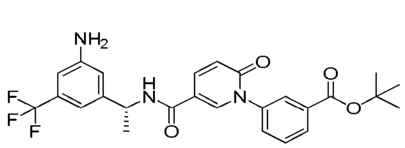, 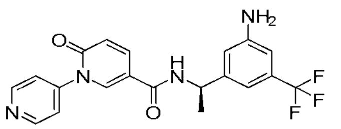, 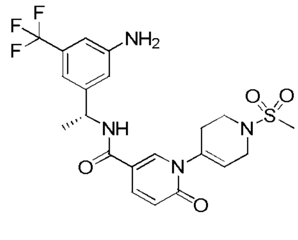,

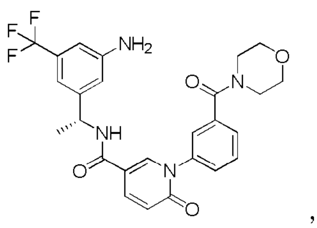, 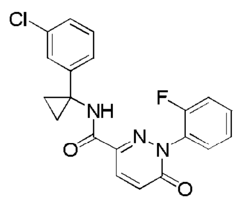, 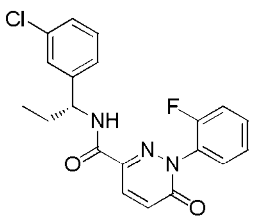, 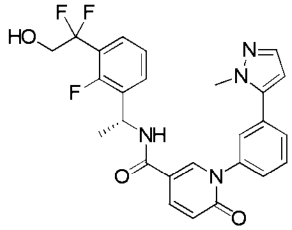,

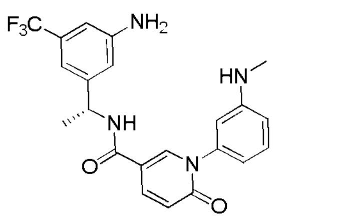, 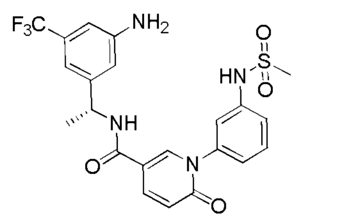, 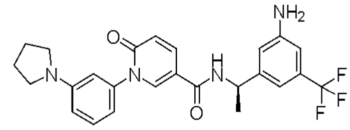,

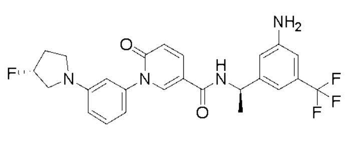, 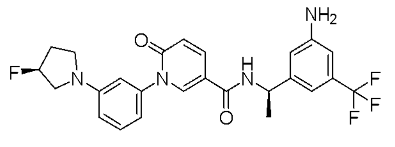, 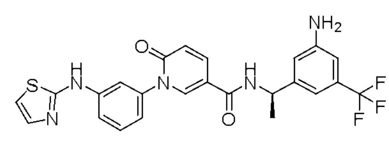,

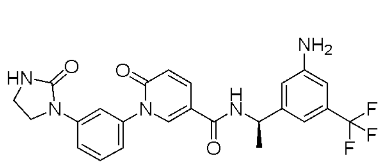, 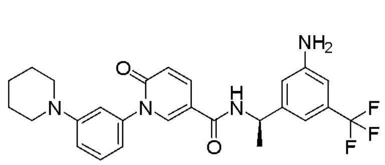, 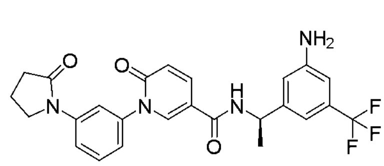,

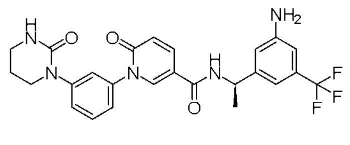

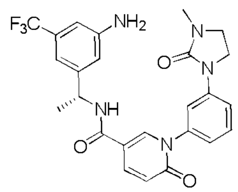

,

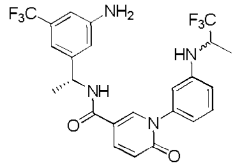

,

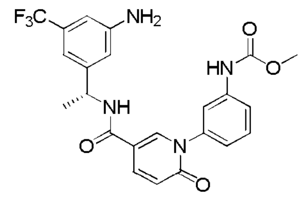

,

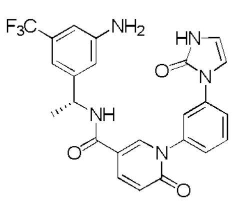

,

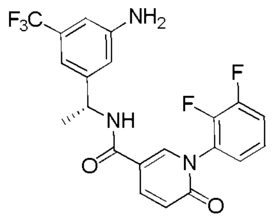

,

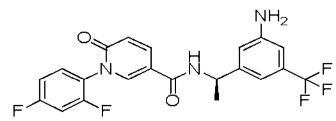

,

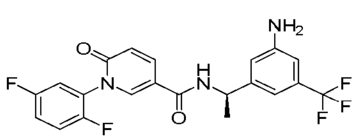

,

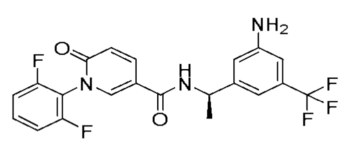

,

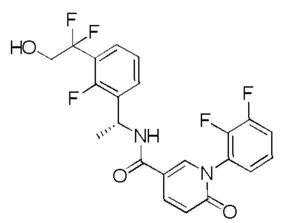

,

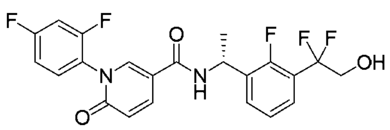

,

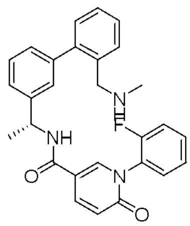

,

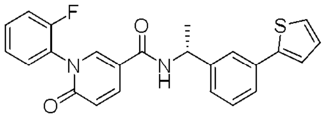

,

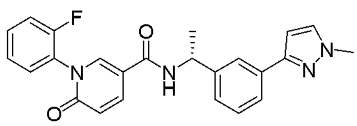

,

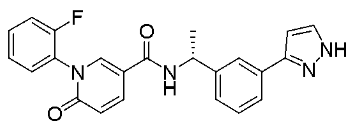

,

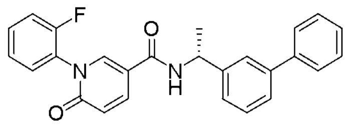

,

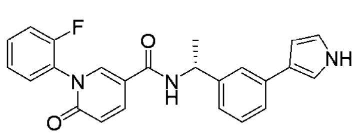

,

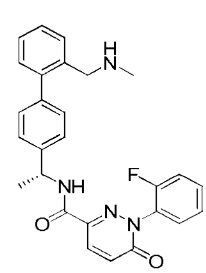

,

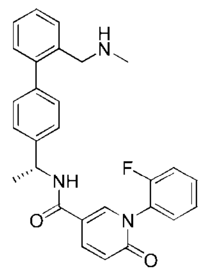

,

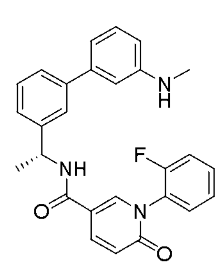

,

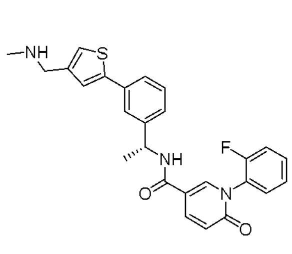

,

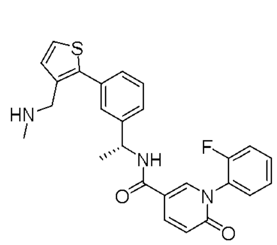

,

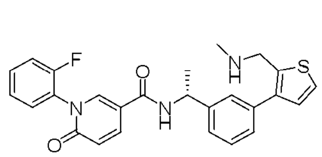

,

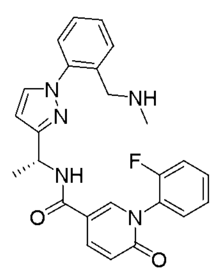

,

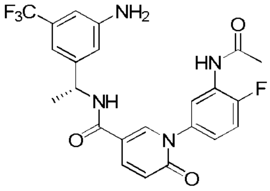 , 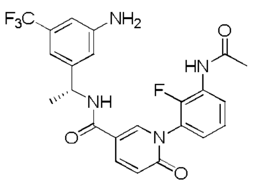 , 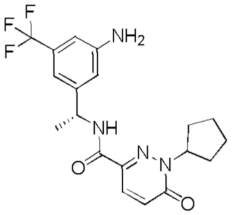 , 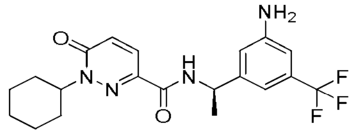 ,

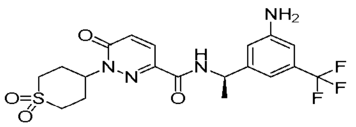 , 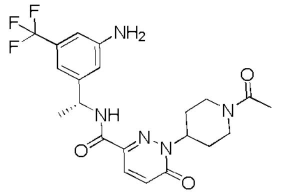 , 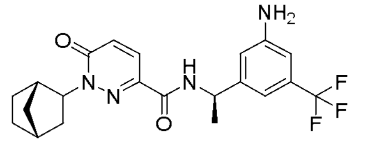 ,

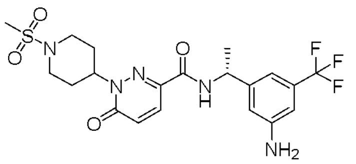 , 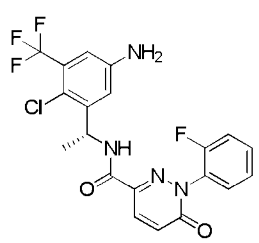 , 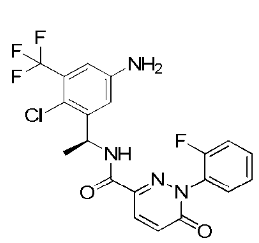 , 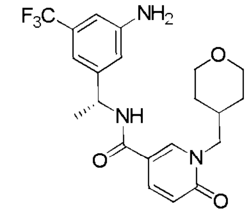 ,

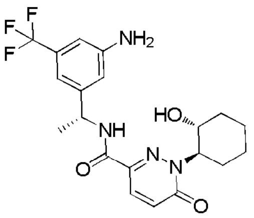 , 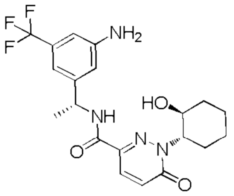 , 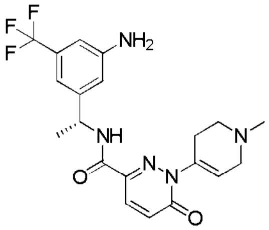 , 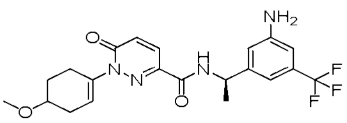 ,

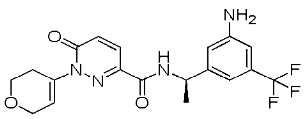 , 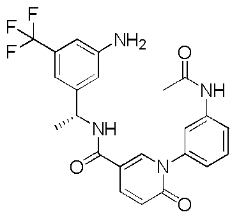 , 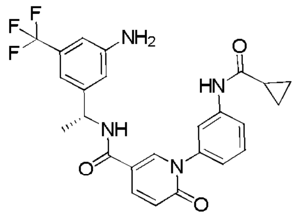 , 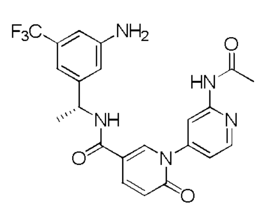 ,

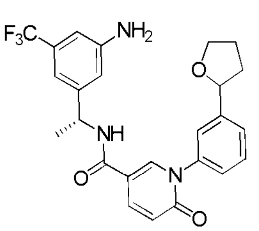 , 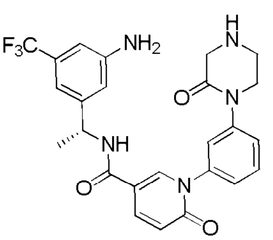 , 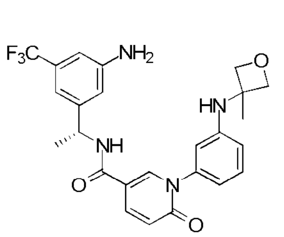 , 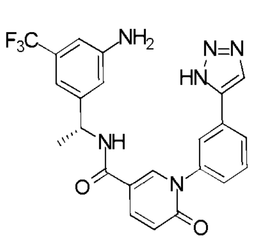 ,

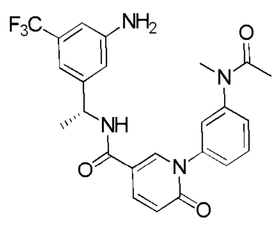,
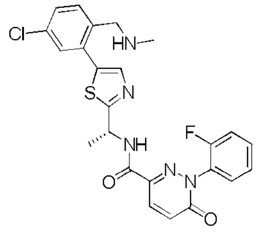,
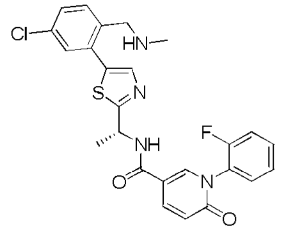,
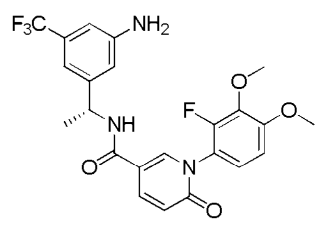,

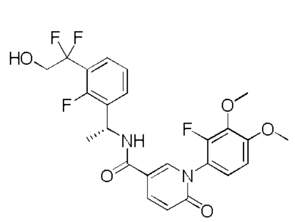,
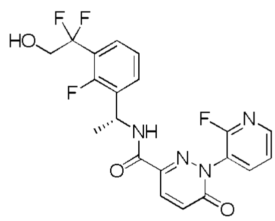,
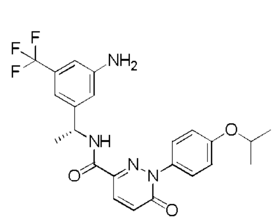,
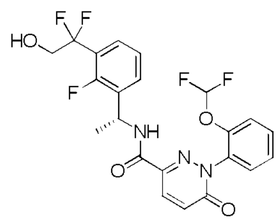,

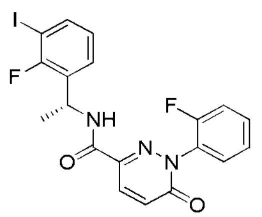,
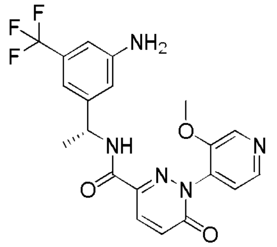,
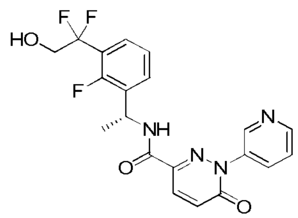,
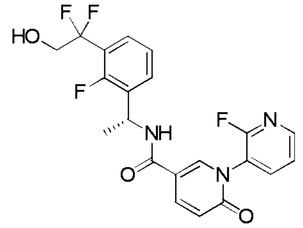,

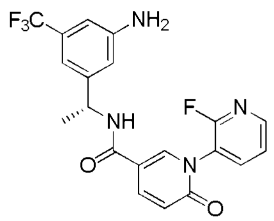,
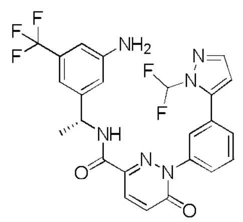,
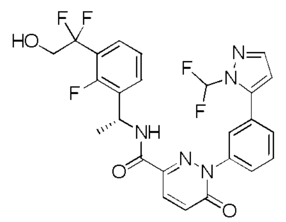,
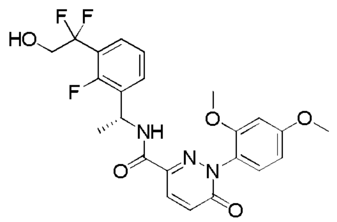,

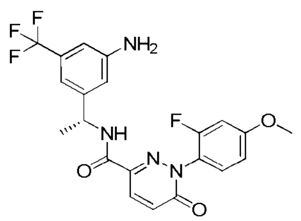,
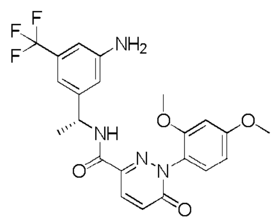,
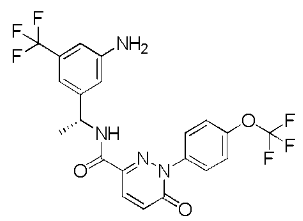,
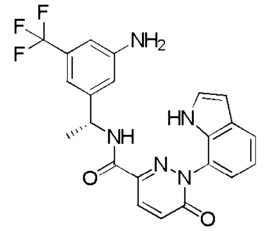,

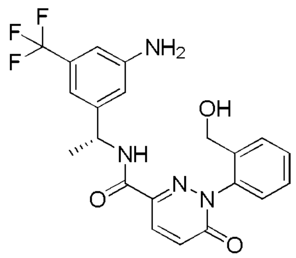,
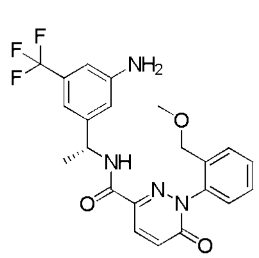,
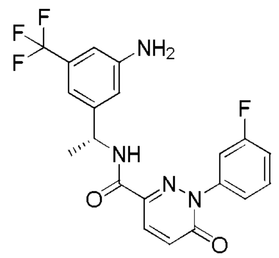,
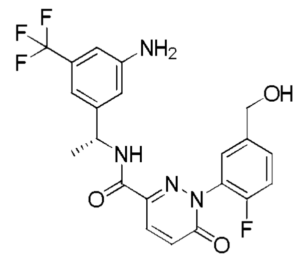,

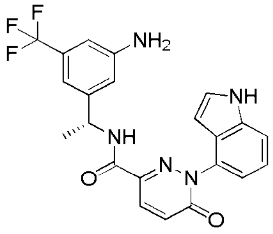,
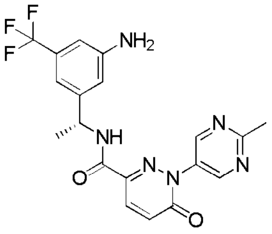,
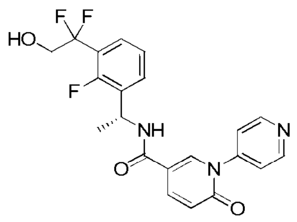,
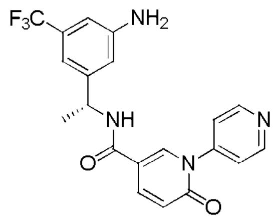,

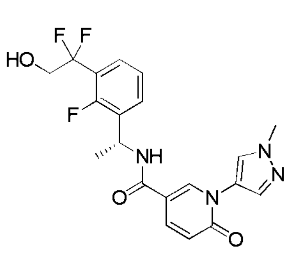,
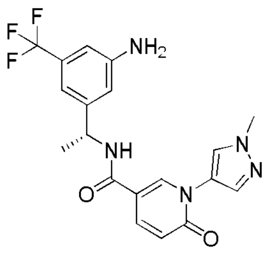,
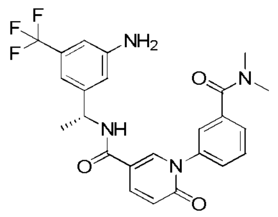,
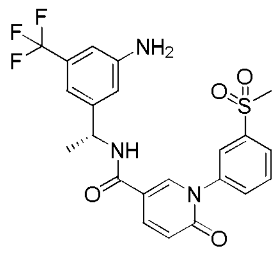,

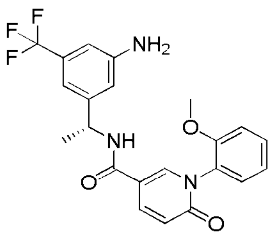,
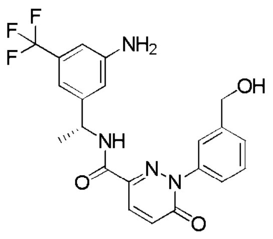,
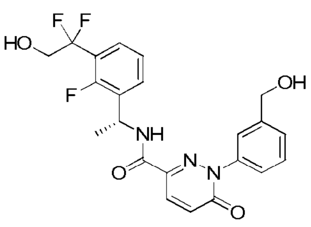,
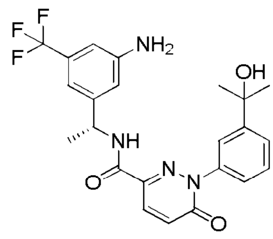,

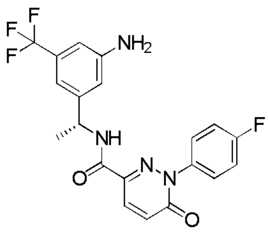,
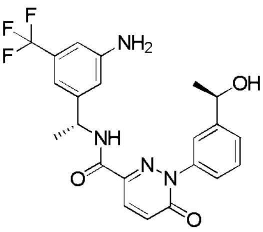,
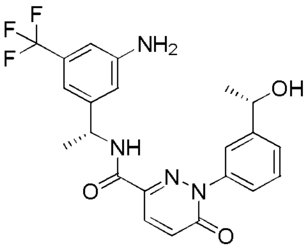,
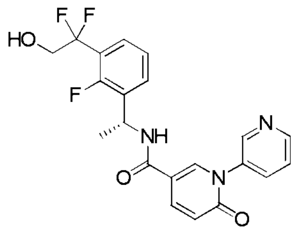,

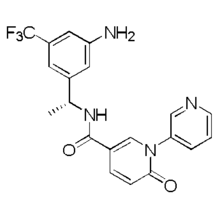,
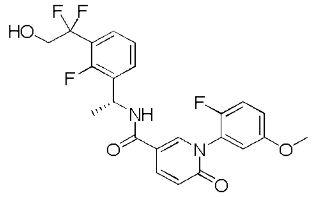,
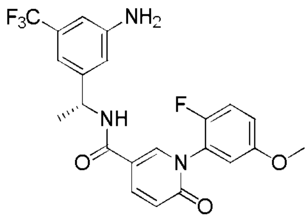,
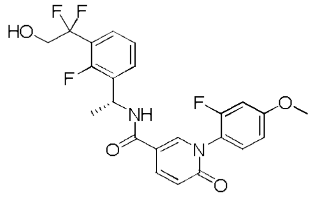,

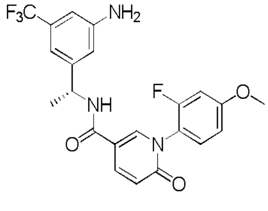,
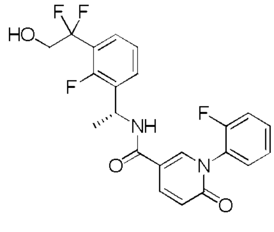,
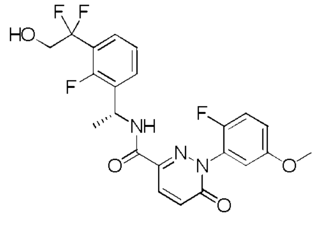,
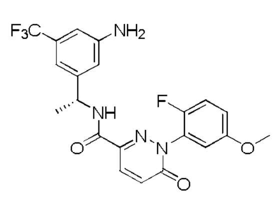,

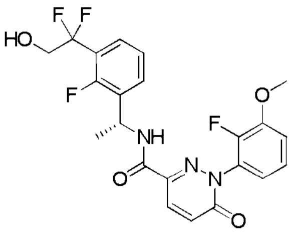,
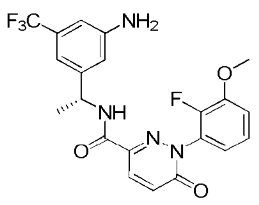,
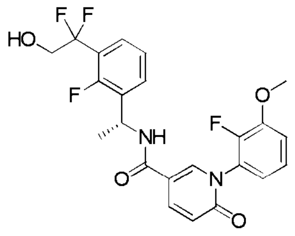,
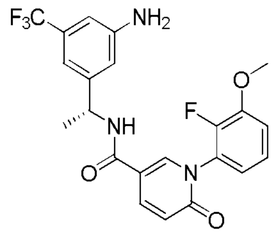,

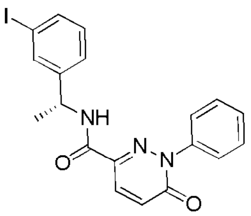,
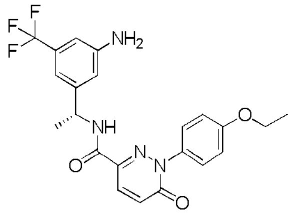,
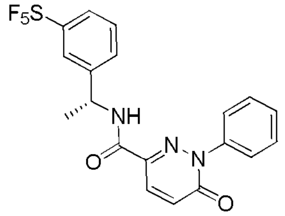,
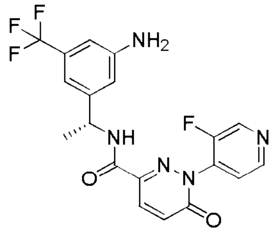,

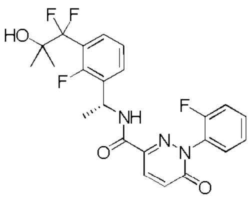,
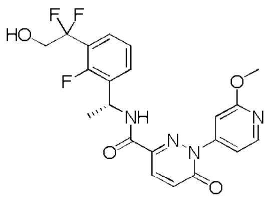,
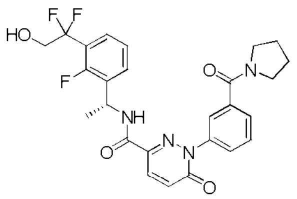,
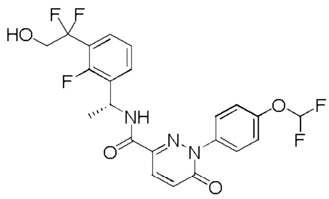,

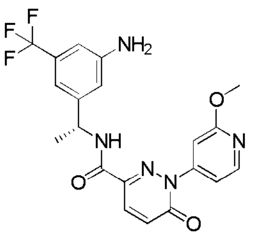,
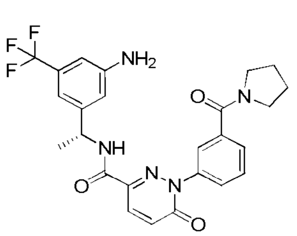,
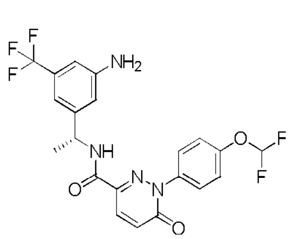,
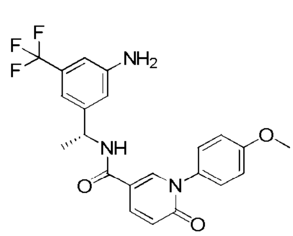,

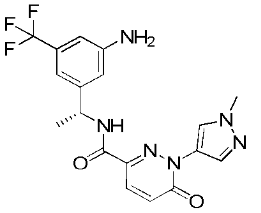,
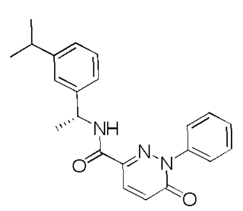,
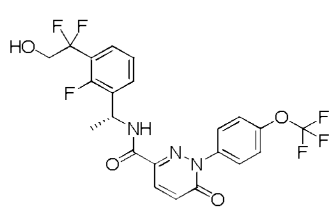,
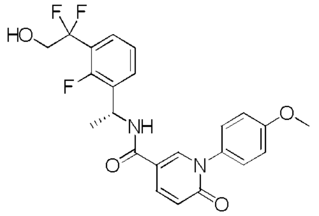,

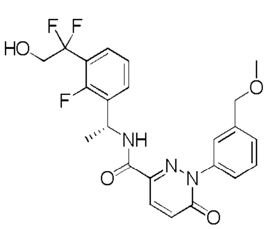,
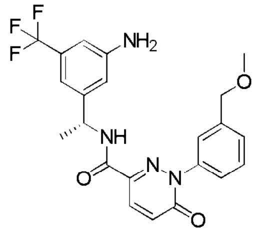,
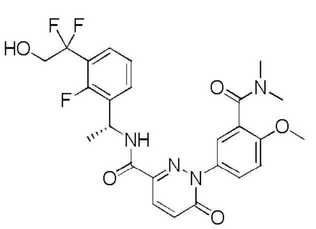,
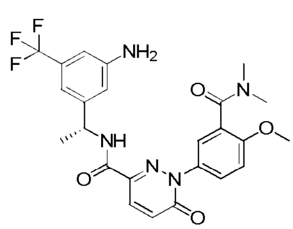,

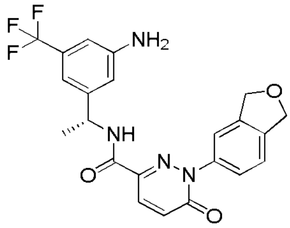 , 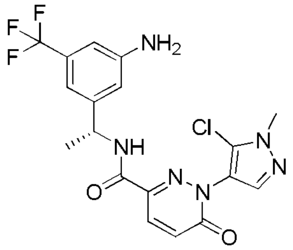 , 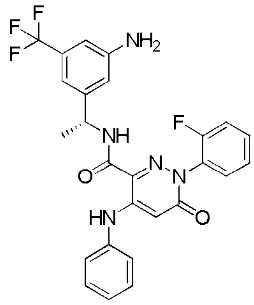 , 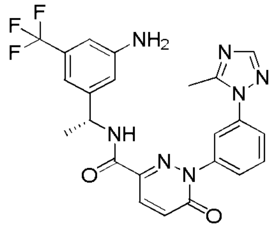 ,

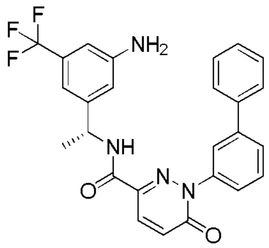 , 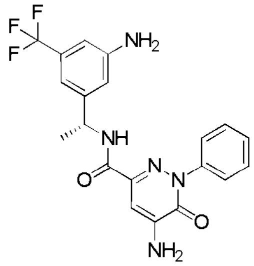 , 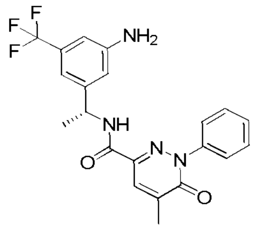 , 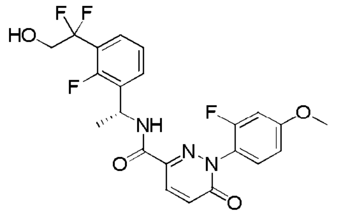 ,

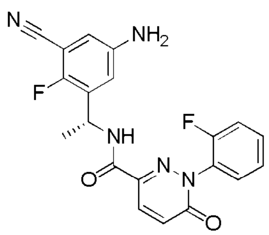 , 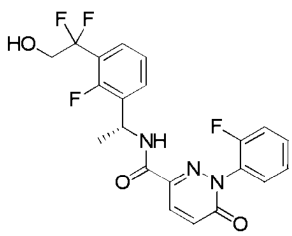 , 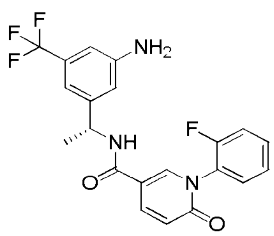 , 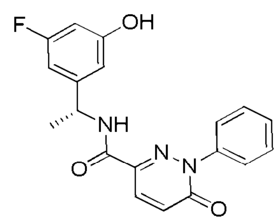 ,

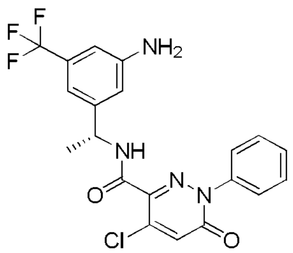 , 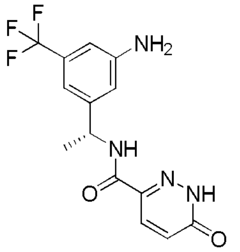 , 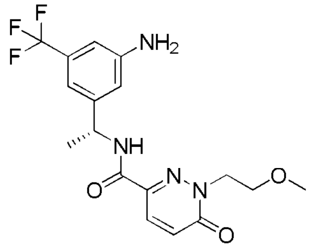 , 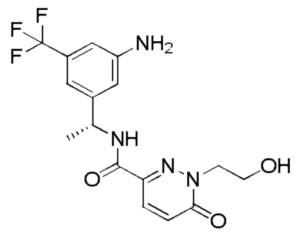 ,

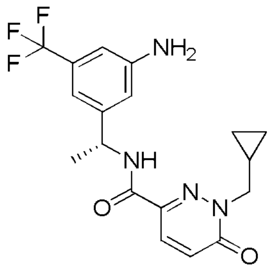 , 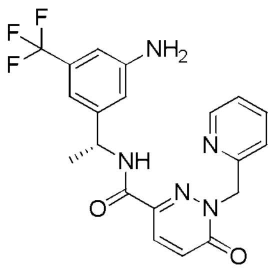 , 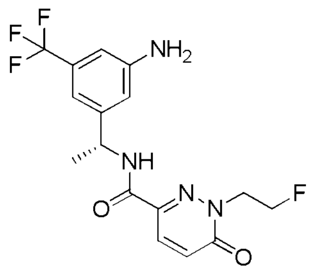 , 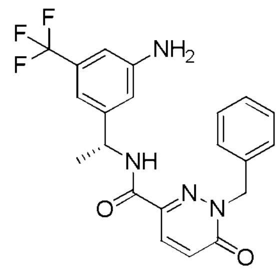 ,

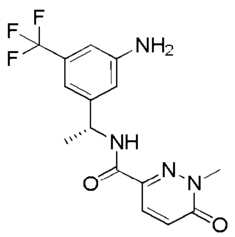 , 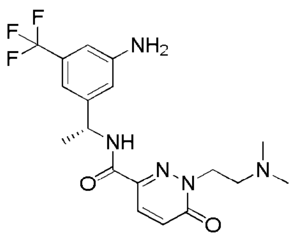 , 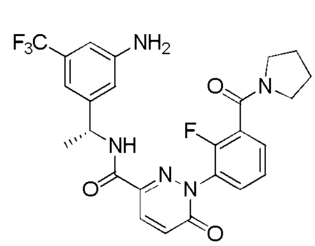 , 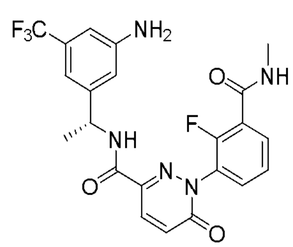 ,

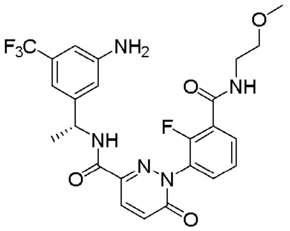 , 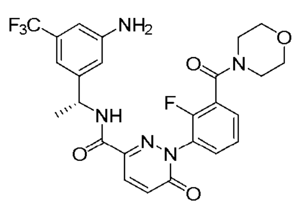 , 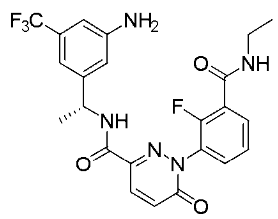 , 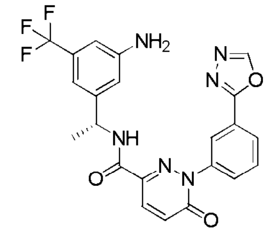 ,

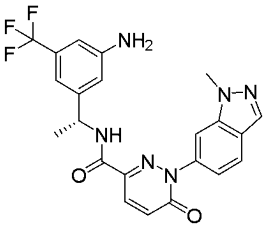 , 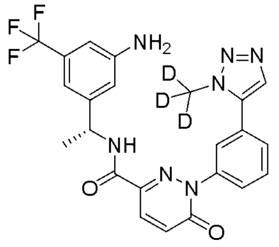 , 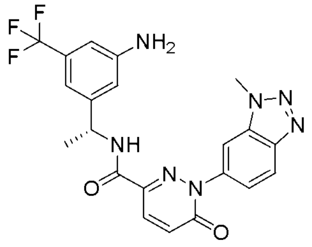 , 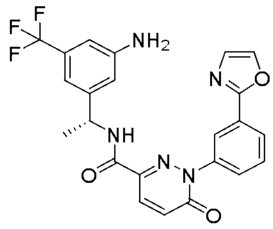 ,

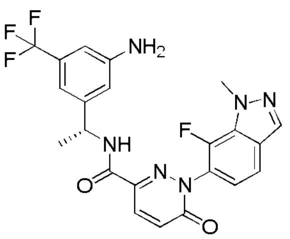 , 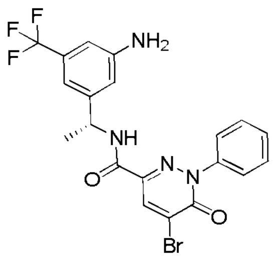 , 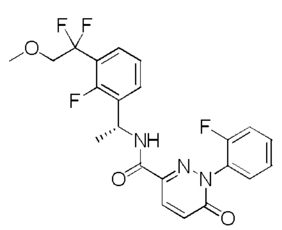 , 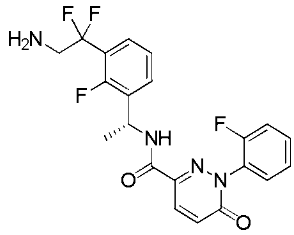 ,

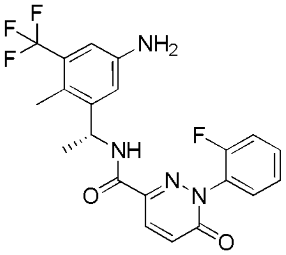 , 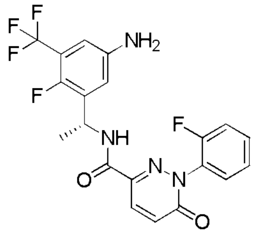 , 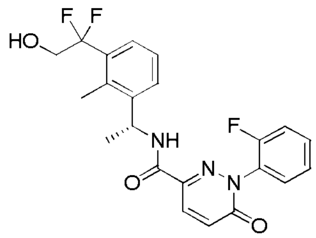 , 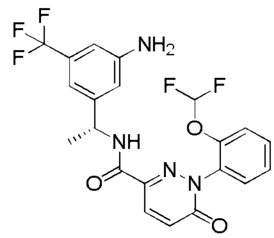 ,

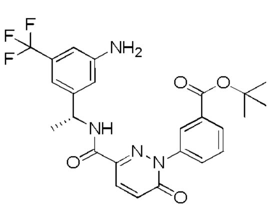 , 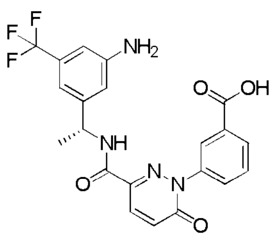 , 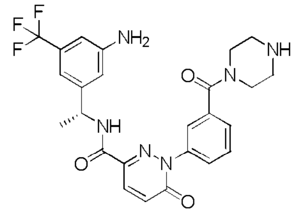 , 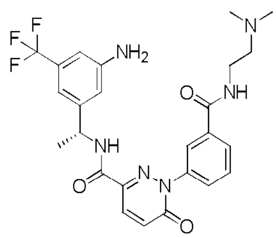 ,

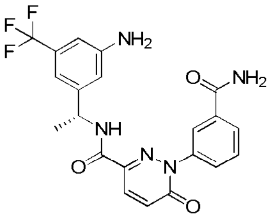 , 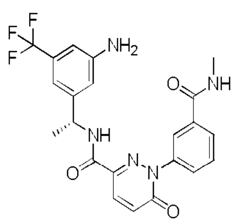 , 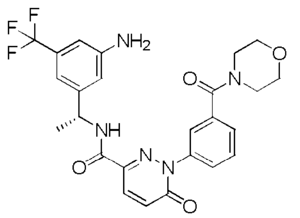 , 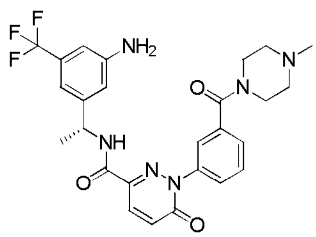 ,

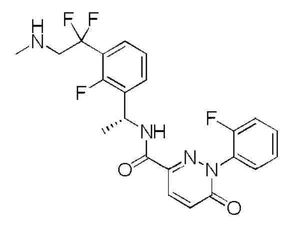 ,

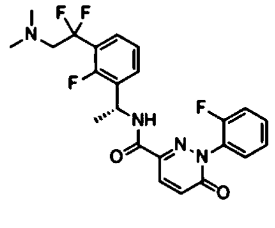 ,

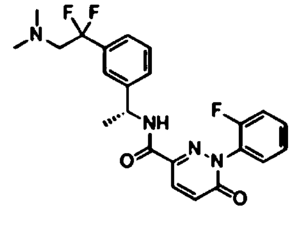 ,

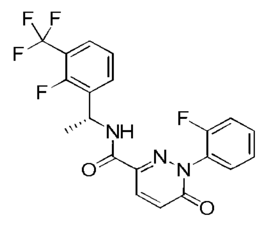 ,

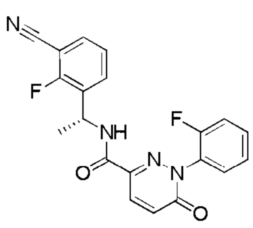 ,

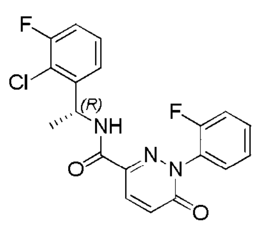 ,

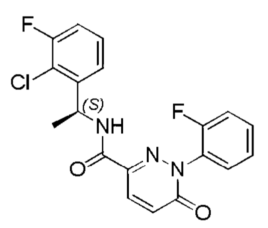 ,

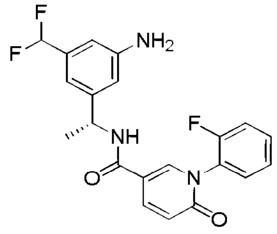 ,

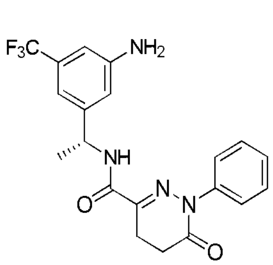 ,

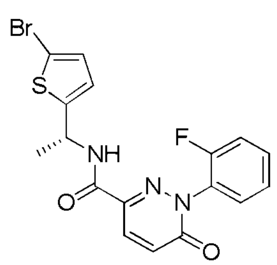 ,

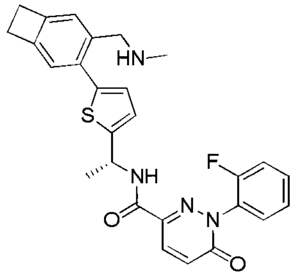 ,

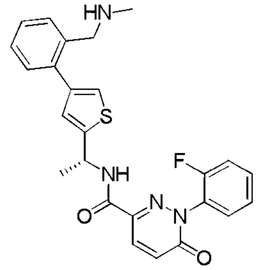 ,

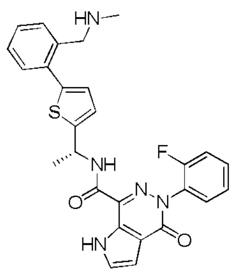 ,

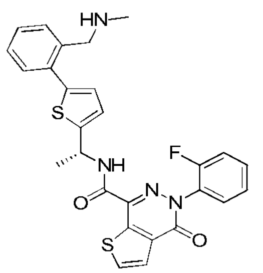 ,

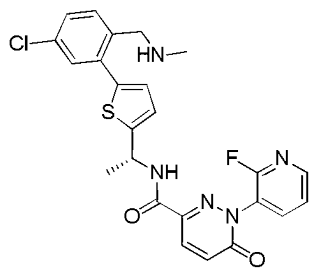 ,

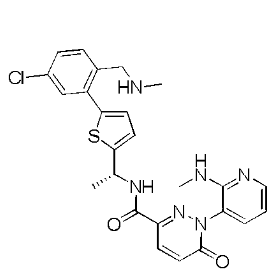 ,

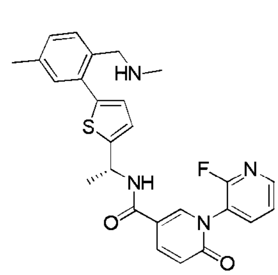 ,

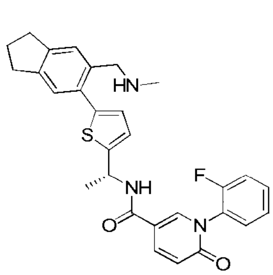 ,

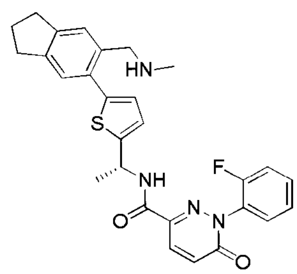 ,

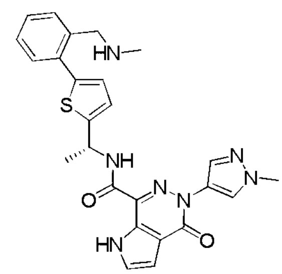 ,

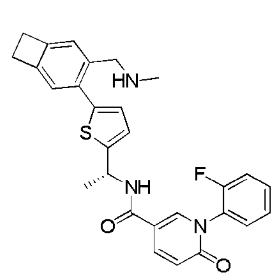 ,

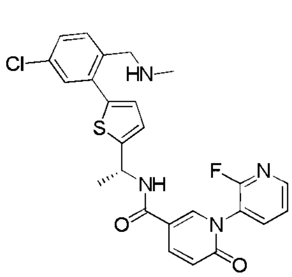 ,

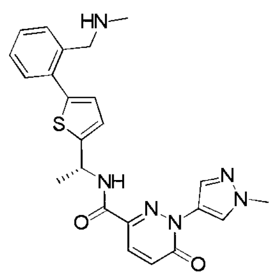 ,

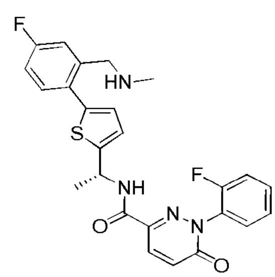 ,

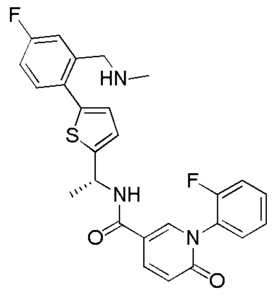 , 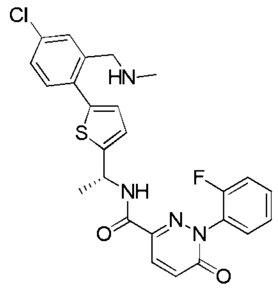 , 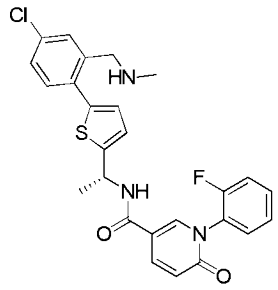 , 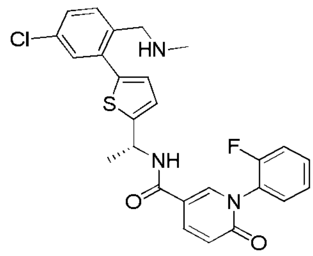 ,

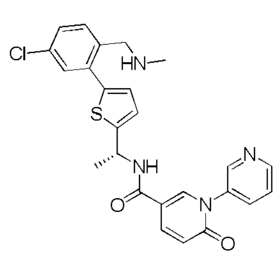 , 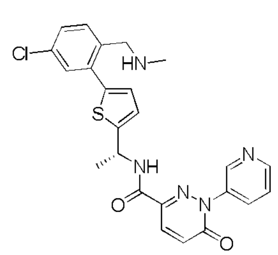 , 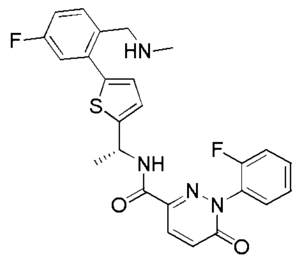 , 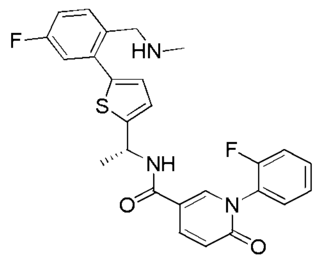 ,

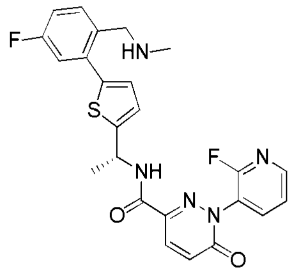 , 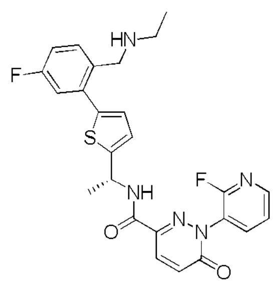 , 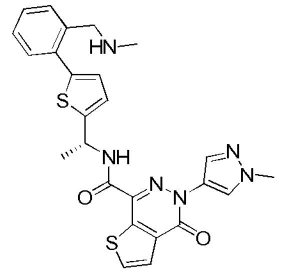 , 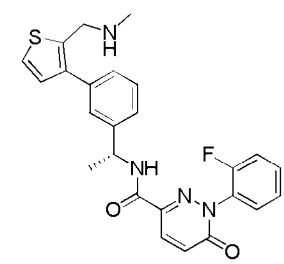 ,

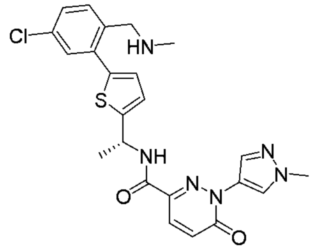 , 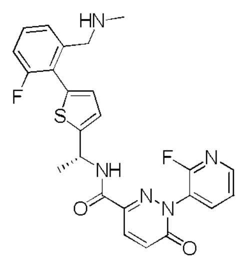 , 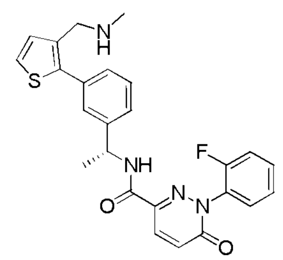 , 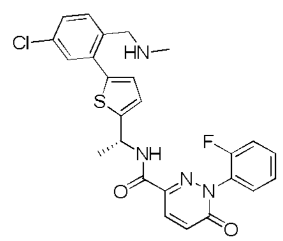 ,

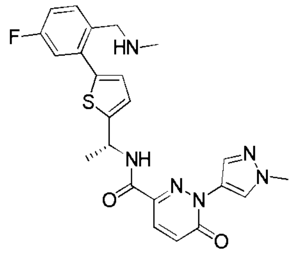 , 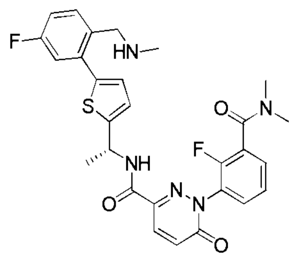 , 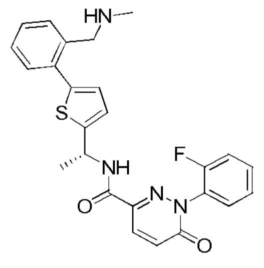 , 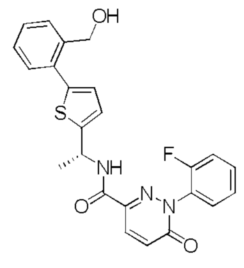 ,

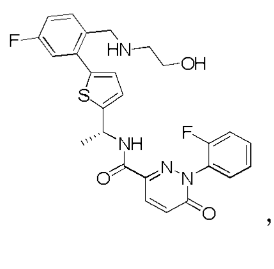 , 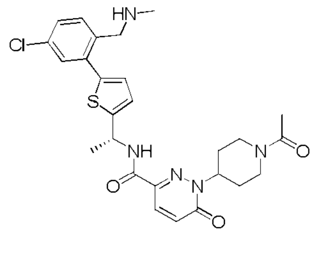 , 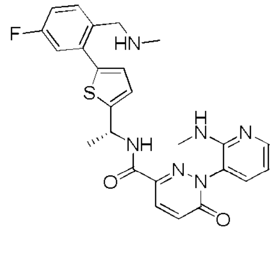 , 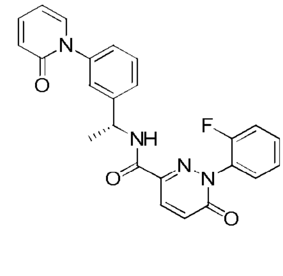 ,

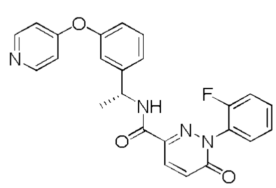, 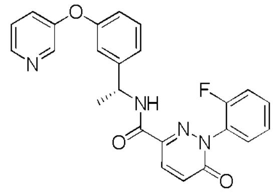, 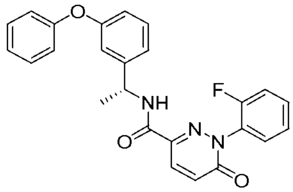, 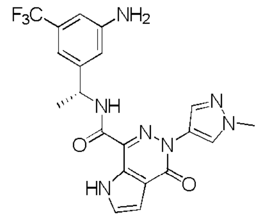,

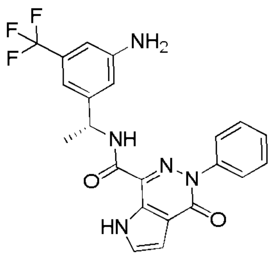, 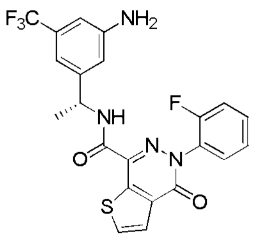, 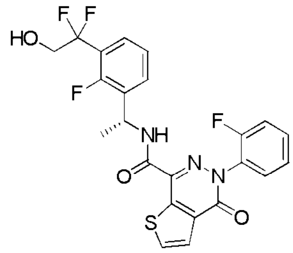, 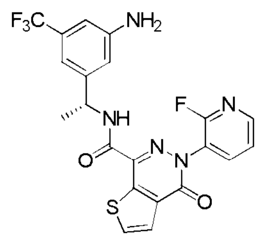,

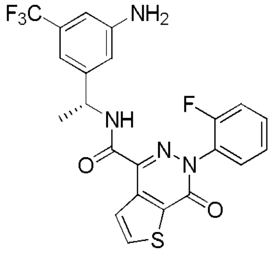, 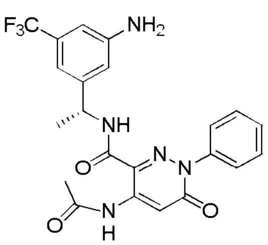, 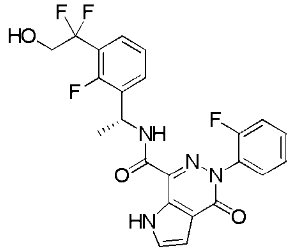, 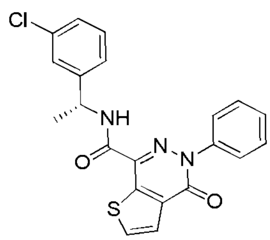,

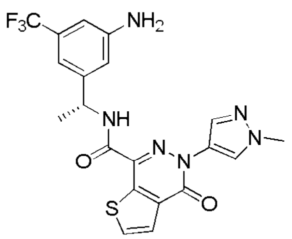, 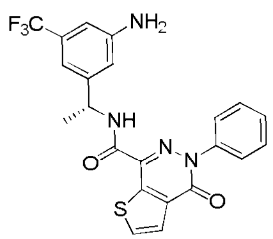, 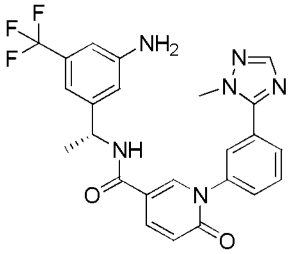, 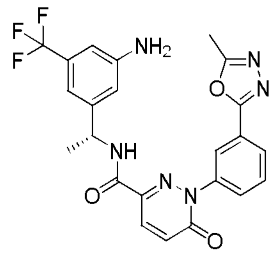,

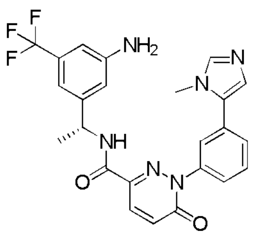, 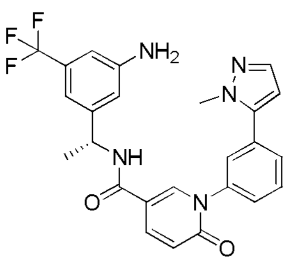, 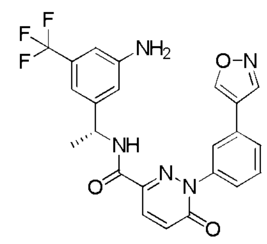, 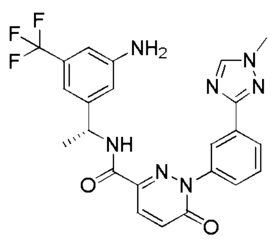,

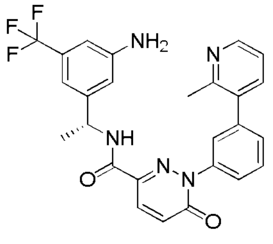, 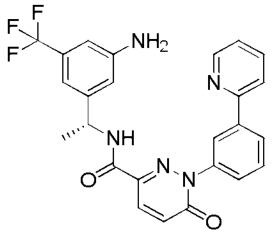, 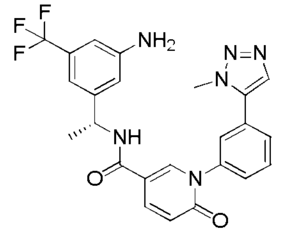, 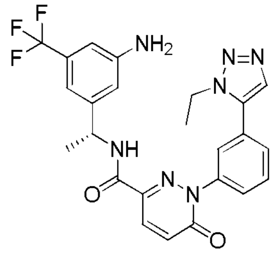,

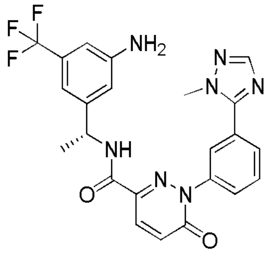 , 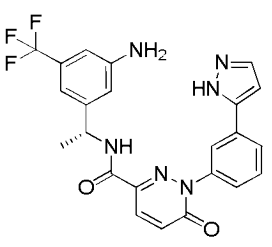 , 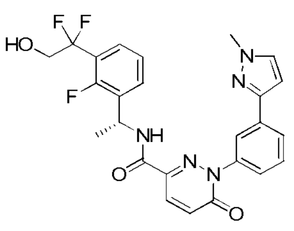 , 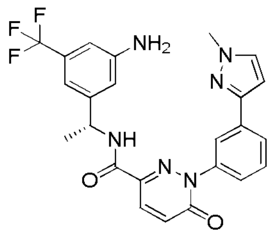 ,

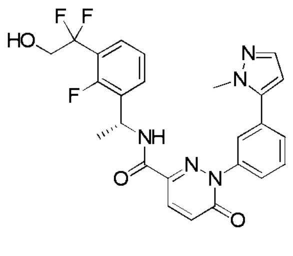 , 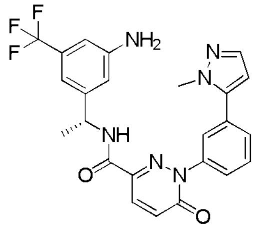 , 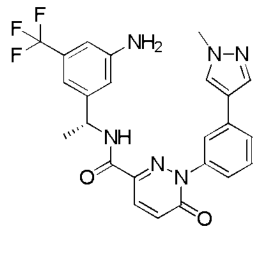 , 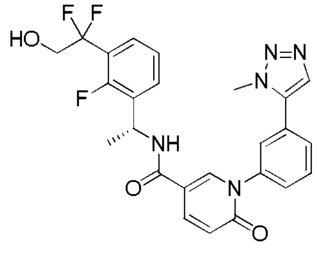 ,

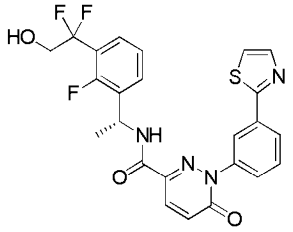 , 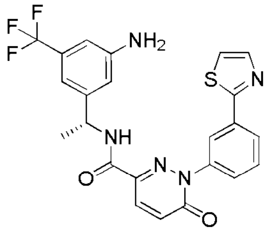 , 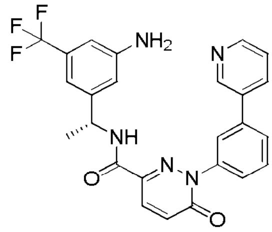 , 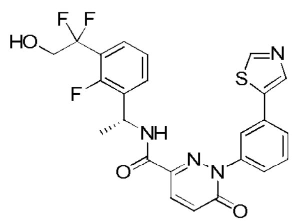 ,

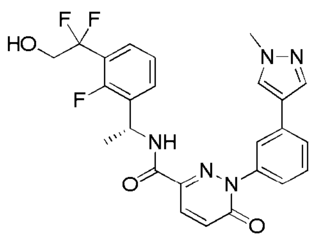 , 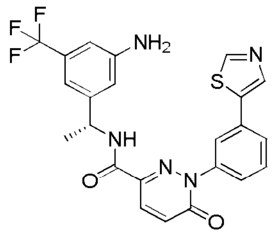 , 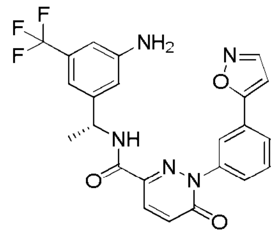 , 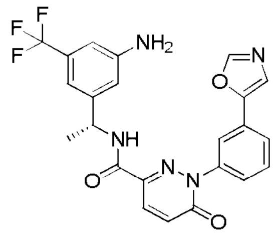 ,

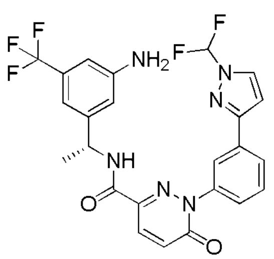 , 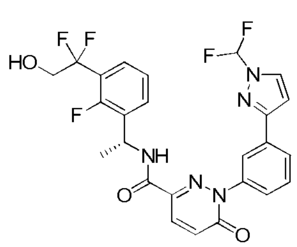 , 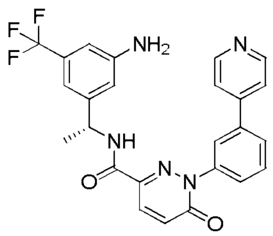 , 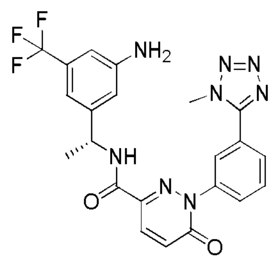 ,

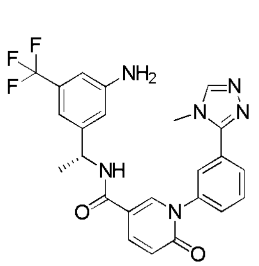 , 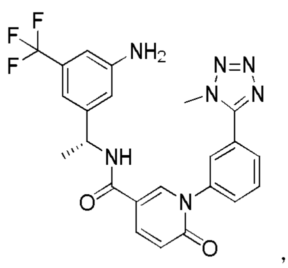 , 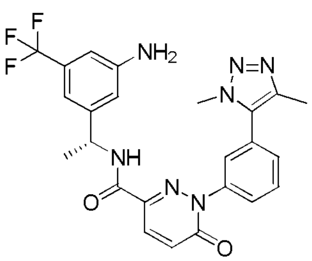 , 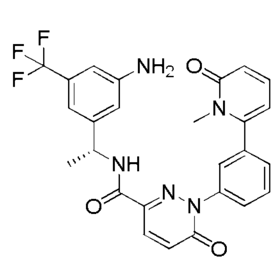 ,

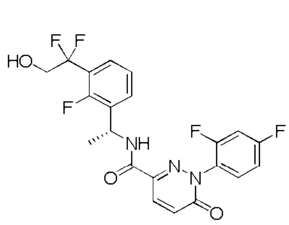 , 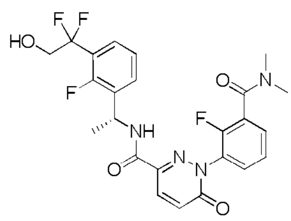 , 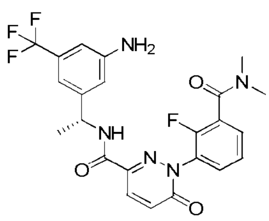 , 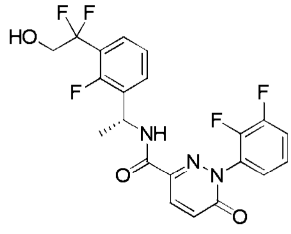 ,

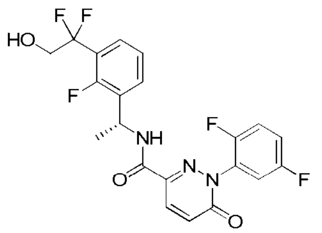 , 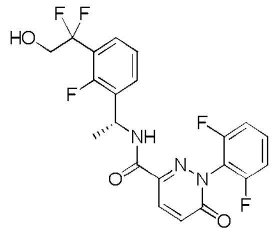 , 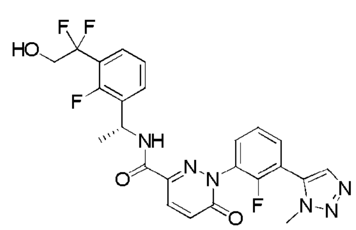 ,

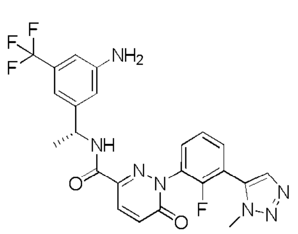 , 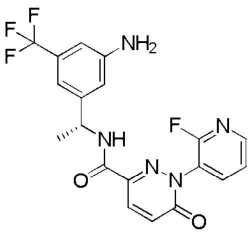 , 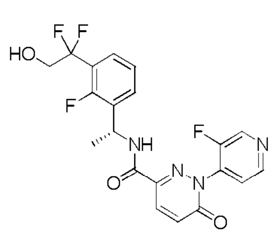 , 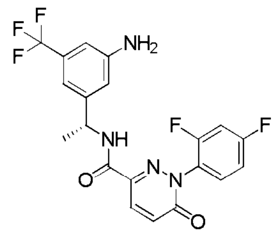 ,

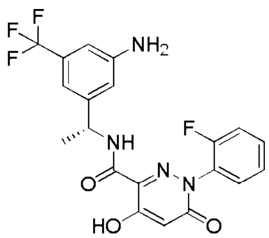 , 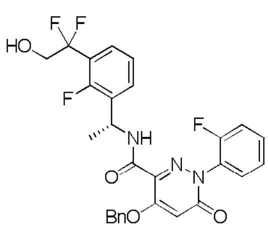 , 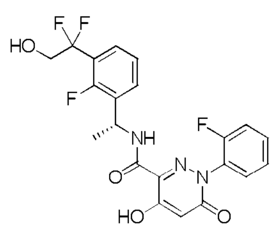 , 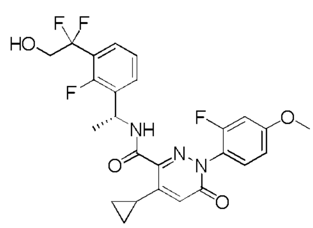 ,

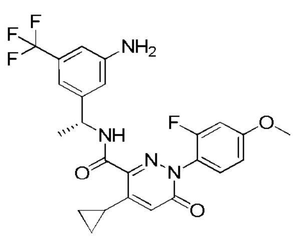 , 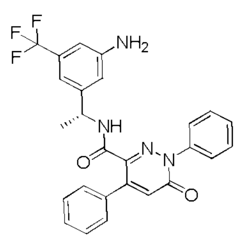 , 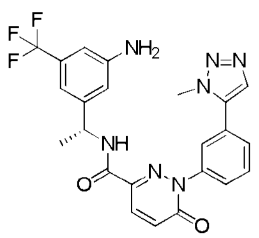 , 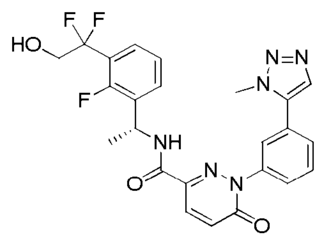 ,

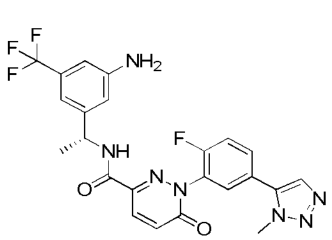 , 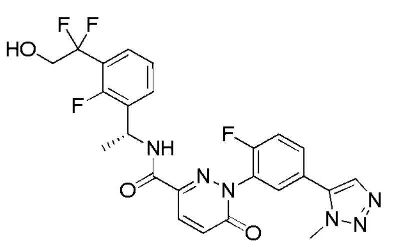 , 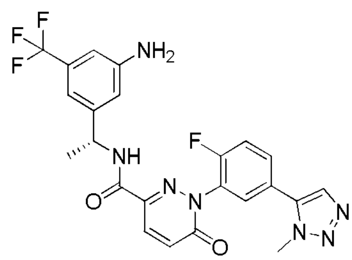 ,

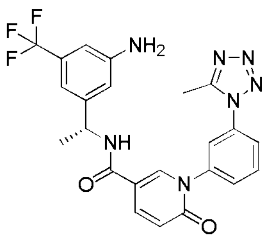 ,
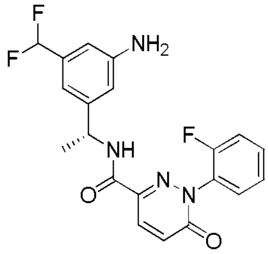 ,
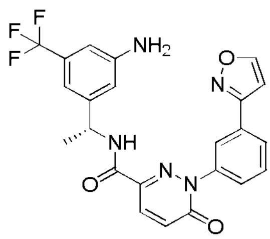 ,
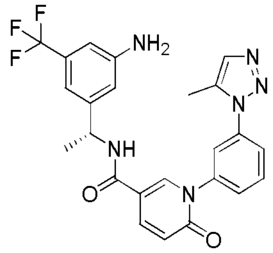 ,
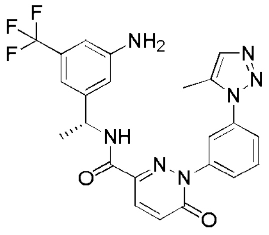 ,
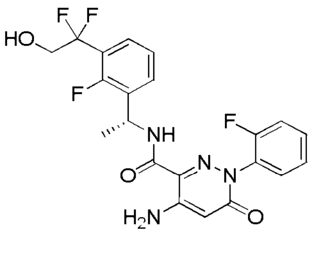 ,
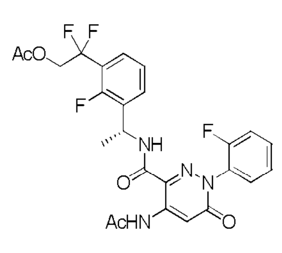 ,
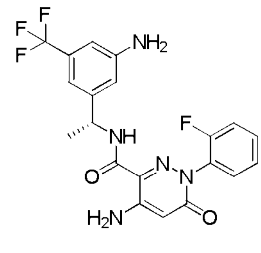 ,
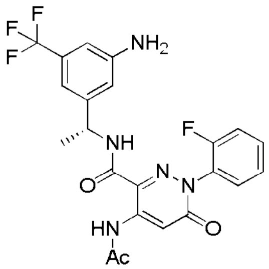 ,
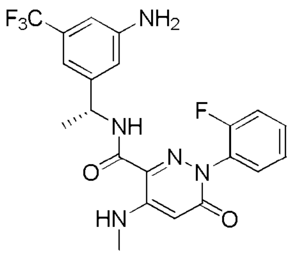 ,
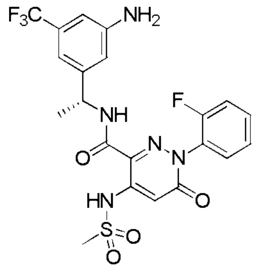 ,
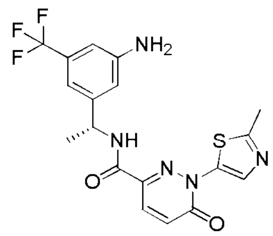 ,
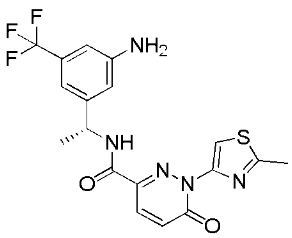 ,
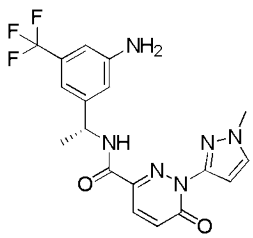 ,
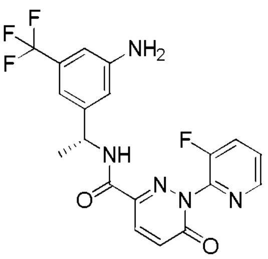 ,
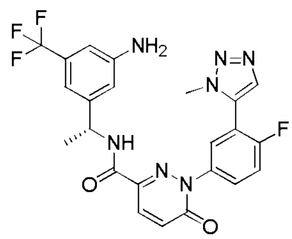 ,
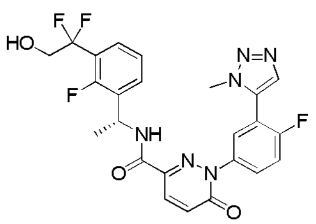 ,
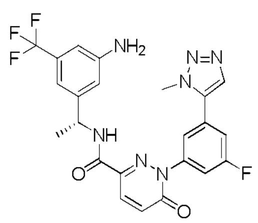 ,
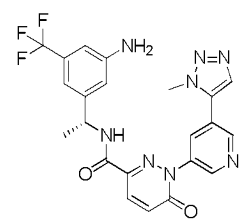 ,
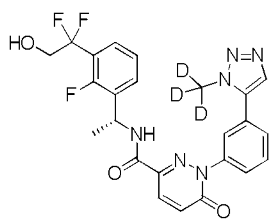 ,
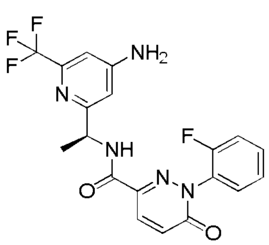 ,
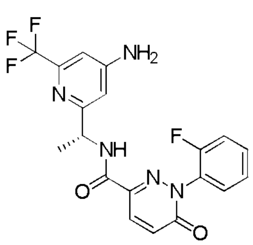 ,
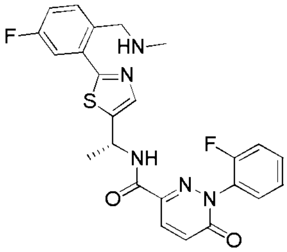 ,
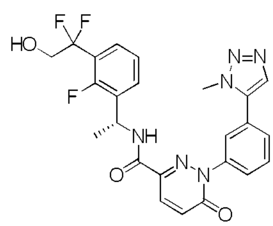 ,

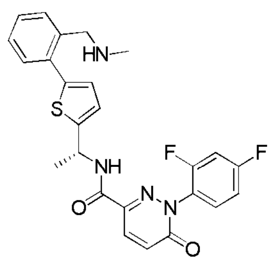 , 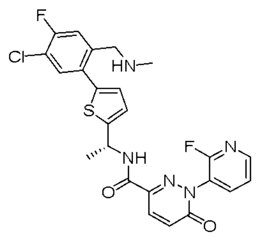 , 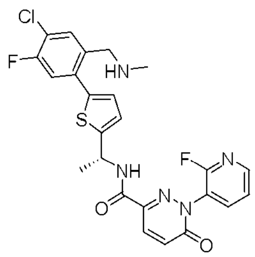 , 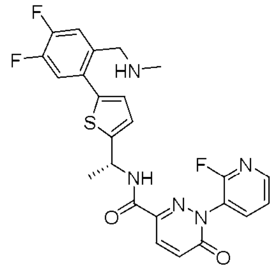 ,

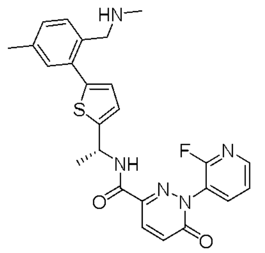 , 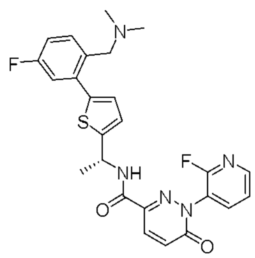 , 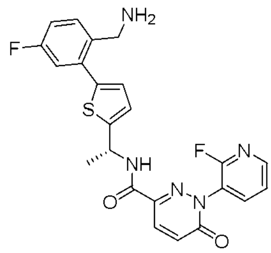 , 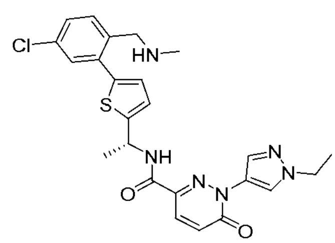 ,

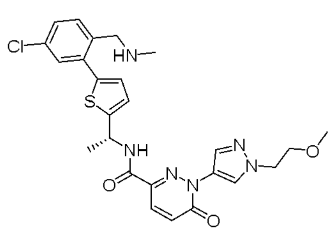 , 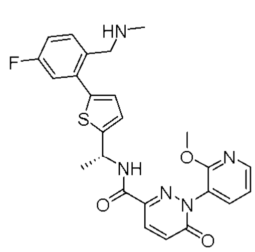 , 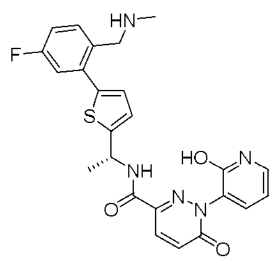 , 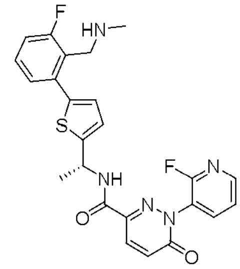 ,

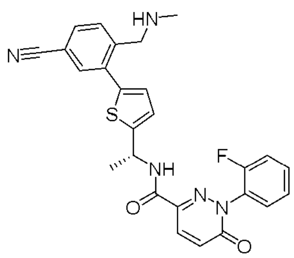 , 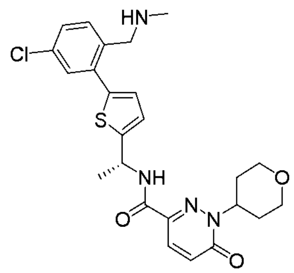 , 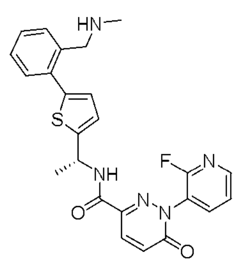 , 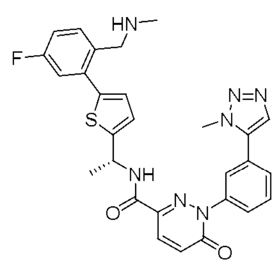 ,

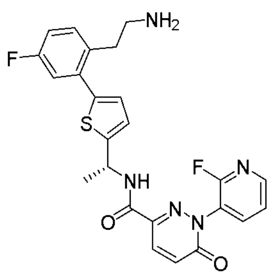 , 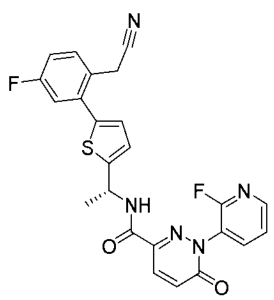 , 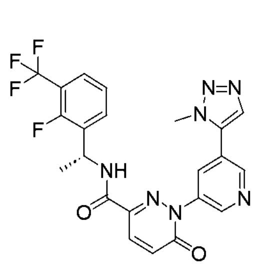 , 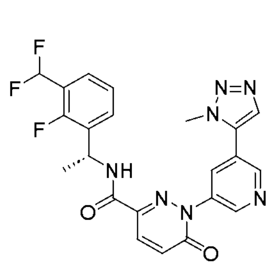 ,

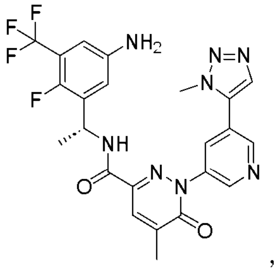,
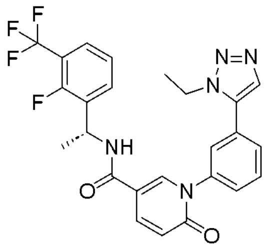,
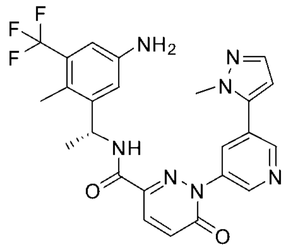,
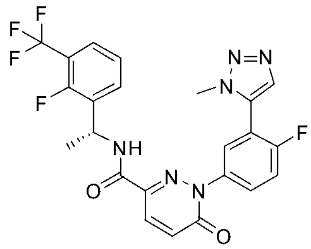,

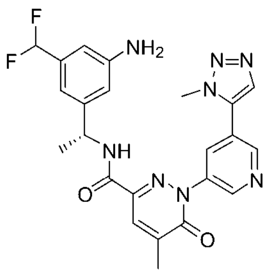,
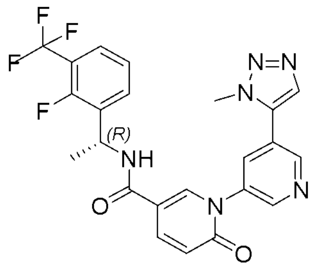,
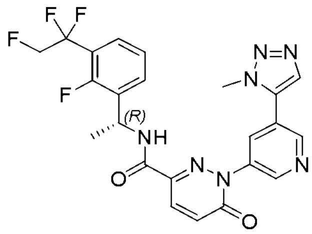,

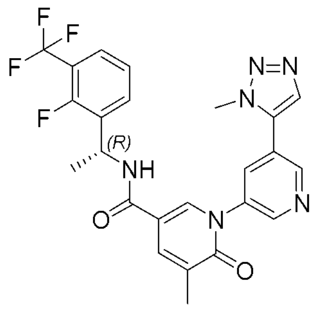, and
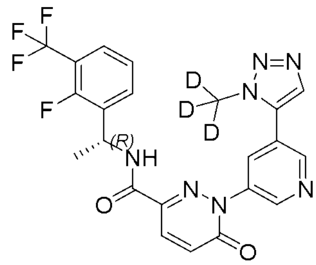.

**Definition**

All technical and scientific terms used herein have the meanings commonly understood by one of ordinary skill in the art, and unless otherwise stated, conventional methods of measurement, methods of manufacture, conventional ingredients or substances are used based on conventional techniques such as pharmacology, pharmaceutical manufacturing chemistry, mass spectrometry, NMR, HPLC, biochemistry, and the like.

Individual features and components of each embodiment described and illustrated herein may be combined with features and components of any other embodiment without departing from the scope or spirit of the present disclosure.

Unless otherwise specified, in the present specification and the appended claims, "or" and "and" mean "and/or". The terms "include" and "included" are open-ended, and mean that a compound, composition, or method may include additional features or ingredients in addition to the listed features or ingredients.

In the present specification, the numerical range indicated using the term "to" refers to a range including the numerical values described before and after the term "to" as the lower limit and the upper limit, respectively.

As used herein, the term "optional" or "optionally" is intended to include that the subsequently described event or circumstance may or may not occur, and that the description includes instances where the event or circumstance occurs and instances where it does not. For example, the term "optionally substituted" is intended to include both unsubstituted or substituted with the specified substituent.

**Compound**

As used herein, the term "alkyl" refers to a fully saturated branched or unbranched (or straight chain or linear) hydrocarbon. The alkyl may be a substituted or unsubstituted alkyl group. The alkyl may be optionally interrupted by at least one oxygen atom or nitrogen atom, and the alkyl group interrupted by an oxygen atom or nitrogen atom refers to an alkyl group in which an oxygen atom or a nitrogen atom is inserted between carbon atoms of the alkyl chain. For example, the alkyl interrupted by an oxygen atom or nitrogen atom includes alkoxyalkyl, alkylaminoalkyl, and the like, and includes one in which an oxygen atom or nitrogen atom is located at the end of a substituent, such as hydroxyalkyl or aminoalkyl.

The $C_1$-$C_6$ alkyl may be a $C_1$ to $C_6$, $C_1$ to $C_5$, $C_1$ to $C_4$, $C_1$ to $C_3$, or $C_1$ to $C_2$ alkyl group. Non-limiting examples of the alkyl may be methyl, ethyl, n-propyl, isopropyl, n-butyl, isobutyl, sec-butyl, n-pentyl, isopentyl, neopentyl, iso-amyl, or n-hexyl.

As used herein, the term "alkenyl" refers to a straight chain or branched chain hydrocarbon group having 2 to 6 carbon atoms, 2 to 5 carbon atoms, or 2 to 4 carbon atoms having one or more double bonds at any position. For example, it may include vinyl, propenyl, isopropenyl, butenyl, isobutenyl, prenyl, butadienyl, pentenyl, isopentenyl, pentadienyl, hexenyl, isohexenyl, hexadienyl, and the like.

As used herein, the term "alkynyl" refers to a hydrocarbon group containing at least one triple bond, and includes a straight chain or branched chain alkynyl having 2 to 6 carbon atoms, 2 to 5 carbon atoms, or 2 to 4 carbon atoms. For example, it may include ethynyl, propynyl, butynyl, pentynyl, hexynyl, and the like.

As used herein, unless otherwise stated, the term "alkoxy" refers to a substituent in which a substituted or unsubstituted straight chain or branched chain alkyl moiety is linked to another chemical structure by oxygen. The alkoxy may include all possible isomers thereof such as, for example, methoxy, ethoxy, propoxy, and butoxy, or isopropoxy, isobutoxy, and t-butoxy, but is not limited thereto.

As used herein, the term "cycloalkyl" refers to a saturated hydrocarbon ring having the specified number of carbon atoms as ring elements (that is, $C_3$-$C_8$ cycloalkyl refers to a cycloalkyl group having 3, 4, 5, 6, 7 or 8 carbon atoms as ring elements). The cycloalkyl may be $C_3$-$C_6$ monocyclic or $C_5$-$C_{20}$ polycyclic (for example, bicyclic, tricyclic or tetracyclic). For example, monocyclic cycloalkyl may be $C_3$-$C_6$, $C_3$-$C_5$, or $C_3$-$C_4$ cycloalkyl. Monocyclic cycloalkyl may be, for example, cyclopropyl, cyclobutyl, cyclopentyl, cyclohexyl, and the like. Bicyclic, tricyclic or tetracyclic cycloalkyl may be $C_5$-$C_{18}$ cycloalkyl, $C_5$-$C_{15}$ cycloalkyl, $C_5$-$C_{11}$ cycloalkyl, $C_5$-$C_{10}$ cycloalkyl. Polycyclic cycloalkyl may be one in which two or more cycloalkyls are bridged, fused, or spiro bonded, and in tricyclic or tetracyclic cycloalkyl, each cycloalkyl ring may be bonded in the form of two or more of bridged, fused and spiro bonded forms. For example, polycyclic bridged, fused or spiro cycloalkyl may include bicyclo[1.1.1]pentanyl, bicyclo[2.2.2]octanyl, adamantyl, bicyclo[2.2.1]heptanyl, bicyclo[3.1.0]hexanyl, bicyclo[3.2.0]heptanyl, bicyclo[3.2.1]octanyl, bicyclo[3.3.1]octanyl, bicyclo[3.3.0]octanyl, bicyclo[4.2.0]octanyl, spiro[2.3]hexanyl, spiro[2.4]heptanyl, spiro[3.3]heptanyl, spiro[2.5]octanyl, spiro[3.4]octanyl, octahydro-1H-indenyl, decahydronaphthalenyl, and the like. As used herein, cycloalkyl may optionally include one fused with heteroaryl or heterocycloalkyl (for example, cyclohexyl fused with pyrazole, piperazine or tetrahydropyran), in which case heteroaryl or heterocycloalkyl is as defined below.

As used herein, the term "cycloalkenyl" refers to a non-aromatic unsaturated monocyclic or polycyclic hydrocarbon ring having at least one carbon-carbon double bond and containing the specified number of carbon atoms. For example, monocyclic cycloalkenyl may include cyclopent-1-en-1-yl, cyclohex-1-en-1-yl, cyclohex-1,3-dien-1-yl, and the like, but is not limited thereto. The above matters regarding the carbon number and bond form of bicyclic, tricyclic or tetracyclic cycloalkyl apply equally to bicyclic, tricyclic or tetracyclic cycloalkenyl. For example, bicyclic, tricyclic or tetracyclic cycloalkenyl includes those in which a carbon-carbon double bond is introduced at any position in the bicyclic, tricyclic or tetracyclic cycloalkyl exemplified above. In the present specification, cycloalkenyl may optionally include one fused with heteroaryl or heterocycloalkyl (for example, cyclohexenyl fused with pyrazole, piperazine or tetrahydropyran), in which case heteroaryl or heterocycloalkyl is as defined below.

As used herein, the term "aryl" refers to a monocyclic or polycyclic aromatic hydrocarbon group. The aryl has alternating (resonance) double bonds between adjacent carbon atoms or suitable heteroatoms, and may also include a form in which two or more rings are simply attached to each other (pendant) or condensed. The aryl may be, for example, $C_6$-$C_{10}$ aryl, or $C_6$-$C_9$ aryl, and may include, for example, phenyl, naphthalenyl (naphthyl), toluyl, or all possible isomers thereof, but is not limited thereto. In the present specification, aryl may be fused with cycloalkyl. For example, $C_{6-10}$ aryl may be fused with 3- to 8-membered cycloalkyl. In this case, phenyl and cyclobutyl may be fused to form bicyclo[4.2.0]octa-1,3,5-trienyl, or phenyl and cyclopentyl may be fused to form 2,3-dihydroindenyl. In addition, in the present specification, aryl may be optionally fused with heterocycloalkyl. For example, $C_{6-10}$ aryl may be fused with 5- to 10-membered heterocycloalkyl. For example, phenyl and tetrahydrofuranyl may be fused to form dihydrobenzofuranyl or dihydroisobenzofuranyl.

As used herein, the term "heteroaryl" refers to a heterocyclic aromatic group containing at least one heteroatom selected from B, N, O, S, P(=O), Si and P as a ring-forming atom. The heteroaryl may also include a form in which two or more rings are simply attached to each other (pendant) or condensed. The heteroaryl may contain 1 to 4 heteroatoms, 1 to 3 heteroatoms, 1 or 2 heteroatoms, or 1 heteroatom selected from N, O and S. The heteroaryl may contain 5 to 10, or 5 to 6 ring atoms. Examples of monocyclic heteroaryl may include thiophenyl, furanyl, pyrrolyl, pyrazolyl, thiazolyl, isothiazolyl, oxazolyl, isooxazolyl, imidazolyl, triazolyl, oxadiazolyl, thiadiazolyl, tetrazolyl, pyridinyl, pyridazinyl, pyrimidinyl, pyrazinyl, triazinyl and similar groups thereto, but are not limited thereto. Examples of bicyclic heteroaryl may include indolyl, isoindolyl, indazolyl, indolizinyl, benzothiophenyl, benzofuranyl, benzimidazolyl, benzopyrazolyl, benzoxazolyl, benzisoxazolyl, benzthiazolyl, benzisothiazolyl, benzthiadiazolyl, benztriazolyl, quinolinyl, isoquinolinyl, quinoxalinyl, quinazolinyl, purinyl, phthalazinyl, pteridinyl, furopyridinyl, oxochromenyl, dioxoisoindolinyl, imidazopyridinyl, pyrrolopyridinyl, pyrrolopyrimidinyl, pyrazolopyridinyl and similar groups thereto, but are not limited thereto. In the present specification, heteroaryl optionally includes one fused with a cycloalkyl group (for example, pyrazolyl fused with cyclohexyl). In addition,

heteroaryl may be a functional group in which the aromaticity of the ring is maintained by replacing the carbon of the ring with oxo, sulfanyldiene (=S), imino (=NH or =N($C_{1-6}$ alkyl)), and the like. For example, it may include pyridinonyl (pyridonyl), pyridazinonyl, pyrimidinonyl (pyrimidonyl), pyrazinonyl, and the like. When the heteroaryl contains N, B or P in the ring, N, B or P of the heteroaryl may be linked to another moiety.

As used herein, unless otherwise stated, the term "heterocycloalkyl" refers to a monocyclic or polycyclic, saturated or partially unsaturated ring system containing at least one heteroatom selected from B, N, O, S, P(=O), Si and P and having the specified number of ring elements (that is, 3- to 7-membered heterocycloalkyl refers to a heterocycloalkyl group having 3, 4, 5, 6 or 7 ring elements, including heteroatoms). The polycyclic heterocycloalkyl may also include a form in which two or more heterocycloalkyl rings are simply attached to each other (pendant) or bridged or condensed or spiro bonded. The heterocycloalkyl may contain 1 to 4 heteroatoms, 1 to 3 heteroatoms, 1 or 2 heteroatoms, or 1 heteroatom selected from N, O and S. In addition, the heterocycloalkyl may contain 5 to 10, 4 to 7, 5 or 6 ring atoms. For example, the heterocycloalkyl group includes azetidinyl, oxetanyl, thietanyl, pyrrolidinyl, pyrrolinyl, dihydrofuranyl, tetrahydrofuranyl (oxanyl), dihydrothiophenyl, tetrahydrothiophenyl, sulfolanyl, thianyl, dioxolanyl, imidazolinyl, imidazolidinyl, pyrazolinyl, pyrazolidinyl, thiazolinyl, thiazolidinyl, isothiazolinyl, isothiazolidinyl, oxazolinyl, oxazolidinyl, isoxazolinyl, isoxazolidinyl, triazolinyl, triazolidinyl, tetrazolinyl, tetrazolidinyl, pyranyl, dihydropyranyl, tetrahydropyranyl, thiopyranyl, tetrahydro 2H-thiopyranyl, dihydrothiopyranyl, dioxanyl, tetrahydrotriazinyl, hexahydrotriazinyl, morpholinyl, thiomorpholinyl, piperidinyl, dihydropyridinyl, tetrahydropyridinyl, piperazinyl, hexahydropyrimidinyl, tetrahydropyrimidinyl, dihydropyrimidinyl, dihydropyridazinyl, tetrahydropyridazinyl, tetrahydrooxazinyl, hexahydroazepinyl, perhydroazepinyl, perhydrooxepinyl, indolinyl, isoindolinyl, dihydrobenzimidazolyl, dihydrobenzofuranyl, dihydrobenzoxazolyl, dihydrobenzothiazolyl, chromanyl, isochromanyl, 3-oxabicyclo[2.1.1]hexanyl, 2-oxabicyclo[2.1.1]hexanyl, 2-azabicyclo[2.1.1]hexanyl, 3-azabicyclo[2.1.1]hexanyl, azabicyclo[2.2.1]heptanyl, 3-azabicyclo[3.2.1]heptanyl, 7-azabicyclo[4.1.0]-heptanyl, 2,5-diazabicyclo[2.2.1]heptanyl, 2-oxa-5-azabicyclo[2.2.1]heptanyl, tropanyl, 2-oxa-6-azaspiro[3.3]heptanyl, and N-oxide, sulfone or sulfoxide thereof, but is not limited thereto. In the present specification, heterocycloalkyl optionally includes one fused with a cycloalkyl group (for example, piperidinyl fused with cyclohexyl). When the heterocycloalkyl contains N, B or P in the ring, N, B or P of the heterocycloalkyl may be linked to another moiety.

As used herein to indicate a chemical bond between ring atoms, "------" indicates that two atoms are bonded by a single bond or a double bond, and each atom may have as many H or substituents as its valence allows. For example, when ------ is used to link these two ring carbon atoms, it represents -CH=CH- or -$CH_2$-$CH_2$-, and each H may be substituted with an appropriate substituent.

As used herein, the term "halogen" refers to an atom belonging to group 17 of the periodic table. The halogen atom includes fluorine, chlorine, bromine, iodine, and the like, and may be used interchangeably with the term "halo," which means a monovalent functional group composed of halogen.

As used herein, the term "cyano" refers to -CN, which is a functional group having a triple bond between a carbon atom and a nitrogen atom.

As used herein, the term "hydroxy" refers to a -OH functional group (hydroxyl group).

As used herein, the term "oxy" refers to a divalent functional group of -O-.

As used herein, the term "oxo" refers to a substituent having the structure =O, in which a double bond is present between the atom to which the substituent is attached and the oxygen atom.

As used herein, the term "carbonyl" refers to a divalent functional group of -C(=O)-.

As used herein, the term "acyl" refers to a functional group in which the carbon atom at the 1$^{st}$ position of alkyl is substituted with oxo, and includes "formyl" and "alkylcarbonyl." For example, $C_{1-6}$ acyl is one in which the carbon atom at the 1st position of $C_{1-6}$ alkyl is substituted with oxo, and may include formyl (HC(O)-), acetyl ($CH_3C(O)$-), propionyl ($CH_3CH_2C(O)$-), butanoyl ($CH_3CH_2CH_2C(O)$-), pentanoyl ($CH_3CH_2CH_2CH_2CO$-), hexanoyl ($CH_3CH_2CH_2CH_2CH_2C(O)$-), and the like.

As used herein, the term "acyloxy" refers to a functional group in which acyl is bonded to one end of oxy, and includes "formyloxy" and "alkylcarbonyloxy." For example, $C_{1-3}$ acyloxy may include formyloxy, acetyloxy (acetoxy), propionyloxy, and the like.

As used herein, the term "carboxy" refers to -COOH.

As used herein, the term "sulfonyl" refers to a divalent functional group of -$S(O)_2$-. For example, $C_{1-6}$ alkylsulfonyl may include methylsulfonyl, ethylsulfonyl, propylsulfonyl, butylsulfonyl, pentylsulfonyl, hexylsulfonyl, and the like.

As used herein, the term "amino" refers to -$NH_2$.

As used herein, the term "alkylamino" refers to a functional group in which one hydrogen of amino is substituted with alkyl. For example, $C_{1-6}$ alkylamino is -NH($C_1$-$C_6$ alkyl), and may include methylamino, ethylamino, propylamino, butylamino, and the like, but is not limited thereto.

As used herein, the term "dialkylamino" refers to a functional group in which two hydrogens of amino are each substituted with alkyl. In this case, the substituted alkyl may be the same or different from each other. For example, di($C_{1-6}$ alkyl)amino is -N($C_1$-$C_6$ alkyl)$_2$, and may include dimethylamino, diethylamino, dipropylamino, dibutylamino, ethylmethylamino, methylpropylamino, ethylpropylamino, and the like, but is not limited thereto.

As used herein, the term "acylamino" refers to a functional group in which the carbon atom at the 1st position of alkyl in alkylamino is substituted with oxo, and includes "formylamino" and "alkylcarbonylamino."

As used herein, the term "carbamoyl" refers to $-CONH_2$.

As used herein, the term "alkylcarbamoyl" refers to a functional group in which one hydrogen of carbamoyl is substituted with alkyl. For example, $C_{1-6}$ alkylcarbamoyl is -CONH($C_{1-6}$ alkyl), and may include $-CONHCH_3$, $-CONHCH_2CH_3$, $-CONHCH_2CH_2CH_3$, - $CONHCH_2CH_2CH_2CH_3$, and the like, but is not limited thereto.

As used herein, the term "dialkylcarbamoyl" refers to a functional group in which two hydrogens of carbamoyl are each substituted with alkyl. For example, $C_{1-6}$ alkylcarbamoyl is - CON($C_{1-6}$ alkyl)$_2$, and may include $-CON(CH_3)_2$, $-CON(CH_2CH_3)_2$, $-CON(CH_3)(CH_2CH_3)$, and the like, but is not limited thereto.

As used herein, the term "substituted" group refers to one in which one or more hydrogen atoms are replaced with one or more non-hydrogen atom groups, provided that valence requirements should be met and a chemically stable compound should occur from the substitution. In the present specification, unless explicitly stated as "unsubstituted," all substituents should be construed as being capable of being unsubstituted or substituted.

In the present specification, the "optionally substituted" moiety mentioned herein without limitation of a particular substituent may encompass a moiety unsubstituted or substituted with any substituent. For example, the "optionally substituted" moiety may refer to a moiety substituted with the following substituents:

(i) halogen, OH, CN, oxo, $NH_2$, NH($C_1$-$C_6$ alkyl), or N($C_1$-$C_6$ alkyl)$_2$;
(ii) $C_1$-$C_3$ alkyl optionally substituted with at least one substituent selected from the group consisting of halogen, OH, CN, oxo, $NH_2$, NH($C_1$-$C_6$ alkyl) and N($C_1$-$C_6$ alkyl)$_2$;
(iii) $C_1$-$C_3$ alkoxy optionally substituted with at least one substituent selected from the group consisting of halogen, OH, CN, oxo, $NH_2$, NH($C_1$-$C_6$ alkyl) and N($C_1$-$C_6$ alkyl)$_2$; or
(iv) $C_3$-$C_6$ cycloalkyl optionally substituted with at least one substituent selected from the group consisting of halogen, OH, CN, oxo, $NH_2$, NH($C_1$-$C_6$ alkyl) and N($C_1$-$C_6$ alkyl)$_2$.

In the present specification, when a combination of substituents is mentioned as one group, for example, arylalkyl, cycloalkylalkyl, or the like, the last-mentioned group generally contains the atom attached to the end of the molecule.

In the present specification, "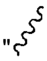 ", "*", or "-" is used to indicate a position at which a substituent is bonded to the remaining moiety of the compound. For example, if - is indicated at the end of a substituent, it means that the end is attached to the remaining moiety of the compound. In addition, when two or more substituents are linked by "-", it means that the substituent immediately before "-" is bonded to a substitutable atom of the substituent immediately after "-".

As used herein, the term "solvate" may refer to a compound of the present invention or a salt thereof comprising a stoichiometric or non-stoichiometric amount of a solvent bound by noncovalent intermolecular forces. Preferred solvents therefor may be solvents that are volatile, nontoxic, and/or suitable for administration to humans.

As used herein, the term "stereoisomer" may refer to a compound of the present invention or a salt thereof that has the same chemical formula or molecular formula but is optically or sterically different, and specifically, may be a diastereomer, an enantiomer or a geometric isomer.

In some embodiments, the compound of the present invention may be in the form of a racemate, a single enantiomer, a mixture of enantiomers, a single diastereomer, a mixture of diastereomers, and the like, containing one or more asymmetric centers. In one embodiment, due to the limited rotation or nature of the asymmetric center, the compound of the present invention may be in the form of an enantiomer or a diastereomer.

When two or more asymmetric centers are present in the compound of the present invention, several diastereomers and enantiomers of the chemical structures disclosed herein may exist, and pure isomers, separated isomers, partially pure isomers, racemic mixtures or the like are all intended to fall within the scope of the present invention.

Purification of the isomers and separation of a mixture of the isomers may be achieved by standard techniques known in the art. For example, a diastereomeric mixture may be separated into its respective diastereomers by a chromatographic process or crystallization, and a racemate may be separated into its respective enantiomers by resolution or a chromatographic process on a chiral phase.

The compound of the present invention may be used in the form of a pharmaceutically acceptable salt derived from an inorganic acid or organic acid, and for example, the salt may be a salt derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, toluenesulfonic acid, or the like.

A pharmaceutically acceptable salt of the compound may be prepared by dissolving the compound of formula I in a water-miscible organic solvent, such as acetone, methanol, ethanol, acetonitrile, or the like, and adding an excess of an

organic acid or adding an aqueous acid solution of an inorganic acid, and then precipitating or crystallizing. Subsequently, after evaporating the solvent or an excess of acid from this mixture, it may be prepared by drying to obtain an addition salt or by suction filtration of the precipitated salt.

## General preparation method of compound

The compound according to the present invention can be prepared through chemical modifications well known to one of ordinary skill in the art of organic/pharmaceutical chemistry according to the method representatively shown below.

The following general reaction scheme is a general illustration of a representative preparation method of the compound of formula I. One of ordinary skill in the art will be able to easily prepare the compound of formula I by appropriately selecting a starting material, a reaction temperature, a reaction condition, a catalyst, a solvent, a treatment method, and the like suitable for the desired compound, based on the preparation methods specifically disclosed in the examples herein. Hereinafter, in Reaction Schemes 1 to 9, the representation of each substituent of Formula I is the same as that of the substituent at the corresponding position in Formula I unless otherwise limited. In addition, in Reaction Schemes 1 to 9, the same variables are defined the same, and the descriptions of repeated definitions may be omitted.

In one aspect, a compound of Formula I can be prepared by reacting Intermediate a and Intermediate b according to the method of Reaction Scheme 1 below.

[Reaction Scheme 1]

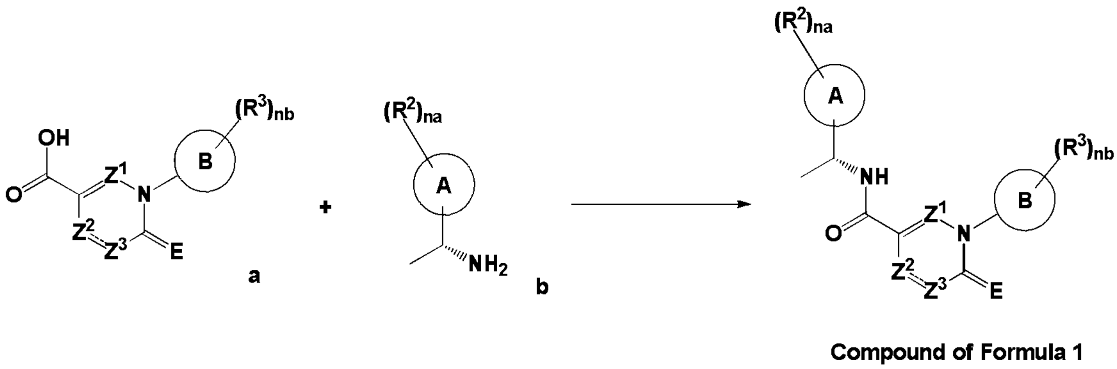

(In Reaction Scheme 1, na and nb are each independently appropriate integers satisfying the number of $R^2$ and $R^3$ defined in Formula I above.)

For example, a compound of Formula I can be prepared by coupling Intermediate a with Intermediate b through an amide coupling reaction using HATU. In one embodiment, when $R^2$ is an $NO_2$ group, the compound of Formula I having an $NH_2$ substituent on ring B can be prepared by reduction to an $NH_2$ group under reducing reaction conditions.

One of ordinary skill in the art can replace the reaction reagent used in Reaction Scheme 1 with various reagents for performing the amide coupling reaction based on common knowledge in the related field, and accordingly, will be able to select reaction conditions such as appropriate reaction time and reaction temperature. In one embodiment, Intermediates a and b may be reacted in HATU, TEA and DMF at about 20 °C to about room temperature for about 2 h to about 3 h. Alternatively, Intermediates a and b may be reacted in HATU, DIEA and DMF at about 10 °C to about 30 °C for about 2 h to about 15 h. Alternatively, Intermediates a and b may be reacted in EDCI, HOBT, DMAP and DCM at about 10 °C to about 20 °C for about 10 h to about 15 h. Alternatively, Intermediates a and b may be reacted in TEA, HOBT, EDCI and DCM at about 20 °C to about 30 °C for about 2 h to about 5 h. Alternatively, Intermediates a and b may be reacted in DIEA, HOBT, EDCI and DMF at about 15 °C to about 25 °C for about 2 h to about 15 h.

In one embodiment, a compound of Formula I can be prepared according to the reaction of Reaction Scheme 1A below.

[Reaction Scheme 1A]

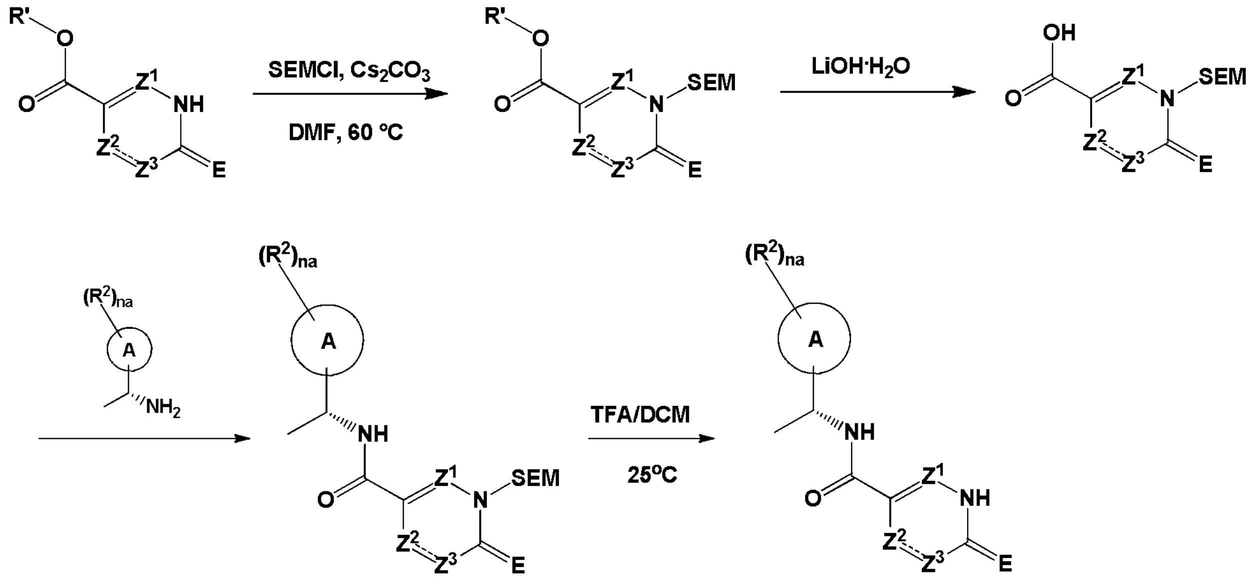


(In Reaction Scheme 1A, R' is alkyl.)

For example, according to Reaction Scheme 1A, a compound of Formula I in which the nitrogen atom of the ring is unsubstituted can be prepared by protecting the nitrogen atom in the ring of Intermediate a with SEM, and reacting with Intermediate b, and then removing the SEM.

In one embodiment, a compound of Formula I can be prepared according to the reaction of Reaction Scheme 1B below.

[Reaction Scheme 1B]

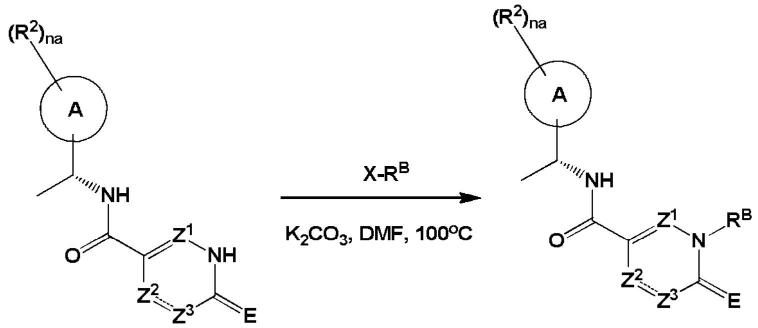


(In Reaction Scheme 1B, $R^B$ is alkyl optionally substituted with, for example, halogen, hydroxy, alkoxy, amino, alkylamino, dialkylamino, aryl or cycloalkyl.)

For example, $R^B$ may be introduced into the nitrogen atom by reacting a compound prepared according to Reaction Scheme 1A with a halide of $R^B$.

In one embodiment, a compound of Formula I can be prepared according to the method of Reaction Scheme 1C below.

[Reaction Scheme 1C]

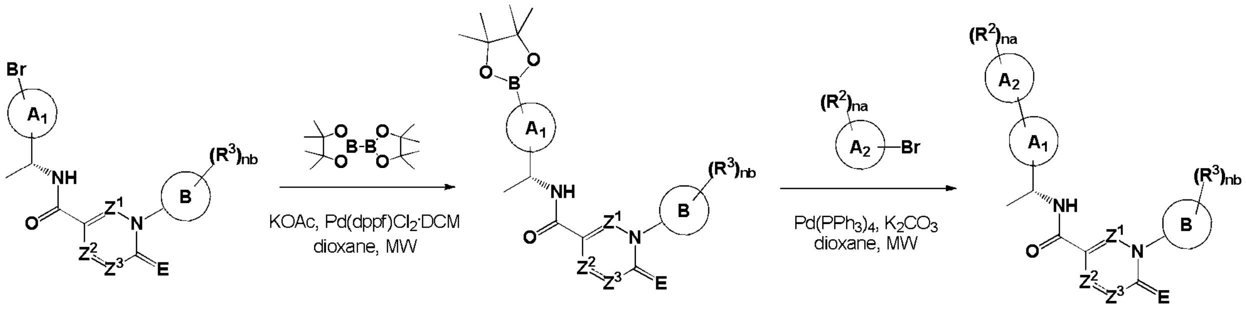


(In Reaction Scheme 1C, $A^1$ and $A^2$ are structures corresponding to $Cy_1$ and $Cy_2$ of Formula I, respectively.)

For example, according to Reaction Scheme 1C, a compound of Formula I can be prepared by coupling a starting material in which ring $A_1$ is halogenated with bis(pinacolato)diborane under an appropriate catalyst (for example, Pd(dppf)$Cl_2$) to synthesize a pinacolborane compound, and then coupling with a halide of ring $A_2$.

In one embodiment, a compound of Formula I can be prepared according to the method of Reaction Scheme 1D below.

[Reaction Scheme 1D]

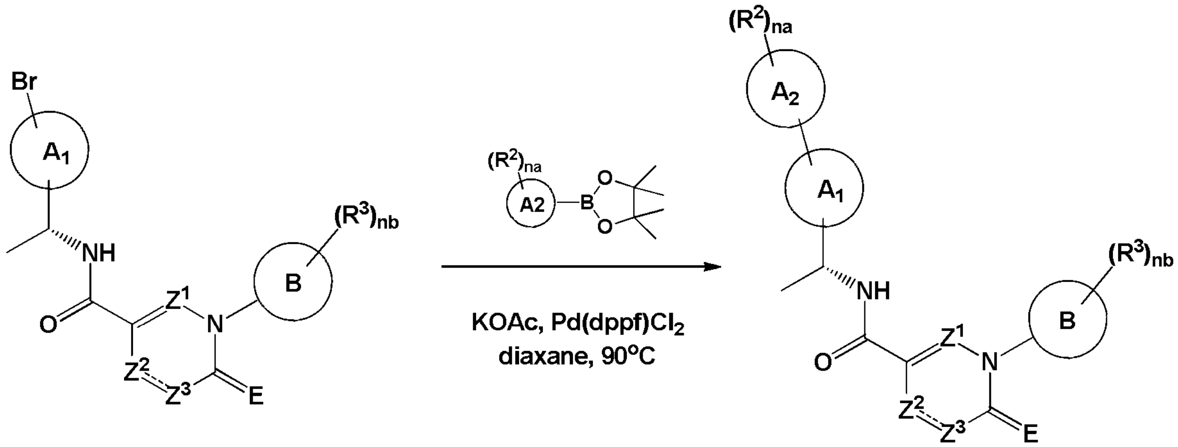


For example, according to Reaction Scheme 1D, a compound of Formula I can be prepared by coupling a starting material in which ring $A_1$ is halogenated with a pinacolborane or boronic acid derivative of ring $A_2$ under an appropriate catalyst (for example, Pd(dppf)$Cl_2$).

In one embodiment, a compound of Formula I can be prepared according to the method of Reaction Scheme 1E below.

[Reaction Scheme 1E]

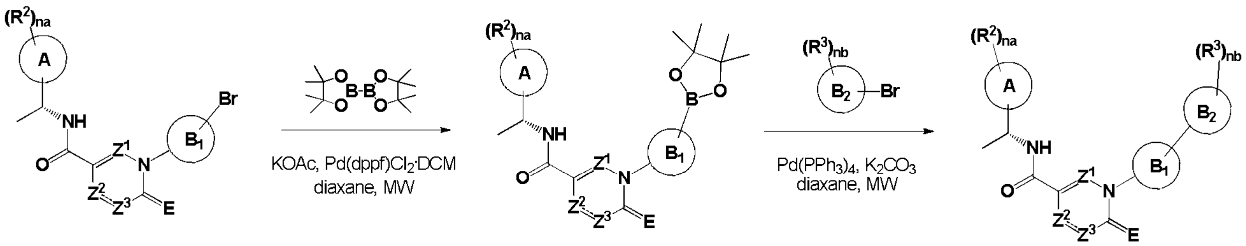


(In Reaction Scheme 1E, $B_1$ and $B_2$ are structures corresponding to $Cy_3$ and $Cy_4$ of Formula I, respectively.)

For example, according to Reaction Scheme 1E, a compound of Formula I can be prepared by coupling a starting material in which ring $B_1$ is halogenated with bis(pinacolato)diborane under an appropriate catalyst (for example, Pd(dppf)$Cl_2$) to synthesize a pinacolborane compound, and then coupling with a halogenated derivative of ring $B_2$.

In one embodiment, a compound of Formula I can be prepared according to the method of Reaction Scheme 1F below.

[Reaction Scheme 1F]

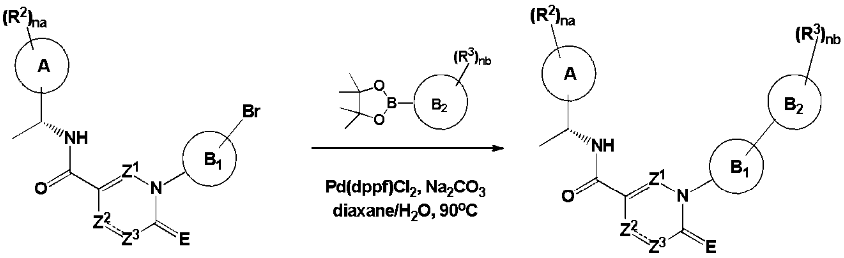


For example, according to Reaction Scheme 1F, a compound of Formula I can be prepared by coupling a starting material in which ring $B_1$ is halogenated with a pinacolborane or boronic acid compound of ring $B_2$ under an appropriate catalyst (for example, $Pd(dppf)Cl_2$).

In one embodiment, the $R^1$ group of Formula I may be introduced after Intermediate b is coupled with Intermediate a. For example, as illustrated in Reaction Scheme 2 below, when $R^1$ is CN, a compound of Formula I in which CN is substituted can be prepared using CuCN under an appropriate solvent (for example, N-methyl-2-pyrrolidone) after coupling an appropriate halogenated Intermediate a to which ring B is attached with Intermediate b in an appropriate solvent (for example, DCM, toluene), if necessary, in the presence of an appropriate catalyst (for example, $AlMe_3$).

[Reaction Scheme 2]

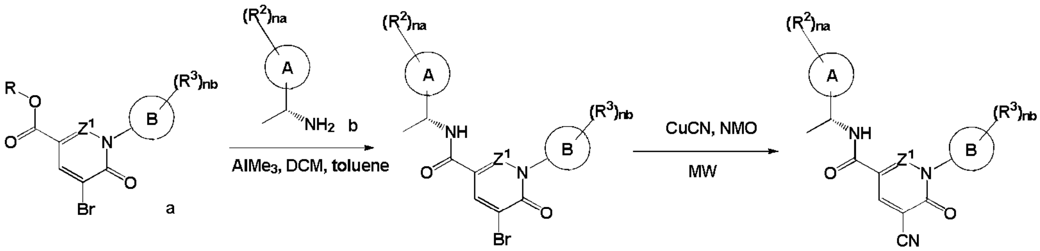


Compound of Formula 1

(In Reaction Scheme 2, R is H or alkyl.)

In one embodiment, Intermediate a in which $R^1$ is substituted can be prepared according to the method of Reaction Scheme 2A below.

[Reaction Scheme 2A]

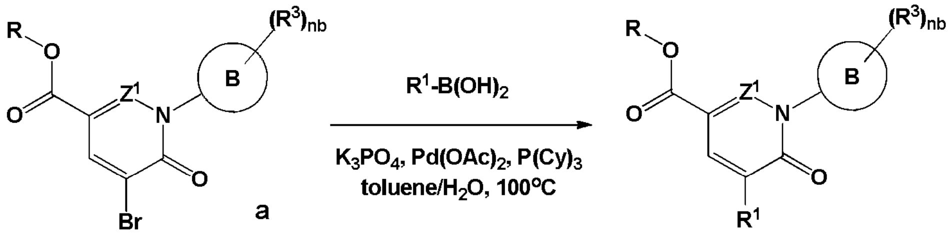


(In Reaction Scheme 2A, R is H or alkyl.)

For example, according to Reaction Scheme 2A, Intermediate a into which $R^1$ is introduced can be prepared by reacting an appropriate halogenated Intermediate a to which ring B is attached with a boronic acid compound of $R^1$.

In another embodiment, Intermediate a in which $R^1$ is alkyl can be prepared according to the method of Reaction Scheme 2B below.

[Reaction Scheme 2B]

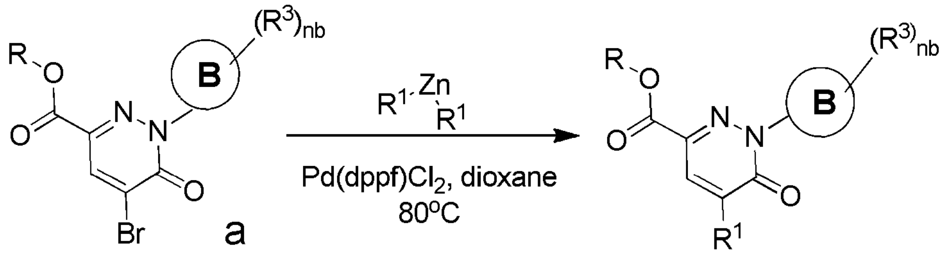


(In Reaction Scheme 2B, $R^1$ is alkyl.)

For example, Intermediate a into which an alkyl group is introduced as $R^1$ can be prepared by reacting an appropriate halogenated Intermediate a to which ring B is attached with a dialkyl zinc (Negishi reaction) in an appropriate solvent (for example, THF, dioxane, etc.) in the presence of an appropriate catalyst (for example, $Pd(PPh_3)_4$, Pd(dppf)$Cl_2$, etc.).

In one embodiment, Intermediate a can be prepared according to the method of Reaction Scheme 3 below.

[Reaction Scheme 3]

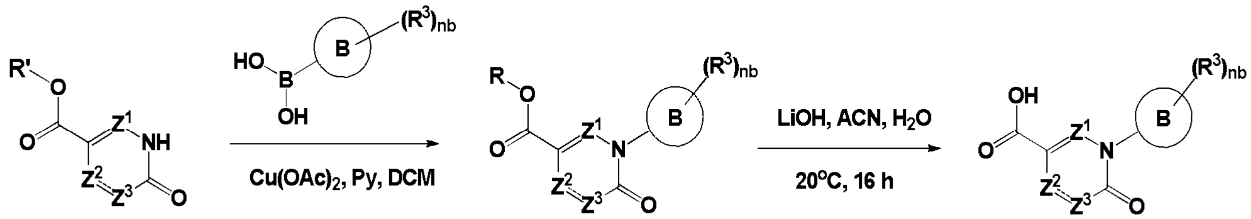


For example, Intermediate a can be prepared by dissolving an appropriate starting material in a solvent (for example, DCM), adding an appropriate amount of a base (for example, pyridine) and $Cu(OAc)_2$, then reacting with a boronic acid or 4,4,5,5-tetramethyl-1,3,2-dioxaborolane (pinacolborane) derivative of ring B, and hydrolyzing an ester group by addition of an appropriate base (for example, LiOH).

In another embodiment, Intermediate a can be prepared according to the method of Reaction Scheme 4 below.

[Reaction Scheme 4]

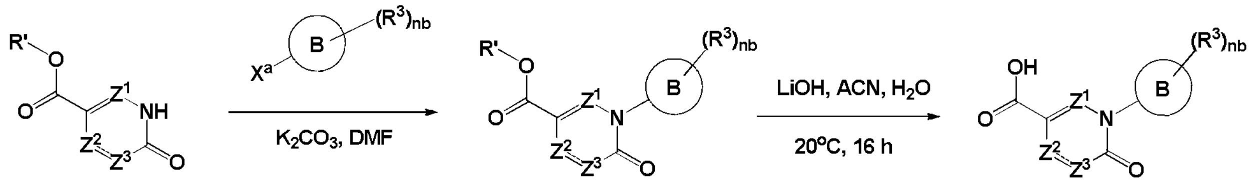


(In Reaction Scheme 4, $X^a$ is halogen, methylsulfonyloxy or trifluoromethylsulfonyloxy.)

For example, Intermediate a can be prepared by dissolving an appropriate starting material in a solvent (for example, DMF), adding an appropriate amount of a base (for example, $K_2CO_3$), then reacting with a halide, methylsulfonate or trifluoromethylsulfonate derivative of ring B, and hydrolyzing an ester group by addition of an appropriate base (for example, LiOH).

In another embodiment, Intermediate a can be prepared according to the method of Reaction Scheme 5 below.

[Reaction Scheme 5]

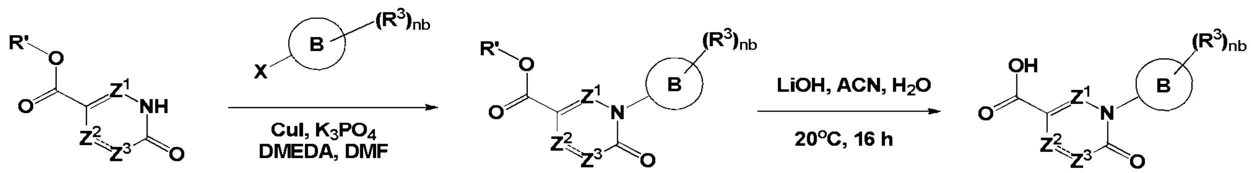


(In Reaction Scheme 5, X is halogen.)

For example, Intermediate a can be prepared by dissolving an appropriate starting material in a solvent (for

example, DMF), adding an appropriate amount of DMEDA, $K_3PO_4$, and CuI, then reacting with a halide compound of ring B, and hydrolyzing an ester group by addition of an appropriate base (for example, LiOH).

In another embodiment, Intermediate a can be prepared according to the method of Reaction Scheme 6 below.

[Reaction Scheme 6]

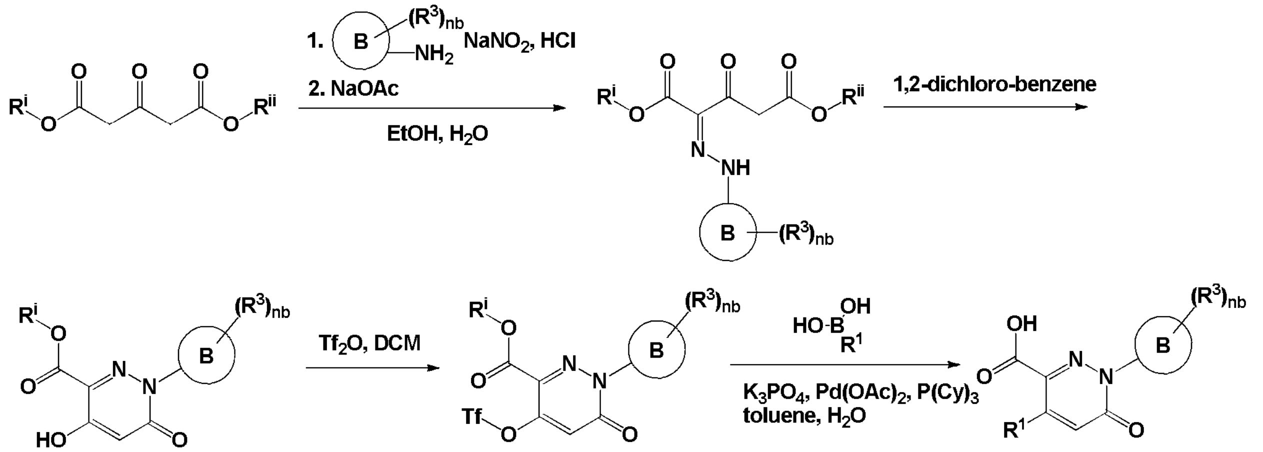


(In Reaction Scheme 6, $R^i$ and $R^{ii}$ are each alkyl.)

For example, Intermediate a can be prepared by treating a mixture of an aminated ring B compound, water and HCl with $NaNO_2$, adding to NaOAc and 3-oxopentandioate in an appropriate solvent (for example, EtOH, water) to form a hydrazone compound, then stirring in an appropriate solvent (for example, 1,2-dichlorobenzene) to form a hydroxy group substituted dihydropyridazinone ring, then adding $Tf_2O$ in an appropriate solvent (for example, DCM) to introduce a trifluoromethylsulfonyloxy group, and reacting with a boronic acid compound of $R^1$.

In one embodiment, Intermediate a can be prepared according to the method of Reaction Scheme 6A below.

[Reaction Scheme 6A]

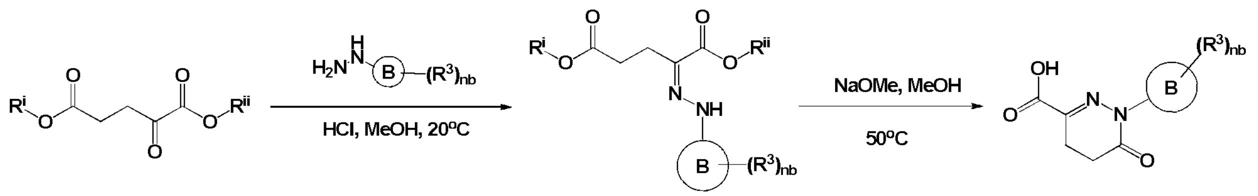


For example, Intermediate a can be prepared by reacting a hydrazinated ring B compound with 2-oxopentandioate in the presence of MeOH and HCl to form a hydrazone compound, and then stirring under NaOMe and MeOH to form a tetrahydropyridazinone ring.

In one embodiment, Intermediate a can be prepared according to the method of Reaction Scheme 6B below.

[Reaction Scheme 6B]

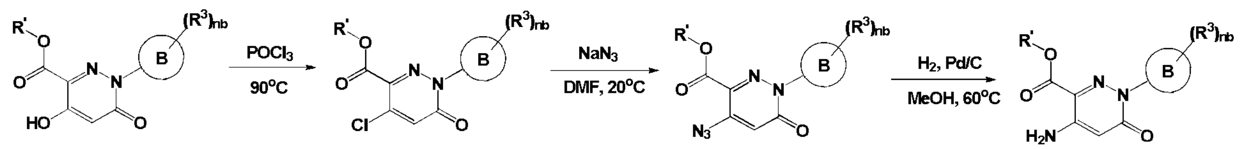


For example, according to Reaction Scheme 6B, Intermediate a into which an amino group is introduced as $R^1$ can be prepared by sequentially reacting a hydroxy substituted dihydropyridazinone ester compound with $POCl_3$ and $NaN_3$ to change a hydroxy group to a chloro group, and then to an azido group, and reducing the azido group under a Pd/C catalyst.

In one embodiment, Intermediate a can be prepared according to the method of Reaction Scheme 6C below.

[Reaction Scheme 6C]

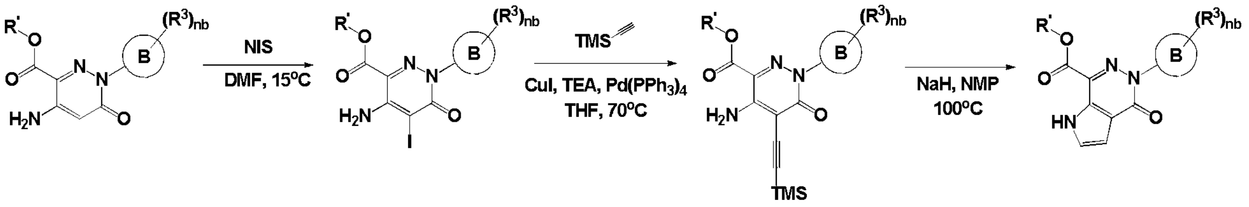


For example, a compound into which a trimethylsilylethynyl group is introduced can be synthesized by reacting Intermediate a prepared according to Reaction Scheme 6B with NIS to introduce an iodo group, and coupling with ethynyl(trimethyl)silane. Thereafter, Intermediate a having a pyrrolodihydropyridazinone core can be prepared through a cyclization reaction under NaH and NMP conditions.

In one embodiment, Intermediate a can be prepared according to the method of Reaction Scheme 6D below.

[Reaction Scheme 6D]

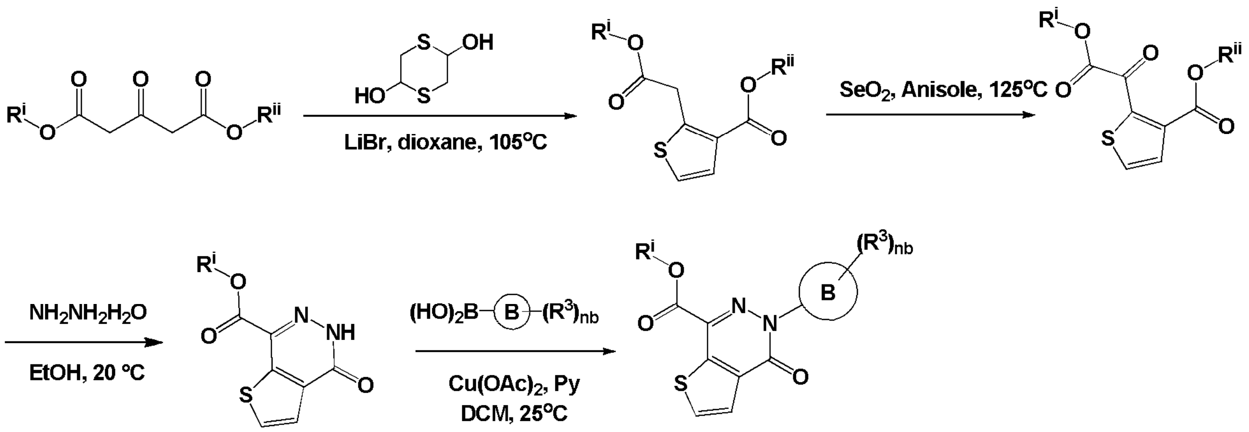


For example, according to Reaction Scheme 6D, a 3-oxopentandioate compound may be reacted with 1,4-dithiane-2,5-diol in the presence of LiBr to form a thiophene diester compound. Intermediate a having a thienodihydropyridazinone core can be prepared by reacting a thiophene diester compound with $SeO_2$ in an anisole solvent to additionally introduce an oxo group to the thiophene diester compound, and performing cyclization reaction with hydrazine, and then reacting with a boronic acid derivative of ring B.

In one embodiment, Intermediate a can be prepared according to the method of Reaction Scheme 6E below.

[Reaction Scheme 6E]

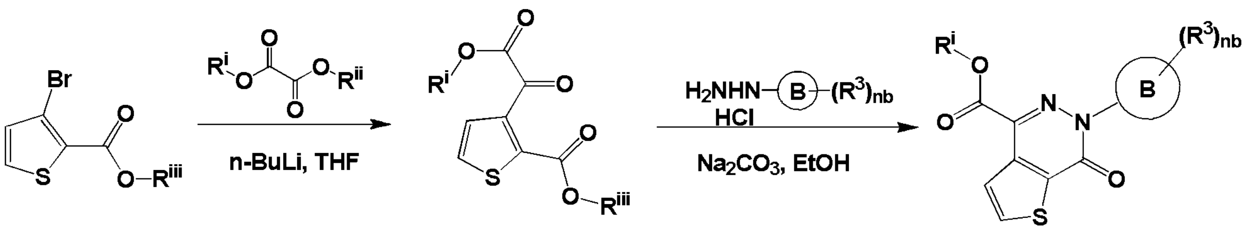


(In Reaction Scheme 6E, $R^i$ to $R^{iii}$ are alkyl.)

For example, according to Reaction Scheme 6E, Intermediate a having a thienodihydropyridazinone core can be prepared by reacting a brominated thiophene ester compound with oxalate in the presence of n-BuLi to synthesize a thiophene oxodiester compound, and then reacting with a ring B compound substituted with hydrazine.

In another embodiment, Intermediate a in which $R^1$ is alkyl can be prepared according to the method of Reaction Scheme 7 below.

[Reaction Scheme 7]

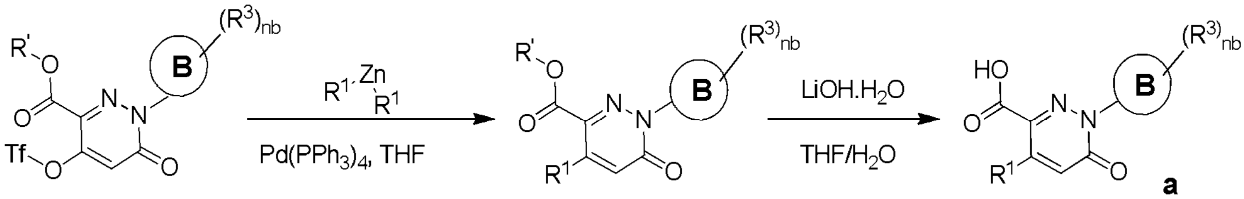


a

Intermediate a can be prepared by reacting a compound into which a trifluoromethylsulfonyloxy group is introduced, which is prepared in the method of Reaction Scheme 6, with a dialkyl zinc (Negishi reaction) in an appropriate solvent (for example, THF, dioxane, etc.) in the presence of appropriate catalyst (for example, $Pd(PPh_3)_4$, $Pd(dppf)Cl_2$, etc.) to introduce an alkyl group into a dihydropyridazinone ring, and then hydrolyzing an ester group by addition of an appropriate base (for example, LiOH).

In one embodiment, Intermediate a can be prepared according to the method of Reaction Scheme 7A below.

[Reaction Scheme 7A]

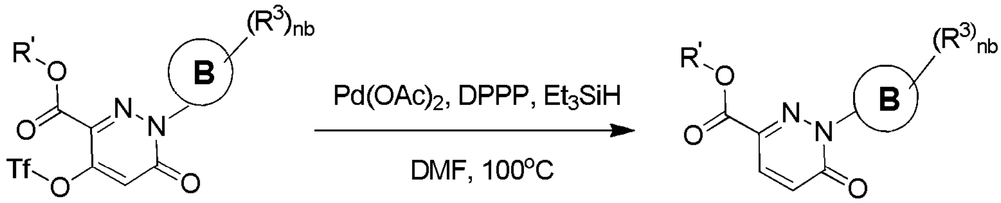


According to Reaction Scheme 7A, a compound into which a trifluoromethylsulfonyloxy group is introduced may be reacted with DPPP and $Et_3SiH$ in an appropriate solvent (for example, DMF) in the presence of an appropriate catalyst (for example, $Pd(OAc)_2$) to remove a trifluoromethylsulfonyloxy group.

In one embodiment, Intermediate a can be prepared according to the method of Reaction Scheme 8 below.

[Reaction Scheme 8]

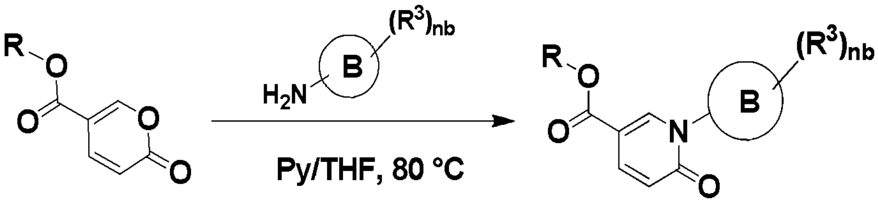


For example, according to Reaction Scheme 8, Intermediate a having a pyridinone core can be prepared by reacting a coumalate compound with an amine compound of ring B in the presence of pyridine.

In one embodiment, Intermediate b can be prepared according to the method of Reaction Scheme 9 below.

[Reaction Scheme 9]

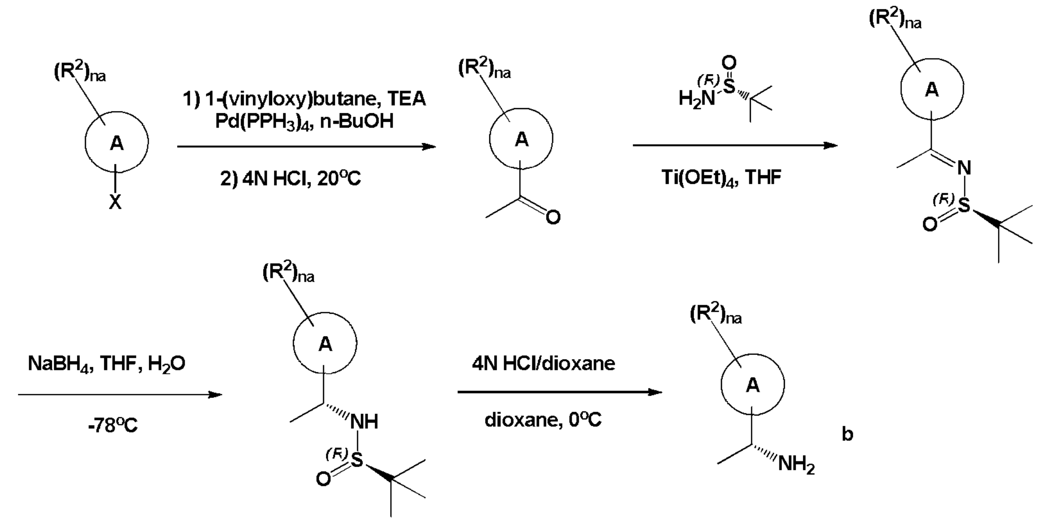


b

For example, a halogenated ring A compound is reacted with 1-vinyloxybutane or tributyl(1-ethoxyvinyl) stannane under Heck reaction conditions, followed by treatment with an acid to obtain an acetylated ring A compound. The reaction may be performed in the presence of $Pd(PP_3)_4$ or $Pd(PP_3)_2Cl_2$, and a solvent such as TEA, butanol, or dioxane may be used. Thereafter, the acetylated ring A compound may be reacted with tert-butyl sulfinamide having an (R) orientation in the presence of titanium alkoxide, and the imine bond may be reduced to an amine bond, and treated with an acid to prepare Intermediate b.

In one embodiment, when $R_2$ is an alkylsilane group, Intermediate b can be prepared according to the method of Reaction Scheme 10 below.

[Reaction Scheme 10]

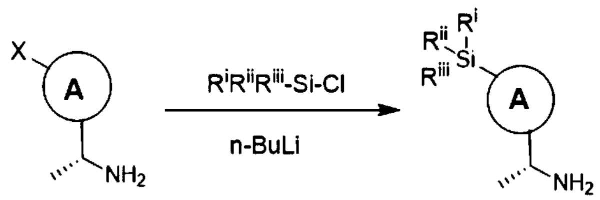


(In Reaction Scheme 10, $R^i$, $R^{ii}$ and $R^{iii}$ are each an alkyl group, and two of $R^i$, $R^{ii}$ and $R^{iii}$ may be optionally linked to each other to form cycloalkyl.)

For example, a halogenated ring A compound may be reacted with an appropriate alkyl silane halide compound in the presence of n-BuLi to introduce an alkylsilane group into ring A.

**Anticancer agent**

The anticancer agent of the present invention may be selected from the group consisting of chemical anticancer agents, targeted anticancer agents, anticancer viruses, antibody therapeutic agents, cell therapeutic agents, immune checkpoint inhibitors, and a combination thereof.

As used herein, the term "chemical anticancer agent" is also referred to as an anti-tumor drug (antineoplastic agent) or a cytotoxic agent. It is a general term for drugs that exhibit anticancer activity mainly by acting directly on DNA to block DNA replication, transcription, and translation processes, or by interfering with the synthesis of nucleic acid precursors in metabolic pathways and inhibiting cell division. The anti-tumor drug acts not only on tumor cells but also on normal cells and exhibits cytotoxicity. The chemical anticancer agent can be used for maintenance therapy. In addition, as used herein, the term "maintenance therapy" refers to treating cancer with drugs after initial anti-cancer treatment, and refers to a treatment method performed to prevent or delay the recurrence of cancer.

Specifically, chemical anticancer agent may be anyone selected from the group consisting of alkylating agents, microtubule inhibitors, antimetabolites and topoisomerase inhibitors. The alkylating agent may be anyone selected from the group consisting of Mechlorethamine, Cyclophosphamide, Ifosfamide, Melphalan, Chlorambucil, Thiotepa, Altretamine, Procarbazine, Busulfan, Streptozotocin, Carmustine, Lomustine, Dacarbazine, Cisplatin, Carboplatin and Oxaliplatin. The microtubule inhibitor may be anyone selected from the group consisting of Docetaxel, Paclitaxel, Velban, Oncovin and Navelbine. The anti-metabolite may be any one selected from the group consisting of Fluorouracil, Capecitabine, Cytarabine, Gemcitabine, Fludarabine, Methotrexate, Pemetrexed, 6-thioguanine and Mercaptopurine. The topoisomerase inhibitor may be anyone selected from the group consisting of Hycamtin, Camptosar, Vepesid, Blenoxane, Adriamycin, SN-38, Doxorubicin and Cerubidine.

As used herein, the term "targeted anticancer agent" is a therapeutic agent that specifically kills cancer cells by blocking signals involved in the growth and development of cancer by targeting changes in specific proteins or specific genes that frequently appear only in cancer cells. It is classified into monoclonal antibodies that react outside cells and small molecule substances that act inside cells. Monoclonal antibodies are anticancer agents that block cancer cell-inducing signals transmitted to the outside of cells, and act on initiation signals related to proliferation, death and the like, and small molecule substances act on complex signal transduction occurring inside cells.

Specifically, a protein to be targeted may be mTOR, PI3K, EGFR, VEGFR, CD20, CD38, RNAK-L, BTK, BCR-ABL, PDGFR/FGFR family, MEK, KRAS, ERK1/2, HER2/Neu, Ubiquitin, JAK, ALK, PARP, TGFβR, Proteasome, Bcl-2, C-Met, VR1, VR2, VR3, c-KIT, AXL, RET, BRAF, pan-RAF, SHP2, SRC, LCK, DNMT, CDK4/6, CDK9, BET, MDM2, IGF1/2 or IGF1-R, ROS1, NTRK1, PIK, DHFR, pan Aurora, Aurora A, WEE1, HSP90, A3AR, EZH2, ARID1A, Chk1, ATR, HDAC1/3, Akt, PLK1, SUMOylation-related proteins, STING and the like.

The targeted anticancer agent may be any one selected from the group consisting of Rapamycin, Sirolimus, Temsilorimus, Everolimus, Ridaforolimus, INK-128, Alpelisib, Cetuximab, Trastuzumab, Pertuzumab, Gefitinib, Erlotinib, Osimertinib, Lazertinib, Panitumumab, Axitinib, Lenvatinib, Bevacizumab, Ramucirumab, Aflibercept, Rituximab, Obinutuzumab, Daratumumab, Denosumab, Ibrutinib, Dasatinib, Nilotinib, Imatinib, Bosutinib, Galunisertib, Vactosertib, Futibatinib, Nintedanib, Sunitinib, Sorafenib, Cabozantinib, Regorafenib, Masitinib, Semaxanib, Tivozanib, Vandetanib, Pazopanib, Dabrafenib, Sotorasib, Adagrasib, JDQ443, MRTX1133, Ulixertinib, Afatinib, Lapatinib, Neratinib, Lenalidomide, Ixazomib, Ruxolitinib, Lestaurtinib, Pacritinib, Trametinib, Cobimetinib, Selumetinib, Binimetinib, Alectinib, Lorlatinib, Crizotinib, Venetoclax, Bemcentinib, Gilteritinib, Selpercatinib, Pralsetinib, Encorafenib, Vemurafenib, Belvarafenib, RMC-4630, Batoprotafib, WH-4-023, Olaparib, Talazoparib, Niraparib, Rucaparib, Azacitidine, Decitabine, Guadecitabine, Abemaciclib, Ribociclib, Palbociclib, CDNs, SB11285, Rineterkib, Repotrectinib, Tepotinib, Alrizomadlin, JQ1, NVP-ADW742, Duvelisib, Irbinitinib, Danusertib, MK-1775, AMG-900, BIIB021, Reversine, MLN-7243, ABT-737, MK-5108, GSK-343, 2-D08, SCH-900776, Entinostat, Carfilzomib, Apitolisib, Ipatasertib, Volasertib, AT-7519, Methotrexate, Wortmannin, ERAS-007, PYR-41, MLN4924, RO-5503781, MK-8242, SAR-405838, CGM097, DS3032b, Lactacystin, Disulfiram, Epigallocatechin-3-gallate, Marizomib, Oprozomib, Delanzomib, Epoxomicin, MG132, Beta-hydroxy beta-methylbutyrate, Bortezomib, Navitoclax, Naporafenib, PF-07284892, TNO155, Hesperadin, LY3295668 , Tozasertib, Azenosertib, ZNL-02-096, RP-6306, GSK-1520489A, BIIB028, MPC-3100, PU-H71, Debio093, SNX-5422, AUY922, KF-26777, MRS-545, CAY10498, DZNep, EPZ005687, EI1, GSK126, UNC1999, Tazemetostat, Sinefungin, GSK-343, Davidiin, CID9549553, SRA737, V158411, PF-477736, AZD7762, Prexasertib, Berzosertib, Gartisertib, Ceralasertib, Panobinostat, Mocetinostat, Trichostatin A, CBUD-1001, Abexinostat, VQD-002, Perifosine, Miltefosine, MK-2206, AZD5363, Rigosertib, I-BET 151, I-BET 762, OTX-015, TEN-010, CPI-203, CPI-0610, Olinone, RVX-208, ABBV-744, LY294002, AZD5153, MT-1, MS645, Figitumumab, Mecasermin, rhIGF-1, BI 885578, Buparlisib, Copanlisib, Dactolisib, Idelalisib, Parsaclisib, Paxalisib, Taselisib, Zandelisib, Inavolisib, AZD4573, Atuveciclib, VIP152, A-1592668, JSH-150, SLS009, Roscovitine and DMXAA.

As used herein, the term "mTOR (mammalian target of rapamycin)" is also referred to as mechanistic target of rapamycin or FRAP1 (FK506 binding protein 12-rapamycin associated protein 1), and is a protein belonging to the PIKK (phosphatidylinositol 3-kinase-related kinase) family. mTOR is encoded by the FRAP1 gene in humans and is a serine/threonine protein kinase that regulates cell growth, cell proliferation, cell motility, cell survival, protein synthesis, and transcription. The mTOR inhibitor can inhibit tumor survival by inhibiting autophagy, lipogenesis, proliferation, and protein synthesis, etc. The mTOR inhibitor may be, for example, rapamycin, sirolimus, temsilorimus, everolimus, ridaforolimus or INK-128 (sapanisertib, MLN0128, TAK-228).

As used herein, the term "PI3K (Phosphoinositide 3-kinase)" is also referred to as phosphatidylinositol 3-kinase, and is an enzyme involved in cellular functions such as cell growth, proliferation, differentiation, motility, survival, and intracellular signal regulation, and is associated with cancer. PI3K includes subunits such as p110-$\alpha/\beta/\gamma/\delta$ (PI3K$\alpha/\beta/\gamma/\delta$). PI3K targeted anticancer agents may include alpelisib, wortmannin, LY294002, idelalisib, copanlisib, duvelisib, apitolisib (GDC-0980, RG7422, GNE 390), and the like.

As used herein, the term "epidermal growth factor receptor (EGFR)" is a cell membrane receptor that regulates cell growth, division, survival and death, and expression of EGFR is increased in tumor tissues in various cancers. Tumor tissues with increased EGFR are known to have high invasion, metastasis and resistance to anticancer agents. In one embodiment, a substance that inhibits the EGFR as an EGFR inhibitor may be cetuximab, trastuzumab, pertuzumab, gefitinib, erlotinib, osimertinib, lazertinib or panitumumab.

As used herein, the term "vascular endothelial growth factor receptor (VEGFR)" is a cell membrane receptor for

vascular endothelial growth factor that induces angiogenesis, and a VEGFR inhibitor inhibits the angiogenesis to inhibit tumor growth and metastasis. In one embodiment, a VEGF inhibitor or a VEGFR inhibitor may be axitinib, lenvatinib, bevacizumab, ramucirumab or aflibercept.

As used herein, the term "CD20 (B lymphocyte antigen CD20)" is a protein expressed on the surface of B cells and is used as a target protein for treatment of B cell lymphoma. An inhibitor targeting CD20 may be rituximab or obinutuzumab.

As used herein, the term "CD38 (cluster of differentiation 38)" is a protein that regulates cell proliferation and death while acting as a signal transduction system receptor in immune cells, and an inhibitor targeting this protein may be daratumumab.

As used herein, the term "RNAK-L (receptor activator of nuclear factor kappa-B ligand)" is a RANK receptor expressed on the surface of osteoclasts, and when activated by binding to the ligand, it acts to cause bone destruction. A RANK-L inhibitor is mainly used for cancer patients suffering from bone metastasis or osteoporosis, and may be specifically denosumab.

As used herein, the term "BTK (Bruton's tyrosine kinase)" is an enzyme involved in the proliferation of B cells, and when overexpressed, it can develop into hematological cancer. In one embodiment, an inhibitor targeting BTK may be ibrutinib.

As used herein, the term "BCR-ABL" is a fusion protein that is highly expressed in patients with chronic myeloid leukemia, and is known to induce abnormal proliferation of blood cells. Specifically, the inhibitor of this protein may be dasatinib, nilotinib, imatinib or bosutinib.

As used herein, the term "tumor growth factor β receptor (TGFβR)" is a cell membrane receptor for tumor growth factor, and regulates the growth, migration, differentiation and death and the like of epithelial cells and hematopoietic cells. An inhibitor targeting TGFβR may include galunisertib or vactosertib, but is not limited thereto.

As used herein, the term "PDGFR (platelet derived growth factor receptor)" is a cell membrane receptor for PDGF that is frequently expressed in cancer cells, and is known to be involved in angiogenesis to regulate cancer growth, metastasis, and drug resistance. FGFR (fibroblast growth factor receptor) is a receptor for fibroblast growth factor (FGF) and regulates various biological processes including cell growth, differentiation and migration and the like. The FGFR gene is frequently mutated, and these mutants are commonly observed in breast cancer, uterine cancer, ovarian cancer, cervical cancer, and the like. An inhibitor targeting PDGFR or FGFR may be futibatinib, nintedanib, sunitinib, imatinib, sorafenib, cabozantinib, lenvatinib, regorafenib, masitinib, semaxanib, tivozanib, vandetanib, axitinib or pazopanib.

As used herein, the term "MEK (mitogen-activated protein kinase kinase)" is a dual-specificity kinase enzyme that phosphorylates MAPK (mitogen-activated protein kinase), also referred to as MAP2K, MEK, or MAPKK. When MEK is inhibited, cell proliferation is blocked and cell death is induced. MEK targeted anticancer agents may be cobimetinib, selumetinib, trametinib or binimetinib.

As used herein, the term "KRAS (Kirsten rat sarcoma virus)" refers to a gene that produces a protein referred to as K-Ras, which is part of the RAS/MAPK pathway, and is an oncogene that instructs the growth, division, proliferation, and differentiation signals of cells. KRAS targeted anticancer agents may be sotorasib, adagrasib, JDQ443 or MRTX1133.

As used herein, the term "ERK1/2 (extracellular signal-regulated kinases 1/2)" refers to a widely expressed protein kinase intracellular signal molecule involved in functions including the regulation of meiotic, mitotic, and post-mitotic functions in cells. Disruption of the ERK pathway is commonly observed in cancer. ERK1/2 targeted anticancer agents may be rineterkib, ulixertinib (BVD-523) or ERAS-007.

As used herein, the term "HER-2/neu (human epidermal growth factor receptor 2) regulates cell proliferation by the activation of PI3K/AKT. It is overexpressed in metastatic breast cancer and ovarian cancer and the like, and is known to induce resistance to anticancer agents. Her2/neu targeted anticancer agents may be trastuzumab, afatinib, lapatinib, irbinitinib (tucatinib) or neratinib.

As used herein, the term "ubiquitin" maintains cellular homeostasis by binding to other proteins and inducing proteolysis by proteasome, a proteolytic enzyme (ubiquitin-proteasome system, UPS). Abnormal expression or activity of the UPS is observed in various tumors, and the inhibitor of UPS exhibits anticancer activity. For example, an inhibitor targeting ubiquitin E1 enzyme may include MLN-7243 (TAK-243), PYR-41, MLN4924, and the like, and MDM2 E3 ubiquitin ligase inhibitors may include RO-5503781 (idasanutlin), MK-8242, SAR-405838, CGM097, DS3032b, and the like.

As used herein, a "proteasome inhibitor" can treat cancer by blocking the action of the proteasome, a cellular complex that degrades proteins. The inhibition of proteasome prevents the degradation of pro-apoptotic factors such as p53 protein, thereby activating programmed cell death in tumor cells that relies on inhibition of pro-apoptotic pathways. Proteasome inhibitors may include lactacystin, disulfiram, epigallocatechin-3-gallate, marizomib (salinosporamide A), oprozomib (ONX-0912), delanzomib (CEP-18770), epoxomicin, MG132, beta-hydroxy beta-methylbutyrate, bortezomib, carfilzomib, ixazomib, and the like.

As used herein, the term "JAK (Janus kinase)" is an upstream protein to STAT, which is a transcription factor that regulates cell proliferation, cell survival, cell migration and immune response, and an inhibitor of JAK is known to reduce cell proliferation and induce cell death through inhibition of STAT activity. JAK includes JAK1, JAK2, JAK3 and TYK2

(tyrosine kinase 2). An inhibitor targeting JAK may be ruxolitinib, lestaurtinib or pacritinib.

As used herein, the term "ALK (anaplastic lymphoma kinase)" is a signal transduction mediator that promotes cell proliferation, cell migration and angiogenesis and inhibits cell death, and is overactivated in various cancer tissues. An inhibitor targeting ALK may be alectinib, lorlatinib or crizotinib.

As used herein, the term "BCL-2" is a protein that inhibits cell death, and is overexpressed or overactivated in various cancer tissues. An inhibitor targeting BCL-2 may include venetoclax, ABT-737, navitoclax (ABT-263), and the like.

As used herein, the term "C-MET" is a receptor for hepatocyte growth factor (HGF), and activates signal transduction related to cell growth, formation, motility, survival and angiogenesis and the like. A C-MET targeted anticancer agent may be crizotinib, tepotinib or cabozantinib.

As used herein, the term "VR (vanilloid receptor) is also known as TRPV (transient receptor potential vanilloid), and exists in the form of VR1, VR2, VR3, VR4, VR5 and VR6. VR is known to regulate proliferation, death, migration, infiltration and angiogenesis of cancer cells at each stage in the cancer progression process.

As used herein, the term "c-KIT" is also known as CD117, and induces signal transduction that activates cell survival, proliferation and differentiation. c-KIT is a proto-oncogene, and overexpression or mutation of this gene is associated with cancer development. In one embodiment, a c-KIT targeted anticancer agent may be imatinib, dasatinib or regorafenib.

As used herein, the term "AXL (tyrosine-protein kinase receptor UFO)" is a tyrosine kinase receptor present on the cell surface and mediates signal transduction involved in cell proliferation and survival. It is known to be involved in resistance to anticancer agents in anticancer treatment. In one embodiment, an AXL targeted anticancer agent may be bemcentinib or gilteritinib.

As used herein, the term "RET (rearranged during transfection)" is a receptor that mediates signals involved in cell proliferation, cell death and survival, and mutations in RET are known to be involved in cancer development. An inhibitor targeting RET may be selpercatinib or pralsetinib, but is not limited thereto.

As used herein, the term "BRAF" is a MAPK signal transduction mediator involved in cell proliferation, cell cycle regulation, cell survival, angiogenesis, cell migration, and the like, and genetic mutations are observed in cancer cells. An inhibitor targeting BRAF may be dabrafenib, encorafenib (LGX818) or vemurafenib.

As used herein, the term "pan-RAF" encompasses RAF family substances such as BRAF, ARAF, and CRAF. An inhibitor targeting pan-RAF may be naporafenib, belvarafenib or sorafenib.

As used herein, the term "SHP2 (Src homology region 2 domain-containing phosphatase-2)" is also referred to as tyrosine-protein phosphatase non-receptor type 11 (PTPN11) or protein-tyrosine phosphatase 1D/2C (PTP-1D/2C), and it is known to be a signal molecule that regulates various cellular processes, including cell growth, differentiation, mitotic cycle, and oncogenic transformation. Activation of SHP2 mutations is found in neuroblastoma, melanoma, acute myeloid leukemia, breast cancer, lung cancer, colorectal cancer, and the like. An inhibitor targeting SHP2 may include, for example, PF-07284892, RMC-4630 (SHP2-IN-7) or batoprotafib (TNO155).

As used herein, the term "SRC (proto-oncogene tyrosine-protein kinase)" refers to a non-receptor tyrosine kinase protein, also known as c-Src, that regulates embryonic development and cell growth, and an elevated level of its activity is known to be associated with cancer progression. The SRC inhibitor may be, for example, dasatinib or bosutinib.

As used herein, the term "LCK (lymphocyte-specific protein tyrosine kinase)" belongs to the SFK (Src kinase family) and activates T cell receptor signal transduction. Mutations and dysfunction of LCK inhibit T cell activation, and overexpression of LCK is known to be associated with cancer, asthma, type 1 diabetes mellitus, rheumatoid arthritis, psoriasis, systemic lupus erythematosus, inflammatory bowel disease (Crohn's disease and ulcerative colitis), and the like. An inhibitor targeting LCK may be, for example, WH-4-023.

As used herein, the term "PARP (poly [ADP-ribose] polymerase)" is a protein that is activated by recognition of damaged DNA in the nucleus and then activates DNA repair related proteins. An inhibitor targeting PARP inhibits the proliferation of cancer cells by inhibiting DNA repair in cancer cells. In one embodiment, an inhibitor targeting PARP may be olaparib, talazoparib, niraparib or rucaparib.

As used herein, the term "DNA methyltransferase (DNMT)" is an enzyme that attaches a methyl group to a histone protein wrapped around DNA, and through this process, the expression of the gene is inhibited. An inhibitor targeting DMNT exhibits anticancer activity by inhibiting hypermethylation of cancer suppressor genes and inducing normal expression of cancer suppressor genes. In one embodiment, an inhibitor targeting DNMT may be azacitidine, decitabine, or guadecitabine.

As used herein, the term "CDK (cyclin dependent kinase) 4/6" is a protein that promotes cell growth by regulating the cell cycle, and is overactivated during the development and progression of various malignant tumors. An inhibitor targeting CDK4/6 exhibits anticancer activity by inhibiting the cell cycle of cancer cells, inhibiting cell proliferation and inducing cell death. An inhibitor targeting CDK4/6 may be abemaciclib (LY2835219), ribociclib or palbociclib.

As used herein, the term "Aurora kinase" is a serine/threonine kinase essential for cell proliferation and refers to a phosphotransferase enzyme that helps dividing cells distribute genetic material to daughter cells. Aurora kinase plays an important role in cell division by controlling chromatid segregation, and defects in segregation can lead to tumorigenesis.

Aurora A (Aurora 2) functions during mitotic prophase and is involved in the correct replication and segregation of centrosomes (microtubule-organizing centers in eukaryotic cells). Aurora B (Aurora 1) is responsible for attaching the mitotic spindle to the centromere. Aurora C (AURKC) acts in germ cells. An inhibitor targeting Aurora A may include MK-5108, hesperadin, LY3295668, and the like. An inhibitor targeting pan-Aurora may include danusertib, AMG-900, reversine, tozasertib (VX-680), and the like.

As used herein, the term "WEE1" is a 96 kDa nuclear kinase belonging to the Ser/Thr protein kinase family, also referred to as mitosis inhibitor protein kinase Wee1. Mitosis promoting factor (MPF) regulates apoptosis caused by DNA damage, and negative regulation of MPF by WEE1 causes abnormal mitosis and resistance to apoptosis caused by DNA damage. An inhibitor targeting WEE1 can reduce the sensitivity to DNA damage-induced apoptosis in cancer cells by regulating WEE1. An inhibitor targeting WEE1 may include MK-1775 (adavosertib), azenosertib (ZN-C3), ZNL-02-096, and the like.

As used herein, the term "PKMYT1 (protein kinase, membrane-associated tyrosine/threonine 1)" belongs to the Wee1 protein kinase family, and is a regulator of CDK1 phosphorylation, and is a potent therapeutic target for the treatment of certain types of DNA damage-responsive cancers through the synthetic lethality of CCNE1 amplification. An inhibitor targeting PKMYT1 may include RP-6306, GSK-1520489A, and the like.

As used herein, the term "HSP90 (heat shock protein 90)" refers to a chaperone protein that helps other proteins fold properly, stabilizes proteins from heat stress, and assists in protein degradation. It can stabilize a number of proteins required for tumor growth. HSP90 inhibitors may include BIIB021, BIIB028, MPC-3100, PU-H71, Debio093, SNX-5422, AUY922, and the like.

As used herein, the term "A3AR (adenosine A3 receptor; ADORA3)" refers to a G protein-coupled receptor that binds to Gi/Gq and is involved in various intracellular signaling pathways and physiological functions, and is overexpressed in pathological human cells, and can mediate cell proliferation and cell death. A therapeutic agent targeting A3AR may include reversine, KF-26777, MRS-545, CAY10498, and the like.

As used herein, the term "EZH2 (enhancer of zeste homolog 2)" refers to a histone-lysine N-methyltransferase enzyme encoded by the EZH2 gene, which participates in histone methylation and ultimately transcriptional repression. EZH2 is an attractive target for anticancer treatment because it helps cancer cells divide and proliferate, and is found in greater amounts than in healthy cells in a wide range of cancers, including breast cancer, prostate cancer, bladder cancer, uterine cancer, kidney cancer, as well as melanoma and lymphoma. An inhibitor targeting EZH2 may include DZNep, EPZ005687, EI1, GSK126, UNC1999, tazemetostat, sinefungin, and the like.

As used herein, the term "ARID1A (AT-rich interactive domain-containing protein 1A)" is a member of the SWI/SNF family, has helicase and ATPase activities, and regulates the transcription of specific genes by altering the chromatin structure around those genes. The ARID domain is a DNA binding domain that can specifically bind to AT-rich DNA sequences known to be recognized by the SWI/SNF complex at the beta-globin locus, and the C-terminus of the protein can stimulate glucocorticoid receptor-dependent transcriptional activation. Mutations in this gene are commonly found in gastric cancer, ovarian clear cell carcinoma, and pancreatic cancer. An inhibitor targeting EZH2/ARID1A may include GSK-343, and the like.

As used herein, the term "SUMOylation" is a post-translational modification that covalently attaches a small ubiquitin-like modifier (SUMO) polypeptide to a lysine residue of a target protein. The enzymatic pathway of SUMOylation is very similar to ubiquitination and includes activating enzymes, conjugation enzymes, ligases, and deconjugation enzymes. Dysregulation of the SUMOylation pathway is observed in cancer and neurological diseases, where SUMO enzymes are upregulated in many cancers and SUMO levels are directly correlated with prognosis and disease progression. SUMOylation inhibitors may include Davidiin, CID9549553, 2-D08, and the like.

As used herein, the term "CHK1 (checkpoint kinase 1; CHEK1)" is a serine/threonine-specific protein kinase that modulates DNA damage response (DDR) and cell cycle checkpoint responses. Activation of CHK1 results in the initiation of cell cycle checkpoints, cell cycle arrest, DNA repair, and apoptosis, thereby preventing damaged cells from progressing through the cell cycle. CHK1 is overexpressed in numerous tumors, including breast cancer, colon cancer, liver cancer, gastric cancer, and nasopharyngeal cancer, and the positive correlation between CHK1 expression and tumor grade and disease recurrence suggests that CHK1 can promote tumor growth. An inhibitor targeting CHK1 may include SCH-900776, SRA737, V158411, PF-477736, AZD7762, LY2880070 (prexasertib), and the like.

As used herein, the term "ATR (ataxia telangiectasia mutated (ATM) and RAD3-related kinase)" refers to a protein kinase implicated in cellular responses to certain forms of DNA damage (for example, double-strand breaks and replication stress). Normal cells repair damaged DNA using the ATM/ATR signal transduction system, which regulates the cellular response to double-strand DNA breaks and replication stress, referred to as the DNA damage response ("DDR"). On the other hand, many cancer cells show a high dependence on DNA repair proteins, including ATR, due to defects in ATM in the DNA repair process. An inhibitor targeting ATR may be berzosertib (VX-970), gartisertib (VX-803) or ceralasertib (AZD6738).

As used herein, the term "HDAC (histone deacetylase)" refers to an enzyme that removes the acetyl group from the ε-N-acetyl lysine amino acid of histone and non-histone proteins, and histones wrap DNA more tightly to regulate the

expression of DNA through acetylation and deacetylation. HDAC includes subgroups such as Class I, such as HDAC1, HDAC2, and HDAC3, and Class IIA, such as HDAC4, HDAC5, and HDAC7. An HDAC inhibitor exhibits anticancer efficacy in studies on pancreatic cancer, esophageal squamous cell carcinoma (ESCC), multiple myeloma, prostate carcinoma, gastric cancer, leukemia, breast cancer, liver cancer, ovarian cancer, nasal cancer, Hodgkin’s lymphoma, and neuroblastoma. An inhibitor targeting HDAC may include panobinostat (LBH589), entinostat, mocetinostat, trichostatin A, CBUD-1001, abexinostat (PCI-24781, CRA-024781), and the like.

As used herein, the term "AKT (protein kinase B: PKB)" is a set of serine/threonine-specific protein kinases that play key roles in several cellular processes such as glucose metabolism, apoptosis, cell proliferation, and transcription, and is associated with tumor cell survival, proliferation and invasiveness. AKT is commonly observed in tumor cells, and these cells depend on AKT for survival. An inhibitor targeting AKT may include VQD-002, perifosine, miltefosine, MK-2206, AZD5363, ipatasertib, and the like.

As used herein, the term "PLK1 (Polo-like kinase 1)" is also referred to as serine/threonine-protein kinase 1 or serine/threonine-protein kinase 13 (STPK13), and is a 66 kDa enzyme composed of 603 amino acids. Most colorectal cancer and lung cancer are caused by K-RAS mutations and are known to depend on PLK1. When PLK1 expression is silenced by RNA interference in cell culture, K-RAS cells can be selectively killed without harming normal cells. An inhibitor targeting PLK1 may include volasertib, rigosertib, and the like.

As used herein, the term "BET (bromodomain and extraterminal domain protein)" refers to a bromodomain composed of approximately 110 amino acid protein that recognize acetylated lysine residues, including BRD2, BRD3, BRD4, and BRDT. It converts the signals transmitted by the acetylated lysine residues and converts them into various normal or abnormal phenotypes. Bromodomains translate the dysregulated cellular acetylome into disease phenotypes, and BETs are targets in cancer and multiple sclerosis. An inhibitor targeting BET may include JQ1, I-BET 151 (GSK1210151A), I-BET 762 (GSK525762), OTX-015, TEN-010, CPI-203, CPI-0610, olinone, RVX-208, ABBV-744, LY294002, AZD5153, MT-1, MS645, and the like.

As used herein, the term "IGF (insulin-like growth factor)" is a protein with high sequence similarity to insulin and is involved in communication between cells and the physiological environment, and includes IGF1/2, IGF-1R, IGF-2R, and the like. IGF-1 stimulates the growth of prostate and breast cancer cells, and IGF has been found to be involved in diseases such as cancer and diabetes. An inhibitor targeting IGF1/2 or IGF-1R may include NVP-ADW742, figitumumab, mecasermin, rhIGF-1, BI 885578, and the like.

As used herein, the term "PIK (phosphatidylinositol kinase)" consists of phosphatidylinositol 3-kinase (PI3K) and phosphatidylinositol 4-kinase (PI4K). PI3K is involved in cell signal transduction by phosphorylating phosphoinositide on the 3-hydroxyl group of the inositol ring, and PI4K acts on phosphatidylinositol (PI) to produce the second messenger, inositol-1,4,5-trisphosphate, and their abnormalities are associated with cancer. An inhibitor targeting PIK may include duvelisib, buparlisib, copanlisib, dactolisib, idelalisib, parsaclisib, paxalisib, taselisib, zandelisib, inavolisib, and the like.

As used herein, the term "CDK9 (cyclin dependent kinase 9)" is a cyclin dependent kinase associated with P-TEFb and is a cell cycle regulator. CDK9 is involved in several protein-protein interaction networks that are often involved in transcriptional deregulation in cancer. An inhibitor targeting CDK9 may include, for example, AZD4573, atuveciclib, VIP152, A-1592668, JSH-150, SLS009, AT-7519, roscovitine, and the like.

As used herein, the term "DHFR (dihydrofolate reductase)" is an enzyme that reduces dihydrofolate to tetrahydrofolate using NADPH as an electron donor, and is a component of the multiprotein complex TAK/P-TEFb, an elongation factor for transcription and function by RNA polymerase II by phosphorylating the C-terminal domain of the largest subunit of RNA polymerase II. DHFR is responsible for intracellular levels of tetrahydrofolate, and inhibiting DHFR can limit cell growth and proliferation, a hallmark of cancer and bacterial infections. An inhibitor targeting DHFR may include methotrexate, pralatrexate, pemetrexed, raltitrexed, trimetrexate, nolatrexed, piritrexim, talotrexin, and the like.

As used herein, the term "STING (stimulator of interferon genes)" is an in vivo sensor that recognizes DNA fragments from cancer cells, and activates immune cells in the body, such as dendritic cells, by stimulating interferon genes. A STING agonist exhibits an immune enhancing effect and cancer angiogenesis inhibitory effect. For example, a STING agonist may be CDNs, SB11285, DMXAA, and the like.

The compound represented by Formula I of the present invention can be used in combination with other targeted inhibitors described above to enhance the action of the targeted inhibitors, thereby significantly inhibiting the expression and activity of the target protein or gene. Specifically, the compound represented by Formula I not only has its own anticancer efficacy, but can also improve the anticancer efficacy of the targeted inhibitor. Therefore, when the compound represented by Formula I are used together with the targeted inhibitor, they can exhibit anticancer efficacy that is superior to the sum of the anticancer efficacy when they are used alone.

As used herein, the term "anticancer virus therapeutic agent" is a therapeutic agent that kills cancer by inserting a specific gene targeting cancer cells into a virus capable of proliferation and having infectivity. The anticancer virus therapeutic agent may be Talimogene Laherparepvec.

As used herein, the term "antibody therapeutic agent" is a therapeutic agent that exhibits an anticancer effect using an antibody that recognizes a specific protein of cancer cells as an antigen. An antibody therapeutic agent may be

cetuximab, trastuzumab, rituximab, ibritumomab, tositumomab, brentuximab, ofatumumab, obinutuzumab, necitumumab, bevacizumab, ramucirumab, nivolumab, pembrolizumab, atezolizumab, durvalumab, ipilimumab, and the like.

As used herein, the term "immune cell therapeutic agent" is a therapeutic agent that exhibits an anticancer effect by activating an immune response in the body using immune cells such as dendritic cells, natural killer cells, T cells, and the like. An immune cell therapeutic agent is used by extracting and enhancing immune cells in the body or genetically modifying them and then injecting them back into the body. Representative immune cell therapeutic agents may include T cell receptor-modified T cells (TCR-T), chimeric antigen receptor-modified T cells (CAR-T), and the like. Specifically, it may be tisagenlecleucel or axicabtagene ciloleucel, but is not limited thereto.

As used herein, the term "immune checkpoint inhibitor" is a substance that inhibits the activity of an immune checkpoint protein, which inhibits the differentiation, proliferation, and activity of immune cells, and is known to eliminate cancer cells by preventing them from exerting functions to evade the immune system. The immune checkpoint inhibitor may be any one selected from the group consisting of an anti-CTLA-4 antibody, an anti-PD-1 antibody, an anti-PD-L1 antibody, an anti-PD-L2 antibody, an anti-B7-H4 antibody, an anti-HVEM antibody, an anti-TIM3 antibody, an anti-GAL9 antibody, an anti-LAG3 antibody, an anti-VISTA antibody, an anti-KIR antibody, an anti-BTLA antibody and an anti-TIGIT antibody. In one embodiment, the immune checkpoint inhibitor may be ipilimumab, pembrolizumab, nivolumab, cemiplimab, atezolizumab, avelumab and durvalumab and the like, but is not limited thereto.

As used herein, the term "ADC (antibody drug conjugate)" is a therapeutic agent that exhibits high anticancer effects through targeted delivery by chemical binding of an antibody and a cytotoxic drug. It may be gemtuzumab-ozogamicin, brentuximab-vedotin, trastuzumab-emtansine, inotuzumab-ozogamicin, and eribulin-mesylate, and the like.

The anticancer agent may include one or more anticancer agents. Specifically, the compound, solvate, stereoisomer or pharmaceutically acceptable salt thereof may be used together with two anticancer agents. For example, two anticancer agents may be a chemical anticancer agent and a targeted anticancer agent; a chemical anticancer agent and an anticancer virus; a targeted anticancer agent and an antibody therapeutic agent; a chemical anticancer agent and a cell therapeutic agent; and a chemical anticancer agent and an immune checkpoint inhibitor. In addition, two anticancer agents may be a targeted anticancer agent and an anticancer virus; a targeted anticancer agent and an antibody therapeutic agent; a targeted anticancer agent and a cell therapeutic agent; a targeted anticancer agent and an immune checkpoint inhibitor. In addition, two anticancer agents may be an anticancer virus and an antibody therapeutic agent; an anticancer virus and a cell therapeutic agent; and an anticancer virus and an immune checkpoint inhibitor. In addition, two anticancer agents may be an antibody therapeutic agent and a cell therapeutic agent; and an antibody therapeutic agent and an immune checkpoint inhibitor.

The compound, solvate, stereoisomer or pharmaceutically acceptable salt thereof may be used together with three anticancer agents. In addition to the two anticancer agents, a different anticancer agent may be further included and used.

The compound, solvate, stereoisomer or pharmaceutically acceptable salt thereof may be used together with four anticancer agents. In addition to the three anticancer agents, a different anticancer agent may be further included and used.

The compound, solvate, stereoisomer or pharmaceutically acceptable salt thereof may be used together with five anticancer agents. In addition to the four anticancer agents, a different anticancer agent may be further included and used.

The compound, solvate, stereoisomer or pharmaceutically acceptable salt thereof may be used together with six anticancer agents.

The compound, solvate, stereoisomer or pharmaceutically acceptable salt thereof may be used in combination with an anticancer vaccine.

As used herein, the term "anticancer vaccine" is an active immunotherapy that removes cancer cells by enhancing the immune function in vivo by administering a tumor-specific antigen (TSA) possessed by cancer cells to cancer patient to activate the immune system. An anticancer vaccine may include a DNA vaccine, a peptide vaccine, a cell vaccine, and the like, depending on the type of antigen and the delivery method of antigen, and a cell vaccine and a DNA vaccine developed by introducing antigens are currently being developed representatively.

The compound, solvate, stereoisomer or pharmaceutically acceptable salt thereof may be used in combination with the anticancer agent and the anticancer vaccine. Here, the compound and the anticancer agent are the same as described above.

## Medicinal use, pharmaceutical composition, and administration method

The compound of Formula 1 or Formula I, or a stereoisomer, solvate or pharmaceutically acceptable salt thereof may be used for preventing or treating a SOS1 mediated disease. The compound of Formula I, the stereoisomer, the solvate and the pharmaceutically acceptable salt are as described above.

In the present specification, the term "preventing" or "prevention" refers to preventing a disease, for example, preventing a disease, condition or disorder in a subject who may be predisposed to the disease, condition or disorder but

has not yet experienced or exhibited the pathology or signs of the disease.

As used herein, the term "treating" or "treatment" refers to inhibiting a disease, for example, inhibiting a disease, condition or disorder in a subject who experiences or exhibits the pathology or signs of the disease, condition or disorder, i.e., preventing further development of the pathology and/or signs, or ameliorating the disease, for example, ameliorating the disease, condition or disorder in a subject who experiences or exhibits the pathology or signs of the disease, condition or disorder, i.e., reversing the pathology and/or signs, for example, reducing the severity of the disease.

The SOS1 mediated disease may include a disease that can be prevented or treated by inhibiting the interaction between SOS1 and RAS family proteins, or between SOS1 and RAC1. The SOS1 mediated disease may include a disease associated with the abnormal activity of SOS1 and/or RAS family proteins. The SOS1 mediated disease may be, for example, cancer. The cancer may be, for example, pancreatic cancer, lung cancer, colorectal cancer, biliary tract cancer, multiple myeloma, melanoma, uterine cancer, cervical cancer, endometrial cancer, thyroid cancer, chronic lymphocytic leukemia, acute myeloid leukemia, bladder cancer, urothelial cancer, gastric cancer, squamous cell carcinoma of the head and neck, diffuse large B cell lymphoma, esophageal cancer, hepatocellular cancer, breast cancer, ovarian cancer, prostate cancer, glioblastoma, kidney cancer or sarcoma. In one embodiment, the cancer may be pancreatic cancer, lung cancer (for example, non-small cell lung cancer), biliary tract cancer or colorectal cancer.

The cancer may be, for example, cancer dependent on the RAS family and the MAPK signaling pathway. The cancer may include, for example, cancer having mutation of proteins or genes, gene amplification and/or overexpression in the RAS family and MAPK signaling pathway, e.g., KRAS, NRAS, HRAS, receptor tyrosine kinases (for example, EGFR, ErbB2, ErbB3, ErbB4, PDGFR-A/B, FGFR1/2/3, IGF1R, INSR, ALK, ROS, TrkA, TrkB, TrkC, RET, c-MET, VEGFR1/2/3, AXL), GAP (for example, NF1) and SOS1 (for example, mutation, amplification or overexpression of RAF, MEK). In addition, the cancer may be a RAC1 dependent cancer.

The SOS1 mediated disease may be, for example, a disease associated with dysregulation of RAS family protein pathways, i.e., RASopathy. The RASopathy may include neurofibromatosis type 1 (NF1), Noonan syndrome, Noonan syndrome with multiple lentigines (NSML, also referred to as Leopard syndrome), capillary malformation-arteriovenous malformation syndrome (CM-AVM), Costello syndrome, CFC syndrome (Cardio-Facio-Cutaneous syndrome), Legius syndrome (also referred to as NF1-like syndrome) or hereditary gingival fibromatosis.

According to one embodiment, a compound of Formula I can be used in the treatment of a disease associated with the abnormal activity of SOS1 or RAS family proteins, or dysregulation of RAS family protein pathways by inhibiting the interaction between SOS1 and RAS family proteins, or between SOS1 and RAC1.

When used for the treatment of cancer, the compound of the present invention may be administered alone or in combination with other anticancer therapies, such as radiation therapy, taxane derivatives (for example, paclitaxel, docetaxel), platinum compounds (for example, cisplatin, carboplatin), antimetabolites (for example, 5-FU, gemcitabine, cytarabine, 6-thioguanine), CDK4/6 inhibitors (for example, abemaciclib, palbociclib), immunotherapeutic agents (for example, anti-CTLA4 antibody, anti-PD1 antibody), angiogenesis inhibitors (for example, bevacizumab, nintedanib, regorafenib), topoisomerase inhibitors (for example, irinotecan, SN-38, doxorubicin), ERK inhibitors (for example, ulixertinib, rineterkib), MDM2 inhibitors (for example, alrizomadlin), PARP inhibitors (for example, niraparib), MCL-1 inhibitors, mTOR inhibitors (for example, rapamycin, temsirolimus, INK-128 (sapanisertib), everolimus), BET inhibitors (for example,JQ1), CDK9 inhibitors (for example, AT-7519), IGF1/2 or IGF1-R inhibitors (for example, NVP-ADW742), PIK inhibitors (for example, duvelisib), EGFR inhibitors (for example, apatinib, osimertinib, cetuximab, lazertinib, gefitinib, neratinib), ErbB2 (HER2) inhibitors (for example, trastuzumab, irbinitinib, neratinib), ALK inhibitors (for example, crizotinib, alectinib), MEK inhibitors (for example, trametinib, cobimetinib), BCR-ABL inhibitors (for example, imatinib, nilotinib, dasatinib), FGFR1, FGFR2 or FGFR3 inhibitors (for example, nintedanib), ROS1 inhibitors (for example, crizotinib, entrectinib, repotrectinib), c-MET inhibitors (for example, tepotinib), AXL inhibitors (for example, bemcentinib), NTRK1 inhibitors (for example, repotrectinib), RET inhibitors (for example, pralsetinib), KRAS G12C inhibitors (for example, sotorasib, adagrasib, trametinib, JDQ443), KRAS G12D inhibitors (for example, MRTX1133), SHP2 inhibitors (for example, TNO155), mutBRAF inhibitors (for example, dabrafenib), PI3K inhibitors (for example, alpelisib, apitolisib), Aurora A inhibitors (for example, MK-5108), pan Aurora inhibitors (for example, danusertib, AMG-900, reversine), BTK inhibitors (for example, ibrutinib), Wee1 inhibitors (for example, MK-1775), DHFR inhibitors (for example, methotrexate), HSP90 inhibitors (for example, BIIB021), A3AR antagonists (for example, reversine), ubiquitin E1 enzyme inhibitors (for example, MLN-7243), Bcl-2 inhibitors (for example, ABT-737), EZH2 inhibitors (for example, GSK-343), ARID1A inhibitors (for example, GSK-343), SUMOylation inhibitors (for example, 2-D08), Chk1 inhibitors (for example, SCH-900776), HDAC inhibitors (for example, entinostat), JAK1/2 inhibitors (for example, ruxolitinib), Proteasome inhibitors (for example, carfilzomib), Akt inhibitors (for example, ipatasertib), GR inhibitors (for example, prednisolone), PLK1 inhibitors (for example, volasertib) or pan-RAF inhibitors (for example, sorafenib), etc.

The compound represented by Formula I of the present invention can be used together with other anticancer therapies described above to enhance the action of anticancer therapies, thereby significantly inhibiting the expression and activity of target proteins or genes. Specifically, the compound represented by Formula I not only has its own anticancer efficacy, but can also improve the anticancer efficacy of the targeted inhibitor. Therefore, when Formula I are

used together with the above anticancer therapy, they can exhibit anticancer efficacy that is superior to the sum of the anticancer efficacy when they are used alone.

In one embodiment, the pharmaceutical composition may comprise conventional pharmaceutically acceptable carriers, excipients or additives. The pharmaceutical composition may be formulated according to a conventional method, and may be prepared as various oral dosage forms such as tablets, pills, powders, capsules, syrups, emulsions, microemulsions, or parenteral dosage forms such as intramuscular, intravenous or subcutaneous dosage form. The pharmaceutical composition may be a single composition or separate compositions. The pharmaceutical composition comprises the compound, stereoisomer, solvate, or pharmaceutically acceptable salt according to one aspect as an active ingredient of the pharmaceutical composition.

When the pharmaceutical composition is prepared in the form of an oral formulation, examples of additives or carriers used may include cellulose, calcium silicate, corn starch, lactose, sucrose, dextrose, calcium phosphate, stearic acid, magnesium stearate, calcium stearate, gelatin, talc, surfactant, suspending agent, emulsifying agent, diluent, and the like. When the pharmaceutical composition of the present invention is prepared in the form of an injection, the additive or carrier may include water, saline, aqueous glucose solution, similar aqueous sugar solution, alcohol, glycol, ether (for example, polyethylene glycol 400), oil, fatty acid, fatty acid ester, glyceride, surfactant, suspending agent, emulsifying agent, and the like.

The dosage of the pharmaceutical composition is an amount effective for treatment or prevention of a subject or patient, and may be administered orally or parenterally as desired. It may be administered in one to several divided doses to be administered in an amount of 0.01 to 1000 mg, more specifically 0.1 to 300 mg per kg of body weight daily based on the active ingredient when administered orally, or in an amount of 0.01 to 100 mg, more specifically 0.1 to 50 mg per kg of body weight daily based on the active ingredient when administered parenterally. The dose to be administered to a specific subject or patient should be determined in light of several related factors such as body weight, age, sex, health condition of the patient, diet, administration time, administration method, the severity of the disease, and the like, and it should be understood that it may be appropriately increased or decreased by a specialist. The above dosage is not intended to limit the scope of the present invention in any way. A physician or veterinarian of ordinary skill in the art may readily determine and prescribe the required effective amount of the pharmaceutical composition. For example, by a physician or veterinarian, a dose of the compound of the present invention used in a pharmaceutical composition may start at a level lower than that required to achieve the desired therapeutic effect, and may gradually increase until the desired effect is achieved.

In one embodiment, the pharmaceutical composition includes within its scope a pharmaceutical composition comprising, as an active ingredient, a therapeutically effective amount of at least one of the compounds according to one embodiment, alone or in combination with a pharmaceutical carrier. The term "therapeutically effective amount" or "effective amount" refers to an amount sufficient to produce a beneficial or desired clinical result, for example, an amount sufficient to alleviate, ameliorate, stabilize, reverse, slow or delay the progression of a disease.

Optionally, the compound according to one embodiment may be administered alone, in combination with the compound according to another embodiment, or simultaneously, separately, or sequentially in combination with one or more other therapeutic agents, for example, an anticancer agent or other pharmaceutically active substances. Examples of the anticancer agent that can be administered in combination are as described above.

In another aspect, there is provided a method for preventing or treating a SOS1 mediated disease, comprising administering to a subject a compound of Formula I, a solvate, stereoisomer or pharmaceutically acceptable salt thereof, or the pharmaceutical composition comprising the same.

Among the terms or elements mentioned in the description of the method, the same as those already mentioned are the same as described above.

The administration may be oral or parenteral administration. It may be administered in one to several divided doses to be administered in an amount of 0.01 to 1000 mg, more specifically 0.1 to 300 mg per kg of body weight daily based on the active ingredient when administered orally, or in an amount of 0.01 to 100 mg, more specifically 0.1 to 50 mg per kg of body weight daily based on the active ingredient when administered parenterally. The dose to be administered to a specific subject or patient should be determined in light of several related factors such as body weight, age, sex, health condition of the patient, diet, administration time, administration method, the severity of the disease, and the like, and it may be appropriately increased or decreased by a specialist.

As used herein, the term "subject" refers to a subject in need of treatment for a disease, and more specifically means a mammal such as a human or non-human primate, a mouse, a dog, a cat, a horse, and a cow.

In another aspect, there is provided a medicinal use of the compound of Formula I, solvate, stereoisomer or pharmaceutically acceptable salt thereof for the prevention or treatment of a SOS1 mediated disease; or a use of the compound of Formula I, solvate, stereoisomer or pharmaceutically acceptable salt thereof for the manufacture of a medicament for the prevention or treatment of a SOS1 mediated disease.

Among the terms or elements mentioned in the description of the method or use, the same as those already mentioned are the same as described above.

## Effects of Invention

The compound of Formula I, a solvate, stereoisomer or pharmaceutically acceptable salt thereof has an effective inhibitory activity against SOS1, in particular, inhibits the interaction between SOS1 and RAS family proteins, or between SOS1 and RAC1. In addition, when combined with other anticancer agents, it exhibits synergistic effects for inhibiting growth of cancer cells in lung cancer, pancreatic cancer, stomach cancer, and colorectal cancer. Accordingly, a pharmaceutical composition for treating cancer comprising the novel compound and an anticancer agent as active ingredients may be usefully used for preventing or treating cancer.

## Brief Description of Drawings

FIG. 1 is a graph showing the results obtained by evaluating cell viability according to the administration of INK-128 alone or INK-128 in combination with the compound of Example 295 to the lung cancer cell NCI-H358.

FIG. 2 is a graph showing the results obtained by evaluating cell viability according to the administration of Sotorasib alone or Sotorasib in combination with the compound of Example 295 to the lung cancer cell NCI-H358.

FIG. 3 is a graph showing the results obtained by evaluating cell viability according to the administration of the example compounds and sotorasib alone or in combination to the lung cancer cell NCI-H358.

FIG. 4 is a graph showing the results obtained by evaluating cell viability according to the administration of the example compounds and sotorasib alone or in combination to the pancreatic cancer cell MIA PaCa-2.

FIG. 5 is a graph showing the results obtained by evaluating cell viability according to the administration of the example compounds and adagrasib alone or in combination to the lung cancer cell NCI-H358.

FIG. 6 is a graph showing the results obtained by evaluating cell viability according to the administration of the example compounds and adagrasib alone or in combination to the pancreatic cancer cell MIA PaCa-2.

FIGs. 7 and 8 are graphs showing the results obtained by evaluating cell viability according to the administration of the example compounds and trametinib alone or in combination to the lung cancer cell NCI-H358.

FIGs. 9 and 10 are graphs showing the results obtained by evaluating cell viability according to the administration of the example compounds and trametinib alone or in combination to the gastric cancer cell SNU-1.

FIGs 11 and 12 are graphs showing the results obtained by evaluating cell viability according to the administration of the example compounds and trametinib alone or in combination to the colorectal cancer cell SW480.

FIG. 13 is a graph showing the results obtained by evaluating cell viability according to the administration of the example compounds and osimertinib alone or in combination to the lung cancer cell H1975.

FIG. 14 is a graph showing the results obtained by evaluating cell viability according to the administration of the example compounds and osimertinib alone or in combination to the lung cancer cell HCC827.

FIG. 15 is a graph showing the results obtained by evaluating cell viability according to the administration of the example compounds and lazertinib alone or in combination to the lung cancer cell H1975.

FIG. 16 is a graph showing the results obtained by evaluating cell viability according to the administration of the example compounds and lazertinib alone or in combination to the lung cancer cell HCC827.

FIG. 17 is a graph showing the results obtained by evaluating cell viability according to the administration of the example compounds and alpelisib alone or in combination to the PIK3CA mutant breast cancer cell MCF7.

FIG. 18 is a graph showing the results obtained by evaluating cell viability according to the administration of the example compounds and JDQ443 alone or in combination to the lung cancer cell H358 having the KRAS G12C variant.

FIG. 19 is a graph showing the results obtained by evaluating cell viability according to the administration of the example compounds and TNO155 alone or in combination to the lung cancer cell H358 having the KRAS G12C variant.

FIG. 20 is a graph showing the results obtained by evaluating cell viability according to the administration of the example compounds and cisplatin alone or in combination to the lung cancer cell H358 having the KRAS G12C variant.

FIG. 21 is a graph showing the results obtained by evaluating cell viability according to the administration of the example compounds and rineterkib alone or in combination to the lung cancer cell H358 having the KRAS G12C variant.

FIG. 22 is a graph showing the results obtained by evaluating cell viability according to the administration of the example compounds and ulixertinib alone or in combination to the lung cancer cell H358 having the KRAS G12C variant.

FIG. 23 is a graph showing the results obtained by evaluating cell viability according to the administration of the example compounds and pralsetinib alone or in combination to the lung cancer cell H358 having the KRAS G12C

variant.

FIG. 24 is a graph showing the results obtained by evaluating cell viability according to the administration of the example compounds and repotrectinib alone or in combination to the lung cancer cell H358 having the KRAS G12C variant.

FIG. 25 is a graph showing the results obtained by evaluating cell viability according to the administration of the example compounds and tepotinib alone or in combination to the c-Met overexpressing gastric cancer cell SNU-5.

FIG. 26 is a graph showing the results obtained by evaluating cell viability according to the administration of the example compounds and bemcentinib alone or in combination to the lung cancer cell PC-9 with high AXL expression.

FIG. 27 is a graph showing the results obtained by evaluating cell viability according to the administration of the example compounds and alrizomadlin alone or in combination to the lung cancer cell A549 having the KRAS G12S variant.

FIG. 28 is a graph showing the results obtained by evaluating cell viability according to the administration of the example compounds and everolimus alone or in combination to the colorectal cancer cell LoVo having the KRAS G13D variant.

FIG. 29 is a graph showing the results obtained by evaluating cell viability according to the administration of the example compounds and MRTX1133 alone or in combination to the pancreatic cancer cell AsPC-1 having the KRAS G12D variant.

FIGs. 30a to 30c are graph showing the results obtained by evaluating the synergy of administration of the example compounds in combination to the lung cancer cell H358 using SynergyScreen.

FIGs. 31a to 31c are graph showing the results obtained by evaluating the synergy of administration of the example compounds in combination to the lung cancer cell H358 using SynergyScreen.

**Detailed Descriptions for Carrying out the Invention**

Hereinafter, the present invention will be described in more detail by way of the following examples. However, the following examples are only for illustrating the present invention, and the scope of the present invention is not limited thereto.

**[Preparation Examples]**

**Preparation Example 1: 6-oxo-1-phenyl-pyridazine-3-carboxylic acid**

<u>Step 1: Synthesis of methyl-6-oxo-1-phenyl-pyridazine-3-carboxylate</u>

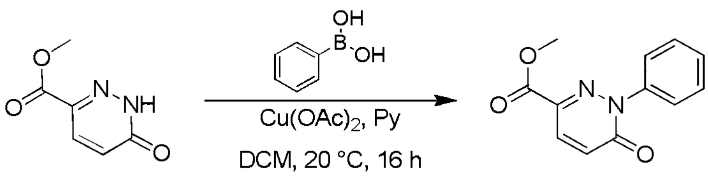


A mixture of phenylboronic acid (380 mg, 3.1 mmol), methyl-6-oxo-1H-pyridazine-3-carboxylate (504 mg), $Cu(OAc)_2$ (113 mg, 623 μmol), pyridine (1.6 g, 19.8 mmol) in DCM (10 mL) was degassed and purged with $N_2$ for 3 times, and then the mixture was stirred at 20 °C for 16 h under $N_2$ atmosphere. The reaction mixture was poured into distilled water (20 mL) and extracted with EtOAc. The combined organic layer was washed with brine, dried over $Na_2SO_4$ and concentrated under reduced pressure to obtain a crude product, which was purified by silica gel column chromatography (21% EtOAc in petroleum ether) to give methyl-6-oxo-1-phenyl-pyridazine-3-carboxylate (450 mg, 62.7% yield) as a yellow solid. $^1$H NMR (400 MHz, DMSO-$d_6$) δ 7.93 (d, *J* = 10.0 Hz, 1H), 7.57-7.53 (m, 4H), 7.52-7.47 (m, 1H), 7.16 (d, *J* = 10.0 Hz, 1H), 3.86 (s, 3H); LC/MS (ESI) m/z = 231.0 $[M+H]^+$.

<u>Step 2: Synthesis of 6-oxo-1-phenyl-pyridazine-3-carboxylic acid</u>

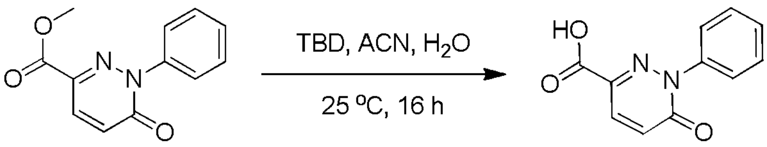


**Intermediate A**

To a solution of methyl-6-oxo-1-phenyl-pyridazine-3-carboxylate (450 mg, 2.0 mmol) in ACN (5 mL) and $H_2O$ (1 mL) was added 3, 4, 6, 7, 8, 9-hexahydro-2H-pyrimido[1, 2-a] pyrimidine (544 mg, 3.9 mmol), followed by stirring at 25 °C for 16 h. The reaction mixture was concentrated under reduced pressure to obtain a residue, which was added with water (20 mL), acidified (pH = 2.0) with 1 N aqueous HCl solution and extracted with EtOAc. The combined organic layer was washed with brine, dried over $Na_2SO_4$ and concentrated under reduced pressure to give Intermediate A (410 mg, crude, 95% yield) as a yellow solid. $^1H$ NMR (400 MHz, DMSO-$d_6$) δ 13.67 (bs, 1H), 7.91 (d, $J$ = 10.0 Hz,1H), 7.52 (m, 5H), 7.13(d, $J$ = 10.0 Hz, 1H); LC/MS (ESI) m/z = 217.0 $[M+H]^+$.

**Preparation Example 2: (1R)-1-[3-nitro-5-(trifluoromethyl)phenyl]ethanamine**

Step 1: Synthesis of 1-(3-nitro-5-(trifluoromethyl)phenyl]ethanone

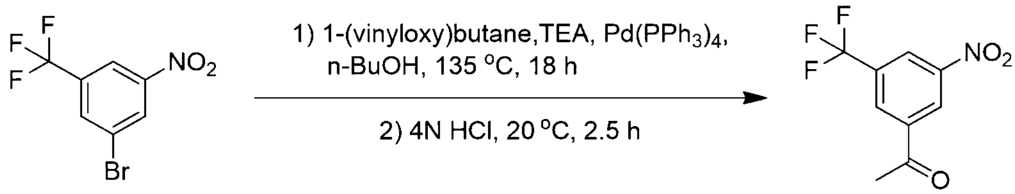


1-vinyloxybutane (74.2 g, 741 mmol) and TEA (11.2 g, 111 mmol) were added dropwise to a mixture of 1-bromo-3-nitro-5-(trifluoromethyl)benzene (20.0 g, 74.1 mmol) and $Pd(PPh_3)_4$ (4.3 g, 3.7 mmol) in n-BuOH (200 mL), and the mixture was degassed, purged with $N_2$ for 3 times, and then stirred at 135 °C for 18 h under $N_2$ atmosphere. The mixture was added with 4N HCl (120 mL) and THF (100 mL) and stirred at 20 °C for 2.5 h. The reaction mixture was poured into water (600 mL) and extracted with EtOAc. The combined organic layer was washed with brine, dried over $Na_2SO_4$ and concentrated under reduced pressure to obtain a residue, which was purified by column chromatography on silica gel (3% EtOAc in PE) to give 1-[3-nitro-5-(trifluoromethyl)phenyl]ethanone (20.45 g, 47.37% yield) as yellow oil. $^1H$ NMR (400 MHz, CHLOROFORM-d) δ 8.94 (d, $J$ = 1.6 Hz, 1H), 8.69 (s, 1H), 8.53 (s, 1H), 2.75 (s, 3H).

Step 2: Synthesis of (R)-2-methyl-N-[1-[3-nitro-5-(trifluoromethyl)phenyl]ethylidene]propane-2-sulfinamide

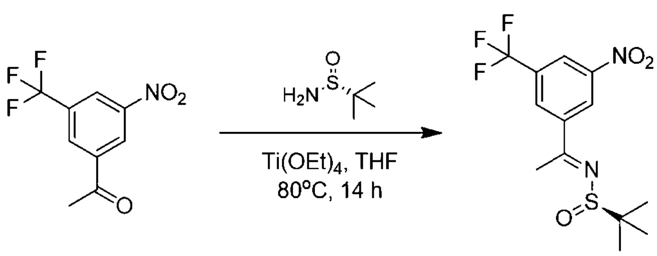


To a solution of 1-[3-nitro-5-(trifluoromethyl)phenyl]ethanone (20.5 g, 87.7 mmol) in THF (200 mL), $Ti(OEt)_4$ (50.0 g, 219 mmol) and (R)-2-methylpropane-2-sulfinamide (13.8 g, 114 mmol) were added, followed by stirring at 80 °C for 14 h under $N_2$. The mixture was quenched with ice water (300 mL) at 20 °C, and the precipitate was dissolved in EtOAc (500 mL) and filtered out. The organic layer was concentrated in vacuo to obtain a residue, which was purified by silica gel column chromatography (15-20% EtOAc in PE) to give (R)-2-methyl-N-[1-[3-nitro-5-(trifluoromethyl)phenyl]ethylidene]propane-2-sulfinamide (20.4 g, 69.0% yield) as yellow oil. $^1H$ NMR (400 MHz, DMSO-$d_6$) δ 8.84 (s, 1H), 8.64 (s, 1H), 8.54 (s, 1H), 2.85 (s, 3H), 1.25 (s, 9H); LC/MS (ESI) m/z = 337.0 $[M+H]^+$.

Step 3 : Synthesis of (R)-2-methyl-N-[(1R)-1-[3-nitro-5-(trifluoromethyl)phenyl]ethyl]propane-2-sulfinamide

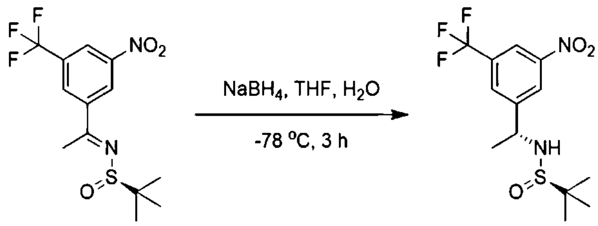


To a solution of (R)-2-methyl-N-[1-[3-nitro-5-(trifluoromethyl)phenyl]ethylidene]propane-2-sulfinamide (20.4 g, 60.5 mmol) in THF (200 mL) and $H_2O$ (4 mL) was added $NaBH_4$ (1.6 g, 42.4 mmol), followed by stirring at -78 °C for 3 h under $N_2$. The reaction mixture was quenched with sat. aq. $NH_4Cl$ (150 mL) at 20 °C, diluted with EtOAc (100 mL) and extracted with EtOAc. The combined organic layer was washed with brine, dried over $Na_2SO_4$, filtered and concentrated under reduced pressure to obtain a residue. Two diastereomers in the ratio of 95:5 were purified by silica gel column chromatography (20% EtOAc in petroleum ether) to give the main product, (R)-2-methyl-N-[(1R)-1-[3-nitro-5-(trifluoromethyl)phenyl]ethyl]propane-2-sulfinamide (14.3 g, 69.9% yield, >99% ee) as a light-yellow solid. $^1H$ NMR (400 MHz, DMSO-$d_6$) δ 8.63 (s, 1H), 8.37 (s, 1H), 8.29 (s, 1H), 6.07 (d, *J* = 8.8 Hz, 1H), 4.73-4.63 (m, 1H), 1.45 (d, *J* = 7.2 Hz, 3H), 1.13 (s, 9H); LC/MS (ESI) m/z = 339.0 $[M+H]^+$.

Step 4: Synthesis of (1R)-1-[3-nitro-5-(trifluoromethyl)phenyl]ethanamine

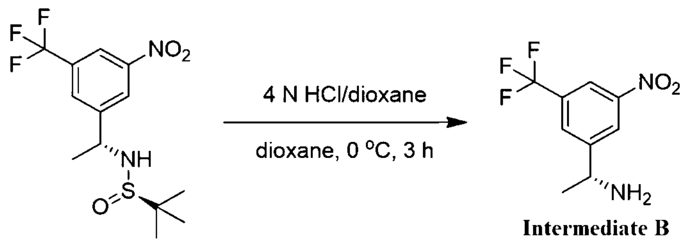


To a solution of (R)-2-methyl-N-[(1R)-1-[3-nitro-5-(trifluoromethyl)phenyl]ethyl]propane-2-sulfinamide (14.3 g, 42.3 mmol) in dioxane (50 mL) was added 4N HCl/dioxane (50 mL) at 0 °C, followed by stirring at 0 °C for 3 h. The mixture was concentrated under reduced pressure to obtain a residue, the residue was triturated with MTBE (200 mL) for 20 min at 20 °C, and the mixture was filtered to give Intermediate B (8.4 g, 73.4% yield, HCl salt) as an off-white solid. $^1H$ NMR (400 MHz, DMSO-$d_6$) δ 8.90 (br s, 3H), 8.79 (s, 1H), 8.50 (s, 2H), 4.75 (q, *J* = 6.8 Hz, 1H), 1.59 (d, *J* = 6.8 Hz, 3H); LC/MS (ESI) m/z = 235.1 $[M+H]^+$.

**Preparation Example 3: 3-[(1R)-1-aminoethyl]-5-(trifluoromethyl)aniline**

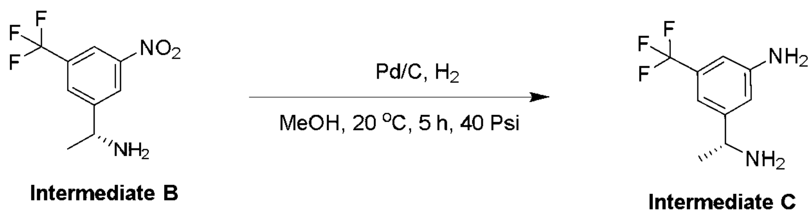


To a solution of Intermediate B (3.00g, 11.09 mmol, HCl salt) in MeOH (30 mL) was added Pd/C (600 mg, 10% purity), followed by stirring at 20 °C for 5 h under $H_2$ (40 Psi). The reaction mixture was filtered, and the filtrate was concentrated under reduced pressure to give Intermediate C (2.5 g, 93.72% yield, HCl salt) as a light yellow solid. $^1H$ NMR (400 MHz, DMSO-$d_6$) δ 8.49 (brs, 3H), 6.97 (s, 1H), 6.84 (brd, *J* = 5.2 Hz, 2H), 5.75 (s, 2H), 4.44-4.24 (m, 1H), 1.47 (d, *J* =

6.8 Hz, 3H); LC/MS (ESI) m/z = 205.0 $[M+H]^+$.

**Preparation Example 4: 2-[3-[(1R)-1-aminoethyl]phenyl]-2,2-difluoroethanol**

Step 1: Synthesis of ethyl 2-(3-acetylphenyl)-2,2-difluoro-acetate

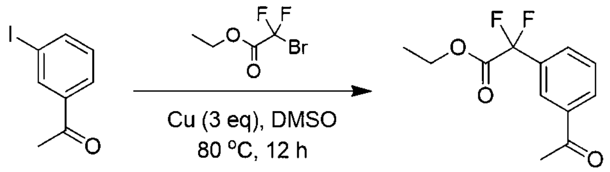


To a solution of 1-(3-iodophenyl)ethanone (5.0 g, 20.32 mmol) in DMSO (50 mL), Cu (3.87 g, 60.96 mmol) and ethyl 2-bromo-2,2-difluoro-acetate (12.37 g, 60.96 mmol) were added, followed by stirring at 80 °C for 12 h under $N_2$. The reaction mixture was poured into water (100 mL) and extracted with EtOAc. The combined organic layer was washed with brine, dried over $Na_2SO_4$ and concentrated under reduced pressure to obtain a crude product. The product was purified by silica gel column chromatography (8% EtOAc in petroleum ether) to give ethyl 2-(3-acetylphenyl)-2, 2-difluoro-acetate (2.93 g, 52.38% yield) as colorless oil. $^1H$ NMR (400 MHz, CHLOROFORM-*d*) δ 8.19 (s, 1H), 8.09 (d, *J* = 8.0 Hz, 1H), 7.81 (d, *J* = 8.0 Hz, 1H), 7.58 (t, *J* = 8.0 Hz, 1H), 4.31 (q, *J* = 7.2 Hz, 2H), 2.64 (s, 3H), 1.31 (t, *J* = 7.2 Hz, 3H); LC/MS (ESI) m/z = 243.0 $[M+H]^+$.

Step 2: Synthesis of ethyl 2-[3-[(Z)-N-[(R)-tert-butylsulfinyl]-C-methyl-carboimidoyl]phenyl]-2,2-difluoro-acetate

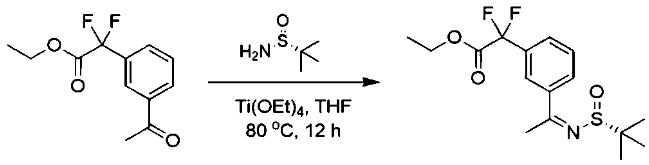


To a solution of ethyl 2-(3-acetylphenyl)-2,2-difluoro-acetate (2.93 g, 12.10 mmol) in THF (30 mL), $Ti(OEt)_4$ (6.90 g, 30.24 mmol) and (R)-2-methylpropane-2-sulfinamide (1.91 g, 15.73 mmol) were added, followed by stirring at 80 °C for 12 h. The mixture was quenched with ice water (80 mL) at 20 °C, and the precipitate was dissolved in EtOAc (200 mL) and filtered out. The organic layer was concentrated in vacuo to obtain a residue, which was purified by silica gel column chromatography (12% EtOAc in petroleum ether) to give ethyl 2-[3-[(Z)-N-[(R)-tert-butylsulfinyl]-C-methyl-carboimidoyl] phenyl]-2,2-difluoro-acetate (3.0 g, 63.90% yield) as yellow oil. $^1H$ NMR (400 MHz, CHLOROFORM-*d*) δ 8.10 (s, 1H), 8.02 (d, *J* = 8.0 Hz, 1H), 7.74 (d, *J* = 8.0 Hz, 1H), 7.54 (t, *J* = 8.0 Hz, 1H), 4.32 (q, *J* = 7.2 Hz, 2H), 2.80 (s, 3H), 1.34 (s, 12H); LC/MS (ESI) m/z = 346.0 $[M+H]^+$.

Step 3: Synthesis of (R)-N-[(1R)-1-[3-(1,1-difluoro-2-hydroxy-ethyl)phenyl]ethyl]-2-methyl-propane-2-sulfinamide

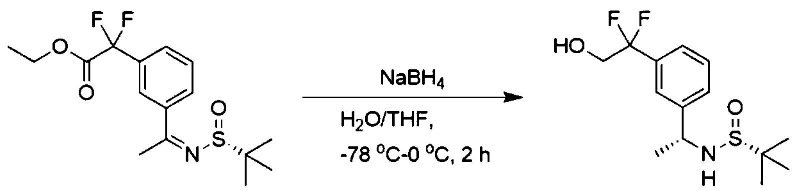


To a solution of ethyl 2-[3-[(Z)-N-[(R)-tert-butylsulfinyl]-C-methyl-carboimidoyl]phenyl]-2,2-difluoro-acetate (1 g, 2.90 mmol) in THF (10 mL) was added $NaBH_4$ (240.97 mg, 6.37 mmol) at -78 °C, followed by stirring at 0 °C for 2 h. The reaction mixture was quenched with sat. aq. $NH_4Cl$ (40 mL) at 20 °C, diluted with EtOAc (30 mL) and extracted with EtOAc. The combined organic layer was washed with brine, dried over $Na_2SO_4$, filtered and concentrated under reduced pressure

to obtain a residue. Diastereomers produced in the ratio of about 3:1 were subjected to a first purification with silica gel column chromatography (22% EtOAc in petroleum ether) to obtain a product. The product was subjected to prep-HPLC (Xtimate C18 150*40mm*10um; mobile phase: [water ($NH_3H_2O$)-ACN]; B%: 25%-55%, 10min) to separate a main product. Then, $CH_3CN$ was removed under reduced pressure, and the remaining solvent was removed by lyophilization to give (R)-N-[(1R)-1-[3-(1,1-difluoro-2-hydroxy-ethyl)phenyl]ethyl]-2-methyl-propane-2-sulfinamide (613 mg, 46.17% yield, 99.90% purity, 94.9% ee) as colorless oil. $^1H$ NMR (400 MHz, DMSO-$d_6$) δ 7.54 (s, 1H), 7.48-7.40 (m, 3H), 4.59-4.50 (m, 1H), 3.95 (t, *J* = 13.6 Hz, 2H), 3.52 (d, *J* = 5.2 Hz, 1H), 1.75 (s, 1H), 1.55 (d, *J* = 6.8 Hz, 3H), 1.23 (s, 9H); LC/MS (ESI) m/z = 306.3 $[M+H]^+$.

Step 4: Synthesis of 2-[3-[(1R)-1-aminoethyl]phenyl]-2,2-difluoro-ethanol

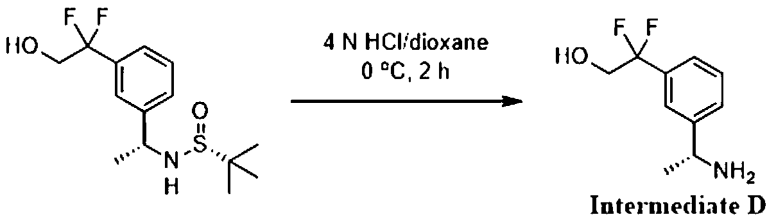


Intermediate D

To a solution of (R)-N-[(1R)-1-[3-(1,1-difluoro-2-hydroxy-ethyl)phenyl]ethyl]-2-methylpropane-2-sulfinamide (613 mg, 2.01 mmol) in dioxane (5 mL) was added 4N HCl/dioxane (5 mL), followed by stirring at 0 °C for 2 h. The mixture was concentrated under reduced pressure to give Intermediate D (400 mg, crude) as light yellow oil. LC/MS (ESI) m/z = 202.0 $[M+H]^+$.

**Preparation Example 5: 2-[3-[(1R)-1-aminoethyl]-2-fluoro-phenyl]-2,2-difluoro-ethanol**

Step 1: Synthesis of 1-(2-fluoro-3-iodo-phenyl)ethanol

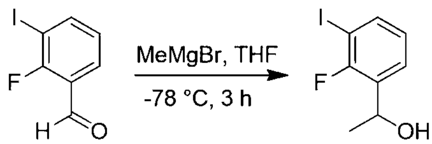


To a solution of 2-fluoro-3-iodo-benzaldehyde (4.0 g, 16.00 mmol) in THF (40 mL) was added MeMgBr (3 M, 8.00 mL) dropwise at -78 °C, followed by stirring for 3 h. The reaction mixture was poured into sat. aq. $NH_4Cl$ (50 mL) and extracted with EtOAc. The combined organic layer was washed with brine, dried over anhydrous $Na_2SO_4$ and concentrated under reduced pressure to obtain a crude product. The product was purified by silica gel column chromatography (7% EtOAc in petroleum ether) to give 1-(2-fluoro-3-iodo-phenyl)ethanol (4.45 g, 83.63% yield) as yellow oil. $^1H$ NMR (400 MHz, CHLOROFORM-*d*) δ 7.72-7.64 (m, 1H), 7.55-7.45 (m, 1H), 6.93 (t, *J* = 7.6 Hz, 1H), 5.20 (q, *J* = 6.4 Hz, 1H), 1.52 (d, *J* = 6.4 Hz, 3H).

Step 2: Synthesis of 1-(2-fluoro-3-iodo-phenyl)ethanone

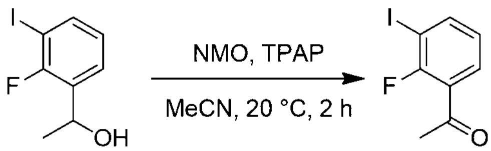


To a solution of 1-(2-fluoro-3-iodo-phenyl)ethanol (4.45 g, 16.73 mmol) in MeCN (50 mL), TPAP (587.80 mg, 1.67 mmol) and NMO (2.94 g, 25.09 mmol) were added, followed by stirring at 20 °C for 2 h. The mixture was filtrated and

concentrated under reduced pressure to obtain a crude product, which was purified by silica gel column chromatography (0% EtOAc in petroleum ether) to give 1-(2-fluoro-3-iodo-phenyl)ethanone (3.8 g, 12.95 mmol, 77.44% yield) as a white solid. $^1$H NMR (400 MHz, CHLOROFORM-d) δ 7.97-7.88 (m, 1H), 7.85-7.78 (dm, 1H), 7.00 (t, *J* = 7.6 Hz, 1H), 2.65 (d, *J* = 5.2 Hz, 3H).

Step 3: Synthesis of ethyl 2-(3-acetyl-2-fluoro-phenyl)-2,2-difluoro-acetate

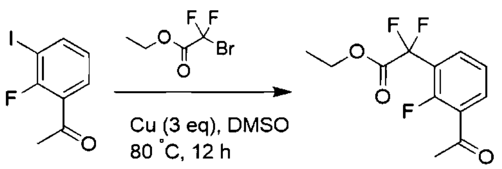


To a solution of 1-(2-fluoro-3-iodo-phenyl)ethanone (3.0 g, 11.36 mmol) and ethyl 2-bromo-2,2-difluoro-acetate (6.92 g, 34.09 mmol, 4.38 mL) in DMSO (30 mL) was added Cu (2.17 g, 34.09 mmol), followed by stirring at 80 °C for 12 h. The reaction mixture was poured into water (50 mL) and extracted with EtOAc. The combined organic layer was washed with brine, dried over $Na_2SO_4$ and concentrated under reduced pressure to obtain a crude product. The product was purified by silica gel column chromatography (5% EtOAc in petroleum ether) to give ethyl 2-(3-acetyl-2-fluoro-phenyl)-2,2-difluoro-acetate (1.8 g, 56.05% yield) as colorless oil. $^1$H NMR (400 MHz, CHLOROFORM-*d*) δ 8.08-8.00 (m, 1H), 7.87-7.81 (m, 1H), 7.36 (t, *J*= 7.6 Hz, 1H), 4.42-4.37 (m, 2H), 2.66 (d, *J* = 5.2 Hz, 3H), 1.35 (t, *J* = 7.2 Hz, 3H); LC/MS (ESI) m/z = 261.0 $[M+H]^+$.

Step 4: Synthesis of ethyl 2-[3-[[(R)-tert-butylsulfinyl]-C-methyl-carboimidoyl]-2-fluorophenyl]-2,2-difluoro-acetate

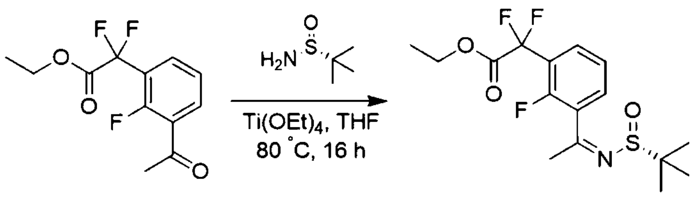


To a solution of ethyl 2-(3-acetyl-2-fluoro-phenyl)-2,2-difluoro-acetate (1.8 g, 6.92 mmol) and (R)-2-methylpropane-2-sulfinamide (1.26 g, 10.38 mmol) in THF (20 mL) was added $Ti(OEt)_4$ (4.73 g, 20.75 mmol), followed by stirring at 80 °C for 16 h. The reaction mixture was poured into water (30 mL) and EtOAc (30 mL) and filtrated, and the filtrate was extracted with EtOAc. The combined organic layer was washed with brine, dried over $Na_2SO_4$ and concentrated under reduced pressure to obtain a crude product. The crude product was purified by silica gel column chromatography (6% EtOAc in petroleum ether) to give ethyl 2-[3-[[(R)-tert-butylsulfinyl]-C-methyl-carboimidoyl]-2-fluoro-phenyl]-2,2-difluoro-acetate (1.9 g, 74.09% yield) as yellow oil. $^1$H NMR (400 MHz, CHLOROFORM-*d*) δ 7.86-7.72 (m, 2H), 7.32 (t, *J*= 7.6 Hz, 1H), 4.44-4.30 (m, 2H), 2.77 (s, 3H), 1.32 (s, 9H); LC/MS (ESI) m/z = 364.0 $[M+H]^+$.

Step 5: Synthesis of (R)-N-[(1R)-1-[3-(1,1-difluoro-2-hydroxyethyl)-2-fluorophenyl]ethyl]-2-methyl-propane-2-sulfinamide

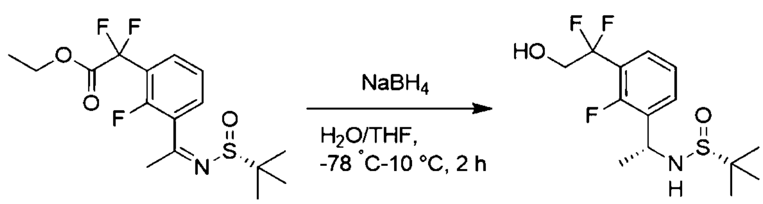


To a solution of ethyl 2-[3-[[(R)-tert-butylsulfinyl]-C-methyl-carboimidoyl]-2-fluorophenyl]-2,2-difluoro-acetate

(900 mg, 2.48 mmol) in THF (10 mL) and $H_2O$ (0.2 mL) was added $NaBH_4$ (210 mg, 5.55 mmol) at -78 °C, and the mixture was warmed to 10 °C slowly and then stirred at 10 °C for 2 h. The reaction mixture was poured into ice water (30 mL) and extracted with EtOAc. The combined organic layer was washed with brine, dried over $Na_2SO_4$ and concentrated under reduced pressure to obtain a crude product. The product was subjected to a first purification with silica gel column chromatography (50% EtOAc in petroleum ether), and then two diastereomers (in the ratio of about 3:1) were separated and purified by using prep-HPLC (Xtimate C18 150*40 mm*10 um;mobile phase: [water($NH_3H_2O$)-ACN];B%: 25%-55%,10 min). $CH_3CN$ was removed under reduced pressure, and the remaining solvent was removed by lyophilization to give a main product, (R)-N-[(1R)-1-[3-(1,1-difluoro-2-hydroxyethyl)-2-fluoro-phenyl]ethyl]-2-methyl-propane-2-sulfinamide (440 mg, 50.90% yield, 92.65% purity, >99% ee) as a white solid. $^1H$ NMR (400 MHz, DMSO-$d_6$) $\delta$ 7.70 (t, *J* = 6.8 Hz, 1H), 7.48-7.39 (m, 1H), 7.35-7.25 (m, 1H), 5.87 (d, *J* = 7.6 Hz, 1H), 5.70 (t, *J* = 6.4 Hz, 1H), 4.68 (quin, *J* = 7.2 Hz, 1H), 3.90 (dt, *J* = 6.4, 14.4 Hz, 2H), 1.40 (d, *J*= 6.8 Hz, 3H), 1.10 (s, 9H); LC/MS (ESI) m/z = 324.3 $[M+H]^+$.

Step 6: Synthesis of 2-[3-[(1R)-1-aminoethyl]-2-fluoro-phenyl]-2,2-difluoro-ethanol

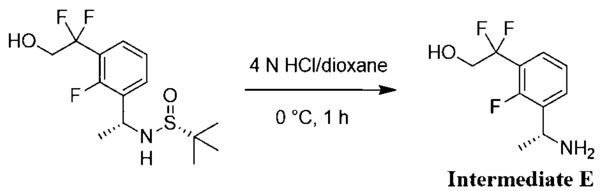


**Intermediate E**

To a solution of (R)-N-[(1R)-1-[3-(1,1-difluoro-2-hydroxy-ethyl)-2-fluoro-phenyl]ethyl]-2-methyl-propane-2-sulfinamide (440 mg, 1.36 mmol) in dioxane (4 mL) was added 4 N HCl/dioxane (2 mL) at 0 °C, followed by stirring at 0 °C for 1 hour. The mixture was concentrated under reduced pressure to give Intermediate E (347 mg, 100% yield, HCl salt) as yellow oil. LC/MS (ESI) m/z = 220.0 $[M+H]^+$.

**Preparation Example 6: Methyl-5-bromo-6-oxo-1-phenyl-pyridazine-3-carboxylate**

Step 1: Synthesis of methyl-5-bromo-6-oxo-1H-pyridazine-3-carboxylate

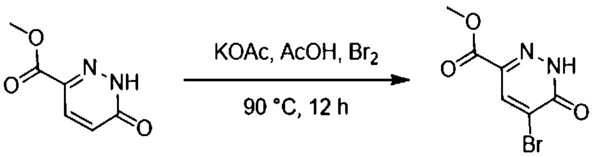


To a solution of methyl-6-oxo-1H-pyridazine-3-carboxylate (3.0 g, 19.46 mmol) in AcOH (60 mL), KOAc (6.69 g, 68.13 mmol) and $Br_2$ (6.84 g, 42.82 mmol, 2.21 mL) were added, followed by stirring at 90 °C for 12 h. The mixture was quenched by the addition of aqueous $NaHSO_3$ solution (500 mL, 3 mol/L) and extracted with EtOAc. The combined organic layer was washed with brine, dried over $Na_2SO_4$ and concentrated under reduced pressure to give methyl-5-bromo-6-oxo-1H-pyridazine-3-carboxylate (3.0 g, 54.24% yield) as a white solid. $^1H$ NMR (400MHz, DMSO-$d_6$) $\delta$ 13.94 (brs, 1H), 8.26 (s, 1H), 3.85 (s, 3H); LC/MS (ESI) m/z = 232.9 $[M+H]^+$.

Step 2: Synthesis of methyl-5-bromo-6-oxo-1-phenyl-pyridazine-3-carboxylate

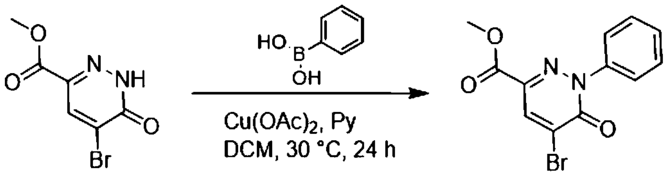


**Intermediate F**

To a solution of methyl-5-bromo-6-oxo-1H-pyridazine-3-carboxylate (3.00 g, 12.87 mmol) and phenylboronic acid (2.35 g, 19.31 mmol) in DCM (40 mL), pyridine (6.62 g, 83.68 mmol) and $Cu(OAc)_2$ (1.17 g, 6.44 mmol) were added, followed by stirring at 30 °C for 24 h. The reaction mixture was poured into water (50 mL) and extracted with EtOAc, and the combined organic layer was washed with brine, dried over anhydrous $Na_2SO_4$ and concentrated under reduced pressure to obtain a crude product. The crude product was purified by silica gel column chromatography (15% EtOAc in petroleum ether) to give Intermediate F (2.5 g, 57.43% yield) as a light yellow solid. LC/MS (ESI) m/z = 309.0 $[M+H]^+$.

**Preparation Example 7: Methyl-1-(2-nitrophenyl)-6-oxopyridazine-3-carboxylate**

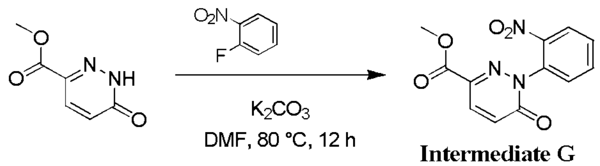


Intermediate G

A mixture of methyl-6-oxo-1H-pyridazine-3-carboxylate (1 g, 6.49 mmol), 1-fluoro-2-nitro-benzene (1.10 g, 7.79 mmol) and $K_2CO_3$ (1.35 g, 9.73 mmol) in DMF (10 mL) was degassed and purged with $N_2$ for 3 times, and then stirred at 80 °C for 12 h under $N_2$ atmosphere. The reaction mixture was poured into water (10 mL) and extracted with EtOAc. The combined organic layer was washed with brine, dried over $Na_2SO_4$ and concentrated under reduced pressure to obtain a crude product. The crude product was purified by flash silica gel chromatography (40% EtOAc in petroleum ether) to give Intermediate G (1.2 g, 57.34% yield) as a white solid. LC/MS (ESI) m/z = 275.9 $[M+H]^+$.

Further, the following Intermediate G-1 to Intermediate G-4 were prepared in a similar method to that of Intermediate G.

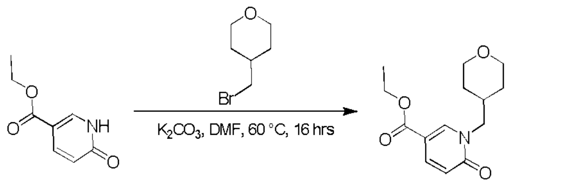


Intermediate G-1

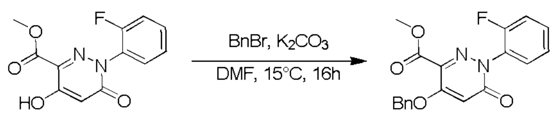


Intermediate AK

Intermediate G-2

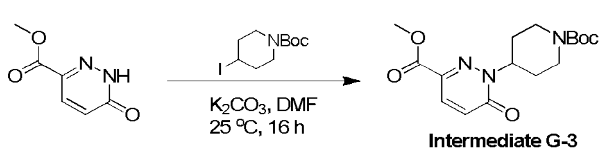


Intermediate G-3

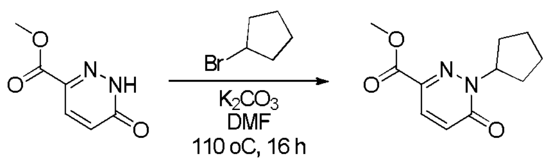


Intermediate G-4

**Preparation Example 8: Methyl-6-oxo-1-phenyl-4-(trifluoromethylsulfonyloxy)pyridazine-3-carboxylate**

Step 1: Synthesis of dimethyl 3-oxo-2-(phenylhydrazono)pentanedioate

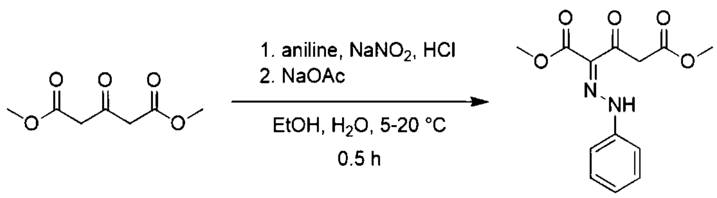


A mixture of HCl (10.20 g, 100.71 mmol, 10 mL, 36% purity), distilled water (20 mL) and aniline (1.86 g, 19.98 mmol, 1.82 mL) was treated with a solution of $NaNO_2$ (1.38 g, 19.98 mmol) in distilled water (15 mL) at 5 °C. The solution was poured into a mixture of dimethyl 3-oxopentanedioate (3.48 g, 19.98 mmol, 2.88 mL) in EtOH (12 mL) and NaOAc (12

g, 146.28 mmol) in distilled water (40 mL), and the reaction mixture was extracted with EtOAc. The combined organic layer was washed with brine, dried over anhydrous $Na_2SO_4$ and concentrated under reduced pressure to give dimethyl 3-oxo-2-(phenylhydrazono)pentanedioate (5.5 g, 89.02% yield) as yellow oil. $^1$H NMR (400MHz, DMSO-$d_6$) δ 11.99 (s, 1H), 7.49-7.40 (m, 4H), 7.19-7.10 (m, 1H), 3.89 (s, 2H), 3.85 (s, 3H), 3.62 (s, 3H).

Step 2: Synthesis of methyl-4-hydroxy-6-oxo-1-phenylpyridazine-3-carboxylate

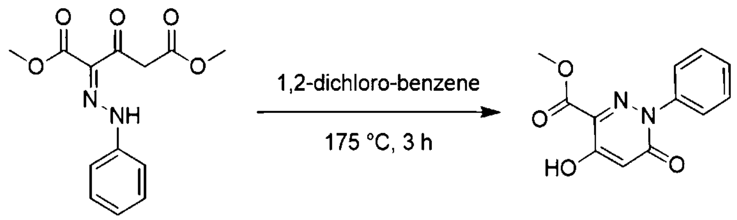


A solution of dimethyl 3-oxo-2-(phenylhydrazono)pentanedioate (5.30 g, 19.05 mmol) in 1,2-dichlorobenzene (50 mL) was stirred at 175 °C for 3 h. The mixture was purified by silica gel column chromatography (35% EtOAc in petroleum ether) to give methyl-4-hydroxy-6-oxo-1-phenyl-pyridazine-3-carboxylate (2.6 g, 52.67% yield) as a yellow solid. $^1$H NMR (400MHz, DMSO-$d_6$) δ 13.10-10.54 (m, 1H), 7.58-7.42 (m, 5H), 6.21 (s, 1H), 3.88-3.78 (m, 3H).

Step 3: Synthesis of methyl-6-oxo-1-phenyl-4-(trifluoromethylsulfonyloxy)pyridazine-3-carboxylate

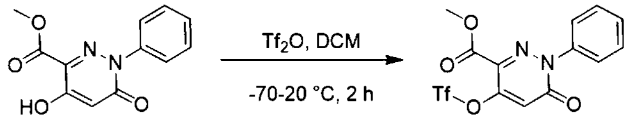


**Intermediate H**

To a solution of methyl-4-hydroxy-6-oxo-1-phenyl-pyridazine-3-carboxylate (200 mg, 812.29 μmol) in DCM (4 mL) was added trifluoromethanesulfonic acid anhydride ($Tf_2O$, 297.93 mg, 1.06 mmol, 174.23 μL) in DCM (10 mL) at -70 °C dropwise, and the mixture was stirred at 20 °C for 1 hour. The reaction mixture was poured into water (20 mL) and extracted with EtOAc, and the combined organic layer was washed with brine, dried over $Na_2SO_4$ and concentrated under reduced pressure to obtain a crude product. The crude product was purified by silica gel column chromatography (12% EtOAc in petroleum ether) to give Intermediate H (220 mg, 71.60% yield) as a yellow solid. LC/MS (ESI) m/z = 379.0 $[M+H]^+$).

**Preparation Example 9: (1R)-1-(3-ethoxyphenyl)ethanamine**

Step 1: Synthesis of 1-(3-ethoxyphenyl)ethanone

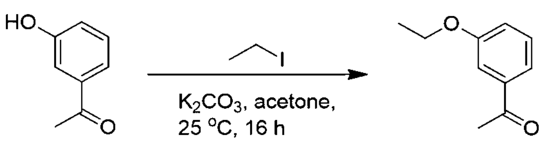


To a mixture of 1-(3-hydroxyphenyl)ethanone (5.00 g, 36.7 mmol) and iodoethane (10.5 g, 67.2 mmol) in acetone (50 mL) was added $K_2CO_3$ (10.2 g, 73.5 mmol), followed by stirring at 25 °C for 16 h under $N_2$. The reaction mixture was filtered, and the filtrate was concentrated under reduced pressure to obtain a residue. The residue was purified by silica gel column chromatography (6% EtOAc in petroleum ether) to give 1-(3-ethoxyphenyl)ethanone (5.65 g, 93.69% yield) as white oil. $^1$H NMR (400MHz, CHLOROFORM-d) δ = 7.52 (td, *J* = 1.2, 7.6 Hz, 1H), 7.49-7.45 (m, 1H), 7.36 (t, *J* = 8.0 Hz, 1H), 7.05-7.13 (m, 1H), 4.08 (q, *J* = 6.8 Hz, 2H), 2.60-2.58 (m, 3H), 1.43 (t, *J* = 6.8 Hz, 3H).

Step 2: Synthesis of (R)-N-[1-(3-ethoxyphenyl)ethylidene]-2-methyl-propane-2-sulfinamide

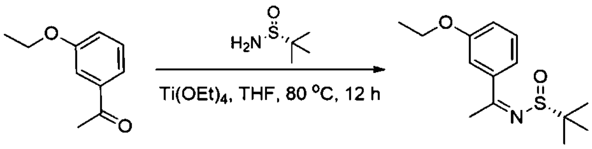


To a solution of 1-(3-ethoxyphenyl)ethanone (1.50 g, 9.14 mmol) and (R)-2-methylpropane-2-sulfinamide (1.66 g, 13.7 mmol) in THF (20 mL) was added $Ti(OEt)_4$ (6.25 g, 27.4 mmol), followed by stirring at 80 °C for 12 h. The reaction mixture was poured into water (50 mL), then a large amount of white solid was obtained, and the mixture was filtrated. The filter cake was washed with EtOAc, then the filtrate was combined and separated, and the organic layer was dried over $Na_2SO_4$ and concentrated under reduced pressure to obtain a residue. The residue was purified by silica gel column chromatography (0-18% EtOAc in petroleum ether) to give (R)-N-[1-(3-ethoxyphenyl)ethylidene]-2-methyl-propane-2-sulfinamide (2.20 g, 90.07% yield) as yellow oil. $^1H$ NMR (400MHz, CHLOROFORM-d) $\delta$ = 7.50-7.36 (m, 2H), 7.32 (t, $J$ = 8.4 Hz, 1H), 7.02 (dd, $J$ = 1.6, 8.0 Hz, 1H), 4.07 (q, $J$ = 7.2 Hz, 2H), 2.75 (s, 3H), 1.43 (t, $J$ = 7.2 Hz, 3H), 1.32 (s, 9H); LC/MS (ESI) m/z = 268.1 $[M+H]^+$.

Step 3: Synthesis of (R)-N-[(1R)-1-(3-ethoxyphenyl)ethyl]-2-methyl-propane-2-sulfinamide

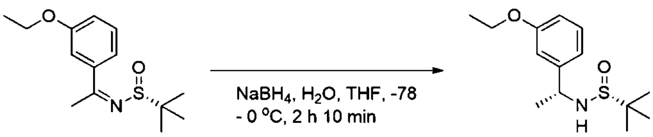


To a solution of (R)-N-[1-(3-ethoxyphenyl)ethylidene]-2-methyl-propane-2-sulfinamide (900 mg, 3.37 mmol) in THF (10 mL) and $H_2O$ (0.2 mL) was added $NaBH_4$ (285 mg, 7.53 mmol) dropwise at -78 °C, and then the mixture was stirred at -78 °C for 10 min and warmed to 0 °C. The resulting mixture was stirred at 0 °C for another 2 h. The mixture was diluted with water (20 mL) and extracted with EtOAc. The combined organic layer was washed with brine, dried over $Na_2SO_4$ and concentrated under reduced pressure to obtain a residue as a mixture of diastereomers. The residue was purified by silica gel column chromatography (0-17% EtOAc in petroleum ether) to give a main product, (R)-N-[(1R)-1-(3-ethoxyphenyl)ethyl]-2-methyl-propane-2-sulfinamide (716 mg, 78.96% yield) as colorless oil. $^1H$ NMR (400MHz, CHLOROFORM-d) $\delta$ 7.29 (s, 1H), 7.28-7.30 (m, 1H), 6.92-6.97 (m, 2H), 6.84 (dd, $J$ = 8.4, 2.0 Hz, 1H), 4.51-4.58 (m, 1H), 4.04-4.09 (m, 2H), 3.45 (brs, 1H), 1.29-1.79 (m, 15H); LC/MS (ESI) m/z = 270.1 $[M+H]^+$.

Step 4: Synthesis of (1R)-1-(3-ethoxyphenyl)ethanamine

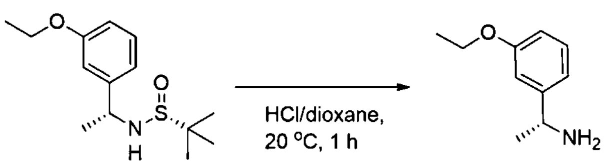


Intermediate I

A solution of (R)-N-[(1R)-1-(3-ethoxyphenyl)ethyl]-2-methyl-propane-2-sulfinamide (60.0 mg, 223 $\mu$mol) in 4 N HCl/dioxane (1 mL) was stirred at 20 °C for 1 hour. The reaction mixture was concentrated under reduced pressure to give Intermediate I (53 mg, crude, HCl salt) as colorless oil. LC/MS (ESI) m/z = 166.1 $[M+H]^+$.

**Preparation Example 10: (1R)-1-(3-trimethylsilylphenyl)ethanamine**

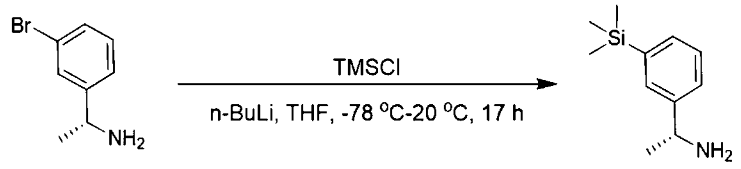


**Intermediate J**

To a solution of (1R)-1-(3-bromophenyl)ethanamine (100 mg, 499.81 μmol) in THF (2 mL) was added n-BuLi (2.5 M, 899.66 μL) dropwise at -78 °C, and the mixture was stirred for 1 hour and then added with trimethylsilyl chloride (135.75 mg, 1.25 mmol, 158.59 μL) at -78°C. The resulting mixture was stirred at 20 °C for 17 h. The reaction mixture was poured into water (20 mL) and extracted with EtOAc, and the organic layer was washed with brine, dried over $Na_2SO_4$ and concentrated under reduced pressure to obtain a crude product. The crude product was purified by silica gel column chromatography (25% EtOAc in petroleum ether) to give Intermediate J (22 mg, 22.76% yield) as light-yellow oil. $^1H$ NMR (400 MHz, CHLOROFORM-d) δ 7.49 (s, 1H), 7.44-7.40 (m, 1H), 7.38-7.32 (m, 2H), 4.14 (dd, *J* = 3.6, 6.8 Hz, 1H), 1.42 (d, *J* = 6.8 Hz, 3H), 0.28 (s, 9H).

**Preparation Example 11: Methyl-5-oxo-4-phenyl-pyrazine-2-carboxylate**

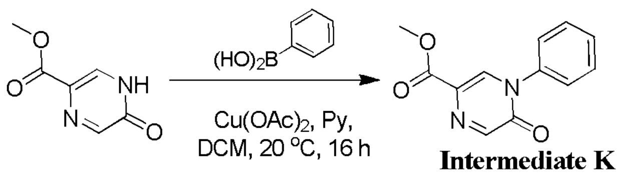


**Intermediate K**

Intermediate K was prepared in the same method as in Step 1 of Preparation Example 1, except that methyl-6-oxo-1H-pyrazine-3-carboxylate was used instead of methyl-6-oxo-1H-pyridazine-3-carboxylate. LC/MS (ESI) m/z = 231.0 [M+H]$^+$.

Further, the following Intermediates K-1 to K-22 were prepared in a similar method to that of Intermediate K.

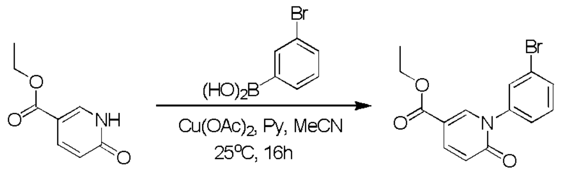


**Intermediate K-1**

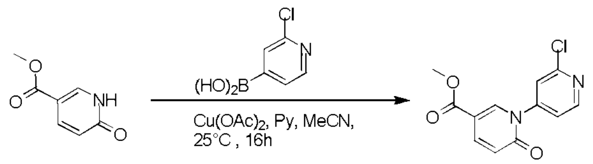


**Intermediate K-2**

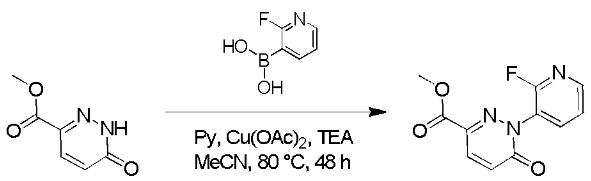


**Intermediate K-3**

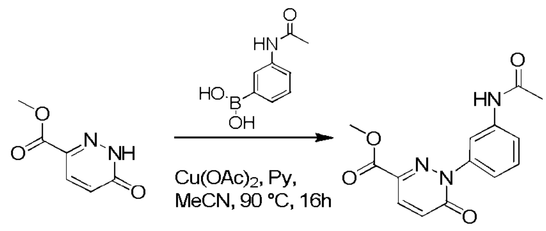


**Intermediate K-4**

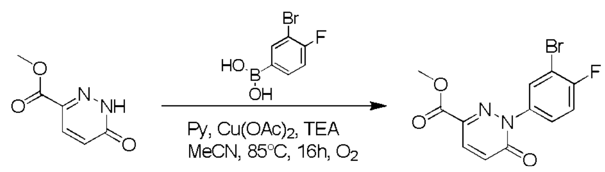


**Intermediate K-5**

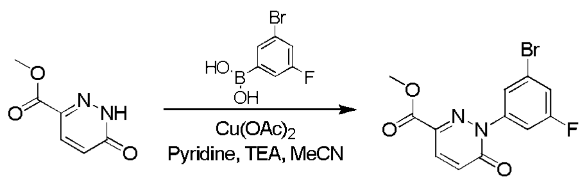


**Intermediate K-6**

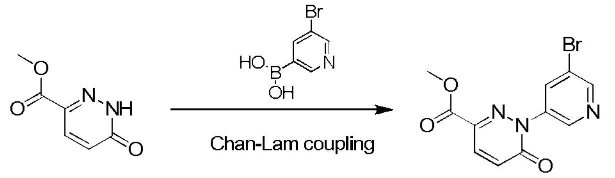


Intermediate K-7

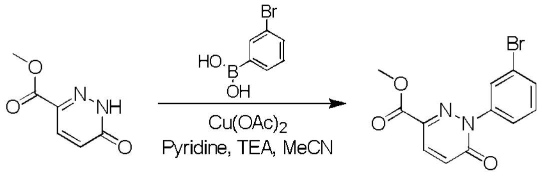


Intermediate K-8

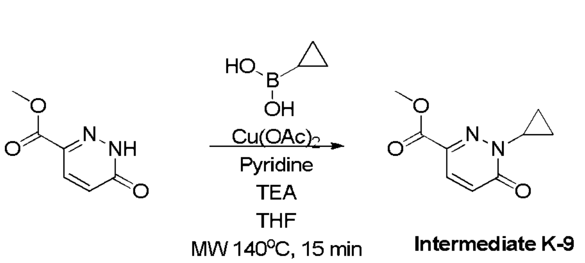


Intermediate K-9

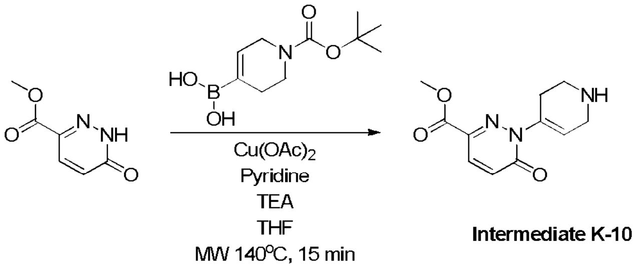


Intermediate K-10

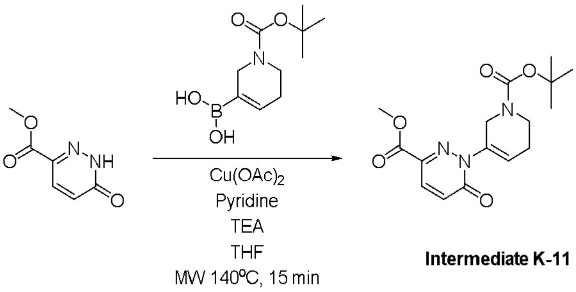


Intermediate K-11

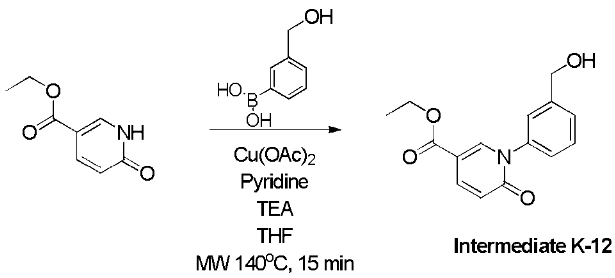


Intermediate K-12

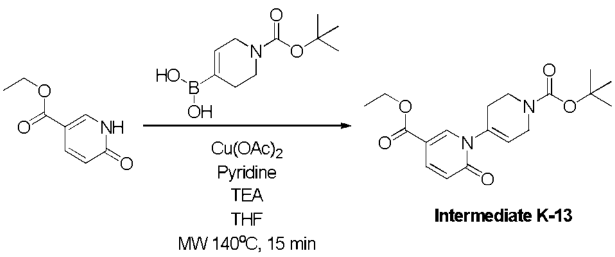


Intermediate K-13

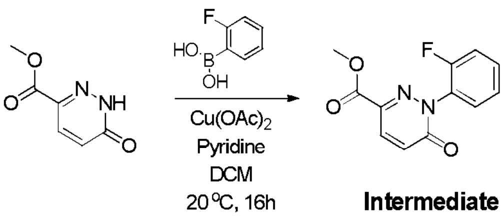


Intermediate K-14

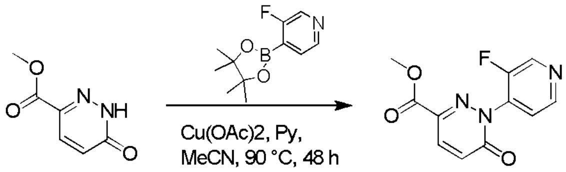


Intermediate K-15

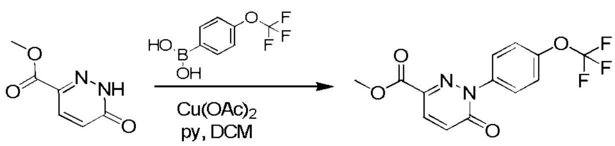


Intermediate K-16

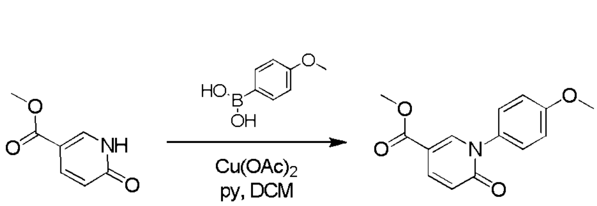


Intermediate K-17

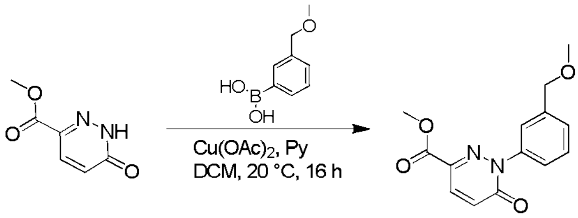


Intermediate K-18

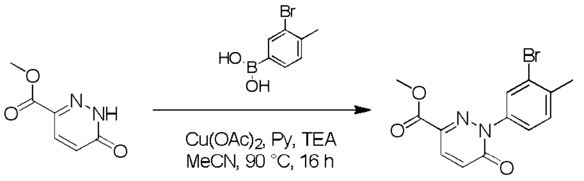


Intermediate K-19

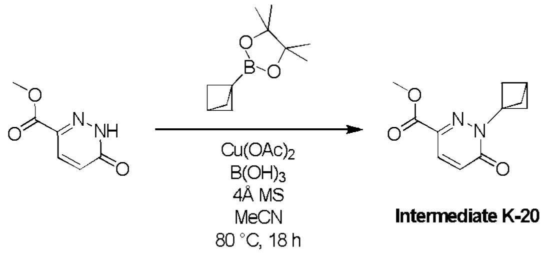


Intermediate K-20

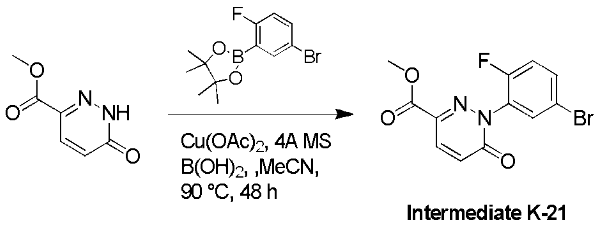


Intermediate K-21

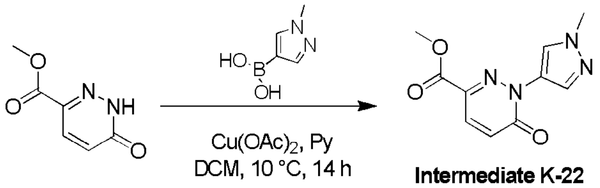


Intermediate K-22

**Preparation Example 12: (1R)-1-[(3-pentafluoro-λ$^6$-sulfanyl)phenyl]ethanamine**

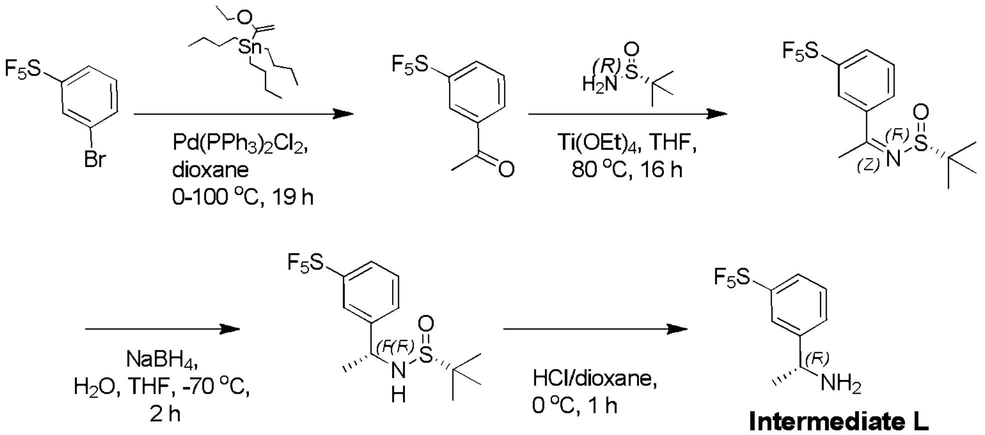


Intermediate L

Intermediate L was prepared in the same manner as in Steps 1 to 4 of Preparation Example 2 by changing the starting materials and the reagents used in Step 1 of Preparation Example 2 to 1-bromo-3-pentafluoro-λ$^6$-sulfanylbenzene, tributyl(1-ethoxyvinyl)stannane, Pd(PPh$_3$)$_2$Cl$_2$ and dioxane. LC/MS (ESI) m/z = 247.1 [M+H]$^+$.

**Preparation Example** 13: (R)-3-(1-aminoethyl)-2-fluorobenzonitrile

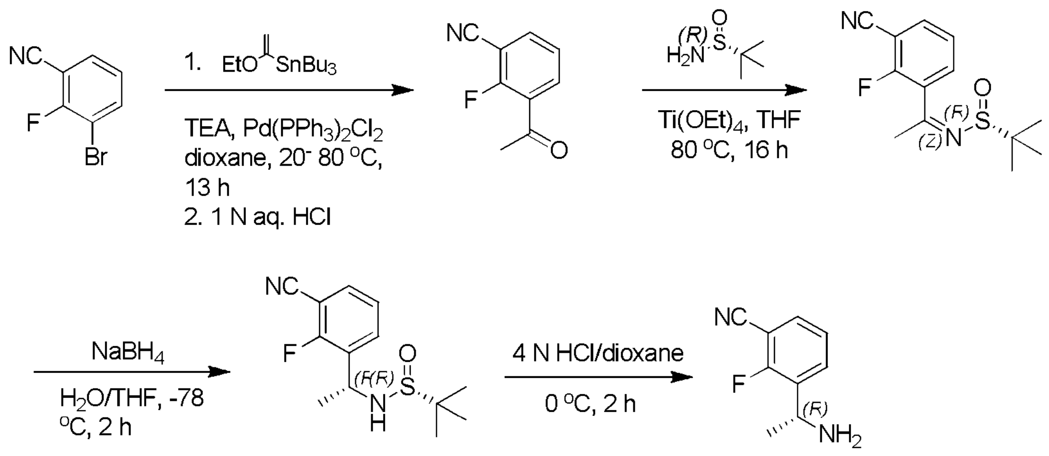


**Intermediate M**

Intermediate M was prepared in a similar method to Preparation Example 12. MS (ESI) m/z = 164.1 [M+H]$^+$.

**Preparation Example 14: (R)-1-(2-methyl-5-nitro-3-(trifluoromethyl)phenyl)ethanamine**

Step 1: Synthesis of 1-bromo-2-methyl-5-nitro-3-(trifluoromethyl)benzene

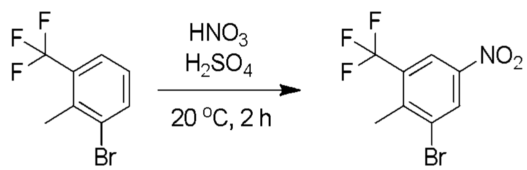


To a mixture of 1-bromo-2-methyl-3-(trifluoromethyl)benzene (10 g, 41.84 mmol) in $H_2SO_4$ (80 mL) was added $HNO_3$ (54.480 g, 864.59 mmol, 38.91 mL) slowly at 0 °C, followed by stirring at 20°C for 2 h under $N_2$ atmosphere. The reaction mixture was quenched with ice-cold water (100 mL) and extracted with EtOAc (100 mL × 3). The combined organic layer was washed with brine (50 mL), dried over anhydrous $Na_2SO_4$ and concentrated under reduced pressure to obtain a crude product. The crude product was purified by flash silica gel chromatography (0% EtOAc in PE) to give 1-bromo-2-methyl-5-nitro-3-(trifluoromethyl)benzene (6 g, 50.49% yield) as colourless oil. $^1H$ NMR (400 MHz, DMSO-$d_6$) $\delta$ = 8.69 (d, $J$ = 2.4 Hz, 1H), 8.38 (d, $J$ = 2.4 Hz, 1H), 2.58 (d, $J$ = 1.2 Hz, 3H).

Steps 2 to 5: Synthesis of (R)-1-(2-methyl-5-nitro-3-(trifluoromethyl)phenyl)ethanamine

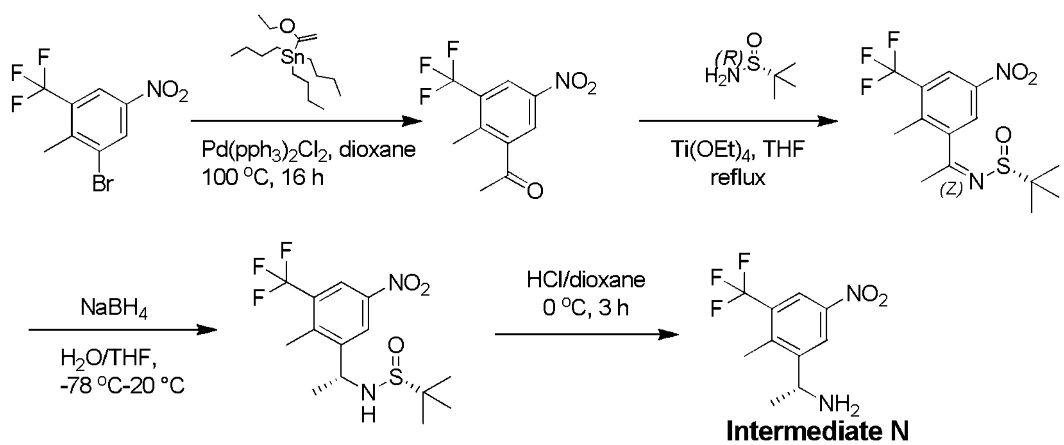


**Intermediate N**

Intermediate N was prepared in the same manner as in Preparation Example 12 by using 1-bromo-2-methyl-5-nitro-3-(trifluoromethyl)benzene as a starting material. MS (ESI) m/z = 248.08 $[M+H]^+$.

**Preparation Example 15: 1-[3-(dimethylcarbamoyl)-4-methoxy-phenyl]-6-oxo-pyridazine-3-carboxylic acid**

Step 1: Synthesis of 5-bromo-2-methoxy-N,N-dimethyl-benzamide

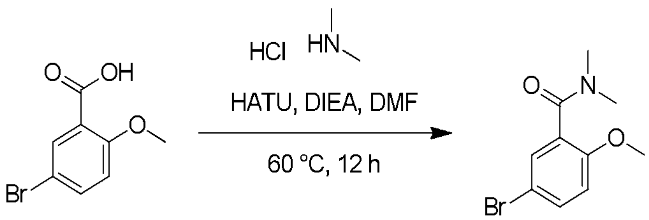


To a solution of 5-bromo-2-methoxy-benzoic acid (1 g, 4.33 mmol) in DMF (15 mL), DIEA (2.24 g, 17.31 mmol, 3.02 mL) and HATU (2.47 g, 6.49 mmol) were aded. The mixture was stirred at 25 °C for 0.5 hour. N-methylmethanamine;hydrochloride (1.06 g, 12.98 mmol) was added thereto, and the resulting mixture was stirred at 60 °C for 12 h. The reaction mixture was poured into water (20 mL) and extracted with EtOAc (30 mL × 3). The combined organic layer was washed with brine (20 mL × 2), dried over anhydrous $Na_2SO_4$ and concentrated under reduced pressure to obtain a crude product. The crude product was purified by silica gel column chromatography (35% EtOAc in PE) to give 5-bromo-2-methoxy-N,N-dimethyl-benzamide (930 mg, 83.25% yield) as yellow oil. MS (ESI) m/z = 258.0 $[M+H]^+$.

Step 2: Synthesis of 1-[3-(dimethylcarbamoyl)-4-methoxy-phenyl]-6-oxo-pyridazine-3-carboxylic acid

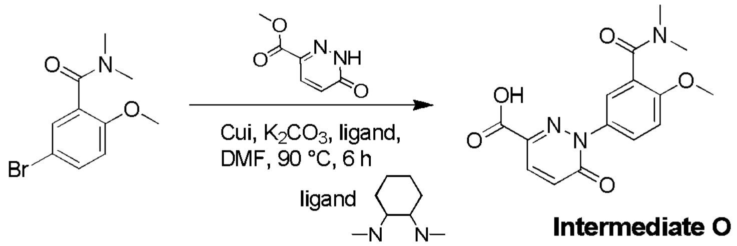


A mixture of methyl-6-oxo-1H-pyridazine-3-carboxylate (89.57 mg, 581.14 μmol), 5-bromo-2-methoxy-N,N-dimethylbenzamide (100 mg, 387.43 μmol), CuI (73.79 mg, 387.43 μmol), (1R,2R)-N1,N2-dimethylcyclohexane-1,2-diamine (110.22 mg, 774.86 μmol) and $K_2CO_3$ (160.64 mg, 1.16 mmol) in DMF (3 mL) was degassed and purged with $N_2$ for 3 times, and then the mixture was stirred at 90 °C for 6 h under $N_2$ atmosphere. The reaction mixture was poured into water (20 mL) and extracted with EtOAc (20 mL ×3). The combined organic layer was discarded. The aqueous layer was adjusted by 1 N aq. HCl to pH = 3-4, and extracted with EtOAc (30 mL × 3). The combined organic layer was washed with brine (20 mL × 2), dried over anhydrous $Na_2SO_4$ and concentrated under reduced pressure to give Intermediate O (50 mg, 40.67% yield) as yellow oil. MS (ESI) m/z = 318.1 $[M+H]^+$.

**Preparation Example 16: (R)-1-(3-(1-aminoethyl)-2-fluorophenyl)-1,1-difluoro-2-methylpropan-2-ol**

Step 1: Synthesis of ethyl 2-[3-(1,1-dimethoxyethyl)-2-fluoro-phenyl]-2,2-difluoro-acetate

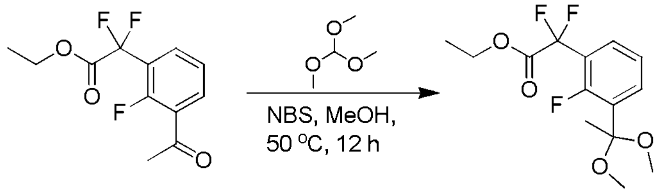


To a solution of ethyl 2-(3-acetyl-2-fluoro-phenyl)-2,2-difluoro-acetate (7.1 g, 27.29 mmol) obtained in Step 3 of Preparation Example 5 in MeOH (100 mL), trimethoxymethane (8.69 g, 81.86 mmol, 8.97 mL) and NBS (291.39 mg, 1.64 mmol) were added. The mixture was stirred at 50 °C for 12 h. The mixture was concentrated under reduced pressure to obtain a residue. The residue was purified by flash silica gel chromatography (10% EtOAc in PE) to give ethyl 2-[3-(1,1-dimethoxyethyl)-2-fluoro-phenyl]-2,2-difluoro-acetate (6.42 g, 76.82% yield) as colorless oil. $^1H$ NMR (400 MHz, DMSO-$d_6$) δ = 7.80 (t, *J* = 7.2 Hz, 1H), 7.70 (t, *J* = 6.8 Hz, 1H), 7.40 (t, *J* = 7.6 Hz, 1H), 4.39 - 4.31 (m, 2H), 3.08 (s, 6H), 1.53 (s, 3H), 1.20 (t, *J* = 7.2 Hz, 3H).

Step 2: Synthesis of 1-[3-(1,1-dimethoxyethyl)-2-fluorophenyl]-1,1-difluoro-2-methyl-propan-2-ol

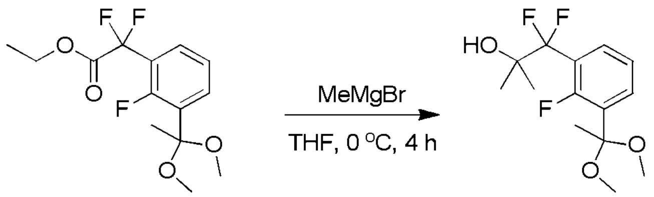


A mixture of ethyl 2-[3-(1,1-dimethoxyethyl)-2-fluoro-phenyl]-2,2-difluoro-acetate (6.42 g, 20.96 mmol) and MeMgBr (1 M, 62.88 mL) in THF (65 mL) was degassed and purged with $N_2$ for 3 times at 0 °C, and then the mixture was stirred at 0°C for 4 h under $N_2$ atmosphere. The reaction mixture was quenched by addition of sat. aq. $NH_4Cl$ (100 mL) at 20°C, diluted with EtOAc (100 mL) and extracted with EtOAc (100 mL × 3). The mixture was dried over anhydrous $Na_2SO_4$ and concentrated under reduced pressure to give a crude product. The residue was purified by flash silica gel chromatography (10% EtOAc in PE) to give 1-[3-(1,1-dimethoxyethyl)-2-fluorophenyl]-1,1-difluoro-2-methyl-propan-2-ol (4.5 g, 73.45% yield) as colorless oil. $^1H$ NMR (400 MHz, DMSO-$d_6$) δ = 7.70 (t, *J* = 7.2 Hz, 1H), 7.43 (t, *J* = 6.8 Hz, 1H), 7.26 (t, *J* = 7.6 Hz, 1H), 5.33 (s, 1H), 3.11 - 3.07 (m, 6H), 1.54 (s, 3H), 1.20 (s, 6H).

Step 3: Synthesis of 1-[3-(1,1-dimethoxyethyl)-2-fluorophenyl]-1,1-difluoro-2-methyl-propan-2-ol

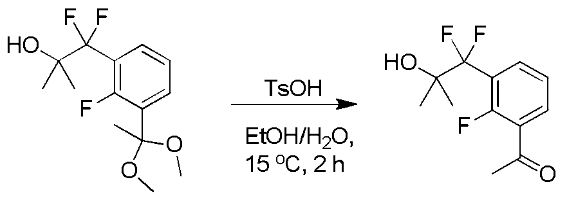


A solution of 1-[3-(1,1-dimethoxyethyl)-2-fluoro-phenyl]-1,1-difluoro-2-methyl-propan-2-ol (4.5 g, 15.40 mmol) and TsOH (5.30 g, 30.79 mmol) in $H_2O$ (4.5 mL) and EtOH (45 mL) was prepared. The reaction mixture was stirred at 15°C for 2 h under $N_2$. The solution was concentrated under reduced pressure, and the reaction mixture was poured into water (50 mL) and extracted with EtOAc (50 mL × 3). The combined organic layer was washed with brine (50 mL), dried over anhydrous $Na_2SO_4$ and concentrated under reduced pressure to obtain a crude product. The crude product as yellow oil, 1-[3-(1,1-difluoro-2-hydroxy-2-methyl-propyl)-2-fluorophenyl]ethanone (3.7 g, 97.61% yield) was used in the next step without further purification. $^1H$ NMR (400 MHz, DMSO-$d_6$) δ = 7.94 - 7.87 (m, 1H), 7.67 (t, *J* = 7.2 Hz, 1H), 7.42 - 7.38 (m, 1H), 5.42 (s, 1H), 2.60 - 2.57 (m, 3H), 1.22 (s, 6H).

Steps 4 to 6: Synthesis of (R)-1-(3-(1-aminoethyl)-2-fluorophenyl)-1,1-difluoro-2-methylpropan-2-ol

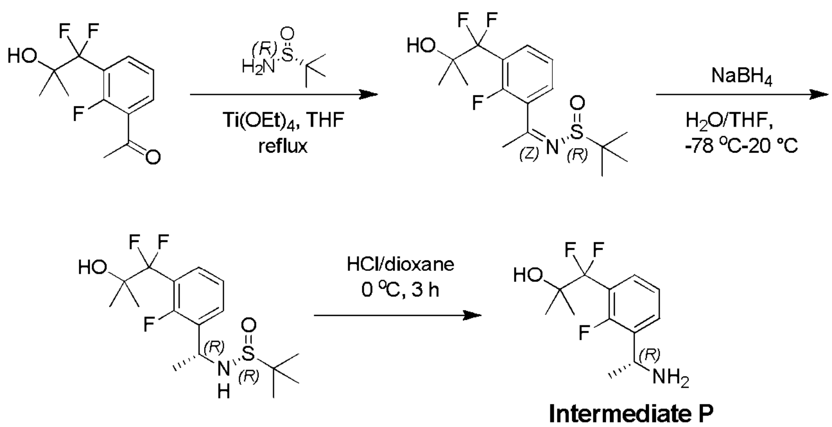


**Intermediate P**

Intermediate P was prepared in the same manner as in Steps 4 to 6 of Preparation Example 5. LC/MS (ESI) m/z = 247.1 $[M+H]^+$.

**Preparation Example 17: methyl-1-(2-methylthiazol-5-yl)-6-oxopyridazine-3-carboxylate**

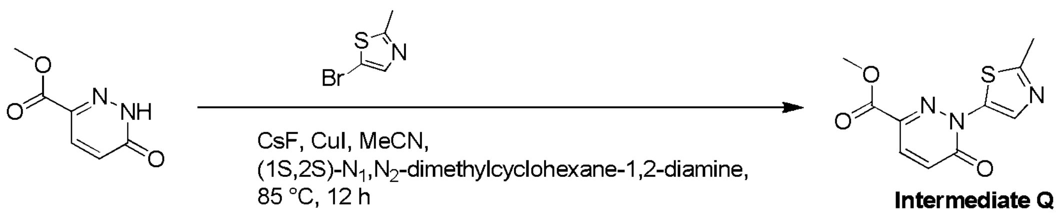


**Intermediate Q**

A mixture of methyl-6-oxo-1H-pyridazine-3-carboxylate (150 mg, 973.25 μmoL), 5-bromo-2-methyl-thiazole (207.94 mg, 1.17 mmol), CuI (18.54 mg, 97.32 μmoL), CsF (443.52 mg, 2.92 mmol) and (1S,2S)-N1,N2-dimethylcyclohexane-1,2-diamine (27.69 mg, 194.60 μmoL) in MeCN (3 mL) was degassed and purged with $N_2$ for 3 times, and then the mixture was stirred at 85 °C for 12 h under $N_2$ atmosphere. The reaction mixture was poured into water (20 mL) and extracted with EtOAc (30 mL × 3). The combined organic layer was washed with brine (20 mL×2), dried over anhydrous $Na_2SO_4$ and concentrated under reduced pressure to obtain a crude product. The product was purified by silica gel column chromatography (45% EtOAc in PE) to give Intermediate Q (70 mg, 16.89% yield) as an off-white solid. MS (ESI) m/z = 252.1 $[M+H]^+$

The following Intermediates Q-1, Q-2, and Q-3 were prepared in a similar manner.

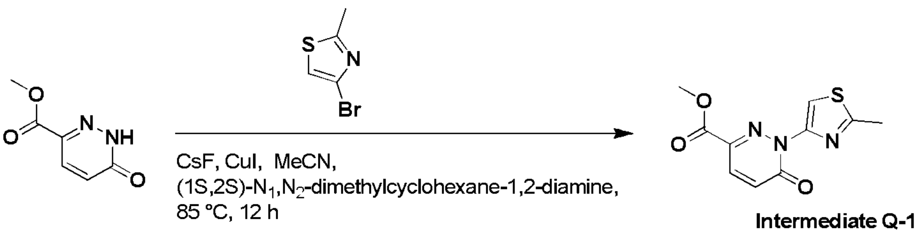


**Intermediate Q-1**

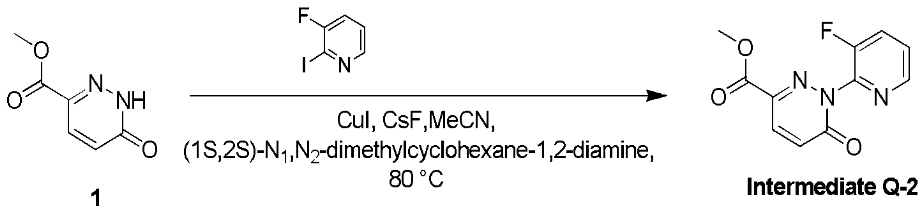


1

Intermediate Q-2

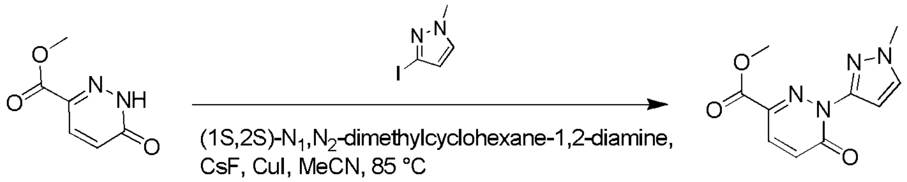


Intermediate Q-3

**Preparation Example 18: methyl-1-(1,3-dihydroisobenzofuran-5-yl)-6-oxo-pyridazine-3-carboxylate**

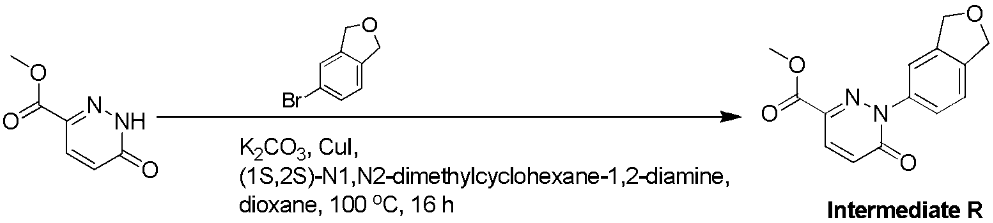


Intermediate R

A mixture of methyl-6-oxo-1H-pyridazine-3-carboxylate (150.99 mg, 979.65 μmol), 5-bromo-1,3-dihydroisobenzofuran (194.99 mg, 979.65 μmol, 1.0 eq), $K_2CO_3$ (406.18 mg, 2.94 mmol), CuI (186.58 mg, 979.65 μmol) and (1S,2S)-N1,N2-dimethylcyclohexane-1,2-diamine (278.70 mg, 1.96 mmol) in dioxane (3 mL) was degassed and purged with $N_2$ for 3 times, and then the mixture was stirred at 100 °C for 16 h under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The crude product was purified by flash silica gel chromatography (32% EtOAc in PE) to give Intermediate R (60 mg, 14.32% yield) as a white solid. MS (ESI) m/z = 273.1 $[M+H]^+$.

**Preparation Example 19: methyl-1-(5-chloro-1-methyl-pyrazol-4-yl)-6-oxo-pyridazine-3-carboxylate**

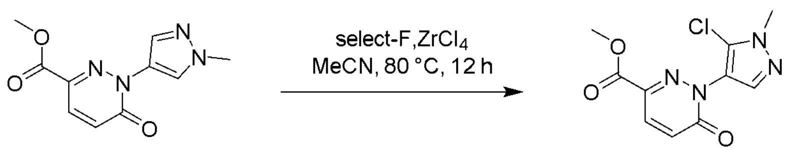


Intermediate K-22 Intermediate S

Methyl-1-(1-methylpyrazol-4-yl)-6-oxo-pyridazine-3-carboxylate, Intermediate K-22 (300 mg, 1.28 mmol), Select F (680.66 mg, 1.92 mmol) and $ZrCl_4$ (59.70 mg, 256.18 μmol, 21.32 μL) were added in MeCN (6 mL). The mixture was stirred at 80 °C for 12 h. The reaction mixture was poured into water (20 mL) and extracted with EtOAc (30 mL × 3). The combined organic layer was washed with brine (20 mL × 2), dried over anhydrous $Na_2SO_4$ and concentrated under reduced pressure to obtain a crude product. The product was purified by silica gel column chromatography (35% EtOAc in PE) to give Intermediate S (123 mg, 35.74% yield) as yellow oil. MS (ESI) m/z = 269.1 $[M+H]^+$.

**Preparation Example 20: methyl-4-anilno-1-(2-fluorophenyl)-6-oxo-pyridazine-3-carboxylate**

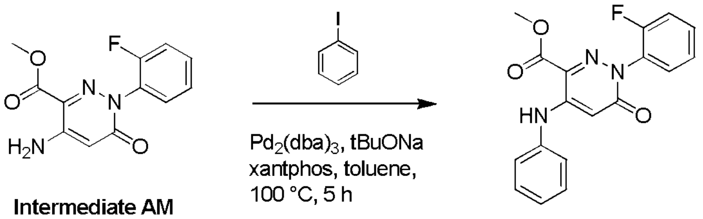


Intermediate AM

Intermediate T

To a mixture of iodobenzene (155.01 mg, 759.81 µmol, 84.70 µL) and methyl-4-amino-1-(2-fluorophenyl)-6-oxo-pyridazine-3-carboxylate (100 mg, 379.90 µmol) in toluene (5 mL), Xantphos (21.98 mg, 37.99 µmol), t-BuONa (54.77 mg, 569.86 µmol) and $Pd_2(dba)_3$ (34.79 mg, 37.99 µmol) were added, and the reaction mixture was stirred at 100 °C for 5 h under $N_2$. The reaction mixture was poured into water (30 mL) and extracted with EtOAc (30 mL×3). The combined organic layer was washed with brine (50 mL), dried over anhydrous $Na_2SO_4$ and concentrated under reduced pressure to obtain a residue. The residue was purified by silica gel column chromatography (61% EtOAc in PE) to give Intermediate T (28 mg, 15.91% yield) as a yellow solid. MS (ESI) m/z = 340.0 $[M+1+H]^+$.

**Preparation Example 21: 1-[3-(4-methyl-1,2,4-triazol-3-yl)phenyl]-6-oxo-pyridine-3-carboxylate**

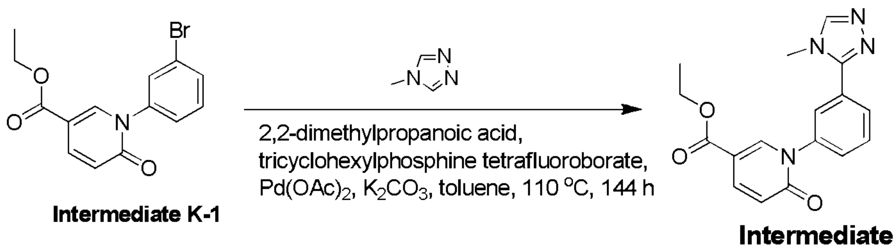


Intermediate K-1

Intermediate U

A mixture of Intermediate K-1 (200 mg, 620.82 µmol), 4-methyl-1,2,4-triazole (67.06 mg, 807.07 µmol), $Pd(OAc)_2$ (13.94 mg, 62.08 µmol), tricyclohexylphosphonium;tetrafluoroborate (45.72 mg, 124.16 µmol), 2,2-dimethylpropanoic acid (126.81 mg, 1.24 mmol, 142.64 µL) and $K_2CO_3$ (171.61 mg, 1.24 mmol) in toluene (2 mL) was degassed and purged with $N_2$ for 3 times, and then the mixture was stirred at 120 °C for 144 h under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The crude product was purified by flash silica gel chromatography (8% MeOH in DCM) to give Intermediate U (180 mg, 21.38% yield, 23.912%) as a colorless oil. MS (ESI) m/z = 324.9 $[M+H]^+$.

Also, the following Intermediates U-1, U-2, U-3, U-4, and U-5 were prepared in a similar manner.

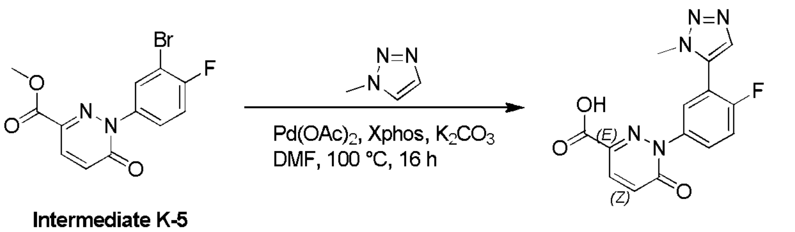


Intermediate K-5

Intermediate U-1

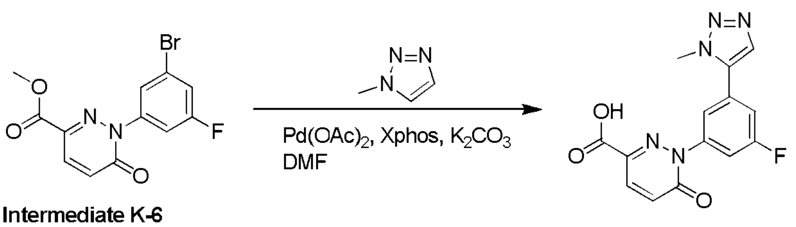


Intermediate K-6

Intermediate U-2

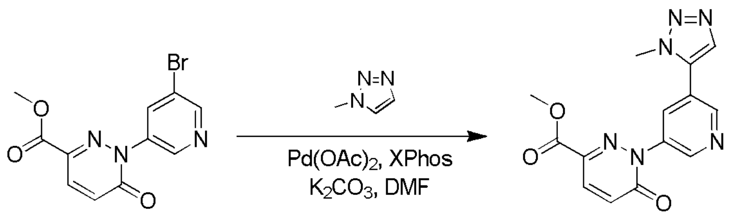


Intermediate K-7 Intermediate U-3

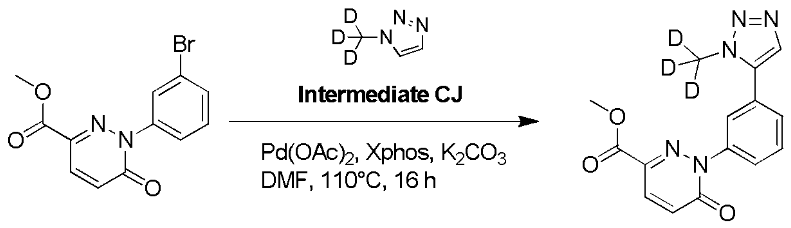


Intermediate K-8 Intermediate U-4

**Preparation Example 21A: 1-[4-methyl-3-(3-methyltriazol-4-yl)phenyl]-6-oxo-pyridazine-3-carboxylic acid**

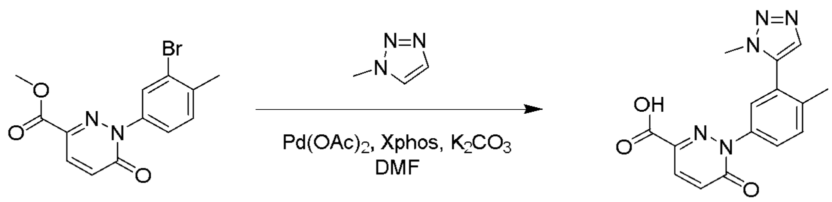


Intermediate K-19 Intermediate U-5

A mixture of methyl-1-(3-bromo-4-methyl-phenyl)-6-oxo-pyridazine-3-carboxylate (100 mg, 309.46 μmol), 1-methyltriazole (51.43 mg, 618.92 μmol), $K_2CO_3$ (85.54 mg, 618.92 μmol), Pd(OAc)$_2$ (6.95 mg, 30.95 μmol) and XPhos (29.51 mg, 61.89 μmol) in DMF (4 mL) was degassed and purged with $N_2$ for 3 times, and then the mixture was stirred at 100 °C for 12 h under $N_2$ atmosphere. The reaction mixture was poured into water (20 mL) and extracted with EtOAc (20 mL). The combined organic layer was discarded. The aqueous layer was adjusted by 1 N aq. HCl to pH = 2-3 and extracted with EtOAc (30 mL × 3). The combined organic layer was washed with brine (20 mL × 2), dried over anhydrous $Na_2SO_4$ and concentrated under reduced pressure to give Intermediate U-5 (80 mg, 29.26% yield) as yellow oil. LCMS (ESI) m/z = 321.1 [M+H]$^+$).

**Preparation Example 22: Ethyl-1-[3-(1-methyltetrazol-5-yl)phenyl]-6-oxo-pyridine-3-carboxylate**

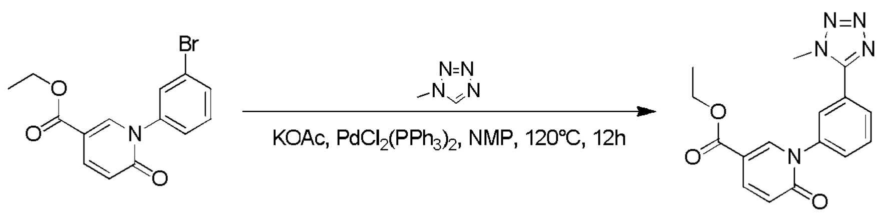


Intermediate K-1 Intermediate V

A mixture of Intermediate K-1 (104.55 mg, 324.54 μmol), 1-methyltetrazole (54.57 mg, 649.08 μmol), KOAc (63.70 mg, 649.08 μmol) and Pd(PPh$_3$)$_2$Cl$_2$ (22.78 mg, 32.45 μmol) in NMP (3 mL) was degassed and purged with $N_2$ for 3 times, and then the mixture was stirred at 120 °C for 12 h under $N_2$ atmosphere. The reaction mixture was poured into water (50 mL) and extracted with EtOAc (50 mL × 3). The combined organic layer was washed with brine (30 mL × 2), dried over

anhydrous $Na_2SO_4$ and concentrated under reduced pressure to obtain a crude product. The residue was purified by flash silica gel chromatography (48% EtOAc in PE) to give Intermediate V (154 mg, 68.66% yield) as yellow oil. MS (ESI) m/z = 326.0 [M+H]+.

**Preparation Example 23: (R)-1-(2-fluoro-3-(trifluoromethyl)phenyl)ethanamine**

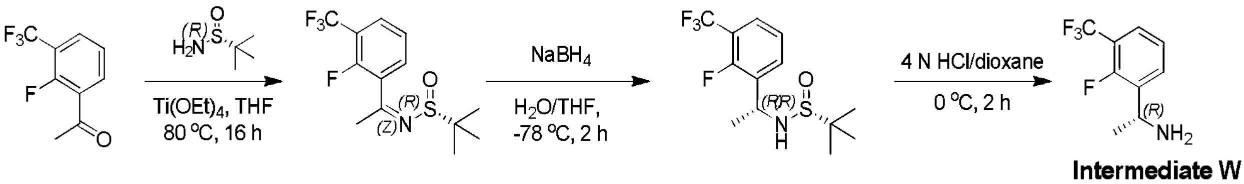


**Intermediate W**

Intermediate W was prepared in the same manner as in Steps 2 to 4 of Preparation Example 2.

**Preparation Example 24: 1-(2-chloro-3-fluorophenyl)ethanamine**

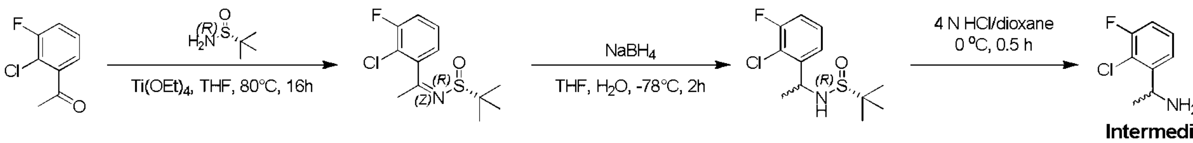


**Intermediate X**

Intermediate X was prepared in the same manner as in Steps 2 to 4 of Preparation Example 2.

**Preparation Example 25: (R)-1-(1H-pyrazol-3-yl)ethane-1-amine**

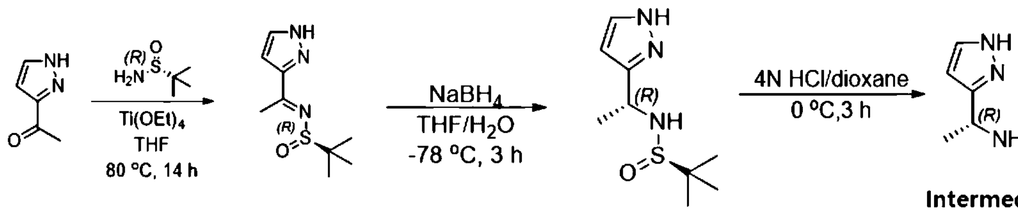


**Intermediate Y**

Intermediate Y was prepared in the same manner as in Steps 2 to 4 of Preparation Example 2. LC/MS m/z = 112.7 [M+H]+.

**Preparation Example 26: (R)-1-(5-bromothiophen-2-yl)ethanamine**

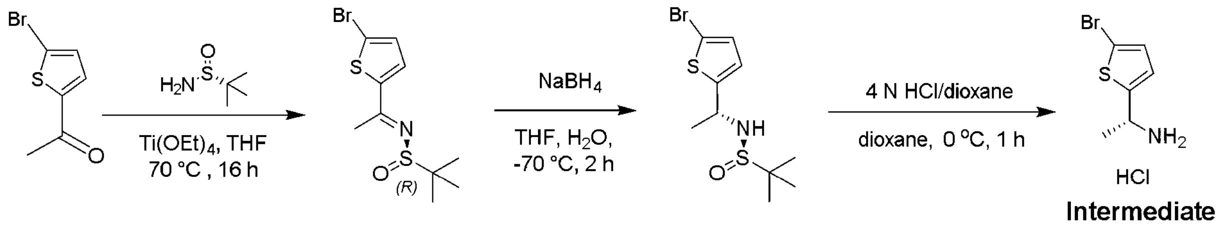


**Intermediate Z**

Intermediate Z was prepared as a yellow solid in the same manner as in Steps 2 to 4 of Preparation Example 2. $^1$H NMR (400 MHz, DMSO-$d_6$) δ = 7.06 (d, *J* = 4.0 Hz, 1H), 6.89 (dd, J = 0.8, 4.0 Hz, 1H), 5.90 (d, *J* = 7.2 Hz, 1H), 4.57 (t, V = 6.8

Hz, 1H), 1.47 (d, *J* = 6.8 Hz, 3H), 1.12 (s, 9H); MS (ESI) m/z = 311.8 $[M-16+H]^+$.

**Preparation Example 27: (R)-1-(5-bromothiazol-2-yl)ethanamine**

Step 1: Synthesis of (NZ,S)-N-[1-(5-bromothiazol-2-yl)ethylidene]-2-methyl-propane-2-sulfinamide

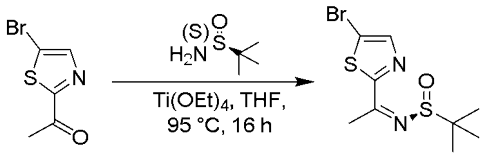


To a solution of 1-(5-bromothiazol-2-yl)ethanone (500 mg, 2.43 mmol) in THF (8 mL), $Ti(OEt)_4$ (8.31 g, 36.5 mmol) and (S)-2-methylpropane-2-sulfinamide (1.18 g, 9.72 mmol) were added, followed by stirring at 95 °C for 16 h under $N_2$. The mixture was diluted with EtOAc (20 mL), quenched with water (30 mL) and extracted with EtOAc (10 mL X 2). The mixture was dried over anhydrous $Na_2SO_4$ and concentrated under reduced pressure to obtain a residue, which was purified by silica gel column chromatography (20% EtOAc in PE) to give (NZ,S)-N-[1-(5-bromothiazol-2-yl)ethylidene]-2-methyl-propane-2-sulfinamide (700 mg, 93.0% yield) as yellow oil. $^1H$ NMR (400 MHz, $CDCl_3$) δ 7.82 (s, 1H), 2.81 (s, 3H), 1.32 (s, 9H); LC/MS (EI) m/z = 310.9 $[M+H]^+$.

Step 2: Synthesis of (S)-N-[(1R)-1-(5-bromothiazol-2-yl)ethyl]-2-methylpropane-2-sulfinamide

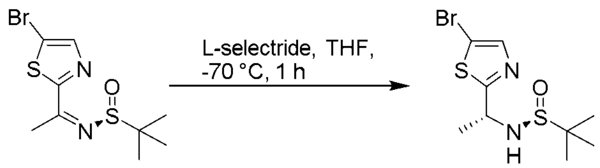


To a solution of (NZ,S)-N-[1-(2-bromothiazol-5-yl)ethylidene]-2-methyl-propane-2-sulfinamide (620 mg, 2.00 mmol) in THF (5 mL) was added L-selectride (1 M, 4.01 mL), followed by stirring at -70 °C for 1 hour under $N_2$. The reaction mixture was quenched with sat. aq. $NH_4Cl$ (10 mL) at 20 °C and extracted with EtOAc (10 mL X 3). The combined organic layer was dried over $Na_2SO_4$, filtered and concentrated under reduced pressure to obtain a residue. The residue was purified by silica gel column chromatography (25% EtOAc in PE) to give a main product, (S)-N-[(1R)-1-(5-bromothiazol-2-yl)ethyl]-2-methylpropane-2-sulfinamide (610 mg, 95% yield) as yellow oil. $^1H$ NMR (400 MHz, $CDCl_3$) δ 7.62 (s, 1H), 4.84-4.75 (m, 1H), 3.55 (d, *J* = 6.4 Hz, 1H), 1.73 (d, *J* = 6.8 Hz, 3H), 1.30 (s, 9H); LC/MS (EI) m/z = 311.0 $[M+H]^+$.

Step 3: Synthesis of (R)-1-(5-bromothiazol-2-yl)ethyl]ethanamine

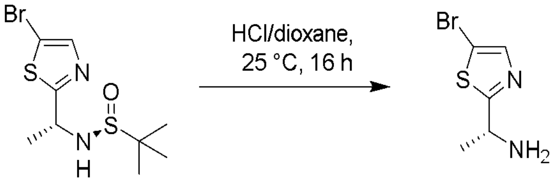


**Intermediate AA**

To a solution of (S)-N-[(1R)-1-(5-bromothiazol-2-yl)ethyl]-2-methylpropane-2-sulfinamide (200 mg, 643 μmol) in MeOH (2 mL) was added 4N HCl/dioxane solution (2.00 mL) at 0 °C, followed by stirring at 20 °C for 1 h. The mixture was concentrated under reduced pressure to give the HCl salt of Intermediate AA as a yellow solid. $^1H$ NMR (400 MHz, DMSO-$d_6$) δ 8.84 (s, 3H), 7.98 (s, 1H), 4.88-4.75 (m, 1H), 1.59 (d, *J* = 6.8 Hz, 3H)

**Preparation Example 27A: (R)-1-(2-bromothiazol-5-yl)ethanamine**

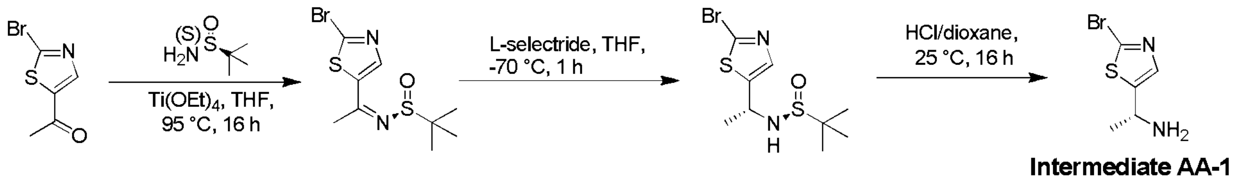


**Intermediate AA-1**

Intermediate AA-1 was prepared in the same manner as in Preparation Example 27. $^1$H NMR (400 MHz, DMSO-$d_6$) δ = 8.52 (s, 3H), 7.79 (s, 1H), 4.86 - 4.76 (m, 1H), 1.57 (d, *J* = 6.8 Hz, 3H).

**Preparation Example 28: methyl-1-[3-(dimethylcarbamoyl)-2-fluoro-phenyl]-4-hydroxy-6-oxo-pyridazine-3-carboxylate**

Step 1: Synthesis of 2-fluoro-N, N-dimethyl-3-nitrobenzamide

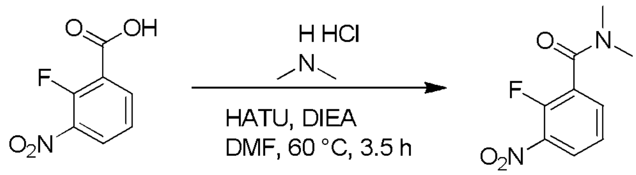


A mixture of 2-fluoro-3-nitro-benzoic acid (2.9 g, 15.67 mmol), N-methylmethanamine;hydrochloride (5.55 g, 47.00 mmol, HCl), DIEA (8.10 g, 62.67 mmol, 10.92 mL) and HATU (8.94 g, 23.50 mmol) in DMF (30 mL) was degassed and purged with $N_2$ for 3 times, and then the mixture was stirred at 60 °C for 3.5 h under $N_2$ atmosphere. The reaction mixture was poured into water (250 mL) and extracted with EtOAc (100 mL × 3). The combined organic layer was washed with brine (50 mL × 4), dried over anhydrous $Na_2SO_4$ and concentrated under reduced pressure to obtain a crude product. The product was purified by flash silica gel chromatography (12% EtOAc in PE) to give 2-fluoro-N, N-dimethyl-3-nitrobenzamide (4.3 g, 68.56% yield) as a yellow solid. MS (ESI) m/z = 213.1 $[M+H]^+$.

Step 2: Synthesis of 3-amino-2-fluoro-N,N-dimethyl-benzamide

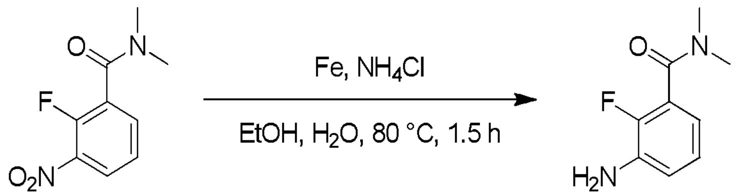


A mixture of 2-fluoro-N,N-dimethyl-3-nitro-benzamide (4.3 g, 20.27 mmol), Fe (11.32 g, 202.66 mmol) and $NH_4Cl$ (10.84 g, 202.66 mmol) in EtOH (40 mL) and $H_2O$ (8 mL) was degassed and purged with $N_2$ for 3 times, and then the mixture was stirred at 80 °C for 1.5 h under $N_2$ atmosphere. The mixture was filtrated, and the filtrate was poured into water (50 mL) and extracted with EtOAc (50 mL × 4). The combined organic layer was washed with brine (50 mL × 2), dried over anhydrous $Na_2SO_4$ and concentrated under reduced pressure to give 3-amino-2-fluoro-N,N-dimethyl-benzamide (crude, 2.81 g, 71.75% yield). MS (ESI) m/z = 183.1 $[M+H]^+$.

Steps 3 and 4: Synthesis of methyl-1-[3-(dimethylcarbamoyl)-2-fluoro-phenyl]-4-hydroxy-6-oxo-pyridazine-3-carboxylate

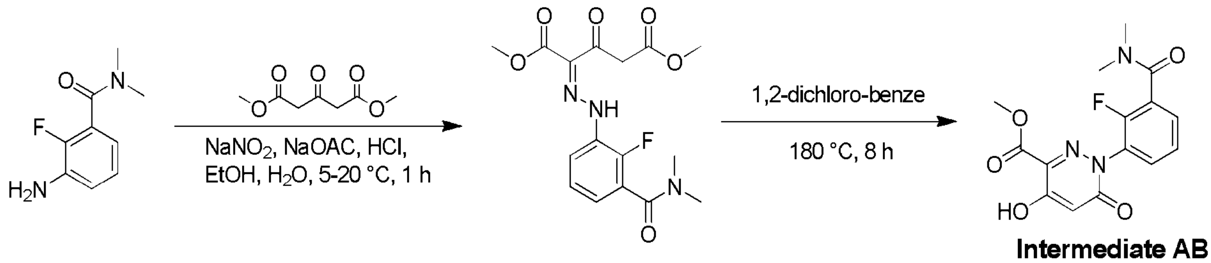


**Intermediate AB**

Intermediate AB was prepared in the same manner as in Steps 1 and 2 of Preparation Example 8 except that 3-amino-2-fluoro-N,N-dimethyl-benzamide was used instead of aniline in Step 1 of Preparation Example 8. $^1H$ NMR (400MHz, DMSO-$d_6$) $\delta$= 12.46 - 12.04 (m, 1H), 7.65 - 7.61 (m, 1H), 7.53 (ddd, $J$ = 1.6, 6.0, 7.6 Hz, 1H), 7.45 - 7.40 (m, 1H), 6.23 (s, 1H), 3.83 (s, 3H), 3.01 (s, 3H), 2.86 (s, 3H); MS (ESI) m/z = 336.1 $[M+H]^+$.

**Preparation Example 29: methyl-1-[3-(dimethylcarbamoyl)-2-fluoro-phenyl]-6-oxo-pyridazine-3-carboxylate**

Step 1: Synthesis of methyl-1-[3-(dimethylcarbamoyl)-2-fluoro-phenyl]-6-oxo-4-(trifluoromethylsulfonyloxy)pyridazine-3-carboxylate

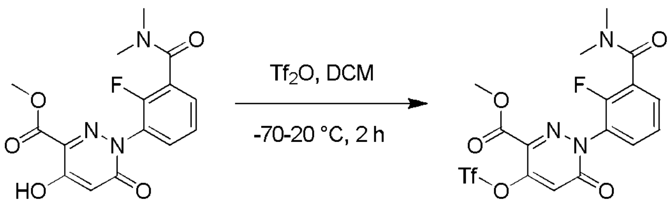


**Intermediate AB**

Methyl-1-[3-(dimethylcarbamoyl)-2-fluoro-phenyl]-6-oxo-4-(trifluoromethylsulfonyloxy)pyridazine-3-carboxylate (554 mg, 73.37% yield) was obtained as a yellow oil in the same manner as in Step 3 of Preparation Example 8. MS (ESI) m/z = 468.0 $[M+H]^+$.

Step 2: Synthesis of methyl-1-[3-(dimethylcarbamoyl)-2-fluoro-phenyl]-6-oxo-pyridazine-3-carboxylate

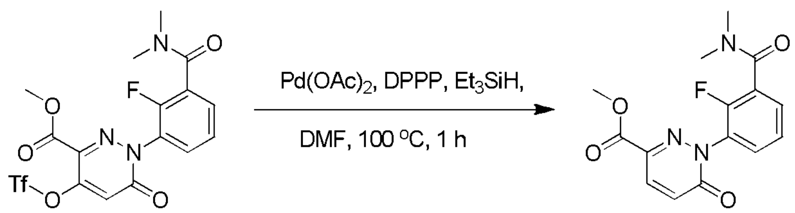


**Intermediate AC**

A mixture of methyl-1-[3-(dimethylcarbamoyl)-2-fluoro-phenyl]-6-oxo-4-(trifluoromethylsulfonyloxy)pyridazine-3-carboxylate (554 mg, 1.19 mmol), $Pd(OAc)_2$ (39.92 mg, 177.81 μmoL), DPPP (146.67 mg, 355.62 μmoL) and $Et_3SiH$ (179.19 mg, 1.54 mmol, 246.14 μL) in DMF (5 mL) was degassed and purged with $N_2$ for 3 times, and then the mixture was stirred at 100 °C for 1 hour under $N_2$ atmosphere. The reaction mixture was poured into water (30 mL) and extracted with EtOAc (30 mL × 3). The combined organic layer was washed with brine (30 mL × 2), dried over anhydrous $Na_2SO_4$ and concentrated under reduced pressure to obtain a residue. The residue was purified by flash silica gel chromatography (46% EtOAc in PE) to give Intermediate AC (215 mg, 50.51% yield) as yellow oil. MS (ESI) m/z = 320.0 $[M+H]^+$.

**Preparation Example 30: 5-(1-methylpyrazol-4-yl)-4-oxo-1H-pyrolo[2,3-d]pyridazine-7-carboxylic acid**

Steps 1 and 2: Synthesis of 4-hydroxy-1-(1-methyl-1H-pyrazol-4-yl)-6-oxo-1,6-dihydropyridazine-3-carboxylate

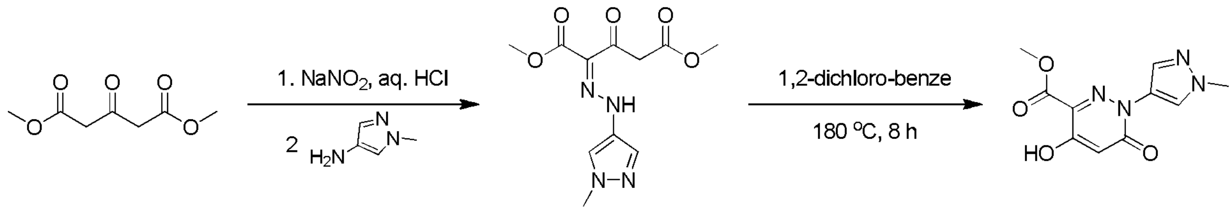


Methyl-4-hydroxy-1-(1-methyl-1H-pyrazol-4-yl)-6-oxo-1,6-dihydropyridazine-3-carboxylate was prepared in the same manner as in Steps 1 and 2 of Preparation Example 8 except that 1-methyl-1H-pyrazol-4-amine was used instead of aniline in Step 1 of Preparation Example 8. MS (ESI) m/z = 250.1 $[M+H]^+$.

Step 3: Synthesis of methyl-4-chloro-1-(1-methylpyrazol-4-yl)-6-oxo-pyridazine-3-carboxylate

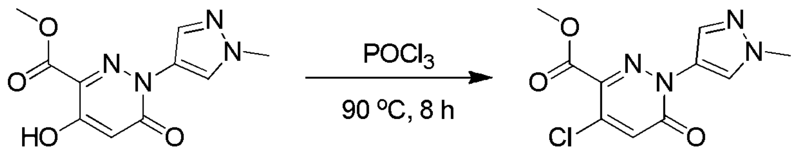


A mixture of methyl-4-hydroxy-1-(1-methylpyrazol-4-yl)-6-oxo-pyridazine-3-carboxylate (4.3 g, 17.19 mmol) in $POCl_3$ (50 mL) was degassed and purged with $N_2$ for 3 times, and then the mixture was stirred at 90 °C for 8 h under $N_2$ atmosphere. The reaction mixture was poured into water (100 mL) and extracted with EtOAc (100 mL × 3). The combined organic layer was washed with brine (100 mL), dried over anhydrous $Na_2SO_4$ and concentrated under reduced pressure to obtain a crude product. Methyl-4-chloro-1-(1-methylpyrazol-4-yl)-6-oxo-pyridazine-3-carboxylate (crude, 4.1 g, 82.21% yield) as a yellow solid was used in the next step without further purification. $^1H$ NMR (400 MHz, DMSO-$d_6$) $\delta$ = 8.36 (s, 1H), 7.86 (s, 1H), 7.51 (s, 1H), 3.91 (s, 3H), 3.89 (s, 3H); MS (ESI) m/z = 268.9 $[M+H]^+$.

Step 4: Synthesis of methyl-4-azido-1-(1-methylpyrazol-4-yl)-6-oxo-pyridazine-3-carboxylate

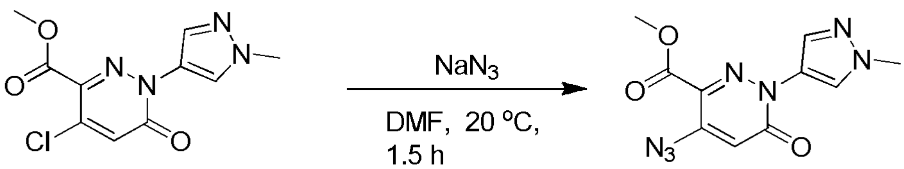


To a solution of methyl-4-chloro-1-(1-methylpyrazol-4-yl)-6-oxo-pyridazine-3-carboxylate (4.1 g, 15.26 mmol) in DMF (45 mL) was added $NaN_3$ (1.4 g, 21.54 mmol). The mixture was stirred at 20 °C for 5 h. The reaction mixture was adjusted to PH > 9 with aq. $Na_2CO_3$, and extrated with EtOAc (100 mL × 3). The combined organic layer was washed with brine (100 mL), dried over anhydrous $Na_2SO_4$ and concentrated under reduced pressure to obtain a crude product. The aqueous layer was added with ice-cold water (aqueous layer:water = 1:50) and adjusted to pH= 11 with aq. NaOH. Then, the aqueous layer was added dropwise with sat. aq. NaClO (50 mL) and stirred at 20 °C for 12 h. Methyl-4-azido-1-(1-methylpyrazol-4-yl)-6-oxo-pyridazine-3-carboxylate (crude, 4.2 g, 99.71% yield) as a yellow solid was used in the next step without further purification. MS (ESI) m/z = 275.9 $[M+H]^+$.

Step 5: Synthesis of methyl-4-amino-1-(1-methylpyrazol-4-yl)-6-oxopyridazine-3-carboxylate

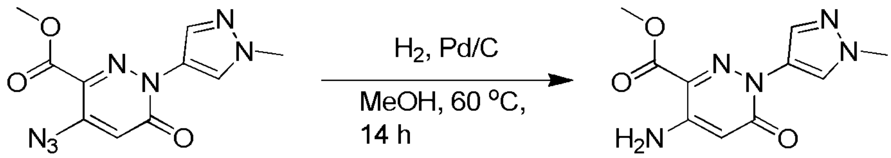


A mixture of methyl-4-azido-1-(1-methylpyrazol-4-yl)-6-oxo-pyridazine-3-carboxylate (4.2 g, 15.26 mmol) and Pd/C (1 g, 15.26 mmol, 10% purity) in MeOH (30 mL) and AcOH (30 mL) was degassed and purged with $H_2$ for 3 times, and then the mixture was stirred at 60 °C for 6 h under $H_2$ atmosphere. The mixture was filtrated and concentrated under reduced pressure to give methyl-4-amino-1-(1-methylpyrazol-4-yl)-6-oxopyridazine-3-carboxylate (4 g, 68.17% yield) as a yellow solid. $^1H$ NMR (400 MHz, DMSO-$d_6$) δ= 8.23 (s, 1H), 7.76 (s, 1H), 7.02 (br s, 2H), 5.85 (s, 1H), 3.87 (s, 6H); MS (ESI) m/z = 249.9 $[M+H]^+$.

Step 6: Synthesis of methyl-4-amino-5-iodo-1-(1-methylpyrazol-4-yl)-6-oxo-pyridazine-3-carboxylate

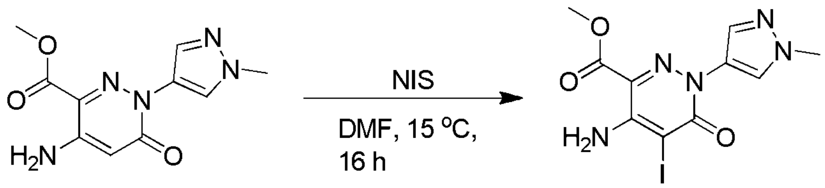


A mixture of methyl-4-amino-1-(1-methylpyrazol-4-yl)-6-oxo-pyridazine-3-carboxylate (4 g, 16.05 mmol), NIS (3.79 g, 16.85 mmol) in DMF (40 mL) was degassed and purged with $N_2$ for 3 times, and then the mixture was stirred at 20 °C for 3 h under $N_2$ atmosphere. The mixture was filtered to obtain a residue. The residue was triturated with DCM (50 mL) for 30 min at 20 °C. The mixture was filtered to give methyl-4-amino-5-iodo-1-(1-methylpyrazol-4-yl)-6-oxo-pyridazine-3-carboxylate (2.4 g, 38.07% yield) as an off-white solid. $^1H$ NMR (400 MHz, DMSO-$d_6$) δ = 8.23 (br s, 1H), 7.78 (br s, 1H), 7.08 (br s, 2H), 3.88 (br s, 6H); MS (ESI) m/z = 375.8 $[M+H]^+$.

Step 7: Synthesis of methyl-4-amino-1-(1-methylpyrazol-4-yl)-6-oxo-5-(2-trimethylsilylethynyl)pyridazine-3-carboxylate

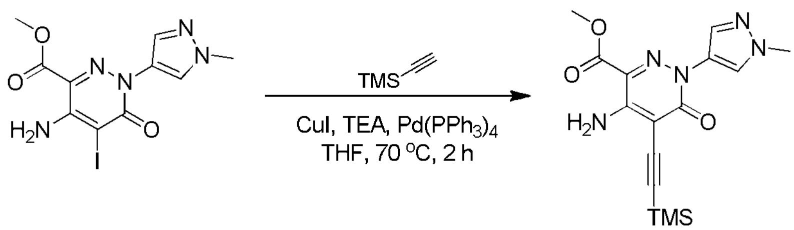


A mixture of methyl-4-amino-5-iodo-1-(1-methylpyrazol-4-yl)-6-oxo-pyridazine-3-carboxylate (900 mg, 2.40 mmol), ethynyl(trimethyl)silane (589.12 mg, 6.00 mmol), CuI (45.69 mg, 239.92 μmol), TEA (728.32 mg, 7.20 mmol, 1.00 mL) and $Pd(PPh_3)_2Cl_2$ (168.40 mg, 239.92 μmol) in THF (5 mL) was degassed and purged with $N_2$ for 3 times, and then the mixture was stirred at 70 °C for 2 h under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The crude product was purified by flash silica gel chromatography (30% EtOAc in PE) to give methyl-4-amino-1-(1-methylpyrazol-4-yl)-6-oxo-5-(2-trimethylsilylethynyl)pyridazine-3-carboxylate (700 mg, 74.25% yield) as a yellow solid. MS (ESI) m/z = 346.1 $[M+H]^+$.

Step 8: Synthesis of 5-(1-methylpyrazol-4-yl)-4-oxo-1H-pyrolo[2,3-d]pyridazine-7-carboxylic acid

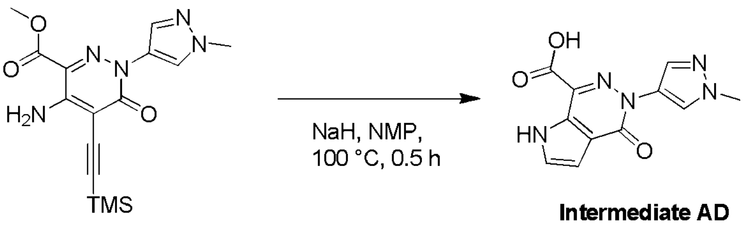


**Intermediate AD**

To a solution of methyl-4-amino-1-(1-methylpyrazol-4-yl)-6-oxo-5-(2-trimethylsilylethynyl)pyridazine-3-carboxylate (200 mg, 578.99 μmol) in NMP (2 mL) was added NaH (27.79 mg, 694.79 μmol) at 0 °C. The mixture was stirred at 100 °C for 0.5 hour under $N_2$ atmosphere. The reaction was quenched with EtOH slowly until no hydrogen was released. The reaction mixture was poured into water (30 mL) and extracted with EtOAc (30 mL × 3). The organic layer was discarded. The aqueous layer was adjusted to pH = 3-4 with aq.1 N HCl. The aqueous layer was purified by reversed-phase HPLC(10% MeOH in $H_2O$) to give Intermediate AD (110 mg, 65.52% yield) as a yellow solid. $^1H$ NMR (400 MHz, DMSO-$d_6$) δ = 11.83 (br s, 1H), 8.27 (br s, 1H), 7.89 (s, 1H), 7.40 (br s, 1H), 6.68 (br s,1H), 4.03 (q, *J* = 7.2 Hz, 1H), 3.88 (s, 3H); MS (ESI) m/z = 260.0 [M+H]+.

Also, the following Intermediate AD-1 was prepared in the same manner as for Intermediate AD.

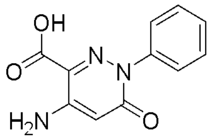

**Intermediate AD-1**

**Preparation Example 31: Methyl-4-oxo-5-phenyl-1H-pyrolo[2,3-d]pyridazine-7-carboxylate**

Steps 1 to 5: Synthesis of methyl-4-amino-6-oxo-1-phenyl-5-(2-trimethylsilylethynyl)pyridazine-3-carboxylate

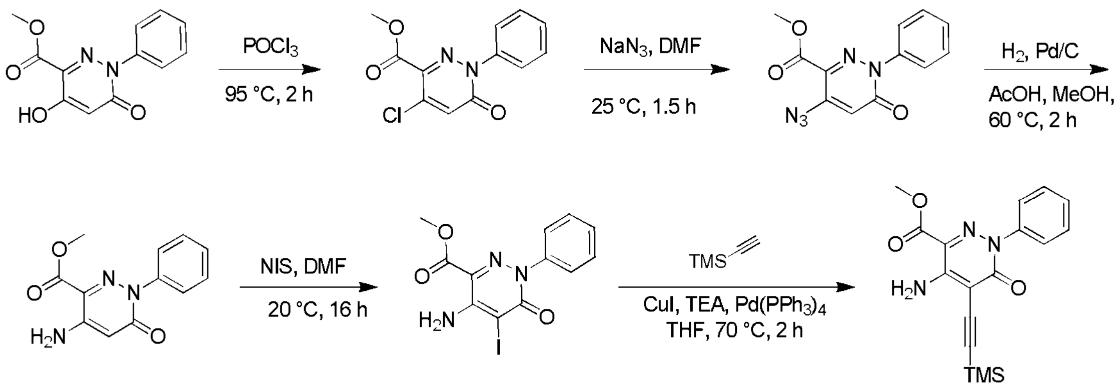


Methyl-4-amino-6-oxo-1-phenyl-5-(2-trimethylsilylethynyl)pyridazine-3-carboxylate was prepared in the same manner as in Steps 3 to 7 of Preparation Example 30, using methyl-4-hydroxy-6-oxo-1-phenyl-pyridazine-3-carboxylate obtained in Step 2 of Preparation Example 8 as a starting material.

Step 6: Synthesis of methyl-4-oxo-5-phenyl-1H-pyrolo[2,3-d]pyridazine-7-carboxylate

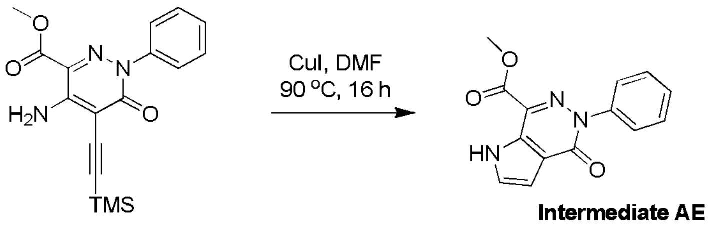


**Intermediate AE**

A mixture of methyl-4-amino-6-oxo-1-phenyl-5-(2-trimethylsilylethynyl)pyridazine-3-carboxylate (90 mg, 263.59 μmol) and CuI (25.10 mg, 131.80 μmol) in DMF (2 mL) was degassed and purged with $N_2$ for 3 times, and then the mixture was stirred at 100 °C for 16 h under $N_2$ atmosphere. The reaction mixture was poured into water (30 mL) and extracted with EtOAc (30 mL × 3). The combined organic layer was washed with brine (30 mL), dried over anhydrous $Na_2SO_4$ and concentrated under reduced pressure to obtain a crude product. The residue was purified by flash silica gel chromatography (30% EtOAc in PE) to give Intermediate AE (20 mg, 25.99% yield) as a yellow solid. MS (ESI) m/z = 270.1 $[M+H]^+$

**Preparation Example 32: methyl-5-(2-fluorophenyl)-4-oxo-1H-pyrolo[2,3-d]pyridazine-7-carboxylate**

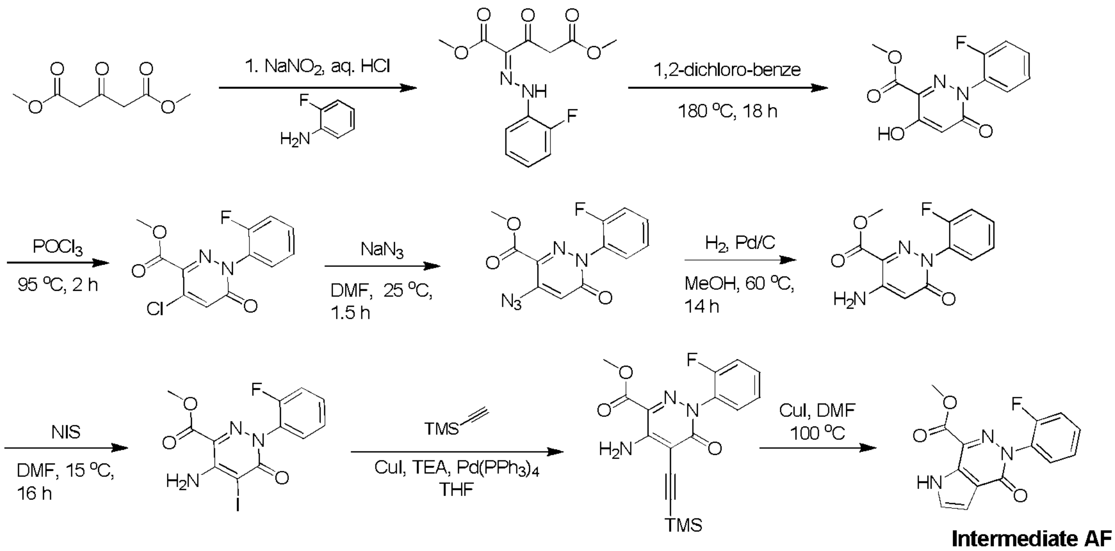


**Intermediate AF**

Intermediate AF (1.23 g, 58.08% yield) was prepared as a yellow solid in the same manner as in Preparation Example 30, using 2-fluoroaniline as a starting material. $^1H$ NMR (400MHz, CHLOROFORM-*d*) δ = 10.09 (s, 1H), 7.39-7.51 (m, 2H), 7.33 (t, *J* = 2.8 Hz, 1H), 7.26-7.30 (m, 1H), 7.20-7.25 (m, 1H), 6.99 (t, *J* = 2.8 Hz, 1H), 4.02 (s, 3H); MS (ESI) m/z = 165.1 $[M+H]^+$.

**Preparation Example 33: Ethyl-4-oxo-5-phenyl-thieno[2,3-d]pyridazine-7-carboxylate**

Step 1: Synthesis of ethyl 2-(2-ethoxy-2-oxo-ethyl)thiophene-3-carboxylate

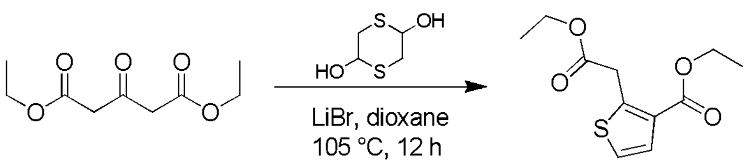


To a solution of 1,4-dithiane-2,5-diol (5.0 g, 32.84 mmol) and diethyl 3-oxopentanedioate (19.92 g, 98.53 mmol) in dioxane (50 mL) was added LiBr (855.75 mg, 9.85 mmol). The mixture was stirred at 105 °C for 12 h. The mixture was concentrated under reduced pressure to obtain a residue. The residue was purified by silica gel column chromatography

(10% EtOAc in PE) to give ethyl 2-(2-ethoxy-2-oxo-ethyl)thiophene-3-carboxylate (4.74 g, 59.56% yield) as colorless oil. $^1$H NMR (400 MHz, CHLOROFORM-d) δ = 7.45 (d, *J* =5.6 Hz, 1H), 7.14 (d, *J* = 5.6 Hz, 1H), 4.30 (q, *J*=7.2 Hz, 2H), 4.22-4.16 (m, 4H), 1.35 (t, *J* = 7.2 Hz, 3H), 1.27 (t, *J* = 7.2 Hz, 3H).

Step 2: Synthesis of ethyl 2-(2-ethoxy-2-oxoacetyl)thiophene-3-carboxylate

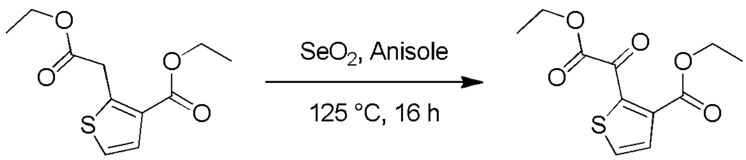


To a solution of ethyl 2-(2-ethoxy-2-oxo-ethyl)thiophene-3-carboxylate (2.0 g, 8.25 mmol) in anisole (50 mL) was added $SeO_2$ (2.29 g, 20.64 mmol). The mixture was stirred at 125 °C for 16 h. The mixture was filtrated, and the filtrate was concentrated under reduced pressure to obtain a residue. The residue was purified by silica gel column chromatography (10% EtOAc in PE) to obtain a crude product. The product was purified by silica gel column chromatography (10% EtOAc in PE) to give ethyl 2-(2-ethoxy-2-oxoacetyl)thiophene-3-carboxylate (840 mg, 39.71% yield) as yellow oil. $^1$H NMR (400 MHz, CHLOROFORM-d) δ = 7.64 (d, *J* = 5.2 Hz, 1H), 7.47 (d, *J* = 5.2 Hz, 1H), 4.41-4.35 (m, 2H), 4.35-4.29 (m, 2H), 1.38 (td, *J*=7.2, 14.0 Hz, 6H).

Step 3: Synthesis of ethyl-4-oxo-5H-thieno[2,3-d]pyridazine-7-carboxylate

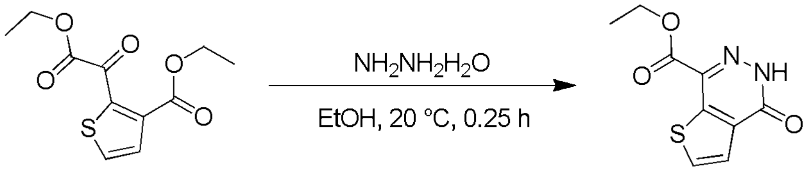


To a solution of ethyl 2-(2-ethoxy-2-oxo-acetyl)thiophene-3-carboxylate (640 mg, 2.50 mmol) in EtOH (7 mL) was added $NH_2NH_2 \cdot H_2O$ (160 mg, 3.13 mmol). The mixture was stirred at 20 °C for 0.25 hour. The reaction mixture was filtrated to obtain the filter cake. The product was purified by silica gel column chromatography (10% EtOAc in DCM) to give ethyl-4-oxo-5H-thieno[2,3-d]pyridazine-7-carboxylate (180 mg, 32.14% yield) as a white solid. $^1$H NMR (400 MHz, DMSO-$d_6$) δ = 13.95-12.84 (m, 1H), 8.16 (d, *J*=5.4 Hz, 1H), 7.66 (d, *J*=5.2 Hz, 1H), 4.40 (q, *J*=7.2 Hz, 2H), 1.36 (t, *J*=7.2 Hz, 3H); MS (ESI) m/z = 225.0 $[M+H]^+$.

Step 4: Synthesis of ethyl-4-oxo-5-phenyl-thieno[2,3-d]pyridazine-7-carboxylate

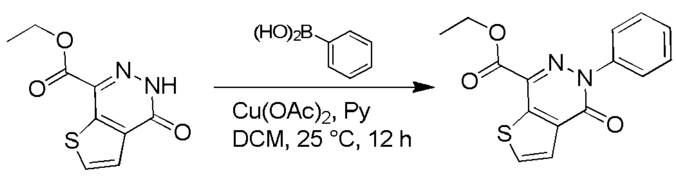


**Intermediate AG**

To a solution of ethyl-4-oxo-5H-thieno[2,3-d]pyridazine-7-carboxylate (180 mg, 802.73 μmol) and phenylboronic acid (146.81 mg, 1.20 mmol) in DCM (4 mL), pyridine (380.97 mg, 4.82 mmol) and $Cu(OAc)_2$ (29.16mg, 160.55 μmol) were added. The mixture was stirred at 25 °C for 12 h under air. The mixture was concentrated under reduced pressure to obtain a residue. The residue was purified by silica gel column chromatography (100% EtOAc in PE) to give Intermediate AG (84 mg, 34.84% yield) as yellow oil. $^1$H NMR (400 MHz, DMSO-$d_6$) δ = 8.24 (d, *J* = 5.2 Hz, 1H), 7.74 (d, *J* = 5.2 Hz, 1H), 7.62-7.53 (m, 4H), 7.53-7.47 (m, 1H), 7.42-7.36 (m, 1H), 7.18-7.12 (m, 1H), 4.42 (q, *J* = 7.2 Hz, 2H), 1.34 (t, *J* = 7.2 Hz, 3H); MS (ESI) m/z = 300.9 $[M+H]^+$.

Also, the following Intermediate AG-1 was prepared in the same manner as for Intermediate AG.

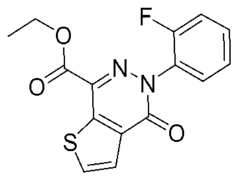

**Intermediate AG-1**

**Preparation Example 34: Ethyl-6-(2-fluorophenyl)-7-oxo-thieno[2,3-d]pyridazine-4-carboxylate**

Step 1: Synthesis of methyl 3-(2-ethoxy-2-oxo-acetyl)thiophene-2-carboxylate

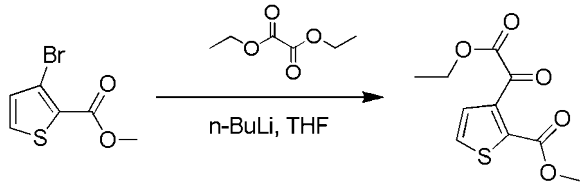


To a mixture of methyl 3-bromothiophene-2-carboxylate (5 g, 22.62 mmol) in THF (100 mL) were added n-BuLi (2.5 M, 9.95 mL) at -78 °C under $N_2$ atmosphere. After 10 minutes, the mixture was added wtih methyl 3-bromothiophene-2-carboxylate (5 g, 22.62 mmol) and diethyl oxalate (9.92 g, 67.85 mmol) in THF (100 mL) at -20 °C. After 10 minutes, the cold bath was removed and the reaction mixture was warmed to 15 °C. The mixture was diluted with sat. aq. $NH_4Cl$ (50 mL) and extracted with EtOAc (100 mL × 3). The combined organic layer was washed with brine (100 mL), dried over anhydrous $Na_2SO_4$ and concentrated under reduced pressure to obtain a residue. The residue was purified by silica gel column (PE/EtOAc = 10/1) to give methyl 3-(2-ethoxy-2-oxo-acetyl)thiophene-2-carboxylate (1.1 g) as yellow oil. $^1H$ NMR (400 MHz, DMSO-$d_6$) $\delta$ = 8.17 (d, $J$ = 5.2 Hz, 1H), 7.65 (d, $J$ = 5.2 Hz, 1H), 4.25 (s, 2H), 3.80 (s, 3H), 1.28 (s, 3H).

Step 2: Synthesis of ethyl-6-(2-fluorophenyl)-7-oxo-thieno[2,3-d]pyridazine-4-carboxylate

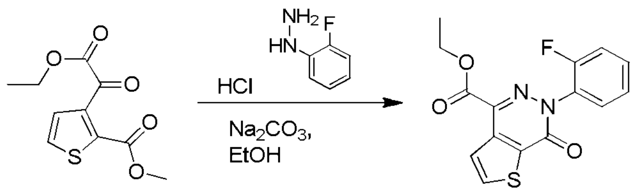


**Intermediate AH**

A mixture of methyl 3-(2-ethoxy-2-oxo-acetyl)thiophene-2-carboxylate (600 mg), (2-fluorophenyl)hydrazine (374.88 mg, 2.97 mmol) and $Na_2CO_3$ (525.03 mg, 4.95 mmol) in EtOH (15 mL) was degassed and purged with $N_2$ for 3 times, and then the mixture was stirred at 20 °C for 2 h under $N_2$ atmosphere. The mixture was concentrated under reduced pressure to obtain a residue. The residue was purified by prep-HPLC (column: Phenomenex C18 75*30mm*3um;mobile phase: [water($NH_3H_2O$+$NH_4HCO_3$)-ACN];B%: 33%-63%,10min). The desired fraction was concentrated under reduced pressure, and the remaining solvent was removed by lyophilization to give Intermediate AH (54 mg, 13.47% 2-steps yield) as a yellow solid. MS (ESI) m/z = 319.1 $[M+H]^+$.

**Preparation Example 35: 1-(difluoromethyl)-5-iodo-pyrazole**

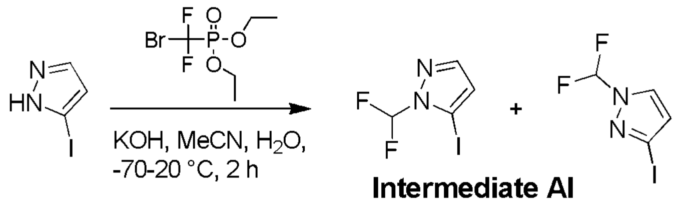


To a mixture of 5-iodo-1H-pyrazole (1.21 g, 6.24 mmol) and KOH (4.20 g, 74.90 mmol) in MeCN (10 mL)/$H_2O$ (10 mL) was added 1-[[bromo(difluoro)methyl]-ethoxy-phosphoryl]oxyethane (5 g, 18.73 mmol) at -70 °C. Then, the mixture was stirred at 20°C for 2 h under $N_2$. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by silica gel column chromatography (3% EtOAc in PE) to give Intermediate AI (0.54 g, 34.71% yield) as a colorless oil. $^1$H NMR (400 MHz, DMSO-$d_6$) δ ppm 6.76 (d, *J* =2.6 Hz, 1 H) 6.79 (d, *J* = 1.6 Hz, 1 H) 7.64 (s, 1 H) 7.70 (s, 1 H) 7.79 (s, 1 H) 7.80 (m, 1 H) 7.85 (s, 1 H) 7.94 (s, 1 H) 7.99 (s, 1 H) 8.15 (d, *J* =2.6 Hz, 1 H); MS (ESI) m/z = 244.9 [M+1+H]$^+$.

**Preparation Example 36: (R)-1-(3-(difluoromethyl)-5-nitrophenyl)ethanamine**

Step 1: Synthesis of 1-bromo-3-(difluoromethyl)-5-nitro-benzene

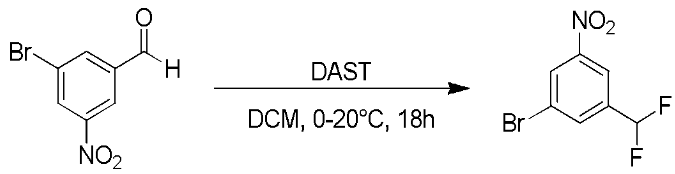


A mixture of 3-bromo-5-nitro-benzaldehyde (13.7 g, 59.56 mmol) and DAST (48.00 g, 297.81 mmol, 39.35 mL) in DCM (140 mL) was degassed and purged with $N_2$ for 3 times, stirred at 0-20 °C for 18 h, and then the mixture was stirred at 0-20 °C for 18 h under $N_2$ atmosphere. The resulting solution was poured over ice and extracted with dichloromethane (300 mL). Then, the reaction mixture was extracted with EtOAc (200 mL×3). The combined organic layer was washed with brine (200 mL×2), dried over anhydrous $Na_2SO_4$ and concentrated under reduced pressure to obtain a crude product. The product was purified by silica gel column chromatography (8% EtOAc in PE) to give 1-bromo-3-(difluoromethyl)-5-nitro-benzene (14.27 g, 56.62 mmol, 95.07% yield) as colorless oil. $^1$H NMR (400MHz, DMSO-$d_6$) δ = 8.57 (s, 1H), 8.40 (s, 1H), 8.29 (s, 1H), 7.34 - 7.05 (m, 1H)

Steps 2 to 5: Synthesis of (R)-1-(3-(difluoromethyl)-5-nitrophenyl)ethanamine

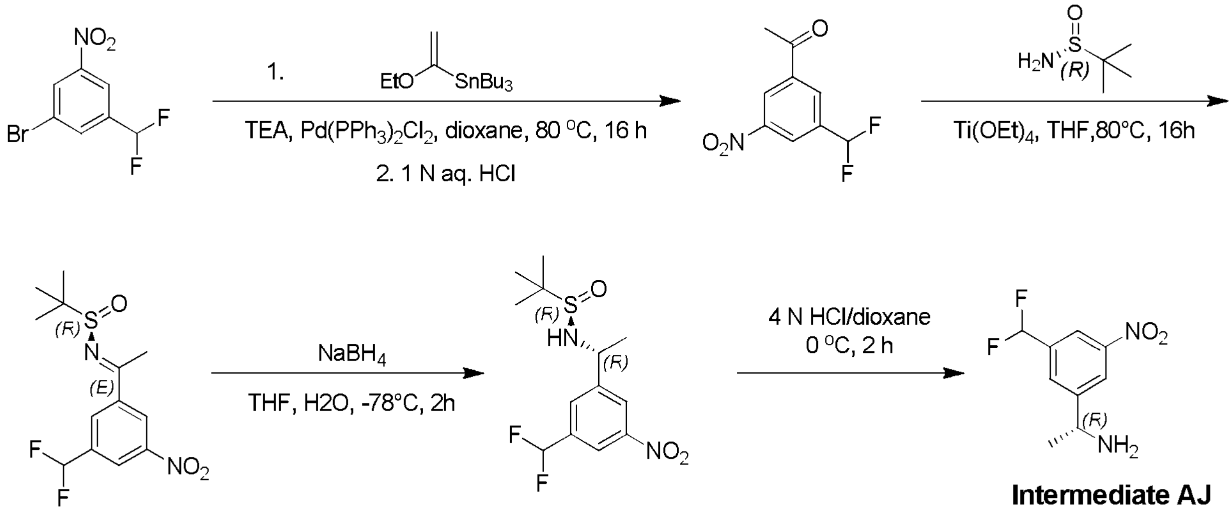


**Intermediate AJ**

Intermediate AJ was prepared in the same manner as in Preparation Example 13, using 1-bromo-3-(difluoromethyl)-5-nitro-benzene. MS (ESI) m/z = 216.1 [M+H]+.

**Preparation Example 37: Methyl-1-(2-fluorophenyl)-4-hydroxy-6-oxo-1,6-**dihydropyridazine-3-carboxylate

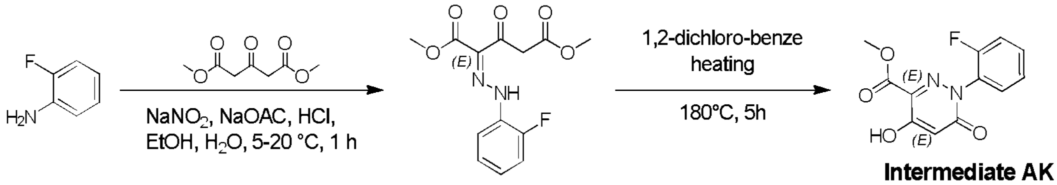


**Intermediate AK**

Intermediate AK was prepared in the same manner as in Steps 1 and 2 of Preparation Example 8, using 2-fluoroaniline instead of aniline. MS (ESI) m/z = 264.1 [M+H]+.

**Preparation Example 38: methyl-1-(2-fluoro-4-methoxyphenyl)-6-oxo-4-(((trifluoromethyl)sulfonyl)oxy)-1,6-dihydropyridazine-3-carboxylate**

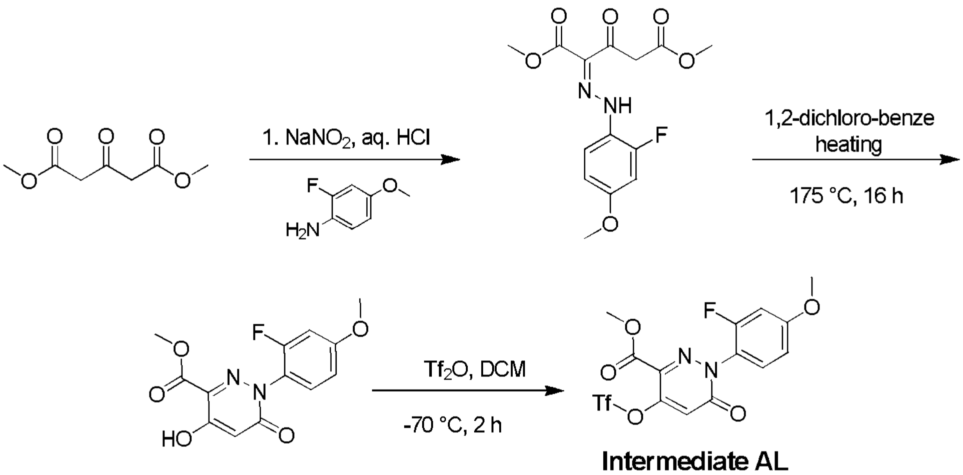


**Intermediate AL**

Intermediate AL was prepared in the same manner as in Preparation Example 8, using 2-fluoro-4-methoxyaniline. MS (ESI) m/z = 426.0 [M+H]+.

**Preparation Example 39: methyl-4-amino-1-(2-fluorophenyl)-6-oxo-1,6-dihydropyridazine-3-carboxylate**

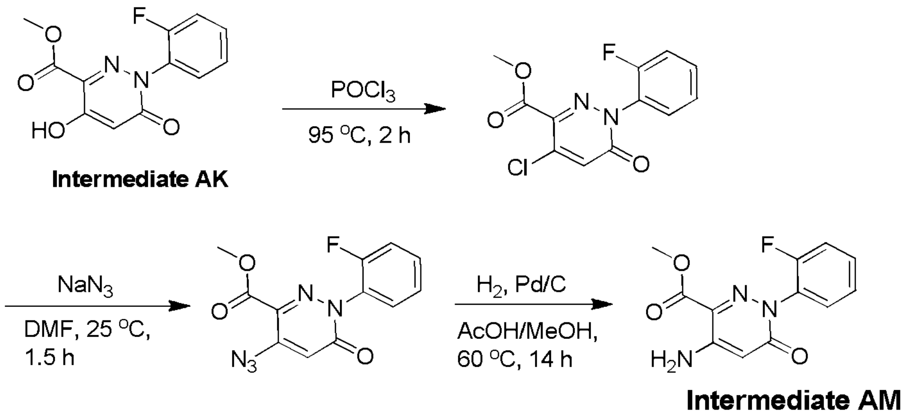


Intermediate AK

Intermediate AM

Intermediate AM was prepared in the same manner as in Steps 3 to 5 of Preparation Example 30. MS (ESI) m/z = 246.1 $[M+H]^+$

**Preparation Example 40: Methyl-5-amino-6-oxo-1-phenyl-pyridazine-3-carboxylate**

Step 1: Synthesis of methyl-5-(tert-butoxycarbonylamino)-6-oxo-1-phenyl-pyridazine-3-carboxylate

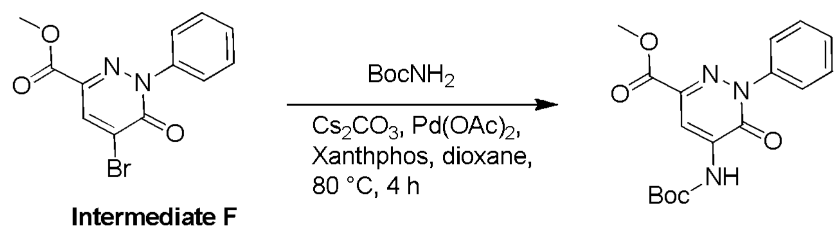


Intermediate F

A mixture of Intermediate F (200.00 mg, 647.01 μmol), tert-butyl carbamate (151.59 mg, 1.29 mmol), $Pd(OAc)_2$ (7.26 mg, 32.35 μmol), Xantphos (56.16 mg, 97.05 μmol) and $Cs_2CO_3$ (421.62 mg, 1.29 mmol) in dioxane (3 mL) was degassed and purged with $N_2$ for 3 times, and then the mixture was stirred at 80 °C for 4 h under $N_2$ atmosphere. The reaction mixture was poured into water (10 mL) and extracted with EtOAc (20 mL × 3). The combined organic layer was washed with brine (30 mL), dried over anhydrous $Na_2SO_4$ and concentrated under reduced pressure to obtain a crude product. The crude product was purified by flash silica gel chromatography (9% EtOAc in PE) to give methyl-5-(tert-butoxycarbonylamino)-6-oxo-1-phenyl-pyridazine-3-carboxylate (120 mg, 44.49% yield) as a yellow oil. MS (ESI) m/z = 346.0 $[M+H]^+$.

Step 2: Synthesis of methyl-5-amino-6-oxo-1-phenyl-pyridazine-3-carboxylate

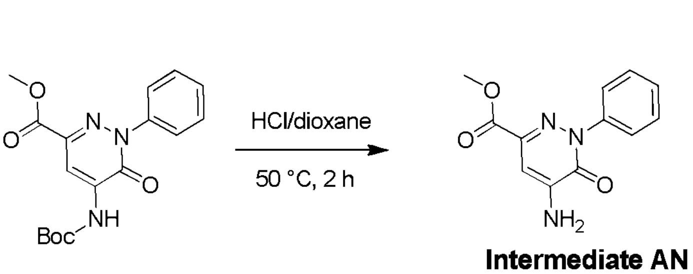


Intermediate AN

A mixture of methyl-5-(tert-butoxycarbonylamino)-6-oxo-1-phenyl-pyridazine-3-carboxylate (120.00 mg, 347.47 μmol) in HCl/dioxane (3 mL) was stirred at 50 °C for 2 h under air. The mixture was concentrated under reduced

pressure to obtain a crude product. Intermediate AN (crude, 110 mg, 95.06% yield, HCl) as a yellow solid was used in the next step without further purification. MS (ESI) m/z = 246.0 $[M+H]^+$.

**Preparation Example 41: Methyl-5-methyl-6-oxo-1-phenylpyridazine-3-carboxylate**

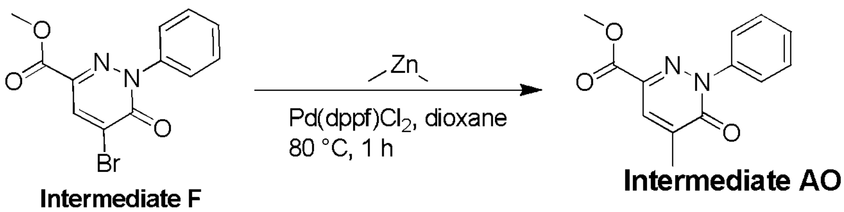


**Intermediate F** **Intermediate AO**

A mixture of Intermediate F (200 mg, 647.01 μmol), dimethylzinc (30.88 mg, 323.51 μmol) and $Pd(dppf)Cl_2$ (94.68 mg, 129.40 μmol) in dioxane (3 mL) was degassed and purged with $N_2$ for 3 times, and then the mixture was stirred at 80 °C for 1 hour under $N_2$ atmosphere. The reaction mixture was poured into water (10 mL) and extracted with EtOAc (20 mL × 3). The combined organic layer was washed with brine (30 mL), dried over anhydrous $Na_2SO_4$ and concentrated under reduced pressure to obtain a crude product. The crude product was purified by flash silica gel chromatography (10% EtOAc in PE) to give Intermediate AO (55 mg, 32.89% yield) as a brown solid. MS (ESI) m/z = 245.0 $[M+H]^+$.

**Preparation Example 42: methyl-4-[tert-butoxycarbonyl(methyl)amino]-1-(2-fluorophenyl)-6-oxo-pyridazine-3-carboxylate**

Step 1: Synthesis of methyl-4-(tert-butoxycarbonylamino)-1-(2-fluorophenyl)-6-oxo-pyridazine-3-carboxylate

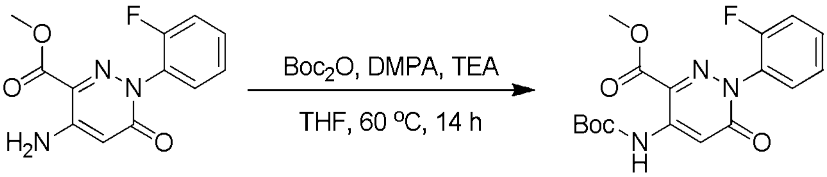


**Intermediate AM**

A mixture of Intermediate AM (300 mg, 1.14 mmol), DMAP (69.62 mg, 569.86 μmol), TEA (172.99 mg, 1.71 mmol) and $Boc_2O$ (298.49 mg, 1.37 mmol) in THF (2 mL) was stirred at 60 °C for 14 h. The mixture was diluted with water (50 mL) and extracted with EtOAc (50 mL × 3). The combined organic layer was washed with brine (50 mL), dried over anhydrous $Na_2SO_4$ and concentrated under reduced pressure to give methyl-4-(tert-butoxycarbonylamino)-1-(2-fluorophenyl)-6-oxo-pyridazine-3-carboxylate (558 mg, crude) as yellow oil. MS (ESI) m/z = 364.1 $[M+H]^+$

Step 2: Synthesis of methyl-4-[tert-butoxycarbonyl(methyl)amino]-1-(2-fluorophenyl)-6-oxo-pyridazine-3-carboxylate

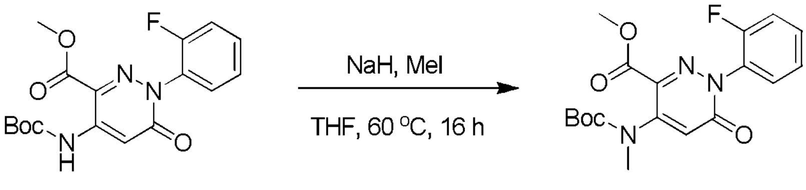


**Intermediate AP**

To a mixture of methyl-4-(tert-butoxycarbonylamino)-1-(2-fluorophenyl)-6-oxo-pyridazine-3-carboxylate (558 mg, crude) in THF (10 mL) wase added NaH (184.27 mg, 4.61 mmol, 60% purity) at 0 °C for 0.5 hour under $N_2$ atmosphere, then MeI (1.09 g, 7.68 mmol) was added thereto, and the mixture was stirred at 60 °C for 16 h under $N_2$ atmosphere. The

mixture was diluted with water (50 mL) and extracted with EtOAc (50 mL × 3). The combined organic layer was washed with brine (50 mL), dried over anhydrous $Na_2SO_4$ and concentrated under reduced pressure to obtain a residue. The residue was purified by silica gel column (PE/EtOAc = 5/1) to give Intermediate AP (220 mg, 51.14% 2-steps yield) as a yellow solid. $^1H$ NMR (400MHz, DMSO-$d_6$) δ = 7.55-7.62 (m, 2H), 7.36-7.48 (m, 2H), 7.12 (s, 1H), 3.78 (s, 3H), 3.26 (s, 3H), 1.40 (s, 9H); MS (ESI) m/z = 378.2 [M+H]$^+$.

**Preparation Example 43: 1-(2,3-difluorophenyl)-6-oxo-1,6-dihydropyridine-3-carboxylic acid**

Step 1: Synthesis of methyl-1-(2,3-difluorophenyl)-6-oxo-1,6-dihydropyridine-3-carboxylate

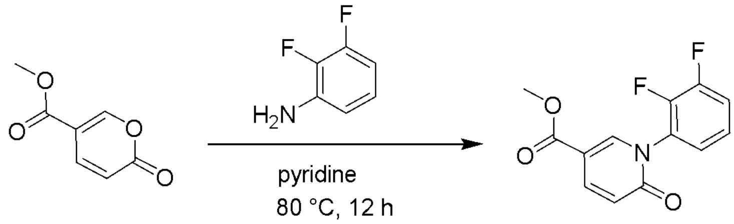


A mixture of methyl 2-oxo-2H-pyran-5-carboxylate (100 mg, 0.65 mmol) and 2,3-difluoroaniline (84 mg, 0.65 mmol) in pyridine (2 mL) was stirred at 80 °C for overnight. The mixture was added with DW and extracted with EtOAc. The organic layer was washed with water and 1N HCl, dried over $MgSO_4$, filtered and concentrated under reduced pressure to obtain a crude product. Methyl-1-(2,3-difluorophenyl)-6-oxo-1,6-dihydropyridine-3-carboxylate (51 mg, crude) was obtained as a yellow solid and used in the next step without further purification. LC/MS (ESI) m/z = 266.1 [M+H]$^+$.

Step 2: Synthesis of 1-(2,3-difluorophenyl)-6-oxo-1,6-dihydropyridine-3-carboxylic acid

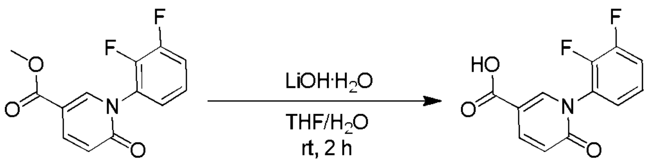


**Intermediate AQ**

To a solution of methyl-1-(2,3-difluorophenyl)-6-oxo-1,6-dihydropyridine-3-carboxylate (90mg, 0.34mmol) in THF (4 mL) and $H_2O$ (2 mL) was added LiOH·$H_2O$ (35.6 mg, 0.84 mmol). The mixture was stirred at room temperature for 2 h. The reaction mixture was poured into water and extracted with EtOAc. The aqueous layer was adjusted to pH = 3-4 with aq.1 N HCl and then extracted with EtOAc (10 mL × 2). The organic layer was washed with water (20 mL) and brine (20 mL), dried over $MgSO_4$, filtered and concentrated under reduced pressure to give Intermediate AQ (43.9 mg, 51.5% yield) as a white solid. LC/MS (ESI) m/z = 266.1 [M+H]$^+$.

Also Intermediates AQ-1, AQ-2, AQ-3, and AQ-4 were prepared in the same manner as for Intermediate AQ.

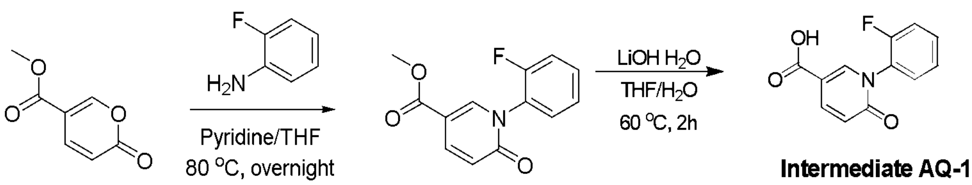


**Intermediate AQ-1**

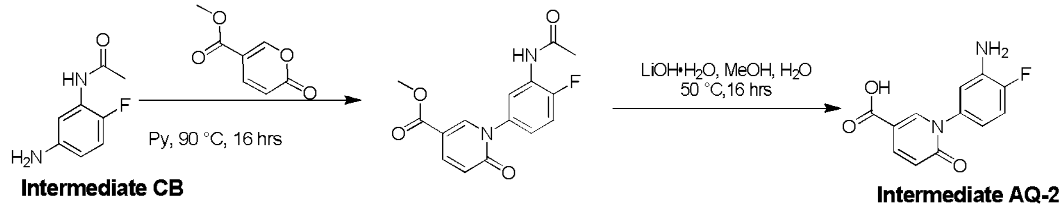


Intermediate CB

Intermediate AQ-2

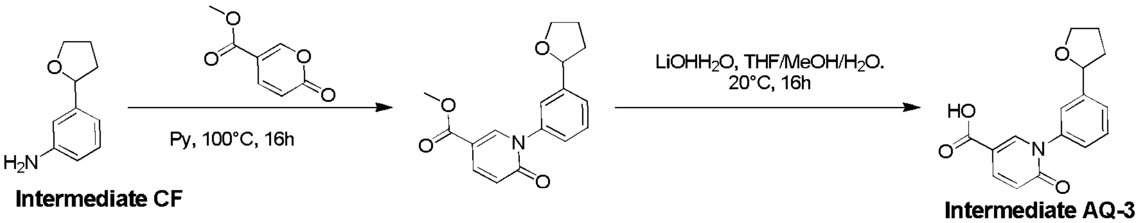


Intermediate CF

Intermediate AQ-3

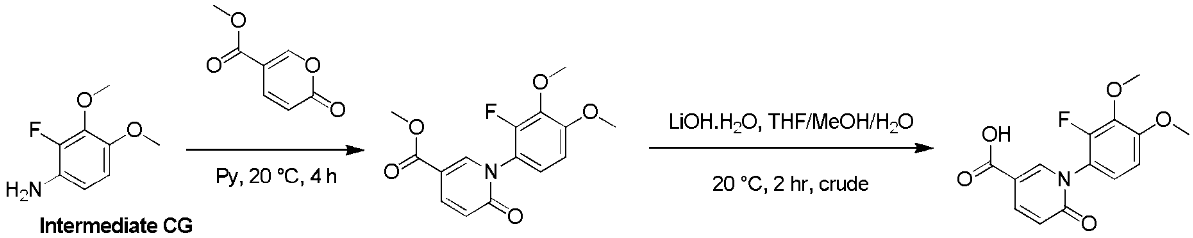


Intermediate CG

Intermediate AQ-4

**Preparation Example 44: 1-(1-acetylpiperidin-4-yl)-6-oxo-1,6-dihydropyridazine-3-carboxylic acid**

Step 1: Synthesis of methyl-1-(1-acetyl-4-piperidyl)-6-oxo-pyridazine-3-carboxylate

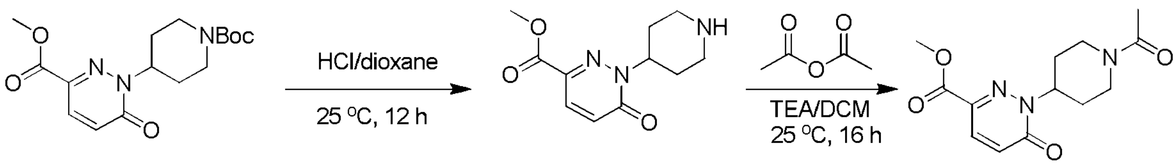


Intermediate G-3

The Boc group of Intermediate G-3 was deprotected. Subsequently, a mixture of methyl-6-oxo-1-(4-piperidyl) pyridazine-3-carboxylate (100 mg, 421.49 μmol), $Ac_2O$ (60.00 mg, 587.73 μmol) and TEA (127.95 mg, 1.26 mmol) in DCM (6 mL) was degassed and purged with $N_2$ for 3 times, and then stirred at 25 °C for 16 h under $N_2$ atmosphere. The reaction mixture was poured into water (30 mL) and extracted with EtOAc (30 mL × 3). The combined organic layer was washed with brine (30 mL × 2), dried over anhydrous $Na_2SO_4$ and concentrated under reduced pressure to obtain a crude product. The product was purified by silica gel column chromatography (97% EtOAc in PE) to give methyl-1-(1-acetyl-4-piperidyl)-6-oxo-pyridazine-3-carboxylate (184.9 mg, 63.16% yield) as a white solid. MS (ESI) m/z = 280.0 $[M+H]^+$

Step 2: Synthesis of 1-(1-acetylpiperidin-4-yl)-6-oxo-1,6-dihydropyridazine-3-carboxylic acid

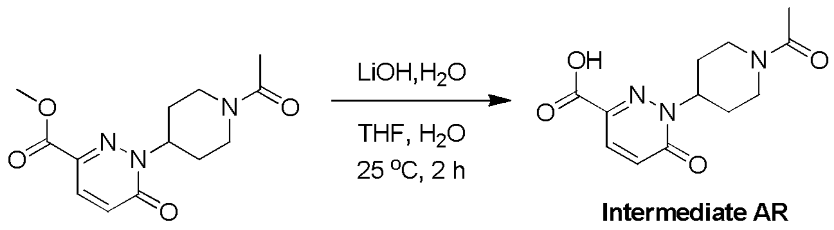


Intermediate AR was prepared in the same manner as in Step 2 of Preparation Example 43.

**Preparation Example 45: 1-(2-fluorophenyl)-6-oxo-1,6-dihydropyridazine-3-carboxylic acid**

Step 1: Synthesis of dimethyl (2Z)-2-(phenylhydrazono)pentanedioate

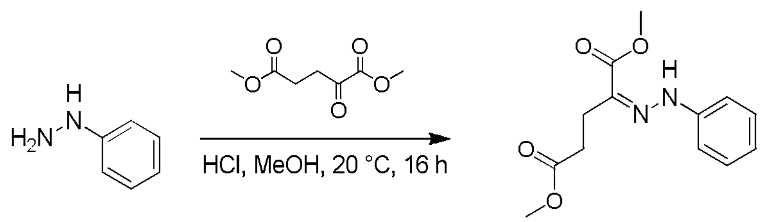


A mixture of dimethyl 2-oxopentanedioate (200 mg, 1.15 mmol), phenylhydrazine (124.36 mg, 1.15 mmol, 113.06 μL) and HCl (23.29 mg, 230.00 μmol, 22.84 μL) in MeOH (2 mL) was degassed and purged with $N_2$ for 3 times, and then the mixture was stirred at 20 °C for 16 h under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The residue was purified by flash silica gel chromatography (12% EtOAc in PE) to give dimethyl (2Z)-2-(phenylhydrazono)pentanedioate (400 mg, 64.88% yield) as a yellow oil. $^1$H NMR (400 MHz, DMSO-$d_6$) δ = 8.36 (s, 1H), 7.86 (s, 1H), 7.51 (s, 1H), 3.91 (s, 3H), 3.89 (s, 3H); MS (ESI) m/z = 265.0 $[M+H]^+$.

Step 2: Synthesis of 6-oxo-1-phenyl-4,5-dihydropyridazine-3-carboxylic acid

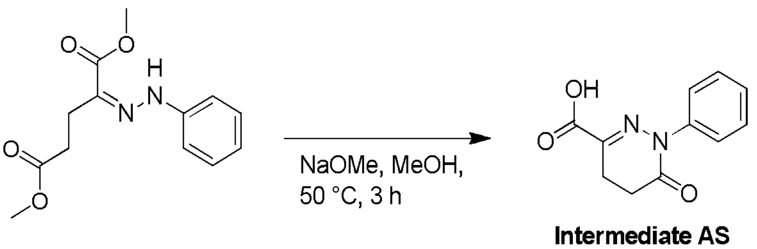


A mixture of dimethyl (2Z)-2-(phenylhydrazono)pentanedioate (270 mg, 1.02 mmol) and NaOMe (66.23 mg, 1.23 mmol) in MeOH (1 mL) was degassed and purged with $N_2$ for 3 times, and then the mixture was stirred at 50 °C for 3 h under $N_2$ atmosphere. The reaction mixture was poured into water (30 mL) and extracted with EtOAc (30 mL × 3). The aqueous layer was adjusted to pH = 3-4 with aq.1 N HCl and then extracted with EtOAc (30 mL × 3). The organic layer was washed with water (30 mL) and brine (30 mL), dried over $Na_2SO_4$, filtered and concentrated under reduced pressure to obtain a residue. Intermediate AS (100 mg, 13.80% yield) was obtained as yellow oil and used in the next step without further purification. MS (ESI) m/z = 218.9 $[M+H]^+$.

**Preparation Example 46: methyl-6-oxo-1-[3-(5-trimethylsilylisoxazol-3-yl)phenyl]pyridazine-3-carboxylate**

Step 1: Synthesis of methyl-1-(3-formylphenyl)-6-oxo-1,6-dihydropyridazine-3-carboxylate

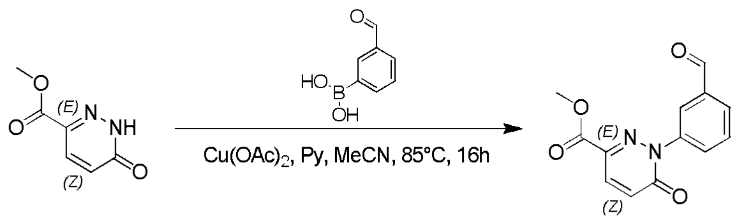


Methyl-1-(3-formylphenyl)-6-oxo-1,6-dihydropyridazine-3-carboxylate was prepared in the same manner as in Preparation Example 11. MS (ESI) m/z = 258.1 $[M+H]^+$.

Step 2: Synthesis of methyl-1-[3-(hydroxyiminomethyl)phenyl]-6-oxo-pyridazine-3-carboxylate

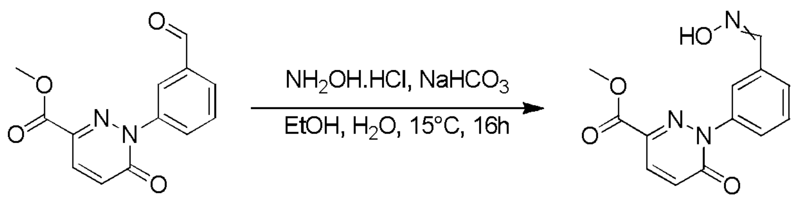


To a solution of $NaHCO_3$ (39.04 mg, 464.70 μmol, 18.07 μL) in $H_2O$ (5 mL) was added $NH_2OH.HCl$ (32.29 mg, 464.70 μmol). The resulting solution was then added to a vigorously stirred suspension of methyl-1-(3-formylphenyl)-6-oxo-pyridazine-3-carboxylate (100 mg, 387.25 μmol) in EtOH (5 mL) at 15 °C for 15 h. The product was filtered. Then, the filtrate was poured into water (20 mL) and extracted with EtOAc (20 mL×4). The combined organic layer was washed with brine (520 mL×2), dried over anhydrous $Na_2SO_4$ and concentrated under reduced pressure to give methyl-1-[3-(hydroxyiminomethyl)phenyl]-6-oxo-pyridazine-3-carboxylate (crude, 100 mg, 317.70 μmol, 82.04% yield). MS (ESI) m/z = 273.9 $[M+H]^+$.

Step 3: Synthesis of methyl-6-oxo-1-[3-(5-trimethylsilylisoxazol-3-yl)phenyl]pyridazine-3-carboxylate

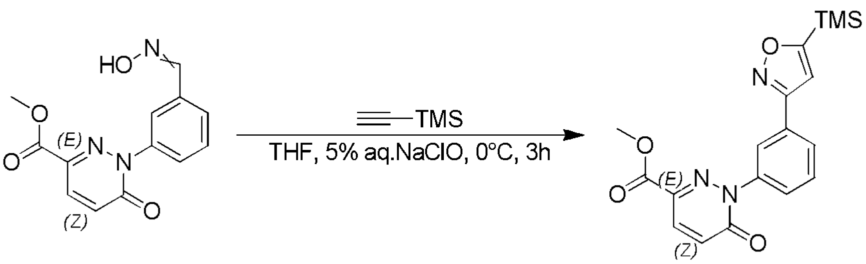


**Intermediate AT**

To a solution of ethynyl(trimethyl)silane (107.84 mg, 1.10 mmol, 152.10 μL) and NaClO (0.6 mL, 5% purity) in THF (1 mL) was added a solution of methyl-1-[3-(hydroxyiminomethyl)phenyl]-6-oxo-pyridazine-3-carboxylate (100 mg, 365.97 μmol) obtained in THF (1 mL) at 0°C, and the mixture was stirred for 3 h. The reaction mixture was poured into water (50 mL) and extracted with EtOAc (50 mL×3). The combined organic layer was washed with brine (30 mL×2), dried over anhydrous $Na_2SO_4$ and concentrated under reduced pressure to obtain a crude product. The residue was purified by flash silica gel chromatography (18% EtOAc in PE) to give Intermediate AT (35 mg, 76.38 μmol, 20.87% yield) as a white solid. MS (ESI) m/z = 370.1 $[M+H]^+$.

**Preparation Example 47: 2-[(1S)-1-aminomethyl]-6-(trifluoromethyl)pyridin-4-amine and 2-[(1R)-1-aminomethyl]-6-(trifluoromethyl)pyridin-4-amine**

Step 1: Synthesis of 1-[4-amino-6-(trifluoromethyl)-2-pyridyl]ethanone

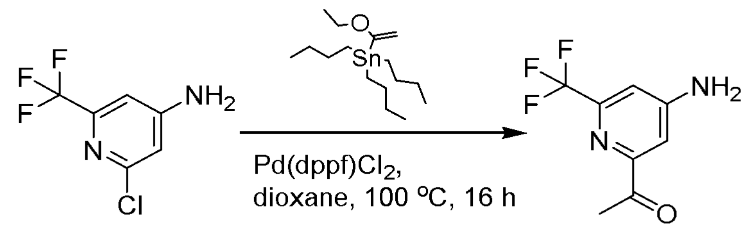


1-tributyl(1-ethoxyvinyl)stannane (2.23 g, 2.09 mmol) and Pd(dppf)$Cl_2$ (93.1 mg, 127 μmol) were added to a mixture of 2-chloro-6-(trifluoromethyl)pyridin-4-amine (500 mg, 2.54 mmol) in dioxane (5 mL), and the mixture was degassed, purged with N2 three times and stirred at 100 °C for 16 h under $N_2$ atmosphere. The mixture was added with 1N HCl solution (3 mL) and stirred at 25 °C for 30 minutes. The mixture was quenched with sat. aq. CsF (30 mL) and extracted with EtOAc (10 mL X 3). The combined organic layer was washed with brine, dried over $Na_2SO_4$ and concentrated under reduced pressure to obtain a residue, which was purified by silica gel column chromatography (20% EtOAc in PE) to give 1-[4-amino-6-(trifluoromethyl)-2-pyridyl]ethanone (420 mg, 80.0% yield) as yellow oil. $^1$H NMR (400 MHz, CHLOROFORM-d) δ 7.37 (d, J = 2.4 Hz, 1H), 7.02 (d, J = 2.4 Hz, 1H), 4.56 (s, 2H), 2.70 (s, 3H).

Steps 2 to 4: Synthesis of (R)-N-[(1S)-1-[4-amino-6-(trifluoromethyl)-2-pyridyl]ethyl]-2-methyl-propane)-2-sulfinamide and (R)-N-[(1R)-1-[4-amino-6-(trifluoromethyl)-2-pyridyl]ethyl]-2-methyl-propane)-2-sulfinamide

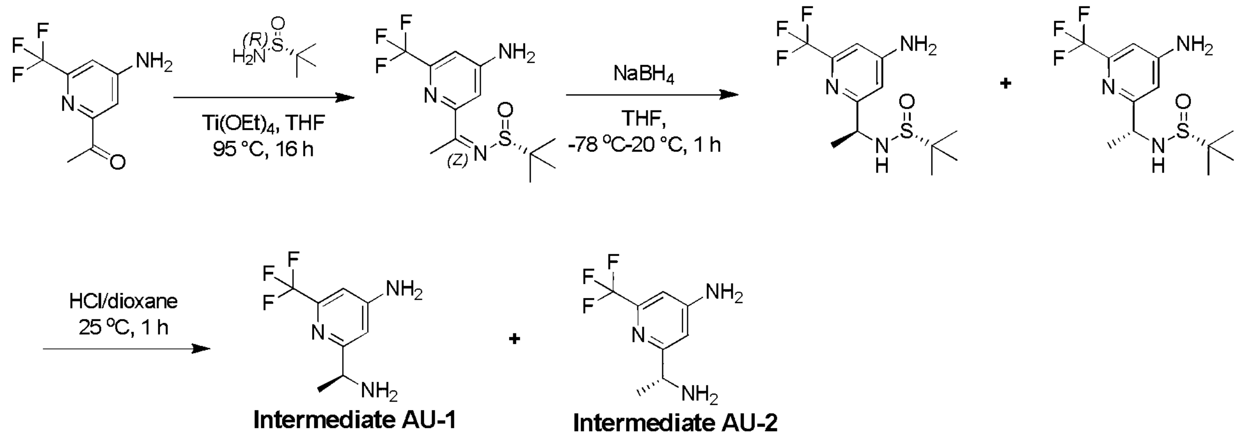


**Intermediate AU-1** **Intermediate AU-2**

Intermediate AU-1 and AU-2 were prepared in the same method as in Steps 2 to 4 of Preparation Example 2. $^1$H NMR (400 MHz, DMSO-$d_6$) δ = 8.28 (d, J = 1.2 Hz, 3H), 6.91 (d, J = 1.6 Hz, 1H), 6.71 (d, J = 1.6 Hz, 1H), 4.38 - 4.27 (m, 1H), 1.44 (d, J = 6.8 Hz, 3H). $^1$H NMR (400 MHz, DMSO-$d_6$) δ = 8.28 (d, J = 1.2 Hz, 3H), 6.91 (d, J = 1.6 Hz, 1H), 6.71 (d, J = 1.6 Hz, 1H), 4.38 - 4.27 (m, 1H), 1.44 (d, J = 6.8 Hz, 3H).

**Preparation Example I: 1-(5-bromothiophen-3-yl)-N-methylmethanamine**

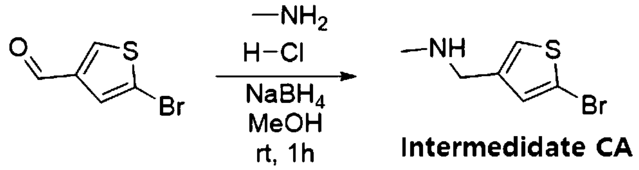


**Intermediate CA**

To a solution of 5-bromothiophene-3-carbaldehyde (0.3 ml, 2.75 mmol) in MeOH (5 mL) was added methylamine hydrochloride (223 mg, 3.30 mmol). The reaction mixture was stirred at room temperature for 15 min and cooled to 0 °C. To the mixture was added sodium borohydride (104 mg, 2.75 mmol). The mixture was stirred at room temperature for 1 hour. The mixture was quenched with water and concentrated under reduced pressure. The residue was extracted with DCM. The organic layer was dried over $Na_2SO_4$ and concentrated under reduced pressure. The product was purified by flash chromatography (0-5% MeOH in DCM) to give Intermediate CA (58.7 mg, 10.4% yield) as brown liquid. $^1$H NMR (500 MHz, MeOD) δ 8.26 (d, J = 2.6 Hz, 1H), 8.08 (dd, J = 9.7, 2.6 Hz, 1H), 7.62 (d, J = 1.9 Hz, 1H), 7.61 - 7.54 (m, 1H), 7.54 - 7.47 (m, 2H), 7.46 - 7.42 (m, 2H), 7.41 - 7.31 (m, 4H), 6.66 (d, J = 9.7 Hz, 1H), 5.20 (q, J = 7.1 Hz, 1H), 4.05 (s, 2H), 2.63 (s, 3H), 1.56 (d, J = 7.1 Hz, 3H); LC/MS m/z = 206 $[M+H]^+$.

**Preparation Example II: N-(5-amino-2-fluoro-phenyl)acetamide**

Step 1: Synthesis of N-(2-fluoro-5-nitro-phenyl)acetamide

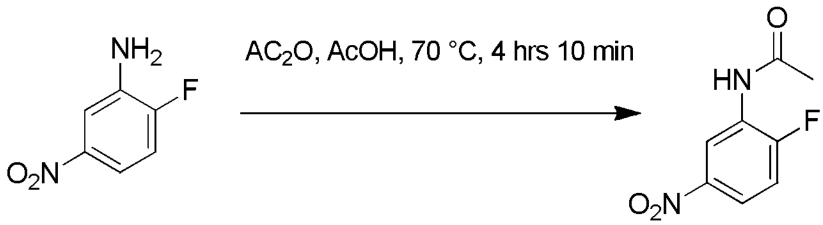


A mixture of 2-fluoro-5-nitro-aniline (2.0 g, 12.8 mmol) in AcOH (4 mL) was stirred at 70 °C for 10 min. The mixture was added with $Ac_2O$ (2.62 g, 25.6 mmol) and stirred at 70 °C for 4 h. The mixture was filtered, and the filtrate was concentrated in vacuo to give N-(2-fluoro-5-nitro-phenyl)acetamide (2.27 g, 89% yield) as a white solid. $^1$H NMR (400 MHz, DMSO-$d_6$) δ = 10.18 (s, 1H), 9.00 (dd, *J* = 2.8, 6.8 Hz, 1H), 8.05 - 8.00 (m, 1H), 7.56 (dd, *J* = 9.2, 10.4 Hz, 1H), 2.16 (s, 3H).

Step 2: Synthesis of N-(5-amino-2-fluoro-phenyl)acetamide

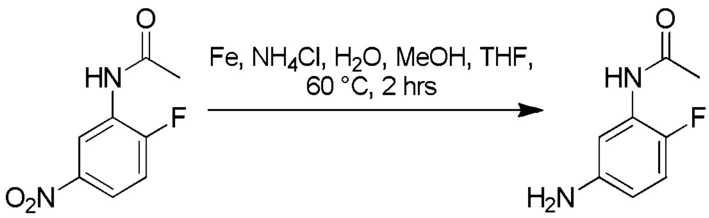


**Intermediate CB**

To a mixture of N-(2-fluoro-5-nitro-phenyl)acetamide (2.27 g, 11.4 mmol), Fe (3.20 g, 57.2 mmol) and $NH_4Cl$ (6.13 g, 114.5 mmol) in THF (8 mL), $H_2O$ (4 mL) and MeOH (32 mL) were added. The reaction mixture was stirred at 60 °C for 2 h. The reaction mixture was diluted in MeOH (100 mL) and filtered through celite. The filtrate was concentrated in vacuo to give Intermediate CB (1.75 g, 90% yield) as a yellow solid. $^1$H NMR (400 MHz, DMSO-$d_6$) δ = 9.40 (s, 1H), 7.12 (d, *J* = 4.8 Hz, 1H), 6.84 (t, *J* = 9.6 Hz, 1H), 6.32 - 6.18 (m, 1H), 4.96 (s, 2H), 2.04 (s, 3H).

**Preparation Example III: 1-acetylpiperidin-4-yl methanesulfonate**

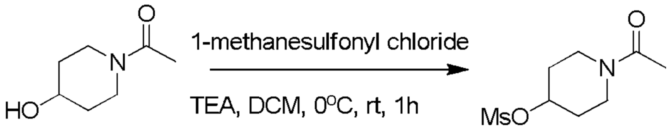


**Intermediate CC**

To a solution of 1-(4-hydroxypiperidin-1-yl)ethan-1-one (200 mg, 1.39 mmol) in DCM (3 mL) was added triethylamine (0.29 mL, 2.09 mmol). Methanesulfonyl chloride (0.11 mL, 1.39 mmol) was dropwise added to the reaction mixture at 0 °C. The reaction mixture was stirred at room temperature for 1 hour. The mixture was added with DW and extracted with MC. The combined organic layer was dried over $MgSO_4$, filtered and concentrated to give Intermediate CC (240 mg, 78% yield). LC/MS m/z = 222.1 $[M+H]^+$.

Preparation Example IV: 1-methyl-1,2,3,6-tetrahydropyridin-4-yl trifluoromethanesulfonate

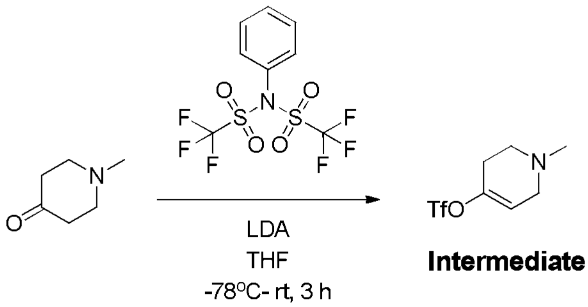


**Intermediate CD**

To a solution of 1-methylpiperidin-4-one (0.92 g, 8.13 mmol) in THF (10 mL) was added LDA (1.0 M in THF/Hexane, 8.13 mL) at -78 °C. The mixture was allowed to be warmed to room temperature and stirred for 30 min. The solution was cooled once more to -78 °C, and 1,1,1-trifluoro-N-phenyl-N-((trifluoromethyl)sulfonyl)methanesulfonamide (4.36 g, 12.2 mmol) was added in one portion. The solution was warmed to room temperature and stirred for 3 h. The reaction mixture was poured into water and extracted with ether. The organic layer was dried over anhydrous $Na_2SO_4$ and concentrated under reduced pressure to obtain a crude product. The residue was purified by flash chromatography (0-20% EtOAc in Hx) to give Intermediate CD (1.22 g, 61.3%). LC/MS m/z = 246 $[M+H]^+$.

**Preparation Example V: N-(3-bromophenyl)cyclopropanecarboxamide**

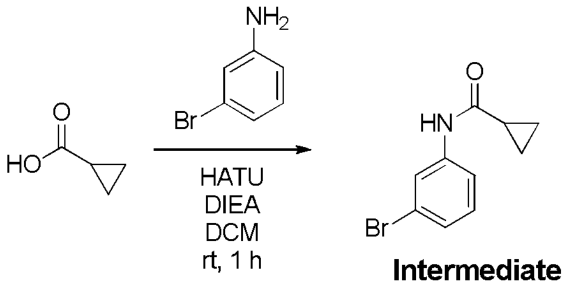


**Intermediate CE**

To a solution of cyclopropanecarboxylic acid (253 μL, 3.20 mmol) in DCM (5 mL), HATU (1.66 g, 4.36 mmol), DIEA (1.01 mL, 5.81 mmol) and 3-bromoaniline (0.5 g, 2.91 mmol) were added. The mixture was stirred at room temperature for 1 hour. The precipitate was collected by filtration and washed with DCM. The product was purified by flash chromatography (25-50% EtOAc in Hx) to give Intermediate CE (0.98 g, 140%). LC/MS m/z = 240 $[M+H]^+$.

**Preparation Example VI: 3-tetrahydrofuran-2-ylaniline**

Step 1: Synthesis of 5-(3-nitrophenyl)-2,3-dihydrofuran

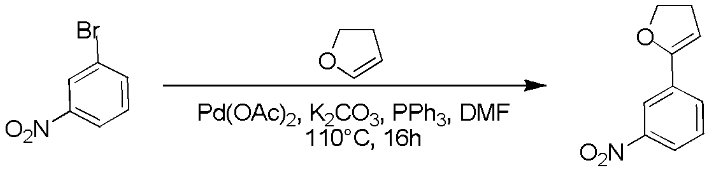


A mixture of 1-bromo-3-nitro-benzene (2.0 g, 9.90 mmol), 2,3-dihydrofuran (3.47 g, 49.5 mmol), $Pd(OAc)_2$ (222 mg, 990 μmol), $PPh_3$ (519 mg, 1.98 mmol) and $K_2CO_3$ (13.6 g, 99.0 mmol) in DMF (20 mL) was stirred at 110°C for 16 h. The reaction mixture was filtered, and the filtrate was added with water (50 mL). The mixture was extracted with EA (3 x 20 mL). The organic layers were combined, washed with saturated NaCl solution (2 x 30 mL) and concentrated in vacuo. The residue was purified by column chromatography ($SiO_2$, Petroleum ether/Ethyl acetate = 1/0 to 10/1) to give 5-(3-nitrophenyl)-2,3-dihydrofuran (1.1 g, 58% yield) as yellow oil. $^1H$ NMR (400 MHz, $CDCl_3$) δ = 8.21 - 8.11 (m, 2H), 7.65 (d, *J* = 7.6 Hz, 1H), 7.57 - 7.48 (m, 1H), 6.12 (d, *J* = 5.6 Hz, 1H), 5.90 (d, *J* = 5.6 Hz, 2H), 5.02 - 4.89 (m, 1H), 4.87 - 4.78 (m, 1H).

Step 2: Synthesis of 3-tetrahydrofuran-2-ylaniline

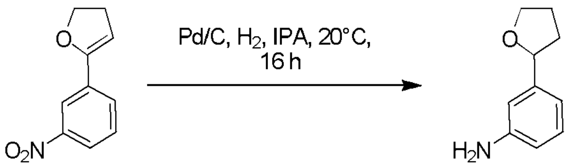


**Intermediate CF**

To a solution of 5-(3-nitrophenyl)-2,3-dihydrofuran (0.9 g, 4.71 mmol) in IPA (10 mL) was added Pd/C (90 mg, 5% purity) under $N_2$ atmosphere. The reaction mixture was stirred at 20 °C for 16 h under $H_2$ at 15 psi. The reaction mixture was filtered, and the filtrate was concentrated in vacuo to give Intermediate CF (0.7 g, 91% yield) as yellow gum. $^1H$ NMR (400 MHz, $CDCl_3$) δ = 7.12 (d, *J*= 7.6 Hz, 1H), 6.75 - 6.65 (m, 2H), 6.60 - 6.55 (m, 1H), 4.82 (t, *J* = 7.2 Hz, 1H), 4.13 - 4.04 (m, 1H), 3.96-3.88 (m,1H), 2.36 - 2.22 (m, 1H), 2.03 - 1.93 (m, 2H), 1.84-1.79 (m, 1H).

**Preparation Example VII: 2-fluoro-3,4-dimethoxy-aniline**

Step 1: Synthesis of 3-fluoro-1,2-dimethoxy-4-nitro-benzene

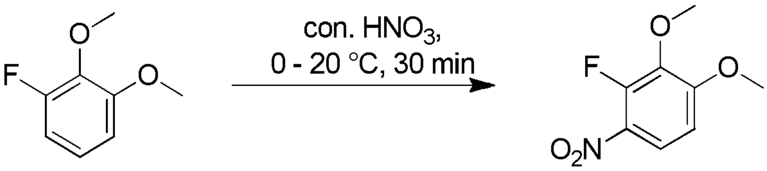


To 1-fluoro-2,3-dimethoxy-benzene (300 mg, 1.92 mmol) was added $HNO_3$ (5.6 mL) dropwise at 0 °C. The mixture was stirred at 0 °C for 15 min and stirred at 20 °C for another 15 min. The reaction mixture was poured into ice, and the resultant solid was filtered, washed with water and concentrated under reduced pressure to obtain a residue. The residue was purified by Prep-TLC ($SiO_2$, PE: EtOAc = 10: 1) to give 3-fluoro-1,2-dimethoxy-4-nitro-benzene (120 mg, 31% yield). $^1H$ NMR (400 MHz, DMSO-$d_6$) δ = 7.97 (dd, *J* = 8.4, 9.6 Hz, 1H), 7.11 (dd, *J* = 1.6, 9.6 Hz, 1H), 3.96 (s, 3H), 3.85 (s, 3H).

Step 2: Synthesis of 2-fluoro-3,4-dimethoxy-aniline

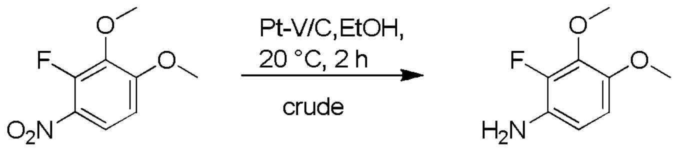


**Intermediate CG**

To a solution of 3-fluoro-1,2-dimethoxy-4-nitro-benzene (120 mg, 597 μmol) in EtOH (5 mL) was added Pt-V/C (16 mg) under $N_2$ atmosphere. The suspension was degassed and purged with $H_2$ for 3 times. Then, the mixture was stirred under $H_2$ (15 Psi) for 1 hour at 20 °C. The reaction mixture was filtered, and the filtrate was concentrated under reduced pressure to give Intermediate CG (100 mg, crude) as brown liquid. $^1H$ NMR (400 MHz, DMSO-$d_6$) δ = 6.59 (dd, *J*= 2.0, 8.8 Hz, 1H), 6.47 - 6.38 (m, 1H), 4.70 (s, 2H), 3.75 (s, 3H), 3.68(s, 3H).

**Preparation Example VIII: 4-(4,4,5,5-tetramethyl-1,3,2-dioxaborolan-2-yl)bicyclo[4.2.0]octa-1,3,5-triene-3-carbaldehyde**

Step 1: Synthesis of 4-bromobicyclo[4.2.0]octa-1,3,5-triene-3-carbaldehyde

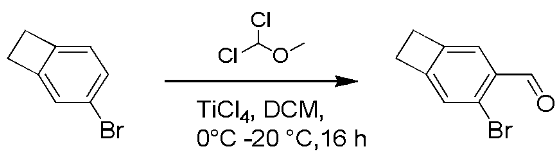


To a solution of dichloro(methoxy)methane (251.21 mg, 2.19 mmol) and $TiCl_4$ (497.40 mg, 2.62 mmol) in DCM (6 mL) was added 3-bromobicyclo[4.2.0]octa-1,3,5-triene (200 mg, 1.09 mmol) in DCM (2 mL) dropwise at 0 °C. The reaction mixture was stirred at 20 °C for 16 h under $N_2$. The mixture was added with a cold 5% aqueous HCl solution (20 mL) at 0 °C and stirred for 15 min. The mixture was extracted with $CH_2Cl_2$ (20 × 3 mL). The combined organic layer was washed with brine (50 mL), dried over anhydrous $Na_2SO_4$ and concentrated under reduced pressure to obtain a residue. The residue was purified by silica gel column chromatography (1% EtOH in PE) to give 4-bromobicyclo[4.2.0]octa-1,3,5-triene-3-carbaldehyde (180 mg , 33.77% yield) as a yellow solid. $^1H$ NMR (400 MHz, DMSO-$d_6$) δ = 10.23 (s, 1H), 7.53 (d, *J* = 7.6 Hz, 2H), 3.26 - 3.21 (m, 2H), 3.18 - 3.13 (m, 2H); MS (ESI) m/z = 212.8 [M+1+H]$^+$.

Step 2: Synthesis of 4-(4,4,5,5-tetramethyl-1,3,2-dioxaborolan-2-yl)bicyclo[4.2.0]octa-1,3,5-triene-3-carbaldehyde

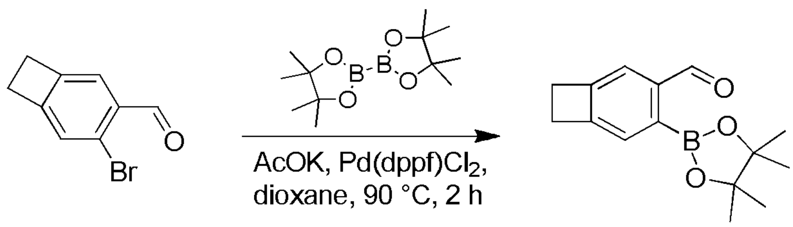


**Intermediate CH**

To a mixture of 4-bromobicyclo[4.2.0]octa-1,3,5-triene-3-carbaldehyde (50 mg, 236.91 μmol) and 4,4,5,5-tetramethyl-2-(4,4,5,5-tetramethyl-1,3,2-dioxaborolan-2-yl)-1,3,2-dioxaborolane (120.32 mg, 473.81 μmol) in dioxane (2 mL), AcOK (69.75 mg, 710.72 μmol) and Pd(dppf)$Cl_2$ (17.33 mg, 23.69 μmol) were added. The mixture was stirred at 90 C for 2 h. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by silica gel column chromatography (2% EtOAc in PE) to give Intermediate CH (52 mg,

67.44% yield) as yellow oil. $^1H$ NMR (400 MHz, DMSO-$d_6$) δ = 10.26 (s, 1H), 7.62 (d, *J* = 0.8 Hz, 1H), 7.40 (s, 1H), 3.22 (s, 4H), 1.33 (s, 12H); MS (ESI) m/z = 259.0 $[M+2+H]^+$.

**Preparation Example IX: 5-methyl-1-(3-(4,4,5,5-tetramethyl-1,3,2-dioxaborolan-2-yl)phenyl)-1H-1,2,3-triazole**

Step 1: Synthesis of 1-azido-3-bromobenzene

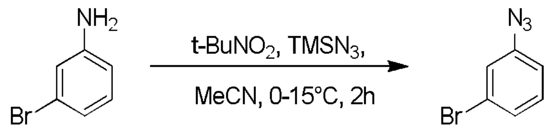


A solution of 3-bromoaniline (2 g, 11.63 mmol, 1.27 mL) in MeCN (20 mL) was cooled to 0 °C in an ice bath, t-$BuONO_2$ (1.44 g, 13.95 mmol, 1.66 mL) was added thereto, followed by adding $TMSN_3$ (1.61 g, 13.95 mmol, 1.83 mL) slowly while stirring. After the resulting solution was stirred at 15 °C for 2 h, TLC (EtOAc: Petroleum ether= 0: 1) indicated that the reaction had completed. Without workup, the product was concentrated under reduced pressure and purified by silica gel chromatography (0% EtOAc in Petroleum ether) to give 1-azido-3-bromobenzene (1.56 g, 67.4% yield) as yellow oil. $^1H$ NMR (400MHz, DMSO-$d_6$) δ 7.40 - 7.34 (m, 2H), 7.34 - 7.30 (m, 1H), 7.13 (td, *J* = 2.0, 7.2 Hz, 1H); LC/MS (ESI) m/z = 318.3 $[M+H]^+$

Step 2: Synthesis of 1-(3-bromophenyl)-5-methyl-triazole

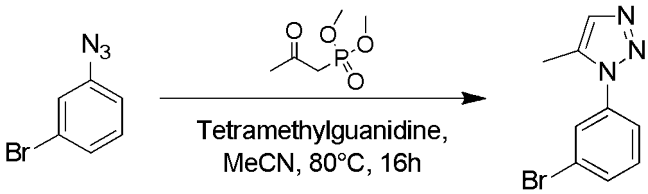


To a solution of 1-azido-3-bromo-benzene (800 mg, 4.04 mmol) in MeCN (10 mL), tetramethylguanidine (1.40 g, 12.12 mmol) and 1-dimethoxyphosphorylpropan-2-one (671.09 mg, 4.04 mmol, 554.62 μL) were added. The mixture was stirred at 80 °C for 16 h. The reaction mixture was poured into water (50 mL) and extracted with EtOAc (30 mL×3). The combined organic layer was washed with brine (30 mL), dried over anhydrous $Na_2SO_4$ and concentrated under reduced pressure to obtain a residue. The residue was purified by flash silica gel chromatography (15% EtOAc in PE) to give 1-(3-bromophenyl)-5-methyl-triazole (350 mg, 1.32 mmol, 32.73% yield) as a yellow solid. $^1H$ NMR (400MHz, DMSO-$d_6$) δ = 7.89 (t, *J* = 2.0 Hz, 1H), 7.81 - 7.77 (m, 1H), 7.71 (d, *J* = 0.8 Hz, 1H), 7.67 - 7.63 (m, 1H), 7.60 - 7.55 (m, 1H), 2.34 (d, *J* = 0.4 Hz, 3H); MS (ESI) m/z = 238.0 $[M+H]^+$.

Step 3: Synthesis of 5-methyl-1-(3-(4,4,5,5-tetramethyl-1,3,2-dioxaborolan-2-yl)phenyl)-1H-1,2,3-triazole

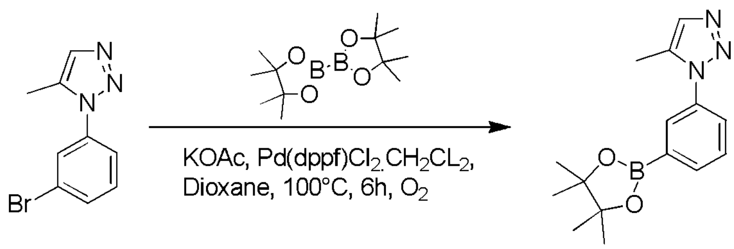


**Intermediate CI**

Intermediate CI was prepared in the same method as in Step 2 of Preparation Example VIII. MS (ESI) m/z = 285.2

[M+H]+.

**Preparation Example X: 1-(trideuteriomethyl)triazole**

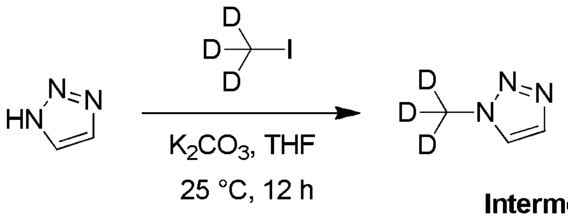


**Intermediate CJ**

To a solution of 1H-triazole (1.99 g, 28.74 mmol) in THF (25 mL), $K_2CO_3$ (7.95 g, 57.49 mmol) and trideuterio(iodo)methane (5 g, 2.15 mL, 34.49 mmol) were added. The mixture was stirred at 25 °C for 12 h. The reaction mixture was poured into water (20 mL) and extracted with EtOAc (30 mL×3). The combined organic layer was washed with brine (20 mL × 2), dried over anhydrous $Na_2SO_4$ and concentrated under reduced pressure to obtain Intermediate CJ (crude product, 800 mg, 32.32% yield) as yellow oil. $^1$H NMR (400MHz, DMSO-$d_6$) δ = 8 8.06 (s, 1H), 7.70 (s, 1H).

**Preparation Example XI: tributyl-(3-methyltriazol-4-yl)stannane (T-3)**

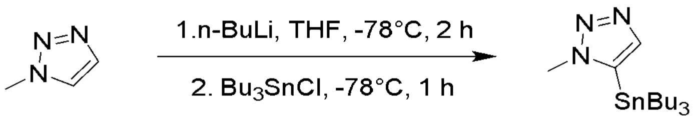


A solution of 1-methyltriazol (3 g, 36.10 mmol) in THF (30 mL) was cooled to -78 °C, and then n-BuLi (2.5 M, 16.17 mL) was slowly added and the reaction mixture was stirred at -78 °C for 2 h. Thereafter, tributyl(chloro)stannane (16.94 g, 52.04 mmol, 14mL) was slowly added and the reaction mixture was stirred at -78 °C for 1 under nitrogen atmosphere. The reaction mixture was quenched with water added slowly at 0 °C, and extracted with EtOAc (70 mL×3). The combined organic layers were washed with brine (50 mL x 2), dried over $Na_2SO_4$ and concentrated under reduced pressure to give crude product **Intermediate** CK (17.35 g, 34.97 mmol, 96.85% yield, 75% purity) as light yellow oil. $^1$H NMR (400 MHz, DMSO-$d_6$) δ 7.58 (br s, 1H), 4.06 - 4.00 (m, 3H), 1.50 - 1.44 (m, 4H), 1.28 (br d, *J* = 7.2 Hz, 8H), 1.19 - 1.12 (m, 6H), 0.87 - 0.82 (m, 9H).

**Preparation Example XII: tributyl-[3-(triduteriomethyl)triazol-4-yl]stannane (T-9)**

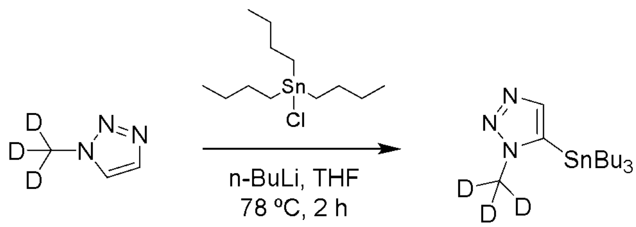


A solution of 1-(triduteriomethyl)triazol (1 g, 11.61 mmol) in THF (20 mL) was cooled to -78 °C, and then n-BuLi (2.5 M, 5.20 mL) was slowly added and the reaction mixture was stirred at -78 °C for 1 h. Thereafter, tributyl(chloro) stannane (5.22 g, 16.03 mmol, 4.31 mL) was slowly added and the reaction mixture was stirred at -78 °C for 1 h under nitrogen atmosphere. The reaction mixture was quenched with aq. $NH_4Cl$ (20 mL) added dropwise slowly at 0 °C, and extracted with EtOAc (40 mL×3). The combined organic layers were washed with brine (20 mL x 2), dried over $Na_2SO_4$ and concentrated under reduced pressure to give crude product **Intermediate CL** (4 g, 10.66 mmol) as yellow oil. It was used in the next reaction without further purification. $^1$H NMR (400 MHz, DMSO-$d_6$) δ 7.60 (s, 1H), 1.49 (m, 4H), 1.35 - 1.21 (m, 8H), 1.19 - 1.09 (m, 6H), 0.88 - 0.83 (m, 9H).

**Preparation Example BA: 1-[5-(3-methyltriazol-4-yl)-3-pyridyl]-6-oxo-pyridazine-3-carboxylic acid**

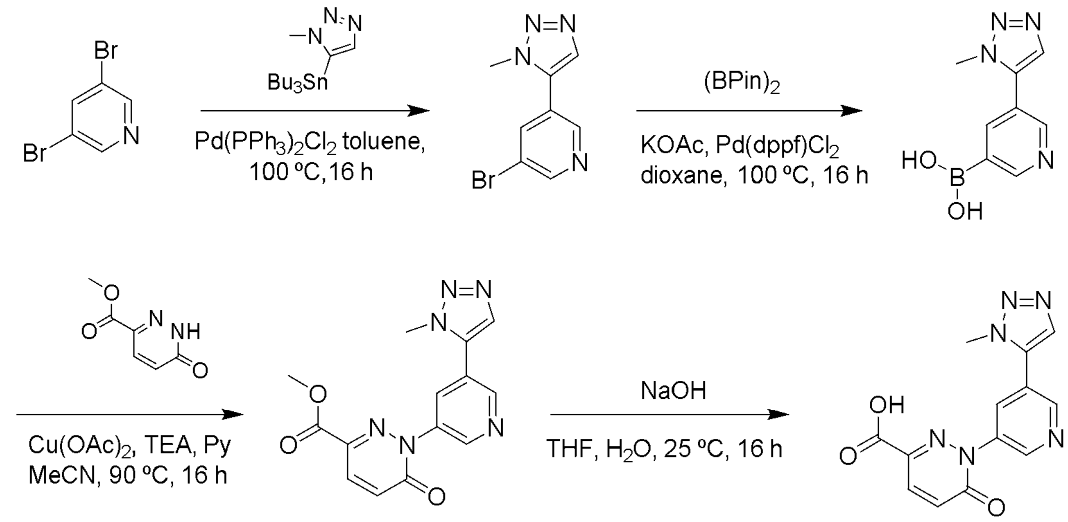


Step 1: Synthesis of 3-bromo-5-(3-methyltriazol-4-yl)pyridine

A solution of 3,5-dibromopyridine (10.92 g, 46.10 mmol), tributyl-(3-methyltriazol-4-yl)stannane (22.3 g, 59.92 mmol) and $Pd(PPh_3)_2Cl_2$ (3.24 g, 4.61 mmol) in toluene (200 mL) were substituted with nitrogen for 3 times, and then the mixture was stirred at 90 °C for 16 h under nitrogen atmosphere. The reaction mixture was poured into water (150 mL) and extracted with EtOAc (200 mL x 3). The combined organic layers were washed with brine (200 mL x 3), dried over $Na_2SO_4$ and concentrated under reduced pressure to give crude product. The crude product was purified by silica gel column chromatography (80% EA in petroleum ether) to give 3-bromo-5-(3-methyltriazol-4-yl)pyridine (4.4 g, 39.93% yield) as a yellow solid. $^1H$ NMR (400 MHz, DMSO-$d_6$) δ 8.83-8.83 (m, 1H), 8.40 (t, *J* = 2.0 Hz, 1H), 8.09 (s, 1H), 7.66-7.49 (m, 1H), 4.13 (s, 3H); LC/MS (ESI) m/z = 153.0 $[M+H]^+$.

Step 2: Synthesis of [5-(3-methyltriazol-4-yl)-3-pyridyl]boronic acid

A solution of 3-bromo-5-(3-methyltriazol-4-yl)pyridine (6.76 g, 28.28 mmol), 4,4,5,5-tetramethyl-2-(4,4,5,5-tetramethyl-1,3,2-dioxoborrolan-2-yl)-1,3,2-dioxoborrolan (14.36 g, 56.55 mmol), $Pd(dppf)Cl_2$ (2.31 g, 2.83 mmol) and KOAc (5.55 g, 56.55 mmol) in dioxane (120 mL) was substituted with nitrogen for 3 times, and then the mixture was stirred at 100 °C for 16 h under nitrogen atmosphere. The reaction mixture was filtered and concentrated under reduced pressure to give crude product. The crude product was purified by silica gel column chromatography (3% MeOH in DCM) to give [5-(3-methyltriazol-4-yl)-3-pyridyl]boronic acid (6.76 g, 78.52% yield, 67% purity) as a dark brown solid. LCMS (ESI) m/z = 205.1 $[M+H]^+$.

Step 3: Synthesis of methyl-1-[5-(3-methyltriazol-4-yl)-3-pyridyl]-6-oxo-pyridazine-3-carboxylate

A solution of methyl-6-oxo-1H-pyridazine-3-carboxylate (3.78 g, 24.51 mmol), [5-(3-methyltriazol-4-yl)-3-pyridyl]boronic acid (5 g, 24.51 mmol), $Cu(OAc)_2$ (4.45 g, 24.51 mmol), TEA (4.96 g, 49.02 mmol, 6.82 mL) and pyridine (3.88 g, 49.02 mmol, 3.96 mL) in MeCN (100 mL) was substituted with oxygen for 3 times, and then the mixture was stirred at 90 °C for 16 h under oxygen atmosphere. The reaction mixture was filtered and concentrated under reduced pressure to give crude product. The crude product was purified by silica gel column chromatography (3% MeOH in DCM) to give methyl-1-[5-(3-methyltriazol-4-yl)-3-pyridyl]-6-oxo-pyridazine-3-carboxylate (1.83 g, 5.27 mmol, 21.51% yield, 89.98% purity) as a dark brwon solid. LCMS (ESI) m/z = 312.9 $[M+H]^+$.

Step 4: Synthesis of 1-[5-(3-methyltriazol-4-yl)-3-pyridyl]-6-oxo-pyridazine-3-carboxylic acid

To a solution of methyl-1-[5-(3-methyltriazol-4-yl)-3-pyridyl]-6-oxo-pyridazine-3-carboxylate (1.0 g, 3.20 mmol) in THF (4 mL) and $H_2O$ (2 mL) was added NaOH (256.16 mg, 6.40 mmol). The mixture was substituted with nitrogen for 3 times, and then stirred at 25 °C for 16 h under nitrogen atmosphere. The mixture was adjusted to pH = 2-3 with aq. 1 N HCl

and then **Intermediate BA** (725 mg, 74.01% yield, 97.5% purity) was obtained by solid filtration as a yellow solid. LCMS (ESI) m/z = 299.0 $[M+H]^+$.

**Preparation Example BB: (1R)-1-[3-(difluoromethyl)-2-fluoro-phenyl]ethanamine**

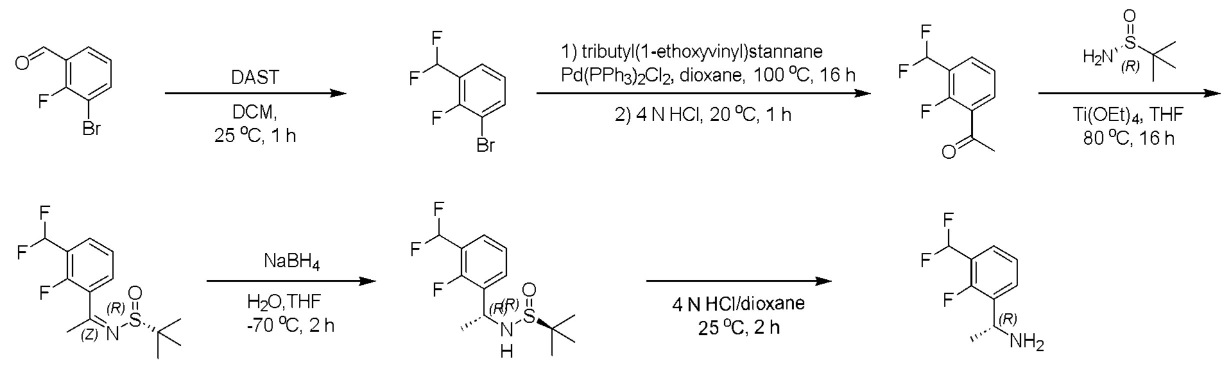


Step 1: Synthesis of 1-bromo-3-(difluoromethyl)-2-fluoro-benzene

To a solution of 3-bromo-2-fluoro-benzaldehyde (9 g, 44.33 mmol) in DCM (150 mL) was added DAST (Diethylaminosulfur trifluoride; 14.29 g, 88.67 mmol, 11.71 mL), and then the mixture was stirred at 25 °C for 1 h. After the addition of sat. $NaHCO_3$ (50 mL), the combined organic layers were extracted with DCM (50 mL X 3). It was washed with brine, dried over $Na_2SO_4$ and concentrated under reduced pressure to give crude product. The organic layers were dried over anhydrous $Na_2SO_4$ and concentrated under reduced pressure to give crude product. The crude product was purified by silica gel column chromatography (100% petroleum ether) to give 1-bromo-3-(difluoromethyl)-2-fluoro-benzene (3.9 g, 39.1% yield) as colorless oil. $^1$H NMR (400 MHz, DMSO-$d_6$) $\delta$ 7.98 - 7.79 (m, 1H), 7.72 - 7.57 (m, 1H), 7.37 (s, 1H), 7.31 (br t, $J$ = 7.8 Hz, 1H), 7.24 (s, 1H), 7.10 (s, 1H).

Step 2: Synthesis of 1-[3-(difluoromethyl)-2-fluoro-phenyl]ethanone

A mixture of 1-bromo-3-(difluoromethyl)-2-fluoro-benzene (17 g, 75.55 mmol), tributyl(1-ethoxyvinyl)stannane (28.65 g, 79.33 mmol, 26.80 mL) and $Pd(PPh_3)_2Cl_2$ (2.65 g, 3.78 mmol ) in dioxane (300 mL) was degassed and purged with $N_2$ for 3 times, and then the mixture was stirred at 100 °C for 16 h under $N_2$ atmosphere. The mixture was quenched with 4 N HCl (30 mL) and then stirred at 20 °C for 1 h. The mixture was poured into water (200 mL) and extracted with EtOAc (100 mL X 3). The organic layers were washed with brine (100 mL X 3), dried over anhydrous $Na_2SO_4$ and concentrated under reduced pressure to give crude product. The crude product was purified by silica gel column chromatography (2% EtOAc in petroleum ether) to give 1-[3-(difluoromethyl)-2-fluoro-phenyl]ethanone (10 g, 70.35% yield) as colorless oil. $^1$H NMR (400 MHz, DMSO-$d_6$) $\delta$ 8.03-7.93 (m, 1H), 7.93-7.82 (m, 1H), 7.49-7.14 (m, 2H), 2.63-2.58 (m, 3H).

Step 3: Synthesis of (NZ,R)-N-[1-[3-(difluoromethyl)-2-fluoro-phenyl]ethylidene]-2-methyl-propane-2-sulfinamide

A mixture of 1-[3-(difluoromethyl)-2-fluoro-phenyl]ethanone (10 g, 53.15 mmol), 2-methylpropane-2-sulfinamide (6.44 g, 53.15 mmol) and $Ti(OEt)_4$ (36.37 g, 159.45 mmol, 33.07 mL) in THF (80 mL) was degassed and purged with $N_2$ for 3 times, and then the mixture was stirred at 80 °C for 16 h under $N_2$ atmosphere. The mixture was poured into water (100 mL) and filtered, and then the filtrate was extracted with EtOAc (100 mL X 3). The organic layers were washed with brine (50 mL X 3), dried over anhydrous $Na_2SO_4$ and concentrated under reduced pressure to give crude product. The crude product was purified by silica gel column chromatography (35% EtOAc in petroleum ether) to give (NZ,R)-N-[1-[3-(difluoromethyl)-2-fluoro-phenyl]ethylidene]-2-methyl-propane-2-sulfinamide (13.0 g, 81.65% yield, 97.25% purity) as yellow oil. $^1$H NMR (400 MHz, DMSO-$d_6$) $\delta$ 7.90-7.81 (m, 1H), 7.81-7.73 (m, 1H), 7.47-7.40 (m, 1H), 7.29-7.10 (m, 1H), 2.71 (br s, 3H), 1.22 (s, 9H); MS (ESI) m/z = 292.1 $[M+H]^+$.

Step 4: Synthesis of (R)-N-[(1R)-1-[3-(difluoromethyl)-2-fluoro-phenyl]ethyl]-2-methylpropane-2-sulfinamide

A solution of (NZ,R)-N-[1-[3-(difluoromethyl)-2-fluoro-phenyl]ethylidene]-2-methylpropane-2-sulfinamide (13 g, 44.62 mmol) in THF (200 mL) and $H_2O$ (5 mL) was cooled to -70 °C and stirred at -70 °C for 2 h under $N_2$ atmosphere after

the addition of $NaBH_4$ (1.35 g, 35.70 mmol) in 3 batches. The mixture was quenched at 20 °C with sat. $NH_4Cl$ (200 mL), diluted with EtOAc (150 mL), and then extracted with EtOAc (150 mL X 3). The organic layers were dried over anhydrous $Na_2SO_4$ and concentrated under reduced pressure to give crude product. The crude product was purified by silica gel column chromatography (30% EtOAc in petroleum ether) to give (R)-N-[(1R)-1-[3-(difluoromethyl)-2-fluoro-phenyl]ethyl]-2-methyl-propane-2-sulfinamide (4.4 g, 33.61% yield, 100% purity, 98.34% ee) as colorless oil. $^1H$ NMR (400 MHz, DMSO-$d_6$) δ 7.74 (t, *J* = 7.2 Hz, 1H), 7.52 (t, *J* = 6.8 Hz, 1H), 7.37-7.33 (m, 1H), 7.33-7.06 (m, 1H), 5.88 (d, *J* = 8.0 Hz, 1H), 5.75 (s, 1H), 4.69 (quint, *J* = 7.0 Hz, 1H), 1.41 (d, *J* = 6.8 Hz, 3H), 1.10 (s, 9H); LC/MS (ESI) m/z = 294.1 $[M+H]^+$.

Step 5: Synthesis of (1R)-1-[3-(difluoromethyl)-2-fluoro-phenyl]ethanamine

A mixture of (R)-N-[(1R)-1-[3-(difluoromethyl)-2-fluoro-phenyl]ethyl]-2-methylpropane-2-sulfinamide (2.00 g, 6.82 mmol) in 4 N HCl/dioxane (15 mL) was degassed and purged with $N_2$ for 3 times, and then the mixture was stirred at 25 °C for 2 h under $N_2$ atmosphere. The mixture was concentrated under reduced pressure to give **Intermediate BB** (1.5 g, 94.02% yield, 96.43% purity, HCl salt) as a white solid. LC/MS (ESI) m/z = 190.1 $[M+H]^+$.

**Preparation Example BC: 5-methyl-1-[5-(3-methyltriazol-4-yl)-3-pyridyl]-6-oxo-pyridazine-3-carboxylic acid**

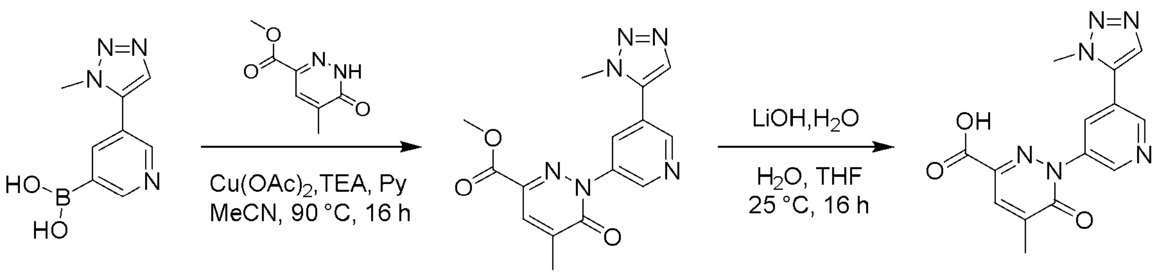


Step 1: Synthesis of methyl-5-methyl-1-[5-(3-methyltriazol-4-yl)-3-pyridyl]-6-oxo-pyridazine-3-carboxylate

A solution of methyl-5-methyl-6-oxo-1H-pyridazine-3-carboxylate (200 mg, 1.19 mmol), [5-(3-methyltriazol-4-yl)-3-pyridyl]boronic acid (242.63 mg, 1.19 mmol), $Cu(OAc)_2$ (216.03 mg, 1.19 mmol), TEA (240.71 mg, 2.38 mmol, 331.11 μL) and pyridine (188.17 mg, 2.38 mmol, 192.01 μL) in MeCN (5 mL) was substituted with nitrogen for 3 times, and then stirred at 90 °C for 16 h under nitrogen atmosphere. After the addition of water (30 mL), the reaction mixture was extracted with EtOAc (30 mL × 3). The organic layers were washed with brine (50 mL), dried over $Na_2SO_4$, and then concentrated under reduced pressure to give crude product. The crude product was purified by silica gel column chromatography (1% MeOH in DCM) to give methyl-5-methyl-1-[5-(3-methyltriazol-4-yl)-3-pyridyl]-6-oxo-pyridazine-3-carboxylate (305 mg, 69.15% yield, 88% purity) as a yellow solid. LC/MS (ESI) m/z = 327.1 $[M+H]^+$.

Step 2: Synthesis of 5-methyl-1-[5-(3-methyltriazol-4-yl)-3-pyridyl]-6-oxo-pyridazine-3-carboxylic acid

$LiOH \cdot H_2O$ (117.67 mg, 2.80 mmol) and $H_2O$ (2 mL) were added to a solution of methyl-5-methyl-1-[5-(3-methyltriazol-4-yl)-3-pyridyl]-6-oxo-pyridazine-3-carboxylate (305 mg, 934.70 μmol) in THF (4 mL), and then it was stirred at 25 °C for 16 h. The reaction mixture was concentrated under reduced pressure to remove solvent, and then diluted with water (10 mL). The reaction mixture was adjusted to pH = 3-4 with aq. 1 N HCl, and then extracted with EtOAc (20 mL × 3). The organic layers were washed with brine (30 mL), dried over $Na_2SO_4$, and then concentrated under reduced pressure to give **Intermediate BC** (63 mg, 20.29% yield, 94% purity) as a white solid. LC/MS (ESI) m/z = 313.1 $[M+H]^+$.

**Preparation Example BD: 1-[5-(2-methylpyrazole-3-yl)-3-pyridyl]-6-oxo-pyridazine-3-carboxylic acid**

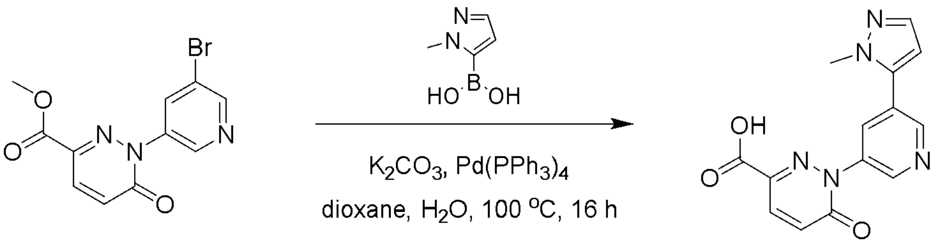


**Intermediate K-7**

A solution of Intermediate K-7 (500 mg, 1.61 mmol), (2-methylpyrazole-3-yl)boronic acid (406.06 mg, 3.22 mmol), $K_2CO_3$ (557.09 mg, 4.03 mmol) and $Pd(PPh_3)_4$ (186.32 mg, 161.24 μmol) in dioxane (5 mL) and $H_2O$ (0.5 mL) was substituted with nitrogen for 3 times, and then stirred at 100 °C for 16 h under nitrogen atmosphere. The reaction mixture was concentrated under reduced pressure to remove the solvent, and then diluted with water (10 mL). With the addition of aq. 1 N HCl, the reaction mixture was adjusted to pH = 3-4, and then extracted with EtOAc (20 mL × 3). The organic layers were washed with brine (30 mL), dried over $Na_2SO_4$ and concentrated under reduced pressure to give crude product, **Intermediate BD** (835 mg, crude) as a brown solid. LC/MS (ESI) m/z = 298.1 $[M+H]^+$.

**Preparation Example BE: 1-[5-(3-methyltriazol-4-yl)-3-pyridyl]-6-oxo-pyridine-3-carboxylic acid (4-4)**

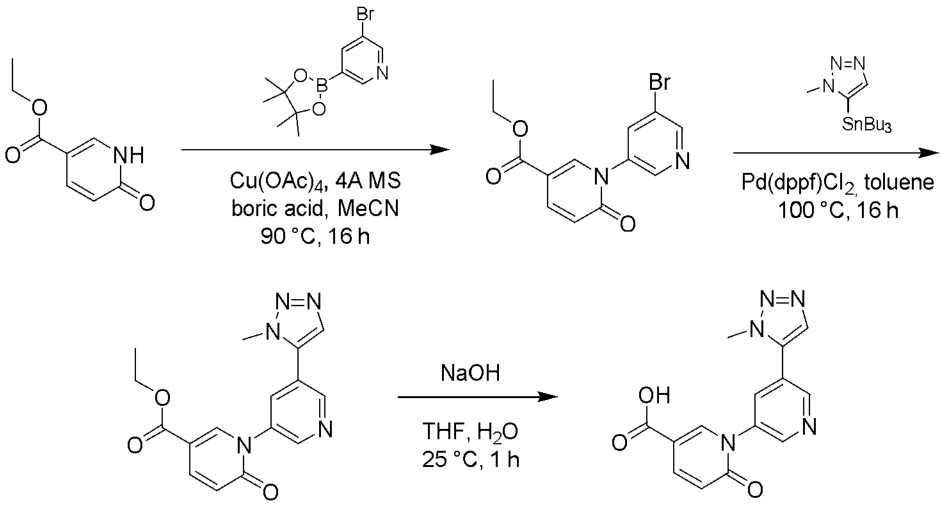


Step 1: Synthesis of ethyl-1-(5-bromo-3-pyridyl)-6-oxo-pyridine-3-carboxylate

A solution of ethyl-6-oxo-1H-pyridine-3-carboxylate (535.17 mg, 3.20 mmol), 3-bromo-5-(4,4,5,5-tetramethyl-1,3,2-dioxaborolan-2-yl)pyridine (1 g, 3.52 mmol), $Cu(OAc)_2$ (290.75 mg, 1.60 mmol), 4A MS (500 mg, 3.20 mmol) and boric acid (395.92 mg, 6.40 mmol) in MeCN (15 mL) was substituted with oxygen for 3 times, and then stirred at 90 °C for 16 h under oxygen atmosphere. The reaction mixture was filtered, and then the filtrate was concentrated under reduced pressure to give crude product. The crude product was purified by silica gel column chromatography (30% EtOAc in petroleum ether) to give ethyl-1-(5-bromo-3-pyridyl)-6-oxo-pyridine-3-carboxylate (87 mg, 7.65% yield, 90.95% purity) as a white solid. LCMS (ESI) m/z = 322.9 $[M + H]^+$.

Step 2: Synthesis of ethyl-1-[5-(3-methyltriazol-4-yl)-3-pyridyl]-6-oxo-pyridine-3-carboxylate

A solution of ethyl-1-(5-bromo-3-pyridyl)-6-oxo-pyridine-3-carboxylate (87 mg, 269.23 μmol), tributyl-(3-methyltriazol-4-yl)stannane (250.48 mg, 673.08 μmol) and $Pd(dppf)Cl_2$ (19.70 mg, 26.92 μmol) in toluene (2 mL) was substituted with nitrogen for 3 times, and then stirred at 100 °C for 16 h under nitrogen atmosphere. The reaction mixture was filtered, and then the filtrate was concentrated under reduced pressure to give crude product. The crude product was purified by silica gel column chromatography (2% MeOH in DCM) to give ethyl-1-[5-(3-methyltriazol-4-yl)-3-pyridyl]-6-oxo-pyridine-3-carboxylate (30 mg, 32.07% yield, 93.62% purity) as a white solid. LCMS (ESI) m/z = 326.1 $[M + H]^+$.

Step 3: Synthesis of 1-[5-(3-methyltriazol-4-yl)-3-pyridyl]-6-oxo-pyridine-3-carboxylic acid

To a solution of ethyl-1-[5-(3-methyltriazol-4-yl)-3-pyridyl]-6-oxo-pyridine-3-carboxylate (30 mg, 92.22 μmol) in THF (1 mL) and $H_2O$ (0.5 mL) was added NaOH (7.38 mg, 184.43 μmol). After the nitrogen substitution for 3 times, the mixture was stirred at 25 °C for 1 h under nitrogen atmosphere. The mixture was adjusted to pH = 5-6 with aq. 1 N HCl, and then the organic layers were concentrated under reduced pressure to give **Intermediate** BE (25 mg, crude) as yellow oil. LCMS (ESI) m/z = 298.0 $[M + H]^+$.

**Preparation Example BF: (R)-1-(2-fluoro-3-(1,1,2-trifluoroethyl)phenyl)ethane-1-amine (A-5)**

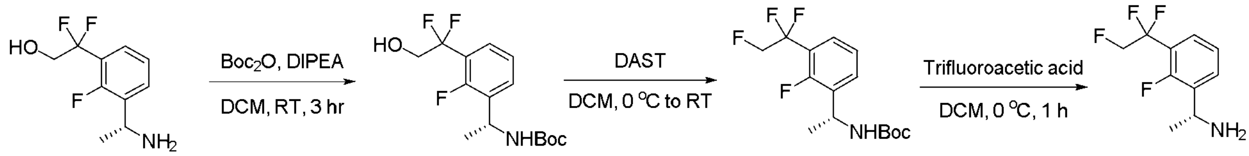


Step 1: Synthesis of (R)-(1-(3-(1,1-difluoro-2-hydroxyethyl)-2-fluorophenyl)ethyl)carbamate

To a solution of 2-{3-[(1R)-1-aminoethyl]-2-fluorophenyl}-2,2-difluoromethane-1-ol hydrochloride (**Intermediate E**; 500 mg, 1.96 mmol) in DCM (3.99 mL) was added di-tert-butyl dicarbonate (854 mg, 3.91 mmol) and DIPEA (0.68 mL, 3.91 mmol) at 0 °C, and then the mixture was stirred at room temperature for 3 h. DCM was added, and it was washed with water. The organic layers were dried over anhydrous $Na_2SO_4$, filtered and concentrated under reduced pressure. The residue was purified by silica gel column chromatography (Hex : EtOAc= 20: 1 to 10: 1) to give (R)-(1-(3-(1,1-difluoro-2-hydroxyethyl)-2-fluorophenyl)ethyl)carbamate (456 mg, 73% yield) as colorless oil. LC/ MS (ESI) m/z = 264.04 $[M+H]^+$.

Step 2: Synthesis of tert-butyl (R)-(1-(2-fluoro-3-(1,1,2-trifluoroethyl)phenyl)ethyl)carbamate

To a solution of (R)-(1-(3-(1,1-difluoro-2-hydroxyethyl)-2-fluorophenyl)ethyl)carbamate (221 mg, 0.692 mmol) in DCM (6.92 mL) was added DAST (0.37 mL, 2.77 mmol) at 0 °C under $N_2$ atmosphere, and then the mixture was warmed to room temperature and stirred overnight. The mixture was quenched with sat. $NaHCO_3$ and extracted with DCM. The organic layers were extracted with sat. $NH_4Cl$ and washed with brine. The organic layers were dried over anhydrous $Na_2SO_4$, filtered and concentrated under reduced pressure. It was purified by silica gel column chromatography (hexane : EtOAc = 20:1 to 10:1) to give tert-butyl (R)-(1-(2-fluoro-3-(1,1,2-trifluoroethyl)phenyl)ethyl)carbamate (26 mg, 12% yield) as colorless oil. LC/ MS (ESI) m/z = 265.99 $[M+H]^+$.

Step 3: Synthesis of (R)-1-(2-fluoro-3-(1,1,2-trifluoroethyl)phenyl)ethane-1-amine

TFA (0.15 mL) was added dropwise to a solution of tert-butyl (R)-(1-(2-fluoro-3-(1,1,2-trifluoroethyl)phenyl)ethyl) carbamate (56 mg, 0.174 mmol) in DCM (0.7 mL), and then the mixture was stirred at 0 °C for 1 h. The mixture was warmed to room temperature and quenched with sat. $NaHCO_3$. The organic solvent was removed by filtration under reduced pressure, and the aqueous layers were extracted with DCM. The organic layers were dried over anhydrous $Na_2SO_4$ and concentrated under reduced pressure to give **Intermediate** BF (34.5 mg, 89% yield) as yellow oil. LC/ MS (ESI) m/z = 222.06 $[M+H]^+$.

**Preparation Example BG: 3-methyl-5'-(1-methyl-1H-1,2,3-triazol-5-yl)-2-oxo-2H-[1,3'-bipyridine]-5-carboxylic acid (4-5)**

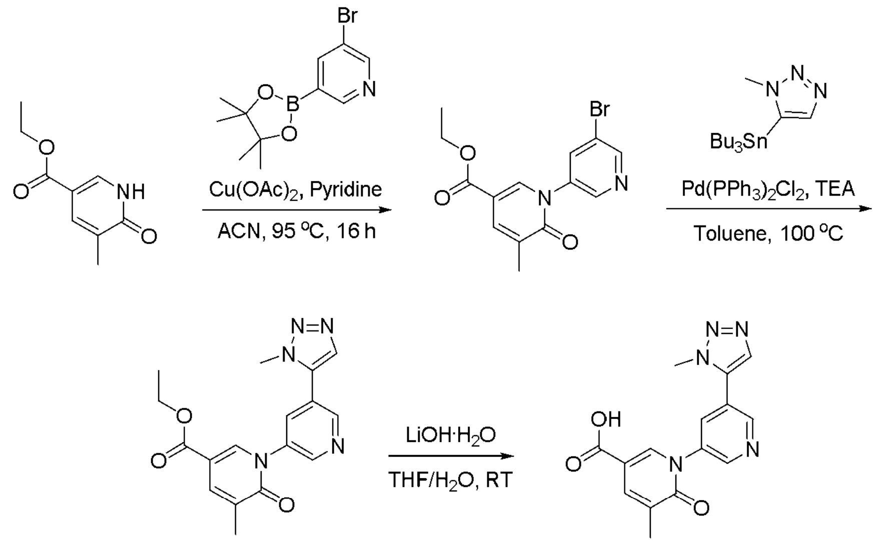


Step 1: Synthesis of ethyl-5'-bromo-3-methyl-2-oxo-2*H*-[1,3'-bipyridine]-5-carboxylate

To a solution of ethyl-5-methyl-6-oxo-1,6-dihydronicotinate (500 mg, 2.76 mmol) in MeCN (21.2 mL) was added 5-bromo-3-pyridineboronic acid pinacol ester (1.18 mg, 4.14 mmol), $Cu(OAc)_2$ (501 mg, 2.76 mmol) and pyridine (0.89 mL, 11 mmol), and then the mixture was stirred at 90 °C for 16 h. Following the addition of water, the reaction mixture was extracted with EtOAc. The combined organic layers were washed with copper sulfate(II) pentahydrate, dried over $Na_2SO_4$ and concentrated under reduced pressure to give crude product. The crude product was purified by silica gel column chromatography (50% EtOAc in hexane) to give ethyl-5'-bromo-3-methyl-2-oxo-2H-[1,3'-bipyridine]-5-carboxylate (460 mg, 49% yield) as a white solid. $^1H$ NMR (400 MHz, $CDCl_3$) δ ppm 8.77 (d, J = 2.0 Hz, 1H), 8.60 (d, J = 2.4 Hz, 1H), 8.06 (d, J = 2.4 Hz, 1H), 8.01 (t, J = 2.0 Hz, 1H), 7.84 (d, J = 0.8 Hz, 1H), 4.34 (q, J = 7.0 Hz, 2H), 2.22 (s, 3H), 1.37 (t, J = 7.2 Hz, 3H); LC/ MS (ESI) m/z = 336.95, 338.95 $[M+H]^+$.

Step 2: Synthesis of ethyl-3-methyl-5'-(1-methyl-1*H*-1,2,3-triazol-5-yl)-2-oxo-2*H*-[1,3'-bipyridine]-5-carboxylate

A solution of ethyl-5'-bromo-3-methyl-2-oxo-2*H*-[1,3'-bipyridine]-5-carboxylate (300 mg, 0.890 mmol), tributyl(1-methyl-1H-1,2,3-triazol-5-yl)stannane (**Intermediate CK**; 993 mg, 2.67 mmol), TEA (124 μL, 0.890 mmol) and $Pd(PPh_3)_2Cl_2$ (62.6 mg, 0.089 mmol) in toluene (8.9 mL) was substituted with nitrogen for 3 times, and then stirred at 100 °C for 17 h under nitrogen atmosphere. The reaction mixture was poured into water (30 mL) and extracted with EtOAc (10 mL x 3). The combined organic layers were washed with brine (10 mL x 5), dried over $Na_2SO_4$ and concentrated under reduced pressure to give crude product. The crude product was purified by silica gel column chromatography (17% EtOAc in hexane) to give ethyl-3-methyl-5'-(1-methyl-1H-1,2,3-triazol-5-yl)-2-oxo-2H-[1,3'-bipyridine]-5-carboxylate (81 mg, 27% yield) as a white solid. $^1H$ NMR (400 MHz, $CDCl_3$) δ 8.79 (d, *J* = 2.0 Hz, 1H), 8.74 (d, *J* = 2.4 Hz, 1H), 8.16 (d, *J* = 2.0 Hz, 1H), 7.97 (t, *J* = 2.2 Hz, 1H), 7.87-7.86 (m, 2H), 4.36 (q, *J* = 7.2 Hz, 2H), 4.18 (s, 3H), 2.24 (s, 3H), 1.37 (t, *J* = 7.2 Hz, 3H); LC/ MS (ESI) m/z = 340.07 $[M+H]^+$.

Step 3: Synthesis of 3-methyl-5'-(1-methyl-1*H*-1,2,3-triazol-5-yl)-2-oxo-2*H*-[1,3'-bipyridine]-5-carboxylic acid

To a solution of 5'-bromo-3-methyl-2-oxo-2H-[1,3'-bipyridine]-5-carboxylate (81 mg, 0.239 mmol) in THF (2.39 mL) was added $LiOH \cdot H_2O$ (30 mg, 0.716 mmol) amd $H_2O$ (1.19 mL), and then the mixture was stirred at 25 °C for 1 h. The reaction mixture was poured into water and adjusted to pH = 1 wtih aq. 1 N HCl, and then **Intermediate BG** (73 mg, 98% yield) was obtained by solid filtration as a white solid. LC/ MS (ESI) m/z = 312.04 $[M+H]^+$.

**Preparation Example BH: 6-oxo-1-[5-[3-(triduteriomethyl)triazol-4-yl]-3-pyridyl]pyridazine-3-carboxylic acid (4-2)**

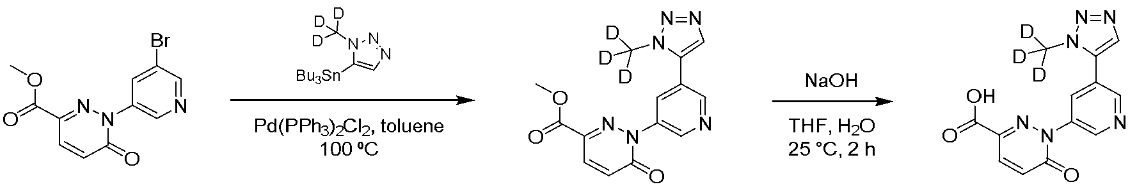


Step 1: Synthesis of methyl-6-oxo-1-[5-[3-(triduteriomethyl)triazol-4-yl]-3-pyridyl]pyridazine-3-carboxylate

A solution of 1-(5-bromo-3-pyridyl)-6-oxo-pyridazine-3-carboxylate (1 g, 3.22 mmol), tributyl-[3-(triduteriomethyl)triazol-4-yl]stannane (**Intermediate CL**; 3.63 g, 9.67 mmol) and $Pd(PPh_3)_2Cl_2$ (226.34 mg, 322.47 μmol) in toluene (10 mL) was substituted with nitrogen for 3 times, and stirred at 100 °C for 16 h under nitrogen atmosphere. The reaction mixture was poured into water (20mL) and extracted with EtOAc (30 mL x 3). The combined organic layers were washed with brine (20 mL x 2), dried over $Na_2SO_4$ and concentrated under reduced pressure to give crude product. The crude product was purified by silica gel column chromatography (5% MeOH in DCM) to give methyl-6-oxo-1-[5-[3-(triduteriomethyl)triazol-4-yl]-3-pyridyl]pyridazine-3-carboxylate (420 mg, 30.57% yield, 74% purity) as a yellow solid. LCMS (ESI) m/z = 316.1 $[M + H]^+$.

Step 2: Synthesis of 6-oxo-1-[5-[3-(triduteriomethyl)triazol-4-yl]-3-pyridyl]pyridazine-3-carboxylic acid

To a solution of methyl-6-oxo-1-[5-[3-(triduteriomethyl)triazol-4-yl]-3-pyridyl]pyridazine-3-carboxylate (420 mg, 1.33 mmol) in THF (8 mL) and $H_2O$ (2 mL) was added NaOH (79.92 mg, 2.00 mmol). After the nitrogen substitution for 3 times, the mixture was stirred at 25 °C for 2 h under nitrogen atmosphere. The mixture was adjusted to pH = 6-7 with aq. 1 N HCl and then concentrated under reduced pressure to give **Intermediate BH** (420 mg, 65.93% yield, 63% purity) as a yellow solid. LCMS (ESI) m/z = 302.1 $[M + H]^+$.

**[Examples]**

**Example 1: N-[(1R)-1-(3-chlorophenyl)ethyl]-6-oxo-1-phenyl-pyridazine-3-carboxamide**

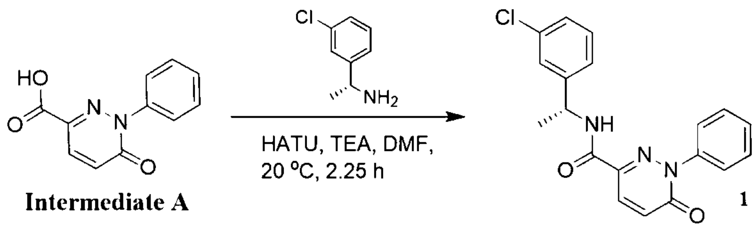


To a solution of Intermediate A (60 mg, 278 μmol) in DMF (1.5 mL), HATU (158 mg, 416 μmol) and TEA (84.3 mg, 833 μmol) were added, and the mixture was stirred at 20 °C for 15 min. The mixture was added with (1R)-1-(3-chlorophenyl)ethanamine (51.8 mg, 333 μmol) and stirred at 20 °C for about 2 h under $N_2$. The reaction mixture was poured into water (10 mL) and extracted with EtOAc. The combined organic layer was washed with brine (10 mL × 3), dried over $Na_2SO_4$ and concentrated under reduced pressure to obtain a crude product, which was purified by prep-HPLC (Phenomenex Luna C18 100*30 mm*3 μm; mobile phase: [water (0.225%FA)-ACN]; B%: 45%-75%, 8min). $CH_3CN$ was removed under reduced pressure, and the remaining solvent was removed by lyophilization to give the compound of Example 1 (46.1 mg, 47.0% yield) as a white solid. $^1H$ NMR (400 MHz, DMSO-$d_6$) δ 8.89 (d, *J* = 8.4 Hz, 1H), 7.88 (d, *J* = 10.0 Hz, 1H), 7.70-7.64 (m, 2H), 7.57-7.52 (m, 2H), 7.50-7.44 (m, 2H), 7.35 (d, *J* = 5.2 Hz, 2H), 7.31-7.26 (m, 1H), 7.14 (d, *J* = 10.0 Hz, 1H), 5.17-5.08 (m, 1H), 1.47 (d, *J* = 7.2 Hz, 3H); LC/MS (ESI) m/z = 354.3 $[M+H]^+$.

**Example 2: N-[(1R)-1-(3-bromophenyl)ethyl]-6-oxo-1-phenyl-pyridazine-3-carboxamide**

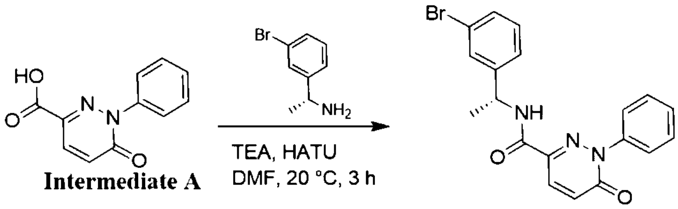

2

To a solution of Intermediate A (1.00 g, 4.63 mmol) in DMF (10 mL), HATU (2.64 g, 6.94 mmol) and TEA (1.40 g, 13.88 mmol) were added. The mixture was stirred at 20 °C for 15 min. The mixture was added with (1R)-1-(3-bromophenyl) ethanamine (1.11 g, 5.55 mmol) and then was stirred at 20 °C for about 3 h under $N_2$ atmosphere. The reaction mixture was poured into water (25 mL) and extracted with EtOAc. The combined organic layer was washed with brine, dried over anhydrous $Na_2SO_4$ and concentrated under reduced pressure to obtain a crude product (1.00 g). 50 mg of the crude product was purified by prep-HPLC (Phenomenex Luna C18 100*30mm*3um; mobile phase: [water (0.225%FA)-ACN]; B%: 50%-80%, 8 min). $CH_3CN$ was removed under reduced pressure, and the remaining solvent was removed by lyophilization to give the compound of Example 2 (9.9 mg, 10.76% yield) as a white solid. $^1H$ NMR (400 MHz, DMSO-$d_6$) δ 8.92 (d, *J* = 8.0 Hz, 1H), 7.89 (d, *J* = *9.6* Hz, 1H), 7.71-7.64 (m, 2H), 7.60 (s, 1H), 7.56 (t, *J* = 7.2 Hz, 2H), 7.51-7.45 (m, 1H), 7.45-7.37 (m, 2H), 7.34-7.23 (m, 1H), 7.14 (d, *J* = 9.6 Hz, 1H), 5.12 (quin, *J* = 7.2, 14.8 Hz 1H), 1.47 (d, *J* = 6.8 Hz, 3H); LC/MS (ESI) m/z = 400.3 $[M+H]^+$.

**Example 3 to Example 10**

The compounds shown in the following table were prepared in the same method as in Example 2 by replacing (1R)-1-(3-bromophenyl)ethanamine with appropriate amine compounds.

[Table 1]

| No. | Structure/Name | Spectral Data |
|---|---|---|
| 3 | 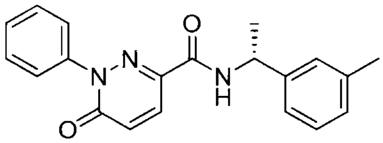 (R)-6-oxo-1-phenyl-N-(1-(m-tolyl)ethyl)-1,6-dihydropyridazine-3-carboxamide | $^1H$ NMR (400 MHz, DMSO-$d_6$) δ 8.77 (d, *J* = 8.4 Hz, 1H), 7.89 (d, J = 10.0 Hz, 1H), 7.67 (d, *J* = 8.0 Hz, 2H), 7.57-7.51 (m, 2H), 7.50-7.44 (m, 1H), 7.23-7.11 (m, 4H), 7.03 (br d, *J* = 6.8 Hz, 1H), 5.10 (quin, *J* = 7.2 Hz, 1H), 2.28 (s, 3H), 1.46 (d, *J* = *7.2* Hz, 3H); LC/MS (ESI) m/z = 334.3 $[M+H]^+$ |
| 4 | 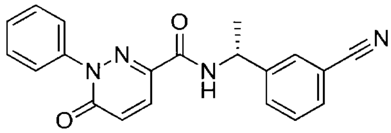 (R)-N-(1-(3-cyanophenyl)ethyl)-6-oxo-1-phenyl-1,6-dihydropyridazine-3-carboxamide | $^1H$ NMR (400 MHz, DMSO-$d_6$) δ 8.90 (d, *J* = 8.4 Hz, 1H), 7.90-7.84 (m, 2H), 7.72 (d, *J* = 4.4 Hz, 1H), 7.69-7.66 (m, 2H), 7.63 (s, 1H), 7.54 (d, *J* = 6.4 Hz, 3H), 7.50-7.45 (m, 1H), 7.14 (d, *J* = 9.6 Hz, 1H), 5.17 (quin, *J* = 7.2 Hz, 1H), 1.49 (d, *J* = 7.2 Hz, 3H); LC/MS (ESI) m/z = 345.3 $[M+H]^+$ |

(continued)

| No. | Structure/Name | Spectral Data |
|---|---|---|
| 5 | 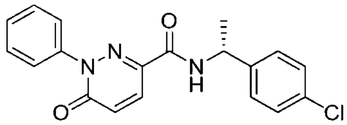 (R)-N-(1-(4-chlorophenyl)ethyl)-6-oxo-1-phenyl-1,6-dihydropyridazine-3-carboxamide | $^1$H NMR (400 MHz, DMSO-$d_6$) δ 8.86 (d, *J* = 8.0 Hz, 1H), 7.88 (d, J = 9.6 Hz, 1H), 7.66 (d, *J* = 7.6 Hz, 2H), 7.55 (t, *J* = 7.6 Hz, 2H), 7.49-7.45 (m, 1H), 7.43-7.35 (m, 4H), 7.14 (d, *J* = 9.6 Hz, 1H), 5.12 (quin, *J* = 7.2 Hz, 1H), 1.46 (d, *J* = 7.2 Hz, 3H); LC/MS (ESI) m/z = 354.3 $[M+H]^+$ |
| 6 | 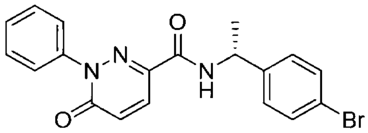 (R)-N-(1-(4-bromophenyl)ethyl)-6-oxo-1-phenyl-1,6-dihydropyridazine-3-carboxamide | $^1$H NMR (400 MHz, DMSO-$d_6$) δ 8.92-8.82 (m, 1H), 7.88 (d, *J* = 9.6 Hz, 1H), 7.66 (d, *J* = 8.0 Hz, 2H), 7.59-7.43 (m, 5H), 7.34 (d, *J* = 8.4 Hz, 2H), 7.14 (d, *J* = 9.6 Hz, 1H), 5.10 (quin, *J* = 7.2 Hz, 1H), 1.46 (d, *J* = 7.2 Hz, 3H); LC/MS (ESI) m/z = 398.2 $[M+H]^+$ |
| 7 | 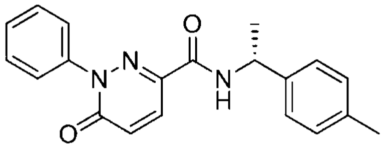 (R)-6-oxo-1-phenyl-N-(1-(p-tolyl)ethyl)-1,6-dihydropyridazine-3-carboxamide | $^1$H NMR (400 MHz, DMSO-$d_6$) δ 8.73 (d, *J* = 8.4 Hz, 1H), 7.88 (d, J = 9.6 Hz, 1H), 7.69-7.63 (m, 2H), 7.57-7.50 (m, 2H), 7.49-7.43 (m, 1H), 7.26 (d, *J* = 8.0 Hz, 2H), 7.16-7.08 (m, 3H), 5.09 (quin, *J* = 7.3 Hz, 1H), 2.25 (s, 3H), 1.45 (d, *J* = 7.2 Hz, 3H); LC/MS (ESI) m/z = 334.4 $[M+H]^+$ |
| 8 | 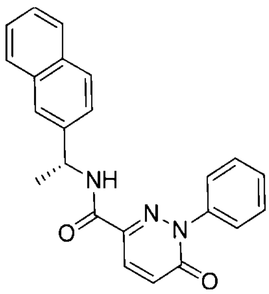 N-[(1R)-1-(2-naphthyl)ethyl]-6-oxo-1-phenyl-pyridazine-3-carboxamide | $^1$H NMR (400 MHz, DMSO-$d_6$) δ 8.93 (d, *J* = 8.4 Hz, 1H), 7.92-7.85 (m, 5H), 7.70-7.66 (m, 2H), 7.59-7.45 (m, 6H), 7.14 (d, *J* = 9.6 Hz, 1H), 5.31 (quin, *J* = 7.2 Hz, 1H), 1.58 (d, *J* = 7.2 Hz, 3H); LC/MS (ESI) m/z = 370.4 $[M+H]^+$ |

(continued)

| No. | Structure/Name | Spectral Data |
|---|---|---|
| 9 | 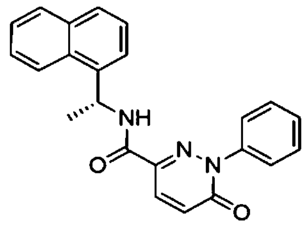 N-[(1R)-1-(1-naphthyl)ethyl]-6-oxo-1-phenyl-pyridazine-3-carboxamide | $^1$H NMR (400 MHz, DMSO-$d_6$) δ 8.99 (d, *J* = 8.0 Hz, 1H), 8.19 (d, *J* = 8.4 Hz, 1H), 7.96-7.87 (m, 2H), 7.83 (d, *J* = 8.0 Hz, 1H), 7.68-7.60 (m, 3H), 7.60-7.50 (m, 4H), 7.50-7.41 (m, 2H), 7.14 (d, *J* = 10.0 Hz, 1H), 5.93 (quin, *J* = 7.2, 14.4 Hz, 1H), 1.61 (d, *J* = 6.8 Hz, 3H); LC/MS (ESI) m/z = 370.3 $[M+H]^+$ |
| 10 | 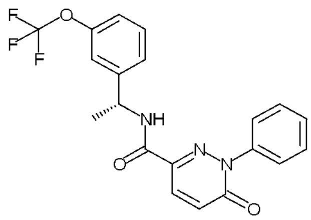 6-oxo-1-phenyl-N-[(1R)-1-[3-(trifluoromethoxy)phenyl]ethyl]pyridazine-3-carboxamide | $^1$H NMR (400 MHz, DMSO-$d_6$) δ 8.94 (d, J = 8.4 Hz, 1H), 7.88 (d, J = 10.0 Hz, 1H), 7.67 (d, J = 7.6 Hz, 2H), 7.55 (t, J = 7.2 Hz, 2H), 7.49 - 7.38 (m, 4H), 7.22 (br d, J = 7.6 Hz, 1H), 7.14 (d, J = 10.4 Hz, 1H), 5.17 (quin, J = 7.2 Hz, 1H), 1.48 (d, J = 7.2 Hz, 3H); LC/MS (ESI) m/z = 404.3 $[M+H]^+$ |

**Example 11: N-[(1R)-1-(3-methylsulfonylphenyl)ethyl]-6-oxo-1-phenyl-pyridazine-3-carboxamide**

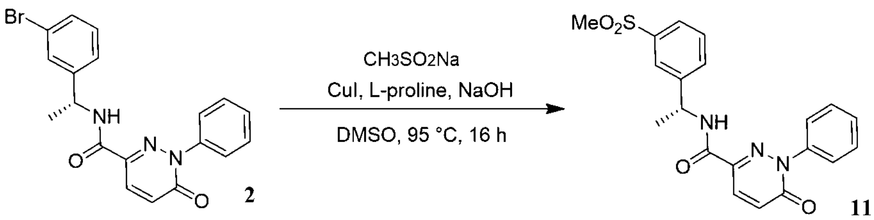

2
11

A mixture of the compound of Example 2 (50 mg, 125.55 μmol), $CH_3SO_2Na$ (15.38 mg, 150.66 μmol), CuI (2.39 mg, 12.55 μmol), L-proline (2.89 mg, 25.11 μmol) and NaOH (1.00 mg, 25.11 μmol) in DMSO (1.5 mL) was degassed and purged with $N_2$ for 3 times, and then the mixture was stirred at 95 °C for 16 h under $N_2$ atmosphere. The reaction mixture was poured into water (10 mL) and extracted with EtOAc. The combined organic layer was washed with brine, dried over anhydrous $Na_2SO_4$ and concentrated under reduced pressure to obtain a crude product. The product was purified by prep-HPLC (Phenomenex Luna C18 100*30 mm*3 μm; mobile phase: [water (0.225%FA)-ACN]; B%: 35%-65%, 8 min). ACN was removed under reduced pressure, and the remaining solvent was removed by lyophilization to give the compound of Example 11 (14.5 mg, 29.06% yield). $^1$H NMR (400 MHz, DMSO-$d_6$) δ 9.02 (d, *J* = 8.3 Hz, 1H), 7.96 (s, 1H), 7.89 (d, *J* = 9.6 Hz, 1H), 7.80 (d, *J* = 7.6 Hz, 1H), 7.75 (d, *J* = 8.0 Hz, 1H), 7.70-7.64 (m, 2H), 7.64-7.58 (m, 1H), 7.58-7.51 (m, 2H), 7.50-7.44 (m, 1H), 7.14 (d, *J* = 9.6 Hz, 1H), 5.24 (quin, *J* = 7.3 Hz, 1H), 3.20 (s, 3H), 1.51 (d, *J* = 7.2 Hz, 3H); LC/MS (ESI) m/z = 398.3 $[M+H]^+$.

**Example 12: N-[(1R)-1-(4-methylsulfonylphenyl)ethyl]-6-oxo-1-phenyl-pyridazine-3-carboxamide**

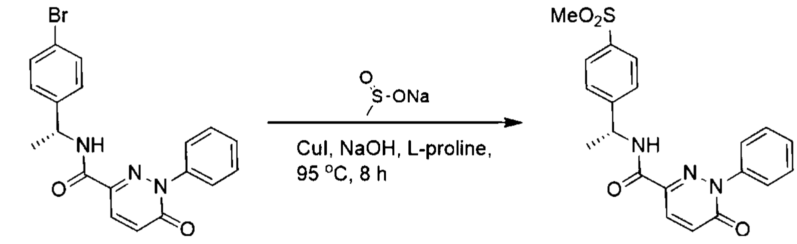


12

The compound of Example 12 was prepared as a white solid in the same method as in Example 11, except that N-[(1R)-1-(4-bromophenyl)ethyl]-6-oxo-1-phenyl-pyridazine-3-carboxamide was used instead of the compound of Example 2. $^1$H NMR (400 MHz, DMSO-$d_6$) δ 8.97 (d, *J* = 6.8 Hz, 1H), 7.87 (d, *J* = 5.6 Hz, 3H), 7.66 (s, 4H), 7.55 (s, 2H), 7.14 (d, *J* = 8.8 Hz, 1H), 5.20 (s, 1H), 3.19-3.16 (m, 3H), 1.50 (d, *J* = 5.2 Hz, 3H); LC/MS (ESI) m/z = 398.3 $[M+H]^+$.

**Example 13: 6-oxo-N-[(1R)-1-(3-phenoxyphenyl)ethyl]-1-phenyl-pyridazine-3-carboxamide**

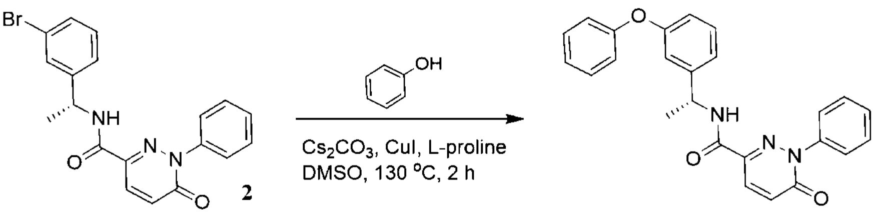


2

13

A mixture of the compound of Example 2 (100 mg, 251.10 μmol), phenol (35.45 mg, 376.65 μmol), CuI (9.56 mg, 50.22 μmol), $CS_2CO_3$ (245.44 mg, 753.29 μmol) and L-proline (5.78 mg, 50.22 μmol) in DMSO (2 mL) was stirred at 130 °C for 2 hr under $N_2$ atmosphere and microwave. The reaction mixture was poured into water (10 mL) and extracted with EtOAc, and the combined organic layer was washed with brine, dried over $Na_2SO_4$ and concentrated under reduced pressure to obtain a crude product. The crude product was purified by prep-HPLC (Gemini NX C18 5 um*10*150 mm;mobile phase: [ACN/EtOH(0.1%$NH_3H_2O$)];B%:25%-75%, 30min), ACN was removed under reduced pressure, and the remaining solvent was removed by lyophilization to give the compound of Example 13 (2.2 mg, 2.12% yield) as a white solid. $^1$H NMR (400 MHz, DMSO-$d_6$) δ 8.88-8.79 (m, 1H), 7.89 (d, *J* = 9.6 Hz, 1H), 7.93-7.85 (m, 1H), 7.92-7.84 (m, 1H), 7.68-7.62 (m, 2H), 7.57-7.51 (m, 2H), 7.48 (d, *J* = 7.2 Hz, 1H), 7.39-7.31 (m, 3H), 7.18-7.08 (m, 4H), 7.01-6.95 (m, *J* = 8.0 Hz, 2H), 6.86-6.80 (m, 1H), 5.13 (quin, *J* = 7.6 Hz, 1H), 1.47 (d, *J* = 7.2 Hz, 3H); LC/MS (ESI) m/z = 412.3 $[M+H]^+$.

**Example 14: N-[(1R)-1-(3-cyclopropylphenyl)ethyl]-6-oxo-1-phenyl-pyridazine-3-carboxamide**

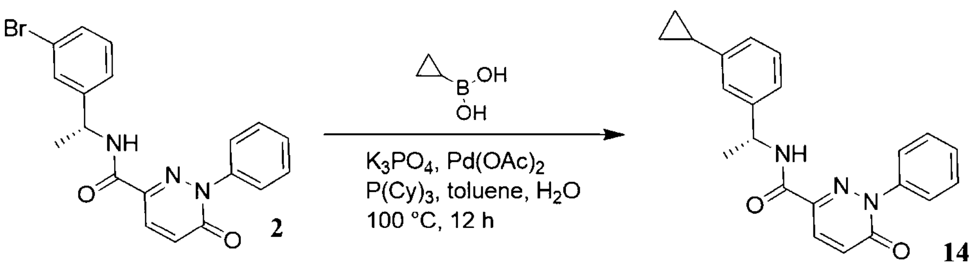


2

14

A mixture of the compound of Example 2 (50 mg, 125.55 μmol), cyclopropylboronic acid (14.02 mg, 163.21 μmol), $K_3PO_4$ (93.27 mg, 439.42 μmol), $Pd(OAc)_2$ (2.82 mg, 12.55 μmol) and $P(Cy)_3$ (7.04 mg, 25.11 μmol) in toluene (1 mL) and $H_2O$ (0.1 mL) was degassed and purged with $N_2$ for 3 times, and then the mixture was stirred at 100 °C for 12 h under $N_2$ atmosphere. The reaction mixture was poured into water (20 mL) and extracted with EtOAc. The combined organic layer was washed with brine, dried over $Na_2SO_4$ and concentrated under reduced pressure to obtain a crude product. The product was purified by prep-HPLC (Phenomenex Luna C18 100*30 mm*3 μm, mobile phase: [water(0.225% FA)-ACN];B%: 48%-78%,15 min), ACN was removed under reduced pressure, and the remaining solvent was removed by lyophilization to give the compound of Example 14 (15.8 mg, 35.01% yield) as a white solid. $^1$H NMR

(400MHz, 400MHz, DMSO-$d_6$) δ 8.79 (d, *J* = 8.6 Hz, 1H), 7.88 (d, *J* = 9.6 Hz, 1H), 7.71-7.64 (m, 2H), 7.60-7.50 (m, 2H), 7.50-7.44 (m, 1H), 7.21-7.08 (m, 4H), 6.90 (d, *J* = 7.6 Hz, 1H), 5.09 (quin, *J* = 7.6 Hz, 1H), 1.96-1.80 (m, 1H), 1.45 (d, *J* = 7.2 Hz, 3H), 0.99-0.84 (m, 2H), 0.69-0.57 (m, 2H); LC/MS (ESI) m/z = 360.3 $[M+H]^+$.

**Example 15: 6-oxo-1-phenyl-N-[(1R)-1-[3-(trifluoromethyl)phenyl]ethyl]pyridazine-3-carboxamide**

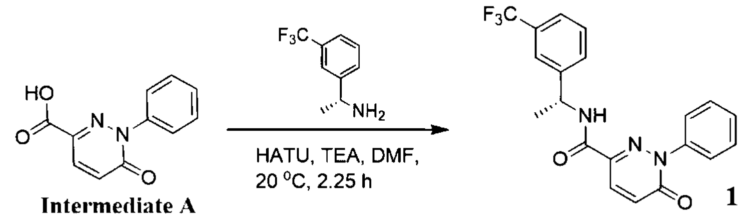


15

To a solution of Intermediate A (40 mg, 185 μmol) in DMF (1.5 mL), HATU (106 mg, 278 μmol) and TEA (56.2 mg, 555 μmol) were added, followed by stirring at 20 °C for 15 min. Then, the mixture was added with (1R)-1-[3-(trifluoromethyl)phenyl]ethanamine (42.0 mg, 222 μmol) and stirred at 20 °C for 2 h under $N_2$. The reaction mixture was poured into water (10 mL) and extracted with EtOAc. The combined organic layer was washed with brine, dried over anhydrous $Na_2SO_4$ and concentrated under reduced pressure to obtain a crude product, which was purified by prep-HPLC (Phenomenex Luna C18 100*30 mm*3 μm, mobile phase: [water (0.225% FA)-ACN]; B%: 50%-80%, 8 min). ACN was removed under reduced pressure, and the remaining solvent was removed by lyophilization to give the compound of Example 15 (34.4 mg, 48.0% yield) as a white solid. $^1H$ NMR (400 MHz, DMSO-$d_6$) δ 8.99 (d, *J* = 8.4 Hz, 1H), 7.88 (d, *J* = 9.6 Hz, 1H), 7.76 (s, 1H), 7.72-7.65 (m, 3H), 7.61-7.52 (m, 4H), 7.50-7.45 (m, 1H), 7.14 (d, *J* = 9.6 Hz, 1H), 5.17-5.26 (m, 1H), 1.50 (d, *J* = 7.2 Hz, 3H); LC/MS (ESI) m/z = 388.3 $[M+H]^+$.

**Example 16: N-[(1R)-1-[3-amino-5-(trifluoromethyl)phenyl]ethyl]-6-oxo-1-phenyl-pyridazine-3-carboxamide**

Step 1: Synthesis of N-[(1R)-1-[3-nitro-5-(trifluoromethyl)phenyl]ethyl]-6-oxo-1-phenyl-pyridazine-3-carboxamide

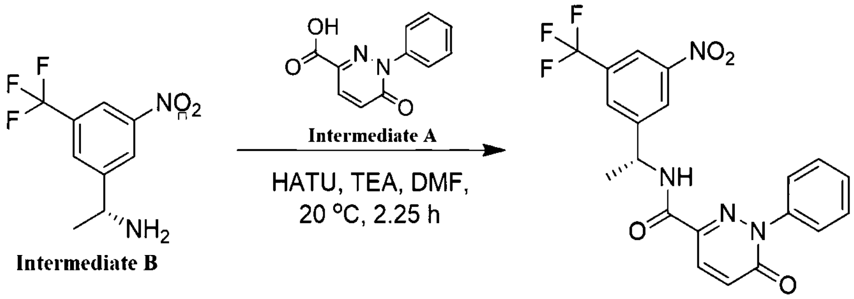


To a solution of Intermediate A (65 mg, 300 μmol) in DMF (1.5 mL), HATU (171 mg, 451 μmol) and TEA (91.3 mg, 902 μmol) were added, followed by stirring at 20 °C for 15 min. Then, the mixture was added with Intermediate B (84.5 mg, 361 μmol) and stirred at 20 °C for 2 h under $N_2$. The reaction mixture was poured into water (15 mL) and extracted with EtOAc. The combined organic layer was washed with brine, dried over $Na_2SO_4$ and concentrated under reduced pressure to obtain a crude product, which was purified by silica gel column chromatography (25% EtOAc in petroleum ether) to give N-[(1R)-1-[3-nitro-5-(trifluoromethyl)phenyl]ethyl]-6-oxo-1-phenyl-pyridazine-3-carboxamide (99 mg, 76.2% yield) as yellow oil. LC/MS (ESI) m/z = 433.0 $[M+H]^+$.

Step 2: Synthesis of N-[(1R)-1-[3-amino-5-(trifluoromethyl)phenyl]ethyl]-6-oxo-1-phenyl-pyridazine-3-carboxamide

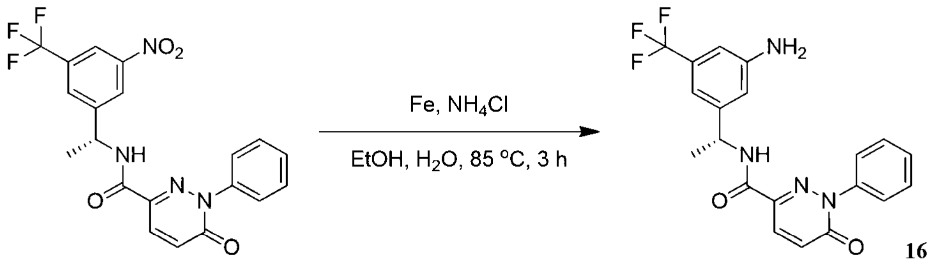


16

To a solution of N-[(1R)-1-[3-nitro-5-(trifluoromethyl)phenyl]ethyl]-6-oxo-1-phenyl-pyridazine-3-carboxamide (99 mg, 229 μmol) in EtOH (2 mL) and $H_2O$ (0.2 mL), Fe (63.9 mg, 1.1 mmol) and $NH_4Cl$ (98 mg, 1.8 mmol) were added, followed by stirring at 85 °C for 3 h. The reaction mixture was adjusted to pH=8-9 with aq. $NaHCO_3$ and extracted with EtOAc. The combined organic layer was washed with brine, dried over $Na_2SO_4$ and concentrated under reduced pressure to obtain a crude product, which was purified by prep-HPLC (Phenomenex Gemini-NX C18 75*30mm*3μm, mobile phase: [water(0.225%FA)-ACN]; B%: 50%-80%, 8min). ACN was removed under reduced pressure, and the remaining solvent was removed by lyophilization to give the compound of Example 16 (24.5 mg, 26.6% yield) as a white solid. $^1H$ NMR (400 MHz, DMSO-$d_6$) δ 8.85 (d, *J* = 8.4 Hz, 1H), 7.89 (d, *J* = 10.0 Hz, 1H), 7.70-7.63 (m, 2H), 7.57-7.51 (m, 2H), 7.50-7.44 (m, 1H), 7.14 (d, *J* = 9.6 Hz, 1H), 6.82 (s, 1H), 6.78 (s, 1H), 6.70 (s, 1H), 5.54 (s, 2H), 4.98-5.07 (m, 1H), 1.44 (d, *J* = 7.2 Hz, 3H); LC/MS (ESI) m/z = 403.3 $[M+H]^+$.

**Example 17: N-[(1R)-1-(3-chlorophenyl)ethyl]-6-oxo-1-tetrahydropyran-4-yl-pyridazine-3-carboxamide**

Step 1: Synthesis of methyl-6-oxo-1-tetrahydropyran-4-yl-pyridazine-3-carboxylate

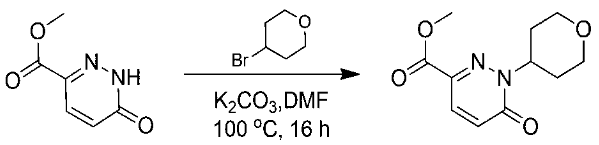


To a solution of methyl-6-oxo-1H-pyridazine-3-carboxylate (300 mg, 1.95 mmol) in DMF (5 mL), $K_2CO_3$ (538.0 mg, 3.89 mmol) and 4-bromotetrahydropyran (481.8 mg, 2.92 mmol) were added, followed by stirring at 100 °C for 16 h. The reaction mixture was poured into water (20 mL) and extracted with EtOAc. The combined organic layer was washed with brine, dried over $Na_2SO_4$ and concentrated under reduced pressure to obtain a crude product. The product was purified by silica gel column chromatography (28% EtOAc in petroleum ether) to give methyl-6-oxo-1-tetrahydropyran-4-yl-pyridazine-3-carboxylate (266.0 mg, 57.36% yield) as a white solid. $^1H$ NMR (400 MHz, DMSO-$d_6$) δ 7.87-7.82 (m, 1H), 7.05-7.00 (m, 1H), 5.12-4.95 (m, 1H), 3.95-3.99 (m, 2H), 3.89-3.85 (m, 3H), 3.53-3.43 (m, 2H), 1.97-1.83 (m, 2H), 1.73-1.77 (m, 2H); LC/MS (ESI) m/z = 239.0 $[M+H]^+$.

Step 2: Synthesis of 6-oxo-1-tetrahydropyran-4-yl-pyridazine-3-carboxylic acid

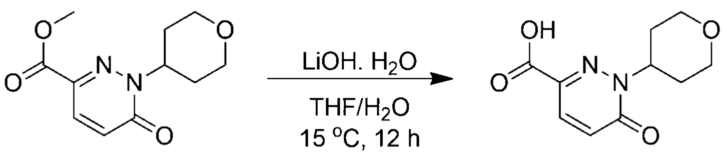


To a solution of methyl-6-oxo-1-tetrahydropyran-4-yl-pyridazine-3-carboxylate (100 mg, 419.8 μmol) in THF (2 mL) and $H_2O$ (1 mL) was added $LiOH \cdot H_2O$ (70.46 mg, 1.68 mmol), followed by stirring at 15 °C for 12 h. The reaction mixture was acidified with aq. 1N HCl (pH = 3-4) and then extracted with EtOAc. The organic layer was washed with water and brine, dried over $Na_2SO_4$, filtered and concentrated under reduced pressure to give 6-oxo-1-tetrahydropyran-4-yl-pyridazine-3-carboxylic acid (88 mg, crude) as a white solid. LC/MS (ESI) m/z = 225.1 $[M+H]^+$.

Step 3: Synthesis of N-[(1R)-1-(3-chlorophenyl)ethyl]-6-oxo-1-tetrahydropyran-4-yl-pyridazine-3-carboxamide

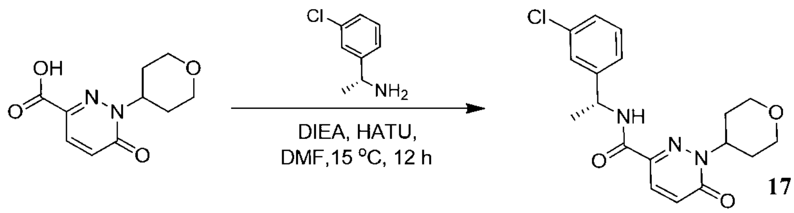


**17**

To a solution of 6-oxo-1-tetrahydropyran-4-yl-pyridazine-3-carboxylic acid (88 mg, 392.5 μmol) in DMF (1.5 mL), (1R)-1-(3-chlorophenyl)ethanamine (73.30 mg, 471.0 μmol), HATU (194.0 mg, 510.2 μmol) and DIEA (152.2 mg, 1.18 mmol) were added, followed by stirring at 15 °C for 12 h. The reaction mixture was poured into water (20 mL) and extracted with EtOAc. The combined organic layer was washed with brine, dried over $Na_2SO_4$ and concentrated under reduced pressure to obtain a crude product, which was purified by prep-HPLC (Phenomenex Luna C18 100*3 0 mm*3 μm, mobile phase: [water (0.225% FA)-ACN]; B%: 40%-70%, 8 min). ACN was removed under reduced pressure, and the remaining solvent was removed by lyophilization to give the compound of Example 17 (38.5 mg, 27.11% yield). $^1$H NMR (400 MHz, DMSO-$d_6$) δ 8.85 (d, *J* = 8.4 Hz, 1H), 7.81 (d, *J* = 9.6 Hz, 1H), 7.49 (s, 1H), 7.42-7.34 (m, 2H), 7.34-7.27 (m, 1H), 7.01 (d, *J* = *9.6* Hz, 1H), 5.17 (quin, *J* = 7.2 Hz, 1H), 5.10-4.99 (m, 1H), 3.99-4.03 (m, 2H), 3.56-3.47 (m, 2H), 2.30-2.18 (m, 2H), 1.74-1.66 (m, 2H), 1.53 (d, *J*= 7.2 Hz, 3H); LC/MS (ESI) m/z = 362.3 [M+H]$^+$.

**Example 18: 1-(1-acetyl-4-piperidyl)-N-[(1R)-1-(3-chlorophenyl)ethyl]-6-oxo-pyridazine-3-carboxamide**

Steps 1 to 3: Synthesis of tert-butyl 4-[3-[[(1R)-1-(3-chlorophenyl)ethyl]carbamoyl]-6-oxo-pyridazin-1-yl]piperidine-1-carboxylate

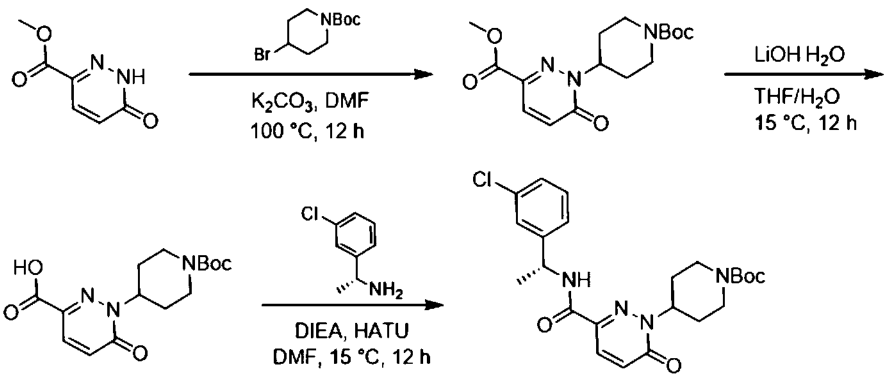


Tert-butyl 4-[3-[[(1R)-1-(3-chlorophenyl)ethyl]carbamoyl]-6-oxo-pyridazin-1-yl]piperidine-1-carboxylate was obtained as yellow oil in the same method as in Steps 1 to 3 of Example 17, except that tert-butyl 4-bromopiperidine-1-carboxylate was used instead of 4-bromotetrahydropyran in Step 1. $^1$H NMR (400 MHz, CHLOROFORM-*d*) δ 7.94 (d, *J* = 9.6 Hz, 1H), 7.34 (s, 1H), 7.31-7.27 (m, 2H), 7.25-7.22 (m, 1H), 7.14 (d, *J* = 8.0 Hz, 1H), 6.98 (d, *J* = *9.6* Hz, 1H), 5.23 (quin, *J* = 7.2 Hz, 1H), 5.14-5.03 (m, 1H), 4.30 (br s, 2H), 2.95-2.87 (m, 2H), 1.94-1.88 (m, 4H), 1.61 (d, *J* = 7.2 Hz, 3H), 1.48 (s, 9H).

Step 4: Synthesis of N-[(1R)-1-(3-chlorophenyl)ethyl]-6-oxo-1-(4-piperidyl)pyridazine-3-carboxamide

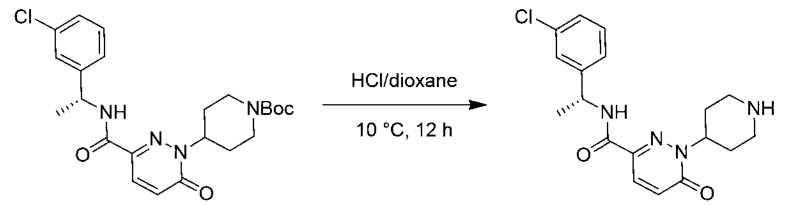


To a solution of tert-butyl 4-[3-[[(1R)-1-(3-chlorophenyl)ethyl]carbamoyl]-6-oxo-pyridazin-1-yl]piperidine-1-carboxylate (417 mg, 904.65 μmol) in dioxane (3 mL) was added HCl/dioxane (4 M, 3 mL), followed by stirring at 10 °C for 12 h. The mixture was concentrated under reduced pressure to give N-[(1R)-1-(3-chlorophenyl)ethyl]-6-oxo-1-(4-piperidyl) pyridazine-3-carboxamide (350 mg, 100% yield, HCl salt) as a yellow solid. LC/MS (ESI) m/z = 261.0 [M+H]$^+$.

Step 5: Synthesis of 1-(1-acetyl-4-piperidyl)-N-[(1R)-1-(3-chlorophenyl)ethyl]-6-oxo-pyridazine-3-carboxamide

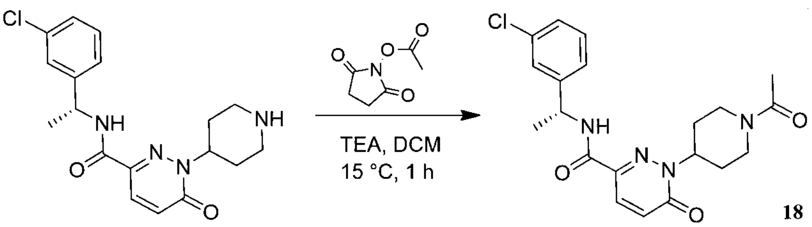


18

To a solution of N-[(1R)-1-(3-chlorophenyl)ethyl]-6-oxo-1-(4-piperidyl)pyridazine-3-carboxamide (50 mg, 125.85 μmol, HCl salt) in DCM (1 mL), TEA (38.20 mg, 377.55 μmol) and (2,5-dioxopyrrolidin-1-yl) acetate (20 mg, 127.29 μmol) were added, and the mixture was stirred at 15 °C for 1 hour. The reaction mixture was poured into water (20 mL) and extracted with EtOAc. The combined organic layer was washed with brine, dried over $Na_2SO_4$ and concentrated under reduced pressure to obtain a crude product. The crude product was purified by prep-HPLC (Phenomenex Luna C18 100*30 mm*3 μm, mobile phase: [water (0.225% FA)-ACN]; B%: 30%-90%, 8 min). ACN was removed under reduced pressure, and the remaining solvent was removed by lyophilization to give the compound of Example 18 (19.2 mg, 37.87% yield,) as a white solid. $^1$H NMR (400 MHz, DMSO-$d_6$) δ 8.78 (br d, *J* = 8.8 Hz, 1H), 7.80 (d, *J* = 9.6 Hz, 1H), 7.47 (br d, *J* = 4.4 Hz, 1H), 7.40-7.33 (m, 2H), 7.30 (td, *J*=2.8, 5.6 Hz, 1H), 7.01 (d, *J* = 9.6 Hz, 1H), 5.20-5.10 (m, 1H), 5.05-4.95 (m, 1H), 4.58 (d, *J* = 13.6 Hz, 1H), 3.99 (d, *J* = 11.6 Hz, 1H), 3.22 (s, 1H), 2.75-2.67 (m, 1H), 2.04 (s, 3H), 2.04-1.92 (m, 2H), 1.89-1.76 (m, 2H), 1.52 (d, *J* = 7.2 Hz, 3H); LC/MS (ESI) m/z = 403.3 [M+H]$^+$.

**Example 19: (R)-N-(1-(3-chlorophenyl)ethyl)-1-(1-(methylsulfonyl)piperidin-4-yl)-6-oxo-1,6-dihydropyridazine-3-carboxamide**

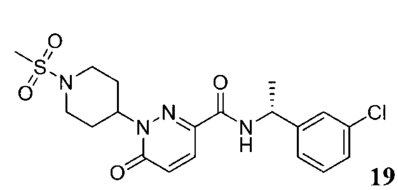

19

The compound of Example 19 was obtained in the same method as in Example 18, except that methanesulfonyl chloride was used instead of (2,5-dioxopyrrolidin-1-yl)acetate in Step 5. $^1$H NMR (400MHz, DMSO-$d_6$) δ 8.84 (d, J = 8.4 Hz, 1H), 7.81 (d, J = 9.6 Hz, 1H), 7.47 (s, 1H), 7.41-7.34 (m, 2H), 7.34-7.27 (m, 1H), 7.01 (d, J = 9.6 Hz, 1H), 5.16 (quin, J = 7.2 Hz, 1H), 4.98-4.87 (m, 1H), 3.72 (d, J = 12.0 Hz, 2H), 3.01-2.93 (m, 2H), 2.92 (s, 3H), 2.25 (q, J = 12.0 Hz, 2H), 1.89 (d, J = 11.2 Hz, 2H), 1.53 (d, J = 7.2 Hz, 3H). LC/MS (ESI) m/z = 403.3 [M+H]$^+$.

**Example 20: N-[(1R)-1-[3-amino-5-(trifluoromethyl)phenyl]ethyl]-6-oxo-1-(2-pyridyl)pyridazine-3-carboxamide**

Step 1: Synthesis of methyl-6-oxo-1-(2-pyridyl)pyridazine-3-carboxylate

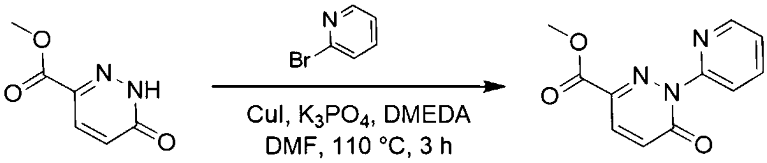


A mixture of methyl-6-oxo-1H-pyridazine-3-carboxylate (200 mg, 1.30 mmol), 2-bromopyridine (410.05 mg, 2.60 mmol), DMEDA (68.63 mg, 778.60 μmol), CuI (123.57 mg, 648.83 μmol) and $K_3PO_4$ (688.62 mg, 3.24 mmol) in DMF (5 mL) was degassed and purged with $N_2$ for 3 times, and then the mixture was stirred at 110 °C for 3 h under $N_2$ atmosphere. The reaction mixture was poured into water (20 mL) and extracted with EtOAc. The combined organic layer was washed with brine, dried over $Na_2SO_4$ and concentrated under reduced pressure to obtain a crude product. The product was purified by silica gel column chromatography (59% EtOAc in petroleum ether) to give methyl-6-oxo-1-(2-pyridyl)pyridazine-3-carboxylate (100 mg, 30.66% yield) as yellow oil. LC/MS (ESI) m/z = 232.0 $[M+H]^+$.

Step 2: Synthesis of 6-oxo-1-(2-pyridyl)pyridazine-3-carboxylic acid

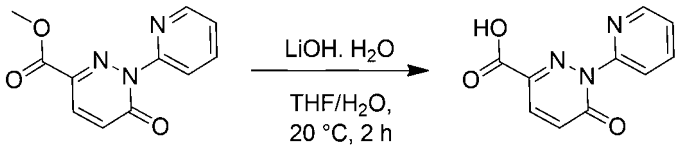


To a solution of methyl-6-oxo-1-(2-pyridyl)pyridazine-3-carboxylate (100 mg, 432.51 μmol) in THF (2 mL), $LiOH{\cdot}H_2O$ (72.60 mg, 1.73 mmol) and $H_2O$ (1 mL) were added, followed by stirring at 20 °C for 2 h. The mixture was adjusted to pH = 3-4 and concentrated under reduced pressure to obtain a residue, which was purified by prep-HPLC (Phenomenex Luna C18 100*30 mm*3 μm, mobile phase: [water(0.225% FA)-ACN];B%: 0%-30%, 15min). ACN was removed under reduced pressure, and the remaining solvent was removed by lyophilization to give 6-oxo-1-(2-pyridyl) pyridazine-3-carboxylic acid (40 mg, 40.45% yield) as a white solid. LC/MS (ESI) m/z = 218.0 $[M+H]^+$.

Step 3: Synthesis of N-[(1R)-1-[3-nitro-5-(trifluoromethyl)phenyl]ethyl]-6-oxo-1-(2-pyridyl)pyridazine-3-carboxamide

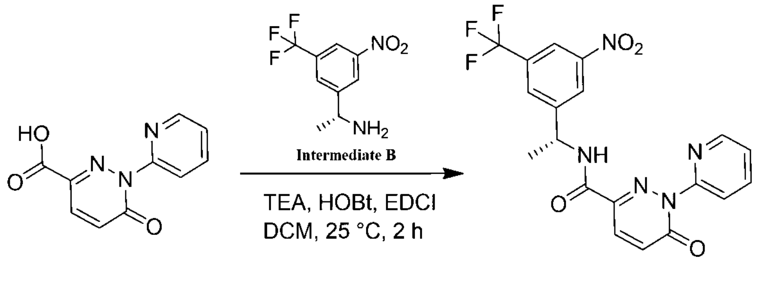


To a solution of 6-oxo-1-(2-pyridyl)pyridazine-3-carboxylic acid (30 mg, 138.13 μmol) and Intermediate B (37.38 mg, 138.13 μmol) in DCM (1 mL), TEA (41.93 mg, 414.40 μmol), HOBt (22.40 mg, 165.76 μmol) and EDCI (31.78 mg, 165.76 μmol) were added, followed by stirring at 25 °C for 2 h. The reaction mixture was poured into water (20 mL) and extracted with EtOAc. The combined organic layer was washed with brine, dried over $Na_2SO_4$ and concentrated under reduced pressure to obtain a crude product. The product was purified by silica gel column chromatography (80% EtOAc in petroleum ether) to give N-[(1R)-1-[3-nitro-5-(trifluoromethyl)phenyl]ethyl]-6-oxo-1-(2-pyridyl)pyridazine-3-carboxamide (59 mg, 89.34% yield) as colorless solid. LC/MS (ESI) m/z = 434.0 $[M+H]^+$.

Step 4: Synthesis of N-[(1R)-1-[3-amino-5-(trifluoromethyl)phenyl]ethyl]-6-oxo-1-(2-pyridyl)pyridazine-3-carboxamide

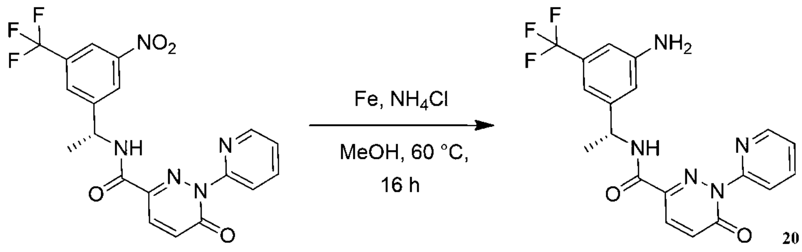


20

To a solution of N-[(1R)-1-[3-nitro-5-(trifluoromethyl)phenyl]ethyl]-6-oxo-1-(2-pyridyl)pyridazine-3-carboxamide (59 mg, 136.15 μmol) in sat. aq. $NH_4Cl$ (1 mL) and MeOH (3 mL) was added Fe (60.83 mg, 1.09 mmol), followed by stirring at 60 °C for 16 h. The mixture was filtrated, and the filtrate was concentrated under reduced pressure to obtain a crude product, which was then purified by prep-HPLC (Phenomenex Gemini-NX C18 75*30 mm*3 μm, mobile phase:[water(0.05% $NH_3H_2O$+10 mM $NH_4HCO_3$)-ACN];B%: 21%-41%,10 min). ACN was removed under reduced pressure, and the remaining solvent was removed by lyophilization to give the compound of Example 20 (7.3 mg, 12.70% yield) as a light-yellow solid. $^1H$ NMR (400 MHz, DMSO-$d_6$) δ 8.87 (d, $J$ = 8.4 Hz, 1H), 8.64 (dd, $J$ = 1.2, 4.8 Hz, 1H), 8.11-8.03 (m, 1H), 7.94 (d, $J$ = 10.0 Hz, 1H), 7.69 (d, $J$ = 8.0 Hz, 1H), 7.61-7.54 (m, 1H), 7.17 (d, $J$ = 10.0 Hz, 1H), 6.79 (d, $J$ = 15.2 Hz, 2H), 6.69 (s, 1H), 5.54 (s, 2H), 5.02 (quin, $J$ = 7.2 Hz, 1H), 1.42 (d, $J$ = 7.2 Hz, 3H); LC/MS (ESI) m/z = 404.3 $[M+H]^+$.

**Example 21: N-[(1R)-1-[3-amino-5-(trifluoromethyl)phenyl]ethyl]-6-oxo-1-(4-pyridyl)pyridazine-3-carboxamide**

Step 1: Synthesis of methyl-6-oxo-1-(4-pyridyl)pyridazine-3-carboxylate

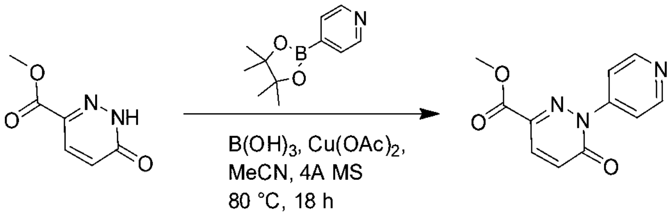


A mixture of methyl-6-oxo-1H-pyridazine-3-carboxylate (300 mg, 1.95 mmol), 4-(4,4,5,5-tetramethyl-1,3,2-dioxaborolan-2-yl)pyridine (478.98 mg, 2.34 mmol), $Cu(OAc)_2$ (70.71 mg, 389.30 μmol), 4A MS (300 mg) and boric acid (240.72 mg, 3.89 mmol) in ACN (8 mL) was degassed and purged with $O_2$ for 3 times, and then the mixture was stirred at 80 °C for 18 h under $O_2$ atmosphere. The reaction mixture was poured into water (20 mL) and extracted with EtOAc. The combined organic layer was washed with brine, dried over $Na_2SO_4$ and concentrated under reduced pressure to obtain a crude product, methyl-6-oxo-1-(4-pyridyl)pyridazine-3-carboxylate (380 mg, 67.55% yield) as a white solid. LC/MS (ESI) m/z = 232.0 $[M+H]^+$.

Step 2: Synthesis of 6-oxo-1-(4-pyridyl)pyridazine-3-carboxylic acid

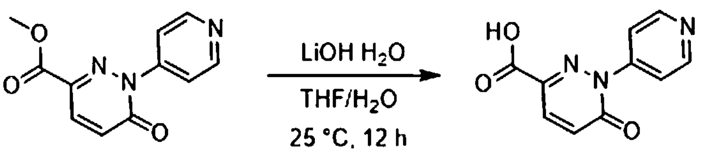


To a solution of methyl-6-oxo-1-(4-pyridyl)pyridazine-3-carboxylate (180 mg, 778.52 μmol) in THF (2 mL), LiOH·$H_2O$ (65.34 mg, 1.56 mmol) and $H_2O$ (1 mL) were added, and the mixture was stirred at 25 °C for 12 h. The reaction mixture was acidified with aq. 1 N HCl (pH = 3-4) and extracted with EtOAc, and the material precipitated in the aqueous

layer was filtrated to obtain 6-oxo-1-(4-pyridyl)pyridazine-3-carboxylic acid (50 mg, 28.09% yield) as a white solid (filter cake). LC/MS (ESI) m/z = 218.0 [M+H]+.

Step 3: Synthesis of N-[(1R)-1-[3-amino-5-(trifluoromethyl)phenyl]ethyl]-6-oxo-1-(4-pyridyl)pyridazine-3-carboxamide

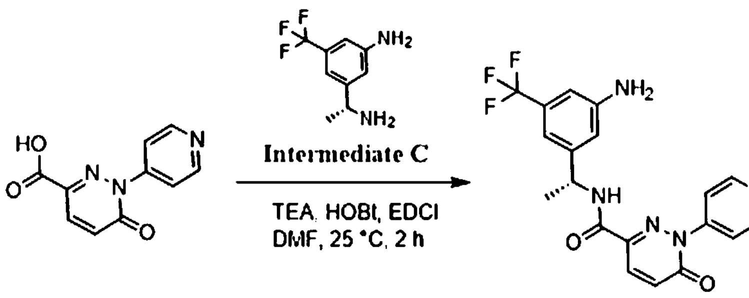


21

To a solution of 6-oxo-1-(4-pyridyl)pyridazine-3-carboxylic acid (40 mg, 184.18 μmol) and Intermediate C (44.32 mg, 184.18 μmol, HCl salt) in DMF (1 mL), TEA (55.91 mg, 552.54 μmol), HOBt (29.86 mg, 221.01 μmol) and EDCI (42.37 mg, 221.01 μmol) were added, followed by stirring at 25 °C for 2 h. The reaction mixture was poured into water (20 mL) and extracted with EtOAc. The combined organic layer was washed with brine, dried over $Na_2SO_4$ and concentrated under reduced pressure to obtain a crude product, which was purified by prep-HPLC (Phenomenex C18 75*30 mm*3 μm, mobile phase: [water($NH_3H_2O$+$NH_4HCO_3$)-ACN];B%: 21%-51%,11 min). ACN was removed under reduced pressure, and the remaining solvent was removed by lyophilization to give the compound of Example 21 (11.6 mg, 14.66% yield) as a white solid. $^1$H NMR (400MHz, DMSO-$d_6$) δ 8.96 (d, *J* = 8.4 Hz, 1H), 8.78-8.73 (m, 2H), 7.92-7.87 (m, 3H), 7.19 (d, *J* = 9.6 Hz, 1H), 6.81 (d, *J* = 14.4 Hz, 2H), 6.71 (s, 1H), 5.55 (s, 2H), 5.06 (quin, *J* = 7.2 Hz, 1H), 1.47 (d, *J*= 7.2 Hz, 3H); LC/MS (ESI) m/z = 404.3 [M+H]+.

**Example 22: N-[(1R)-1-[3-amino-5-(trifluoromethyl)phenyl]ethyl]-1-(5-methyl-2-thienyl)-6-oxo-pyridazine-3-carboxamide**

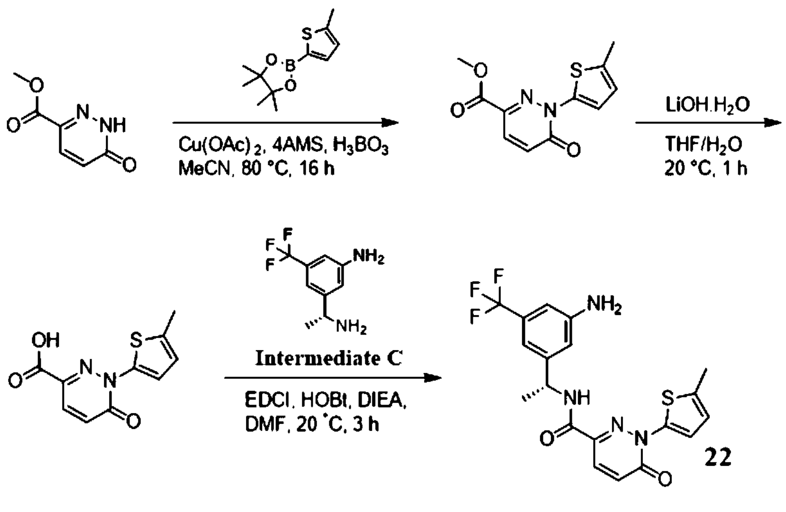


22

The compound of Example 22 was prepared in the same method in Example 21, except that 4,4,5,5-tetramethyl-2-(5-methyl-2-thienyl)-1,3,2-dioxaborolane was used instead of 4-(4,4,5,5-tetramethyl-1,3,2-dioxaborolan-2-yl) pyridine in Step 1. $^1$H NMR (400 MHz, DMSO-$d_6$) δ 8.98 (d, *J* = 8.4 Hz, 1H), 7.89 (d, *J* = *9.6* Hz, 1H), 7.84 (d, *J* = *4.0* Hz, 1H), 7.23 (d, *J* = 9.6 Hz, 1H), 6.88-6.81 (m, 3H), 6.72 (s, 1H), 5.56 (s, 2H), 5.09 (quin, *J* = 7.2 Hz, 1H), 2.46 (s, 3H), 1.51 (d, *J* = 7.2 Hz, 3H); LC/MS (ESI) m/z = 423.3 [M+H]+.

**Example 23: N-[(1R)-1-[3-amino-5-(trifluoromethyl)phenyl]ethyl]-1-(2-methoxyphenyl)-6-oxo-pyridazine-3-carboxamide**

Step 1: Synthesis of methyl-1-(2-methoxyphenyl)-6-oxopyridazine-3-carboxylate

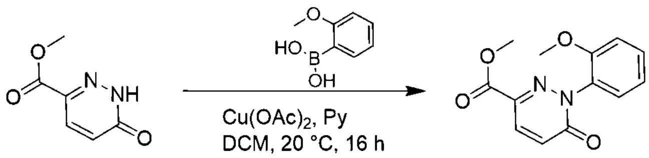


A mixture of methyl-6-oxo-1H-pyridazine-3-carboxylate (200 mg, 1.30 mmol), (2-methoxyphenyl)boronic acid (236.62 mg, 1.56 mmol), $Cu(OAc)_2$ (47.14 mg, 259.53 μmol) and pyridine (667.19 mg, 8.43 mmol) in DCM (3 mL) was stirred at 20 °C for 16 h under air. The reaction mixture was poured into water (10 mL) and extracted with EtOAc. The combined organic layer was washed with brine, dried over $Na_2SO_4$ and concentrated under reduced pressure to obtain a crude product. The product was purified by flash silica gel chromatography (30% EtOAc in petroleum ether) to give methyl-1-(2-methoxyphenyl)-6-oxopyridazine-3-carboxylate (180 mg, 47.16% yield) as a white oil. LC/MS (ESI) m/z = 261.0 $[M+H]^+$.

Step 2: Synthesis of 1-(2-methoxyphenyl)-6-oxo-pyridazine-3-carboxylic acid

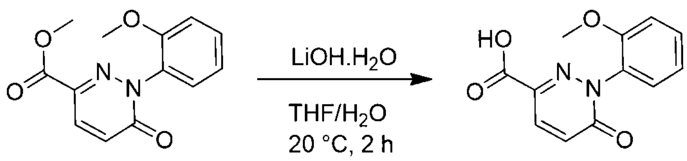


A mixture of methyl-1-(2-methoxyphenyl)-6-oxo-pyridazine-3-carboxylate (180 mg, 691.66 μmol) and $LiOH\cdot H_2O$ (87.07 mg, 2.07 mmol) in THF (3 mL) and $H_2O$ (1.5 mL) was stirred at 20 °C for 2 h under air. The reaction mixture was acidified with aq.1 N HCl (pH = 3-4) and extracted with EtOAc. The organic layer was washed with water and brine, dried over $Na_2SO_4$, filtered and concentrated under reduced pressure to give 1-(2-methoxyphenyl)-6-oxo-pyridazine-3-carboxylic acid (160 mg, 81.91% yield) as a crude product of a yellow solid. LC/MS (ESI) m/z = 247.0 $[M+H]^+$.

Step 3: Synthesis of N-[(1R)-1-[3-amino-5-(trifluoromethyl)phenyl]ethyl]-1-(2-methoxyphenyl)-6-oxo-pyridazine-3-carboxamide

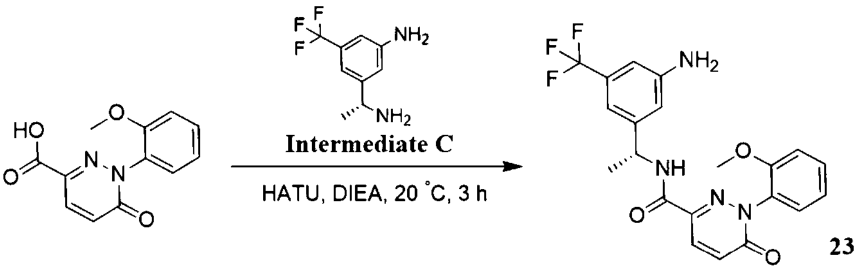


**23**

A mixture of 1-(2-methoxyphenyl)-6-oxo-pyridazine-3-carboxylic acid (50 mg, 203.07 μmol), Intermediate C (45.61 mg, 223.38 μmol), HATU (115.82 mg, 304.61 μmol) and DIPEA (78.74 mg, 609.22μmol) in DMF (2 mL) was degassed and purged with $N_2$ for 3 times, and then stirred at 20 °C for 3 h under $N_2$ atmosphere. The reaction mixture was poured into water (10 mL) and extracted with EtOAc. The combined organic layer was washed with brine, dried over $Na_2SO_4$ and concentrated under reduced pressure to obtain a crude product, which was then purified by prep-HPLC (Phenomenex Luna C18 100*30 mm*3 μm, mobile phase: [water(0.225% FA)-ACN];B%: 38%-68%,7 min). ACN was removed under reduced pressure, and the remaining solvent was removed by lyophilization to give the compound of Example 23 (26.6 mg, 30.28% yield) as a white solid. $^1H$ NMR (400 MHz, DMSO-$d_6$) δ 8.82 (d, $J$ = 8.4 Hz, 1H), 7.90 (d, $J$ = 10.0 Hz, 1H), 7.51-7.47 (m, 1H), 7.43 (dd, $J$ = 1.6, 7.6 Hz, 1H), 7.21 (d, $J$ = 8.0 Hz, 1H), 7.12-7.08 (m, 2H), 6.82 (s, 1H), 6.78

(s, 1H), 6.71 (s, 1H), 6.48-5.10 (m, 2H), 5.02 (t, *J* = 7.6 Hz, 1H), 3.76 (s, 3H), 1.42 (d, *J* = 7.2 Hz, 3H); LC/MS (ESI) m/z = 433.3 $[M+H]^+$.

**Example 24 to Example 45**

The compounds of Examples 24 to 45 were prepared in a similar method to Example 23, using starting materials and intermediates corresponding to the respective structures of the desired compounds. Meanwhile, the coupling reagent used in Step 3 was changed to HOBt and EDCI when the compounds of Examples 40 and 41 were prepared.

[Table 2]

| No. | Structure/Name | Spectral Data |
|---|---|---|
| 24 | 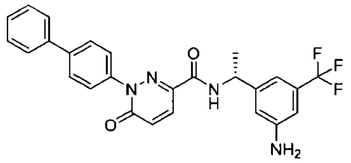 (R)-1-([1,1'-biphenyl]-4-yl)-N-(1-(3-amino-5-(trifluoromethyl)phenyl) ethyl)-6-oxo-1,6-dihydropyridazine-3-carboxamide | $^1$H NMR (400 MHz, DMSO-$d_6$) δ8.91 (d, J = 8.4 Hz, 1H), 7.91 (d, J = 9.6 Hz, 1H), 7.84 - 7.67 (m, 6H), 7.51 (t, J = 7.6 Hz, 2H), 7.46 - 7.38 (m, 1H), 7.17 (d, J = 10.0 Hz, 1H), 6.83 (s, 1H), 6.80 (s, 1H), 6.71 (s, 1H), 5.57 (br s, 2H), 5.05 (quin, J = 7.2 Hz, 1H), 1.46 (d, J = 6.8 Hz, 3H); LC/MS (ESI) m/z = 403.3 |
| 25 | 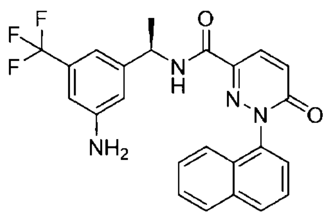 (R)-N-(1-(3-amino-5-(trifluoromethyl)phenyl)ethyl)-1-(naphthalen-1-yl)-6-oxo-1,6-dihydropyridazine-3-carboxamide | $^1$H NMR (400 MHz, DMSO-$d_6$) δ 8.82 (br d, J = 8.4 Hz, 1H), 8.17 - 8.02 (m, 3H), 7.77 - 7.59 (m, 3H), 7.55 (s, 1H), 7.50 7.37 (m, 1H), 7.23 (d, J = 10.0 Hz, 1H), 6.75 (br s, 2H), 6.68 (br s, 1H), 6.33 - 5.13 (m, 2H), 5.02 (quin, J = 7.2 Hz, 1H), 1.37 (br d, J = 6.8 Hz, 3H), 1.05 (br d, J = 5.6 Hz, 1H); LC/MS (ESI) m/z = 453.3 $[M+H]^+$ |
| 26 | 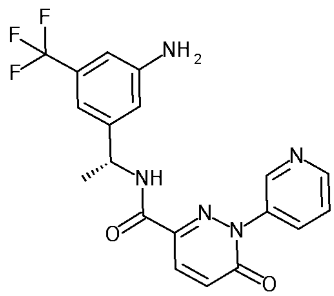 (R)-N-(1-(3-amino-5-(trifluoromethyl)phenyl)ethyl)-6-oxo-1-(pyridin-3-yl)-1,6-dihydropyridazine-3-carboxamide | $^1$H NMR (400 MHz, DMSO-$d_6$) δ 8.98 - 8.93 (m, 2H), 8.65 (dd, J = 1.6, 4.8 Hz, 1H), 8.18 (d, J = 7.6 Hz, 1H), 7.91 (d, J = 9.6 Hz, 1H), 7.60 (dd, J = 4.8, 8.4 Hz, 1H), 7.18 (d, J = 9.6 Hz, 1H), 6.82 (s, 1H), 6.78 (s, 1H), 6.70 (s, 1H), 5.59 - 5.51 (m, 2H), 5.05 (t, J = 7.6 Hz, 1H), 1.45 (d, J = 6.8 Hz, 2H); LC/MS (ESI) m/z = 404.3 $[M+H]^+$ |
| 27 | 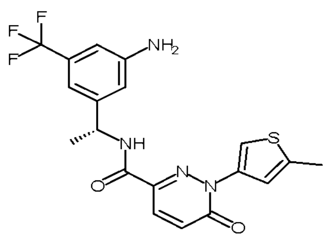 (R)-N-(1-(3-amino-5-(trifluoromethyl)phenyl)ethyl)-1-(5-methylthiophen-3-yl)-6-oxo-1,6-dihydropyridazine-3-carboxamide | $^1$H NMR (400 MHz, CHLOROFORM-d) δ 7.97 (d, J = 9.6 Hz, 1H), 7.73 - 7.66 (m, 1H), 7.14 (s, 1H), 7.09 (d, J = 9.6 Hz, 1H), 6.97 (s, 1H), 6.82 (s, 2H), 5.20 (quin, J = 7.3 Hz, 1H), 4.50 - 3.26 (m, 2H), 2.55 - 2.52 (m, 3H), 1.59 (d, J = 7.0 Hz, 3H); LC/MS (ESI) m/z = 423.3 $[M+H]^+$ |

(continued)

| No. | Structure/Name | Spectral Data |
|---|---|---|
| 28 | 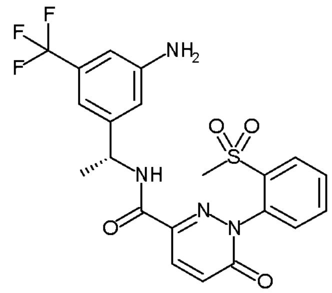 (R)-N-(1-(3-amino-5-(trifluoromethyl)phenyl)ethyl)-1-(2-(methylsulfonyl) phenyl)-6-oxo-1,6-dihydropyridazine-3-carboxamide | $^1$H NMR (400 MHz, DMSO-$d_6$) δ9.40 (d, J = 8.4 Hz, 1H), 8.24 (d, J = 9.2 Hz, 1H), 7.99 (dd, J = 1.4, 8.0 Hz, 1H), 7.85 (t, J = 8.0 Hz, 1H), 7.76 (d, J = 8.8 Hz, 1H), 7.63 - 7.57 (m, 2H), 6.87 (s, 1H), 6.80 (s, 1H), 6.69 (s, 1H), 5.55 (s, 2H), 5.12 - 5.05 (m, 1H), 3.34 - 3.34 (m, 3H), 1.47 (d, J = 6.8 Hz, 3H); LC/MS (ESI) m/z = 481.3 [M+H]$^+$ |
| 29 | 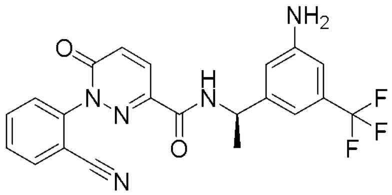 N-[(1R)-1-[3-amino-5-(trifluoromethyl)phenyl]ethyl]-1-(2-cyanophenyl)-6-oxo-pyridazine-3-carboxamide | $^1$H NMR (400 MHz, DMSO-$d_6$) δ8.94 (d, J = 8.4 Hz, 1H), 8.08 (dd, J = 1.2, 7.6 Hz, 1H), 8.00-7.92 (m, 2H), 7.85 (d, J = 7.6 Hz, 1H), 7.73 (t, J = 7.2 Hz, 1H), 7.25 (d, J = 10.0 Hz, 1H), 6.80 (s, 1H), 6.78 (s, 1H), 6.70 (s, 1H), 5.53 (s, 2H),5.03 (quin, J = 7.2 Hz, 1H), 1.42 (d, J = 6.8 Hz, 3H); LC/MS (ESI) m/z = 428.3 [M+H]$^+$ |
| 30 | 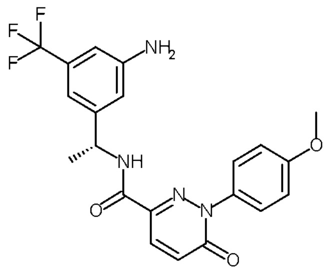 N-[(1R)-1-[3-amino-5-(trifluoromethyl)phenyl]ethyl]-1-(4-methoxyphenyl)-6-oxo-pyridazine-3-carboxamide | $^1$H NMR (400 MHz, DMSO-$d_6$) δ8.82 (d, J = 8.4 Hz, 1H), 7.87 (d, J = 9.6 Hz, 1H), 7.60 - 7.55 (m, 2H), 7.11 (d, J = 9.6 Hz, 1H), 7.08 - 7.04 (m, 2H), 6.82 (s, 1H), 6.78 (s, 1H), 6.70 (s, 1H), 5.55 (s, 2H), 5.06 - 4.99 (m, 1H), 3.82 (s, 3H), 1.44 (d, J = 7.2 Hz, 3H); LC/MS (ESI) m/z = 433.3 [M+H]$^+$ |
| 31 | 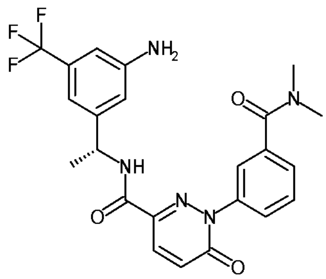 (R)-N-(1-(3-amino-5-(trifluoromethyl)phenyl)ethyl)-1-(3-(dimethylcarbamoyl)phenyl)-6-oxo-1,6-dihydropyridazine-3-carboxamide | $^1$H NMR (400 MHz, DMSO-$d_6$) δ8.89 (d, J = 8.4 Hz, 1H), 7.89 (d, J = 9.6 Hz, 1H), 7.79 - 7.72 (m, 2H), 7.60 (t, J = 7.6 Hz, 1H), 7.50 (d, J = 7.6 Hz, 1H), 7.15 (d, J = 9.6 Hz, 1H), 6.80 (d, J = 12.8 Hz, 2H), 6.70 (s, 1H), 5.54 (s, 2H), 5.04 (quin, J = 7.2 Hz, 1H), 2.98 (d, J = 15.6 Hz, 6H), 1.44 (d, J = 7.2 Hz, 3H); LC/MS (ESI) m/z = 474.4 [M+H]$^+$ |

(continued)

| No. | Structure/Name | Spectral Data |
|---|---|---|
| 32 | 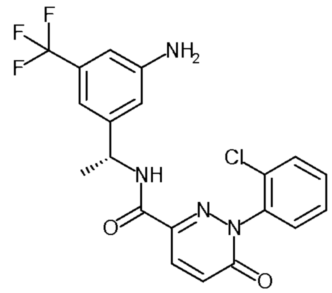 (R)-N-(1-(3-amino-5-(trifluoromethyl)phenyl)ethyl)-1-(2-chlorophenyl)-6-oxo-1,6-dihydropyridazine-3-carboxamide | $^1$H NMR (400 MHz, DMSO-$d_6$) δ8.89 (d, J = 8.4 Hz, 1H), 7.96 (d, J = 9.8 Hz, 1H), 7.72 - 7.63 (m, 2H), 7.58 - 7.53 (m, 2H), 7.19 (d, J = 10.0 Hz, 1H), 6.79 (d, J = 11.6 Hz, 2H), 6.70 (s, 1H), 5.54 (s, 2H), 5.03 (quin, J = 7.2 Hz, 1H), 1.42 (d, J = 7.2 Hz, 3H) ; LC/MS (ESI) m/z = 437.2 $[M+H]^+$ |
| 33 | 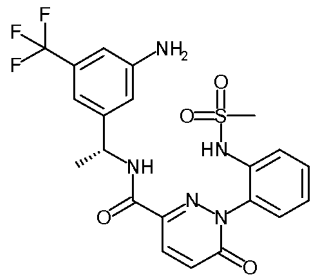 N-[(1R)-1-[3-amino-5-(trifluoromethyl)phenyl]ethyl]-1-[2-(methanesulfonamido)phenyl]-6-oxo-pyridazine-3-carboxamide | $^1$H NMR (400 MHz, DMSO-$d_6$) δ 9.40 (br s, 1H), 8.77 (d, J = 8.4 Hz, 1H), 7.89 (d, J = 10.0 Hz, 1H), 7.65 (dd, J = 1.2, 8.4 Hz, 1H), 7.48 (t, J = 7.6 Hz, 1H), 7.41 (dd, J = 1.6, 8.0 Hz, 1H), 7.29 (t, J = 7.2 Hz, 1H), 7.12 (d, J = 9.6 Hz, 1H), 6.79 (s, 1H), 6.76 (s, 1H), 6.69 (s, 1H), 5.53 (s, 2H), 5.03 (quin, J = 14.4 Hz, 1H), 3.00 - 2.85 (m, 3H), 1.40 (d, J = 6.8 Hz, 3H) ; LC/MS (ESI) m/z = 496.3 $[M+H]^+$ |
| 34 | 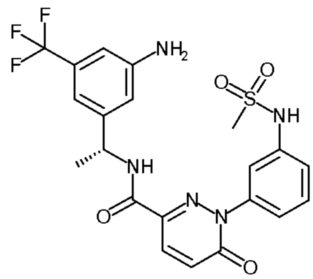 (R)-N-(1-(3-amino-5-(trifluoromethyl)phenyl)ethyl)-1-(3-(methylsulfonamido)phenyl)-6-oxo-1,6-dihydropyridazine-3-carboxamide | $^1$H NMR (400 MHz, DMSO-$d_6$) δ 10.20 - 9.77 (m, 1H), 8.85 (d, J = 8.4Hz, 1H), 7.89 (d, J = 9.6Hz, 1H), 7.52 - 7.46 (m, 2H), 7.42 - 7.37 (m, 1H), 7.30 - 7.26 (m, 1H), 7.14 (d, J = 9.6Hz, 1H), 6.80 (d, J = 14.4Hz, 2H), 6.70 (s, 1H), 5.54 (s, 2H), 5.03 (quin, J = 7.2Hz, 1H), 3.06 (s, 3H), 1.44 (d, J = 7.2Hz, 3H) ; LC/MS (ESI) m/z = 496.3 $[M+H]^+$ |
| 35 | 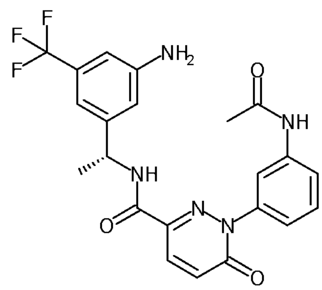 (R)-1-(3-acetamidophenyl)-N-(1-(3-amino-5-(trifluoromethyl)phenyl)ethyl)-6-oxo-1,6-dihydropyridazine-3-carboxamide | $^1$H NMR (400 MHz, DMSO-$d_6$) δ10.18 (s, 1H), 8.84 (d, J = 8.4Hz, 1H), 7.93 - 7.83 (m, 2H), 7.62 (d, J = 8.4 Hz, 1H), 7.45 (t, J = 8.0Hz, 1H), 7.30 (d, J = 8.0Hz, 1H), 7.14 (d, J = 9.6 Hz, 1H), 6.80 (d, J = 14.0 Hz, 2H), 6.70 (s, 1H), 5.56 (s, 2H), 5.03 (quin, J = 7.2Hz, 1H), 2.06 (s, 3H), 1.43 (d, J = 7.2Hz, 3H) ; LC/MS (ESI) m/z = 460.4 $[M+H]^+$ |

(continued)

| No. | Structure/Name | Spectral Data |
|---|---|---|
| 36 | 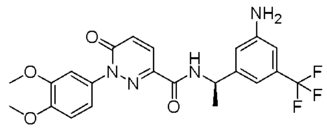 N-[(1R)-1-[3-amino-5-(trifluoromethyl)phenyl]ethyl]-1-(3,4-dimethoxyphenyl)-6-oxo-pyridazine-3-carboxamide | $^1$H NMR (400 MHz, DMSO-$d_6$) δ8.84 (d, J = 8.4 Hz, 1H), 7.88 (d, J = 9.6 Hz, 1H), 7.28-7.14 (m, 2H), 7.11 (d, J = 9.6 Hz, 1H), 7.06 (d, J = 8.4 Hz, 1H), 6.82 (s, 1H), 6.78 (s, 1H), 6.69 (s, 1H), 5.56 (brs, 2H), 5.09-4.96 (m, 1H), 3.82 (s, 3H), 3.77 (s, 3H), 1.44 (d, J = 7.2 Hz, 3H) ; LC/MS (ESI) m/z = 463.3 $[M+H]^+$ |
| 37 | 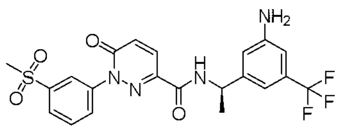 N-[(1R)-1-[3-amino-5-(trifluoromethyl)phenyl]ethyl]-1-(3-methylsulfonylphenyl)-6-oxo-pyridazine-3-carboxamide | $^1$H NMR (400 MHz, DMSO-$d_6$) δ8.96 (d, J = 8.4 Hz, 1H), 8.29 (t, J = 1.6 Hz, 1H), 8.10-8.02 (m, 2H), 7.91 (d, J = 9.6 Hz, 1H), 7.84 (t, J = 7.6 Hz, 1H), 7.19 (d, J = 9.6 Hz, 1H), 6.82 (s, 1H), 6.78 (s, 1H), 6.70 (s, 1H), 5.56 (s, 2H), 5.04 (t, J = 7.6 Hz, 1H), 3.29 (s, 3H), 1.44 (d, J = 7.2 Hz, 3H) ; LC/MS (ESI) m/z = 481.3 $[M+H]^+$ |
| 38 | 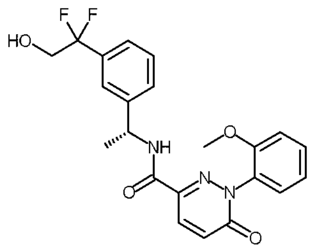 N-[(1R)-1-[3-(1,1-difluoro-2-hydroxyethyl)phenyl]ethyl]-1-(2-methoxyphenyl)-6-oxo-pyridazine-3-carboxamide | $^1$H NMR (400 MHz, DMSO-$d_6$) δ8.89 (d, J = 8.0 Hz, 1H), 7.90 (d, J = 10.0 Hz, 1H), 7.53 - 7.47 (m, 3H), 7.44 - 7.36 (m, 3H), 7.21 (dd, J = 0.8, 8.4 Hz, 1H), 7.13 - 7.08 (m, 2H), 5.60 (br s, 1H), 5.17 (quin, J = 7.2 Hz, 1H), 3.82 (br t, J = 14.4 Hz, 2H), 3.76 (s, 3H), 1.46 (d, J = 7.2 Hz, 3H) ; LC/MS (ESI) m/z = 430.3 $[M+H]^+$ |
| 39 | 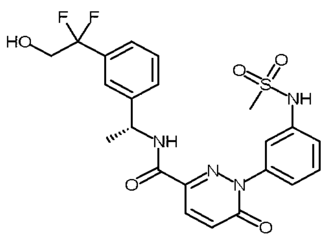 (R)-N-(1-(3-(1,1-difluoro-2-hydroxyethyl)phenyl)ethyl)-1-(3-(methylsulfonamido)phenyl)-6-oxo-1,6-dihydropyridazine-3-carboxamide | $^1$H NMR (400 MHz, DMSO-$d_6$) δ 10.25 - 9.77 (m, 1H), 8.93 (d, J = 8.4 Hz, 1H), 7.88 (d, J = 9.6 Hz, 1H), 7.55 - 7.51 (m, 2H), 7.50 - 7.41 (m, 3H), 7.40 - 7.37 (m, 2H), 7.29 - 7.26 (m, 1H), 7.14 (d, J = 9.6 Hz, 1H), 5.83 - 5.41 (m, 1H), 5.22 - 5.15 (m, 1H), 3.83 (t, J = 14.0 Hz, 2H), 3.06 (s, 3H), 1.48 (d, J = 7.2 Hz, 3H) ; LC/MS (ESI) m/z = 493.3 $[M+H]^+$ |
| 40 | 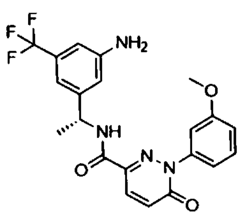 N-[(1R)-1-[3-amino-5-(trifluoromethyl)phenyl]ethyl]-1-(3-methoxyphenyl)-6-oxo-pyridazine-3-carboxamide | $^1$H NMR (400MHz, DMSO-$d_6$) δ 8.86 (d, *J* = 8.4 Hz, 1H), 7.88 (d, J = 9.6 Hz, 1H), 7.44 (t, *J* = 8.0 Hz, 1H), 7.28-7.20 (m, 2H), 7.13 (d, *J* = 9.6 Hz, 1H), 7.08-7.02 (m, 1H), 6.80 (d, *J* = 16.4 Hz, 2H), 6.70 (s, 1H), 5.55 (s, 2H), 5.03 (quin, *J*=7.2 Hz, 1H), 3.80 (s, 3H), 1.44 (d, *J* = 7.2 Hz, 3H) ; LC/MS (ESI) m/z = 433.3 $[M+H]^+$ |

(continued)

| No. | Structure/Name | Spectral Data |
|---|---|---|
| 41 | 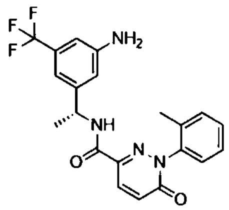 N-[(1R)-1-[3-amino-5-(trifluoromethyl)phenyl]ethyl]-1-(o-tolyl)-6-oxo-pyridazine-3-carboxamide | $^1$H NMR (400 MHz, DMSO-$d_6$) δ 8.85 (d, *J* = 8.8 Hz, 1H), 7.94 (d, *J* = 9.6 Hz, 1H), 7.43-7.34 (m, 4H), 7.16 (d, *J* = 9.6 Hz, 1H), 6.80 (s, 1H), 6.76 (s, 1H), 6.69 (s, 1H), 5.54 (s, 2H), 5.03 (quin, *J* = 7.2 Hz, 1H), 2.09 (s, 3H), 1.42 (d, *J* = 7.2 Hz, 3H) ; LC/MS (ESI) m/z = 417.3 [M+H]$^+$ |
| 42 | 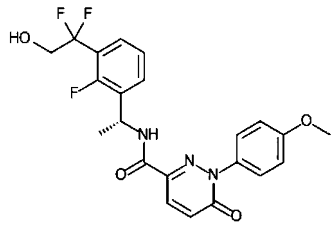 (R)-N-(1-(3-(1,1-difluoro-2-hydroxyethyl)-2-fluorophenyl)ethyl)-1-(4-methoxyphenyl)-6-oxo-1,6-dihydropyridazine-3-carboxamide | $^1$H NMR (400MHz, DMSO-$d_6$) δ 8.94 (d, J=8.0 Hz, 1H), 7.86 (d, J=9.6 Hz, 1H), 7.70 - 7.54 (m, 3H), 7.44 (br t, J=6.8 Hz, 1H), 7.33 - 7.23 (m, 1H), 7.10 (dd, J=9.2, 12.4 Hz, 3H), 5.72 (t, J=6.4 Hz, 1H), 5.47 - 5.35 (m, 1H), 3.92 (dt, J=6.4, 14.4 Hz, 2H), 3.83 (s, 3H), 1.48 (d, J=7.2 Hz, 3H) ; LC/MS (ESI) m/z = 448.3 [M+H]$^+$ |
| 43 | 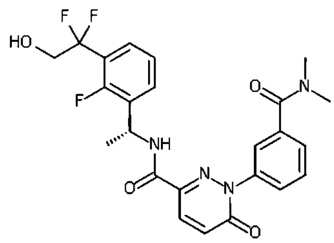 N-[(1R)-1-[3-(1,1-difluoro-2-hydroxy-ethyl)-2-fluorophenyl] ethyl]-1-[3-(dimethylcarbamoyl)phenyl]-6-oxo-pyridazine-3-carboxamide | $^1$H NMR (400 MHz, DMSO-$d_6$) δ 9.00 (d, J = 8.0 Hz, 1H), 7.87 (d, J = 9.6 Hz, 1H), 7.82-7.69 (m, 2H), 7.66-7.59 (m, 2H), 7.52 (d, J = 7.2 Hz, 1H), 7.43 (t, J = 6.8 Hz, 1H), 7.27 (t, J = 7.7 Hz, 1H), 7.15 (d, J = 9.8 Hz, 1H), 5.71 (t, J = 6.4 Hz, 1H), 5.40 (quin, J = 7.2 Hz, 1H), 3.91 (dt, J = 6.4, 14.4 Hz, 2H), 2.99 (br d, J = 13.6 Hz, 6H), 1.48 (d, J = 7. Hz, 3H) ; LC/MS (ESI) m/z = 489.4 [M+H]$^+$ |
| 44 | 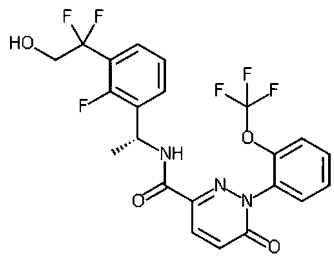 (R)-N-(1-(3-(1,1-difluoro-2-hydroxyethyl)-2-fluorophenyl)ethyl)-6-oxo-1-(2-(trifluoromethoxy)phenyl)-1,6-dihydropyridazine-3-carboxamide | $^1$H NMR (400MHz, DMSO-$d_6$) δ 8.96 (d, J=8.1 Hz, 1H), 7.94 (d, J=9.8 Hz, 1H), 7.83 - 7.75 (m, 1H), 7.71 - 7.65 (m, 1H), 7.64 - 7.57 (m, 3H), 7.42 (t, J = 6.8 Hz, 1H), 7.29 - 7.22 (m, 1H), 7.19 (d, J = 9.6 Hz, 1H), 5.71 (t, J=6.4 Hz, 1H), 5.40 (quin, J=7.2 Hz, 1H), 3.90 (dt, J=6.4, 14.4 Hz, 2H), 1.45 (d, J=7.2 Hz, 3H) ; LC/MS (ESI) m/z = 502.3 [M+H]$^+$ |

(continued)

| No. | Structure/Name | Spectral Data |
|---|---|---|
| 45 | 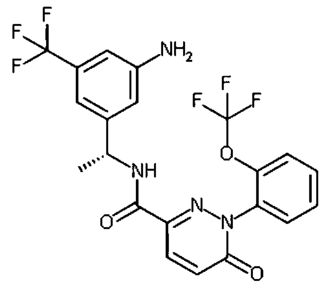 N-[(1R)-1-[3-amino-5-(trifluoromethyl)phenyl]ethyl]-6-oxo-1-[2-(trifluoromethoxy)phenyl]pyridazine-3-carboxamide | $^1$H NMR (400 MHz, DMSO-$d_6$) δ 8.84 (d, J = 8.4 Hz, 1H), 7.95 (d, J = 10.0 Hz, 1H), 7.80-7.72 (m, 1H), 7.70-7.64 (m, 1H), 7.63-7.57 (m, 2H), 7.19 (d, J = 9.6 Hz, 1H), 6.76 (br s, 2H), 6.69 (s, 1H), 5.54 (s, 2H), 5.03 (quin, J = 7.2 Hz, 1H), 1.42 (d, J = 7.2 Hz, 3H) ; LC/MS (ESI) m/z = 487.3 [M+H]$^+$ |

**Example 46: (R)-N-(1-(3-amino-5-(trifluoromethyl)phenyl)ethyl)-1-(2-fluorophenyl)-6-oxo-1,6-dihydropyridazine-3-carboxamide**

Step 1: Synthesis of 1-[3-nitro-5-(trifluoromethyl)phenyl]ethanone

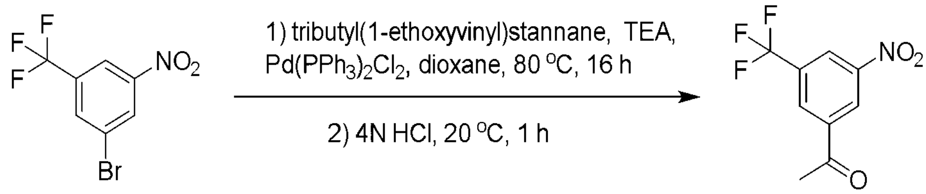


A mixture of 1-bromo-3-nitro-5-(trifluoromethyl)benzene (50 g, 185.18 mmol), tributyl(1-ethoxyvinyl)stannane (70.2 g, 194.38 mmol, 65.61 mL), $Pd(PPh_3)_2Cl_2$ (13.00 g, 18.52 mmol) and TEA (37.48 g, 370.37 mmol, 51.55 mL) in dioxane (500 mL) was degassed and purged with $N_2$ for 3 times, and then the mixture was stirred at 80 °C for 16 h under $N_2$ atmosphere. The mixture was quenched with 6 N HCl (200 mL) and stirred at 20 °C for 1 hour. The reaction mixture was poured into water (200 mL) and extracted with EtOAc (150 mL × 3). The combined organic layer was washed with brine (100 mL × 3), dried over $Na_2SO_4$ and concentrated under reduced pressure to obtain a residue, which was purified by silica gel column chromatography (5% EtOAc in PE) to give 1-[3-nitro-5-(trifluoromethyl)phenyl]ethanone (66 g, 76.43% yield) as yellow oil. $^1$H NMR (400 MHz, DMSO-$d_6$) δ 8.85 (s, 1H), 8.73 (s, 1H), 8.63 (s, 1H), 2.76 (s, 3H).

Step 2: Synthesis of (R)-2-methyl-N-[1-[3-nitro-5-(trifluoromethyl)phenyl]ethylidene]propane-2-sulfinamide

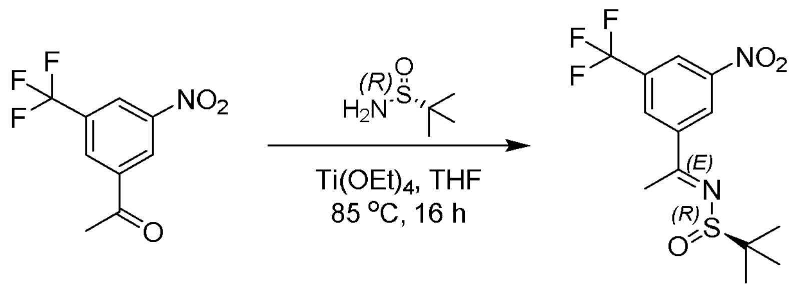


To a solution of 1-[3-nitro-5-(trifluoromethyl)phenyl]ethanone (66 g, 283.09 mmol) in THF (650 mL), $Ti(OEt)_4$ (161.44 g, 707.72 mmol, 146.76 mL) and (R)-2-methylpropane-2-sulfinamide (44.60 g, 368.01 mmol) were added, followed by stirring at 85 °C for 16 h under $N_2$ atmosphere. The reaction mixture was poured into water (200 mL) and EtOAc (200 mL) and filtrated, and the filtrate was extracted with EtOAc (200 mL × 3). The combined organic layer was washed with brine (200 mL × 2), dried over $Na_2SO_4$ and concentrated under reduced pressure to obtain a residue, which was

purified by silica gel column chromatography (12% EtOAc in PE) to give (R,E)-2-methyl-N-[1-[3-nitro-5-(trifluoromethyl)phenyl]ethylidene]propane-2-sulfinamide (77 g, 71.07% yield) as yellow oil. $^1$H NMR (400 MHz, DMSO-$d_6$) δ 8.85 (s, 1H), 8.73 (s, 1H), 8.63 (s, 1H), 2.76 (s, 3H); LC/MS (ESI) m/z = 336.9 [M+H]$^+$.

Step 3: Synthesis of (R)-2-methyl-N-[(1R)-1-[3-nitro-5-(trifluoromethyl)phenyl]ethyl]propane-2-sulfinamide

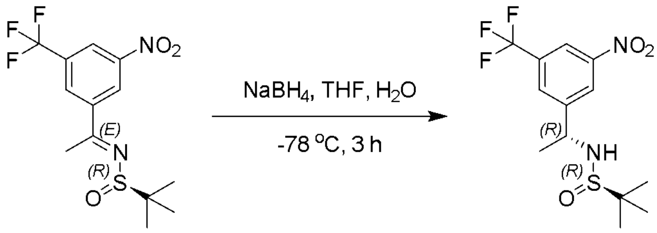


To a solution of (R)-2-methyl-N-[1-[3-nitro-5-(trifluoromethyl)phenyl]ethylidene]propane-2-sulfinamide (38.5 g, 114.47 mmol) in THF (350 mL) and $H_2O$ (7 mL) was added $NaBH_4$ (3.27 g, 86.43 mmol) for 3 batches, and the mixture was stirred at -78 °C for 3 h under $N_2$. The reaction mixture was quenched with sat. aq. $NH_4Cl$ (150 mL) at 20°C, poured into water (200 mL) and extracted with EtOAc (200 mL × 3). The combined organic layer was washed with brine (100 mL), dried over $Na_2SO_4$, filtered and concentrated under reduced pressure to obtain a residue. The residue was purified by silica gel column chromatography (15% EtOAc in PE) to give a main product, (R)-2-methyl-N-[(1R)-1-[3-nitro-5-(trifluoromethyl)phenyl]ethyl]propane-2-sulfinamide (30 g, 32.01% yield) as a green solid. $^1$H NMR (400 MHz, DMSO-$d_6$) δ 8.63 (s, 1H), 8.37 (s, 1H), 8.29 (s, 1H), 6.07 (d, *J* = 8.8 Hz, 1H), 4.73-4.63 (m, 1H), 1.45 (d, *J* = 7.2 Hz, 3H), 1.13 (s, 9H); LC/MS (ESI) m/z = 338.9 [M+H]$^+$.

Step 4: Synthesis of (1R)-1-[3-nitro-5-(trifluoromethyl)phenyl]ethanamine

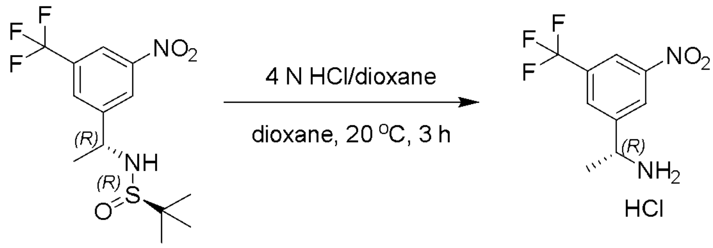


To a solution of (R)-2-methyl-N-[(1R)-1-[3-nitro-5-(trifluoromethyl)phenyl]ethyl]propane-2-sulfinamide (30 g, 88.67 mmol) in dioxane (30 mL) was added 4N HCl/dioxane (60 mL) at 0 °C, followed by stirring at 20 °C for 3 h. The mixture was concentrated under reduced pressure to obtain a residue, which was triturated with MTBE (100 mL) for 12 h at 20 °C and then filtered to give the HCl salt of (1R)-1-[3-nitro-5-(trifluoromethyl)phenyl]ethanamine (23 g, 91.02% yield) as a white solid. $^1$H NMR (400 MHz, DMSO-$d_6$) δ 8.93 - 8.61 (m, 4H), 8.51 (s, 1H), 8.45 (s, 1H), 4.74 (q, *J* = 6.8 Hz, 1H), 1.58 (d, *J* = 6.8 Hz, 3H); LC/MS (ESI) m/z = 234.9 [M+H]$^+$.

Step 5: Synthesis of 3-[(1R)-1-aminoethyl]-5-(trifluoromethyl)aniline

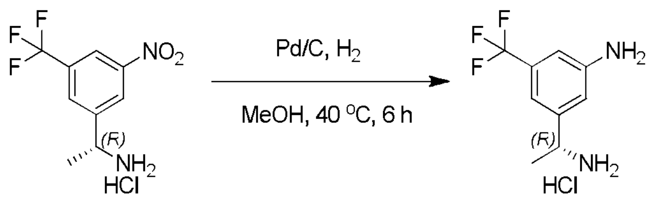


**Intermediate C**

To a solution of (1R)-1-[3-nitro-5-(trifluoromethyl)phenyl]ethanamine (10 g, 42.70 mmol, HCl salt) in MeOH (100 mL) was added Pd/C (2 g, 10% purity), followed by stirring at 40 °C for 6 h under $H_2$ atmosphere. The reaction mixture was filtered, and the filtrate was concentrated under reduced pressure to give Intermediate C (8.5 g, 93.87% yield) as a yellow solid, which was used in the next step without further purification. $^1$H NMR (400 MHz, DMSO-$d_6$) δ 8.44 (br s, 3H), 6.97 (s, 1H), 6.84 (s, 2H), 5.75 (s, 2H), 4.30 (q, *J* = 6.8 Hz, 1H), 1.47 (d, *J* = 6.8 Hz, 3H); LC/MS (ESI) m/z = 204.9 [M+H]$^+$.

Step 6: Synthesis of 1-(2-fluorophenyl)-6-oxo-pyridazine-3-carboxylate

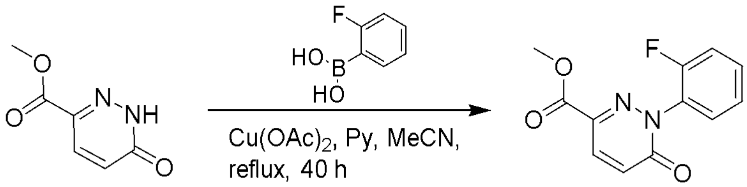


A mixture of (2-fluorophenyl)boronic acid (17.70 g, 126.52 mmol), methyl-6-oxo-1H-pyridazine-3-carboxylate (15 g, 97.32 mmol), $Cu(OAc)_2$ (5.30 g, 29.20 mmol) and pyridine (50.04 g, 632.61 mmol, 51.06 mL) in MeCN (500 mL) was degassed and purged with $O_2$ three times, and stirred at 110 °C for 40 h under $O_2$ atmosphere. The reaction mixture was concentrated under reduced pressure, poured into distilled water (500 mL) and extracted with EtOAc. The combined organic layer was washed with brine (500 mL), dried over $Na_2SO_4$ and concentrated under reduced pressure to obtain a crude product, which was purified by silica gel column chromatography (30% EtOAc in petroleum ether) to give 1-(2-fluorophenyl)-6-oxo-pyridazine-3-carboxylate (20 g, 20.35% yield) as a white solid. $^1$H NMR (400 MHz, DMSO-$d_6$) δ 7.97 (d, *J* = 10.0 Hz, 1H), 7.60 (ddquin, 2H), 7.46 (t, *J* = 9.6 Hz, 1H), 7.40 (dt, *J* = 1.2, 7.6 Hz, 1H), 7.21 (d, *J* = 9.6 Hz, 1H), 3.85 (s, 3H); LC/MS (ESI) m/z = 249.1 [M+H]$^+$.

Step 7: Synthesis of 1-(2-fluorophenyl)-6-oxo-pyridazine-3-carboxylic acid

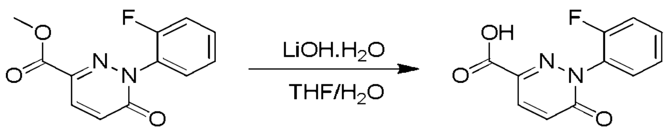


**Intermediate DA**

To a solution of 1-(2-fluorophenyl)-6-oxo-pyridazine-3-carboxylate (20 g, 80.58 mmol) in THF (180 mL), LiOH·$H_2O$ (10.14 g, 241.73 mmol) and $H_2O$ (20 mL) were added. The mixture was stirred at 20 °C for 1 hour. The mixture was poured into water (200 mL) and extracted with EtOAc (100 mL × 3). After the organic layer was removed, the mixture was adjusted to pH 3-4 with aq. 1N HCl and then extracted with EtOAc (200 mL × 3). The combined organic layer was washed with brine (30 mL × 2), dried over $Na_2SO_4$ and concentrated under reduced pressure to give Intermediate DA (18 g, 92.36% yield) as a white solid. $^1$H NMR (400 MHz, DMSO-$d_6$) δ 13.79 (br s, 1H), 7.95 (d, *J* = 9.6 Hz, 1H), 7.65 - 7.53 (m, 2H), 7.46 (t, *J* = 9.2 Hz, 1H), 7.42 - 7.36 (m, 1H), 7.18 (d, *J* = 10.0 Hz, 1H); LC/MS (ESI) m/z = 235.0 [M+H]$^+$.

Step 8: Synthesis of (R)-N-(1-(3-amino-5-(trifluoromethyl)phenyl)ethyl)-1-(2-fluorophenyl)-6-oxo-1,6-dihydropyridazine-3-carboxamide

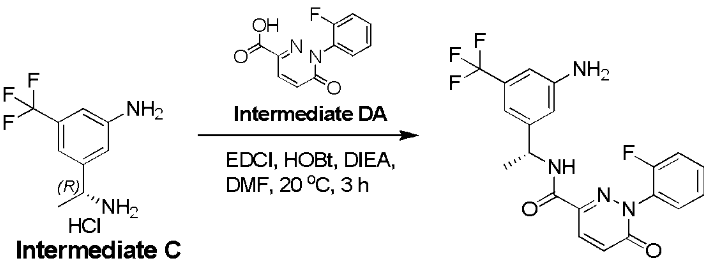


Intermediate C

46

To a solution of Intermediate DA (6.45 g, 27.54 mmol) and Intermediate C (7.29 g, 30.30 mmol, HCl salt) in DMF (65 mL), DIEA (10.68 g, 82.63 mmol, 14.39 mL), HOBt (7.44 g, 55.09 mmol) and EDCI (10.56 g, 55.09 mmol) were added, and the mixture was degassed and purged with $N_2$ for 3 times, and then stirred at 20 °C for 2 h under $N_2$ atmosphere. The reaction mixture was poured into water (100 mL) and extracted with EtOAc (100 mL × 3). The combined organic layer was washed with brine (100 mL), dried over $Na_2SO_4$ and concentrated under reduced pressure to obtain a crude product, which was purified by silica gel column chromatography (40% EtOAc in PE) to give a main product, the compound of Example 46 (7.1 g, 60.75% yield) as a white solid. $^1H$ NMR (400MHz, DMSO-$d_6$) δ 8.91 (d, *J* = 8.4 Hz, 1H), 7.94 (d, *J* = 9.6 Hz, 1H), 7.67 (dt, *J* = 1.6, 7.6 Hz, 1H), 7.61 - 7.54 (m, 1H), 7.47 - 7.37 (m, 2H), 7.18 (d, *J* = 9.6 Hz, 1H), 6.80 (s, 1H), 6.77 (s, 1H), 6.70 (s, 1H), 5.54 (s, 2H), 5.02 (quin, *J* = 7.2 Hz, 1H), 1.42 (d, *J* = 7.2 Hz, 3H); LC/MS (ESI) m/z = 421.3 $[M+H]^+$.

Example 47: N-[(1R)-1-[3-amino-5-(trifluoromethyl)phenyl]ethyl]-1-(2-hydroxyphenyl)-6-oxo-pyridazine-3-carboxamide

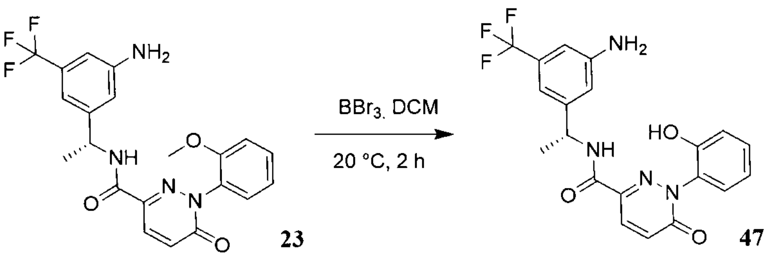


23

47

A mixture of the compound of Example 23 (30 mg, 69.38 μmol) and $BBr_3$ (1 M, 346.91 μL) was stirred at 20 °C for 2 hous under air, poured onto ice-water (5 mL) and extracted with DCM. The combined organic layer was washed with brine, dried over $Na_2SO_4$ and concentrated under reduced pressure to obtain a crude product. The product was purified by prep-HPLC (Phenomenex Gemini-NX C18 75*30 mm*3 μm, mobile phase:[water(0.05% $NH_3H_2O$+10 mM $NH_4HCO_3$)-ACN]; B%: 18%-38%, 10min). ACN was removed under reduced pressure, and the remaining solvent was removed by lyophilization to give the compound of Example 47 (12.9 mg, 43.87% yield). $^1H$ NMR (400 MHz, DMSO-$d_6$) δ 9.79 (br s, 1H), 8.78 (d, *J* = 8.4 Hz, 1H), 7.89 (d, *J* = 9.6 Hz, 1H), 7.34-7.27 (m, 2H), 7.09 (d, *J* = 9.6 Hz, 1H), 6.98 (d, *J* = 7.6 Hz, 1H), 6.93 (t, *J* = 7.6 Hz, 1H), 6.81 (s, 1H), 6.77 (s, 1H), 6.69 (s, 1H), 5.54 (s, 2H), 5.06-4.98 (m, 1H), 1.42 (d, *J* = 6.8 Hz, 3H); LC/MS (ESI) m/z = 419.3 $[M+H]^+$.

**Example 48: (R)-N-(1-(3-amino-5-(trifluoromethyl)phenyl)ethyl)-5-hydroxy-6-oxo-1-phenyl-1,6-dihydropyridazine-3-carboxamide**

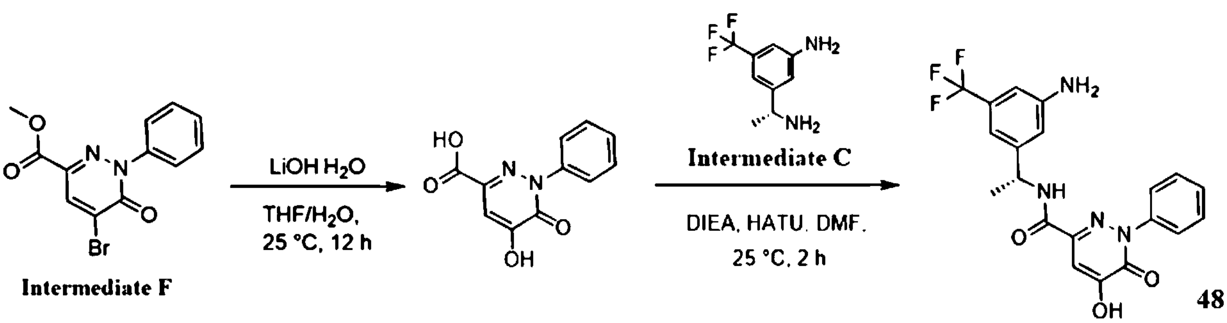


Intermediate F

48

The compound of Example 48 was obtained by reacting Intermediate F in the same method as in Steps 2 and 3 of Example 23. $^1$H NMR (400 MHz, DMSO-$d_6$) δ 8.60 (brs, 1H), 7.55-7.49 (m, 2H), 7.49-7.38 (m, 3H), 7.18 (brs, 1H), 7.01 (brs, 1H), 6.83 (s, 1H), 6.79 (s, 1H), 6.69 (s, 1H), 5.56 (br s, 2H), 5.04 (quin, J = 7.2 Hz, 1H), 1.43 (d, J = 7.2 Hz, 3H); LC/MS (ESI) m/z = 419.3 $[M+H]^+$.

**Example 49: N-[(1R)-1-[3-amino-5-(trifluoromethyl)phenyl]ethyl]-4-methyl-6-oxo-1-phenyl-pyridazine-3-carboxamide**

Step 1: Synthesis of methyl-4-methyl-6-oxo-1-phenyl-pyridazine-3-carboxylate

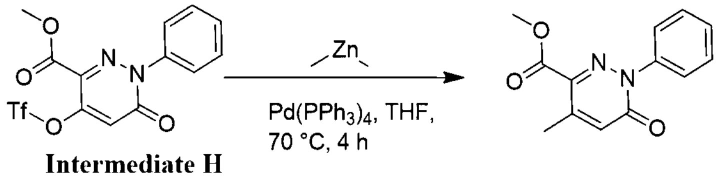


A mixture of Intermediate H (80 mg, 211.48 μmol), dimethylzinc (2 M, 52.87 μL) and $Pd(PPh_3)_4$ (48.88 mg, 42.30 μmol) in THF (2 mL) was degassed and purged with $N_2$ for 3 times, and then stirred at 70 °C for 4 h under $N_2$ atmosphere. The reaction mixture was poured into distilled water (10 mL) and extracted with EtOAc, and the combined organic layer was washed with brine, dried over $Na_2SO_4$ and concentrated under reduced pressure to obtain a crude product. The product was purified by flash silica gel chromatography (20% EtOAc in petroleum ether) to give methyl-4-methyl-6-oxo-1-phenyl-pyridazine-3-carboxylate (60 mg, 79.08% yield) as a white solid. LC/MS (ESI) m/z = 245.0 $[M+H]^+$.

Steps 2 and 3: Synthesis of N-[(1R)-1-[3-amino-5-(trifluoromethyl)phenyl]ethyl]-4-methyl-6-oxo-1-phenyl-pyridazine-3-carboxamide

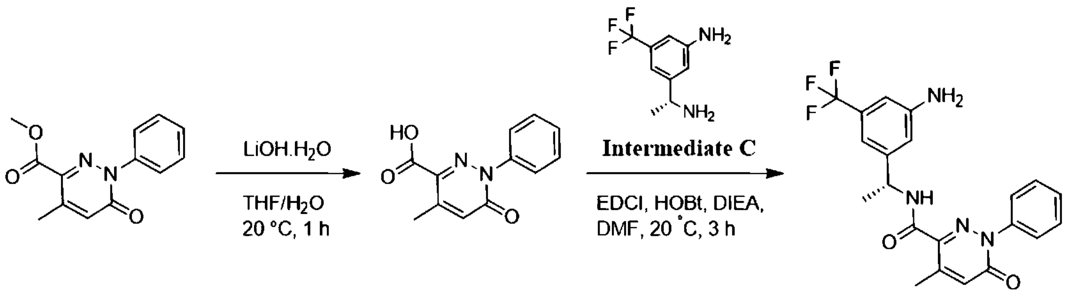


49

The compound of Example 49 was obtained by reacting methyl-4-methyl-6-oxo-1-phenyl-pyridazine-3-carboxylate in the same method as in Steps 2 and 3 of Example 23, except that the coupling reagent used in Step 3 of Example 23 was changed to HOBt and EDCI. $^1$H NMR (400 MHz, DMSO-$d_6$) δ 9.01 (br d, J = 8.0 Hz, 1H), 7.64 (br d, J = 7.6 Hz, 2H), 7.51 (br t, J = 7.6 Hz, 2H), 7.47-7.41 (m, 1H), 6.98 (s, 1H), 6.80 (s, 1H), 6.77 (br s, 1H), 6.71 (br s, 1H), 5.58 (br s, 2H), 4.98 (br t, J = 7.2 Hz, 1H), 2.25 (s, 3H), 1.40 (br d, J = 6.8 Hz, 3H); LC/MS (ESI) m/z = 417.3 $[M+H]^+$.

**Example 50: N-[(1R)-1-[3-amino-5-(trifluoromethyl)phenyl]ethyl]-4-ethynyl-6-oxo-1-phenyl-pyridazine-3-carboxamide**

Step 1: Synthesis of methyl-6-oxo-1-phenyl-4-(2-trimethylsilylethynyl)pyridazine-3-carboxylate

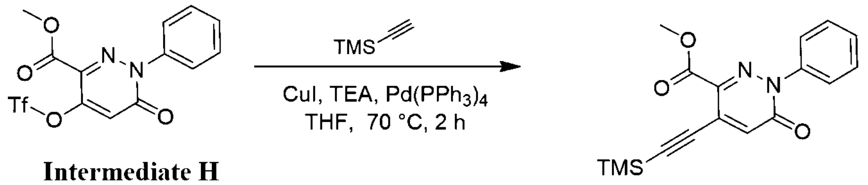


**Intermediate H**

A mixture of Intermediate H (200 mg, 528.71 μmol), ethynyl(trimethyl)silane (129.82 mg, 1.32 mmol, 183.11 μL), CuI (10.07 mg, 52.87 μmol), TEA (160.50 mg, 1.59 mmol, 220.77 μL) and $Pd(PPh_3)_4$ (61.10 mg, 52.87 μmol) in THF (2 mL) was degassed and purged with $N_2$ for 3 times, and then stirred at 70 °C for 2 h under $N_2$ atmosphere. The reaction mixture was poured into water (20 mL) and extracted with EtOAc, and the combined organic layer was washed with brine, dried over $Na_2SO_4$ and concentrated under reduced pressure to obtain a crude product. The product was purified by flash silica gel chromatography (10% EtOAc in petroleum ether) to give methyl-6-oxo-1-phenyl-4-(2-trimethylsilylethynyl)pyridazine-3-carboxylate (50 mg, 17.85% yield) as a white solid. LC/MS (ESI) m/z = 327.0 $[M+H]^+$.

Steps 2 and 3: Synthesis of N-[(1R)-1-[3-amino-5-(trifluoromethyl)phenyl]ethyl]-4-ethynyl-6-oxo-1-phenyl-pyridazine-3-carboxamide

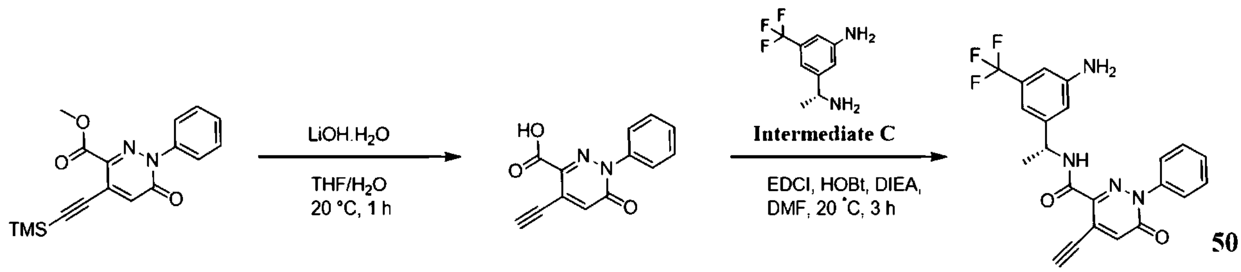


**50**

The compound of Example 50 was obtained by reacting methyl-6-oxo-1-phenyl-4-(2-trimethylsilylethynyl) pyridazine-3-carboxylate in the same method as in Steps 2 and 3 of Example 23, except that the coupling reagent used in Step 3 of Example 23 was changed to HOBt and EDCI. $^1H$ NMR (400 MHz, DMSO-$d_6$) δ 9.05 (d, J = 8.0 Hz, 1H), 7.63 (d, J = 7.6 Hz, 2H), 7.54-7.49 (m, 2H), 7.48-7.43 (m, 1H), 7.34 (s, 1H), 6.80 (s, 1H), 6.76 (s, 1H), 6.71 (s, 1H), 5.56 (s, 2H), 5.01-4.94 (m, 1H), 4.86 (s, 1H), 1.40 (d, J = 7.2 Hz, 3H); LC/MS (ESI) (m/z) = 427.3 $[M+H]^+$.

**Example 51: (R)-N-(1-(3-amino-5-(trifluoromethyl)phenyl)ethyl)-1-(2-aminophenyl)-6-oxo-1,6-dihydropyridazine-3-carboxamide**

Steps 1 and 2: Synthesis of 1-(2-nitrophenyl)-N-[(1R)-1-[3-nitro-5-(trifluoromethyl)phenyl]ethyl]-6-oxo-pyridazine-3-carboxamide

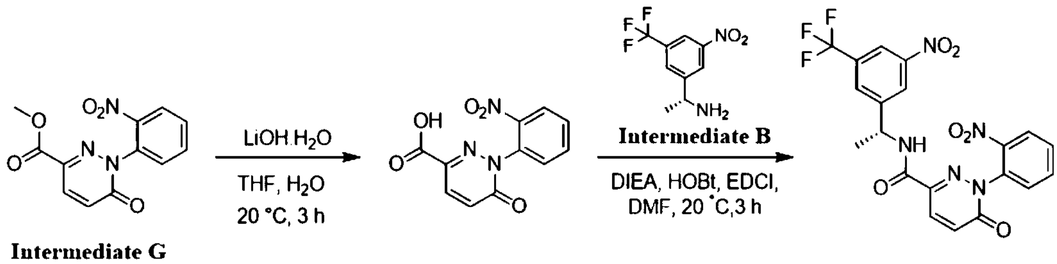


**Intermediate G**

1-(2-nitrophenyl)-N-[(1R)-1-[3-nitro-5-(trifluoromethyl)phenyl]ethyl]-6-oxo-pyridazine-3-carboxamide was obtained as a crude product of a yellow solid by reacting Intermediate G in the same method as in Steps 2 and 3 of Example 23, except that, in Step 3, Intermediate C was replaced with Intermediate B, and the coupling reagent was changed to HOBt and EDCI. LC/MS (ESI) m/z = 278.1 $[M+H]^+$.

Step 3: Synthesis of (R)-N-(1-(3-amino-5-(trifluoromethyl)phenyl)ethyl)-1-(2-aminophenyl)-6-oxo-1,6-dihydropyridazine-3-carboxamide

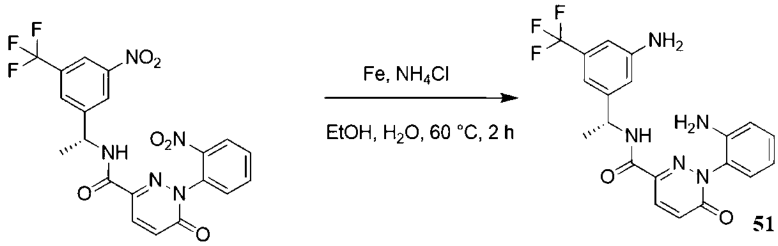


51

A mixture of 1-(2-nitrophenyl)-N-[(1R)-1-[3-nitro-5-(trifluoromethyl)phenyl]ethyl]-6-oxo-pyridazine-3-carboxamide (100 mg, 209.49 μmol) and Fe (117.00 mg, 2.09 mmol) in sat. aq $NH_4Cl$ (1 mL) and EtOH (3 mL) was stirred at 60 °C for 2 h under air. The reaction mixture was poured into distilled water (10 mL) and extracted with EtOAc. The combined organic layer was washed with brine, dried over $Na_2SO_4$ and concentrated under reduced pressure to obtain a crude product, which was purified by prep-HPLC (Phenomenex C18 75*30 mm*3 μm, mobile phase: [water($NH_3H_2O$+$NH_4HCO_3$)-ACN]; B%: 23%-63%, 15 min). ACN was removed under reduced pressure, and the remaining solvent was removed by lyophilization to give the compound of Example 51 (49 mg, 55.84% yield) as a yellow solid. $^1$H NMR (400 MHz, DMSO-$d_6$) δ 8.72 (d, *J* = 8.4 Hz, 1H), 7.87 (d, *J* = 9.6 Hz, 1H), 7.12 (br d, *J* = 1.6 Hz, 1H), 7.11-7.08 (m, 1H), 7.08-7.06 (m, 1H), 6.83-6.77 (m, 3H), 6.69 (s, 1H), 6.62 (t, *J* = 7.2 Hz, 1H), 5.53 (s, 2H), 5.12 (s, 2H), 5.05-4.99 (m, 1H), 1.43 (d, *J* = 7.2 Hz, 3H). LC/MS (ESI) m/z = 418.4 $[M+H]^+$.

**Example 52: (R)-1-(2-acetamidophenyl)-N-(1-(3-amino-5-(trifluoromethyl)phenyl)ethyl)-6-oxo-1,6-dihydropyridazine-3-carboxamide**

Step 1: Synthesis of methyl-1-(2-aminophenyl)-6-oxo-pyridazine-3-carboxylate

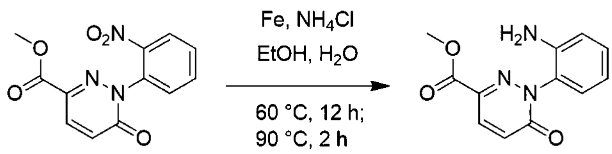


Intermediate G

To a solution of Intermediate G (400 mg, 1.45 mmol) in MeOH (6 mL) and sat. aq. $NH_4Cl$ (2 mL) was added Fe (405.83 mg, 7.27 mmol), followed by stirring at 60 °C for 12 h. The mixture was concentrated under reduced pressure, and then EtOH (6 mL) was added thereto. The resulting mixture was stirred at 90 °C for 2 h. The mixture was filtrated, and the filtrate was concentrated under reduced pressure, added with distilled water (10 mL) and extracted with EtOAc. The combined organic layer was washed with brine, dried over anhydrous $Na_2SO_4$ and concentrated under reduced pressure to give methyl-1-(2-aminophenyl)-6-oxo-pyridazine-3-carboxylate (180 mg, 21.21% yield) as yellow oil. LC/MS (ESI) m/z = 246.0 $[M+H]^+$.

Step 2: Synthesis of methyl-1-(2-acetamidophenyl)-6-oxopyridazine-3-carboxylate

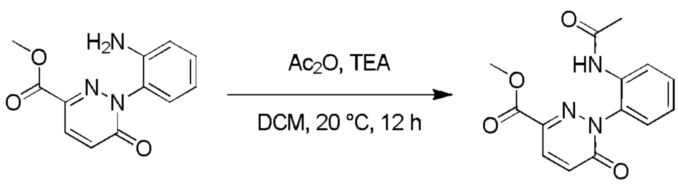

To a solution of methyl-1-(2-aminophenyl)-6-oxo-pyridazine-3-carboxylate (180 mg, 733.99 μmol) in DCM (3 mL), TEA (222.82 mg, 2.20 mmol) and $Ac_2O$ (112.40 mg, 1.10 mmol) were added, followed by stirring at 20 °C for 12 h. The reaction mixture was poured into distilled water (20 mL) and extracted with EtOAc. The combined organic layer was washed with brine, dried over $Na_2SO_4$ and concentrated under reduced pressure to obtain a crude product. The product was purified by silica gel column chromatography (50% EtOAc in petroleum ether) to give methyl-1-(2-acetamidophenyl)-6-oxopyridazine-3-carboxylate (180 mg, 32.44% yield) as a white solid. LC/MS (ESI) m/z = 288.0 $[M+H]^+$.

Steps 3 and 4: Synthesis of (R)-1-(2-acetamidophenyl)-N-(1-(3-amino-5-(trifluoromethyl)phenyl)ethyl)-6-oxo-1,6-dihydropyridazine-3-carboxamide

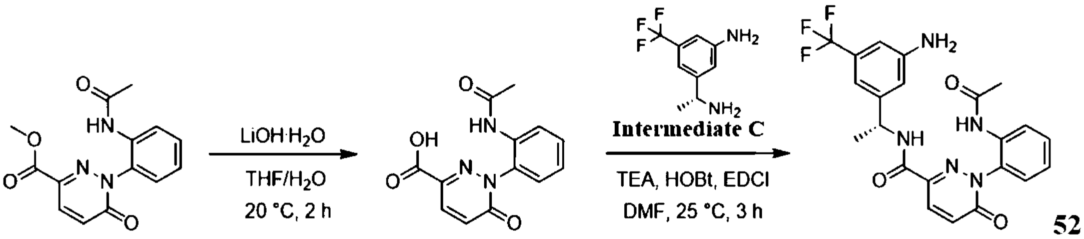

52

The compound of Example 52 was obtained by reacting methyl-1-(2-acetamidophenyl)-6-oxopyridazine-3-carboxylate in the same method as in Steps 2 and 3 of Example 23, except that the coupling reagent used in Step 3 of Example 23 was changed to HOBt and EDCI. $^1H$ NMR (400MHz, DMSO-$d_6$) δ 9.29 (s, 1H), 8.71 (d, *J* = 8.4 Hz, 1H), 7.97-7.84 (m, 2H), 7.50-7.40 (m, 2H), 7.32-7.21 (m, 1H), 7.12 (d, *J* = 10.0 Hz, 1H), 6.79 (d, *J* = 16.4 Hz, 2H), 6.69 (s, 1H), 5.55 (s, 2H), 5.04 (quin, *J* = 7.2 Hz, 1H), 1.91 (s, 3H), 1.42 (d, *J* = 7.2 Hz, 3H); LC/MS (ESI) m/z = 460.3 $[M+H]^+$.

**Example 53: 1-(3-acetamidophenyl)-N-[(1R)-1-[3-(1,1-difluoro-2-hydroxyethyl)phenyl] ethyl]-6-oxo-pyridazine-3-carboxamide**

Step 1: Synthesis of methyl-1-(3-acetamidophenyl)-6-oxo-pyridazine-3-carboxylate

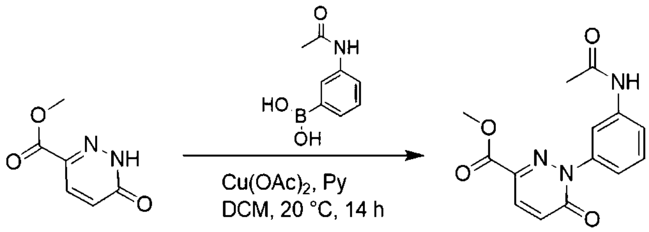


A mixture of methyl-6-oxo-1H-pyridazine-3-carboxylate (500 mg, 3.24 mmol), (3-acetamidophenyl) boronic acid (754.83 mg, 4.22 mmol), $Cu(OAc)_2$ (294.62 mg, 1.62 mmol) and pyridine (1.67 g, 21.09 mmol, 1.70 mL) in DCM (5 mL) was stirred at 20 °C for 14 h under air atmosphere. The reaction mixture was poured into water (20 mL) and extracted with EtOAc, and the combined organic layer was washed with brine, dried over $Na_2SO_4$ and concentrated under reduced pressure to obtain a crude product. The product was purified by silica gel column chromatography (65% EtOAc in petroleum ether) to give methyl-1-(3-acetamidophenyl)-6-oxo-pyridazine-3-carboxylate (400 mg, 35.62% yield) as a light-yellow solid. LC/MS (ESI) m/z = 288.0 $[M+H]^+$.

Steps 2 and 3: Synthesis of 1-(3-acetamidophenyl)-N-[(1R)-1-[3-(1,1-difluoro-2-hydroxy-ethyl)phenyl]ethyl]-6-oxo-pyridazine-3-carboxamide

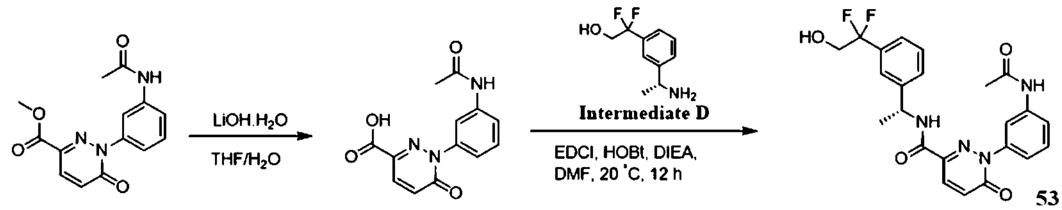

53

The compound of Example 53 was obtained by reacting methyl-1-(3-acetamidophenyl)-6-oxo-pyridazine-3-carboxylate in the same method as in Steps 2 and 3 of Example 23, except that, in Step 3, Intermediate C was replaced with Intermediate D, and the coupling reagent was changed to HOBt and EDCI. $^1$H NMR (400 MHz, DMSO-$d_6$) $\delta$ 10.18 (s, 1H), 8.91 (d, *J* = 8.4 Hz, 1H), 7.88 (d, *J* = 9.6 Hz, 1H), 7.85 (s, 1H), 7.63 (d, *J* = 8.4 Hz, 1H), 7.54-7.48 (m, 2H), 7.41 (s, 2H), 7.39-7.36 (m, 1H), 7.29 (br d, *J* = 7.6 Hz, 1H), 7.13 (d, *J* = 9.6 Hz, 1H), 5.63 (br s, 1H), 5.18 (quin, *J* = 7.2 Hz, 1H), 3.82 (br t, *J* = 14.4 Hz, 2H), 2.06 (s, 3H), 1.49-1.44 (m, 3H); LC/MS (ESI) m/z = 457.4 [M+H]$^+$.

**Example 54: (R)-N-(1-(3-amino-5-(trifluoromethyl)phenyl)ethyl)-5-cyclopropyl-6-oxo-1-phenyl-1,6-dihydropyridazine-3-carboxamide**

Step 1: Synthesis of 5-cyclopropyl-6-oxo-1-phenyl-pyridazine-3-carboxylic acid

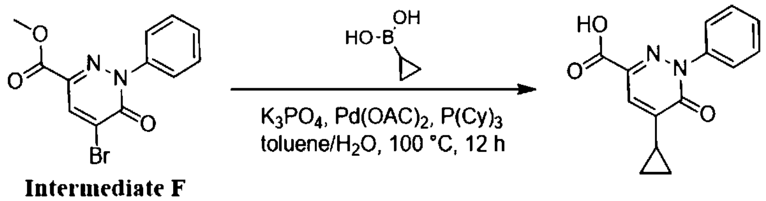

Intermediate F

A mixture of Intermediate F (50 mg, 161.75 μmol), cyclopropylboronic acid (18.06 mg, 210.28 μmol), $K_3PO_4$ (120.17 mg, 566.13 μmol), $Pd(OAc)_2$ (3.63 mg, 16.18 μmol) and $P(Cy)_3$ (9.07 mg, 32.35 μmol) in toluene (1 mL) and $H_2O$ (0.1 mL) was degassed and purged with $N_2$ for 3 times, and then stirred at 100 °C for 12 h under $N_2$ atmosphere. The reaction mixture was poured into water (20 mL) and extracted with EtOAc, and the combined organic layer was discarded. The aqueous layer was acidified with 1 N aq. HCl (pH = 3-4) and extracted with EtOAc. The combined organic layer was washed with brine, dried over $Na_2SO_4$ and concentrated under reduced pressure to give 5-cyclopropyl-6-oxo-1-phenyl-pyridazine-3-carboxylic acid (41 mg, 84.67% yield) as a yellow solid. LC/MS (ESI) m/z = 257.1 [M+H]$^+$.

Step 2: Synthesis of (R)-N-(1-(3-amino-5-(trifluoromethyl)phenyl)ethyl)-5-cyclopropyl-6-oxo-1-phenyl-1,6-dihydropyridazine-3-carboxamide

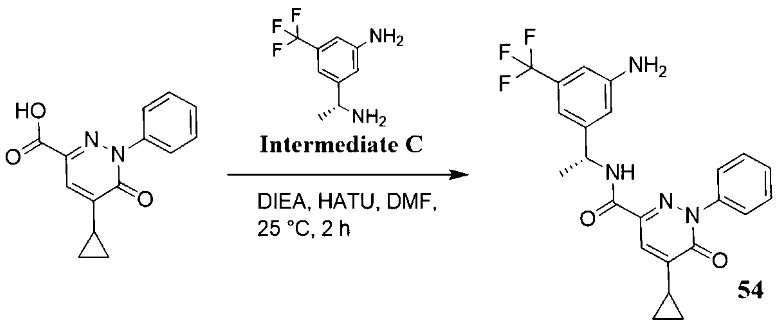

54

The compound of Example 54 was obtained by reacting 5-cyclopropyl-6-oxo-1-phenyl-pyridazine-3-carboxylic acid in the same method as in Step 3 of Example 23. $^1$H NMR (400MHz, DMSO-$d_6$) $\delta$8.78 (d, *J* = 8.4 Hz, 1H), 7.69-7.58 (m, 2H), 7.57-7.49 (m, 2H), 7.49-7.43 (m, 1H), 7.34 (s, 1H), 6.79 (d, *J* = 16.4 Hz, 2H), 6.69 (s, 1H), 5.53 (s, 2H), 5.01 (quin, *J* = 7.2 Hz, 1H), 2.26-2.13 (m, 1H), 1.43 (d, *J* = 7.2 Hz, 3H), 1.13-1.05 (m, 2H), 0.97-0.88 (m, 2H). LC/MS (ESI) m/z = 443.3

[M+H]+.

**Example 55: (R)-N-(1-(3-amino-5-(trifluoromethyl)phenyl)ethyl)-4-cyclopropyl-6-oxo-1-phenyl-1,6-dihydropyridazine-3-carboxamide**

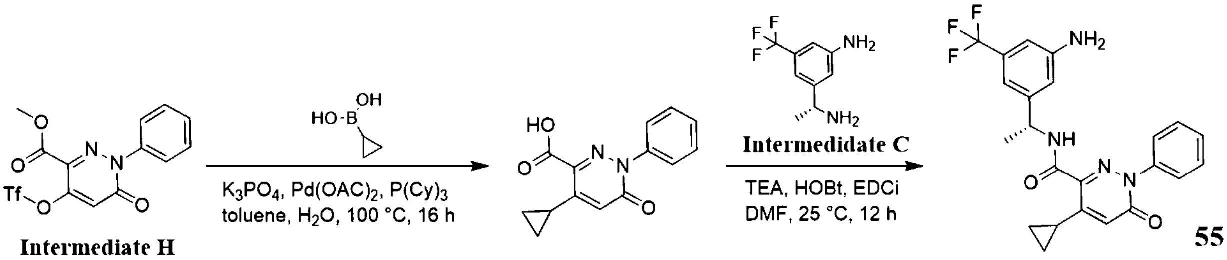


Intermediate H

55

The compound of Example 55 was obtained in the same method as in Example 54, except that Intermediate F was replaced with Intermediate H in Step 1 of Example 54, and the coupling reagent used in Step 2 was changed to HOBt and EDCI. $^1$H NMR (400MHz, DMSO-$d_6$) δ 9.13 (d, J = 8.0 Hz, 1H), 7.66-7.58 (m, 2H), 7.53-7.47 (m, 2H), 7.45-7.40 (m, 1H), 6.79 (d, J = 14.4 Hz, 2H), 6.71 (s, 1H), 6.60 (s, 1H), 5.56 (s, 2H), 4.99 (quin, J =7.2 Hz, 1H), 2.05-1.95 (m, 1H), 1.40 (d, J = 7.2 Hz, 3H), 1.03-0.81 (m, 4H); LC/MS (m/z) = 443.3 [M+H]+.

**Example 56: N-[(1R)-1-[3-amino-5-(trifluoromethyl)phenyl]ethyl]-5-cyano-6-oxo-1-phenyl-pyridazine-3-carboxamide**

Step 1: Synthesis of 5-bromo-N-[(1R)-1-[3-nitro-5-(trifluoromethyl)phenyl]ethyl]-6-oxo-1-phenyl-pyridazine-3-carboxamide

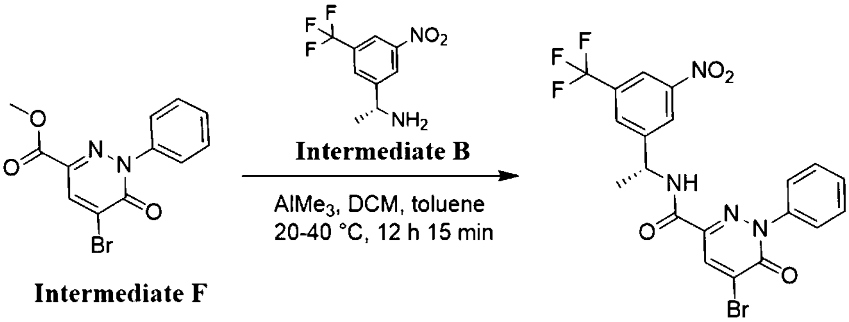


Intermediate F

To a stirred solution of Intermediate B (400 mg, 1.71 mmol) in DCM (2 mL) and toluene (2 mL) was added $AlMe_3$ (2.67 mL, 4.27 mmol, 1.6 M solution in toluene) dropwise, and the mixture was stirred at 20 °C for 15 min under $N_2$ atmosphere. Then, Intermediate F (528 mg, 1.71 mmol) was added thereto, and the reaction mixture was stirred at 40 °C under $N_2$ atmosphere for another 12 h. The reaction mixture was poured into water (20 mL) and extracted with EtOAc. The combined organic layer was washed with brine, dried over $Na_2SO_4$ and concentrated under reduced pressure to obtain a crude product. The product was purified by silica gel column chromatography (0-20% EtOAc in petroleum ether) to give 5-bromo-N-[(1R)-1-[3-nitro-5-(trifluoromethyl)phenyl]ethyl]-6-oxo-1-phenyl-pyridazine-3-carboxamide (253 mg, 28.97% yield) as a yellow solid. $^1$H NMR (400MHz, CHLOROFORM-d) δ 8.39 (s, 3H), 7.93 (s, 1H), 7.59-7.54 (m, 4H), 7.53-7.50 (m, 1H), 7.35 (d, *J* = 7.2 Hz, 1H), 5.38-5.30 (m, 1H), 1.65 (d, *J* = 7.2 Hz, 3H); LC/MS (ESI) m/z = 511.1 [M+H]+.

Step 2: Synthesis of 5-cyano- N-[(1R)-1-[3-nitro-5-(trifluoromethyl)phenyl]ethyl]-6-oxo 1-phenyl-pyridazine-3 -carboxamide

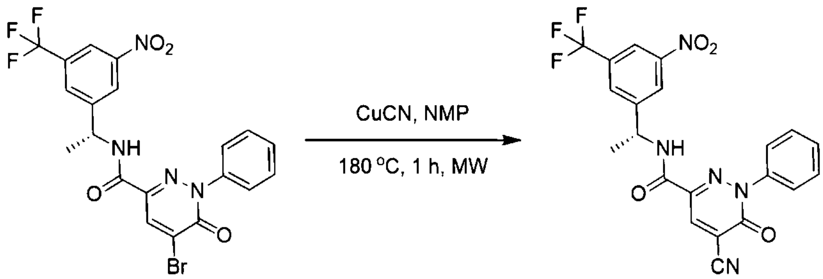


To a solution of 5-bromo-N-[(1R)-1-[3-nitro-5-(trifluoromethyl)phenyl]ethyl]-6-oxo-1-phenyl-pyridazine-3-carboxamide (163 mg, 318.83 μmol) in NMP (2 mL) was added CuCN (142.78 mg, 1.59 mmol, 348.24 μL), and the mixture was stirred at 180 °C for 1 hour in microwave under $N_2$. The reaction mixture was poured into water (20 mL) and extracted with EtOAc, and the combined organic layer was washed with brine, dried over $Na_2SO_4$ and concentrated under reduced pressure to obtain a crude product. The product was purified by silica gel column chromatography (15% EtOAc in petroleum ether) to give 5-cyano-N-[(1R)-1-[3-nitro-5-(trifluoromethyl)phenyl]ethyl]-6-oxo-1-phenyl-pyridazine-3-carboxamide (140 mg, 81.99% yield) as yellow oil. LC/MS (ESI) m/z = 456.1 $[M+H]^+$.

Step 3: Synthesis of N-[(1R)-1-[3-amino-5-(trifluoromethyl)phenyl]ethyl]-5-cyano-6-oxo-1-phenyl-pyridazine-3-carboxamide

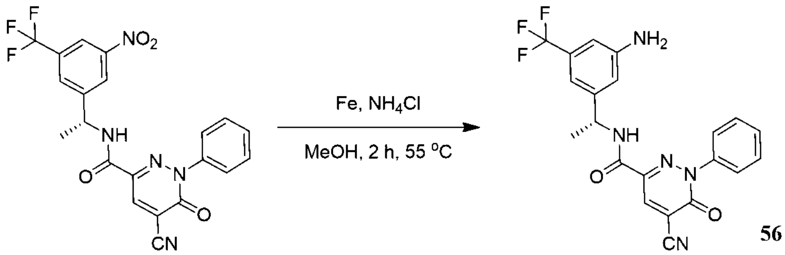

56

To a solution of 5-cyano-N-[(1R)-1-[3-nitro-5-(trifluoromethyl)phenyl]ethyl]-6-oxo-1-phenyl-pyridazine-3-carboxamide (140 mg, 306.10 μmol) in MeOH (3 mL) and $NH_4Cl$ (1 mL) was added Fe (170.94 mg, 3.06 mmol), followed by stirring at 55 °C for 2 h. The reaction mixture was poured into water (30 mL) and extracted with EtOAc, and the combined organic layer was washed with brine, dried over $Na_2SO_4$ and concentrated under reduced pressure to obtain a residue. The residue was purified by prep-HPLC (Phenomenex C18 75*30mm*3μm, mobile phase: [water ($NH_3H_2O+NH_4HCO_3$)-ACN]; B%: 31%-71%, 14min). Most of ACN was removed under reduced pressure, and the remaining solvent was removed by lyophilization to give the compound of Example 56 (9.6 mg, 7.27% yield) as a yellow solid. $^1H$ NMR (400 MHz, DMSO-$d_6$) δ 9.02 (d, *J* = 8.0 Hz, 1H), 8.58 (s, 1H), 7.68 (d, *J* = 7.2 Hz, 2H), 7.59-7.49 (m, 3H), 6.82 (s, 1H), 6.78 (s, 1H), 6.70 (s, 1H), 5.55 (s, 2H), 5.05 (quin, *J* = 7.2 Hz, 1H), 1.44 (d, *J* = 7.2 Hz, 3H); LC/MS (ESI) m/z = 428.3 $[M+H]^+$.

Example 57: N-[(1R)-1-[3-(1,1-difluoro-2-hydroxy-ethyl)phenyl]ethyl]-6-oxo-1-phenyl-pyridazine-3-carboxamide

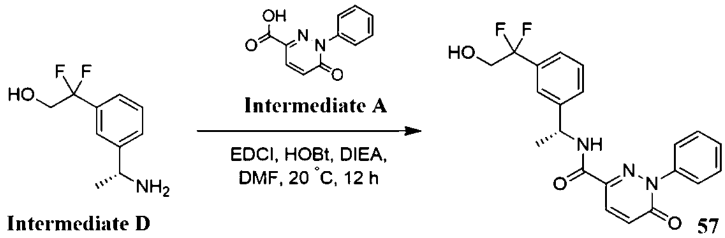

57

A mixture of Intermediate A (70 mg, 323.79 μmol), Intermediate D (91.21 mg, 453.30 μmol), EDCI (124.14 mg, 647.57 μmol), HOBt (87.50 mg, 647.57 μmol) and DIEA (104.62 mg, 809.46 μmol, 140.99 μL) in DMF (2 mL) was degassed and purged with $N_2$ for 3 times, and then was stirred at 20 °C for 12 h under $N_2$ atmosphere. The reaction mixture

was poured into water (20 mL) and extracted with EtOAc. The combined organic layer was washed with brine, dried over $Na_2SO_4$ and concentrated under reduced pressure to obtain a residue. The residue was purified by prep-HPLC (Phenomenex C18 75*30 mm*3 μm, mobile phase: [water ($NH_3H_2O$+$NH_4HCO_3$)-ACN]; B%: 22%-52%, 10 min). ACN was removed under reduced pressure, and the remaining solvent was removed by lyophilization to give the compound of Example 57 (28.0 mg, 15.32% yield) as a white solid. $^1$H NMR(400 MHz, DMSO-$d_6$) δ 8.92 (d, *J* = 8.4 Hz, 1H), 7.89 (d, *J* = 9.8 Hz, 1H), 7.70-7.64 (m, 2H), 7.57-7.50 (m, 4H), 7.50-7.46 (m, 1H), 7.45-7.37 (m, 2H), 7.14 (d, *J* = 9.8 Hz, 1H), 5.60 (t, *J* = 6.4 Hz, 1H), 5.19 (quin, *J* = 7.2 Hz, 1H), 3.83 (dt, *J* = 6.4, 14.4 Hz, 2H), 1.49 (d, *J* = 7.2 Hz, 3H); LC/MS (ESI) m/z = 400.3 $[M+H]^+$.

**Example 58: N-[(1R)-1-[3-(1,1-difluoro-2-hydroxy-ethyl)phenyl]ethyl]-1-(3-methylsulfonylphenyl)-6-oxo-pyridazine-3-carboxamide**

Step 1: Synthesis of methyl-1-(3-methylsulfonylphenyl)-6-oxo-pyridazine-3-carboxylate

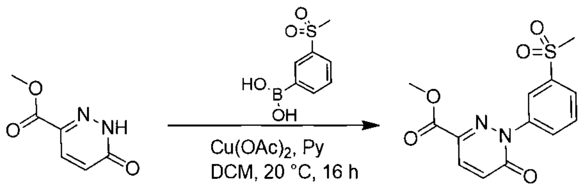


A mixture of methyl-6-oxo-1H-pyridazine-3-carboxylate (150 mg, 973.25 μmol), (3-methylsulfonylphenyl)boronic acid (214.14 mg, 1.07 mmol), $Cu(OAc)_2$ (35.35 mg, 194.65 μmol) and pyridine (500.39 mg, 6.33 mmol) in DCM (3 mL) was degassed and purged with $N_2$ for 3 times, and then stirred at 20 °C for 16 h under $N_2$ atmosphere. The reaction mixture was poured into water (10 mL) and extracted with EtOAc. The combined organic layer was washed with brine, dried over $Na_2SO_4$ and concentrated under reduced pressure to obtain a crude product. The product was purified by flash silica gel chromatography (60% EtOAc in petroleum ether) to give methyl-1-(3-methylsulfonylphenyl)-6-oxo-pyridazine-3-carboxylate (300 mg, 41.67% yield) as a white solid. LC/MS (ESI) m/z = 308.9 $[M+H]^+$.

Step 2: Synthesis of N-[(1R)-1-[3-(1,1-difluoro-2-hydroxy-ethyl)phenyl]ethyl]-1-(3-methylsulfonylphenyl)-6-oxo-pyridazine-3-carboxamide

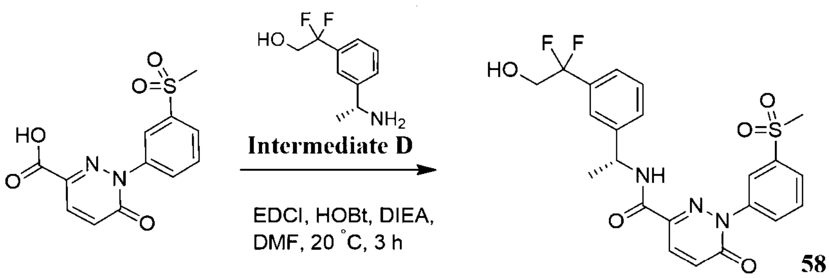


58

The compound of Example 58 was obtained in the same method as in Example 57, except that Intermediate A was replaced with 1-(3-methylsulfonylphenyl)-6-oxo-pyridazine-3-carboxylic acid. $^1$H NMR (400 MHz, DMSO-$d_6$) δ 9.03 (d, *J* = 8.4 Hz, 1H), 8.31 (t, *J* = 2.0 Hz, 1H), 8.12 - 8.01 (m, 2H), 7.92 (d, *J* = 9.6 Hz, 1H), 7.86 (t, *J* = 8.0 Hz, 1H), 7.57-7.52 (m, 2H), 7.45 (t, *J*= 7.2 Hz, 1H), 7.41-7.38 (m, 1H), 7.20 (d, *J* = 9.6 Hz, 1H), 5.63 (br s, 1H), 5.21 (quin, J = 7.2 Hz, 1H), 3.84 (t, *J* = 14.4 Hz, 2H), 3.41-3.31 (s, 3H), 1.51 (d, *J* = 7.2 Hz, 3H); LC/MS (ESI) m/z = 478.3 $[M+H]^+$.

**Example 59: N-[(1R)-1-[3-(1,1-difluoro-2-hydroxy-ethyl)-2-fluoro-phenyl]ethyl]-6-oxo-1-phenyl-pyridazine-3-carboxamide**

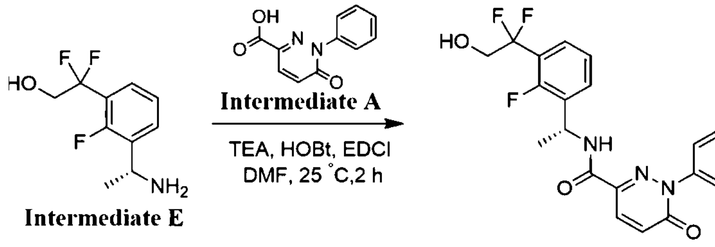

59

The compound of Example 59 was obtained in the same method as in Example 57, except that Intermediate D was replaced with Intermediate E. $^1$H NMR (400MHz, DMSO-$d_6$) δ 8.97 (d, *J* = 8.2 Hz, 1H), 7.88 (d, *J* = 9.6 Hz, 1H), 7.72-7.60 (m, 3H), 7.56 (t, *J* = 7.6 Hz, 2H), 7.51-7.39 (m, 2H), 7.32-7.25 (m, 1H), 7.14 (d, *J* = *9.6* Hz, 1H), 5.72 (t, *J* = 6.4 Hz, 1H), 5.40 (quin, *J* = 7.2 Hz, 1H), 3.91 (dt, *J* = 6.4, 14.4 Hz, 2H), 1.47 (d, *J* = 7.2 Hz, 3H); LC/MS (ESI) m/z = 418.3 [M+H]$^+$.

**Example 60: (R)-N-(1-(3-ethoxyphenyl)ethyl)-6-oxo-1-phenyl-1,6-dihydropyridazine-3-carboxamide**

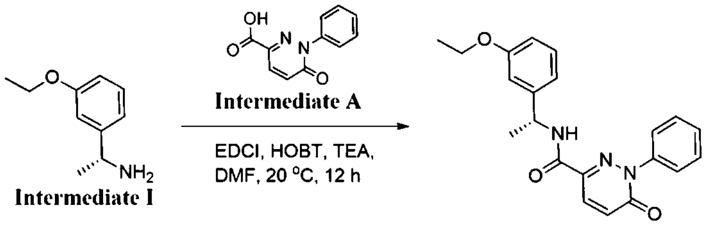

60

The compound of Example 60 was obtained in the same method as in Example 57, except that Intermediate D was replaced with Intermediate I. $^1$H NMR (400MHz, DMSO-$d_6$) δ 8.80 (brd, *J* = 8.4 Hz, 1H), 7.88 (d, *J* = 9.6 Hz, 1H), 7.67 (d, *J* = 7.6 Hz, 2H), 7.54 (t, *J* = 7.6 Hz, 2H), 7.50-7.43 (m, 1H), 7.20 (t, *J*=7.6 Hz, 1H), 7.14 (d, *J* = 9.6 Hz, 1H), 6.98-6.88 (m, 2H), 6.77 (d, *J* = 8.0 Hz, 1H), 5.10 (quin, *J* =7 .2 Hz, 1H), 3.97 (q, *J* = 6.8 Hz, 2H), 1.45 (d, *J* = 6.8 Hz, 3H), 1.29 (t, *J* = 6.8 Hz, 3H); LC/MS (ESI) m/z = 364.3 [M+H]$^+$.

**Example 61: 6-oxo-1-phenyl-N-[(1R)-1-(3-trimethylsilylphenyl)ethyl]pyridazine-3-carboxamide**

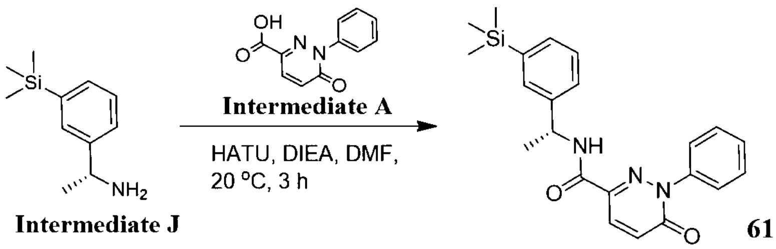

61

To a solution of Intermediate A (24.60 mg, 113.78 μmol) in DMF (2 mL), HATU (64.89 mg, 170.67 μmol) and TEA (34.54 mg, 341.33 μmol, 47.51 μL) were added, and the mixture was stirred at 20 °C for 15 min and added with Intermediate J (22 mg, 113.78 μmol), followed by stirring at 20 °C for 2.75 h under $N_2$. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by prep-HPLC (C18-6 100*30 mm*5 μm, mobile phase: [water (FA)-ACN]; B%: 62%-92%, 15 min). Most of ACN was removed under reduced pressure, and the remaining solvent was removed by lyophilization to give the compound of Example 61 (11.4 mg, 23.65% yield) as an off-white solid. $^1$H NMR (400 MHz, DMSO-$d_6$) δ 8.84 (d, *J* = 8.4Hz, 1H), 7.88 (d, *J* = 9.6Hz, 1H), 7.70-7.64 (m, 2H), 7.56-7.50 (m, 3H), 7.49-7.44 (m, 1H), 7.40-7.35 (m, 2H), 7.32-7.27 (m, 1H), 7.14 (d, *J* = 9.6Hz, 1H), 5.14 (quin, *J* = 7.2Hz, 1H), 1.47 (d, *J* = 7.2Hz, 3H), 0.22 (s, 9H); LC/MS (ESI) m/z = 392.4 [M+H]$^+$.

**Example 62: N-[(1R)-1-[3-amino-5-(trifluoromethyl)phenyl]ethyl]-6-oxo-1-phenylpyridine-3-carboxamide**

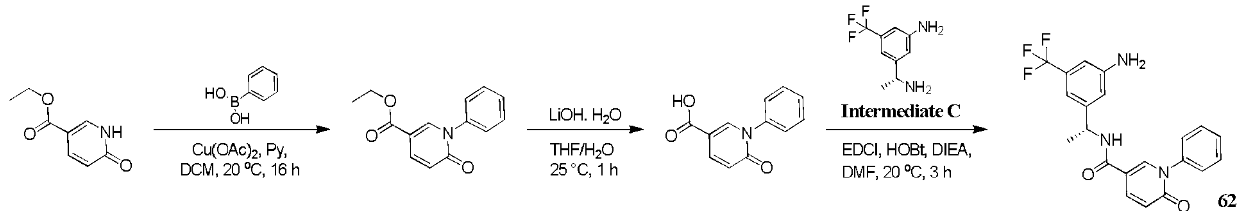

62

The compound of Example 62 was obtained in the same method as in Example 23, except that ethyl-6-oxo-1H-pyridine-3-carboxylate and phenylboronic acid were used as starting materials in Step 1 of Example 23, and the coupling reagent used in Step 3 was changed to HOBt and EDCI. $^1$H NMR (400 MHz, DMSO-$d_6$) δ 8.59 (d, *J* = 7.6 Hz, 1H), 8.38 (d, *J* = 2.4 Hz, 1H), 7.95 (dd, *J* = 2.6, 9.6 Hz, 1H), 7.59-7.53 (m, 2H), 7.53-7.46 (m, 3H), 6.75 (s, 2H), 6.69 (s, 1H), 6.54 (d, *J* = 9.6 Hz, 1H), 5.56 (s, 2H), 5.01 (quin, *J* = 7.2 Hz, 1H), 1.39 (d, *J* = 7.2 Hz, 3H); LC/MS (ESI) m/z = 402.3 [M+H]$^+$.

**Example 63: N-[(1R)-1-[3-amino-5-(trifluoromethyl)phenyl]ethyl]-5-oxo-4-phenyl-pyrazine-2-carboxamide**

Step 1: Synthesis of N-[(1R)-1-[3-nitro-5-(trifluoromethyl)phenyl]ethyl]-5-oxo-4-phenyl-pyrazine-2-carboxamide

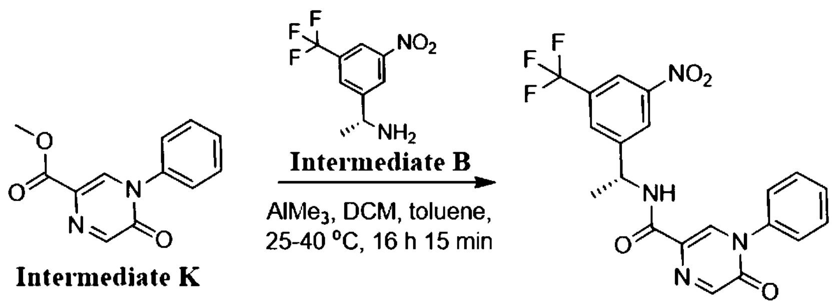

Intermediate K

N-[(1R)-1-[3-nitro-5-(trifluoromethyl)phenyl]ethyl]-5-oxo-4-phenyl-pyrazine-2-carboxamide was obtained in the same method as in Step 1 of Example 56, with replacing Intermediate F with Intermediate K. LC/MS (ESI) m/z = 433.0 [M+H]$^+$.

Step 2: Synthesis of N-[(1R)-1-[3-amino-5-(trifluoromethyl)phenyl]ethyl]-5-oxo-4-phenyl-pyrazine-2-carboxamide

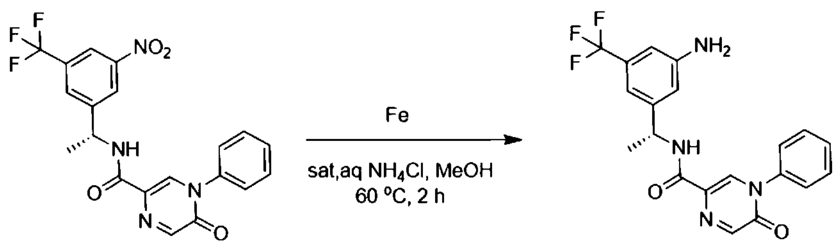

63

The compound of Example 63 was obtained by reacting N-[(1R)-1-[3-nitro-5-(trifluoromethyl)phenyl]ethyl]-5-oxo-4-phenyl-pyrazine-2-carboxamide in the same method as in Step 3 of Example 56. $^1$H NMR (400 MHz, DMSO-$d_6$) δ 8.82 (d, *J* = 8.4 Hz, 1H), 8.14 (s, 1H), 8.03 (s, 1H), 7.58-7.50 (m, 5H), 6.87 (s, 1H), 6.80 (s, 1H), 6.70 (s, 1H), 5.55 (s, 2H), 5.08-5.00 (m, 1H), 1.47 (d, *J* = 7.2 Hz, 3H); LC/MS (ESI) m/z = 403.3 [M+H]$^+$.

**Example 64: N-[(1R)-1-[3-amino-5-(trifluoromethyl)phenyl]ethyl]-1-cyclopropyl-6-oxo-1,6-dihydropyridazine-3-carboxamide**

Step 1: Synthesis of 1-cyclopropyl-6-oxo-1,6-dihydropyridazine-3-carboxylic acid

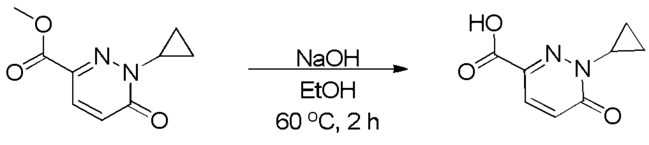


**Intermediate K-9**

To a solution of Intermediate K-9 (57.6 mg, 0.30 mmol) in EtOH (1.3 mL) was added NaOH (2N, 297 μL). The mixture was stirred at 60 °C for 2 h. The reaction mixture was cooled to room temperature and extracted with EtOAc. The aqueous layer was adjusted to pH = 2-3 with aq. 2 N HCl, and then extracted with EtOAc. The combined organic layer was washed with brine, dried over anhydrous $Na_2SO_4$ and concentrated under reduced pressure to give 1-cyclopropyl-6-oxo-1,6-dihydropyridazine-3-carboxylic acid (65.7 mg, crude). LC/MS (m/z) = 181.1 $[M + H]^+$.

Step 2: Synthesis of N-[(1R)-1-[3-amino-5-(trifluoromethyl)phenyl]ethyl]-1-cyclopropyl-6-oxo-1,6-dihydropyridazine-3-carboxamide

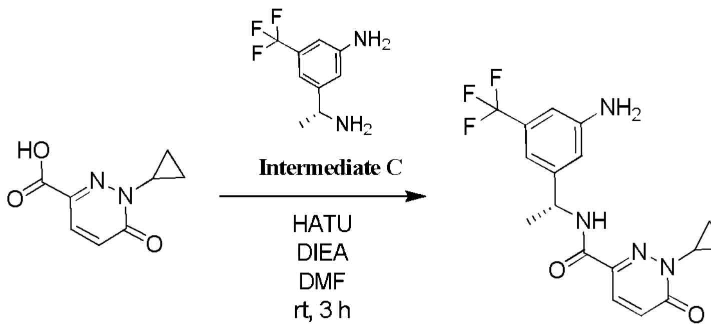


**64**

A mixture of 1-cyclopropyl-6-oxo-1,6-dihydropyridazine-3-carboxylic acid (65.7 mg, 0.36 mmol), Intermediate C (82 mg, 0.40 mmol), HATU (208 mg, 0.55 mmol) and DIEA (191 μL, 1.09 mmol) in DMF (2 mL) was degassed and purged with $N_2$ for 3 times, and then stirred at room temperature for 3 h under $N_2$ atmosphere. The reaction mixture was poured into water and extracted with EtOAc. The combined organic layer was washed with brine, dried over anhydrous $Na_2SO_4$ and concentrated under reduced pressure to obtain a crude product. The residue was purified by prep-HPLC. The solvent was removed under reduced pressure to give the compound of Example 64 (20.3 mg, 15.2% yield). $^1H$ NMR (400 MHz, MeOD) δ 7.90 (d, J = 9.6 Hz, 1H), 7.06 - 6.98 (m, 2H), 6.96 (s, 1H), 6.88 (s, 1H), 5.17 - 5.10 (m, 1H), 4.05 (tt, J = 7.6, 3.9 Hz, 1H), 2.19 (t, J = 7.6 Hz, 1H), 2.03 (d, J = 6.1 Hz, 1H), 1.56 (d, J = 7.0 Hz, 3H), 1.13 - 1.04 (m, 2H). LC/MS (m/z) = 367.1 $[M + H]^+$.

**Example 65 and Example 66**

The compounds shown in the following table were prepared in the same manner as in Example 64 by using starting materials corresponding to the respective structures of the desired compounds based on Preparation Example 11.

[Table 3]

| No. | Structure/Name | Spectral Data |
|---|---|---|
| 65 | 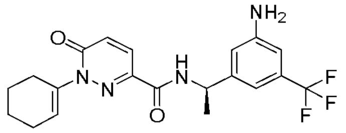 N-[(1R)-1-[3-amino-5-(trifluoromethyl)phenyl] ethyl]-1-(cyclohex-1-en-1-yl)-6-oxo-1,6-dihydropyridazine-3-carboxamide | $^1H$ NMR (400 MHz, DMSO) δ 8.81 (d, J = 8.3 Hz, 1H), 7.80 (d, J = 9.7 Hz, 1H), 7.01 (d, J = 9.7 Hz, 1H), 6.88 (s, 1H), 6.83 (s, 1H), 6.75 (s, 1H), 6.05 - 5.98 (m, 1H), 5.08 - 5.00 (m, 1H), 3.96 (s, 1H), 2.37 - 2.30 (m, 1H), 2.21 (dd, J = 6.6, 3.4 Hz, 2H), 1.79 - 1.70 (m, 2H), 1.68 - 1.59 (m, 2H), 1.48 (d, J = 7.1 Hz, 3H); LC/MS (ESI) m/z = 407.1 $[M+H]^+$ |

(continued)

| No. | Structure/Name | Spectral Data |
|---|---|---|
| 66 | 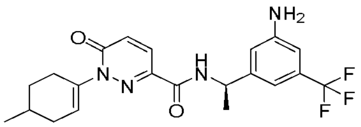 N-[(1R)-1-[3-amino-5-(trifluoromethyl)phenyl]ethyl]-1-(4-methylcyclohex-1-en-1-yl)-6-oxo-1,6-dihydropyridazine-3-carboxamide | $^1$H NMR (400 MHz, DMSO) δ 8.78 (d, J = 8.3 Hz, 1H), 7.80 (d, J = 9.7 Hz, 1H), 7.01 (d, J = 9.7 Hz, 1H), 6.81 (d, J = 16.6 Hz, 2H), 6.71 (s, 1H), 5.99 (s, 1H), 5.57 (s, 2H), 5.07 - 4.99 (m, 1H), 2.43 (s, 1H), 2.38 - 2.25 (m, 2H), 1.80 (s, 2H), 1.75 (s, 1H), 1.47 (d, J = 7.1 Hz, 3H), 1.40 (dt, J = 11.9, 5.5 Hz, 1H), 1.01 (d, J = 6.3 Hz, 3H); LC/MS (ESI) m/z = 421.2 $[M+H]^+$ |

**Example 67: N-[(1R)-1-[3-amino-5-(trifluoromethyl)phenyl]ethyl]-1-(1-methanesulfonyl-1,2,3,6-tetrahydropyridin-4-yl)-6-oxo-1,6-dihydropyridazine-3-carboxamide**

Step 1: Synthesis of methyl-1-(1-(methylsulfonyl)-1,2,3,6-tetrahydropyridin-4-yl)-6-oxo-1,6-dihydropyridazine-3-carboxylate

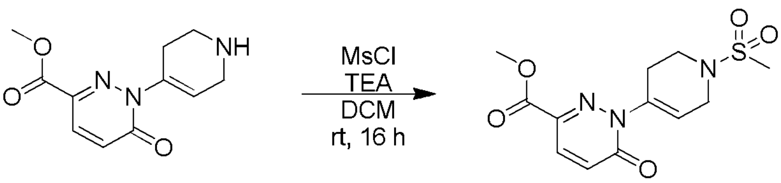


**Intermediate K-10**

To a solution of Intermediate K-10 (48.5 mg, 0.21 mmol) in DCM (200 μL) was added TEA (86 μL, 0.62 mmol). The mixture was cooled to 0-5 °C and then added slowly dropwise with methanesulfonyl chloride (19.15 μL, 0.25 mmol), maintaining the reaction temperature to be lower than 20 °C. After the addition, the mixture was stirred at room temperature for 16 h. The reaction was quenched by slow addition of $H_2O$, and the layers were separated. The aqueous layer was extracted with DCM. The combined organic layer was washed successively with sat. $NH_4C1$, sat. $NaHCO_3$, sat. $NH_4C1$ and brine, dried over anhydrous $Na_2SO_4$, and concentrated under reduced pressure to obtain a crude product. The residue was purified by prep-HPLC. The solvent was removed under reduced pressure to give methyl-1-(1-(methylsulfonyl)-1,2,3,6-tetrahydropyridin-4-yl)-6-oxo-1,6-dihydropyridazine-3-carboxylate (7.8 mg, 12.1% yield). LC/MS (m/z) = 314.1 $[M + H]^+$.

Steps 2 and 3: Synthesis of N-[(1R)-1-[3-amino-5-(trifluoromethyl)phenyl]ethyl]-1-(1-methanesulfonyl-1,2,3,6-tetrahydropyridin-4-yl)-6-oxo-1,6-dihydropyridazine-3-carboxamide

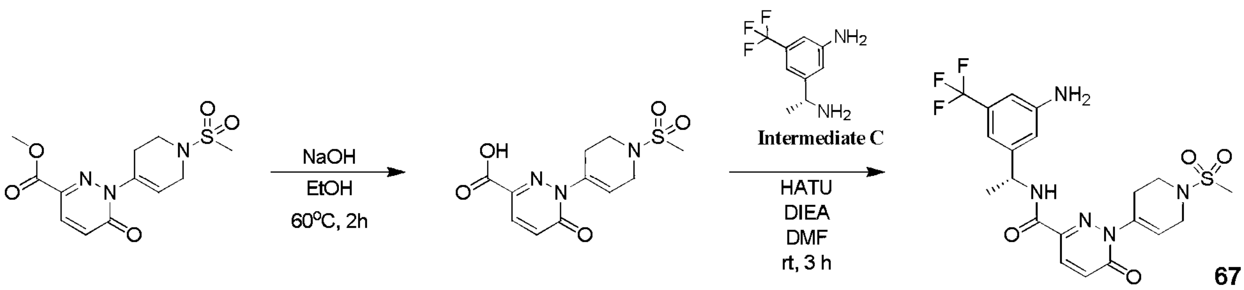


**67**

The compound of Example 67 (2.6 mg, 31.4% yield) was obtained in the same manner as in Example 64. $^1$H NMR (400 MHz, DMSO) δ 8.84 (d, J = 8.3 Hz, 1H), 7.83 (d, J = 9.6 Hz, 1H), 7.05 (d, J = 9.8 Hz, 1H), 6.83 (s, 1H), 6.79 (s, 1H), 6.70 (d, J = 7.2 Hz, 3H), 6.28 (s, 2H), 5.04 (s, 1H), 3.94 (s, 2H), 3.42 (d, J = 5.4 Hz, 1H), 2.99 (s, 3H), 2.67 (s, 2H), 1.47 (d, J = 6.9 Hz, 3H); LC/MS m/z = 486.2 $[M+H]^+$.

**Example 68: 1-(1-acetyl-1,2,3,6-tetrahydropyridin-4-yl)-N-[(1R)-1-[3-amino-5-(trifluoromethyl)phenyl]ethyl]-6-oxo-1,6-dihydropyridazine-3-carboxamide**

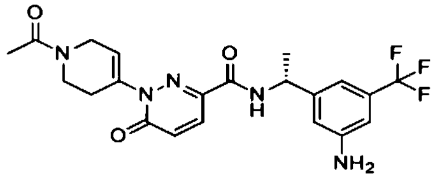

68

The compound of Example 68 (5.3 mg, 15% yield) was obtained in the same manner as in Example 67, except that methanesulfonyl chloride was replaced with acetic anhydride in Step 1 of Example 67. $^1$H NMR (400 MHz, MeOD) δ 7.94 (dd, J = 9.7, 1.3 Hz, 1H), 7.06 (dd, J = 9.7, 1.3 Hz, 1H), 6.90 (d, J = 9.7 Hz, 2H), 6.81 (s, 1H), 6.18 (s, 1H), 5.13 (q, J = 7.1 Hz, 1H), 4.31 - 4.25 (m, 2H), 3.90 - 3.77 (m, 2H), 2.67 (s, 1H), 2.59 (s, 1H), 2.18 (d, J = 7.6 Hz, 3H), 1.54 (d, J = 7.1 Hz, 3H); LC/MS m/z = 450.2 [M+H]$^+$.

**Example 69: N-[(1R)-1-[3-amino-5-(trifluoromethyl)phenyl]ethyl]-1-(1-methanesulfonyl-1,2,5,6-tetrahydropyridin-3-yl)-6-oxo-1,6-dihydropyridazine-3-carboxamide**

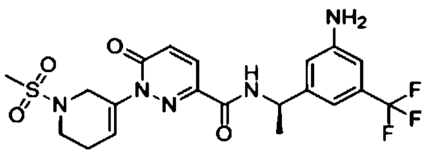

69

Intermediate K-11 used as a starting material was acidified, and the compound of Example 69 (3.5 mg, 11.5% yield) was then obtained in the same manner as in Example 67. $^1$H NMR (400 MHz, MeOD) δ 7.94 (d, J = 9.7 Hz, 1H), 7.26 (s, 1H), 7.18 (s, 1H), 7.11 - 7.03 (m, 2H), 6.30 (dq, J = 4.2, 2.0 Hz, 1H), 5.18 (q, J = 6.9 Hz, 1H), 4.14 (d, J = 2.2 Hz, 1H), 3.53 (t, J = 5.8 Hz, 2H), 3.00 (s, 3H), 2.53 (dq, J = 5.9, 3.2 Hz, 2H), 1.57 (d, J = 7.1 Hz, 3H); LC/MS m/z = 486.2 [M+H]$^+$.

**Example 70: N-[(1R)-1-[3-amino-5-(trifluoromethyl)phenyl]ethyl]-1-{bicyclo[1.1.1]pentan-1-yl}-6-oxo-1,6-dihydropyridazine-3-carboxamide**

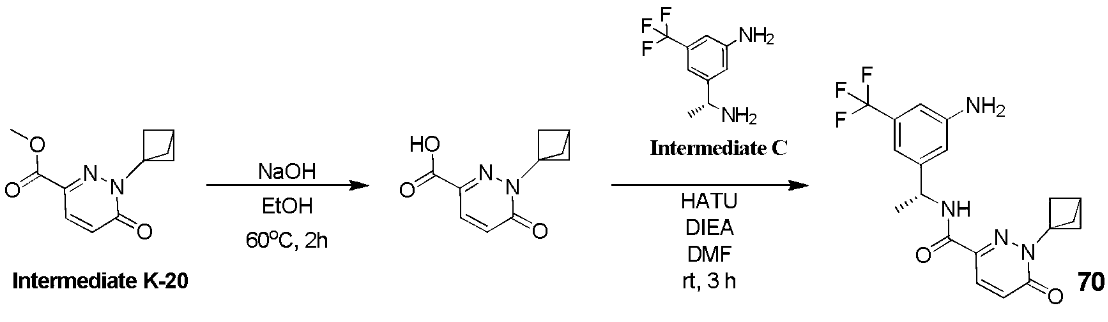


70

The compound of Example 70 (2.9 mg, 90% yield) was obtained in the same manner as in Example 64. $^1$H NMR (400 MHz, MeOD) δ 7.61 (s, 1H), 7.29 (s, 1H), 7.20 (s, 1H), 7.12 (s, 1H), 5.16 (q, J = 7.0 Hz, 1H), 2.58 (s, 1H), 2.19 (s, 6H), 1.56 (d, J = 7.1 Hz, 3H); LC/MS m/z = 393.2 [M+H]$^+$.

**Example 71: N-[(1R)-1-[3-amino-5-(trifluoromethyl)phenyl]ethyl]-1-[3-(hydroxymethyl)phenyl]-6-oxo-1,6-dihydropyridine-3-carboxamide**

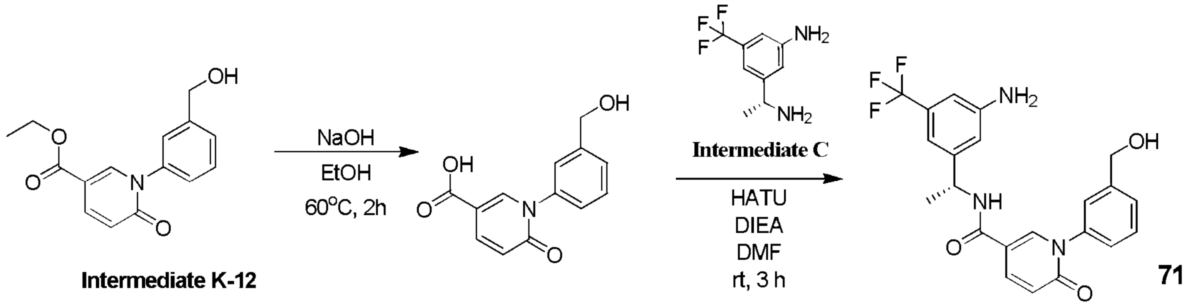


The compound of Example 71 (38.1 mg, 87% yield) was obtained in the same manner as in Example 64. $^1$H NMR (400 MHz, DMSO) δ 8.59 (d, J = 7.7 Hz, 1H), 8.35 (d, J = 2.6 Hz, 1H), 7.96 (dd, J = 9.6, 2.7 Hz, 1H), 7.55 - 7.30 (m, 5H), 6.77 (s, 2H), 6.71 (s, 1H), 6.53 (d, J = 9.6 Hz, 1H), 5.01 (t, J = 7.3 Hz, 1H), 4.58 (s, 2H), 1.39 (d, J = 7.1 Hz, 3H); LC/MS m/z = 432.2 $[M+H]^+$.

## Example 72 and Example 73

The compounds shown in the table below were prepared in the same manner as in Example 71 by using appropriate starting materials corresponding to the respective structures of the desired compounds based on Preparation Example 7.

[Table 4]

| No. | Structure/Name | Spectral Data |
|---|---|---|
| 72 | 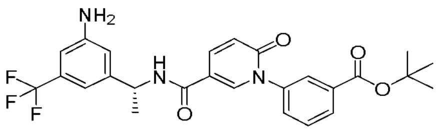<br>Tert-butyl 3-(5-{[(1R)-1-[3-amino-5-(trifluoromethyl) phenyl]ethyl]carbamoyl}-2-oxo-1,2-dihydropyridin-1-yl)benzoate | $^1$H NMR (400 MHz, MeOD) δ 8.30 (dd, J = 7.6, 2.7 Hz, 1H), 8.13 - 7.99 (m, 3H), 7.66 (dd, J = 4.9, 2.0 Hz, 3H), 6.99 (d, J = 13.3 Hz, 2H), 6.90 (s, 1H), 6.66 (d, J = 9.6 Hz, 1H), 5.12 (d, J = 7.0 Hz, 1H), 1.60 (s, 9H), 1.51 (d, J = 7.1 Hz, 3H). LC/MS (m/z) = 502.2 $[M+H]^+$ |
| 73 | 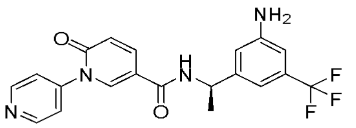<br>N-[(1R)-1-[3-amino-5-(trifluoromethyl)phenyl]ethyl]-2-oxo-2H-[1,4'-bipyridine]-5-carboxamide | $^1$H NMR (400 MHz, MeOD) δ 8.78 - 8.72 (m, 2H), 8.30 (d, J = 2.6 Hz, 1H), 8.04 (dd, J = 9.6, 2.6 Hz, 1H), 7.65 - 7.59 (m, 2H), 6.91 - 6.85 (m, 2H), 6.80 (d, J = 2.0 Hz, 1H), 6.65 (d, J = 9.6 Hz, 1H), 5.19 - 5.05 (m, 1H), 1.51 (d, J = 7.1 Hz, 3H). LC/MS (m/z) = 403.2 [M+H]+. LC/MS (m/z) = 403.2 $[M+H]^+$ |

## Example 74: N-[(1R)-1-[3-amino-5-(trifluoromethyl)phenyl]ethyl]-1'-methanesulfonyl-6-oxo-2',3'-dihydro-1'H,6H,6'H-[1,4'-bipyridine]-3-carboxamide

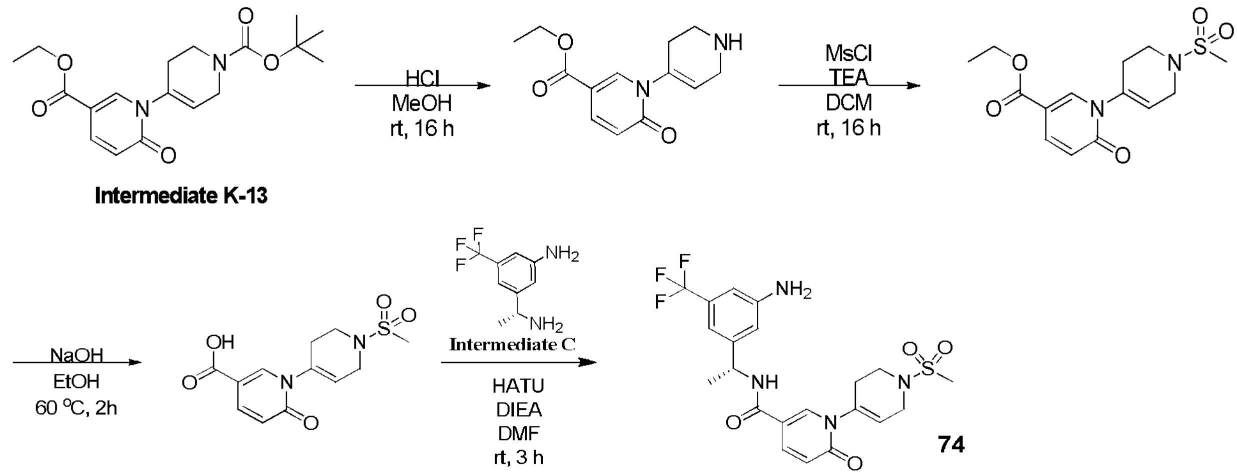


Intermediate K-13

74

Intermediate K-13 was acidified, and the compound of Example 74 (7.2 mg, 7.3% yield) was then obtained in the same manner as in Example 67. $^1$H NMR (400 MHz, MeOD) δ 8.18 (d, J = 2.5 Hz, 1H), 7.99 (dd, J = 9.6, 2.6 Hz, 1H), 6.88 (q, J = 1.7 Hz, 2H), 6.80 (d, J = 2.0 Hz, 1H), 6.54 (d, J = 9.6 Hz, 1H), 6.04 - 5.97 (m, 1H), 5.10 (q, J = 7.0 Hz, 1H), 4.01 (q, J = 2.9 Hz, 2H), 3.61 - 3.53 (m, 2H), 2.95 (s, 3H), 2.61 (dq, J = 6.1, 3.1 Hz, 2H), 1.51 (d, J = 7.1 Hz, 3H); LC/MS m/z = 485.2 [M+H]$^+$.

**Example 75: N-[(1R)-1-[3-amino-5-(trifluoromethyl)phenyl]ethyl]-1-[3-(morpholine-4-carbonyl)phenyl]-6-oxo-1,6-dihydropyridine-3-carboxamide**

Step 1: Synthesis of (R)-3-(5-((1-(3-amino-5-(trifluoromethyl)phenyl)ethyl)carbamoyl)-2-oxopyridin-1(2H)-yl)benzoic acid

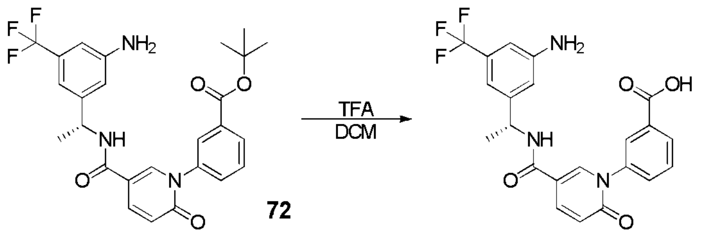


72

To a solution of the compound of Example 72 (10.2 mg, 0.020 mmol) in DCM (1 mL) was added TFA (7.83 μL, 0.10 mmol). The mixture was stirred at room temperature. The mixture was concentrated under reduced pressure to give (R)-3-(5-((1-(3-amino-5-(trifluoromethyl)phenyl)ethyl)carbamoyl)-2-oxopyridin-1(2H)-yl)benzoic acid (crude, 10 mg, 0.022 mmol), which was used in the next step without further purification. LC/MS m/z = 446.2 [M+H]$^+$.

Step 2: Synthesis of N-[(1R)-1-[3-amino-5-(trifluoromethyl)phenyl]ethyl]-1-[3-(morpholine-4-carbonyl) phenyl]-6-oxo-1,6-dihydropyridine-3-carboxamide

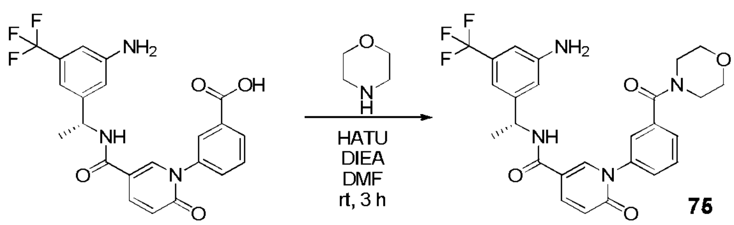


75

A mixture of (R)-3-(5-((1-(3-amino-5-(trifluoromethyl)phenyl)ethyl)carbamoyl)-2-oxopyridin-1(2H)-yl)benzoic acid (10 mg, 0.022 mmol), morpholine (2.15 mg, 0.025 mmol), HATU (12.81 mg, 0.034 mmol) and DIEA (8.6 μL,

0.067 mmol) in DMF (0.1 mL) was degassed and purged with $N_2$ for 3 times, and then stirred at room temparature for 3 h under $N_2$ atmosphere. The reaction mixture was poured into water and extracted with EtOAc. The combined organic layer was washed with brine, dried over anhydrous $Na_2SO_4$ and concentrated under reduced pressure to obtain a crude product. The residue was purified by prep-HPLC. The solvent was removed under reduced pressure to give the compound of Example 75 (0.9 mg, 7.8% yield). $^1H$ NMR (400 MHz, MeOD) δ 8.31 (d, J = 2.7 Hz, 1H), 8.05 (dd, J = 9.6, 2.6 Hz, 1H), 7.71 - 7.63 (m, 1H), 7.58 (d, J = 7.4 Hz, 3H), 7.11 (s, 1H), 7.06 (s, 1H), 6.99 (s, 1H), 6.66 (d, J = 9.6 Hz, 1H), 5.14 (q, J = 7.1 Hz, 1H), 3.76 (s, 5H), 3.65 (s, 3H), 3.52 (s, 2H), 1.52 (d, J = 7.1 Hz, 3H); LC/MS m/z = 515.2 $[M+H]^+$ .

**Example 76: N-(1-(3-chlorophenyl)cyclopropyl)-1-(2-fluorophenyl)-6-oxo-1,6-dihydropyridazine-3-carboxamide**

Step 1: Synthesis of 1-(2-fluorophenyl)-6-oxo-1,6-dihydropyridazine-3-carboxylic acid

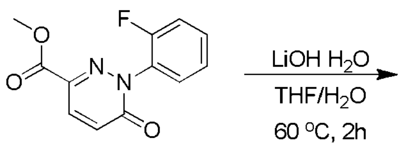


**Intermediate K-14**

To a solution of Intermediate K-14 (91.3 mg, 0.37 mmol) in THF (1 mL), $LiOH \cdot H_2O$ (30.9 mg, 0.74 mmol) and $H_2O$ (0.5 mL) were added. The mixture was stirred at 25 °C for 3 h. The reaction mixture was adjusted to pH = 3-4 with aq. 1 N HCl and extracted with EtOAc. The aqueous layer was filtrated to give 1-(2-fluorophenyl)-6-oxo-1,6-dihydropyridazine-3-carboxylic acid (79.9 mg, 0.37 mmol, 93% yield) as a yellow solid. LC/MS (m/z) = 235.1 $[M+H]^+$.

Step 2: Synthesis of N-(1-(3-chlorophenyl)cyclopropyl)-1-(2-fluorophenyl)-6-oxo-1,6-dihydropyridazine-3-carboxamide

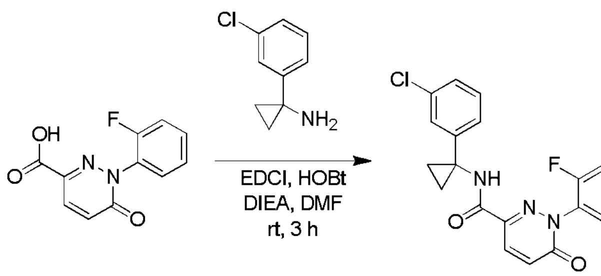


**76**

To a solution of 1-(2-fluorophenyl)-6-oxo-1,6-dihydropyridazine-3-carboxylic acid (25 mg, 0.11 mmol) and 1-(3-chlorophenyl)cyclopropan-1-amine (15.9 μL, 0.12 mmol) in DMF (1 mL), EDCI (21.7 mg, 0.14 mmol), HOBt (18.9 mg, 0.14 mmol) and DIEA (55.8 μL, 0.32 mmol) were added, and the mixture was degassed and purged with $N_2$ for 3 times, and then stirred at 25 °C for 1 hour under $N_2$ atmosphere. The reaction mixture was partitioned between $H_2O$ and EtOAc. The organic layer was separated, washed with brine, dried over $Na_2SO_4$, filtered and concentrated under reduced pressure to obtain a residue. The residue was purified by prep-HPLC to give the compound of Example 76 (9.4 mg, 22.9% yield). LC/MS m/z = 384.1 $[M+H]^+$.

**Example 77: N-[(1R)-1-(3-chlorophenyl)propyl]-1-(2-fluorophenyl)-6-oxo-1,6-dihydropyridazine-3-carboxamide**

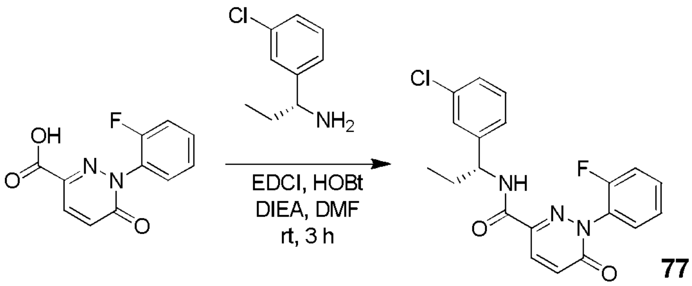


77

The compound of Example 77 (17.5 mg, 38.6% yield) was obtained in the same manner as in Example 76, except that (R)-1-(3-chlorophenyl)propan-1-amine was used instead of 1-(3-chlorophenyl)cyclopropan-1-amine. LC/MS m/z = 386.1 [M+H]+.

**Example 78: N-[(1R)-1-[3-(1,1-difluoro-2-hydroxyethyl)-2-fluorophenyl]ethyl]-1-[3-(1-methyl-1H-pyrazol-5-yl) phenyl]-6-oxo-1,6-dihydropyridine-3-carboxamide**

Steps 1 and 2: Synthesis of (R)-1-(3-bromophenyl)-N-(1-(3-(1,1-difluoro-2-hydroxyethyl)-2-fluorophenyl)ethyl)-6-oxo-1,6-dihydropyridine-3-carboxamide

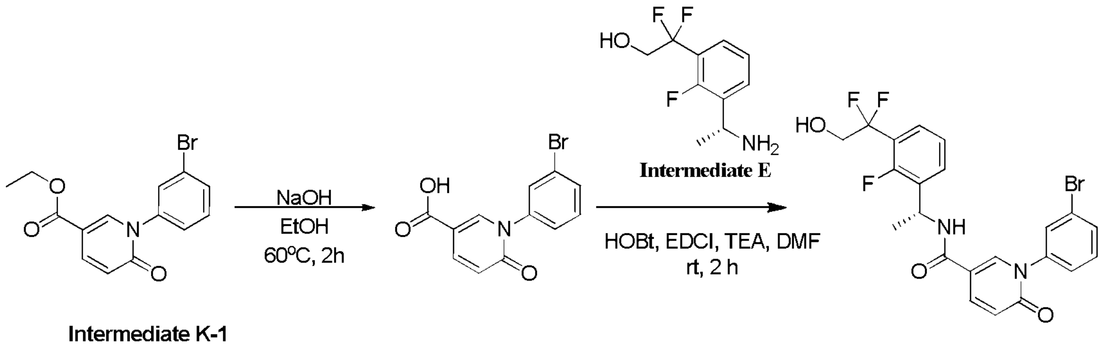


Intermediate K-1

(R)-1-(3-bromophenyl)-N-(1-(3-(1,1-difluoro-2-hydroxyethyl)-2-fluorophenyl)ethyl)-6-oxo-1,6-dihydropyridine-3-carboxamide (20.2 mg, 55.3% yield) was obtained in a similar manner to Example 64, with changing the coupling reagents used in Step 2 to HOBt and EDCI. LC/MS m/z = 495 [M+H]+.

Step 3: Synthesis of N-[(1R)-1-[3-(1,1-difluoro-2-hydroxyethyl)-2-fluorophenyl]ethyl]-1-[3-(1-methyl-1H-pyrazol-5-yl) phenyl]-6-oxo-1,6-dihydropyridine-3-carboxamide

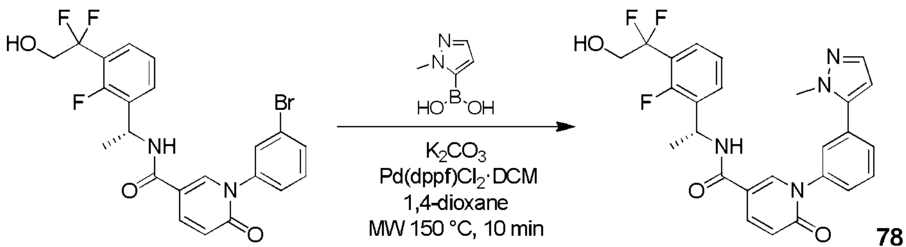


78

To a solution of (R)-1-(3-bromophenyl)-N-(1-(3-(1,1-difluoro-2-hydroxyethyl)-2-fluorophenyl)ethyl)-6-oxo-1,6-dihydropyridine-3-carboxamide (13.4 mg, 0.027 mmol) in 1,4-dioxane (0.5 mL) and water (0.1 mL), (1-methyl-1H-pyrazol-5-yl)boronic acid (3.75 mg, 0.030 mmol), potassium carbonate (14.96 mg, 0.11 mmol) and $Pd(dppf)Cl_2 \cdot DCM$ (2.21 mg, 2.71 μmol) were added. The mixture was heated by microwave at 150 °C for 10 min. The mixture was cooled to room temperature, added with water and extracted with DCM. The organic layer was washed with brine, dried over anhydrous $Na_2SO_4$, and concentrated under reduced pressure to obtain a crude product. The residue was purified by

prep-HPLC. The solvent was removed under reduced pressure to give the compound of Example 78 (3.7 mg, 27.5% yield). $^1H$ NMR (400 MHz, MeOD) δ 8.35 (d, J = 2.6 Hz, 1H), 8.05 (dd, J = 9.6, 2.6 Hz, 1H), 7.73 - 7.61 (m, 3H), 7.54 (dt, J = 9.1, 1.9 Hz, 2H), 7.32 (s, 1H), 7.24 (s, 1H), 7.16 (s, 1H), 6.67 (dd, J = 9.6, 0.7 Hz, 1H), 6.47 (d, J = 2.0 Hz, 1H), 5.16 (q, J = 7.1 Hz, 1H), 3.92 (s, 3H), 1.54 (d, J = 7.1 Hz, 3H); LC/MS m/z = 482.2 $[M+H]^+$.

**Example 79: N-[(1R)-1-[3-amino-5-(trifluoromethyl)phenyl]ethyl]-1-[3-(methylamino)phenyl]-6-oxo-1,6-dihydropyridine-3-carboxamide**

Step 1: Synthesis of ethyl-1-[3-[tert-butoxycarbonyl(methyl)amino]phenyl]-6-oxo-pyridine-3-carboxylate

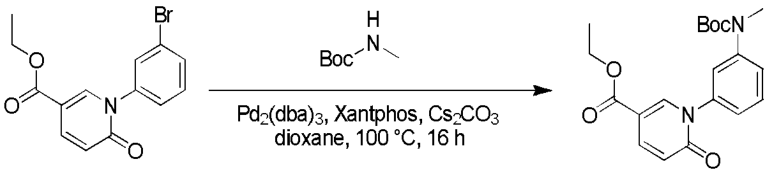


**Intermediate K-1**

A mixture of Intermediate K-1 (300 mg, 931 μmol), tert-butyl N-methylcarbamate (146 mg, 1.12 mmol), $Pd_2(dba)_3$ (85.2 mg, 93.1 μmol), Xantphos (53.8 mg, 93.1 μmol) and $Cs_2CO_3$ (758 mg, 2.33 mmol) in dioxane (6 mL) was stirred at 100 °C for 16 h. The mixture was concentrated in vacuo. The residue was purified by column chromatography ($SiO_2$, PE: EA = 2: 1 to 5: 1) to give ethyl-1-[3-[tert-butoxycarbonyl(methyl)amino]phenyl]-6-oxo-pyridine-3-carboxylate (200 mg, 57% yield) as yellow oil. $^1H$ NMR (400 MHz, DMSO-$d_6$) δ = 8.21 (d, *J* = 2.0 Hz, 1H), 7.88 (dd, *J* = 2.4, 9.6 Hz, 1H), 7.54 - 7.41 (m, 3H), 7.26 (br d, *J* = 7.2 Hz, 1H), 6.56 (d, *J* = 9.6 Hz, 1H), 4.25 (q, *J* = 7.2 Hz, 2H), 3.23 (s, 3H), 1.42 (s, 9H), 1.26 (t, *J* = 7.2 Hz, 3H).

Steps 2 and 3: Synthesis of tert-butyl N-[3-[5-[[(1R)-1-[3-nitro-5-(trifluoromethyl)phenyl]ethyl]carbamoyl]-2-oxo-1-pyridyl]phenyl]-N-methyl-carbamate

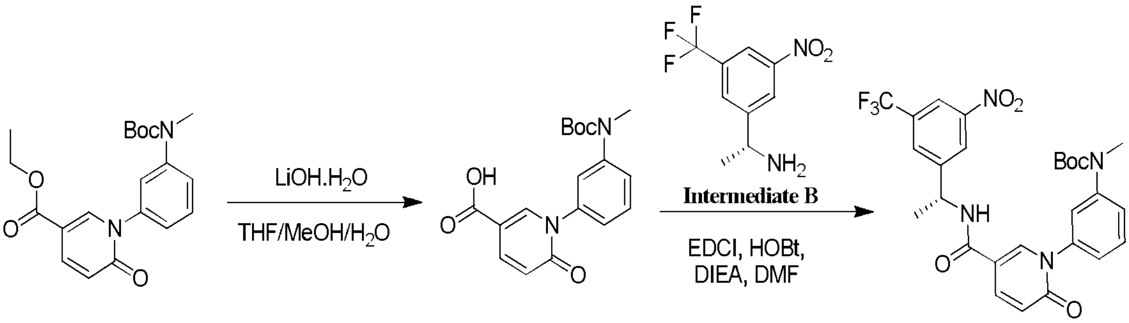


Tert-butyl N-[3-[5-[[(1R)-1-[3-nitro-5-(trifluoromethyl)phenyl]ethyl]carbamoyl]-2-oxo-1-pyridyl]phenyl]-N-methyl-carbamate (150 mg, 76% yield) was obtained in the same manner as in Example 76. $^1H$ NMR (400 MHz, DMSO-$d_6$) δ = 8.79 (d, *J* = 7.2 Hz, 1H), 8.52 (s, 1H), 8.36 (d, *J* = 2.4 Hz, 2H), 8.22 (s, 1H), 7.97 - 7.88 (m, 1H), 7.54 - 7.48 (m, 1H), 7.46 - 7.41 (m, 2H), 7.28 (d, *J* = 7.6 Hz, 1H), 6.54 (d, *J* = 9.6 Hz, 1H), 5.31 (t, *J* = 6.8 Hz, 1H), 3.32 (s, 3H), 1.49 (d, *J* = 7.2 Hz, 3H), 1.42 (s, 9H).

Step 4: Synthesis of tert-butyl N-[3-[5-[[(1R)-1-[3-amino-5-(trifluoromethyl)phenyl]ethyl]carbamoyl]-2-oxo-1-pyridyl]phenyl]-N-methyl-carbamate

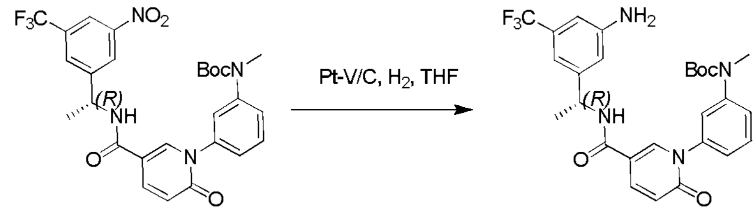


A mixture of tert-butyl N-methyl-N-[3-[5-[[(1R)-1-[3-nitro-5-(trifluoromethyl)phenyl]ethyl]carbamoyl]-2-oxo-1-pyridyl]phenyl]carbamate (100 mg, 178 μmol) and Pt-V/C (100 mg, 5% purity) in THF (1 mL) was stirred at 25 °C under $H_2$ (15 psi) for 1 hour. The mixture was filtrated, and the filtrate was concentrated in vacuo to give tert-butyl N-[3-[5-[[(1R)-1-[3-amino-5-(trifluoromethyl)phenyl]ethyl]carbamoyl]-2-oxo-1-pyridyl]phenyl]-N-methyl-carbamate (90 mg, 95% yield) as a colorless solid. MS (EI) m/z: 553.2 [M+Na]+.

Step 5: Synthesis of N-[(1R)-1-[3-amino-5-(trifluoromethyl)phenyl]ethyl]-1-[3-(methylamino)phenyl]-6-oxo-pyridine-3-carboxamide

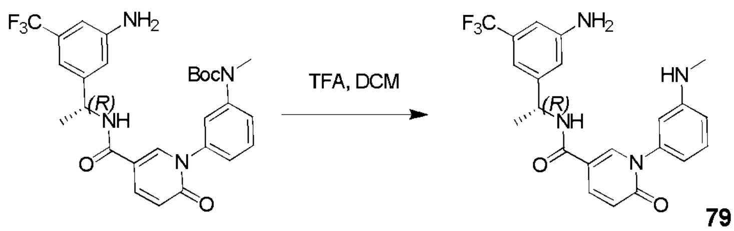


79

To a mixture of tert-butyl N-[3-[5-[[(1R)-1-[3-amino-5-(trifluoromethyl)phenyl]ethyl]carbamoyl]-2-oxo-1-pyridyl]phenyl]-N-methyl-carbamate (50 mg, 94.2 μmol) in TFA (0.3 mL) was added DCM (1 mL) at 0 °C, followed by stirring at 0 °C for 1 hour. The mixture was purified by Prep-HPLC (column: Phenomenex luna C18 150*25 mm*10 μm, mobile phase: [water(FA)-ACN];B%: 24%-54%,10 min) to give the compound of Example 79 (13.9 mg, 33% yield) as a white solid. $^1$H NMR (400 MHz, DMSO-$d_6$) δ = 8.58 (d, *J* = 7.6 Hz, 1H), 8.35 (d, *J* = 2.4 Hz, 1H), 7.94 (dd, *J* = 2.4, 9.6 Hz, 1H), 7.23 (t, *J* = 8.0 Hz, 1H), 6.77 (s, 2H), 6.70 (s, 1H), 6.65 (dd, *J* = 1.6, 8.4 Hz, 1H), 6.57 - 6.48 (m, 3H), 6.16 - 5.37 (m, 2H), 5.01 (t, *J* = 7.2 Hz, 1H), 2.70 (s, 3H), 1.40 (d, *J* = 7.2 Hz, 3H). MS (EI) m/z: 431.1 [M+H]+.

**Example 80: N-[(1R)-1-[3-amino-5-(trifluoromethyl)phenyl]ethyl]-1-(3-methanesulfonamidophenyl)-6-oxo-1,6-dihydropyridine-3-carboxamide**

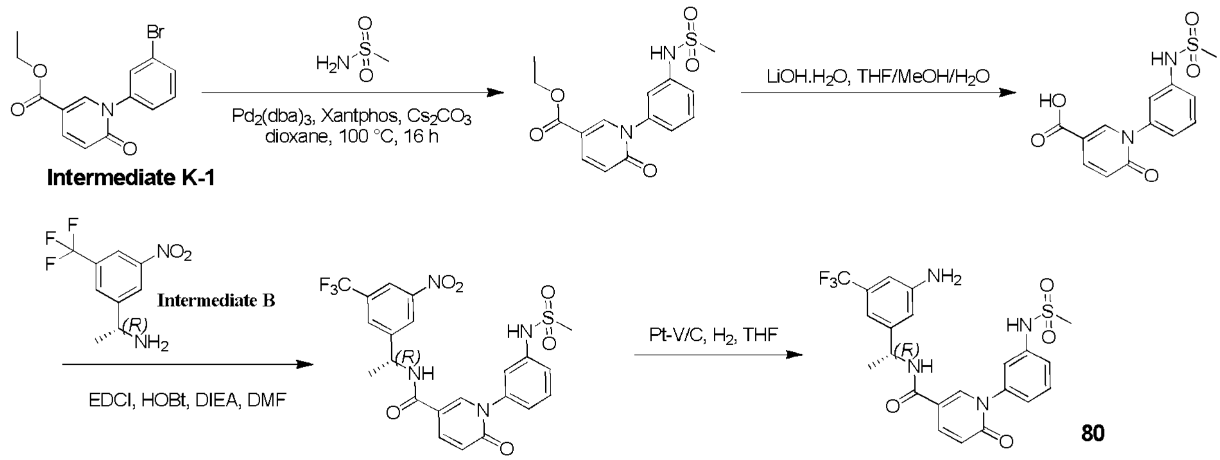


Intermediate K-1

80

The compound of Example 80 (16.94 mg, 35% yield) was obtained as a white solid in the same manner as in Steps 1 to 4 of Example 79, except that methanesulfonamide was used instead of tert-butyl N-methylcarbamate in Step 1.

$^1$H NMR (400 MHz, DMSO-$d_6$) δ = 10.16 - 9.90 (m, 1H), 8.58 (d, *J* = 7.6 Hz, 1H), 8.35 (d, *J* = 2.4 Hz, 1H), 7.97 (dd, *J* = 2.8, 9.6 Hz, 1H), 7.58 - 7.45 (m, 1H), 7.30 (d, *J* = 8.0 Hz, 1H), 7.24 (t, *J* = 2.0 Hz, 1H), 7.19 (d, *J* = 8.8 Hz, 1H), 6.76 (s, 2H), 6.69 (s, 1H), 6.53 (d, *J* = 9.6 Hz, 1H), 5.54 (s, 2H), 5.01 (t, *J* = 7.2 Hz, 1H), 3.07 (s, 3H), 1.40 (d, *J* = 7.2 Hz, 3H); MS (EI) m/z: 495.1 $[M+H]^+$.

**Example 81 to Example 88**

The compounds shown in the following table were prepared in the same manner as in Steps 1 to 4 of Example 79, with replacing tert-butyl N-methylcarbamate with secondary amines in Step 1 of Example 79.

[Table 5]

| No. | Structure/Name | Spectral Data |
|---|---|---|
| 81 | 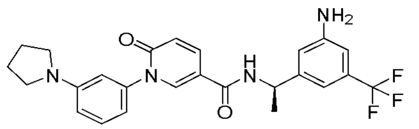 N-[(1R)-1-[3-amino-5-(trifluoromethyl)phenyl] ethyl]-6-oxo-1-[3-(pyrrolidin-1-yl)phenyl]-1,6-dihydropyridine-3-carboxamide | $^1$H NMR (400 MHz, $CDCl_3$) δ = 8.09 (d, J = 2.4 Hz, 1H), 7.68 (dd, J = 2.8, 9.6 Hz, 1H), 7.30 (t, J = 8.0 Hz, 1H), 6.95 (s, 1H), 6.81 (d, J = 3.6 Hz, 2H), 6.71 - 6.54 (m, 3H), 6.46 (d, J = 2.0 Hz, 1H), 6.09 (d, J = 7.2 Hz, 1H), 5.21 (t, J = 7.2 Hz, 1H), 3.29 (t, J = 6.4 Hz, 4H), 2.05 - 1.98 (m, 4H), 1.55 (d, J = 7.2 Hz, 3H); LC/MS (ESI) m/z = 471.2 $[M+H]^+$ |
| 82 | 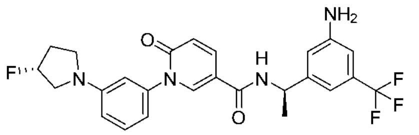 | $^1$H NMR (400 MHz, $CDCl_3$) δ = 8.11 - 8.03 (m, 1H), 7.68 (dd, J = 2.8, 9.6 Hz, 1H), 7.33 (t, J = 8.0 Hz, 1H), 7.00 - 6.91 (m, 2H), 6.81 (s, 1H), 6.69 - 6.58 (m, 3H), 6.48 (s, 1H), 6.24 |
| | N-[(1R)-1-[3-amino-5-(trifluoromethyl)phenyl] ethyl]-1-{3-[(3R)-3-fluoropyrrolidin-1-yl]phenyl}-6-oxo-1,6-dihydropyridine-3-carboxamide | - 6.12 (m, 1H), 5.49 - 5.36 (m, 1H), 5.34 - 5.16 (m, 2H), 3.89 (t, J = 6.8 Hz, 1H), 3.62 - 3.45 (m, 4H), 2.44 - 2.19 (m, 2H), 1.55 (d, J = 6.8 Hz, 3H; LC/MS (ESI) m/z = 489.2 $[M+H]^+$ |
| 83 | 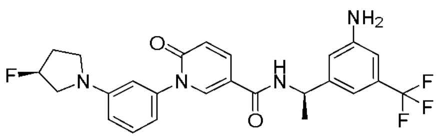 N-[(1R)-1-[3-amino-5-(trifluoromethyl)phenyl] ethyl]-1-13-[(3S)-3-fluoropyrrolidin-1-yl]phenyl}-6-oxo-1,6-dihydropyridine-3-carboxamide | $^1$H NMR (400 MHz, $CDCl_3$) δ = 8.08 (d, J = 2.4 Hz, 1H), 7.68 (dd, J = 2.8, 9.6 Hz, 1H), 7.34 (t, J = 8.0 Hz, 1H), 6.95 (s, 1H), 6.84-6.78 (m, 2H), 6.72 - 6.58 (m, 3H), 6.48 (s, 1H), 6.08 (d, J = 7.2 Hz, 1H), 5.47 - 5.29 (m, 1H), 5.22 - 5.18 (m, 1H), 3.89 (br s, 2H), 3.66 - 3.40 (m, 4H), 2.45 - 2.32 (m, 1H), 2.28 - 2.06 (m, 1H), 1.57 - 1.49 (m, 3H); LC/MS (ESI) m/z = 489.1 $[M+H]^+$ |
| 84 | 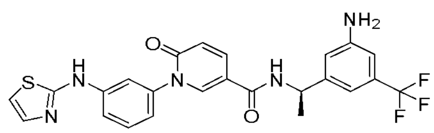 N-[(1R)-1-[3-amino-5-(trifluoromethyl)phenyl] ethyl]-6-oxo-1-{3-[(1,3-thiazol-2-yl)amino]phenyl}-1,6-dihydropyridine-3-carboxamide | $^1$H NMR (400 MHz, DMSO-$d_6$) δ = 10.46 (s, 1H), 8.59 (d, J = 7.6 Hz, 1H), 8.38 (d, J = 2.4 Hz, 1H), 7.97 (dd, J = 2.4, 9.6 Hz, 1H), 7.87 (s, 1H), 7.61 (dd, J = 1.2, 8.0 Hz, 1H), 7.47 (t, J = 8.0 Hz, 1H), 7.27 (d, J = 3.6 Hz, 1H), 7.00 (dd, J = 1.2, 7.6 Hz, 1H), 6.96 (d, J = 3.6 Hz, 1H), 6.75 (s, 2H), 6.69 (s, 1H), 6.54 (d, J = 9.6 Hz, 1H), 5.54 (d, J = 7.2 Hz, 1H), 5.01 (t, J = 7.2 Hz, 1H), 1.39 (d, J = 7.2 Hz, 3H); LC/MS (ESI) m/z = 500.1 $[M+H]^+$ |
| 85 | 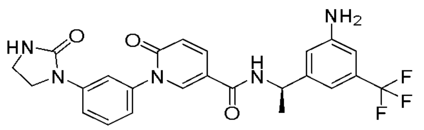 N-[(1R)-1-[3-amino-5-(trifluoromethyl)phenyl] ethyl]-6-oxo-1-[3-(2-oxoimidazolidin-1-yl)phenyl]-1,6-dihydropyridine-3-carboxamide | $^1$H NMR (400 MHz, DMSO-$d_6$) δ = 8.58 (d, J = 7.8 Hz, 1H), 8.35 (d, J = 2.4 Hz, 1H), 7.96 (dd, J = 2.4, 9.6 Hz, 1H), 7.72 (t, J = 2.0 Hz, 1H), 7.62 - 7.55 (m, 1H), 7.47 (t, J = 8.0 Hz, 1H), 7.12 (s, 1H), 7.06 (d, J = 7.6 Hz, 1H), 6.75 (s, 2H), 6.69 (s, 1H), 6.52 (d, J = 9.6 Hz, 1H), 5.53 (d, J = 7.2 Hz, 1H), 5.05 - 4.97 (m, 1H), 3.93 - 3.79 (m, 2H), 3.42 (t, J = 8.0 Hz, 2H), 1.39 (d, J = 7.2 Hz, 3H); LC/MS (ESI) m/z = 486.2 $[M+H]^+$ |

(continued)

| No. | Structure/Name | Spectral Data |
|---|---|---|
| 86 | 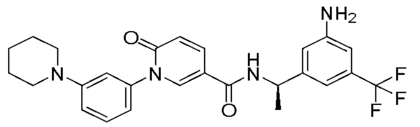 N-[(1R)-1-[3-amino-5-(trifluoromethyl)phenyl] ethyl]-6-oxo-1-[3-(piperidin-1-yl)phenyl]-1,6-dihydropyridine-3-carboxamide | $^1$H NMR (400 MHz, DMSO-$d_6$) δ = 8.56 (d, J = 8.0 Hz, 1H), 8.35 (d, J = 2.4 Hz, 1H), 7.93 (dd, J = 2.4, 9.6 Hz, 1H), 7.33 (t, J = 8.0 Hz, 1H), 7.02 (dd, J = 2.4, 8.4 Hz, 1H), 6.95 (t, J = 2.0 Hz, 1H), 6.75 (d, J = 2.4 Hz, 3H), 6.69 (s, 1H), 6.50 (d, J = 9.6 Hz, 1H), 5.54 (d, J = 7.2 Hz, 1H), 5.00 (J = 7.2 Hz, 1H), 3.26 - 3.14 (m, 4H), 1.66 - 1.51 (m, 6H), 1.39 (d, J = 7.2 Hz, 3H); LC/MS (ESI) m/z = 485.3 [M+H]$^+$ |
| 87 | 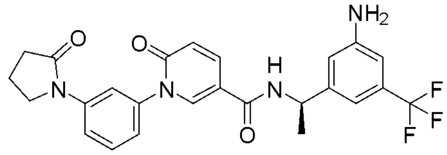 N-[(1R)-1-[3-amino-5-(trifluoromethyl)phenyl] ethyl]-6-oxo-1-[3-(2-oxopyrrolidin-1-yl)phenyl] pyridine-3-carboxamide | $^1$H NMR (400 MHz, DMSO-$d_6$) δ = 8.58 (d, J = 7.6 Hz, 1H), 8.35 (d, J = 2.8 Hz, 1H), 8.06 - 7.90 (m, 1H), 7.90 - 7.70 (m, 1H), 7.78 - 7.70 (m, 1H), 7.60 - 7.50 (m, 1H), 7.26 - 7.20 (m, 1H), 6.76 (s, 2H), 6.70 (s, 1H), 6.58 - 6.50 (m, 1H), 5.85 - 5.24 (m, 1H), 7.60 - 5.46 (m, 1H), 7.94 - 3.80 (m, 2H), 3.47 - 3.43 (m, 2H), 2.16 - 2.00 (m, 2H), 1.39 (d, J = 7.6 Hz, 3H); LC/MS (ESI) m/z = 485.1 [M+H]$^+$ |
| 88 | 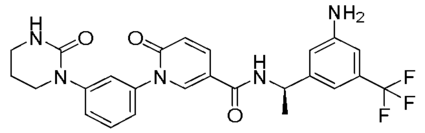 N-[(1R)-1-[3-amino-5-(trifluoromethyl)phenyl] ethyl]-6-oxo-1-[3-(2-oxohexahydropyrimidin-1-yl)phenyl]pyridine-3-carboxamide | $^1$H NMR (400 MHz, DMSO-$d_6$) δ = 8.61 (d, J = 7.6 Hz, 1H), 8.36 (d, J = 2.8 Hz, 1H), 8.00- 7.90 (m, 1H), 7.50 - 7.40 (m, 3H), 7.28 - 7.10 (m, 1H), 6.76 (s, 2H), 6.80 - 6.60 (m, 2H), 6.53 (d, J = 9.6 Hz, 1H), 5.59 - 5.49 (m, 2H), 5.06 - 4.95 (m, 1H), 3.76 - 3.60 (m, 2H), 3.25 - 3.19 (m, 2H), 2.00 - 1.93 (m, 2H), 1.39 (d, J = 7.2 Hz, 3H); LC/MS (ESI) m/z = 500.1 [M+H]$^+$ |

**Example 89: N-[(1R)-1-[3-amino-5-(trifluoromethyl)phenyl]ethyl]-1-[3-(3-methyl-2-oxo-imidazolidin-1-yl)phenyl]-6-oxo-pyridine-3-carboxamide**

Step 1: Synthesis of ethyl-1-[3-(3-methyl-2-oxo-imidazolidin-1-yl)phenyl]-6-oxo-pyridine-3-carboxylate

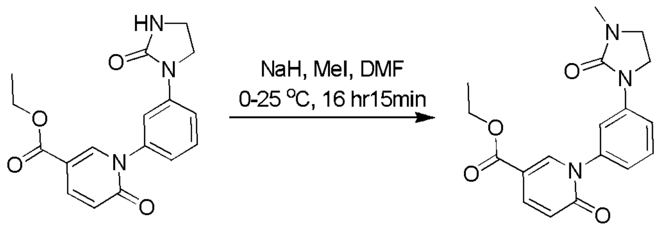


A mixture of ethyl-6-oxo-1-[3-(2-oxoimidazolidin-1-yl)phenyl]pyridine-3-carboxylate (200 mg, 611 μmol, which was synthesized in the same manner as in Step 1 of Example 79, in DMF (4 mL) was added to NaH (36.6 mg, 916 μmol, 60% purity) at 0 °C, and the mixture was stirred at 0 °C for 15 min. Then, the mixture was added with MeI (130 mg, 916 μmol) and stirred at 25 °C for 16 h. The mixture was quenched with ice water (10 mL) and extracted with EA (3 x 10 mL). The combined organic layer was dried over $Na_2SO_4$ and filtered, and the filtrate was concentrated in vacuo. The residue was purified by reversed-phase HPLC (0.1% FA condition) to give ethyl-1-[3-(3-methyl-2-oxo-imidazolidin-1-yl)phenyl]-6-oxo-pyridine-3-carboxylate (150 mg, 71% yield) as a yellow solid. MS (EI) m/z: 342.1 [M+H]$^+$.

Steps 2 to 4: Synthesis of N-[(1R)-1-[3-amino-5-(trifluoromethyl)phenyl]ethyl]-1-[3-(3-methyl-2-oxo-imidazolidin-1-yl)phenyl]-6-oxo-pyridine-3-carboxamide

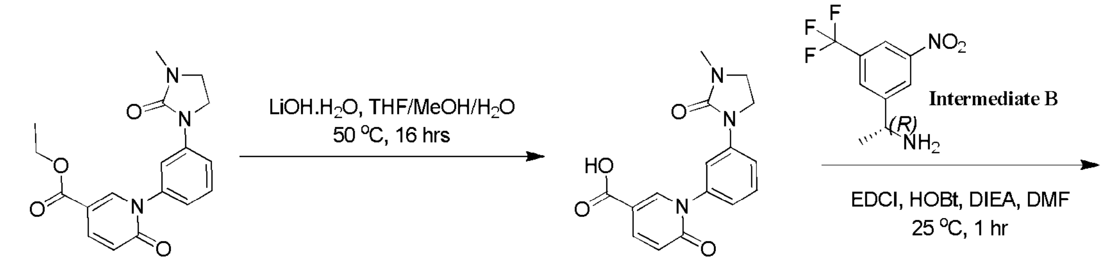


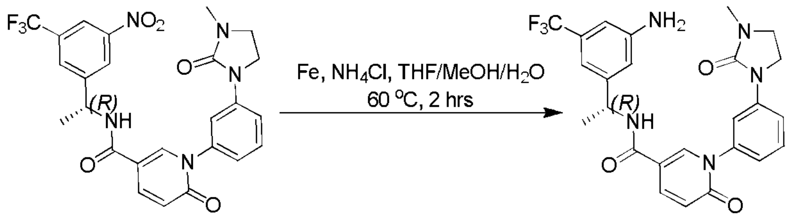


89

The compound of Example 89 (5.97 mg, 41% yield) was obtained in the same manner as in Steps 2 to 4 of Example 79, except that the reagents for the reduction reaction were changed in Step 4. $^1$H NMR (400 MHz, DMSO-$d_6$) $\delta$ = 8.59 (d, $J$ = 7.6 Hz, 1H), 8.36 (d, $J$ = 2.4 Hz, 1H), 7.96 (dd, $J$ = 2.8, 9.6 Hz, 1H), 7.70 (t, $J$ = 2.0 Hz, 1H), 7.63 (dd, $J$ = 1.6, 8.4 Hz, 1H), 7.48 (t, $J$ = 8.0 Hz, 1H), 7.07 (dd, $J$ = 1.2, 7.6 Hz, 1H), 6.76 (s, 2H), 6.69 (s, 1H), 6.53 (d, $J$ = 9.6 Hz, 1H), 5.54 (d, $J$ = 7.2 Hz, 1H), 5.07 - 4.94 (m, 1H), 3.86 - 3.75 (m, 2H), 3.46 (t, $J$ = 8.0 Hz, 2H), 2.77 (s, 3H), 1.39 (d, $J$ = 7.2 Hz, 3H); MS (EI) m/z: 500.2 [M+H]$^+$.

**Example 90 and Example 91: N-[(1R)-1-[3-amino-5-(trifluoromethyl)phenyl]ethyl]-6-oxo-1-{3-[((2S)-1,1,1-trifluoropropan-2-yl)amino]phenyl}-1,6-dihydropyridine-3-carboxamide and N-[(1R)-1-[3-amino-5-(trifluoromethyl)phenyl]ethyl]-6-oxo-1-{3-[((2R)-1,1,1-trifluoropropan-2-yl)amino]phenyl}-1,6-dihydropyridine-3-carboxamide**

Step 1: Synthesis of ethyl-6-oxo-1-[3-[(1,1,1-trifluoropropan-2-yl)amino]phenyl]dihydropyridine-3-carboxylate

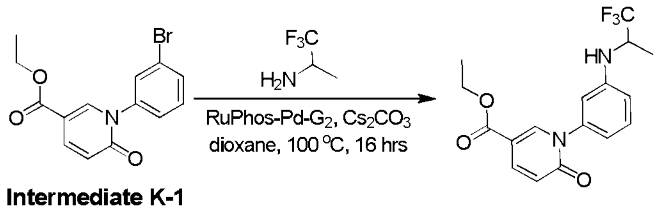


**Intermediate K-1**

A mixture of 1,1,1-trifluoropropan-2-amine (510 mg, 3.41 mmol, HCl), Intermediate EA (1.0 g, 3.10 mmol), RuPhos Pd $G_2$ (241 mg, 310 $\mu$mol) and $Cs_2CO_3$ (5.06 g, 15.5 mmol) in dioxane (5 mL) was stirred at 100 °C for 16 h. The mixture was added with water (20 mL) and extracted with EA (3 X 15 mL). The combined organic layer was dried over $Na_2SO_4$. The residue was purified by reversed-phase HPLC (0.1% FA condition) to give ethyl-6-oxo-1-[3-[(1,1,1-trifluoropropan-2-yl)amino]phenyl]dihydropyridine-3-carboxylate (800 mg, 72% yield) as yellow oil. MS (EI) m/z: 355.2 [M+H]$^+$.

Steps 2 and 3: Synthesis of N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide

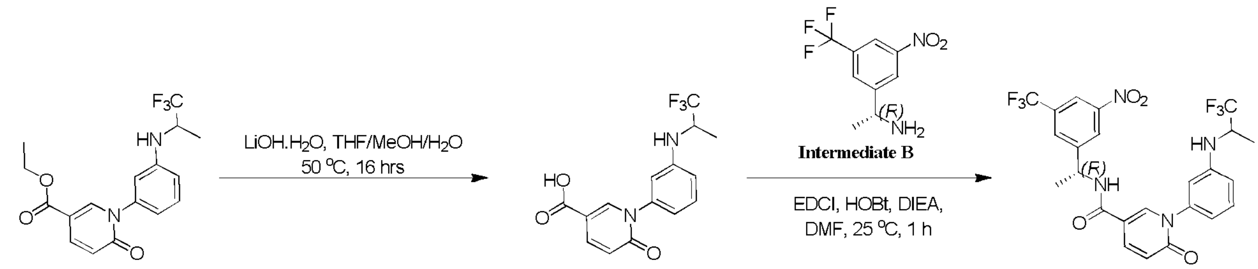


N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide (140 mg, 55% yield) was obtained in the same manner as in Example 76. MS (EI) m/z: 543.4 $[M+H]^+$.

Step 4: Separation of N-[(1R)-1-[3-nitro-5-(trifluoromethyl)phenyl]ethyl]-6-oxo-1-[3-[[(2S)-1,1,1-trifluoropropan-2-yl]amino]phenyl]dihydropyridine-3-carboxamide and N-[(1R)-1-[3-nitro-5-(trifluoromethyl)phenyl]ethyl]-6-oxo-1-[3-[[(2R)-1,1,1-trifluoropropan-2-yl]amino]phenyl]dihydropyridine-3-carboxamide

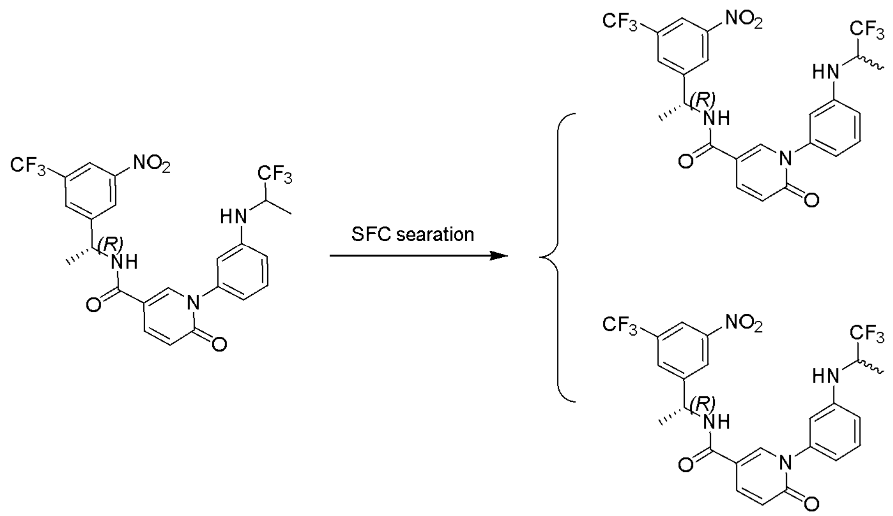


N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide (140 mg) was purified by Prep-HPLC (column: DAICEL CHIRALCEL OJ-H(250mm*30mm, 5μm); mobile phase: [0.1% $NH_3H_2O$ IPA]; B%: 25%-25%, 2.65min) to give one of the diastereomers, N-[(1R)-1-[3-nitro-5-(trifluoromethyl)phenyl]ethyl]-6-oxo-1-[3-[[(2S)-1,1,1-trifluoropropan-2-yl]amino]phenyl]dihydropyridine-3-carboxamide (30 mg) as a yellow solid.

N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide (140 mg) was purified by Prep-HPLC (column: DAICEL CHIRALCEL OJ-H(250mm*30mm, 5μm); mobile phase: [0.1% $NH_3H_2O$ IPA]; B%: 25%-25%, 2.65min) to give the other of the diastereomers, N-[(1R)-1-[3-nitro-5-(trifluoromethyl)phenyl]ethyl]-6-oxo-1-[3-[[(2R)-1,1,1-trifluoropropan-2-yl]amino]phenyl]dihydropyridine-3-carboxamide (30 mg) as a yellow solid.

Step 5: Synthesis of N-[(1R)-1-[3-amino-5-(trifluoromethyl)phenyl]ethyl]-6-oxo-1-{3-[((2S)-1,1,1-trifluoropropan-2-yl)amino]phenyl}-1,6-dihydropyridine-3-carboxamide and N-[(1R)-1-[3-amino-5-(trifluoromethyl)phenyl]ethyl]-6-oxo-1-{3-[((2R)-1,1,1-trifluoropropan-2-yl)amino]phenyl}-1,6-dihydropyridine-3-carboxamide

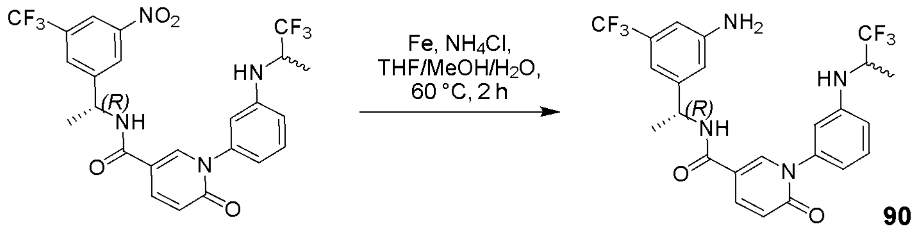


**90**

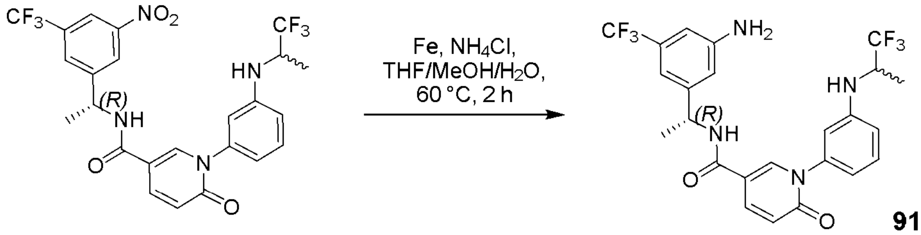


**91**

The compound of Example 90 (5.64 mg, 23% yield) and the compound of Example 91 (5.47 mg, 23% yield) were obtained by reacting each of the two diastereomers in the same manner as in Step 4 of Example 89.

$^1H$ NMR (400 MHz, DMSO-$d_6$) δ = 8.63 - 8.51 (m, 1H), 8.35 (s, 1H), 7.95 (d, *J* = 8.8 Hz, 1H), 7.32 - 7.18 (m, 1H), 6.87 - 6.83 (m, 1H), 6.77 (s, 3H), 6.70 (s, 1H), 6.67 - 6.61 (m, 1H), 6.55 - 6.46 (m, 1H), 6.37 - 6.27 (m, 1H), 5.55 (d, *J* = 7.6 Hz, 1H), 5.08 - 4.94 (m, 1H), 4.50 - 4.35 (m, 1H), 1.39 (d, *J* = 6.8 Hz, 3H), 1.31 (d, *J* = 6.4 Hz, 3H); MS (EI) m/z: 513.2 $[M+H]^+$.

$^1H$ NMR (400 MHz, DMSO-$d_6$) δ = 8.63 - 8.50 (m, 1H), 8.34 (d, *J* = 0.8 Hz, 1H), 7.98 - 7.89 (m, 1H), 7.30 - 7.21 (m, 1H), 6.84 (d, *J* = 7.2 Hz, 1H), 6.79 - 6.73 (m, 3H), 6.69 (s, 1H), 6.64 (d, *J* = 7.2 Hz, 1H), 6.50 (d, *J* = 9.6 Hz, 1H), 6.32 (d, *J* = 8.8 Hz, 1H), 5.54 (d, *J* = 6.4 Hz, 2H), 5.00 (t, *J* = 6.4 Hz, 1H), 4.43 (d, *J* = 7.6 Hz, 1H), 1.39 (d, *J* = 7.2 Hz, 3H), 1.30 (d, *J* = 6.4 Hz, 3H); MS (EI) m/z: 513.2 $[M+H]^+$.

**Example 92: Methyl N-[3-[5-[[(1R)-1-[3-amino-5-(trifluoromethyl)phenyl] ethyl]carbamoyl]-2-oxo-1-pyridyl]phenyl]carbamate**

Step 1: Synthesis of ethyl-1-[3-(tert-butoxycarbonylamino)phenyl]-6-oxo-pyridine-3-carboxylate

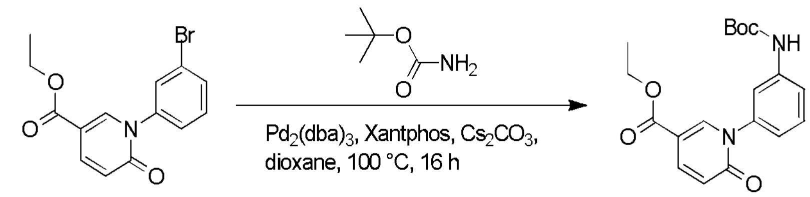


**Intermediate K-1**

Ethyl-1-[3-(tert-butoxycarbonylamino)phenyl]-6-oxo-pyridine-3-carboxylate (200 mg, 35% yield) was obtained as a yellow solid by using tert-butyl N-carbamate in the same manner as in Step 1 of Example 79. MS (EI) m/z: 359.0 $[M+H]^+$.

Step 2: Synthesis of ethyl-1-(3-aminophenyl)-6-oxo-pyridine-3-carboxylate

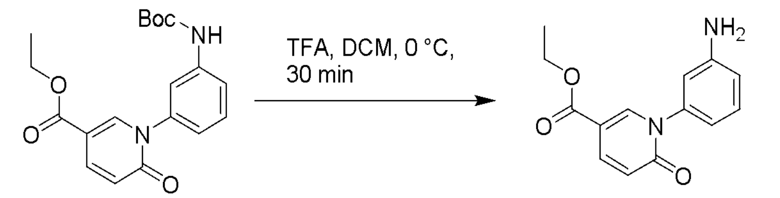

A mixture of ethyl-1-[3-(tert-butoxycarbonylamino)phenyl]-6-oxo-pyridine-3-carboxylate (800 mg, 1.34 mmol) in DCM (20 mL) was added to TFA (600 μL) at 0 °C, and the mixture was stirred at 0 °C for 30 min. The mixture was concentrated in vacuo. The residue was purified by reversed-phase HPLC (0.1% FA condition) to give ethyl-1-(3-aminophenyl)-6-oxo-pyridine-3-carboxylate (320 mg, 92% yield) as yellow oil. $^1$H NMR (400 MHz, DMSO-$d_6$) δ =8.15 (s, 1H), 7.87 - 7.83 (m, 1H), 7.14 (t, *J* = 8.0 Hz, 1H), 6.66 - 6.58 (m, 1H), 6.54 - 6.49 (m, 3H), 5.42 - 5.38 (m, 2H), 4.27 - 4.21 (m, 2H), 1.26 (t, *J* = 7.2 Hz, 3H).

Step 3: Synthesis of ethyl-1-[3-(methoxycarbonylamino)phenyl]-6-oxo-pyridine-3-carboxylate

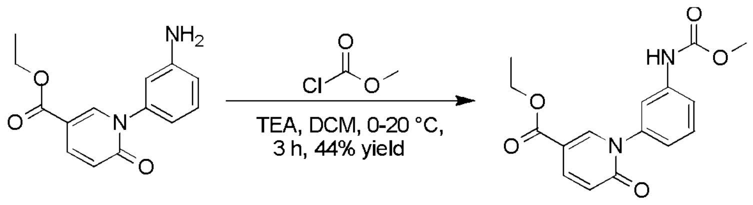


To a solution of ethyl-1-(3-aminophenyl)-6-oxo-pyridine-3-carboxylate (240 mg, 929 μmol) and TEA (470 mg, 4.65 mmol) in DCM (5 mL) was added methyl carbonochloridate (0.53 g, 5.61 mmol) at 0 °C. The reaction mixture was warmed to 20 °C and stirred for 3 h. The reaction mixture was added to water (30 mL) and then adjusted to pH 9 with $Na_2CO_3$, and extracted with DCM (3 x 50 mL). The combined organic layer was concentrated in vacuo. The residue was purified by reversed-phase HPLC (0.1% FA condition) to give ethyl-1-[3-(methoxycarbonylamino)phenyl]-6-oxo-pyridine-3-carboxylate (130 mg, 44% yield) as a white solid. $^1$H NMR (400 MHz, DMSO-$d_6$) δ = 9.92 (s, 1H), 8.22 (d, *J* = 2.4 Hz, 1H), 7.96 - 8.80 (m, 1H), 7.61 - 7.50 (m, 2H), 7.48 - 7.39 (m, 1H), 7.16 - 7.68 (m, 1H), 6.55 (d, *J*= 9.6 Hz, 1H), 4.28 - 4.20 (m, 2H), 3.68 (s, 3H),1.26 (t, *J* = 7.2 Hz, 3H).

Steps 4 to 6: Synthesis of methyl N-[3-[5-[[(1R)-1-[3-amino-5-(trifluoromethyl)phenyl]ethyl]carbamoyl]-2-oxo-1-pyridyl]phenyl]carbamate

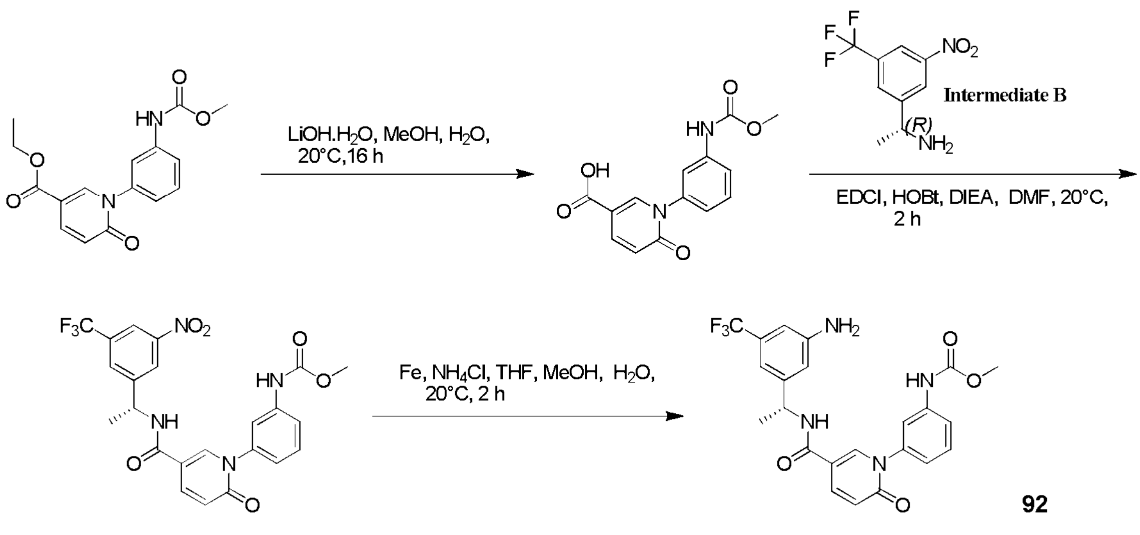


92

The compound of Example 92 (23.0 mg, 39% yield) was obtained in the same manner as in Steps 2 to 4 of Example 89 as a white solid. $^1$H NMR (400 MHz, DMSO-$d_6$) δ = 9.92 (s, 1H), 8.59 (d, *J* = 7.6 Hz, 1H), 8.39 - 8.31 (m, 1H), 8.03 - 7.89 (m, 1H), 7.59 - 7.44 (m, 3H), 7.15 - 7.04 (m, 1H), 6.80 - 6.75 (m, 2H), 6.69 (s, 1H), 5.59 - 5.51 (m, 2H), 5.11 - 4.92 (m, 1H), 3.71 - 3.65 (m, 3H), 1.39 (d, *J* = 7.6 Hz, 3H); MS (EI) m/z: 475.1 [M+H]$^+$.

**Example 93: N-[(1R)-1-(3-amino-5-trifluoromethyl)phenyl]ethyl]-6-oxo-1-[3-(2-oxo-1H-imidazol-3-yl)phenyl]pyridine-3-carboxamide**

Step 1: Synthesis of ethyl-1-[3-(2,2-diethoxyethylcarbonylamino)phenyl]-6-oxo-pyridine-3-carboxyl(2-fluorophenyl)-6-oxo-1,6-dihydropyridazine-3-carboxylate

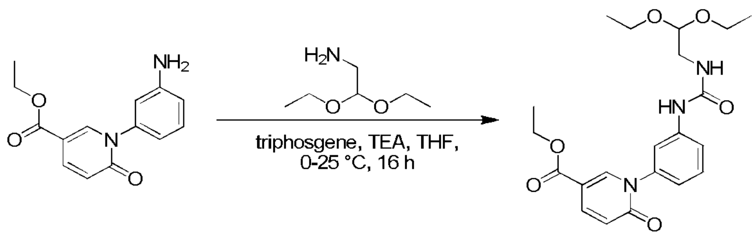


To a mixture of ethyl-1-(3-aminophenyl)-6-oxo-pyridine-3-carboxylate (50 mg, 193 μmol) obtained from Step 2 of Example 92 and TEA (58.7 mg, 580 μmol) in THF (2 mL) was added triphosgene (11.4 mg, 38.7 μmol ) at 0 °C for 30 min, and 2,2-diethoxyethanamine (30.9 mg, 232 μmol) and TEA (58.7 mg, 580 μmol) were subsequently added thereto. The reaction mixture was stirred at 25 °C for 16 h. The reaction mixture was quenched with 1N $K_2CO_3$ aqueous solution (5 mL), adjusted to pH 9 and extracted with EtOAc (10 mL X 2). The combined organic layer was dried over anhydrous $Na_2SO_4$ and concentrated under reduced pressure to give ethyl-1-[3-(2,2-diethoxyethylcarbonylamino)phenyl]-6-oxo-pyridine-3-carboxyl(2-fluorophenyl)-6-oxo-1,6-dihydropyridazine-3-carboxylate (80 mg, crude) as a yellow solid. $^1$H NMR (400 MHz, DMSO-$d_6$) δ = 8.88 (s, 1H), 8.20 (d, *J* = 2.4 Hz, 1H), 7.80 - 7.96 (m, 1H), 7.59 (s, 1H), 7.39 - 7.35 (m, 2H), 6.90 - 7.04 (m, 1H), 6.55 (d, *J* = 9.6 Hz, 1H), 6.20 - 7.24 (m, 1H), 4.40 - 4.58 (m, 1H), 4.26 - 4.22 (m, 2H), 3.65 - 3.59 (m, 2H), 3.40 - 3.58 (m, 2H), 3.10 - 3.26 (m, 2H), 1.29 - 1.24 (m, 3H), 1.15 - 1.11 (m, 6H).

Step 2: Synthesis of ethyl-6-oxo-1-[3-(2-oxo-1H-imidazol-3-yl)phenyl]pyridine-3-carboxylate

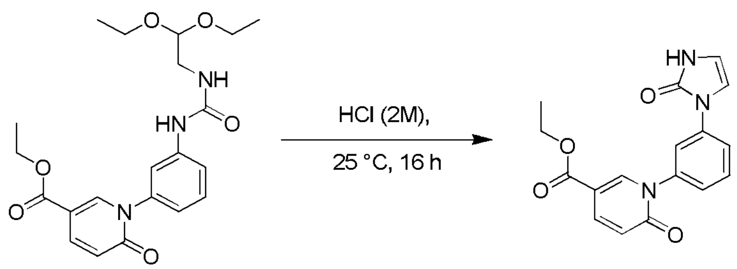


Ethyl-1-[3-(2,2-diethoxyethylcarbonylamino)phenyl]-6-oxo-pyridine-3-carboxyl(2-fluorophenyl)-6-oxo-1,6-dihydropyridazine-3-carboxylate (80 mg, 191 μmol) in 2M HCl (2 mL) was stirred at 25 °C for 16 h. The reaction mixture was diluted with water (8 mL), adjusted to pH 9 with $Na_2CO_3$ aqueous solution and then extracted with EtOAc (10 mL X 3). The combined organic layer was dried over anhydrous $Na_2SO_4$ and concentrated under reduced pressure to give ethyl-6-oxo-1-[3-(2-oxo-1H-imidazol-3-yl)phenyl]pyridine-3-carboxylate (52 mg, 83% yield) as a yellow solid. $^1$H NMR (400 MHz, DMSO-$d_6$) δ = 8.29 (d, J = 2.4 Hz, 1H), 7.93 - 7.88 (m, 3H), 7.63 - 7.52 (m, 2H), 7.29 (s, 1H), 7.07 (d, J = 3.2 Hz, 1H), 6.64 (d, J = 3.2 Hz, 1H), 6.57 (d, J = 9.6 Hz, 1H), 4.25 (d, J = 7.2 Hz, 2H), 1.27 - 1.25 (m, 3H).

Steps 3 and 4: Synthesis of N-[(1R)-1-(3-amino-5-trifluoromethyl)phenyl]ethyl]-6-oxo-1-[3-(2-oxo-1H-imidazol-3-yl)phenyl]pyridine-3-carboxamide

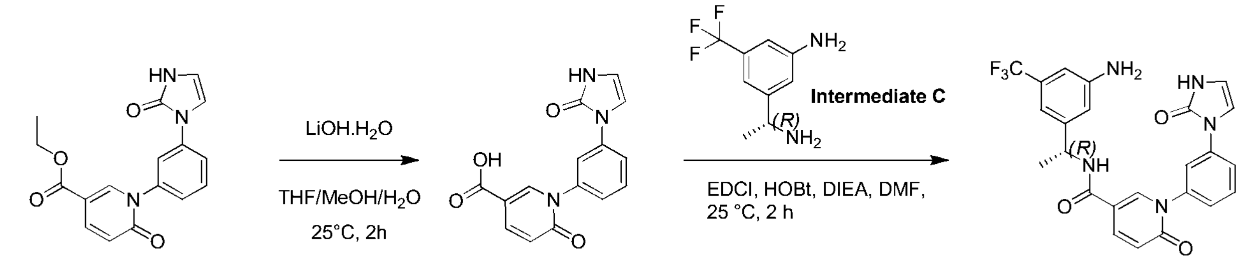


93

The compound of Example 93 (4.99 mg, 28% yield) was obtained in the same manner as in Example 76 as a white solid. 1H NMR (400 MHz, CDCl3) δ = 8.35 (d, J = 2.2 Hz, 1H), 8.00 - 8.10 (m, 1H), 7.86 - 7.73 (m, 2H), 7.60 - 7.68 (m, 1H), 7.30 - 7.46 (m, 1H), 6.99 - 6.86 (m, 3H), 6.80 (s, 1H), 6.65 (d, J = 9.7 Hz, 1H), 6.58 (d, J = 2.8 Hz, 1H), 5.00 - 5.20 (m, 1H), 1.40 - 1.60(m, 3H). MS (EI) m/z: $[M+H]^+$ 484.1.

**Example 94: N-[(1R)-1-[3-amino-5-(trifluoromethyl)phenyl]ethyl]-1-(2,3-difluorophenyl)-6-oxo-1,6-dihydropyridine-3-carboxamide**

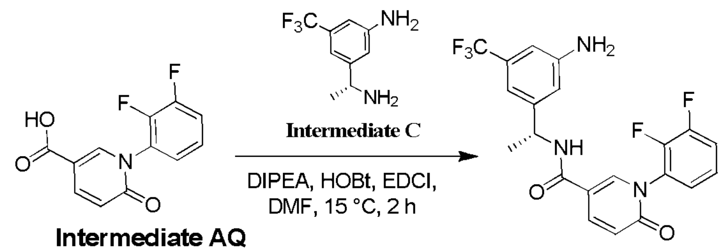


Intermediate AQ

94

A solution of Intermediate C (22 mg, 0.10 mmol), Intermediate AQ (30 mg, 0.12 mmol), HOBt (32.9 mg, 0.21 mmol), EDCI (32.1 mg, 0.16 mmol) and DIPEA (42 μL, 0.24 mmol) in DMF (1 mL) was prepared. The reaction mixture was stirred at room temperature for 2 h. The mixture was added with water and sat. $NaHCO_3$ solution and extracted with EA. The organic layer was dried over $MgSO_4$. The solvent was removed under reduced pressure and the crude residue was purified by Prep/LC to give the compound of Example 94 (24.7 mg, 47.3% yield). $^1H$ NMR (400 MHz, DMSO) δ 8.58 (d, J = 7.7 Hz, 1H), 8.38 (d, J = 2.6 Hz, 1H), 8.01 (dd, J = 9.7, 2.6 Hz, 1H), 7.66 (q, J = 7.9 Hz, 1H), 7.53 - 7.38 (m, 2H), 6.77 (s, 2H), 6.71 (s, 1H), 6.59 (d, J = 9.7 Hz, 1H), 5.01 (t, J = 7.2 Hz, 1H), 1.40 (d, J = 7.1 Hz, 3H); LC/MS (ESI) m/z = 438.1 $[M+H]^+$.

**Example 95 to Example 99**

The compounds shown in the following table were prepared in the same manner as in Example 94 by using starting materials corresponding to the respective structures of the desired compounds based on Preparation Example 43 or by replacing Intermediate C with Intermediate E.

[Table 6]

| No. | Structure/Name | Spectral Data |
|---|---|---|
| 95 | 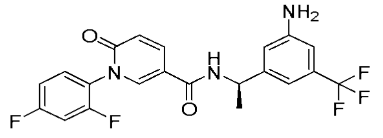 N-[(1R)-1-[3-amino-5-(trifluoromethyl)phenyl]ethyl]-1-(2,4-difluorophenyl)-6-oxo-1,6-dihydropyridine-3-carboxamide | $^1H$ NMR (400 MHz, DMSO) δ 8.61 (d, J = 7.7 Hz, 1H), 8.36 (d, J = 2.6 Hz, 1H), 8.00 (dd, J = 9.7, 2.6 Hz, 1H), 7.71 (td, J = 8.8, 5.9 Hz, 1H), 7.57 (ddd, J = 11.5, 9.1, 2.8 Hz, 1H), 7.37 - 7.27 (m, 1H), 6.76 (d, J = 1.9 Hz, 2H), 6.70 (d, J = 2.0 Hz, 1H), 6.57 (d, J = 9.7 Hz, 1H), 5.57 (s, 2H), 5.00 (p, J = 7.1 Hz, 1H), 1.40 (d, J = 7.1 Hz, 3H); LC/MS (ESI) m/z = 438.1 $[M+H]^+$ |

(continued)

| No. | Structure/Name | Spectral Data |
|---|---|---|
| 96 | 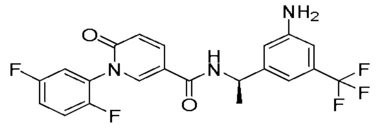 N-[(1R)-1-[3-amino-5-(trifluoromethyl)phenyl]ethyl]-1-(2,5-difluorophenyl)-6-oxo-1,6-dihydropyridine-3-carboxamide | $^1$H NMR (400 MHz, DMSO) δ 8.56 (d, J = 7.7 Hz, 1H), 8.37 (d, J = 2.6 Hz, 1H), 7.99 (dd, J = 9.7, 2.6 Hz, 1H), 7.68 (ddd, J = 8.7, 5.8, 3.1 Hz, 1H), 7.60 - 7.43 (m, 2H), 6.76 (d, J = 1.9 Hz, 2H), 6.70 (s, 1H), 6.58 (d, J = 9.7 Hz, 1H), 5.56 (s, 2H), 5.01 (t, J = 7.2 Hz, 1H), 1.40 (d, J = 7.0 Hz, 3H); LC/MS (ESI) m/z = 438.1 $[M+H]^+$ |
| 97 | 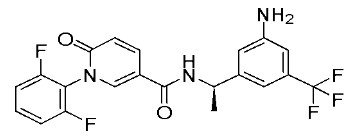 N-[(1R)-1-[3-amino-5-(trifluoromethyl)phenyl]ethyl]-1-(2,6-difluorophenyl)-6-oxo-1,6-dihydropyridine-3-carboxamide | $^1$H NMR (400 MHz, DMSO) δ 8.58 (d, J = 7.6 Hz, 1H), 8.42 (d, J = 2.6 Hz, 1H), 8.04 (dd, J = 9.7, 2.6 Hz, 1H), 7.73 - 7.61 (m, 1H), 7.41 (t, J = 8.6 Hz, 2H), 6.76 (d, J = 1.9 Hz, 2H), 6.70 (d, J = 1.9 Hz, 1H), 6.64 (d, J = 9.7 Hz, 1H), 5.57 (s, 2H), 5.00 (t, J = 7.2 Hz, 1H), 1.40 (d, J = 7.0 Hz, 3H); LC/MS (ESI) m/z =438.1 $[M+H]^+$ |
| 98 | 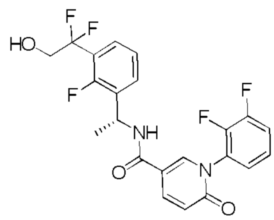 N-[(1R)-1-[3-(1,1-difluoro-2-hydroxyethyl)-2-fluorophenyl] ethyl]-1-(2,3-difluorophenyl)-6-oxo-1,6-dihydropyridine-3-carboxamide | $^1$H NMR (400 MHz, DMSO) δ 8.70 (d, J = 7.3 Hz, 1H), 8.43 (d, J = 2.6 Hz, 1H), 8.00 (dd, J = 9.6, 2.6 Hz, 1H), 7.70 - 7.62 (m, 1H), 7.58 (t, J = 7.3 Hz, 1H), 7.50 (t, J = 7.0 Hz, 1H), 7.44 (q, J = 7.5 Hz, 2H), 7.28 (t, J = 7.7 Hz, 1H), 6.59 (d, J = 9.7 Hz, 1H), 5.73 (t, J = 6.4 Hz, 1H), 5.35 (t, J = 7.1 Hz, 1H), 3.92 (td, J = 14.4, 6.4 Hz, 2H), 1.44 (d, J = 7.1 Hz, 3H); LC/MS (ESI) m/z = 453.2 $[M+H]^+$ |
| 99 | 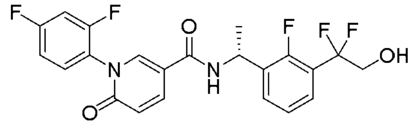 N-[(1R)-1-[3-(1,1-difluoro-2-hydroxyethyl)-2-fluorophenyl] ethyl]-1-(2,4-difluorophenyl)-6-oxo-1,6-dihydropyridine-3-carboxamide | $^1$H NMR (400 MHz, DMSO) δ 8.68 (d, J = 7.4 Hz, 1H), 8.40 (d, J = 2.6 Hz, 1H), 7.98 (dd, J = 9.7, 2.6 Hz, 1H), 7.78 - 7.68 (m, 1H), 7.58 (s, 2H), 7.43 (t, J = 7.4 Hz, 1H), 7.38 - 7.24 (m, 2H), 6.57 (d, J = 9.8 Hz, 1H), 5.73 (t, J = 6.4 Hz, 1H), 5.35 (t, J = 7.1 Hz, 1H), 3.92 (td, J = 14.5, 6.5 Hz, 2H), 1.43 (d, J = 7.0 Hz, 3H); LC/MS (ESI) m/z = 453.1 $[M+H]^+$ |

**Example 100: 1-(2-fluorophenyl)-N-[(1R)-1-{2'-[(methylamino)methyl]-[1,1'-biphenyl]-3-yl}ethyl]-6-oxo-1,6-dihydropyridine-3-carboxamide**

Step 1: Synthesis of (R)-N-(1-(3-bromophenyl)ethyl)-1-(2-fluorophenyl)-6-oxo-1,6-dihydropyridine-3-carboxamide

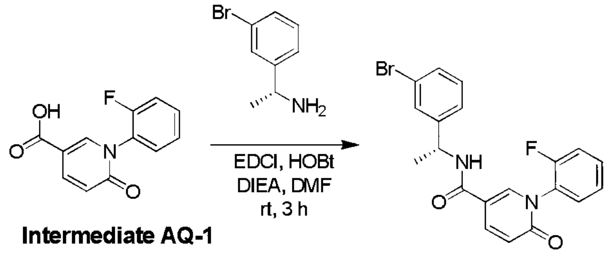


Intermediate AQ-1

(R)-N-(1-(3-bromophenyl)ethyl)-1-(2-fluorophenyl)-6-oxo-1,6-dihydropyridine-3-carboxamide (1.01g, 55.3%

yield) was obtained in the same manner as in Step 2 of Example 76. LC/MS m/z = 415.1 [M+H]+.

Step 2: Synthesis of 1-(2-fluorophenyl)-N-[(1R)-1-{2'-[(methylamino)methyl]-[1,1'-biphenyl]-3-yl}ethyl]-6-oxo-1,6-dihydropyridine-3-carboxamide

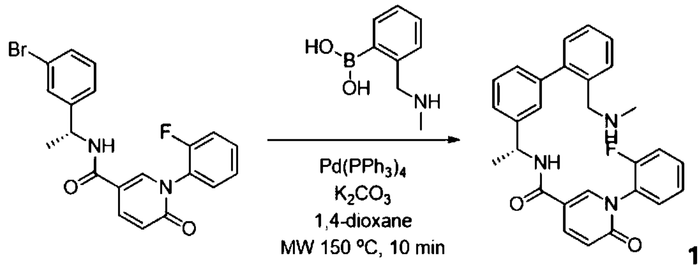


100

To a solution of (R)-N-(1-(3-bromophenyl)ethyl)-1-(2-fluorophenyl)-6-oxo-1,6-dihydropyridine-3-carboxamide (50 mg, 0.12 mmol) in 1,4-dioxane (2.5 mL) and distilled water (0.5 mL), (2-((methylamino)methyl)phenyl)boronic acid (23.8 mg, 0.14 mmol), $Pd(PPh_3)_4$ (13.9 mg, 0.012 mmol) and $K_2CO_3$ (49.6 mg, 0.36 mmol) were added. The reaction mixture was heated by microwave at 150 °C for 10 min. The mixture was cooled, added with water, and extracted with DCM. The organic layer was washed with brine, dried over $Na_2SO_4$ and concentrated under reduced pressure. The residue was purified by prep-HPLC to give the compound of Example 100 (27.8 mg, 50.7% yield). $^1$H NMR (500 MHz, MeOD) δ 8.27 (d, J = 2.6 Hz, 1H), 8.07 (dd, J = 9.6, 2.6 Hz, 1H), 7.57 (dd, J = 6.1, 2.9 Hz, 2H), 7.50 (dd, J = 5.9, 3.6 Hz, 4H), 7.47 (d, J = 6.6 Hz, 2H), 7.40 - 7.32 (m, 5H), 7.23 (dt, J = 6.8, 2.0 Hz, 1H), 6.65 (d, J = 9.6 Hz, 1H), 5.16 (q, J = 7.1 Hz, 1H), 4.18 (s, 2H), 3.98 (s, 1H), 2.53 (s, 3H), 1.57 (d, J = 7.1 Hz, 3H); LC/MS m/z = 456.2 [M+H]+.

**Example 101 to Example 107**

The compounds shown in the following table were prepared in the same method as in Example 100 by using appropriate boronic acid derivatives in Step 2.

[Table 7]

| No. | Structure/Name | Spectral Data |
|---|---|---|
| 101 | 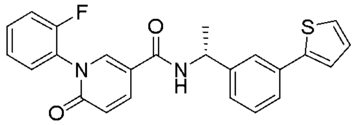 1-(2-fluorophenyl)-6-oxo-N-[(1R)-1-[3-(thiophen-2-yl)phenyl] ethyl]-1,6-dihydropyridine-3-carboxamide | $^1$H NMR (500 MHz, MeOD) δ 8.24 (d, J = 2.6 Hz, 1H), 8.06 (dd, J = 9.7, 2.6 Hz, 1H), 7.62 (d, J = 1.8 Hz, 1H), 7.56 (tdd, J = 7.4, 5.0, 1.7 Hz, 1H), 7.52 - 7.45 (m, 2H), 7.38 - 7.35 (m, 3H), 7.34 (d, J = 2.1 Hz, 2H), 7.33 (d, J = 2.3 Hz, 1H), 7.31 - 7.27 (m, 1H), 7.07 (dd, J = 5.1, 3.6 Hz, 1H), 6.64 (d, J = 9.7 Hz, 1H), 5.19 (td, J = 7.7, 5.7 Hz, 1H), 1.55 (d, J = 7.2 Hz, 3H); LC/MS (ESI) m/z = 419.1 [M+H]+ |
| 102 | 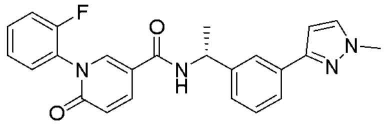 1-(2-fluorophenyl)-N-[(1R)-1-[3-(1-methyl-1H-pyrazol-3-yl)phenyl] ethyl]-6-oxo-1,6-dihydropyridine-3-carboxamide | $^1$H NMR (500 MHz, MeOD) δ 8.25 (d, J = 2.6 Hz, 1H), 8.06 (dd, J = 9.6, 2.6 Hz, 1H), 7.58 - 7.54 (m, 1H), 7.51 (dd, J = 5.0, 2.0 Hz, 1H), 7.50 - 7.46 (m, 4H), 7.37 (t, J = 7.7 Hz, 5H), 6.65 (d, J = 9.6 Hz, 1H), 6.38 (d, J = 2.0 Hz, 1H), 5.22 (d, J = 7.1 Hz, 1H), 3.85 (s, 3H), 1.57 (d, J = 7.1 Hz, 3H); LC/MS (ESI) m/z = 417.2 [M+H]+ |

(continued)

| No. | Structure/Name | Spectral Data |
|---|---|---|
| 103 | 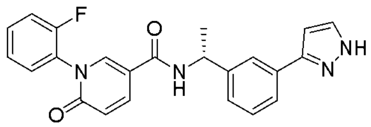 1-(2-fluorophenyl)-6-oxo-N-[(1R)-1-[3-(1H-pyrazol-3-yl)phenyl] ethyl]-1,6-dihydropyridine-3-carboxamide | $^1$H NMR (500 MHz, MeOD) δ 8.26 (d, J = 2.7 Hz, 1H), 8.11 - 8.03 (m, 1H), 7.78 (s, 1H), 7.74 (d, J = 1.9 Hz, 1H), 7.64 (dd, J = 7.4, 1.6 Hz, 1H), 7.60 - 7.53 (m, 1H), 7.50 (t, J = 7.5 Hz, 1H), 7.43 - 7.32 (m, 4H), 6.72 (d, J = 1.9 Hz, 1H), 6.65 (d, J = 9.7 Hz, 1H), 5.22 (q, J = 7.0 Hz, 1H), 1.57 (d, J = 7.1 Hz, 3H); LC/MS (ESI) m/z = 403.2 $[M+H]^+$ |
| 104 | 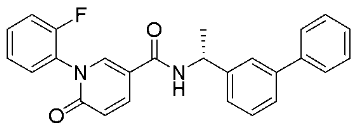 N-[(1R)-1-{[1,1'-biphenyl]-3-yl}ethyl]-1-(2-fluorophenyl)-6-oxo-1,6-dihydropyridine-3-carboxamide | $^1$H NMR (500 MHz, MeOD) δ 8.25 (d, J = 2.6 Hz, 1H), 8.08 (dd, J = 9.6, 2.6 Hz, 1H), 7.67 - 7.63 (m, 1H), 7.60 (ddt, J = 9.5, 6.1, 1.4 Hz, 4H), 7.55 (ddd, J = 11.0, 5.6, 3.1 Hz, 2H), 7.49 (dt, J = 7.6, 1.6 Hz, 2H), 7.41 (q, J = 7.6 Hz, 4H), 7.35 (ddd, J = 16.3, 6.7, 3.8 Hz, 4H), 6.65 (d, J = 9.6 Hz, 1H), 5.29 - 5.20 (m, 1H), 1.58 (d, J = 7.1 Hz, 3H); LC/MS (ESI) m/z = 413.2 $[M+H]^+$ |
| 105 | 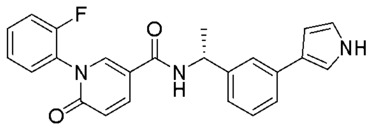 1-(2-fluorophenyl)-6-oxo-N-[(1R)-1-[3-(1H-pyrrol-3-yl)phenyl] ethyl]-1,6-dihydropyridine-3-carboxamide | $^1$H NMR (500 MHz, MeOD) δ 8.22 (d, J = 2.6 Hz, 1H), 8.05 (dd, J = 9.7, 2.6 Hz, 1H), 7.58 - 7.49 (m, 2H), 7.44 (td, J = 7.8, 1.8 Hz, 1H), 7.38 (dt, J = 7.8, 1.4 Hz, 1H), 7.37 - 7.29 (m, 2H), 7.23 (t, J = 7.7 Hz, 1H), 7.10 (dt, J = 7.6, 1.5 Hz, 1H), 7.08 (t, J = 1.8 Hz, 1H), 6.74 (dd, J = 2.8, 1.9 Hz, 1H), 6.63 (d, J = 9.6 Hz, 1H), 6.42 (dd, J = 2.8, 1.6 Hz, 1H), 5.16 (q, J = 7.0 Hz, 1H), 1.53 (d, J = 7.0 Hz, 3H); LC/MS (ESI) m/z = 402.2 $[M+H]^+$ |
| 106 | 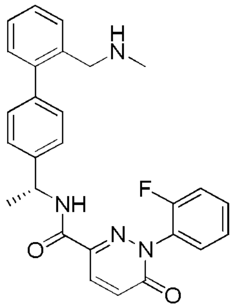 1-(2-fluorophenyl)-N-[(1R)-1-[4-[2-(methylaminomethyl)phenyl]phenyl]ethyl]-6-oxo-pyridazine-3-carboxamide | $^1$H NMR (400 MHz, DMSO-$d_6$) δ = 8.93 (d, *J* = 8.4 Hz, 1H), 7.97 (d, *J* = 10.0 Hz, 1H), 7.69 (dt, *J* = 1.6, 7.6 Hz, 1H), 7.62 - 7.55 (m, 1H), 7.51 (d, *J* = 7.2 Hz, 1H), 7.47 - 7.39 (m, 4H), 7.38 - 7.31 (m, 3H), 7.30 - 7.24 (m, 1H), 7.21 - 7.15 (m, 2H), 5.28 - 5.13 (m, 1H), 3.52 (s, 2H), 2.19 (s, 3H), 1.51 (d, *J* = 7.2 Hz, 3H); LC/MS (ESI) m/z: 457.4 $[M+H]^+$ |

(continued)

| No. | Structure/Name | Spectral Data |
|---|---|---|
| 107 | 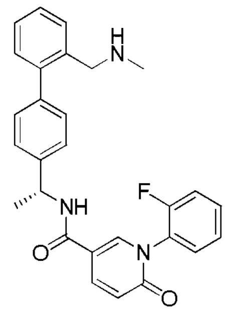 1-(2-fluorophenyl)-N-[(1R)-1-[4-[2-(methylaminomethyl)phenyl]phenyl]ethyl]-6-oxo-pyridine-3-carboxamide | $^{1}H$ NMR (400 MHz, DMSO-$d_6$) δ = 8.62 (br d, *J* = 8.0 Hz, 1H), 8.41 (d, *J* = 2.4 Hz, 1H), 8.02 (dd, *J* = 2.4, 9.6 Hz, 1H), 7.67 - 7.44 (m, 4H), 7.44 - 7.27 (m, 7H), 7.19 (br d, *J* = 7.6 Hz, 1H), 6.57 (d, *J* = 10.0 Hz, 1H), 5.18 (quin, *J* = 7.2 Hz, 1H), 3.55 (s, 2H), 2.21 (s, 3H), 2.05 - 1.94 (m, 1H), 1.48 (br d, *J* = 7.2 Hz, 3H); LC/MS (ESI) m/z: 456.3 $[M+H]^+$ |

**Example 108: 1-(2-fluorophenyl)-N-[(1R)-1-[2'-(methylamino)-[1,1'-biphenyl]-3-yl]ethyl]-6-oxo-1,6-dihydropyridine-3-carboxamide**

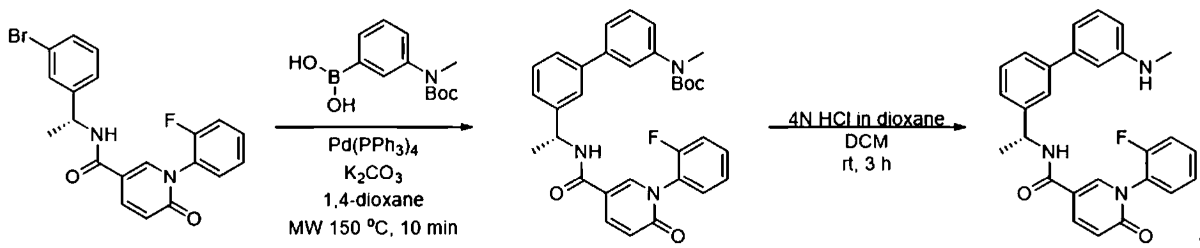


**108**

To a solution of (R)-N-(1-(3-bromophenyl)ethyl)-1-(2-fluorophenyl)-6-oxo-1,6-dihydropyridine-3-carboxamide obtained from Step 1 of Example 100 (50 mg, 0.12 mmol) in 1,4-dioxane (2.5 mL) and distilled water (0.5 mL), (3-((tert-butoxycarbonyl)(methyl)amino)phenyl)boronic acid (35.2 mg, 0.14 mmol), $Pd(PPh_3)_4$ (13.9 mg, 0.012 mmol) and $K_2CO_3$ (49.7 mg, 0.36 mmol) were added. The reaction mixture was heated by microwave at 150 °C for 10 min. The mixture was cooled, added with water, and extracted with DCM. The organic layer was washed with brine, dried over $Na_2SO_4$ and concentrated under reduced pressure. The residue was dissolved in DCM, and 4N HCl (85 μL) in dioxane was added thereto. The reaction mixture was stirred at room temperature for 3 h. The mixture was concentrated and basified with sat. $NaHCO_3$ solution. The aqueous layer was extracted with EA. The combined organic layer was dried over $Na_2SO_4$, filtered and concentrated. The mixture was purified by prep-HPLC to give the compound of Example 108 (9.6 mg, 18.1% yield). $^{1}H$ NMR (500 MHz, MeOD) δ 8.25 (d, J = 2.6 Hz, 1H), 8.08 (dd, J = 9.6, 2.6 Hz, 1H), 7.56 (d, J = 6.4 Hz, 2H), 7.52 - 7.47 (m, 1H), 7.45 (d, J = 7.5 Hz, 1H), 7.37 (dd, J = 8.5, 6.5 Hz, 3H), 7.32 (d, J = 7.9 Hz, 1H), 7.18 (t, J = 7.8 Hz, 1H), 6.86 (d, J = 7.6 Hz, 1H), 6.82 (t, J = 2.1 Hz, 1H), 6.65 (d, J = 9.6 Hz, 1H), 6.61 (dd, J = 7.9, 2.4 Hz, 1H), 5.23 (q, J = 7.1 Hz, 1H), 2.80 (s, 3H), 1.57 (d, J = 7.1 Hz, 3H); LC/MS m/z = 442.2 $[M+H]^+$.

**Example 109: 1-(2-fluorophenyl)-N-[(1R)-1-(3-{4-[(methylamino)methyl]thiophen-2-yl}phenyl)ethyl]-6-oxo-1,6-dihydropyridine-3-carboxamide**

<u>Step 1: Synthesis of (R)-1-(2-fluorophenyl)-6-oxo-N-(1-(3-(4,4,5,5-tetramethyl-1,3,2-dioxaborolan-2-yl)phenyl)ethyl)-1,6-dihydropyridine-3-carboxamide</u>

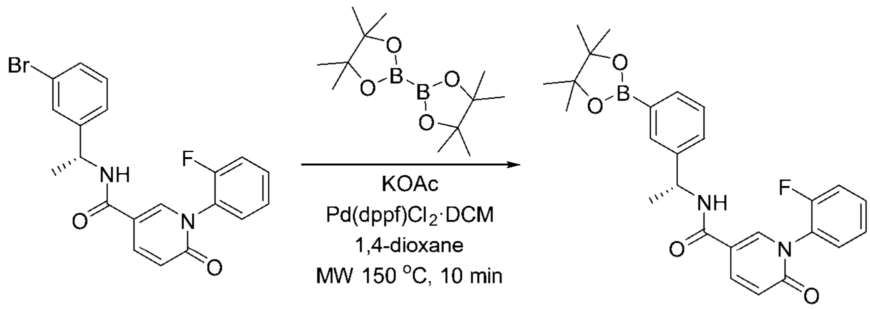


To a solution of (R)-N-(1-(3-bromophenyl)ethyl)-1-(2-fluorophenyl)-6-oxo-1,6-dihydropyridine-3-carboxamide obtained from Step 1 of Example 100 (100 mg, 0.24 mmol) in 1,4-dioxane (3 mL), bis(pinacolato)diborane (70.9 mg, 0.72 mmol), Pd(dppf)$Cl_2$·DCM (19.7 mg, 0.024 mmol) and KOAc (70.6 mg, 0.72 mmol) were added. The reaction mixture was heated by microwave at 150 °C for 10 min. The mixture was cooled and extracted with EtOAc. The organic layer was dried over anhydrous $Na_2SO_4$ and concentrated in vacuo to obtain a crude product. The product was purified by flash chromatography (0-5% MeOH in DCM) to give (R)-1-(2-fluorophenyl)-6-oxo-N-(1-(3-(4,4,5,5-tetramethyl-1,3,2-dioxaborolan-2-yl)phenyl)ethyl)-1,6-dihydropyridine-3-carboxamide (113.4 mg, 102% yield) as brown liquid. LC/MS m/z = 463.3 $[M+H]^+$.

Step 2: Synthesis of 1-(2-fluorophenyl)-N-[(1R)-1-(3-{4-[(methylamino)methyl]thiophen-2-yl}phenyl)ethyl]-6-oxo-1,6-dihydropyridine-3-carboxamide

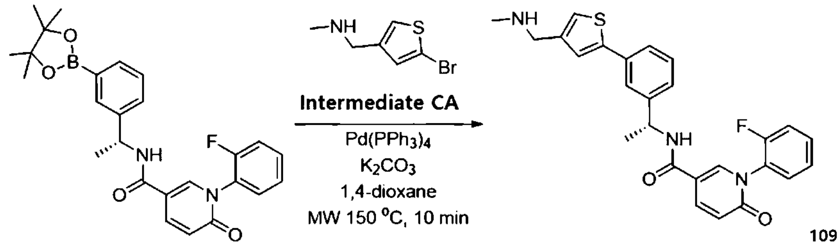

109

The compound of Example 109 (2.3 mg, 3.0% yield) was obtained in the same manner as in Step 2 of Example 100. $^1$H NMR (500 MHz, MeOD) δ 8.26 (d, J = 2.6 Hz, 1H), 8.08 (dd, J = 9.7, 2.6 Hz, 1H), 7.62 (d, J = 1.9 Hz, 1H), 7.61 - 7.54 (m, 1H), 7.54 - 7.47 (m, 2H), 7.46 - 7.42 (m, 2H), 7.41 - 7.31 (m, 4H), 6.66 (d, J = 9.7 Hz, 1H), 5.20 (q, J = 7.1 Hz, 1H), 4.05 (s, 2H), 2.63 (s, 3H), 1.56 (d, J = 7.1 Hz, 3H); LC/MS m/z = 462.2 $[M+H]^+$.

**Example 110: 1-(2-fluorophenyl)-N-[(1R)-(1-(3-(3-[(methylamino)methyl]thiopen-2-yl)phenyl)ethyl)]-6-oxo-1,6-dihydropyridine-3-carboxamide**

Step 1: Synthesis of (R)-1-(2-fluorophenyl)-N-(1-(3-(3-formylthiophen-2-yl)phenyl)ethyl)-6-oxo-1,6-dihydropyridine-3-carboxamide

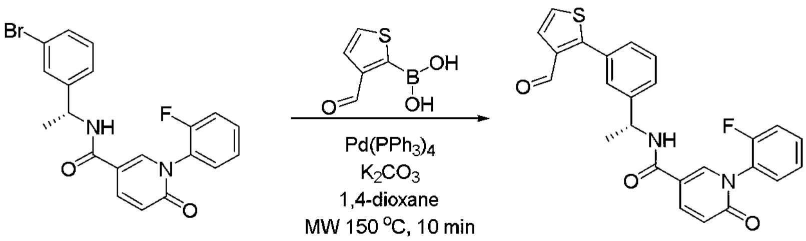


(R)-1-(2-fluorophenyl)-N-(1-(3-(3-formylthiophen-2-yl)phenyl)ethyl)-6-oxo-1,6-dihydropyridine-3-carboxamide (295.9 mg, 92% yield) was obtained by using (3-formylthiophen-2-yl)boronic acid in the same manner as in Step 2 of

Example 100. LC/MS (m/z) = 447.1 [M+H]+.

Step 2: Synthesis of 1-(2-fluorophenyl)-N-[(1R)-(1-(3-(3-[(methylamino)methyl]thiophen-2-yl)phenyl)ethyl)]-6-oxo-1,6-dihydropyridine-a3-carboxamide

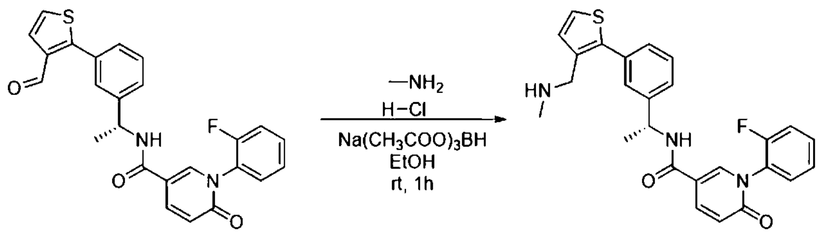


110

To a solution of (R)-1-(2-fluorophenyl)-N-(1-(3-(3-formylthiophen-2-yl)phenyl)ethyl)-6-oxo-1,6-dihydropyridine-3-carboxamide (176.2 mg, 0.39 mmol) in EtOH (4 mL) was added methylamine hydrochloride (40.0 mg, 0.59 mmol). The reaction mixture was stirred at room temperature for 15 min and cooled to 0 °C. To the mixture was added sodium triacetoxyborohydride (167 mg, 0.79 mmol). The mixture was stirred at room temperature for 1 hour. The mixture was quenched with water and concentrated under reduced pressure. The residue was extracted with DCM. The organic layer was dried over $Na_2SO_4$ and concentrated under reduced pressure. The residue was purified by prep-HPLC and prep-TLC to give the compound of Example 110 (26 mg, 14.27% yield). $^1$H NMR (500 MHz, MeOD) δ 8.26 (d, J = 2.6 Hz, 1H), 8.08 (dt, J = 9.7, 1.8 Hz, 1H), 7.56 (t, J = 6.6 Hz, 1H), 7.49 (t, J = 7.5 Hz, 1H), 7.44 (s, 1H), 7.43 - 7.38 (m, 4H), 7.38 (s, 1H), 7.36 (s, 1H), 7.35 - 7.30 (m, 2H), 7.18 (d, J = 5.3 Hz, 1H), 6.65 (d, J = 9.6 Hz, 1H), 5.21 (q, J = 7.1 Hz, 1H), 3.81 (s, 2H), 2.34 (s, 3H), 1.57 (d, J = 7.1 Hz, 3H); LC/MS m/z = 462.2 [M+H]+.

**Example 111: 1-(2-fluorophenyl)-N-[(1R)-1-(3-(2-[(methylamino)methyl]thiophen-3-yl)phenyl)ethyl]-6-oxo-1,6-dihydropyridine-3-carboxamide**

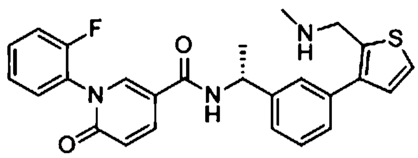

111

The compound of Example 111 (33 mg, 13.0% yield) was obtained by using (2-formylthiophen-3-yl)boronic acid in Step 1 in the same manner as in Example 110. $^1$H NMR (500 MHz, MeOD) δ 8.25 (d, J = 2.6 Hz, 1H), 8.07 (dd, J = 9.7, 2.6 Hz, 1H), 7.56 (ddd, J = 7.7, 5.1, 1.8 Hz, 1H), 7.52 - 7.44 (m, 1H), 7.43 - 7.30 (m, 6H), 7.26 (d, J = 7.7 Hz, 1H), 7.05 (d, J = 5.1 Hz, 1H), 6.65 (d, J = 9.7 Hz, 1H), 5.20 (q, J = 7.0 Hz, 1H), 3.93 (s, 2H), 2.32 (s, 2H), 1.56 (d, J = 7.0 Hz, 3H); LC/MS m/z = 462.2 [M+H]+.

**Example 112: (R)-1-(2-fluorophenyl)-N-(1-(1-(2-((methylamino)methyl)phenyl)-1H-pyrazol-3-yl)ethyl)-6-oxo-1,6-dihydropyridine-3-carboxamide**

Step 1: Synthesis of (R)-N-(1-(1H-pyrazol-3-yl)ethyl)-1-(2-fluorophenyl)-6-oxo-1,6-dihydropyridine-3-carboxamide

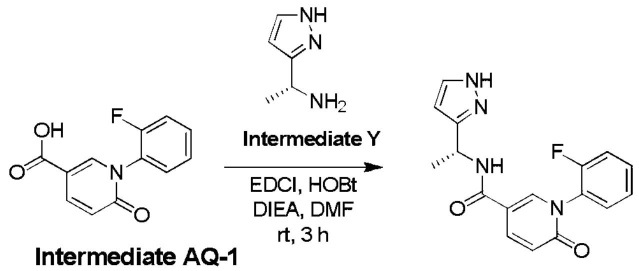

Intermediate AQ-1

(R)-N-(1-(1H-pyrazol-3-yl)ethyl)-1-(2-fluorophenyl)-6-oxo-1,6-dihydropyridine-3-carboxamide (20.1 mg, 17.0% yield) was obtained in the same manner as in Step 2 of Example 76. LC/MS m/z = 327.7 [M+H]$^+$.

<u>Step 2: Synthesis of (R)-1-(2-fluorophenyl)-N-(1-(1-(2-((methylamino)methyl)phenyl)-1H-pyrazol-3-yl)ethyl)-6-oxo-1,6-dihydropyridine-3-carboxamide</u>

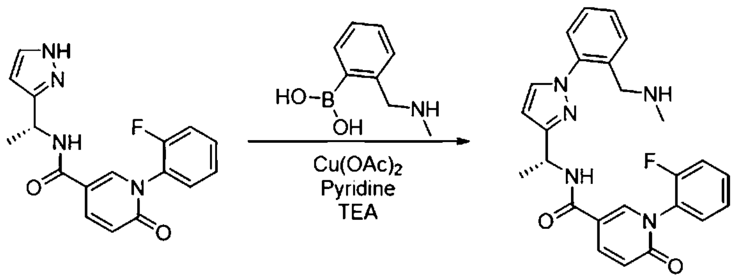

**112**

To a solution of (R)-N-(1-(1H-pyrazol-3-yl)ethyl)-1-(2-fluorophenyl)-6-oxo-1,6-dihydropyridine-3-carboxamide (20.1 mg, 0.062 mmol) in anhydrous THF (0.2 mL), 2-((methylamino)methyl)phenylboronic acid (12.2 mg, 0.074 mmol), $Cu(OAc)_2$ (22.4 mg, 0.12 mmol), pyridine (39.7 μL, 0.49 mmol) and TEA (42.9 μl, 0.31 mmol) were added. The reaction mixture was heated by microwave at 140 °C for 10 min. The mixture was cooled to room temperature, and filtered through celite and rinsed with MeOH. The filtrate was concentrated, and the concentrate was purified by prep-HPLC to give the compound of Example 112 (1.9 mg, 6.9% yield). $^1$H NMR (500 MHz, MeOD) δ 8.25 (d, J = 2.6 Hz, 1H), 8.10 (d, J = 2.6 Hz, 1H), 8.07 (dd, J = 9.6, 2.6 Hz, 1H), 7.63 (ddd, J = 15.1, 7.5, 1.5 Hz, 2H), 7.62 - 7.52 (m, 2H), 7.49 (qd, J = 7.5, 1.6 Hz, 2H), 7.37 (q, J = 8.6 Hz, 2H), 6.65 (d, J = 9.6 Hz, 1H), 6.60 (d, J = 2.5 Hz, 1H), 5.40 - 5.31 (m, 1H), 4.13 (d, J = 3.6 Hz, 2H), 2.87 (s, 3H), 1.65 (d, J = 7.2 Hz, 3H); LC/MS m/z = 446.2 [M+H]$^+$.

**Example 113: 1-(3-acetamido-4-fluoro-phenyl)-N-[(1R)-1-[3-amino-5-(trifluoromethyl)phenyl] ethyl]-6-oxo-pyridine-3-carboxamide**

<u>Step 1: Synthesis of 1-(3-acetamido-4-fluoro-phenyl)-6-oxo-pyridine-3-carboxylic acid</u>

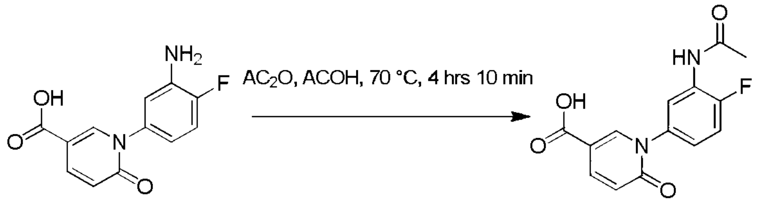

Intermediate AQ-2

A mixture of Intermediate AQ-2 (250 mg, 1.01 mmol) in AcOH (2 mL) was stirred at 70 °C for 10 min. The mixture was added with $Ac_2O$ (514 mg, 5.04 mmol) and stirred at 70 °C for 16 h. The mixture was filtrated, and the filtrate was concentrated in vacuo to give 1-(3-acetamido-4-fluoro-phenyl)-6-oxo-pyridine-3-carboxylic acid (180 mg, 61% yield) as a white solid. $^1$H NMR (400 MHz, DMSO-$d_6$) δ = 9.72 (s, 1H), 8.16 (d, *J* = 2.4 Hz, 1H), 8.02 (dd, *J* = 2.4, 6.8 Hz, 1H), 7.86 (dd, *J*

= 2.4, 9.6 Hz, 1H), 7.38 (dd, *J* = 8.8, 10.4 Hz, 1H), 7.28 - 7.16 (m, 1H), 6.54 (d, *J* = 9.6 Hz, 1H), 2.12 (s, 3H).

Steps 2 and 3: Synthesis of 1-(3-acetamido-4-fluoro-phenyl)-N-[(1R)-1-[3-amino-5-(trifluoromethyl)phenyl]ethyl]-6-oxo-pyridine-3-carboxamide

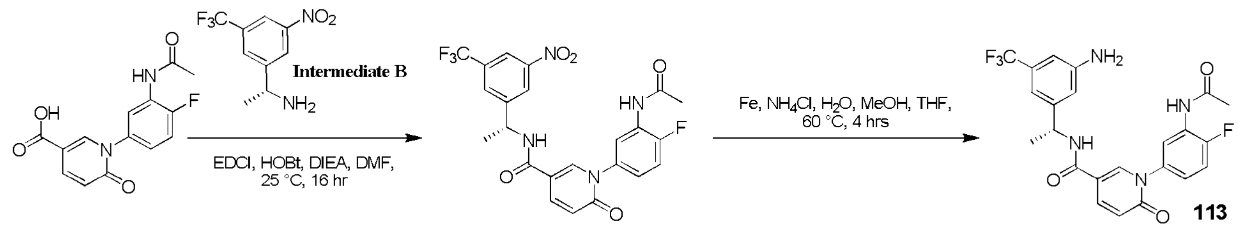


113

The compound of Example 113 (20.3 mg, 53% yield) was obtained in the same manner as in Steps 3 and 4 of Example 89 as a white solid. $^1$H NMR (400 MHz, DMSO-$d_6$) δ = 9.98 (s, 1H), 8.61 - 8.55 (m, 1H), 8.36 - 8.32 (m, 1H), 8.04 (d, *J* = 4.8 Hz, 1H), 7.98 - 7.92 (m, 1H), 7.48 - 7.38 (m, 1H), 7.28 - 7.22 (m, 1H), 6.88 - 6.82 (m, 1H), 6.76 (s, 1H), 6.68 (s, 1H), 6.56 - 6.52 (m, 1H), 5.58 - 5.52 (m, 1H), 5.06 - 4.96 (m, 1H), 2.14 - 2.09 (m, 3H), 1.42 (d, *J= 7.2* Hz, 3H); MS (EI) m/z: 477.1 $[M+H]^+$.

**Example 114: 1-(3-acetamido-2-fluoro-phenyl)-N-[(1R)-1-[3-amino-5-(trifluoromethyl)phenyl] ethyl]-6-oxo-pyridine-3-carboxamide**

Step 1: Synthesis of methyl-1-(3-bromo-2-fluoro-phenyl)-6-oxo-pyridine-3-carboxylate

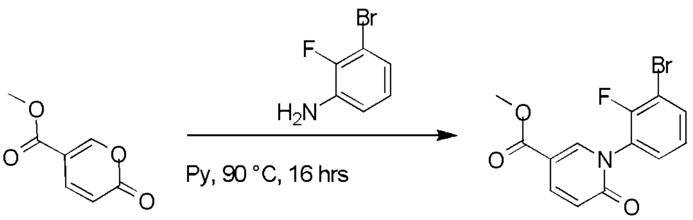


Methyl-1-(3-bromo-2-fluoro-phenyl)-6-oxo-pyridine-3-carboxylate (1.89 g, 22% yield) was obtained as a yellow solid in the same manner as in Step 1 of Preparation Example 43. $^1$H NMR (400 MHz, DMSO-$d_6$) δ = 8.44 (d, *J* = 2.4 Hz, 1H), 7.94 (dd, *J* = 2.4, 9.6 Hz, 1H), 7.89 - 7.81 (m, 1H), 7.66 - 7.60 (m, 1H), 7.34 (dd, *J* = 1.2, 8.0 Hz, 1H), 6.60 (d, *J* = 9.6 Hz, 1H), 3.78 (s, 3H).

Step 2: Synthesis of methyl-1-(3-acetamido-2-fluoro-phenyl)-6-oxo-pyridine-3-carboxylate

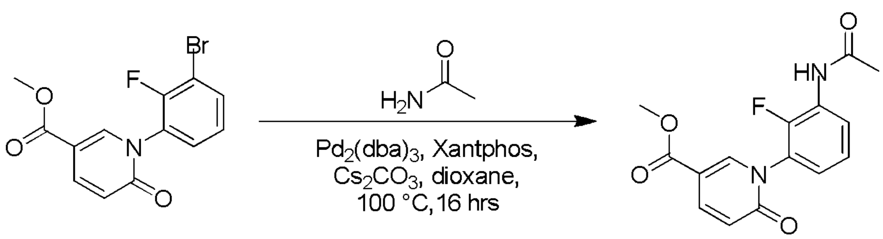


A solution of methyl-1-(3-bromo-2-fluoro-phenyl)-6-oxo-pyridine-3-carboxylate (500 mg, 1.53 mmol), acetamide (226 mg, 3.83 mmol), $Pd_2(dba)_3$ (140 mg, 153 μmol), Xantphos (88.7 mg, 153 μmol) and $Cs_2CO_3$ (1.25 g, 3.83 mmol) in dioxane (10 mL) was stirred at 100 °C for 16 h. The reaction mixture was concentrated in vacuo. The residue was purified by silica gel column chromatograpy (PE/EA = 1/0 to 2/1) to give methyl-1-(3-acetamido-2-fluoro-phenyl)-6-oxo-pyridine-3-carboxylate (380 mg, 81% yield) as a yellow solid. $^1H$ NMR (400 MHz, DMSO-$d_6$) δ = 9.94 (s, 1H), 8.36 (d, *J* = 2.4 Hz, 1H), 7.98 - 7.86 (m, 2H), 7.32 - 7.28 (m, 2H), 6.64 - 6.58 (m, 1H), 3.78 (s, 3H), 2.11 (s, 3H).

Steps 3 to 5: Synthesis of 1-(3-acetamido-2-fluoro-phenyl)-N-[(1R)-1-[3-amino-5-(trifluoromethyl)phenyl]ethyl]-6-oxo-pyridine-3-carboxamide

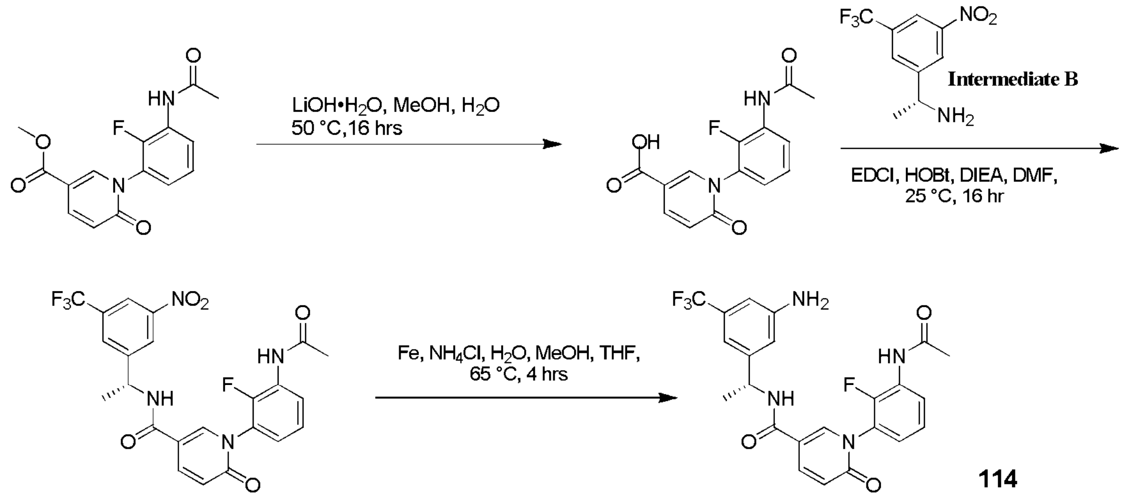


114

The compound of Example 114 (15.4 mg, 47% yield) was obtained as a white solid in the same manner as in Steps 2 to 4 of Example 89. $^1H$ NMR (400 MHz, DMSO-$d_6$) δ = 9.97 (s, 1H), 8.57 (d, *J* = 7.6 Hz, 1H), 8.39 (d, *J* = 2.4 Hz, 1H), 8.08 - 7.96 (m, 2H), 7.34 (d, *J* = 5.6 Hz, 2H), 6.76 (s, 2H), 6.71 (s, 1H), 6.58 (d, *J* = 9.6 Hz, 1H), 5.60 - 5.52 (m, 2H), 5.01 (t, *J* = 7.2 Hz, 1H), 2.12 (s, 3H), 1.40 (d, *J* = 7.2 Hz, 3H); MS (EI) m/z: 477.1 [M+H]$^+$.

**Example 115: N-[(1R)-1-[3-amino-5-(trifluoromethyl)phenyl]ethyl]-1-cyclopentyl-6-oxo-1,6-dihydropyridazine-3-carboxamide**

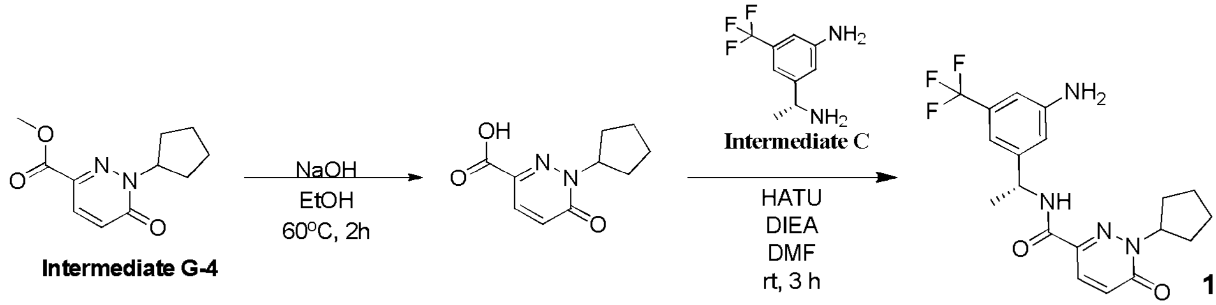


115

The compound of Example 115 (9 mg, 51.6% yield) was obtained in the same manner as in Example 64. $^1H$ NMR (400 MHz, DMSO) δ 8.52 (d, J = 8.3 Hz, 1H), 7.78 (d, J = 9.7 Hz, 1H), 6.98 (d, J = 9.7 Hz, 1H), 6.88 (s, 1H), 6.83 (s, 1H), 6.76 (s, 1H), 5.24 (p, J = 7.7 Hz, 1H), 5.09 - 5.00 (m, 1H), 1.97 (d, J = 8.9 Hz, 5H), 1.83 (t, J = 6.2 Hz, 2H), 1.64 (d, J = 6.9 Hz, 2H), 1.49 (d, J = 7.0 Hz, 3H); LC/MS m/z = 395.2 [M+H]$^+$.

**Example 116 and Example 117**

The compounds shown in the following table were prepared in the same manner as in Example 115 by preparing appropriate starting materials corresponding to the respective structures of the desired compounds based on Preparation Example 7.

[Table 8]

| No. | Structure/Name | Spectral Data |
|---|---|---|
| 116 | 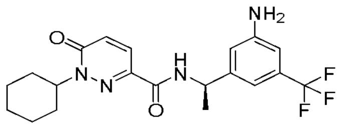 N-[(1R)-1-[3-amino-5-(trifluoromethyl)phenyl]ethyl]-1-cyclohexyl-6-oxo-1,6-dihydropyridazine-3-carboxamide | $^1$H NMR (400 MHz, DMSO) δ 8.65 (d, J = 8.4 Hz, 1H), 7.79 (d, J = 9.6 Hz, 1H), 6.99 (d, J = 9.7 Hz, 1H), 6.91 (s, 1H), 6.84 (s, 1H), 6.77 (d, J = 1.8 Hz, 1H), 5.08 (p, J = 7.3 Hz, 1H), 4.76 (td, J = 11.6, 3.9 Hz, 1H), 1.89 (tdd, J = 15.6, 9.4, 5.0 Hz, 3H), 1.77 (d, J = 7.8 Hz, 2H), 1.68 (d, J = 12.8 Hz, 1H), 1.54 - 1.34 (m, 4H), 1.34 1.22 (m, 1H); LC/MS (ESI) m/z = 409.2 [M+H]$^+$ |
| 117 | 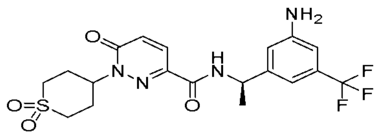 N-[(1R)-1-[3-amino-5-(trifluoromethyl)phenyl]ethyl]-1-(1,1-dioxo-1λ$^6$-thian-4-yl)-6-oxo-1,6-dihydropyridazine-3-carboxamide | $^1$H NMR (400 MHz, DMSO) δ 8.82 (d, J = 8.4 Hz, 1H), 7.81 (d, J = 9.7 Hz, 1H), 7.03 (d, J = 9.7 Hz, 1H), 6.81 (d, J = 9.1 Hz, 2H), 6.72 (s, 1H), 5.57 (s, 2H), 5.16 (t, J = 11.4 Hz, 1H), 5.05 (t, J = 7.5 Hz, 1H), 3.50 (t, J = 13.5 Hz, 2H), 2.61 (s, 1H), 2.14 (d, J = 13.1 Hz, 2H), 1.50 (d, J = 7.1 Hz, 3H); LC/MS (ESI) m/z = 459.2 [M+H]$^+$ |

**Example 118: 1-(1-acetylpiperidin-4-yl)-N-[(1R)-1-[3-amino-5-(trifluoromethyl)phenyl]ethyl]-6-oxo-1,6-dihydropyridazine-3-carboxamide**

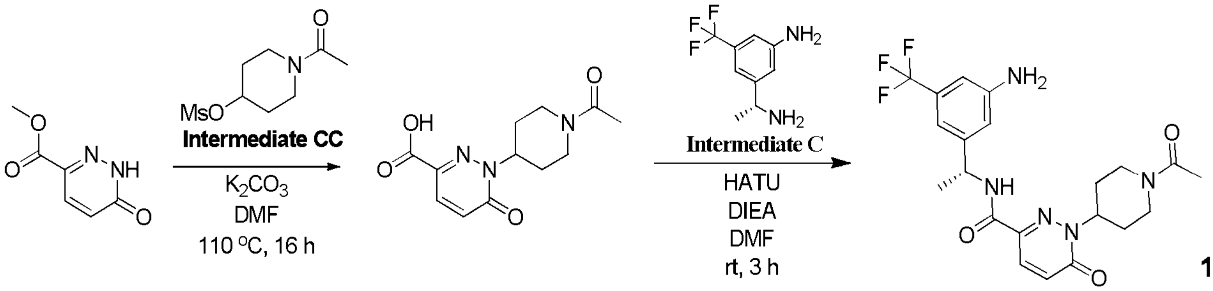


**118**

The compound of Example 118 (10 mg, 15% yield) was obtained in a similar manner to Preparation Example 7 and Step 2 of Example 115. $^1$H NMR (400 MHz, DMSO) δ 8.77 (d, J = 8.5 Hz, 1H), 7.81 (dd, J = 9.7, 1.9 Hz, 1H), 7.02 (d, J = 9.7 Hz, 1H), 6.87 - 6.76 (m, 2H), 6.70 (d, J = 8.9 Hz, 2H), 5.56 (d, J = 9.7 Hz, 2H), 5.11 - 5.01 (m, 1H), 4.98 (d, J = 12.5 Hz, 1H), 4.58 (d, J = 13.3 Hz, 1H), 3.97 (s, 1H), 3.30 - 3.14 (m, 2H), 2.74 - 2.65 (m, 1H), 2.04 (d, J = 3.9 Hz, 3H), 1.87 - 1.75 (m, 2H), 1.49 (d, J = 7.1 Hz, 3H); LC/MS m/z = 425.2 [M+H]$^+$.

**Example 119 and Example 120**

The compounds shown in the following table were prepared in the same manner as in Example 118 by replacing Intermediate CC with appropriate methanesulfonate compounds corresponding to the respective structures of the desired compounds.

[Table 9]

| No. | Structure/Name | Spectral Data |
|---|---|---|
| 119 | 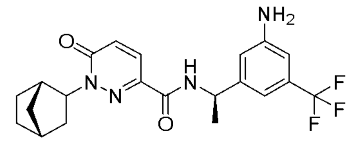 N-[(1R)-1-[3-amino-5-(trifluoromethyl)phenyl] ethyl]-1-[(1S,4R)-bicyclo[2.2.1]heptan-2-yl]-6-oxo-1,6-dihydropyridazine-3-carboxamide | $^1$H NMR (400 MHz, DMSO) δ 8.36 (d, J = 8.1 Hz, 1H), 7.82 (d, J = 9.6 Hz, 1H), 7.00 (d, J = 9.6 Hz, 1H), 6.83 (d, J = 13.8 Hz, 2H), 6.72 (s, 1H), 5.57 (s, 2H), 5.12 (d, J = 11.7 Hz, 1H), 5.00 (t, J = 7.4 Hz, 1H), 2.34 (s, 2H), 1.82 (t, *J* = 12.5 Hz, 1H), 1.63 (d, J = 9.7 Hz, 1H), 1.49 (d, J = 7.0 Hz, 4H), 1.39 (d, J = 9.6 Hz, 1H), 1.23 (s, 3H), 1.08 (s, 1H); LC/MS (ESI) m/z = 421.2 [M+H]$^+$ |
| 120 | 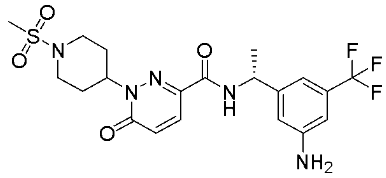 N-[(1R)-1-[3-amino-5-(trifluoromethyl)phenyl]ethyl]-1-(1-methanesulfonylpiperidin-4-yl)-6-oxo-1,6-dihydropyridazine-3-carboxamide | $^1$H NMR (400 MHz, DMSO) δ 8.88 (d, J = 8.4 Hz, 1H), 7.82 (d, J = 9.7 Hz, 1H), 7.02 (d, J = 9.7 Hz, 1H), 6.86 (s, 1H), 6.82 (s, 1H), 6.74 (s, 1H), 5.12 - 5.04 (m, 1H), 4.91 (s, 1H), 3.71 (d, J = 11.8 Hz, 3H), 3.01 - 2.93 (m, 2H), 2.91 (s, 3H), 2.26 (d, J = 11.7 Hz, 2H), 1.83 (m, 2H), 1.50 (d, J = 7.1 Hz, 3H); LC/MS (ESI) m/z = 488.2 [M+H]$^+$ |

**Example 121 and Example 122: N-[(1R)-1-[5-amino-2-chloro-3-(trifluoromethyl)phenyl]ethyl]-1-(2-fluorophenyl)-6-oxo-pyridazine-3-carboxamide and** N-**[(1S)-1-[S-amino-2-chloro-3-(trifluoromethyl)phenyl]ethyl]-1-(2-fluorophenyl)-6-oxo-**pyridazine-3-carboxamide

Step 1: Synthesis of 1-bromo-2-chloro-5-nitro-3-(trifluoromethyl)benzene

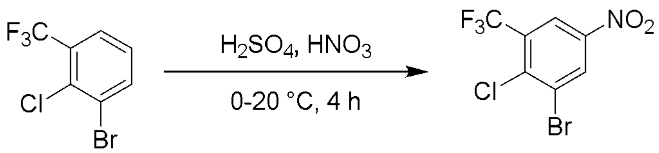


To a solution of 1-bromo-2-chloro-3-(trifluoromethyl)benzene (8.3 g, 31.99 mmol) in $H_2SO_4$ (15 mL) was added $HNO_3$ (10.270 g, 159.72 mmol, 7.34 mL, 98% purity) at 0 °C for 40 min, followed by stirring at 20 °C for 4 h. The reaction mixture was poured onto ice water (30 mL) and extracted wtih EtOAc (40 mL×3). The combined organic layer was washed with brine (50 mL), dried over $Na_2SO_4$ and concentrated under reduced pressure to obtain a crude product, which was purified by silica gel column chromatography (1% EtOAc in PE) to give 1-bromo-2-chloro-5-nitro-3-(trifluoromethyl) benzene (9.3 g, 95.49% yield) as yellow oil. $^1$H NMR (400MHz, DMSO-$d_6$) δ 8.50 (d, *J* = 2.8 Hz, 1 H) 8.90 (d, *J* = 2.8 Hz, 1 H).

Step 2: Synthesis of 1-(2-chloro-5-nitro-3-(trifluoromethyl)phenyl)ethane-1-one

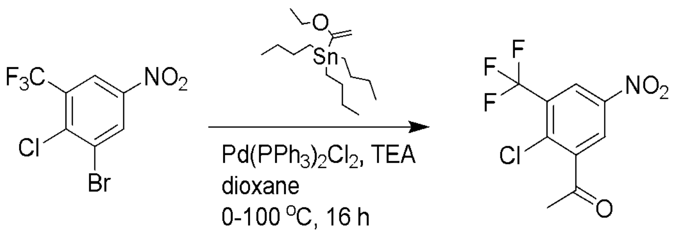


To a mixture of 1-bromo-2-chloro-5-nitro-3-(trifluorophenyl)benzene (3 g, 9.85 mmol), tributyl(1-ethoxyvinyl) stannane (3.340 g, 9.25 mmol, 3.12 mL) in dioxane (40 mL), TEA (1.99 g, 19.71 mmol, 2.74 mL) and $Pd(PPh_3)_2Cl_2$ (691.64

mg, 985.39 μmol) were added, followed by stirring at 100 °C for 16 h under nitrogen atmosphere. The reaction mixture was cooled to 0 °C, treated with 4 M HCl, and stirred for 3 h. The mixture was poured into water (50 mL) and extracted with EtOAc (50 mL x 3). The mixture was dried over $Na_2SO_4$ and filtered, and the filtrate was concentrated. The aqueous layer was adjusted to pH 9 with NaOH aqueous solution, added slowly with sodium hypochlorite (100 mL), and stirred. The product was purified by silica gel column chromatography (5% EtOAc in PE) to give 1-[2-chloro-5-nitro-3-(trifluoromethyl)phenyl]ethanone (1.47 g, 55.75% yield) as a yellow solid. $^1H$ NMR (400MHz, DMSO-$d_6$) δ 2.68 (m, 3 H) 8.59 (d, *J* = 2.8 Hz, 1 H) 8.81 (d, *J* = 2.8 Hz, 1 H).

Step 3: Synthesis of (R,Z)-N-(1-(2-chloro-5-nitro-3-(trifluoromethyl)phenyl)ethylidene)-2-methylpropane-2-sulfinamide

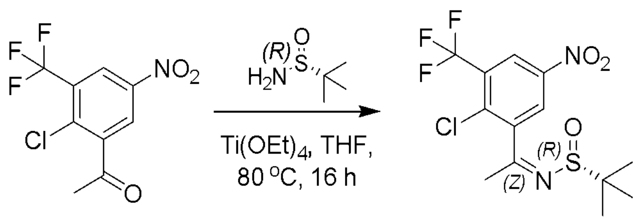


To a solution of 1-[2-chloro-5-nitro-3-(trifluoromethyl)phenyl]ethanone (1.47 g, 5.49 mmol) and (R)-2-methylpropane-2-sulfinamide (665.82 mg, 5.49 mmol) in THF (35 mL) was added $Ti(OEt)_4$ (3.76 g, 16.48 mmol, 3.42 mL). The reaction mixture was stirred 80 °C for 16 h under nitrogen atmosphere. The reaction mixture was poured into water (30 mL) and extracted with EtOAc (30 mL × 3). The combined organic layer was washed with brine (50 mL), dried over $Na_2SO_4$, and concentrated under reduced pressure to obtain a crude product, which was purified by silica gel column chromatography (6% EtOAc in PE) to obtain (NZ,R)-N-[1-[2-chloro-5-nitro-3-(trifluoromethyl)phenyl]ethylidene]-2-methyl-propane-2-sulfinamide (1.66 g, 81.50% yield) as a yellow solid. $^1H$ NMR (400MHz, DMSO-$d_6$) δ 1.20 (br d, *J* = 17.2 Hz, 9 H) 2.50 (s, 2 H) 2.69 (s, 1 H) 8.53 (m, 1 H) 8.67 (br s, 1 H).

Step 4: (R)-N-[(1R)-1-[2-chloro-5-nitro-3-(trifluoro)phenyl]ethyl]-2-methyl-propane-2-sulfinamide and (R)-N-[(1S)-1-[2-chloro-5-nitro-3-(trifluoromethyl)phenyl]ethyl]-2-methylpropane-2-sulfinamide

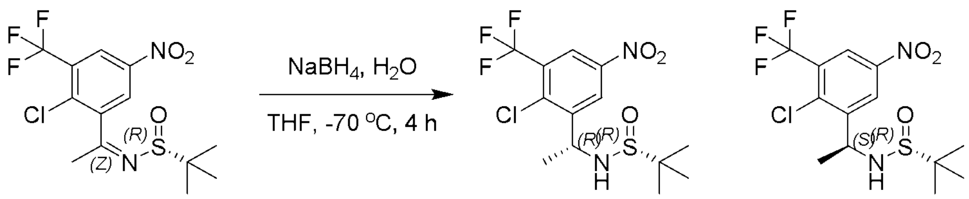


To a solution of (NZ,R)-N-[1-[2-chloro-5-nitro-3-(trifluoro)phenyl]ethylidene]-2-methylpropane-2-sulfinamide (480 mg, 1.29 mmol) in THF (12 mL) and $H_2O$ (0.5 mL) was added $NaBH_4$ (0.210 g, 5.55 mmol), followed by stirring at -70 °C for 4 h under $N_2$ atmosphere. The mixture was quenched with aqueous ammonium chloride solution at room temperature, diluted with EtOAc (50 mL) and extracted with EtOAc (50 mL * 3). The organic layer was dried over $Na_2SO_4$ and filtrated under reduced pressure to obtain a crude product. The product was purified by silica column chromatography (28% EtOAc in PE) to obtain (R)-N-[(1R)-1-[2-chloro-5-nitro-3-(trifluoro)phenyl]ethyl]-2-methyl-propane-2-sulfinamide (70 mg, 14.50% yield, 100% ee) and (R)-N-[(1S)-1-[2-chloro-5-nitro-3-(trifluoromethyl)phenyl]ethyl]-2-methyl-propane-2-sulfinamide (100 mg, 20.72% yield, 98.76% ee) as a yellow solid.

(R)_$^1H$ NMR (400 MHz, DMSO-$d_6$) δ ppm 1.12 (s, 9 H) 1.44 (d, *J* = 6.8 Hz, 3 H) 4.95 (m, 1 H) 6.34 (d, *J* = 8.8 Hz, 1 H) 8.45 (d, *J* = 2.8 Hz, 1 H) 8.84 (d, *J* = 2.8 Hz, 1 H); MS (ESI) m/z = 372.9 $[M+H]^+$. LC/MS $t_R$ = 0.571 min in 5-95AB_1min. (RP-18, 5um, 3.0*25mm), MS (ESI) m/z = 372.9 $[M+H]^+$; SFC (EB5303-244-P1S1): $t_R$ = 0.503 min in IC_3_IPA_DEA_40_25ML, e.e = 100%.

(S)_$^1H$ NMR (400 MHz, DMSO-$d_6$) δ ppm 1.14 (s, 9 H) 1.53 (d, *J* = 6.8 Hz, 3 H) 4.97 (quin, *J* = 6.8 Hz, 1 H) 5.92 (d, *J* = 6.4 Hz, 1 H) 8.44 (d, *J* = 2.8 Hz, 1 H) 8.73 (d, *J* = 2.8 Hz, 1 H); LC/MS (EB5303-244-P1A2): $t_R$ = 0.576 min in 5-95AB_1min. (RP-18, 5um, 3.0*25mm), MS (ESI) m/z = 372.9 $[M+H]^+$; SFC (EB5303-244-P1S2): $t_R$ = 0.421 min in IC_3_IPA_DEA_40_25ML, e.e = 98.76%.

Step 5: (1R)-1-[2-chloro-5-nitro-3-(trifluoromethyl)phenyl]ethanamine hydrochloride and (1S)-1-[2-chloro-5-nitro-3-(trifluoromethyl)phenyl]ethanamine

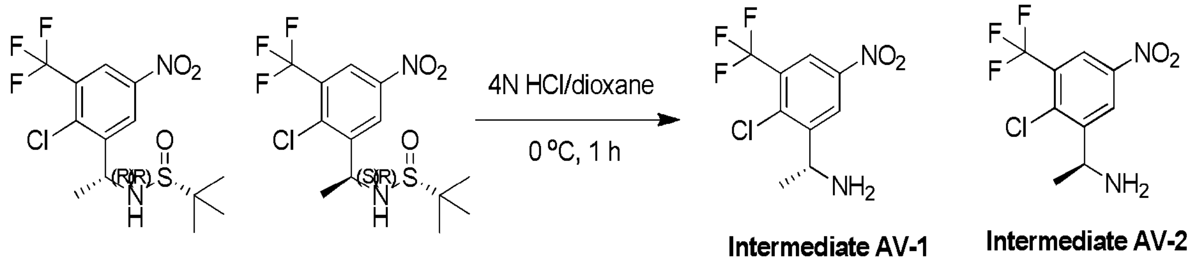


**Intermediate AV-1** **Intermediate AV-2**

(R)-N-[(1R)-1-[2-chloro-5-nitro-3-(trifluoromethyl)phenyl]ethyl]-2-methyl-propane-2-sulfinamide (70 mg, 187.77 μmol) was added to 4N HCl/dioxane (3 mL) and stirred at 0 °C for 1 hour. The mixture was filtrated under reduced pressure to give Intermediate AV-1 (50 mg, 87.28%, HCl salt) as a yellow solid. LC/MS (ESI) m/z = 268.9 $[M+H]^+$.

Intermediate AV-2 (89 mg, 210.19 μmol, 78.36% yield, HCl salt) was obtained as a yellow solid in the same method as above. LC/MS (ESI) m/z = 269.0 $[M+H]^+$.

Step 6-1: Synthesis ofN-[(1R)-1-[2-chloro-5-nitro-3-(trifluoromethyl)phenyl]ethyl]-1-(2-fluorophenyl)-6-oxo-pyridazine-3-carboxamide

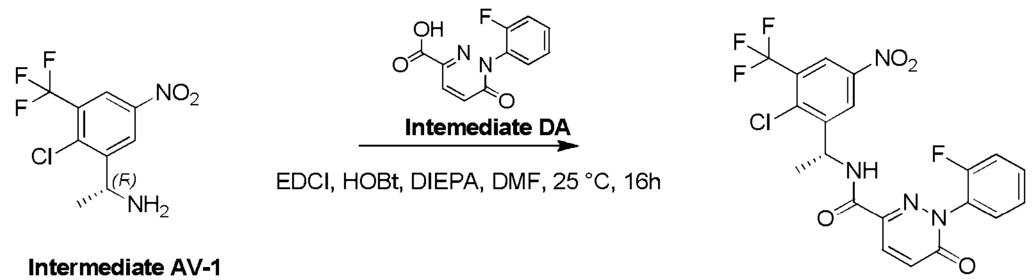


**Intermediate AV-1**

N-[(1R)-1-[2-chloro-5-nitro-3-(trifluoromethyl)phenyl]ethyl]-1-(2-fluorophenyl)-6-oxo-pyridazine-3-carboxamide was obtained in the same manner as in Example 94. MS (ESI) m/z = 485.1 $[M+H]^+$.

Step 7-1: Synthesis of N-[(1R)-1-[5-amino-2-chloro-3-(trifluoromethyl)phenyl]ethyl]-1-(2-fluorophenyl)-6-oxo-pyridazine-3-carboxamide

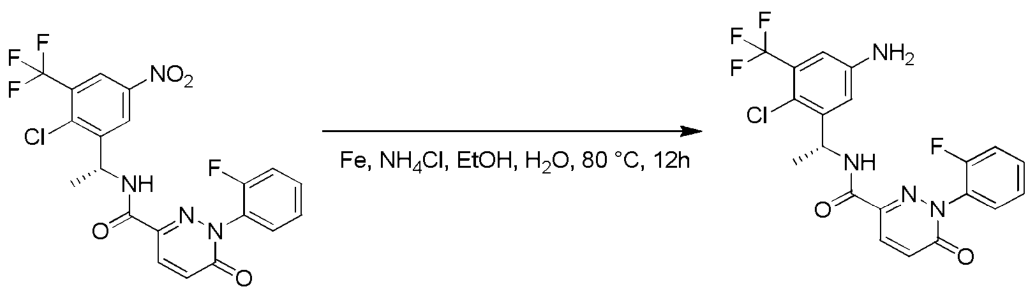


**121**

The compound of Example 121 was obtained in the same manner as in Step 3 of Example 56, except that EtOH and $H_2O$ were used instead of MeOH in Step 3 of Example 56. $^1H$ NMR (400 MHz, DMSO-$d_6$) δ ppm 1.40 (d, J = 7.2 Hz, 3 H) 5.31 (q, J = 7.2 Hz, 1 H) 6.88 (dd, J = 17.6, 2.8 Hz, 2 H) 7.19 (d, J = 10.0 Hz, 1 H) 7.44 (m, 2 H) 7.59 (m, 1 H) 7.71 (td, J = 7.6, 1.6 Hz, 1 H) 7.92 (d, J = 10.0 Hz, 1 H) 9.04 (d, J = 7.2 Hz, 1 H); MS (ESI) m/z = 455.3 $[M+H]^+$.

Step 6-2: N-[(1S)-1-[2-chloro-5-nitro-3-(trifluoromethyl)phenyl]ethyl]-1-(2-fluorophenyl)-6-oxo-pyridazine-3 -carboxamide

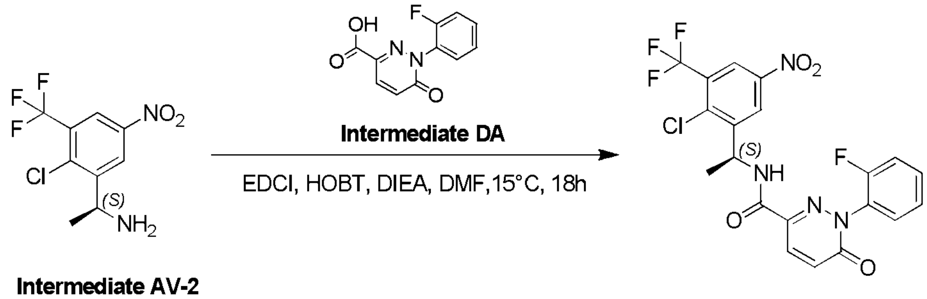


Intermediate AV-2

N-[(1S)-1-[2-chloro-5-nitro-3-(trifluoromethyl)phenyl]ethyl]-1-(2-fluorophenyl)-6-oxo-pyridazine-3-carboxamide was obtained in the same manner as in Example 94. MS (ESI) m/z = 485.1 [M+H] $^+$

Step 7-2: N-[(1S)-1-[5-amino-2-chloro-3-(trifluoromethyl)phenyl]ethyl]-1-(2-fluorophenyl)-6-oxo-pyridazine-3 -carboxamide

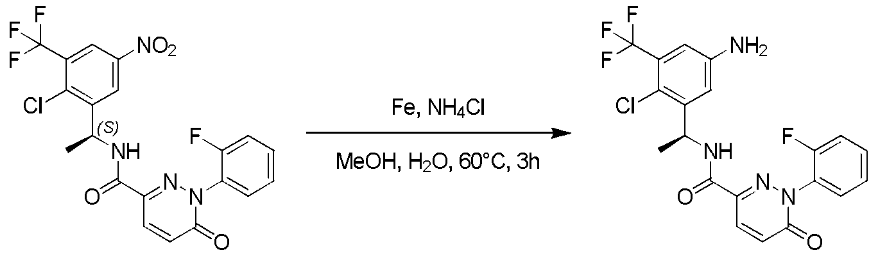


122

The compound of Example 122 was obtained in the same manner as in Step 3 of Example 56, except that $H_2O$ was added in Step 3 of Example 56. $^1$H NMR (400 MHz, DMSO-$d_6$) $\delta$ = 9.03 (d, *J* = 7.2 Hz, 1H), 7.94 - 7.90 (m, 1H), 7.73 - 7.67 (m, 1H), 7.63 - 7.56 (m, 1H), 7.49 - 7.45 (m, 1H), 7.44 - 7.40 (m, 1H), 7.19 (d, *J* = 9.6 Hz, 1H), 6.90 (d, *J* = 2.8 Hz, 1H), 6.85 (d, *J* = 2.4 Hz, 1H), 5.68 (s, 2H), 5.31 (quin, *J* = 7.2 Hz, 1H), 1.40 (d, *J* = 7.2 Hz, 3H); MS (ESI) m/z = 455.3 [M+H]$^+$.

**Example 123: N-[(1R)-1-[3-amino-5-(trifluoromethyl)phenyl]ethyl]-6-oxo-1-(tetrahydropyran-4-ylmethyl)pyridine-3-carboxamide**

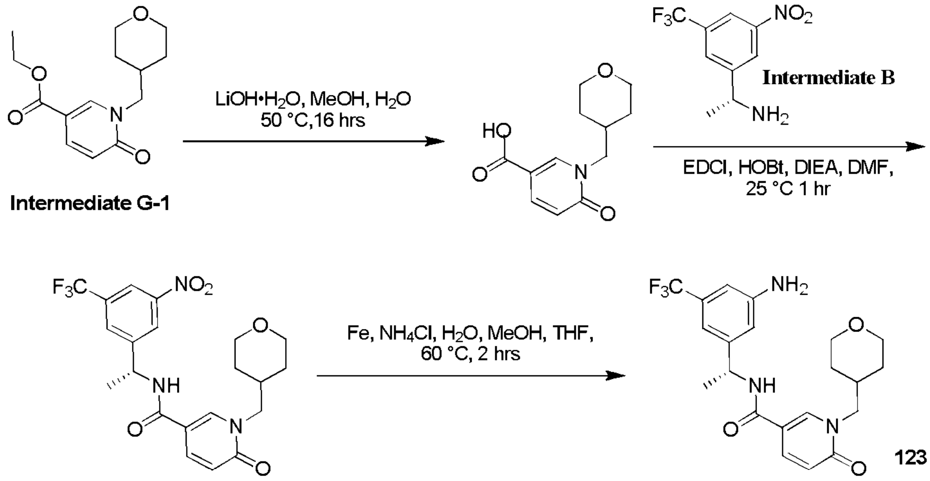


Intermediate G-1

123

The compound of Example 123 (50 mg, 89% yield) was obtained in the same manner as in Steps 2 to 4 of Example 89 as a white solid. $^1$H NMR (400 MHz, DMSO-$d_6$) δ = 8.51 (d, $J$ = 8.0 Hz, 1H), 8.31 (d, $J$ = 2.4 Hz, 1H), 7.93 (dd, $J$ = 2.8, 9.6 Hz, 1H), 6.79 (s, 1H), 6.78 (s, 1H), 6.71 (s, 1H), 6.42 (d, $J$ = 9.6 Hz, 1H), 5.02 ($J$ = 7.2 Hz, 1H), 3.92 - 3.78 (m, 4H), 3.22 (t, $J$ = 11.6 Hz, 2H), 2.08 - 1.95 (m, 1H), 1.42 (d, $J$ = 7.2 Hz, 3H), 1.39 (s, 2H), 1.29 - 1.23 (m, 2H). MS (EI) m/z: 424.2 $[M+H]^+$.

**Example 124 and Example 125: N-((R)-1-(3-amino-5-(trifluoromethyl)phenyl)ethyl)-1-((1R,2R)-2-hydroxycyclohexyl)-6-oxo-1,6-dihydropyridazine-3-carboxamide and N-((R)-1-(3-amino-5-(trifluoromethyl)phenyl)ethyl)-1-((1S,2S)-2-hydroxycyclohexyl)-6-oxo-1,6-dihydropyridazine-3-carboxamide**

Step 1: Synthesis of 1-(2-hydroxycyclohexyl)-6-oxo-1,6-dihydropyridazine-3-carboxylic acid

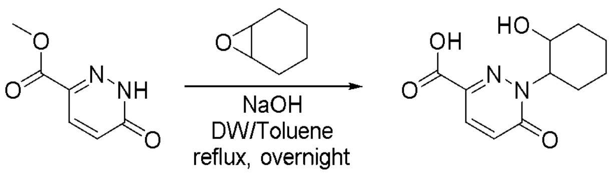


A mixture of methyl-6-oxo-1,6-dihydropyridazine-3-carboxylate (150 mg, 0.97 mmol), 7-oxabicyclo[4.1.0]heptane (478 mg, 4.87 mmol), sodium hydroxide (44.8 mg, 1.12 mmol) and benzyltriethylammonium chloride (22.2 mg, 0.09 mmol) in a mixture of water (2 mL) and toluene (2 mL) was refluxed for 18 h overnight. The reaction mixture was cooled to room temperature. The mixture was added with distilled water and extracted with EA. The aqueous layer was acidified with 1N HCl and extracted with EA. The organic layer was dried over $MgSO_4$, filtered and concentrated under reduced pressure to give 1-(2-hydroxycyclohexyl)-6-oxo-1,6-dihydropyridazine-3-carboxylic acid (182 mg, 78%) as a solid. LC/MS m/z = 239.1 $[M+H]^+$.

Step 2: Synthesis of N-((R)-1-(3-amino-5-(trifluoromethyl)phenyl)ethyl)-1-((1R,2R)-2-hydroxycyclohexyl)-6-oxo-1,6-dihydropyridazine-3-carboxamide and N-((R)-1-(3-amino-5-(trifluoromethyl)phenyl)ethyl)-1-((1S,2S)-2-hydroxycyclohexyl)-6-oxo-1,6-dihydropyridazine-3-carboxamide

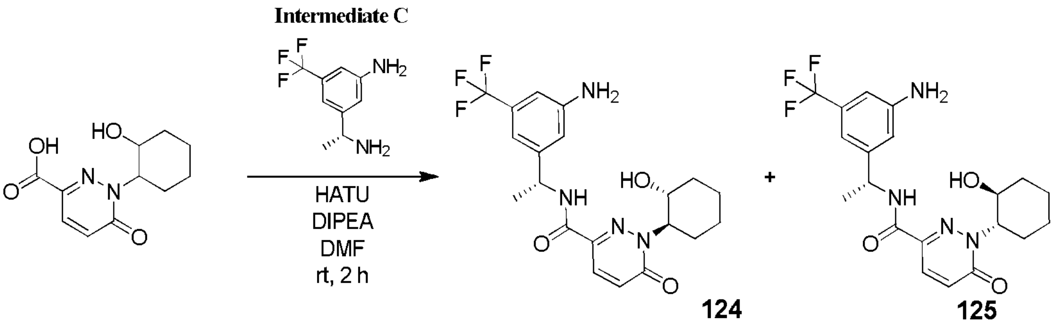


124

125

A solution of (R)-3-(1-aminoethyl)-5-(trifluoromethyl)aniline, 1-(2-hydroxycyclohexyl)-6-oxo-1,6-dihydropyridazine-3-carboxylic acid (30 mg, 0.12 mmol), HATU (47.9 mg, 0.12 mmol) and DIPEA (44 μL, 0.25 mmol) in DMF (2 mL) was prepared. The reaction mixture was stirred at room temperature for 2 h. The mixture was added with distilled water and sat. $NaHCO_3$ solution and extracted with EA. The organic layer was dried over $MgSO_4$. The solvent was removed under reduced pressure, and the crude residue was purified by Prep/LC to give the compound of Example 124 (10 mg, 18.71%) and the compound of Example 125 (6.5 mg, 12.16%).

Compound of Example 124: $^1H$ NMR (400 MHz, DMSO-$d_6$) δ 8.63 (d, J = 8.5 Hz, 1H), 7.77 (d, J = 9.7 Hz, 1H), 6.97 (d, J = 9.6 Hz, 1H), 6.88 (s, 1H), 6.83 (s, 1H), 6.75 (t, J = 2.8 Hz, 1H), 5.09 (p, J = 7.2 Hz, 1H), 4.69 (t, J = 9.2 Hz, 1H), 3.99 (s, 1H), 1.98 (s, 1H), 1.78 (d, J = 17.4 Hz, 2H), 1.72 (s, 2H), 1.51 (d, J = 7.0 Hz, 3H), 1.36 (s, 3H), 1.29 - 1.21 (m, 1H); LC/MS m/z = 425.2 $[M+H]^+$.

Compound of Example 125: $^1H$ NMR (400 MHz, DMSO) δ 8.61 (d, J = 8.5 Hz, 1H), 7.77 (d, J = 9.6 Hz, 1H), 6.97 (d, J = 9.6 Hz, 1H), 6.89 (s, 1H), 6.83 (s, 1H), 6.77 - 6.71 (m, 2H), 5.13 - 5.04 (m, 1H), 4.68 (s, 1H), 3.97 (d, J = 4.9 Hz, 1H), 1.97 (s, 1H), 1.82 (s, 1H), 1.73 (t, J = 10.8 Hz, 3H), 1.51 (d, J = 7.1 Hz, 3H), 1.36 (d, J = 7.6 Hz, 2H), 1.29 - 1.21 (m, 1H); LC/MS m/z = 425.2 $[M+H]^+$.

**Example 126: N-[(1R)-1-[3-amino-5-(trifluoromethyl)phenyl]ethyl]-1-(1-methyl-1,2,3,6-tetrahydropyridin-4-yl)-6-oxo-1,6-dihydropyridazine-3-carboxamide**

Step 1: Synthesis of methyl-1-(1-methyl-1,2,3,6-tetrahydropyridin-4-yl)-6-oxo-1,6-dihydropyridazine-3-carboxylate

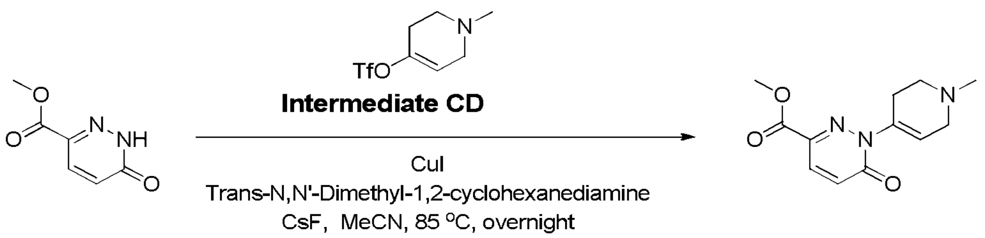


To a solution of methyl-6-oxo-1,6-dihydropyridazine-3-carboxylate (188 mg, 1.22 mmol) in MeCN (3 mL), Intermediate CD (450 mg, 1.84 mmol), CuI (233 mg, 1.23 mmol), trans-N,N'-dimethyl-1,2-cyclohexanediamine (174 mg, 1.22 mmol) and CsF (372 mg, 2.45 mmol) were added. The reaction mixture was stirred at 85 °C overnight under $N_2$ atmosphere and concentrated under reduced pressure. The residue was purified by prep-HPLC. The solvent was removed under reduced pressure to give methyl-1-(1-methyl-1,2,3,6-tetrahydropyridin-4-yl)-6-oxo-1,6-dihydropyridazine-3-carboxylate (173.6 mg, 56.9% yield). LC/MS m/z = 250.1 $[M+H]^+$.

Steps 2 and 3: Synthesis of N-[(1R)-1-[3-amino-5-(trifluoromethyl)phenyl]ethyl]-1-(1-methyl-1,2,3,6-tetrahydropyridin-4-yl)-6-oxo-1,6-dihydropyridazine-3-carboxamide

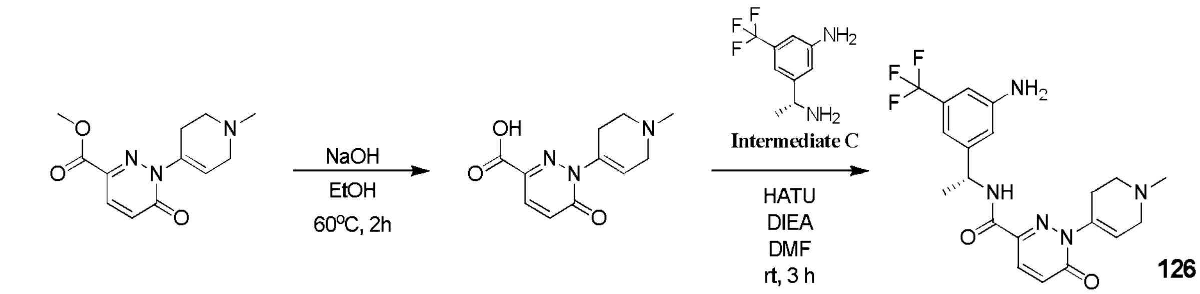


126

The compound of Example 126 (31.1 mg, 6.4% yield) was obtained in the same manner as in Example 64. $^1$H NMR (400 MHz, MeOD) δ 7.93 (d, J = 9.7 Hz, 1H), 7.04 (d, J = 9.7 Hz, 1H), 6.90 (d, J = 7.7 Hz, 2H), 6.81 (s, 1H), 5.13 (q, J = 7.0 Hz, 1H), 3.26 (d, J = 3.3 Hz, 2H), 2.84 (t, J = 5.8 Hz, 2H), 2.47 (s, 3H), 1.55 (d, J = 7.1 Hz, 3H); LC/MS m/z = 422.2 [M+H]$^+$.

**Example 127 and Example 128**

The compounds shown in the following table were prepared in the same manner as in Example 126 by replacing Intermediate CD with appropriate intermediates corresponding to the respective structures of the desired compounds.

[Table 10]

| No. | Structure/Name | Spectral Data |
|---|---|---|
| 127 | 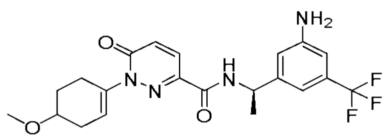 N-[(1R)-1-[3-amino-5-(trifluoromethyl)phenyl] ethyl]-1-(4-methoxycyclohex-1-en-1-yl)-6-oxo-1,6-dihydropyridazine-3-carboxamide | $^1$H NMR (400 MHz, DMSO) δ 8.83 (d, J = 8.2Hz, 1H), 7.80 (d, J = 9.7 Hz, 1H), 7.01 (d, J = 9.7 Hz, 1H), 6.85 - 6.77 (m, 2H), 6.71 (d, J = 1.9 Hz, 1H), 6.00 - 5.93 (m, 1H), 5.57 (s, 2H), 5.04 (t, J = 7.5 Hz, 1H), 3.54 (d, J = 7.4 Hz, 1H), 2.45 (s, 2H), 2.20 - 2.11 (m, 1H), 1.98 (m, 1H), 1.74 (m, 1H), 1.47 (d, J = 7.1 Hz, 3H); LC/MS (ESI) m/z = 437.1 [M+H]$^+$ |
| 128 | 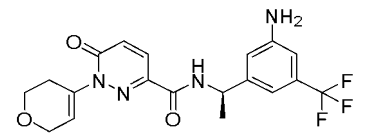 N-[(1R)-1-[3-amino-5-(trifluoromethyl)phenyl] ethyl]-1-(3,6-dihydro-2H-pyran-4-yl)-6-oxo-1,6-dihydropyridazine-3-carboxamide | $^1$H NMR (400 MHz, DMSO) δ 8.83 (d, J = 8.3 Hz, 1H), 7.81 (d, J = 9.7 Hz, 1H), 7.04 (d, J = 9.7 Hz, 1H), 6.81 (d, J = 17.5 Hz, 2H), 6.71 (s, 1H), 6.31 (s, 1H), 5.57 (s, 2H), 5.08 - 5.00 (m, 1H), 4.28 (d, J = 2.9 Hz, 2H), 3.85 (t, J = 5.5 Hz, 2H), 2.54 (s, 2H), 1.97 (m, 1H), 1.47 (d, J = 7.1 Hz, 3H); LC/MS (ESI) m/z = 409.1 [M+H]$^+$ |

**Example 129: N-[(1R)-1-[3-amino-5-(trifluoromethyl)phenyl]ethyl]-1-(3-acetamidophenyl)-6-oxo-1,6-dihydropyridine-3-carboxamide**

Step 1: Synthesis of ethyl-1-(3-acetamidophenyl)-6-oxo-1,6-dihydropyridine-3-carboxylate

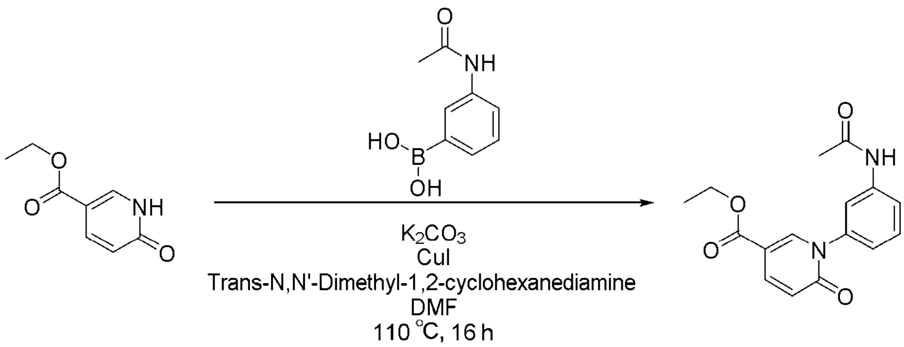

To a solution of ethyl-6-oxo-1,6-dihydropyridine-3-carboxylate (100 mg, 0.60 mmol), (3-acetamidophenyl) boronic acid (154 mg, 0.72 mmol), potassium carbonate (165 mg, 1.20 mmol) and copper(I) iodide (114 mg, 0.60 mmol) in DMF (1.1 mL) was added trans-N,N'-dimethyl-1,2-cyclohexanediamine (95 μL, 0.60 mmol). The reaction mixture was heated at 110 °C for 16 h. The mixture was quenched and extracted with DCM. The organic layer was dried over anhydrous $Na_2SO_4$ and concentrated under reduced pressure to obtain a crude product. The residue was purified by flash chromatography (0-5% MeOH in DCM) to give ethyl-1-(3-acetamidophenyl)-6-oxo-1,6-dihydropyridine-3-carboxylate (117.7 mg, 65.5% yield). LC/MS m/z = 301.1 $[M+H]^+$.

Steps 2 and 3: Synthesis of N-[(1R)-1-[3-amino-5-(trifluoromethyl)phenyl]ethyl]-1-(1-methyl-1,2,3,6-tetrahydropyridin-4-yl)-6-oxo-1,6-dihydropyridazine-3-carboxamide

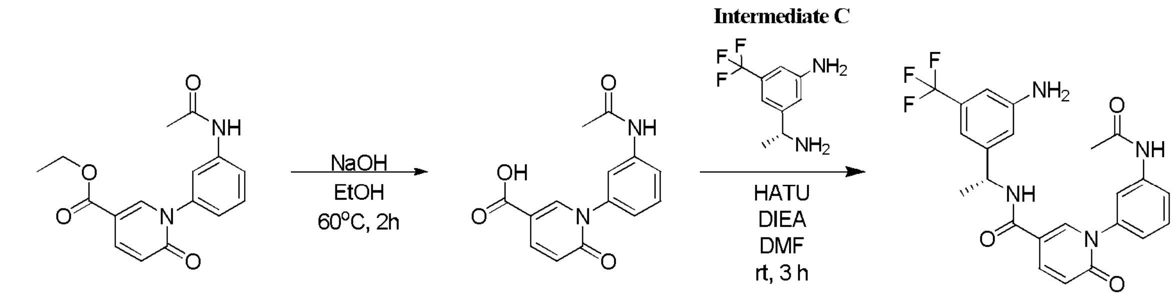


129

The compound of Example 129 (2.5 mg, 1.9% yield) was obtained in the same manner as in Example 64. $^1$H NMR (400 MHz, MeOD) δ 8.28 (d, J = 2.6 Hz, 1H), 8.04 (dd, J = 9.6, 2.7 Hz, 1H), 7.78 (t, J = 2.1 Hz, 1H), 7.60 (ddd, J = 8.2, 2.0, 1.0 Hz, 1H), 7.48 (t, J = 8.1 Hz, 1H), 7.16 (ddd, J = 7.9, 2.1, 1.0 Hz, 1H), 6.90 - 6.85 (m, 2H), 6.80 (d, J = 2.0 Hz, 1H), 6.63 (d, J = 9.6 Hz, 1H), 5.10 (q, J = 7.0 Hz, 1H), 2.14 (s, 3H), 1.50 (d, J = 7.1 Hz, 3H); LC/MS m/z = 459.2 $[M+H]^+$.

**Example 130: N-[(1R)-1-[3-amino-5-(trifluoromethyl)phenyl]ethyl]-1-(3-cyclopropaneamidophenyl)-6-oxo-1,6-dihydropyridine-3-carboxamide**

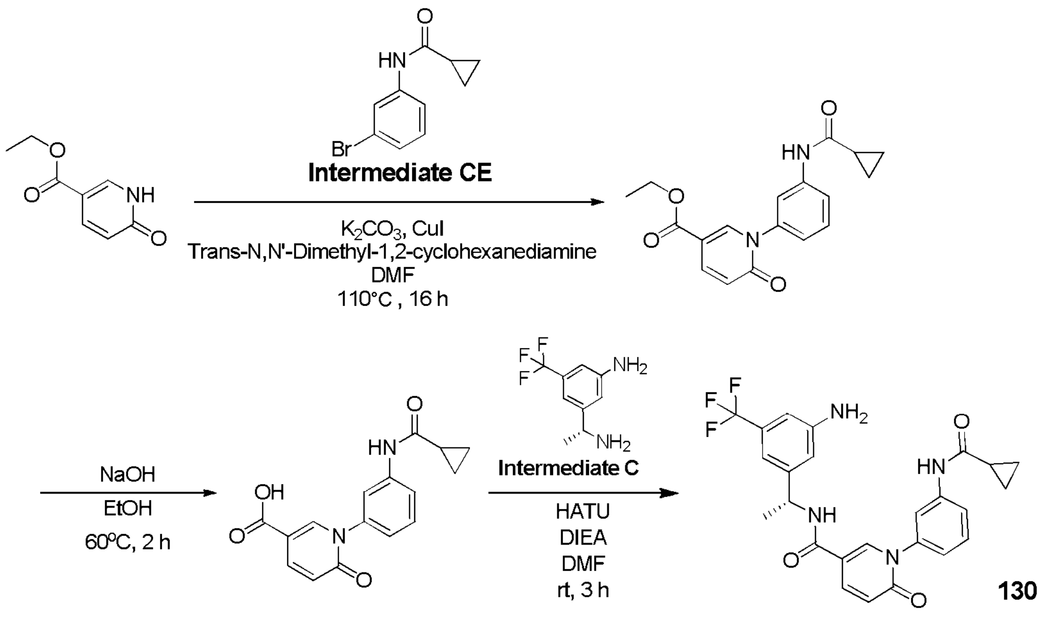


130

The compound of Example 130 (11.2 mg, 45.1%) was obtained in the same manner as in Example 129. $^1$H NMR (400 MHz, MeOD) δ 8.29 (d, J = 2.5 Hz, 1H), 8.04 (dd, J = 9.6, 2.6 Hz, 1H), 7.80 (s, 1H), 7.60 (d, J = 8.2 Hz, 1H), 7.48 (t, J = 8.1 Hz, 1H), 7.15 (d, J = 7.8 Hz, 1H), 6.97 (d, J = 12.2 Hz, 2H), 6.88 (s, 1H), 6.64 (d, J = 9.6 Hz, 1H), 5.14 - 5.08 (m, 1H), 1.51 (d, J = 7.0 Hz, 3H), 0.98 - 0.83 (m, 3H); LC/MS m/z = 485.2 $[M+H]^+$.

**Example 131: N-[(1R)-1-[3-amino-5-(trifluoromethyl)phenyl]ethyl]-1-(3-cyclopropaneamidophenyl)-6-oxo-1,6-dihydropyridine-3-carboxamide**

Steps 1 to 3: Synthesis of 1-(2-acetamido-4-pyridyl)-N-[(1R)-1-[3-nitro-5-(trifluoromethyl)phenyl]ethyl]-6-oxo-pyridine-3-carboxamide

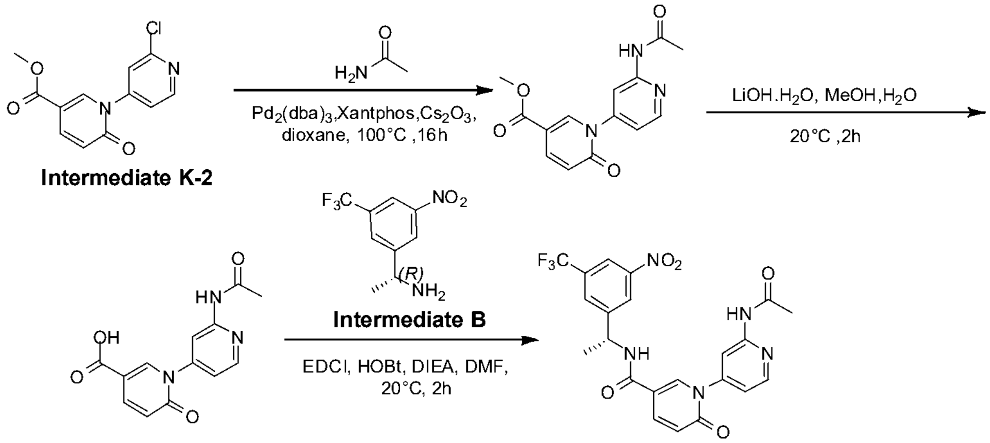


1-(2-acetamido-4-pyridyl)-N-[(1R)-1-[3-nitro-5-(trifluoromethyl)phenyl]ethyl]-6-oxo-pyridine-3-carboxamide (36 mg, 50% yield) was obtained as a white solid in the same manner as in Steps 1 to 3 of Example 79. $^1$H NMR (400 MHz, DMSO-$d_6$) δ = 10.65 (s, 1H), 9.18 (br d, *J* = 7.6 Hz, 1H), 8.72 (s, 1H), 8.58 (s, 1H), 8.41 - 8.33 (m, 2H), 8.30 - 8.25 (m, 2H), 7.86 (s, 1H), 7.29 (d, *J* = 8.4 Hz, 1H), 6.94 - 6.90 (m, 1H),5.44 - 5.36 (m, 1H), 2.07 (s, 3H), 1.55 (d, *J* = 7.2 Hz, 3H).

Step 4: Synthesis of 1-(2-acetamido-4-pyridyl)-N-[(1R)-1-[3-amino-5-(trifluoromethyl)phenyl]ethyl]-6-oxo-pyridine-3-carboxamide

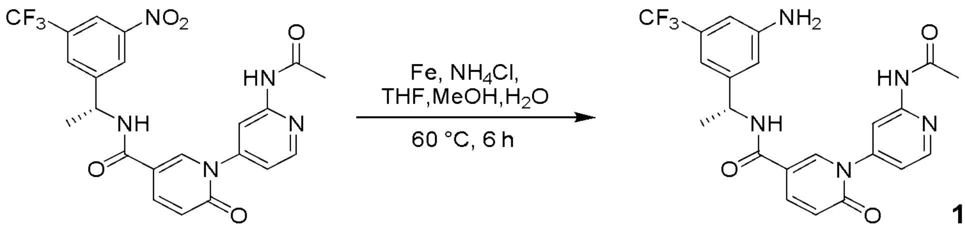


131

The compound of Example 131 (8.79 mg, 30% yield) was obtained as a white solid in the same manner as in Step 4 of Example 89. $^1$H NMR (400 MHz, DMSO-$d_6$) δ = 10.66 (s, 1H), 8.98 (d, *J* = 7.6 Hz, 1H), 8.83 - 8.63 (m, 1H), 8.47 - 8.21 (m, 2H), 7.85 (s, 1H), 7.37 - 7.19 (m, 1H), 7.01 - 6.87 (m, 2H), 6.83 - 6.69 (m, 2H), 5.64 - 5.51 (m, 1H), 5.07 (d, *J* = 7.6, 14.4 Hz, 1H), 2.07 (s, 3H), 1.45 (d, *J* = 7.6 Hz, 3H); MS (EI) m/z: 460.3 [M+H]$^+$.

**Example 132: N-[(1R)-1-[3-amino-5-(trifluoromethyl)phenyl]ethyl]-6-oxo-1-(3-tetrahydrofuran-2-ylphenyl)pyridine-3-carboxamide**

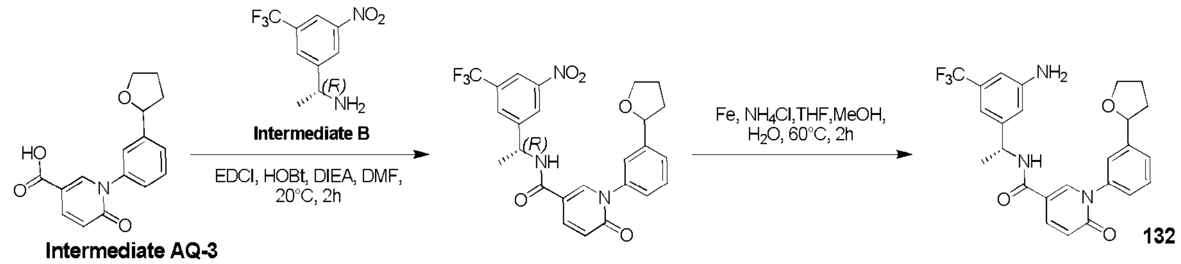


The compound of Example 132 (19.68 mg, 42% yield) was obtained as a white solid in the same manner as in Steps 3 and 4 of Example 89. $^1$H NMR (400 MHz, DMSO-$d_6$) δ = 8.58 (d, *J* = 7.6 Hz, 1H), 8.41 - 8.31 (m, 1H), 8.01 - 7.89 (m, 1H), 7.56 - 7.31 (m, 4H), 6.96 - 6.67 (m, 3H), 6.57 - 6.47 (m, 1H), 5.61 - 5.50 (m, 1H), 5.10 - 4.97 (m, 1H), 4.92 - 4.82 (m, 1H), 4.05 - 3.93 (m, 1H), 3.87 - 3.76 (m, 1H), 2.39 - 2.31 (m, 1H), 1.95 (d, *J* = 7.2 Hz, 2H), 1.76 - 1.63 (m, 1H), 1.39 (d, *J*= 7.2 Hz, 3H); MS (EI) m/z: 472.1 $[M+H]^+$.

**Example 133: N-[(1R)-1-[3-amino-5-(trifluoromethyl)phenyl]ethyl]-6-oxo-1-[3-(2-oxopiperazin-1-yl)phenyl]pyridine-3-carboxamide**

Steps 1 to 3: Synthesis of tert-butyl 4-[3-[5-[[(1R)-1-[3-nitro-5-(trifluoromethyl)phenyl]ethyl]carbamoyl]-2-oxo-1-pyridyl]phenyl]-3-oxo-piperazine-1-carboxylate

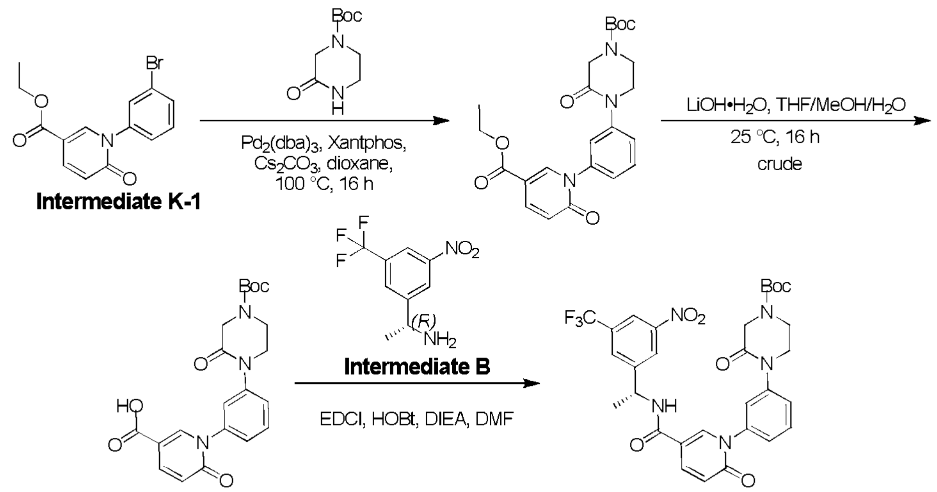


Tert-butyl 4-[3-[5-[[(1R)-1-[3-nitro-5-(trifluoromethyl)phenyl]ethyl]carbamoyl]-2-oxo-1-pyridyl]phenyl]-3-oxo-piperazine-1-carboxylate (70 mg, 44% yield) was obtained as a light yellow solid in the same manner as in Steps 1 to 3 of Example 79. $^1$H NMR (400 MHz, $CDCl_3$) δ = 8.33 (d, *J* = 16.0 Hz, 2H), 8.05 (d, *J* = 2.0 Hz, 1H), 7.88 (s, 1H), 7.76 - 7.69 (m, 1H), 7.47 - 7.39 (m, 1H), 7.35 (s, 1H), 7.31 - 7.26 (m, 1H), 7.20 (s, 1H), 7.19 - 7.17 (m, 1H), 6.47 (d, *J* = 9.6 Hz, 1H), 5.32 - 5.20 (m, 1H), 4.16 (s, 2H), 3.72 (s, 4H), 1.50 (d, *J* = 6.4 Hz, 3H), 1.43 (s, 9H); MS (EI) m/z: 630.3 $[M+H]^+$.

Step 4: Synthesis of tert-butyl 4-[3-[5-[[(1R)-1-[3-amino-5-(trifluoromethyl)phenyl]ethyl]carbamoyl]-2-oxo-1-pyridyl]phenyl]-3-oxo-piperazine-1-carboxylate

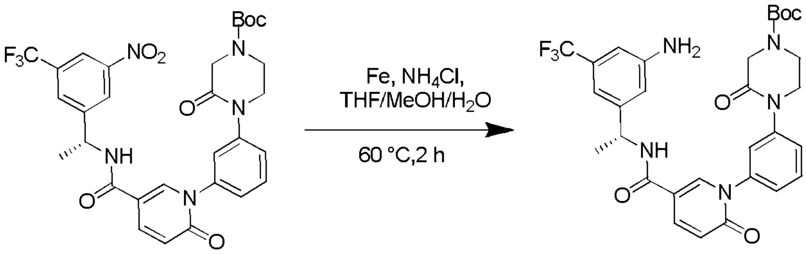


Tert-butyl 4-[3-[5-[[(1R)-1-[3-amino-5-(trifluoromethyl)phenyl]ethyl]carbamoyl]-2-oxo-1-pyridyl]phenyl]-3-oxo-piperazine-1-carboxylate (60 mg, 86% yield) was obtained as a yellow solid in the same manner as in Step 4 of Example 89. $^1$H NMR (400 MHz, $CDCl_3$) $\delta$ = 8.07 (s, 1H), 7.77 (d, *J* = 8.0 Hz, 1H), 7.58 - 7.47 (m, 1H), 7.40 (s, 1H), 7.34 (d, *J* = 7.2 Hz, 1H), 7.29 (s, 1H), 6.97 (s, 1H), 6.82 (d, *J* = 15.2 Hz, 2H), 6.58 (d, *J* = 9.6 Hz, 1H), 5.19 (t, *J* = 7.2 Hz, 1H), 4.23 (s, 2H), 3.78 (s, 4H), 1.53 (s, 3H), 1.51 (s, 9H).

Step 5: Synthesis of N-[(1R)-1-[3-amino-5-(trifluoromethyl)phenyl]ethyl]-6-oxo-1-[3-(2-oxopiperazin-1-yl)phenyl]pyridine-3-carboxamide

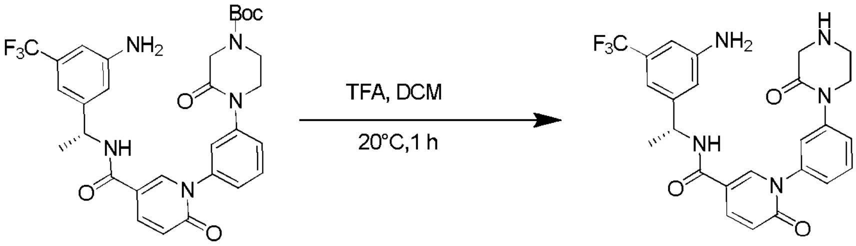

**133**

The compound of Example 133 (5.06 mg, 19% yield) was obtained as an off-white solid in the same manner as in Step 5 of Example 79. $^1$H NMR (400 MHz, DMSO-$d_6$) $\delta$ = 8.61 (d, *J* = 7.6 Hz, 1H), 8.37 (d, *J* = 2.4 Hz, 1H), 8.19 - 8.13 (m, 1H), 7.96 (dd, *J* = 2.4, 9.6 Hz, 1H), 7.59 - 7.53 (m, 1H), 7.52 - 7.45 (m, 2H), 7.37 (d, *J* = 8.0 Hz, 1H), 6.76 (s, 2H), 6.70 (s, 1H), 6.54 (d, *J* = 9.6 Hz, 1H), 5.55 (s, 2H), 5.04 - 4.97 (m, 1H), 3.66 (t, *J* = 5.4 Hz, 2H), 3.42 (s, 2H), 3.04 (t, *J* = 5.3 Hz, 2H), 1.39 (d, *J*= 7.2 Hz, 3H); MS (EI) m/z: 500.2 $[M+H]^+$.

**Example 134: N-[(1R)-1-[3-amino-5-(trifluoromethyl)phenyl]ethyl]-1-[3-[(3-methyloxetan-3-yl)amino]phenyl]-6-oxo-pyridine-3-carboxamide**

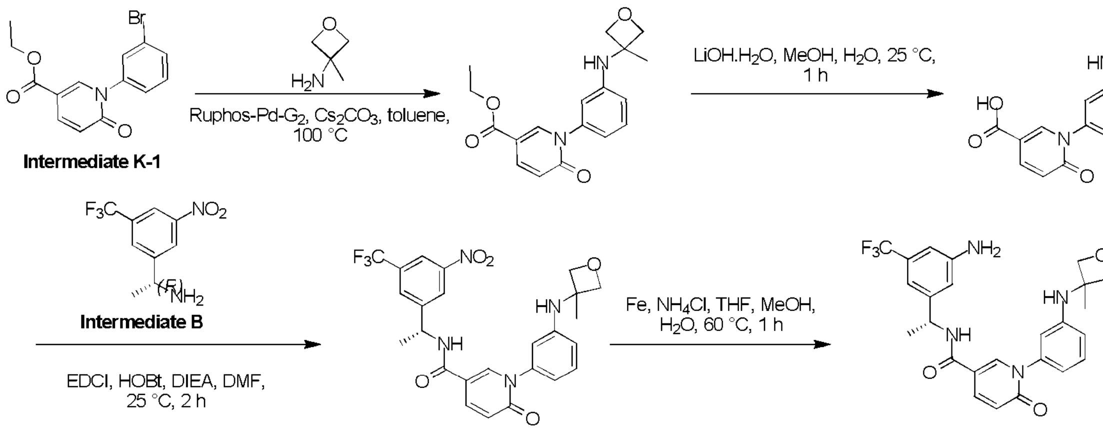

**134**

The compound of Example 134 (25.4 mg, 88% yield) was obtained as a white solid in a similar manner to Example 90, except that 3-methyloxetan-3-amine was used as a starting material and Step 4 of Example 90 was omitted. $^1$H NMR (400 MHz, DMSO-$d_6$) $\delta$ =8.69 - 8.50 (m, 1H), 8.39 - 8.32 (m, 1H), 7.96 - 7.90 (m, 1H),7.28 - 7.20 (m, 1H), 6.75 (s, 1H), 6.69

(s, 1H), 6.68 - 6.50 (m, 1H), 6.49 (d, $J$ = 9.6 Hz, 1H), 6.46 (s, 1H), 6.43 - 6.38 (m, 2H), 5.60 - 5.47 (m, 2H), 5.08 - 4.94 (m, 1H), 4.60 (d, $J$ = 5.6 Hz, 2H), 4.48 (d, $J$ = 5.6 Hz, 2H), 1.57 (s, 3H), 1.39 (d, $J$ = 7.2 Hz, 3H); MS (EI) m/z: 487.2 $[M+H]^+$.

**Example 135: N-[(1R)-1-[3-amino-5-(trifluoromethyl)phenyl]ethyl]-1-[3-[(3-methyloxetan-3-yl)amino]phenyl]-6-oxo-pyridine-3-carboxamide**

Step 1: Synthesis of ethyl-6-oxo-1-[3-(2-trimethylsilylethynyl)phenyl]pyridine-3-carboxylate

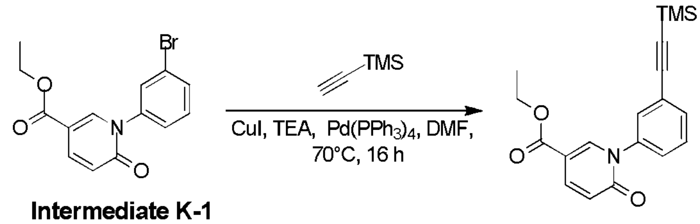


**Intermediate K-1**

A solution of Intermediate K-1 (400 mg, 1.24 mmol), CuI (23.6 mg, 124 μmol), TEA (37.6 mg, 372 μmol), $Pd(PPh_3)_4$ (143 mg, 124 μmol) and ethynyl(trimethyl)silane (182 mg, 1.86 mmol) in DMF (8 mL) was stirred at 70°C for 16 h under $N_2$ atmosphere. The reaction mixture was quenched with water (10 mL) and extracted with PE (3 x 50 mL). The combined organic layer was washed with saturated NaCl solution (2 x 50 mL) and concentrated in vacuo. The residue was purified by column chromatography ($SiO_2$, petroleum ether/ethyl acetate = 1/0 to 3/1) to give ethyl-6-oxo-1-[3-(2-trimethylsilylethynyl)phenyl]pyridine-3-carboxylate (400 mg, 94% yield) as yellow oil. $^1H$ NMR (400 MHz, DMSO-$d_6$) δ = 8.25 - 8.28 (m, 1H), 7.85 - 7.90 (m, 1H), 7.44 - 7.59 (m, 4H), 6.53 - 6.57 (m, 1H), 4.25 (q, $J$ = 7.2 Hz, 2H), 1.27 (t, $J$ = 7.2 Hz, 3H), 0.23 (s, 9H).

Step 2: Synthesis of ethyl-1-(3-ethynylphenyl)-6-oxo-pyridine-3-carboxylate

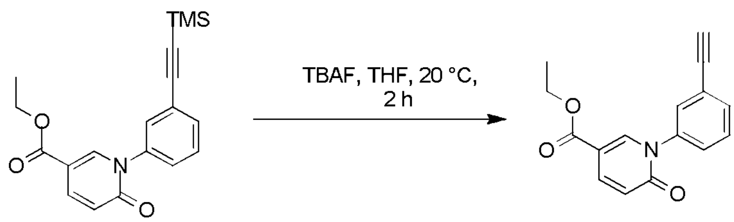


To a solution of ethyl-6-oxo-1-[3-(2-trimethylsilylethynyl)phenyl]pyridine-3-carboxylate (480 mg, 1.41 mmol) in THF (10 mL) was added TBAF (1 M, 3.54 mL). The reaction mixture was stirred at 20°C for 2 h. The reaction mixture was filtered, and the filtrate was concentrated in vacuo. The residue was purified by column chromatography ($SiO_2$, petroleum ether/ethyl acetate = 1/0 to 3/1) to give ethyl-1-(3-ethynylphenyl)-6-oxo-pyridine-3-carboxylate (260 mg, 68% yield) as a yellow solid. $^1H$ NMR (400 MHz, DMSO-$d_6$) δ = 8.26 - 8.29 (m, 1H), 7.80 - 7.98 (m, 1H), 7.69 - 7.46 (m, 4H), 6.56 (d, $J$ = 9.6 Hz, 1H), 4.34 (s, 1H), 4.20 - 4.30 (m, 2H), 1.20 - 1.34 (m, 3H).

Step 3: Synthesis of ethyl-6-oxo-1-[3-(1H-triazol-5-yl)phenyl]pyridine-3-carboxylate

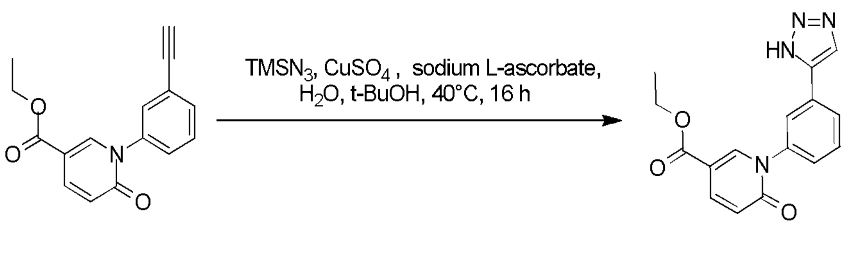

A solution of ethyl-1-(3-ethynylphenyl)-6-oxo-pyridine-3-carboxylate (200 mg, 748 μmol), $TMSN_3$ (344 mg, 2.99 mmol), sodium;(2R)-2-[(1S)-1,2-dihydroxyethyl]-4-hydroxy-5-oxo-2H-furan-3-olate (59.3 mg, 299 μmol) and $CuSO_4$ (23.8 mg, 149 μmol) in a mixture of t-BuOH (3 mL) and $H_2O$ (3 mL) was stirred at 40 °C for 16 h. The reaction mixture was concentrated in vacuo. The residue was purified by reversed-phase HPLC (0.1% FA condition) to give ethyl-6-oxo-1-[3-(1H-triazol-5-yl)phenyl]pyridine-3-carboxylate (40 mg, 17% yield) as a white solid. $^1H$ NMR (400 MHz, DMSO-$d_6$) δ = 8.43 (s, 1H), 8.32 (d, *J* = 2.4 Hz, 1H), 7.99 (d, *J* = 8.4 Hz, 1H), 7.87 - 7.96 (m, 2H), 7.60 - 7.64 (m, 1H), 7.40 - 7.48 (m, 1H), 6.58 (d, *J* = 9.6 Hz, 1H), 4.30 - 4.20 (m, 2H), 1.32 - 1.20 (m, 3H).

Steps 4 to 6: Synthesis of N-[(1R)-1-[3-amino-5-(trifluoromethyl)phenyl]ethyl]-6-oxo-1-[3-(1H-triazol-5-yl)phenyl]pyridine-3-carboxamide

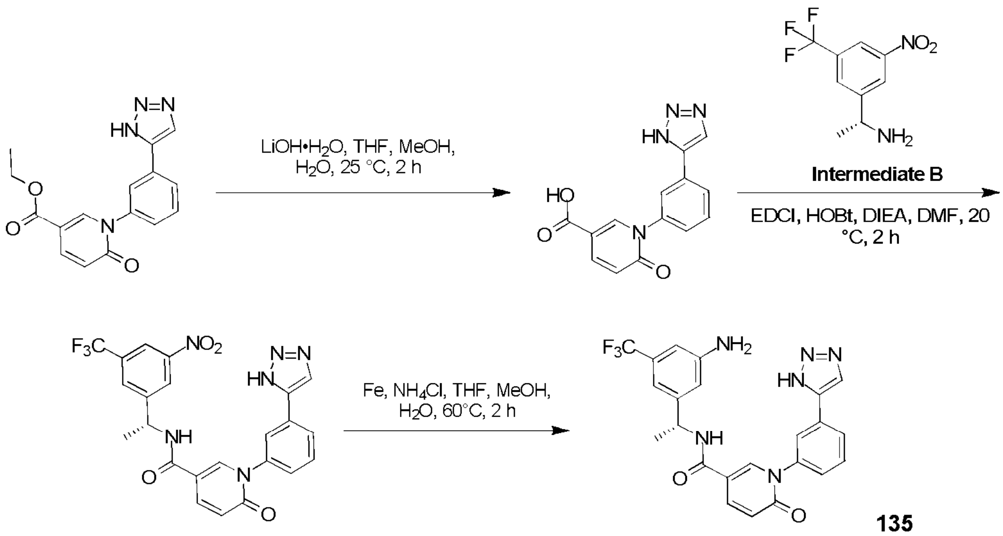


135

The compound of Example 135 (1.31 mg, 12% yield) was obtained as a white solid in the same manner as in Steps 2 to 4 of Example 89. $^1H$ NMR (400 MHz, $CDCl_3$) δ = 8.40 - 8.58 (m, 1H), 8.35 (d, *J* = 2.4 Hz, 1H), 8.20 - 8.30 (m, 1H), 8.00 - 8.14 (m, 1H), 8.03 - 8.00 (m, 1H), 7.96 (d, *J* = 1.6 Hz, 1H), 7.67 - 7.63 (m, 1H), 7.49 - 7.45 (m, 1H), 7.36 - 7.32 (m, 1H), 6.89 (d, *J* = 2.4 Hz, 2H), 6.80 (s, 1H), 6.68 (d, *J* = 9.6 Hz, 1H), 5.07 - 5.18 (m, 1H), 1.51 (d, *J* = 7.6 Hz, 3H); MS (EI) m/z: 469.3 $[M+H]^+$.

**Example 136: 1-[3-[acetyl(methyl)amino]phenyl]-N-[(1R)-1-[3-amino-5-(trifluoromethyl)phenyl]ethyl]-6-oxo-pyridine-3-carboxamide**

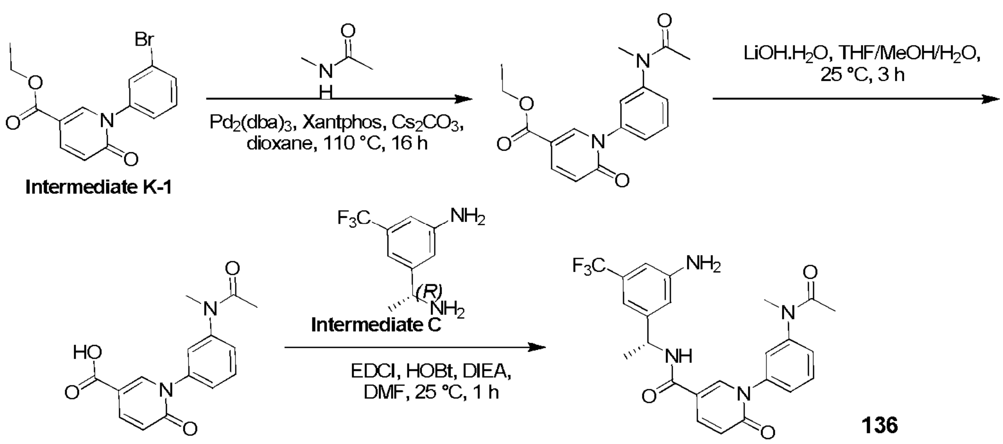


136

The compound of Example 136 (13.3 mg, 13% yield) was obtained as an off-white solid in the same manner as in

Steps 1 to 3 of Example 79. $^1$H NMR (400 MHz, DMSO-$d_6$) δ = 8.60 (d, *J* = 7.6 Hz, 1H), 8.36 (d, *J* = 2.4 Hz, 1H), 7.96 (dd, *J* = 2.4, 9.6 Hz, 1H), 7.65 - 7.40 (m, 4H), 6.78 (s, 2H), 6.72 (s, 1H), 6.55 (d, *J* = 9.6 Hz, 1H), 5.07 - 4.96 (m, 1H), 3.20 (s, 3H), 1.87 (s, 3H), 1.40 (d, *J* = 7.2 Hz, 3H); MS (EI) m/z: 473.2 [M+H]$^+$.

**Example 137: N-[(1R)-1-[5-[5-chloro-2-(methylaminomethyl)phenyl]thiazol-2-yl]ethyl]-1-(2-fluorophenyl)-6-oxo-pyridazine-3-carboxamide**

Step 1: Synthesis of N-[(1R)-1-(5-bromothiazol-2-yl)ethyl]-1-(2-fluorophenyl)-6-oxo-pyridazine-3-carboxamide

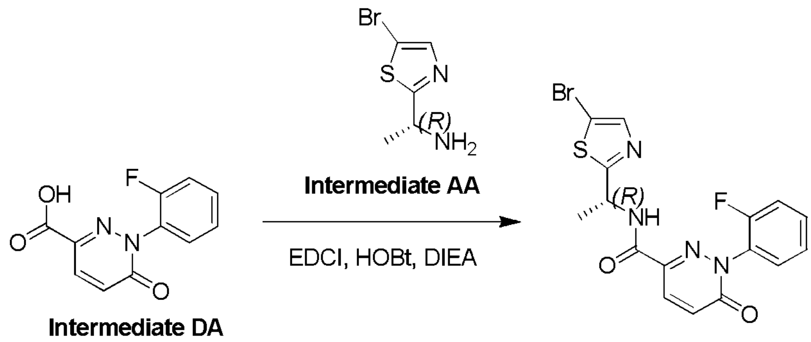


To a solution of Intermediate DA (48.1 mg, 205 μmol) in DMF (1 mL), DIPEA (79.6 mg, 616 μmol), EDCI (47.2 mg, 246 μmol) and HOBt (33.3 mg, 246 μmol) were added. The mixture was stirred at 20 °C for 10 min. Then, the mixture was added with Intermediate AA (50 mg, 205 μmol, HCl) and stirred at 20 °C for 15 h 50 min. The reaction mixture was diluted with $H_2O$ (10 mL) and extracted with EtOAc (3 x 5 mL). The combined organic layer was washed with $H_2O$ (3 x 5 mL), dried over $Na_2SO_4$, filtered and concentrated under reduced pressure to obtain a residue. The residue was purified by Prep-TLC ($SiO_2$, PE: EtOAc = 1: 3) and reversed-phase HPLC (0.1% FA condition) to give N-[(1R)-1-(5-bromothiazol-2-yl) ethyl]-1-(2-fluorophenyl)-6-oxo-pyridazine-3-carboxamide (50 mg, 57% yield) as a light yellow solid. $^1$H NMR (400 MHz, $CDCl_3$) δ = 8.07 (d, *J* = 9.6 Hz, 1H), 7.60 (s, 1H), 7.55 - 7.44 (m, 3H), 7.36 - 7.28 (m, 2H), 7.14 (d, *J* = 9.6 Hz, 1H), 5.52 - 5.43 (m, 1H), 1.69 (d, *J*= 7.2 Hz, 3H); MS (EI) m/z: 425.0 [M+H]$^+$.

Step 2: Synthesis of N-[(1R)-1-[5-(5-chloro-2-formyl-phenyl)thiazol-2-yl]ethyl]-1-(2-fluorophenyl)-6-oxo-pyridazine-3 -carboxamide

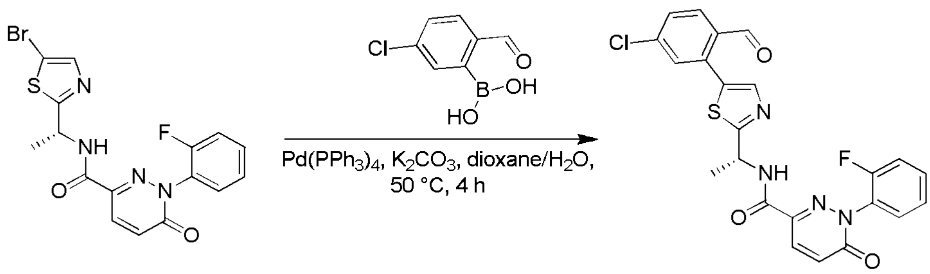


A mixture of N-[(1R)-1-(5-bromothiazol-2-yl)ethyl]-1-(2-fluorophenyl)-6-oxo-pyridazine-3-carboxamide (30 mg, 70.9 μmol), (5-chloro-2-formyl-phenyl)boronic acid (11.8 mg, 63.8 μmol), $K_2CO_3$ (29.4 mg, 213 μmol) and $Pd(PPh_3)_4$ (8.19 mg, 7.09 μmol) in dioxane (1.5 mL) and $H_2O$ (0.5 mL) was degassed and purged with $N_2$ for 3 times, and then the mixture was stirred at 50 °C for 4 h under $N_2$ atmosphere. The reaction mixture was diluted with $H_2O$ (10 mL) and extracted with EtOAc (3 x 5 mL). The combined organic layer was washed with $H_2O$ (3 x 5 mL ), dried over $Na_2SO_4$, filtered and concentrated under reduced pressure to obtain a residue. The residue was purified by Prep-TLC ($SiO_2$, PE: EtOAc = 1: 1) to give N-[(1R)-1-[5-(5-chloro-2-formyl-phenyl)thiazol-2-yl]ethyl]-1-(2-fluorophenyl)-6-oxo-pyridazine-3-carboxamide (30 mg, 84% yield) as a light yellow solid. $^1$H NMR (400 MHz, $CDCl_3$) δ = 10.11 (s, 1H), 8.09 (d, *J* = 10.0 Hz, 1H), 7.97 (d, *J* = 8.4 Hz, 1H), 7.67 - 7.62 (m, 2H), 7.54 - 7.52 (m, 1H), 7.50 (d, *J* = 1.6 Hz, 1H), 7.49 - 7.47 (m, 2H), 7.39 - 7.29 (m, 2H), 7.17 - 7.13 (m, 1H), 5.66 - 5.55 (m, 1H), 1.78 (d, *J* = 7.2 Hz, 3H); MS (EI) m/z: 483.0 [M+H]$^+$.

Step 3: Synthesis of N-[(1R)-1-[5-[5-chloro-2-(methylaminomethyl)phenyl]thiazol-2-yl]ethyl]-1-(2-fluorophenyl)-6-oxo-pyridazine-3-carboxamide

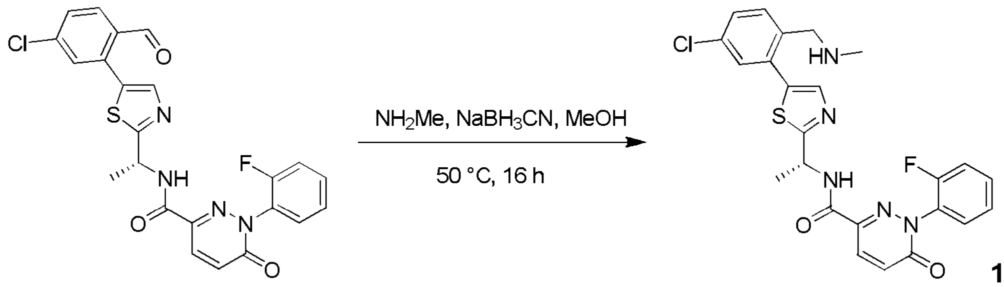


137

To a solution of N-[(1R)-1-[5-(5-chloro-2-formyl-phenyl)thiazol-2-yl]ethyl]-1-(2-fluorophenyl)-6-oxo-pyridazine-3-carboxamide (25 mg, 51.8 μmol) in MeOH (0.5 mL), $MeNH_2$ (2 M, 6.76 mL, THF solution) and $NaBH_3CN$ (6.51 mg, 103 μmol) were added. The mixture was stirred at 50 °C for 16 h. The reaction mixture was concentrated under reduced pressure to obtain a residue. The residue was purified by Prep-HPLC (column: Waters Xbridge 150*25 mm* 5 μm; mobile phase: [water($NH_4HCO_3$)-ACN];B%: 36%-66%,8 min) to give the compound of Example 137 (7.25 mg, 27% yield) as yellow gum. $^1H$ NMR (400 MHz, $CDCl_3$) δ = 8.10 (d, *J* = 9.6 Hz, 1H), 7.76 (s, 1H), 7.66 (d, *J* = 8.4 Hz, 1H), 7.55 - 7.46 (m, 2H), 7.44 - 7.40 (m, 1H), 7.38 - 7.28 (m, 4H), 7.14 (d, *J* = 9.6 Hz, 1H), 5.64 - 5.55 (m, 1H), 3.71 (s, 2H), 2.41 (s, 3H), 1.76 (d, *J* = 6.8 Hz, 3H); MS (EI) m/z: 498.1 $[M+H]^+$.

**Example 138: N-[(1R)-1-[5-[5-chloro-2-(methylaminomethyl)phenyl]thiazol-2-yl]ethyl]-1-(2-fluorophenyl)-6-oxo-pyridine-3-carboxamide**

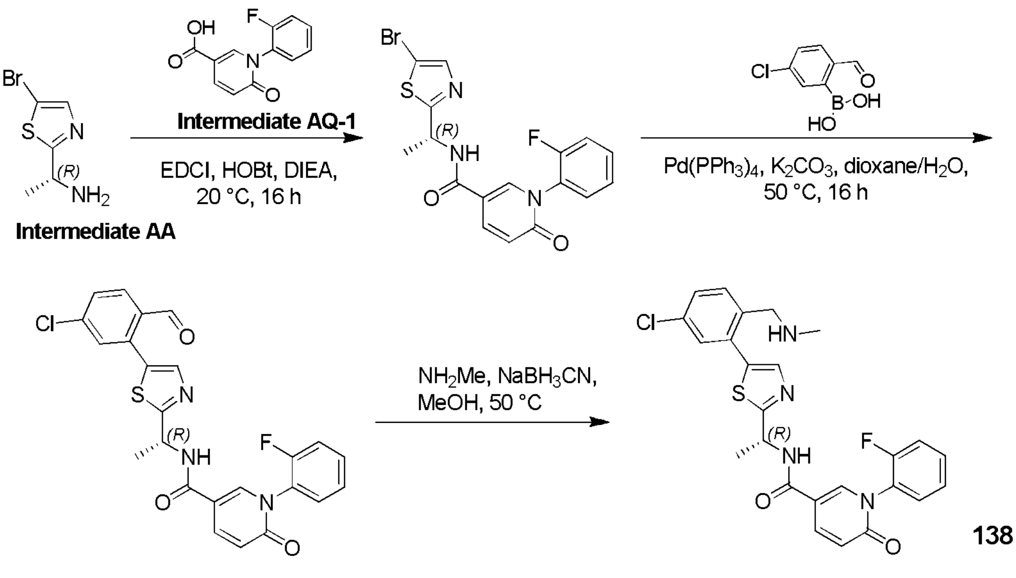


138

The compound of Example 138 (7.64 mg, 49% yield) was obtained as yellow gum in the same manner as in Example 137 using Intermediate AQ-1. $^1H$ NMR (400 MHz, $CDCl_3$) δ = 8.08 (d, *J* = 2.4 Hz, 1H), 7.82 - 7.78 (m, 1H), 7.76 (s, 1H), 7.51 - 7.44 (m, 1H), 7.42 - 7.38 (m, 1H), 7.38 - 7.28 (m, 4H), 7.26 - 7.24 (m, 1H), 7.03 (d, *J* = 7.6 Hz, 1H), 6.67 (d, *J* = 9.6 Hz, 1H), 5.64 - 5.53 (m, 1H), 3.69 (s, 2H), 2.41 (s, 3H), 1.73 (d, *J* = 6.8 Hz, 3H); MS (EI) m/z: 497.1 $[M+H]^+$.

**Example 139: N-[(1R)-1-[3-amino-5-(trifluoromethyl)phenyl]ethyl]-1-(2-fluoro-3,4-dimethoxy-phenyl)-6-oxo-pyridine-3-carboxamide**

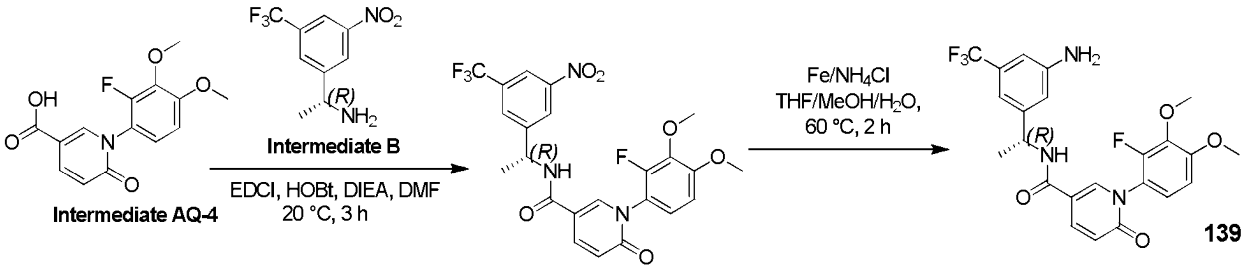

139

The compound of Example 139 (6.97 mg, 28% yield) was obtained as a white solid in the same manner as in Example 132. $^{1}$H NMR (400 MHz, DMSO-$d_6$) δ = 8.55 (d, *J* = 7.6 Hz, 1H), 8.31 (d, *J* = 2.4 Hz, 1H), 7.97 (dd, *J* = 2.4, 9.7 Hz, 1H), 7.27 (t, *J* = 8.4 Hz, 1H), 7.05 (dd, *J* = 1.6, 9.2 Hz, 1H), 6.75 (s, 2H), 6.70 (s, 1H), 6.54 (d, *J* = 9.6 Hz, 1H), 5.55 (s, 2H), 5.03 - 4.97 (m, 1H), 3.90 (s, 3H), 3.84 (s, 3H), 1.39 (d, *J* = 7.2 Hz, 3H); MS (EI) m/z: 480.2 [M+H]$^+$.

**Example 140: N-[(1R)-1-[3-(1,1-difluoro-2-hydroxy-ethyl)-2-fluoro-phenyl]ethyl]-1-(2-fluoro-3,4-dimethoxy-phenyl)-6-oxo-pyridine-3-carboxamide**

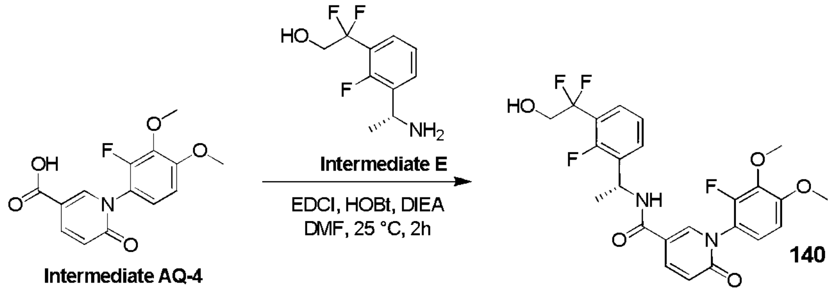

140

The compound of Example 140 (9.12 mg, 14% yield) was obtained as a white solid in the same manner as in Step 1 of Example 139 by replacing Intermediate B with Intermediate E. $^{1}$H NMR (400 MHz, DMSO-$d_6$) δ = 8.68 (d, *J* = 7.2 Hz, 1H), 8.37 (d, *J*= 2.4 Hz, 1H), 7.97 (dd, *J* = 2.4, 9.6 Hz, 1H), 7.58 (t, *J* = 6.8 Hz, 1H), 7.44 (t, *J* = 6.8 Hz, 1H), 7.32 - 7.26 (m, 2H), 7.07 (dd, *J* = 1.6, 9.2 Hz, 1H), 6.55 (d, *J* = 9.6 Hz, 1H), 5.73 (t, *J* = 6.4 Hz, 1H), 5.37 - 5.32 (m, 1H), 3.96 - 3.88 (m, 5H), 3.84 (s, 3H), 1.44 (d, *J* = 7.2 Hz, 3H); MS (EI) m/z: 495.2 [M+H]$^+$.

**Example 141: N-[(1R)-1-[3-(1,1-difluoro-2-hydroxy-ethyl)-2-fluorophenyl]ethyl]-1-(2-fluoro-3-pyridyl)-6-oxo-pyridazine-3-carboxamide**

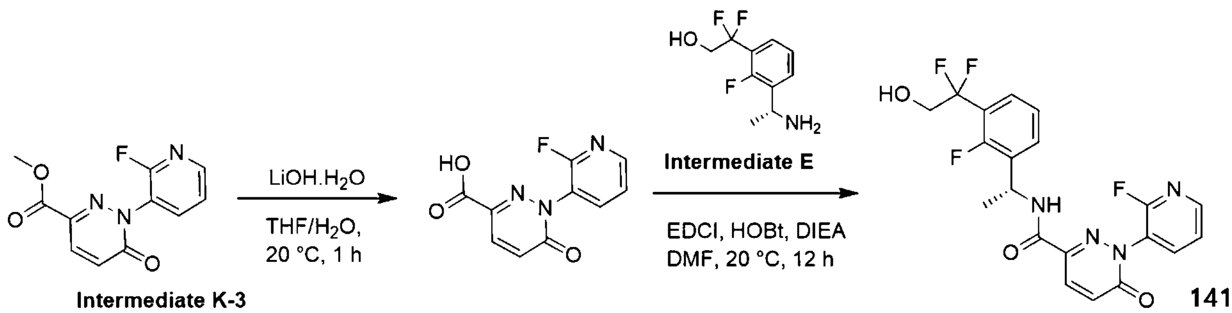

141

The compound of Example 141 (18.8 mg 33.77% yield) was obtained as a white solid in the same manner as in Example 76. $^{1}$H NMR (400MHz, DMSO-$d_6$) δ = 9.07 (d, *J* = 8.0 Hz, 1H), 8.42 (d, *J* = 4.4 Hz, 1H), 8.38 - 8.29 (m, 1H), 7.94 (d, *J* = 9.6 Hz, 1H), 7.68 - 7.57 (m, 2H), 7.43 (br t, *J* = 6.8 Hz, 1H), 7.30 - 7.24 (m, 1H), 7.21 (d, *J* = 10.0 Hz, 1H), 6.06 (br s, 1H), 5.40 (quin, *J* = 7.2 Hz, 1H), 3.91 (br t, *J* = 14.4 Hz, 2H), 1.46 (d, *J* = 7.2 Hz, 3H); MS (ESI) m/z = 437.3 [M+H]$^+$.

**Example 142 to Example 214**

The compounds shown in the following table were prepared by using appropriate starting materials and intermediates corresponding to the respective structures of the desired compounds based on Example 141.

[Table 11]

| No. | Structure/Name | Spectral Data |
|---|---|---|
| 142 | 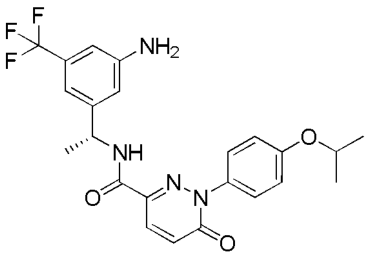 N-[(1R)-1-[3-amino-5-(trifluoromethyl)phenyl]ethyl]-1-(4-isopropoxyphenyl)-6-oxo-pyridazine-3-carboxamide | $^1$H NMR (400MHz, DMSO-$d_6$) $\delta$ = 8.83 (d, $J$ = 8.2 Hz, 1H), 7.87 (d, $J$ = 9.6 Hz, 1H), 7.60 - 7.51 (m, 2H), 7.11 (d, $J$ = 9.6 Hz, 1H), 7.06 - 7.00 (m, 2H), 6.80 (d, $J$ = 15.2 Hz, 2H), 6.70 (s, 1H), 5.55 (s, 2H), 5.03 (quin, $J$ = 7.2 Hz, 1H), 4.69 (td, $J$ = 6.0, 12.1 Hz, 1H), 1.44 (d, $J$ = 7.2 Hz, 3H), 1.30 (d, $J$ = 6.0 Hz, 6H); MS (ESI) m/z = 461.3 [M +H]$^+$. |
| 143 | 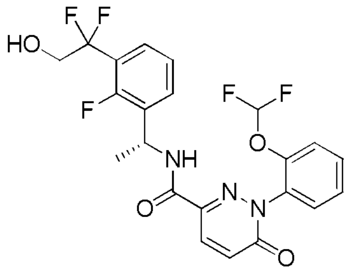 N-[(1R)-1-[3-(1,1-difluoro-2-hydroxy-ethyl)-2-fluoro-phenyl] ethyl]-1-[2-(difluoromethoxy)phenyl]-6-oxo-pyridazine-3-carboxamide | $^1$H NMR (400 MHz, DMSO-$d_6$) $\delta$ = 8.97 (br d, $J$ = 8.0 Hz, 1H), 7.93 (d, $J$ = 10.0 Hz, 1H), 7.85-7.57 (m, 1H), 7.65 - 7.57 (m, 2H), 7.49 - 7.40 (m, 3H), 7.35 (s, 0.25H), 7.29 - 7.22 (m, 1H), 7.19 (s, 0.5H), 7.17 (d, $J$ = 4.0 Hz, 1H), 6.99 (s, 0.25H), 5.71 (s, 1H), 5.40 (quin, $J$ = 7.2 Hz, 1H), 3.91 (br t, $J$ = 14.4 Hz, 2H), 1.45 (d, $J$ = 7.2 Hz, 3H); LC/MS (ESI) m/z: 484.3 [M+H]$^+$ |
| 144 | 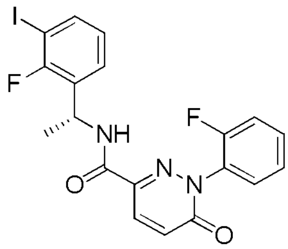 N-[(1R)-1-(2-fluoro-3-iodo-phenyl)ethyl]-1-(2-fluorophenyl)-6-oxo-pyridazine-3-carboxamide | $^1$H NMR (400 MHz, DMSO-$d_6$) $\delta$ = 9.02 (d, $J$ = 8.0 Hz, 1H), 7.91 (d, $J$ = 10.0 Hz, 1H), 7.75 - 7.64 (m, 2H), 7.64 - 7.55 (m, 1H), 7.49 - 7.39 (m, 3H), 7.17 (d, J = 9.8 Hz, 1H), 6.97 (t, $J$ = 7.6 Hz, 1H), 5.32 (quin, $J$ = 7.2 Hz, 1H), 1.44 (d, $J$ = 7.2 Hz, 3H); LC/MS (ESI) m/z: 482.3 [M+H]$^+$ |
| 145 | 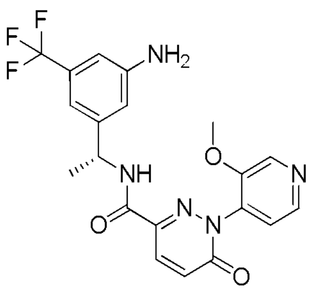 N-[(1R)-1-[3-amino-5-(trifluoromethyl)phenyl]ethyl]-1-(3-methoxy-4-pyridyl)-6-oxo-pyridazine-3-carboxamide | $^1$H NMR (400 MHz, DMSO-$d_6$) $\delta$ = 8.88 (d, $J$ = 8.4 Hz, 1H), 8.63 (s, 1H), 8.41 (d, $J$ = 4.8 Hz, 1H), 7.92 (d, $J$ = 10.0 Hz, 1H), 7.58 (d, $J$ = 5.2 Hz, 1H), 7.15 (d, $J$ = 10.0 Hz, 1H), 6.78 (br d, $J$ = 13.6 Hz, 2H), 6.69 (s, 1H), 5.54 (s, 2H), 5.02 (quin, $J$ = 7.2 Hz, 1H), 3.90 (s, 3H), 1.42 (d, $J$ = 7.2 Hz, 3H) ; LC/MS (ESI) m/z: 434.3 [M+H]$^+$ |

(continued)

| No. | Structure/Name | Spectral Data |
|---|---|---|
| 146 | 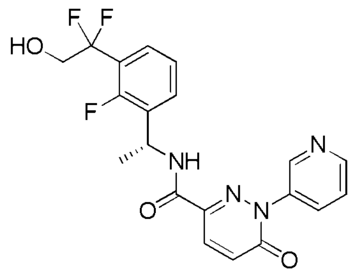 N-[(1R)-1-[3-(1,1-difluoro-2-hydroxy-ethyl)-2-fluoro-phenyl]ethyl]-6-oxo-1-(3-pyridyl)pyridazine-3-carboxamide | $^1$H NMR (400 MHz, DMSO-$d_6$) $\delta$ = 9.05 (d, *J* = 8.0 Hz, 1H), 8.97 (d, *J* = 2.4 Hz, 1H), 8.66 (d, *J* = 4.8 Hz, 1H), 8.23 - 8.16 (m, 1H), 7.90 (d, *J* = 9.6 Hz, 1H), 7.69 - 7.57 (m, 2H), 7.43 (t, *J* = 7.2 Hz, 1H), 7.33 - 7.25 (m, 1H), 7.18 (d, *J* = 9.6 Hz, 1H), 5.72 (t, *J* = 6.4 Hz, 1H), 5.42 (quin, *J* = 7.2 Hz, 1H), 3.92 (dt, *J* = 6.2, 14.4 Hz, 2H), 1.48 (d, *J* = 7.2 Hz, 3H) ; LC/MS (ESI) m/z: 419.3 [M+H]$^+$ |
| 147 | 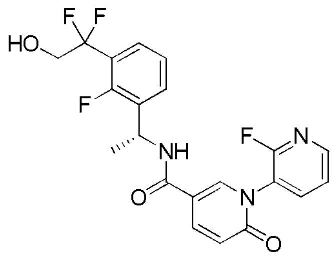 N-[(1R)-1-[3-(1,1-difluoro-2-hydroxy-ethyl)-2-fluoro-phenyl] ethyl]-1-(2-fluoro-3-pyridyl)-6-oxo-pyridine-3-carboxamide | $^1$H NMR (400 MHz, DMSO-$d_6$) $\delta$ = 9.03 (d, *J* = 7.6 Hz, 1H), 8.60 (d, *J* = 2.0 Hz, 1H), 8.35 (dd, *J* = 2.4, 8.4 Hz, 1H), 8.14 (td, *J* = 1.6, 4.8 Hz, 1H), 7.98 (ddd, *J* = 1.6, 8.0, 9.6 Hz, 1H), 7.62 (t, *J* = 6.8 Hz, 1H), 7.48 (ddd, *J* = 0.8, 4.8, 7.6 Hz, 1H), 7.43 (t, *J* = 6.8 Hz, 1H), 7.34 - 7.25 (m, 2H), 5.71 (t, *J* = 6.4 Hz, 1H), 5.40 (quin, *J* = 7.2 Hz, 1H), 3.92 (dt, *J* = 6.4, 14.4 Hz, 2H), 1.47 (d, *J* = 7.2 Hz, 3H); LC/MS (ESI) m/z: 436.6 [M+H]$^+$ |
| 148 | 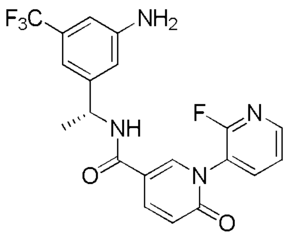 N-[(1R)-1-[3-amino-5-(trifluoromethyl)phenyl]ethyl]-1-(2-fluoro-3-pyridyl)-6-oxo-pyridine-3-carboxamide | $^1$H NMR (400 MHz, DMSO-$d_6$) $\delta$ = 8.90 (d, *J* = 7.6 Hz, 1H), 8.59 (d, *J* = 2.0 Hz, 1H), 8.34 (dd, *J* = 2.4, 8.4 Hz, 1H), 8.17 - 8.10 (m, 1H), 7.98 (ddd, *J* = 1.6, 8.0, 9.6 Hz, 1H), 7.52 - 7.44 (m, 1H), 7.31 (d, *J* = 8.4 Hz, 1H), 6.79 (s, 2H), 6.70 (s, 1H), 5.56 (s, 2H), 5.05 (quin, *J* = 7.2 Hz, 1H), 1.43 (d, *J* = 7.2 Hz, 3H) ; LC/MS (ESI) m/z: 421.3 [M+H]$^+$ |
| 149 | 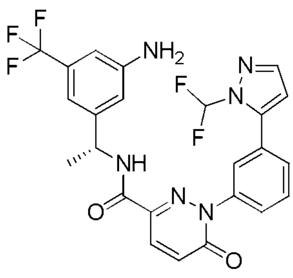 N-[(1R)-1-[3-amino-5-(trifluoromethyl)phenyl]ethyl]-1-[3-[2-(difluoromethyl)pyrazol-3-yl]phenyl]-6-oxo-pyridazine-3-carboxamide | $^1$H NMR (400 MHz, DMSO-$d_6$) $\delta$ = 8.90 (d, *J* = 8.4 Hz, 1H), 7.96 (s, 1H), 7.93 - 7.83 (m, 4H), 7.81 (s, 1H), 7.71 (t, *J* = 8.0 Hz, 1H), 7.67 (s, 1H), 7.61 (d, *J* = 7.6 Hz, 1H), 7.18 (d, *J* = 10.0 Hz, 1H), 6.80 (d, *J* = 12.4 Hz, 2H), 6.75 (d, *J* = 1.6 Hz, 1H), 6.70 (s, 1H), 5.55 (s, 2H), 5.04 (t, *J* = 7.2 Hz, 1H), 1.45 (d, *J* = 7.2 Hz, 3H) ; LC/MS (ESI) m/z: 519.4 [M+H]$^+$ |

(continued)

| No. | Structure/Name | Spectral Data |
|---|---|---|
| 150 | 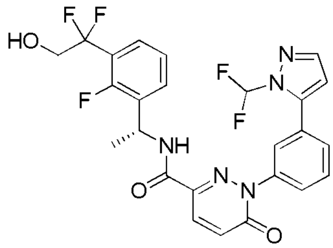 N-[(1R)-1-[3-(1,1-difluoro-2-hydroxy-ethyl)-2-fluoro-phenyl] ethyl]-1-[3-[2-(difluoromethyl)pyrazol-3-yl]phenyl]-6-oxo-pyridazine-3-carboxamide | $^{1}$H NMR (400 MHz, DMSO-$d_6$) δ = 9.02 (d, *J* = 8.0 Hz, 1H), 7.99 (s, 1H), 7.94 - 7.87 (m, 3H), 7.87 - 7.81 (m, 2H), 7.75 - 7.69 (m, 1H), 7.67 - 7.61 (m, 2H), 7.43 (t, *J* = 6.4 Hz, 1H), 7.26 (t, *J* = 7.6 Hz, 1H), 7.18 (d, *J* = 9.6 Hz, 1H), 6.76 (d, *J* = 1.6 Hz, 1H), 5.72 (br s, 1H), 5.41 (quin, *J* = 7.2 Hz, 1H), 3.91 (br t, *J* = 14.4 Hz, 2H), 1.48 (d, *J* = 7.2 Hz, 3H) ; LC/MS (ESI) m/z: 534.3 $[M+H]^+$ |
| 151 | 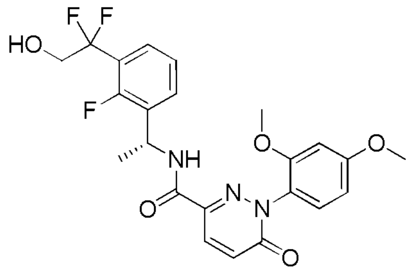 N-[(1R)-1-[3-(1,1-difluoro-2-hydroxy-ethyl)-2-fluoro-phenyl] ethyl]-1-(2,4-dimethoxyphenyl)-6-oxo-pyridazine-3-carboxamide | $^{1}$H NMR (400 MHz, DMSO-$d_6$) δ = 8.91 (d, *J* = 8.2 Hz, 1H), 7.96 - 7.78 (m, 1H), 7.62 (t, *J* = 6.8 Hz, 1H), 7.42 (t, *J* = 7.2 Hz, 1H), 7.38 - 7.30 (m, 1H), 7.26 (t, *J* = 7.8 Hz, 1H), 7.13 - 7.02 (m, 1H), 6.74 (br s, 1H), 6.72 - 6.61 (m, 1H), 5.71 (br s, 1H), 5.47 - 5.32 (m, 1H), 3.91 ( t, *J* = 14.4 Hz, 2H), 3.84 (s, 3H), 3.75 (s, 3H), 1.45 (d, *J* = 6.8 Hz, 3H) ; LC/MS (ESI) m/z: 478.4 $[M+H]^+$ |
| 152 | 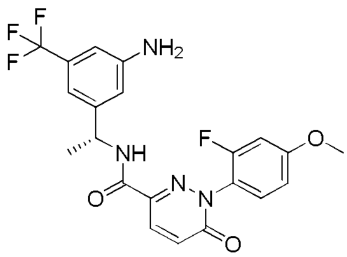 N-[(1R)-1-[3-amino-5-(trifluoromethyl)phenyl]ethyl]-1-(2-fluoro-4-methoxy-phenyl)-6-oxo-pyridazine-3-carboxamide | $^{1}$H NMR (400 MHz, DMSO-$d_6$) δ ppm 1.43 (d, *J* = 7.2 Hz, 3 H) 3.84 (s, 3 H) 5.02 (q, *J* = 7.2 Hz, 1 H) 5.54 (m, 2 H) 6.70 (s, 1 H) 6.77 (s, 1 H) 6.80 (s, 1 H) 6.95 (dd, *J* = 8.8, 2.25 Hz, 1 H) 7.06 (dd, *J* = 12.4, 2.63 Hz, 1 H) 7.2 (d, *J* = 9.76 Hz, 1 H) 7.55 (t, *J* = 8.8 Hz, 1 H) 7.92 *(d, J* = 10.0 Hz, 1 H) 8.87 (d, *J* = 8.4 Hz, 1 H) ; LC/MS (ESI) m/z: 451.2 $[M+H]^+$ |
| 153 | 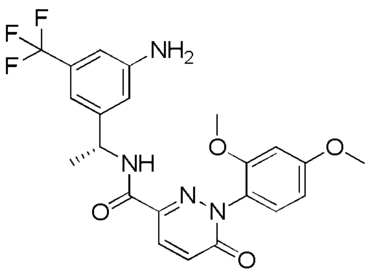 N-[(1R)-1-[3-amino-5-(trifluoromethyl)phenyl]ethyl]-1-(2,4-dimethoxyphenyl)-6-oxo-pyridazine-3-carboxamide | $^{1}$H NMR (400 MHz, DMSO-$d_6$) δ = 8.79 (br d, *J* = 8.0 Hz, 1H), 7.88 (d, *J* = 10.0 Hz, 1H), 7.32 (d, *J* = 8.8 Hz, 1H), 7.07 (d, *J* = 10.0 Hz, 1H), 6.85-6.66 (m, 4H), 6.64 (dd, *J* = 2.4, 8.8 Hz, 1H), 5.55 (s, 2H), 5.01 (br *t, J* = 7.6 Hz, 1H), 3.83 (s, 3H), 3.74 (s, 3H), 1.42 (d, *J* = 7.2 Hz, 3H) ; LC/MS (ESI) m/z: 463.3 $[M+H]^+$ |

(continued)

| No. | Structure/Name | Spectral Data |
|---|---|---|
| 154 | 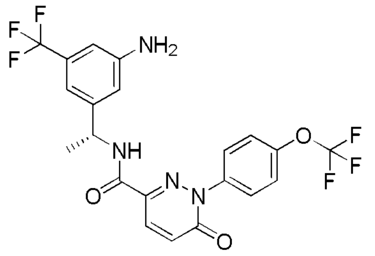 N-[(1R)-1-[3-amino-5-(trifluoromethyl)phenyl]ethyl]-6-oxo-1-[4-(trifluoromethoxy)phenyl]pyridazine-3-carboxamide | $^1$H NMR (400 MHz, DMSO-$d_6$) δ = 8.92 (d, *J* = 8.0 Hz, 1H), 7.90 (d, *J* = 9.8 Hz, 1H), 7.87 - 7.82 (m, 2H), 7.56 (d, *J* = 8.4 Hz, 2H), 7.17 (d, *J* = 10.0 Hz, 1H), 6.80 (br d, *J* = 14.8 Hz, 2H), 6.70 (s, 1H), 5.56 (s, 2H), 5.04 (quin, *J* = 7.2 Hz, 1H), 1.45 (d, *J* = 7.2 Hz, 3H) ; LC/MS (ESI) m/z: 486.3 $[M+H]^+$ |
| 155 | 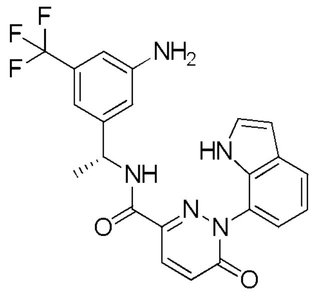 N-[(1R)-1-[3-amino-5-(trifluoromethyl)phenyl]ethyl]-1-(1H-indole-7-yl)-6-oxo-pyridazine-3-carboxamide | $^1$H NMR (400 MHz, DMSO-$d_6$) δ ppm 1.39 (d, *J* = 7.00 Hz, 3 H) 5.02 (quin, J = 7.6 Hz, 1 H) 5.53 (s, 2 H) 6.55 (m, 1 H) 6.68 (s, 1 H) 6.76 (s, 1 H) 6.80 (s, 1 H) 7.17 (m, 3 H) 7.38 (t, *J* = 2.8 Hz, 1 H) 7.68 (d, *J* = 7.6 Hz, 1 H) 7.95 (d, *J* = 9.6 Hz, 1 H) 8.80 (d, *J* = 8.4 Hz, 1 H) 11.17 (br s, 1 H) ; LC/MS (ESI) m/z: 442.3 $[M+H]^+$ |
| 156 | 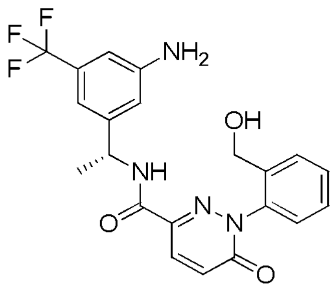 N-[(1R)-1-[3-amino-5-(trifluoromethyl)phenyl]ethyl]-1-[2-(hydroxymethyl)phenyl]-6-oxo-pyridazine-3-carboxamide | $^1$H NMR (400 MHz, DMSO-$d_6$) δ ppm 1.42 (br d, *J* = 7.2 Hz, 3 H) 4.35 (br d, *J* = 5.2 Hz, 2 H) 5.02 (quin, *J* = 7.2 Hz, 1 H) 5.20 (t, *J* = 5.6 Hz, 1 H) 5.54 (s, 2 H) 6.69 (s, 1 H) 6.78 (br d, *J* = 13.2 Hz, 2 H) 7.14 (d, *J* = 9.6 Hz, 1 H) 7.42 (m, 2 H) 7.51 (m, 1 H) 7.63 (br d, *J* = 7.6 Hz, 1 H) 7.93 (d, *J* = 9.6 Hz, 1 H) 8.84 (br d, *J* = 8.4 Hz, 1 H) ; LC/MS (ESI) m/z: 433.3 $[M+H]^+$ |
| 157 | 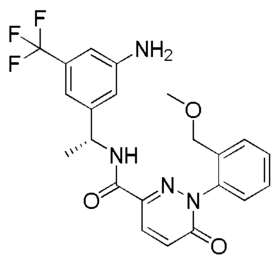 N-[(1R)-1-[3-amino-5-(trifluoromethyl)phenyl]ethyl]-1-[2-(methoxymethyl)phenyl]-6-oxo-pyridazine-3-carboxamide | $^1$H NMR (400 MHz, DMSO-$d_6$) δ ppm 1.42 (d, *J* = 7.2 Hz, 3 H) 3.15 (s, 3 H) 4.31 (s, 2 H) 5.03 (quin, *J* = 7.6 Hz, 1 H) 5.54 (s, 2 H) 6.69 (s, 1 H) 6.76 (s, 1 H) 6.79 (s, 1 H) 7.14 (d, *J* = 9.6 Hz, 1 H) 7.47 (d, *J* = 1.6 Hz, 1 H) 7.48 (d, *J* = 1.6 Hz, 1 H) 7.51 (td, *J* = 6.8, 2.81 Hz, 1 H) 7.56 (m, 1 H) 7.93 (d, *J* = 9.6 Hz, 1 H) 8.83 (d, *J* = 8.4 Hz, 1 H) ; LC/MS (ESI) m/z: 447.3 $[M+H]^+$ |

(continued)

| No. | Structure/Name | Spectral Data |
|---|---|---|
| 158 | 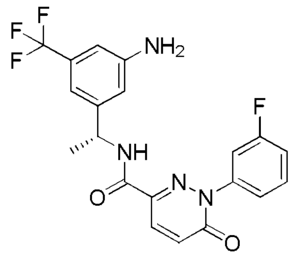 N-[(1R)-1-[3-amino-5-(trifluoromethyl)phenyl]ethyl]-1-(3-fluorophenyl)-6-oxo-pyridazine-3-carboxamide | $^1$H NMR (400 MHz, DMSO-$d_6$) δ = 8.91 (d, *J* = 8.2 Hz, 1H), 7.90 (d, *J* = 9.6 Hz, 1H), 7.66 (d, *J* = 10.0 Hz, 1H), 7.63 - 7.55 (m, 2H), 7.34 (dtd, 1H), 7.17 (d, *J* = 9.6 Hz, 1H), 6.83 (s, 1H), 6.79 (s, 1H), 6.71 (s, 1H), 5.56 (s, 2H), 5.05 (quin, J = 7.2 Hz, 1H), 3.57 (s, 1H), 1.46 (d, *J* = 7.2 Hz, 3H) ; LC/MS (ESI) m/z: 421.0 $[M+H]^+$ |
| 159 | 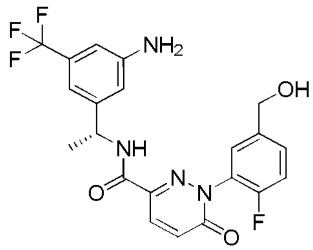 N-[(1R)-1-[3-amino-5-(trifluoromethyl)pheny 1]ethyl]-1-[2-fluoro-5-(hydroxymethyl)phenyl] -6-oxo-pyridazine-3-carboxamide | $^1$H NMR (400 MHz, DMSO-$d_6$) δ = 8.92 (d, *J* = 8.4 Hz, 1H), 7.93 (d, *J* = 10.0 Hz, 1H), 7.63 - 7.46 (m, 2H), 7.44 - 7.32 (m, 1H), 7.17 (d, *J* = 9.6 Hz, 1H), 6.79 (br d, *J* = 14.4 Hz, 2H), 6.69 (s, 1H), 5.54 (s, 2H), 5.48 - 5.30 (m, 1H), 5.02 (t, *J* = 7.6 Hz, 1H), 4.54 (s, 2H), 1.42 (d, *J* = 7.2 Hz, 3H) LC/MS (ESI) m/z: 451.1$[M+H]^+$ |
| 160 | 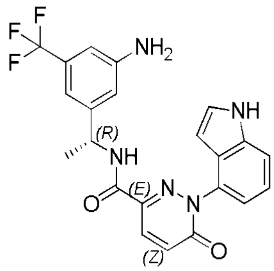 N-[(1R)-1-[3-amino-5-(trifluoromethyl)phenyl]ethyl]-1-(1H-indol-4-yl)-6-oxo-pyridazine-3-carboxamide | $^1$H NMR (400 MHz, DMSO-$d_6$) δ = 11.40 (br s, 1H), 8.74 (br d, *J* = 8.0 Hz, 1H), 7.95 (d, *J* = 9.6 Hz, 1H), 7.53 (d, *J* = 8.0 Hz, 1H), 7.40 (br s, 1H), 7.25 - 7.15 (m, 3H), 6.79 (s, 1H), 6.75 (s, 1H), 6.68 (s, 1H), 6.18 (br s, 1H), 5.54 (s, 2H), 5.01 (br t, *J* = 7.2 Hz, 1H), 1.39 (br d, *J* = 6.8 Hz, 3H) ; LC/MS (ESI) m/z: 442.1 $[M+H]^+$ |
| 161 | 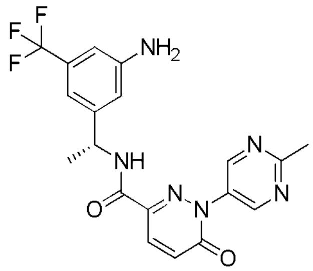 N-[(1R)-1-[3-amino-5-(trifluoromethyl)phenyl]ethyl]-1-(2-methylpyrimidin-5-yl)-6-oxo-pyridazine-3-carboxamide | $^1$H NMR (400 MHz, DMSO-$d_6$) δ = 9.15 (s, 2H), 8.99 (d, *J* = 8.4 Hz, 1H), 7.92 (d, *J* = 10.0 Hz, 1H), 7.20 (d, *J* = 9.6 Hz, 1H), 6.82 (s, 1H), 6.79 (s, 1H), 6.71 (s, 1H), 5.56 (s, 2H), 5.06 (quin, *J* = 7.2 Hz, 1H), 2.71 (s, 3H), 1.46 (d, *J* = 6.8 Hz, 3H) ; LC/MS (ESI) m/z: 419.3 $[M+H]^+$ |
| 162 | 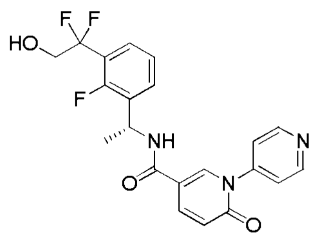 N-[(1R)-1-[3-(1,1-difluoro-2-hydroxy-ethyl)-2-fluoro-phenyl]ethyl]-6-oxo-1-(4-pyridyl)pyridine-3-carboxamide | $^1$H NMR (400 MHz, DMSO-$d_6$) δ = 8.81 - 8.76 (m, 2H), 8.71 (d, *J* = 7.2 Hz, 1H), 8.43 (d, *J* = 2.4 Hz, 1H), 7.95 (dd, *J* = 2.4, 9.6 Hz, 1H), 7.66 - 7.62 (m, 2H), 7.58 (t, *J* = 6.8 Hz, 1H), 7.46 - 7.40 (m, 1H), 7.31 - 7.24 (m, 1H), 6.58 (d, *J* = 9.6 Hz, 1H), 5.72 (t, *J* = 6.4 Hz, 1H), 5.36 (quin, J = 7.2 Hz, 1H), 3.92 (dt, *J* = 6.4, 14.4 Hz, 2H), 1.44 (d, *J* = 7.2 Hz, 3H) ; LC/MS (ESI) m/z: 418.3 $[M+H]^+$ |

(continued)

| No. | Structure/Name | Spectral Data |
|---|---|---|
| 163 | 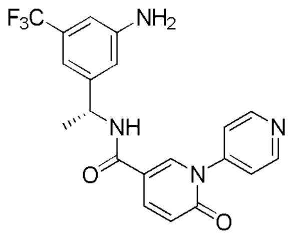 N-[(1R)-1-[3-amino-5-(trifluoromethyl)phenyl]ethyl]-6-oxo-1-(4-pyridyl)pyridine-3-carboxamide | $^1$H NMR (400 MHz, DMSO-$d_6$) δ = 8.81 - 8.74 (m, 2H), 8.59 (d, *J* = 7.6 Hz, 1H), 8.37 (d, *J* = 2.4 Hz, 1H), 7.97 (dd, *J* = 2.4, 9.6 Hz, 1H), 7.68 - 7.58 (m, 2H), 6.76 (s, 2H), 6.70 (s, 1H), 6.58 (d, *J* = 9.6 Hz, 1H), 5.55 (s, 2H), 5.01 (quin, J = 7.2 Hz, 1H), 1.40 (d, *J* = 7.2 Hz, 3H) ; LC/MS (ESI) m/z: 403.3 $[M+H]^+$ |
| 164 | 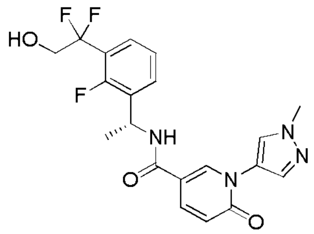 N-[(1R)-1-[3-(1,1-difluoro-2-hydroxy-ethyl)-2-fluoro-phenyl] ethyl]-1-(1-methylpyrazol-4-yl)-6-oxo-pyridine-3-carboxamide | $^1$H NMR (400 MHz, DMSO-$d_6$) δ = 8.73 (d, *J* = 7.2 Hz, 1H), 8.47 (d, *J* = 2.4 Hz, 1H), 8.26 (s, 1H), 7.90 (dd, *J* = 2.4, 9.6 Hz, 1H), 7.85 (s, 1H), 7.60 (br t, *J* = 6.8 Hz, 1H), 7.48 - 7.38 (m, 1H), 7.34 - 7.23 (m, 1H), 6.54 *(d, J* = 9.6 Hz, 1H), 5.72 (t, *J* = 6.4 Hz, 1H), 5.37 (quin, *J* = 7.2 Hz, 1H), 3.98 - 3.90 (m, 2H), 3.90 (s, 3H), 1.46 (d, *J* = 7.2 Hz, 3H) ; LC/MS (ESI) m/z: 421.3 $[M+H]^+$ |
| 165 | 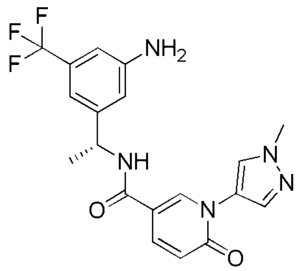 N-[(1R)-1-[3-amino-5-(trifluoromethyl)phenyl]ethyl]-1-(1-methylpyrazol-4-yl)-6-oxo-pyridine-3-carboxamide | $^1$H NMR (400 MHz, DMSO-$d_6$) δ = 8.61 (d, *J* = 7.6 Hz, 1H), 8.42 (d, *J* = 2.4 Hz, 1H), 8.25 (s, 1H), 7.91 (dd, *J* = 2.8, 9.6 Hz, 1H), 7.84 (s, 1H), 6.77 (s, 2H), 6.70 (s, 1H), 6.54 (d, *J* = 9.6 Hz, 1H), 5.55 (s, 2H), 5.02 (quin, *J* = 7.2 Hz, 1H), 3.89 (s, 3H), 1.42 (d, *J* = 7.2 Hz, 3H) ; LC/MS (ESI) m/z: 406.3 $[M+H]^+$ |
| 166 | 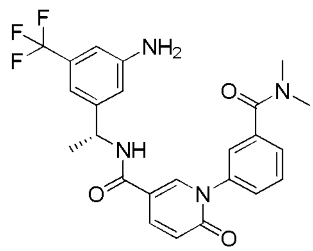 N-[(1R)-1-[3-amino-5-(trifluoromethyl)phenyl]ethyl]-1-[3-(dimethylcarbamoyl)phenyl]-6-oxo-pyridine-3-carboxamide | $^1$H NMR (400 MHz, DMSO-$d_6$) δ = 8.59 (d, *J* = 7.6 Hz, 1H), 8.38 (d, *J* = 2.4 Hz, 1H), 7.96 (dd, *J* = 2.4, 9.6 Hz, 1H), 7.64 - 7.49 (m, 4H), 6.76 (s, 2H), 6.69 (s, 1H), 6.54 (d, *J* = 9.6 Hz, 1H), 5.55 (s, 2H), 5.01 (quin, *J* = 7.2 Hz, 1H), 2.97 (br d, *J* = 16.0 Hz, 6H), 1.40 (d, *J* = 7.2 Hz, 3H) ; LC/MS (ESI) m/z: 473.3 $[M+H]^+$ |
| 167 | 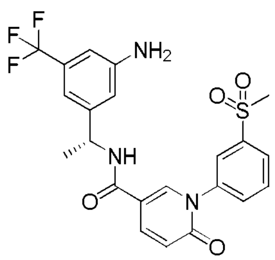 N-[(1R)-1-[3-amino-5-(trifluoromethyl)phenyl]ethyl]-1-(3-methylsulfonylphenyl)-6-oxo-pyridine-3-carboxamide | $^1$H NMR (400 MHz, DMSO-$d_6$) δ = 8.61 (d, *J* = 7.6 Hz, 1H), 8.40 (d, *J* = 2.4 Hz, 1H), 8.09 (s, 1H), 8.05 (d, *J* = 7.6 Hz, 1H), 7.99 (dd, *J* = 2.4, 9.6 Hz, 1H), 7.91 - 7.82 (m, 2H), 6.76 (s, 2H), 6.69 (s, 1H), 6.58 (d, *J* = 9.6 Hz, 1H), 5.56 (s, 2H), 5.02 (quin, *J* = 7.2 Hz, 1H), 3.31 - 3.29 (m, 3H), 1.40 (d, *J* = 7.2 Hz, 3H) ; LC/MS (ESI) m/z: 480.3 $[M+H]^+$ |

(continued)

| No. | Structure/Name | Spectral Data |
|---|---|---|
| 168 | 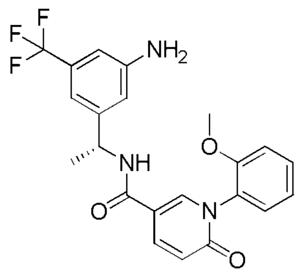 N-[(1R)-1-[3-amino-5-(trifluoromethyl)phenyl]ethyl]-1-(2-methoxyphenyl)-6-oxo-pyridine-3-carboxamide | $^1$H NMR (400 MHz, DMSO-$d_6$) δ = 8.53 (br d, *J* = 7.2 Hz, 1H), 8.25 (s, 1H), 7.95 (dd, *J* = 2.4, 9.6 Hz, 1H), 7.50 (t, *J* = 8.0 Hz, 1H), 7.37 (d, *J* = 7.6 Hz, 1H), 7.24 (d, *J* = 8.4 Hz, 1H), 7.10 (t, *J* = 7.6 Hz, 1H), 6.75 (s, 2H), 6.69 (s, 1H), 6.50 (d, *J* = 9.6 Hz, 1H), 5.56 (s, 2H), 5.04 - 4.93 (m, 1H), 3.76 (s, 3H), 1.38 (d, *J* = 7.2 Hz, 3H) ; LC/MS (ESI) m/z: 432.3 $[M+H]^+$ |
| 169 | 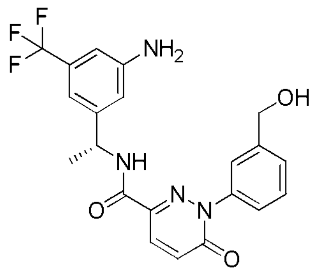 N-[(1R)-1-[3-amino-5-(trifluoromethyl)phenyl]ethyl]-1-[3-(hydroxymethyl)phenyl]-6-oxo-pyridazine-3-carboxamide | $^1$H NMR (400 MHz, DMSO-$d_6$) δ = 8.85 (d, *J* = 8.4 Hz, 1H), 7.90 (d, *J* = 9.6 Hz, 1H), 7.55 (s, 1H), 7.49 (d, *J* = 5.2 Hz, 2H), 7.44 - 7.39 (m, 1H), 7.14 (d, *J* = 9.6 Hz, 1H), 6.82 (s, 1H), 6.78 (s, 1H), 6.70 (s, 1H), 5.55 (s, 2H), 5.36 *(br t, J* = 5.2 Hz, 1H), 5.03 (quin, *J* = 7.2 Hz, 1H), 4.58 (d, *J* = 4.8 Hz, 2H), 1.44 (d, *J* = 7.2 Hz, 3H) ; LC/MS (ESI) m/z: 433.3 $[M+H]^+$ |
| 170 | 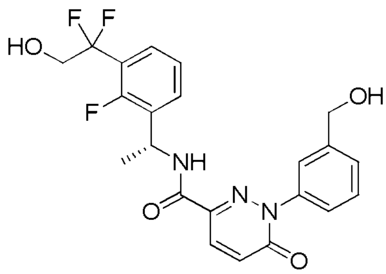 N-[(1R)-1-[3-(1,1-difluoro-2-hydroxy-ethyl)-2-fluoro-phenyl] ethyl]-1-[3-(hydroxymethyl)phenyl]-6-oxo-pyridazine-3-carboxamide | $^1$H NMR (400 MHz, DMSO-$d_6$) δ = 8.96 (d, *J* = 8.0 Hz, 1H), 7.87 (d, *J* = 9.6 Hz, 1H), 7.63 (t, *J* = 6.8 Hz, 1H), 7.56 (s, 1H), 7.54 - 7.47 (m, 2H), 7.45 - 7.40 (m, 2H), 7.27 (t, *J* = 7.6 Hz, 1H), 7.14 (d, *J* = 9.6 Hz, 1H), 5.71 (br s, 1H), 5.51 - 5.24 (m, 2H), 4.58 (s, 2H), 3.91 (t, *J* = 14.4 Hz, 2H), 1.47 (d, *J* = 7.2 Hz, 3H) ; LC/MS (ESI) m/z: 448.3 $[M+H]^+$ |
| 171 | 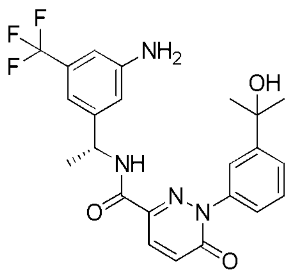 N-[(1R)-1-[3-amino-5-(trifluoromethyl)phenyl]ethyl]-1-[3-(1-hydroxy-1-methyl-ethyl)phenyl]-6-oxo-pyridazine-3-carboxamide | $^1$H NMR (400 MHz, DMSO-$d_6$) δ = 8.84 (d, *J* = 8.4 Hz, 1H), 7.89 (d, *J* = 9.6 Hz, 1H), 7.67 (s, 1H), 7.55 (d, *J* = 6.8 Hz, 1H), 7.49 - 7.42 (m, 2H), 7.13 (d, *J* = 9.6 Hz, 1H), 6.82 (s, 1H), 6.78 (s, 1H), 6.69 (s, 1H), 5.55 (s, 2H), 5.17 (s, 1H), 5.02 (quin, *J* = 7.2 Hz, 1H), 1.45 (s, 6H), 1.43 (d, *J* = 7.2 Hz, 3H) ; LC/MS (ESI) m/z: 443.4 $[M+H]^+$ |

(continued)

| No. | Structure/Name | Spectral Data |
|---|---|---|
| 172 | 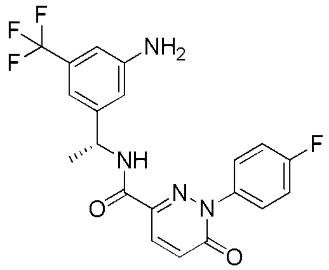 N-[(1R)-1-[3-amino-5-(trifluoromethyl)phenyl]ethyl]-1-(4-fluorophenyl)-6-oxo-pyridazine-3-carboxamide | $^1H$ NMR (400 MHz, DMSO-$d_6$) $\delta$ = 8.86 (d, *J* = 8.4 Hz, 1H), 7.89 (d, *J* = 9.6 Hz, 1H), 7.78 - 7.69 (m, 2H), 7.38 (t, *J* = 8.4 Hz, 2H), 7.14 (d, *J* = 9.6 Hz, 1H), 6.82 (s, 1H), 6.78 (s, 1H), 6.70 (s, 1H), 5.54 (s, 2H), 5.04 (quin, *J* = 7.2 Hz, 1H), 1.45 (d, *J* = 7.2 Hz, 3H) ; LC/MS (ESI) m/z: 436.3 $[M+H]^+$ |
| 173 | 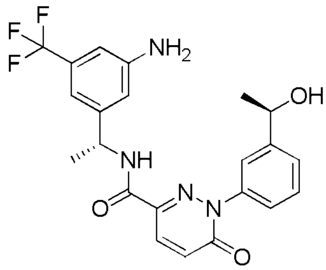 N-[(1R)-1-[3-amino-5-(trifluoromethyl)phenyl]ethyl]-1-[3-[(1R)-1-hydroxyethyl]phenyl]-6-oxo-pyridazine-3-carboxamide | $^1H$ NMR (400 MHz, DMSO-$d_6$) $\delta$ = 8.84 (d, *J* = 8.4 Hz, 1H), 7.89 (d, *J* = 9.6 Hz, 1H), 7.57 (s, 1H), 7.52 - 7.39 (m, 3H), 7.14 (d, *J* = 9.6 Hz, 1H), 6.82 (s, 1H), 6.78 (s, 1H), 6.70 (s, 1H), 5.55 (s, 2H), 5.32 (br s, 1H), 5.03 (quin, *J* = 7.2 Hz, 1H), 4.80 (q, *J* = 6.4 Hz, 1H), 1.43 (d, *J* = 7.2 Hz, 3H), 1.35 (d, *J* = 6.4 Hz, 3H) ; LC/MS (ESI) m/z: 447.3 $[M+H]^+$ |
| 174 | 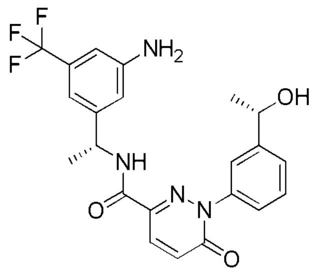 N-[(1R)-1-[3-amino-5-(trifluoromethyl)phenyl]ethyl]-1-[3-[(1S)-1-hydroxyethyl]phenyl]-6-oxo-pyridazine-3-carboxamide | $^1H$ NMR (400 MHz, DMSO-$d_6$) $\delta$ = 8.85 (d, *J* = 8.4 Hz, 1H), 7.89 (d, *J* = 9.6 Hz, 1H), 7.57 (s, 1H), 7.49 - 7.43 (m, 3H), 7.14 (d, *J* = 9.6 Hz, 1H), 6.82 (s, 1H), 6.77 (s, 1H), 6.69 (s, 1H), 5.55 (s, 2H), 5.32 (d, *J* = 4.4 Hz, 1H), 5.03 (quin, *J* = 7.2 Hz, 1H), 4.82 - 4.76 (m, 1H), 1.43 (d, *J* = 7.2 Hz, 3H), 1.35 (d, *J* = 6.4 Hz, 3H) ; LC/MS (ESI) m/z: 447.3 $[M+H]^+$ |
| 175 | 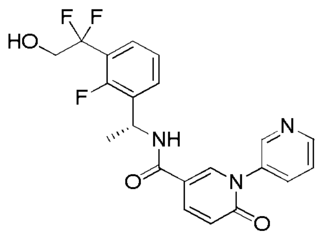 N-[(1R)-1-[3-(1,1-difluoro-2-hydroxy-ethyl)-2-fluoro-phenyl]ethyl]-6-oxo-1-(3-pyridyl)pyridine-3-carboxamide | $^1H$ NMR (400 MHz, DMSO-$d_6$) $\delta$ = 9.02 (d, *J* = 7.2 Hz, 1H), 8.63 (d, *J* = 2.4 Hz, 1H), 8.49 (d, *J* = 2.4 Hz, 1H), 8.47 (dd, *J* = 1.2, 4.8 Hz, 1H), 8.34 (dd, *J* = 2.4, 8.8 Hz, 1H), 7.69 (d, *J* = 8.8 Hz, 1H), 7.61 (t, *J* = 7.2 Hz, 1H), 7.50 (dd, *J* = 4.8, 8.4 Hz, 1H), 7.43 (t, *J* = 6.8 Hz, 1H), 7.28 *(t, J* = 7.2 Hz, 1H), 7.24 (d, *J* = 8.8 Hz, 1H), 5.72 *(t, J* = 6.4 Hz, 1H), 5.40 (quin, *J* = 7.2 Hz, 1H), 3.92 (dt, *J* = 6.4, 14.4 Hz, 2H), 1.47 (d, *J* = 7.2 Hz, 3H) ; LC/MS (ESI) m/z: 418.3 $[M+H]^+$ |

(continued)

| No. | Structure/Name | Spectral Data |
|---|---|---|
| 176 | 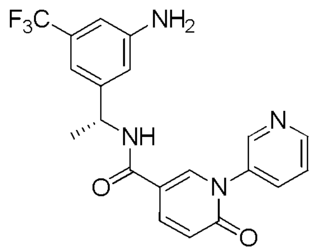 N-[(1R)-1-[3-amino-5-(trifluoromethyl)phenyl]ethyl]-6-oxo-1-(3-pyridyl)pyridine-3-carboxamide | $^1$H NMR (400 MHz, DMSO-d$_6$) δ = 8.90 (d, *J* = 8.0 Hz, 1H), 8.62 (d, *J* = 2.4 Hz, 1H), 8.52 - 8.44 (m, 2H), 8.32 (dd, *J* = 2.4, 8.8 Hz, 1H), 7.68 (ddd, *J* = 1.4, 2.8, 8.4 Hz, 1H), 7.50 (dd, *J* = 4.4, 8.4 Hz, 1H), 7.23 (d, *J* = 8.8 Hz, 1H), 6.79 (s, 2H), 6.70 (s, 1H), 5.57 (s, 2H), 5.05 (t, *J* = 7.2 Hz, 1H), 1.44 (d, *J* = 7.2 Hz, 3H) ; LC/MS (ESI) m/z: 403.3 [M+H]$^+$ |
| 177 | 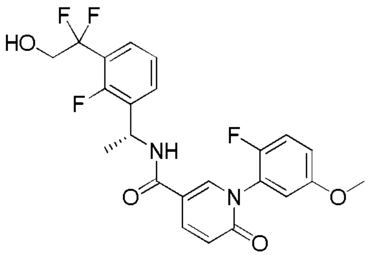 N-[(1R)-1-[3-(1,1-difluoro-2-hydroxy-ethyl)-2-fluoro-phenyl] ethyl]-1-(2-fluoro-5-methoxyphenyl)-6-oxo-pyridine-3-carboxamide | $^1$H NMR (400 MHz, DMSO-d$_6$) δ = 8.66 (br d, *J* = 7.2 Hz, 1H), 8.40 (d, *J* = 2.4 Hz, 1H), 7.96 (dd, *J* = 2.4, 9.6 Hz, 1H), 7.58 (br t, *J* = 6.8 Hz, 1H), 7.45 - 7.35 (m, 2H), 7.28 (t, *J* = 7.6 Hz, 1H), 7.24 (dd, *J* = 3.2, 6.0 Hz, 1H), 7.12 (td, *J* = 3.4, 9.2 Hz, 1H), 6.55 *(d,* *J* = 9.6 Hz, 1H), 5.71 (t, *J* = 6.4 Hz, 1H), 5.35 (t, *J* = 7.2 Hz, 1H), 3.92 (dt, *J* = 6.4, 14.4 Hz, 2H), 3.80 (s,3H), 1.43 (d, *J* = 7.2 Hz, 3H) ; LC/MS (ESI) m/z: 465.3 [M+H]$^+$ |
| 178 | 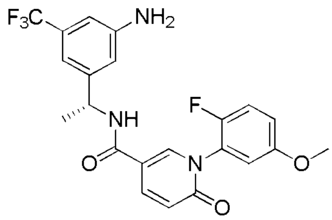 N-[(1R)-1-[3-amino-5-(trifluoromethyl)phenyl]ethyl]-1-(2-fluoro-5-methoxy-phenyl)-6-oxo-pyridine-3-carboxamide | $^1$H NMR (400 MHz, DMSO-d$_6$) δ = 8.55 (d, *J* = 7.6 Hz, 1H), 8.35 (d, *J* = 2.4 Hz, 1H), 7.98 (dd, *J* = 2.4, 9.6 Hz, 1H), 7.38 (t, *J* = 9.2 Hz, 1H), 7.22 (dd, *J* = 3.2, 6.0 Hz, 1H), 7.11 (td, *J* = 3.6, 9.0 Hz, 1H), 6.76 (s, 2H), 6.70 (s, 1H), 6.56 (d, *J* = 9.6 Hz, 1H), 5.55 (s, 2H), 5.00 (quin, *J* = 7.2 Hz, 1H), 3.79 (s, 3H), 1.40 (d, *J* = 7.2 Hz, 3H) ; LC/MS (ESI) m/z: 450.4 [M+H]$^+$ |
| 179 | 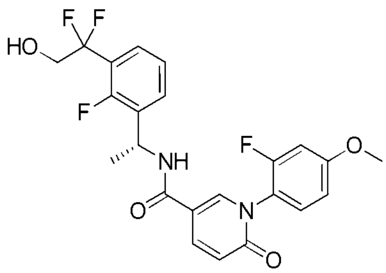 N-[(1R)-1-[3-(1,1-difluoro-2-hydroxy-ethyl)-2-fluoro-phenyl] ethyl]-1-(2-fluoro-4-methoxyphenyl)-6-oxo-pyridine-3-carboxamide | $^1$H NMR (400 MHz, DMSO-d$_6$) δ = 8.67 (d, *J* = 7.2 Hz, 1H), 8.37 (d, *J* = 2.4 Hz, 1H), 7.95 (dd, *J* = 2.8, 9.6 Hz, 1H), 7.60 - 7.47 (m, 2H), 7.43 (br t, *J* = 6.4 Hz, 1H), 7.31 - 7.25 (m, 1H), 7.10 (dd, *J* = 2.4, 12.0 Hz, 1H), 6.95 (dd, *J* = 2.4, 8.8 Hz, 1H), 6.61 - 6.48 (m, 1H), 5.74 (br d, *J* = 14.4 Hz, 1H), 5.39 - 5.31 (m, 1H), 3.91 (br t, *J* = 14.4 Hz, 2H), 3.84 (s, 3H), 1.43 (d, *J* = 7.2 Hz, 3H) ) ; LC/MS (ESI) m/z: 465.3 [M+H]$^+$ |

(continued)

| No. | Structure/Name | Spectral Data |
|---|---|---|
| 180 | 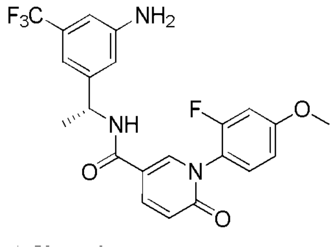 N-[(1R)-1-[3-amino-5-(trifluoromethyl)phenyl]ethyl]-1-(2-fluoro-4-methoxy-phenyl)-6-oxo-pyridine-3-carboxamide | $^1$H NMR (400 MHz, DMSO-$d_6$) δ= 8.21 (d, *J* = 2.4 Hz, 1H), 8.06 (dd, *J* = 2.4, 9.6 Hz, 1H), 7.40 - 7.35(m, 1H), 6.99 - 6.91 (m, 2H), 6.88 (br s, 2H), 6.80 (s, 1H), 6.64 (d, *J* = 9.6 Hz, 1H), 5.11 *(d, J* = 7.2 Hz, 1H), 3.87 (s, 3H), 1.51 *(d, J* = 7.2 Hz, 3H) ; LC/MS (ESI) m/z: 450.4 [M+H]$^+$ |
| 181 | 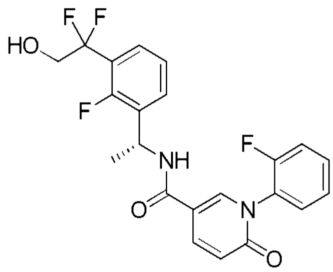 N-[(1R)-1-[3-(1,1-difluoro-2-hydroxy-ethyl)-2-fluoro-phenyl] ethyl]-1-(2-fluorophenyl)-6-oxo-pyridine-3-carboxamide | $^1$H NMR (400 MHz, DMSO-$d_6$) δ = 8.68 (d, *J* = 7.2 Hz, 1H), 8.42 (d, *J* = 2.4 Hz, 1H), 7.97 (dd, *J* = 2.8, 9.6 Hz, 1H), 7.65 - 7.55 (m, 3H), 7.50 - 7.39 (m, 3H), 7.30 - 7.24 (m, 1H), 6.57 (d, *J* = 9.6 Hz, 1H), 5.72 (br t, *J* = 6.4 Hz, 1H), 5.35 (quin, *J* = 7.2 Hz, 1H), 3.91 (dt, *J* = 5.8, 14.4 Hz, 2H), 1.43 (d, *J* = 7.2 Hz, 3H) ; LC/MS (ESI) m/z: 4 35.3 [M+H]$^+$ |
| 182 | 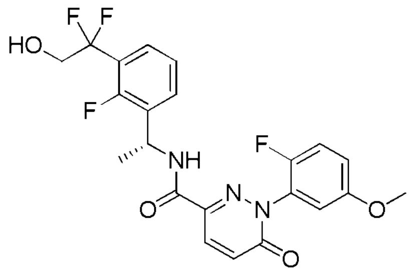 N-[(1R)-1-[3-(1,1-difluoro-2-hydroxy-ethyl)-2-fluoro-phenyl] ethyl]-1-(2-fluoro-5-methoxyphenyl)-6-oxo-pyridazine-3-carboxa-mide | $^1$H NMR (400 MHz, DMSO-$d_6$) δ = 9.01 (d, *J* = 8.0 Hz, 1H), 7.91 (d, *J* = 9.6 Hz, 1H), 7.62 (br t, *J* = 7.2 Hz, 1H), 7.45 - 7.35 (m, 2H), 7.30 - 7.24 (m, 2H), 7.20 - 7.11 (m, 2H), 5.71 (br s, 1H), 5.40 (t, *J* = 7.2 Hz, 1H), 3.91 (dt, *J* = 5.2, 14.4 Hz, 2H), 3.80 (s, 3H), 1.46 (d, *J* = 7.2 Hz, 3H) ; LC/MS (ESI) m/z: 466.3 [M+H]$^+$ |
| 183 | 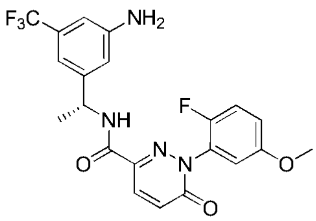 N-[(1R)-1-[3-amino-5-(trifluoromethyl)phenyl]ethyl]-1-(2-fluoro-5-methoxy-phenyl)-6-oxo-pyridazine-3-carboxamide | $^1$H NMR (400 MHz, DMSO-$d_6$) δ = 8.21 (d, *J* = 2.4 Hz, 1H), 8.06 (dd, *J* = 2.4, 9.6 Hz, 1H), 7.40 - 7.35 (m, 1H), 6.99 - 6.91 (m, 2H), 6.88 (br s, 2H), 6.80 (s, 1H), 6.64 (d, *J* = 9.6 Hz, 1H), 5.11 *(d, J* = 7.2 Hz, 1H), 3.87 (s, 3H), 1.51 *(d, J* = 7.2 Hz, 3H) ; LC/MS (ESI) m/z: 451.3 [M+H]$^+$ |

(continued)

| No. | Structure/Name | Spectral Data |
|---|---|---|
| 184 | 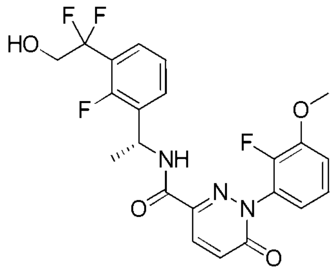<br>N-[(1R)-1-[3-(1,1-difluoro-2-hydroxy-ethyl)-2-fluoro-phenyl] ethyl]-1-(2-fluoro-3-methoxyphenyl)-6-oxo-pyridazine-3-carboxamide | $^1$H NMR (400 MHz, DMSO-$d_6$) δ = 9.01 (d, *J* = 8.0 Hz, 1H), 7.91 (d, *J* = 9.6 Hz, 1H), 7.61 (br t, *J* = 7.2 Hz, 1H), 7.42 (t, *J* = 6.8 Hz, 1H), 7.37 - 7.30 (m, 2H), 7.29 - 7.24 (m, 1H), 7.23 - 7.15 (m, 2H), 5.71 (t, *J* = 6.4 Hz, 1H), 5.39 (quin, *J* = 7.2 Hz, 1H), 3.95 - 3.87 (m, 5H), 1.45 (d, *J* = 7.2 Hz, 3H) ; LC/MS (ESI) m/z: 466.3 $[M+H]^+$ |
| 185 | 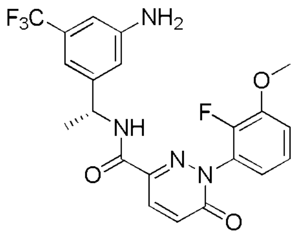<br>N-[(1R)-1-[3-amino-5-(trifluoromethyl)phenyl]ethyl]-1-(2-fluoro-3-methoxy-phenyl)-6-oxo-pyridazine-3-carboxamide | $^1$H NMR (400 MHz, DMSO-$d_6$) δ = 8.89 (br d, *J* = 8.0 Hz, 1H), 7.93 (d, *J* = 9.6 Hz, 1H), 7.37 - 7.29 (m, 2H),7.21 - 7.13 (m, 2H), 6.80 (s, 1H), 6.77 (s, 1H), 6.69 (s, 1H), 5.54 (s, 2H), 5.02 (quin, *J* = 7.2 Hz, 1H), 3.91 (s, 3H), 1.42 (d, *J* = 7.2 Hz, 3H) ; LC/MS (ESI) m/z: 45 1.3 $[M+H]^+$ |
| 186 | 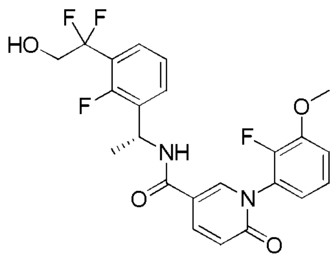<br>N-[(1R)-1-[3-(1,1-difluoro-2-hydroxy-ethyl)-2-fluoro-phenyl] ethyl]-1-(2-fluoro-3-methoxyphenyl)-6-oxo-pyridine-3-carboxamide | $^1$H NMR (400 MHz, DMSO-$d_6$) δ = 8.68 (d, *J* = 7.2 Hz, 1H), 8.39 (d, *J* = 2.4 Hz, 1H), 7.96 (dd, *J* = 2.4, 9.6 Hz, 1H), 7.57 (t, *J* = 7.2 Hz, 1H), 7.45 - 7.25 (m, 4H), 7.14 (dt, *J* = 2.0, 6.8 Hz, 1H), 6.56 (d, *J* = 9.6 Hz, 1H), 5.72 (t, *J* = 6.4 Hz, 1H), 5.34 (quin, *J* = 7.2 Hz, 1H), 3.96 - 3.86 (m, 5H), 1.43 (d, *J* = 7.2 Hz, 3H) ; LC/MS (ESI) m/z: 465.3 $[M+H]^+$ |
| 187 | 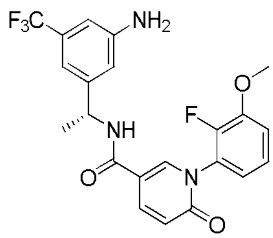<br>N-[(1R)-1-[3-amino-5-(trifluoromethyl)phenyl]ethyl]-1-(2-fluoro-3-methoxy-phenyl)-6-oxo-pyridine-3-carboxamide | $^1$H NMR (400 MHz, DMSO-$d_6$) δ = 8.56 (d, *J* = 7.6 Hz, 1H), 8.34 (d, *J* = 2.4 Hz, 1H), 7.98 (dd, *J* = 2.8, 9.6 Hz, 1H), 7.34 (quin, *J* = 8.0 Hz, 2H), 7.13 (dt, *J* = 2.0, 6.8 Hz, 1H), 6.75 (s, 2H), 6.69 (s, 1H), 6.56 (d, *J* = 9.6Hz, 1H), 5.56 (s, 2H), 5.04 - 4.95 (m, 1H), 3.91 (s, 3H), 1.39 (d, *J* = 7.2 Hz, 3H) ; LC/MS (ESI) m/z: 450.3 $[M+H]^+$ |
| 188 | 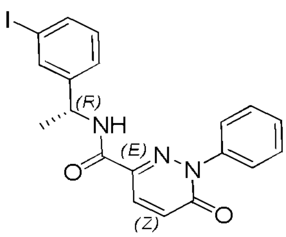<br>(R)-N-(1-(3-iodophenyl)ethyl)-6-oxo-1-phenyl-1,6-dihydropyridazine-3-carboxamide | $^1$H NMR (400 MHz, DMSO-$d_6$) δ = 8.92 (d, *J* = 8.0 Hz, 1H), 7.89 (s, 1H), 7.86 (s, 1H), 7.76 (s, 1H), 7.68 - 7.57 (m, 2H), 7.54-7.41 (m, 3H), 7.30-7.24 (m, 1H), 7.20-7.18 (d, 1H), 7.17-7.10 (m 2H), 5.07 (quin, *J* = 7.2 Hz, 1H), 1.46 (d, *J* = 7.2 Hz, 3H) ; LC/MS (ESI) m/z: 446.2 $[M+H]^+$ |

(continued)

| No. | Structure/Name | Spectral Data |
|---|---|---|
| 189 | 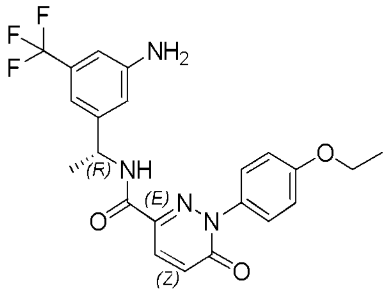<br>(R)-N-(1-(3-amino-5-(trifluoromethyl)phenyl)ethyl)-1-(4-ethoxyphenyl)-6-oxo-1,6-dihydropyridazine-3-carboxamide | $^1$H NMR (400 MHz, DMSO-$d_6$) $\delta$ = 8.86-8.84 (m, 1H), 8.87-8.84 (m, 1H), 7.60-7.50 (m, 2H), 7.11 (d, *J* = 9.6 Hz, 1H), 7.10-7.04 (m, 2H), 7.03-6.80 (m, 2H), 6.69 (m, 1H), 5.54 (s, 1H), 5.07-4.98 (m, 1H), 4.09 (quin, *J* = 7.2 Hz, 1H), 1.44 (d, *J* = 7.2 Hz, 2H), 1.35 (t, *J* = 6.8 Hz, 3H) ; LC/MS (ESI) m/z: 447.3 [M+H]$^+$ |
| 190 | 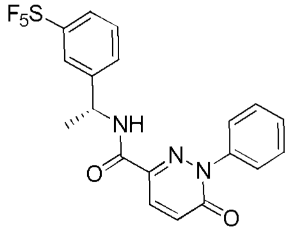<br>6-oxo-N-[(1R)-1-[3-(pentafluoro-λ6-sulfanyl)phenyl]ethyl]-1-phenyl-pyridazine-3-carboxamide | $^1$H NMR (400 MHz, DMSO-$d_6$) $\delta$ =1.50 (d, J = 7.2 Hz, 3 H) 5.22 (q, J = 7.6 Hz, 1 H) 7.14 (d, J = 10.0 Hz, 1 H) 7.48 (m, 1 H) 7.55 (m, 3 H) 7.67 (m, 3 H) 7.76 (dd, J = 8.4, 1.6 Hz, 1 H) 7.87 (d, J = 10.0 Hz, 1 H) 7.93 (s, 1 H) 9.03 (br d, J = 8.4 Hz, 1 H) ; LC/MS (ESI) m/z: 446.3 [M+H]$^+$ |
| 191 | 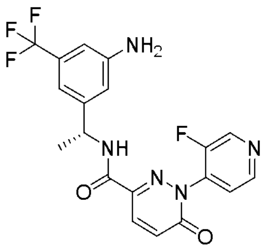<br>N-[(1R)-1-[3-(1,1-difluoro-2-hydroxy-ethyl)-2-fluorophenyl] ethyl]-1-(2-fluoro-3-pyridyl)-6-oxo-pyridazine-3-carboxamide | $^1$H NMR (400 MHz, DMSO-$d_6$) $\delta$ = 8.97 (d, J = 8.4 Hz, 1H), 8.84 (d, J = 1.6 Hz, 1H), 8.69 (d, J = 5.2 Hz, 1H), 7.95 (d, J = 10.0 Hz, 1H), 7.86 (t, J = 5.6 Hz, 1H), 7.23 (d, J = 9.6 Hz, 1H), 6.79 (d, J = 13.2 Hz, 2H), 6.70 (s, 1H), 5.55 (s, 2H), 5.09 - 4.97 (m, 1H), 1.43 (d, J = 7.2 Hz, 3H) ; LC/MS (ESI) m/z = 422.3 [M+H]$^+$ |
| 192 | 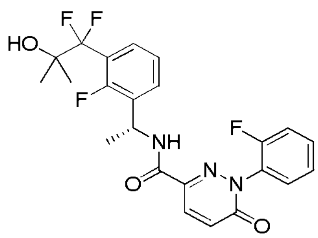<br>N-[(1R)-1-[3-(1,1-difluoro-2-hydroxy-2-methyl-propyl)-2-fluoro-phenyl]ethyl]-1-(2-fluorophenyl)-6-oxo-pyridazine-3-carboxamide | $^1$H NMR (400 MHz, DMSO-$d_6$) $\delta$ = 9.00 (br d, J = 8.0 Hz, 1H), 7.92 (d, J = 9.6 Hz, 1H), 7.69 (br t, J = 7.2 Hz, 1H), 7.62 - 7.53 (m, 2H), 7.49 - 7.40 (m, 2H), 7.32 (t, J = 6.8 Hz, 1H), 7.25 - 7.15 (m, 2H), 5.38 (br t, J = 7.2 Hz, 1H), 5.33 (s, 1H), 1.45 (br d, J = 6.8 Hz, 3H), 1.19 (br s, 6H) ; LC/MS (ESI) m/z: 464.3 [M+H]$^+$ |
| 193 | 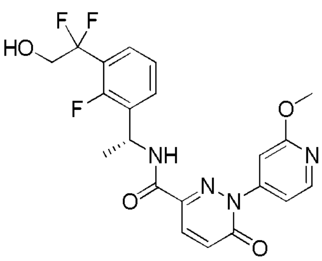<br>N-[(1R)-1-[3-(1,1-difluoro-2-hydroxy-ethyl)-2-fluoro-phenyl] ethyl]-1-(2-methoxy-4-pyridyl)-6-oxo-pyridazine-3-carboxamide | $^1$H NMR (400 MHz, DMSO-$d_6$) $\delta$ = 9.05 (d, *J* = 8.0 Hz, 1H), 8.33 (d, *J* = 5.6 Hz, 1H), 7.86 (d, *J* = 9.6 Hz, 1H), 7.65 (t, *J* = 7.2 Hz, 1H), 7.48 (dd, *J* = 1.6, 5.6 Hz, 1H), 7.46 - 7.41 (m, 1H), 7.36 (d, *J* = 1.6 Hz, 1H), 7.28 - 7.28 (m, 1H), 7.29 (t, *J* = 7.6 Hz, 1H), 7.17 (d, *J* = 9.6 Hz, 1H), 5.72 (t, *J* = 6.4 Hz, 1H), 5.41 (quin, *J* = 7.2 Hz, 1H), 3.96 - 3.89 (m, 5H), 1.50 *(d, J* = 7.2 Hz, 3H) ; LC/MS (ESI) m/z: 449.3 [M+H]$^+$ |

(continued)

| No. | Structure/Name | Spectral Data |
|---|---|---|
| 194 | 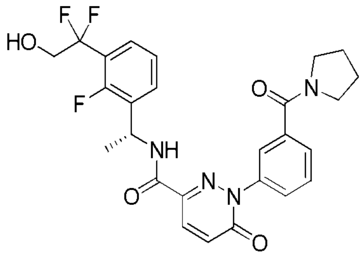 N-[(1R)-1-[3-(1,1-difluoro-2-hydroxy-ethyl)-2-fluoro-phenyl]ethyl]-6-oxo-1-[3-(pyrrolidine-1-carbonyl)phenyl]pyridazine-3-carboxamide | $^1$H NMR (400 MHz, DMSO-$d_6$) δ = 9.00 (d, *J* = 8.0 Hz, 1H), 7.91-7.82 (m, 2H), 7.78 (dt, *J*= 2.4, 4.4 Hz, 1H), 7.67-7.58 (m, 3H), 7.53-7.53 (m, 1H), 7.43 (br t, *J* = 6.8 Hz, 1H), 7.31-7.23 (m, 1H), 7.15 (d, *J* = 9.6 Hz, 1H), 5.71 (t, *J* = 6.4 Hz, 1H), 5.40 (quin, *J* = 7.2 Hz, 1H), 3.96-3.86 (m, 2H), 3.47 (td, *J* = 6.4, 15.6 Hz, 4H), 1.91-1.78 (m, 4H), 1.47 (d, *J* = 7.2 Hz, 3H) ; LC/MS (ESI) m/z: 515.4 $[M+H]^+$ |
| 195 | 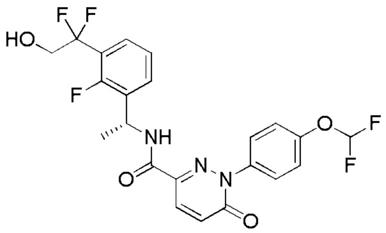 N-[(1R)-1-[3-(1,1-difluoro-2-hydroxy-ethyl)-2-fluoro-phenyl] ethyl]-1-[4-(difluoromethoxy)phenyl]-6-oxo-pyridazine-3-carboxamide | $^1$H NMR (400 MHz, DMSO-$d_6$) δ = 8.97 (d, *J* = 8.0 Hz, 1H), 7.87 (d, *J* = 9.6 Hz, 1H), 7.79-7.71 (m, 2H), 7.63 ( t, *J* = 6.8 Hz, 1H), 7.52 (s, 1H), 7.46-7.39 (m, 1H), 7.38 -7.31 (m, 3H), 7.27 (t, *J* = 7.6 Hz, 1H), 7.17-7.10 (m, 1 H), 5.72 (s, 1H), 5.40 (quin, *J* = 7.2 Hz, 1H), 3.92 (t, *J* = 14.4 Hz, 2H), 1.47 (d, *J* = 7.2 Hz, 3H) ; LC/MS (ESI) m/z: 484.3 $[M+H]^+$ |
| 196 | 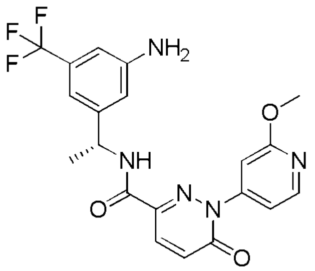 N-[(1R)-1-[3-amino-5-(trifluoromethyl)phenyl]ethyl]-1-(2-methoxy-4-pyridyl)-6-oxo-pyridazine-3-carboxamide | $^1$H NMR (400 MHz, DMSO-$d_6$) δ = 8.95 (d, *J* = 8.4 Hz, 1H), 8.31 (d, *J* = 5.6 Hz, 1H), 7.88 (d, *J* = 9.6 Hz, 1H), 7.48 (dd, *J* = 1.6, 5.6 Hz, 1H), 7.35 (d, *J* = 1.6 Hz, 1H), 7.17 (d, *J* = 9.6 Hz, 1H), 6.81 (d, *J* = 14.0 Hz, 2H), 6.71 (s, 1H), 5.55 (s, 2H), 5.05 (quin, *J* = 7.6 Hz, 1H), 3.92 (s, 3H), 1.47 (d, *J* = 7.2 Hz, 3H), 1.41-1.41 (m, 1H) ; LC/MS (ESI) m/z: 434.3 $[M+H]^+$ |
| 197 | 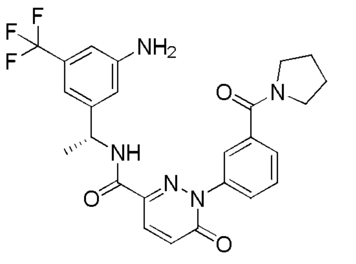 N-[(1R)-1-[3-amino-5-(trifluoromethyl)phenyl]ethyl]-6-oxo-1-[3-(pyrrolidine-1-carbonyl)phenyl]pyridazine-3-carboxamide | $^1$H NMR (400 MHz, DMSO-$d_6$) δ = 8.89 (d, *J* = 8.4 Hz, 1H), 7.89 (d, *J* = 9.6 Hz, 1H), 7.85 (s, 1H), 7.78 (dt, *J* = 2.0, 4.4 Hz, 1H), 7.63-7.58 (m, 2H), 7.15 (d, *J* = 9.6 Hz, 1H), 6.80 (d, *J* = 12.0 Hz, 2H), 6.70 (s, 1H), 5.55 (s, 2H), 5.07-4.99 (m, 1H), 3.49-3.43 (m, 4H), 1.90-1.79 (m, 4H), 1.44 (d, *J* = 7.2 Hz, 3H) ; LC/MS (ESI) m/z: 500.4 $[M+H]^+$ |

(continued)

| No. | Structure/Name | Spectral Data |
|---|---|---|
| 198 | 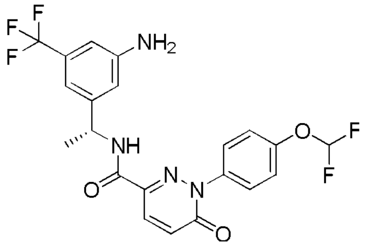 N-[(1R)-1-[3-amino-5-(trifluoromethyl)phenyl]ethyl]-1-[4-(difluoromethoxy)phenyl]-6-oxo-pyridazine-3-carboxamide | $^1H$ NMR (400 MHz, DMSO-$d_6$) δ = 8.88 (d, *J* = 8.4 Hz, 1H), 7.89 (d, *J* = 9.6 Hz, 1H), 7.77-7.72 (m, 2H), 7.52 (s, 0.25H), 7.36-7.31 (m, 2.50H), 7.16 (s, 0.47H), 7.15 (s, 0.25H), 7.14 (s, 0.52H), 6.82 (s, 1H), 6.78 (s, 1H), 6.70 (s, 1H), 5.56 (s, 2H), 5.04 (quin, *J* = 7.2 Hz, 1H), 1.44 (d, *J* = 7.2 Hz, 3H) ; LC/MS (ESI) m/z: 469.3 $[M+H]^+$ |
| 199 | 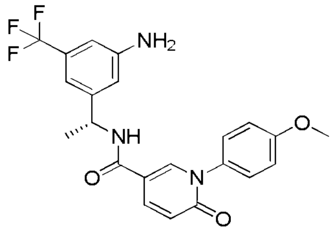 N-[(1R)-1-[3-amino-5-(trifluoromethyl)phenyl]ethyl]-1-(4-methoxyphenyl)-6-oxo-pyridine-3-carboxamide | $^1H$ NMR (400 MHz, DMSO-$d_6$) δ = 8.57 (d, *J* = 7.6 Hz, 1H), 8.33 (d, *J* = 2.4 Hz, 1H), 7.94 (dd, *J* = 2.4, 9.6 Hz, 1H), 7.43-7.34 (m, 2H), 7.11-7.04 (m, 2H), 6.75 (s, 2H), 6.69 (s, 1H), 6.51 (d, *J* = 9.6 Hz, 1H), 5.54 (s, 2H), 5.00 (quin, *J* = 7.2 Hz, 1H), 3.81 (s, 3H), 1.39 (d, *J* = 7.2 Hz, 3H) ; LC/MS (ESI) m/z: 432.3 $[M+H]^+$ |
| 200 | 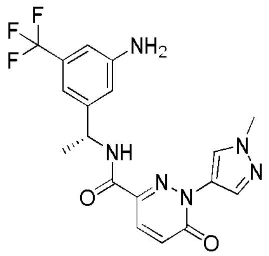 N-[(1R)-1-[3-amino-5-(trifluoromethyl)phenyl]ethyl]-1-(1-methylpyrazol-4-yl)-6-oxo-pyridazine-3-carboxamide | $^1H$ NMR (400 MHz, DMSO-$d_6$) δ = 8.90 (d, *J* = 8.4 Hz, 1H), 8.55 (s, 1H), 8.36 (s, 1H), 7.87 (d, *J* = 9.6 Hz, 1H), 7.16 (d, *J* = 9.6 Hz, 1H), 6.86 (s, 1H), 6.82 (s, 1H), 6.72 (s, 1H), 5.57 (s, 2H), 5.10 (quin, *J* = 7.2 Hz, 1H), 3.91 (s, 3H), 1.52 (d, *J* = 7.2 Hz, 3H) ; LC/MS (ESI) m/z: 40 7.3 $[M+H]^+$ |
| 201 | 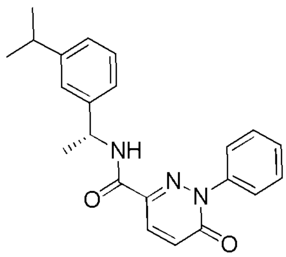 N-[(1R)-1-(3-isopropylphenyl)ethyl]-6-oxo-1-phenyl-pyridazine-3-carboxamide | $^1H$ NMR (400 MHz, DMSO-$d_6$) δ = 8.79 (d, *J* = 8.4 Hz, 1H), 7.89 (d, *J* = 9.6 Hz, 1H), 7.71-7.63 (m, 2H), 7.56-7.50 (m, 2H), 7.49-7.43 (m, 1H), 7.25 (s, 1H), 7.24-7.16 (m, 2H), 7.14 (d, *J* = 9.6 Hz, 1H), 7.10 (d, *J* = 7.2 Hz, 1H), 5.12 (quin, *J* = 7.2 Hz, 1H), 2.85 (td, *J* = 6.8, 13.6 Hz, 1H), 1.47 (d, *J* = 7.2 Hz, 3H), 1.18 (d, J = 6.8 Hz, 6H) ; LC/MS (ESI) m/z: 362.4 $[M+H]^+$ |
| 202 | 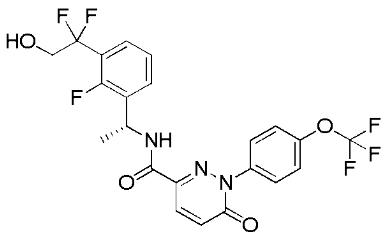 N-[(1R)-1-[3-(1,1-difluoro-2-hydroxy-ethyl)-2-fluoro-phenyl]ethyl]-6-oxo-1-[4-(trifluoromethoxy)phenyl]pyridazine-3-carboxamide | $^1H$ NMR (400 MHz, DMSO-$d_6$) δ = 9.00 (d, *J* = 8.0 Hz, 1H), 7.94-7.80 (m, 3H), 7.64 (t, *J* = 7.0 Hz, 1H), 7.57 (d, *J* = 8.4 Hz, 2H), 7.43 (t, *J* = 6.8 Hz, 1H), 7.31-7.24 (m, 1H), 7.16 (d, *J* = 10.0 Hz, 1H), 5.71 (br s, 1H), 5.41 (q, *J* = 7.2 Hz, 1H), 3.92 (t, *J* = 14.4 Hz, 2H), 1.48 (d, *J* = 7.2 Hz, 3H) ; LC/MS (ESI) m/z: 502.1 $[M+H]^+$ |

(continued)

| No. | Structure/Name | Spectral Data |
|---|---|---|
| 203 | 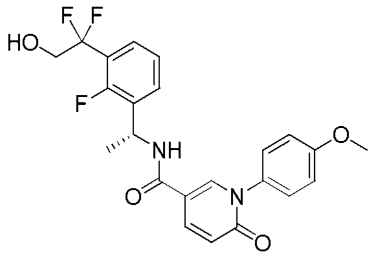 N-[(1R)-1-[3-(1,1-difluoro-2-hydroxy-ethyl)-2 -fluoro-phenyl] ethyl]-1-(4-methoxyphenyl)-6-oxo-pyridine-3-carboxamide | $^1$H NMR (400 MHz, DMSO-$d_6$) δ = 8.70 (br d, *J* = 7.6 Hz, 1H), 8.39 (d, *J* = 2.8 Hz, 1H) , 7.92 (dd, *J* = 2.8, 9.6 Hz, 1H), 7.57 (t, *J* = 7.2 Hz, 1H) , 7.46-7.37 (m, 3H), 7.31-7.24 (m, 1H), 7.11-7.06 (m, 2H), 6.51 (d, *J* = 9.6 Hz, 1H), 5.75 (br s, 1H), 5.35 (quin, *J* = 7.2 Hz, 1H), 3.92 (t, *J* = 14.4 Hz, 2H), 3 .82 (s, 3H), 1.43 (d, *J* = 7.2 Hz, 3H) LC/MS (ESI) m/z: 447.3 $[M+H]^+$ |
| 204 | 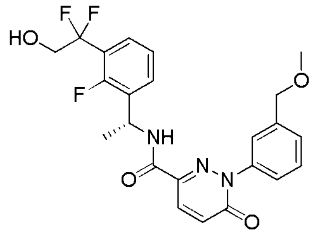 N-[(1R)-1-[3-(1,1-difluoro-2-hydroxy-ethyl)-2-fluoro-phenyl] ethyl]-1-[3-(methoxymethyl)phenyl]-6-oxo-pyridazine-3-carboxamide | $^1$H NMR (400 MHz, CHLORO-FORM-d) δ = 8.00 (d, *J* = 9.6 Hz, 1H), 7.40-7.58 (m, 7H), 7.16-7.22 (m, 1H), 7.09 (d, *J* = 9.6 Hz, 1H), 5.38-5.48 (m, 1H), 4.53-4.60 (m, 2H), 3.98-4.13 (m, 2H), 3.44 (s, 3H), 2.64-2.72 (m, 1H), 1.59 (s, 3H) ; LC/MS (ESI) m/z: 462.2 $[M+H]^+$ |
| 205 | 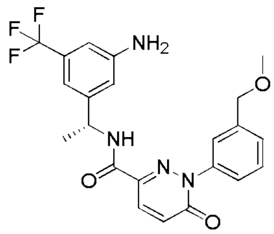 N-[(1R)-1-[3-amino-5-(trifluoromethyl)phenyl]ethyl]-1-[3-(methoxymethyl)phenyl]-6-oxo-pyridazine-3-carboxamide | 1H NMR (400 MHz, CHLOROFORM-d) δ = 8.01 (d, J = 9.6 Hz, 1H), 7.41-7.55 (m, 4H), 7.29 (d, J = 8.0 Hz, 1H), 7.09 (d, J = 9.8 Hz, 1H), 7.00 (s, 1H), 6.89 (d, J = 11.2 Hz, 2H), 5.19 (q, J = 7.2 Hz, 1H), 4.53-4.55 (m, 2H), 3.43 (s, 3H), 1.54 (d, *J* = 6.8 Hz, 3H); LC/MS (ESI) m/z: 447.2 $[M+H]^+$ |
| 206 | 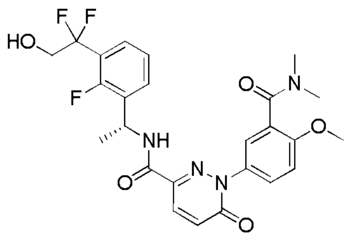 N-[(1R)-1-[3-(1,1-difluoro-2-hydroxy-ethyl)-2-fluoro-phenyl] ethyl]-1-[3-(dimethylcarbamoyl)-4-methoxy-phenyl]-6-oxo-pyridazine-3-carboxamide | 1H NMR (400 MHz, DMSO-$d_6$) δ = 8.98 (d, J = 8.0 Hz, 1H), 7.84 (d, J = 9.6 Hz, 1H), 7.69 (dd, J = 2.4, 8.8 Hz, 1H), 7.63 (t, J = 7.2 Hz, 1H), 7.51 (d, J = 2.8 Hz, 1H), 7.43 (t, J = 7.2 Hz, 1H), 7.31 - 7.21 (m, 2H), 7.12 (d, J = 9.6 Hz, 1H), 5.72 (t, J = 6.6 Hz, 1H), 5.44 - 5.34 (m, 1H), 3.97 - 3.88 (m, 2H), 3.87 (s, 3H), 2.98 (s, 3H), 2.82 (s, 3H), 1.47 (d, J = 7.1 Hz, 3H); LC/MS (ESI) m/z: 519.4 $[M+H]^+$ |
| 207 | 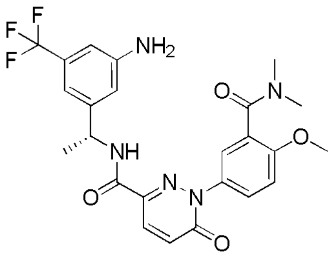 N-[(1R)-1-[3-amino-5-(trifluoromethyl)phenyl]ethyl]-1-[3-(dimethylcarbamoyl)-4-methoxy-phenyl]-6-oxo-pyridazine-3-carboxamide | 1H NMR (400 MHz, DMSO-$d_6$) δ = 8.87 (d, J = 8.3 Hz, 1H), 7.86 (d, J = 9.6 Hz, 1H), 7.68 (dd, J = 2.4, 8.8 Hz, 1H), 7.51 (d, J = 2.4 Hz, 1H), 7.22 (d, J = 9.2 Hz, 1H), 7.12 (d, J = 9.6 Hz, 1H), 6.80 (br d, J = 13.2 Hz, 2H), 6.70 (s, 1H), 5.55 (s, 2H), 5.02 (quin, J = 7.2 Hz, 1H), 3.86 (s, 3H), 2.97 (s, 3H), 2.80 (s, 3H), 1.44 (d, J = 7.0 Hz, 3H) ; LC/MS (ESI) m/z: 504.4 $[M+H]^+$ |

(continued)

| No. | Structure/Name | Spectral Data |
|---|---|---|
| 208 | 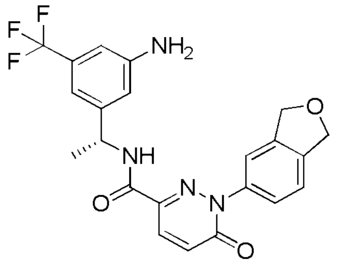 N-[(1R)-1-[3-amino-5-(trifluoromethyl)phenyl]ethyl]-1-(1,3-dihydroisobenzofuran-5-yl)-6-oxo-pyridazine-3-carboxamide | $^1$H NMR (400 MHz, DMSO-$d_6$) δ = 8.83 (d, *J* = 8.4 Hz, 1H), 7.89 (d, *J* = 9.6 Hz, 1H), 7.62 - 7.49 (m, 2H), 7.46 (d, *J*= 7.6 Hz, 1H), 7.14 (d, *J* = 9.6 Hz, 1H), 6.82 (s, 1H), 6.77 (s, 1H), 6.70 (s, 1H), 5.55 (s, 2H), 5.07 - 4.99 (m, 5H), 1.44 (d, *J* = 7.2 Hz, 3H) ; LC/MS (ESI) m/z: 445.4 $[M+H]^+$ |
| 209 | 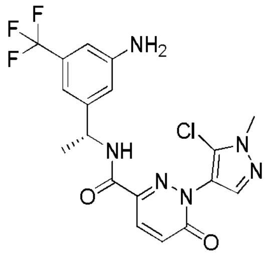 N-[(1R)-1-[3-amino-5-(trifluoromethyl)phenyl]ethyl]-1-(5-chloro-1-methyl-pyrazol-4-yl)-6-oxo-pyridazine-3-carboxamide | $^1$H NMR (400 MHz, DMSO-$d_6$) δ = 8.85 (d, *J* = 8.4 Hz, 1H), 7.92 (s, 1H), 7.89 (d, *J* = 9.6 Hz, 1H), 7.16 (d, *J* = 9.6 Hz, 1H), 6.81 (s, 1H), 6.77 (s, 1H), 6.70 (s, 1H), 5.56 (s, 2H), 5.01 (quin, *J* = 7.2 Hz, 1H), 3.88 (s, 3H), 1.43 (d, *J* = 7.2 Hz, 3H) ; LC/MS (ESI) m/z: 441.3 $[M+H]^+$ |
| 210 | 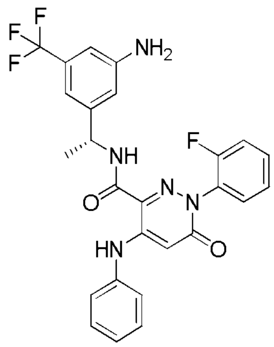 N-[(1R)-1-[3-amino-5-(trifluoromethyl)phenyl]ethyl]-4-anilino-1-(2-fluorophenyl)-6-oxo-pyridazine-3-carboxamide | $^1$H NMR (400 MHz, DMSO-$d_6$) δ ppm 1.45 (d, *J* = 7.2 Hz, 3 H) 5.06 (m, 1 H) 5.57 (s, 2 H) 6.05 (s, 1 H) 6.71 (s, 1 H) 6.80 (br d, *J* = 11.6 Hz, 2 H) 7.24 (m, 1 H) 7.40 (m, 6 H) 7.54 (m, 1 H) 7.62 (m, 1 H) 9.15 (m, 1 H) 10.11 (s, 1 H) ; LC/MS (ESI) m/z: 512.4 $[M+H]^+$ |
| 211 | 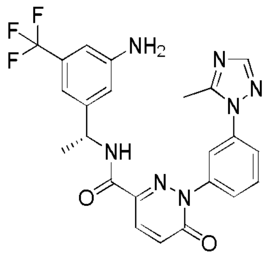 N-[(1R)-1-[3-amino-5-(trifluoromethyl)phenyl]ethyl]-1-[3-(5-methyl-1,2,4-triazol-1-yl)phenyl]-6-oxo-pyridazine-3-carboxamide | $^1$H NMR (400 MHz, DMSO-$d_6$) δ = 9.20 (s, 1H), 8.91 (d, J = 8.4 Hz, 1H), 8.14 (d, J = 1.2 Hz, 1H), 7.97 - 7.88 (m, 2H), 7.74 - 7.67 (m, 2H), 7.18 (d, J = 9.6 Hz, 1H), 6.80 (br d, J = 13.6 Hz, 2H), 6.70 (s, 1H), 5.55 (s, 2H), 5.04 (quin, J = 7.2 Hz, 1H), 2.37 (s, 3H), 1.44 (d, J = 7.2 Hz, 3H) ; LC/MS (ESI) m/z: 484.4 $[M+H]^+$ |

(continued)

| No. | Structure/Name | Spectral Data |
|---|---|---|
| 212 | 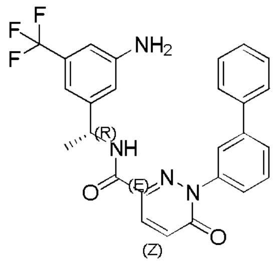 N-[(1R)-1-[3-amino-5-(trifluoromethyl)phenyl]ethyl]-6-oxo-1-(3-phenylphenyl)pyridazine-3-carboxamide | $^1$H NMR (400 MHz, DMSO-$d_6$) δ = 8.90 (d, *J* = 8.4 Hz, 1H), 7.95 - 7.89 (m, 2H), 7.80 - 7.75 (m, 1H), 7.72 (d, *J* = 7.2 Hz, 2H), 7.67 - 7.61 (m, 2H), 7.52 - 7.47 (m, 2H), 7.43 - 7.38 (m, 1H), 7.17 (d, *J* = 9.6 Hz, 1H), 6.82 (s, 1H), 6.79 (s, 1H), 6.70 (s, 1H), 5.53 (s, 2H), 5.04 (quin, *J* = 7.2 Hz, 1H), 1.44 (d, *J* = 6.8 Hz, 3H) ; LC/MS (ESI) m/z: 351.4 [M+H]$^+$ |
| 213 | 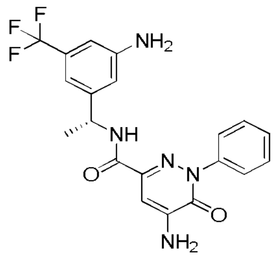 5-amino-N-[(1R)-1-[3-amino-5-(trifluoromethyl)phenyl]ethyl]-6-oxo-1-phenyl-pyridazine-3-carboxamide | $^1$H NMR (400 MHz, DMSO-$d_6$) δ = 8.51 (d, *J* = 8.4 Hz, 1H), 7.67 (d, *J* = 7.6 Hz, 2H), 7.52 (t, *J* = 7.2 Hz, 2H), 7.45-7.40 (m, 1H), 7.00-6.77 (m, 4H), 6.76 (s, 1H), 6.69 (s, 1H), 5.54 (s, 2H), 5.00 (quin, *J* = 7.2 Hz, 1H), 1.42 (d, *J* = 7.2 Hz, 3H); MS (ESI) m/z = 418.4 [M+H]$^+$. |
| 214 | 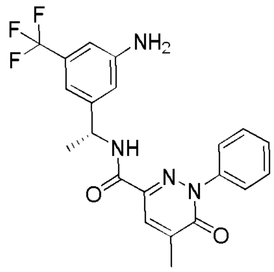 N-[(1R)-1-[3-amino-5-(trifluoromethyl)phenyl]ethyl]-5-methyl-6-oxo-1-phenyl-pyridazine-3-carboxamide | $^1$H NMR (400 MHz, DMSO-$d_6$) δ = 8.79 (d, *J* = 8.4 Hz, 1H), 7.82 (d, *J* = 1.2 Hz, 1H), 7.66 (d, *J* = 7.6 Hz, 2H), 7.53 (t, *J* = 7.2 Hz, 2H), 7.48 - 7.43 (m, 1H), 6.81 (s, 1H), 6.78 (s, 1H), 6.70 (s, 1H), 5.54 (s, 2H), 5.03 (quin, *J* = 7.2 Hz, 1H), 2.19 - 2.17 (m, 3H), 1.44 (d, *J*= 7.2 Hz, 3H); MS (ESI) m/z = 417.2 [M+H]$^+$. |

**Example 215: N-[(1R)-1-[3-(1,1-difluoro-2-hydroxy-ethyl)-2-fluoro-phenyl]ethyl]-1-(2-fluoro-4-methoxy-phenyl)-6-oxo-pyridazine-3-carboxamide**

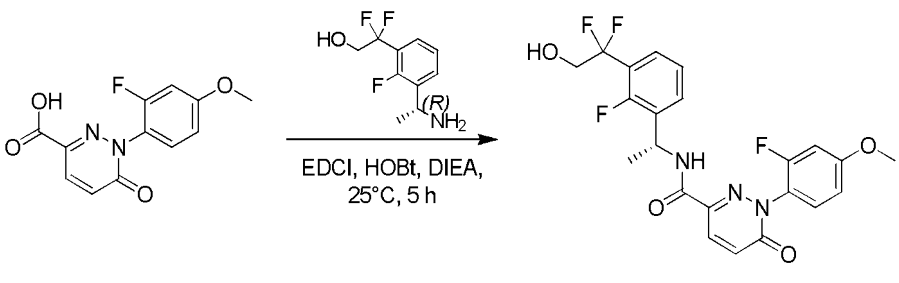


215

To a solution of 1-(2-fluoro-4-methoxy-phenyl)-6-oxo-pyridazine-3-carboxylic acid (125 mg, 473.11 μmol) and 2-[3-[(1R)-1-aminoethyl]-2-fluoro-phenyl]-2,2-difluoro-ethanol (120.96 mg, 473.11 μmol, HCl salt) in DMF (4 mL), DIEA (183.44 mg, 1.42 mmol, 247.22 μL), HOBt (127.86 mg, 946.22 μmol) and EDCI (181.39 mg, 946.22 μmol) were added, and the mixture was degassed, purged with $N_2$ for three times, and stirred at 25 °C for 5 h under $N_2$ atmosphere. The reaction mixture was poured into water (30 mL) and extracted with EtOAc (30 mL × 3). The combined organic layer was washed with brine (50 mL), dried over $Na_2SO_4$ and concentrated under reduced pressure to obtain a crude product, which

was purified by silica gel column chromatography (1% MeOH in DCM) to give yellow oil. The product was purified by prep-HPLC (column: Welch Xtimate C18 150*30 mm*5 μm; mobile phase: [water ($NH_3H_2O$+$NH_4HCO_3$)-ACN];B%: 20%-60%, 28 min). ACN was removed under reduced pressure, and the remaining solvent was removed by lyophilization to obtain the compound of Example 215 (20 mg, 9.08% yield) as a white solid. $^1H$ NMR (400MHz, DMSO-$d_6$) δ 8.99 (br d, *J* = 8.0 Hz, 1H), 7.91 (d, *J* = 10.0 Hz, 1H), 7.67 - 7.54 (m, 2H), 7.43 (br t, *J* = 6.8 Hz, 1H), 7.31 - 7.24 (m, 1H), 7.16 (d, *J* = 10.0 Hz, 1H), 7.08 (dd, *J* = 2.8, 12.0 Hz, 1H), 6.98 (dd, *J*= 2.4, 9.2 Hz, 1H), 5.71 (t, *J* = 6.4 Hz, 1H), 5.41 (br t, *J*= 7.6 Hz, 1H), 3.92 (br d, *J* = 6.8 Hz, 2H), 3.86 (s, 3H), 1.47 (d, *J* = 7.2 Hz, 3H); LC/MS (ESI) m/z = 466.3 $[M+H]^+$.

**Example 216: N-[(1R)-1-(5-amino-3-cyano-2-fluoro-phenyl)ethyl]-1-(2-fluorophenyl)-6-oxo-pyridazine-3-carboxamide**

Step 1: Synthesis of 3-bromo-2-fluoro-5-nitro-benzonitrile

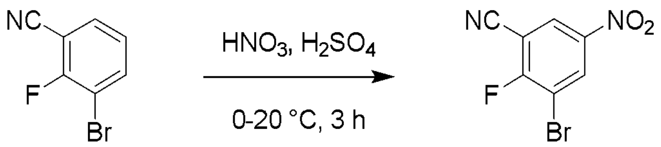


To a solution of 3-bromo-2-fluoro-benzonitrile (10 g, 50.00 mmol) in $H_2SO_4$ (40 mL) was added $HNO_3$ (17.520 g, 278.04 mmol, 12.51 mL) slowly at 0°C for 40 minutes, and the reaction mixture was stirred at 20 °C for 3 h. The reaction mixture was poured into ice-cold water (70 mL) and extracted with EtOAc (40 mL × 3). The combined organic layer was washed with brine (50 mL), dried over $Na_2SO_4$ and concentrated under reduced pressure to obtain a crude product. The product was purified by silica gel column chromatography (14% MeOH in DCM) to give 3-bromo-2-fluoro-5-nitro-benzonitrile (7.57 g, 61.80% yield) as yellow oil. $^1H$ NMR (400MHz, DMSO-$d_6$) δ 8.92 (m, 1 H) 8.95 (m, 1 H).

Step 2: Synthesis of 3-acetyl-2-fluoro-5-nitro-benzonitrile

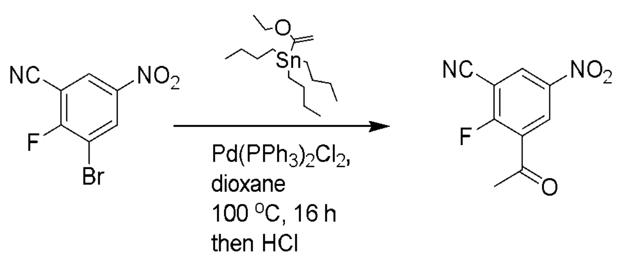


To a mixture of 3-bromo-2-fluoro-5-nitro-benzonitrile (4.5 g, 18.37 mmol) and tributyl(1-ethoxyvinyl)stannane (7.67 g, 21.23 mmol, 7.16 mL) in dioxane (50 mL), $Pd(PPh_3)_2Cl_2$ (1.29 g, 1.84 mmol) and TEA (3.72 g, 36.74 mmol, 5.11 mL) were added, and the reaction mixture was stirred at 100 °C for 16 h under $N_2$ atmosphere. Then, the reaction mixture was cooled to 0°C, treated with 4M HCl (30 mL) and stirred for 3 h. The reaction mixture was diluted with water (50 mL) and extracted with EtOAc (50 mL × 3). The product was dried over $Na_2SO_4$ and filtered, and the filtrate was concentrated. The aqueous layer was adjusted to pH= 9 with aq. NaOH, and then sodium hypochlorite (100 mL) was added slowly under stirring. The product was purified by silica gel column chromatography (14% EtOAc in PE) to give 3-acetyl-2-fluoro-5-nitro-benzonitrile (2.58 g, 67.45% yield) as a yellow solid. $^1H$ NMR (400MHz, DMSO-$d_6$) δ 2.69 (d, *J* = 4.0 Hz, 3H) 8.77 (dd, *J* = 6.0, 3.2 Hz, 1H) 9.14 (dd, *J*= 4.8, 2.8 Hz, 1H).

Step 3: Synthesis of (NZ,R)-N-[1-(3-cyano-2-fluoro-5-nitro-phenyl)ethylidene]-2-methyl-propane-2-sulfinamide

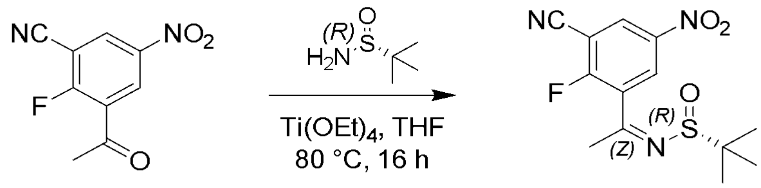


To a solution of 3-acetyl-2-fluoro-5-nitro-benzonitrile (2.58 g, 12.40 mmol) and (R)-2-methylpropane-2-sulfinamide (2.25 g, 18.59 mmol) in THF (25 mL) was added $Ti(OEt)_4$ (8.48 g, 37.19 mmol, 7.71 mL), and the reaction mixture was stirred at 80 °C for 16 h under $N_2$ atmosphere. The reaction mixture was poured into water (30 mL) and extracted with EtOAc (30 mL×3). The combined organic layer was washed with brine (50 mL), dried over $Na_2SO_4$ and concentrated under reduced pressure to obtain a crude product, which was purified by silica gel column chromatography (14% EtOAc in PE) to give (NZ,R)-N-[1-(3-cyano-2-fluoro-5-nitrophenyl)ethylidene]-2-methyl-propane-2-sulfinamide (1.18 g, 30.58% yield) as yellow oil. $^1H$ NMR (400MHz, DMSO-$d_6$) δ 1.24 (s, 9 H) 2.75 (d, *J* = 1.6 Hz, 3H) 8.76 (br dd, *J* = 5.6, 2.6 Hz, 1 H) 9.04 (dd, *J* = 4.4, 2.8 Hz, 1H).

Step 4: Synthesis of (R, R)-N-[1-(3-cyano-2-fluoro-5-nitro-phenyl)ethylidene]-2-methylpropane-2-sulfinamide

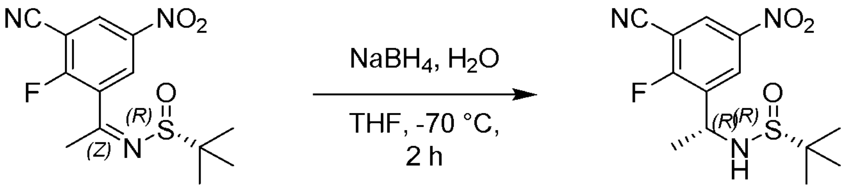


To a solution of (R,R)-N-[1-(3-cyano-2-fluoro-5-nitro-phenyl)ethylidene]-2-methylpropane-2-sulfinamide (1.18 g, 3.79 mmol) in THF (12 mL) and $H_2O$ (0.5 mL) was added $NaBH_4$ (170 mg, 4.49 mmol), and the reaction mixture was stirred at -70 °C for 2 h under $N_2$ atmosphere. The reaction mixture was quenched with sat. aq. $NH_4Cl$ (50 mL) at 20°C, diluted with EtOAc (50 mL) and extracted with EtOAc (50 mL * 3). The organic layer was dried over $Na_2SO_4$ and filtered under reduced pressure to obtain a crude product. The product was purified by prep-HPLC (column: Phenomenex C18 75*30 mm*3 μm, mobile phase: [water($NH_3H_2O$+$NH_4HCO_3$)-ACN];B%: 24%-56%,11 min). ACN was removed under reduced pressure, and the remaining solvent was removed by lyophilization to give (R, R)-N-[1-(3-cyano-2-fluoro-5-nitrophenyl)ethylidene]-2-methyl-propane-2-sulfinamide (156 mg, 13.08% yield) as a yellow solid. $^1H$ NMR (400MHz, DMSO-$d_6$) δ 1.13 (s, 9H) 1.45 (s, 3H) 4.03 (q, *J* = 7.2 Hz, 1 H) 8.80 (dd, *J* = 6.0, 2.8 Hz, 1 H) 8.85 (m, 1H); LC/MS (ESI) m/z = 314.1 $[M+H]^+$.

Step 5: Synthesis of 3-[(1R)-1-aminoethyl]-2-fluoro-5-nitro-benzonitrile

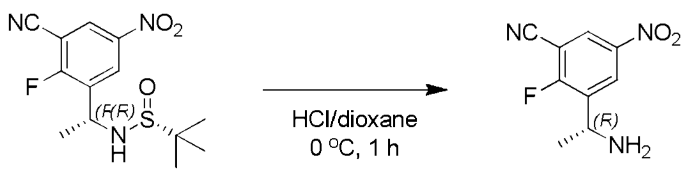


**Intermediate AW**

(R,R)-N-[1-(3-cyano-2-fluoro-5-nitro-phenyl)ethylidene]-2-methyl-propane-2-sulfinamide (156 mg, 497.85 μmol) was added to 4N HCl/dioxane (3 mL), and the mixture was stirred at 0 °C for 1 hour. The mixture was filtered under reduced pressure to give Intermediate AW (104 mg, 85.04% yield, HCl salt) as a yellow solid. LC/MS (ESI) m/z = 210.1 $[M+H]^+$.

Step 6: Synthesis of N-[(1R)-1-(3-cyano-2-fluoro-5-nitro-phenyl)ethyl]-1-(2-fluorophenyl)-6-oxo-pyridazine-3-carboxamide

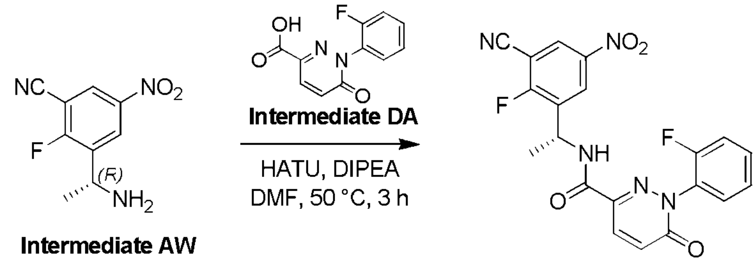


To a solution of Intermediate AW (40 mg, 162.84 μmol, HCl salt) and Intermediate DA (34.67 mg, 148.04 μmol) in DMF (3 mL), HATU (73.17 mg, 192.45 μmol) and DIEA (57.40 mg, 444.11 μmol, 77.36 μL) were added, and the reaction mixture was stirred at 50 °C for 3 h under $N_2$. The reaction mixture was poured into water (30 mL) and extracted with EtOAc (30 mL×3). The combined organic layer was washed with brine (50 mL), dried over anhydrous $Na_2SO_4$ and concentrated under reduced pressure to give N-[(1R)-1-(3-cyano-2-fluoro-5-nitro-phenyl)ethyl]-1-(2-fluorophenyl)-6-oxo-pyridazine-3-carboxamide (120 mg, 55.27% yield) as yellow oil. LC/MS (ESI) m/z = 426.0 $[M+H]^+$.

Step 7: Synthesis of N-[(1R)-1-(5-amino-3-cyano-2-fluoro-phenyl)ethyl]-1-(2-fluorophenyl)-6-oxo-pyridazine-3-carboxamide

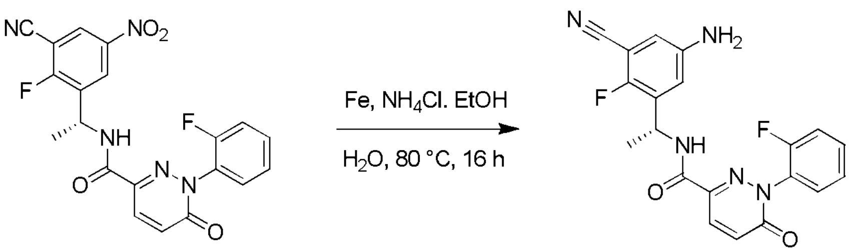


216

To a solution of N-[(1R)-1-(3-cyano-2-fluoro-5-nitro-phenyl)ethyl]-1-(2-fluorophenyl)-6-oxo-pyridazine-3-carboxamide (120.00 mg, 282.12 μmol) in EtOH (2.5 mL) and $H_2O$ (0.5 mL), Fe (78.78 mg, 1.41 mmol) and $NH_4Cl$ (75.46 mg, 1.41 mmol) were added, and the reaction mixture was stirred at 85 °C for 3 h under $N_2$. The reaction mixture was filtered, and the filtrate was concentrated under reduced pressure and purified by prep-HPLC (column: Phenomenex C18 75*30 mm*3 μm, mobile phase: [water($NH_3H_2O$+$NH_4HCO_3$)-ACN];B%: 24%-54%,14 min). ACN was removed under reduced pressure, and the remaining solvent was removed by lyophilization to give the compound of Example 216 (6.8 mg, 16.02, 5.68% yield) as a yellow solid. $^1H$ NMR (400 MHz, DMSO-$d_6$) δ 1.42 (d, *J* = 7.2 Hz, 3 H) 5.18 (quin, *J* = 7.2 Hz, 1 H) 5.45 (s, 2 H) 6.72 (dd, *J* = 4.8, 2.81 Hz, 1 H), 6.87 (dd, *J* = 6.4, 2.75 Hz, 1 H) 7.19 (d, *J* = 10.0 Hz, 1 H) 7.44 (m, 2 H) 7.59 (m, 1 H) 7.69 (td, *J* = 7.6, 1.56 Hz, 1 H) 7.93 (d, *J* = 10.0 Hz, 1 H) 9.00 (d, *J* = 7.6 Hz, 1 H); LC/MS (ESI) m/z = 396.3 $[M+H]^+$.

**Example 217: N-[(1R)-1-[3-(1,1-difluoro-2-hydroxy-ethyl)-2-fluoro-phenyl]ethyl]-1-(2-fluorophenyl)-6-oxo-pyridazine-3-carboxamide**

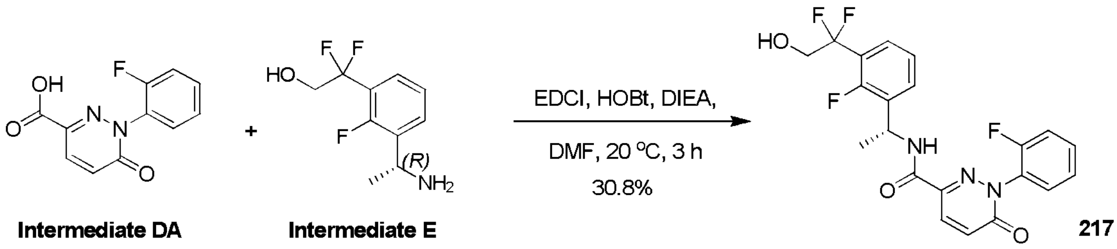


Intermediate DA Intermediate E 217

To a solution of Intermediate DA (30.13 mg, 128.66 μmol) and Intermediate E (31.02 mg, 141.52 μmol) in DMF (2 mL), EDCI (49.33 mg, 257.32 μmol), HOBt (34.77 mg, 257.32 μmol) and DIEA (49.88 mg, 385.98 μmol, 67.23 μL) were added, vacumming of the reaction vessel and purging with nitrogen gas were performed three times, and then the mixture was stirred at 25 °C for 3 h. After LC/MS confirmed the consumption of the starting materials and the MS of the product, the reaction mixture was poured into water (30 mL) and extracted with EtOAc. The combined organic layer was washed with brine, dried over $Na_2SO_4$ and concentrated under reduced pressure to obtain a crude product. The product was purified by prep-HPLC (column: Phenomenex C18 75*30mm*3μm, mobile phase: [water($NH_3H_2O$+$NH_4HCO_3$)-ACN];B%: 22%-52%,14 min), and the resulting mixture was subjected to the lyophilization condition, thereby obtaining the compound of Example 217 (17.3 mg, 30.8% yield) as a pink solid. $^1$H NMR (400 MHz, DMSO-$d_6$) δ 9.03 (d, J = 8.0 Hz, 1H), 7.92 (d, J = 10.0 Hz, 1H), 7.69 (dt, J = 1.6, 7.6Hz, 1H), 7.64 - 7.56 (m, 2H), 7.49 - 7.40 (m, 3H), 7.27 (t, J = 7.6 Hz, 1H), 7.18 (d, J = 9.6 Hz, 1H), 5.71 (br s, 1H), 5.40 (quin, J = 7.2 Hz, 1H), 3.91 (t, J = 14.4 Hz, 2H), 1.46 (d, J = 7.2 Hz, 3H); LC/MS (ESI) m/z = 436.0 $[M+H]^+$

**Example 218: N-[(1R)-1-[3-amino-5-(trifluoromethyl)phenyl]ethyl]-1-(2-fluorophenyl)-6-oxo-pyridine-3-carboxamide**

Step 1: Synthesis of methyl-1-(2-fluorophenyl)-6-oxo-pyridine-3-carboxylate

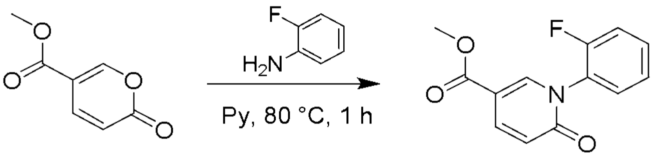


A mixture of methyl-6-oxopyran-3-carboxylate (18 g, 116.79 mmol) and 2-fluoroaniline (14.28 g, 128.47 mmol, 12.41 mL) in pyridine (180 mL) was stirred at 60 °C for 1 hour. The reaction mixture was concentrated under reduced pressure to obtain a residue, which was purified by silica gel column chromatography(11% EtOAc in PE) to obtain methyl-1-(2-fluorophenyl)-6-oxo-pyridine-3-carboxylate (11 g, 21.19% yield, 55.61% purity) as yellow oil. LC/MS (ESI) m/z = 247.9 $[M+H]^+$.

Step 2: Synthesis of 1-(2-fluorophenyl)-6-oxo-pyridine-3-carboxylic acid

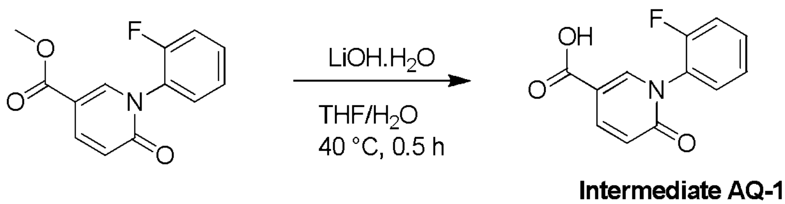


Intermediate AQ-1

To a solution of methyl-1-(2-fluorophenyl)-6-oxo-pyridine-3-carboxylate (10 g, 40.45 mmol) in THF (180 mL), $LiOH \cdot H_2O$ (5.09 g, 121.35 mmol) and $H_2O$ (40 mL) were added. The mixture was stirred at 40 °C for 30 minutes. The reaction mixture was poured into water (50 mL) and extracted with EtOAc (40 mL × 2). The organic layer was discarded, and the mixture was adjusted to pH 3-4 with aq. 1N HCl and extracted with EtOAc (50 mL × 7). The combined organic layer was washed with brine (60 mL × 6), dried over $Na_2SO_4$ and concentrated under reduced pressure, and the crude product

was recrystallized with EtOAc (20 mL) at 20 °C and filtered to give Intermediate AQ-1 (4.4 g, 38.52% yield, 82.59% purity) as a yellow solid. LC/MS (ESI) m/z = 234.1 [M+H]+.

Step 3: Synthesis of N-[(1R)-1-[3-amino-5-(trifluoromethyl)phenyl]ethyl]-1-(2-fluorophenyl)-6-oxo-pyridine-3-carboxamide

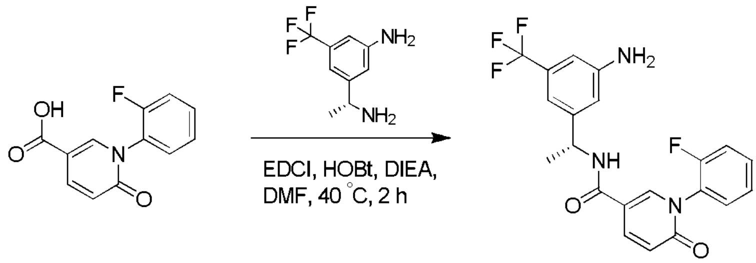


**218**

To a solution of 1-(2-fluorophenyl)-6-oxo-pyridine-3-carboxylic acid (4.4 g, 18.87 mmol) and 3-[(1R)-1-aminoethyl]-5-(trifluoromethyl)aniline (4.09 g, 16.98 mmol, HCl salt) in DMF (60 mL), DIEA (7.32 g, 56.61 mmol, 9.86 mL), HOBt (3.82 g, 28.30 mmol) and EDCI (5.43 g, 28.30 mmol) were added, and the mixture was degassed and purged with $N_2$ for three times, followed by stirring at 40 °C for 2 h under $N_2$ atmosphere. The reaction mixture was poured into water (30 mL) and extracted with EtOAc (30 mL × 3). The combined organic layer was washed with brine (50 mL), dried over $Na_2SO_4$, and concentrated under reduced pressure to obtain a crude product, which was purified by silica gel column chromatography (73% EtOAc in PE) to give a main product, the compound of Example 218 (4.40 g, 55.27% yield) as an off-white solid. $^1$H NMR (400MHz, DMSO-$d_6$) δ 8.59 (br d, *J* = 7.6 Hz, 1H), 8.38 (d, *J* = 2.4 Hz, 1H), 7.99 (dd, *J* = 2.4, 9.6 Hz, 1H), 7.66 - 7.53 (m, 2H), 7.52 - 7.34 (m, 2H), 6.76 (s, 2H), 6.70 (s, 1H), 6.57 (d, *J* = 9.6 Hz, 1H), 5.58 (s, 2H), 5.00 (br t, *J* = 7.2 Hz, 1H), 1.39 (d, *J* = 7.2 Hz, 3H); LC/MS (ESI) m/z = 421.3 [M+H]+.

**Example 219: N-[(1R)-1-(3-fluoro-5-hydroxy-phenyl)ethyl]-6-oxo-1-phenyl-pyridazine-3-carboxamide**

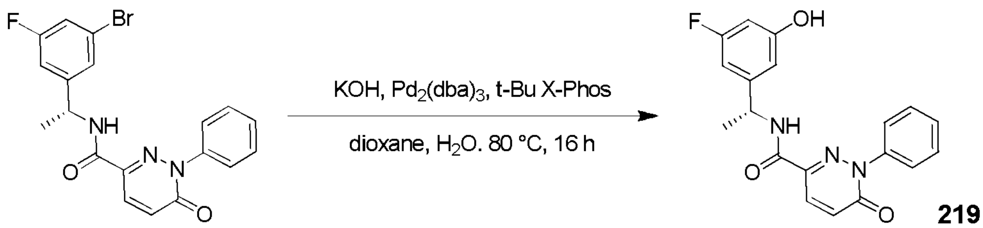


**219**

A mixture of N-[(1R)-1-(3-bromo-5-fluoro-phenyl)ethyl]-6-oxo-1-phenyl-pyridazine-3-carboxamide (125 mg, 0.300 mmol), KOH (84.2 mg, 1.50 mmol), t-Bu X-Phos (15.3 mg, 0.0360 mmol, 0.12 eq) and $Pd_2(dba)_3$ (11.0 mg, 0.0120 mmol, 0.04 *eq*) in dioxane (1 mL) and $H_2O$ (0.6 mL) was degassed and purged with $N_2$ for 3 times, and then the mixture was stirred at 80 °C for 16 h under $N_2$ atmosphere. The reaction mixture was poured into water (20 mL) and extracted with EtOAc (30 mL × 3). The combined organic layer was washed with brine (20 mL ×3), dried over anhydrous $Na_2SO_4$ and concentrated under reduced pressure to obtain a crude product. The product was purified by silica gel column chromatography (50% EtOAc in PE) to obtain a residue, which was purified by prep-HPLC (C18-6 100*30mm*5μm; mobile phase: [water (FA)-ACN]; B%: 32%-62%, 15min). Most of ACN was removed under reduced pressure, and the remaining solvent was removed by lyophilization to give the compound of Example 219 (15.4 mg, 13.54% yield) as a light-yellow solid. $^1$H NMR (400MHz, DMSO-$d_6$) δ = 9.84 (s, 1H), 8.80 (d, *J* = 8.4 Hz, 1H), 7.89 (d, *J* = 9.6 Hz, 1H), 7.70-7.62 (m, 2H), 7.57-7.51 (m, 2H), 7.50-7.44 (m, 1H), 7.14 (d, *J*= 9.6 Hz, 1H), 6.65-6.58 (m, 2H), 6.40 (td, *J* = 2.4, 10.8 Hz, 1H), 5.03 (quin, *J* = 7.2 Hz, 1H), 1.43 (d, *J* = 7.2 Hz, 3H); MS (ESI) m/z = 354.1[M+H]+.

**Example 220: N-[(1R)-1-[3-amino-5-(trifluoromethyl)phenyl]ethyl]-4-chloro-6-oxo-1-phenyl-pyridazine-3-carboxamide**

Step 1: Synthesis of 4-hydroxy-6-oxo-1-phenyl-pyridazine-3-carboxylic acid

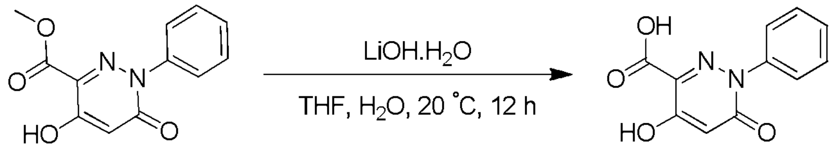


4-hydroxy-6-oxo-1-phenyl-pyridazine-3-carboxylic acid was prepared by reacting methyl-4-hydroxy-6-oxo-1-phenyl-pyridazine-3-carboxylate obtained from Step 2 of Preparation Example 8 in the same manner as in Step 1 of Example 76.

Step 2: Synthesis of 4-chloro-6-oxo-1-phenyl-pyridazine-3-carbonyl chloride

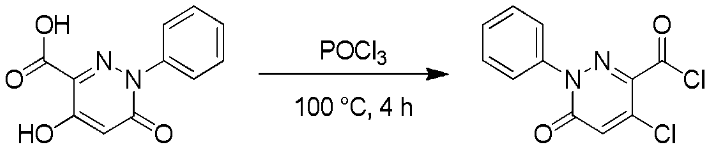


A solution of 4-hydroxy-6-oxo-1-phenyl-pyridazine-3-carboxylic acid (400 mg, 1.72 mmol) in $POCl_3$ (9.90 g, 64.6 mmol, 6.00 mL) was stirred at 100 °C for 4 h under $N_2$. The reaction mixture was concentrated under reduced pressure to give 4-chloro-6-oxo-1-phenyl-pyridazine-3-carbonyl chloride (450 mg, crude) as brown oil, which was used in next step without further purification. MS (ESI) m/z = 265.0 $[M-2-H]^+$).

Steps 3 and 4: Synthesis of N-[(1R)-1-[3-amino-5-(trifluoromethyl)phenyl]ethyl]-4-chloro-6-oxo-1-phenyl-pyridazine-3-carboxamide

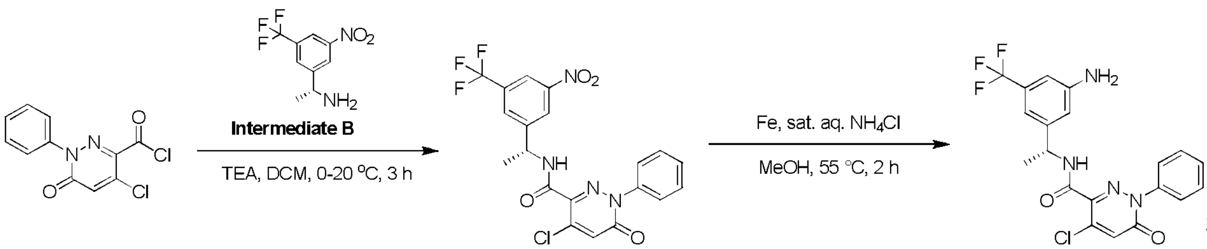


**220**

The compound of Example 220 (28.0 mg, 39.90% yield) was obtained in the same manner as in Steps 3 and 4 of Example 20 as a white solid. $^1H$ NMR (400MHz, DMSO-$d_6$) $\delta$ = 9.23 (d, $J$ = 8.0 Hz, 1H), 7.63-7.57 (m, 2H), 7.56-7.49 (m, 3H), 7.49-7.44 (m, 1H), 6.79 (d, $J$ = 14.0 Hz, 2H), 6.72 (s, 1H), 5.57 (s, 2H), 4.96 (quin, $J$ = 7.2 Hz, 1H), 1.40 (d, $J$ = 7.2 Hz, 3H); MS (ESI) m/z = 437.3 $[M+H]^+$.

**Example 221: N-[(1R)-1-[3-amino-5-(trifluoromethyl)phenyl]ethyl]-6-oxo-1H-pyridazine-3-carboxamide**

Step 1: Synthesis of methyl-6-oxo-1-(2-trimethylsilylethoxymethyl)pyridazine-3-carboxylate

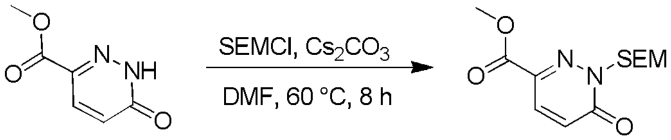


To a solution of methyl-6-oxo-1H-pyridazine-3-carboxylate (1 g, 6.49 mmol) in DMF (15 mL), $Cs_2CO_3$ (6.34 g, 19.46 mmol) and 2-(chloromethoxy)ethyl-trimethyl-silane (2.70 g, 16.22 mmol, 2.87 mL) were added. The mixture was stirred at 60 °C for 8 h. The reaction mixture was poured into water (20 mL) and extracted with EtOAc (30 mL ×3). The combined organic layer was washed with brine (20 mL × 3), dried over anhydrous $Na_2SO_4$ and concentrated under reduced pressure to obtain a crude product. The product was purified by silica gel column chromatography (1% EtOAc in PE) to give methyl-6-oxo-1-(2-trimethylsilylethoxymethyl)pyridazine-3-carboxylate (650 mg, 34.22% yield) as light-yellow oil. $^1$H NMR (400MHz, DMSO-$d_6$) δ = 7.83 (d, *J* = 4.4 Hz, 1H), 6.96 (d, *J* = 4.0 Hz, 1H), 5.54 (d, *J* = 2.4 Hz, 2H), 3.96 (s, 3H), 3.76-3.72 (m, 2H), 1.19-1.09 (m, 2H), 0.08 (s, 9H); MS (ESI) m/z = 227.0 $[M+H-58]^+$.

Steps 2 to 5: Synthesis of N-[(1R)-1-[3-amino-5-(trifluoromethyl)phenyl]ethyl]-6-oxo-1H-pyridazine-3-carboxamide

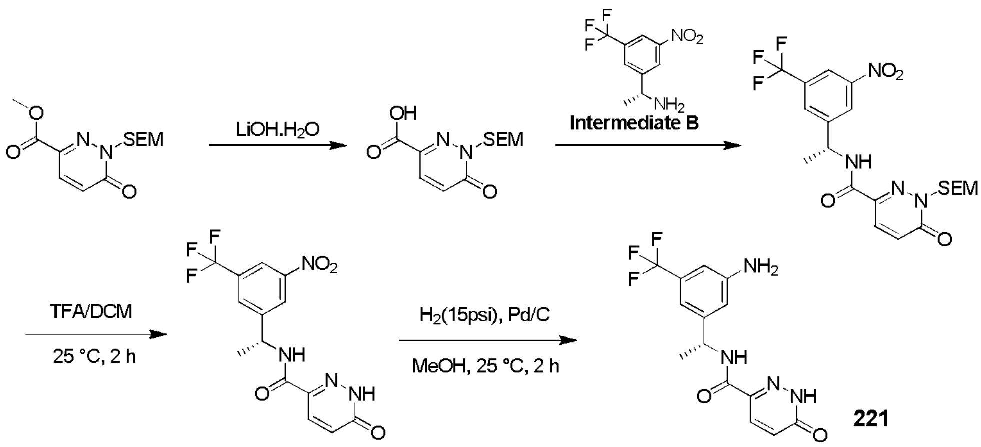


**221**

The compound of Example 221 (25.7 mg, 46.77% yield) was obtained as a white solid in a similar manner to Steps 2 to 5 of Example 79, except that Steps 4 and 5 of Example 79 were exchanged from each other and Pd/C catalyst was used instead of Pt-V/C in Step 4 of Example 79. $^1$H NMR (400MHz, DMSO-$d_6$) δ = 9.67 (d, *J* = 8.0 Hz, 1H), 9.12 (br s, 1H), 8.89 (s, 1H), 8.80 (s, 1H), 8.32 (d, *J* = 10.0 Hz, 1H), 7.54 (d, *J* = 10.0 Hz, 1H), 5.95 (q, *J* = 9.6 Hz, 2H), 2.08 (d, *J* = 7.2 Hz, 3H); MS (ESI) m/z = 327.3 $[M+H]^+$.

**Example 222: N-[(1R)-1-[3-amino-5-(trifluoromethyl)phenyl]ethyl]-1-(2-methoxyethyl)-6-oxo-pyridazine-3-carboxamide**

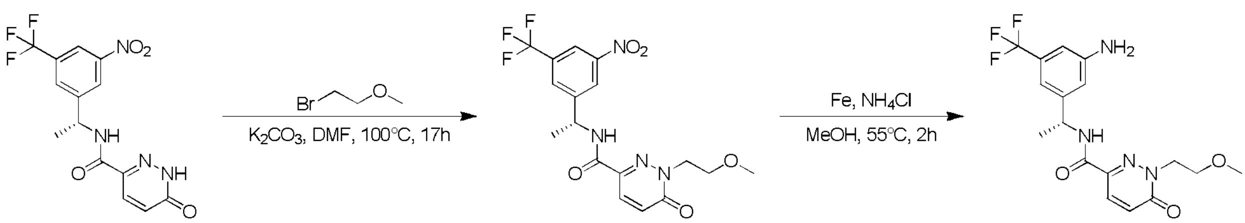


**222**

The compound of Example 222 (10.3 mg, 32.53% yield) was obtained as a white solid by reacting 1-[3-nitro-5-(trifluoromethyl)phenyl]ethyl]-6-oxo-1H-pyridazine-3-carboxamide obtained from Step 4 of Example 221 and 1-bromo-2-methoxy-ethane in the same manner as in Step 1 of Example 118 and Step 4 of Example 89. $^1$H NMR (400MHz, DMSO-$d_6$) δ = 8.81 (d, *J* = 8.8 Hz, 1H), 7.81 (d, *J* = 9.6 Hz, 1H), 7.01 (d, *J* = 9.6 Hz, 1H), 6.81 (d, *J* = 16.4 Hz, 2H), 6.71 (s, 1H),

5.57 (s, 2H), 5.09 - 5.00 (m, 1H), 4.36 - 4.29 (m, 2H), 3.77 (t, *J* = 6.0 Hz, 2H), 3.25 (s, 3H), 1.47 (d, *J*= 7.2 Hz, 3H); MS (ESI) m/z = 385.1 $[M+H]^+$.

**Example 223 to Example 228**

The compounds of Examples 223 to 228 were obtained in the same manner as in Example 222 by using appropriate starting materials corresponding to the respective structures of the desired compounds.

[Table 12]

| No. | Structure/Name | Spectral Data |
|---|---|---|
| 223 | 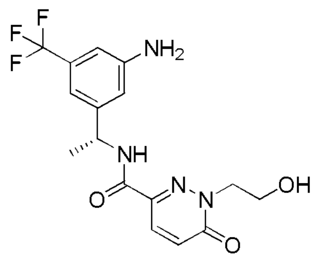 N-[(1R)-1-[3-amino-5-(trifluoromethyl)phenyl]ethyl]-1-(2-hydroxyethyl)-6-oxo-pyridazine-3-carboxamide | $^1H$ NMR (400 MHz, DMSO-$d_6$) δ = 8.78 (br d, *J* = 8.2 Hz, 1H), 7.81 (d, *J* = 9.6 Hz, 1H), 7.00 (d, *J* = 9.6 Hz, 1H), 6.83 (s, 1H), 6.80 (s, 1H), 6.72 (s, 1H), 5.56 (s, 2H), 5.04 (quin, *J* = 7.2 Hz, 1H), 4.87 (t, *J* = 5.6 Hz, 1H), 4.28 - 4.16 (m, 2H), 3.80 (q, *J* = 6.0Hz, 2H), 1.48 (d, *J* = 7.2 Hz, 3H); LC/MS (ESI) m/z: 371.1 $[M+H]^+$ |
| 224 | 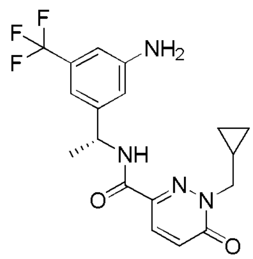 N-[(1R)-1-[3-amino-5-(trifluoromethyl)phenyl]ethyl]-1-(cyclopropylmethyl)-6-oxo-pyridazine-3-carboxamide | $^1H$ NMR (400 MHz, DMSO-$d_6$) δ = 8.81 (d, *J* = 8.4 Hz, 1H), 7.82 (d, *J* = 10.0 Hz, 1H), 7.01 (d, *J* = 9.6 Hz, 1H), 6.83 (s, 1H), 6.78 (s, 1H), 6.71 (s, 1H), 5.57 (s, 2H), 5.09 - 4.99 (m, 1H), 4.07 - 3.93 (m, 2H), 1.47 (d, *J* = 7.2 Hz, 3H), 1.42 - 1.34 (m, 1H), 0.53 - 0.41 (m, 4H); LC/MS (ESI) m/z: 381.1 $[M+H]^+$ |
| 225 | 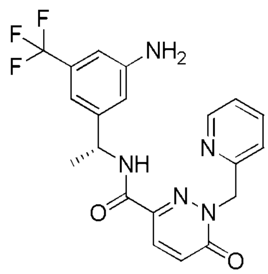 N-[(1R)-1-[3-amino-5-(trifluoromethyl)phenyl]ethyl]-6-oxo-1-(2-pyridyl-methyl)pyridazine-3-carboxamide | $^1H$ NMR (400 MHz, DMSO-$d_6$) δ ppm 1.44 (d, *J* = 7.00 Hz, 3H) 5.02 (t, *J* = 7.6 Hz, 1H) 5.41-5.51 (m, 2H) 5.55 (s, 2H) 6.70 (s, 1H) 6.79 (br d, *J* = 8.8 Hz, 2H) 7.06 (d, *J* = 10 Hz, 1H) 7.25-7.32 (m, 2H) 7.77 (td, *J* = 7.6, 1.69 Hz, 1H) 7.89 (d, *J* = 10 Hz, 1H) 8.48 (d, *J* = 4.4 Hz, 1H) 8.85 (d, *J* = 8.4 Hz, 1H); LC/MS (ESI) m/z: 418.3 $[M+H]^+$ |
| 226 | 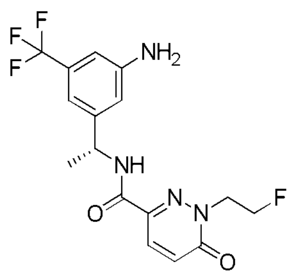 N-[(1R)-1-[3-amino-5-(trifluoromethyl)phenyl]ethyl]-1-(2-fluoroethyl)-6-oxo-pyridazine-3-carboxamide | $^1H$ NMR (400 MHz, DMSO-$d_6$) δ = 8.86 - 8.77 (m, 1H), 7.89 - 7.80 (m, 1H), 7.04 (d, *J* = 9.6 Hz, 1H), 6.80 (br d, *J* = 11.2 Hz, 2H), 6.71 (s, 1H), 5.56 (s, 2H), 5.04 (t, *J* = 7.4 Hz, 1H), 4.95 (t, *J* = 4.8 Hz, 1H), 4.84 (t, *J* = 5.2 Hz, 1H), 4.52 - 4.40 (m, 2H), 1.47 (d, *J* = 7.2 Hz, 3H); LC/MS (ESI) m/z: 373.3 $[M+H]^+$ |

(continued)

| No. | Structure/Name | Spectral Data |
|---|---|---|
| 227 | 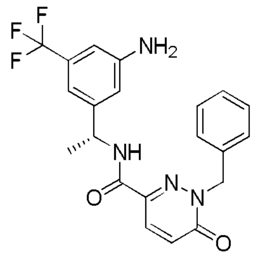 N-[(1R)-1-[3-amino-5-(trifluoromethyl)phenyl]ethyl]-1-benzyl-6-oxo-pyridazine-3-carboxamide | $^1$H NMR (400 MHz, DMSO-$d_6$)$\delta$ = 8.88 (d, *J* = 8.4 Hz, 1H), 7.84 (d, *J* = 10.0 Hz, 1H), 7.39 - 7.29 (m, 5H), 7.03 (d, *J* = 9.6 Hz, 1H), 6.84 (s, 1H), 6.79 (s, 1H), 6.71 (s, 1H), 5.57 (s, 2H), 5.33 (s, 2H), 5.04 ( t, *J* = 7.6 Hz, 1H), 1.48 (d, *J* = 7.2 Hz, 3H); LC/MS (ESI) m/z: 417.3 [M+H]$^+$ |
| 228 | 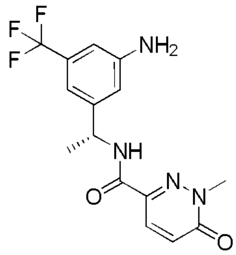 N-[(1R)-1-[3-amino-5-(trifluoromethyl)phenyl]ethyl]-1-methyl-6-oxo-pyridazine-3-carboxamide | $^1$H NMR (400 MHz, DMSO-$d_6$) $\delta$ = 8.85 (d, *J* = 8.4 Hz, 1H), 7.82 (d, *J* = 9.6 Hz, 1H), 7.00 (d, *J* = 9.6 Hz, 1H), 6.83 (s, 1H), 6.79 (s, 1H), 6.70 (s, 1H), 5.56 (s, 2H), 5.07 - 4.97 (m, 1H), 3.75 (s, 3H), 1.46 (d, *J* = 7.2 Hz, 3H); LC/MS (ESI) m/z: 341.3 [M+H]$^+$ |

**Example 229: N-[(1R)-1-[3-amino-5-(trifluoromethyl)phenyl]ethyl]-1-[2-(dimethylamino)ethyl]-6-oxo-pyridazine-3-carboxamide**

Step 1: Synthesis of 1-[2-(dimethylamino)ethyl]-N-[(1R)-1-[3-nitro-5-(trifluoromethyl)phenyl]ethyl]-6-oxo-pyridazine-3-carboxamide

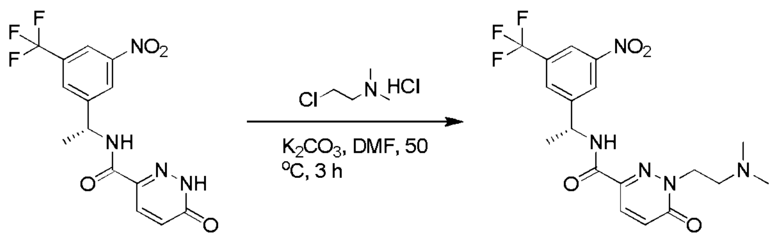


To a solution of N-[(1R)-1-[3-nitro-5-(trifluoromethyl)phenyl]ethyl]-6-oxo-1H-pyridazine-3-carboxamide (30.0 mg, 0.0842 mmol), which was obtained from Step 4 of Example 221, in DMF (1.5 mL), $K_2CO_3$ (34.9 mg, 0.253 mmol) and 2-chloro-N,N-dimethyl-ethanamine (18.2 mg, 0.126 mmol, HCl) were added. The mixture was stirred at 50 °C for 3 h. The reaction mixture was poured into water (20 mL) and extracted with EtOAc (30 mL ×3). The combined organic layer was washed with brine (20 mL ×3), dried over anhydrous $Na_2SO_4$ and concentrated under reduced pressure to give 1-[2-(dimethylamino)ethyl]-N-[(1R)-1-[3-nitro-5-(trifluoromethyl)phenyl]ethyl]-6-oxo-pyridazine-3-carboxamide (35 mg, 94.33% yield) as yellow oil, which was used in next step without purification. MS (ESI) m/z = 428.2 [M+H]$^+$.

Step 2: Synthesis of N-[(1R)-1-[3-amino-5-(trifluoromethyl)phenyl]ethyl]-1-[2-(dimethylamino)ethyl]-6-oxo-pyridazine-3-carboxamide

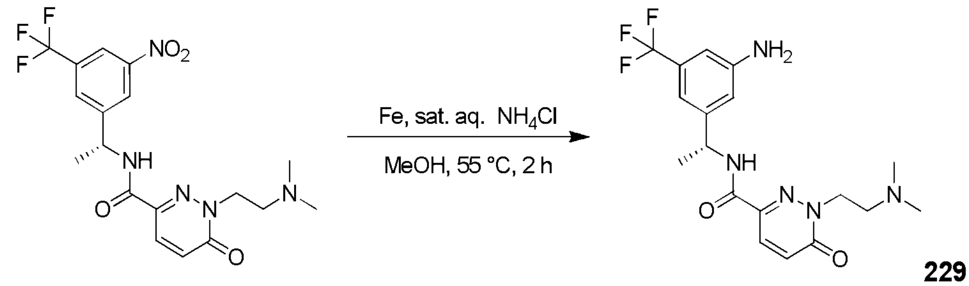


229

The compound of Example 229 (28.0 mg, 39.90% yield) was obtained as a white solid in the same manner as in Step 4 of Example 20. $^1H$ NMR (400MHz, DMSO-$d_6$) δ = 8.82 (d, *J* = 8.4 Hz, 1H), 7.81 (d, *J* = 9.6 Hz, 1H), 7.01 (d, *J* = 9.6 Hz, 1H), 6.83 (s, 1H), 6.78 (s, 1H), 6.71 (s, 1H), 5.57 (s, 2H), 5.04 (quin, *J* = 7.2 Hz, 1H), 4.39-4.21 (m, 2H), 3.11-2.68 (m, 2H), 2.38-2.10 (m, 6H), 1.47 (d, *J* = 7.2 Hz, 3H); MS (ESI) m/z = 398.4 $[M+H]^+$.

**Example 230: (R)-1-(2-fluoro-3-(pyrrolidine-1-carbonyl)phenyl)-N-(1-(3-nitro-5-(trifluoromethyl)phenyl)ethyl)-6-oxo-1,6-dihydropyridazine-3-carboxamide**

Step 1: Synthesis of methyl (R)-2-fluoro-3-(3-((1-(3-nitro-5-(trifluoromethyl)phenyl)ethyl)carbamoyl)-6-oxopyridazin-1(6H)-yl)benzoate

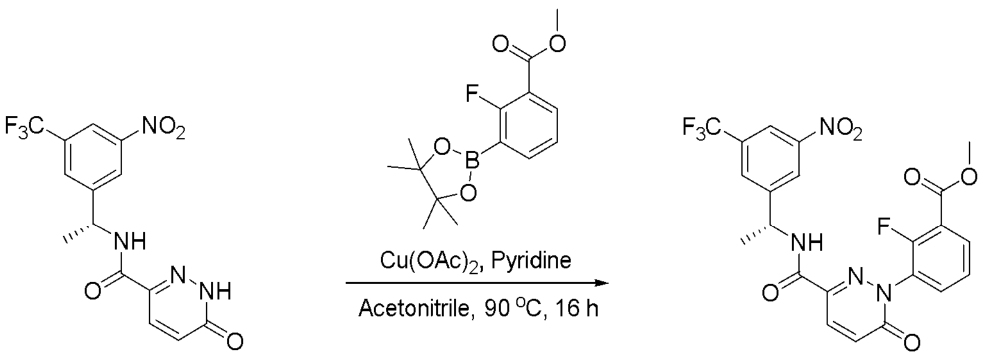


A mixture of the compound obtained from Step 4 of Example 221 (400 mg, 1.12 mmol), methyl 2-fluoro-3-(4,4,5,5-tetramethyl-1,3,2-dioxaborolan-2-yl)benzoate (472 mg, 1.68 mmol), $Cu(OAc)_2$ (204 mg, 1.12 mmol) and pyridine (362 μL, 4.49 mmol) in ACN (8.64 mL) was stirred at 90 °C for 16 h. The reaction mixture was poured into water (20 mL) and extracted with EtOAc. The combined organic layer was washed with aq. $CuSO_4.5H_2O$, dried over $Na_2SO_4$ and concentrated under reduced pressure to obtain a crude product. The product was purified by silica gel column chromatography (60% EtOAc in Hexane) to give methyl (R)-2-fluoro-3-(3-((1-(3-nitro-5-(trifluoromethyl)phenyl)ethyl) carbamoyl)-6-oxopyridazin-1(6H)-yl)benzoate (114 mg, 19.97% yield) as colorless oil. $^1H$ NMR (400MHz, CHLOROFORM-*d*) δ 8.40 (s, 2H), 8.15-8.11 (m, 1H), 8.02 (d, *J* = 10.0 Hz, 1H), 7.94 (s, 1H), 7.69-7.65 (m, 1H), 7.44-7.39 (m, 1H), 7.29 (d, *J* = 7.6 Hz, 1H), 7.14 (d, *J* = 10.0 Hz, 1H), 5.32 (quin, *J* = 7.1 Hz, 1H), 1.65 (d, *J* = 7.2 Hz, 3H); LC/MS (ESI) m/z = 509.0 $[M+H]^+$.

Step 2: Synthesis of (R)-2-fluoro-3-(3-((1-(3-nitro-5-(trifluoromethyl)phenyl)ethyl)carbamoyl)-6-oxopyridazin-1(6H)-yl) benzoic acid

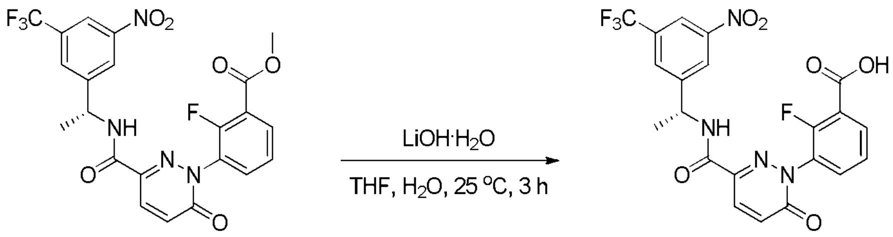


To a solution of methyl (R)-2-fluoro-3-(3-((1-(3-nitro-5-(trifluoromethyl)phenyl)ethyl)carbamoyl)-6-oxopyridazin-1(6H)-yl)benzoate (114 mg, 0.224 mmol) in THF (2.24 mL) and $H_2O$ (1.12 mL) was added $LiOH \cdot H_2O$ (28.2 mg, 0.673 mmol). The mixture was stirred at room temperature for 3 h. The reaction mixture was poured into water and extracted with EtOAc. The aqueous layer was adjusted to pH= 3-4 with aq. 1N HCl and extracted with EtOAc (10 mL × 2). The organic layer was washed with water (20 mL) and brine (20 mL), dried over $Na_2SO_4$, filtered and concentrated under reduced pressure to give (R)-2-fluoro-3-(3-((1-(3-nitro-5-(trifluoromethyl)phenyl)ethyl)carbamoyl)-6-oxopyridazin-1(6H)-yl)benzoic acid (112 mg, 100% yield) as a white solid. $^1$H NMR (400MHz, DMSO-$d_6$) δ9.20 (d, *J* = 8.0 Hz, 1H), 8.59 (s, 1H), 8.37 (s, 1H), 8.27 (s, 1H), 8.02 (t, *J* = 6.8 Hz, 1H), 7.95-7.88 (m, 2H), 7.51 (t, *J* = 7.8 Hz, 1H), 7.20 (d, *J* = 10.0 Hz, 1H), 5.36 (quin, *J* = 7.2 Hz, 1H), 1.52 (d, *J* = 7.2 Hz, 3H); LC/MS (ESI) m/z = 493.0 $[M-H]^+$.

Step 3: Synthesis of (R)-1-(2-fluoro-3-(pyrrolidin-1-carbonyl)phenyl)-N-(1-(3-nitro-5-(trifluoromethyl)phenyl)ethyl)-6-oxo-1,6-dihydropyridazine-3-carboxamide

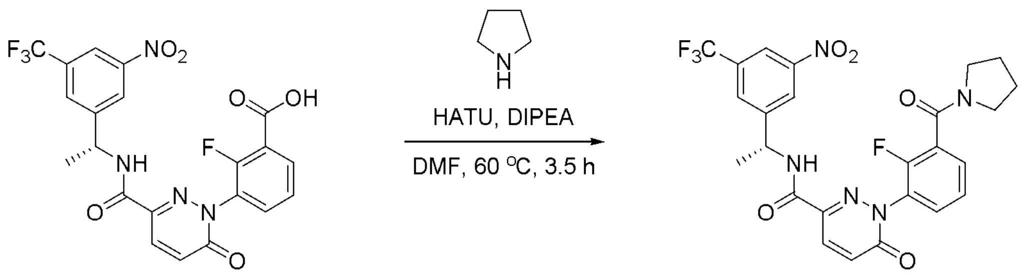


A mixture of (R)-2-fluoro-3-(3-((1-(3-nitro-5-(trifluoromethyl)phenyl)ethyl)carbamoyl)-6-oxopyridazin-1(6H)-yl) benzoic acid (19 mg, 38.4 μmol), pyrrolidine (9.47 μL, 115 μmol), DIPEA (26.8 μL, 154 μmol) and HATU (13.6 mg, 57.7 μmol) in DMF (192 μL) was degassed and purged with $N_2$ for 3 times, and then the mixture was stirred at 60 °C for 3.5 h under $N_2$ atmosphere. The reaction mixture was poured into water (10 mL) and extracted with EtOAc (10 mL × 3). The combined organic layer was washed with brine (10 mL x 4), dried over anhydrous $Na_2SO_4$ and concentrated under reduced pressure to obtain a crude product. The product was purified by silica gel column chromatography (5% MeOH in DCM) to give (R)-1-(2-fluoro-3-(pyrrolidine-1-carbonyl)phenyl)-N-(1-(3-nitro-5-(trifluoromethyl)phenyl)ethyl)-6-oxo-1,6-dihydropyridazine-3-carboxamide (19 mg, 90.30% yield) as a light-yellow solid. $^1$H NMR (400MHz, CHLOROFORM-d) δ 8.41 (s, 2H), 8.02 (d, *J* = 9.6 Hz, 1H), 7.94 (s, 1H), 7.61-7.58 (m, 1H), 7.55-7.51 (m, 1H), 7.40 (t, *J* = 8.0 Hz, 1H), 7.34 (d, *J* = 6.8 Hz, 1H), 7.13 (d, *J* = 10.0 Hz, 1H), 5.31 (quin, *J* = 7.3 Hz, 1H), 3.67 (t, *J* = 6.8 Hz, 2H), 3.40 (t, *J* = 6.6 Hz, 2H), 2.02-1.90 (m, 4H), 1.66 (d, *J* = 7.2 Hz, 3H); LC/MS (ESI) m/z = 548.1 $[M+H]^+$.

Step 4: Synthesis of (R)-1-(2-fluoro-3-(pyrrolidine-1-carbonyl)phenyl)-N-(1-(3-nitro-5-(trifluoromethyl)phenyl)ethyl)-6-oxo-1,6-dihydropyridazine-3-carboxamide

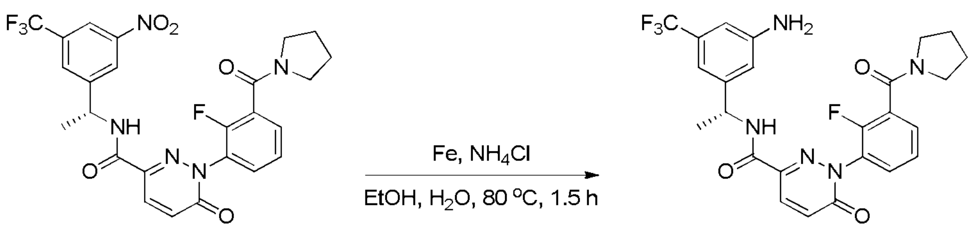


**230**

To a solution of (R)-1-(2-fluoro-3-(pyrrolidine-1-carbonyl)phenyl)-N-(1-(3-nitro-5-(trifluoromethyl)phenyl)ethyl)-6-oxo-1,6-dihydropyridazine-3-carboxamide (19 mg, 34.7 μmol) in EtOH (0.347 mL) and $H_2O$ (69.4 μL), Fe (19.4 mg, 0.347 mmol) and $NH_4Cl$ (18.6 mg, 0.347 mmol) were added, and the mixture was stirred at 80 °C for 3 h. The mixture was filtered, and the filtrate was concentrated under reduced pressure, followed by the addition of distilled water (10 mL) and then extracted with EtOAc. The combined organic layer was washed with brine, dried over anhydrous $Na_2SO_4$ and concentrated under reduced pressure to obtain a crude product. The product was purified by prep-HPLC (XBridge® Prep C18 19*250 mm*5 μm, mobile phase: [water-ACN]; B%: 50%-80%, 7min). ACN was removed under reduced pressure, and the remaining solvent was removed by lyophilization to give the compound of Example 230 (1.8 mg, 10.02% yield) as a white solid. $^1$H NMR (400 MHz, DMSO-$d_6$) δ8.97 (d, *J* = 8.0 Hz, 1H), 7.95 (d, *J* = 9.6 Hz, 1H), 7.78-7.75 (m, 1H), 7.61-7.58 (m, 1H), 7.47 (t, *J* = 7.8 Hz, 1H), 7.19 (d, *J*= 9.6 Hz, 1H), 6.79 (br d, *J* = 14.4 Hz, 2H), 6.69 (s, 1H), 5.56 (s, 2H), 5.04-5.01 (m, 1H), 3.50-3.47 (m, 2H), 3.26-3.23 (m, 2H), 1.90-1.83 (m, 4H), 1.43 (d, *J* = 7.2 Hz, 3H); LC/MS (ESI) m/z = 518.2 $[M+H]^+$.

**Example 231 to Example 234**

The compounds shown in the following table were prepared in a similar manner to Example 230 by using starting materials and intermediates corresponding to the respective structures of the desired compounds, except that the purification method was changed from prep-HPLC to silica gel column chromatography when the compounds of Examples 232 to 234 were prepared.

[Table 13]

| No. | Structure/Name | Spectral Data |
|---|---|---|
| 231 | 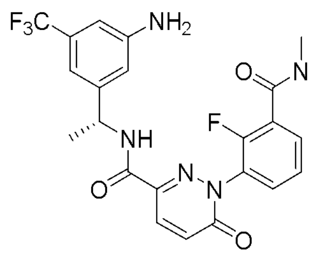 (R)-N-(1-(3-amino-5-(trifluoromethyl)phenyl)ethyl)-1-(2-fluoro-3-(methylcarbamoyl)phenyl)-6-oxo-1,6-dihydropyridazine-3-carboxamide | $^1$H NMR (400 MHz, DMSO-$d_6$) δ 8.94 (d, *J* = 8.4 Hz, 1H), 8.44-8.43 (m, 1H), 7.96 (d, *J* = 10.0 Hz, 1H), 7.80-7.73 (m, 2H), 7.46 (t, *J* = 7.8 Hz, 1H), 7.20 (d, *J*= 10.0 Hz, 1H), 6.79 (br d, *J* = 15.6 Hz, 2H), 6.69 (s, 1H), 5.56 (s, 2H), 5.02 (quin, *J* = 7.5 Hz, 1H), 2.78 (d, *J* = 4.8 Hz, 3H), 1.42 (d, *J* = 6.8 Hz, 3H); LC/MS (ESI) m/z = 478.2 $[M+H]^+$ |
| 232 | 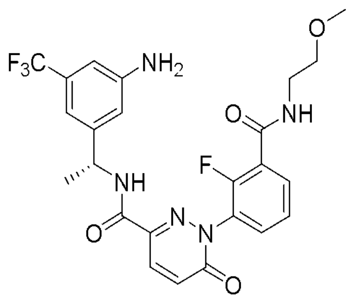 (R)-N-(1-(3-amino-5-(trifluoromethyl)phenyl)ethyl)-1-(2-fluoro-3-((2-methoxyethyl)carbamoyl)phenyl)-6-oxo-1,6-dihydropyridazine-3-carboxamide | $^1$H NMR (400 MHz, DMSO-$d_6$) δ 8.94 (d, *J* = 8.4 Hz, 1H), 8.54-8.52 (m, 1H), 7.96 (d, *J* = 9.6 Hz, 1H), 7.80-7.72 (m, 2H), 7.46 (t, *J* = 8.0 Hz, 1H), 7.21 (d, *J* = 9.6 Hz, 1H), 6.79 (br d, *J* = 15.6 Hz, 2H), 6.69 (s, 1H), 5.56 (s, 2H), 5.02 (quin, *J* = 7.5 Hz, 1H), 3.47-3.43 (m, 4H), 3.26 (s, 3H), 1.42 (d, *J* = 7.2 Hz, 3H); LC/MS (ESI) m/z = 522.2 $[M+H]^+$ |

(continued)

| No. | Structure/Name | Spectral Data |
|---|---|---|
| 233 | 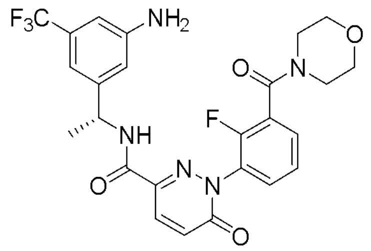 (R)-N-(1-(3-amino-5-(trifluoromethyl)phenyl)ethyl)-1-(2-fluoro-3-(morpholine-4-carbonyl)phenyl)-6-oxo-1,6-dihydropyridazine-3-carboxamide | $^1$H NMR (400 MHz, DMSO-d$_6$) δ 8.97 (d, *J* = 8.4 Hz, 1H), 7.95 (d, *J* = 10.0 Hz, 1H), 7.82-7.77 (m, 1H), 7.63-7.59 (m, 1H), 7.50 (t, *J* = 7.8 Hz, 1H), 7.20 (d, *J* = 9.6 Hz, 1H), 6.79 (br d, *J* = 13.6 Hz, 2H), 6.70 (s, 1H), 5.56 (s, 2H), 5.03 (quin, *J* = 7.2 Hz, 1H), 3.66 (s, 4H), 3.56 (s, 2H), 3.30-3.27 (m, 2H), 1.43 (d, *J* = 7.2 Hz, 3 H).; LC/MS (ESI) m/z = 534.2 [M+H]$^+$ |
| 234 | 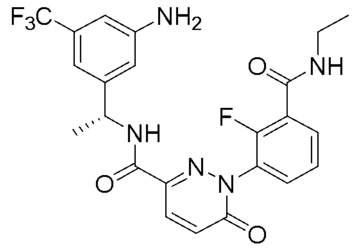 (R)-N-(1-(3-amino-5-(trifluoromethyl)phenyl)ethyl)-1-(3-(ethylcarbamoyl)-2-fluorophenyl)-6-oxo-1,6-dihydropyridazine-3-carboxamide | $^1$H NMR (400 MHz, DMSO-d$_6$) δ 8.92 (d, *J* = 8.0 Hz, 1H), 8.50 (t, *J* = 5.4 Hz, 1H), 7.95 (d, *J* = 10.0 Hz, 1H), 7.79-7.71 (m, 2H), 7.45 (t, *J* = 7.8 Hz, 1H), 7.20 (d, *J* = 9.6 Hz, 1H), 6.78 (br d, *J* = 15.2 Hz, 2H), 6.69 (s, 1H), 5.56 (s, 2H), 5.02 (quin, *J* = 7.4 Hz, 1H), 3.31-3.24 (m, 2H), 1.42 (d, *J* = 6.8 Hz, 3H), 1.11 (t, *J* = 7.2 Hz, 3H); LC/MS (ESI) m/z = 492.2 [M+H]$^+$ |

**Example 235: N-[(1R)-1-[3-amino-5-(trifluoromethyl)phenyl]ethyl]-1-[3-(1,3,4-oxadiazol-2-yl)phenyl]-6-oxo-1,6-dihydropyridazine-3-carboxamide**

Step 1: 2-[3-(4,4,5,5-tetramethyl-1,3,2-dioxaborolan-2-yl)phenyl]-1,3,4-oxadiazole

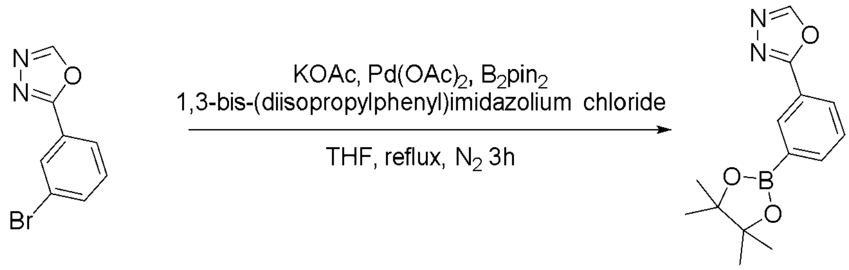


To a solution of 2-(3-bromophenyl)-1,3,4-oxadiazole (570 mg, 2.53 mmol) in THF (3.2 mL), bis(pinacolato) diborane (753 mg, 2.96 mmol), 1,3-bis-(diisopropylphenyl)-imidazolium chloride (65 mg, 0.152 mmol), Pd(OAc)$_2$ (17 mg, 0.076 mmol) and KOAc (621 mg, 6.33 mmol) were added, and the mixture was degassed and purged with N$_2$ for 2 min, and then stirred at 75 °C for 3 h. The reaction mixture was cooled to room temperature, diluted with DCM and filtered through celite. The filtrate was concentrated under reduced pressure to obtain a crude product. The product was purified by silica gel column chromatography to give 2-[3-(4,4,5,5-tetramethyl-1,3,2-dioxaborolan-2-yl)phenyl]-1,3,4-oxadiazole (580 mg, 84%) as a yellow-white solid. $^1$H NMR (400 MHz, CDCl$_3$) δ ppm 8.51 (s, 1H), 8.48 (s, 1H), 8.20 (dt, *J*=7.6, 4.0 Hz, 1H), 7.99 (dt, *J* = 7.6, 1.0 Hz, 1H), 7.37 (br, 1H), 7.54 (t, *J* = 7.8 Hz, 1H), 1.37 (s, 12 Hz); LC/MS (ESI)m/z = 273.0 [M+H]$^+$.

Step 2: N-[(1R)-1-[3-nitro-5-(trifluoromethyl)phenyl]ethyl]-1-[3-(1,3,4-oxadiazol-2-yl) phenyl]-6-oxo-1,6-dihydropyridazine-3-carboxamide

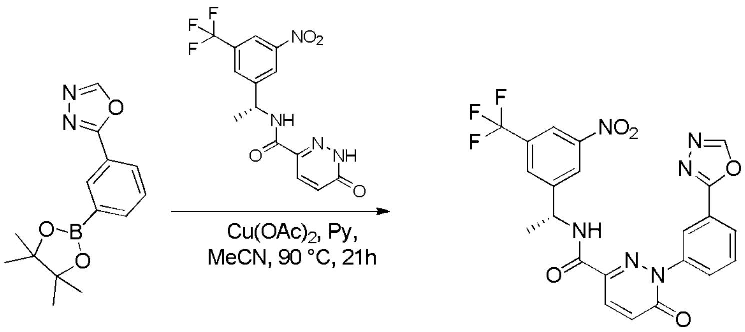


2-[3-(4,4,5,5-tetramethyl-1,3,2-dioxaborolan-2-yl)phenyl]-1,3,4-oxadiazole (100 mg, 0.281 mmol), the compound obtained from Step 4 of Example 221 (191 mg, 0.702 mmol), $Cu(OAc)_2$ (51 mg, 0.281 mmol) and pyridine (0.09 mL, 1.12 mmol) were added to acetonitrile (4 mL), and then the mixture was stirred at 90 °C for 21 h under air. The reaction mixture was cooled to room termerature and filtered under reduced pressure. The residue was purified by silica gel column chromatography to give N-[(1R)-1-[3-nitro-5-(trifluoromethyl)phenyl]ethyl]-1-[3-(1,3,4-oxadiazol-2-yl)phenyl]-6-oxo-1,6-dihydropyridazine-3-carboxamide (81 mg, 58%) as a yellow-white solid. $^1$H NMR (400 MHz, $CDCl_3$) δ ppm 8.53 (s, 1H), 8.41 (s, 1H), 8.32 (t, *J* = 1.8 Hz, 1H), 8.21 (dt, *J* = 8.0, 1.4 Hz, 1H), 8.03 (d, *J* = 10 Hz, 1H), 7.95 (s, 1H), 7.84 (dt, *J* = 2.0, 1.0 Hz, 1H), 7.73 (t, *J* = 7.8 Hz, 1H), 7.40 (d, *J* = 7.2 Hz, 1H), 7.16 (d, *J* = 10 Hz, 1H), 5.35 (quin., *J* = 7.1, 14.3 Hz, 1H), 1.67 (J= 7.2 Hz, 3H).

Step 3: N-[(1R)-1-[3-amino-5-(trifluoromethyl)phenyl]ethyl]-1-[3-(1,3,4-oxadiazol-2-yl)-phenyl]-6-oxo-1,6-dihydropyridazine-3-carboxamide

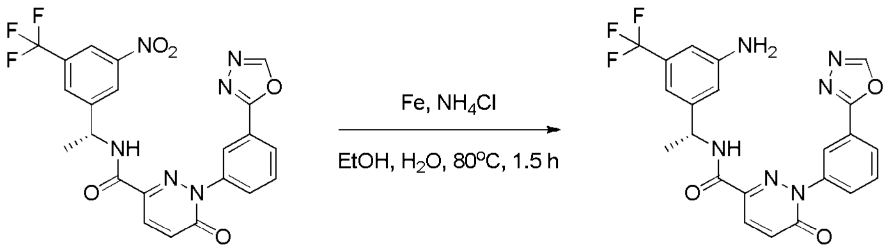


**235**

A mixture of N-[(1R)-1-[3-nitro-5-(trifluoromethyl)phenyl]ethyl]-1-[3-(1,3,4-oxadiazol-2-yl)phenyl]-6-oxo-1,6-dihydropyridazine-3-carboxamide (40 mg, 0.0799 mmol), Fe (45 mg, 0.799 mmol) and $NH_4Cl$ (43 mg, 0.799 mmol) in EtOH (0.8 mL) and $H_2O$ (0.16 mL) was degassed and purged with $N_2$, and then stirred at 80 °C for 1.5 h under $N_2$ atmosphere. After filteration, the filtrate was poured into water and extracted with DCM. The organic layer was dried over $Na_2SO_4$ and filtered under reduced pressure to obtain a crude product. The product was purified by silica gel column chromatogrphy to give the compound of Example 234 (12.7 mg, 34% yield) as a white solid. $^1$H NMR (400 MHz, DMSO-$d_6$) δ ppm 9.42 (s, 1H), 8.94 (d, *J* = 8.0 Hz, 1H), 8.35 (t, *J* = 1.8 Hz, 1H), 8.13 (dt, *J* = 7.6, 1.4 Hz, 1H), 7.99-7.96 (m, 1H), 7.92 (d, *J* = 9.6 Hz, 1H), 7.80 (t, *J* = 9.2 Hz, 1H), 7.19 (d, *J*= 9.6 Hz, 1H), 6.80 (d, *J*= 14.0 Hz, 1H), 6.70 (s, 1H), 5.56 (s, 2H), 5.05 (t, *J*= 7.8 Hz, 1H), 1.44 (d, *J*= 6.8 Hz, 3H). LC/MS: m/z 471.16 (M+H)+(ES). LC/MS (ESI)m/z = 471.2 $[M+H]^+$.

**Example 236 to Example 239**

The compounds shown in the following table were prepared in the same manner as in Example 235.

[Table 14]

| No | Structure / Name | Spectral Data |
|---|---|---|
| 236 | 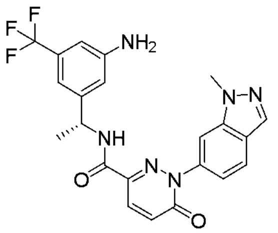 N-[(1R)-1-[3-amino-5-(trifluoromethyl)phenyl]ethyl]-1-(1-methyl-1H-indazol-6-yl)-6-oxo-1,6-dihydropyridazine-3-carboxamide | $^1$H NMR (400 MHz, DMSO-$d_6$) δ ppm 8.88 (d, *J* = 8.4 Hz, 1H), 8.16 (d, *J* = 0.8 Hz, 1H), 7.97 (d, *J* = 9.6 Hz, 1H), 7.93 (s, 1H), 7.89 (d, *J* = 8.4 Hz, 1H), 7.37 (dd, *J* = 8.6, 1.8 Hz, 1H), 7.19 (d, *J* = 9.6 Hz, 1H), 6.80 (d, *J* = 16.0 Hz, 2H), 6.70 (s, 1H), 5.56 (s, 2H), 5.05 (t, *J* = 7.6 Hz, 1H), 4.08 (s, 3H), 1.44 (d, *J* = 7.2 Hz, 3H). LC/MS (ESI) m/z = 457.2 [M+H]$^+$ |
| 237 | 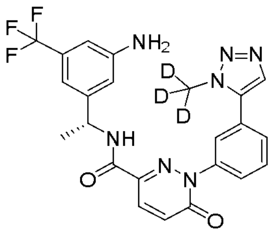 N-[(1R)-1-[3-amino-5-(trifluoromethyl)phenyl]ethyl]-1-[3-(3-(trideuteriomethyl)triazol-4-yl)phenyl]-6-oxo-pyridazine-3-carboxamide | $^1$H NMR (400 MHz, DMSO-$d_6$) δ = 8.91 (d, J = 8.4 Hz, 1H), 7.99 (s, 1H), 7.96 (s, 1H), 7.92 (d, J = 9.6 Hz, 1H), 7.88 - 7.81 (m,1H), 7.76 - 7.69 (m, 2H), 7.19 (d, J = 9.6 Hz, 1H), 6.81 (br d, J = 13.2 Hz, 2H), 6.72 (s, 1H), 5.57 (s, 2H), 5.05 (quin, J = 7.2 Hz, 1H), 1.44 (d, J = 7.2 Hz, 3H). LC/MS (ESI) m/z = 487.2 [M+H]$^+$ |
| 238 | 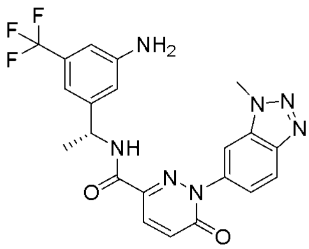 N-[(1R)-1-[3-amino-5-(trifluoromethyl)phenyl]ethyl]-1-(1-methyl-1H-1,2,3-benzotriazol-6-yl)-6-oxo-1,6-dihydropyridazine-3-carboxamide | $^1$H NMR (400 MHz, DMSO-$d_6$) δ ppm 8.90 (d, *J* = 8.0 Hz, 1H), 8.24 (s, 1H), 8.18 (d, *J* = 8.8 Hz, 1H), 7.95 (d, *J* = 10.0 Hz, 1H), 7.68 (dd, *J* = 9.0, 1.8 Hz, 1H), 6.80 (d, *J* = 15.6 Hz, 2H), 6.70 (s, 1H), 5.56 (s, 2H), 5.05 (t, *J* = 7.6 Hz, 1H), 4.35 (s, 3H), 1.44 (d, *J* = 7.2 Hz, 3H). LC/MS (ESI) m/z = 458.1 [M+H]$^+$ |
| 239 | 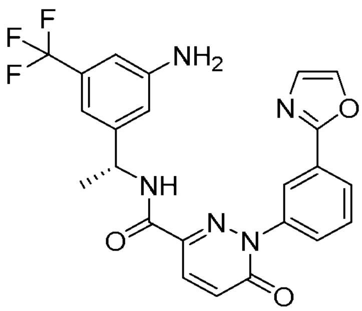 N-[(1R)-1-[3-amino-5-(trifluoromethyl)phenyl]ethyl]-1-[3-(1,3-oxazol-2-yl)phenyl]-6-oxo-1,6-dihydropyridazine-3-carboxamide | $^1$H NMR (400 MHz, DMSO-$d_6$) δ ppm 8.93 (d, *J* = 8.4 Hz, 1H), 8.28-8.27 (m, 2H), 8.07 (d, *J* = 7.6 Hz, 1H), 7.91 (d, *J* = 9.6 Hz, 1H), 7.84 (d, *J* = 9.2 Hz, 1H), 7.20 (t, *J* = 8.0 Hz, 1H), 7.44 (s, 1H), 7.18 (d, *J* = 10.0 Hz, 1H), 6.80 (d, *J* = 14.4 Hz, 2H), 6.69 (s, 1H), 5.56 (s, 2H), 5.04 (quin, *J* = 7.7, 15.2, 1H), 1.44 (d, *J* = 7.2 Hz, 3H). LC/MS (ESI) m/z = 470.1 [M+H]$^+$ |

**Example 240: N-[(1R)-1-[3-amino-5-(trifluoromethyl)phenyl]ethyl]-1-(7-fluoro-1-methyl-1H-indazol-6-yl)-6-oxo-1,6-dihydropyridazine-3-carboxamide**

Step 1: 6-bromo-7-fluoro-1-methyl-1H-indazole

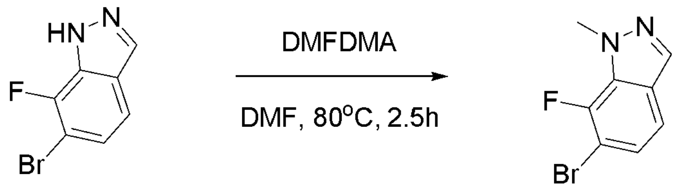


To a solution of 6-bromo-7-fluoro-1H-indazole (700 mg, 3.26 mmol) in DMF (7 mL) was added N,N-dimethylformamide dimethyl acetal (1.74 mL, 13.0 mmol), and the mixture was then stirred at 80 °C for 2.5 h. After being cooled to room temperature, the mixture was poured into water and extracted with EtOAc. The organic layer was washed with brine, dried over $Na_2SO_4$ and filtered under reduced pressure to obtain a crude product. The product was purified by silica column chromatography to give 6-bromo-7-fluoro-1-methyl-1H-indazole (392 mg, 53%) as a light-yellow solid. $^1H$ NMR (400 MHz, $CDCl_3$) δ 7.94 (d, *J* = 2.4 Hz, 1H), 7.34 (d, *J* = 8.8 Hz, 1H), 7.22-7.18 (m, 1H), 4.24 (d, *J*= 1.2 Hz, 3H); LC/MS (ESI) m/z = 228.9 $[M+H]^+$.

Step 2: 7-fluoro-1-methyl-6-(4,4,5,5-tetramethyl-1,3,2-dioxaborolan-2-yl)-1H-indazole

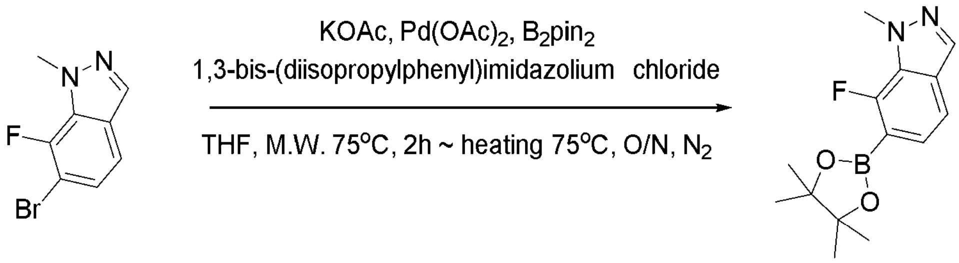


To a solution of 6-bromo-7-fluoro-1-methyl-1H-indazole (300 mg, 1.31 mmol) in THF (2 mL), bis(pinacolato) diborane (389 mg, 1.53 mmol), 1,3-bis-(diisopropylphenyl)-imidazolium chloride (34 mg, 0.0786 mmol), KOAc (321 mg, 3.27 mmol) and $Pd(OAc)_2$ (9 mg, 0.0393 mmol) were added, and the mixture was then stirred at 75 °C for 2 h with microwave irradiation. The reaction mixture was additionally stirred at 75 °C for 17 h. The reaction mixture was cooled to room temperature and filtered through celite. The filtrate was concentrated under reduced pressure to obtain a crude product. The product was purified by silica column chromatography to give 7-fluoro-1-methyl-6-(4,4,5,5-tetramethyl-1,3,2-dioxaborolan-2-yl)-1H-indazole (300 mg, 83%) as a white solid. $^1H$ NMR (400 MHz, $CDCl_3$) δ 7.94 (d, *J* = 2.4 Hz, 1H), 7.46-7.43 (m, 1H), 7.39-7.36 (m, 1H), 4.27 (d, *J* = 1.2 Hz, 3H), 1.40 (s, 12H); LC/MS (ESI) m/z = 277.0 $[M+H]^+$.

Step 3: 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide

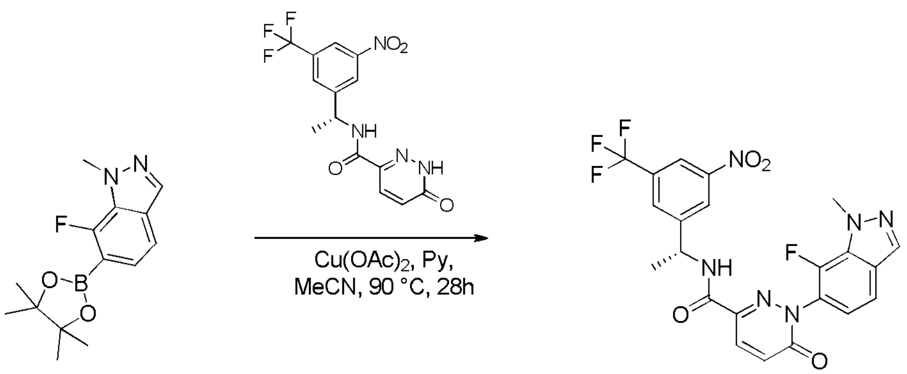


The compound obtained from Step 4 of Example 221 (150 mg, 0.421 mmol), 7-fluoro-1-methyl-6-(4,4,5,5-tetramethyl-1,3,2-dioxaborolan-2-yl)-1H-indazole (291 mg, 1.05 mmol), $Cu(OAc)_2$ (77 mg, 0.421 mmol) and pyridine (0.14 mL, 1.68 mmol) were added to acetonitrile (6 mL), and the mixture was then stirred at 90 °C for 28 h under air. The

mixture was cooled to room temperature, poured into water and extracted with EtOAc. The organic layers were washed with aq. $CuSO_4.5H_2O$, dried over $Na_2SO_4$ and concentrated under reduced pressure to obtain a crude product. The product was purified by silica column chromatography to give 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide (97 mg, 46%) as an off-white solid. $^1H$ NMR (400 MHz, $CDCl_3$) δ 8.40 (s, 2H), 8.06-8.02 (m, 2H), 7.93 (s, 1H), 7.63 (d, *J* = 8.8 Hz, 1H), 7.36 (d, *J* = 7.2 Hz, 1H), 7.18-7.12 (m, 2H), 5.33 (quin, *J* = 6.3, 13.2 Hz, 1H), 4.30 (s, 3H), 1.63 (d, *J* = 6.8 Hz, 3H); LC/MS (ESI) m/z = 505.1 $[M+H]^+$.

Step 4: 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide

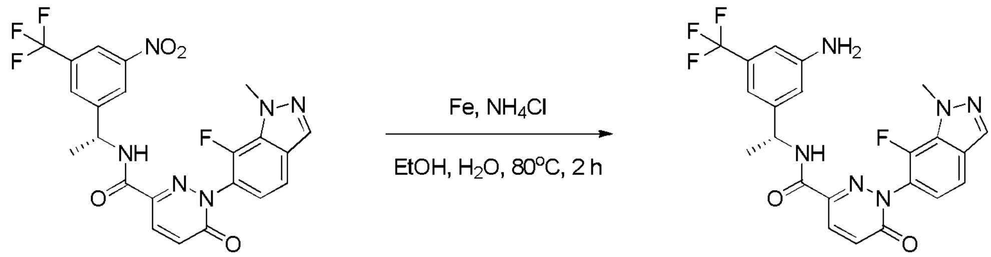


**240**

A mixture of 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide (97 mg, 0.192 mmol), Fe (107 mg, 1.92 mmol) and $NH_4Cl$ (103 mg, 1.92 mmol) in EtOH (2.0 mL) and $H_2O$ (0.8 mL) was degassed, purged with $N_2$ and stirred at 80 °C for 2 h under $N_2$ atmosphere. After filteration, the filtrate was poured into water and extracted with DCM. The organic layer was dried over $Na_2SO_4$ and filtered under reduced pressure to obtain a crude product. The product was purified by silica gel column chromatography to give the compound of Example 240 (77 mg, 85% yield) as a light-pink solid. $^1H$ NMR (400 MHz, DMSO-$d_6$) δ 8.90 (d, *J* = 8.4 Hz, 1H), 8.24(d, *J* = 2.4 Hz, 1H), 7.97 (d, *J* = 9.6 Hz, 1H), 7.75 (d, *J* = 8.4 Hz, 1H), 7.32(dd, *J* = 8.4, 6.0 Hz, 1H), 7.22 (d, *J* = 10.0 Hz, 1H), 6.78 (d, *J* = 14.0 Hz, 2H), 6.69 (s, 1H), 5.56 (s, 2H), 5.03 (quin, *J* = 7.7, 15.2 Hz, 1H), 4.21 (s, 3H), 1.42 (d, *J* = 7.2 Hz, 1H); LC/MS (ESI) m/z = 475.1 $[M+H]^+$.

**Example 241: (R)-N-(1-(3-amino-5-(trifluoromethyl)phenyl)ethyl)-5-bromo-6-oxo-1-phenyl-1,6-dihydropyridazine-3-carboxamide**

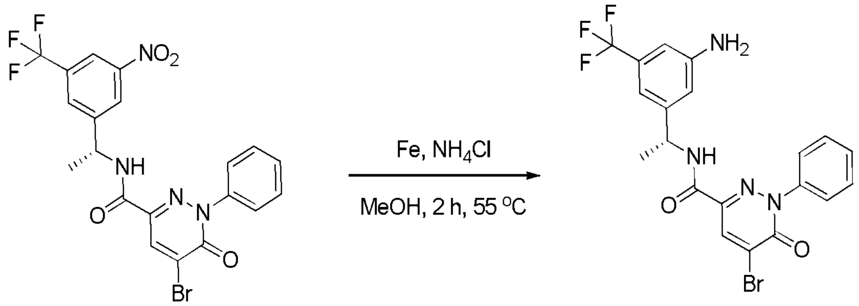


**241**

The compound of Example 241 (7.2 mg, 12% yield) was obtained as a white solid in the same manner as in Example 56, except that Step 2 of Example 56 was omitted. $^1H$ NMR (400M Hz, DMSO-$d_6$) δ = 8.92 (d, *J* = 8.4 Hz, 1H), 8.32 (s, 1H), 7.71-7.60 (m, 2H), 7.59-7.45 (m, 3H), 6.80 (d, *J*= 16.4 Hz, 2H), 6.70 (s, 1H), 5.54 (s, 2H), 5.07-5.00 (m, 1H), 1.44 (d, *J* = 6.8 Hz, 3H); MS (ESI) m/z = 481.0 $[M+H]^+$).

**Example 242: N-[(1R)-1-[3-(1,1-difluoro-2-methoxy-ethyl)-2-fluoro-phenyl]ethyl]-1-(2-fluorophenyl)-6-oxo-pyridazine-3-carboxamide**

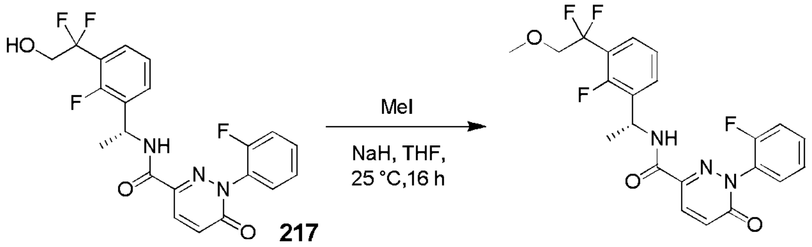


217 242

To a mixture of the compound of Example 217 (30 mg, 68.91 μmol) in THF (2 mL) was added NaH (8.27 mg, 206.72 μmol, 344.53 μL) at 50 °C. The resulting mixture was stirred at 50 °C for 15 minutes. The mixture was added with MeI (19.56 mg, 137.81 μmol, 8.58 μL) and stirred at 25 °C for 16 h. The reaction mixture was quenched with ice-cold water (10 mL) and extracted with EtOAc (30 mL × 3). The combined organic layer was washed with brine (50 mL), dried over anhydrous $Na_2SO_4$ and concentrated under reduced pressure to obtain a crude product. The product was purified by prep-HPLC (column: Phenomenex C18 75*30mm*3μm; mobile phase: [water($NH_3H_2O$+$NH_4HCO_3$)-ACN];B%: 34%-64%,30 min). Most of ACN was removed under reduced pressure, and the remaining solvent was removed by lyophilization to give the compound of Example 242 (3.5 mg, 11.30% yield) as a white solid. $^1$H NMR (400 MHz, DMSO-$d_6$) δ = 9.04 (d, *J* = 8.0 Hz, 1H), 7.92 (d, *J* = 10.0 Hz, 1H), 7.69 (dt, *J* = 1.6, 7.6 Hz, 1H), 7.65 - 7.61 (m, 1H), 7.61 - 7.56 (m, 1H), 7.48 - 7.40 (m, 3H), 7.31 - 7.26 (m, 1H), 7.18 (d, *J* = 9.6 Hz, 1H), 5.39 (quin, *J*=7.2 Hz, 1H), 3.96 (t, *J*= 14.0 Hz, 2H), 3.33 (s, 3H), 1.46 (d, *J*=7.2 Hz, 3H); MS (ESI) m/z = 450.1 $[M+H]^+$.

**Example 243: N-[(1R)-1-[3-(2-amino-1,1-difluoro-ethyl)-2-fluoro-phenyl]ethyl]-1-(2-fluorophenyl)-6-oxo-pyridazine-3-carboxamide**

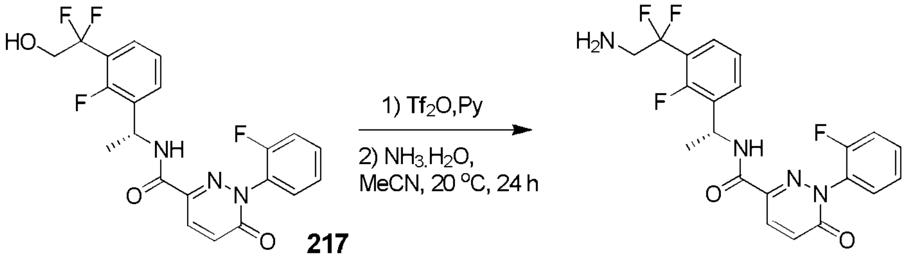


217 243

To a solution of the compound of Example 217 (30 mg, 68.91 μmol) in MeCN (1 mL) was added pyridine (8.72 mg, 110.25 μmol, 8.90 μL). The solution was cooled in an ice-water bath, and $Tf_2O$ (21.39 mg, 75.80 μmol, 12.51 μL) was added dropwise. The mixture was stirred at 25 °C for 5 minutes. Concentrated $NH_3.H_2O$ (0.25 M, 103.86 mL) was added thereto, and the solution was stirred at 20 °C for 24 h under $N_2$ atmosphere. The reaction mixture was poured into water (30 mL) and extracted with EtOAc (30 mL × 3). The combined organic layer was washed with brine (30 mL), dried over anhydrous $Na_2SO_4$ and concentrated under reduced pressure to obtain a crude product. The product was purified by prep-HPLC (column: Phenomenex C18 75*30 mm*3 μm; mobile phase: [water($NH_3H_2O$+$NH_4HCO_3$)-ACN];B%: 30%-70%,30 min). Most of ACN was removed under reduced pressure, and the remaining solvent was removed by lyophilization to give the compound of Example 243 (4.6 mg, 15.28% yield) as a white solid. $^1$H NMR (400 MHz, DMSO-$d_6$) δ = 9.03 (d, *J* = 8.0 Hz, 1H), 7.92 (d, *J* = 9.6 Hz, 1H), 7.69 (dt, *J* = 1.6, 7.6 Hz, 1H), 7.63 - 7.56 (m, 2H), 7.49 - 7.39 (m, 3H), 7.28 - 7.23 (m, 1H), 7.18 (d, *J* = 10.0 Hz, 1H), 5.40 (quin, *J* = 7.2 Hz, 1H), 3.20 (br t, *J* = 15.2 Hz, 2H), 1.68 (br s, 2H), 1.46 (d, *J* = 7.2 Hz, 3H); MS (ESI) m/z = 435.0 $[M+H]^+$.

**Example 244: N-[(1R)-1-[S5-amino-2-methyl-3-(trifluoromethyl)phenyl]ethyl]-1-(2-fluorophenyl)-6-oxo-pyridazine-3-carboxamide**

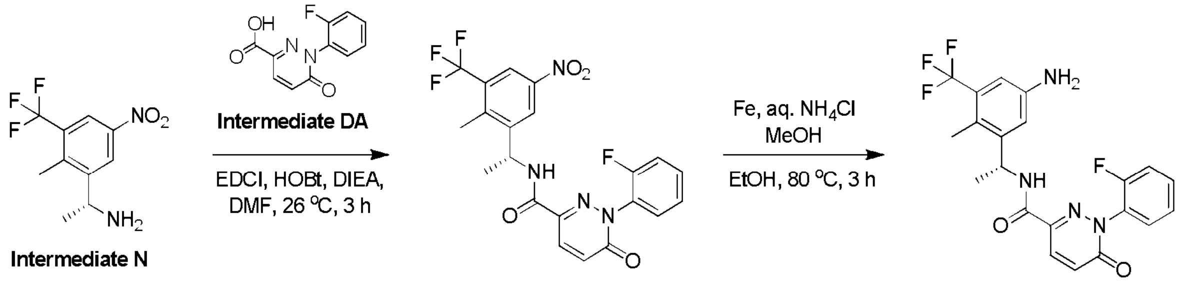


244

The compound of Example 244 (35.2 mg, 37.14% yield) was obtained as a white solid by reacting Intermediate N and Intermediate DA in the same manner as in Steps 3 and 4 of Example 89. $^1$H NMR (400MHz, DMSO-$d_6$) δ = 8.90 (br d, *J* = 7.2 Hz, 1H), 7.92 (d, *J* = 9.6 Hz, 1H), 7.69 (br t, *J* = 7.2 Hz, 1H), 7.63 - 7.53 (m, 1H), 7.49 - 7.38 (m, 2H), 7.18 (d, *J* = 9.6 Hz, 1H), 6.84 (s, 1H), 6.78 (s, 1H), 5.27 (br s, 2H), 5.24 - 5.17 (m, 1H), 2.23 (br s, 3H), 1.38 (br d, *J* = 6.6 Hz, 3H); MS (ESI) m/z = 435.3 $[M+H]^+$.

**Example 245 : N-[(1R)-1-[5-amino-2-fluoro-3-(trifluoromethyl)phenyl]ethyl]-1-(2-fluorophenyl)-6-oxo-pyridazine-3-carboxamide**

Step 1: Synthesis of 1-bromo-2-fluoro-5-nitro-3-(trifluoromethyl)benzene

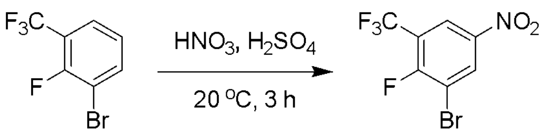


1-bromo-2-fluoro-5-nitro-3-(trifluoromethyl)benzene was obtained in the same manner as in Preparation Example 14 by using 1-bromo-2-fluoro-3-(trifluoromethyl)benzene as a starting material. $^1$H NMR (400MHz, DMSO-$d_6$) δ 8.95 (dd, *J*= 2.4, 5.2 Hz, 1H), 8.55 - 8.51 (m, 1H).

Step 2: Synthesis of 1-[2-fluoro-5-nitro-3-(trifluoromethyl)phenyl]ethanone

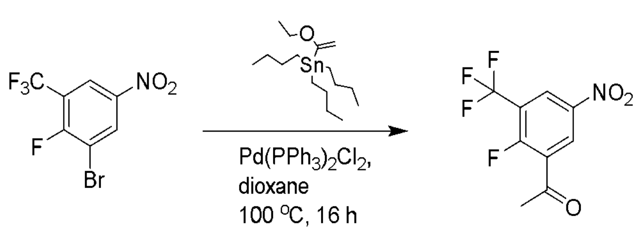


A mixture of 1-bromo-2-fluoro-5-nitro-3-(trifluoromethyl)benzene (5.9 g, 20.49 mmol), tributyl(1-ethoxyvinyl) stannane (8.41 g, 23.29 mmol, 7.86 mL) and $Pd(PPh_3)_2Cl_2$ (1.44 g, 2.05 mmol) in dioxane (50 mL) was stirred at 100°C for 16 h under $N_2$ atmosphere. The reaction mixture was cooled to 0°C, treated with 4 M HCl (50 mL) and stirred for 1 hour. The reaction mixture was diluted with water (100 mL) and then extracted with EtOAc (100 mL x 3). The extract was dried over $Na_2SO_4$ and filtered, and the filtrate was concentrated. The aqueous layer was adjusted to pH= 9 with aq. NaOH, and then sodium hypochlorite (100 mL) was added slowly while stirring. The product was purified by silica gel column chromatography (5% EtOAc in PE) to give 1-[2-fluoro-5-nitro-3-(trifluoromethyl)phenyl]ethanone (1.6 g, 31.10% yield) as colourless oil. $^1$H NMR (400MHz, DMSO-$d_6$) δ 8.81 (dd, *J* = 2.8, 5.6 Hz, 1H), 8.71 (dd, *J*= 2.8, 5.2 Hz, 1H), 2.70 (d, J=4.0 Hz, 3H).

Step 3: Synthesis of (NZ,R)-N-[1-[2-fluoro-5-nitro-3-(trifluoromethyl)phenyl]ethylidene]-2-methyl-propane-2-sulfinamide

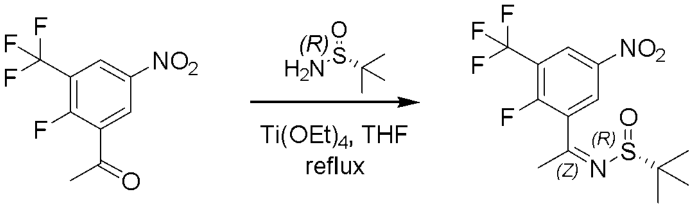


(NZ,R)-N-[1-[2-fluoro-5-nitro-3-(trifluoromethyl)phenyl]ethylidene]-2-methyl-propane-2-sulfinamide was obtained in the same manner as in Step 2 of Preparation Example 2 by using 1-[2-fluoro-5-nitro-3-(trifluoromethyl) phenyl]ethanone as a starting material. $^1$H NMR (400MHz, DMSO-$d_6$) δ 8.80 (br d, *J* = 2.4 Hz, 1H), 8.64 - 8.52 (m, 1H), 2.78 - 2.54 (m, 3H), 1.24(s, 6H), 1.21 - 1.11 (m, 3H); LC/MS (ESI) m/z = 354.9 [M+H]$^+$.

Step 4: Synthesis of (R)-N-[(1R)-1-[2-fluoro-5-nitro-3-(trifluoro)phenyl]ethyl]-2-methylpropane-2-sulfinamide

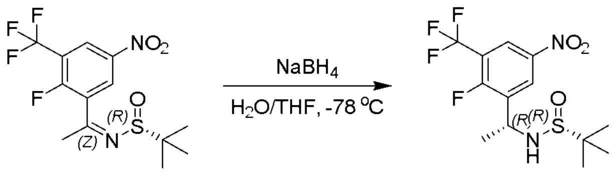


(R)-N-[(1R)-1-[2-fluoro-5-nitro-3-(trifluoro)phenyl]ethyl]-2-methyl-propane-2-sulfinamide was obtained in the same manner as in Step 3 of Preparation Example 2 by using (NZ,R)-N-[1-[2-fluoro-5-nitro-3-(trifluoromethyl)phenyl] ethylidene]-2-methyl-propane-2-sulfinamide as a starting material. $^1$H NMR (400MHz, DMSO-$d_6$) δ 8.83 (dd, *J* = 2.8, 5.6 Hz, 1H), 8.46 (dd, *J* = 2.8, 5.6 Hz, 1H), 6.14 (d, *J* = 8.8 Hz, 1H), 4.83 - 4.73 (m, 1H), 1.48 (d, *J* = 6.8 Hz, 3H), 1.13 (s, 9H); LC/MS (ESI) m/z = 356.9 [M+H]$^+$.

Step 5: Synthesis of (1R)-1-[2-fluoro-5-nitro-3-(trifluoromethyl)phenyl]ethanamine

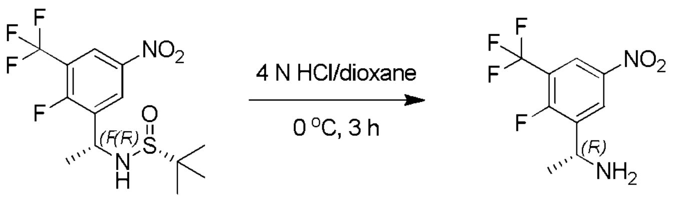


**Intermediate AX**

Intermediate AX was obtained in the same manner as in Step 4 of Preparation Example 2 by using (R)-N-[(1R)-1-[2-fluoro-5-nitro-3-(trifluoro)phenyl]ethyl]-2-methyl-propane-2-sulfinamide as a starting material. LC/MS (ESI) m/z = 252.9 [M+H]$^+$.

Step 6: Synthesis of N-[(1R)-1-[5-amino-2-fluoro-3-(trifluoromethyl)phenyl]ethyl]-1-(2-fluorophenyl)-6-oxo-pyridazine-3-carboxamide

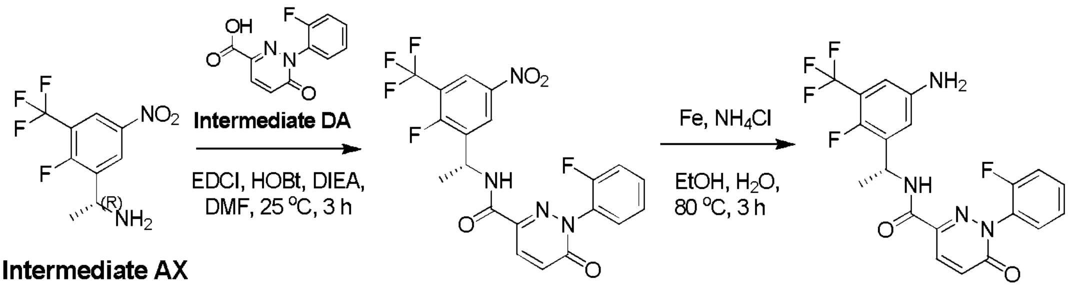


Intermediate AX

245

The compound of Example 245 was obtained by reacting Intermediate AX in the same manner as in Steps 3 and 4 of Example 89. $^1$H NMR (400 MHz, DMSO-$d_6$) δ = 8.97 (d, J = 7.6 Hz, 1H), 7.93 (d, J = 9.6 Hz, 1H), 7.69 (dt, J = 1.6, 7.6 Hz, 1H), 7.62 - 7.56 (m, 1H), 7.48 - 7.40 (m, 2H), 7.19 (d, J = 10.0 Hz, 1H), 6.81 (dd, J = 2.8, 5.6 Hz, 1H), 6.72 (dd, J = 2.8, 5.6 Hz, 1H), 5.38 (s, 2H), 5.23 (quin, J = 7.2 Hz, 1H), 1.42 (d, J = 7.2 Hz, 3H); ; LC/MS (ESI) m/z: 439.3 $[M+H]^+$

**Example 246: N-[(1R)-1-[3-(1,1-difluoro-2-hydroxy-ethyl)-2-methyl-phenyl]ethyl]-1-(2-fluorophenyl)-6-oxo-pyridazine-3-carboxamide**

Step 1: Synthesis of 1-(2-methyl-3-nitrophenyl)ethanone

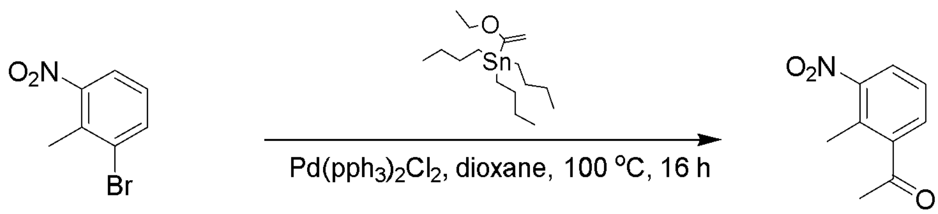


1-(2-methyl-3-nitrophenyl)ethenone was obtained in the same manner as in Preparation Example 12 by using 1-bromo-2-methyl-3-nitrobenzene as a starting material. $^1$H NMR (400 MHz, DMSO-$d_6$) δ ppm 8.05 - 7.89 (m, 2H), 7.56 (t, *J* = 8.0 Hz, 1H), 2.60 (s, 3H), 2.37 (s, 3H).

Step 2: Synthesis of 1-(3-amino-2-methyl-phenyl)ethanone

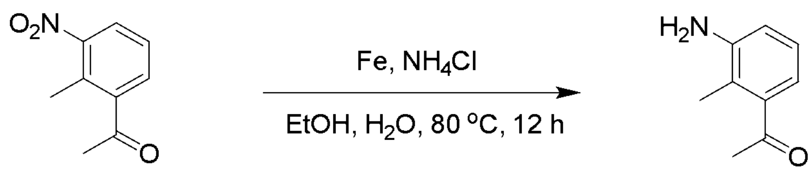


A mixture of 1-(2-methyl-3-nitrophenyl)ethanone (9.2 g, 51.35 mmol), Fe (28.67 g, 513.47 mmol), $NH_4Cl$ (27.47g, 513.47 mmol) in EtOH (100 mL) and $H_2O$ (20 mL) was degassed and purged with $N_2$ for 3 times, and the mixture was then stirred at 80 °C for 12 h under $N_2$ atmosphere. The reaction mixture was poured into water (100 mL) and extracted with EtOAc (100 mL × 3). The combined organic layer was washed with brine (100 mL), dried over anhydrous $Na_2SO_4$ and concentrated under reduced pressure to give 1-(3-amino-2-methyl-phenyl)ethanone (6.8 g, 88.77% yield) as a crude product of a yellow solid, which was used into the next step without further purification. $^1$H NMR (400 MHz, DMSO-$d_6$) δ ppm 6.98 (t, J = 7.6 Hz, 1H), 6.84 (dd, J = 1.2, 7.6 Hz, 1H), 6.77 (dd, J =1.2, 8.0 Hz, 1H), 5.05 (s, 2H), 2.46 (s, 3H), 2.06 (s, 3H).

Step 3: Synthesis of 1-(3-iodo-2-methyl-phenyl)ethanone

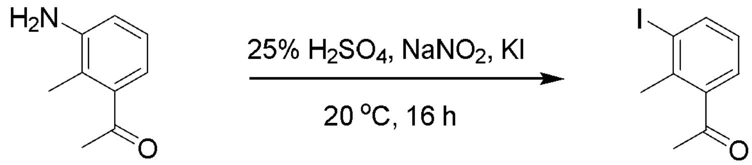


A mixture of 1-(3-amino-2-methyl-phenyl)ethanone (6.5 g, 43.57 mmol) in $H_2SO_4$ (21.6 mL, 975.55 mmol) was cooled to 0 °C, added with a mixed solution of $NaNO_2$ (3.01 g, 43.57 mmol) and $H_2O$ (65 mL), and stirred for 1 hour at 0°C. The reaction mixture was added to a mixed solution of KI (21.70 g, 130.71 mmol) in $H_2O$ (220 mL), followed by stirring at 20 °C for 18 h. The reaction mixture was poured into water (100 mL) and extracted with EtOAc (100 mL × 3). The combined organic layer was washed with brine (100 mL), dried over anhydrous $Na_2SO_4$ and concentrated under reduced pressure to obtain a crude product. The product was purified by silica gel column chromatography (6% EtOAc in PE) to give 1-(3-iodo-2-methyl-phenyl)ethanone (8.2 g, 72.37% yield) as a yellow solid. $^1$N MR (EB4267-390-P1N, 400 MHz, DMSO-$d_6$) δ = 7.99 (dd, J = 1.2, 8.0 Hz, 1H), 7.67 (d, J = 7.6 Hz, 1H), 7.07 (t, J = 7.6 Hz, 1H), 2.53 (s, 3H), 2.41 (s, 3H).

Step 4: Synthesis of ethyl 2-(3-acetyl-2-methyl-phenyl)-2,2-difluoroacetate

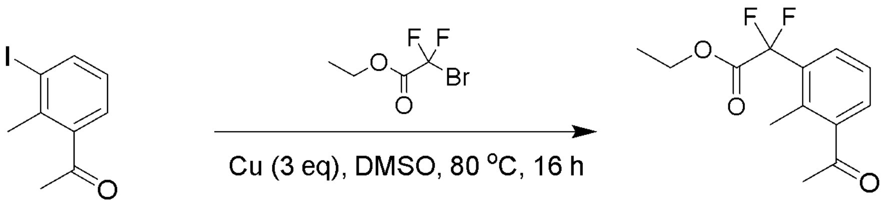


Ethyl 2-(3-acetyl-2-methyl-phenyl)-2,2-difluoro acetate was obtained in the same manner as in Step 1 of Preparation Example 4 by using 1-(3-iodo-2-methyl-phenyl)ethanone as a starting material. $^1$H NMR (400 MHz, DMSO-$d_6$) δ ppm 7.86 (d, *J* = 7.6 Hz, 1H), 7.72 (d, *J* = 7.6 Hz, 1H), 7.49 (t, *J* = 7.6 Hz, 1H), 4.34 (q, *J* = 7.2 Hz, 2H), 2.57 (s, 3H), 2.29 (s, 3H), 1.22 (t, *J* = 7.2 Hz, 3H); MS (ESI) m/z = 257.0 [M+H]$^+$.

Step 5: Synthesis of ethyl 2-[3-[(Z)-N-[(R)-tert-butylsulfinyl]-C-methyl-carboimidoyl]-2-methyl-phenyl]-2,2-difluoroacetate

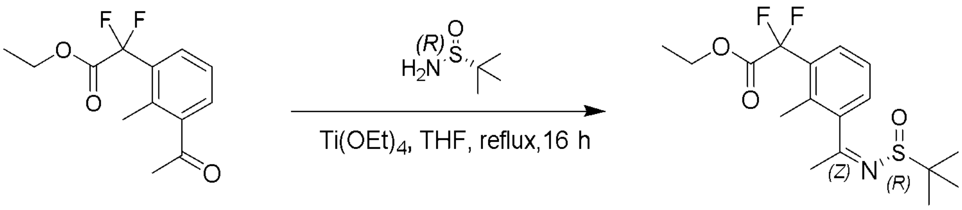


Ethyl 2-(3-acetyl-2-methyl-phenyl)-2,2-difluoroacetate was obtained in the same manner as in Step 2 of Preparation Example 4 by using ethyl 2-(3-acetyl-2-methyl-phenyl)-2,2-difluoro acetate as a starting material. MS (ESI) m/z = 360.0 [M+H]$^+$.

Step 6: Synthesis of (R)-N-[(1R)-1-[3-(1,1-difluoro-2-hydroxy-ethyl)-2-methylphenyl]ethyl]-2-methyl-propane-2-sulfinamide

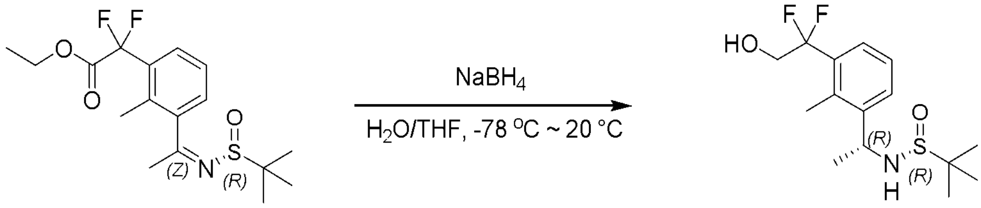


(R)-N-[(1R)-1-[3-(1,1-difluoro-2-hydroxy-ethyl)-2-methyl-phenyl]ethyl]-2-methylpropane-2-sulfinamide was obtained in the same manner as in Step 3 of Preparation Example 4 by using ethyl 2-(3-acetyl-2-methyl-phenyl)-2,2-difluoroacetate as a starting material. $^1H$ NMR (400 MHz, DMSO-$d_6$) δ ppm 7.61 (d, *J* = 7.6 Hz, 1H), 7.39 - 7.34 (m, 1H), 7.28 (t, *J* = 7.2 Hz, 1H), 5.73 - 5.60 (m, 2H), 4.69 (quin, *J* = 6.8 Hz, 1H), 3.88 (dt, *J*= 6.4, 14.8 Hz, 2H), 2.37 (s, 3H), 1.37 (d, *J* = 6.8 Hz, 3H), 1.11 - 1.06 (m,9H); MS (ESI) m/z = 320.0 $[M+H]^+$.

Step 7: Synthesis of 2-[3-[(1R)-1-aminoethyl]-2-methyl-phenyl]-2,2-difluoro-ethanol

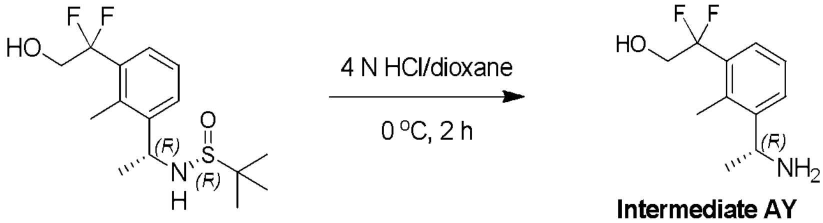


Intermediate AY

Intermediate AY was obtained in the same manner as in Step 4 of Preparation Example 4 by using (R)-N-[(1R)-1-[3-(1,1-difluoro-2-hydroxy-ethyl)-2-methyl-phenyl]ethyl]-2-methylpropane-2-sulfinamide as a starting material. MS (ESI) m/z = 216.0 $[M+H]^+$.

Step 8: Synthesis of N-[(1R)-1-[3-(1,1-difluoro-2-hydroxy-ethyl)-2-methylphenyl]ethyl]-1-(2-fluorophenyl)-6-oxo-pyridazine-3-carboxamide

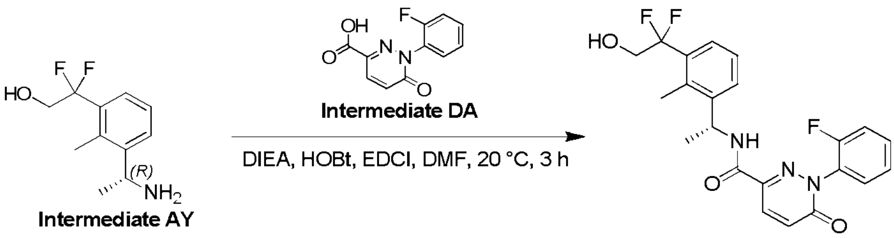


246

A mixture of Intermediate AY (30.33 mg, 140.92 μmol,), Intermediate DA (30 mg, 128.10 μmol), EDCI (49.12 mg, 256.21 μmol), HOBt (34.62 mg, 256.21 μmol) and DIEA (49.67 mg, 384.31 μmol, 66.94 μL) in DMF (2 mL) was degassed and purged with $N_2$ for 3 times, and then the mixture was stirred at 20 °C for 3 h under $N_2$ atmosphere. The reaction mixture was poured into water (30 mL) and extracted with EtOAc (30 mL × 3). The organic layer was washed with brine (50 mL), dried over anhydrous $Na_2SO_4$ and concentrated under reduced pressure to obtain a crude product. The main product was separated by using prep-HPLC (column: Phenomenex C18 75*30mm*3um;mobile phase: [water($NH_3H_2O$+$NH_4HCO_3$)-ACN];B%: 26%-56%,11 min). Then, ACN was removed under reduced pressure, and the remaining solvent was removed by lyophilization to give the compound of Example 246. $^1H$ NMR (400MHz, DMSO-$d_6$) δ ppm 8.99 (d, *J* = 8.0 Hz, 1H), 7.91 (d, *J* = 9.6 Hz, 1H), 7.68 (dt, *J* = 1.6, 7.6 Hz, 1H), 7.62 - 7.55 (m, 2H), 7.48 - 7.39 (m, 2H), 7.35 (d, *J* = 7.2 Hz, 1H), 7.25 (t, *J* = 7.6 Hz, 1H), 7.17 (d, *J* = 10.0 Hz, 1H), 6.14 - 5.46 (m, 1H), 5.37 (quin, *J*= 7.2 Hz, 1H), 3.93 - 3.84 (m, 2H), 2.43 (s, 3H), 1.41 (d, *J* = 7.2 Hz, 3H); MS (ESI) m/z = 432.3 $[M+H]^+$.

**Example 247: N-[(1R)-1-[3-amino-5-(trifluoromethyl)phenyl]ethyl]-1-[2-(difluoromethoxy)phenyl]-6-oxo-pyridazine-3-carboxamide**

Steps 1 and 2: Synthesis of (R)-1-(2-hydroxyphenyl)-N-(1-(3-nitro-5-(trifluoromethyl)phenyl)ethyl)-6-oxo-1,6-dihydropyridazine-3-carboxamide

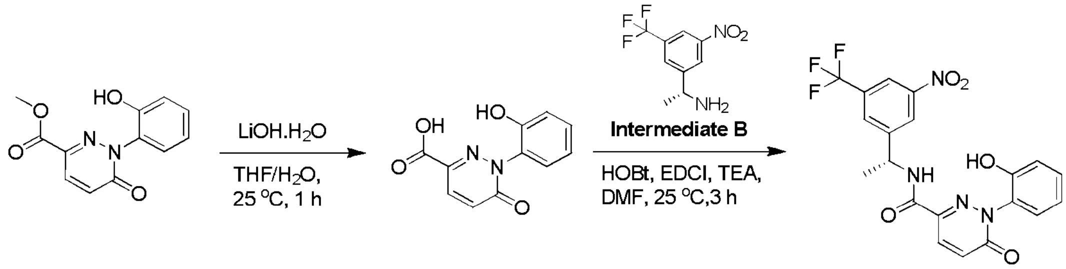


(R)-1-(2-hydroxyphenyl)-N-(l-(3-nitro-5-(trifluoromethyl)phenyl)ethyl)-6-oxo-1,6-dihydropyridazine-3-carboxamide was obtained in the same manner as in Steps 1 and 2 of Example 51 by using as a starting material methyl-1-(2-hydroxyphenyl)-6-oxo-1,6-dihydropyridazine-3-carboxylate prepared by referring to Preparation Example 11.

Step 3: Synthesis of 1-[2-(difluoromethoxy)phenyl]-N-[(1R)-1-[3-nitro-5-(trifluoromethyl)phenyl]ethyl]-6-oxo-pyridazine-3-carboxamide

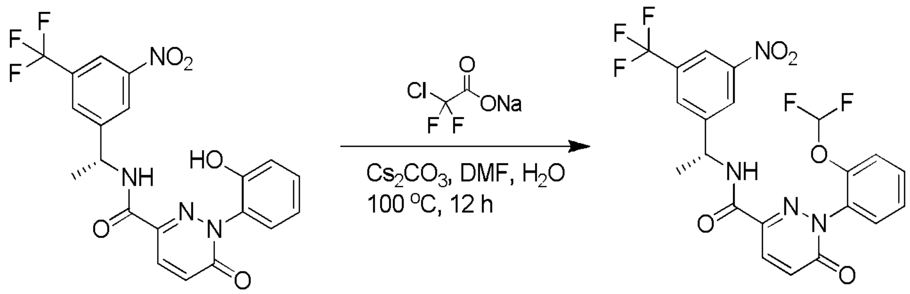


A mixture of 1-(2-hydroxyphenyl)-N-[(1R)-1-[3-nitro-5-(trifluoromethyl)phenyl]ethyl]-6-oxo-pyridazine-3-carboxamide (60 mg, 133.82 μmol), (2-chloro-2,2-difluoro-acetyl)oxysodium (204.03 mg, 1.34 mmol) and $Cs_2CO_3$ (436.02 mg, 1.34 mmol) in DMF (2 mL) and $H_2O$ (0.2 mL) was degassed and purged with $N_2$ for 3 times, and then the mixture was stirred at 100 °C for 16 h under $N_2$ atmosphere. The reaction mixture was poured into water (30 mL) and extracted with EtOAc (30 mL × 3). The combined organic layer was washed with brine (30 mL), dried over anhydrous $Na_2SO_4$ and concentrated under reduced pressure to obtain a crude product. The residue was purified by flash silica gel chromatography (30% EtOAc in PE) to give 1-[2-(difluoromethoxy)phenyl]-N-[(1R)-1-[3-nitro-5-(trifluoromethyl)phenyl]ethyl]-6-oxo-pyridazine-3-carboxamide (38 mg, 17.21% yield) as yellow oil. MS (ESI) m/z = 499.1 $[M+H]^+$.

Step 4: Synthesis of N-[(1R)-1-[3-amino-5-(trifluoromethyl)phenyl]ethyl]-1-[2-(difluoromethoxy)phenyl]-6-oxo-pyridazine-3-carboxamide

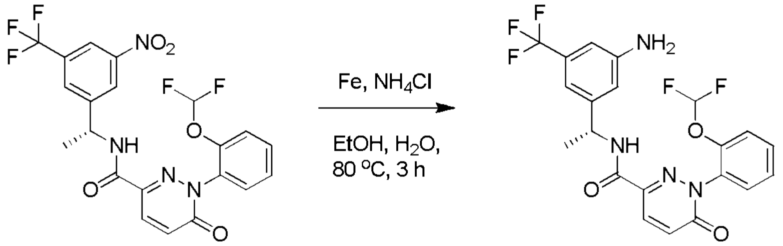


**247**

The compound of Example 247 (3.3 mg, 8.58% yield) was obtained as a white solid in the same manner as in Step

3 of Example 51. $^1H$ NMR (400MHz, DMSO-$d_6$) δ = 8.83 (d, *J* = 8.4 Hz, 1H), 7.94 (d, *J* = 9.6 Hz, 1H), 7.66 (dd, *J* = 1.6, 8.0 Hz, 1H), 7.61 (t, *J* = 7.6 Hz, 1H), 7.48 - 7.43 (m, 1H), 7.41 (d, *J* = 8.0 Hz, 1H), 7.33 (s, 1H), 7.18 (s, 1H), 7.18 - 7.12 (m, 1H), 6.96 (s, 1H), 6.79 (s, 1H), 6.76 (s, 1H), 6.69 (s, 1H), 5.53 (s, 2H), 5.07 - 4.99 (m, 1H), 1.42 (d, *J* = 7.2 Hz, 3H); MS (ESI) m/z = 469.3 $[M+H]^+$.

**Example 248: Tert-butyl 3-[3-[[(1R)-1-[3-amino-5-(trifluoromethyl)phenyl]ethyl]carbamoyl]-6-oxo-pyridazin-1-yl]benzoate**

Step 1: Synthesis of methyl-1-(3-tert-butoxycarbonylphenyl)-6-oxo-pyridazine-3-carboxylate

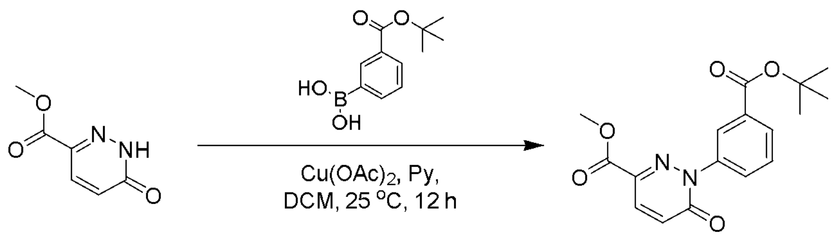


To a mixture of methyl-6-oxo-1H-pyridazine-3-carboxylate (300 mg, 1.95 mmol) and (3-tert-butoxycarbonylphenyl)boronic acid (432.99 mg, 1.95 mmol) in DCM (4 mL), $Cu(OAc)_2$ (177.09 mg, 975.00 μmol), Py (1.00 g, 12.68 mmoL, 1.02 mL) and 4A MS (300 mg) were added, and the mixture was stirred at 25 °C for 16 h. The reaction mixture was poured into distilled water (20 mL) and extracted with EtOAc (20 mL × 3). The combined organic layer was washed with brine (50 mL x 4), dried over anhydrous $Na_2SO_4$ and concentrated under reduced pressure to give methyl-1-(3-tert-butoxycarbonylphenyl)-6-oxo-pyridazine-3-carboxylate (670 mg, 99.21% yield) as yellow oil. MS (ESI) m/z = 275.0 $[M+H]^+$.

Step 2: Synthesis of 1-(3-tert-butoxycarbonylphenyl)-6-oxo-pyridazine-3-carboxylic acid

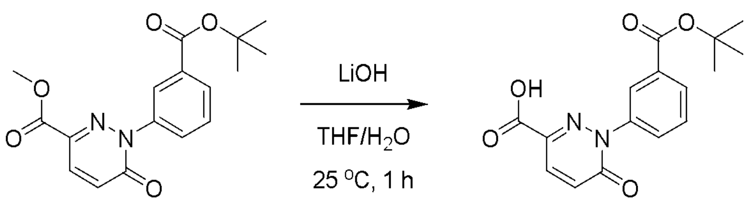


To a solution of methyl-1-(3-tert-butoxycarbonylphenyl)-6-oxo-pyridazine-3-carboxylate (670 mg, 2.03 mmol) in THF (9 mL), $LiOH.H_2O$ (255.34 mg, 6.08 mmol) and $H_2O$ (4.5 mL) were added, and the reaction mixture was stirred at 25 °C for 1.5 h under air. The mixture was poured into water (30 mL) and extracted with EtOAc (30 mL × 3). The combined organic layer was washed with brine (50 mL), dried over anhydrous $Na_2SO_4$ and concentrated under reduced pressure to give 1-(3-tert-butoxycarbonylphenyl)-6-oxo-pyridazine-3-carboxylic acid (493 mg, 69.41% yield) as a yellow solid. $^1H$ NMR (400 MHz, DMSO-$d_6$) δ = 8.08 (t, *J* = 1.6 Hz, 1H), 8.00 (d, *J* = 7.6 Hz, 1H), 7.92 (d, *J* = 10 Hz, 1H), 7.84 (td, *J* = 1.0, 7.2 Hz, 1H), 7.71 - 7.63 (m, 1H), 7.15 (d, *J* = 10 Hz, 1H), 1.56 (s, 9H); MS (EI) m/z: 261.1 $[M+H]^+$.

Step 3: Synthesis of tert-butyl 3-[3-[[(1R)-1-[3-amino-5-(trifluoromethyl)phenyl]ethyl]carbamoyl]-6-oxo-pyridazin-1-yl] benzoate

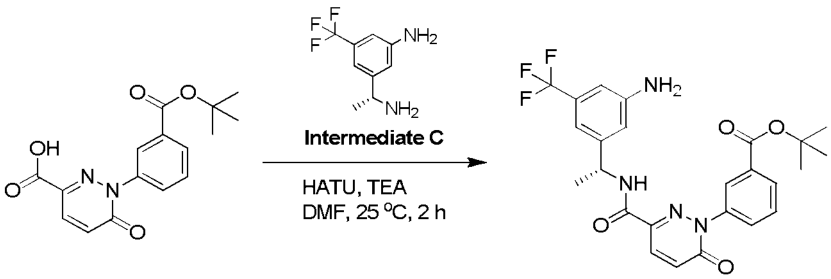


248

To a solution of 1-(3-tert-butoxycarbonylphenyl)-6-oxo-pyridazine-3-carboxylic acid (100 mg, 316.15 μmol) and Intermediate C (64.55 mg, 316.15 μmol) in DMF (5 mL), EDCI (90.91 mg, 474.22 μmol), HOBt (64.08 mg, 474.22 μmol) and TEA (159.95 mg, 1.58 mmol, 220.02 μL) were added, and the reaction mixture was stirred at 25 °C for 12 h under $N_2$ atmosphere. The reaction mixture was poured into distilled water (10 mL) and extracted with EtOAc (20 mL × 3). The combined organic layer was washed with brine (30 mL), dried over anhydrous $Na_2SO_4$ and concentrated under reduced pressure to obtain a crude product. The product was purified by silica gel column chromatography (58% EtOAc in PE) to give the compound of Example 248 (50 mg, 15.36% yield) as yellow oil. $^1$H NMR (400 MHz, DMSO-$d_6$) δ = 8.88 (d, J = 8.4 Hz, 1H), 8.15 (t, J = 1.6 Hz, 1H), 7.99 (td, J = 1.2, 8.0 Hz, 1H), 7.95-7.89 (m, 2H), 7.68 (t, J = 8.0 Hz, 1H), 7.64-7.64 (m, 1H), 7.16 (d, J = 9.6 Hz, 1H), 6.80 (d, J = 12.8 Hz, 2H), 6.70 (s, 1H), 5.54 (s, 2H), 5.04 (quin, J = 7.2 Hz, 1H), 1.56 (s, 9H), 1.44 (d, J = 7.2 Hz, 3H) ; LC/MS (ESI) m/z = 447.3 $[M+H]^+$

**Example 249: 3-(3--{[(1R)-1-[3-amino-5-(trifluoromethyl)phenyl]ethyl]carbamoyl}-6-oxo-1,6-dihydropyridazin-1-yl)benzoic acid**

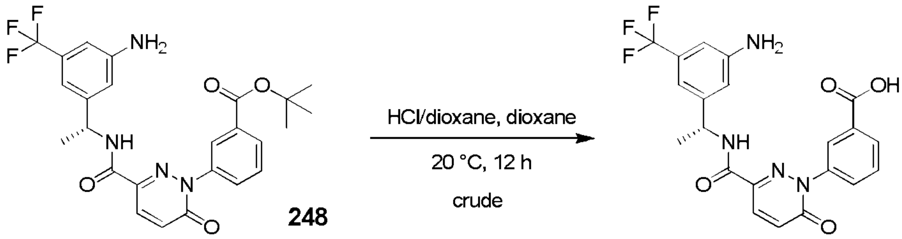

248

249

To a solution of the compound of Example 248 (30 mg, 59.70 μmol) in dioxane (1 mL) was added HCl/dioxane (4 M, 2 mL). The mixture was stirred at 20 °C for 12 h and concentrated under reduced pressure to give the compound of Example 249 (25 mg, crude, HCl) as a yellow solid, which was used into the next step without further purification.

**Example 250 and Example 251**

Carboxylic acid compounds were prepared in the same manner as in Example 249, and the compounds shown in the following table were prepared through an amide coupling reaction.

[Table 15]

| No | Structure / Name | Spectral Data |
|---|---|---|
| 250 | 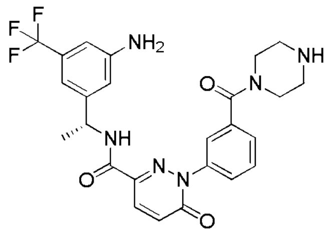 N-[(1R)-1-[3-amino-5-(trifluoromethyl)phenyl]ethyl]-6-oxo-1-[3-(piperazine-1-carbonyl)phenyl]pyridazine-3-carboxamide | $^1H$ NMR (400 MHz, DMSO-$d_6$) $\delta$ = 8.88 (d, $J$ = 8.4 Hz, 1H), 7.89 (d, $J$ = 9.6 Hz, 1H), 7.80-7.74 (m, 1H), 7.72 (t, $J$ = 1.6 Hz, 1H), 7.61 (t, $J$ = 8.0 Hz, 1H), 7.47 (td, $J$ = 1.2, 7.6 Hz, 1H), 7.15 (d, $J$= 9.6 Hz, 1H), 6.80 (d, $J$ = 11.6 Hz, 2H), 6.70 (s, 1H), 5.54 (s, 2H), 5.03 (quin, $J$ = 7.2 Hz, 1H), 3.65-3.38 (m, 4H), 2.84-2.58 (m, 5H), 1.44 (d, $J$ = 7.2 Hz, 3H) ; LC/MS (ESI) m/z: 515.4 $[M+H]^+$ |
| 251 | 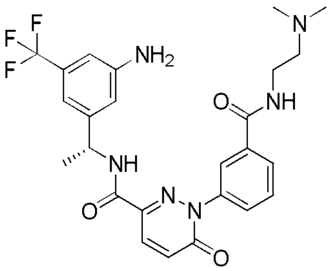 N-[(1R)-1-[3-amino-5-(trifluoromethyl)phenyl]ethyl]-1-[3-[2-(dimethylamino)ethylcarbamoyl]phenyl]-6-oxo-pyridazine-3-carboxamide | $^1H$ NMR (400 MHz, DMSO-$d_6$) $\delta$ = 8.91 (d, $J$ = 8.4 Hz, 1H), 8.51 (t, $J$ = 5.6 Hz, 1H), 8.09 (t, $J$ = 1.6 Hz, 1H), 7.96-7.88 (m, 2H), 7.84-7.78 (m, 1H), 7.67-7.60 (m, 1H), 7.17 (d, $J$ = 9.6 Hz, 1H), 6.79 (d, $J$ = 12.4 Hz, 2H), 6.70 (s, 1H), 5.55 (s, 2H), 5.04 (quin, $J$ = 7.2 Hz, 1H), 2.82 (s, 2H), 2.39 (t, $J$ = 6.8 Hz, 2H), 2.16 (s, 6H), 1.44 (d, $J$ = 7.2 Hz, 3H) ; LC/MS (ESI) m/z: 517.4 $[M+H]^+$ |

**Example 252: N-[(1R)-1-[3-amino-5-(trifluoromethyl)phenyl]ethyl]-1-(3-carbamoylphenyl)-6-oxo-pyridazine-3-carboxamide**

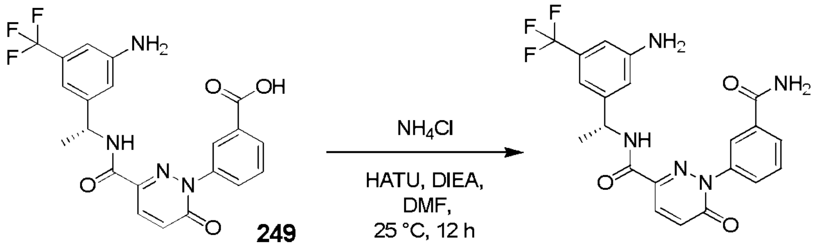


To a mixture of the compound of Example 249 (30 mg, 67.21 μmol) in DMF (2 mL), DIEA (43.43 mg, 336.04 μmol, 58.53 μL) and HATU (38.33 mg, 100.81 μmol) were added, and the reaction mixture was stirred at 25 °C for 2 h under $N_2$. Then, the reaction mixture was stirred at 60 °C for 1 hour under $N_2$. $NH_4Cl$ (10.79 mg, 201.62 μmol) was added, and the reaction mixture was stirred at 25°C for 10 h under $N_2$. The mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue, which was purified by prep-HPLC (column: Phenomenex C18 75*30 mm*3 μm; mobile phase: [water($NH_3H_2O$+$NH_4HCO_3$)-ACN]; B%: 22%-52%,11 min). The remaining solvent was removed by lyophilization to give the compound of Example 252 (5.4 mg, 17.92% yield). $^1H$ NMR (400MHz, DMSO-$d_6$) $\delta$ = 8.88 (d, $J$ = 8.4 Hz, 1H), 8.13 (d, $J$ = 2.0 Hz, 1H), 8.07 (br s, 1H), 7.96 (d, $J$ = 8.0 Hz, 1H), 7.91 (d, $J$ = 10.0Hz, 1H), 7.82 (dd, $J$ = 1.2, 8.0 Hz, 1H), 7.63 (t, $J$ = 8.0 Hz, 1H), 7.51 (s, 1H), 7.17 (d, $J$ = 10.0 Hz, 1H), 6.80 (d, $J$ = 11.2 Hz, 2H), 6.70 (s, 1H), 5.53 (s, 2H), 5.10 - 4.95 (m, 1H), 1.44 (d, $J$ = 7.2 Hz, 3H); MS (ESI) m/z = 446.2 $[M+H]^+$.

**Example 253 to Example 255**

The compounds shown in the following table were prepared by amidation of a carboxylic acid group in a similar manner to Example 252.

[Table 16]

| No. | Structure/Name | Spectral Data |
|---|---|---|
| 253 | 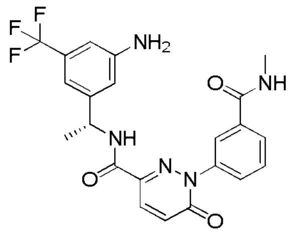 N-[(1R)-1-[3-amino-5-(trifluoromethyl)phenyl] ethyl]-1-[3-(methylcarbamoyl)phenyl]-6-oxo-pyridazine-3-carboxamide | $^1$H NMR (400 MHz, DMSO-d$_6$) $\delta$ = 8.88 (d, *J* = 8.4 Hz, 1H), 8.55 ( d, *J* = 4.6 Hz, 1H), 8.10 - 8.07 (m, 1H), 7.92 (s, 1H), 7.91 - 7.89 (m, 1H), 7.81 (dd, *J* = 0.8, 7.9 Hz, 1H), 7.67 - 7.60 (m, 1H), 7.17 (d, *J* = 10.0 Hz, 1H), 6.81 (s, 1H), 6.78 (s, 1H), 6.70 (s, 1H), 5.54 (s, 2H), 5.04 (t, *J* = 7.6 Hz, 1H), 2.80 (d, *J* = 4.4 Hz, 3H), 1.47 - 1.42 (m, 3H); LC/MS (ESI) m/z: 460.3 [M+H]$^+$ |
| 254 | 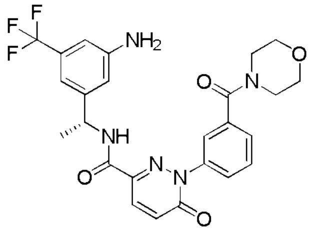 N-[(1R)-1-[3-amino-5-(trifluoromethyl)phenyl] ethyl]-1-[3-(morpholine-4-carbonyl)phenyl]-6-oxo-pyridazine-3-carboxamide | $^1$H NMR (400 MHz, DMSO-d$_6$) $\delta$ = 8.88 (d, *J* = 8.4 Hz, 1H), 7.89 (d, *J* = 10.0 Hz, 1H), 7.81 - 7.77 (m, 1H), 7.77 - 7.75 (m, 1H), 7.62 (t, *J* = 8.0 Hz, 1H), 7.51 (td, *J* = 1.3, 7.6 Hz, 1H), 7.16 (d, *J* = 9.6 Hz, 1H), 6.81 (s, 1H), 6.78 (s, 1H), 6.70 (s, 1H), 5.54 (s, 2H), 5.04 (q, *J* = 7.2 Hz, 1H), 3.72 - 3.41 (m, 8H), 1.45 (d, *J* = 7.2 Hz, 3H); LC/MS (ESI) m/z: 516.4 [M+H]$^+$ |
| 255 | 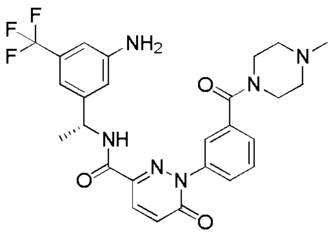 N-[(1R)-1-[3-amino-5-(trifluoromethyl)phenyl]ethyl]-1-[3-(4-methylpiperazine-1-carbonyl)phenyl]-6-oxo-pyridazine-3-carboxamide | $^1$H NMR (400 MHz, DMSO-d$_6$) $\delta$ = 8.88 (d, *J* = 8.4 Hz, 1H), 7.89 (d, *J* = 10.0 Hz, 1H), 7.78 (ddd, *J* = 1.2, 2.0, 8.0 Hz, 1H), 7.72 (t, *J* = 2.0 Hz, 1H), 7.61 (t, *J* = 8.0 Hz, 1H), 7.48 (td, *J* = 1.4, 7.6 Hz, 1H), 7.16 (d, *J* = 10.0 Hz, 1H), 6.81 (s, 1H), 6.78 (s, 1H), 6.70 (s, 1H), 5.54 (s, 2H), 5.10 - 4.96 (m, 1H), 4.79 - 4.76 (m, 1H), 3.62 (br s, 2H), 3.39 (d, *J* = 6.8 Hz, 2H), 2.40 - 2.25 (m, 4H), 2.18 (s, 3H), 1.47 - 1.43 (m, 3H) ; LC/MS (ESI) m/z: 529.4 [M+H]$^+$ |

**Example 256: N-[(1R)-1-[3-[1,1-difluoro-2-(methylamino)ethyl]-2-fluorophenyl]ethyl]-1-(2-fluorophenyl)-6-oxo-pyridazine-3-carboxamide**

Step 1: Synthesis of [2,2-difluoro-2-[2-fluoro-3-[(1R)-1-[[1-(2-fluorophenyl)-6-oxo-pyridazine-3-carbonyl]amino]ethyl] phenyl]ethyl]trifluoromethanesulfonate

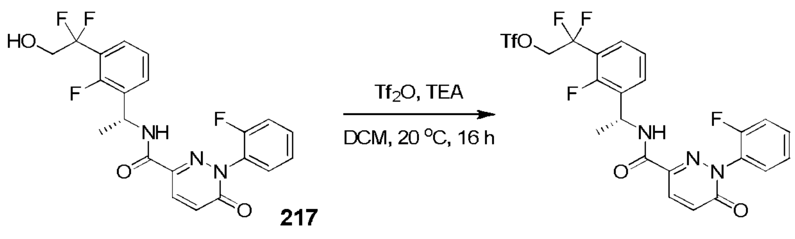


To a solution of the compound of Example 217 (100 mg, 229.69 $\mu$mol) in DCM (2 mL), $Tf_2O$ (129.61 mg, 459.38 $\mu$mol) and TEA (69.73 mg, 689.07 $\mu$mol) were added, and then the mixture was stirred at 20 °C for 16 h. The reaction mixture was poured into water (15 mL) and extracted with DCM (15 mL $\times$ 3). The combined organic layer was washed with brine (15 mL), dried over anhydrous $Na_2SO_4$ and concentrated under reduced pressure to give [2,2-difluoro-2-[2-fluoro-3-[(1R)-1-[[1-(2-fluorophenyl)-6-oxo-pyridazine-3-carbonyl]amino]ethyl]phenyl]ethyl]trifluoromethanesulfonate (120 mg, 92.0% yield) as yellow oil. MS (ESI) m/z = 568.1 [M+H]+.

Step 2: Synthesis of N-[(1R)-1-[3-[1,1-difluoro-2-(methylamino)ethyl]-2-fluorophenyl]ethyl]-1-(2-fluorophenyl)-6-oxo-pyridazine-3-carboxamide

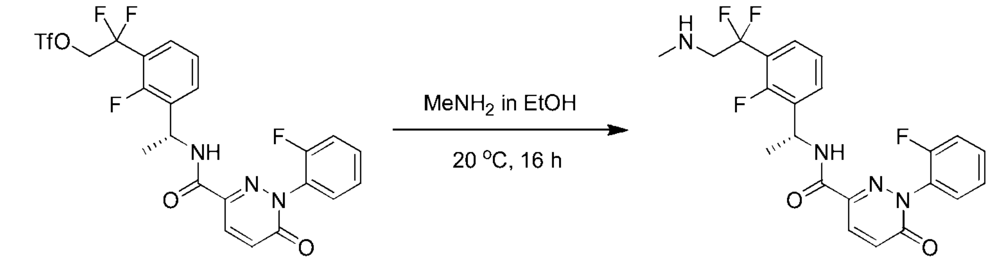


256

A mixture of [2,2-difluoro-2-[2-fluoro-3-[(1R)-1-[[1-(2-fluorophenyl)-6-oxo-pyridazine-3-carbonyl]amino]ethyl] phenyl]ethyl]trifluoromethanesulfonate (60 mg, 105.74 μmol) and methanamine (1 g, 10.63 mmol, 33% purity) was stirred at 20 °C for 16 h. The reaction mixture was concentrated under vacuum to obtain a crude product. The crude product was purified by prep-TLC (8% MeOH in DCM) to give an impure product. The product was purified by prep-HPLC (column: C18-6 100*30mm*5μm; mobile phase: [water(FA)-ACN]; B%: 10%-40%), and most of MeCN was removed under reduced pressure. The remaining solvent was removed by lyophilization to give the compound of Example 256 (2.0 mg, 3.7% yield) as a white solid. $^1$H NMR (400MHz, DMSO-d$_6$) δ = 9.01 (d, *J*= 8.0 Hz, 1H), 7.97-7.88 (m, 1H), 7.69 (dt, *J*= 1.6, 7.6 Hz, 1H), 7.64-7.55 (m, 2H), 7.49-7.38 (m, 3H), 7.28-7.22 (m, 1H), 7.18 (d, *J*= 10.0 Hz, 1H), 5.39 (quin, *J* = 7.2 Hz, 1H), 3.20 (s, 2H), 3.16 (br s, 1H), 2.26 (s, 3H), 1.45 (d, *J* = 7.2 Hz, 3H); MS (ESI) m/z = 449.1 [M+H]+.

**Example 257: N-[(1R)-1-[3-[2-(dimethylamino)-1,1-difluoro-ethyl]-2-fluorophenyl]ethyl]-1-(2-fluorophenyl)-6-oxo-pyridazine-3-carboxamide**

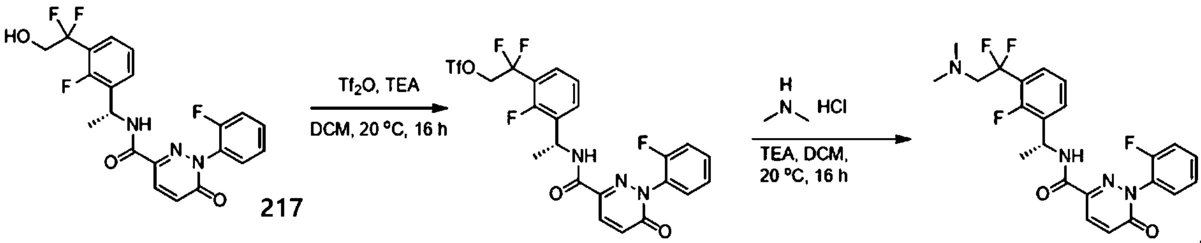


257

The compound of Example 257 (2.3 mg, 3.58% yield) was obtained as a white solid in the same manner as in Example 256 by replacing methanamine with dimethanamine in Step 2. $^1$H NMR (400MHz, DMSO-d$_6$) δ = 9.01 (d, *J* = 8.0 Hz, 1H), 7.92 (d, *J*= 10.0 Hz, 1H), 7.68 (dt, *J* = 1.6, 7.6 Hz, 1H), 7.63-7.55 (m, 2H), 7.50-7.38 (m, 3H), 7.30-7.23 (m, 1H), 7.18 (d, *J* = 10.0 Hz, 1H), 5.39 (quin, *J* = 7.2 Hz, 1H), 3.07 (t, *J* = 15.2 Hz, 2H), 2.18 (s, 6H), 1.46 (d, *J* = 7.2 Hz, 3H); MS (ESI) m/z = 463.3 [M+H]+.

**Example 258: N-[(1R)-1-[3-[2-(dimethylamino)-1,1-difluoro-ethyl]phenyl]ethyl]-1-(2-fluorophenyl)-6-oxo-pyridazine-3-carboxamide**

Step 1: Synthesis of (R)-N-(1-(3-(1,1-difluoro-2-hydroxyethyl)phenyl)ethyl)-1-(2-fluorophenyl)-6-oxo-1,6-dihydropyridazine-3-carboxamide

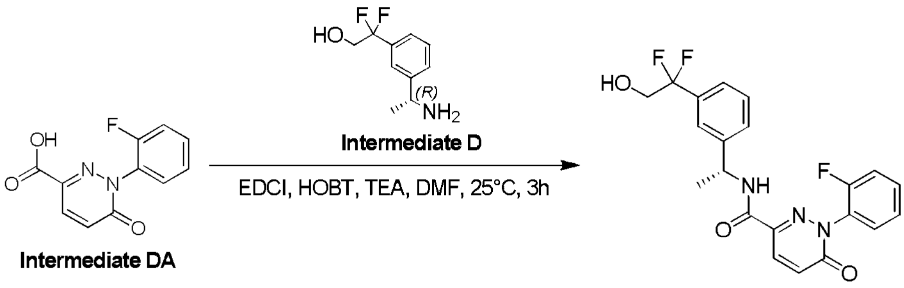


(R)-N-(1-(3-(1,1-difluoro-2-hydroxyethyl)phenyl)ethyl)-1-(2-fluorophenyl)-6-oxo-1,6-dihydropyridazine-3-carboxamide was obtained in a similar manner to Step 2 of Example 51 by using Intermediate D and Intermediate DA.

Steps 2 and 3: Synthesis of N-[(1R)-1-[3-[2-(dimethylamino)-1,1-difluoroethyl]phenyl]ethyl]-1-(2-fluorophenyl)-6-oxo-pyridazine-3-carboxamide

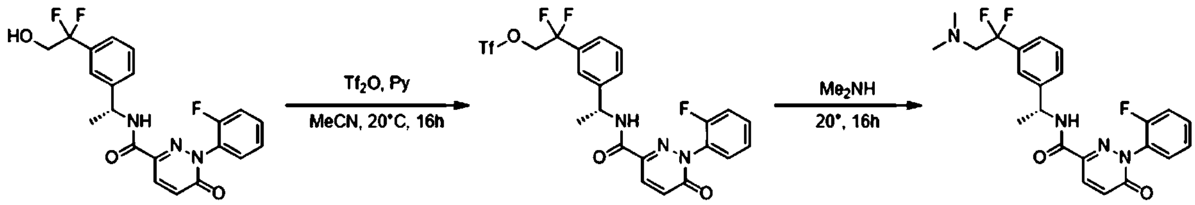


258

The compound of Example 258 (10.3 mg, 12.67% yield) was obtained as a yellow solid in the same manner as in Example 243, except that $Me_2NH$ was used instead of $NH_3 \cdot H_2O$. $^1$H NMR (400MHz, DMSO-$d_6$) $\delta$ = 8.99 (d, $J$ = 8.4 Hz, 1H), 7.93 (d, $J$ = 9.6 Hz, 1H), 7.72 - 7.64 (m, 1H), 7.62 - 7.53 (m, 2H), 7.51 - 7.44 (m, 2H), 7.43 - 7.36 (m, 3H), 7.18 (d, $J$ = 10.0 Hz, 1H), 5.17 (t, $J$ = 8.0 Hz, 1H), 2.98 (t, $J$ = 14.8 Hz, 2H), 2.16 (s, 6H), 1.47 (d, $J$ = 7.2 Hz, 3H); MS (ESI) m/z = 445.2 [M+H]$^+$.

**Example 259: 1-(2-fluorophenyl)-N-[(1R)-1-[2-fluoro-3-(trifluoromethyl)phenyl]ethyl]-6-oxo-pyridazine-3-carboxamide**

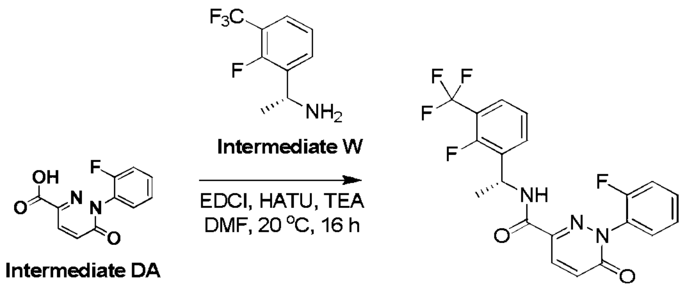


259

The compound of Example 259 (9.3 mg, 10.54% yield) was obtained as a white solid in a similar manner to Step 2 of Example 76 by using Intermediate W and changing the coupling reagents. $^1$H NMR (400MHz, DMSO-$d_6$) $\delta$ = 9.11 (d, $J$ = 7.6 Hz, 1H), 7.92 (d, $J$ = 10.0 Hz, 1H), 7.80 (t, $J$ = 7.2 Hz, 1H), 7.73-7.63 (m, 2H), 7.62-7.55 (m, 1H), 7.49-7.33 (m, 3H), 7.18 (d, $J$ = 10.0 Hz, 1H), 5.40 (quin, $J$ = 7.2 Hz, 1H), 1.48 (d, $J$ = 7.2 Hz, 3H); MS (ESI) m/z = 424.3 [M+H]$^+$.

**Example 260: N-[(1R)-1-(3-cyano-2-fluoro-phenyl)ethyl]-1-(2-fluorophenyl)-6-oxo-pyridazine-3-carboxamide**

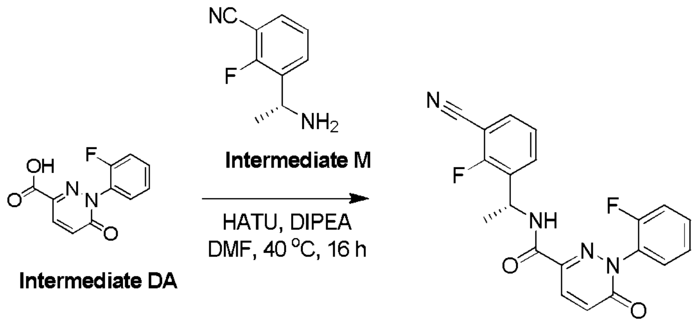


260

The compound of Example 260 (20.1 mg, 20.14% 2-step yield) was obtained as a white solid by reacting Intermediate M and Intermediate DA in a similar manner to Step 2 of Example 64. $^1$H NMR (400MHz, DMSO-$d_6$) δ = 9.09 (d, *J* = 7.6 Hz, 1H), 7.91 (d, *J* = 9.6 Hz, 1H), 7.79-7.86 (m, 2H), 7.69 (td, *J* = 7.6, 1.6 Hz, 1H), 7.55-7.64 (m, 1H), 7.37-7.49 (m, 3H), 7.18 (d, *J* = 9.6 Hz, 1H), 5.36 (quin, J = 7.2 Hz, 1H), 1.47 (d, J = 7.2 Hz, 3H); MS (ESI) m/z = 381.1 $[M+H]^+$.

**Example 261 and Example 262: N-[(1R)-1-(2-chloro-3-fluoro-phenyl)ethyl]-1-(2-fluorophenyl)-6-oxo-pyridazine-3-carboxamide and N-[(1S)-1-(2-chloro-3-fluorophenyl)ethyl]-1-(2-fluorophenyl)-6-oxo-pyridazine-3-carboxamide**

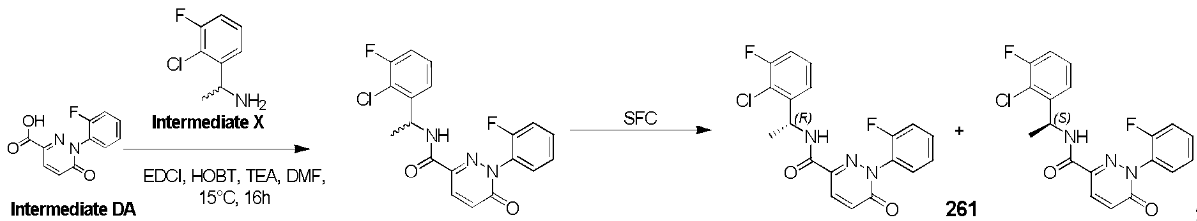


261 262

After performing Step 2 of Example 76 using Intermediate X, the product was separated by SFC to obtain the compound of Example 261 (42.5 mg, 41.97% yield) and the compound of Example 262 (28.1 mg, 68.76 μmol, 26.80% yield) as a white solid, respectively.

Compound of Example 261: $^1$H NMR (400MHz, DMSO-$d_6$) δ = 9.12 (d, *J* = 7.6 Hz, 1H), 7.91 (d, *J* = 10.0 Hz, 1H), 7.70 (dt, *J* = 1.6, 7.6 Hz, 1H), 7.63 - 7.55 (m, 1H), 7.50 - 7.40 (m, 2H), 7.39 - 7.33 (m, 2H), 7.32 - 7.25 (m, 1H), 7.18 (d, *J* = 9.6 Hz, 1H), 5.41 (quin, *J* = 7.2 Hz, 1H), 1.43 (d, *J* = 6.8 Hz, 3H); MS (ESI) m/z = 390.3 $[M+H]^+$.

Compound of Example 262: $^1$H NMR (400MHz, DMSO-$d_6$) δ = 9.12 (d, *J* = 7.6 Hz, 1H), 7.91 (d, *J* = 10.0 Hz, 1H), 7.73 - 7.67 (m, 1H), 7.63 - 7.56 (m, 1H), 7.49 - 7.41 (m, 2H), 7.39 - 7.34 (m, 2H), 7.32 - 7.26 (m, 1H), 7.18 (d, *J* = 9.6 Hz, 1H), 5.41 (quin, *J* = 7.2 Hz, 1H), 1.43 (d, *J* = 6.8 Hz, 3H); MS (ESI) m/z = 390.3 $[M+H]^+$.

**Example 263: N-[(1R)-1-[3-amino-5-(difluoromethyl)phenyl]ethyl]-1-(2-fluorophenyl)-6-oxo-pyridine-3-carboxamide**

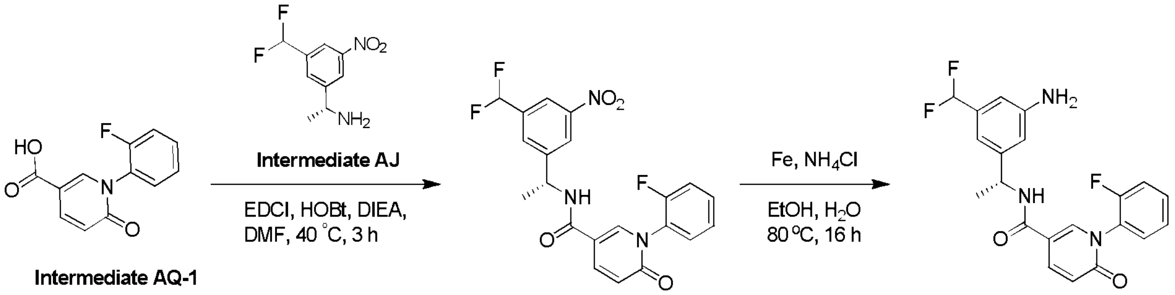


263

The compound of Example 263 (13.1 mg, 19.03% 2-step yield) was obtained as a white solid in a similar manner to steps 2 and 3 of Example 51. $^1$H NMR (400MHz, DMSO-$d_6$) δ = 8.54 (d, *J* = 7.6 Hz, 1H), 8.37 (d, *J* = 2.4 Hz, 1H), 7.99 (dd,

*J* = 9.6, 2.4 Hz, 1H), 7.55-7.64 (m, 2H), 7.38-7.50 (m, 2H), 6.66-6.96 (m, 1H), 6.65 (s, 2H), 6.54-6.59 (m, 2H), 5.35 (s, 2H), 4.99 (q, *J* = 7.2 Hz, 1H), 1.39 (d, *J*= 6.8 Hz, 3H); MS (ESI) m/z = 402.3 $[M+H]^+$.

**Example 264: N-[(1R)-1-[3-amino-5-(trifluoromethyl)phenyl]ethyl]-6-oxo-1-phenyl-4,5-dihydropyridazine-3-carboxamide**

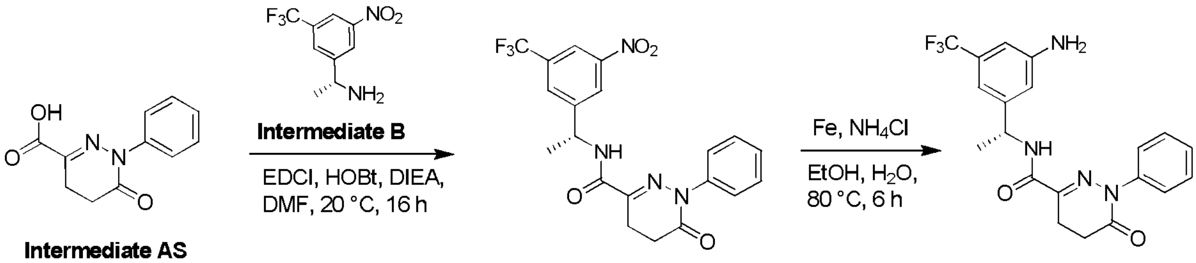


264

The compound of Example 264 (19.8 mg, 42.46% yield) was obtained as a white solid in a similar manner to Steps 2 and 3 of Example 51. $^1$H NMR (400 MHz, DMSO-$d_6$) δ = 8.54 (d, *J* = 8.4 Hz, 1H), 7.52 (d, *J* = 7.6 Hz, 2H), 7.43 (t, *J* = 7.2 Hz, 2H), 7.34 - 7.26 (m, 1H), 6.82 (s, 1H), 6.76 (s, 1H), 6.70 (s, 1H), 5.53 (s, 2H), 4.96 (quin, *J* = 7.2 Hz, 1H), 2.89 (t, *J* = 8.4 Hz, 2H), 2.65 (t, *J*= 8.4 Hz, 2H), 1.42 (d, *J*= 7.2 Hz, 3H); MS (ESI) m/z = 405.0 $[M+H]^+$.

**Example 265: N-[(1R)-1-(5-bromo-2-thienyl)ethyl]-1-(2-fluorophenyl)-6-oxo-pyridazine-3-carboxamide**

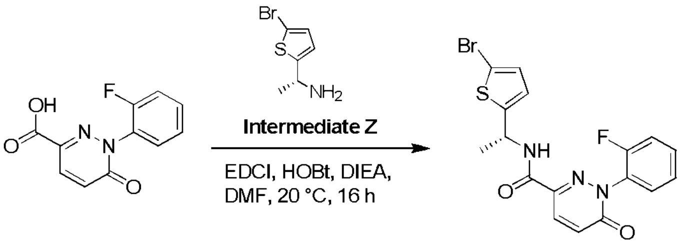


265

The compound of Example 265 (10.1 mg, 19.49% yield) was obtained as a white solid by using Intermediate Z in the same manner as in Step 2 of Example 76. $^1$H NMR (400 MHz, DMSO-$d_6$) δ = 9.04 (d, *J*=8.4 Hz, 1H), 7.97 (d, *J*=10.0 Hz, 1H), 7.66 (dt, *J*=1.8, 7.6 Hz, 1H), 7.61-7.54 (m, 1H), 7.48-7.37 (m, 2H), 7.19 (d, *J* = 10.0 Hz, 1H), 7.05 (d, *J* = 3.6 Hz, 1H), 6.83 (dd, *J* = 0.8, 3.6 Hz, 1H), 5.28 (quin, *J* = 7.2 Hz, 1H), 1.53 (d, *J* = 7.2 Hz, 3H); MS (ESI) m/z = 424.4 $[M+H]^+$.

**Example 266: 1-(2-fluorophenyl)-N-[(1R)-1-[5-[4-(methylaminomethyl)-3-bicyclo [4.2.0]octa-1,3,5-trienyl]-2-thienyl]ethyl]-6-oxo-pyridazine-3-carboxamide**

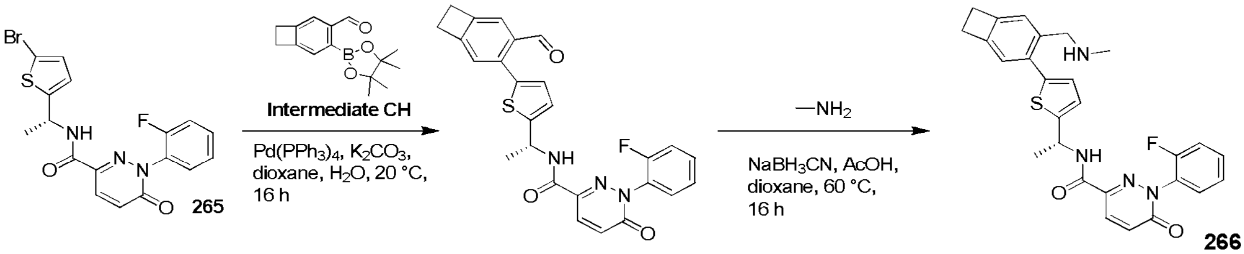


266

The compound of Example 266 (8.2 mg, 12.51% yield) was obtained as a white solid by reacting the compound of Example 265 and Intermediate CH in the same manner as in Steps 2 and 3 of Example 137. $^1$H NMR (400 MHz, DMSO-$d_6$) δ = 9.04 (d, *J* = 8.6 Hz, 1H), 7.99 (d, *J* = M 10.0 Hz, 1H), 7.66 (dt, *J* = 1.6, 7.6 Hz, 1H), 7.61 - 7.53 (m, 1H), 7.48 - 7.35 (m, 2H),

7.24 - 7.14 (m, 2H), 7.05 - 6.92 (m, 3H), 5.45 - 5.34 (m, 1H), 3.58 (s, 2H), 3.14 (s, 4H), 2.22 (s, 3H), 1.58 (d, *J* = 7.2 Hz, 3H); MS (ESI) m/z = 489.1 [M-16+H] $^+$.

**Example 267 to Example 293**

The compounds shown in the following table were obtained in the same manner as in Example 266 by using appropriate starting materials and intermediates corresponding to the respective structures of the desired compounds.

[Table 17]

| No. | Structure/Name | Spectral Data |
|---|---|---|
| 267 | 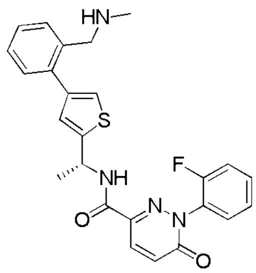 1-(2-fluorophenyl)-N-[(1R)-1-[4-[2-(methylaminomethyl)phenyl]-2-thienyl]ethyl]-6-oxo-pyridazine-3-carboxamide | $^1$H NMR (400 MHz, DMSO-$d_6$) δ = 9.03 (d, J = 8.4 Hz, 1H), 7.99 (d, J = 9.6 Hz, 1H), 7.66 (dt, J = 1.6, 7.6 Hz, 1H), 7.61 - 7.53 (m, 1H), 7.49 - 7.41 (m, 3H), 7.41 - 7.35 (m, 1H), 7.32 - 7.24 (m, 3H), 7.23 - 7.17 (m, 2H), 5.48 - 5.35 (m, 1H), 3.56 (s, 2H), 2.24 (s, 3H), 1.60 (d, J = 7.2 Hz, 3H); LC/MS (ESI) m/z: 463.3 [M+H]$^+$ |
| 268 | 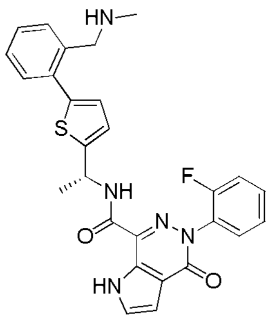 5-(2-fluorophenyl)-N-[(1R)-1-[5-[2-(methylaminomethyl)phenyl]-2-thienyl]ethyl]-4-oxo-1H-pyrrolo[2,3-d]pyridazine-7-carboxamide | $^1$H NMR (400 MHz, DMSO-$d_6$) δ = 12.24 (br s, 1H), 9.09 (br d, *J* = 8.4 Hz, 1H), 7.67 - 7.59 (m, 1H), 7.57 -7.51 (m, 1H), 7.50 - 7.47 (m, 1H), 7.45 (d, *J* = 2.8 Hz, 1H), 7.44 - 7.36 (m, 2H), 7.36 - 7.30 (m, 2H), 7.30 - 7.26 (m, 1H), 7.13 (d, *J* = 3.6 Hz, 1H), 7.04 (dd, *J* = 0.8, 3.6 Hz, 1H), 6.78 (d, *J* = 2.8 Hz, 1H), 5.49 (t, *J* = 7.2 Hz, 1H), 3.64 (s, 2H), 2.24 (s, 3H), 1.64 (d, *J* = 7.2 Hz, 3H); LC/MS (ESI) m/z: 502.4 [M+H]$^+$ |
| 269 | 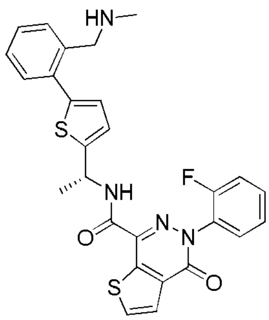 5-(2-fluorophenyl)-N-[(1R)-1-[5-[2-(methylaminomethyl)phenyl]-2-thienyl]ethyl]-4-oxo-thieno[2,3-d]pyridazine-7-carboxamide | $^1$H NMR (400 MHz, DMSO-$d_6$) δ = 9.27 (d, *J* = 8.6 Hz, 1H), 8.27 (d, *J* = 5.2 Hz, 1H), 7.77 - 7.68 (m, 2H), 7.63 - 7.54 (m, 1H), 7.51 - 7.38 (m, 3H), 7.38 - 7.23 (m, 3H), 7.13 (*d, J* = 3.6 Hz, 1H), 7.07 - 7.01 (m, 1H), 5.48 (quin, *J* = 7.2 Hz, 1H), 3.64 (s, 2H), 2.24 (s, 3H), 2.14 - 1.86 (m, 1H), 1.64 (d, *J* = 7.2 Hz, 3H); LC/MS (ESI) m/z: 519.3 [M+H]$^+$ |

(continued)

| No. | Structure/Name | Spectral Data |
|---|---|---|
| 270 | 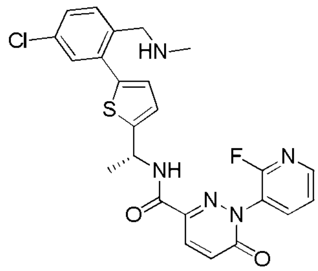 N-[(1R)-1-[5-[5-chloro-2-(methylaminomethyl)phenyl]-2-thienyl] ethyl]-1-(2-fluoro-3-pyridyl)-6-oxo-pyridazine-3-carboxamide | $^1$H NMR (400 MHz, DMSO-$d_6$) δ = 9.13 (*d*, *J* = 8.4 Hz, 1H), 8.40 (br d, *J* = 4.8 Hz, 1H), 8.35 - 8.28 (m, 1H), 8.16 (s, 1H), 8.00 (d, *J* = 9.6 Hz, 1H), 7.68 - 7.56 (m, 2H), 7.49 (dd, *J* = 2.0, 8.4 Hz, 1H), 7.40 (d, *J* = 2.0 Hz, 1H), 7.23 (*d, J* = 9.6 Hz, 1H), 7.16 (d, *J* = 3.6 Hz, 1H), 7.05 (*d, J* = 3.6 Hz, 1H), 5.54 - 5.33 (m, 1H), 3.90 (s, 2H), 2.37 (s, 3H), 1.60 (d, *J* = 7.2 Hz, 3H); LC/MS (ESI) m/z: 498.3 [M+H]$^+$ |
| 271 | 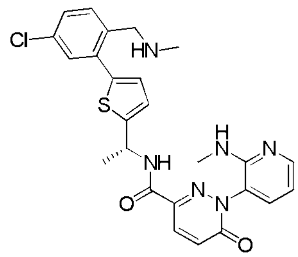 N-[(1R)-1-[5-[5-chloro-2-(methylaminomethyl)phenyl]-2-thienyl] ethyl]-1-[2-(methylamino)-3-pyridyl]-6-oxo-pyridazine-3-carboxamide | $^1$H NMR (400 MHz, DMSO-$d_6$) δ = 8.97 (d, *J* = 8.4 Hz, 1H), 8.18 (s, 1H), 8.12 (dd, *J* = 1.6, 4.8 Hz, 1H), 7.94 (d, *J* = 10.0 Hz, 1H), 7.57 (d, *J* = 8.4 Hz, 1H), 7.45 (dt, *J* = 2.0, 8.0 Hz, 2H), 7.36 (d, *J* = 2.4 Hz, 1H), 7.16 (d, *J* = 3.6 Hz, 1H), 7.12 (d, *J* = 9.8 Hz, 1H), 7.03 (d, *J* = 2.8 Hz, 1H), 6.62 (dd, *J* = 4.8, 7.2 Hz, 1H), 6.35 (q, *J* = 4.4 Hz, 1H), 5.41 (quin, *J* = 7.2 Hz, 1H), 3.77 (s, 2H), 2.76 (d, *J* = 4.4 Hz, 3H), 2.31 (s, 3H), 1.59 (d, *J* = 6.8 Hz, 3H); LC/MS (ESI) m/z: 509.4 [M+H]$^+$ |
| 272 | 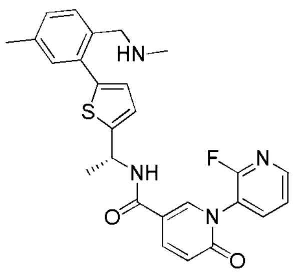 1-(2-fluoro-3-pyridyl)-N-[(1R)-1-[5-[5-methyl-2-(methylaminomethyl)phenyl]-2-thienyl]ethyl]-6-oxo-pyridine-3-carboxamide | $^1$H NMR (400 MHz, DMSO-$d_6$) δ = 9.08 (d, *J* = 8.0 Hz, 1H), 8.60 (d, *J* = 2.0 Hz, 1H), 8.36 (dd, *J* = 2.4, 8.4 Hz, 1H), 8.14 (br d, *J* = 4.8 Hz, 1H), 8.04 - 7.93 (m, 1H), 7.48 (dd, *J* = 5.2, 7.6 Hz, 1H), 7.37 (d, *J* = 8.0 Hz, 1H), 7.31 (d, *J* = 8.4 Hz, 1H), 7.20 - 7.10 (m, 3H), 7.02 (d, *J* = 3.2 Hz, 1H), 5.49 - 5.41 (m, 1H), 3.62 (s, 2H), 2.29 (s, 3H), 2.25 (s, 3H), 1.61 (d, *J* = 6.8 Hz, 3H); LC/MS (ESI) m/z: 477.4 [M+H]$^+$ |
| 273 | 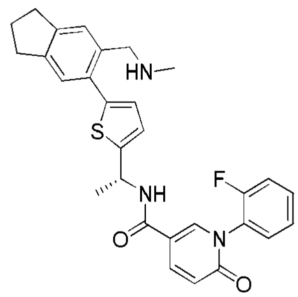 1-(2-fluorophenyl)-N-[(1R)-1-[5-[6-(methylaminomethyl)indan-5-yl]-2-thienyl]ethyl]-6-oxo-pyridine-3-carboxamide | $^1$H NMR (400 MHz, DMSO-$d_6$) δ = 8.74 (d, *J* = 8.0 Hz, 1H), 8.37 (d, *J* = 2.4 Hz, 1H), 8.01 (dd, *J* = 2.4, 9.6 Hz, 1H), 7.63 - 7.55 (m, 2H), 7.47 (t, *J* = 8.8 Hz, 1H), 7.42 - 7.37 (m, 2H), 7.21 (s, 1H), 7.04 (d, *J* = 4.0 Hz, 1H), 7.00 (d, *J* = 3.2 Hz, 1H), 6.58 (d, *J* = *9.6* Hz, 1H), 5.43 - 5.35 (m, 1H), 3.79 (s, 2H), 2.86 (q, *J* = 7.2 Hz, 4H), 2.34 (s, 3H), 2.05 - 1.99 (m, 2H), 1.56 (d, *J* = 7.2 Hz, 3H) ; LC/MS (ESI) m/z: 502.4 [M+H]$^+$ |

(continued)

| No. | Structure/Name | Spectral Data |
|---|---|---|
| 274 | 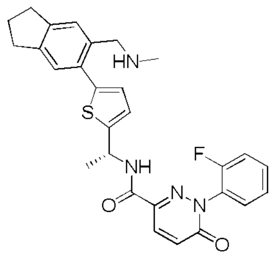<br>1-(2-fluorophenyl)-N-[(1R)-1-[5-[6-(methylaminomethyl)indan-5-yl]-2-thienyl]ethyl]-6-oxo-pyridazine-3-carboxamide | $^1$H NMR (400 MHz, DMSO-$d_6$) δ = 9.05 (d, *J* = 8.8 Hz, 1H), 7.99 (d, *J* = 10.0 Hz, 1H), 7.67 (t, *J* = 7.2 Hz, 1H), 7.60 - 7.54 (m, 1H), 7.46 - 7.37 (m, 2H), 7.34 (s, 1H), 7.21 - 7.15 (m, 2H), 7.05 (d, *J* = 3.6 Hz, 1H), 6.97 (d, *J* = 3.6 Hz, 1H), 5.43 - 5.36 (m, 1H), 3.33 (s, 3H), 2.84 (q, *J* = 6.8 Hz, 4H), 2.24 (s, 3H), 2.04 - 1.97 (m, 2H), 1.58 (d, *J* = 7.2 Hz, 3H) ; LC/MS (ESI) m/z: 503.4 [M+H]$^+$ |
| 275 | 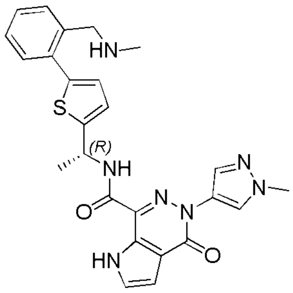<br>N-[(1R)-1-[5-[2-(methylaminomethyl)phenyl]-2-thienyl]ethyl]-5-(1-methylpyrazol-4-yl)-4-oxo-1H-pyrrolo[2,3-d]pyridazine-7-carboxamide | $^1$H NMR (400 MHz, DMSO-$d_6$) δ = 9.16 (d, *J* = 8.8 Hz, 1H), 8.48 (s, 1H), 8.31 (s, 1H), 7.49 (d, *J* = 6.4 Hz, 1H), 7.42 (d, *J* = 3.2 Hz, 1H), 7.32 (dq, *J* = 1.6, 7.6 Hz, 2H), 7.29 - 7.24 (m, 1H), 7.17 (d, *J* = 3.6 Hz, 1H), 7.09 (dd, *J* = 1.2, 3.6Hz, 1H), 6.78 (d, *J* = 3.2 Hz, 1H), 5.59 - 5.52 (m, 1H), 3.90 (s, 3H), 3.67 (s, 2H), 2.25 (s, 3H), 1.73 (d, *J* = 7.2 Hz, 3H) ; LC/MS (ESI) m/z: 488.4 [M+H]$^+$ |
| 276 | 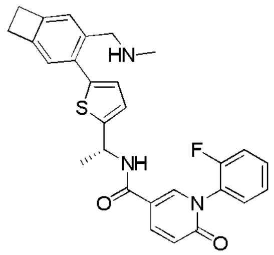<br>1-(2-fluorophenyl)-N-[(1R)-1-[5-[4-(methylaminomethyl)-3-bicyclo [4.2.0]octa-1,3,5-trienyl]-2-thienyl]ethyl]-6-oxo-pyridine-3-carboxamide | $^1$H NMR (400 MHz, DMSO-$d_6$) δ = 8.73 (d, *J* = 8.0 Hz, 1H), 8.36 (d, *J* = 2.4 Hz, 1H), 8.01 (dd, *J* = 2.4, 9.6 Hz, 1H), 7.63 - 7.55 (m, 2H), 7.49 - 7.37 (m, 2H), 7.21 (s, 1H), 7.05 - 6.92 (m, 3H), 6.57 (d, *J* = 9.6 Hz, 1H), 5.39 (br t, *J* = 7.2 Hz, 1H), 3.62 (s, 2H), 3.14 (s, 4H), 2.24 (s, 3H), 1.55 (d, *J* = 6.8 Hz, 3H); LC/MS (ESI) m/z: 488.4 [M+H]$^+$ |
| 277 | 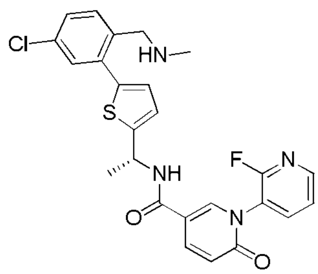<br>N-[(1R)-1-[5-[5-chloro-2-(methylaminomethyl)phenyl]-2-thienyl] ethyl]-1-(2-fluoro-3-pyridyl)-6-oxo-pyridine-3-carboxamide | $^1$H NMR (400 MHz, DMSO-$d_6$) δ = 9.09 (d, *J* = 8.4 Hz, 1H), 8.60 (d, *J* = 2.4 Hz, 1H), 8.36 (dd, *J* = 2.4, 8.8 Hz, 1H), 8.14 (br d, *J* = 4.8 Hz, 1H), 8.01 - 7.94 (m, 1H), 7.53 (d, *J* = 8.0 Hz, 1H), 7.47 (dd, *J* = 5.2, 7.6 Hz, 1H), 7.41 - 7.34 (m, 2H), 7.31 (d, *J* = 8.4 Hz, 1H), 7.20 (d, *J* = 3.6 Hz, 1H), 7.05 *(d, J = 3.6* Hz, 1H), 5.48 - 5.41 (m, 1H), 3.64 (s, 2H), 2.24 (s, 3H), 1.61 (d, *J* = 7.2 Hz, 3H); LC/MS (ESI) m/z: 497.3 [M+H]$^+$ |

(continued)

| No. | Structure/Name | Spectral Data |
|---|---|---|
| 278 | 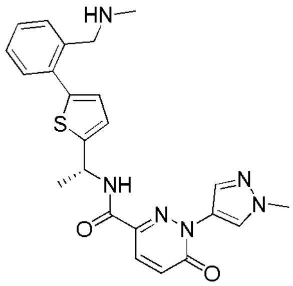 N-[(1R)-1-[5-[2-(methylaminomethyl)phenyl]-2-thienyl]ethyl]-1-(1-methylpyrazol-4-yl)-6-oxo-pyridazine-3-carboxamide | $^1$H NMR (400 MHz, DMSO-$d_6$) δ = 9.09 (d, *J* = 8.8 Hz, 1H), 8.55 (s, 1H), 8.36 (s, 1H), 7.93 (d, *J* = 9.6 Hz, 1H), 7.52 - 7.48 (m, 1H), 7.36 - 7.24 (m, 3H), 7.19 - 7.15 (m, 2H), 7.05 (dd, *J* = 0.8, 3.6 Hz, 1H), 5.48 (quin, *J* = 7.2 Hz, 1H), 3.90 (s, 3H), 3.66 (s, 2H), 2.25 (s, 3H), 1.68 (d, *J* = 6.8 Hz, 3H) ; LC/MS (ESI) m/z: 449.3 $[M+H]^+$ |
| 279 | 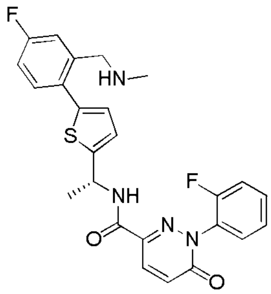 N-[(1R)-1-[5-[4-fluoro-2-(methylaminomethyl)phenyl]-2-thienyl]ethyl]-1-(2-fluorophenyl)-6-oxo-pyridazine-3-carboxamide | $^1$H NMR (400 MHz, DMSO-$d_6$) δ = 9.07 (d, *J* = 8.4 Hz, 1H), 7.99 (d, *J* = 9.6 Hz, 1H), 7.67 (dt, *J* = 1.6, 7.6 Hz, 1H), 7.61 - 7.54 (m, 1H), 7.48 - 7.32 (m, 4H), 7.19 (d, *J* = 9.6 Hz, 1H), 7.10 (dt, *J* = 2.8, 8.4 Hz, 1H), 7.04 (d, *J* = 3.6 Hz, 1H), 7.01 - 6.98 (m, 1H), 5.40 (quin, *J* = 7.2 Hz, 1H), 3.64 (s, 2H), 2.23 (s, 3H), 1.59 (d, *J* = 6.8 Hz, 3H) ; LC/MS (ESI) m/z: 481.4 $[M+H]^+$ |
| 280 | 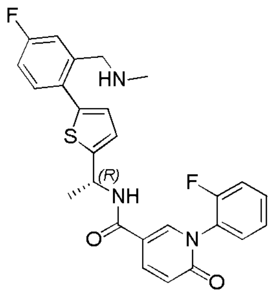 N-[(1R)-1-[5-[4-fluoro-2-(methylaminomethyl)phenyl]-2-thienyl]ethyl]-1-(2-fluorophenyl)-6-oxo-pyridine-3-carboxamide | $^1$H NMR (400 MHz, DMSO-$d_6$) δ = 8.74 (d, *J* = 8.0 Hz, 1H), 8.36 (d, *J* = 2.4 Hz, 1H), 8.01 (dd, *J* = 2.8, 9.6 Hz, 1H), 7.65 - 7.53 (m, 2H), 7.51 - 7.43 (m, 1H), 7.42 - 7.32 (m, 3H), 7.12 (dt, *J* = 2.8, 8.4 Hz, 1H), 7.05 (d, *J* = 3.6 Hz, 1H), 7.01 (d, *J* = 3.6 Hz, 1H), 6.57 (d, *J* = 9.6 Hz, 1H), 5.46 - 5.34 (m, 1H), 3.69 (s, 2H), 2.26 (s, 3H), 1.56 (d, *J* = 6.8 Hz, 3H) ; LC/MS (ESI) m/z: 480.3 $[M+H]^+$ |
| 281 | 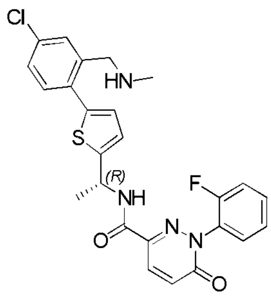 N-[(1R)-1-[5-[4-chloro-2-(methylaminomethyl)phenyl]-2-thienyl]ethyl]-1-(2-fluorophenyl)-6-oxo-pyridazine-3-carboxamide | $^1$H NMR (400 MHz, DMSO-$d_6$) δ = 9.08 (d, *J* = 8.4 Hz, 1H), 7.99 (d, *J* = 9.6 Hz, 1H), 7.67 (dt, *J* = 1.6, 7.6 Hz, 1H), 7.61 - 7.51 (m, 2H), 7.47 - 7.37 (m, 2H), 7.37 - 7.30 (m, 2H), 7.20 (d, *J* = 10.0 Hz, 1H), 7.11 (d, *J* = 3.6 Hz, 1H), 7.01 (dd, *J* = 0.8, 3.6 Hz, 1H), 5.40 (quin, *J* = 7.2 Hz, 1H), 3.64 (s, 2H), 2.23 (s, 3H), 1.59 (d, *J* = 6.8 Hz, 3H) ; LC/MS (ESI) m/z: 497.3 $[M+H]^+$ |

(continued)

| No. | Structure/Name | Spectral Data |
|---|---|---|
| 282 | 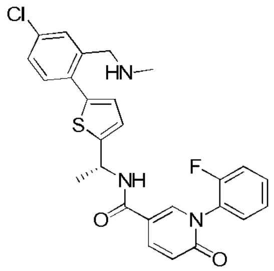 N-[(1R)-1-[5-[4-chloro-2-(methylaminomethyl)phenyl]-2-thienyl] ethyl]-1-(2-fluorophenyl)-6-oxo-pyridine-3-carboxamide | $^1$H NMR (400 MHz, DMSO-$d_6$) δ = 8.75 (br d, *J* = 7.6 Hz, 1H), 8.36 (s, 1H), 8.00 (dd, *J* = 1.6, 9.6 Hz, 1H), 7.66 - 7.52 (m, 3H), 7.50 - 7.43 (m, 1H), 7.42 - 7.31 (m, 3H), 7.12 (d, *J* = 3.2 Hz, 1H), 7.02 (br d, *J* = 3.6 Hz, 1H), 6.57 (d, *J* = 9.6 Hz, 1H), 5.46 - 5.33 (m, 1H), 3.69 (s, 2H), 2.26 (s, 3H), 1.56 (br d, *J* = 6.8 Hz, 3H) ; LC/MS (ESI) m/z: 4 96.3 [M+H]$^+$ |
| 283 | 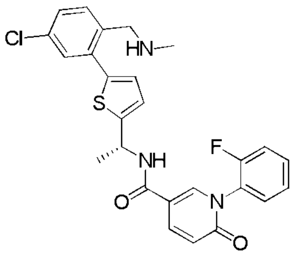 N-[(1R)-1-[5-[5-chloro-2-(methylaminomethyl)phenyl]-2-thienyl] ethyl]-1-(2-fluorophenyl)-6-oxo-pyridine-3-carboxamide | $^1$H NMR (400 MHz, DMSO-$d_6$) δ = 8.75 (d, J = 8.4 Hz, 1H), 8.37 (d, J = 2.4 Hz, 1H), 8.18 (s, 1H), 8.01 (dd, J = 2.8, 9.6 Hz, 1H), 7.64 - 7.52 (m, 3H), 7.50 - 7.35 (m, 4H), 7.18 (d, J = 3.6 Hz, 1H), 7.04 (dd, J = 0.8, 3.6 Hz, 1H), 6.58 (d, J = 9.6 Hz, 1H), 5.41 (t, J = 7.2 Hz, 1H), 3.70 (s, 2H), 2.27 (s, 3H), 1.57 (d, J = 7.2 Hz, 3H) ; LC/MS (ESI) m/z: 496. 3 [M+H]$^+$ |
| 284 | 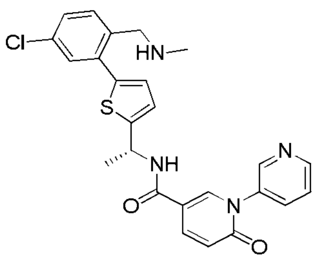 N-[(1R)-1-[5-[5-chloro-2-(methylaminomethyl)phenyl]-2-thienyl] ethyl]-6-oxo-1-(3-pyridyl)pyridine-3-carboxamide | $^1$H NMR (400 MHz, DMSO-$d_6$) δ = 8.77 (d, J = 8.2 Hz, 1H), 8.72 (d, J = 2.0 Hz, 1H), 8.70 - 8.65 (m, 1H), 8.41 (d, J = 2.0 Hz, 1H), 8.00 (dd, J = 2.0, 9.2 Hz, 2H), 7.61 (dd, J = 4.8, 8.4 Hz, 1H), 7.53 (d, J = 8.4 Hz, 1H), 7.42 - 7.34 (m, 2H), 7.21 - 7.17 (m, 1H), 7.06 - 7.02 (m, 1H), 6.58 (d, J = 10.0 Hz, 1H), 5.41 (br t, J = 7.2 Hz, 1H), 3.62 (s, 2H), 2.23 (s, 3H), 1.57 (d, J = 6.8 Hz, 3H) ; LC/MS (ESI) m/z: 496.3 [M+H]$^+$ |
| 285 | 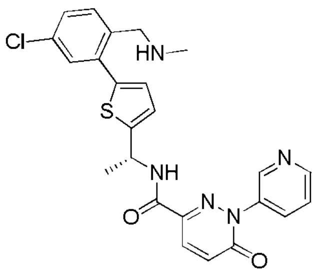 N-[(1R)-1-[5-[5-chloro-2-(methylaminomethyl)phenyl]-2-thienyl] ethyl]-6-oxo-1-(3-pyridyl)pyridazine-3-carboxamide | $^1$H NMR (400 MHz, DMSO-$d_6$) δ = 9.13 (d, J = 8.6 Hz, 1H), 8.95 (d, J = 2.0 Hz, 1H), 8.64 (dd, J = 1.6, 4.8 Hz, 1H), 8.22 - 8.13 (m, 1H), 7.96 (d, J = 10.0 Hz, 1H), 7.63 - 7.55 (m, 1H), 7.53 (d, J = 8.4 Hz, 1H), 7.42 - 7.31 (m, 2H), 7.23 - 7.15 (m, 2H), 7.04 (dd, J = 1.2, 3.6 Hz, 1H), 5.49 - 5.37 (m, 1H), 3.62 (s, 2H), 2.23 (s, 3H), 1.62 (d, J = 7.2 Hz, 3H) ; LC/MS (ESI) m/z: 480.2 [M+H]$^+$ |

(continued)

| No. | Structure/Name | Spectral Data |
|---|---|---|
| 286 | 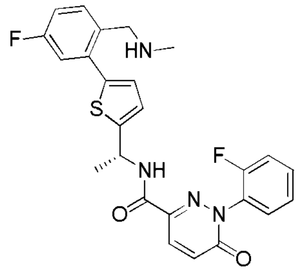 N-[(1R)-1-[5-[5-fluoro-2-(methylaminomethyl)phenyl]-2-thienyl] ethyl]-1-(2-fluorophenyl)-6-oxo-pyridazine-3-carboxamide | $^1$H NMR (400 MHz, Methanol-$d_4$) δ = 8.09 (d, J = 10.0 Hz, 1H), 7.62 - 7.51 (m, 2H), 7.50 - 7.43 (m, 1H), 7.40 - 7.29 (m, 2H), 7.19 (d, J = 10.0 Hz, 1H), 7.14 - 7.06 (m, 2H), 7.04 (dd, J = 0.8, 3.6 Hz, 1H), 6.99 (d, J = 3.6 Hz, 1H), 5.50 (q, J = 6.8 Hz, 1H), 3.78 (s, 2H), 2.30 (s, 3H), 1.67 (d, J = 7.2 Hz, 3H) ; LC/MS (ESI) m/z: 481.3 [M+H]$^+$ |
| 287 | 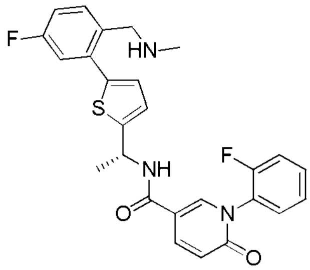 N-[(1R)-1-[5-[5-fluoro-2-(methylaminomethyl)phenyl]-2-thienyl] ethyl]-1-(2-fluorophenyl)-6-oxo-pyridine-3-carboxamide | $^1$H NMR (400 MHz, DMSO-$d_6$) δ = 8.75 (d, J = 8.0 Hz, 1H), 8.37 (d, J = 2.8 Hz, 1H), 8.01 (dd, J = 2.8, 9.6 Hz, 1H), 7.66 - 7.34 (m, 5H), 7.26 - 7.11 (m, 3H), 7.03 (d, J = 3.6 Hz, 1H), 6.57 (d, J = 9.6 Hz, 1H), 5.40 (t, J = 7.2 Hz, 1H), 3.61 (s, 2H), 2.24 (s, 3H), 1.56 (d, J = 7.2 Hz, 3H) ; LC/MS (ESI) m/z: 480.4 [M+H]$^+$ |
| 288 | 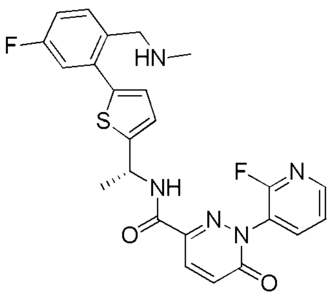 N-[(1R)-1-[5-[5-fluoro-2-(methylaminomethyl)phenyl]-2-thienyl] ethyl]-1-(2-fluoro-3-pyridyl)-6-oxo-pyridazine-3-carboxamide | $^1$H NMR (400 MHz, DMSO-$d_6$) δ = 9.12 (d, J = 8.8 Hz, 1H), 8.40 (d, J = 4.8 Hz, 1H), 8.35 - 8.28 (m, 1H), 8.00 (d, J = 10.0 Hz, 1H), 7.62 (dd, J = 5.6, 7.2 Hz, 1H), 7.52 (br t, J = 7.6 Hz, 1H), 7.26 - 7.07 (m, 4H), 7.02 (d, J = 3.6 Hz, 1H), 5.41 (quin, J = 7.6 Hz, 1H), 3.60 (s, 2H), 2.23 (s, 3H), 1.59 (d, J = 7.2 Hz, 3H) ; LC/MS (ESI) m/z: 482. 4 [M+H]$^+$ |
| 289 | 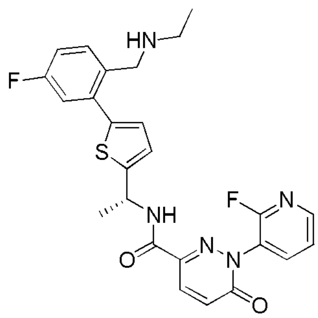 N-[(1R)-1-[5-[2-(ethylaminomethyl)-5-fluorophenyl]-2-thienyl] ethyl]-1-(2-fluoro-3-pyridyl)-6-oxo-pyridazine-3-carboxamide | $^1$H NMR (400 MHz, Methanol-$d_4$) δ = 8.37 (br d, J = 4.8 Hz, 1H), 8.28 - 8.15 (m, 1H), 8.11 (d, J = 10.0 Hz, 1H), 7.58 - 7.44 (m, 2H), 7.22 (d, J = 10.0 Hz, 1H), 7.15 - 7.04 (m, 3H), 7.01 (d, J = 3.6 Hz, 1H), 5.52 (q, J = 7.2 Hz, 1H), 3.83 (s, 2H), 2.56 (q, J = 7.2 Hz, 2H), 1.69 (d, J = 7.2 Hz, 3H), 1.03 (t, J = 7.2 Hz, 3H) ; LC/MS (ESI) m/z: 496.3 [M+H]$^+$ |

(continued)

| No. | Structure/Name | Spectral Data |
|---|---|---|
| 290 | 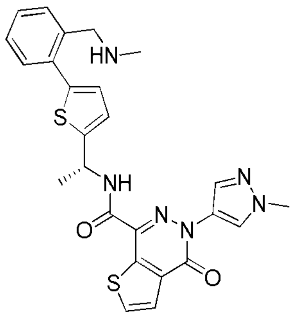 N-[(1R)-1-[5-[2-(methylaminomethyl)phenyl]-2-thienyl]ethyl]-5-(1-methylpyrazol-4-yl)-4-oxo-thieno[2,3-d]pyridazine-7-carboxamide | $^1$H NMR (400 MHz, DMSO-$d_6$) δ = 9.28 (d, *J* = 8.8 Hz, 1H), 8.57 (s, 1H), 8.41 (s, 1H), 8.22 (d, *J* = 5.6 Hz,1H), 7.73 (d, *J* = 5.2 Hz, 1H), 7.50 (d, *J* = 7.2 Hz, 1H), 7.36 - 7.26 (m, 3H), 7.17 (d, *J* = 3.6 Hz, 1H), 7.09 (dd, *J* = 0.8, 3.6 Hz, 1H), 5.59 - 5.52 (m, 1H), 3.92 (s, 3H), 3.70 (s, 2H), 2.27 (s, 3H), 1.73 (d, *J* = 7.2 Hz, 3H) ; LC/MS (ESI) m/z: 505.4 $[M+H]^+$ |
| 291 | 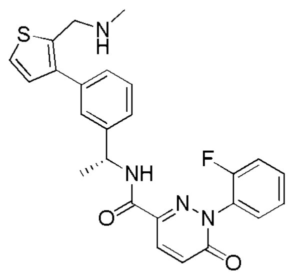 1-(2-fluorophenyl)-N-[(1R)-1-[3-[2-(methylaminomethyl)-3-thienyl] phenyl]ethyl]-6- | $^1$H NMR (400 MHz, DMSO-$d_6$) δ = 8.92 (d, *J* = 8.0 Hz, 1H), 7.94 (d, *J* = 9.6 Hz, 1H), 7.54-7.71 (m, 1H), 7.53-7.70 (m, 2H), 7.07-7.47 (m, 9H), 5.10-5.24 (m, 1H), 3.79 (s, 2H), 2.50 (s, 3H), 2.24 (s, 3H), 1.50 (d, *J* = 6.4 Hz, 3H); LC/MS (ESI) m/z: 463.3 $[M+H]^+$ |
| | oxo-pyridazine-3-carboxamide | |
| 292 | 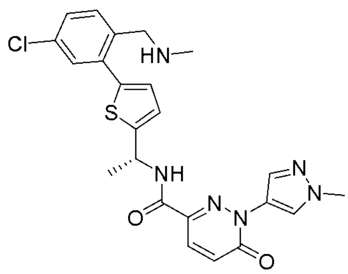 (R)-N-(1-(5-(5-chloro-2-((methylamino)methyl)phenyl)thiophen-2-yl)ethyl)-1-(1-methyl-1H-pyrazol-4-yl)-6-oxo-1,6-dihydropyridazine-3-carboxamide | $^1$H NMR (400MHz, DMSO-$d_6$) δ = 9.11 (d, J = 8.4 Hz, 1H), 8.55 (s, 1H), 8.37 (s, 1H), 7.93 (d, J = 9.6 Hz, 1H), 7.53 (br d, J = 8.0 Hz, 1H), 7.44 - 7.31 (m, 2H), 7.26-7.13 (m, 2H), 7.07 (d, J = 3.6 Hz, 1H), 5.48 (br t, J = 7.2 Hz, 1H), 3.90 (s, 3H), 3.79 - 3.43 (m, 2H), 2.34 - 2.09 (m, 3H), 1.68 (d, J = 7.2 Hz, 3H) ; MS (ESI) m/z = 483.4 $[M+H]^+$ |
| 293 | 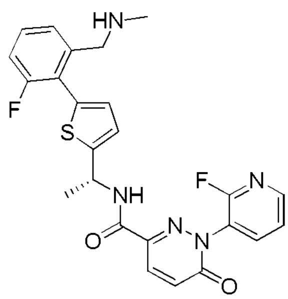 N-[(1R)-1-[5-[2-fluoro-6-(methylaminomethyl)phenyl]-2-thienyl] ethyl]-1-(2-fluoro-3-pyridyl)-6-oxo-pyridazine-3-carboxamide | $^1$H NMR (400MHz, DMSO-$d_6$) δ = 9.14 (d, *J* = 8.8 Hz, 1H), 8.40 (d, *J* = 4.8 Hz, 1H), 8.35 - 8.25 (m, 1H), 8.00 (d, *J* = 10.0 Hz, 1H), 7.66 - 7.57 (m, 1H), 7.44 - 7.33 (m, 2H), 7.23 (d, *J* = 10.0 Hz, 1H), 7.18 - 7.12 (m, 1H), 7.06 - 7.02 (m, 1H), 7.00 (d, *J* = 3.6 Hz, 1H), 5.49 -5.38 (m, 1H), 3.52 (s, 2H), 2.18 (s, 3H), 1.60 (d, *J* = 7.0 Hz, 3H); MS (ESI) m/z = 482.4 $[M+H]^+$ |

**Example 294: 1-(2-fluorophenyl)-N-[(1R)-1-[3-[3-(methylaminomethyl)-2-thienyl]phenyl]ethyl]-6-oxo-pyridazine-3-carboxamide**

Step 1: 1-(2-fluorophenyl)-6-oxo-pyridazine-3-carboxylic acid

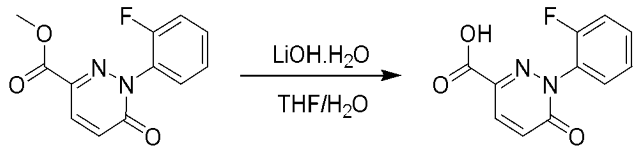


To a solution of 1-(2-fluorophenyl)-6-oxo-pyridazine-3-carboxylate (20 g, 80.58 mmol) in THF (180 mL), LiOH·$H_2O$(10.14 g, 241.73 mmol) and $H_2O$ (20 mL) were added. The mixture was stirred at 20 °C for 1 hour. The reaction mixture was poured into water (200 mL) and extracted with EtOAc (100 mL × 3). The organic layer was discarded, and the reaction mixture was then adjusted to pH = 3-4 with aq. 1 N HCl and extracted with EtOAc (200 mL × 3), The combined organic layer was washed with brine (30 mL x 2), dried over anhydrous $Na_2SO_4$ and concentrated under reduced pressure to give 1-(2-fluorophenyl)-6-oxo-pyridazine-3-carboxylic acid (18 g, 92.36% yield) as a white solid. $^1H$ NMR (400 MHz, DMSO-$d_6$) δ 13.79 (br s, 1H), 7.95 (d, *J* = 9.6 Hz, 1H), 7.65 - 7.53 (m, 2H), 7.46 (t, *J* = 9.2 Hz, 1H), 7.42 - 7.36 (m, 1H), 7.18 (d, *J* = 10.0 Hz, 1H); LC/MS (ESI) m/z = 235.0 $[M+H]^+$.

Step 2: N-[(1R)-1-(3-bromophenyl)ethyl]-1-(2-fluorophenyl)-6-oxo-pyridazine-3-carboxamide

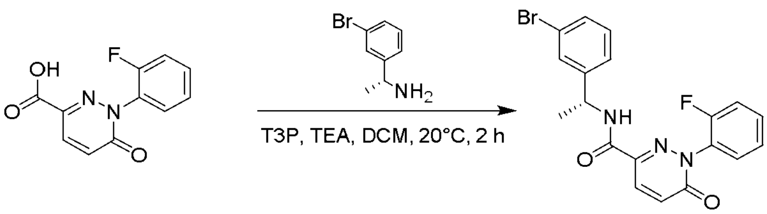


To a solution of (1R)-1-(3-bromophenyl)ethanamine (1 g, 5.00 mmol) and 1-(2-fluorophenyl)-6-oxo-pyridazine-3-carboxylic acid (1.17 g, 5.00 mmol) in DCM (15 mL), $T_3P$ (2.97 mL, 10.00 mmol) and triethylamine (2.09 mL, 14.99 mmol) were added, followed by stirring at 20 °C for 2 h under $N_2$ atmosphere. The reaction mixture was poured into water (10 mL) and extracted with EtOAc (10 mL×3). The combined organic layer was washed with brine (30 mL), dried over anhydrous $Na_2SO_4$ and filtered under reduced pressure to obtain a crude product. The product was purified by silica gel column chromatography (42% EtOAc in PE) to give N-[(1R)-1-(3-bromophenyl)ethyl]-1-(2-fluorophenyl)-6-oxo-pyridazine-3-carboxamide (1.61 g, 77.56% yield) as a yellow solid. $^1H$ NMR (400 MHz, DMSO-$d_6$) δ ppm 0.93 (m, 1 H) 1.45 (d, *J* = 7.2 Hz, 3 H) 5.11 (quin, *J* = 7.2 Hz, 1 H) 5.76 (s, 1 H) 7.17 (d, *J* = *9.6* Hz, 1 H) 7.28 (m, 1 H) 7.42 (m, 5 H) 7.59 (m, 3 H) 7.68 (t, *J* = 7.6 Hz, 1 H) 7.94 (m, 1 H) 8.96 (d, *J* = 8.0 Hz, 1 H); MS (ESI) m/z = 416.05 $[M+H]^+$.

Step 3: 1-(2-fluorophenyl)-N-[(1R)-1-[3-(3-formyl-2-thienyl)phenyl]ethyl]-6-oxo-pyridazine-3-carboxamide

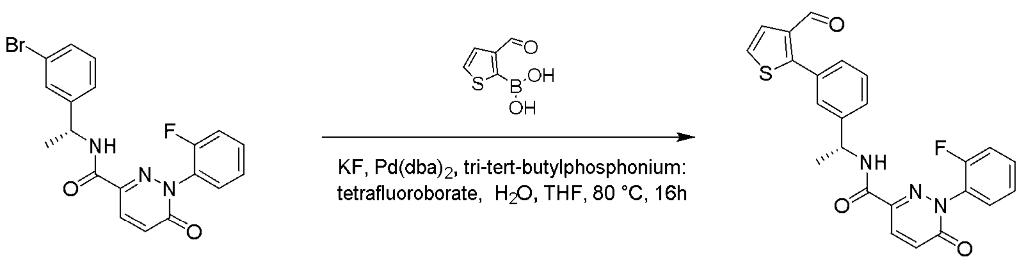


To a solution of N-[(1R)-1-(3-bromophenyl)ethyl]-1-(2-fluorophenyl)-6-oxo-pyridazine-3-carboxamide (556.00 mg, 1.34 mmol) and (3-formyl-2-thienyl)boronic acid (250 mg, 1.60 mmol) in THF (15 mL), KF (143.94 μL, 6.14 mmol), Pd(dba)$_2$ (38.40 mg, 66.79 μmol), $H_2O$ (144.38 μL, 8.01 mmol) and tri-tert-butylphosphoniumtetrafluoroborate (38.75 mg, 133.58 μmol) were added, followed by stirring at 80 °C for 16 h under $N_2$ atmosphere. The reaction mixture was poured into

water (10 mL) and extracted with EtOAc (10 mL×3). The combined organic layer was washed with brine (30 mL), dried over anhydrous $Na_2SO_4$ and concentrated under reduced pressure to obtain a crude product. The product was purified by silica gel column chromatography (37% EtOAc in PE) to give 1-(2-fluorophenyl)-N-[(1R)-1-[3-(3-formyl-2-thienyl)phenyl]ethyl]-6-oxo-pyridazine-3-carboxamide (450 mg, 70.02% yield) as a yellow solid. MS (ESI) m/z = 448.0 $[M+H]^+$.

Step 4: 1-(2-fluorophenyl)-N-[(1R)-1-[3-[3-(methylaminomethyl)-2-thienyl]phenyl]ethyl]-6-oxo-pyridazine-3-carboxamide

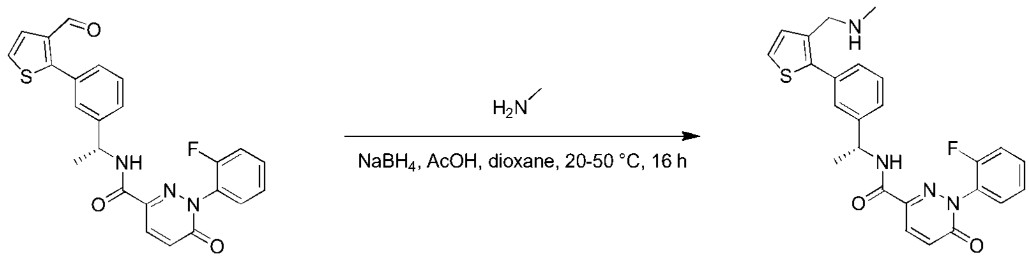


294

To a solution of 1-(2-fluorophenyl)-N-[(1R)-1-[3-(3-formyl-2-thienyl)phenyl]ethyl]-6-oxo-pyridazine-3-carboxamide (150 mg, 335.21 μmol) and methylamine hydrochloride (27.16 mg, 402.25 μmol) in dioxane (4 mL) was added acetic acid (38.34 μL, 670.42 μmol), followed by stirring at 20 °C for 30 min. The resulting mixture was added with $NaBH_4$ (126.39 mg, 2.01 mmol) and then stirred at 50 °C for 16 h. The reaction mixture was poured into water (30 mL) and extracted with EtOAc (30 mL×3). The organic layer was washed with brine (50 mL), dried over anhydrous $Na_2SO_4$ and filtered under reduced pressure to obtain a crude product. The product was purified by prep-HPLC (column: C18-6 100*30mm*5μm; mobile phase: [water(FA)-ACN];B%: 18%-48%,15 min). ACN was removed under reduced pressure, and the remaining solvent was removed by lyophilization to give the compound of Example 294 (7.4 mg, 4.31% yield) as a white solid. $^1H$ NMR (400 MHz, DMSO-$d_6$) δ ppm 1.50 (d, *J* = 7.2 Hz, 3 H) 2.29 (s, 3 H) 3.71 (s, 2 H) 5.18 (quin, *J* = 7.2 Hz, 1 H) 7.18 (d, *J* = 10.0 Hz, 1 H) 7.22 (d, *J* = 5.2 Hz, 1 H) 7.40 (m, 5 H) 7.52 (m, 2 H) 7.58 (m, 1 H) 7.67 (td, *J* = 7.6, 1.56 Hz, 1 H) 7.94 (d, *J* = 10.0 Hz, 1 H) 8.26 (br s, 1 H) 8.97 (d, *J* = 8.4 Hz, 1 H); MS (ESI) m/z = 463.3 $[M+H]^+$.

**Example 295: N-[(1R)-1-[5-[5-chloro-2-(methylaminomethyl)phenyl]-2-thienyl]ethyl]-1-(2-fluorophenyl)-6-oxo-pyridazine-3-carboxamide**

Step 1: Synthesis of N-[(1R)-1-(5-bromo-2-thienyl)ethyl]-1-(2-fluorophenyl)-6-oxo-pyridazine-3-carboxamide

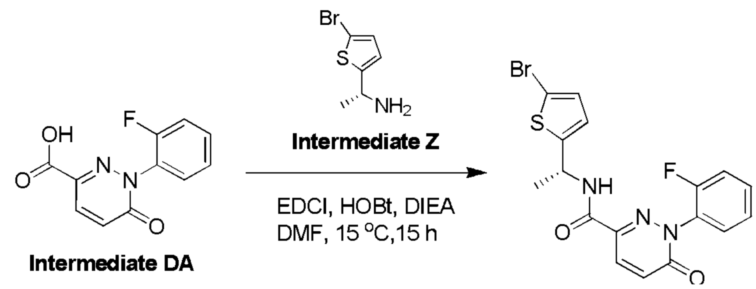


To a solution of Intermediate DA (5 g, 21.35 mmol) and Intermediate Z (5.18 g, 21.35 mmol) in DMF (70 mL), EDCI (6.14 g, 32.03 mmol), DIEA (8.28 g, 64.05 mmol, 11.16 mL) and HOBt (4.33 g, 32.03 mmol) were added. The mixture was degassed and purged with $N_2$ for 3 times, and then stirred at 15 °C for 15 h under $N_2$ atmosphere. The reaction mixture was poured into water (200 mL) and extracted with EtOAc (50 mL × 10). The combined organic layer was washed with brine (50 mL × 4), dried over anhydrous $Na_2SO_4$ and concentrated under reduced pressure to obtain a crude product. The product was purified by silica gel column chromatography (25% EtOAc in PE) to give N-[(1R)-1-(5-bromo-2-thienyl)ethyl]-1-(2-fluorophenyl)-6-oxo-pyridazine-3-carboxamide (9.11 g, 92.99% yield) as an off-white solid. $^1H$ NMR (400 MHz, DMSO-$d_6$) δ = 9.04 (br d, *J* = 8.4 Hz, 1H), 7.97 (d, *J* = 9.6 Hz, 1H), 7.66 (br t, *J* = 7.6 Hz, 1H), 7.62 - 7.52 (m, 1H), 7.50 - 7.34 (m, 2H), 7.19 (d, *J*= 10.0 Hz, 1H), 7.05 (d, *J* = 3.6 Hz, 1H), 6.87 - 6.76 (m, 1H), 5.29 (quin, *J* = 7.2 Hz, 1H), 1.53 (d, *J* = 7.2 Hz, 3H). LC/MS (ESI) m/z = 424.0 $[M+H]^+$.

Step 2: Synthesis of N-[(1R)-1-[5-(5-chloro-2-formyl-phenyl)-2-thienyl]ethyl]-1-(2-fluorophenyl)-6-oxo-pyridazine-3-carboxamide

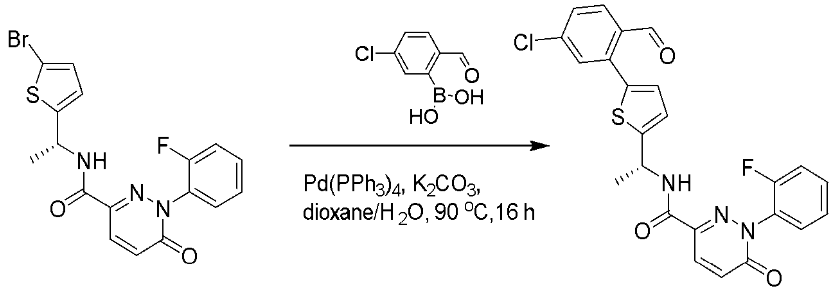


A mixture of N-[(1R)-1-(5-bromo-2-thienyl)ethyl]-1-(2-fluorophenyl)-6-oxo-pyridazine-3-carboxamide (9 g, 21.31 mmol), (5-chloro-2-formyl-phenyl)boronic acid (4.95 g, 26.85 mmol), $K_2CO_3$ (7.36 g, 53.28 mmol), and $Pd(PPh_3)_4$ (2.46 g, 2.13 mmol) in dioxane (90 mL) and $H_2O$ (5 mL) was degassed, purged with $N_2$ for 3 times, and then stirred at 90 °C for 16 h under $N_2$ atmosphere. The reaction mixture was diluted to $H_2O$ (150 mL) and extracted with EtOAc (100 mL × 3). The combined organic layer was washed with $H_2O$ (100 mL), dried over $Na_2SO_4$, filtered and concentrated under reduced pressure to obtain a residue. The residue was purified by silica gel column chromatography (50% EtOAc in PE) to give N-[(1R)-1-[5-(5-chloro-2-formylphenyl)-2-thienyl]ethyl]-1-(2-fluorophenyl)-6-oxo-pyridazine-3-carboxamide (5.3 g, 47.00% yield) as a light yellow solid. $^1H$ NMR (400 MHz, DMSO-$d_6$) δ = 10.04 (s, 1H), 9.11 (d, *J* = 8.4 Hz, 1H), 7.99 (d, *J* = 9.6 Hz, 1H), 7.87 (d, *J* = 8.8 Hz, 1H), 7.66 (dt, *J* = 1.6, 7.6 Hz, 1H), 7.61 (dd, *J* = 2.4, 4.4 Hz, 2H), 7.59 - 7.54 (m, 1H), 7.46 - 7.38 (m, 2H), 7.22 - 7.17 (m, 2H), 7.11 (d, *J* = 3.2 Hz, 1H), 5.43 (quin, *J* = 7.2 Hz, 1H), 1.61 (d, *J* = 7.2 Hz, 3H); MS (EI) m/z: 482.1 $[M+H]^+$.

Step 3: Synthesis of N-[(1R)-1-[5-[5-chloro-2-(methylaminomethyl)phenyl]-2-thienyl]ethyl]-1-(2-fluorophenyl)-6-oxo-pyridazine-3-carboxamide

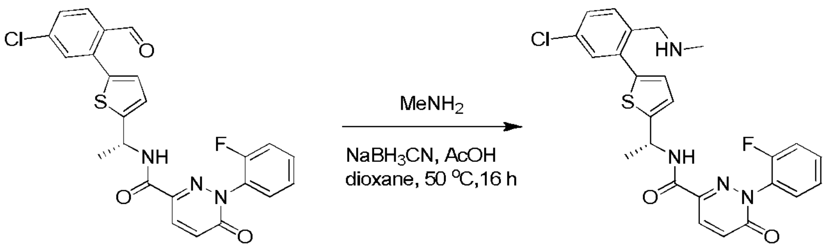


**295**

To a solution of N-[(1R)-1-[5-(5-chloro-2-formyl-phenyl)-2-thienyl]ethyl]-1-(2-fluorophenyl)-6-oxo-pyridazine-3-carboxamide (5.3 g, 11.00 mmol) and AcOH (1.32 g, 22.00 mmol) in dioxane (55 mL) was added $MeNH_2$ (46.12 g, 445.50 mmol, 30% purity), followed by stirring at 15 °C for 1 hour under $N_2$ atmosphere. The reaction mixture was added with $NaBH_3CN$ (2.07 g, 32.99 mmol) and stirred at 50 °C for 16 h under $N_2$ atmosphere. The reaction mixture was poured into distilled water (100 mL) and extracted with EtOAc (100 mL × 3). The combined organic layer was washed with brine (50 mL × 2), dried over anhydrous $Na_2SO_4$ and concentrated under reduced pressure to obtain a crude product. The product was purified by silica gel column chromatography (2% MeOH in DCM) to give the compound of Example 295 (2.05 g, 36.22% yield) as a yellow solid. $^1H$ NMR (400 MHz, DMSO-$d_6$) δ = 9.08 (d, *J* = 8.4 Hz, 1H), 7.99 (d, *J* = 9.6 Hz, 1H), 7.67 (t, *J* = 7.6 Hz, 1H), 7.61 - 7.51 (m, 2H), 7.47 - 7.33 (m, 4H), 7.22 - 7.14 (m, 2H), 7.02 (d, *J* = 3.6 Hz, 1H), 5.45 - 5.36 (m, 1H), 3.64 (s, 2H), 2.24 (s, 3H), 1.59 (d, *J* = 7.2 Hz, 3H); MS (EI) m/z: 497.1 $[M+H]^+$.

**Example 296: N-[(1R)-1-[5-[5-fluoro-2-(methylaminomethyl)phenyl]-2-thienyl]ethyl]-1-(1-methylpyrazol-4-yl)-6-oxo-pyridazine-3-carboxamide**

Step 1: methyl-1-(1-methylpyrazol-4-yl)-6-oxo-pyridazine-3-carboxylate

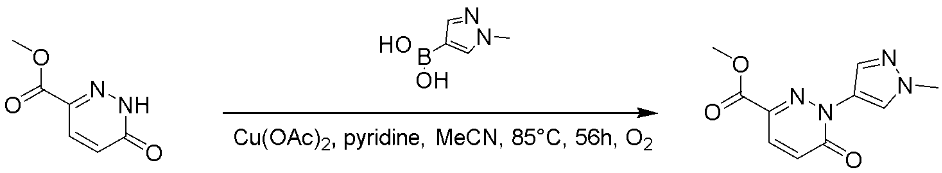


To a solution of methyl-6-oxo-1H-pyridazine-3-carboxylate (2 g, 12.98 mmol) and (1-methylpyrazol-4-yl)boronic acid (1.96 g, 15.57 mmol) in acetonitrile (100 mL), $Cu(OAc)_2$ (707.10 mg, 3.89 mmol) and pyridine (3.14 mL, 38.93 mmol) were added, followed by stirring at 85 °C for 56 h under $O_2$ atmosphere. The mixture was filtrated, the filtrate was concentrated under reduced pressure and purified by silica gel column chromatography (10% EA in DCM) to give methyl-1-(1-methylpyrazol-4-yl)-6-oxo-pyridazine-3-carboxylate (3.08 g, 39.68% yield) as a white solid. MS (ESI) m/z = 235.0 $[M+H]^+$.

Step 2: 1-(1-methylpyrazol-4-yl)-6-oxo-pyridazine-3-carboxylic acid

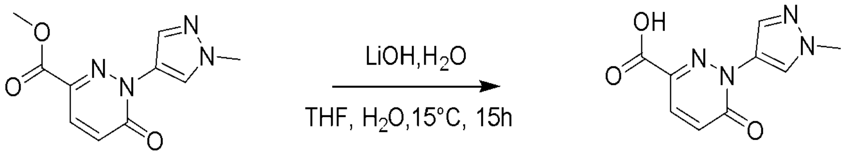


To a solution of methyl-1-(1-methylpyrazol-4-yl)-6-oxo-pyridazine-3-carboxylate (1.5 g, 6.40 mmol) in THF (20 mL) and $H_2O$ (7 mL) was added $LiOH.H_2O$ (806.27 mg, 19.21 mmol), followed by stirring at 15 °C for 15 h. The reaction mixture was adjusted to pH = 3-4 with aq. 1 N HCl and extracted with EtOAc (50 mL × 3). The organic layer was washed with water (30 mL) and brine (30 mL), dried over anhydrous $Na_2SO_4$, and filtered under reduced pressure to give 1-(1-methylpyrazol-4-yl)-6-oxo-pyridazine-3-carboxylic acid (840 mg, 55.99% yield) as a white solid. MS (ESI) m/z = 220.9 $[M+H]^+$.

Step 3: N-[(1R)-1-(5-bromo-2-thienyl)ethyl]-1-(1-methylpyrazol-4-yl)-6-oxo-pyridazine-3-carboxylic acid

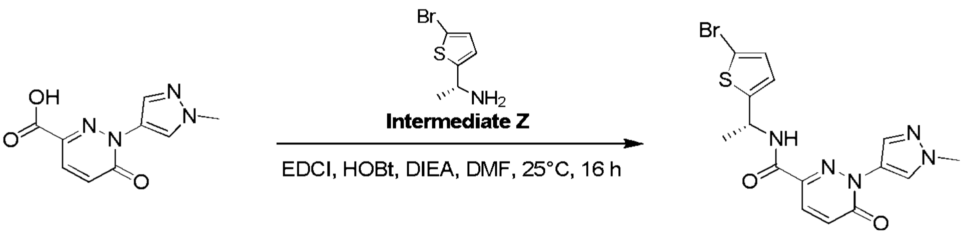


A solution of 1-(1-methylpyrazol-4-yl)-6-oxo-pyridazine-3-carboxylic acid (300 mg, 1.36 mmol), Intermediate E (330.49 mg, 1.36 mmol, HCl), EDCI (522.39 mg, 2.72 mmol), HOBt (368.21 mg, 2.72 mmol) and DIEA (711.97 μL, 4.09 mmol) in DMF (6 mL) was degassed and purged with $N_2$ for 3 times, and then stirred at 25 °C for 3 h under $N_2$ atmosphere. The reaction mixture was poured into water (30 mL) and extracted with EtOAc (30 mL×3). The combined organic layer was washed with brine (50 mL), dried over anhydrous $Na_2SO_4$ and concentrated under reduced pressure to obtain a crude product. The product was purified by silica gel column chromatography (4% MeOH in DCM) to give N-[(1R)-1-(5-bromo-2-thienyl)ethyl]-1-(1-methylpyrazol-4-yl)-6-oxo-pyridazine-3-carboxylic acid (210 mg, 30.53% yield) as yellow oil. MS (ESI) m/z = 407.8 $[M+H]^+$.

Step 4: N-[(1R)-1-[5-(5-fluoro-2-formyl-phenyl)-2-thienyl]ethyl]-1-(1-methylpyrazol-4-yl)-6-oxo-pyridazine-3-carboxamide

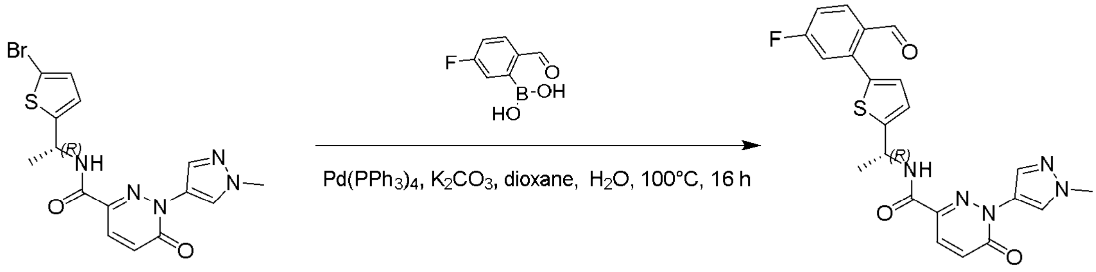


A solution of N-[(1R)-1-(5-bromo-2-thienyl)ethyl]-1-(1-methylpyrazol-4-yl)-6-oxo-pyridazine-3-carboxylic acid (60 mg, 146.96 μmol), (5-fluoro-2-formyl-phenyl)boronic acid (37.02 mg, 220.44 μmol), $K_2CO_3$ (50.78 mg, 367.40 μmol), $Pd(PPh_3)_4$ (16.98 mg, 14.70 μmol), and $H_2O$ (0.4 mL) in dioxane (4 mL) was degassed and purged with $N_2$ for 3 times, and then stirred at 100 °C for 16 h under $N_2$ atmosphere. The mixture was filtered under reduced pressure to obtain a crude product. The product was purified by silica gel column chromatography (48% EtOAc in PE) to give N-[(1R)-1-[5-(5-fluoro-2-formyl-phenyl)-2-thienyl]ethyl]-1-(1-methylpyrazol-4-yl)-6-oxo-pyridazine-3-carboxamide (75 mg, 68.95% yield, 61% purity) as yellow oil. MS (ESI) m/z = 452.0 $[M+H]^+$.

Step 5: Synthesis of N-[(1R)-1-[5-[5-fluoro-2-(methylaminomethyl)phenyl]-2-thienyl]ethyl]-1-(1-methylpyrazol-4-yl)-6-oxo-pyridazine-3-carboxamide

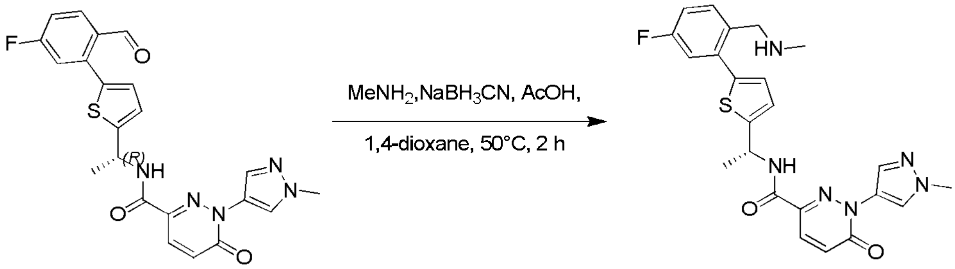


296

A solution of N-[(1R)-1-[5-(5-fluoro-2-formyl-phenyl)-2-thienyl]ethyl]-1-(1-methylpyrazol-4-yl)-6-oxo-pyridazine-3-carboxamide (75 mg, 166.12 μmol), methylamine (1.230 g, 11.88 mmol, 30% purity), AcOH (19.00 μL, 332.25 μmol) and $NaBH_3CN$ (31.32 mg, 498.37 μmol) in dioxane (3 mL) was degassed and purged with $N_2$ for 3 times, and then the mixture was stirred at 50 °C for 2 h under $N_2$ atmosphere. The reaction mixture was poured into water (30 mL) and extracted with EtOAc (30 mL×3). The organic layer was washed with brine (50 mL), dried over anhydrous $Na_2SO_4$ and filtered under reduced pressure to obtain a crude product. The product was purified by prep-HPLC (column: Welch Xtimate C18 150*30 mm*5 μm; mobile phase: [water($NH_3H_2O$+$NH_4HCO_3$)-ACN];B%: 20%-60%,36 min). Most of ACN was removed under reduced pressure, and the remaining solvent was removed by lyophilization to give the compound of Example 296 (5.3 mg, 6.81% yield) as a white solid. $^1H$ NMR (400 MHz, DMSO-$d_6$) δ ppm 9.10 (d, J = 8.8 Hz, 1H), 8.55 (s, 1H), 8.36 (s, 1H), 7.92 (d, J = 9.6 Hz, 1H), 7.52 (dd, J = 6.4, 9.6 Hz, 1H), 7.23 (d, J = 3.6 Hz, 1H), 7.19 - 7.12 (m, 3H), 7.07 (dd, J = 0.8, 3.6 Hz, 1H), 5.54 - 5.40 (m, 1H), 3.90 (s, 3H), 3.62 (s, 2H), 2.24 (s, 3H), 1.68 (d, J = 7.2 Hz, 3H); MS (ESI) m/z = 467.1 $[M+H]^+$.

**Example 297: 1-[3-(dimethylcarbamoyl)-2-fluoro-phenyl]-N-[(1R)-1-[5-[5-fluoro-2-(methylaminomethyl)phenyl]-2-thienyl]ethyl]-6-oxo-pyridazine-3-carboxamide**

Step 1: N-[(1R)-1-(5-bromo-2-thienyl)ethyl]-1-[3-(dimethylcarbamoyl)-2-fluorophenyl]-6-oxo-pyridazine-3-carboxamide

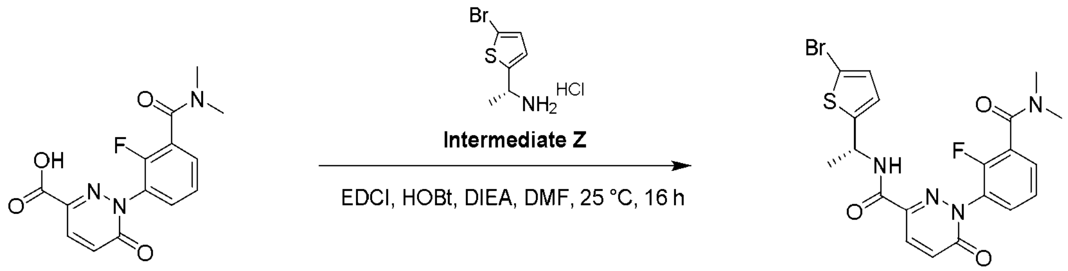


A solution of 1-[3-(dimethylcarbamoyl)-2-fluoro-phenyl]-6-oxo-pyridazine-3-carboxylic acid (30 mg, 98.28 μmol) which was obtained by ester hydrolysis of Intermediate AC, Intermediate Z (23.84 mg, 98.28 μmol), HOBt (26.56 mg, 196.55 μmol), EDCI (37.68 mg, 196.55 μmol) and DIEA (51.35 μL , 294.83 μmol) in DMF (2 mL) was degassed and purged with $N_2$ for 3 times, and stirred at 25 °C for 16 h under $N_2$ atmosphere. The reaction mixture was poured into water (30 mL) and extracted with EtOAc (30 mL×3). The organic layer was washed with brine (50 mL), dried over anhydrous $Na_2SO_4$ and concentrated under reduced pressure to obtain a crude product. The product was purified by silica gel column chromatography (4% MeOH in DCM) to give N-[(1R)-1-(5-bromo-2-thienyl)ethyl]-1-[3-(dimethylcarbamoyl)-2-fluoro-phenyl]-6-oxo-pyridazine-3-carboxamide (42 mg, 66.70% yield) as yellow oil. MS (ESI) m/z = 515.0 [M+Na] $^+$, 494.8 [M+H] $^+$.

Step 2: 1-[3-(dimethylcarbamoyl)-2-fluoro-phenyl]-N-[(1R)-1-[5-(5-fluoro-2-formylphenyl)-2-thienyl]ethyl]-6-oxo-pyridazine-3-carboxamide

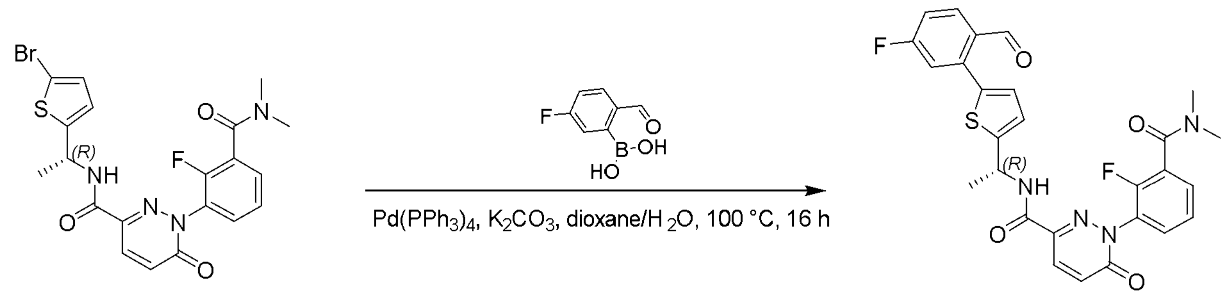


A solution of N-[(1R)-1-(5-bromo-2-thienyl)ethyl]-1-[3-(dimethylcarbamoyl)-2-fluorophenyl]-6-oxo-pyridazine-3-carboxamide (42 mg, 85.13 μmol), (5-fluoro-2-formylphenyl)boronic acid (17.16 mg, 102.16 μmol), $Pd(PPh_3)_4$ (9.84 mg, 8.51 μmol), $K_2CO_3$ (29.41 mg, 212.83 μmol) and $H_2O$ (0.3 mL) in dioxane (3 mL) was degassed and purged with $N_2$ for 3 times, and then stirred at 100 °C for 16 h under $N_2$ atmosphere. The reaction mixture was filtered under reduced pressure to obtain a crude product. The product was purified by silica gel column chromatography to give 1-[3-(dimethylcarbamoyl)-2-fluoro-phenyl]-N-[(1R)-1-[5-(5-fluoro-2-formyl-phenyl)-2-thienyl]ethyl]-6-oxo-pyridazine-3-carboxamide (50 mg, 86.18% yield) as yellow oil. MS (ESI) m/z = 537.1 [M+H] $^+$).

Step 3: 1-[3-(dimethylcarbamoyl)-2-fluoro-phenyl]-N-[(1R)-1-[5-[5-fluoro-2-(methylaminomethyl)phenyl]-2-thienyl]ethyl]-6-oxo-pyridazine-3-carboxamide

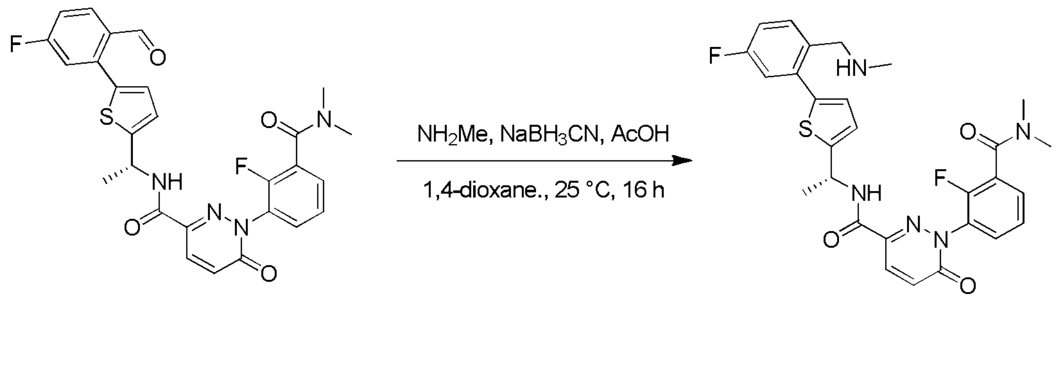


**297**

A solution of 1-[3-(dimethylcarbamoyl)-2-fluoro-phenyl]-N-[(1R)-1-[5-(5-fluoro-2-formyl-phenyl)-2-thienyl] ethyl]-6-oxo-pyridazine-3-carboxamide (50 mg, 93.19 μmol), methylamine (330 mg, 3.19 mmol, 30% purity), AcOH (10.66 μL, 186.38 μmol) and $NaBH_3CN$ (17.57 mg, 279.56 μmol) in dioxane (2 mL) was degassed and purged with $N_2$ for 3 times, and then stirred at 25 °C for 16 h under $N_2$ atmosphere. The reaction mixture was poured into water (20 mL) and extracted with EtOAc (30 mL×3). The organic layer was dried over anhydrous $Na_2SO_4$ and concentrated under reduced pressure to obtain a crude product. The product was purified by silica gel column chromatography (4% MeOH in DCM) to obtain a yellow oil residue. The residue was purified by prep-HPLC (column: Welch Xtimate C18 150*30 mm*5 μm; mobile phase: [water($NH_3H_2O$+$NH_4HCO_3$)-ACN];B%: 22%-62%,36 min). Most of ACN was removed under reduced pressure, and the remaining solvent was removed by lyophilization to give the compound of Example 297 (1.9 mg, 3.66% yield) as a white solid. $^1H$ NMR (400MHz, DMSO-$d_6$) δ ppm 9.12 (d, J = 8.8 Hz, 1H), 7.99 (d, J = 10.0 Hz, 1H), 7.76 (dt, J = 2.0, 7.6 Hz, 1H), 7.59 - 7.42 (m, 3H), 7.23 - 7.12 (m, 4H), 7.02 (d, J = 3.6 Hz, 1H), 5.41 (t, J = 7.6 Hz, 1H), 3.61 (s, 2H), 3.01 (s, 3H), 2.87 (s, 3H), 2.24 (s, 3H), 1.59 (d, J = 7.2 Hz, 3H); MS (ESI) m/z = 552.4 [M+H]$^+$.

**Example 298: 1-(2-fluorophenyl)-N-[(1R)-1-[5-[2-(methylaminomethyl)phenyl]-2-thienyl]ethyl]-6-oxo-pyridazine-3-carboxamide**

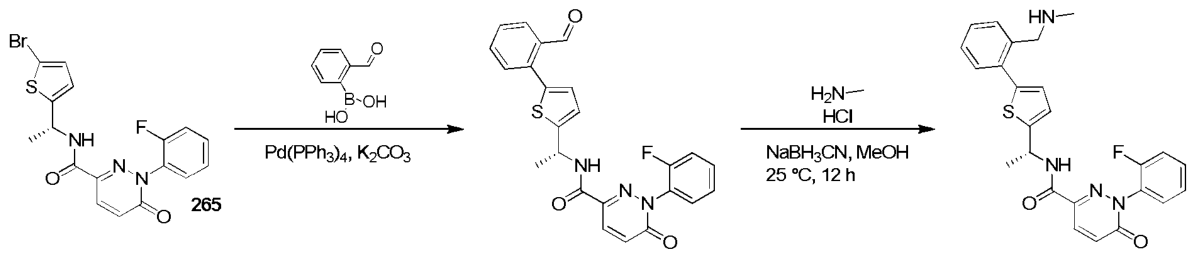


265

298

The compound of Example 298 was obtained in the same manner as in Example 266 by using the compound of Example 265 as a starting material. $^1H$ NMR (400 MHz, DMSO-$d_6$) δ = 9.08 (d, J = 8.4 Hz, 1H), 7.99 (d, J = 9.6 Hz, 1H), 7.67 (dt, J = 1.6, 7.6 Hz, 1H), 7.61 - 7.54 (m, 1H), 7.48 (t, J = 7.6 Hz, 1H), 7.46 - 7.35 (m, 2H), 7.35 - 7.24 (m, 3H), 7.20 (d, J = 9.6 Hz, 1H), 7.12 (d, J = 3.6 Hz, 1H), 7.01 - 6.97 (m, 1H), 5.47 - 5.35 (m, 1H), 3.63 (s, 2H), 2.24 (s, 3H), 1.59 (d, J = 7.0 Hz, 3H); LC/MS (ESI) m/z: 463.3 [M+H]$^+$

**Example 299: 1-(2-fluorophenyl)-N-[(1R)-1-[5-[2-(hydroxymethyl)phenyl]-2-thienyl]ethyl]-6-oxo-pyridazine-3-carboxamide**

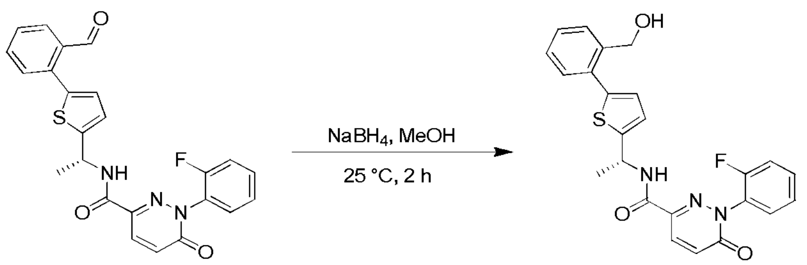


299

The compound of Example 299 was obtained by reducing the ketone group of the compound obtained in Step 2 of Example 298 to an alcohol using $NaBH_4$. $^1H$ NMR (400 MHz, DMSO-$d_6$) δ = 9.07 (d, *J* = 8.4 Hz, 1H), 7.99 (d, *J* = 10.0 Hz, 1H), 7.67 (dt, *J* = 1.6, 7.6 Hz, 1H), 7.61 - 7.53 (m, 2H), 7.47 - 7.38 (m, 2H), 7.38 - 7.28 (m, 3H), 7.19 (d, *J* = 10.0 Hz, 1H), 7.09 (d, *J* = 3.6 Hz, 1H), 7.01 (d, *J* = 3.6 Hz, 1H), 5.52 - 5.32 (m, 1H), 5.29 - 5.19 (m, 1H), 4.53 (d, *J* = 5.2 Hz, 2H), 1.64 - 1.56 (m, 3H); LC/MS (ESI) m/z: 450.3 [M+H]$^+$

**Example 300: N-[(1R)-1-[5-[5-fluoro-2-[(2-hydroxyethylamino)methyl]phenyl]-2-thienyl]ethyl]-1-(2-fluorophenyl)-6-oxo-pyridazine-3-carboxamide**

Step 1: Synthesis of N-[(1R)-1-[5-(5-fluoro-2-formyl-phenyl)-2-thienyl]ethyl]-1-(2-fluorophenyl)-6-oxo-pyridazine-3-carboxamide

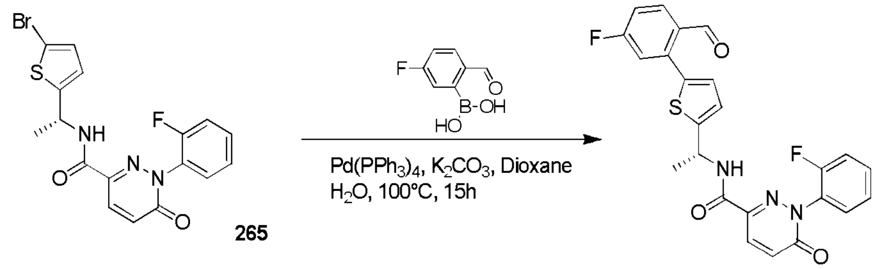


265

N-[(1R)-1-[5-(5-fluoro-2-formyl-phenyl)-2-thienyl]ethyl]-1-(2-fluorophenyl)-6-oxo-pyridazine-3-carboxamide was obtained in the same manner as in Step 1 of Example 266 by changing the boronic acid derivative.

Step 2: Synthesis of N-[(1R)-1-[5-[5-fluoro-2-[(2-hydroxyethylamino)methyl]phenyl]-2-thienyl]ethyl]-1-(2-fluorophenyl)-6-oxo-pyridazine-3 -carboxamide

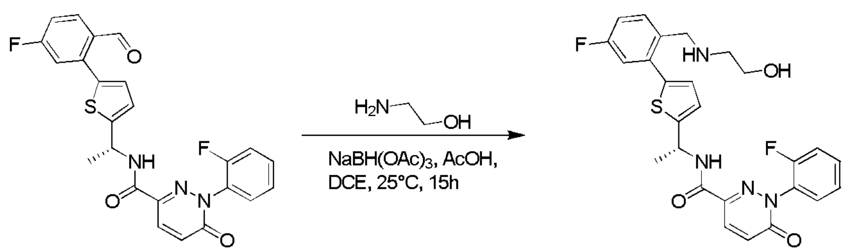


300

To a solution of N-[(1R)-1-[5-(5-fluoro-2-formyl-phenyl)-2-thienyl]ethyl]-1-(2-fluorophenyl)-6-oxo-pyridazine-3-carboxamide (56 mg, 120.31 μmol) and 2-aminoethanol (36.74 mg, 601.54 μmol) in DCE (3 mL) was added AcOH (21.67 mg, 360.92 μmol), and the reaction mixture was stirred at 25 °C for 30 min under $N_2$ atmosphere. After the addition of $NaBH(OAc)_3$ (76.49 mg, 360.92 μmol), the reaction mixture was stirred at 25 °C for 15 h under $N_2$ atmosphere. The reaction mixture was poured into water (20 mL) and extracted with EtOAc (20 mL × 3). The combined organic layer was washed with brine (20 mL × 2), dried over anhydrous $Na_2SO_4$ and concentrated under reduced pressure to obtain a crude product. The residue was purified by flash silica gel chromatography (7% MeOH in DCM) to give crude product. The crude product was purified by prep-HPLC (column: Welch Xtimate C18 150*30 mm*5 μm; mobile phase: [water ($NH_3H_2O$+$NH_4HCO_3$)-ACN]; B%: 22%-62%, 36min), and most of ACN was removed under reduced pressure. The remaining solvent was removed by lyophilization to give the compound of Example 300 (1.6 mg, 2.60% yield) as a white solid. $^1H$ NMR (400MHz, DMSO-$d_6$) δ = 9.07 (d, *J* = 8.4 Hz, 1H), 7.99 (d, *J* = 9.6 Hz, 1H), 7.66 (dt, *J* = 1.6, 7.6 Hz, 1H), 7.61 - 7.51 (m, 2H), 7.47 - 7.37 (m, 2H), 7.21 (s, 1H), 7.20 - 7.12 (m, 3H), 7.02 (d, *J* = 3.2 Hz, 1H), 5.40 (quin, *J* = 7.2 Hz, 1H), 4.48 (br t, *J* = 4.8 Hz, 1H), 3.69 (s, 2H), 3.43 (br s, 2H), 2.54 (t, *J* = 5.6 Hz, 2H), 1.59 (d, *J* = 7.2 Hz, 3H); MS (ESI) m/z = 511.4 $[M+H]^+$.

**Example 301: 1-(1-acetyl-4-piperidyl)-N-[(1R)-1-[5-[5-chloro-2-(methylaminomethyl)phenyl]-2-thienyl]ethyl]-6-oxo-pyridazine-3-carboxamide**

Step 1: Synthesis of (R)-1-(1-acetylpiperidin-4-yl)-N-(1-(5-bromothiophen-2-yl)ethyl)-6-oxo-1,6-dihydropyridazine-3-carboxamide

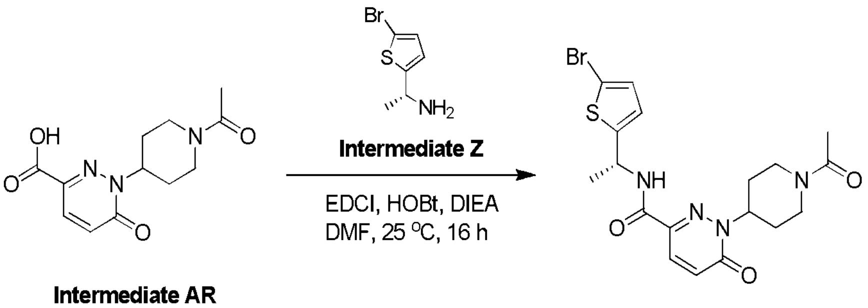


Intermediate AR

(R)-1-(1-acetylpiperidin-4-yl)-N-(1-(5-bromothiophen-2-yl)ethyl)-6-oxo-1,6-dihydropyridazine-3-carboxamide was obtained in the same manner as in Example 265 by using Intermediate AR.

Steps 2 and 3: Synthesis of 1-(1-acetyl-4-piperidyl)-N-[(1R)-1-[5-[5-chloro-2-(methylaminomethyl)phenyl]-2-thienyl]ethyl]-6-oxo-pyridazine-3-carboxamide

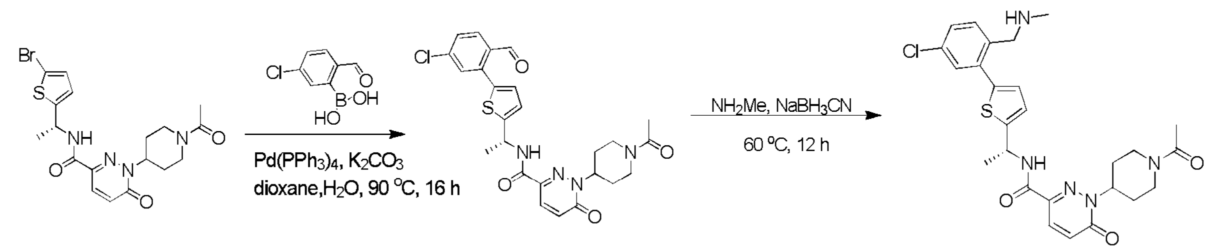


301

The compound of Example 301 was obtained in the same manner as in Example 266. $^1H$ NMR (400MHz, DMSO-$d_6$) δ = 7.52 (br d, *J* = 8.8 Hz, 1H), 6.48 - 6.36 (m, 1H), 6.10 (d, *J* = 8.4 Hz, 1H), 6.01 - 5.86 (m, 2H), 5.82 - 5.71 (m, 1H), 5.60 (br d, *J* = 9.6 Hz, 2H), 4.06 - 3.94 (m, 1H), 3.63 - 3.50 (m, 1H), 3.12 (br dd, *J* = 3.6, 12.4 Hz, 1H), 2.59 - 2.46 (m, 1H), 2.20 (s, 2H), 1.79 - 1.71 (m, 1H), 1.25 - 1.17 (m, 1H), 0.81 (s, 3H), 0.71 - 0.43 (m, 6H), 0.42 - 0.29 (m, 2H), 0.22 (br d, *J* = 6.8 Hz, 3H); MS (ESI) m/z = 528.3 $[M+H]^+$.

**Example 302: N-[(1R)-1-[5-[5-fluoro-2-(methylaminomethyl)phenyl]-2-thienyl]ethyl]-1-[2-(methylamino)-3-pyridyl]-6-oxo-pyridazine-3-carboxamide**

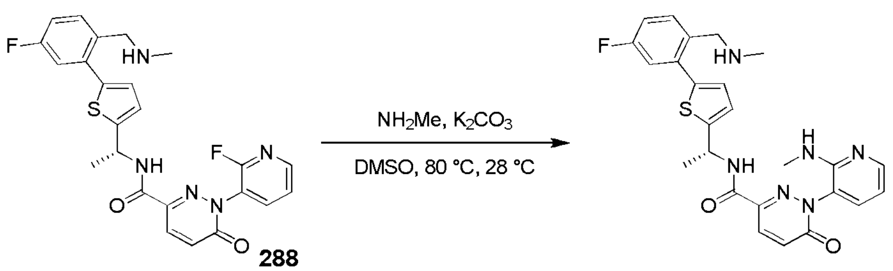


288 302

A mixture of the compound of Example 288 (25 mg, 51.92 μmoL), methanamine (7.01 mg, 103.84 μmoL, HCl) and $K_2CO_3$ (14.35 mg, 103.84 μmoL) in DMSO (2 mL) was degassed and purged with $N_2$ for 3 times, and then the mixture was stirred at 80 °C for 28 h under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by silica gel column chromatography (8% MeOH in DCM) to obtain a residue. The residue was purified by prep-HPLC (Column: Welch Xtimate C18 150*30 mm*5 μm; mobile phase: [Water ($NH_3H_2O$+$NH_4HCO_3$)-ACN];B%: 20%-60%, 36 min). Most of ACN was removed under reduced pressure, and the remaining solvent was removed by lyophilization to give the compound of Example 302 (1.8 mg, 3.63 μmoL, 7.00% yield) as a yellow solid. $^1H$ NMR (400 MHz, DMSO-$d_6$) δ = 8.97 (d, J = 8.8 Hz, 1H), 8.12 (dd, J = 1.6, 5.2 Hz, 1H), 7.94 (d, J = 4.0 Hz, 1H), 7.52 (dd, J = 6.4, 8.3 Hz, 1H), 7.46 (dd, J = 2.0, 7.6 Hz, 1H), 7.22 - 7.09 (m, 4H), 7.04 - 6.98

(m, 1H), 6.61 (dd, J = 5.2, 7.6 Hz, 1H), 6.35 (q, J = 4.4 Hz, 1H), 5.47 - 5.34 (m, 1H), 3.60 (s, 2H), 2.76 (d, J = 4.4 Hz, 3H), 2.24 (s, 3H), 1.59 (d, J = 7.2 Hz, 3H); MS (ESI) m/z = 493.4 $[M+H]^+$.

**Example 303: 1-(2-fluorophenyl)-6-oxo-N-[(1R)-1-[3-(2-oxo-1-pyridyl)phenyl]ethyl]pyridazine-3-carboxamide**

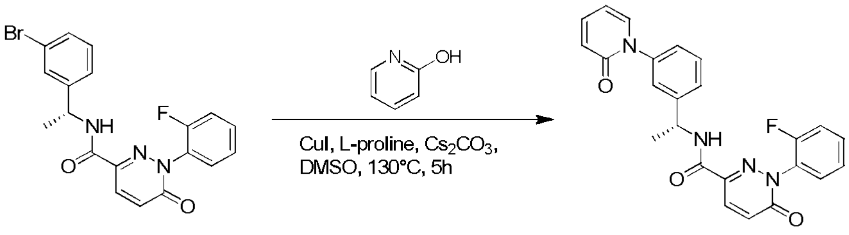


303

A mixture of N-[(1R)-1-(3-bromophenyl)ethyl]-1-(2-fluorophenyl)-6-oxo-pyridazine-3-carboxamide (50 mg, 120.12 μmol), pyridin-2-ol (17.14 mg, 180.18 μmol), CuI (4.58 mg, 24.02 μmol), $Cs_2CO_3$ (117.41 mg, 360.37 μmol) and (2S)-pyrrolidine-2-carboxylic acid (2.77 mg, 24.02 μmol) in DMSO (2 mL) was degassed and purged with $N_2$ for 3 times, and then the mixture was stirred at 130 °C for 5 h under $N_2$ atmosphere. The reaction mixture was poured into water (30 mL) and extracted with EtOAc (15 mL × 3). The combined organic layer was washed with brine (20 mL × 2), dried over anhydrous $Na_2SO_4$ and concentrated under reduced pressure to obtain a crude product. The crude product was purified by prep-TLC (DCM: MeOH = 10:1) to obtain a crude product. The product was purified by prep-HPLC (column: Phenomenex C18 75*30 mm*3 μm; mobile phase: [water($NH_3H_2O$+$NH_4HCO_3$)-ACN];B%: 20%-50%, 14 min), and most of ACN was removed under reduced pressure. The remaining solvent was removed by lyophilization to give the compound of Example 303 (2.6 mg, 4.97% yield) as a light-yellow solid. $^1H$ NMR (400MHz, DMSO-$d_6$) δ = 8.97 (d, *J* = 8.4 Hz, 1H), 7.94 (d, *J* = 10.0 Hz, 1H), 7.68 (dt, *J* = 1.6, 7.6 Hz, 1H), 7.62 - 7.56 (m, 2H), 7.51 (ddd, *J* = 2.0, 6.8, 9.2 Hz, 1H), 7.48 - 7.43 (m, 3H), 7.42 (s, 2H), 7.27 (dt, *J* = 2.0, 4.4 Hz, 1H), 7.18 (d, *J* = 10.0 Hz, 1H), 6.47 (d, *J* = 9.2 Hz, 1H), 6.31 (dt, *J* = 1.2, 6.8 Hz, 1H), 5.21 (quin, *J* = 7.2 Hz, 1H), 1.49 (d, *J* = 7.2 Hz, 3H); MS (ESI) m/z = 431.3 $[M+H]^+$.

**Example 304: 1-(2-fluorophenyl)-6-oxo-N-[(1R)-1-[3-(4-pyridyloxy)phenyl]ethyl]pyridazine-3-carboxamide**

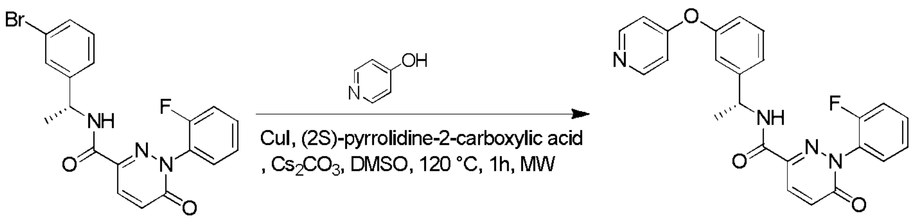


304

To a mixture of N-[(1R)-1-(3-bromophenyl)ethyl]-1-(2-fluorophenyl)-6-oxo-pyridazine-3-carboxamide (50 mg, 120.12 μmol) and pyridin-4-ol (17.14 mg, 180.18 μmol) in DMSO (2 mL), CuI (4.58 mg, 24.02 μmol), $Cs_2CO_3$ (117.41 mg, 360.37 μmol) and (2S)-pyrrolidine-2-carboxylic acid (2.77 mg, 24.02 μmol) were added, and the reaction mixture was stirred at 120 °C for 1 hour under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by prep-HPLC (column: Phenomenex C18 75*30 mm*3 μm; mobile phase: [water ($NH_3H_2O$+$NH_4HCO_3$)-ACN]; B%: 16%-46%, 11 min). Most of ACN was removed under reduced pressure, and the remaining solvent was removed by lyophilization to give the compound of Example 304 (3.3 mg, 6.38% yield) as a white solid. $^1H$ NMR (400 MHz, DMSO-$d_6$) δ ppm 1.50 (d, *J*=7.2 Hz, 3 H) 5.20 (m, 1 H) 6.23 (m, 2 H) 7.18 (d, *J*=10.0 Hz, 1 H) 7.44 (m, 5 H) 7.58 (m, 2 H) 7.67 (td, *J*=7.6, 1.75 Hz, 1 H) 7.95 (m, 3 H) 8.94 (d, *J*=8.4 Hz, 1 H); MS (ESI) m/z = 431.2 $[M+1+H]^+$,

**Example 305 and Example 306**

The compounds shown in the following table were obtained in the same manner as in Example 304, while appropriately changing the alcohol derivative in Example 304.

[Table 18]

| No. | Structure/Name | Spectral Data |
|---|---|---|
| 305 | 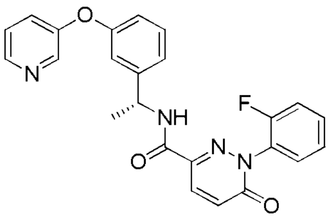 1-(2-fluorophenyl)-6-oxo-N-[(1R)-1-[3-(3-pyridyloxy)phenyl]ethyl] pyridazine-3-carboxamide | $^1$H NMR (400 MHz, DMSO-$d_6$) δ ppm 1.45 (d, *J* =7.2 Hz, 3 H) 5.12 (quin, *J* =7.6 Hz, 1 H) 6.89 (dd, *J* = 8.0, 2.0 Hz, 1 H) 7.12 (s, 1 H) 7.18 (m, 2 H) 7.40 (m, 5 H) 7.58 (m, 1 H) 7.65 (t, *J* =7.6 Hz, 1 H) 7.93 (d, *J* = 10.0 Hz, 1 H) 8.36 (s, 2 H) 8.92 (br d, *J* = 8.4 Hz, 1 H); LC/MS (ESI) m/z: 431.3 [M+H]$^+$ |
| 306 | 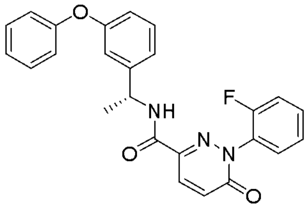 1-(2-fluorophenyl)-6-oxo-N-[(1R)-1-(3-phenoxyphenyl)ethyl]pyridazine-3-carboxamide | $^1$H NMR (400 MHz, DMSO-$d_6$) δ ppm 1.44 (d, *J* =7.2 Hz, 3 H) 5.11 (quin, *J* =7.2 Hz, 1 H) 6.82 (dd, *J* =8.00, 1.75 Hz, 1 H) 6.98 (br d, *J* = 8.0 Hz, 2 H) 7.07 (s, 1 H) 7.15 (m, 3 H) 7.37 (m, 5 H) 7.58 (m, 1 H) 7.64 (br t, *J*=7.6 Hz, 1 H) 7.93 (d, *J* =10.0 Hz, 1 H) 8.91 (br d, *J* =8.4 Hz, 1 H); LC/MS (ESI) m/z: 430.3 [M+H]$^+$ |

**Example 307: N-[(1R)-1-[3-amino-5-(trifluoromethyl)phenyl]ethyl]-5-(1-methylpyrazol-4-yl)-4-oxo-1H-pyrrolo [2,3-d]pyridazine-7-carboxamide**

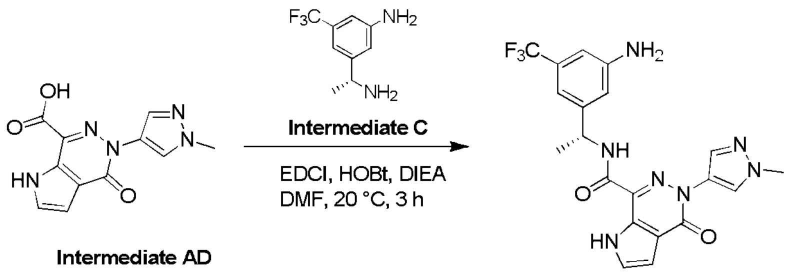


Intermediate AD

307

The compound of Example 307 (1.7 mg, 2.47% yield) was obtained as a white solid in the same manner as in Step 2 of Example 76. $^1$H NMR (400MHz, DMSO-$d_6$) δ= 12.13 (br s, 1H), 8.96 (d, *J* = 8.4 Hz, 1H), 8.47 (s, 1H), 8.30 (s, 1H), 7.39(d, *J* = 3.2 Hz, 1H), 6.89 (s, 1H), 6.85 (s, 1H), 6.77 (d, *J* = 2.8 Hz, 1H), 6.72 (s, 1H), 5.58 (s, 2H), 5.16 (t, *J* = 7.6 Hz, 1H), 3.91(s, 3H), 1.57 (d, *J* = 7.2 Hz, 3H); MS (ESI) m/z = 446.4 [M+H]$^+$.

**Example 308: N-[(1R)-1-[3-amino-5-(trifluoromethyl)phenyl]ethyl]-4-oxo-5-phenyl-1H-pyrrolo [2,3-d] pyridazine-7-carboxamide**

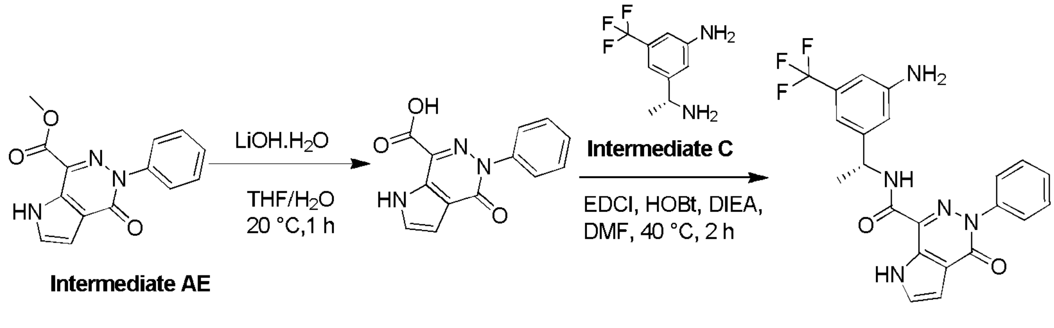


Intermediate AE

308

The compound of Example 308 (10.4 mg, 24.05% yield) was obtained as a white solid in the same manner as in Example 76. $^1H$ NMR (400MHz, DMSO-$d_6$) $\delta$ = 12.08 (br s, 1H), 8.88 (br s, 1H), 7.65 (d, *J* = 7.6 Hz, 2H), 7.52 (t, *J* = 7.6 Hz, 2H), 7.45 - 7.38 (m, 2H), 6.86 (s, 1H), 6.82 (s, 1H), 6.75 (d, *J* = 2.8 Hz, 1H), 6.70 (s, 1H), 5.54 (s, 2H), 5.10 (quin, *J* = 7.2 Hz, 1H), 1.49 (d, *J* = 7.2 Hz, 3H); MS (ESI) m/z = 442.3 $[M+H]^+$.

**Example 309: N-[(1R)-1-[3-amino-5-(trifluoromethyl)phenyl]ethyl]-5-(2-fluorophenyl)-4-oxo-thieno[2,3-d]pyridazine-7-carboxamide**

Step 1: Synthesis of 5-(2-fluorophenyl)-4-oxo-thieno[2,3-d]pyridazine-7-carboxylic acid

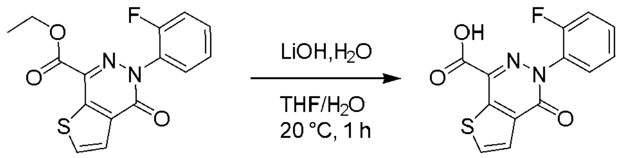


Intermediate AG-1

A mixture of Intermediate AG-1 (30 mg, 94.24 μmol) and LiOH (11.28 mg, 471.22 μmol) in THF (2 mL) and $H_2O$ (1 mL) was stirred at 15 °C for 1 hour. The reaction mixture was acidified to pH = 3-4 with 1 N aq. HCl and then extracted with EtOAc (10 mL × 3). The combined organic layer was washed with brine (20 mL), dried over anhydrous $Na_2SO_4$ and concentrated under reduced pressure to give 5-(2-fluorophenyl)-4-oxo-thieno[2,3-d]pyridazine-7-carboxylic acid (20 mg, 73.11% yield) as a white solid. MS (ESI) m/z = 291.0 $[M+H]^+$.

Step 2: Synthesis of N-[(1R)-1-[3-amino-5-(trifluoromethyl)phenyl]ethyl]-5-(2-fluorophenyl)-4-oxo-thieno[2,3-d]pyridazine-7-carboxamide

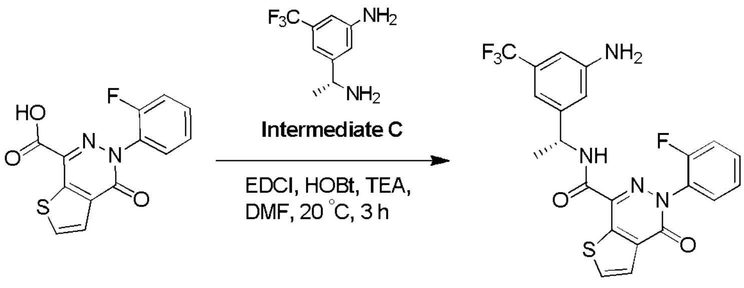


309

To a solution of 5-(2-fluorophenyl)-4-oxo-thieno[2,3-d]pyridazine-7-carboxylic acid (20 mg, 68.90 μmol) and Intermediate C (18.24 mg, 75.79 μmol) in DMF (2 mL), TEA (20.92 mg, 206.70 μmol), EDCI (19.81 mg, 103.35 μmol) and HOBt (13.96 mg, 103.35 μmol) were added, and then the reaction mixture was stirred at 50 °C for 16 h. The mixture was poured into water (10 mL) and extracted with EtOAc (10 mL × 3). The combined organic layer was washed with brine (20 mL), dried over anhydrous $Na_2SO_4$ and concentrated under reduced pressure to obtain a crude product. The crude product was purified by prep-HPLC (column: Phenomenex C18 75*30mm*3μm; mobile phase: [water($NH_3H_2O$+$NH_4HCO_3$)-ACN];B%: 39%-69%,11min), the MeCN solvent was removed under reduced pressure, and the product was lyophilized to give the compound of Example 309 (2.8 mg, 8.44% yield) as a yellow solid. $^1H$ NMR (400MHz, CHLOROFORM-*d*) $\delta$ = 7.75-7.88 (m, 2H), 7.42-7.55 (m, 3H), 7.27-7.38 (m, 2H), 7.03-7.16 (m, 2H), 5.26 (q, *J* = 7.2 Hz, 1H), 1.53-1.62 (m, 3H); MS (ESI) m/z = 477.3 $[M+H]^+$.

**Example 310 and Example 311**

The compounds shown in the following table were prepared in the same manner as in Example 309.

[Table 19]

| No. | Structure/Name | Spectral Data |
|---|---|---|
| 310 | 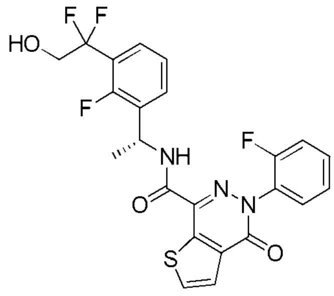 N-[(1R)-1-[3-(1,1-difluoro-2-hydroxy-ethyl)-2-fluoro-phenyl]ethyl]-5-(2-fluoro-phenyl)-4-oxo- thieno[2,3-d]pyridazine-7-carboxamide | 1H NMR (400 MHz, DMSO-$d_6$) δ = 9.22 (d, J = 8.0 Hz, 1H), 8.25 (d, J = 5.2 Hz, 1H), 7.78 - 7.69 (m, 2H), 7.68 - 7.57 (m, 2H), 7.51 - 7.41 (m, 3H), 7.28 (t, J = 7.2 Hz, 1H), 5.72 (br s, 1H), 5.47 (quin, J = 7.2 Hz, 1H), 3.92 (br t, J = 14.4 Hz, 2H), 1.50 (d, J = 7.2 Hz, 3H); LC/MS (ESI) m/z: 492.3 $[M+H]^+$ |
| 311 | 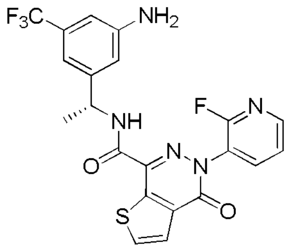 N-[(1R)-1-[3-amino-5-(trifluoromethyl)phenyl]ethyl]-5-(2-fluoro-3-pyridyl)-4-oxo-thieno[2,3-d]pyridazine-7-carboxamide | $^1$H NMR (400 MHz, DMSO-$d_6$) δ = 9.16 (d, *J* = 8.4 Hz, 1H), 8.42 (br d, *J* = 4.4 Hz, 1H), 8.36 (t, *J* = 8.8 Hz, 1H), 8.28 (d, *J* = 5.2 Hz, 1H), 7.74 (d, *J* = 5.2 Hz, 1H), 7.65 (dd, *J* = 5.2, 7.2 Hz, 1H), 6.84 (s, 1H), 6.80 (s, 1H), 6.70 (s, 1H), 5.57 (s, 2H), 5.10 (br t, *J* = 7.6 Hz, 1H), 1.47 (d, *J* = 7.2 Hz, 3H); LC/MS (ESI) m/z: 478.3 $[M+H]^+$ |

**Example 312: N-[(1R)-1-[3-amino-5-(trifluoromethyl)phenyl]ethyl]-6-(2-fluorophenyl)-7-oxo-thieno[2,3-d]pyridazine-4-carboxamide**

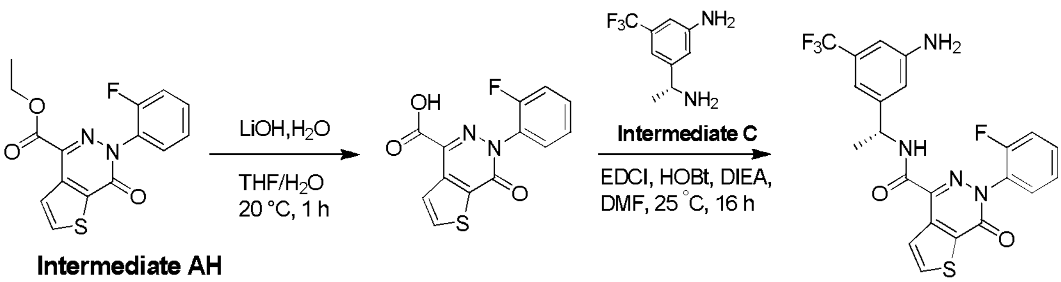


Intermediate AH

312

The compound of Example 312 (6.1 mg, 8.36% yield) was obtained as a white solid in the same manner as in Example 76. 1H NMR (400MHz, DMSO-$d_6$) δ = 8.99 (d, *J* = 8.0 Hz, 1H), 8.37 (d, *J* = 5.2 Hz, 1H), 8.03 (d, *J* = 5.2 Hz, 1H), 7.74 (t, *J* = 7.2 Hz, 1H), 7.55-7.64 (m, 1H), 7.39-7.51 (m, 2H), 6.81 (d, *J* = 11.6 Hz, 2H), 6.70 (s, 1H), 5.56 (s, 2H), 5.09 (q, *J* = 7.2 Hz, 1H), 1.45 (d, *J* = 6.8 Hz, 3H); MS (ESI) m/z = 476.9 $[M+H]^+$.

**Example 313: 4-acetamido-N-[(1R)-1-[3-amino-5-(trifluoromethyl)phenyl]ethyl]-6-oxo-1-phenyl-pyridazine-3-carboxamide**

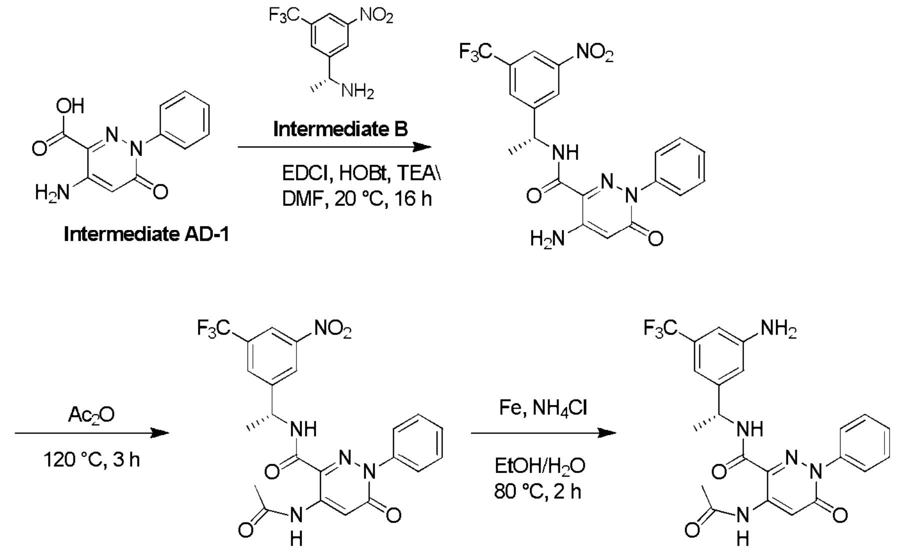


313

The compound of Example 313 (8.4 mg, 16.75% yield) was obtained as a white solid by performing the preparation method described in Example 113 in the order of Step 2, Step 1 and Step 3. $^1H$ NMR (400MHz, DMSO-$d_6$) δ = 11.41 (s, 1H), 9.15 (d, *J* = 8.4 Hz, 1H), 7.79 (s, 1H), 7.66 (d, *J* = 8.0 Hz, 2H), 7.57 - 7.49 (m, 2H), 7.48 - 7.42 (m, 1H), 6.84 (s, 1H), 6.78 (s, 1H), 6.71 (s, 1H), 5.57 (s, 2H), 5.06 (quin, *J* = 7.2 Hz, 1H), 2.17 (s, 3H), 1.46 (d, *J* = 7.2 Hz, 3H); MS (ESI) m/z = 460.3 $[M+H]^+$.

**Example 314: N-[(1R)-1-[3-(1,1-difluoro-2-hydroxy-ethyl)-2-fluoro-phenyl]ethyl]-5-(2-fluorophenyl)-4-oxo-1H-pyrrolo[2,3-d]pyridazine-7-carboxamide**

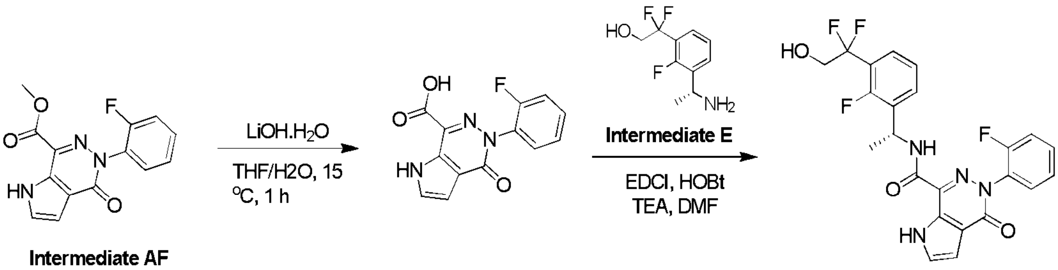


314

The compound of Example 314 (14.4 mg, 27.64% yield) was obtained as a white solid in the same manner as in Steps 2 and 3 of Example 21. $^1H$ NMR (400MHz, DMSO-$d_6$) δ = 12.13 (s, 1H), 9.07 (s, 1H), 7.62-7.71 (m, 2H), 7.52-7.60 (m, 1H), 7.35-7.47 (m, 4H), 7.23-7.31 (m, 1H), 6.76 (d, *J* = 2.8 Hz, 1H), 5.72 (t, *J* = 6.4 Hz, 1H), 5.48 (q, *J* = 7.2 Hz, 1H), 3.92 (td, *J* = 14.4, 6.0 Hz, 2H), 1.50 (d, *J* = 7.2 Hz, 3H); MS (ESI) m/z = 475.2 $[M+H]^+$.

**Example 315: N-[(1R)-1-(3-chlorophenyl)ethyl]-4-oxo-5-phenyl-thieno[2,3-d]pyridazine-7-carboxamide**

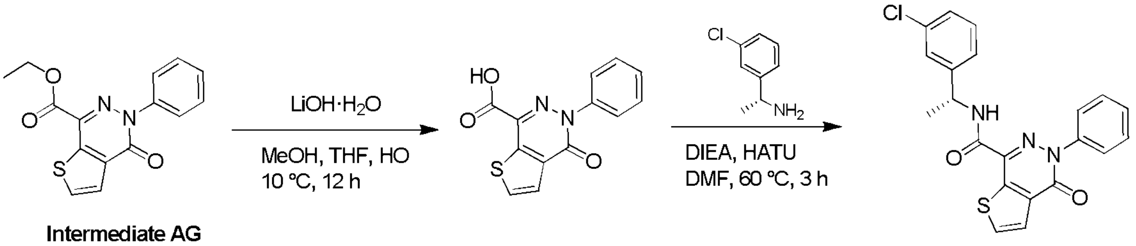


315

The compound of Example 315 (6.3 mg, 7.75% yield) was obtained as a white solid in the same manner as in

Steps 2 and 3 of Example 17. $^1$H NMR (400MHz, DMSO-$d_6$) δ = 9.07 (d, *J* = 8.4 Hz, 1H), 8.20 (d, *J* = 5.4 Hz, 1H), 7.75-7.65 (m, 3H), 7.57 (t, *J* = 7.6 Hz, 2H), 7.52-7.45 (m, 2H), 7.37 (q, *J* = 7.6 Hz, 2H), 7.32-7.24 (m, 1H), 5.26-5.14 (m, 1H), 1.51 (d, *J* = 7.2 Hz, 3H); MS (ESI) m/z = 410.1 [M+H]$^+$.

**Example 316 and Example 317**

The compounds shown in the following table were prepared in the same manner as in Example 315 by using appropriate starting materials and appropriate intermediates corresponding to the respective structures of the desired compounds based on Preparation Example 33.

[Table 20]

| No. | Structure/Name | Spectral Data |
|---|---|---|
| 316 | 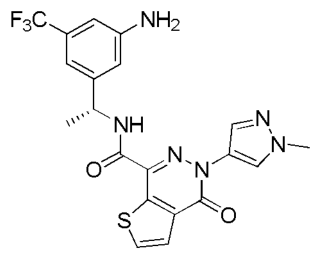 N-[(1R)-1-[3-amino-5-(trifluoromethyl)phenyl]ethyl]-5-(1-methylpyrazol-4-yl)-4-oxo-thieno[2,3-d]pyridazine-7-carboxamide | $^1$H NMR (400 MHz, DMSO-$d_6$) δ = 9.07 (d, *J* = 8.4 Hz, 1H), 8.56 (s, 1H), 8.41 (s, 1H), 8.20 (d, *J* = 5.4 Hz, 1H), 7.72 (d, *J* = 5.4 Hz, 1H), 6.89 (s, 1H), 6.85 (s, 1H), 6.72 (s, 1H), 5.59 (s, 2H), 5.20 - 5.14 (m, 1H), 3.93 (s, 3H), 1.57 (d, *J* = 7.2 Hz, 3H); LC/MS (ESI) m/z: 463.3 [M+H]$^+$ |
| 317 | 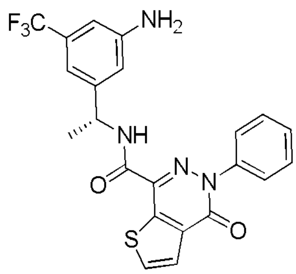 N-[(1R)-1-[3-amino-5-(trifluoromethyl)phenyl]ethyl]-4-oxo-5-phenyl-thieno [2,3-d]pyridazine-7-carboxamide | $^1$H NMR (400 MHz, DMSO-$d_6$) δ = 9.02 (d, *J* = 8.4 Hz, 1H), 8.20 (d, *J* = 5.2 Hz, 1H), 7.73 - 7.68 (m, 3H),7.56 (t, *J* = 7.6 Hz, 2H), 7.50 - 7.45 (m, 1H), 6.86 (s, 1H), 6.82 (s, 1H), 6.71 (s, 1H), 5.56 (s, 2H), 5.10 (quin, *J* = 7.2 Hz, 1H), 1.49 (d, *J* = 7.2 Hz, 3H); LC/MS (ESI) m/z: 459.3 [M+H]$^+$ |

**Example 318: N-[(1R)-1-[3-amino-5-(trifluoromethyl)phenyl]ethyl]-1-[3-(2-methyl-1,2,4-triazol-3-yl)phenyl]-6-oxo-pyridine-3-carboxamide**

Step 1: Synthesis of ethyl-6-oxo-1-(3-(4,4,5,5-tetramethyl-1,3,2-dioxaborolan-2-yl)phenyl)-1,6-dihydropyridine-3-carboxylate

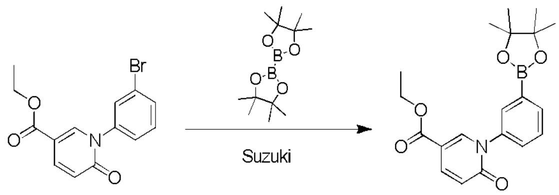


Ethyl-6-oxo-1-(3-(4,4,5,5-tetramethyl-1,3,2-dioxaborolan-2-yl)phenyl)-1,6-dihydropyridine-3-carboxylate was obtained in the same manner as in Step 1 of Example 109.

Step 2: Synthesis of ethyl-1-[3-(2-methyl-1,2,4-triazol-3-yl)phenyl]-6-oxo-pyridine-3-carboxylate

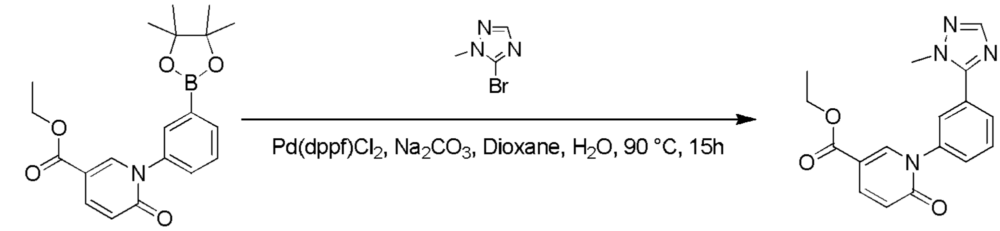


To a solution of ethyl-6-oxo-1-[3-(4,4,5,5-tetramethyl-1,3,2-dioxaborolan-2-yl)phenyl]pyridine-3-carboxylate (100 mg, 270.84 μmol) and 5-bromo-1-methyl-1,2,4-triazole (65.81 mg, 406.26 μmol) in dioxane (3 mL) and $H_2O$ (0.75 mL), Pd(dppf)$Cl_2$ (19.82 mg, 27.08 μmol) and $Na_2CO_3$ (86.12 mg, 812.53 μmol) were added. The mixture was stirred at 90 °C for 15 h. The reaction mixture was poured into water (50 mL) and extracted with EtOAc (50 mL × 3). The combined organic layer was washed with brine (30 mL × 2), dried over anhydrous $Na_2SO_4$ and concentrated under reduced pressure to obtain a crude product. The crude product was purified by flash silica gel chromatography (32% EtOAc in PE) to give ethyl-1-[3-(2-methyl-1,2,4-triazol-3-yl)phenyl]-6-oxo-pyridine-3-carboxylate (30 mg, 7.14% yield) as yellow oil. MS (ESI) m/z = 325.1 $[M+H]^+$.

<u>Steps 3 and 4: Synthesis of N-[(1R)-1-[3-amino-5-(trifluoromethyl)phenyl]ethyl]-1-[3-(2-methyl-1,2,4-triazol-3-yl)phenyl]-6-oxo-pyridine-3-carboxamide</u>

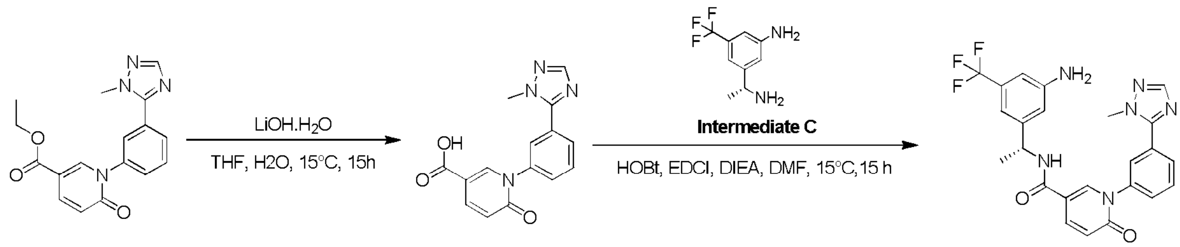


**318**

The compound of Example 318 (15.2 mg, 53.89% yield) was obtained as a white solid in the same manner as in Example 76. $^1$H NMR (400MHz, DMSO-$d_6$) δ = 8.58 (d, *J* = 7.6 Hz, 1H), 8.44 (d, *J* = 2.4 Hz, 1H), 8.04 (s, 1H), 7.98 (dd, *J* = 2.8, 9.6 Hz, 1H), 7.95 - 7.84 (m, 2H), 7.79 - 7.72 (m, 1H), 7.72 - 7.63 (m, 1H), 6.76 (s, 2H), 6.70 (s, 1H), 6.57 (d, *J* = 9.6 Hz, 1H), 5.54 (s, 2H), 5.02 (quin, *J* = 7.2 Hz, 1H), 4.00 (s, 3H), 1.40 (d, *J* = 7.2 Hz, 3H); MS (ESI) m/z = 483.4 $[M+H]^+$.

**Example 319 and Example 320**

The compounds shown in the following table were prepared in the same manner as in Example 318.

[Table 21]

| No. | Structure/Name | Spectral Data |
|---|---|---|
| 319 | 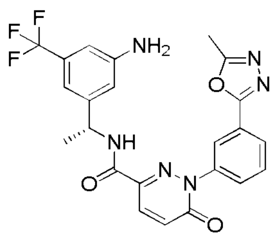 N-[(1R)-1-[3-amino-5-(trifluoromethyl)phenyl]ethyl]-1-[3-(5-methyl-1,3,4-oxadiazol-2-yl)phenyl]-6-oxo-pyridazine-3-carboxamide | $^1$H NMR (400 MHz, METHANOL-$d_4$) δ = 8.37 (s, 1H), 8.16 (d, *J* = 8.4 Hz, 1H), 8.05 (d, *J* = 10.0 Hz, 1H), 7.94 (br d, *J* = 8.0 Hz, 1H), 7.80 - 7.73 (m, 1H), 7.21 (d, *J* = 10.0 Hz, 1H), 6.92 (br d, *J* = 6.0 Hz, 2H), 6.82 (s, 1H), 5.16 (q, *J* = 7.2 Hz, 1H), 2.66 (s, 3H), 1.55 (d, *J* = 7.2 Hz, 3H); LC/MS (ESI) m/z: 485.4 $[M+H]^+$ |

(continued)

| No. | Structure/Name | Spectral Data |
|---|---|---|
| 320 | 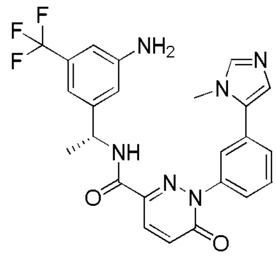 N-[(1R)-1-[3-amino-5-(trifluoromethyl)phenyl]ethyl]-1-[3-(3-methylimidazol-4-yl)phenyl]-6-oxo-pyridazine-3-carboxamide | $^1H$ NMR (400 MHz, DMSO-$d_6$) $\delta$ = 8.89 (d, *J* = 8.4 Hz, 1H), 7.91 (d, *J* = 10.0 Hz, 1H), 7.81 (s, 1H), 7.74 (s, 1H), 7.70 - 7.65 (m, 1H), 7.64 - 7.57 (m, 2H), 7.19 - 7.11 (m, 2H), 6.80 (br d, *J* = 13.6 Hz, 2H), 6.70 (s, 1H), 5.54 (s, 2H), 5.04 (quin, *J* = 7.2 Hz, 1H), 3.73 (s, 3H), 1.44 (d, *J* = 7.2 Hz, 3H); LC/MS (ESI) m/z: 483.4 $[M+H]^+$ |

**Example 321: N-[(1R)-1-[3-amino-5-(trifluoromethyl)phenyl]ethyl]-1-[3-(2-methylpyrazol-3-yl)phenyl]-6-oxo-pyridine-3-carboxamide**

Step 1: Synthesis of ethyl-1-[3-(2-methylpyrazol-3-yl)phenyl]-6-oxo-pyridine-3-carboxylate

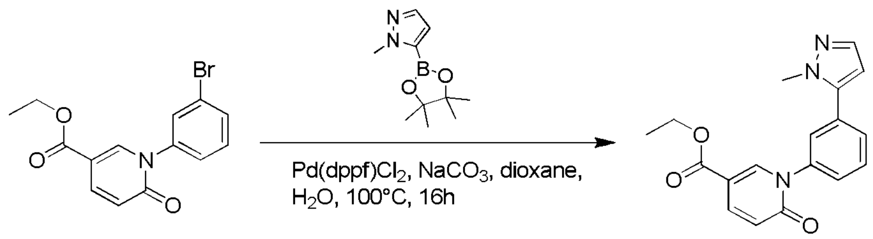


**Intermediate K-1**

A mixture of Intermediate K-1 (800 mg, 2.48 mmol), 1-methyl-5-(4,4,5,5-tetramethyl-1,3,2-dioxaborolan-2-yl) pyrazole (775.03 mg, 3.72 mmol), Pd(dppf)$Cl_2$ (181.70 mg, 248.33 μmol, 0.1*eq*) and $Na_2CO_3$ (789.60 mg, 7.45 mmol) in dioxane (15 mL) and $H_2O$ (1.5 mL) was degassed and purged with $N_2$ for 3 times, and then the mixture was stirred at 100 °C for 16 h under $N_2$ atmosphere. The reaction mixture was poured into water (60 mL) and extracted with EtOAc (40 mL × 3). The combined organic layer was washed with brine (30 mL × 2), dried over anhydrous $Na_2SO_4$ and concentrated under reduced pressure to obtain a crude product. The product was purified by flash silica gel chromatography (35% EtOAc in PE) to give ethyl-1-[3-(2-methylpyrazol-3-yl)phenyl]-6-oxo-pyridine-3-carboxylate (710 mg, 71.84% yield) as a white solid. MS (ESI) m/z = 324.0 $[M+H]^+$.

Steps 2 and 3: Synthesis of N-[(1R)-1-[3-amino-5-(trifluoromethyl)phenyl]ethyl]-1-[3-(2-methylpyrazol-3-yl)phenyl]-6-oxo-pyridine-3-carboxamide

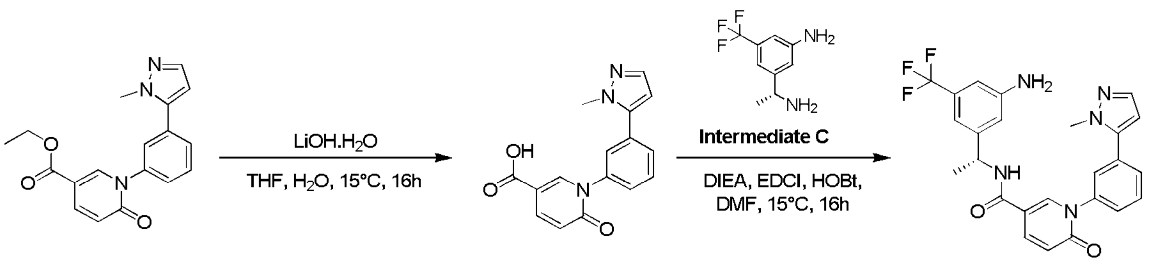


**321**

The compound of Example 321 (15.2 mg, 31.03% yield) was obtained as a white solid in the same manner as in Example 76. $^1H$ NMR (400MHz, DMSO-$d_6$) $\delta$ = 8.57 (d, *J* = 7.6 Hz, 1H), 8.43 (d, *J* = 2.4 Hz, 1H), 7.97 (dd, *J* = 2.8, 9.6 Hz, 1H), 7.71 - 7.65 (m, 3H), 7.59 - 7.53 (m, 1H), 7.50 (d, *J* = 2.0 Hz, 1H), 6.76 (s, 2H), 6.69 (s, 1H), 6.56 (d, *J* = 9.6 Hz, 1H), 6.50

(d, *J* = 2.0 Hz, 1H), 5.55 (s, 2H), 5.01 (quin, *J* = 7.2 Hz, 1H), 3.90 (s, 3H), 1.39 (d, *J* = 7.2 Hz, 3H); MS (ESI) m/z = 482.4 $[M+H]^+$.

**Example 322: N-[(1R)-1-[3-amino-5-(trifluoromethyl)phenyl]ethyl]-1-(3-isoxazol-4-ylphenyl)-6-oxo-pyridazine-3-carboxamide**

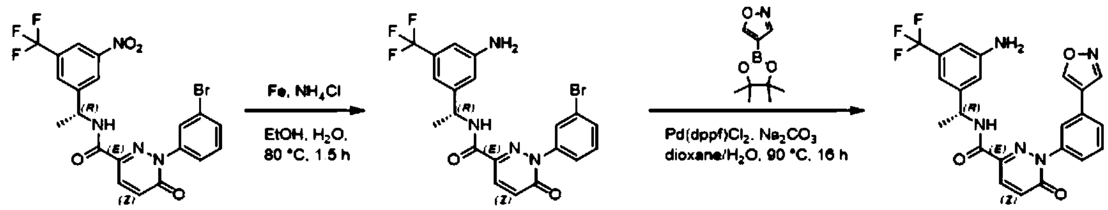


The compound of Example 322 (2.7 mg, 5.33% yield) was obtained as a white solid by subjecting (R)-1-(3-bromophenyl)-N-(1-(3-nitro-5-(trifluoromethyl)phenyl)ethyl)-6-oxo-1,6-dihydropyridazine-3-carboxamide as a starting material to the same reaction as in Step 2 of Example 16 and Step 1 of Example 321. $^1$H NMR (400MHz, DMSO-$d_6$) δ = 9.52 (s, 1H), 9.22 (s, 1H), 8.90 (d, *J* = 8.4 Hz, 1H), 7.99 (s, 1H), 7.93 (d, *J* = 9.6 Hz, 1H), 7.84 - 7.80 (m, 1H), 7.64 - 7.59 (m, 2H), 7.18 (d, *J* = 9.6 Hz, 1H), 6.82 (s, 1H), 6.78 (s, 1H), 6.69 (s, 1H), 5.55 (s, 2H), 5.04 (br t, *J* = 7.6 Hz, 1H), 1.44 (d, *J* = 7.2 Hz, 3H); MS (ESI) m/z = 407.3 $[M+H]^+$.

**Example 323 to Example 346**

The compounds of Examples 323 to 341 were prepared in the same manner as in Example 321 by using appropriate starting materials and intermediates corresponding to the respective structures of the desired compounds.

[Table 22]

| No. | Structure/Name | Spectral Data |
|---|---|---|
| 323 | 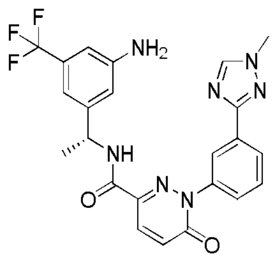 N-[(1R)-1-[3-amino-5-(trifluoromethyl)phenyl]ethyl]-1-[3-(1-methyl-1,2,4-triazol-3-yl)phenyl]-6-oxo-pyridazine-3-carboxamide | $^1$H NMR (400 MHz, DMSO-$d_6$) δ = 8.90 (d, J=8.4 Hz, 1H), 8.56 (s, 1H), 8.23 (t, J=1.6 Hz, 1H), 8.07 (td, J=1.2, 7.6 Hz, 1H), 7.91 (d, J=9.6 Hz, 1H), 7.71 - 7.67 (m, 1H), 7.66 - 7.61 (m, 1H), 7.16 (d, J=9.8 Hz, 1H), 6.82 (s, 1H), 6.78 (s, 1H), 6.69 (s, 1H), 5.54 (s, 2H), 5.04 (quin, J=7.2 Hz, 1H), 3.93 (s, 3H), 1.44 (d, J=7.2 Hz, 3H) ; LC/MS (ESI) m/z: 484.4 $[M+H]^+$ |
| 324 | 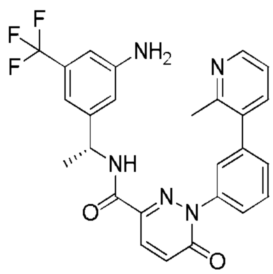 N-[(1R)-1-[3-amino-5-(trifluoromethyl)phenyl]ethyl]-1-[3-(2-methyl-3-pyridyl)phenyl]-6-oxo-pyridazine-3-carboxamide | $^1$H NMR (400 MHz, DMSO-$d_6$) δ = 8.86 (d, *J* = 8.4 Hz, 1H), 8.49 (dd, *J* = 1.6, 4.8 Hz, 1H), 7.89 (d, *J* = 9.6 Hz, 1H), 7.81 - 7.70 (m, 2H), 7.70 - 7.59 (m, 2H), 7.51 (d, *J* = 7.6 Hz, 1H), 7.33 (dd, *J* = 4.8, 7.6 Hz, 1H), 7.16 (d, *J* = 9.6 Hz, 1H), 6.82 (s, 1H), 6.78 (s, 1H), 6.70 (s, 1H), 5.54 (br s, 2H), 5.03 (quin, *J* = 7.2 Hz, 1H), 2.49 - 2.48 (m, 3H), 1.45 (d, *J* = 7.2 Hz, 3H) ; LC/MS (ESI) m/z: 494.4 $[M+H]^+$ |

(continued)

| No. | Structure/Name | Spectral Data |
|---|---|---|
| 325 | 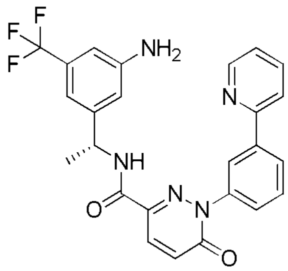 N-[(1R)-1-[3-amino-5-(trifluoromethyl)phenyl]ethyl]-6-oxo-1-[3-(2-pyridyl)phenyl]pyridazine-3-carboxamide | $^1$H NMR (400 MHz, DMSO-$d_6$) δ = 8.90 (d, *J* = 8.4 Hz, 1H), 8.69 (d, *J* = 4.0 Hz, 1H), 8.33 (t, *J* = 1.6 Hz, 1H), 8.19 (td, *J* = 1.2, 7.6 Hz, 1H), 8.03 (d, *J* = 8.0 Hz, 1H), 7.95 - 7.89 (m, 2H), 7.75 - 7.70 (m, 1H), 7.69 - 7.64 (m, 1H), 7.44 - 7.37 (m, 1H), 7.17 (d, *J* = 9.6 Hz, 1H), 6.82 (s, 1H), 6.78 (s, 1H), 6.69 (s, 1H), 5.54 (s, 2H), 5.04 (quin, *J* = 7.2 Hz, 1H), 1.44 (d, *J* = 6.8 Hz, 3H) ; LC/MS (ESI) m/z: 480.4 [M+H]$^+$ |
| 326 | 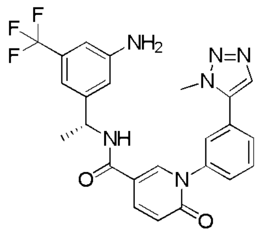 N-[(1R)-1-[3-amino-5-(trifluoromethyl)phenyl]ethyl]-1-[3-(3-methyltriazol-4-yl)phenyl]-6-oxo-pyridine-3-carboxamide | $^1$H NMR (400 MHz, DMSO-$d_6$) δ = 8.59 (br d, *J* = 7.6 Hz, 1H), 8.43 (d, *J* = 2.0 Hz, 1H), 8.04 - 7.93 (m, 2H), 7.81 (s, 1H), 7.77 - 7.70 (m, 2H), 7.63 (br d, *J* = 7.2 Hz, 1H), 6.76 (s, 2H), 6.69 (s, 1H), 6.57 (d, *J* = 9.6 Hz, 1H), 5.56 (s, 2H), 5.01 (br t, *J* = 7.2 Hz, 1H), 4.12 (s, 3H), 1.39 (br d, *J* = 7.2 Hz, 3H) ; LC/MS (ESI) m/z: 483.4 [M+H]$^+$ |
| 327 | 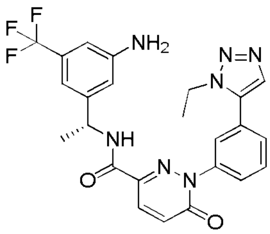 N-[(1R)-1-[3-amino-5-(trifluoromethyl)phenyl]ethyl]-1-[3-(3-ethyltriazol-4-yl)phenyl]-6-oxo-pyridazine-3-carboxamide | $^1$H NMR (400 MHz, DMSO-$d_6$) δ = 8.90 (d, *J* = 8.4 Hz, 1H), 7.94 (s, 1H), 7.93 - 7.88 (m, 2H), 7.85 (d, *J* = 8.0 Hz, 1H), 7.74 - 7.68 (m, 1H), 7.67 - 7.62 (m, 1H), 7.18 (d, *J* = 9.6 Hz, 1H), 6.80 (d, *J* = 11.6 Hz, 2H), 6.70 (s, 1H), 5.55 (s, 2H), 5.04 (quin, *J* = 7.2 Hz, 1H), 4.46 (q, *J* = 7.2 Hz, 2H), 1.44 (d, *J* = 6.8 Hz, 3H), 1.37 (t, *J* = 7.2 Hz, 3H); LC/MS (ESI) m/z: 498.4 [M+H]$^+$ |
| 328 | 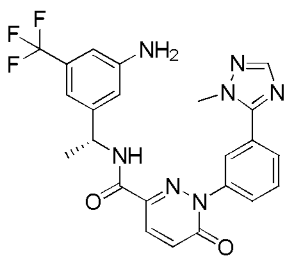 N-[(1R)-1-[3-amino-5-(trifluoromethyl)phenyl]ethyl]-1-[3-(2-methyl-1,2,4-triazol-3-yl)phenyl]-6-oxo-pyridazine-3-carboxamide | $^1$H NMR (400 MHz, DMSO-$d_6$) δ = 8.91 (br d, *J* = 8.4 Hz, 1H), 8.10 (s, 1H), 8.04 (s, 1H), 7.94 - 7.85 (m, 3H),7.77 -7.71 (m, 1H), 7.18 (d, *J* = 9.6 Hz, 1H), 6.81 (s, 1H), 6.78 (s, 1H), 6.70 (s, 1H), 5.55 (s, 2H), 5.04 (quin, *J* = 7.2 Hz, 1H), 4.01 (s, 3H), 1.44 (d, *J* = 7.2 Hz, 3H) ; LC/MS (ESI) m/z: 484.4 [M+H]$^+$ |
| 329 | 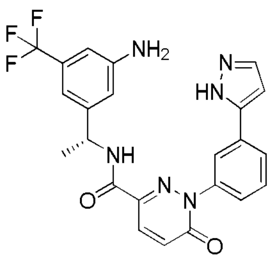 N-[(1R)-1-[3-amino-5-(trifluoromethyl)phenyl]ethyl]-6-oxo-1-[3-(1H-pyrazol-5-yl)phenyl]pyridazine-3-carboxamide | $^1$H NMR (400 MHz, DMSO-$d_6$) δ = 12.99 (br s, 1H), 8.89 (br d, *J* = 8.4 Hz, 1H), 8.03 (s, 1H), 7.95 - 7.88 (m, 2H), 7.77 (br s, 1H), 7.61 - 7.52 (m, 2H), 7.16 (d, *J* = 9.6 Hz, 1H), 6.82 (s, 1H), 6.78 (br s, 2H), 6.69 (s, 1H), 5.54 (s, 2H), 5.07 - 4.99 (m, 1H), 1.44 (br d, *J* = 7.2 Hz, 3H) ; LC/MS (ESI) m/z: 469.3 [M+H]$^+$ |

(continued)

| No. | Structure/Name | Spectral Data |
|---|---|---|
| 330 | 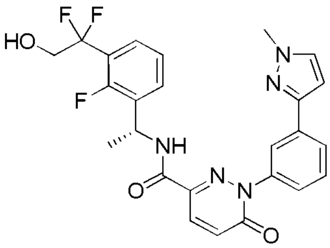 N-[(1R)-1-[3-(1,1-difluoro-2-hydroxy-ethyl)-2-fluoro-phenyl] ethyl]-1-[3-(1-methylpyrazol-3-yl)phenyl]-6-oxo-pyridazine-3-carboxamide | $^1$H NMR (400 MHz, DMSO-d$_6$) δ = 9.01 (d, *J* = 8.0 Hz, 1H), 8.02 (s, 1H), 7.91 - 7.85 (m, 2H), 7.77 (d, *J* = 2.0Hz, 1H), 7.64 (br t, *J* = 7.2 Hz, 1H), 7.60 - 7.54 (m, 2H), 7.45 - 7.40 (m, 1H), 7.26 (t, *J* = 7.6 Hz, 1H), 7.15 (d, *J* = 9.6 Hz, 1H), 6.76 (d, *J* = 2.4 Hz, 1H), 5.72 (br s, 1H), 5.41 (quin, *J* = 7.2 Hz, 1H), 3.95 - 3.91 (m, 1H), 3.90 (s, 3H), 3.88 (br s, 1H), 1.47 (d, *J* = 7.2 Hz, 3H) ; LC/MS (ESI) m/z: 498.3 [M+H]$^+$ |
| 331 | 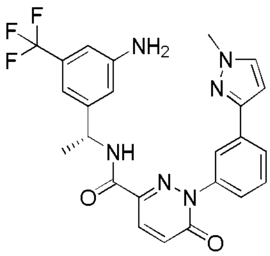 N-[(1R)-1-[3-amino-5-(trifluoromethyl)phenyl]ethyl]-1-[3-(1-methyl-pyrazol-3-yl)phenyl]-6-oxo-pyridazine-3-carboxamide | $^1$H NMR (400 MHz, DMSO-d$_6$) δ = 8.89 (d, *J* = 8.4 Hz, 1H), 8.01 (s, 1H), 7.93 - 7.84 (m, 2H), 7.76 (d, *J* = 2.0 Hz, 1H), 7.58 - 7.51 (m, 2H), 7.16 (d, *J* = 9.6 Hz, 1H), 6.82 (s, 1H), 6.78 (s, 1H), 6.75 (d, *J* = 2.0 Hz, 1H), 6.69 (s, 1H), 5.54 (s, 2H), 5.07 - 4.99 (m, 1H), 3.89 (s, 3H), 1.44 (d, *J* = 7.2 Hz, 3H) ; LC/MS (ESI) m/z: 483.4 [M+H]$^+$ |
| 332 | 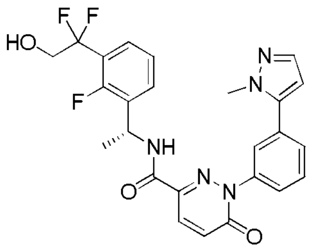 N-[(1R)-1-[3-(1,1-difluoro-2-hydroxy-ethyl)-2-fluoro-phenyl] ethyl]-1-[3-(2-methylpyrazol-3-yl)phenyl]-6-oxo-pyridazine-3-carboxamide | $^1$H NMR (400 MHz, DMSO-d$_6$) δ = 9.02 (d, *J* = 8.0 Hz, 1H), 7.89 (d, *J* = 9.6 Hz, 2H), 7.78 (td, *J* = 1.6, 7.6 Hz, 1H), 7.70 - 7.62 (m, 3H), 7.51 (d, *J* = 2.0 Hz, 1H), 7.43 (br t, *J* = 7.2 Hz, 1H), 7.27 (t, *J* = 7.6 Hz, 1H), 7.17 (d, *J* = 9.6 Hz, 1H), 6.50 (d, *J* = 2.0 Hz, 1H), 5.91 - 5.61 (m, 1H), 5.41 (quin, *J* = 7.2 Hz, 1H), 3.95 (s, 1H), 3.92 (s, 3H), 3.88 (s, 1H), 1.48 (d, *J* = 7.2 Hz, 3H); LC/MS (ESI) m/z: 498.3 [M+H]$^+$ |
| 333 | 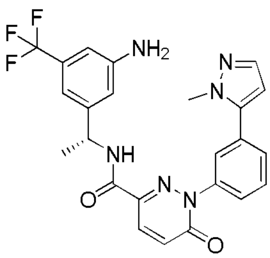 N-[(1R)-1-[3-amino-5-(trifluoromethyl)phenyl]ethyl]-1-[3-(2-methyl-pyrazol-3-yl)phenyl]-6-oxo-pyridazine-3-carboxamide | $^1$H NMR (400 MHz, DMSO-d$_6$) δ = 8.90 (d, *J* = 8.4 Hz, 1H), 7.91 (d, *J* = 9.6 Hz, 1H), 7.86 (s, 1H), 7.76 (td, *J* = 2.0, 7.2 Hz, 1H), 7.68 - 7.62 (m, 2H), 7.50 (d, *J* = 2.0 Hz, 1H), 7.17 (d, *J* = 9.6 Hz, 1H), 6.82 (s, 1H), 6.78 (s, 1H), 6.70 (s, 1H), 6.49 (d, *J* = 2.0 Hz, 1H), 5.55 (s, 2H), 5.04 (quin, *J* = 7.2 Hz, 1H), 3.90 (s, 3H), 1.44 (d, *J* = 7.2 Hz, 3H) ; LC/MS (ESI) m/z: 483.4 [M+H]$^+$ |

(continued)

| No. | Structure/Name | Spectral Data |
|---|---|---|
| 334 | 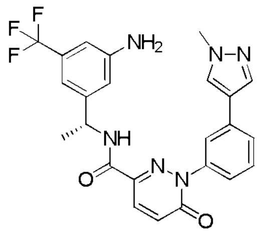 N-[(1R)-1-[3-amino-5-(trifluoromethyl)phenyl]ethyl]-1-[3-(1-methyl-pyrazol-4-yl)phenyl]-6-oxo-pyridazine-3-carboxamide | $^1$H NMR (400 MHz, DMSO-$d_6$) δ = 8.89 (d, *J* = 8.4 Hz, 1H), 8.20 (s, 1H), 7.97 - 7.85 (m, 2H), 7.80 (t, *J* = 1.6 Hz, 1H), 7.66 (d, *J* = 7.6 Hz, 1H), 7.51 (t, *J* = 8.0 Hz, 1H), 7.46 - 7.40 (m, 1H), 7.16 (d, *J* = 9.6 Hz, 1H), 6.82 (s, 1H), 6.78 (s,1H), 6.69 (s, 1H), 5.55 (s, 2H), 5.04 (quin, *J* = 7.2 Hz, 1H), 3.87 (s, 3H), 1.44 (d, *J* = 7.2 Hz, 3H) ; LC/MS (ESI) m/z: 483.4 $[M+H]^+$ |
| 335 | 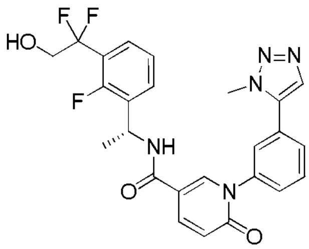 N-[(1R)-1-[3-(1,1-difluoro-2-hydroxy-ethyl)-2-fluoro-phenyl] ethyl]-1-[3-(3-methyltriazol-4-yl)phenyl]-6-oxo-pyridine-3-carboxamide | $^1$H NMR (400 MHz, DMSO-$d_6$) δ = 8.68 (d, *J* = 7.6 Hz, 1H), 8.47 (d, *J* = 2.4 Hz, 1H), 8.00 (s, 1H), 7.97 (dd, *J* = 2.4, 9.6 Hz, 1H), 7.82 (s, 1H), 7.74 (q, *J* = 7.6 Hz, 2H), 7.66 - 7.62 (m, 1H), 7.57 (br t, *J* = 7.2 Hz, 1H), 7.42 (br t, *J* = 7.2 Hz, 1H), 7.27 (t, *J* = 7.6 Hz, 1H), 6.57 (d, *J* = 9.6 Hz, 1H), 5.71 (br s, 1H), 5.36 (t, *J* = 7.2 Hz, 1H), 4.13 (s, 3H), 3.91 (br t, *J* = 14.4 Hz, 2H), 1.43 (d, *J* = 7.2 Hz, 3H) ; LC/MS (ESI) m/z: 498.4 $[M+H]^+$ |
| 336 | 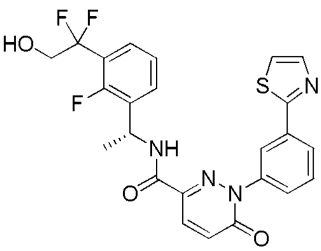 N-[(1R)-1-[3-(1,1-difluoro-2-hydroxy-ethyl)-2-fluoro-phenyl]ethyl]-6-oxo-1-(3-thiazol-2-ylphenyl)pyridazine-3-carboxamide | $^1$H NMR (400 MHz, DMSO-$d_6$) δ = 9.05 (d, *J* = 8.0 Hz, 1H), 8.25 (t, *J* = 2.0Hz, 1H), 8.06 (d, *J* = 7.6 Hz, 1H),7.99 (d, *J* = 3.2 Hz, 1H), 7.93 - 7.85 (m, 2H), 7.84 - 7.77 (m, 1H), 7.72 - 7.62 (m, 2H), 7.46 - 7.40 (m, 1H), 7.30 - 7.24 (m, 1H), 7.17 (d, *J* = 9.6 Hz, 1H), 5.72 (t, *J* = 6.4 Hz, 1H), 5.42 (quin, *J* = 7.2 Hz, 1H), 3.91 (dt, *J* = 6.4, 14.4 Hz, 2H), 1.47 (d, *J* = 7.2 Hz, 3H) ; LC/MS (ESI) m/z: 501.3 $[M+H]^+$ |
| 337 | 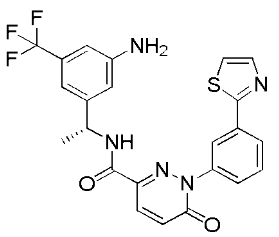 N-[(1R)-1-[3-amino-5-(trifluoromethyl)phenyl]ethyl]-6-oxo-1-(3-thiazol-2-ylphenyl)pyridazine-3-carboxamide | $^1$H NMR (400 MHz, DMSO-$d_6$) δ = 8.92 (d, *J* = 8.4 Hz, 1H), 8.24 (t, *J* = 1.6 Hz, 1H), 8.05 (d, *J* = 8.0 Hz, 1H),7.97 (d, *J* = 3.2 Hz, 1H), 7.92 (d, *J* = 9.6 Hz, 1H), 7.86 (d, *J* = 3.2 Hz, 1H), 7.81 - 7.76 (m, 1H), 7.71 - 7.66 (m, 1H), 7.17 (d, *J* =9.6 Hz, 1H), 6.82 (s, 1H), 6.79 (s, 1H), 6.70 (s, 1H), 5.54 (s, 2H), 5.04 (quin, *J* = 7.2 Hz, 1H), 1.44 (d, *J* = 7.2 Hz, 3H) ; LC/MS (ESI) m/z: 486.3 $[M+H]^+$ |

(continued)

| No. | Structure/Name | Spectral Data |
|---|---|---|
| 338 | 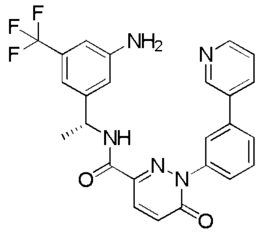 N-[(1R)-1-[3-amino-5-(trifluoromethyl)phenyl]ethyl]-6-oxo-1-[3-(3-pyridyl)phenyl]pyridazine-3-carboxamide | $^1$H NMR (400 MHz, DMSO-$d_6$) δ = 8.95 (d, *J* = 2.0 Hz, 1H), 8.91 (d, *J* = 8.0 Hz, 1H), 8.61 (d, *J* = 4.8 Hz, 1H), 8.13 (br d, *J* = 8.8 Hz, 1H), 8.02 (s, 1H), 7.92 (d, *J* = 9.6 Hz, 1H), 7.85 (d, *J* = 7.2 Hz, 1H), 7.75 - 7.71 (m, 1H), 7.68 (d, *J* = 7.6 Hz, 1H), 7.52 (dd, *J* = 4.8, 7.6 Hz, 1H), 7.18 (d, *J* = 9.6 Hz, 1H), 6.82 (s, 1H), 6.79 (s, 1H), 6.69 (s, 1H), 5.53 (s, 2H), 5.07 - 5.01 (m, 1H), 1.44 (d, *J* = 7.2 Hz, 3H); LC/MS (ESI) m/z: 480.4 $[M+H]^+$ |
| 339 | 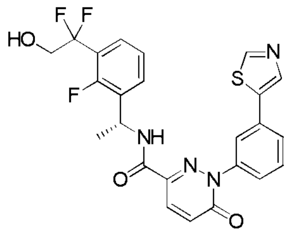 N-[(1R)-1-[3-(1,1-difluoro-2-hydroxy-ethyl)-2-fluorophenyl]ethyl]-6-oxo-1-(3-thiazol-5-ylphenyl)pyridazine-3-carboxamide | $^1$H NMR (400 MHz, CHLOROFORM-d) δ = 8.90 (s, 1H), 8.33 (s, 1H), 8.02 (d, *J* = 9.6 Hz, 1H), 7.92 - 7.86 (m, 2H), 7.81 (d, *J* = 9.2 Hz, 1H), 7.62 - 7.51 (m, 3H), 7.47 (t, *J* = 7.2 Hz, 1H), 7.24 - 7.20 (m, 1H), 7.12 (d, *J* = 9.6 Hz, 1H), 5.47 - 5.37 (m, 1H),4.30 - 4.19 (m, 1H), 4.05 - 3.93 (m, 1H), 1.60 (d, *J* = 7.2 Hz, 3H) ; LC/MS (ESI) m/z: 501.3 $[M+H]^+$ |
| 340 | 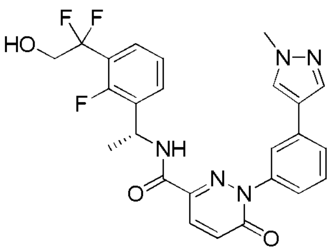 N-[(1R)-1-[3-(1,1-difluoro-2-hydroxy-ethyl)-2-fluoro-phenyl] ethyl]-1-[3-(1-methylpyrazol-4-yl)phenyl]-6-oxo-pyridazine-3-carboxamide | $^1$H NMR (400 MHz, DMSO-$d_6$) δ = 9.01 ( d, *J* = 8.0 Hz, 1H), 8.21 (s, 1H), 7.92 (s, 1H), 7.89 (d, *J* = 10.0 Hz, 1H), 7.81 (s, 1H), 7.69 - 7.61 (m, 2H), 7.53 (t, *J* = 8.0 Hz, 1H), 7.48 - 7.39 (m, 2H), 7.29 - 7.23 (m, 1H), 7.15 (d, *J* = 10.0 Hz, 1H), 5.73 (t, *J* = 6.4 Hz, 1H), 5.40 (m, *J* = 7.2 Hz, 1H), 3.96 - 3.90 (m, 2H), 3.87 (s, 3H), 1.47 (d, *J* = 7.2 Hz, 3H) ; LC/MS (ESI) m/z: 498.4 $[M+H]^+$ |
| 341 | 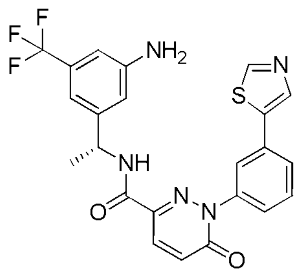 N-[(1R)-1-[3-amino-5-(trifluoromethyl)phenyl]ethyl]-6-oxo-1-(3-thiazol-5-ylphenyl)pyridazine-3-carboxamide | $^1$H NMR (400 MHz, CHLOROFORM-d) δ = 8.85 (s, 1H), 8.18 (s, 1H), 8.04 (d, *J* = 9.6 Hz, 1H), 7.82 - 7.78 (m, 1H), 7.69 - 7.63 (m, 1H), 7.57 (dd, *J* = 2.0, 3.8 Hz, 2H), 7.29 (d, *J* = 8.0 Hz, 1H), 7.12 (d, *J* = 10.0 Hz, 1H), 7.01 (s, 1H), 6.92 (d, *J* = 17.6 Hz, 2H), 5.20 (q, *J* = 7.2 Hz, 1H), 1.55 (d, *J* = 7.2 Hz, 3H) ; LC/MS (ESI) m/z: 486.3 $[M+H]^+$ |

(continued)

| No. | Structure/Name | Spectral Data |
|---|---|---|
| 342 | 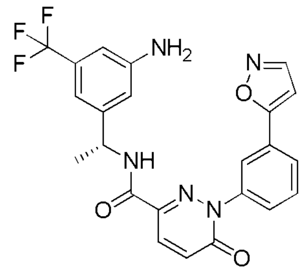 N-[(1R)-1-[3-amino-5-(trifluoromethyl)phenyl]ethyl]-1-(3-isoxazol-5-ylphenyl)-6-oxo-pyridazine-3-carboxamide | $^1$H NMR (400 MHz, DMSO-$d_6$) δ = 8.93 (d, *J* = 8.4 Hz, 1H), 8.71 (d, *J* = 2.0 Hz, 1H), 8.19 (s, 1H), 7.99 (d, J = 8.0 Hz, 1H), 7.92 (d, *J* = 9.6 Hz, 1H), 7.84 - 7.78 (m, 1H), 7.75 - 7.69 (m, 1H), 7.19 (d, *J* = 10.0 Hz, 1H), 7.12 (d, *J* = 2.0 Hz, 1H), 6.80 (br d, *J* = 14.4 Hz, 2H), 6.69 (s, 1H), 5.55 (s, 2H), 5.04 (s, 1H), 1.44 (d, *J* = 7.2 Hz, 3H) ; LC/MS (ESI) m/z: 470.3 $[M+H]^+$ |
| 343 | 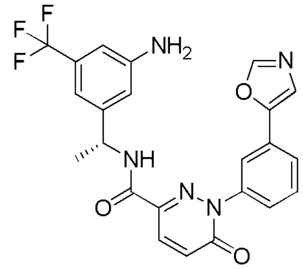 N-[(1R)-1-[3-amino-5-(trifluoromethyl)phenyl]ethyl]-1-(3-oxazol-5-ylphenyl)-6-oxo-pyridazine-3-carboxamide | $^1$H NMR (400 MHz, DMSO-$d_6$) δ = 8.91 (d, *J* = 8.4 Hz, 1H), 8.50 (s, 1H), 8.02 (s, 1H), 7.92 (d, *J* = 10.0 Hz, 1H), 7.85 - 7.81 (m, 1H), 7.78 (s, 1H), 7.68 - 7.65 (m, 2H), 7.17 (d, *J* = 9.6 Hz, 1H), 6.80 (d, *J* = 14.8 Hz, 2H), 6.70 (s, 1H), 5.55 (s, 2H), 5.04 (t, *J* = 7.6 Hz, 1H), 1.44 (d, *J* = 7.2 Hz, 3H); LC/MS (ESI) m/z: 470.3 $[M+H]^+$ |
| 344 | 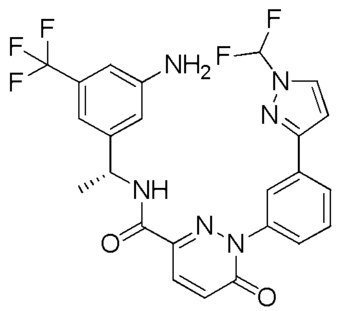 N-[(1R)-1-[3-amino-5-(trifluoromethyl)phenyl]ethyl]-1-[3-[1-(difluoromethyl)pyrazol-3-yl]phenyl]-6-oxo-pyridazine-3-carboxamide | $^1$H NMR (400 MHz, DMSO-$d_6$) δ ppm 1.44 (d, *J* =7.2 Hz, 3 H) 5.04 (quin, *J* =7.2 Hz, 1 H) 5.55 (s, 2 H) 6.69 (s, 1 H) 6.80 (br d, *J* =15.2 Hz, 2 H) 7.10 (d, *J* =2.8 Hz, 1 H) 7.17 (d, *J* =10.0 Hz, 1 H) 7.66 (m, 2 H) 7.72 (s, 1 H) 7.87 (s, 1 H) 7.92 (d, *J* = 10.0 Hz, 1 H) 7.99 (m, 1 H) 8.02 (s, 1 H) 8.14 (s, 1 H) 8.34 (d, *J* =2.8 Hz, 1 H) 8.91 (d, *J* =8.4 Hz, 1 H) ; LC/MS (ESI) m/z: 519.3 $[M+H]^+$ |
| 345 | 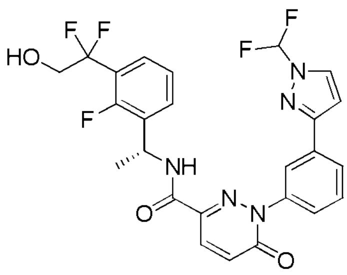 N-[(1R)-1-[3-(1,1-difluoro-2-hydroxy-ethyl)-2-fluoro-phenyl] ethyl]-1-[3-[1-(difluoromethyl)pyrazol-3-yl]phenyl]-6-oxo-pyridazine-3-carboxamide | $^1$H NMR (400 MHz, DMSO-$d_6$) δ ppm 1.47 (d, *J* =7.2 Hz, 3 H) 3.91 (br t, *J* =14.4 Hz, 2 H) 5.41 (quin, *J* =7.6 Hz, 1 H) 5.71 (m, 1 H) 7.12 (d, *J*=2.8 Hz, 1 H) 7.17 (d, *J*=10.0 Hz, 1 H) 7.27 (m, 1 H) 7.43 (m, 1 H) 7.67 (m, 3 H) 7.73 (s, 1 H) 7.88 (d, *J* =4.0 Hz, 1 H) 7.91 (s, 1 H) 8.00 (d, *J* =7.6 Hz, 1 H) 8.03 (s, 1 H) 8.16 (d, *J* =1.6 Hz, 1 H) 8.35 (d, *J* =2.8 Hz, 1 H) 9.03 (d, *J* =8.00 Hz, 1 H) ; LC/MS (ESI) m/z: 534.3 $[M+H]^+$ |

(continued)

| No. | Structure/Name | Spectral Data |
|---|---|---|
| 346 | 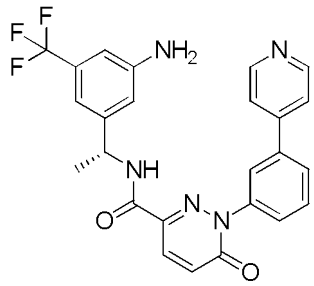<br>N-[(1R)-1-[3-amino-5-(trifluoromethyl)phenyl]ethyl]-6-oxo-1-[3-(4-pyridyl)phenyl]pyridazine-3-carboxamide | $^1$H NMR (400 MHz, DMSO-$d_6$) δ ppm 1.44 (d, *J*=7.2 Hz, 3 H) 5.05 (q, *J* =7.2 Hz, 1 H) 5.54 (s, 2 H) 6.70 (s, 1 H) 6.81 (br d, *J* =14.0 Hz, 2 H) 7.18 (d, *J* =10.0 Hz, 1 H) 7.70 (m, 1 H) 7.76 (m, 3 H) 7.92 (m, 2 H) 8.09 (t, *J* =2.0 Hz, 1 H) 8.67 (m, 2 H) 8.91 (d, *J* =8.4 Hz, 1 H) ; LC/MS (ESI) m/z: 535.4 [M+H]$^+$ |

**Example 347: N-[(1R)-1-[3-amino-5-(trifluoromethyl)phenyl]ethyl]-1-[3-(1-methyltetrazol-5-yl)phenyl]-6-oxo-pyridazine-3-carboxamide**

Step 1: Synthesis of 1-[3-(1-methyltetrazol-5-yl)phenyl]-6-oxo-pyridazine-3-carboxylic acid

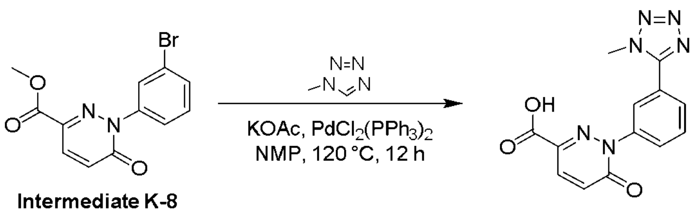


**Intermediate K-8**

A mixture of Intermediate K-8 (100 mg, 323.59 μmol), 1-methyltetrazole (68.0 mg, 808.76 μmol), KOAc (47.62 mg, 485.26 μmol) and $Pd(PPh_3)_2Cl_2$ (22.71 mg, 32.35 μmol) in NMP (3 mL) was degassed and purged with $N_2$ for 3 times, and then the mixture was stirred at 120 °C for 12 h under $N_2$ atmosphere. The reaction mixture was poured into water (20 mL), adjusted to pH= 3-4 with 1 N aq. HCl and extracted with EtOAc (30 mL×3). The combined organic layer was washed with brine (30 mL×2), dried over anhydrous $Na_2SO_4$ and concentrated under reduced pressure to obtain a crude product. The product was purified by prep-HPLC (column: C18-6 100*30mm*5μm; mobile phase: [water(FA)-ACN];B%: 0%-30%,15min), most of MeCN was removed under reduced pressure, and the remaining solvent was completely removed by lyophilization to give (1-[3-(1-methyltetrazol-5-yl)phenyl]-6-oxo-pyridazine-3-carboxylic acid (13 mg, 12.26% yield) as a white solid. MS (ESI) m/z = 299.1 [M+H]$^+$.

Step 2: Synthesis of N-[(1R)-1-[3-amino-5-(trifluoromethyl)phenyl]ethyl]-1-[3-(1-methyltetrazol-5-yl)phenyl]-6-oxo-pyridazine-3-carboxamide

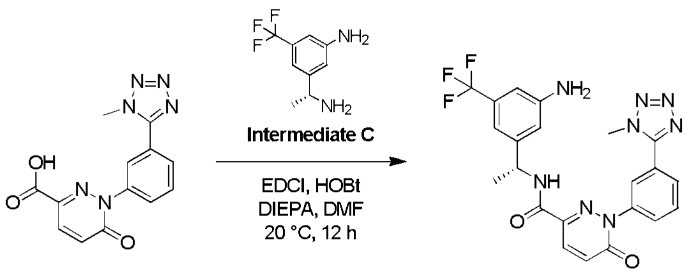


**347**

To a solution of 1-[3-(1-methyltetrazol-5-yl)phenyl]-6-oxo-pyridazine-3-carboxylic acid (13 mg, 43.59 μmol) and Intermediate C (10.49 mg, 43.59 μmol) in DMF (3 mL), DIEA (16.90 mg, 130.76 μmol, 22.78 μL), EDCI (10.03 mg, 52.30 μmol) and HOBt (7.07 mg, 52.30 μmol) were added, and the mixture was stirred at 20 °C for 12 h. The reaction mixture was

poured into water (20 mL) and extracted with EtOAc (30 mL × 3). The combined organic layer was washed with brine (20 mL×2), dried over anhydrous $Na_2SO_4$ and concentrated under reduced pressure to obtain a crude product. The product was purified by silica gel column chromatography (3% MeOH in DCM) and prep-HPLC (column: Phenomenex C18 75*30 mm*3 μm; mobile phase: [water($NH_3H_2O$+$NH_4HCO_3$)-ACN]; B%: 18%-48%, 10 min), the ACN solvent was removed by concentration under reduced pressure, and the product was lyophilized to give the compound of Example 347 (3.6 mg, 17.05% yield) as a white solid. $^1$H NMR (400MHz, DMSO-$d_6$) δ = 8.91 (d, *J*= *8.4* Hz, 1H), 8.20 (s, 1H), 8.05 -7.89 (m, 3H), 7.85 - 7.77 (m, 1H), 7.20 (d, *J* = 9.6 Hz, 1H), 6.80 (br d, *J* = 11.2 Hz, 2H), 6.70 (s, 1H), 5.55 (s, 2H), 5.05 (quin, *J* = 7.2 Hz, 1H), 4.21 (s, 3H), 1.44 (d, *J*= 7.2 Hz, 3H); MS (ESI) m/z = 485.3 [M+H]$^+$.

**Example 348 to Example 350**

The compounds shown in the following table were prepared in the same manner as in Example 347.

[Table 23]

| No. | Structure/Name | Spectral Data |
|---|---|---|
| 348 | 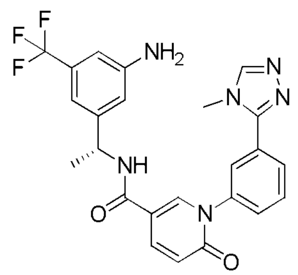 N-[(1R)-1-[3-amino-5-(trifluoromethyl)phenyl]ethyl]-1-[3-(4-methyl-1,2,4-triazol-3-yl)phenyl]-6-oxo-pyridine-3-carboxamide | $^1$H NMR (400 MHz, DMSO-$d_6$) δ = 8.62 (s, 1H), 8.59 (d, *J* = 8.0 Hz, 1H), 8.44 (d, *J* = 2.4 Hz, 1H), 7.98 (dd, *J* = 2.4, 9.6 Hz, 1H), 7.90 - 7.86 (m, 2H), 7.76 - 7.71 (m, 1H), 7.69 - 7.65 (m, 1H), 6.76 (s, 2H), 6.69 (s, 1H), 6.57 (d, *J* = 9.6 Hz, 1H), 5.55 (s, 2H), 5.01 (t, *J* = 7.2 Hz, 1H), 3.78 (s, 3H), 1.40 (d, *J* = 7.2 Hz, 3H) ; LC/MS (ESI) m/z: 483.4 [M+H]$^+$ |
| 349 | 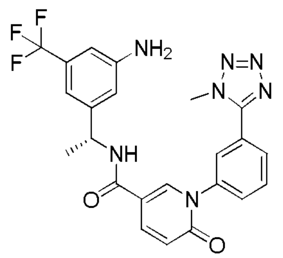 N-[(1R)-1-[3-amino-5-(trifluoromethyl)phenyl]ethyl]-1-[3-(1-methyltetrazol-5-yl)phenyl]-6-oxo-pyridine-3-carboxamide | $^1$ H NMR (400 MHz, DMSO-$d_6$) δ = 8.59 (d, *J* = 8.0 Hz, 1H), 8.44 (d, *J* = 2.4 Hz, 1H), 8.04 - 7.95 (m, 3H), 7.85 - 7.77 (m, 2H), 6.76 (s, 2H), 6.69 (s, 1H), 6.58 (d, *J* = 9.6 Hz, 1H), 5.55 (s, 2H), 5.02 (quin, *J* = 7.2 Hz, 1H), 4.20 (s, 3H), 1.40 (d, *J* = 6.8 Hz, 3H) ; LC/MS (ESI) m/z: 484.4 [M+H]$^+$ |
| 350 | 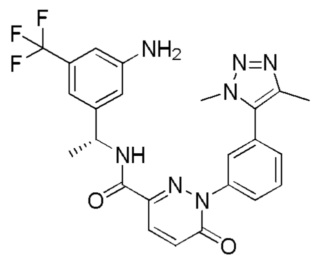 N-[(1R)-1-[3-amino-5-(trifluoromethyl)phenyl]ethyl]-1-[3-(3,5-dimethyltriazol-4-yl)phenyl]-6-oxo-pyridazine-3-carboxamide | $^1$H NMR (400 MHz, DMSO-$d_6$) δ = 8.88 (d, *J* = 8.4 Hz, 1H), 7.90 (d, *J* = 9.6 Hz, 1H), 7.88 - 7.81 (m, 2H), 7.72 (t, *J* = 7.6 Hz, 1H), 7.63 - 7.58 (m, 1H), 7.18 (d, *J* = 10.0 Hz, 1H), 6.80 (d, *J* = 11.2 Hz, 2H), 6.70 (s, 1H), 5.55 (s, 2H), 5.04 (quin, *J* = 7.2 Hz, 1H), 3.98 (s, 3H), 2.27 (s, 3H), 1.45 (d, *J* = 7.2 Hz, 3H) ; LC/MS (ESI) m/z: 498.4 [M+H]$^+$ |

**Example 351: N-[(1R)-1-[3-amino-5-(trifluoromethyl)phenyl]ethyl]-1-[3-(1-methyl-6-oxo-2-pyridyl)phenyl]-6-oxo-pyridazine-3-carboxamide**

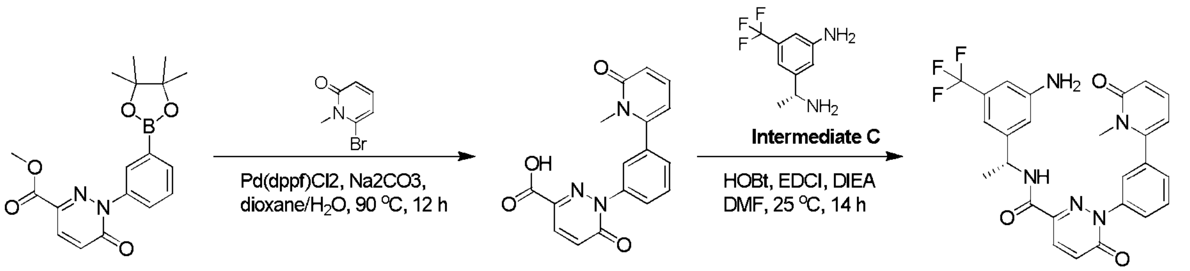


351

The compound of Example 351 was prepared in a similar manner to Steps 2 and 4 of Example 318. $^1$H NMR (400 MHz, DMSO-$d_6$) δ = 8.89 (d, *J* = 8.4 Hz, 1H), 7.81-7.95 (m, 3H), 7.69 (t, *J* = 7.6 Hz, 1H), 7.59 (d, *J* = 7.6 Hz, 1H), 7.48 (dd, *J* = 9.2, 7.2 Hz, 1H), 7.18 (d, *J* = 9.6 Hz, 1H), 6.81 (d, *J* = 11.2 Hz, 2H), 6.71 (s, 1H), 6.48 (d, *J* = 8.8 Hz, 1H), 6.22 (d, *J* = 6.8 Hz, 1H), 5.55 (s, 2H), 5.05 (q, *J* = 7.2 Hz, 1H), 3.31-3.32 (m, 3H), 1.46 (d, *J* = 7.2 Hz, 3H) ; LC/MS (ESI) m/z: 510.2 $[M+H]^+$

**Example 352: N-[(1R)-1-[3-(1,1-difluoro-2-hydroxy-ethyl)-2-fluoro-phenyl]ethyl]-1-(2,4-difluorophenyl)-6-oxo-pyridazine-3-carboxamide**

Step 1: Synthesis of dimethyl (2E)-2-[(2,4-difluorophenyl)hydrazono]-3-oxo-glutarate

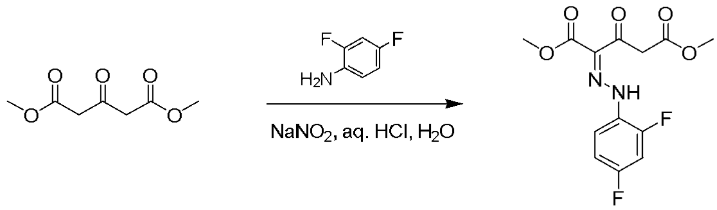


A mixed solution of 10 M HCl (10 mL) and water (20 mL), and 2,4-difluoroaniline (2.58 g, 19.98 mmol) were added to a solution of $NaNO_2$ (1.38 g, 19.98 mmol) in water (15 mL) at 5 °C. The reaction mixture was poured into a mixed solution of dimethyl 3-oxopentanedioate (3.48 g, 19.98 mmol, 2.88 mL) in EtOH (12 mL) and NaOAc (12.00 g, 146.25 mmol) in water (40 mL). The mixture was stirred at 25 °C for 0.5 hour. The reaction mixture was poured into water (100 mL) and extracted with EtOAc (80 mL×3). The organic layer was washed with brine (50 mL × 2), dried over anhydrous $Na_2SO_4$ and filtered under reduced pressure. The crude product of dimethyl (2E)-2-[(2,4-difluorophenyl)hydrazono]-3-oxo-glutarate (6.2 g, 83.61% yield) as yellow oil was used in the next step without further purification. MS (ESI) m/z = 315.0 $[M+H]^+$).

Step 2: Synthesis of methyl-1-(2,4-difluorophenyl)-4-hydroxy-6-oxo-pyridazine-3-carboxylate

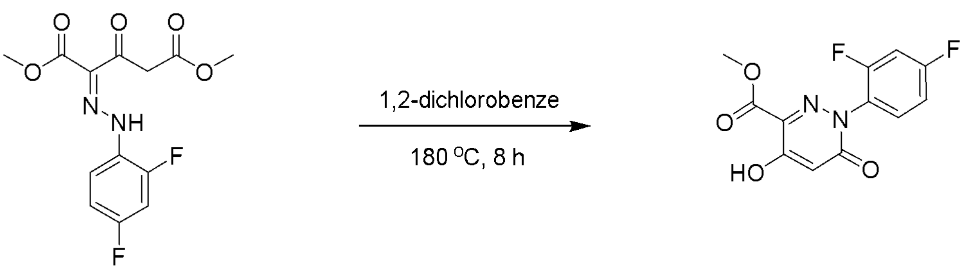


A solution of dimethyl (2E)-2-[(2,4-difluorophenyl)hydrazono]-3-oxo-glutarate (6.2 g, 19.73 mmol) in 1,2-dichlorobenzene (62 mL) was stirred at 175 °C for 6 h under air. The product was purified by silica gel column chromatography (25% EtOAc in PE) to give methyl-1-(2,4-difluorophenyl)-4-hydroxy-6-oxo-pyridazine-3-carboxylic acid (3.3 g, 59.01% yield) as yellow oil. $^1$H NMR (400 MHz, DMSO-$d_6$) δ ppm 12.21 (br s, 1H), 7.63 (dt, J = 6.0, 8.8 Hz, 1H), 7.53 - 7.48 (m, 1H), 7.28 -7.23 (m, 1H), 6.22 (s, 1H), 3.83 (s, 3H); MS (ESI) m/z = 282.9 $[M+H]^+$.

Step 3: Synthesis of methyl-1-(2,4-difluorophenyl)-6-oxo-4-(trifluoromethylsulfonyloxy)pyridazine-3-carboxylate

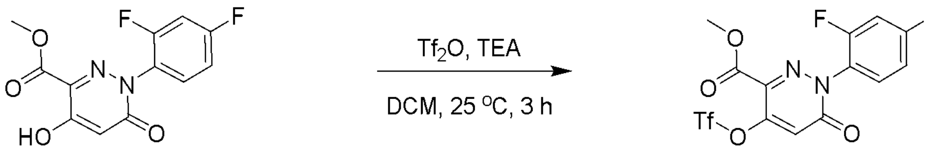


To a solution of methyl-1-(2,4-difluorophenyl)-4-hydroxy-6-oxo-pyridazine-3-carboxylate (500 mg, 1.77 mmol) in DCM (30 mL) was added dropwise a mixed solution of $Tf_2O$ (1.25 g, 4.43 mmol, 730.84 μL) and DCM (20 mL) at -70 °C, followed by stirring at 25°C for 3 h under $N_2$ atmosphere. The reaction mixture was poured into water (30 mL) and extracted with EtOAc (30 mL×3). The organic layer was washed with brine (30 mL), dried over anhydrous $Na_2SO_4$ and filtered under reduced pressure to obtain a crude product. The crude product was purified by silica gel column chromatography (20% EtOAc in PE) to give methyl-1-(2,4-difluorophenyl)-6-oxo-4-(trifluoromethylsulfonyloxy)pyridazine-3-carboxylate (600 mg, 76.04% yield) as colorless oil. MS (ESI) m/z = 414.9 $[M+H]^+$.

Step 4: Synthesis of methyl-1-(2,4-difluorophenyl)-6-oxopyridazine-3-carboxylate

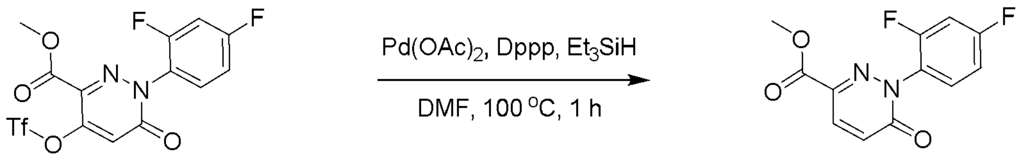


A solution of methyl-1-(2,4-difluorophenyl)-6-oxo-4-(trifluoromethylsulfonyloxy)pyridazine-3-carboxylate (250 mg, 603.48 μmol), $Pd(OAc)_2$ (20.32 mg, 90.52 μmol), DPPP (74.67 mg, 181.05 μmol) and $Et_3SiH$ (91.22 mg, 784.53 μmol, 125.31 μL) in DMF (3 mL) was degassed and purged with $N_2$ for 3 times, and then the mixture was stirred at 100 °C for 1 hour under $N_2$ atmosphere. The reaction mixture was poured into water (30 mL) and extracted with EtOAc (30 mL×3). The organic layer was washed with brine (30 mL), dried over anhydrous $Na_2SO_4$ and filtered under reduced pressure to obtain a crude product. The crude product was purified by silica gel column chromatography (20% EtOAc in PE) to give methyl-1-(2,4-difluorophenyl)-6-oxopyridazine-3-carboxylate (70 mg, 39.83% yield) as colorless oil. MS (ESI) m/z = 247.0 $[M+H]^+$).

Step 5: 1-(2,4-difluorophenyl)-6-oxo-pyridazine-3-carboxylic acid

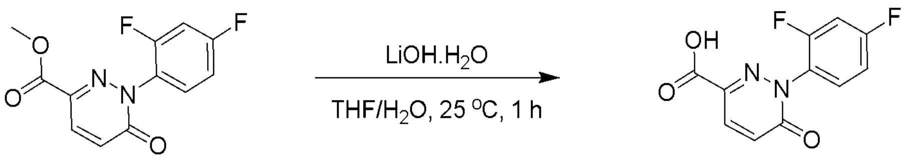


A solution of methyl-1-(2,4-difluorophenyl)-6-oxo-pyridazine-3-carboxylate (70 mg, 262.96 μmol) and LiOH·-$H_2O$ (33.10 mg, 788.88 μmol) in THF (2 mL) and $H_2O$ (1 mL) was stirred at 25 °C for 1 hour under air. The reaction mixture was adjusted to pH = 3-4 with 1 N HCl and then extracted with EtOAc (30 mL×3). The organic layer was washed with water (30 mL) and brine (30 mL), dried over anhydrous $Na_2SO_4$ and filtered under reduced pressure to give 1-(2,4-difluorophenyl)-6-oxo-pyridazine-3-carboxylic acid (60 mg, 77.10% yield) as a crude product of a white solid, which was used in the next step without further purification. MS (ESI) m/z = 253 $[M+H]^+$.

Step 6: N-[(1R)-1-[3-(1,1-difluoro-2-hydroxy-ethyl)-2-fluoro-phenyl]ethyl]-1-(2,4-difluorophenyl)-6-oxo-pyridazine-3-carboxamide

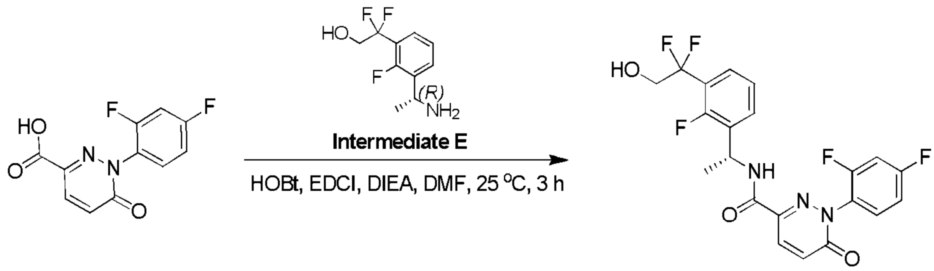


352

A solution of 1-(2,4-difluorophenyl)-6-oxo-pyridazine-3-carboxylic acid (30 mg, 118.97 μmol), Intermediate E (31.29 mg, 122.40 μmol, HCl), EDCI (45.61 mg, 237.93 μmol), HOBt (32.15 mg, 237.93 μmol) and DIEA (46.13 mg, 356.90 μmol, 62.17 μL) in DMF (2 mL) was degassed and purged with $N_2$ for 3 times, and then the mixture was stirred at 25 °C for 3 h under $N_2$ atmosphere. The reaction mixture was poured into water (30 mL) and extracted with EtOAc (30 mL×3). The organic layer was washed with brine (30 mL), dried over anhydrous $Na_2SO_4$ and filtered under reduced pressure to obtain a crude product. The product was purified by silica gel column chromatography (20% EtOAc in PE) to obtain a residue, which was purified by prep-HPLC (column: Phenomenex C18 75*30mm*3μm; mobile phase: [water($NH_3H_2O$+$NH_4HCO_3$)-ACN];B%: 28%-58%,11min). Most of $CH_3CN$ was removed under reduced pressure, and the remaining solvent was removed by lyophilization to give the compound of Example 352 (3.4 mg, 6.21% yield) as a white solid. 1H NMR (400 MHz, DMSO-$d_6$) δ ppm 9.03 (d, J = 8.0 Hz, 1H), 7.92 (d, J = 10.0 Hz, 1H), 7.79 (dt, J = 6.0, 8.8 Hz, 1H), 7.65 - 7.51 (m, 2H), 7.43 (t, J = 6.8 Hz, 1H), 7.34 (t, J = 8.8 Hz, 1H), 7.30 - 7.25 (m, 1H), 7.18 (d, J = 9.6 Hz, 1H), 5.71 (t, J = 6.4 Hz, 1H), 5.40 (quin, J = 7.2 Hz, 1H), 3.91 (dt, J = 6.4, 14.4 Hz, 2H), 1.46 (d, J = 7.2 Hz, 3H); MS (ESI) m/z = 454.1 $[M+H]^+$.

**Example 353 to Example 362**

The compounds shown in the following table were obtained in the same manner as in Example 352 by using starting materials and intermediates corresponding to the respective structures of the desired compounds, which were prepared based on Steps 1 to 3 of Preparation Example 8 and Step 2 of Preparation Example 29.

[Table 24]

| No. | Structure/Name | Spectral Data |
|---|---|---|
| 353 | 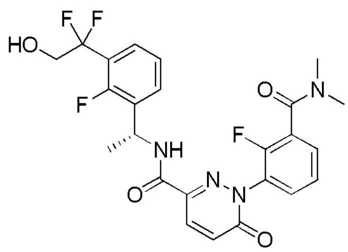 N-[(1R)-1-[3-(1,1-difluoro-2-hydroxy-ethyl)-2-fluoro-phenyl] ethyl]-1-[3-(dimethylcarbamoyl)-2-fluoro-phenyl]-6-oxo-pyridazine-3-carboxamide | $^1$H NMR (400MHz, DMSO-$d_6$) δ = 9.06 (d, *J* = 8.0 Hz, 1H), 7.93 (d, *J* = 10.0 Hz, 1H), 7.82 - 7.74 (m, 1H), 7.62 (br t, *J* = 7.2 Hz, 1H), 7.59 - 7.55 (m, 1H), 7.52 - 7.46 (m, 1H), 7.43 (br t, *J* = 6.8 Hz, 1H), 7.31 - 7.24 (m, 1H), 7.19 (d, *J* = 9.6 Hz, 1H), 5.70 (t, *J* = 6.4 Hz, 1H), 5.40 (quin, *J* = 7.2 Hz, 1H), 3.91 (dt, *J* = 6.4, 14.4 Hz, 2H), 3.03 (s, 3H), 2.90 (s, 3H), 1.46 (d, *J* = 7.2 Hz, 3H); MS (ESI) m/z = 507.4 $[M+H]^+$. |
| 354 | 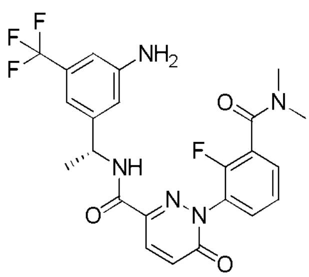 N-[(1R)-1-[3-amino-5-(trifluoromethyl)phenyl]ethyl]-1-[3-(dimethylcarbamoyl)-2-fluoro-phenyl]-6-oxo-pyridazine-3-carboxamide | $^1$H NMR (400 MHz, DMSO-$d_6$) δ = 8.94 (d, *J* = 8.4 Hz, 1H), 7.94 (d, *J* = 10.0 Hz, 1H), 7.84 - 7.69 (m, 1H), 7.61-7.52 (m, 1H), 7.51-7.42 (m, 1H), 7.19 (d, *J* = 9.6 Hz, 1H), 6.79 (br d, *J* = 12.8 Hz, 2H), 6.70 (s, 1H), 5.53 (s, 2H), 5.02 (quin, *J* = 7.2 Hz, 1H), 3.02 (s, 3H), 2.89 (s, 3H), 1.43 (d, *J* = 7.2 Hz, 3H); MS (ESI) m/z = 492.3 $[M+H]^+$. |

(continued)

| No. | Structure/Name | Spectral Data |
|---|---|---|
| 355 | 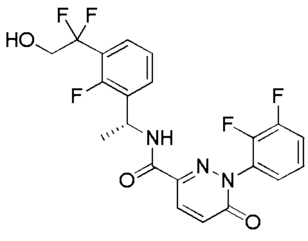 N-[(1R)-1-[3-(1,1-difluoro-2-hydroxy-ethyl)-2-fluoro-phenyl] ethyl]-1-(2,3-difluorophenyl)-6-oxo-pyridazine-3-carboxamide | $^1$H NMR (400 MHz, DMSO-$d_6$) δ = 9.05 (d, *J* = 8.0 Hz, 1H), 7.93 (d, *J* = 10.0 Hz, 1H), 7.71 - 7.54 (m, 3H), 7.49 - 7.40 (m, 2H), 7.30 - 7.24 (m, 1H), 7.21 (d, *J* = 9.6 Hz, 1H), 5.71 (br s, 1H), 5.40 (quin, *J* = 7.2 Hz, 1H), 3.97 - 3.86 (m, 2H), 1.46 (d, *J* = 6.8 Hz, 3H) LC/MS (ESI) m/z: 454.3 $[M+H]^+$ |
| 356 | 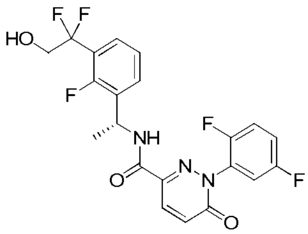 N-[(1R)-1-[3-(1,1-difluoro-2-hydroxy-ethyl)-2-fluoro-phenyl] ethyl]-1-(2,5-difluorophenyl)-6-oxo-pyridazine-3-carboxamide | $^1$H NMR (400 MHz, DMSO-$d_6$) δ = 9.04 (d, *J* = 8.0 Hz, 1H), 7.92 (d, *J* = 10.0 Hz, 1H), 7.72 (ddd, *J* = 3.2, 5.6, 8.4 Hz, 1H), 7.62 (br t, *J* = 6.8 Hz, 1H), 7.58 - 7.52 (m, 1H), 7.51 - 7.46 (m, 1H), 7.46 - 7.41 (m, 1H), 7.30 - 7.25 (m, 1H), 7.19 (d, *J* = 9.6 Hz, 1H), 5.71 (br t, *J* = 6.0 Hz, 1H), 5.40 (quin, *J* = 7.2 Hz, 1H), 3.91 (dt, *J* = 5.6, 14.4 Hz, 2H), 1.47 (d, *J* = 7.2 Hz, 3H); LC/MS (ESI) m/z: 454.3 $[M+H]^+$ |
| 357 | 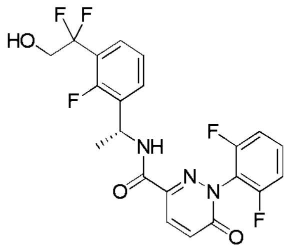 N-[(1R)-1-[3-(1,1-difluoro-2-hydroxy-ethyl)-2-fluoro-phenyl] ethyl]-1-(2,6-difluorophenyl)-6-oxo-pyridazine-3-carboxamide | $^1$H NMR (400 MHz, DMSO-$d_6$) δ = 9.11 (br d, *J* = 8.0 Hz, 1H), 7.97 (d, *J* =10.0 Hz, 1H), 7.74 - 7.65 (m, 1H), 7.60 (br t, *J* = 7.2 Hz, 1H), 7.42 (br t, *J* = 8.4 Hz, 3H), 7.31 - 7.22 (m, 2H), 5.70 (t, *J* = 6.4 Hz, 1H), 5.39 (quin, *J* = 7.2 Hz, 1H), 3.91 (dt, *J* = 6.4, 14.4 Hz, 2H), 1.45 (d, *J* = 7.2 Hz, 3H) LC/MS (ESI) m/z: 454.3 $[M+H]^+$ |
| 358 | 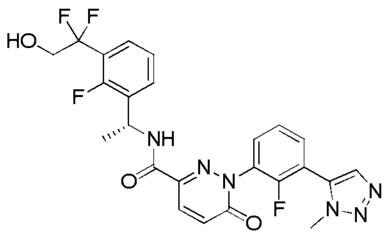 N-[(1R)-1-[3-(1,1-difluoro-2-hydroxy-ethyl)-2-fluoro-phenyl] ethyl]-1-[2-fluoro-3-(3-methyltriazol-4-yl)phenyl]-6-oxo-pyridazine-3-carboxamide | $^1$H NMR (400 MHz, DMSO-$d_6$) ) δ = 9.06 (d, *J* = 8.0 Hz, 1H), 8.00 (s, 1H), 7.97 - 7.85 (m, 2H), 7.83 - 7.75 (m, 1H), 7.66 - 7.57 (m, 2H), 7.46 - 7.40 (m, 1H), 7.30 - 7.24 (m, 1H), 7.21 (d, *J* = 9.6 Hz, 1H), 5.72 (br s, 1H), 5.41 (quin, *J* = 7.2 Hz, 1H), 4.04 (s, 3H), 3.91 (t, *J* = 14.4 Hz, 2H), 1.47 (d, *J* = 7.2 Hz, 3H); LC/MS (ESI) m/z: 517.4 $[M+H]^+$ |
| 359 | 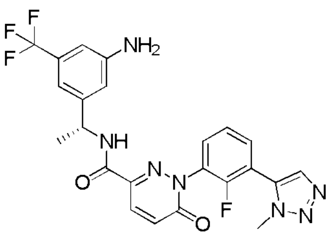 N-[(1R)-1-[3-amino-5-(trifluoromethyl)phenyl]ethyl]-1-[2-fluoro-3-(3-methyltriazol-4-yl)phenyl]-6-oxo-pyridazine-3-carboxamide | $^1$H NMR (400 MHz, DMSO-$d_6$) δ = 8.95 (d, *J* = 8.4 Hz, 1H), 7.99 (s, 1H), 7.96 (d, *J* = 10.0 Hz, 1H), 7.93 - 7.84 (m, 1H), 7.78 (t, *J* = 6.4 Hz, 1H), 7.64 - 7.56 (m, 1H), 7.21 (d, *J* = 9.6 Hz, 1H), 6.79 (br d, *J* = 13.2 Hz, 2H), 6.70 (s, 1H), 5.55 (s, 2H), 5.03 (quin, *J* = 7.2 Hz, 1H), 4.03 (s, 3H), 1.43 (d, *J* = 7.2 Hz, 3H); LC/MS (ESI) m/z: 502.4 $[M+H]^+$ |

(continued)

| No. | Structure/Name | Spectral Data |
|---|---|---|
| 360 | 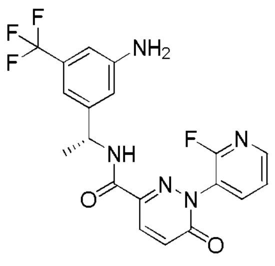 N-[(1R)-1-[3-amino-5-(trifluoromethyl)phenyl]ethyl]-1-(2-fluoro-3-pyridyl)-6-oxo-pyridazine-3-carboxamide | $^1$H NMR (400 MHz, DMSO-$d_6$) $\delta$ = 8.95 (d, $J$ = 8.4 Hz, 1H), 8.41 (d, $J$ = 4.8 Hz, 1H), 8.32 (ddd, $J$ = 1.6, 7.6, 9.6 Hz, 1H), 7.95 (d, $J$ = 10.0 Hz, 1H), 7.63 (t, $J$ = 6.4 Hz, 1H), 7.21 (d, $J$ = 9.6 Hz, 1H), 6.80 (s, 1H), 6.77 (s, 1H), 6.70 (s, 1H), 5.54 (s, 2H), 5.03 (quin, $J$ = 7.2 Hz, 1H), 1.42 (d, $J$ = 7.2 Hz, 3H); LC/MS (ESI) m/z: 422.3 [M+H]$^+$ |
| 361 | 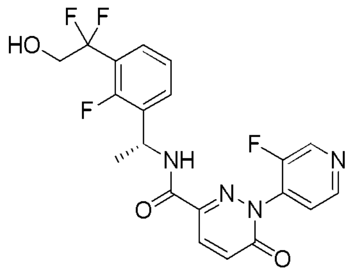 N-[(1R)-1-[3-(1,1-difluoro-2-hydroxy-ethyl)-2-fluoro-phenyl] ethyl]-1-(3-fluoro-4-pyridyl)-6-oxo-pyridazine-3-carboxamide | 1H NMR (400 MHz, DMSO-$d_6$) $\delta$ = 9.08 (d, J = 8.0 Hz, 1H), 8.86 (d, J = 2.0 Hz, 1H), 8.70 (d, J = 5.2 Hz, 1H), 7.94 (d, J = 10.0 Hz, 1H), 7.88 (t, J = 5.6 Hz, 1H), 7.62 (t, J = 6.8 Hz, 1H), 7.43 (t, J = 6.8 Hz, 1H), 7.28 (t, J = 7.6 Hz, 1H), 7.23 (d, J = 9.6 Hz, 1H), 5.72 (t, J = 6.4 Hz, 1H), 5.44 - 5.37 (m, 1H), 3.91 (dt, J = 6.4, 14.4 Hz, 2H), 1.46 (d, J = 7.2 Hz, 3H); LC/MS (ESI) m/z: 437.3 [M+H]$^+$ |
| 362 | 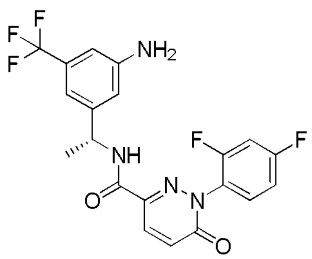 N-[(1R)-1-[3-amino-5-(trifluoromethyl)phenyl]ethyl]-1-(2,4-difluorophenyl)-6-oxo-pyridazine-3-carboxamide | 1H NMR (400 MHz, DMSO-$d_6$) $\delta$ = 8.92 (d, J = 8.4 Hz, 1H), 7.94 (d, J = 10.0 Hz, 1H), 7.77 (dt, J = 6.0, 8.8 Hz, 1H), 7.54 (t, J = 9.6 Hz, 1H), 7.35 - 7.30 (m, 1H), 7.18 (d, J = 10.0 Hz, 1H), 6.80 (s, 1H), 6.77 (s, 1H), 6.70 (s, 1H), 5.54 (br s, 2H), 5.02 (quin, J = 7.2 Hz, 1H), 1.43 (d, J = 7.2 Hz, 3H) ; LC/MS (ESI) m/z: 439.3 [M+H]$^+$ |

**Example 363: N-[(1R)-1-[3-amino-5-(trifluoromethyl)phenyl]ethyl]-1-(2-fluorophenyl)-4-hydroxy-6-oxo-pyridazine-3-carboxamide**

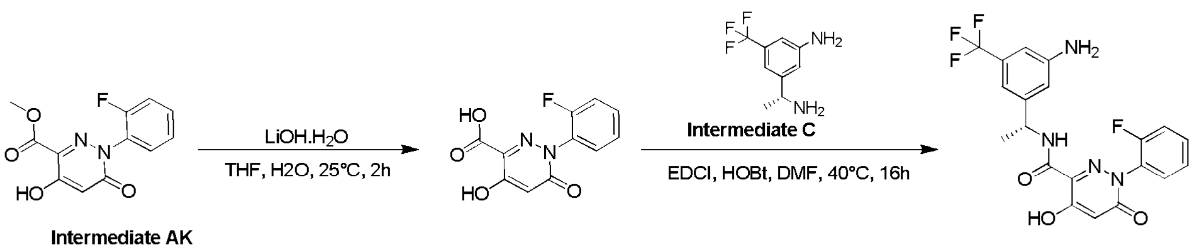


Intermediate AK

**363**

The compound of Example 363 (1.5 mg, 2.81% yield) was obtained in the same manner as in Example 76 as a white solid. $^1$H NMR (400 MHz, DMSO-$d_6$) $\delta$ = 13.46 - 11.56 (m, 1H), 10.28 - 9.58 (m, 1H), 7.55 - 7.49 (m, 2H), 7.42 - 7.32 (m, 2H), 6.78 (br d, $J$ = 10.4 Hz, 2H), 6.71 (s, 1H), 6.00 (br s, 1H), 5.77 - 5.37 (m, 2H), 5.01 (quin, $J$ = 7.2 Hz, 1H), 1.41 (d, $J$ = 6.8 Hz, 3H); MS (ESI) m/z = 437.1 [M+H]$^+$.

**Example 364: 4-benzyloxy-N-[(1R)-1-[3-(1,1-difluoro-2-hydroxy-ethyl)-2-fluorophenyl]ethyl]-1-(2-fluorophenyl)-6-oxo-pyridazine-3-carboxamide**

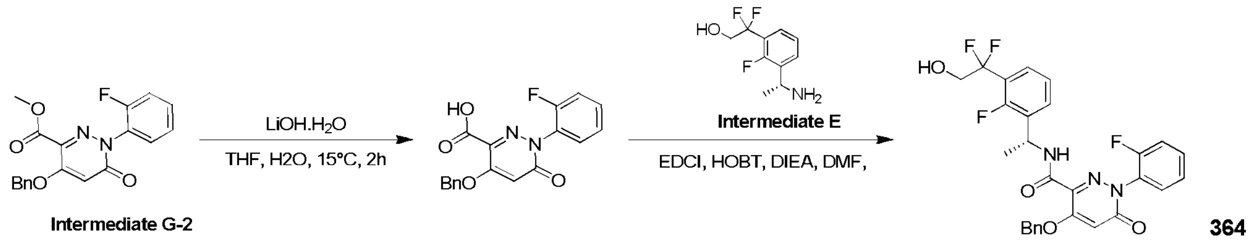


Intermediate G-2

364

The compound of Example 364 (2.4 mg, 3.02% yield) was obtained as a white solid in the same manner as in Example 76. $^1H$ NMR (400MHz, DMSO-$d_6$) $\delta$ = 9.25 (d, *J* = 7.6 Hz, 1H), 7.59 - 7.50 (m, 2H), 7.46 - 7.34 (m, 9H), 7.03 (t, *J* = 7.6 Hz, 1H),6.68 (s, 1H), 5.71 (t, *J* = 6.4 Hz, 1H), 5.29 - 5.20 (m, 3H), 3.89 (dt, *J* = 6.4, 14.4 Hz, 2H), 1.36 (d, *J* = 7.2 Hz, 3H); MS (ESI) m/z = 542.4 $[M+H]^+$.

**Example 365: N-[(1R)-1-[3-(1,1-difluoro-2-hydroxy-ethyl)-2-fluoro-phenyl]ethyl]-1-(2-fluorophenyl)-4-hydroxy-6-oxo-pyridazine-3-carboxamide**

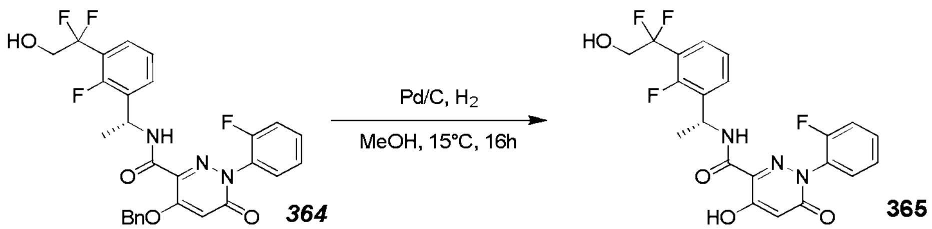


364

365

The compound of Example 365 (6.1 mg, 24.27% yield) was obtained as a white solid by reacting the compound of Example 364 in the same manner as in Preparation Example 3. $^1H$ NMR (400MHz, DMSO-$d_6$) $\delta$ = 12.66 - 12.16 (m, 1H), 9.50 (br s, 1H), 7.62 (t, *J* = 6.8 Hz, 1H), 7.59 - 7.52 (m, 2H), 7.48 - 7.35 (m, 3H), 7.32 - 7.26 (m, 1H), 6.21 (s, 1H), 5.71 (br t, *J* = 6.0 Hz, 1H), 5.37 (quin, *J* = 7.2 Hz, 1H), 3.91 (dt, *J* = 5.2, 14.2 Hz, 2H), 1.45 (d, *J* = 7.2 Hz, 3H); MS (ESI) m/z = 452.3 $[M+H]^+$.

**Example 366: 4-cyclopropyl-N-[(1R)-1-[3-(1,1-difluoro-2-hydroxy-ethyl)-2-fluorophenyl]ethyl]-1-(2-fluoro-4-methoxy-phenyl)-6-oxopyridazine-3-carboxamide**

Step 1: Synthesis of 4-cyclopropyl-1-(2-fluoro-4-methoxy-phenyl)-6-oxo-pyridazine-3-carboxylic acid

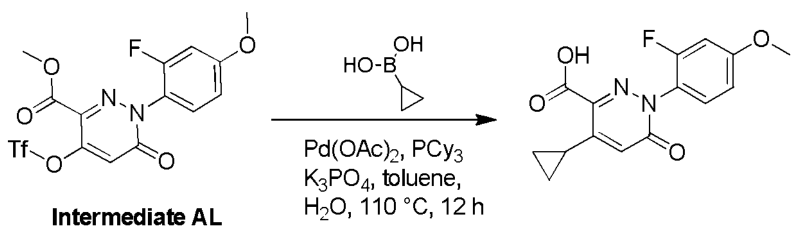


Intermediate AL

A mixture of Intermediate AL (300 mg, 703.74 μmol), cyclopropylboronic acid (120.90 mg, 1.41 mmol), $Pd(OAc)_2$ (23.70 mg, 105.56 μmol), $K_3PO_4$ (746.90 mg, 3.52 mmol) and $P(Cy)_3$ (59.20 mg, 211.12 μmol, 68.44 μL) in toluene (4 mL) and $H_2O$ (0.5 mL) was degassed and purged with $N_2$ for 3 times, and then the mixture was stirred at 100 °C for 12 h under $N_2$ atmosphere. The reaction mixture was poured into water (20 mL) and extracted with EtOAc (20 mL × 3). The combined organic layer was discarded, and the aqueous layer was adjusted to pH = 3-4 with 1 N aq. HCl and extracted with EtOAc (30 mL × 3). The organic layer was washed with water (30 mL) and brine (30 mL), dried over $Na_2SO_4$, filtered and concentrated

under reduced pressure to obtain a crude product. 4-cyclopropyl-1-(2-fluoro-4-methoxy-phenyl)-6-oxo-pyridazine-3-carboxylic acid (crude product, 200 mg, 79.31% yield) as a yellow solid was used in the next step without further purification. MS (ESI) m/z = 304.9 [M+H]$^+$.

Step 2: Synthesis of 4-cyclopropyl-N-[(1R)-1-[3-(1,1-difluoro-2-hydroxy-ethyl)-2-fluoro-phenyl]ethyl]-1-(2-fluoro-4-methoxy-phenyl)-6-oxopyridazine-3-carboxamide

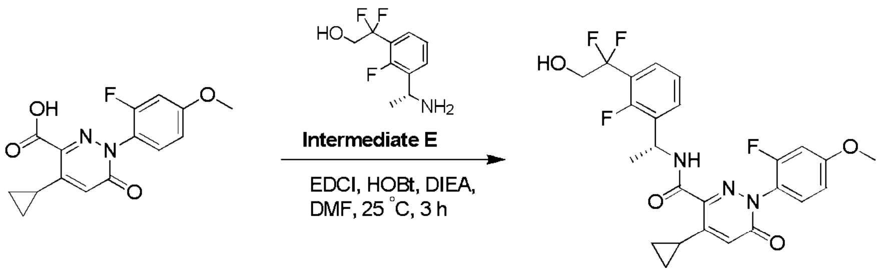


**366**

The compound of Example 366 (6 mg, 11.83% yield) was obtained in the same manner as in Step 2 of Example 76 as a white solid. $^1$H NMR (400MHz, DMSO-$d_6$) δ = 9.26 (d, *J* = 7.6 Hz, 1H), 7.58 (t, *J* = 6.8 Hz, 1H), 7.51 - 7.40 (m, 2H),7.30 (t, *J* = 7.6 Hz, 1H), 7.04 (dd, *J* = 2.4, 12.0 Hz, 1H), 6.93 (dd, *J* = 2.4, 8.8 Hz, 1H), 6.62 (s, 1H), 5.72 (br s, 1H), 5.33 (quin, *J* = 7.2 Hz, 1H), 3.91 (br t, *J* = 14.4 Hz, 2H), 3.83 (s, 3H), 1.98 - 1.90 (m, 1H), 1.44 (d, *J* = 6.8 Hz, 3H), 1.04 - 0.94 (m, 2H), 0.93 - 0.88 (m, 1H), 0.87 - 0.80 (m, 1H); MS (ESI) m/z = 506.4 [M+H]$^+$.

**Example 367 and Example 368**

The compounds shown in the following table were obtained in the same manner as in Example 366 by using appropriate starting materials and intermediates corresponding to the respective structures of the desired compounds based on Preparation Example 38.

[Table 25]

| No. | Structure/Name | Spectral Data |
|---|---|---|
| 367 | 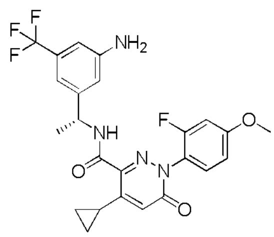 N-[(1R)-1-[3-amino-5-(trifluoromethyl)phenyl]ethyl]-4-cyclopropyl-1-(2-fluoro-4-methoxy-phenyl)-6-oxo-pyridazine-3-carboxamide | $^1$H NMR (400 MHz, DMSO-$d_6$) δ = 9.14 (d, *J* = 8.0 Hz, 1H), 7.45 (t, *J* = 8.8 Hz, 1H), 7.03 (dd, *J* = 2.4, 12.0Hz, 1H), 6.91 (dd, *J* = 2.4, 8.8 Hz, 1H), 6.77 (d, *J* = 8.4 Hz, 2H), 6.71 (s, 1H), 6.61 (s, 1H), 5.58 (s, 2H), 4.97 (quin, *J* = 7.2 Hz, 1H), 3.82 (s, 3H), 1.97 - 1.90 (m, 1H), 1.39 (d, *J* = 7.2 Hz, 3H), 1.02 - 0.82 (m, 4H); LC/MS (ESI) m/z: 491.3 [M+H]$^+$ |
| 368 | 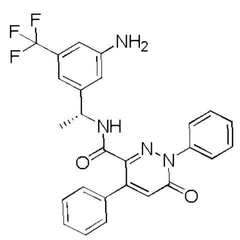 N-[(1R)-1-[3-amino-5-(trifluoromethyl)phenyl]ethyl]-6-oxo-1,4-diphenyl-pyridazine-3-carboxamide | $^1$H NMR (400 MHz, DMSO-$d_6$) δ = 9.28 (d, *J* = 8.0 Hz, 1H), 7.67 (d, *J* = 7.6 Hz, 2H), 7.55 (t, *J* = 7.2 Hz, 2H),7.49 - 7.38 (m, 4H), 7.34 - 7.28 (m, 2H), 7.12 (s, 1H), 6.81 (s, 1H), 6.74 (s, 2H), 5.59 (s, 2H), 4.83 (quin, *J* = 7.2 Hz, 1H), 1.34(d, *J* = 7.2 Hz, 3H); LC/MS (ESI) m/z: 479.3 [M+H]$^+$ |

**Example 369: N-[(1R)-1-[3-amino-5-(trifluoromethyl)phenyl]ethyl]-1-[3-(3-methyltriazol-4-yl)phenyl]-6-oxo-pyridazine-3-carboxamide**

Step 1: Synthesis of tributyl-(3-methyltriazol-4-yl)stannane

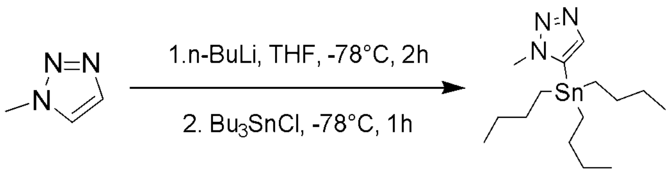


A solution of 1-methyltriazole (3 g, 36.10 mmol) in THF (30 mL) was cooled to -78 °C, followed by the dropwise addition of n-BuLi (2.5 M, 899.66 μL), and the mixture was stirred at - 78 °C for 2 h, added dropwise with tributyl(chloro) stannane (16.94 g, 52.04 mmol, 14 mL), and then the resulting mixture was stirred at -78°C for 1 hour under $N_2$ atmosphere. The reaction mixture was added dropwise slowly with $H_2O$ at 0°C and extracted with EtOAc (70 mL× 3). The combined organic layer was washed with brine (50 × 2 mL), dried over $Na_2SO_4$ and concentrated under reduced pressure to obtain a crude product of tributyl-(3-methyltriazol-4-yl)stannane (17.35 g, 34.97 mmol, 96.85% yield) as light yellow oil. $^1$H NMR (400 MHz, DMSO-$d_6$) δ = 7.58 (br s, 1H), 4.06 - 4.00 (m, 3H), 1.50 - 1.44 (m, 4H), 1.28 (br d, *J* = 7.2 Hz, 8H), 1.19 - 1.12 (m, 6H), 0.87 - 0.82 (m, 9H).

Step 2: Synthesis of methyl-1-(3-bromophenyl)-6-oxo-pyridazine-3-carboxylate

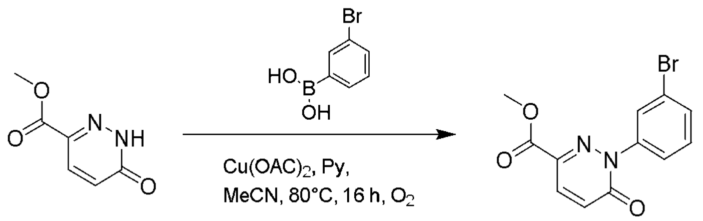


**Intermediate K-8**

A mixture of (3-bromophenyl)boronic acid (15.64 g, 77.86 mmol), methyl-6-oxo-1H-pyridazine-3-carboxylate (10 g, 64.88 mmol), $Cu(OAc)_2$ (3.54 g, 19.46 mmol) and Py (33.36 g, 421.74 mmol, 34.04 mL) in acetonitrile (200 mL) was degassed and purged with $O_2$ for 3 times, and then stirred at 80 °C for 16 h under $O_2$ atmosphere. The reaction mixture was poured into distilled water (100 mL) and extracted with EtOAc (100 mL×3). The combined organic layer was washed with brine (100 mL), dried over $Na_2SO_4$ and concentrated under reduced pressure to obtain a crude product, which was purified by silica gel column chromatography (100% DCM) to give methyl-1-(3-bromophenyl)-6-oxo-pyridazine-3-carboxylate (17.5 g, 51.96 mmol, 80.09% yield) as a white solid. $^1$H NMR (400 MHz, DMSO-$d_6$) δ = 7.93 (d, J = 9.6 Hz, 1H), 7.83 (t, J = 2.0 Hz, 1H), 7.71 (d, J = 8.0 Hz, 1H),7.60 (d, J = 8.8 Hz, 1H), 7.51 (t, J = 8.0 Hz, 1H), 7.17 (d, J = 10.0 Hz, 1H), 3.87 (s, 3H). LC/MS (ESI) m/z = 310.8 [M+H] +

Step 3: Synthesis of 1-[3-(3-methyltriazol-4-yl)phenyl]-6-oxopyridazine-3-carboxylic acid

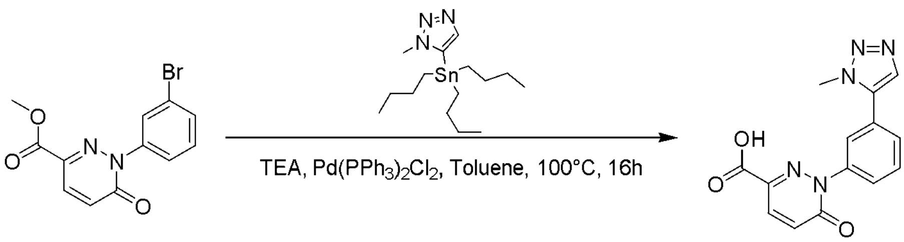


**Intermediate K-8**

To Intermediate K-8 (8.05 g, 26.04 mmol) in toluene (100 mL), tributyl-(3-methyltriazol-4-yl)stannane (14.54 g,

39.06 mmol), TEA (2.64 g, 26.04 mmol, 3.62 mL) and $Pd(PPh_3)_2Cl_2$ (1.83 g, 2.60 mmol) were added. The mixture was degassed and purged with $N_2$ for 3 times, and then stirred at 100 °C for 16 h under $N_2$ atmosphere. The reaction mixture was poured into water (200 mL) and extracted with EtOAc (100 mL×4). The combined organic layer was washed with brine (50 mL×3), dried over anhydrous $Na_2SO_4$ and concentrated under reduced pressure to obtain a crude product. The product was purified by silica gel column chromatography (8% MeOH in DCM) to give 1-[3-(3-methyltriazol-4-yl)phenyl]-6-oxo-pyridazine-3-carboxylic acid (2.96 g, 9.75 mmol, 37.45% yield) as yellow oil. LC/MS (ESI) m/z = 298.0 $[M+H]^+$

Step 4: Synthesis of N-[(1R)-1-[3-amino-5-(trifluoromethyl)phenyl]ethyl]-1-[3-(3-methyltriazol-4-yl)phenyl]-6-oxo-pyridazine-3-carboxamide

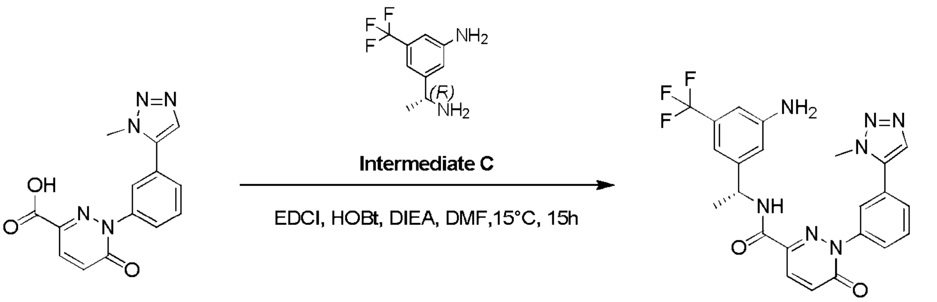


369

To a solution of 1-[3-(3-methyltriazol-4-yl)phenyl]-6-oxo-pyridazine-3-carboxylic acid (2.96 g, 9.96 mmol) and Intermediate C (2.40 g, 9.96 mmol) in DMF (40 mL), EDCI (2.86 g, 14.94 mmol), DIEA (3.86 g, 29.87 mmol, 5.20 mL) and HOBt (2.02 g, 14.94 mmol) were added. The mixture was degassed and purged with $N_2$ for 3 times, and then stirred at 15 °C for 15 h under $N_2$ atmosphere. The reaction mixture was poured into distilled water (100 mL) and extracted with EtOAc (50 mL×6). The combined organic layer was washed with brine (30 mL×5), dried over anhydrous $Na_2SO_4$ and concentrated under reduced pressure to obtain a crude product as yellow oil. The product was purified by silica gel column chromatography (75% EtOAc in PE) to give the compound of Example 369 (1.02 g, 21.19% yield) as a light yellow solid. $^1H$ NMR (400 MHz, DMSO-$d_6$) δ = 8.91 (d, J = 8.4 Hz, 1H), 7.99 (s, 1H), 7.96 (s, 1H), 7.92 (d, J = 9.6 Hz, 1H), 7.87 - 7.80 (m,1H), 7.75 - 7.68 (m, 2H), 7.18 (d, J = 9.6 Hz, 1H), 6.80 (br d, J = 13.2 Hz, 2H), 6.70 (s, 1H), 5.56 (s, 2H), 5.04 (quin, J = 7.2 Hz, 1H), 4.12 (s, 3H), 1.44 (d, J = 7.2 Hz, 3H). LC/MS (ESI) m/z = 484.4 $[M+H]^+$.

**Example 370: N-[(1R)-1-[3-(1,1-difluoro-2-hydroxy-ethyl)-2-fluoro-phenyl]ethyl]-1-[3-(3-methyltriazol-4-yl)phenyl]-6-oxo-pyridazine-3-carboxamide**

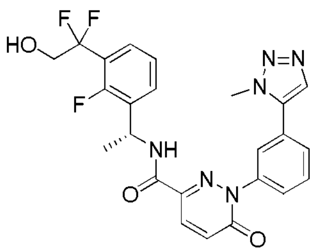

370

The compound of Example 370 was prepared in the same manner as in Example 369 by using Intermediate E. $^1H$ NMR (400 MHz, DMSO-$d_6$) δ = 9.02 (d, J = 8.0 Hz, 1H), 8.01 (s, 1H), 7.97 (s, 1H), 7.90 (d, J = 9.6 Hz, 1H), 7.87 - 7.81 (m,1H), 7.76 - 7.71 (m, 2H), 7.64 (br t, J = 7.2 Hz, 1H), 7.44 (br t, J = 6.8 Hz, 1H), 7.31 - 7.24 (m, 1H), 7.18 (d, J = 9.6 Hz, 1H),5.72 (t, J = 6.0 Hz, 1H), 5.41 (br t, J = 7.6 Hz, 1H), 4.14 (s, 3H), 3.91 (dt, J = 5.6, 14.0 Hz, 2H), 1.48 (d, J = 7.2 Hz, 3H) ; LC/MS (ESI) m/z: 499.4 $[M+H]^+$.

**Example 371: N-[(1R)-1-[3-amino-5-(trifluoromethyl)phenyl]ethyl]-1-[2-fluoro-5-(3-methyltriazol-4-yl)phenyl]-6-oxo-pyridazine-3-carboxamide**

Step 1: Synthesis of methyl-1-(2-fluoro-5-(1-methyl-1H-1,2,3-triazol-5-yl)phenyl)-6-oxo-1,6-dihydropyridazine-3-carboxylate

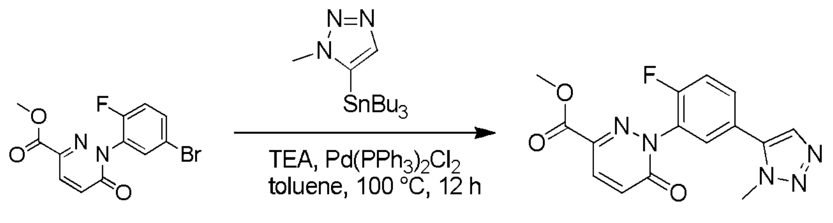


**Intermediate K-21**

Methyl-1-(2-fluoro-5-(1-methyl-1H-1,2,3-triazol-5-yl)phenyl)-6-oxo-1,6-dihydropyridazine-3-carboxylate was obtained by using a tributylstannyl derivative as an intermediate and changing the reagents and solvents accordingly in Step 2 of Example 318.

Steps 2 and 3: Synthesis of N-[(1R)-1-[3-amino-5-(trifluoromethyl)phenyl]ethyl]-1-[2 fluoro-5-(3-methyltriazol-4-yl)phenyl]-6-oxo-pyridazine-3-carboxamide

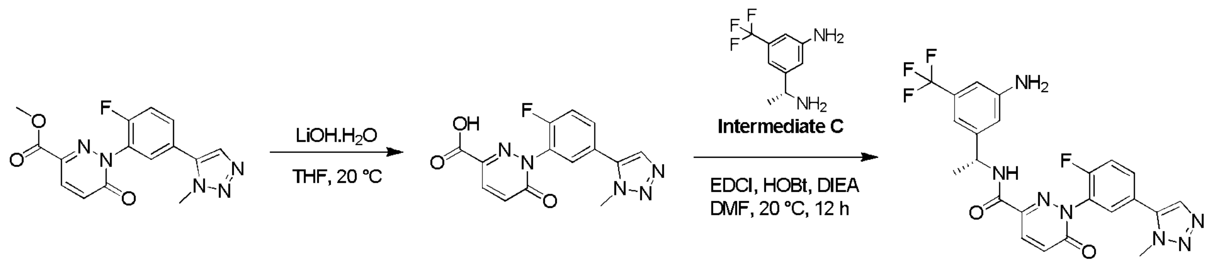


**371**

The compound of Example 371 (4.9 mg, 9.50 μmol, 9.98% yield) was obtained in the same manner as in Example 76 as a white solid. $^1$H NMR (400MHz, DMSO-$d_6$) δ = 8.93 (d, *J* =8.4 Hz, 1H), 8.03 - 7.92 (m, 3H), 7.85 (td, *J* =2.0, 8.8 Hz, 1H), 7.66 (t, *J* =9.2 Hz, 1H), 7.22 (d, *J* =10.0 Hz, 1H), 6.78 (br d, *J* =12.4 Hz, 2H), 6.69 (s, 1H), 5.54 (s, 2H), 5.03 (br t, *J* =7.2 Hz, 1H), 4.11 (s, 3H), 1.42 (d, *J* =7.2 Hz, 3H); MS (ESI) m/z = 502.4 [M+H]$^+$.

**Example 372: N-[(1R)-1-[3-(1,1-difluoro-2-hydroxy-ethyl)-2-fluoro-phenyl]ethyl]-1-[2-fluoro-5-(3-methyltriazol-4-yl)phenyl]-6-oxo-pyridazine-3-carboxamide**

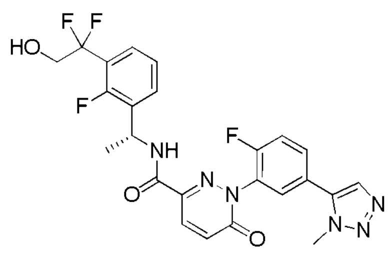

**372**

The compound of Example 372 (12.2 mg, 20.26% yield) was obtained as a white solid in the same manner as in Example 371 by replacing Intermediate C with Intermediate E. $^1$H NMR (400MHz, DMSO-$d_6$) δ = 9.05 (br d, *J* =8.0 Hz, 1H), 8.07 - 7.98 (m, 2H), 7.95 (d, *J* =10.0 Hz, 1H), 7.89 - 7.79 (m, 1H), 7.71 - 7.57 (m, 2H), 7.43 (br t, *J* =6.8 Hz, 1H), 7.32 - 7.19 (m, 2H), 5.71 (t, *J* =6.4 Hz, 1H), 5.40 (quin, *J* =7.2 Hz, 1H), 4.13 (s, 3H), 3.91 (dt, *J* =6.4, 14.3 Hz, 2H), 1.46 (br d, *J* =7.2 Hz, 3H); MS (ESI) m/z = 517.4 [M+H]$^+$).

**Example 373: N-[(1R)-1-[3-amino-5-(trifluoromethyl)phenyl]ethyl]-1-[3-(5-methyltetrazol-1-yl)phenyl]-6-oxo-pyridazine-3-carboxamide**

Step 1: Synthesis of methyl-1-[3-(5-methyltetrazol-1-yl)phenyl]-6-oxo-pyridazine-3-carboxylate

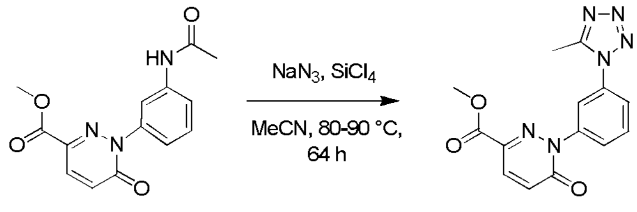


**Intermediate K-4**

A mixture of Intermediate K-4 (200 mg, 696.21 μmol), $NaN_3$ (400 mg, 6.15 mmol) and $SiCl_4$ (354.85 mg, 2.09 mmol, 239.77 μL) in dry acetonitrile (4 mL) was stirred at 20 °C for 16 h and then at 90 °C for additional 30 h. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The aqueous layer was adjusted to pH = 11 with aq. NaOH and then added with NaClO (10 mL). The product was purified by silica gel column chromatography (57% EtOAc in PE) to give methyl-1-[3-(5-methyltetrazol-1-yl)phenyl]-6-oxo-pyridazine-3-carboxylate (120 mg, 30.62% yield) as yellow oil. MS (ESI) m/z = 312.9 $[M+H]^+$.

Steps 2 and 3: Synthesis of N-[(1R)-1-[3-amino-5-(trifluoromethyl)phenyl]ethyl]-1-[2-fluoro-5-(3-methyltriazol-4-yl)phenyl]-6-oxo-pyridazine-3-carboxamide

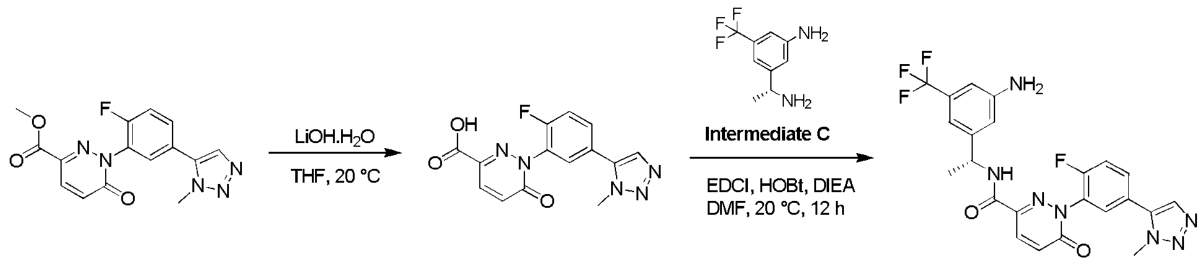


**373**

The compound of Example 373 (9.6 mg, 12.27% yield) was obtained as a white solid in the same manner as in Example 76. $^1H$ NMR (400MHz, DMSO-$d_6$) δ = 8.92 (d, *J* = 8.4 Hz, 1H), 8.15 - 8.08 (m, 1H), 8.04 - 7.97 (m, 1H), 7.92 (d, *J* = 10.0 Hz, 1H), 7.86 - 7.78 (m, 2H), 7.20 (d, *J* = 10.0 Hz, 1H), 6.80 (br d, *J* = 12.0 Hz, 2H), 6.70 (s, 1H), 5.55 (s, 2H), 5.04 (br t, *J* = 7.6 Hz, 1H), 2.61 (s, 3H), 1.45 (d, *J* = 7.2 Hz, 3H); MS (ESI) m/z = 485.1 $[M+H]^+$.

**Example 374: N-[(1R)-1-[3-amino-5-(trifluoromethyl)phenyl]ethyl]-1-[3-(5-methyltetrazol-1-yl)phenyl]-6-oxo-pyridine-3-carboxamide**

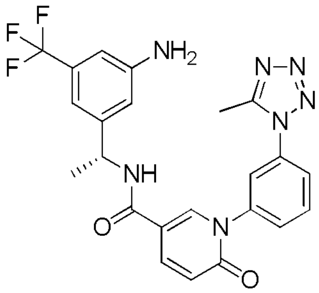

**374**

The compound of Example 374 (23.7 mg, 53.11% yield) was obtained as a white solid in the same manner as in Example 373 by using a starting material corresponding to the structure of the desired compound based on Preparation Example 7. $^1$H NMR (400 MHz, DMSO-$d_6$) δ = 8.60 (d, *J* = 8.0 Hz, 1H), 8.44 (d, *J* = 2.4 Hz, 1H), 8.01 - 7.93 (m, 2H), 7.86 - 7.77 (m, 3H), 6.76 (s, 2H), 6.69 (s, 1H), 6.58 (d, *J* = 9.6 Hz, 1H), 5.55 (s, 2H), 5.02 (br t, *J* = 7.2 Hz, 1H), 2.61 (s, 3H), 1.40 (d, *J* = 7.2 Hz, 3H); MS (ESI) m/z = 484.4 [M+H]$^+$.

**Example 375: N-[(1R)-1-[3-amino-5-(difluoromethyl)phenyl]ethyl]-1-(2-fluorophenyl)-6-oxo-pyridazine-3-carboxamide**

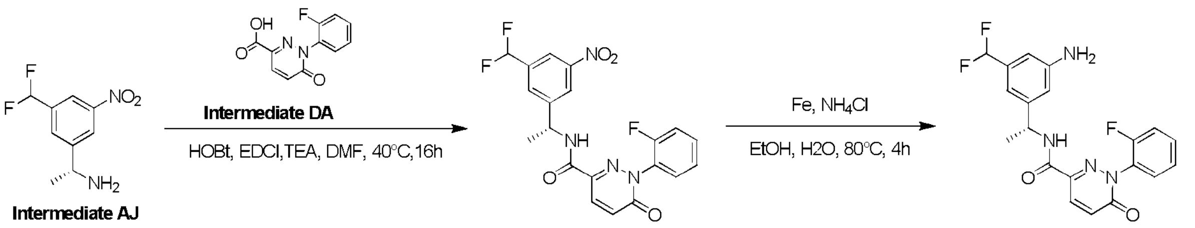


**375**

The compound of Example 375 (11.9 mg, 30.97% yield) was obtained as a white solid by reacting Intermediate AJ with Intermediate DA in the same manner as in Steps 3 and 4 of Example 89. H NMR (400 MHz, DMSO-$d_6$) δ = 8.88 (br d, *J* = 8.4 Hz, 1H), 7.94 (d, *J* = 10.0 Hz, 1H), 7.68 (br t, *J* = 7.2 Hz, 1H), 7.62 - 7.54 (m, 1H), 7.48 - 7.38 (m, 2H), 7.18 (d, *J* = 10.0 Hz, 1H), 6.95 (s, 1H), 6.81 (s, 1H), 6.68 - 6.65 (m, 2H), 6.57 (s, 1H), 5.36 (s, 2H), 5.00 (quin, *J* = 7.2 Hz, 1H), 1.41 (br d, *J*= 6.8 Hz, 3H); MS (ESI) m/z = 403.1 [M+H]$^+$.

**Example 376: N-[(1R)-1-[3-amino-5-(trifluoromethyl)phenyl]ethyl]-1-(3-isoxazol-3-ylphenyl)-6-oxo-pyridazine-3-carboxamide**

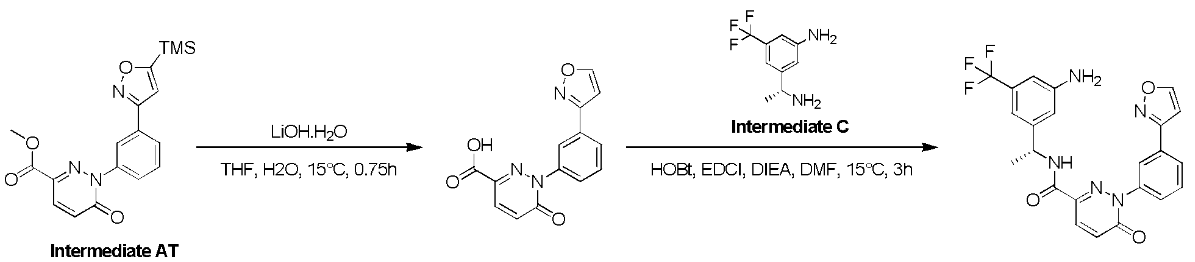


**376**

The compound of Example 376 (7.9 mg, 12.40% yield) was obtained as a white solid in the same manner as in Example 76. $^1$H NMR (400MHz, DMSO-$d_6$) δ = 9.06 (d, *J* = 1.6 Hz, 1H), 8.92 (d, *J* = 8.4 Hz, 1H), 8.18 (t, *J* = 1.6 Hz, 1H), 8.01 (td, *J* = 1.2, 8.0 Hz, 1H), 7.92 (d, *J* = 9.6 Hz, 1H), 7.84 - 7.77 (m, 1H), 7.74 - 7.66 (m, 1H), 7.21 (d, *J* = 1.6 Hz, 1H), 7.18 (d, *J* = 9.6 Hz, 1H), 6.82 (s, 1H), 6.78 (s, 1H), 6.70 (s, 1H), 5.54 (s, 2H), 5.04 (quin, *J* = 7.2 Hz, 1H), 1.44 (d, *J* = 7.2 Hz, 3H); MS (ESI) m/z = 470.3 [M+H]$^+$.

**Example 377: N-[(1R)-1-[3-amino-5-(trifluoromethyl)phenyl]ethyl]-1-[3-(5-methyltriazol-1-yl)phenyl]-6-oxo-pyridine-3-carboxamide**

Step 1: Synthesis of ethyl-1-(3-(5-methyl-1H-1,2,3-triazol-1-yl)phenyl)-6-oxo-1,6-dihydropyridine-3-carboxylate

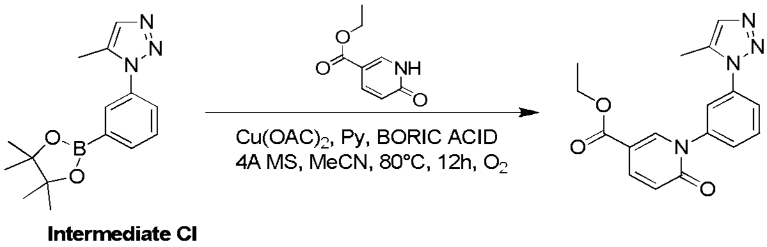


The compound of ethyl-1-(3-(5-methyl-1H-1,2,3-triazol-1-yl)phenyl)-6-oxo-1,6-dihydropyridine-3-carboxylate was obtained in the same manner as in Step 1 of Example 21.

Steps 2 and 3: Synthesis of N-[(1R)-1-[3-amino-5-(trifluoromethyl)phenyl]ethyl]-1-[3-(5 -methyltriazol-1-yl)phenyl]-6-oxo-pyridine-3-carboxamide

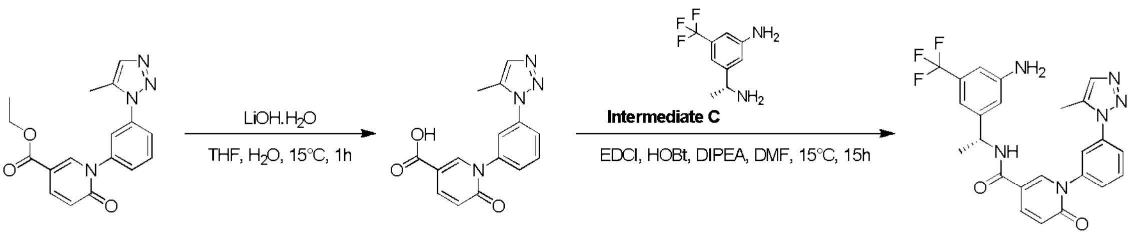


377

The compound of Example 377 (7.7 mg, 11.82% yield) was obtained as a white solid in a similar manner to Example 76. $^1$H NMR (400MHz, DMSO-$d_6$) $\delta$ = 8.59 (d, *J* = 7.6 Hz, 1H), 8.44 (d, *J* = 2.4 Hz, 1H), 7.98 (dd, *J* = 2.8, 9.6 Hz, 1H), 7.84 (t, *J* = 1.6 Hz, 1H), 7.79 - 7.76 (m, 1H), 7.75 - 7.70 (m, 2H), 6.76 (s, 2H), 6.69 (s, 1H), 6.57 (d, *J* = 9.6 Hz, 1H), 5.54 (s, 2H), 5.02 (quin, *J* = 7.2 Hz, 1H), 2.38 (s, 3H), 1.40 (d, *J* = 6.8 Hz, 3H); MS (ESI) m/z = 483.4 [M+H]$^+$.

**Example 378: N-[(1R)-1-[3-amino-5-(trifluoromethyl)phenyl]ethyl]-1-[3-(5-methyltriazol-1-yl)phenyl]-6-oxo-pyridazine-3-carboxamide**

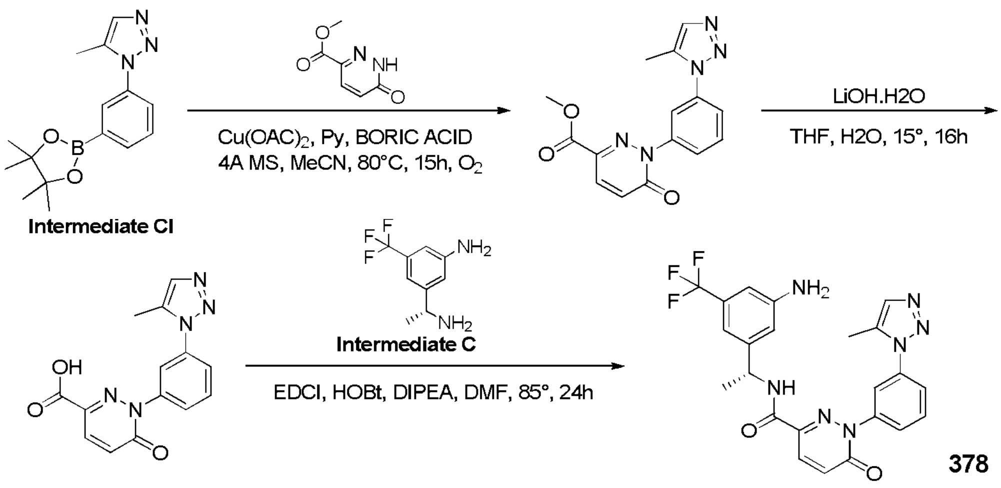


378

The compound of Example 378 (11.1 mg, 22.75% yield) was obtained as a white solid in the same manner as in Example 377 by using methyl-6-oxo-1,6-dihydropyridazine-3-carboxylate instead of ethyl-6-oxo-1,6-dihydropyridine-3-carboxylate in Step 1. $^1$H NMR (400 MHz, DMSO-$d_6$) $\delta$ = 8.92 (d, *J* = 8.4 Hz, 1H), 8.02 (t, *J* = 2.0 Hz, 1H), 7.97 - 7.86 (m, 2H), 7.82 - 7.76 (m, 1H), 7.76 - 7.68 (m, 2H), 7.19 (d, *J* = 9.6 Hz, 1H), 6.80 (br d, *J* = 11.2 Hz, 2H), 6.70 (s, 1H), 5.54 (s, 2H), 5.04 (quin, *J* = 7.2 Hz, 1H), 2.38 (s, 3H), 1.44 (d, *J* = 7.2 Hz, 3H); MS (ESI) m/z = 484.4 [M+H]$^+$.

**Example 379: 4-amino-N-[(1R)-1-[3-(1,1-difluoro-2-hydroxy-ethyl)-2-fluorophenyl]ethyl]-1-(2-fluorophenyl)-6-oxo-pyridazine-3-carboxamide**

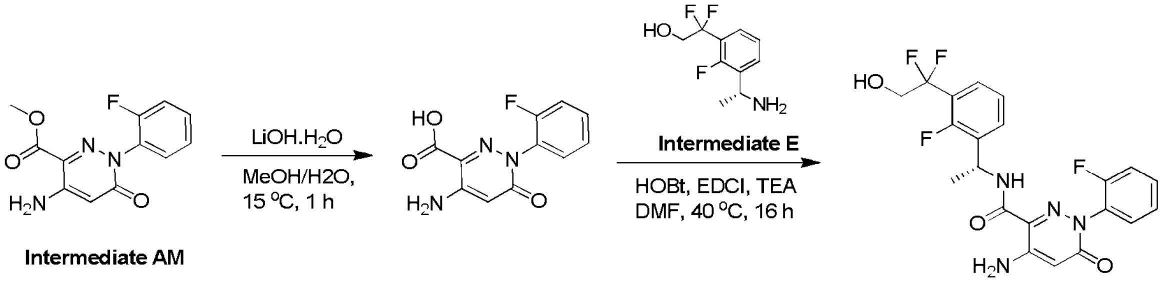


Intermediate AM

379

The compound of Example 379 (7.8 mg, 7.12% 5-Step yield) was obtained as a white solid in a similar manner to Example 76. $^1$H NMR (400MHz, DMSO-$d_6$) δ = 8.94 (d, *J* = 8.0 Hz, 1H), 7.49-7.65 (m, 3H), 7.33-7.45 (m, 3H), 7.19-7.30 (m, 3H), 5.78 (s, 2H), 5.36 (q, *J* = 7.2 Hz, 1H), 3.91 (t, *J* = 14.4 Hz, 2H), 1.44 (d, *J* = 7.2 Hz, 3H); MS (ESI) m/z = 451.1 [M+H]$^+$.

**Example 380: [2-[3-[(1R)-1-[[4-acetamido-1-(2-fluorophenyl)-6-oxo-pyridazine-3-carbonyl]amino]ethyl]-2-fluoro-phenyl]-2,2-difluoro-ethyl] acetate**

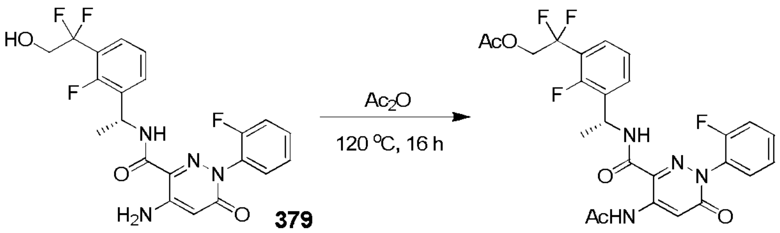


379 380

A mixture of the compound of Example 379 (76 mg, 168.74 μmol) in $Ac_2O$ (5 mL) was stirred at 120 °C for 16 h. The mixture was neutralized with sat. aq. $NaHCO_3$ and extracted with EtOAc (10 mL × 3). The combined organic layer was washed with brine (10 mL), dried over anhydrous $Na_2SO_4$ and concentrated under reduced pressure to obtain a crude product. The product was purified by prep-HPLC (column: Phenomenex C18 75*30mm*3μm; mobile phase: [water($NH_3H_2O$+$NH_4HCO_3$)-ACN];B%: 37%-67%, 10min). The desired fraction was concentrated under reduced pressure, and the remaining solvent was removed by lyophilization to give the compound of Example 380 (5.7 mg, 6.19% yield) as a white solid. $^1$H NMR (400MHz, DMSO-$d_6$) δ = 11.37 (s, 1H), 9.31 (d, *J* = 8.0 Hz, 1H), 7.79 (s, 1H), 7.67 (td, *J* = 7.6, 1.6 Hz, 2H), 7.55-7.62 (m, 1H), 7.39-7.52 (m, 3H), 7.30-7.36 (m, 1H), 5.42 (q, *J* = 7.2 Hz, 1H), 4.61-4.76 (m, 2H), 2.17 (s, 3H), 2.04 (s, 3H), 1.48 (d, *J* = 7.2 Hz, 3H); MS (ESI) m/z = 535.3 [M+H]$^+$.

**Example 381: 4-amino-N-[(1R)-1-[3-amino-5-(trifluoromethyl)phenyl]ethyl]-1-(2-fluorophenyl)-6-oxo-pyridazine-3-carboxamide**

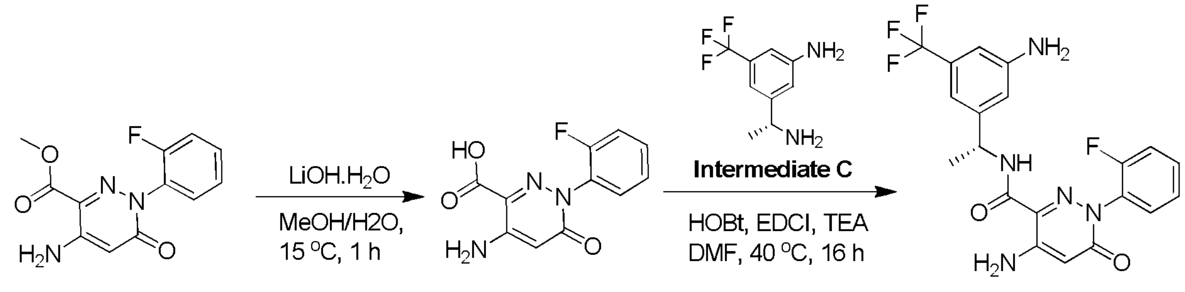


**381**

**Intermediate AM**

The compound of Example 381 (12.8 mg, 18.13% yield) was obtained as a white solid in the same manner as in Example 379 by replacing Intermediate E with Intermediate C. $^1H$ NMR (400MHz, DMSO-$d_6$) $\delta$ = 8.84 (d, J = 8.4 Hz, 1H), 7.46-7.62 (m, 2H), 7.32-7.41 (m, 2H), 7.25 (s, 2H), 6.77 (d, *J* = 11.2 Hz, 2H), 6.70 (s, 1H), 5.79 (s, 1H), 5.56 (s, 2H), 4.94-5.04 (m, 1H), 1.41 (d, *J* = 7.2 Hz, 3H), 1.33-1.49 (m, 1H); MS (ESI) m/z = 436.3 $[M+H]^+$.

**Example 382: 4-acetamido-N-[(1R)-1-[3-amino-5-(trifluoromethyl)phenyl]ethyl]-1-(2-fluorophenyl)-6-oxo-pyridazine-3-carboxamide**

Steps 1 and 2: Synthesis of 4-amino-1-(2-fluorophenyl)-N-[(1R)-1-[3-nitro-5-(trifluoromethyl)phenyl]ethyl]-6-oxo-pyridazine-3-carboxamide

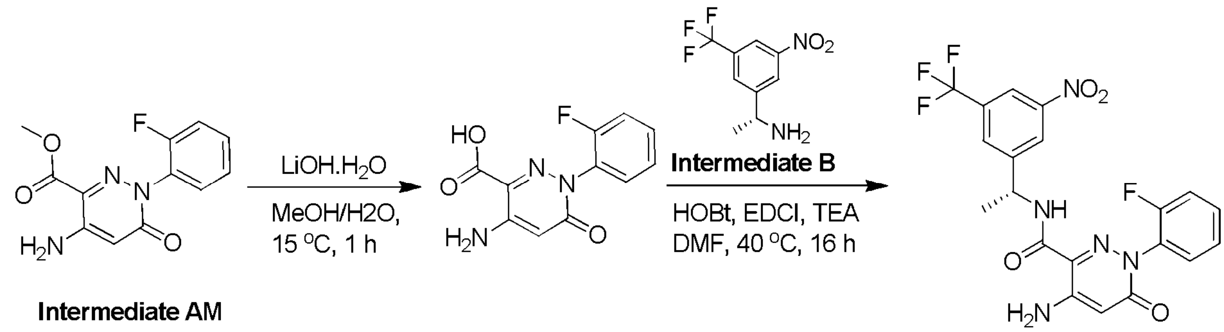


**Intermediate AM**

4-amino-1-(2-fluorophenyl)-N-[(1R)-1-[3-nitro-5-(trifluoromethyl)phenyl]ethyl]-6-oxo-pyridazine-3-carboxamide (36 mg, 13.46% yield) was obtained as a white solid in the same manner as in Example 379 by replacing Intermediate E with Intermediate B. MS (ESI) m/z = 466.2 $[M+H]^+$.

Step 3: Synthesis of 4-acetamido-1-(2-fluorophenyl)-N-[(1R)-1-[3-nitro-5-(trifluoromethyl)phenyl]ethyl]-6-oxo-pyridazine-3-carboxamide

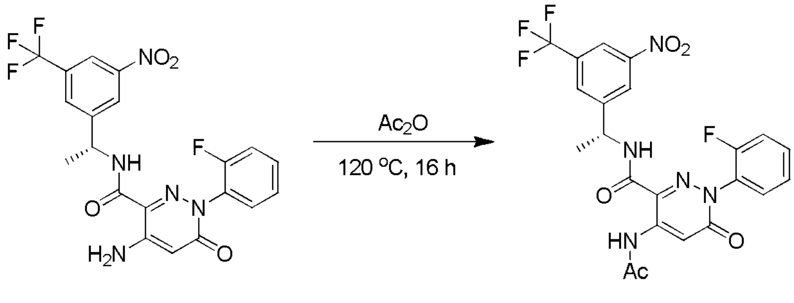


A mixture of 4-amino-1-(2-fluorophenyl)-N-[(1R)-1-[3-nitro-5-(trifluoromethyl)phenyl]ethyl]-6-oxo-pyridazine-3-carboxamide (36 mg, 77.36 μmol) in $Ac_2O$ (3 mL) was stirred at 120 °C for 16 h. The mixture was concentrated under reduced pressure to give 4-acetamido-1-(2-fluorophenyl)-N-[(1R)-1-[3-nitro-5-(trifluoromethyl)phenyl]ethyl]-6-oxo-pyridazine-3-carboxamide (crude product, 45 mg) as a yellow solid. MS (ESI) m/z = 343.2 [M+H]$^+$.

Step 4: Synthesis of 4-acetamido-N-[(1R)-1-[3-amino-5-(trifluoromethyl)phenyl]ethyl]-1-(2-fluorophenyl)-6-oxo-pyridazine-3-carboxamide

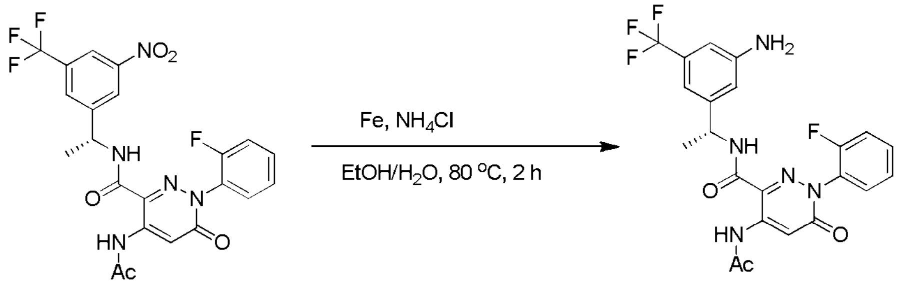


**382**

A mixture of 4-acetamido-1-(2-fluorophenyl)-N-[(1R)-1-[3-nitro-5-(trifluoromethyl)phenyl]ethyl]-6-oxo-pyridazine-3-carboxamide (45 mg, 88.69 μmol), Fe (49.53 mg, 886.89 μmol) and $NH_4Cl$ (47.44 mg, 886.89 μmol) in EtOH (2.5 mL) and $H_2O$ (0.5 mL) was stirred at 80 °C for 2 h. The crude product was filtered to obtain a filtrate, and the filtrate was concentrated under reduced pressure to obtain a crude product. The residue was purified by prep-HPLC (column: Phenomenex C18 75*30 mm*3 μm; mobile phase: [water($NH_3H_2O$+$NH_4HCO_3$)-ACN]; B%: 30%-60%, 14 min). The desired fraction was concentrated under reduced pressure, and the remaining solvent was removed by lyophilization to give the compound of Example 382 (6.2 mg, 20.73% 2-steps yield) as a white solid. $^1$H NMR (400MHz, DMSO-$d_6$) δ = 11.47 (s, 1H), 9.22 (d, *J* = 7.6 Hz, 1H), 7.79 (s, 1H), 7.51-7.69 (m, 2H), 7.35-7.49 (m, 2H), 6.79 (d, *J* = 15.6 Hz, 2H), 6.71 (s, 1H), 5.57 (s, 2H), 5.05 (t, *J= 7.2* Hz, 1H), 2.18 (s, 3H), 1.44 (d, *J* = 7.2 Hz, 3H); MS (ESI) m/z = 478.2 [M+H]$^+$.

**Example 383: N-[(1R)-1-[3-amino-5-(trifluoromethyl)phenyl]ethyl]-1-(2-fluorophenyl)-4-(methylamino)-6-oxo-pyridazine-3-carboxamide**

Steps 1 and 2: Synthesis of tert-butyl N-[3-[[(1R)-1-[3-amino-5-(trifluoromethyl)phenyl]ethyl]carbamoyl]-1-(2-fluoro-phenyl)-6-oxo-pyridazin-4-yl]-N-methylcarbamate

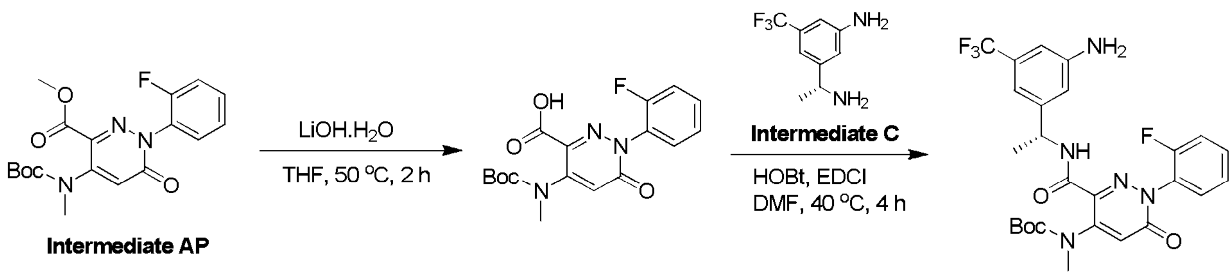


Tert-butyl N-[3-[[(1R)-1-[3-amino-5-(trifluoromethyl)phenyl]ethyl]carbamoyl]-1-(2-fluorophenyl)-6-oxo-pyridazin-4-yl]-N-methyl-carbamate (139 mg, 65.62% yield) was obtained as a yellow solid in the same manner as in Example 76. MS (ESI) m/z = 364.1 [M+H]$^+$.

Step 3: Synthesis of N-[(1R)-1-[3-amino-5-(trifluoromethyl)phenyl]ethyl]-1-(2-fluorophenyl)-4-(methylamino)-6-oxo-pyridazine-3-carboxamide

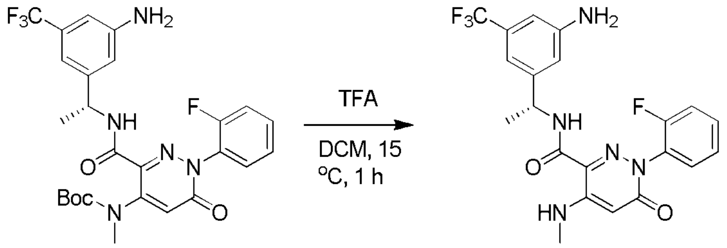


383

The compound of Example 383 (21.3 mg, 18.00% 2-Step yield) was obtained as a white solid in the same manner as in Step 5 of Example 79. $^1$H NMR (400MHz, DMSO-$d_6$) $\delta$ = 8.89 (d, $J$ = 8.0 Hz, 1H), 7.98-8.10 (m, 1H), 7.59 (td, $J$ = 7.6, 1.6 Hz, 1H), 7.48-7.55 (m, 1H), 7.32-7.41 (m, 2H), 6.77 (d, $J$ = 11.6 Hz, 2H), 6.69 (s, 1H), 5.64 (s, 1H), 5.55 (s, 2H), 4.98 (q, $J$ = 7.2 Hz, 1H), 2.77 (d, $J$ = 4.8 Hz, 3H), 1.41 (d, $J$ = 7.2 Hz, 3H); MS (ESI) m/z = 450.2 $[M+H]^+$).

**Example 384: N-[(1R)-1-[3-amino-5-(trifluoromethyl)phenyl]ethyl]-1-(2-fluorophenyl)-4-(methanesulfonamido)-6-oxo-pyridazine-3-carboxamide**

Step 1: Synthesis of 1-(2-fluorophenyl)-4-(methanesulfonamido)-N-[(1R)-1-[3-nitro-5-(trifluoromethyl)phenyl]ethyl]-6-oxo-pyridazine-3-carboxamide

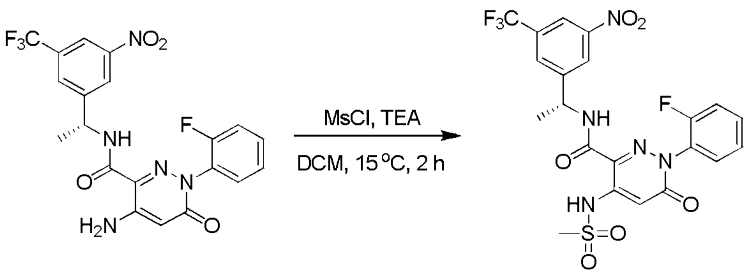


To a mixture of 4-amino-1-(2-fluorophenyl)-N-[(1R)-1-[3-nitro-5-(trifluoromethyl)phenyl]ethyl]-6-oxo-pyridazine-3-carboxamide (35 mg, 75.21 µmol), which was obtained from Step 2 of Example 382, and TEA (76.11 mg, 752.11 µmol) in DCM (3 mL) was added MsCl (86.16 mg, 752.11 µmol) at 0 °C, and the mixture was stirred at 15 °C for 2 h. After the addition of methanesulfonyl chloride (129.23 mg, 1.13 mmol) and DMAP (2.76 mg, 22.56 µmol) at 0°C, the mixture was stirred at 15 °C for 2 h. The mixture was diluted with water (10 mL) and extracted with EtOAc (10 mL × 3). The combined organic layer was washed with brine (10 mL), dried over anhydrous $Na_2SO_4$ and concentrated under reduced pressure to give 1-(2-fluorophenyl)-4-(methanesulfonamido)-N-[(1R)-1-[3-nitro-5-trifluoromethyl)phenyl]ethyl]-6-oxo-pyridazine-3-carboxamide (crude product, 44 mg) as a yellow solid. MS (ESI) m/z = 544.2 $[M+H]^+$.

Step 2: Synthesis of N-[(1R)-1-[3-amino-5-(trifluoromethyl)phenyl]ethyl]-1-(2-fluorophenyl)-4-(methanesulfonamido)-6-oxo-pyridazine-3-carboxamide

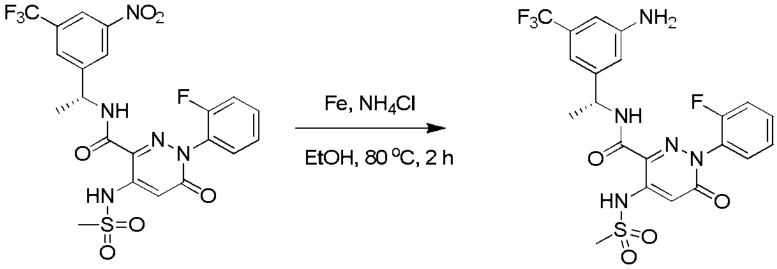


384

The compound of Example 384 (4.3 mg, 10.02% 2-Step yield) was obtained as a white solid in the same manner as in Step 4 of Example 382. $^1$H NMR (400MHz, DMSO-$d_6$) $\delta$ = 8.21 (d, $J$ = 7.6 Hz, 1H), 8.15-8.24 (m, 1H), 7.52 (s, 2H), 7.32-7.44 (m, 2H), 6.81-7.30 (m, 3H), 6.78 (s, 1H), 6.70 (s, 1H), 5.56 (s, 2H), 4.98 (s, 1H), 3.18 (s, 3H), 1.41 (d, $J$ = 6.8 Hz,

3H); MS (ESI) m/z = 514.3 [M+H]$^+$.

**Example 385: N-[(1R)-1-[3-amino-5-(trifluoromethyl)phenyl]ethyl]-1-(2-methylthiazol-5-yl)-6-oxo-pyridazine-3-carboxamide**

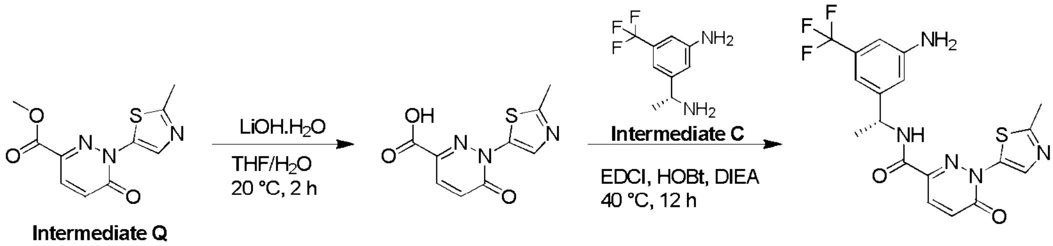


Intermediate Q

385

The compound of Example 385 (8.4 mg, 15.03% yield) was prepared in the same manner as in Example 76. $^1$H NMR (400MHz, DMSO-$d_6$) $\delta$ = 9.05 (d, J = 8.4 Hz, 1H), 8.66 (s, 1H), 7.94 (d, J = 9.6 Hz, 1H), 7.27 (d, J = 9.6 Hz, 1H), 6.87 (s, 1H), 6.83 (s, 1H), 6.72 (s, 1H), 5.57 (s, 2H), 5.17 - 5.06 (m, 1H), 2.66 (s, 3H), 1.52 (d, J = 7.2 Hz, 3H); MS (ESI) m/z = 424.3 [M+H]$^+$

**Example 386 to Example 388**

The compounds in the following table were prepared in the same manner as in Example 385 by using starting materials corresponding to the respective structures of the desired compounds based on Preparation Example 7.

[Table 26]

| No. | Structure/Name | Spectral Data |
|---|---|---|
| 386 | 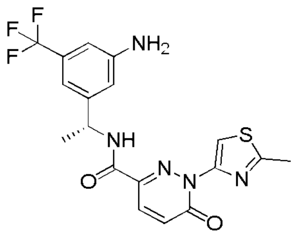 N-[(1R)-1-[3-amino-5-(trifluoromethyl)phenyl]ethyl]-1-(2-methylthiazol-4-yl)-6-oxo-pyridazine-3-carboxamide | $^1$H NMR (400MHz, DMSO-$d_6$) $\delta$ = 8.87 (d, J = 8.4 Hz, 1H), 7.90 (d, J = 9.6 Hz, 1H), 7.85 (s, 1H), 7.15 (d, J = 9.6 Hz, 1H), 6.82 (s, 1H), 6.77 (s, 1H), 6.70 (s, 1H), 5.55 (s, 2H), 5.02 (quin, J = 7.2 Hz, 1H), 2.70 (s, 3H), 1.44 (d, J = 7.2 Hz, 3H); MS (ESI) m/z = 424.3 [M+H]$^+$ |
| 387 | 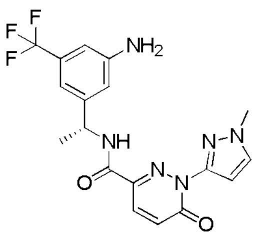 N-[(1R)-1-[3-amino-5-(trifluoromethyl)phenyl]ethyl]-1-(1-methylpyrazol-3-yl)-6-oxo-pyridazine-3-carboxamide | $^1$H NMR (400MHz, DMSO-$d_6$) $\delta$ = 8.82 (d, J = 8.0 Hz, 1H), 7.88 (d, J = 9.6 Hz, 1H), 7.82 (d, J = 2.4 Hz,1H), 7.12 (d, J = 9.6 Hz, 1H), 6.83 (s, 1H), 6.78 (s, 1H), 6.70 (s, 1H), 6.50 (d, J = 2.4 Hz, 1H), 5.55 (s, 2H), 5.02 (quin, J = 7.2 Hz, 1H), 3.88 (s, 3H), 1.44 (d, J = 7.2 Hz, 3H); MS (ESI) m/z = 407.3 [M+H]$^+$ |

(continued)

| No. | Structure/Name | Spectral Data |
|---|---|---|
| 388 | 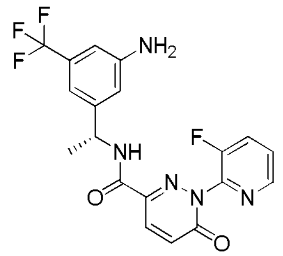 N-[(1R)-1-[3-amino-5-(trifluoromethyl)phenyl]ethyl]-1-(2-methylthiazol-4-yl)-6-oxo-pyridazine-3-carboxamide | $^1$H NMR (400MHz, DMSO-$d_6$) δ = 8.87 (d, J = 8.4 Hz, 1H), 7.90 (d, J = 9.6 Hz, 1H), 7.85 (s, 1H), 7.15 (d, J = 9.6 Hz, 1H), 6.82 (s, 1H), 6.77 (s, 1H), 6.70 (s, 1H), 5.55 (s, 2H), 5.02 (quin, J = 7.2 Hz, 1H), 2.70 (s, 3H), 1.44 (d, J = 7.2 Hz, 3H); MS (ESI) m/z = 422.2 [M+H]$^+$ |

**Example 389: N-[(1R)-1-[3-amino-5-(trifluoromethyl)phenyl]ethyl]-1-[4-fluoro-3-(3-methyltriazol-4-yl)phenyl]-6-oxo-pyridazine-3-carboxamide**

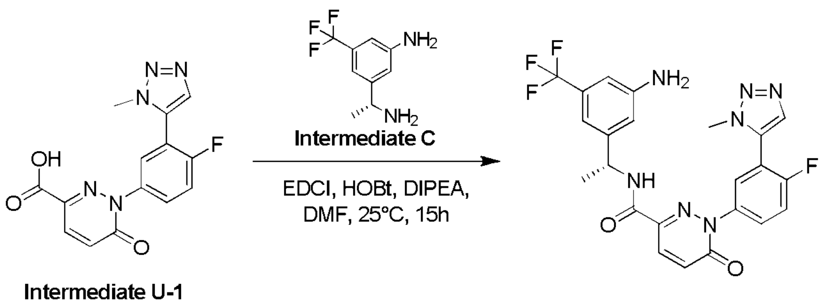


389

The compound of Example 389 was prepared in a similar manner to Step 2 of Example 76. $^1$H NMR (400MHz, DMSO-$d_6$) δ = 8.89 (d, *J* = 8.4 Hz, 1H), 8.03 - 7.96 (m, 2H), 7.96 - 7.87 (m, 2H), 7.63 (t, *J* = 9.2 Hz, 1H), 7.18 (d, *J* = 9.6 Hz, 1H), 6.80 (br d, *J* = 11.2 Hz, 2H), 6.70 (s, 1H), 5.55 (s, 2H), 5.11 - 4.95 (m, 1H), 4.04 (s, 3H), 1.45 (d, *J* = 7.2 Hz, 3H); MS (ESI) m/z = 502.3 [M+H]$^+$.

**Example 390: N-[(1R)-1-[3-(1,1-difluoro-2-hydroxy-ethyl)-2-fluoro-phenyl]ethyl]-1-[4-fluoro-3-(3-methyltriazole-4)-yl)phenyl]-6-oxo-pyridazine-3-carboxamide**

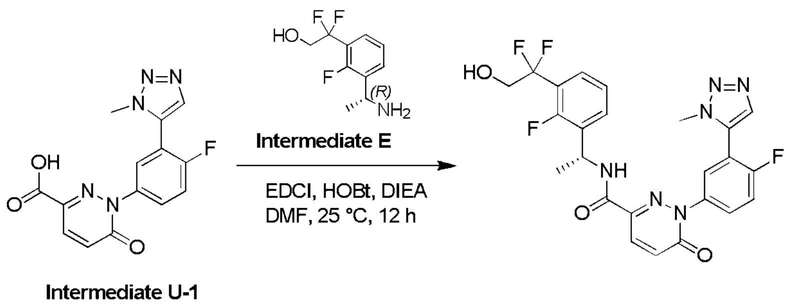


390

The compound of Example 390 was prepared in the same manner as in Example 389 by using Intermediate E. $^1$H NMR (400MHz, DMSO-$d_6$) δ = 8.99 (d, J = 8.0 Hz, 1H), 8.07 - 7.98 (m, 2H), 7.96 (ddd, J = 2.8, 4.8, 8.8 Hz, 1H), 7.89 (d, J = 9.6 Hz, 1H), 7.68 - 7.61 (m, 2H), 7.44 (t, J = 7.2 Hz, 1H), 7.27 (t, J = 7.6 Hz, 1H), 7.18 (d, J = 9.6 Hz, 1H), 5.71 (br t, J = 6.0 Hz, 1H), 5.41 (quin, J = 7.2 Hz, 1H), 4.06 (s, 3H), 3.91 (dt, J = 5.2, 14.4 Hz, 2H), 1.48 (d, J = 7.2 Hz, 3H); MS (ESI) m/z = 517.4 [M+H]$^+$.

**Example 391: N-[(1R)-1-[3-amino-5-(trifluoromethyl)phenyl]ethyl]-1-[3-fluoro-5-(3-methyltriazol-4-yl)phenyl]-6-oxo-pyridazine-3-carboxamide**

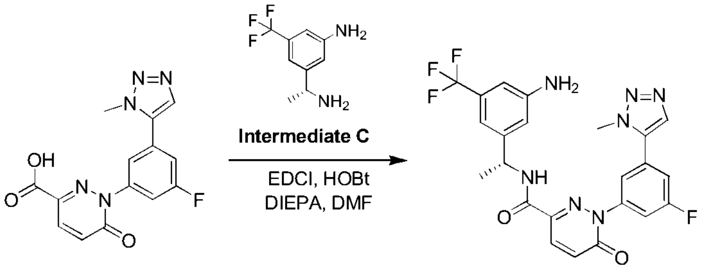


Intermediate U-2

391

The compound of Example 391 was prepared in the same manner as in Example 389 by using Intermediated U-2. $^1$H NMR (400 MHz, DMSO-$d_6$): δ = 8.92 (d, *J* = 8.4 Hz, 1H), 8.04 (s, 1H), 7.92 (d, *J* = 9.6 Hz, 1H), 7.88 (t, *J* = 1.6 Hz, 1H), 7.84 (td, *J* = 2.0, 9.6 Hz, 1H), 7.68 (td, *J* = 2.0, 9.6 Hz, 1H), 7.20 (d, *J* = 9.6 Hz, 1H), 6.80 (br d, *J* = 12.0 Hz, 2H), 6.71 (s, 1H), 5.54 (s, 2H), 5.09 - 5.02 (m, 1H), 4.14 (s, 3H), 1.46 (d, *J* = 7.2 Hz, 3H); MS (ESI) m/z = 502.4 [M+H] $^+$.

**Example 392: N-[(1R)-1-[3-amino-5-(trifluoromethyl)phenyl]ethyl]-1-[5-(3-methyltriazol-4-yl)-3-pyridyl]-6-oxo-pyridazine-3-carboxamide**

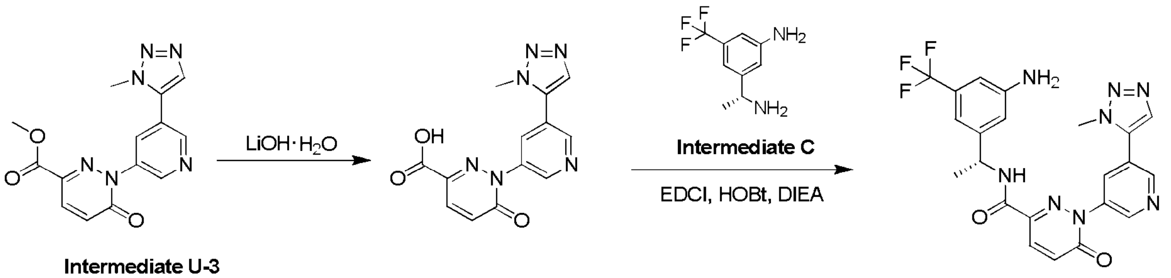


Intermediate U-3

392

The compound of Example 392 was prepared in the same manner as in Example 76 by using Intermediate U-3 and Intermediate C. $^1$H NMR (400 MHz, METHANOL-$d_4$): δ = 9.09 (d, *J* = 2.4 Hz, 1H), 8.85 (d, *J* = 2.0 Hz, 1H), 8.48 (t, *J* = 2.4 Hz, 1H), 8.05 - 8.02 (m, 2H), 7.21 (d, *J*= 9.6 Hz, 1H), 6.90 (br d, *J* = 6.0 Hz, 2H), 6.81 (s, 1H), 5.15 (q, *J* = 7.2 Hz, 1H), 4.19 (s, 3H), 1.54 (d, *J* = 7.2 Hz, 3H); MS (ESI) m/z = 485.4 [M+H] $^+$

**Example 393: N-[(1R)-1-[3-(1,1-difluoro-2-hydroxy-ethyl)-2-fluoro-phenyl]ethyl]-6-oxo-1-[3-[3-(trideuteriomethyl)triazol-4-yl]phenyl]pyridazine-3-carboxamide**

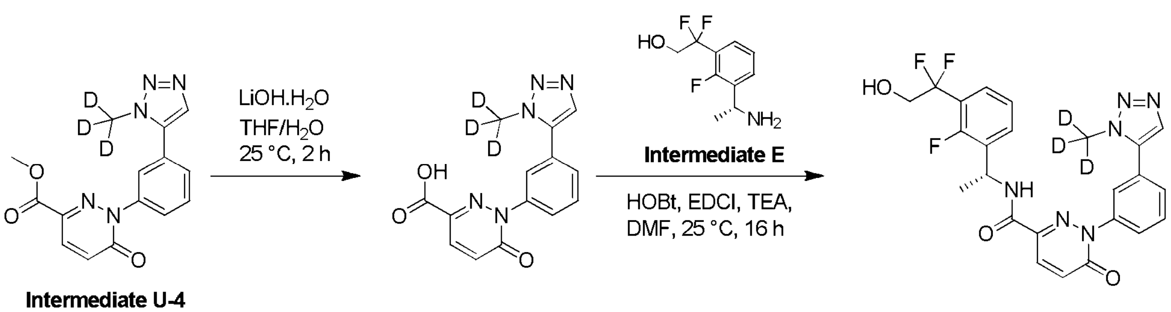


Intermediate U-4

393

The compound of Example 373 was prepared in the same manner as in Steps 2 and 3 of Example 21 by using

Intermediate U-4 and Intermediate E. $^1$H NMR (400MHz, DMSO-$d_6$) δ = 9.02 (d, J = 8.0 Hz, 1H), 8.01 (s, 1H), 7.97 (s, 1H), 7.90 (d, J = 10.0 Hz, 1H), 7.85 (dt, J = 2.4, 4.8 Hz, 1H), 7.76 - 7.71 (m, 2H), 7.68 - 7.60 (m, 1H), 7.48 - 7.40 (m, 1H), 7.31 - 7.24 (m, 1H), 7.18 (d, J = 10.0 Hz, 1H), 5.72 (t, J = 6.4 Hz, 1H), 5.41 (t, J = 7.6 Hz, 1H), 4.04 - 3.79 (m, 2H), 1.48 (d, J = 7.2 Hz, 3H); MS (ESI) m/z = 502.4 [M+H]$^+$.

**Example 394: N-[(1S)-1-[4-amino-6-(trifluoromethyl)-2-pyridyl]ethyl]-1-(2-fluorophenyl)-6-oxo-pyridazine-3-carboxamide**

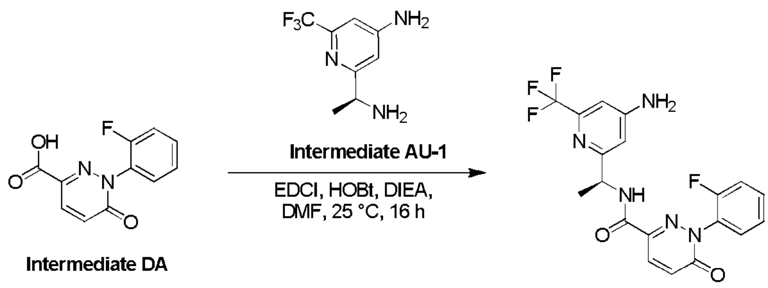


394

The compound of Example 394 (11.5 mg, 8.2% yield) was obtained as a yellow solid in the same manner as in Step 2 of Example 76 by using Intermediate AU-1. $^1$H NMR (400 MHz, $CDCl_3$) δ = 8.48 (d, J = 7.2 Hz, 1H), 8.05 (d, J = 9.6 Hz, 1H), 7.56 - 7.45 (m, 2H), 7.37 - 7.27 (m, 2H), 7.25 (s, 1H), 7.11 (d, J = 9.6 Hz, 1H), 6.78 (d, J = 2.0 Hz, 1H), 6.60 (d, J = 1.6 Hz, 1H), 5.17 - 5.06 (m, 1H), 4.48 (s, 2H), 1.54 (d, J = 6.8 Hz, 3H); MS (EI) m/z: 422.2 [M+H]$^+$.

**Example 395: N-[(1R)-1-[4-amino-6-(trifluoromethyl)-2-pyridyl]ethyl]-1-(2-fluorophenyl)-6-oxo-pyridazine-3-carboxamide**

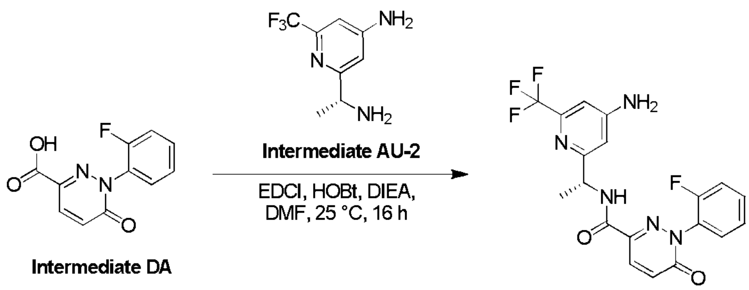


395

The compound of Example 395 (6.27 mg, 17.9% yield) was obtained as a colorless solid in the same manner as in Step 2 of Example 76 by using Intermediate AU-2. $^1$H NMR (400 MHz, $CDCl_3$) δ = 8.48 (d, J = 7.2 Hz, 1H), 8.05 (d, J = 9.6 Hz, 1H), 7.56 - 7.45 (m, 2H), 7.37 - 7.28 (m, 2H), 7.12 (d, J = 9.6 Hz, 1H), 6.79 (d, J = 2.0 Hz, 1H), 6.60 (d, J = 2.0 Hz, 1H), 5.18 - 5.08 (m, 1H), 4.44 (s, 2H), 1.54 (d, J = 6.8 Hz, 3H); MS (EI) m/z: 422.2 [M+H]$^+$.

**Example 396: N-[(1R)-1-[2-[5-fluoro-2-(methylaminomethyl)phenyl]thiazol-5-yl]ethyl]-1-(2-fluorophenyl)-6-oxo-pyridazine-3-carboxamide**

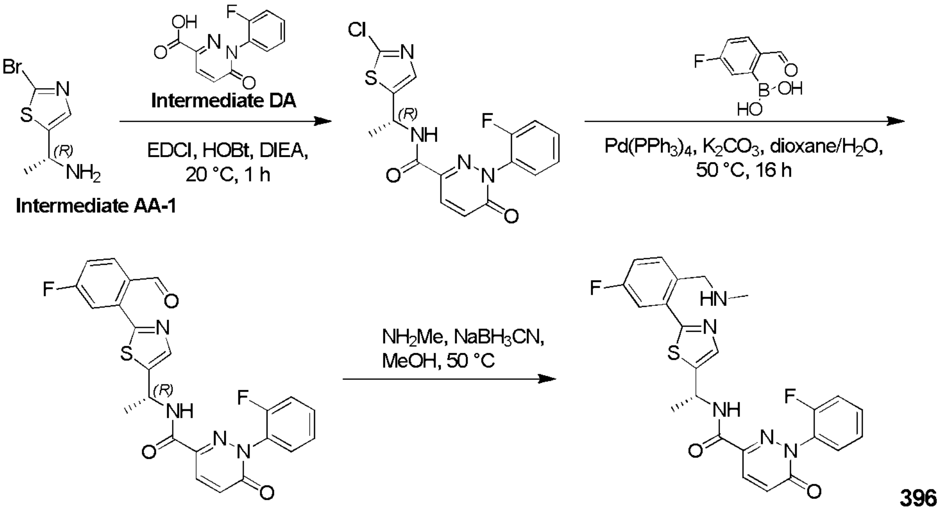


396

The compound of Example 396 (7.49 mg, 58% yield) was obtained as a yellow solid in the same manner as in Example 132 by using 1-(2-fluorophenyl)-6-oxo-1,6-dihydropyridazine-3-carboxylic acid, which was obtained from Step 1 of Example 76, and Intermediate AA-1. $^1$H NMR (400 MHz, $CDCl_3$) $\delta$ = 8.54 (s, 1H), 8.08 (d, J = 9.6 Hz, 1H), 7.82 (s, 1H), 7.63 - 7.56 (m, 1H), 7.55 - 7.45 (m, 2H), 7.43 - 7.38 (m, 1H), 7.36 - 7.31 (m, 2H), 7.21 - 7.12 (m, 2H), 5.65 - 5.52 (m, 1H), 4.14 (s, 2H), 2.63 (s, 3H), 1.76 (d, J = 7.2 Hz, 3H); MS (EI) m/z: 482.1 $[M+H]^+$.

**Example 397: N-[(1R)-1-[3-(1,1-difluoro-2-hydroxy-ethyl)-2-fluoro-phenyl]ethyl]-1-[3-(3-methyltriazol-4-yl)phenyl]-6-oxo-pyridazine-3-carboxamide**

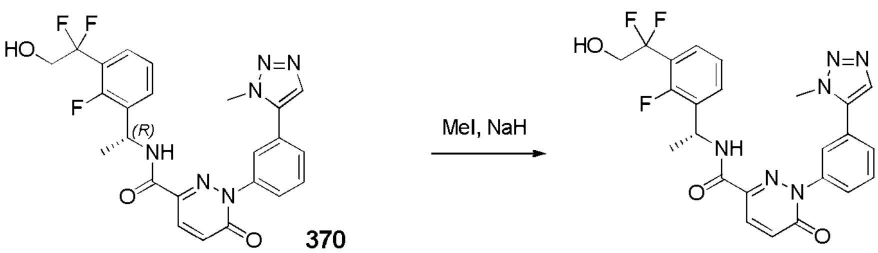


370 397

The compound of Example 397 was prepared in the same manner as in Example 242 by using the compound of Example 370. $^1$H NMR (400 MHz, DMSO-$d_6$) $\delta$ = 8.98 (br d, *J* = 8.0 Hz, 1H), 8.03 - 7.91 (m, 2H), 7.91 - 7.86 (m, 1H), 7.83 (dt, *J* = 2.0, 4.4 Hz, 1H), 7.71 (d, *J* = 4.8 Hz, 2H), 7.63 (br t, *J* = 7.2 Hz, 1H), 7.44 (br t, *J* = 7.2 Hz, 1H), 7.27 (t, *J* = 7.6 Hz, 1H), 7.17 (d, *J* = 9.6 Hz, 1H), 5.43 - 5.33 (m, 1H), 4.12 (s, 3H), 3.94 (s, 2H), 3.31 (s, 3H), 1.47 (d, *J* = 7.2 Hz, 3H); LC/MS (ESI) m/z: 513.4 $[M+H]^+$.

**Example 398: (R)-1-(2,4-difluorophenyl)-N-(1-(5-(2-((methylamino)methyl)phenyl)thiophenyl-2-yl)ethyl)-6-oxo-1,6-dihydropyridazine-3-carboxamide**

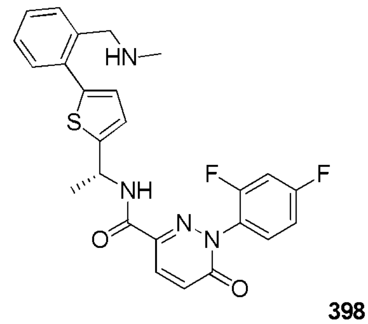

**398**

The compound of Example 398 was prepared in the same manner as in Examples 265 and 266 by using the product obtained from Step 5 of Example 352 and (2-formylphenyl)boronic acid. $^1$H NMR (400 MHz, DMSO-$d_6$) δ = 9.06 (d, *J* = 8.4 Hz, 1H), 7.98 (d, *J* = 10.0 Hz, 1H), 7.76 (dt, *J* = 6.4, 8.4 Hz, 1H), 7.59 - 7.45 (m, 2H), 7.36 - 7.24 (m, 4H), 7.20 (d, *J* = 10.0 Hz, 1H), 7.12 (d, *J= 3.6* Hz, 1H), 7.01 (d, *J* = 3.6 Hz, 1H), 5.41 (quin, *J* = 7.6 Hz, 1H), 3.67 (s, 2H), 2.26 (s, 3H), 1.59 (d, *J*= 7.2 Hz, 3H); LC/MS (ESI) m/z: 481.4 $[M+H]^+$

**Example 399 to Example 415**

The compounds in the following table were prepared in a similar manner to Example 398.

[Table 27]

| No. | Structure/Name | Spectral Data |
|---|---|---|
| 399 | 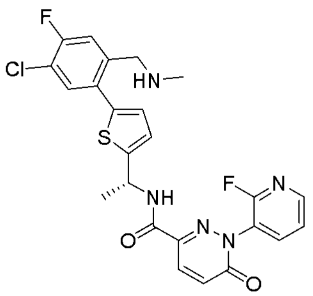 N-[(1R)-1-(5-{5-chloro-4-fluoro-2-[(methylamino)methyl]phenyl}thiophen-2-yl)ethyl]-1-(2-fluoropyridin-3-yl)-6-oxo-1,6-dihydropyridazine-3-carboxamide | $^1$H NMR (400 MHz, DMSO-$d_6$) δ = 9.13 (d, *J* = 8.4 Hz, 1H), 8.40 (d, *J* = 5.2 Hz, 1H), 8.31 (ddd, *J* = 2.0, 7.6,9.2 Hz, 1H), 8.00 (d, *J* = 10.0 Hz, 1H), 7.62 (ddd, *J* = 1.2, 5.6, 6.8 Hz, 1H), 7.58 - 7.44 (m, 2H), 7.23 (d, *J* = 10.0 Hz, 1H), 7.11 (d, *J* = 3.6 Hz, 1H), 7.04 - 7.00 (m, 1H), 5.44 - 5.36 (m, 1H), 3.65 - 3.63 (m, 2H), 2.23 (s, 3H), 1.59 (d, *J* = 6.8 Hz, 3H); LC/MS (ESI) m/z: 516.3 $[M+H]^+$ |
| 400 | 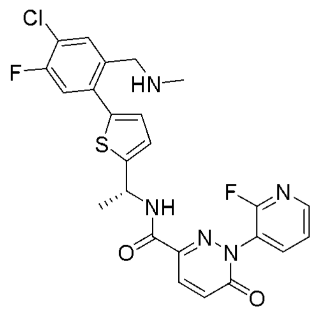 N-[(1R)-1-(5-{4-chloro-5-fluoro-2-[(methylamino)methyl]phenyl} thiophen-2-yl)ethyl]-1-(2-fluoropyridin-3-yl)-6-oxo-1,6-dihydropyridazine-3-carboxamide | $^1$H NMR (400 MHz, DMSO-$d_6$) δ = 8.35 (br d, *J* = 4.8 Hz, 1H), 8.18 (t, *J* = 8.0 Hz, 1H), 8.09 (d, *J* = 9.6 Hz, 1H), 7.60 (d, *J* = 7.6 Hz, 1H), 7.53 (dd, *J* = 5.2, 6.8 Hz, 1H), 7.22 (dd, *J* = 10.0, 11.2 Hz, 2H), 7.04 (d, *J* = 4.0 Hz, 1H),7.01 (d, *J* = 3.6 Hz, 1H), 5.50 (q, *J* = 6.8 Hz, 1H), 3.73 (s, 2H), 2.29 (s, 3H), 1.67 (d, *J* = 7.2 Hz, 3H); LC/MS (ESI) m/z: 516.3 $[M+H]^+$ |

(continued)

| No. | Structure/Name | Spectral Data |
|---|---|---|
| 401 | 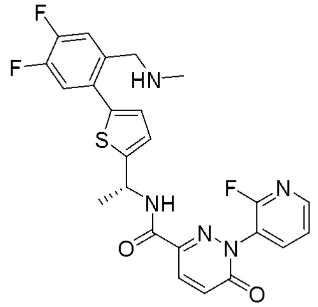 N-[(1R)-1-(5-{4,5-difluoro-2-[(methylamino)methyl]phenyl}thiophen-2-yl)ethyl]-1-(2-fluoropyridin-3-yl)-6-oxo-1,6-dihydropyridazine-3-carboxamide | $^1$H NMR (400 MHz, DMSO-$d_6$) $\delta$ = 9.12 (d, *J* = 8.8 Hz, 1H), 8.40 (d, *J* = 4.8 Hz, 1H), 8.31 (ddd, *J* = 2.0, 8.0, 9.6 Hz, 1H), 8.00 (d, *J* = 10.0 Hz, 1H), 7.62 (ddd, *J* = 1.2, 5.2, 7.6 Hz, 1H), 7.55 (dd, *J* = 8.8, 12.4 Hz, 1H), 7.39 (dd, *J* = 8.4, 11.6 Hz, 1H), 7.23 (d, *J* = 10.0 Hz, 1H), 7.12 (d, *J* = 3.6 Hz, 1H), 7.02 (dd, *J* = 0.8, 3.6 Hz, 1H), 5.40 (br t, *J* = 7.6 Hz, 1H), 3.60 (s, 2H), 2.22 (s, 3H), 1.59 (d, *J* = 7.2 Hz, 3H); LC/MS (ESI) m/z: 500.3 [M+H]$^+$ |
| 402 | 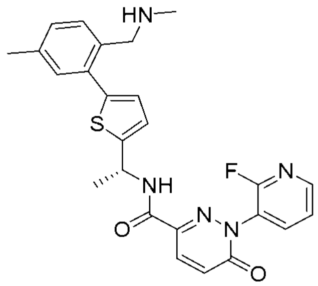 1-(2-fluoropyridin-3-yl)-N-[(1R)-1-(5-{5-methyl-2-[(methylamino) methyl]phenyl}thiophen-2-yl)ethyl]-6-oxo-1,6-dihydropyridazine-3-carboxamide | $^1$H NMR (400 MHz, DMSO-$d_6$) $\delta$ = 8.42 (br s, 1H), 8.36 (br d, J = 4.8 Hz, 1H), 8.23 - 8.16 (m, 1H), 8.09 (d, J = 10.0 Hz, 1H), 7.53 (dd, J = 5.2, 7.2 Hz, 1H), 7.45 (d, J = 8.0 Hz, 1H), 7.34 - 7.27 (m, 2H), 7.21 (d, J = 10.0 Hz, 1H), 7.07 (d, J = 3.2 Hz, 1H), 6.95 (d, J = 3.6 Hz, 1H), 5.50 (q, J = 6.8 Hz, 1H), 4.27 (s, 2H), 2.60 (s, 3H), 2.38 (s, 3H), 1.68 (d, J = 6.8 Hz, 3H); LC/MS (ESI) m/z: 478.4 [M+H]$^+$ |
| 403 | 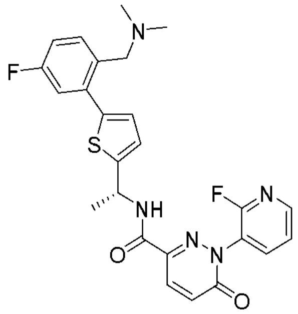 N-[(1R)-1-(5-{2-[(dimethylamino)methyl]-5-fluorophenyl}thiophen-2-yl)ethyl]-1-(2-fluoropyridin-3-yl)-6-oxo-1,6-dihydropyridazine-3-carboxamide | $^1$H NMR (400 MHz, DMSO-$d_6$) $\delta$ = 8.34 (br d, *J* = 3.6 Hz, 1H), 8.17 (br t, *J* = 8.0 Hz, 1H), 8.08 (br d, *J* = 10.0 Hz, 1H), 7.60 - 7.45 (m, 2H), 7.17 (br dd, *J* = 9.6, 18.0 Hz, 3H), 7.03 (br s, 2H), 5.58 - 5.41 (m, 1H), 3.85 (br s, 2H), 2.35 (s, 6H), 1.66 (br d, *J* = 6.8 Hz, 3H); LC/MS (ESI) m/z: 496.3 [M+H]$^+$ |
| 404 | 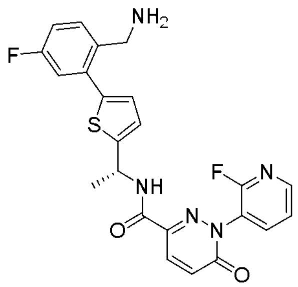 N-[(1R)-1-(5-{2-[(aminomethyl)-5-fluorophenyl]thiophen-2-yl) ethyl]-1-(2-fluoropyridin-3-yl)-6-oxo-1,6-dihydropyridazine-3-carboxamide | $^1$H NMR (400 MHz, DMSO-$d_6$) $\delta$ = 9.12 (d, *J* = 8.8 Hz, 1H), 8.40 (d, *J* = 4.8 Hz, 1H), 8.32 (ddd, *J* = 2.0, 7.6, 9.6 Hz, 1H), 8.00 (d, *J* = 10.0 Hz, 1H), 7.66 - 7.56 (m, 2H), 7.27 - 7.06 (m, 4H), 7.03 (d, *J* = 3.6 Hz, 1H), 5.41 (quin, *J* = 7.6 Hz, 1H), 3.74 (s, 2H), 2.26 - 1.71 (m, 2H), 1.59 (d, *J* = 7.2 Hz, 3H); LC/MS (ESI) m/z: 468.1 [M+H]$^+$ |

(continued)

| No. | Structure/Name | Spectral Data |
|---|---|---|
| 405 | 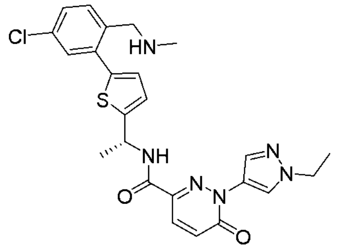 N-[(1R)-1-(5-{5-chloro-2-[(methylamino)[methyl]phenyl}thiophen-2-yl)ethyl]-1-(1-ethyl-1H-pyrazol-4-yl)-6-oxo-1,6-dihydropyridazine-3-carboxamide | $^1$H NMR (400 MHz, DMSO-$d_6$) $\delta$ = 8.52 (s, 1H), 8.28 (s, 1H), 7.99 (d, *J* = 9.6 Hz, 1H), 7.44 (d, *J* = 7.6 Hz, 1H), 7.36 - 7.32 (m, 2H), 7.15 (d, *J* = 9.6 Hz, 1H), 7.09 (d, *J* = 4.0 Hz, 1H), 7.01 (d, *J* = 3.6 Hz, 1H), 5.56 (q, *J* = 6.8 Hz, 1H), 4.23 (q, *J* = 7.2 Hz, 2H), 3.76 (s, 2H), 2.29 (s, 3H), 1.75 (d, *J* = 6.8 Hz, 3H), 1.49 (t, *J* = 7.2 Hz, 3H); LC/MS (ESI) m/z: 497.4 $[M+H]^+$ |
| 406 | 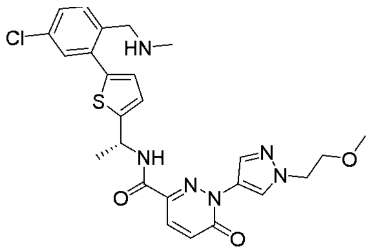 N-[(1R)-1-(5-{5-chloro-2-[(methylamino)[methyl]phenyl}thiophen-2-yl)ethyl]-1-[1-(2-methoxyethyl)-1H-pyrazol-4-yl)-6-oxo-1,6-dihydro-pyridazine-3-carboxamide | $^1$H NMR (400 MHz, DMSO-$d_6$) $\delta$ = 9.10 (d, J = 8.8 Hz, 1H), 8.55 (s, 1H), 8.38 (s, 1H), 7.92 (d, J = 10.0 Hz, 1H), 7.53 (d, J = 8.0 Hz, 1H), 7.42 - 7.31 (m, 2H), 7.21 (d, J = 3.6 Hz, 1H), 7.17 (d, J = 9.6 Hz, 1H), 7.08 (d, J = 2.8 Hz, 1H), 5.56 - 5.39 (m, 1H), 4.32 (t, J = 5.2 Hz, 2H), 3.70 (t, J = 5.2 Hz, 2H), 3.64 (s, 2H), 3.22 (s, 3H), 2.24 (s, 3H), 2.05 (br d, J = 18.0 Hz, 1H), 1.68 (d, J = 7.2 Hz, 3H); LC/MS (ESI) m/z: 527.4 $[M+H]^+$ |
| 407 | 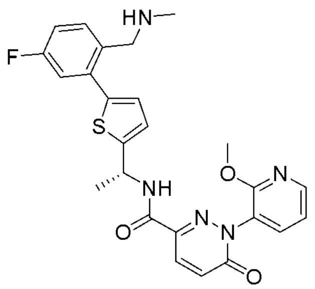 N-[(1R)-1-(5-{5-fluoro-2-[(methylamino)[methyl]phenyl}thiophen-2-yl) ethyl]-1-(2-methoxypyridin-3-yl)-6-oxo-1,6-dihydropyridazine-3-carboxamide | $^1$H NMR (400 MHz, DMSO-$d_6$) $\delta$ = 9.04 (d, *J* = 8.8 Hz, 1H), 8.31 (dd, *J* = 2.0, 5.2 Hz, 1H), 8.01 - 7.91 (m, 2H), 7.55 - 7.48 (m, 1H), 7.22 - 7.16 (m, 3H), 7.16 - 7.12 (m, 2H), 7.01 (dd, *J* = 0.8, 3.6 Hz, 1H), 5.40 (quin, *J* = 7.2 Hz, 1H), 3.86 (s, 3H), 3.60 (s, 2H), 2.23 (s, 3H), 1.58 (d, *J* = 7.2 Hz, 3H); LC/MS (ESI) m/z: 494.4 $[M+H]^+$ |
| 408 | 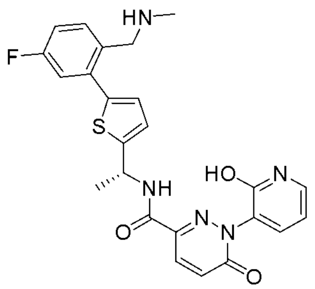 N-[(1R)-1-(5-{5-fluoro-2-[(methylamino)[methyl]phenyl}thiophen-2-yl) ethyl]-1-(2-hydroxypyridin-3-yl)-6-oxo-1,6-dihydropyridazine-3-carboxamide | $^1$H NMR (400 MHz, DMSO-$d_6$) $\delta$ = 8.42 (td, *J* = 1.2, 3.2 Hz, 1H), 8.25 (ddd, *J* = 1.8, 7.6, 9.6 Hz, 1H), 7.99 (d, *J* = 10.0 Hz, 1H), 7.62 (ddd, *J* = 1.2, 4.8, 7.6 Hz, 1H), 7.24 (d, *J* = 10.0 Hz, 1H), 0.99 (t, *J* = 7.2 Hz, 3H); LC/MS (ESI) m/z: 250.1 $[M+H]^+$ |

(continued)

| No. | Structure/Name | Spectral Data |
|---|---|---|
| 409 | 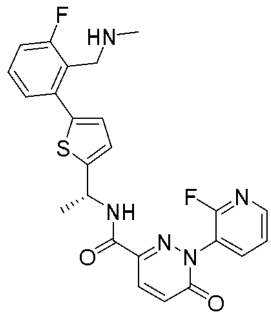 N-[(1R)-1-(5-{3-fluoro-2-[(methylamino)[methyl]phenyl}thiophen-2-yl) ethyl]-1-(2-fluoropyridin-3-yl)-6-oxo-1,6-dihydropyridazine-3-carboxamide | $^1$H NMR (400 MHz, DMSO-d$_6$) δ = 9.13 (d, *J* = 8.6 Hz, 1H), 8.40 (br d, *J* = 4.8 Hz, 1H), 8.35 - 8.26 (m, 1H), 8.00 (d, *J* = 10.0 Hz, 1H), 7.62 (dd, *J* = 5.6, 7.1 Hz, 1H), 7.38 - 7.28 (m, 2H), 7.26 - 7.14 (m, 3H), 7.03 (d, *J* = 3.6 Hz, 1H), 5.41 (quin, *J* = 7.6 Hz, 1H), 3.59 (br s, 2H), 2.26 (s, 3H), 1.81 (br s, 1H), 1.59 (br d, *J* = 7.2 Hz, 3H); LC/MS (ESI) m/z: 482.4 [M+H]$^+$ |
| 410 | 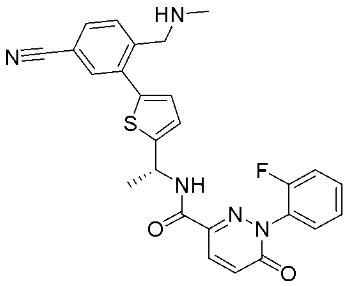 N-[(1R)-1-(5-{5-cyano-2-[(methylamino)[methyl]phenyl}thiophen-2-yl)ethyl]-1-(2-fluorophenyl)-6-oxo-1,6-dihydropyridazine-3-carboxamide | $^1$H NMR (400 MHz, DMSO-d$_6$) δ = 9.10 (d, *J* = 8.4 Hz, 1H), 7.99 (d, *J* = 9.6 Hz, 1H), 7.83 - 7.75 (m, 2H),7.73 (d, *J* = 7.2 Hz, 1H), 7.67 (dt, *J* = 1.6, 7.6 Hz, 1H), 7.61 - 7.55 (m, 1H), 7.47 - 7.38 (m, 2H), 7.23 - 7.17 (m, 2H), 7.05 (dd, *J* = 0.8, 3.6 Hz, 1H), 5.46 - 5.38 (m, 1H), 3.71 (s, 2H), 2.23 (s, 3H), 1.60 (d, *J* = 7.2 Hz, 3H); LC/MS (ESI) m/z: 488.4 [M+H]$^+$ |
| 411 | 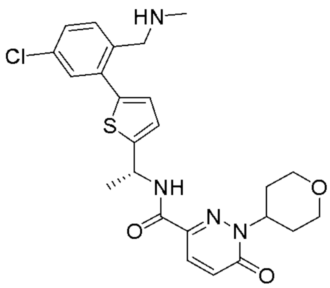 N-[(1R)-1-(5-{5-chloro-2-[(methylamino)[methyl]phenyl}thiophen-2-yl)ethyl]-1-(oxan-4-yl)-6-oxo-1,6-dihydropyridazine-3-carboxamide | $^1$H NMR (400 MHz, DMSO-d$_6$) δ = 9.01 (d, *J* = 8.8 Hz, 1H), 7.86 (d, *J* = 9.6 Hz, 1H), 7.54 (d, *J* = 8.4 Hz, 1H), 7.42 - 7.34 (m, 2H), 7.21 (d, *J* = 3.6 Hz, 1H), 7.07 - 7.04 (m, 1H), 7.02 (d, *J* = 9.6 Hz, 1H), 5.46 (quin, *J* = 7.2 Hz, 1H), 5.04 (tt, *J* = 4.0, 11.6 Hz, 1H), 4.04 - 3.94 (m, 2H), 3.64 (s, 2H), 3.60 (br t, *J* = 11.2 Hz, 2H), 2.30 - 2.16 (m, 5H), 2.16 - 1.94 (m, 1H), 1.71 - 1.63 (m, 5H); LC/MS (ESI) m/z: 487.4 [M+H]$^+$ |
| 412 | 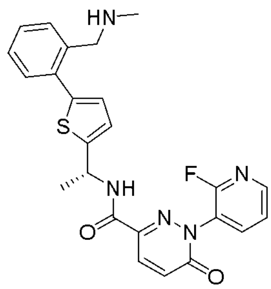 1-(2-fluoropyridin-3-yl)-N-[(1R)-1-(5-{2-[(methylamino)methyl]phenyl} thiophen-2-yl)ethyl]-6-oxo-1,6-dihydropyridazine-3-carboxamide | $^1$H NMR (400 MHz, DMSO-d$_6$) δ = 9.09 (d, *J* = 8.8 Hz, 1H), 8.43 - 8.35 (m, 1H), 8.35 - 8.26 (m, 1H), 8.04 - 7.97 (m, 1H), 7.62 (ddd, *J* = 1.2, 4.8, 7.6 Hz, 1H), 7.49 (d, *J* = 7.6 Hz, 1H), 7.35 - 7.26 (m, 3H), 7.25 - 7.20 (m, 1H), 7.14 - 7.09 (m, 1H), 7.00 (dd, *J* = 0.8, 3.6 Hz, 1H), 5.41 (quin, *J* = 7.2 Hz, 1H), 3.64 (s, 2H), 2.24 (s, 3H), 1.59 (d, *J* = 7.2 Hz, 3H); LC/MS (ESI) m/z: 464.4 [M+H]$^+$ |

(continued)

| No. | Structure/Name | Spectral Data |
|---|---|---|
| 413 | 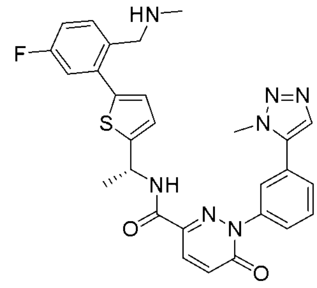<br>N-[(1R)-1-(5-{5-fluoro-2-[(methylamino)[methyl]phenyl}thiophen-2-yl) ethyl]-1-[3-(1-methyl-1H-1,2,3-triazol-5-yl)phenyl]-6-oxo-1,6-dihydro-pyridazine-3-carboxamide | $^1$H NMR (400 MHz, DMSO-$d_6$) δ = 9.08 (d, *J* = 8.4 Hz, 1H), 8.01 - 7.93 (m, 3H), 7.86 - 7.82 (m, 1H), 7.73 - 7.70 (m, 2H), 7.69 - 7.65 (m, 1H), 7.35 (dt, *J* = 2.8, 8.8 Hz, 1H), 7.24 (dd, *J* = 2.8, 10.0 Hz, 1H), 7.21 (d, *J* = 10.0 Hz, 1H), 7.14 (d, *J* = 3.6 Hz, 1H), 7.09 (d, *J* = 3.6 Hz, 1H), 5.49 - 5.39 (m, 1H), 4.12 (s, 3H), 4.10 (br s, 2H), 2.49 (br s, 3H), 1.63 (d, *J* = 7.2 Hz, 3H); LC/MS (ESI) m/z: 544.4 [M+H]$^+$ |
| 414 | 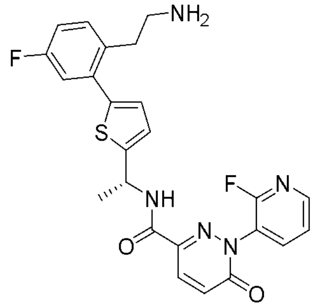<br>N-[(1R)-1-[5-[2-(2-aminoethyl)-5-fluorophenyl]-2-thienyl]ethyl]-1-(2-fluoro-3-pyridyl)-6-oxopyridazine-3-carboxamide | $^1$H NMR (400 MHz, DMSO-$d_6$) δ = 9.11 (d, *J* = 8.4 Hz, 1H), 8.40 (d, *J* = 4.8 Hz, 1H), 8.31 (t, *J* = 8.4 Hz, 1H),8.00 (d, *J* = 10.0Hz, 1H), 7.62 (dd, *J* = 5.6, 7.2 Hz, 1H), 7.37 - 7.32 (m, 1H), 7.23 (d, *J* = 9.6 Hz, 1H), 7.19 - 7.12 (m, 1H), 7.11 - 7.08 (m, 1H), 7.02 (s, 2H), 5.44 - 5.37 (m, 1H), 2.77 - 2.61 (m, 4H), 1.59 (d, *J* = 6.8 Hz, 3H); MS (ESI) m/z = 482.4 [M+H]$^+$ |
| 415 | 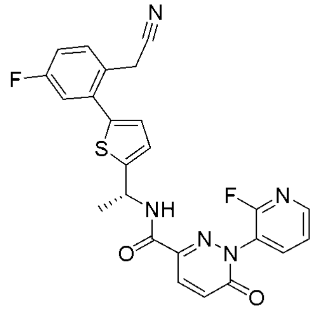<br>N-[(1R)-1-[5-[2-(cyanomethyl)-5-fluorophenyl]-2-thienyl]ethyl]-1-(2-fluoro-3-pyridyl)-6-oxo-pyridazine-3-carboxamide | $^1$H NMR (400 MHz, DMSO-$d_6$) δ = 9.13 (d, *J* = 8.4 Hz, 1H), 8.40 (d, *J* = 4.8 Hz, 1H), 8.36 - 8.25 (m, 1H), 8.00 (d, *J* = 9.6 Hz, 1H), 7.68 - 7.53 (m, 2H), 7.33 - 7.20 (m, 3H), 7.13 (d, *J* = 3.6 Hz, 1H), 7.08 (d, *J* = 3.6 Hz, 1H), 5.42 (quin, *J* = 7.2 Hz, 1H), 4.04 (s, 2H), 1.60 (d, J=7.2 Hz, 3H); MS (ESI) m/z = 478.3 [M+H]$^+$ |

**Example B01: N-[(1R)-1-[2-fluoro-3-(trifluoromethyl)phenyl]ethyl]-1-[5-(3-methyltriazol-4-yl)-3-pyridyl]-6-oxo-pyridazine-3-carboxamide**

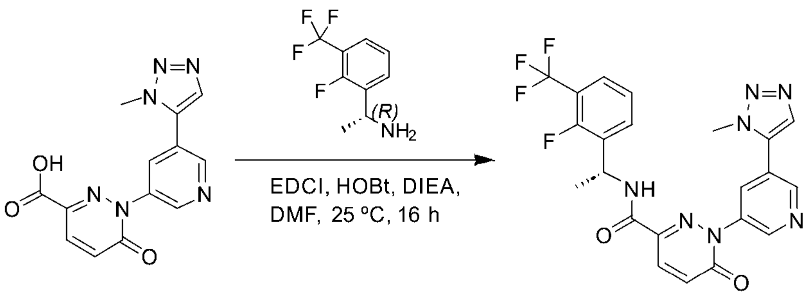


To a solution of Intermediate BA (40 mg, 0.13 mmol, 1 eq) and Intermediate W (25.17 mg, 0.1 mmol, 0.8 eq, HCl

salt) in DMF (8 mL), HOBt (26.34 mg, 0.19 mmol, 1.5 eq), DIEA (0.67 mL, 0.39 mmol, 3 eq) and EDCI (37.38 mg, 0.19 mmol, 1.5 eq) were added. Then, the mixture was degassed and purged with $N_2$ for three times, and stirred at 25 °C for 16 h under $N_2$ atmosphere. The reaction mixture was poured into water and extracted with EtOAc (10 mL × 2). The combined organic layer was washed with brine (10 mL × 2), dried over anhydrous $Na_2SO_4$, concentrated under reduced pressure to give a crude product. The crude product was purified by silica gel column chromatography (2% MeOH in DCM) to give a compound of Example B01 (24.7 mg, 38.98% yield) as a white solid. $^1$H NMR (400 MHz, DMSO-$d_6$) δ 9.16-9.10 (m, 2H), 8.92 (d, *J* = 2.0 Hz, 1H), 8.51 (t, *J* = 2.0 Hz, 1H), 8.13 (s, 1H), 7.92 (d, *J* = 9.6 Hz, 1H), 7.83 (br t, *J* = 7.2 Hz, 1H), 7.68 (br t, *J* = 7.2 Hz, 1H), 7.40 (t, *J* = 7.6 Hz, 1H), 7.22 (d, *J* = 9.6 Hz, 1H), 5.46 - 5.39 (m, 1H), 4.17 (s, 3H), 1.51 (d, *J* = 7.2 Hz, 3H); LCMS: $t_R$ = 2.105 min, MS (ESI) m/z = 488.1 [M+H]$^+$.

**Example B02: N-[(1R)-1-[3-(difluoromethyl)-2-fluoro-phenyl]ethyl]-1-[5-(3-methyltriazol-4-yl)-3-pyridyl]-6-oxo-pyridazine-3-carboxamide**

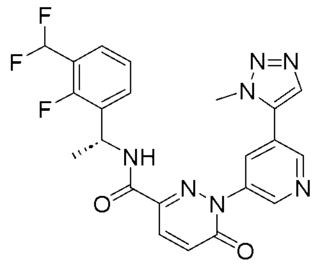

The compound of Example B02 was obtained in the same manner as in Example B01, except for using Intermediate BB as a starting material. $^1$H NMR (400 MHz, DMSO-$d_6$) δ 9.12 (d, *J* = 2.4 Hz, 1H), 9.08 (d, *J* = 8.0 Hz, 1H), 8.92 (d, *J* = 2.0 Hz, 1H), 8.51 (t, *J* = 2.0 Hz, 1H), 8.13 (s, 1H), 7.92 (d, *J* = 10.0 Hz, 1H), 7.69 (t, *J* = 7.2 Hz, 1H), 7.53 (t, *J* = 6.8 Hz, 1H), 7.36 - 7.32 (m, 1H), 7.24 - 7.08 (m, 2H), 5.42 (t, *J* = 7.2 Hz, 1H), 4.17 (s, 3H), 1.50 (d, *J* = 7.2 Hz, 3H); LCMS: $t_R$ = 0.540 min, MS (ESI) m/z = 470.2 [M+H]$^+$.

**Example B03: N-[(1R)-1-[5-amino-2-fluoro-3-(trifluoromethyl)phenyl]ethyl]-5-methyl-1-[5-(3-methyltriazol-4-yl)-3-pyridyl]-6-oxo-pyridazine-3-carboxamide**

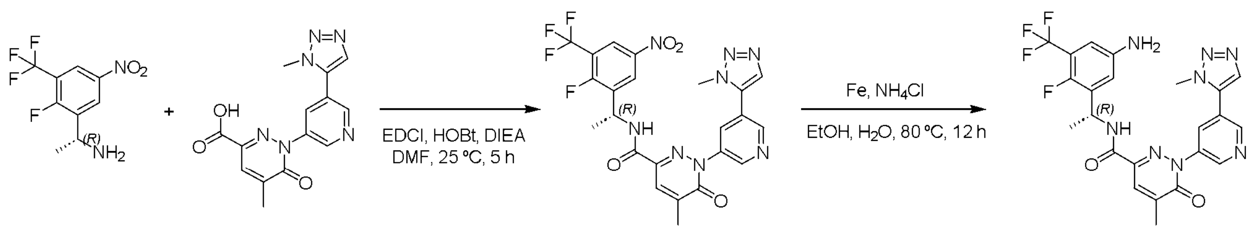


Step 1: Synthesis of N-[(1R)-1-[2-fluoro-5-nitro-3-(trifluoromethyl)phenyl]ethyl]-5-methyl-1-[5-(3-methyltriazol-4-yl)-3-pyridyl]-6-oxo-pyridazine-3-carboxamide

To a solution of Intermediate BC (40 mg, 128.09 μmol, 1 eq) and Intermediate AX (36.97 mg, 128.09 μmol, 1 eq, HCl) in DMF (2 mL), HOBt (34.62 mg, 256.18 μmol, 2 eq), EDCI (49.11 mg, 256.18 μmol, 2 eq) and DIEA (49.66 mg, 384.27 μmol, 66.93 μL, 3 eq) were added. Then, the mixture was degassed and purged with $N_2$ for three times, and stirred at 30 °C for 5 h under $N_2$ atmosphere. The reaction mixture was poured into water (10 mL) and extracted with EtOAc (30 mL × 3). The combined organic layer was washed with brine (10 mL × 3), dried over anhydrous $Na_2SO_4$, concentrated under reduced pressure to give a crude product. The crude product was purified by silica gel column chromatography (2% MeOH in DCM) to give a main product, N-[(1R)-1-[2-fluoro-5-nitro-3-(trifluoromethyl)phenyl]ethyl]-5-methyl-1-[5-(3-methyltriazol-4-yl)-3-pyridyl]-6-oxo-pyridazine-3-carboxamide (98 mg, 92% yield) as a brown oil. LCMS: $t_R$ = 1.026 min, MS (ESI) m/z = 497.1 [M+H]$^+$.

Step 2: Synthesis of N-[(1R)-1-[5-amino-2-fluoro-3-(trifluoromethyl)phenyl]ethyl]-5-methyl-1-[5-(3-methyltriazol-4-yl)-3-pyridyl]-6-oxo-pyridazine-3-carboxamide

To a solution of N-[(1R)-1-[2-fluoro-5-nitro-3-(trifluoromethyl)phenyl]ethyl]-5-methyl-1-[5-(3-methyltriazol-4-yl)-3-pyridyl]-6-oxo-pyridazine-3-carboxamide (98 mg, 179.34 μmol, 1 eq) in EtOH (5 mL) and $H_2O$ (2 mL), Fe (50.08 mg, 896.72 μmol, 5 eq) and $NH_4Cl$ (95.93 mg, 1.79 mmol, 10 eq) were added. The mixture was degassed and purged with $N_2$ for three times, and stirred at 80 °C for 5 h under $N_2$ atmosphere. The reaction mixture was concentrated under reduced pressure, poured into water (10 mL) and extracted with EtOAc (10 mL × 3). The combined organic layer was washed with brine (10 mL × 3), dried over anhydrous $Na_2SO_4$, and concentrated under reduced pressure to give a crude product. The crude product was purified by prep-HPLC (Welch Xtimate C18 150*30 mm*5 um; mobile phase: [water($NH_3H_2O$+$NH_4HCO_3$)-ACN]; B%: 8%-48%, 28 min). The remaining solvent was removed by lyophilization to give the compound of Example B03 (29.4 mg, 31.58% yield, 99.5% purity) as a white solid. $^1H$ NMR (400 MHz, DMSO-$d_6$) δ 9.22 (d, $J$ = 2.4 Hz, 1H), 9.05 (d, $J$ = 8.0 Hz, 1H), 8.99 (d, $J$ = 2.0 Hz, 1H), 8.60 (t, $J$ = 2.0 Hz, 1H), 8.21 (s, 1H), 7.96 (d, $J$ = 1.2 Hz, 1H), 6.91 (dd, $J$ = 2.4, 5.6 Hz, 1H), 6.81 (dd, $J$ = 2.8, 5.2 Hz, 1H), 5.49 (s, 2H), 5.37-5.31 (m, 1H), 4.25 (s, 3H), 2.30 (d, $J$ = 1.2 Hz, 3H), 1.51 (d, $J$ = 7.2 Hz, 3H); LCMS: $t_R$ = 1.929 min, MS (ESI) m/z = 517.1 $[M+H]^+$.

**Example B04: N-[(R)-1-[2-fluoro-3-(trifluoromethyl)phenyl]ethyl]-1-[m-(1-ethyl-1H-1,2,3-triazol-5-yl)phenyl]-6-oxo-1,6-dihydronicotinamide**

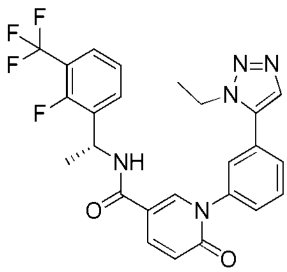

The compound of Example B04 was manufactured in the same manner as in Example 321, except that, in Step 1 of Example 321, 1-ethyl-5-(4,4,5,5-tetramethyl-1,3,2-dioxaborolan-2-yl)triazole was used instead of 1-methyl-5-(4,4,5,5-tetramethyl-1,3,2-dioxaborolan-2-yl)pyrazole; in Step 3 of Example 321, Intermediate W was used instead of Intermediate C. $^1H$ NMR (400 MHz, DMSO-$d_6$) δ 8.75 (d, $J$ = 7.2 Hz, 1H), 8.47 (s, 1H), 7.97 - 7.96 (m, 2H), 7.76 - 7.64 (m, 6H), 7.39 (t, $J$ = 7.6 Hz, 1H), 6.56 (d, $J$ = 9.6 Hz, 1H), 5.38 - 5.31 (m, 1H), 4.47 (q, $J$ = 7.6 Hz, 2H), 1.45 (d, $J$ = 6.8 Hz, 2H), 1.39 (t, $J$ = 7.2 Hz, 3H); MS (ESI) m/z = 500.16 $[M+H]^+$.

**Example B05: N-[(1R)-1-[5-amino-2-methyl-3-(trifluoromethyl)phenyl]ethyl]-1-[5-(2-methylpyrazol-3-yl)-3-pyridyl]-6-oxo-pyridazine-3-carboxamide**

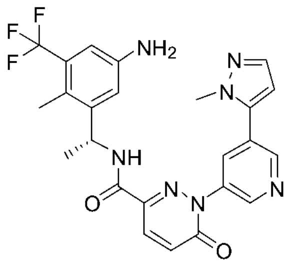

The compound of Example B05 was manufactured by using Intermediate BD instead of Intermediate BC and Intermediate N instead of Intermediate AX in Example B03. $^1H$ NMR (400MHz, DMSO-$d_6$) δ 9.07 (d, $J$ = 2.0 Hz, 1H), 8.95 (d, $J$ = 7.6 Hz, 1H), 8.84 (d, $J$ = 2.0 Hz, 1H), 8.42 (t, $J$ = 2.0 Hz, 1H), 7.92 (d, $J$ = 10.0 Hz, 1H), 7.56 (d, $J$ = 2.0 Hz, 1H), 7.22 (d, $J$ = 10.0 Hz, 1H), 6.86 (s, 1H), 6.79 (d, $J$ = 2.0 Hz, 1H), 6.63 (d, $J$ = 2.0 Hz, 1H), 5.33 - 5.22 (m, 3H), 3.93 (s, 3H), 2.24 (s, 3H), 1.41 (d, $J$ = 6.8 Hz, 3H); LCMS: $t_R$ = 1.876 min, MS (ESI) m/z = 498.2 $[M+H]^+$.

**Example B06: N-[(R)-1-[2-fluoro-3-(trifluoromethyl)phenyl]ethyl]-1-[4-fluoro-3-(1-methyl-1H-1,2,3-triazol-5-yl)phenyl]-6-oxo-1,6-dihydro-3-pyridazinecarboxamide**

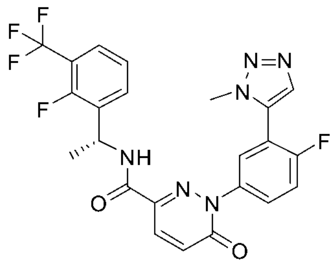

The compound of Example B06 was manufactured by using Intermediate U-1 as a starting material in Step 5 of Example 369 and using Intermediate W instead of Intermediate C in Step 4 of Example 369. $^1$H NMR (400 MHz, DMSO-$d_6$) δ 9.05 (d, *J* = 7.6 Hz, 1H), 8.01 - 7.89 (m, 3H), 7.87 (d, *J* = 1.2 Hz, 1H), 7.81 (m, 1H) 7.67 - 7.62 (q, *J* = 5.2 Hz, 2H), 7.39 (m, 1H), 7.17 (d, *J* = 8 Hz, 1H), 5.46 - 5.39 (m, 1H), 4.17 (s, 3H), 1.51 (d, *J* = 7.2 Hz, 3H); MS (ESI) m/z = 505.1 $[M+H]^+$.

**Example B07: N-[(1R)-1-[3-amino-5-(difluoromethyl)phenyl]ethyl]-5-methyl-1-[5-(3-methyltriazol-4-yl)-3-pyridyl]-6-oxo-pyridizine-3-carboxamide**

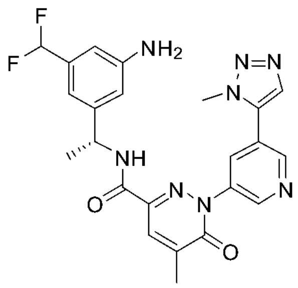

The compound of Example B07 was manufactured by using Intermediate AJ instead of Intermediate AX in Example B03. $^1$H NMR (400 MHz, DMSO-$d_6$) δ 9.11 (d, *J* = 2.4 Hz, 1H), 8.90 - 8.86 (m, 2H), 8.50 (t, *J* = 2.0 Hz, 1H), 8.12 (s, 1H), 7.88 (d, *J* = 1.2 Hz, 1H), 6.94 (s, 1H), 6.80 (s, 1H), 6.69 (br s, 2H), 6.66 (s, 1H), 6.58 (s, 1H), 5.36 (s, 2H), 5.06 - 5.02 (m, 1H), 4.15 (s, 3H), 2.22 (s, 3H), 1.45 (d, *J* = 7.2 Hz, 3H); LCMS: $t_R$ = 1.463 min, MS (ESI) m/z = 481.1 $[M+H]^+$.

**Example B08 to Example B11**

The compounds shown in the following Table 28 were manufactured in the same manner as in Example B01 via one-step amide coupling by using the corresponding intermediates described in Preparation Examples.

[Table 28]

| No. | Chemical Name/Structure | Spectra (LCMS / $^{1}$H NMR) |
|---|---|---|
| B08 | N-[(1R)-1-[2-fluoro-3-(trifluoromethyl)phenyl]ethyl]-1-[5-(3-methyltriazol-4-yl)-3-pyridyl]-6-oxo-pyridine-3-carboxamide 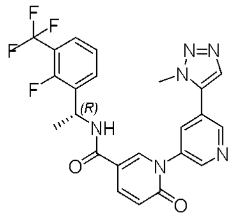 | **LCMS:** $t_R$ = 1.748 min, MS (ESI) m/z = 487.1 [M+ H]$^+$). **$^1$H NMR** (400 MHz, DMSO-$d_6$) δ = 8.95 (d, *J* = 2.0 Hz, 1H), 8.87 (d, *J* = 2.4 Hz, 1H), 8.75 (d, *J* = 7.2 Hz, 1H), 8.51 (d, *J* = 2.4 Hz, 1H), 8.37 (t, *J* = 2.0 Hz, 1H), 8.13 (s, 1H), 7.99 (dd, *J* = 2.4, 9.6 Hz, 1H), 7.77 (t, *J* = 7.2 Hz, 1H), 7.67 (t, *J* = 6.8 Hz, 1H), 7.40 (t, *J* = 8.0 Hz, 1H), 6.61 (d, *J* = 9.6 Hz, 1H), 5.40-5.32 (m, 1H), 4.16 (s, 3H), 1.47 (d, *J* = 7.2 Hz, 3H). |
| B09 | (R)-N-(1-(2-fluoro-3-(1,1,2-trifluoroethyl)phenyl)ethyl)-1-(5-(1-methyl-1H-1,2,3-triazol-5-yl)pyridin-3-yl)-6-oxo-1,6-dihydropyridazine-3-carboxamide 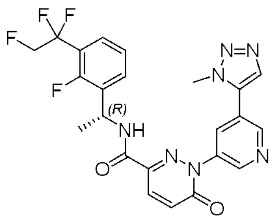 | **LCMS:** $t_R$ = 2.542 min, MS (ESI) m/z = 502.18 [M +H]$^+$. **$^1$H NMR** (400 MHz, DMSO-$d_6$) δ = 9.13-9.11 (m, 1H), 8.93 (d, *J* = 2.0 Hz, 1H), 8.52 (t, *J* = 2.0 Hz, 1H), 8.14 (s, 1H), 7.93 (d, *J* = 9.6 Hz, 1H), 7.72 (t, *J* = 7.2 Hz, 1H), 7.52 (t, *J* = 6.8 Hz, 1H), 7.35 (t, *J* = 7.8 Hz, 1H), 7.23 (d, *J* = 10 Hz, 1H), 5.42 (quint, *J* = 7.3 Hz, 1H), 5.05 (t, *J* = 13.8 Hz, 1H), 4.94 (t, *J* = 13.8 Hz, 1H), 4.18 (s, 3H), 1.50 (d, *J*= 7.2 Hz, 3H). |
| B10 | (R)-N-(1-(2-fluoro-3-(trifluoromethyl)phenyl)ethyl)-3-methyl-5'-(1-methyl-1H-1,2,3-triazol-5-yl)-2-oxo-2H-[1,3'-bipyridine]-5-carboxamide 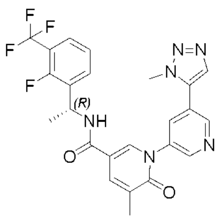 | **LCMS:** $t_R$ = 2.696 min, MS (ESI) m/z = 501.14 [M +H]$^+$. **$^1$H NMR** (400 MHz, DMSO-$d_6$) δ = 8.95 (d, *J* = 2.0 Hz, 2H), 8.86 (d, *J* = 2.0 Hz, 1H), 8.73 (d, *J* = 6.8 Hz, 1H), 8.39 (d, *J* = 2.4 Hz, 1H), 8.36 (t, *J* = 2.2 Hz, 1H), 8.13 (s, 1H), 7.91-7.90 (m, 1H), 7.77 (t, *J* = 7.0 Hz, 1H), 7.67 (t, *J* = 6.8 Hz, 1H), 7.40 (t, *J* = 7.8 Hz, 1H), 5.36 (quint, *J* = 7.0 Hz, 1H), 4.16 (s, 3H), 2.11 (s, 3H), 1.47 (d, *J* = 7.2 Hz, 3H). |
| B11 | N-[(1R)-1-[2-fluoro-3-(trifluoromethyl)phenyl]ethyl]-6-oxo-1-[5-[3-(trideuteriomethyl)triazol-4-yl]-3-pyridyl]pyridazine-3-carboxamide 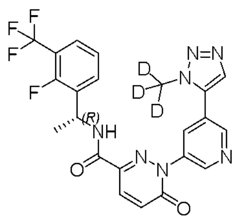 | **LCMS:** $t_R$ = 0.560 min, MS (ESI) m/z = 491.3 [M+ H]$^+$. **$^1$H NMR** (400 MHz, METHANOL-$d_4$) δ = 9.10 (d, *J* = 2.4 Hz, 1H), 8.87 (d, *J* = 1.6 Hz, 1H), 8.50 (t, *J* = 2.0 Hz, 1H), 8.04 - 8.00 (m, 2H), 7.72 (br t, *J* = 7.2 Hz, 1H), 7.59 (br t, *J* = 7.2 Hz, 1H), 7.31 (t, *J* = 7.6 Hz, 1H), 7.21 (d, *J* = 9.6 Hz, 1H), 5.53 - 5.48 (m, 1H), 1.59 (d, *J* = 7.2 Hz, 3H). |

**[Experimental Example]**

**Experimental Example 1: SOS-catalyzed nucleotide exchange assay**

SOS1 activity was measured by SOS1-mediated GTP loading quantification of $KRAS^{G12D}$. GTP-loaded $KRAS^{G12D}$ was detected by HTRF (Homogeneous Time Resolved Fluorescence) using anti-GST terbium (Cisbio, France) as a donor and a fluorescence GTP analogue (EDA-GTP-DY-647P1, [DY-647P1-labeled 2'/3'-O-(2-aminoethyl-carbamoyl)guanosine-5'-triphosphate (Jena bioscience, Germany)]) as an acceptor.

Assay buffer was composed of 10 mM HEPES pH 7.4, 150 mM NaCl, 5 mM $MgCl_2$, 1 mM DTT, 0.05% BSA Fraction V pH 7.0 (Sigma) and 0.0025% (v/v) Igepal (Sigma). $KRAS^{G12D}$ working solution was prepared by adding equal amounts of GST-$KRAS^{G12D}$ (100 nM) and anti-GST-terbium (2 nM; Cisbio, France) to the assay buffer and incubating on ice for 10 minutes. $SOS1_{cat}$ working solution was prepared by adding equal amounts of HIS-$SOS1_{cat}$ (160 nM; Cytoskeleton, US) and EDA-GTP-DY-647P1 (200 nM; Jena bioscience, Germany) to the assay buffer. Control solution was prepared by adding equal volumes of EDA-GTP-DY-647P1 and the assay buffer for normalization control to exclude GEF-independent GTP loading and background signals. In order to induce a reaction, 5 μL of the $SOS1_{cat}$ working solution or the control solution was injected into each well. 5 μL of a compound having various concentrations from 128 pM to 10 μM was added to wells containing the $SOS1_{cat}$ working solution and incubated at room temperature for 10 minutes to bind to SOS1.

10 μL of the $KRAS^{G12D}$ solution was added to each well and incubated for 30 minutes at room temperature while shaking. HTRF was measured using a Thermo Varioskan (excitation 334 nm, emission 665 nm). The $IC_{50}$ of each inhibitor was analyzed using Graphad Prism.

The SOS1 inhibitory activity of the Example compounds measured by the above method was evaluated based on the following criteria, and the results are shown in Table 29.

| $IC_{50}$ range | ≤1,000 nM | 1,000 nM < $IC_{50}$ ≤ 10,000 nM | >10,000 nM |
|---|---|---|---|
| Grade | A | B | C |

As shown in Table 29 below, the Example compounds herein exhibited excellent SOS1 inhibitory activity.

[Table 29]

| Example No. | **SOS-catalyzed nucleotide exchange assay** | Example No. | **SOS-catalyzed nucleotide exchange assay** |
|---|---|---|---|
| 1 | B | 81 | NA |
| 2 | B | 82 | NA |
| 3 | C | 83 | NA |
| 4 | B | 84 | NA |
| 5 | C | 85 | A |
| 6 | C | 86 | NA |
| 7 | C | 87 | NA |
| 8 | B | 88 | NA |
| 9 | B | 89 | A |
| 10 | B | 90 | NA |
| 11 | B | 91 | NA |
| 12 | C | 92 | NA |
| 13 | A | 93 | NA |
| 14 | B | 94 | A |
| 15 | A | 95 | A |
| 16 | A | 96 | A |
| 17 | B | 97 | B |
| 18 | B | 98 | NA |

(continued)

| Example No. | **SOS-catalyzed nucleotide exchange assay** | Example No. | **SOS-catalyzed nucleotide exchange assay** |
|---|---|---|---|
| 19 | B | 99 | NA |
| 20 | A | 100 | NA |
| 21 | A | 101 | NA |
| 22 | A | 102 | NA |
| 23 | A | 103 | NA |
| 24 | B | 104 | NA |
| 25 | A | 105 | NA |
| 26 | A | 106 | NA |
| 27 | A | 107 | NA |
| 28 | C | 108 | NA |
| 29 | A | 109 | NA |
| 30 | A | 110 | NA |
| 31 | A | 111 | NA |
| 32 | A | 112 | NA |
| 33 | A | 113 | A |
| 34 | B | 114 | NA |
| 35 | A | 115 | B |
| 36 | A | 116 | B |
| 37 | A | 117 | B |
| 38 | A | 118 | B |
| 39 | A | 119 | C |
| 40 | A | 120 | B |
| 41 | A | 121 | NA |
| 42 | A | 122 | NA |
| 43 | A | 123 | NA |
| 44 | A | 124 | B |
| 45 | A | 125 | B |
| 46 | A | 126 | A |
| 47 | A | 127 | B |
| 48 | B | 128 | A |
| 49 | B | 129 | A |
| 50 | B | 130 | A |
| 51 | A | 131 | NA |
| 52 | B | 132 | NA |
| 53 | B | 133 | NA |
| 54 | A | 134 | NA |
| 55 | A | 135 | NA |
| 56 | A | 136 | NA |
| 57 | A | 137 | A |

(continued)

| Example No. | **SOS-catalyzed nucleotide exchange assay** | Example No. | **SOS-catalyzed nucleotide exchange assay** |
|---|---|---|---|
| 58 | A | 138 | A |
| 59 | A | 139 | NA |
| 60 | C | 140 | A |
| 61 | B | 141 | A |
| 62 | A | 142 | A |
| 63 | B | 143 | A |
| 64 | B | 144 | B |
| 65 | A | 145 | NA |
| 66 | B | 146 | NA |
| 67 | A | 147 | NA |
| 68 | A | 148 | C |
| 69 | B | 149 | A |
| 70 | B | 150 | A |
| 71 | A | 151 | A |
| 72 | A | 152 | A |
| 73 | A | 153 | A |
| 74 | A | 154 | B |
| 75 | A | 155 | B |
| 76 | NA | 156 | B |
| 77 | NA | 157 | B |
| 78 | A | 158 | A |
| 79 | NA | 159 | A |
| 80 | NA | 160 | B |
| B01 | A | B07 | A |
| B02 | A | B08 | A |
| B03 | A | B09 | A |
| B04 | A | B10 | A |
| B05 | A | B11 | A |
| B06 | A | | |

**Experimental Example 2: $KRAS^{G12C}$::SOS1 protein-protein interaction (PPI) inhibition assay**

Protein-protein interaction between SOS1 and $KRAS^{G12C}$ was detected using KRAS G12C/SOS1 binding kit from CISBIO (64KRASG12PEH, CISBIO, FRANCE) according to the manufacturer's assay method. Briefly, the maximum concentration of an inhibitor sample was set to be 3 μM, and the inhibitor sample was diluted 7 times by 1/3, and samples having a total of 8 concentrations were prepared. In HTRF (Homogeneous Time-Resolved Fluorescence) exclusive 96-well low-volume white plate (66PL96005, CISBIO, FRANCE), 2 μL of an inhibitor sample prepared for each concentration, 5 μL of a mixed solution of Tag1-labeled $KRAS^{G12C}$ protein and GTP diluted with the dilution solution provided by the manufacturer, and 5 μL of Tag2 labeled SOS1 protein were mixed, and added with 8 μL of a diluted solution where anti-Tag1 $KRAS^{G12C}$ XL665 (HTRF acceptor) labeled antibody and anti-Tag2 SOS1 Terbium cryptate (HTRF donor) labeled antibody were mixed in an equal ratio and incubated at room temperature for 2 h. Thereafter, the HTRF signal generated by the antibody was measured using a Thermo Varioskan multi-purpose plate signal detector, and the result value was

calculated as the ratio of the signals emitted at 665 nm and 620 nm as shown below.

$$\text{Result value} = 10^4 \text{ X } 665 \text{ nm signal}/620 \text{ nm signal}$$

The $IC_{50}$ value of each inhibitor was analyzed with the result values of three replicates by using the Graphad Prism 9.0 software.

The degree of inhibition of the Example compounds on SOS1 measured by the above method was evaluated based on the following criteria, and the results are shown in Table 30.

| $IC_{50}$ range | ≤1,000 nM | 1,000 nM < $IC_{50}$ ≤ 10,000 nM | >10,000 nM |
|---|---|---|---|
| Grade | A | B | C |
| NA = Not Applied | | | |

As shown in Table 30 below, the Example compounds herein exhibited excellent SOS1 inhibitory activity.

[Table 30]

| Example No. | **KRAS G12C::SOS1 PPI assay** | Example No. | **KRAS G12C::SOS1 PPI assay** |
|---|---|---|---|
| 140 | A | 278 | A |
| 141 | A | 279 | A |
| 142 | A | 280 | A |
| 143 | A | 281 | A |
| 144 | A | 282 | A |
| 145 | A | 283 | A |
| 146 | A | 284 | A |
| 147 | A | 285 | A |
| 148 | C | 286 | A |
| 149 | A | 287 | A |
| 150 | A | 288 | A |
| 151 | A | 289 | A |
| 152 | A | 290 | A |
| 153 | A | 291 | A |
| 154 | A | 292 | A |
| 155 | A | 293 | A |
| 156 | B | 294 | A |
| 157 | B | 295 | A |
| 158 | A | 296 | A |
| 159 | A | 297 | A |
| 160 | A | 298 | A |
| 161 | A | 299 | B |
| 162 | A | 300 | A |
| 163 | A | 301 | A |
| 164 | A | 302 | A |
| 165 | A | 303 | C |
| 166 | A | 304 | C |

(continued)

| Example No. | **KRAS $^{G12C}$::SOS1 PPI assay** | Example No. | **KRAS $^{G12C}$::SOS1 PPI assay** |
|---|---|---|---|
| 167 | A | 305 | C |
| 168 | A | 306 | C |
| 169 | A | 307 | A |
| 170 | A | 308 | A |
| 171 | A | 309 | A |
| 172 | A | 310 | A |
| 173 | A | 311 | A |
| 174 | A | 312 | A |
| 175 | B | 313 | A |
| 176 | C | 314 | A |
| 177 | A | 315 | B |
| 178 | A | 316 | A |
| 179 | A | 317 | A |
| 180 | A | 318 | A |
| 181 | A | 319 | A |
| 182 | A | 320 | A |
| 183 | A | 321 | A |
| 184 | A | 322 | A |
| 185 | A | 323 | A |
| 186 | A | 324 | A |
| 187 | A | 325 | A |
| 188 | A | 326 | A |
| 189 | A | 327 | A |
| 190 | A | 328 | A |
| 191 | A | 329 | A |
| 192 | A | 330 | A |
| 193 | A | 331 | A |
| 194 | A | 332 | A |
| 195 | A | 333 | A |
| 196 | A | 334 | A |
| 197 | A | 335 | A |
| 198 | A | 336 | A |
| 199 | A | 337 | A |
| 200 | A | 338 | A |
| 201 | A | 339 | A |
| 202 | A | 340 | A |
| 203 | A | 341 | A |
| 204 | A | 342 | A |
| 205 | A | 343 | A |

(continued)

| Example No. | **KRAS $^{G12C}$::SOS1 PPI assay** | Example No. | **KRAS $^{G12C}$::SOS1 PPI assay** |
|---|---|---|---|
| 206 | A | 344 | A |
| 207 | A | 345 | A |
| 208 | A | 346 | A |
| 209 | A | 347 | A |
| 210 | A | 348 | A |
| 211 | A | 349 | A |
| 212 | B | 350 | A |
| 213 | A | 351 | A |
| 214 | A | 352 | A |
| 215 | A | 353 | A |
| 216 | B | 354 | A |
| 217 | A | 355 | A |
| 218 | A | 356 | A |
| 219 | A | 357 | B |
| 220 | A | 358 | A |
| 221 | A | 359 | A |
| 222 | A | 360 | A |
| 223 | A | 361 | A |
| 224 | A | 362 | A |
| 225 | A | 363 | C |
| 226 | A | 364 | B |
| 227 | A | 365 | C |
| 228 | A | 366 | B |
| 229 | B | 367 | B |
| 230 | A | 368 | A |
| 231 | A | 369 | A |
| 232 | A | 370 | A |
| 233 | A | 371 | A |
| 234 | A | 372 | A |
| 235 | A | 373 | A |
| 236 | A | 374 | A |
| 237 | A | 375 | A |
| 238 | A | 376 | A |
| 239 | B | 377 | A |
| 240 | A | 378 | A |
| 241 | A | 379 | A |
| 242 | A | 380 | A |
| 243 | A | 381 | A |
| 244 | A | 382 | A |

(continued)

| Example No. | **KRAS $^{G12C}$::SOS1 PPI assay** | Example No. | **KRAS $^{G12C}$::SOS1 PPI assay** |
|---|---|---|---|
| 245 | A | 383 | A |
| 246 | A | 384 | A |
| 247 | A | 385 | A |
| 248 | A | 386 | A |
| 249 | A | 387 | A |
| 250 | A | 388 | C |
| 251 | A | 389 | A |
| 252 | A | 390 | A |
| 253 | A | 391 | A |
| 254 | A | 392 | A |
| 255 | A | 393 | A |
| 256 | A | 394 | C |
| 257 | A | 395 | B |
| 258 | A | 396 | A |
| 259 | A | 397 | A |
| 260 | A | 398 | A |
| 261 | C | 399 | A |
| 262 | B | 400 | A |
| 263 | A | 401 | A |
| 264 | A | 402 | A |
| 265 | A | 403 | A |
| 266 | A | 404 | A |
| 267 | A | 405 | A |
| 268 | A | 406 | A |
| 269 | A | 407 | A |
| 270 | A | 408 | A |
| 271 | A | 409 | A |
| 272 | A | 410 | A |
| 273 | A | 411 | A |
| 274 | A | 412 | A |
| 275 | A | 413 | A |
| 276 | A | 414 | A |
| 277 | A | 415 | A |
| B01 | A | B07 | A |
| B02 | A | B08 | A |
| B03 | A | B09 | A |
| B04 | A | B10 | A |
| B05 | A | B11 | A |
| B06 | A | | |

**Experimental Example 3: Evaluation of cancer cell growth inhibition according to combined administration**

When the example compound was used in combination with an anticancer agent, the effect on the growth ability of cancer cells expressing KRAS variant or EGFR variant was confirmed. In order to confirm the effect of the example compound in combination with a MEK inhibitor or a KRAS G12C inhibitor in the cell line having the KRAS variant, the cell growth inhibitory effect by the treatment of the example compound in combination with a MEK inhibitor or a KRAS G12C inhibitor was confirmed in the gastric cancer cell line SNU-1 and the pancreatic cancer cell line KP4 having the KRAS G12D variant, the lung cancer cell line NCI-H358 and the pancreatic cancer cell line MIA PaCa-2 having the KRAS G12C variant, the colorectal cancer cell line SW480 having the KRAS G12V variant, the lung cancer cell line A549 having the KRAS G12S variant, the colorectal cancer cell line LoVo having the KRAS G13D variant. In addition, in order to confirm the effect of the example compound in combination with an EGFR inhibitor in the cell line having the EGFR variant, the cell growth inhibitory effect by the treatment of the example compound in combination with an EGFR inhibitor was confirmed in the lung cancer cell line H1975 having the EGFR L858R/T790M variant and the lung cancer cell line HCC827 having the EGFR Del19 variant.

**Experimental Example 3.1: Preparation of Cell dispensing well plate and test solution**

The cell line stored in the liquid nitrogen tank was taken out, quickly thawed at 37 °C, then diluted with 10 times the volume of cell culture medium, and centrifuged to remove the frozen storage medium. The recovered cell pellet was mixed well with the culture medium, placed in a culture flask, and cultured under conditions of 37 °C and 5% carbon dioxide to stabilize the cells. Each cell was cultured in RPMI 1640 Medium (1x) or Dulbecco Modified Eagle Medium (DMEM, 1x) medium containing 10% (v/v) FBS and 1% (v/v) penicillin/streptomycin. Thereafter, the cells were obtained from the flask, centrifuged to remove the culture medium, and washed with PBS (Phosphate Buffered Saline), and then centrifuged again to remove the PBS. The cells were diluted with culture medium to 1.25 x $10^4$ cells/mL and dispensed at 40 μL per well in a 384-well plate. In addition, the cells were diluted with medium to 5 x $10^4$ cells/mL and dispensed at 100 μL per well in a 96-well plate. Thereafter, the plate onto which the cells were dispensed was cultured for 24 h under conditions of 37 °C and 5% carbon dioxide.

Example 46, Example 295, Example B01, Example B02, Example 369, Example 352, Example B03, Example B04, Example 326, Example B05, Example B06, Example B07, Example 141, Example B08, Example B09, Example B10, Example B11, a MEK inhibitor, a KRAS G12C inhibitor and an EGFR inhibitor (INK-128, sotorasib, adagrasib, trametinib, osimertinib, lazertinib, etc.) were each dissolved in 99.5% (v/v) dimethyl sulfoxide (hereinafter, DMSO) to a concentration of 10 mM.

**Experimental Example 3.2: Evaluation of cell viability of lung cancer cell NCI-H358 according to combined administration with INK-128**

A well plate in which the lung cancer cell line NCI-H358 having the KRAS G12C variant was dispensed was treated with test solutions of the compound of Example 295 and INK-128.

The compound of Example 295 was treated so that its final concentration became 0 nM, 4 nM, 11 nM, 33 nM, 100 nM and 300 nM per well, respectively. For each concentration of the compound of Example 295, INK-128 was treated so that its final concentration became 3.2 nM, 4.9 nM, 7.5 nM, 11.6 nM, 17.9 nM, 27.5 nM, 42.3 nM, 65 nM and 100 nM per well, respectively.

The cells treated with the test solutions were cultured for 72 h under conditions of 37 °C and 5% carbon dioxide. Thereafter, the plates in which the cells were cultured were adapted to room temperature for 30 minutes, and then 50 μL of Cell Titer-Glo Reagent (CTG, Promega) was dispensed per well into a 384-well plate and a 96-well plate. Cell lysis was induced by mixing the contents in the 386-well plate for 2 minutes and mixing the contents in the 96-well plate for 10 minutes, the luminescence signal was stabilized for 10 minutes, and then the luminescence intensity was measured using an EnVision™ LUX multimode microplate reader or a Varioskan™ LUX microplate reader. Based on the measured value, the cell viability in the well-treated with each compound was calculated when the final value of the well not treated with the test substance was set to 100%, thereby obtaining the graph shown in FIG. 1.

Referring to FIG. 1, it was confirmed that the cell viability of the lung cancer cell NCI-H358 was decreased when treated with INK-128 and the compound of Example 295 in combination compared to when treated with INK-128 alone.

**Experimental Example 3.3: Evaluation of cell viability of lung cancer cell NCI-H358 according to combined administration with sotorasib**

A well plate in which the lung cancer cell line NCI-H358 having the KRAS G12C variant was dispensed was treated with test solutions of the compound of Example 295 and sotorasib.

The compound of Example 295 was treated so that its final concentration became 0 nM, 4 nM, 11 nM, 33 nM, 100 nM and 300 nM per well, respectively. For each concentration of the compound of Example 295, sotorasib was treated so that its final concentration became 0.14 nM, 0.24 nM, 0.41 nM, 0.70 nM, 1.20 nM, 2.04 nM, 3.46 nM, 5.88 nM, and 10 nM per well, respectively.

In addition, a well plate in which NCI-H358 was dispensed was treated with test solutions of sotorasib and the example compounds.

The cells were treated by adding 10 μL of a test solution of sotorasib, Example B01, Example B02, Example 369, Example 352 and Example B03, respectively, alone or sotorasib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graphs in FIGs. 2 and 3.

Referring to FIG. 2, it was confirmed that the cell viability of the lung cancer cell NCI-H358 was decreased when treated with sotorasib and the compound of Example 295 in combination compared to when treated with sotorasib alone.

Referring to FIG. 3, it was confirmed that the cell viability of the lung cancer cell NCI-H358 was decreased when treated with sotorasib and the example compound in combination compared to when treated with sotorasib and the example compound, respectively, alone.

### Experimental Example 3.4: Evaluation of cell viability of pancreatic cancer cell MIA PaCa-2 according to combined administration with sotorasib

A well plate in which the pancreatic cancer cell line MIA PaCa-2 having the KRAS G12C variant was dispensed was treated with a test solution of sotorasib and the example compounds.

The cells were treated by adding 10 μL of a test solution of sotorasib, Example B01, Example B02, Example 369, Example 352 and Example B03, respectively, alone or sotorasib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graph in FIG. 4.

Referring to FIG. 4, it was confirmed that the cell viability of the pancreatic cancer cell MIA PaCa-2 was decreased when treated with sotorasib and the example compound in combination compared to when treated with sotorasib and the example compound, respectively, alone.

### Experimental Example 3.5: Evaluation of cell viability of lung cancer cell NCI-H358 according to combined administration with adagrasib

A well plate in which the lung cancer cell line NCI-H358 having the KRAS G12C variant was dispensed was treated with a test solution of adagrasib and the example compounds.

The cells were treated by adding 10 μL of a test solution of adagrasib, Example B01, Example B02, Example 369, Example 352 and Example B03, respectively, alone or adagrasib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graph in FIG. 5.

Referring to FIG. 5, it was confirmed that the cell viability of the lung cancer cell NCI-H358 was decreased when treated with adagrasib and the example compound in combination compared to when treated with adagrasib and the example compound, respectively, alone.

### Experimental Example 3.6: Evaluation of cell viability of pancreatic cancer cell MIA PaCa-2 according to combined administration with adagrasib

A well plate in which the pancreatic cancer cell line MIA PaCa-2 having the KRAS G12C variant was dispensed

was treated with a test solution of adagrasib and the example compounds.

The cells were treated by adding 10 μL of a test solution of adagrasib, Example B01, Example B02, Example 369, Example 352 and Example B03, respectively, alone or adagrasib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graph in FIG. 6.

Referring to FIG. 6, it was confirmed that the cell viability of the pancreatic cancer cell MIA PaCa-2 was decreased when treated with adagrasib and the example compound in combination compared to when treated with adagrasib and the example compound, respectively, alone.

### Experimental Example 3.7: Evaluation of cell viability of lung cancer cell NCI-H358 according to combined administration with trametinib

A well plate in which the lung cancer cell line NCI-H358 having the KRAS G12C variant was dispensed was treated with a test solution of trametinib and the example compounds.

The cells were treated by adding 10 μL of a test solution of trametinib, Example B01, Example B02, Example 369, Example 352, Example B03, Example B04, Example 326, Example B05, Example B06 and Example B07, respectively, alone or trametinib in combination with one of Example B01, Example B02, Example 369, Example 352, Example B03, Example B04, Example 326, Example B05, Example B06 and Example B07 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graphs in FIGs. 7 and 8.

Referring to FIGs. 7 and 8, it was confirmed that the cell viability of the lung cancer cell NCI-H358 was decreased when treated with trametinib and the example compound in combination compared to when treated with trametinib and the example compound, respectively, alone.

### Experimental Example 3.8: Evaluation of cell viability of gastric cancer cell SNU-1 according to combined administration with trametinib

A well plate in which the gastric cancer cell line SNU-1 having the KRAS G12D variant was dispensed was treated with a test solution of trametinib and the example compounds.

The cells were treated by adding 10 μL of a test solution of trametinib, Example B01, Example B02, Example 369, Example 352, Example B03, Example B04, Example 326, Example B05, Example B06 and Example B07, respectively, alone or trametinib in combination with one of Example B01, Example B02, Example 369, Example 352, Example B03, Example B04, Example 326, Example B05, Example B06 and Example B07 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graphs in FIGs. 9 and 10.

Referring to FIGs. 9 and 10, it was confirmed that the cell viability of the gastric cancer cell SNU-1 was decreased when treated with trametinib and the example compound in combination compared to when treated with trametinib and the example compound, respectively, alone.

### Experimental Example 3.9: Evaluation of cell viability of colorectal cancer cell SW480 according to combined administration with trametinib

A well plate in which the colorectal cancer cell line SW480 having the KRAS G12V variant was dispensed was treated with a test solution of trametinib and the example compounds.

The cells were treated by adding 10 μL of a test solution of trametinib, Example B01, Example B02, Example 369, Example 352, Example B03, Example B04, Example 326, Example B05, Example B06 and Example B07, respectively, alone or trametinib in combination with one of Example B01, Example B02, Example 369, Example 352, Example B03, Example B04, Example 326, Example B05, Example B06 and Example B07 to a 384-well plate so that the final

concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graphs in FIGs. 11 and 12.

Referring to FIGs. 11 and 12, it was confirmed that the cell viability of the colorectal cancer cell SW480 was decreased when treated with trametinib and the example compound in combination compared to when treated with trametinib and the example compound, respectively, alone.

**Experimental Example 3.10: Evaluation of cell viability of lung cancer cell H1975 according to combined administration with osimertinib**

A well plate in which the lung cancer cell line H1975 having the EGFR L858R/T790M variant was dispensed was treated with a test solution of osimertinib and the example compounds.

The cells were treated by adding 10 μL of a test solution of osimertinib, Example B01, Example B02, Example 369, Example 352 and Example B03, respectively, alone or osimertinib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graph in FIG. 13.

Referring to FIG. 13, it was confirmed that the cell viability of the lung cancer cell H1975 was decreased when treated with osimertinib and the example compound in combination compared to when treated with osimertinib and the example compound, respectively, alone.

**Experimental Example 3.11: Evaluation of cell viability of lung cancer cell HCC827 according to combined administration with osimertinib**

A well plate in which the lung cancer cell line HCC827 having the EGFR Del19 variant was dispensed was treated with a test solution of osimertinib and the example compounds.

The cells were treated by adding 10 μL of a test solution of osimertinib, Example B01, Example B02, Example 369, Example 352 and Example B03, respectively, alone or osimertinib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graph in FIG. 14.

Referring to FIG. 14, it was confirmed that the cell viability of the lung cancer cell HCC827 was decreased when treated with osimertinib and the example compound in combination compared to when treated with osimertinib and the example compound, respectively, alone.

**Experimental Example 3.12: Evaluation of cell viability of lung cancer cell H1975 according to combined administration with lazertinib**

A well plate in which the lung cancer cell line H1975 having the EGFR L858R/T790M variant was dispensed was treated with a test solution of lazertinib and the example compounds.

The cells were treated by adding 10 μL of a test solution of lazertinib, Example B01, Example B02, Example 369, Example 352 and Example B03, respectively, alone or lazertinib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graph in FIG. 15.

Referring to FIG. 15, it was confirmed that the cell viability of the lung cancer cell H1975 was decreased when treated with lazertinib and the example compound in combination compared to when treated with lazertinib and the

example compound, respectively, alone.

**Experimental Example 3.13: Evaluation of cell viability of lung cancer cell HCC827 according to combined administration with lazertinib**

A well plate in which the lung cancer cell line HCC827 having the EGFR Del19 variant was dispensed was treated with a test solution of lazertinib and the example compounds.

The cells were treated by adding 10 μL of a test solution of lazertinib, Example B01, Example B02, Example 369, Example 352 and Example B03, respectively, alone or lazertinib in combination with one of Example B01, Example B02, Example 369, Example 352 and Example B03 to a 384-well plate so that the final concentration of each substance was 100 nM to 97.7 pM in 50 μL of the solution per well. In a 96-well plate, the cells were treated by diluting each substance in medium at 3 times the treatment concentration and adding 50 μL per well so that the final concentration of each substance was 120 nM to 2 nM in 150 μL of a solution.

The cells treated with the test solution were evaluated in the same manner as in Experimental Example 3.1 to obtain the graph in FIG. 16.

Referring to FIG. 16, it was confirmed that the cell viability of the lung cancer cell HCC827 was decreased when treated with lazertinib and the example compound in combination compared to when treated with lazertinib and the example compound, respectively, alone.

**Experimental Example 3.14: Evaluation of cell viability of breast cancer cell MCF7 according to combined administration with alpelisib**

A well plate in which PIK3CA mutant breast cancer cell line MCF7 was dispensed was treated with a test solution of alpelisib and the example compounds.

The cells were treated by adding 10 μL of a test solution of alpelisib, Example B01, Example B03, Example B08, Example B09 and Example B10, respectively, alone or alpelisib in combination with one of Example B01, Example B03, Example B08, Example B09 and Example B10 to a 384-well plate so that the final concentration of each substance was 100 nM to 97.7 pM in 50 μL of the solution per well.

The cells treated with the test solution were cultured under conditions of 37 °C and 5% carbon dioxide for 72 h. Thereafter, the plate with the cells cultured was adapted to room temperature for 30 minutes. Then, in order to quantify cytotoxicity, 50 μL of Cell Titer-Glo Reagent (CTG, Promega), which measures the amount of ATP, was dispensed per well into a 384-well plate. Cell lysis was induced by mixing the contents for 2 minutes, the luminescence signal was stabilized for 10 minutes, and then the luminescence intensity was measured using a Varioskan™ LUX microplate reader. The cell viability in the well-treated with each compound was calculated when the final value of the well not treated with the test substance was set to 100%.

Referring to FIG. 17, it was confirmed that the cell viability of the breast cancer cell MCF7 was decreased when treated with alpelisib and the example compound in combination compared to when treated with alpelisib and the example compound, respectively, alone.

**Experimental Example 3.15: Evaluation of cell viability of lung cancer cell H358 according to combined administration with JDQ443**

A well plate in which the lung cancer cell line H358 having the KRAS G12C variant was dispensed was treated with a test solution of JDQ443 and the example compounds.

The cells were treated by adding 10 μL of a test solution of JDQ443 and Example B01, respectively, alone or JDQ443 in combination with Example B01 to a 384-well plate so that the final concentration of JDQ443 was 100 nM to 6.1 pM and the final concentration of Example B01 was 30 μM to 13.7 nM in 50 μL of the solution per well.

The cells treated with the test solution were evaluated in the same manner as in Experimental Example 3.14 to obtain the graph in FIG. 18.

Referring to FIG. 18, it was confirmed that the cell viability of the lung cancer cell H358 was decreased when treated with JDQ443 and the example compound in combination compared to when treated with JDQ443 and the example compound, respectively, alone.

**Experimental Example 3.16: Evaluation of cell viability of lung cancer cell H358 according to combined administration with TNO155**

A well plate in which the lung cancer cell line H358 having the KRAS G12C variant was dispensed was treated with a test solution of TNO155 and the example compounds.

The cells were treated by adding 10 μL of a test solution of TNO155, Example B01 and Example B11, respectively, alone or TNO155 in combination with one of Example B01 and Example B11 to a 384-well plate so that the final concentration of each substance was 10 μM to 41.1 nM in 50 μL of the solution per well.
The cells treated with the test solution were evaluated in the same manner as in Experimental Example 3.14 to obtain the graph in FIG. 19.
Referring to FIG. 19, it was confirmed that the cell viability of the lung cancer cell H358 was decreased when treated with TNO155 and the example compound in combination compared to when treated with TNO155 and the example compound, respectively, alone.

**Experimental Example 3.17: Evaluation of cell viability of lung cancer cell H358 according to combined administration with cisplatin**

A well plate in which the lung cancer cell line H358 having the KRAS G12C variant was dispensed was treated with a test solution of cisplatin and the example compounds.
The cells were treated by adding 10 μL of a test solution of cisplatin, Example B01 and Example B11, respectively, alone or cisplatin in combination with one of Example B01 and Example B11 to a 384-well plate so that the final concentration of each substance was 5 μM to 156 nM in 50 μL of the solution per well.
The cells treated with the test solution were evaluated in the same manner as in Experimental Example 3.14 to obtain the graph in FIG. 20.
Referring to FIG. 20, it was confirmed that the cell viability of the lung cancer cell H358 was decreased when treated with cisplatin and the example compound in combination compared to when treated with cisplatin and the example compound, respectively, alone.

**Experimental Example 3.18: Evaluation of cell viability of lung cancer cell H358 according to combined administration with rineterkib**

A well plate in which the lung cancer cell line H358 having the KRAS G12C variant was dispensed was treated with a test solution of rineterkib and the example compounds.
The cells were treated by adding 10 μL of a test solution of rineterkib, Example B01 and Example B11, respectively, alone or rineterkib in combination with one of Example B01 and Example B11 to a 384-well plate so that the final concentration of each substance was 1 μM to 31.3 nM in 50 μL of the solution per well.
The cells treated with the test solution were evaluated in the same manner as in Experimental Example 3.14 to obtain the graph in FIG. 21.
Referring to FIG. 21, it was confirmed that the cell viability of the lung cancer cell H358 was decreased when treated with rineterkib and the example compound in combination compared to when treated with rineterkib and the example compound, respectively, alone.

**Experimental Example 3.19: Evaluation of cell viability of lung cancer cell H358 according to combined administration with ulixertinib**

A well plate in which the lung cancer cell line H358 having the KRAS G12C variant was dispensed was treated with a test solution of ulixertinib and the example compounds.
The cells were treated by adding 10 μL of a test solution of ulixertinib, Example B01 and Example B11, respectively, alone or ulixertinib in combination with one of Example B01 and Example B11 to a 384-well plate so that the final concentration of each substance was 1 μM to 31.3 nM in 50 μL of the solution per well.
The cells treated with the test solution were evaluated in the same manner as in Experimental Example 3.14 to obtain the graph in FIG. 22.
Referring to FIG. 22, it was confirmed that the cell viability of the lung cancer cell H358 was decreased when treated with ulixertinib and the example compound in combination compared to when treated with ulixertinib and the example compound, respectively, alone.

**Experimental Example 3.20: Evaluation of cell viability of lung cancer cell H358 according to combined administration with pralsetinib**

A well plate in which the lung cancer cell line H358 having the KRAS G12C variant was dispensed was treated with a test solution of pralsetinib and the example compounds.
The cells were treated by adding 10 μL of a test solution of pralsetinib, Example B01 and Example B11, respectively, alone or pralsetinib in combination with one of Example B01 and Example B11 to a 384-well plate so that the

final concentration of each substance was 1 μM to 31.3 nM in 50 μL of the solution per well.

The cells treated with the test solution were evaluated in the same manner as in Experimental Example 3.14 to obtain the graph in FIG. 23.

Referring to FIG. 23, it was confirmed that the cell viability of the lung cancer cell H358 was decreased when treated with pralsetinib and the example compound in combination compared to when treated with pralsetinib and the example compound, respectively, alone.

**Experimental Example 3.21: Evaluation of cell viability of lung cancer cell H358 according to combined administration with repotrectinib**

A well plate in which the lung cancer cell line H358 having the KRAS G12C variant was dispensed was treated with a test solution of repotrectinib and the example compounds.

The cells were treated by adding 10 μL of a test solution of repotrectinib, Example B01 and Example B11, respectively, alone or repotrectinib in combination with one of Example B01 and Example B11 to a 384-well plate so that the final concentration of each substance was 1 μM to 31.3 nM in 50 μL of the solution per well.

The cells treated with the test solution were evaluated in the same manner as in Experimental Example 3.14 to obtain the graph in FIG. 24.

Referring to FIG. 24, it was confirmed that the cell viability of the lung cancer cell H358 was decreased when treated with repotrectinib and the example compound in combination compared to when treated with repotrectinib and the example compound, respectively, alone.

**Experimental Example 3.22: Evaluation of cell viability of gastric cancer cell SNU-5 according to combined administration with tepotinib**

A well plate in which the c-Met overexpressing gastric cancer cell line SNU-5 was dispensed was treated with a test solution of tepotinib and the example compounds.

The cells were treated by adding 10 μL of a test solution of tepotinib and Example B01, respectively, alone or tepotinib in combination with Example B01 to a 384-well plate so that the final concentration of each substance was 10 μM to 9.77 nM in 50 μL of the solution per well.

The cells treated with the test solution were evaluated in the same manner as in Experimental Example 3.14 to obtain the graph in FIG. 25.

Referring to FIG. 25, it was confirmed that the cell viability of the gastric cancer cell SNU-5 was decreased when treated with tepotinib and the example compound in combination compared to when treated with tepotinib and the example compound, respectively, alone.

**Experimental Example 3.23: Evaluation of cell viability of lung cancer cell PC-9 according to combined administration with bemcentinib**

A well plate in which the lung cancer cell line PC-9 with high AXL expression was dispensed was treated with a test solution of bemcentinib and the example compounds.

The cells were treated by adding 10 μL of a test solution of bemcentinib and Example B01, respectively, alone or bemcentinib in combination with Example B01 to a 384-well plate so that the final concentration of each substance was 10 μM to 9.77 nM in 50 μL of the solution per well.

The cells treated with the test solution were evaluated in the same manner as in Experimental Example 3.14 to obtain the graph in FIG. 26.

Referring to FIG. 26, it was confirmed that the cell viability of the lung cancer cell PC-9 was decreased when treated with bemcentinib and the example compound in combination compared to when treated with bemcentinib and the example compound, respectively, alone.

**Experimental Example 3.24: Evaluation of cell viability of lung cancer cell A549 according to combined administration with alrizomadlin**

A well plate in which the lung cancer cell line A549 having the KRAS G12S variant was dispensed was treated with a test solution of alrizomadlin and the example compounds.

The cells were treated by adding 10 μL of a test solution of alrizomadlin and Example B01, respectively, alone or alrizomadlin in combination with Example B01 to a 384-well plate so that the final concentration of each substance was 10 μM to 9.77 nM in 50 μL of the solution per well.

The cells treated with the test solution were evaluated in the same manner as in Experimental Example 3.14 to

obtain the graph in FIG. 27.

Referring to FIG. 27, it was confirmed that the cell viability of the lung cancer cell A549 was decreased when treated with alrizomadlin and the example compound in combination compared to when treated with alrizomadlin and the example compound, respectively, alone.

### Experimental Example 3.25: Evaluation of cell viability of colorectal cancer cell LoVo according to combined administration with everolimus

A well plate in which the colorectal cancer cell line LoVo having the KRAS G13D variant was dispensed was treated with a test solution of everolimus and the example compounds.

The cells were treated by adding 10 μL of a test solution of everolimus and Example B01, respectively, alone or everolimus in combination with Example B01 to a 384-well plate so that the final concentration of each substance was 10 μM to 9.77 nM in 50 μL of the solution per well.

The cells treated with the test solution were evaluated in the same manner as in Experimental Example 3.14 to obtain the graph in FIG. 28.

Referring to FIG. 28, it was confirmed that the cell viability of the colorectal cancer cell LoVo was decreased when treated with everolimus and the example compound in combination compared to when treated with everolimus and the example compound, respectively, alone.

### Experimental Example 3.26: Evaluation of cell viability of pancreatic cancer cell AsPC-1 according to combined administration with MRTX1133

A well plate in which the pancreatic cancer cell line AsPC-1 having the KRAS G12D variant was dispensed was treated with a test solution of MRTX1133 and the example compounds.

The cells were treated by adding 50 μL of a test solution of MRTX1133, Example B01 and Example B11, respectively, alone or MRTX1133 in combination with one of Example B01 and Example B11 to a 96-well plate so that the final concentration of MRTX1133 was 100 nM to 20 nM and the final concentration of the example compound was 5 μM in 150 μL of the solution per well.

The cells treated with the test solution were evaluated and the cell viability of a well-treated with test solution alone or in combination was evaluated in the same manner as in Experimental Example 3.1 to obtain the graph in FIG. 29.

Referring to FIG. 29, it was confirmed that the cell viability of the pancreatic cancer cell AsPC-1 was decreased when treated with MRTX1133 and the example compound in combination compared to when treated with MRTX1133 and the example compound, respectively, alone.

### Experimental Example 4.1: Determination of combination concentration by SynergyScreen™

The cells were treated by adding 5 μL of a test solution of Example B01 per well to a 384 well plate in which the lung cancer cell line H358 having the KRAS G12C variant was dispensed so that the final concentration of the substance was 31.6 μM to 0.00316 μM in 50 μL of the solution per well. The cells treated with Example B01 were cultured under conditions of 37 °C and 5% carbon dioxide for 72 h or 120 h. In order to measure cell viability, each well was treated with 24 μL of ATPlite 1Step™ (PerkinElmer) solution, then mixed for 2 minutes, and reacted by blocking light for 5 minutes, and then the luminescence signal was measured using an Envision multilabel reader (PerkinElmer). The cell viability was determined according to the formula below, and the concentration ($EFFECT_{20}$) of Example B01 that induces 20% cell death was calculated using IDBS XLfit 5. The $EFFECT_{20}$ concentration of Example B01 determined through the experiment was applied as the final treatment concentration of Example B01 during the combination effect experiment.

% growth= 100% x (luminescence treated wells, t=end / luminescence of untreated wells, t=end).

### Experimental Example 4.2: Evaluation of combination efficacy by SynergyScreen™

In order to confirm the effect of Example B01 in combination with various anticancer agents, a total of 42 anticancer agents, including cytotoxic anticancer agents and targeted anticancer agents, were used for evaluation (Table 31). A 384 well plate in which the lung cancer cell line H358 having the KRAS G12C variant was dispensed was treated with Example B01 at a final concentration of 1854 nM when the culture time was 72 h and was treated with Example B01 at a final concentration of 652 nM when the culture time was 120 h, respectively. After treatment of Example B01, the plate was treated with the combined anticancer agent so that the final concentration of each substance was 31.6 μM to 0.00316 μM in 50 μL of the solution per well. The cells treated with the test solution were cultured under conditions of 37 °C and 5% carbon dioxide for the culture time indicated in Table 31. In order to measure cell viability, each well was treated with 24 μL

of ATPlite Step™ (PerkinElmer) solution, then mixed for 2 minutes, and reacted by blocking light for 5 minutes, and then the luminescence signal was measured using an Envision multilabel reader (PerkinElmer). The cell viability was determined in the same manner as in Experimental Example 4.1, and the effect of the combination treatment was evaluated by comparing the cell viability of the group treated with the combined anticancer agent or Example B01 alone with the cell viability of the combined treatment.

Synergy 1 (Equation 1) was determined from the decrease in cell viability (%) (increase in cell death) when used in combination with Example B01 compared to the cell viability (%) when the combined anticancer agent was used alone, and Synergy 2 (Equation 2) was determined to be the difference between Synergy 1 and cell death rate (%) when Example B01 was used alone.

Synergy 1 (%) = cell viability (%) when the combined anticancer agent is used alone - cell viability (%) when used in combination with the example compound [Equation 1]

Synergy 2 (%) = Synergy 1 (%) - cell death rate (%) when the example compound is used alone [Equation 2]

If the synergy by treatment with Example B01 in combination compared to the combined anticancer agent alone is 50% or more, it is indicated as "++++", if it is less than 50% and more than 30%, it is indicated as "+++", if it is less than 30% and more than 10%, it is indicated as "++", and if it is less than 10%, it is indicated as "+". The results are shown in FIGs. 30a to 31c.

[Table 31]

| No. | Anticancer agent | Target/ Mechanism | Culture time (h) | concentration (log M) | | Synergy 1 | Synergy 2 |
|---|---|---|---|---|---|---|---|
| | | | | Anticancer agent | Ex. B01 | | |
| 1 | Paclitaxel | Taxane derivatives | 72 | -9.0 | -5.9 | ++ | ++ |
| 2 | Cytarabine | Antimetabolite | 72 | -8.5 | -5.9 | ++ | - |
| 3 | Palbociclib | CDK4/6 inhibition | 72 | -8.5 | -5.9 | ++ | - |
| 4 | Nintedanib | Angiogenesis inhibition or FGFR1/FGFR 2/FGFR3 inhibition | 72 | -6.0 | -5.9 | ++ | ++ |
| 5 | Regorafenib | Angiogenesis inhibition | 72 | -8.5 | -5.9 | ++ | + |
| 6 | SN-38 | TOP1 inhibition | 72 | -10.5 | -5.9 | ++ | - |
| 7 | Doxorubicin | TOP2 | 72 | -9.0 | -5.9 | ++++ | ++ |
| | | inhibition | | | | | |
| 8 | Niraparib | PARP inhibition | 72 | -8.5 | -5.9 | ++ | - |
| 9 | JQ1 | BET inhibition | 120 | -8.5 | -6.3 | ++ | - |
| 10 | NVP-ADW742 | IGF1/2 or IGF1-R inhibition | 72 | -8.5 | -5.9 | ++ | - |
| 11 | duvelisib | PIK inhibition | 72 | -7.0 | -5.9 | ++ | + |
| 12 | Gefitinib | EGFR inhibition | 72 | -7.0 | -5.9 | +++ | ++ |
| 13 | Irbinitinib (tucatinib) | ErbB2(HER2) inhibition | 72 | -6.5 | -5.9 | ++ | ++ |
| 14 | Neratinib | HER2, EGFR inhibition | 72 | -9.0 | -5.9 | +++ | ++ |
| 15 | Alectinib | ALK inhibition | 72 | -6.0 | -5.9 | ++ | - |
| 16 | Cobimetinib | MEK inhibition | 72 | -8.0 | -5.9 | +++ | ++ |
| 17 | Imatinib | BCR-ABL inhibition | 72 | -8.5 | -5.9 | ++ | + |
| 18 | Dasatinib | | 72 | -8.0 | -5.9 | +++ | ++ |
| 19 | Dabrafenib | mutBRAF inhibition | 72 | -8.5 | -5.9 | ++ | + |

(continued)

| No. | Anticancer agent | Target/ Mechanism | Culture time (h) | concentration (log M) | | Synergy 1 | Synergy 2 |
|---|---|---|---|---|---|---|---|
| | | | | Anticancer agent | Ex. B01 | | |
| 20 | Sorafenib | pan-RAF | 72 | -8.5 | -5.9 | ++ | + |
| 21 | AT-7519 | CDK9 inhibition | 72 | -8.5 | -5.9 | ++ | - |
| 22 | danusertib | pan Aurora | 72 | -7.0 | -5.9 | +++ | + |
| 23 | ibrutinib | BTK | 72 | -8.0 | -5.9 | +++ | + |
| 24 | MK-1775 | WEE1 | 72 | -7.0 | -5.9 | ++ | ++ |
| 25 | methotrexate | DHFR | 72 | -7.5 | -5.9 | ++ | +++ |
| 26 | AMG-900 | pan Aurora | 72 | -8.0 | -5.9 | +++ | ++ |
| 27 | BIIB021 | HSP90 | 72 | -9.5 | -5.9 | +++ | + |
| 28 | 6-thioguanine | purine antimetabolite | 72 | -7.0 | -5.9 | +++ | + |
| 29 | reversine | A3AR antagonism, pan-aurora inhibition | 120 | -8.0 | -6.3 | ++ | + |
| 30 | MLN-7243 | ubiquitin E1 enzyme | 120 | -7.5 | -6.3 | ++ | + |
| 31 | ABT-737 | Bcl-2 | 72 | -7.0 | -5.9 | +++ | ++ |
| 32 | MK-5108 | Aurora A | 72 | -7.5 | -5.9 | ++ | ++ |
| 33 | GSK-343 | EZH2/ARID1 A | 120 | -8.5 | -6.3 | ++ | - |
| 34 | 2-D08 | SUMOylation | 120 | -6.0 | -6.3 | +++ | + |
| 35 | SCH-900776 | Chk1 | 72 | -7.5 | -5.9 | ++ | + |
| 36 | entinostat | HDAC1, HDAC3 | 72 | -7.5 | -5.9 | ++ | - |
| 37 | ruxolitinib | JAK1/2 | 72 | -6.0 | -5.9 | ++ | + |
| 38 | carfilzomib | proteasome | 72 | -9.0 | -5.9 | ++ | - |
| 39 | apitolisib | PI3Kα/β/δ/γ | 72 | -8.5 | -5.9 | +++ | + |
| 40 | ipatasertib | Akt | 72 | -8.0 | -5.9 | ++ | + |
| 41 | prednisolone | GR | 72 | No concentration -dependent change | -5.9 | NA | NA |
| 42 | volasertib | PLK1 | 72 | -8.0 | -5.9 | ++ | - |

Referring to Table 31 and FIGs. 30a to 31c, when the conventional anticancer agent and the example compound were used in combination, the cell death rate was higher than the simple sum of the cell death rates when the conventional anticancer agent and the example compound were used alone. That is, the example compound can exhibit improved anticancer activity when used in combination with an anticancer agent.

## Claims

1. A pharmaceutical composition for preventing or treating cancer, comprising a compound of Formula I below, a solvate, stereoisomer or pharmaceutically acceptable salt thereof; and an anticancer agent as active ingredients:

[Formula I]

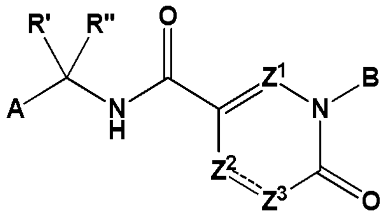

wherein

------ is a single bond or a double bond;
$Z^1$ is N or CH;
when $Z^1$ is N, then both of $Z^2$ and $Z^3$ are $CHR^1$ and ------ is a single bond, or both of $Z^2$ and $Z^3$ are $CR^1$ and ----- is a double bond;
when $Z^1$ is CH, then $Z^2$ is N or $CR^1$, $Z^3$ is $CR^1$, and ------ is a double bond; or
when $Z^1$ is N, both of $Z^2$ and $Z^3$ are $CR^1$, and ------ is a double bond, then two $R^1$ may be optionally linked to each other together with the carbon atom to which they are attached to form a thiophene or pyrrole ring;
each $R^1$ is independently selected from the group consisting of H, halogen, CN, OH, $NR^bR^c$, $C_1$-$C_6$ alkoxy, $C_1$-$C_6$ acylamino, $C_1$-$C_6$ alkylsulfonylamino, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, $C_6$-$C_{10}$ aryloxy, ($C_6$-$C_{10}$ aryl)-($C_1$-$C_6$ alkyl)oxy and $C_6$-$C_{10}$ arylamino;
R' and R" are each independently H or $C_1$-$C_3$ alkyl, or R' and R" may be taken together with the carbon atom to which they are attached to form $C_3$-$C_4$ cycloalkyl, and said $C_1$-$C_3$ alkyl and $C_3$-$C_4$ cycloalkyl may be optionally substituted with at least one halogen, OH, CN, $C_1$-$C_3$ alkoxy or $NR^bR^c$;
A is $Cy_1$ or $Cy_1$-Y-$Cy_2$;
Y is O, S, or a direct bond;
$Cy_1$ is $C_6$-$C_{10}$ aryl or 5- or 6-membered heteroaryl containing 1 or 2 heteroatoms selected from N, O and S;
$Cy_1$ may be optionally substituted with 1 to 3 $R^{2a}$;
$R^{2a}$ is selected from the group consisting of H, halogen, OH, CN, oxo, $SF_5$, $NR^bR^c$, -Si($C_{1-3}$ alkyl)$_3$, -$SO_2R^b$, -C(O)$R^b$, $C_1$-$C_6$ alkyl, $C_1$-$C_6$ haloalkyl, $C_1$-$C_6$ alkoxy, $C_1$-$C_6$ haloalkoxy, $C_3$-$C_6$ cycloalkyl and

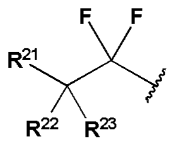,

$R^{21}$ is H, halogen, OH, $NR^bR^c$, $C_1$-$C_6$ alkoxy or $C_1$-$C_6$ acyloxy, and $R^{22}$ and $R^{23}$ are each independently H, halogen or $C_1$-$C_2$ alkyl;
$Cy_2$ is $C_6$-$C_{10}$ aryl, phenyl fused with $C_3$-$C_6$ cycloalkyl, or 5- or 6-membered heteroaryl containing 1 or 2 heteroatoms selected from N, O and S;
$Cy_2$ may be optionally substituted with 1 to 3 $R^{2b}$;
$R^{2b}$ is selected from the group consisting of H, halogen, OH, CN, oxo, $NR^bR^c$; $C_1$-$C_6$ alkyl; $C_1$-$C_6$ alkyl substituted with halogen, CN, OH, $NR^bR^c$ or $C_1$-$C_6$ alkoxy; $C_1$-$C_6$ alkyl optionally interrupted by 1 to 3 oxygen atoms and/or nitrogen atoms; and $C_1$-$C_6$ alkyl substituted with hydroxy-($C_1$-$C_6$ alkyl)amino-;
B is H, $C_1$-$C_6$ alkyl, $C_1$-$C_6$ haloalkyl, $C_1$-$C_6$ hydroxyalkyl, $C_1$-$C_6$ alkoxy-$C_1$-$C_6$ alkyl, $C_1$-$C_6$ alkyl substituted with $NR^bR^c$, -$(CH_2)_o$-$Cy_3$ or -$(CH_2)_o$-$Cy_3$-W-$Cy_4$;
W is NH, C(O) or a direct bond;
o is an integer of 0 or 1;
$Cy_3$ is selected from the group consisting of $C_3$-$C_8$ cycloalkyl, $C_3$-$C_8$ cycloalkenyl, 5- or 6-membered saturated or partially unsaturated heterocycloalkyl containing 1 or 2 heteroatoms selected from N, O and S, bridged bicyclic $C_{5-10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms selected from N, O and S;
$Cy_3$ may be optionally substituted with 1 to 3 $R^{3a}$,
$R^{3a}$ is selected from the group consisting of H, halogen, OH, CN, oxo, $C_1$-$C_6$ alkyl; $C_1$-$C_6$ alkyl substituted with halogen, OH, CN or $C_1$-$C_6$ alkoxy; $C_3$-$C_6$ cycloalkyl, $C_1$-$C_6$ alkoxy, $C_1$-$C_6$ haloalkoxy, $C_1$-$C_6$ haloalkylamino, $C_1$-$C_6$ hydroxyalkylamino, ($C_3$-$C_6$ cycloalkyl)carbonylamino, -$NR^bR^c$, -$NR^bCOR^c$, -$NR^bC(O)OR^c$, -$SO_2R^b$, -C(O)$R^b$,

-C(O)OR$^b$, -NR$^b$SO$_2$R$^c$ and -CONR$^{b1}$R$^{c1}$;
Cy$_4$ is selected from the group consisting of saturated or partially unsaturated 4- to 10-membered heterocycloalkyl containing 1 or 2 heteroatoms selected from N, O or S, $C_{6-}C_{10}$ aryl, and 5- or 6-membered heteroaryl containing 1 to 4 heteroatoms selected from N, O and S;
Cy$_4$ may be optionally substituted with 1 to 3 R$^{3b}$,
R$^{3b}$ is H, deuterium, halogen, OH, CN, oxo, NR$^b$R$^c$, $C_1$-$C_6$ alkyl, $C_1$-$C_6$ alkyl substituted with deuterium, $C_1$-$C_6$ haloalkyl, $C_1$-$C_6$ hydroxyalkyl, $C_1$-$C_6$ alkoxy-$C_{1-}C_6$ alkyl, $C_1$-$C_6$ alkoxy or $C_1$-$C_6$ haloalkoxy;
R$^b$ and R$^c$ are each independently H or $C_1$-$C_6$ alkyl; and
one of R$^{b1}$ and R$^{c1}$ is H or $C_1$-$C_6$ alkyl, and the other of R$^{b1}$ and R$^{c1}$ is H, $C_1$-$C_6$ alkyl, $C_{1-}C_6$ alkyl substituted with NR$^b$R$^c$, or $C_1$-$C_6$ alkyl substituted with $C_1$-$C_6$ alkoxy.

2. The pharmaceutical composition for preventing or treating cancer according to claim 1, **characterized in that** in Formula I above,

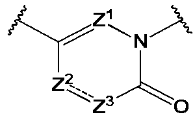

is selected from the group consisting of

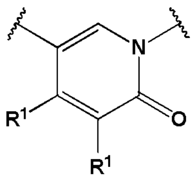
,
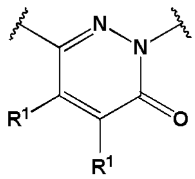
,

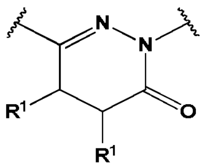
,
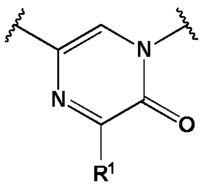
,
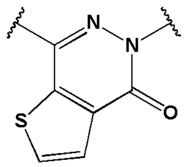
,
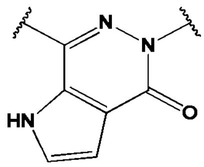
, and
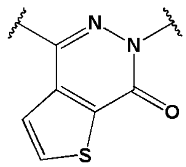
,

wherein said R$^1$ is the same or different from each other.

3. The pharmaceutical composition for preventing or treating cancer according to claim 2, **characterized in that** in Formula I above,

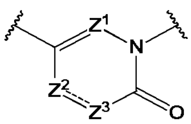
is
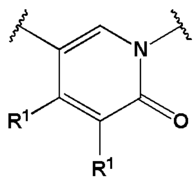
or
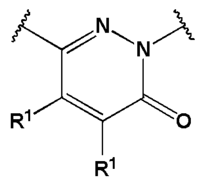
,

wherein said R$^1$ is the same or different from each other.

4. The pharmaceutical composition for preventing or treating cancer according to claim 3, **characterized in that** each R$^1$ is independently selected from the group consisting of H, F, Br, Cl, I, CN, OH, OCH$_3$, amino, methylamino, dimethylamino, ethylamino, acetylamino, methylsulfonylamino, ethylsulfonylamino, methyl, ethyl, ethenyl, ethynyl, cyclopropyl, cyclobutyl, cyclopentyl, phenyl, phenoxy, benzyloxy and phenylamino.

5. The pharmaceutical composition for preventing or treating cancer according to claim 4, **characterized in that** one of two R$^1$ substituted on the same ring is H and the other is not H.

**6.** The pharmaceutical composition for preventing or treating cancer according to claim 4, **characterized in that** two $R^1$ substituted on the same ring are both H.

**7.** The pharmaceutical composition for preventing or treating cancer according to claim 2, **characterized in that** in Formula I above,

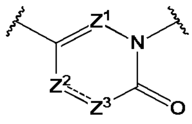 is 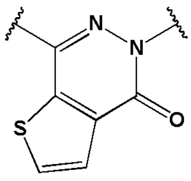, 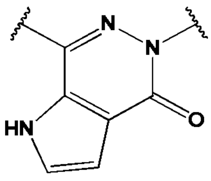, or 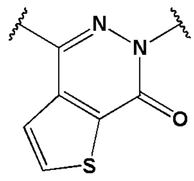.

**8.** The pharmaceutical composition for preventing or treating cancer according to claim 1, **characterized in that** R' and R" are each H or $C_1$-$C_3$ alkyl, and R' and R" may be optionally taken together with the carbon atom to which they are attached to form $C_3$-$C_4$ cycloalkyl.

**9.** The pharmaceutical composition for preventing or treating cancer according to claim 8, **characterized in that** in Formula I,

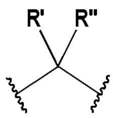 is 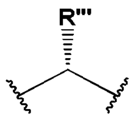, 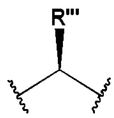, or 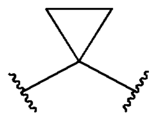,

and R‴ is methyl or ethyl.

**10.** The pharmaceutical composition for preventing or treating cancer according to claim 9, **characterized in that** the compound is a compound represented by Formula IA below:

[Formula IA]

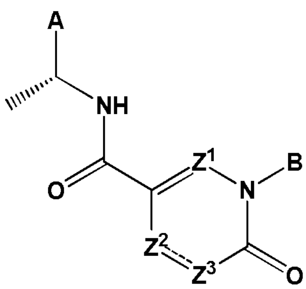

wherein A, $Z^1$, $Z^2$, $Z^3$ and B are as defined in claim 1.

**11.** The pharmaceutical composition for preventing or treating cancer according to claim 1, **characterized in that** A is $Cy_1$.

**12.** The pharmaceutical composition for preventing or treating cancer according to claim 11, **characterized in that** $Cy_1$ is $C_6$-$C_{10}$ aryl, or 5- or 6-membered heteroaryl containing 1 or 2 heteroatoms selected from N and S.

**13.** The pharmaceutical composition for preventing or treating cancer according to claim 12, **characterized in that** A is $Cy_1$, and $Cy_1$ is phenyl, naphthalenyl, thiophenyl or pyridinyl.

**14.** The pharmaceutical composition for preventing or treating cancer according to claim 11, **characterized in that** $Cy_1$ has any one of the following ring structures optionally substituted with 1 to 3 $R^{2a}$:

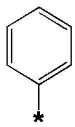 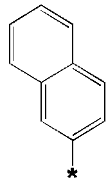 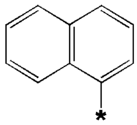 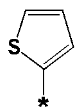 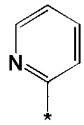 .

**15.** The pharmaceutical composition for preventing or treating cancer according to claim 11, **characterized in that** $Cy_1$ may be optionally substituted with 1 to 3 $R^{2a}$, and each $R^{2a}$ is independently selected from the group consisting of F, Cl, Br, I, OH, CN, $SF_5$, $-Si(CH_3)_3$, $CH_3SO_2$-, methyl, ethyl, propyl, isopropyl, $CF_3$, $CHF_2$, $CH_2F$, $NH_2$, $CH_3NH$-, $(CH_3)_2N$-, methoxy, ethoxy, $OCF_3$, $OCHF_2$, $OCH_2F$, cyclopropyl, cyclobutyl, cyclopentyl, $-CF_2CH_2F$,

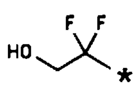 , 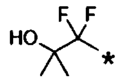 ,

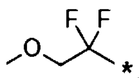 , 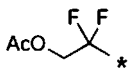 , 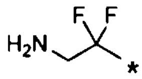 , 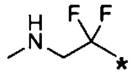 and 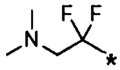 .

**16.** The pharmaceutical composition for preventing or treating cancer according to claim 1, **characterized in that** A is $Cy_1$-Y-$Cy_2$.

**17.** The pharmaceutical composition for preventing or treating cancer according to claim 16, **characterized in that**

$Cy_1$ is $C_6$-$C_{10}$ aryl, or 5- or 6-membered heteroaryl containing 1 or 2 heteroatoms selected from N and S;
Y is O or a direct bond; and
$Cy_2$ is $C_6$-$C_{10}$ aryl, phenyl fused with $C_3$-$C_5$ cycloalkyl, or 5- or 6-membered heteroaryl containing 1 or 2 heteroatoms selected from N and S.

**18.** The pharmaceutical composition for preventing or treating cancer according to claim 17, **characterized in that**

$Cy_1$ is phenyl, and $Cy_2$ is phenyl, pyrrolyl, pyrazolyl, thiophenyl, pyridinyl, or 2-oxo-1,2-dihydropyridinyl, or $Cy_1$ is thiazolyl, thiophenyl or pyrazolyl, and $Cy_2$ is phenyl, 2,3-dihydroindenyl or bicyclo[4.2.0]octa-1,3,5-trienyl.

**19.** The pharmaceutical composition for preventing or treating cancer according to claim 17, **characterized in that** Y is a direct bond.

**20.** The pharmaceutical composition for preventing or treating cancer according to any one of claims 16 to 19, **characterized in that** $Cy_1$-Y-$Cy_2$ has any one of the following ring structures optionally substituted with $R^{2a}$ and $R^{2b}$:

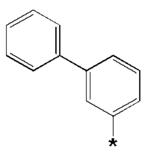 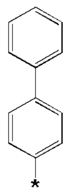 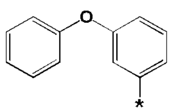 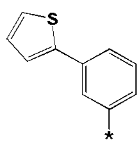 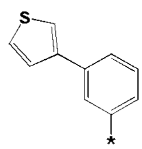 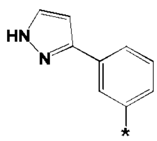 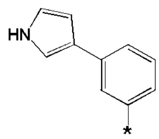

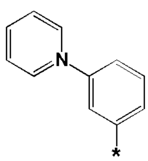 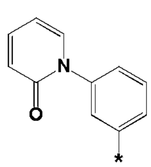 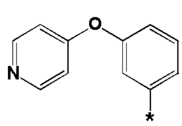 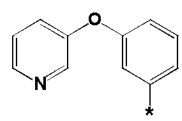 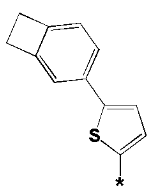 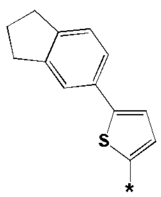

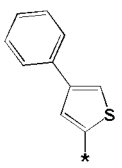 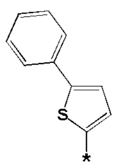 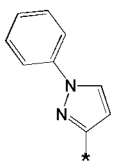 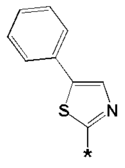 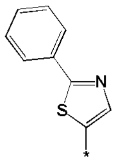 .

**21.** The pharmaceutical composition for preventing or treating cancer according to claim 16, **characterized in that**

$Cy_1$ is optionally substituted with one $R^{2a}$, wherein $R^{2a}$ is selected from the group consisting of H, halogen, OH, CN, amino, $C_1$-$C_6$ alkyl, $C_1$-$C_6$ haloalkyl, $C_1$-$C_6$ alkoxy and $C_1$-$C_6$ haloalkoxy; and

$Cy_2$ is optionally substituted with 1 to 3 $R^{2b}$, wherein $R^{2b}$ is selected from the group consisting of H, halogen, OH, CN, oxo, $NR^bR^c$; $C_1$-$C_6$ alkyl; $C_1$-$C_6$ alkyl substituted with halogen, CN, OH, $NR^bR^c$ or $C_1$-$C_6$ alkoxy; $C_{1-}C_6$ alkyl optionally interrupted by 1 to 3 oxygen atoms and/or nitrogen atoms; and $C_1$-$C_6$ alkyl substituted with hydroxy-($C_1$-$C_6$ alkyl)amino-.

**22.** The pharmaceutical composition for preventing or treating cancer according to claim 21, **characterized in that** $R^{2a}$ is H.

**23.** The pharmaceutical composition for preventing or treating cancer according to claim 21, **characterized in that**

$R^{2a}$ is H; and

each $R^{2b}$ is independently selected from the group consisting of H, F, Cl, Br, I, OH, CN, oxo, amino, $CH_3NH$-, $(CH_3)_2N$-, $(CH_3)_2NCH_2$- methyl, ethyl, cyanomethyl, hydroxymethyl, aminomethyl, $CH_3NHCH_2$-, $C_2H_5NHCH_2$- and $HOC_2H_4NHCH_2$-.

**24.** The pharmaceutical composition for preventing or treating cancer according to claim 1, **characterized in that** A in Formula I is selected from the following structures:

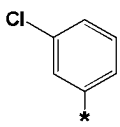 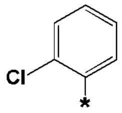 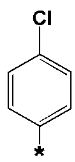 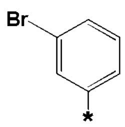 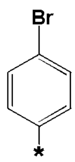 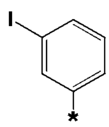 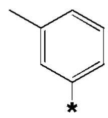 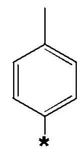 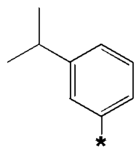

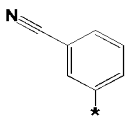 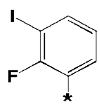 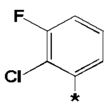 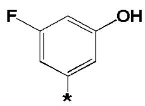 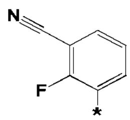 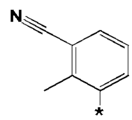 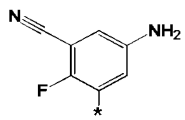 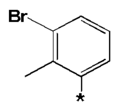

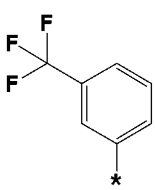 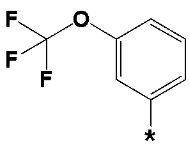 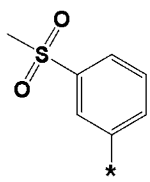 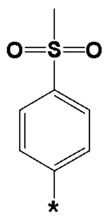 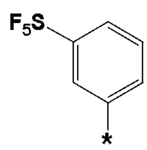 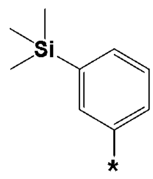 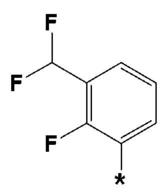

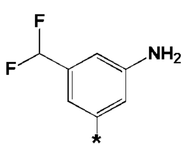 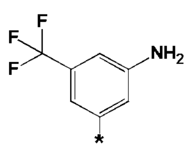 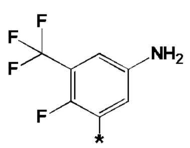 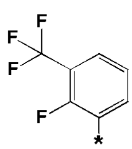 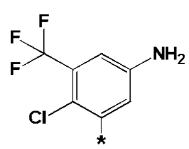 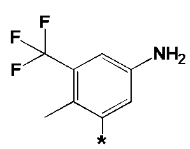

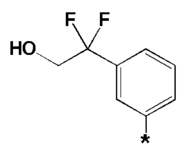 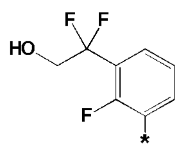 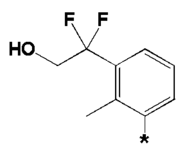 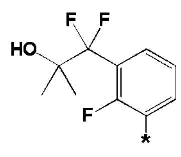 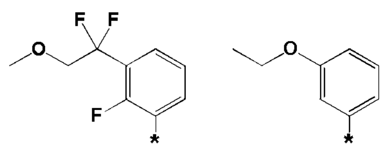

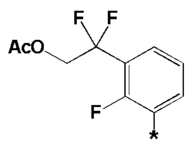 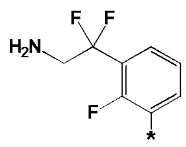 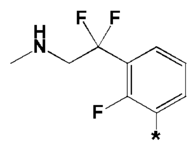 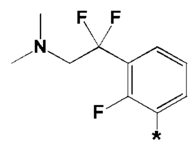 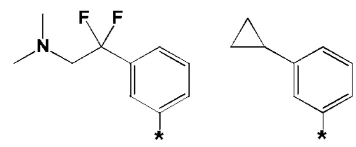

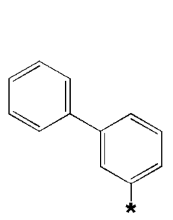 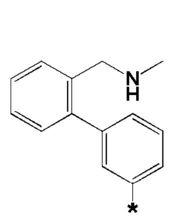 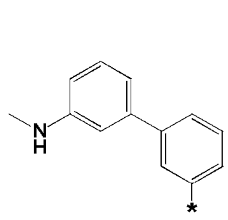 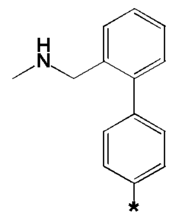 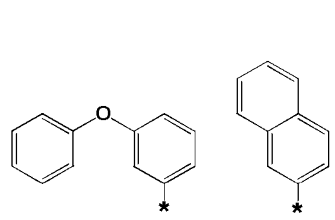

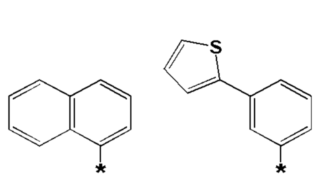 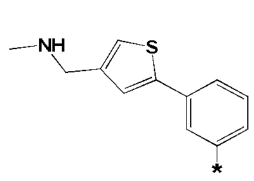 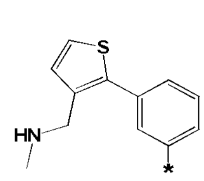 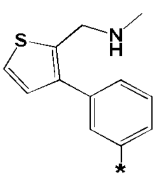 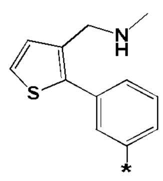

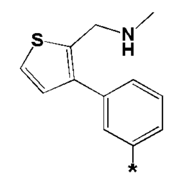 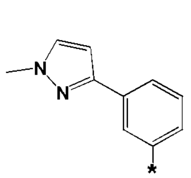 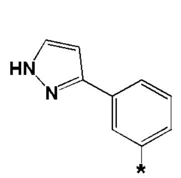 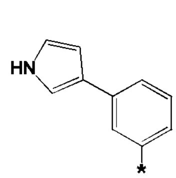 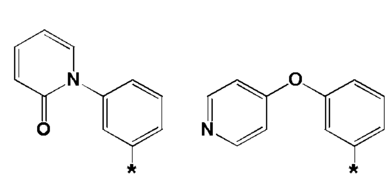

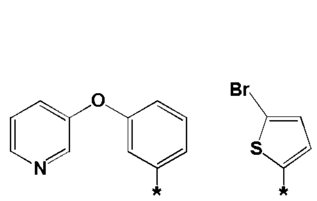 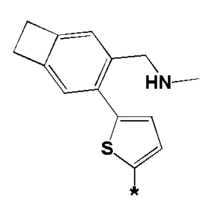 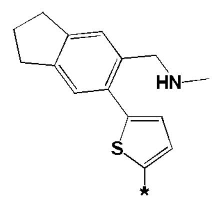 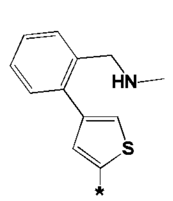 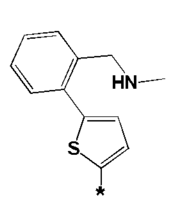

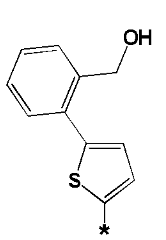 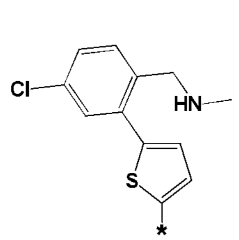 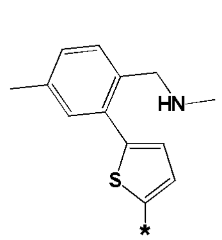 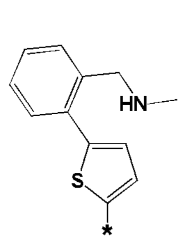 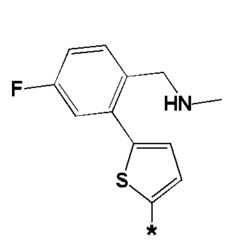

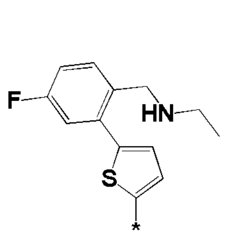 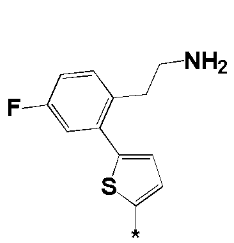 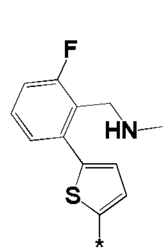 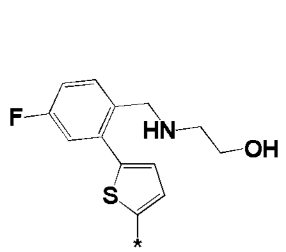 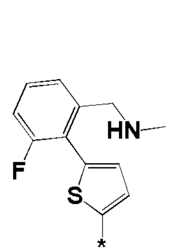

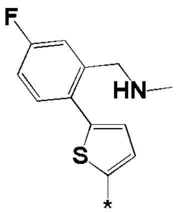 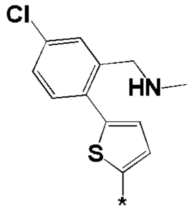 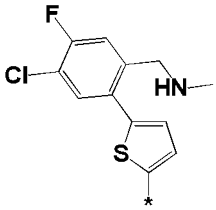 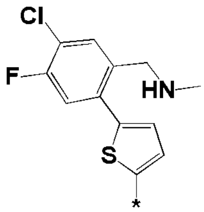 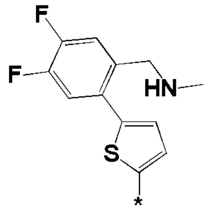

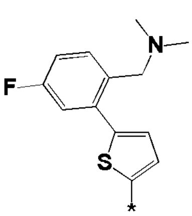 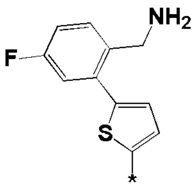 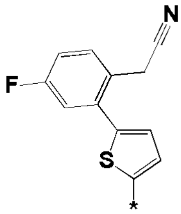 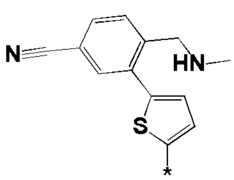 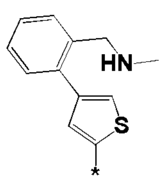

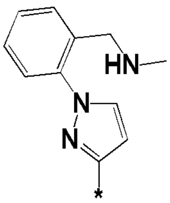 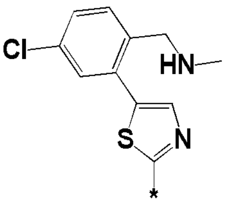 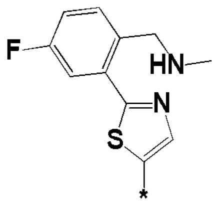 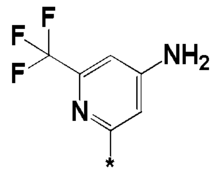 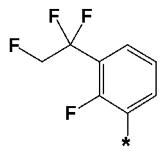 .

**25.** The pharmaceutical composition for preventing or treating cancer according to claim 1, **characterized in that** B is H, $C_1$-$C_6$ alkyl, $C_1$-$C_6$ haloalkyl, $C_1$-$C_6$ hydroxyalkyl, $C_1$-$C_6$ alkoxy-$C_{1-}C_6$ alkyl, or $C_1$-$C_6$ alkyl substituted with $NR^bR^c$.

**26.** The pharmaceutical composition for preventing or treating cancer according to claim 1, **characterized in that** B is $-(CH_2)_o$-$Cy_3$, and o is 0 or 1.

**27.** The pharmaceutical composition for preventing or treating cancer according to claim 26, **characterized in that** $Cy_3$ is selected from the group consisting of $C_{3-}C_8$ cycloalkyl, $C_{3-}C_8$ cycloalkenyl, 6-membered saturated or partially unsaturated heterocycloalkyl containing one N, O or S, bridged bicyclic $C_{5-8}$ cycloalkyl, $C_{6-}C_{10}$ aryl, phenyl fused with 5-membered heterocycloalkyl containing one N, O or S, 5- or 6-membered heteroaryl containing 1 or 2 heteroatoms selected from N or S, and 9- or 10-membered bicyclic heteroaryl containing 1 to 3 N.

**28.** The pharmaceutical composition for preventing or treating cancer according to claim 27, **characterized in that** $Cy_3$ is $C_3$-$C_6$ cycloalkyl, $C_3$-$C_6$ cycloalkenyl, tetrahydropyranyl, dihydropyranyl, thianyl, 1,1-dioxothianyl, piperidinyl, dihydropyridinyl, tetrahydropyridinyl, bicyclo[1.1.1]pentanyl, bicyclo[2.2.1]heptanyl, $C_{6-10}$ aryl, thiophenyl, thiazolyl, pyrazolyl, pyridinyl, pyrimidinyl, dihydroisobenzofuranyl, indolyl, indazolyl or benzotriazolyl.

**29.** The pharmaceutical composition for preventing or treating cancer according to claim 26, **characterized in that** $Cy_3$ has any one of the following ring structures optionally substituted with 1 to 3 $R^{3a}$:

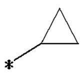 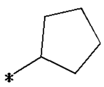 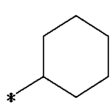 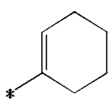 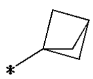 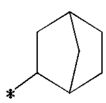 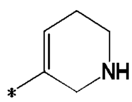 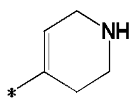

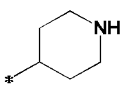 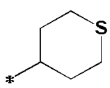 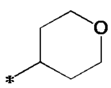 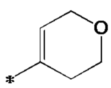 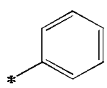 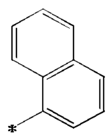 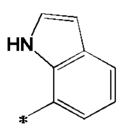 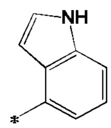

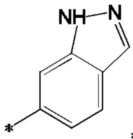 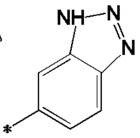 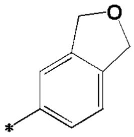 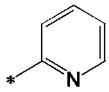 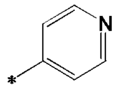 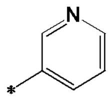 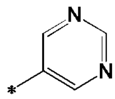 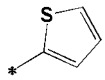

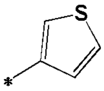 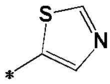 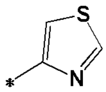 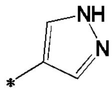 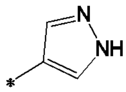

**30.** The pharmaceutical composition for preventing or treating cancer according to claim 26, **characterized in that** $R^{3a}$ is selected from the group consisting of H, F, Cl, Br, I, OH, CN, oxo, methyl, ethyl, amino, $CH_3NH$-, $(CH_3)_2NH$-, 1,1,1-trifluoropropan-2-ylamino, $CH_3CONH$-, $(CH_3CO)(CH_3)N$-, $CH_3OCONH$-, cyclopropylcarbonylamino, hydroxymethyl, 1-hydroxyethyl, 2-hydroxypropan-2-yl, methoxy, ethoxy, isopropoxy, methoxymethyl, 2-methoxyethyl, $OCHF_2$, $OCF_3$, $CH_3SO_2$-, $CH_3CO$-, $CH_3SO_2NH$-, -COOH, $-COOC(CH_3)_3$, $-CONH_2$, $-CONHCH_3$, - $CONHC_2H_5$, $-CON(CH_3)_2$, $-CONHC_2H_4OCH_3$ and $-CONHC_2H_4N(CH_3)_2$.

**31.** The pharmaceutical composition for preventing or treating cancer according to claim 1, **characterized in that** B is $-(CH_2)_o-Cy_3-W-Cy_4$, and o is 0.

**32.** The pharmaceutical composition for preventing or treating cancer according to claim 31, **characterized in that**

$Cy_3$ is $C_6$-$C_{10}$ aryl, or 5- or 6-membered heteroaryl containing 1 or 2 heteroatoms selected from N or S;
W is NH, C(O) or a direct bond; and
$Cy_4$ is selected from the group consisting of saturated or partially unsaturated 4- to 7-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S.

**33.** The pharmaceutical composition for preventing or treating cancer according to claim 32, **characterized in that** $Cy_3$ is $C_6$-$C_{10}$ aryl, $Cy_4$ is saturated or partially unsaturated 4- to 7-membered heterocycloalkyl containing 1 or 2 heteroatoms selected from N, O or S, and W is NH or C(O).

**34.** The pharmaceutical composition for preventing or treating cancer according to claim 32, **characterized in that** W is a direct bond.

**35.** The pharmaceutical composition for preventing or treating cancer according to claim 32, **characterized in that**

$Cy_3$ is phenyl or pyridinyl; and
$Cy_4$ is oxetanyl, tetrahydrofuranyl, pyrrolidinyl, 2-oxo-pyrrolidinyl, piperidinyl, morpholinyl, imidazolidinyl, 2-oxo-imidazolidinyl, piperazinyl, 2-oxo-piperazinyl, hexahydropyrimidinyl, 2-oxo-hexahydropyrimidinyl, phenyl, oxazolyl, isoxazolyl, thiazolyl, pyrazolyl, imidazolyl, oxadiazolyl, triazolyl, tetrazolyl, pyridinyl or 2-oxo-pyridinyl.

**36.** The pharmaceutical composition for preventing or treating cancer according to claim 31, **characterized in that** $Cy_3$-W-$Cy_4$ has any one of the following ring structures optionally substituted with $R^{3a}$ and $R^{3b}$:

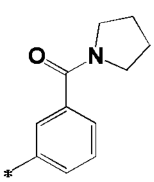 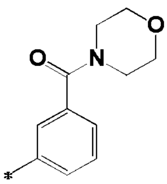 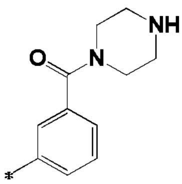 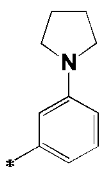 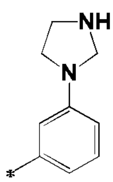 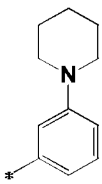 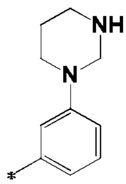 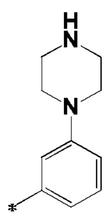

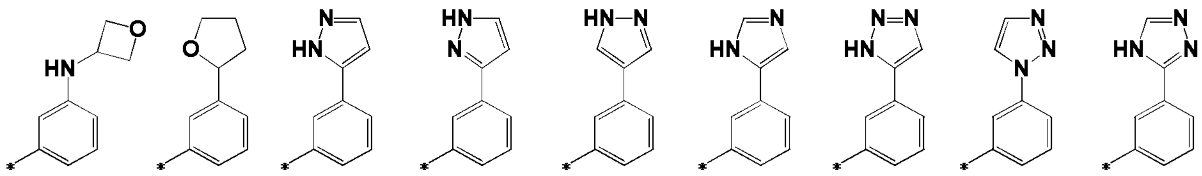

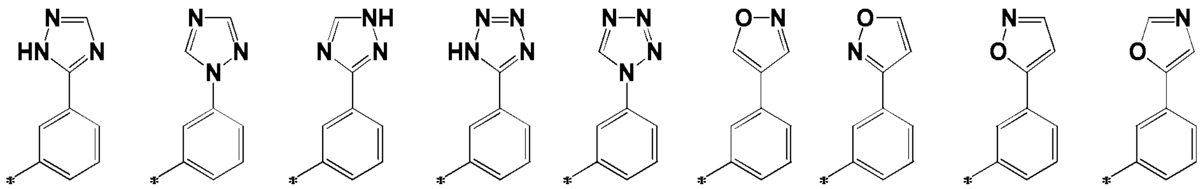

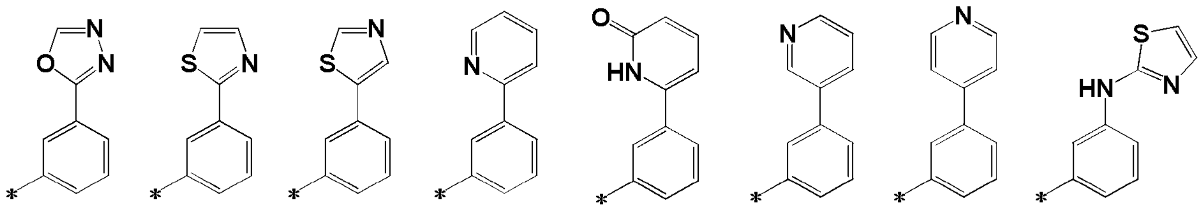

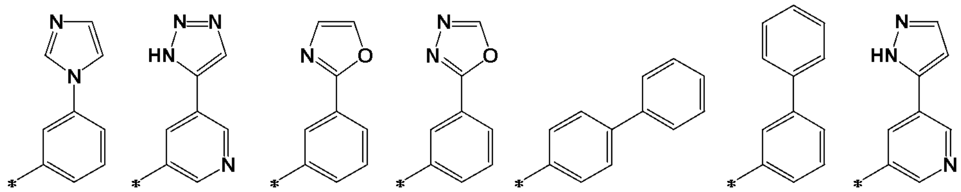

.

**37.** The pharmaceutical composition for preventing or treating cancer according to claim 31, **characterized in that**

$Cy_3$ may be optionally substituted with one or two $R^{3a}$, wherein $R^{3a}$ is H, halogen, OH or CN; and
$Cy_4$ may be optionally substituted with 1 to 3 $R^{3b}$, wherein $R^{3b}$ is H, deuterium, halogen, OH, CN, oxo, $C_1$-$C_6$ alkyl, $C_1$-$C_6$ alkyl substituted with deuterium or $C_1$-$C_6$ haloalkyl.

**38.** The pharmaceutical composition for preventing or treating cancer according to claim 37, **characterized in that**

$R^{3a}$ is H or F; and
$R^{3b}$ is selected from the group consisting of H, F, oxo, methyl, ethyl, $CHF_2$ and $CD_3$.

**39.** The pharmaceutical composition for preventing or treating cancer according to claim 31, **characterized in that** B is any one of the following structures:
H, $CH_3$,

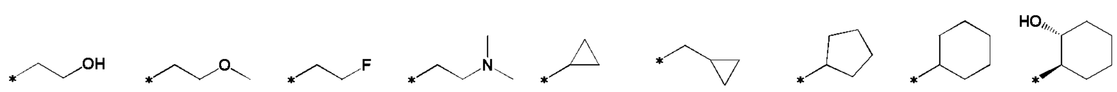

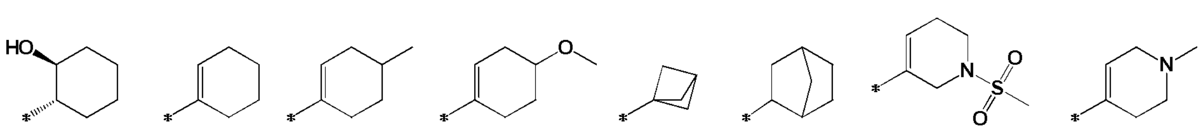

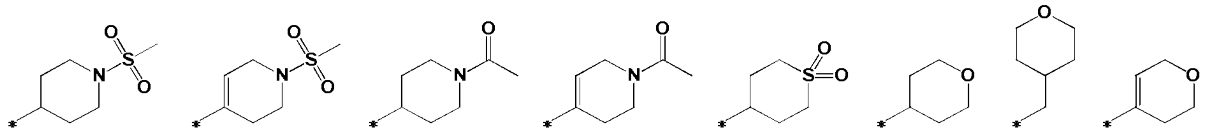

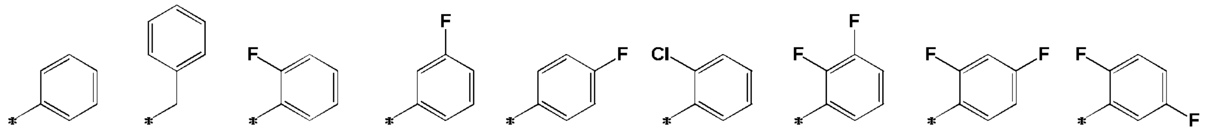

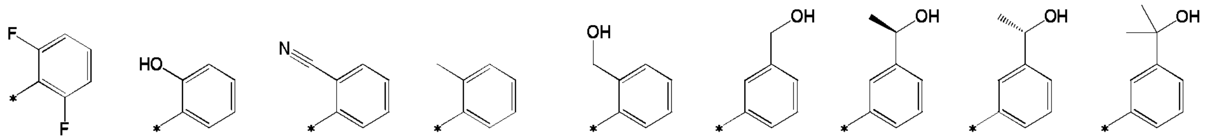

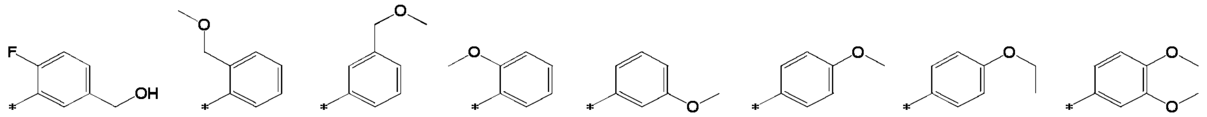

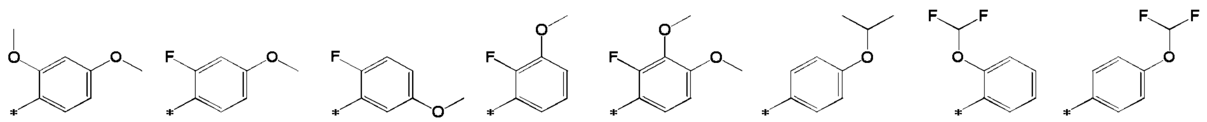

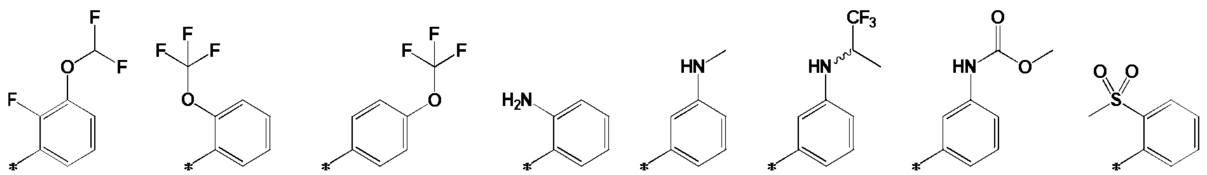

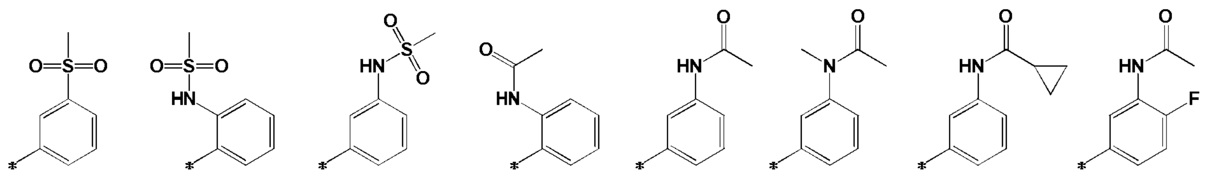

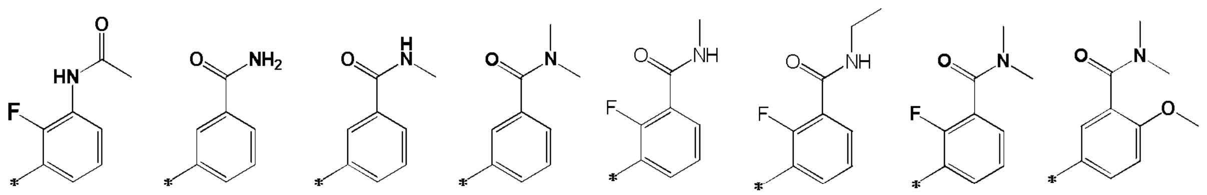

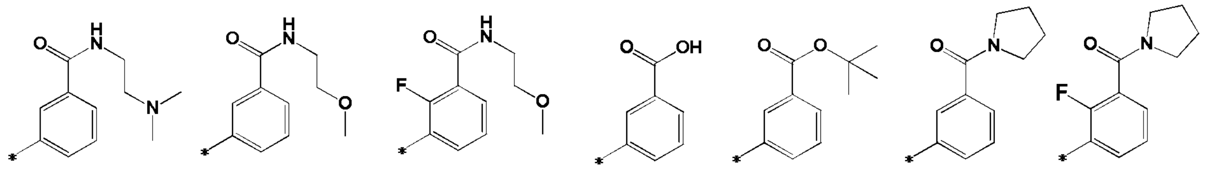

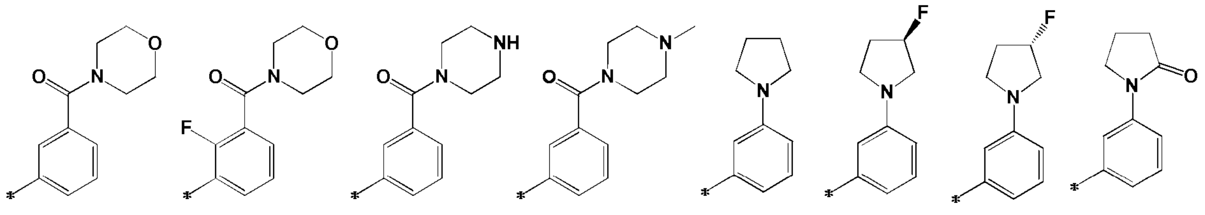

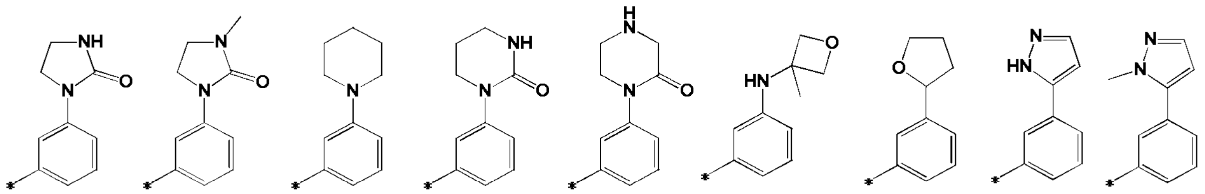

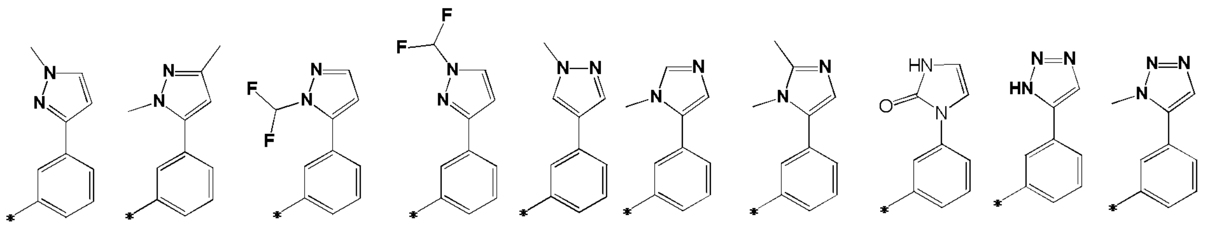

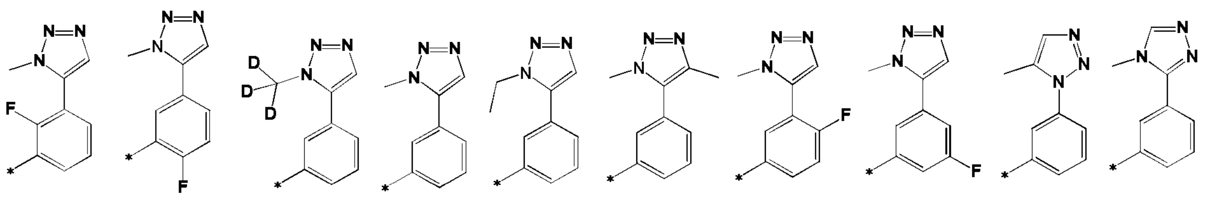

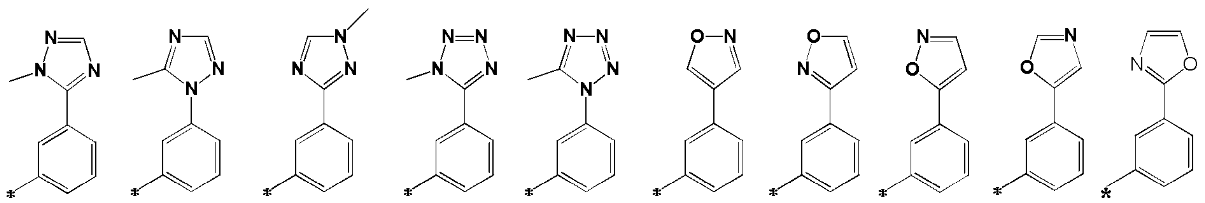

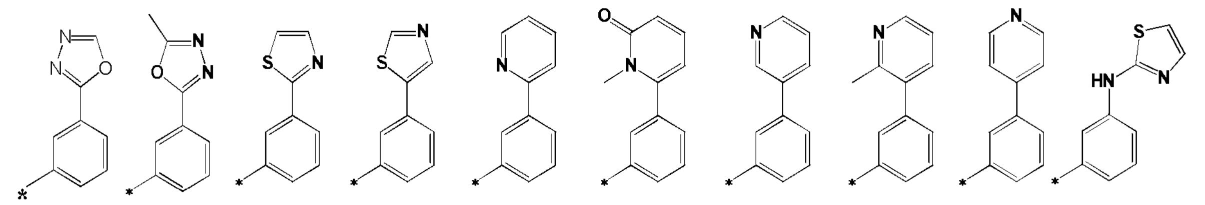

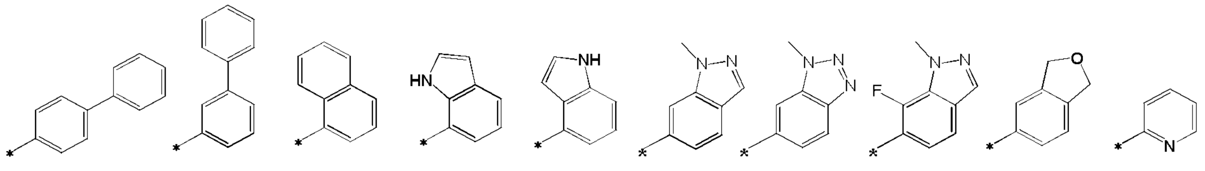

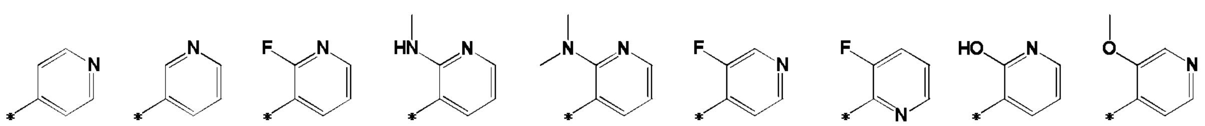

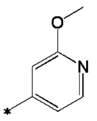 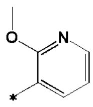 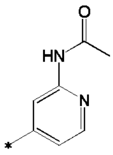 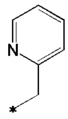 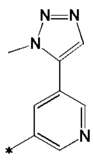 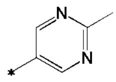 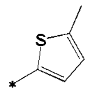 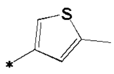 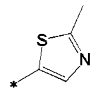 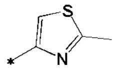

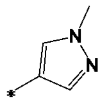 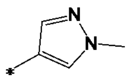 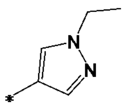 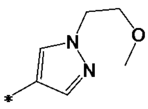 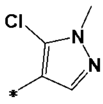 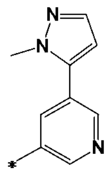 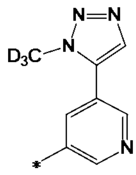.

**40.** The pharmaceutical composition for preventing or treating cancer according to claim 1, **characterized in that** the compound is selected from:

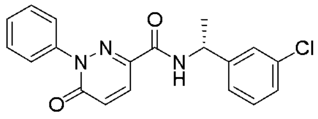, 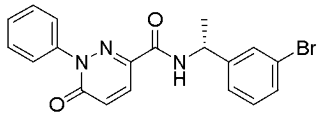, 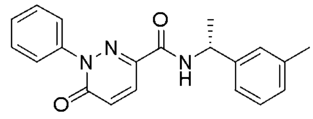,

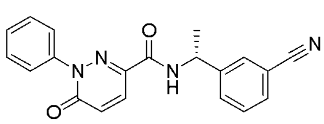, 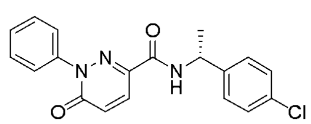, 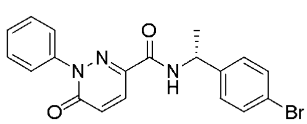,

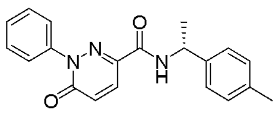, 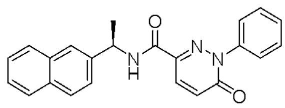, 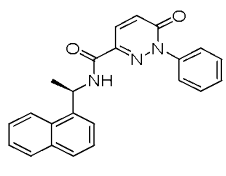, 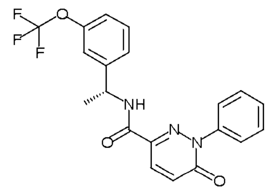,

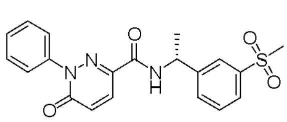, 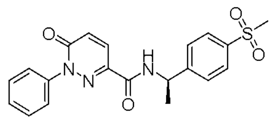, 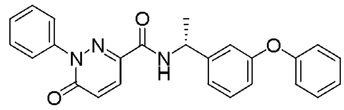,

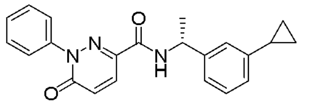, 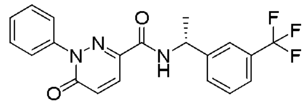, 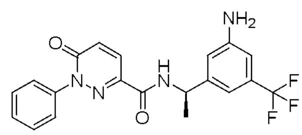,

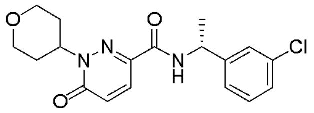, 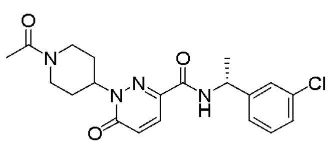, 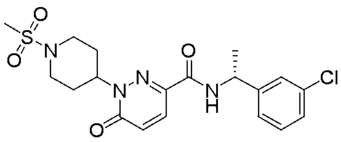,

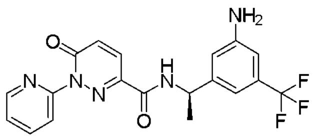 , 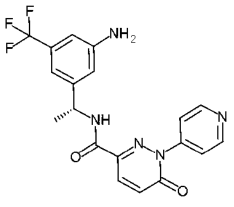 , 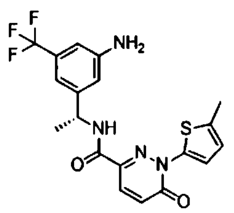 , 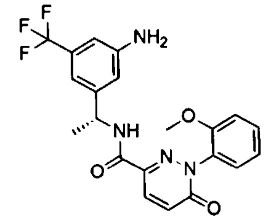 ,

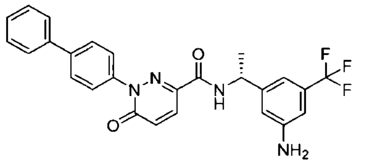 , 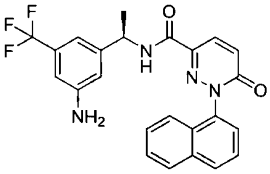 , 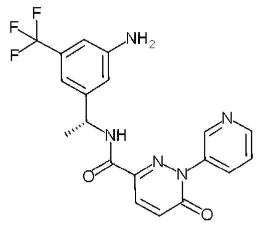 ,

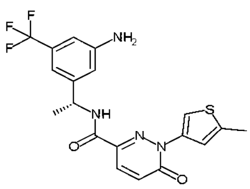 , 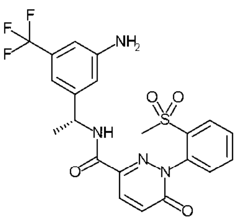 , 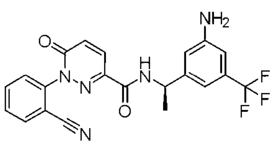 , 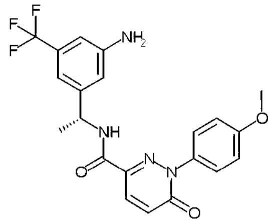 ,

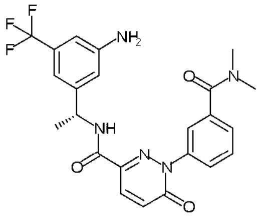 , 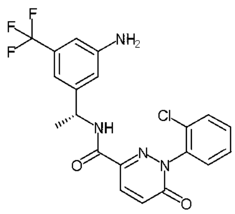 , 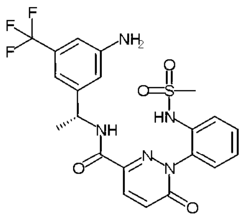 , 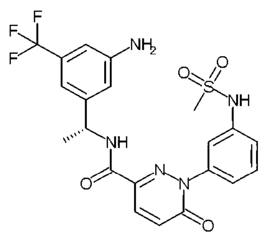 ,

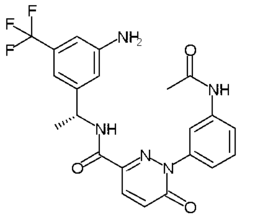 , 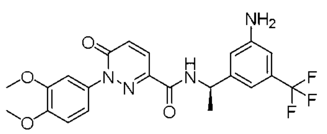 , 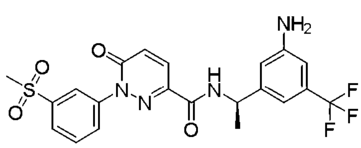 ,

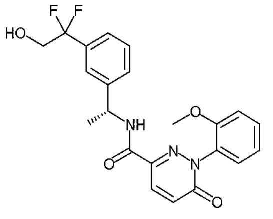 , 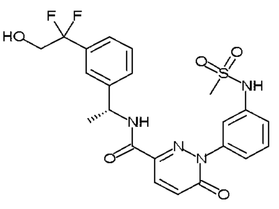 , 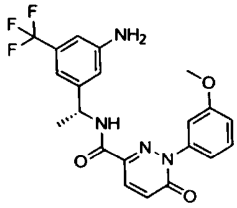 , 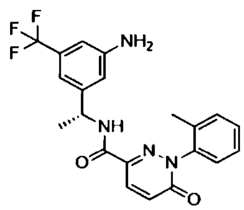 ,

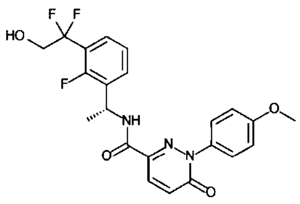,
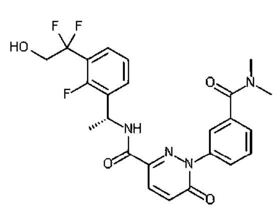,
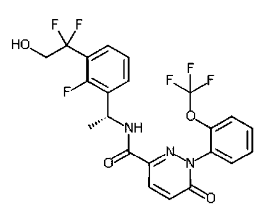,
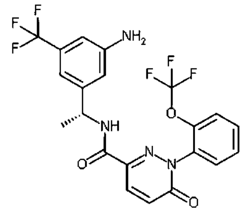,

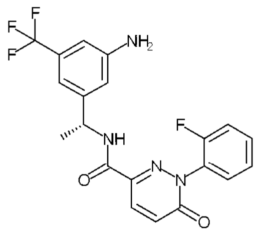,
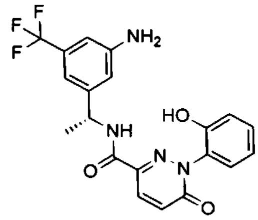,
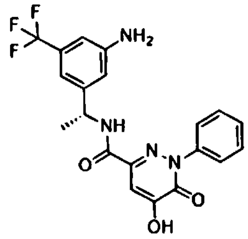,
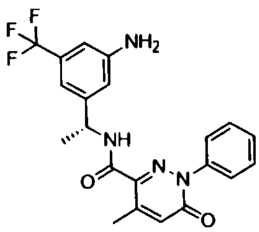,

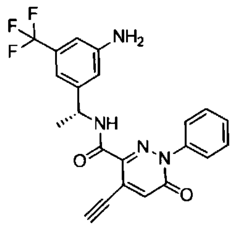,
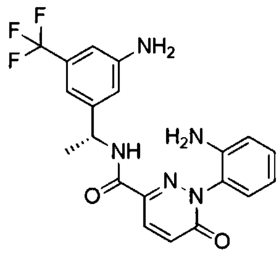,
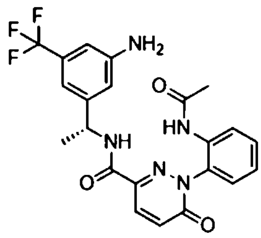,
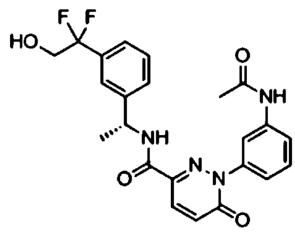,

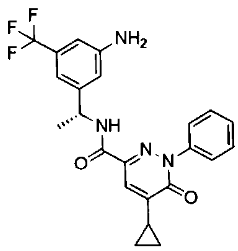,
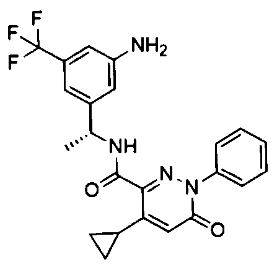,
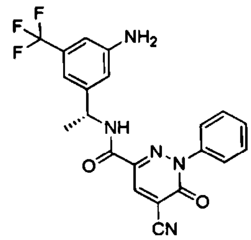,
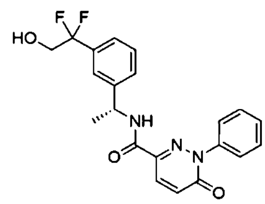,

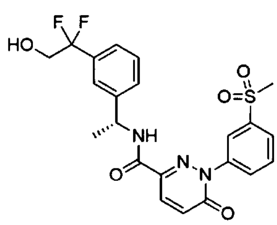,
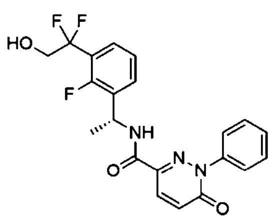,
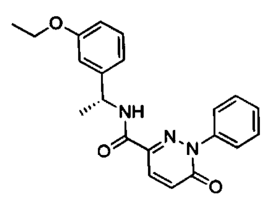,
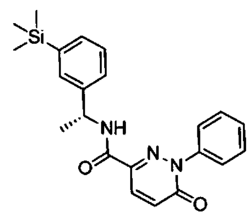,

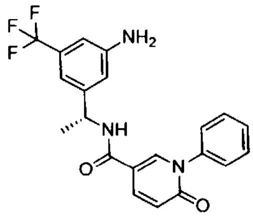,
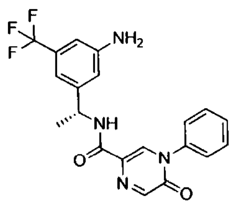,
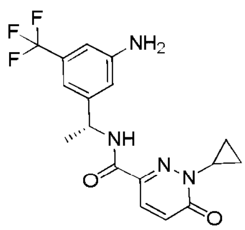,
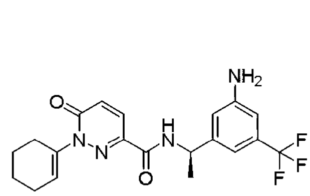,

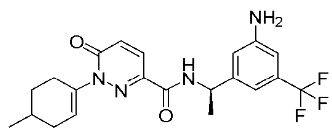 , 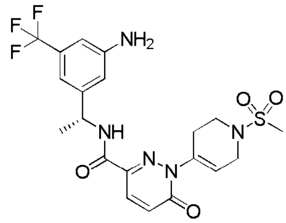 , 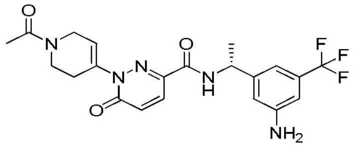 ,

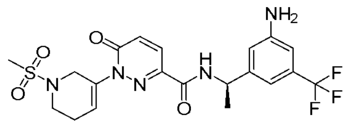 , 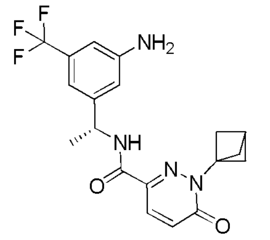 , 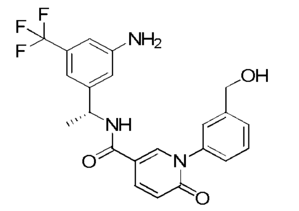 ,

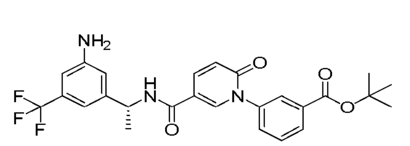 , 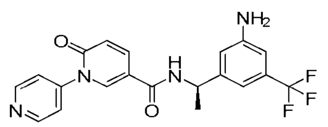 , 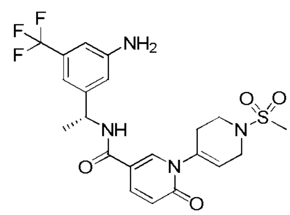 ,

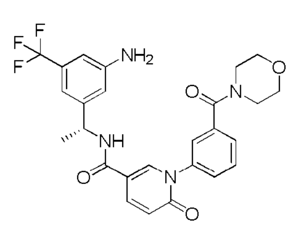 , 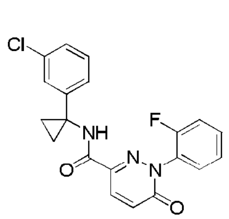 , 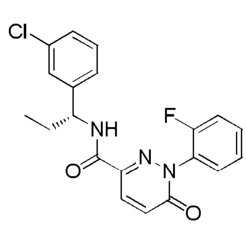 , 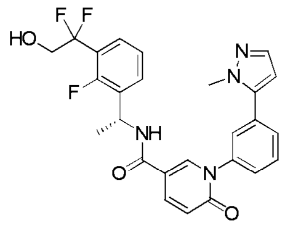 ,

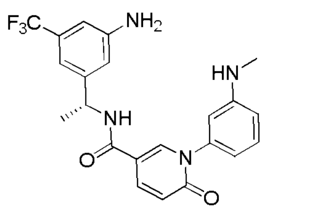 , 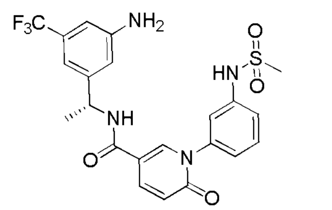 , 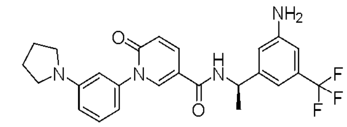 ,

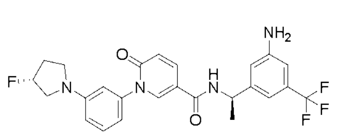 , 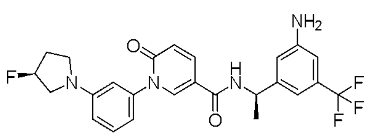 , 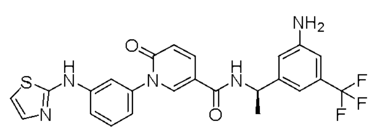 ,

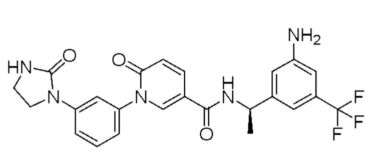 , 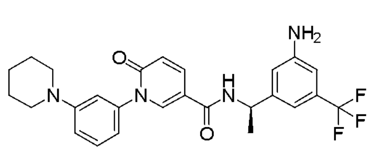 , 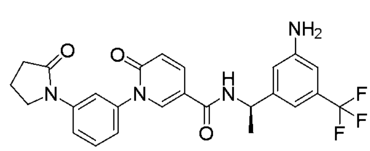 ,

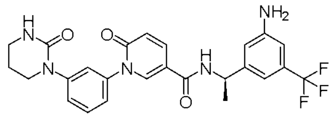

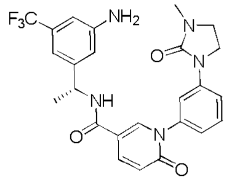,

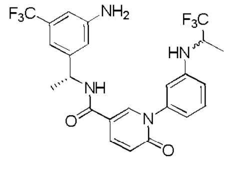,

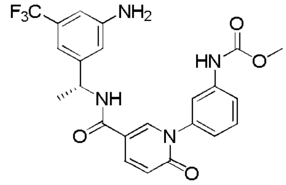,

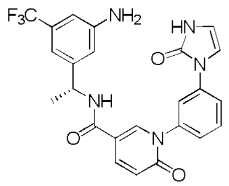,

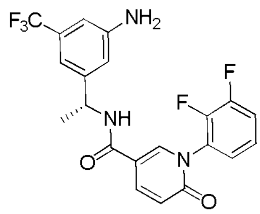,

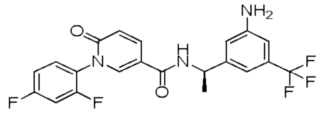,

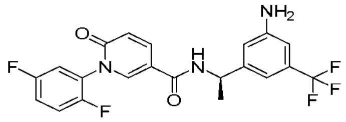,

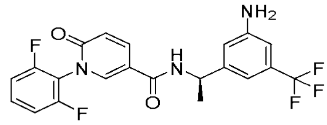,

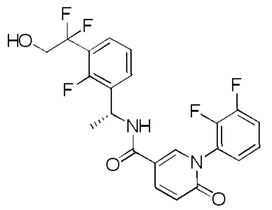,

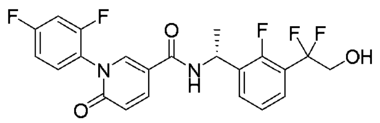,

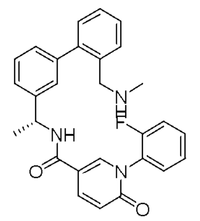,

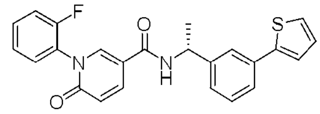,

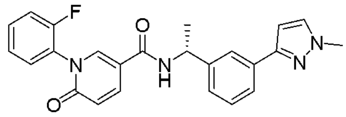,

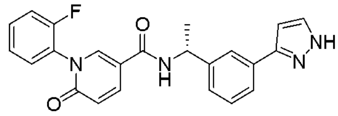,

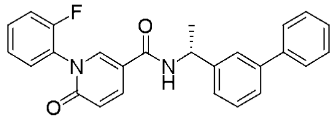,

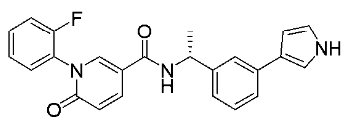,

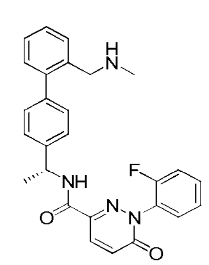,

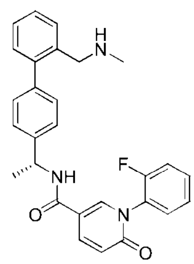,

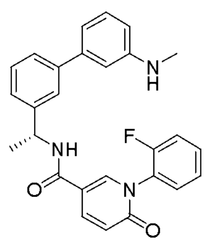,

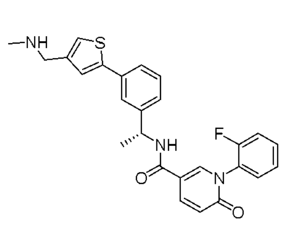,

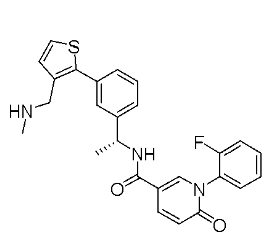,

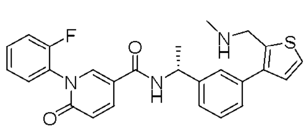,

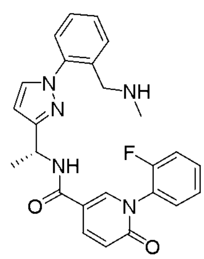,

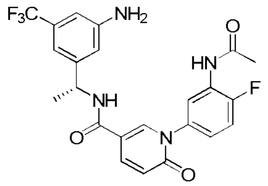 , 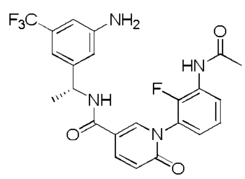 , 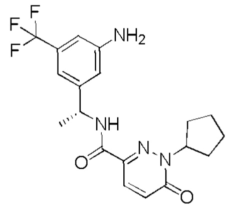 , 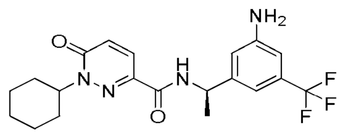 ,

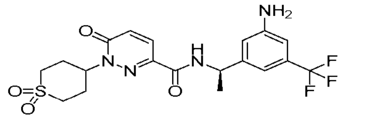 , 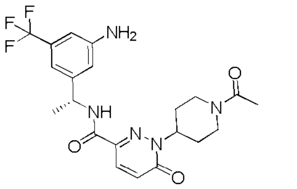 , 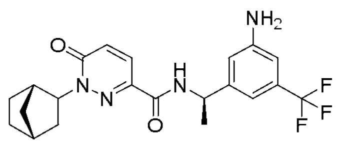 ,

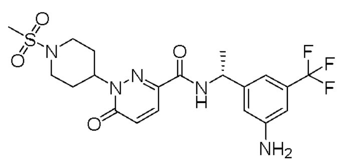 , 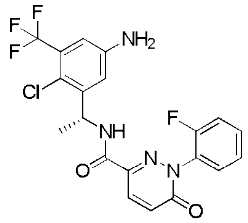 , 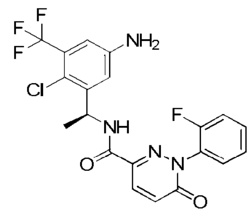 , 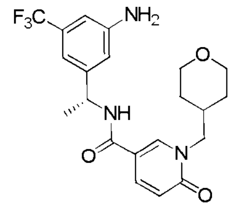 ,

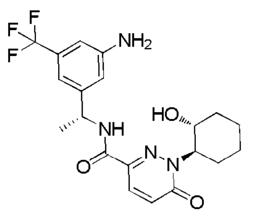 , 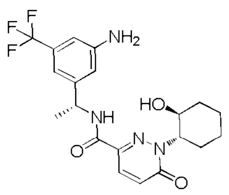 , 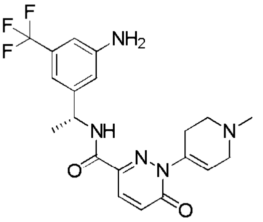 , 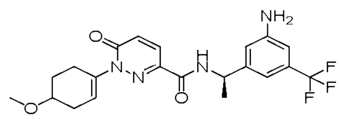 ,

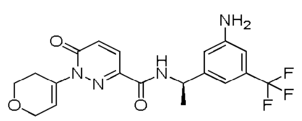 , 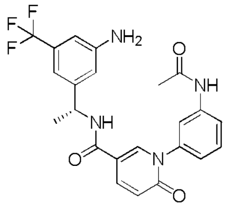 , 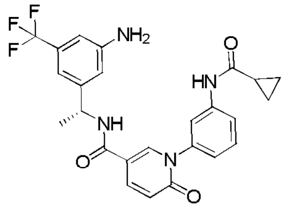 , 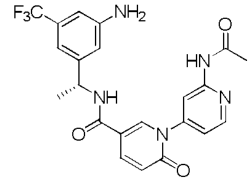 ,

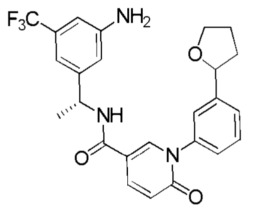 , 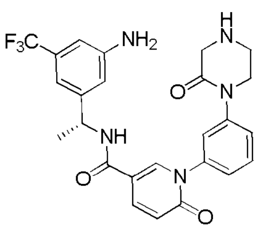 , 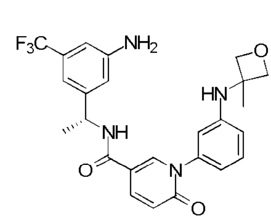 , 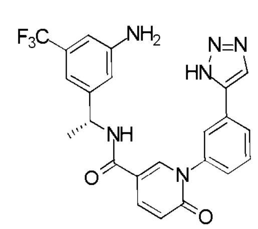 ,

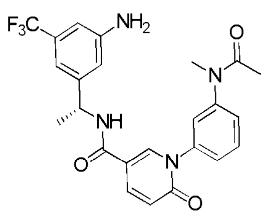 , 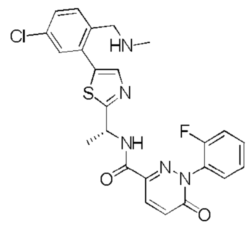 , 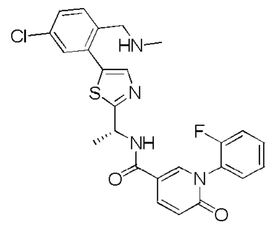 , 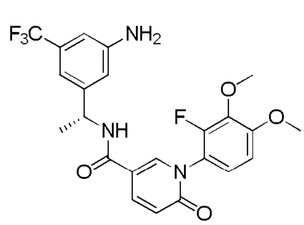 ,

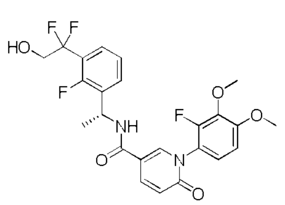 , 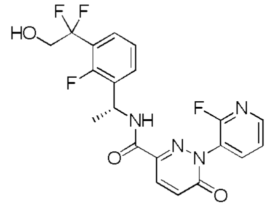 , 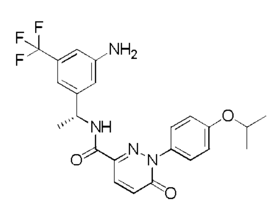 , 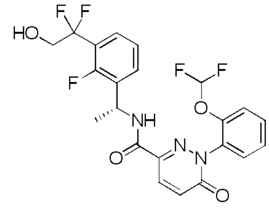 ,

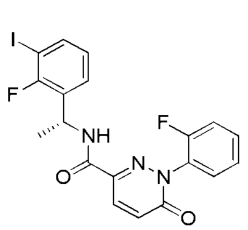 , 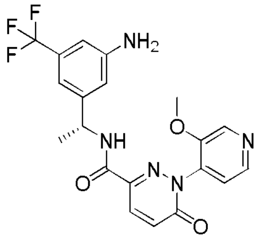 , 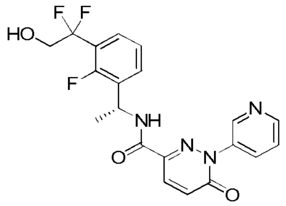 , 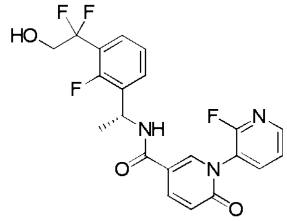 ,

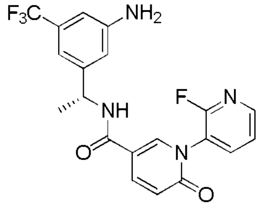 , 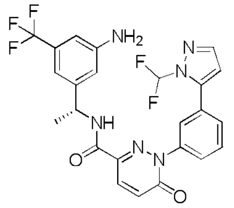 , 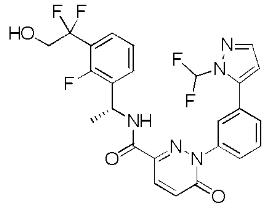 , 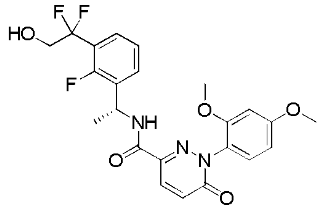 ,

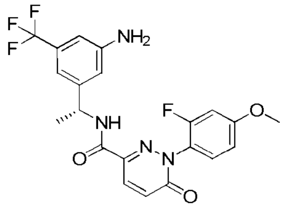 , 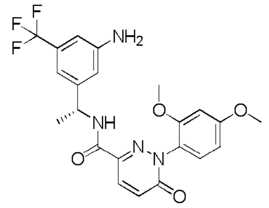 , 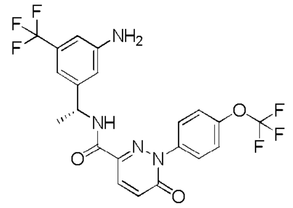 , 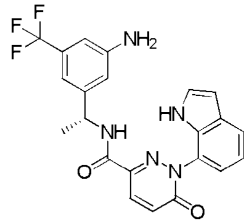 ,

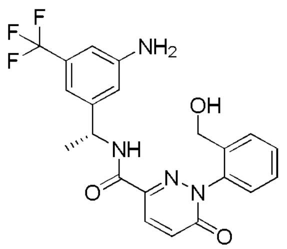 , 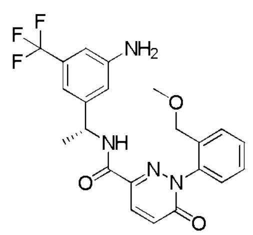 , 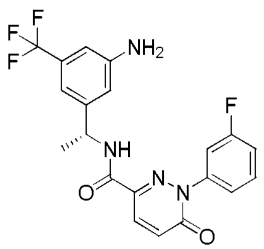 , 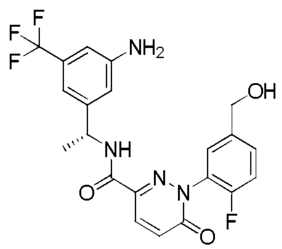 ,

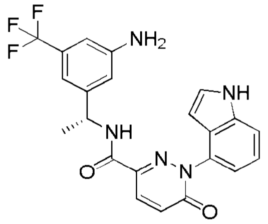 , 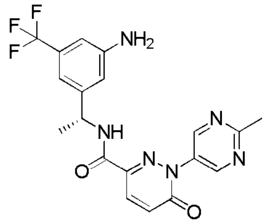 , 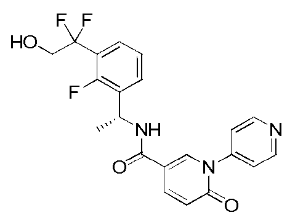 , 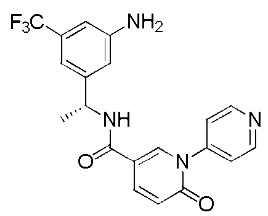 ,

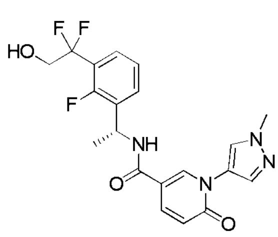 , 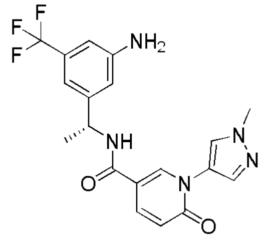 , 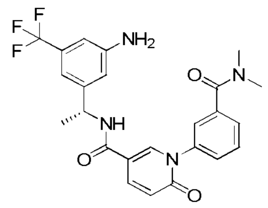 , 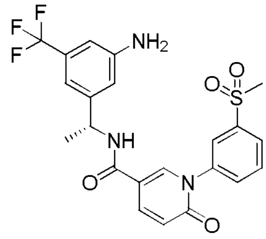 ,

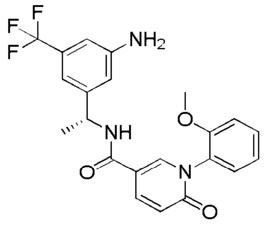 , 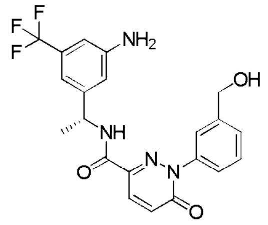 , 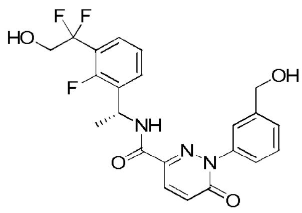 , 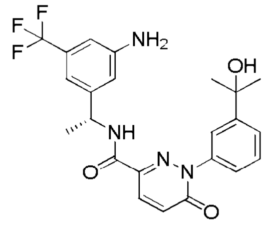 ,

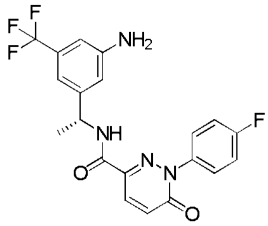 , 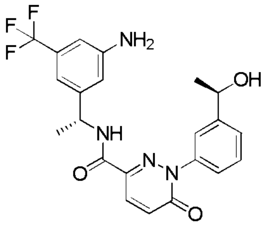 , 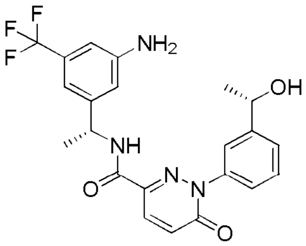 , 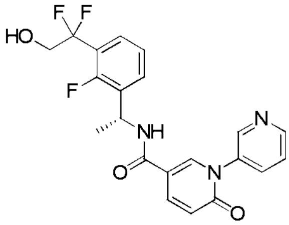 ,

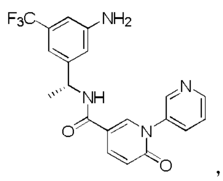 , 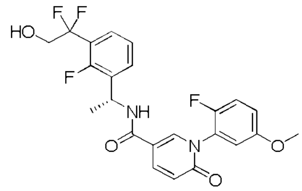 , 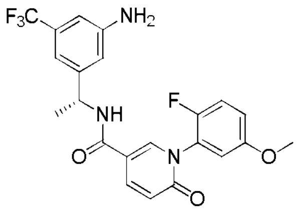 , 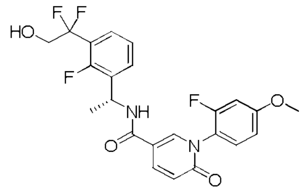 ,

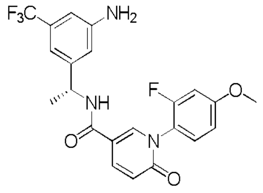 , 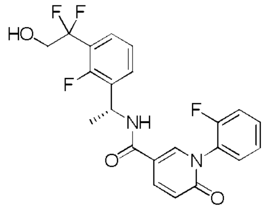 , 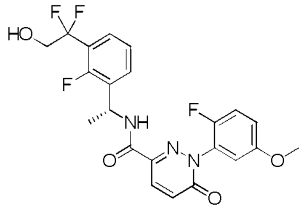 , 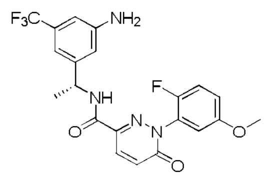 ,

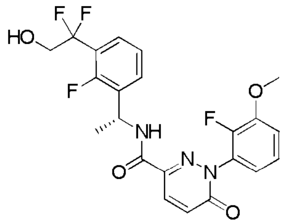 , 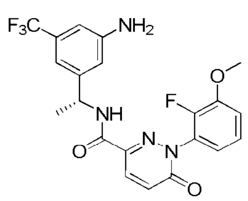 , 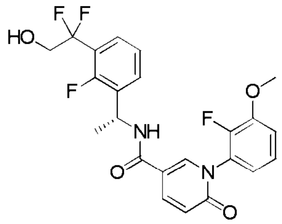 , 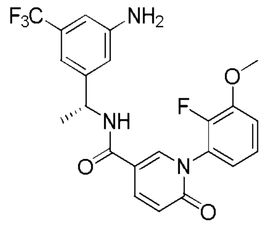 ,

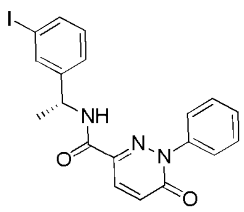 , 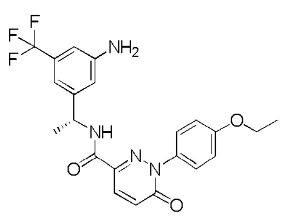 , 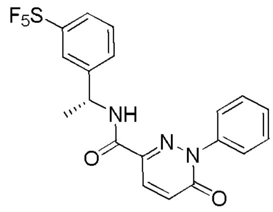 , 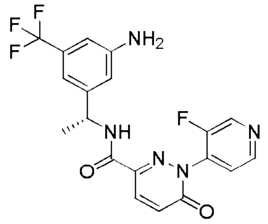 ,

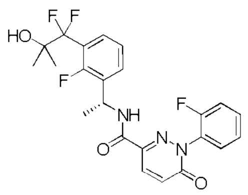 , 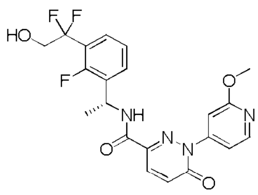 , 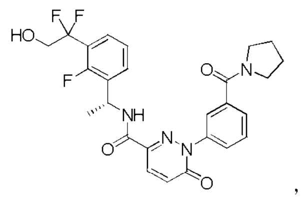 , 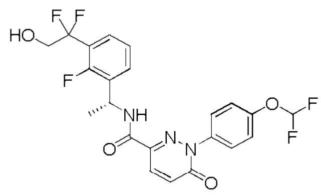 ,

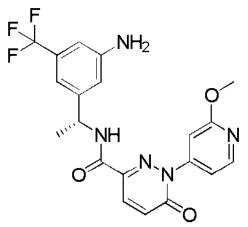 , 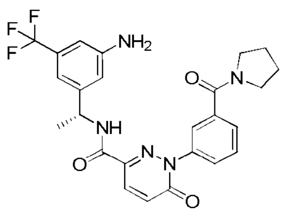 , 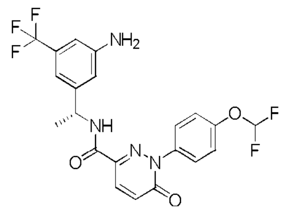 , 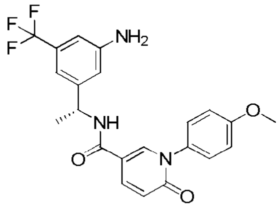 ,

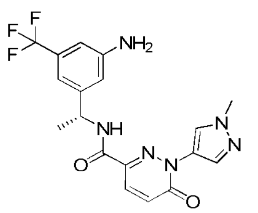 , 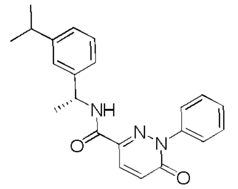 , 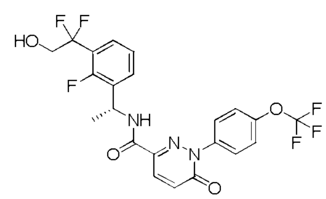 , 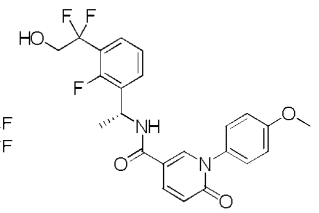 ,

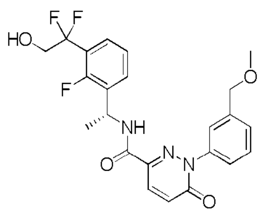 , 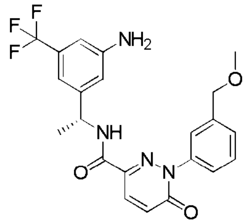 , 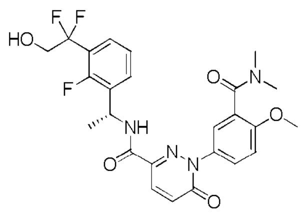 , 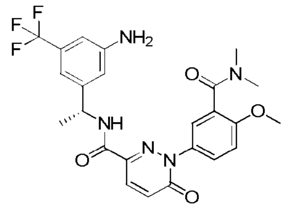 ,

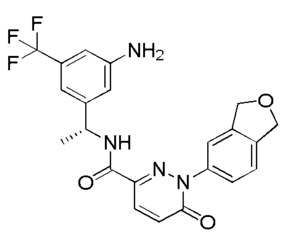 , 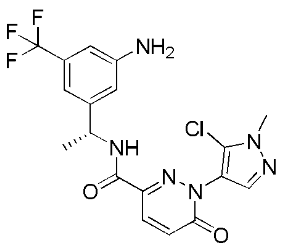 , 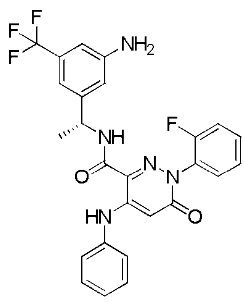 , 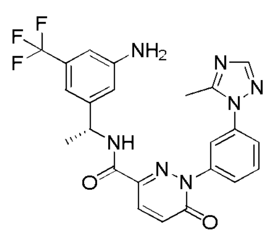 ,

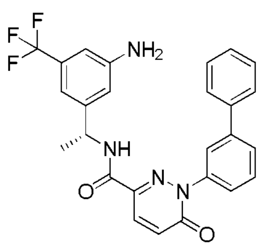,
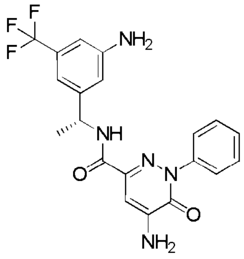,
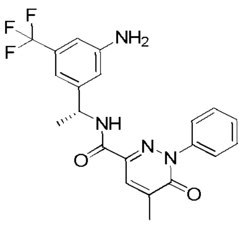,
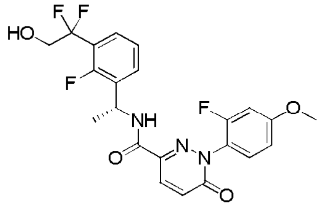,

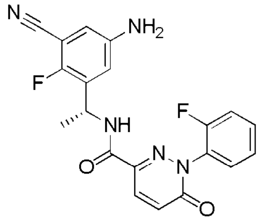,
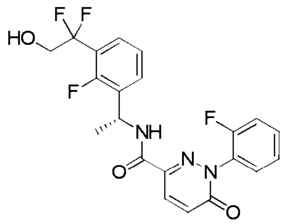,
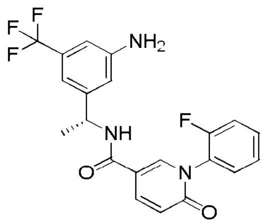,
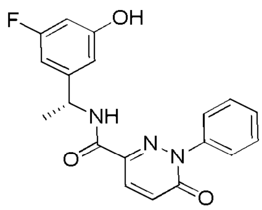,

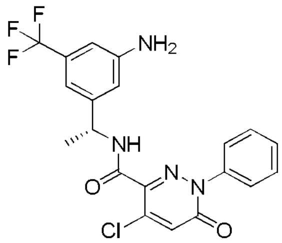,
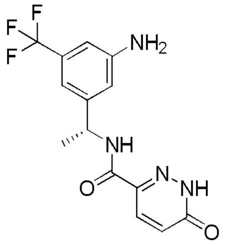,
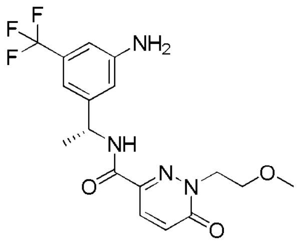,
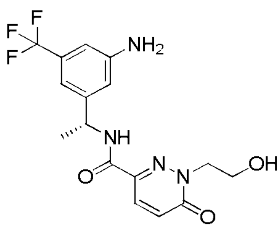,

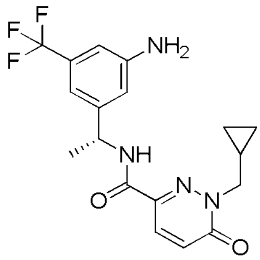,
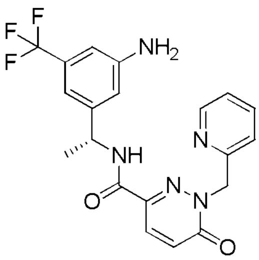,
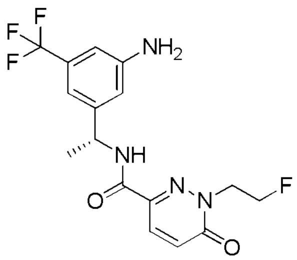,
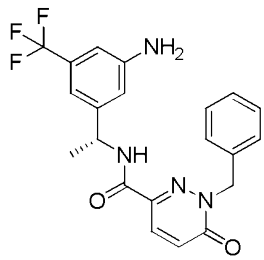,

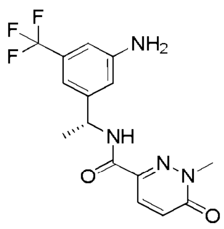,
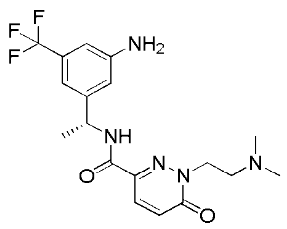,
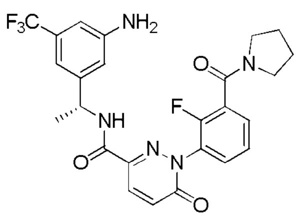,
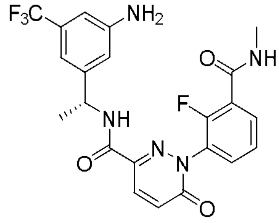,

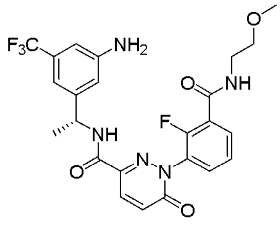,
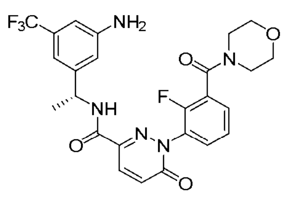,
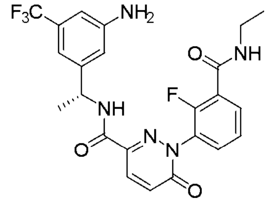,
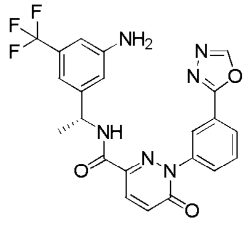,

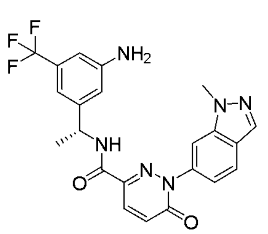 , 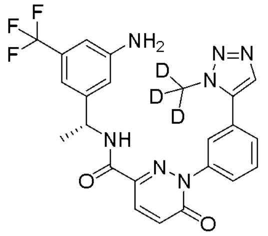 , 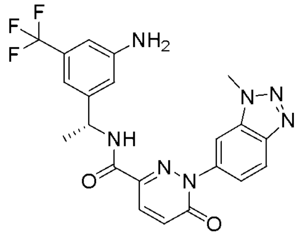 , 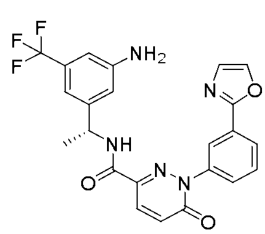 ,

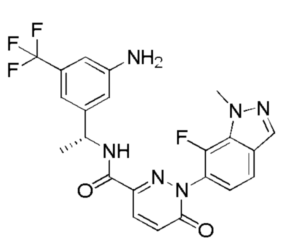 , 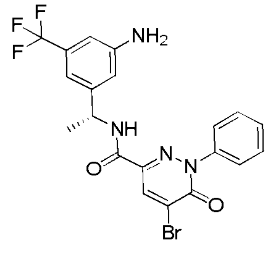 , 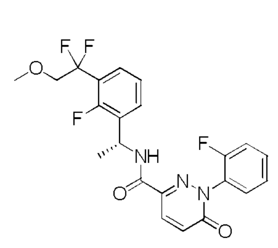 , 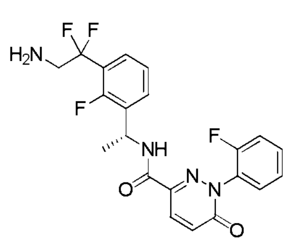 ,

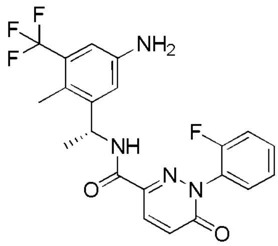 , 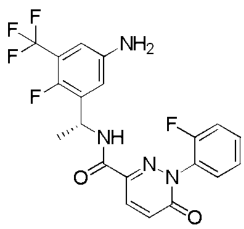 , 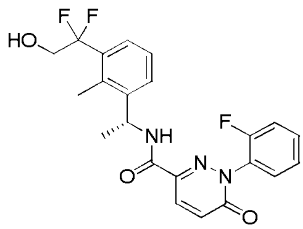 , 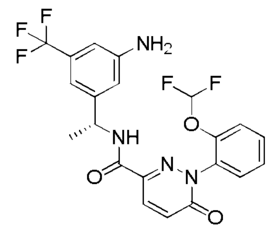 ,

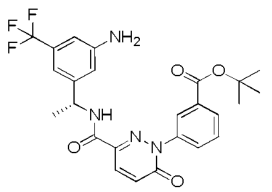 , 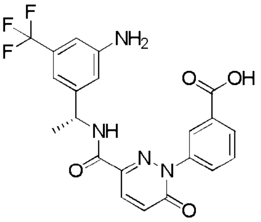 , 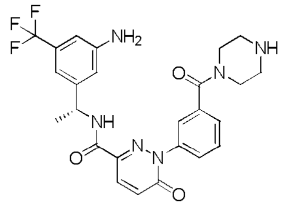 , 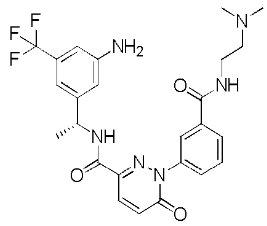 ,

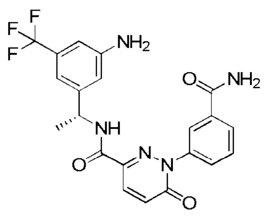 , 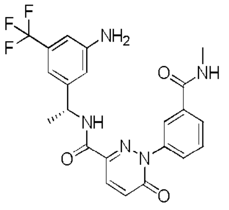 , 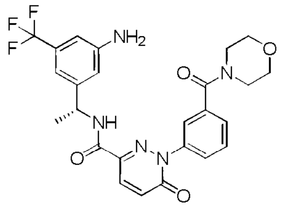 , 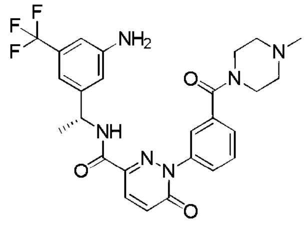 ,

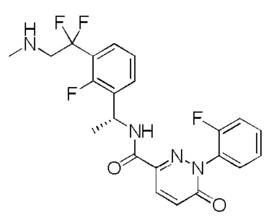 , 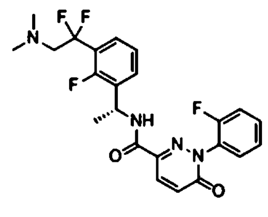 , 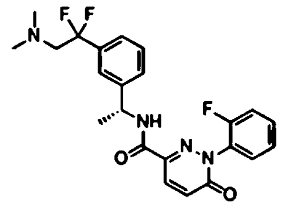 , 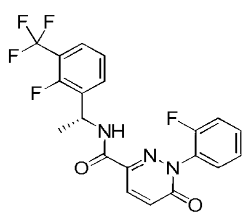 ,

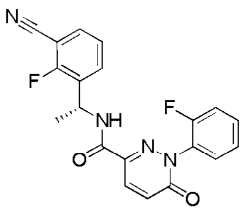,
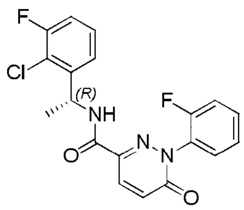,
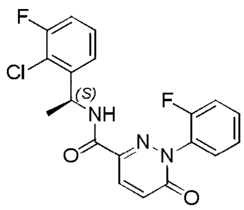,
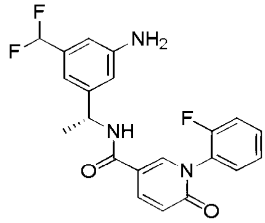,

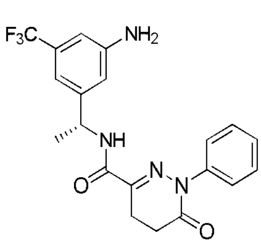,
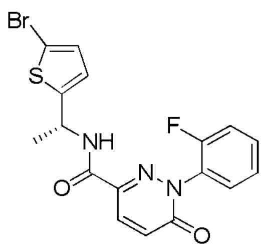,
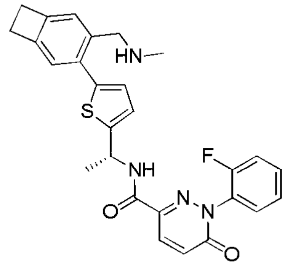,
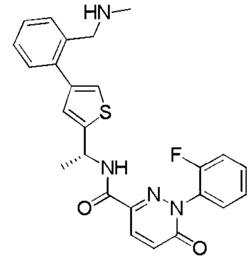,

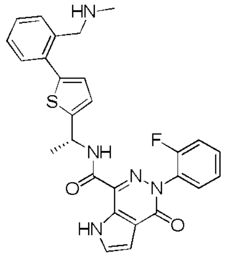,
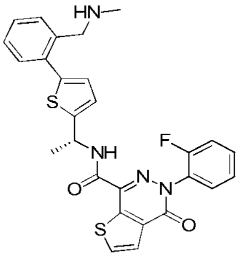,
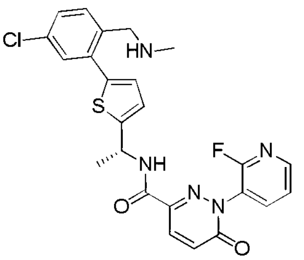,
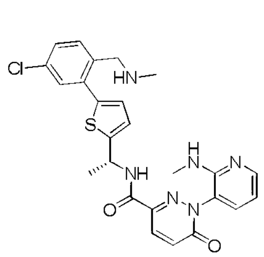,

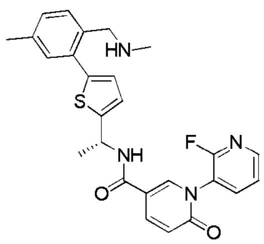,
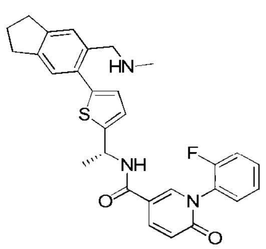,
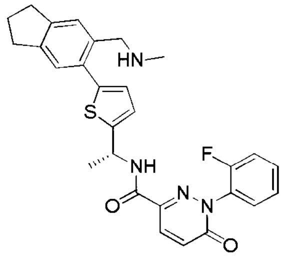,
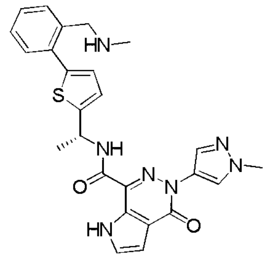,

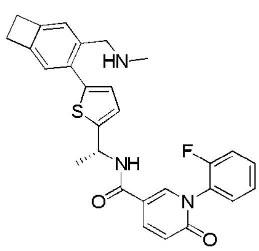,
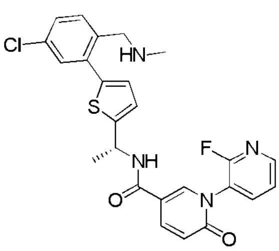,
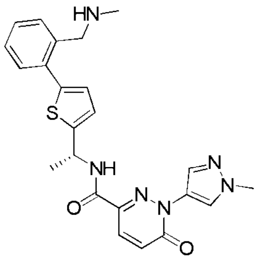,
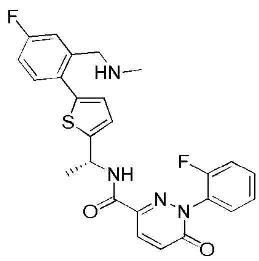,

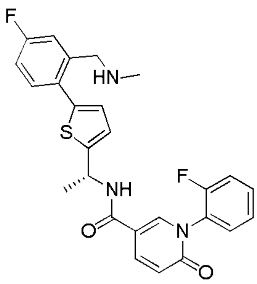,
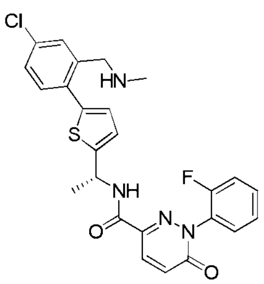,
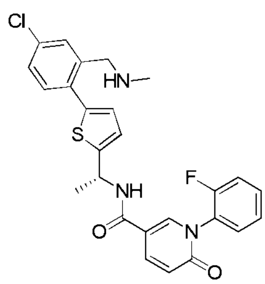,
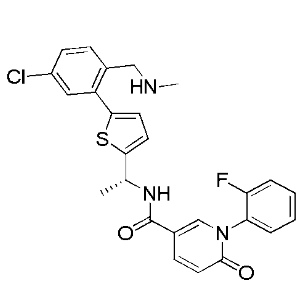,

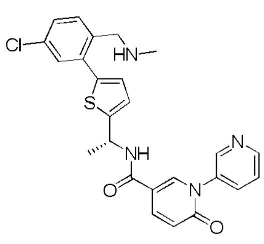,
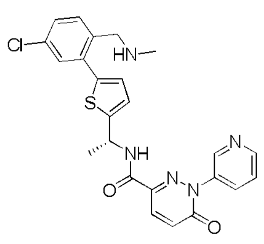,
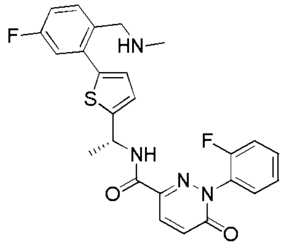,
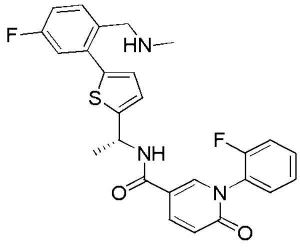,

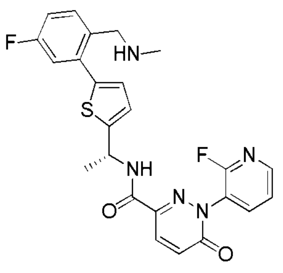,
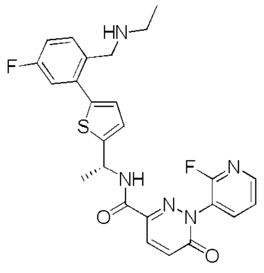,
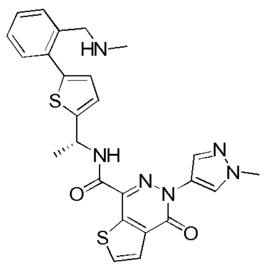,
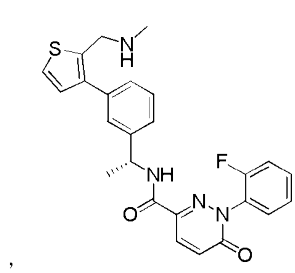,

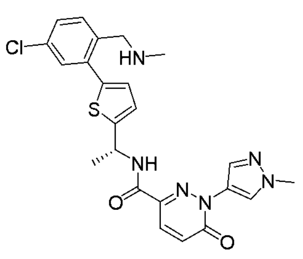,
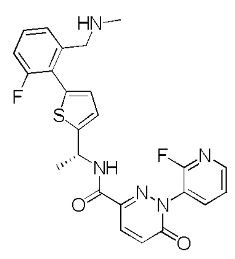,
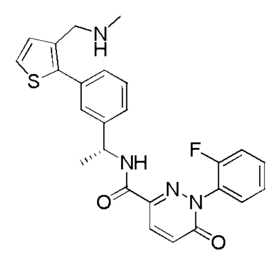,
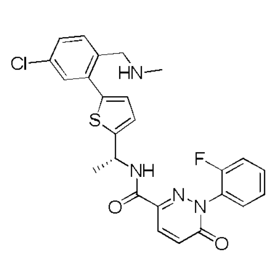,

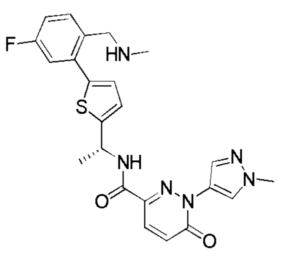,
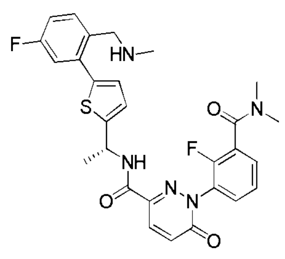,
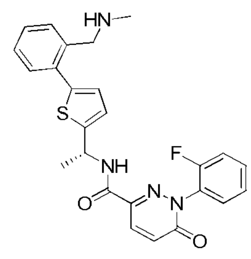,
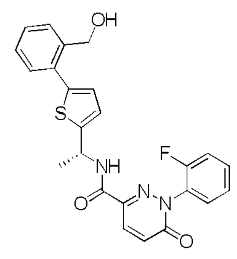,

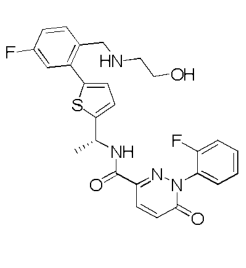,
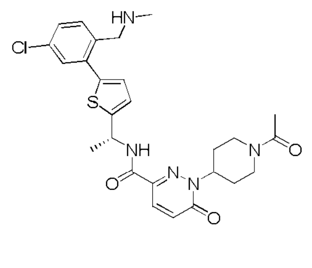,
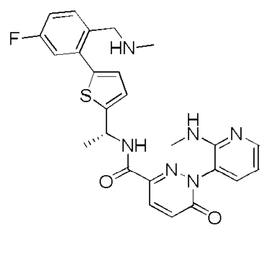,
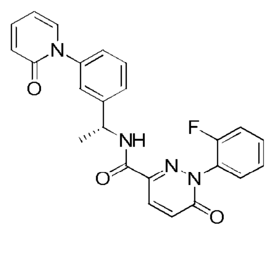,

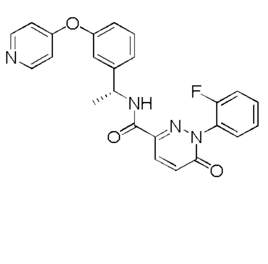,
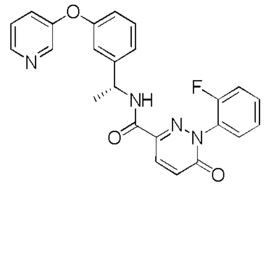,
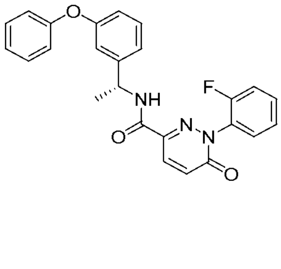,
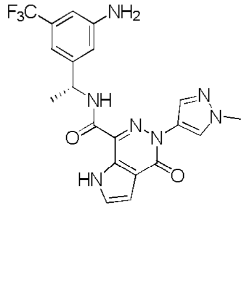,

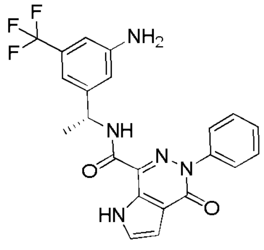 , 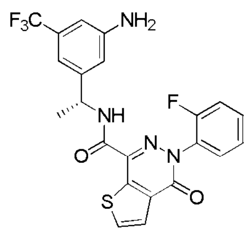 , 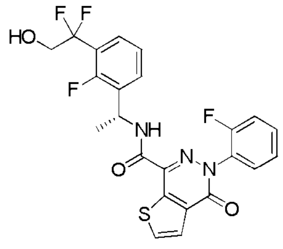 , 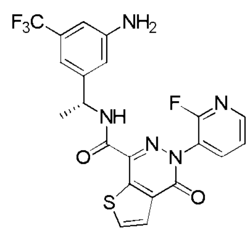 ,

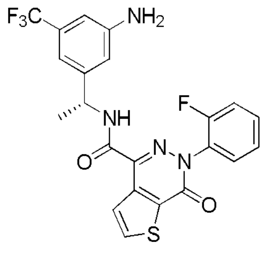 , 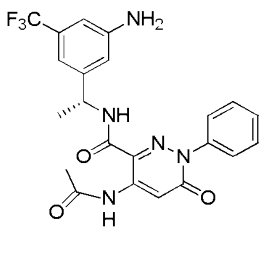 , 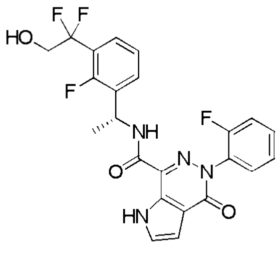 , 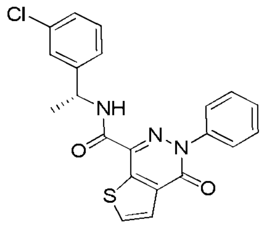 ,

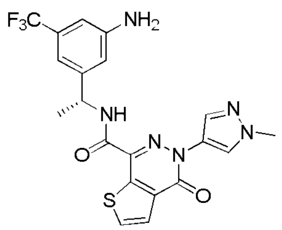 , 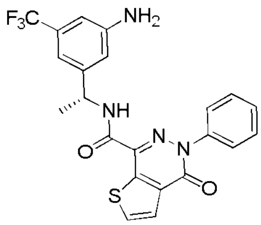 , 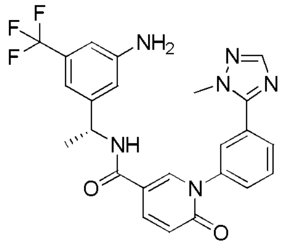 , 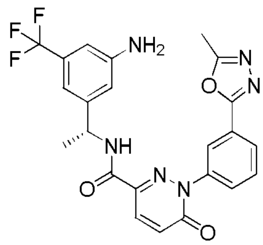 ,

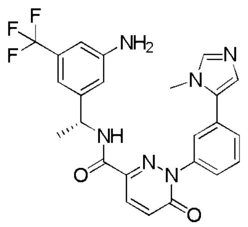 , 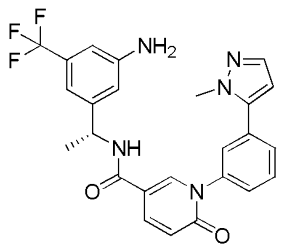 , 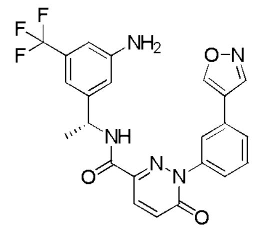 , 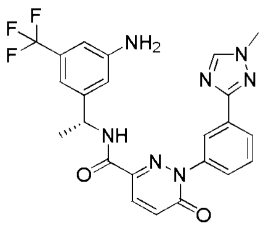 ,

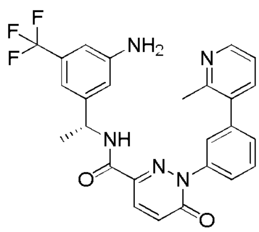 , 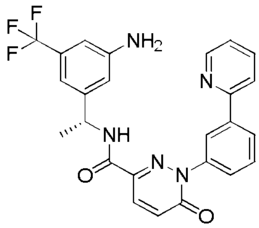 , 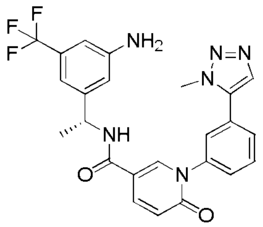 , 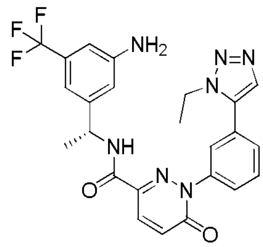 ,

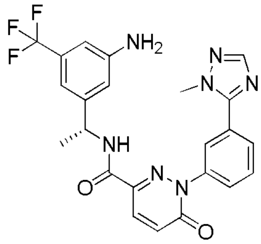 , 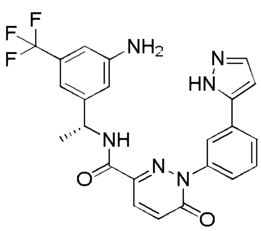 , 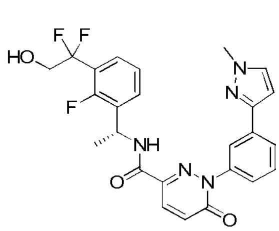 , 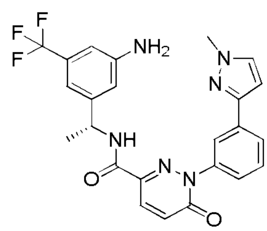 ,

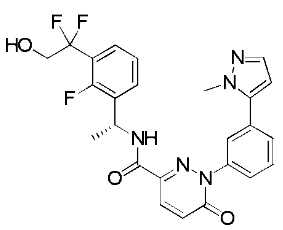 , 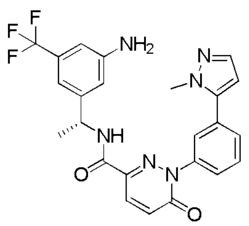 , 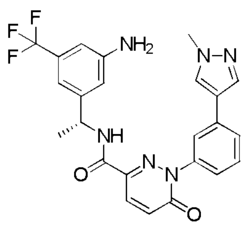 , 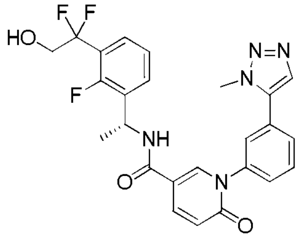 ,

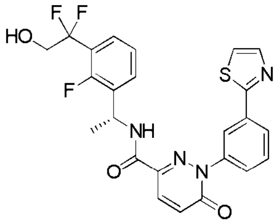 , 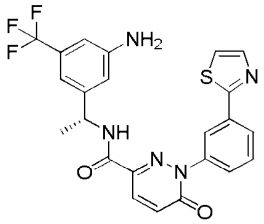 , 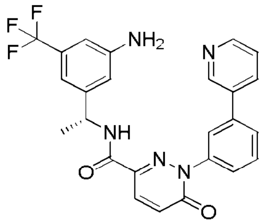 , 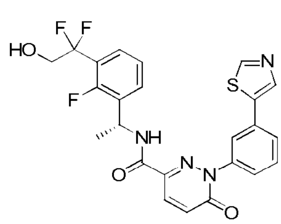 ,

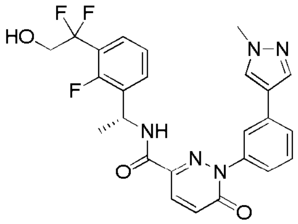 , 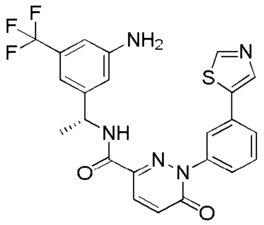 , 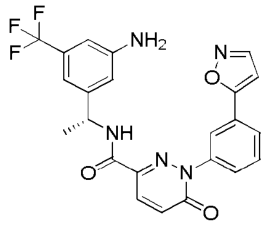 , 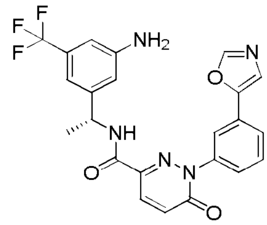 ,

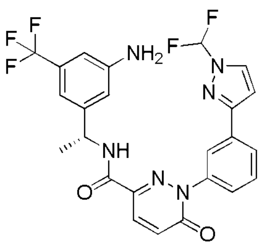 , 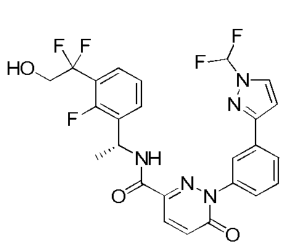 , 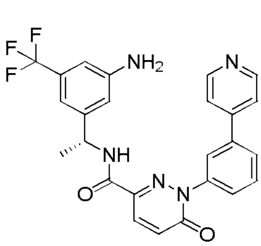 , 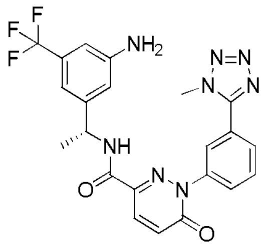 ,

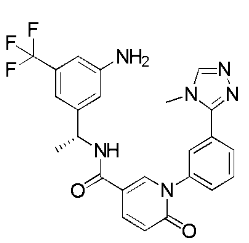 , 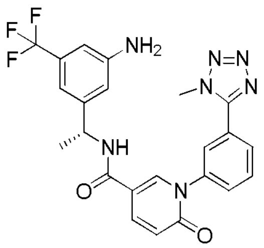 , 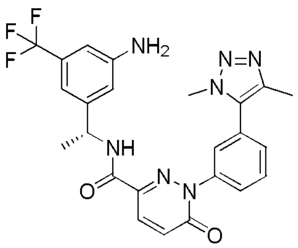 , 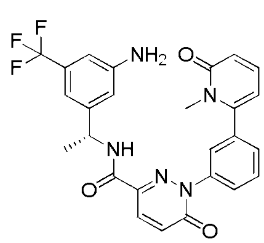 ,

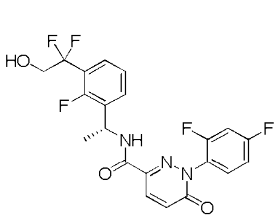 , 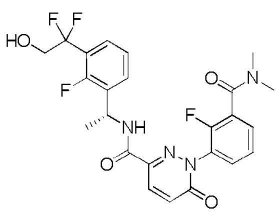 , 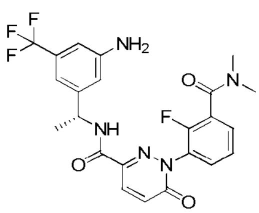 , 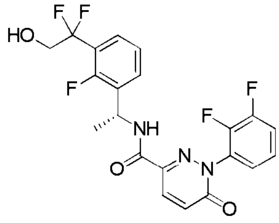 ,

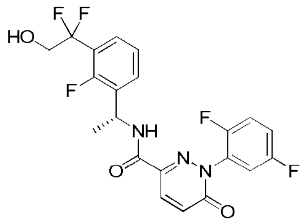 , 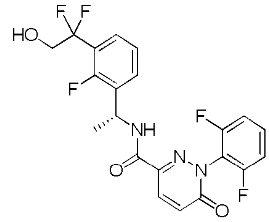 , 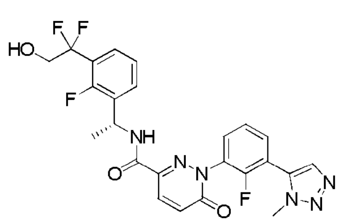 ,

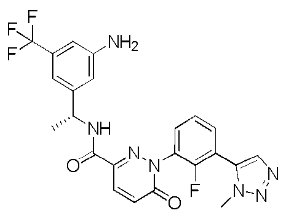 , 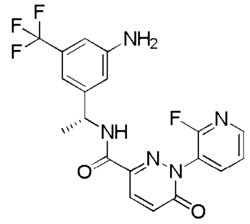 , 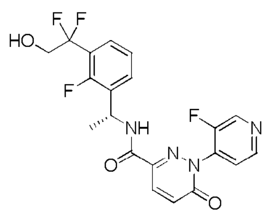 , 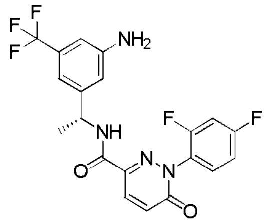 ,

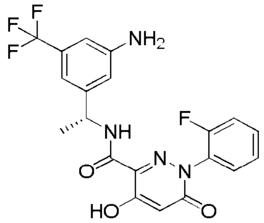 , 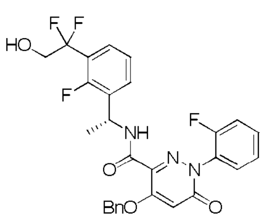 , 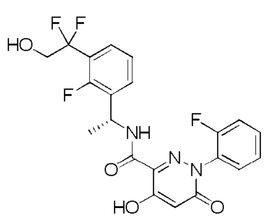 , 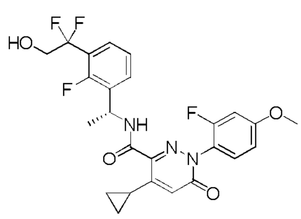 ,

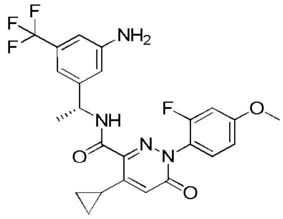 , 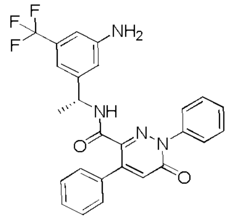 , 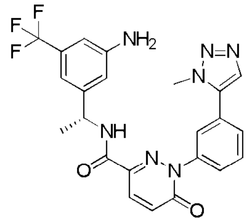 , 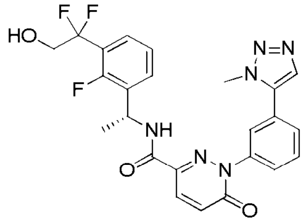 ,

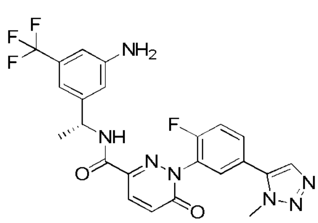 , 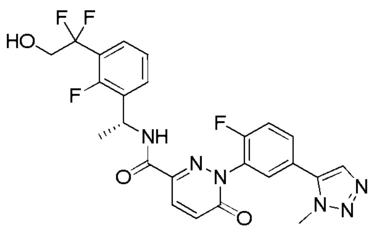 , 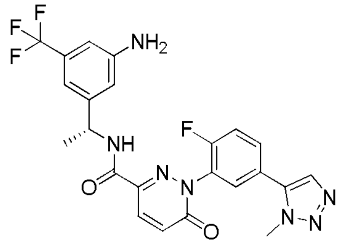 ,

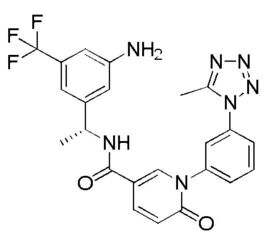 , 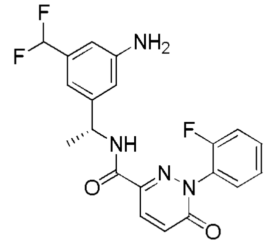 , 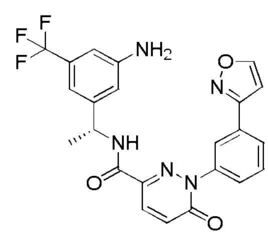 , 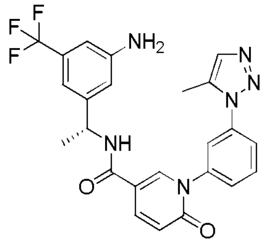 ,

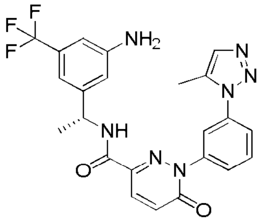 , 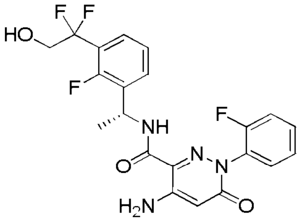 , 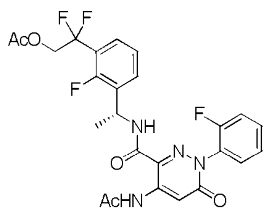 , 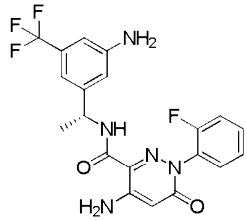 ,

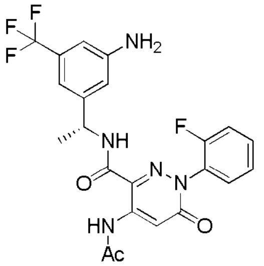 , 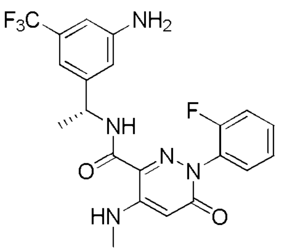 , 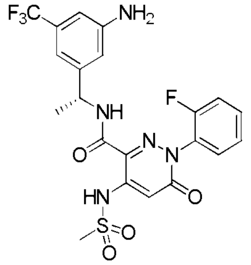 , 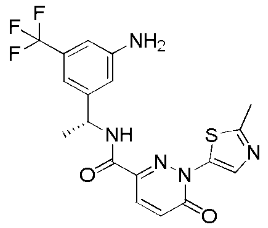 ,

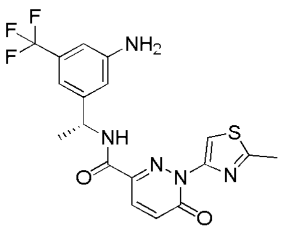 , 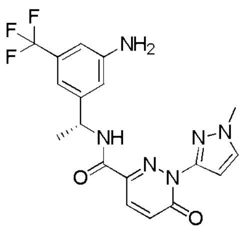 , 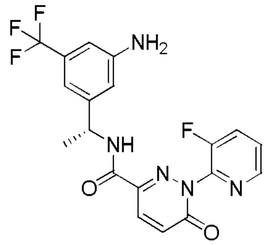 , 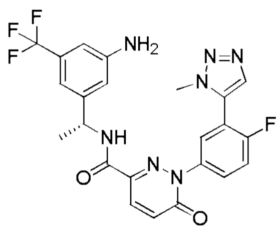 ,

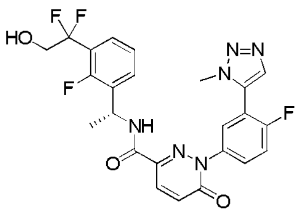 , 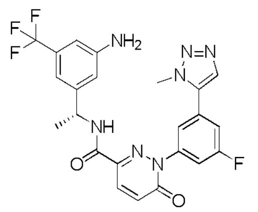 , 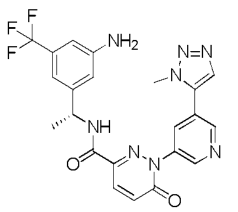 , 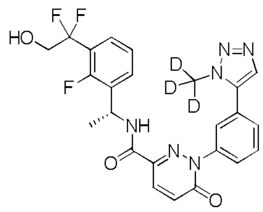 ,

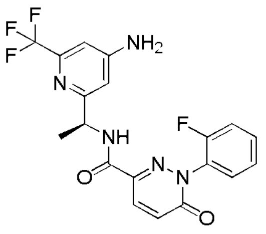 , 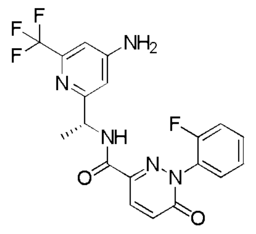 , 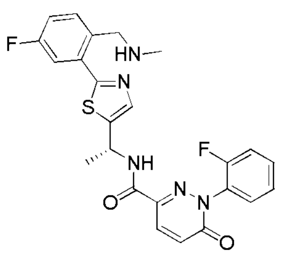 , 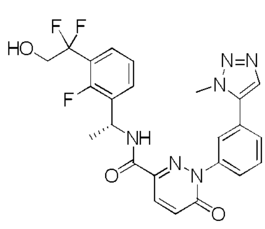 ,

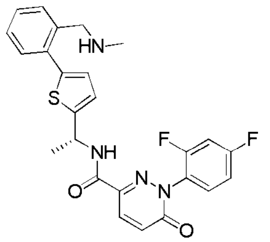 , 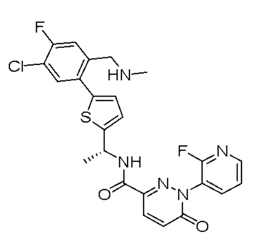 , 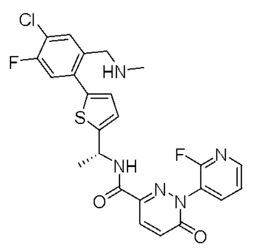 , 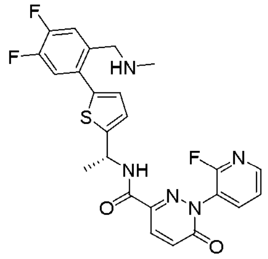 ,

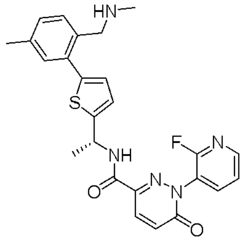 , 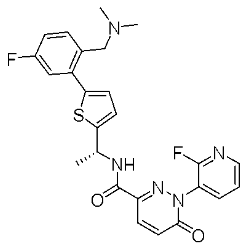 , 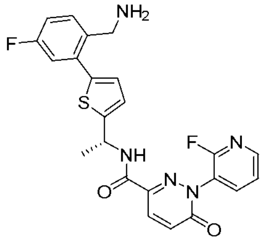 , 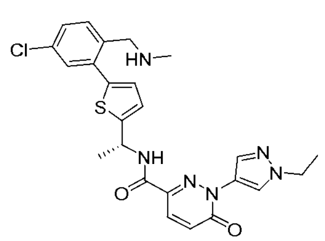 ,

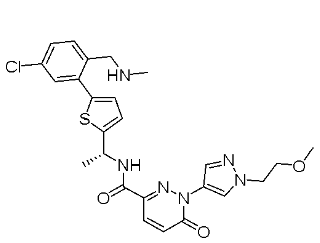 , 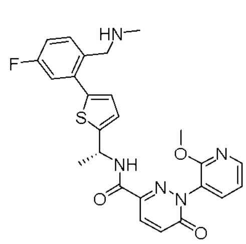 , 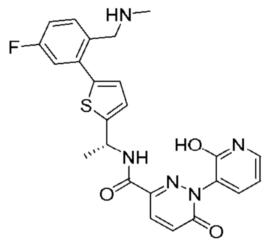 , 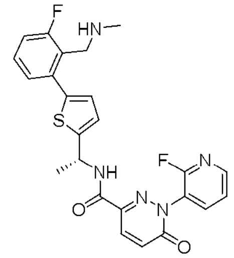 ,

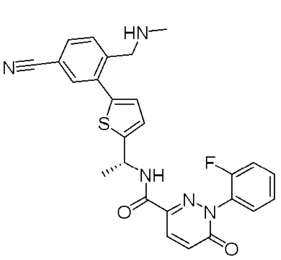 , 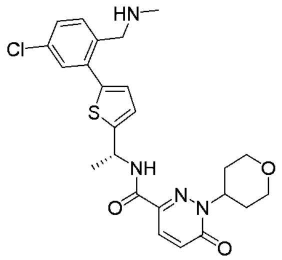 , 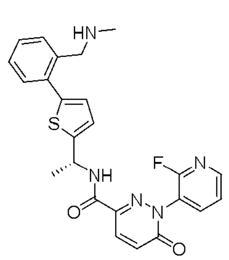 , 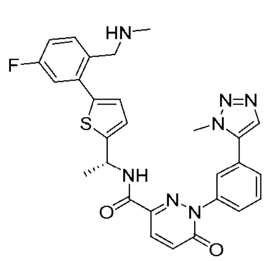 ,

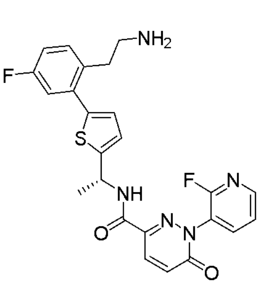 , 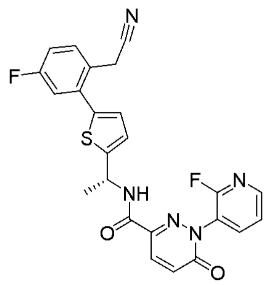 , 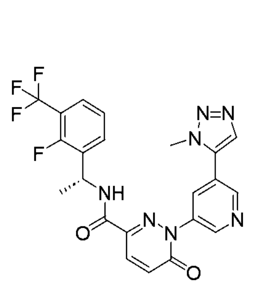 , 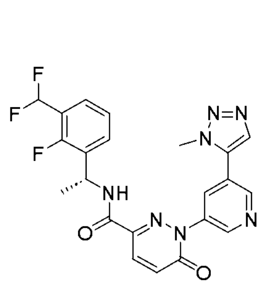 ,

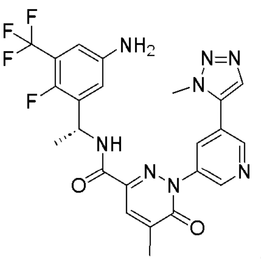 , 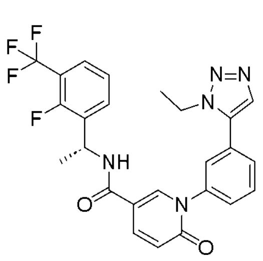 , 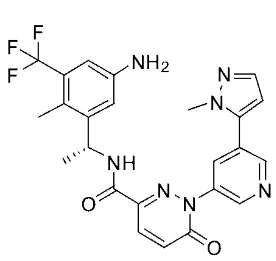 , 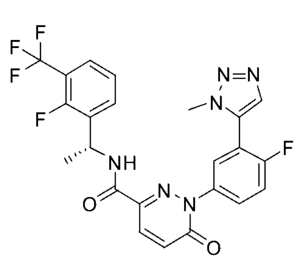 ,

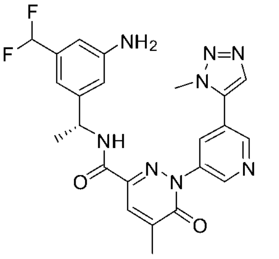 , 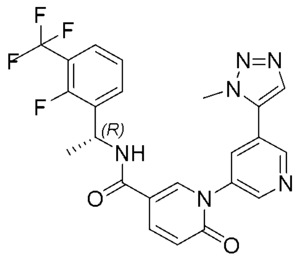 , 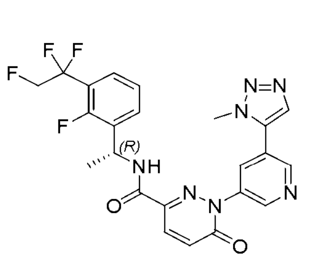 ,

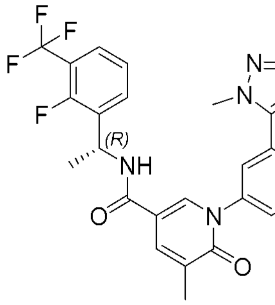

, and

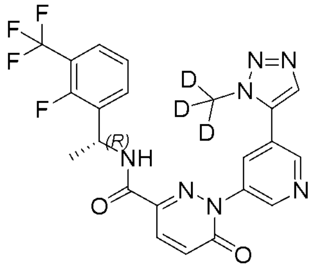

.

**41.** The pharmaceutical composition for preventing or treating cancer according to claim 1, wherein the anticancer agent is any one selected from the group consisting of chemical anticancer agents, targeted anticancer agents, anticancer viruses, antibody therapeutic agents, cell therapeutic agents and immune checkpoint inhibitors.

**42.** The pharmaceutical composition for preventing or treating cancer according to claim 41, wherein the chemical anticancer agent is any one selected from the group consisting of alkylating agents, microtubule inhibitors, antimetabolites and topoisomerase inhibitors.

**43.** The pharmaceutical composition for preventing or treating cancer according to claim 42, wherein the chemical anticancer agent is any one selected from the group consisting of Mechlorethamine, Cyclophosphamide, Ifosfamide, Melphalan, Chlorambucil, Thiotepa, Altretamine, Procarbazine, Busulfan, Streptozotocin, Carmustine, Lomustine, Dacarbazine, Cisplatin, Carboplatin, Oxaliplatin, Docetaxel, Velban, Oncovin, Navelbine, Fluorouracil, Capecitabine, Cytarabine, Gemcitabine, Fludarabine, Methotrexate, Pemetrexed, 6-thioguanine, Mercaptopurine, Hycamtin, Camptosar, Vepesid, Paclitaxel, Blenoxane, Adriamycin, SN-38, Doxorubicin and Cerubidine.

**44.** The pharmaceutical composition for preventing or treating cancer according to claim 41, wherein the targeted anticancer agent targets any one protein selected from the group consisting of mTOR, PI3K, EGFR, VEGFR, CD20, CD38, RNAK-L, BTK, Bcr-abl, PDGFR/FGFR family, MEK, KRAS, ERK1/2, HER2/Neu, Ubiquitin, JAK, ALK, PARP, TGFβR, Proteasome, Bcl-2, C-Met, VR1, VR2, VR3, c-kit, AXL, RET, BRAF, pan-RAF, SHP2, SRC, LCK, DNMT, CDK4/6, CDK9, BET, MDM2, IGF1/2 or IGF1-R, ROS1, NTRK1, PIK, DHFR, pan Aurora, Aurora A, WEE1, HSP90, A3AR, EZH2, ARID1A, Chk1, ATR, HDAC1/3, Akt, PLK1, SUMOylation-related proteins and STING.

**45.** The pharmaceutical composition for preventing or treating cancer according to claim 44, wherein the targeted anticancer agent is any one selected from the group consisting of Rapamycin, Sirolimus, Temsilorimus, Everolimus, Ridaforolimus, INK-128, Alpelisib, Cetuximab, Trastuzumab, Pertuzumab, Gefitinib, Erlotinib, Osimertinib, Lazertinib, Panitumumab, Axitinib, Lenvatinib, Bevacizumab, Ramucirumab, Aflibercept, Rituximab, Obinutuzumab, Daratumumab, Denosumab, Ibrutinib, Dasatinib, Nilotinib, Imatinib, Bosutinib, Galunisertib, Vactosertib, Futibatinib, Nintedanib, Sunitinib, Sorafenib, Cabozantinib, Regorafenib, Masitinib, Semaxanib, Tivozanib, Vandetanib, Pazopanib, Dabrafenib, Sotorasib, Adagrasib, JDQ443, MRTX1133, Ulixertinib, Afatinib, Lapatinib, Neratinib, Lenalidomide, Ixazomib, Ruxolitinib, Lestaurtinib, Pacritinib, Trametinib, Cobimetinib, Selumetinib, Binimetinib, Alectinib, Lorlatinib, Crizotinib, Venetoclax, Bemcentinib, Gilteritinib, Selpercatinib, Pralsetinib, Encorafenib, Vemurafenib, Belvarafenib, RMC-4630, Batoprotafib, WH-4-023, Olaparib, Talazoparib, Niraparib, Rucaparib, Azacitidine, Decitabine, Guadecitabine, Abemaciclib, Ribociclib, Palbociclib, CDNs, SB11285, Rineterkib, Repotrectinib, Tepotinib, Alrizomadlin, JQ1, NVP-ADW742, Duvelisib, Irbinitinib, Danusertib, MK-1775, AMG-900, BIIB021, Reversine, MLN-7243, ABT-737, MK-5108, GSK-343, 2-D08, SCH-900776, Entinostat, Carfilzomib, Apitolisib, Ipatasertib, Volasertib, AT-7519, Methotrexate, Wortmannin, ERAS-007, PYR-41, MLN4924, RO-5503781, MK-8242, SAR-405838, CGM097, DS3032b, Lactacystin, Disulfiram, Epigallocatechin-3-gallate, Marizomib, Oprozomib, Delanzomib, Epoxomicin, MG132, Beta-hydroxy beta-methylbutyrate, Bortezomib, Navitoclax, Naporafenib, PF-07284892, TNO155, Hesperadin, LY3295668 , Tozasertib, Azenosertib, ZNL-02-096, RP-6306, GSK-1520489A, BIIB028, MPC-3100, PU-H71, Debio093, SNX-5422, AUY922, KF-26777, MRS-545, CAY10498, DZNep, EPZ005687, EI1, GSK126, UNC1999, Tazemetostat, Sinefungin, GSK-343 , Davidiin, CID9549553, SRA737, V158411, PF-477736, AZD7762, Prexasertib, Berzosertib, Gartisertib, Ceralasertib, Panobinostat, Mocetinostat, Trichostatin A, CBUD-1001, Abexinostat, VQD-002, Perifosine, Miltefosine, MK-2206, AZD5363, Rigosertib, I-BET 151, I-BET 762, OTX-015, TEN-010, CPI-203, CPI-0610, Olinone, RVX-208, ABBV-744, LY294002, AZD5153, MT-1, MS645, Figitumumab, Mecasermin, rhIGF-1, BI 885578, Buparlisib, Copanlisib, Dactolisib, Idelalisib, Parsaclisib, Paxalisib, Taselisib, Zandelisib, Inavolisib, AZD4573, Atuveciclib, VIP152, A-1592668, JSH-150, SLS009, Roscovitine and DMXAA.

**46.** The pharmaceutical composition for preventing or treating cancer according to claim 41, wherein the anticancer virus is Talimogene Laherparepvec.

**47.** The pharmaceutical composition for preventing or treating cancer according to claim 41, wherein the antibody therapeutic agent is any one selected from the group consisting of Cetuximab, Trastuzumab, Pertuzumab, Panitumumab, Rituximab, Daratumumab, Denosumab, Ibritumomab, Tositumomab, Brentuximab, Ofatumumab, Obinutuzumab, Necitumumab, Bevacizumab, Ramucirumab, Nivolumab, Pembrolizumab, Atezolizumab, Durvalumab and Ipilimumab.

**48.** The pharmaceutical composition for preventing or treating cancer according to claim 41, wherein the cell therapeutic agent is any one selected from the group consisting of Tisagenlecleucel and Axicabtagene Ciloleucel.

**49.** The pharmaceutical composition for preventing or treating cancer according to claim 41, wherein the immune checkpoint inhibitor is any one selected from the group consisting of anti-CTLA-4 antibody, anti-PD-1 antibody, anti-PD-L1 antibody, anti-PD-L2 antibody, anti-B7-H4 antibody, anti-HVEM antibody, anti-TIM3 antibody, anti-GAL9 antibody, anti-LAG3 antibody, anti-VISTA antibody, anti-KIR antibody, anti-BTLA antibody and anti-TIGIT antibody.

**50.** The pharmaceutical composition for preventing or treating cancer according to claim 49, wherein the immune checkpoint inhibitor is any one selected from the group consisting of Ipilimumab, Pembrolizumab, Nivolumab, Cemiplimab, Atezolizumab, Avelumab and Durvalumab.

**51.** The pharmaceutical composition according to claim 1, **characterized in that** the cancer is selected from the group consisting of pancreatic cancer, lung cancer, colorectal cancer, biliary tract cancer, multiple myeloma, melanoma, uterine cancer, cervical cancer, endometrial cancer, thyroid cancer, chronic lymphocytic leukemia, acute myeloid leukemia, bladder cancer, urothelial cancer, gastric cancer, squamous cell carcinoma of the head and neck, diffuse large B cell lymphoma, esophageal cancer, hepatocellular cancer, breast cancer, ovarian cancer, prostate cancer, glioblastoma, kidney cancer and sarcoma.

【FIG. 1】

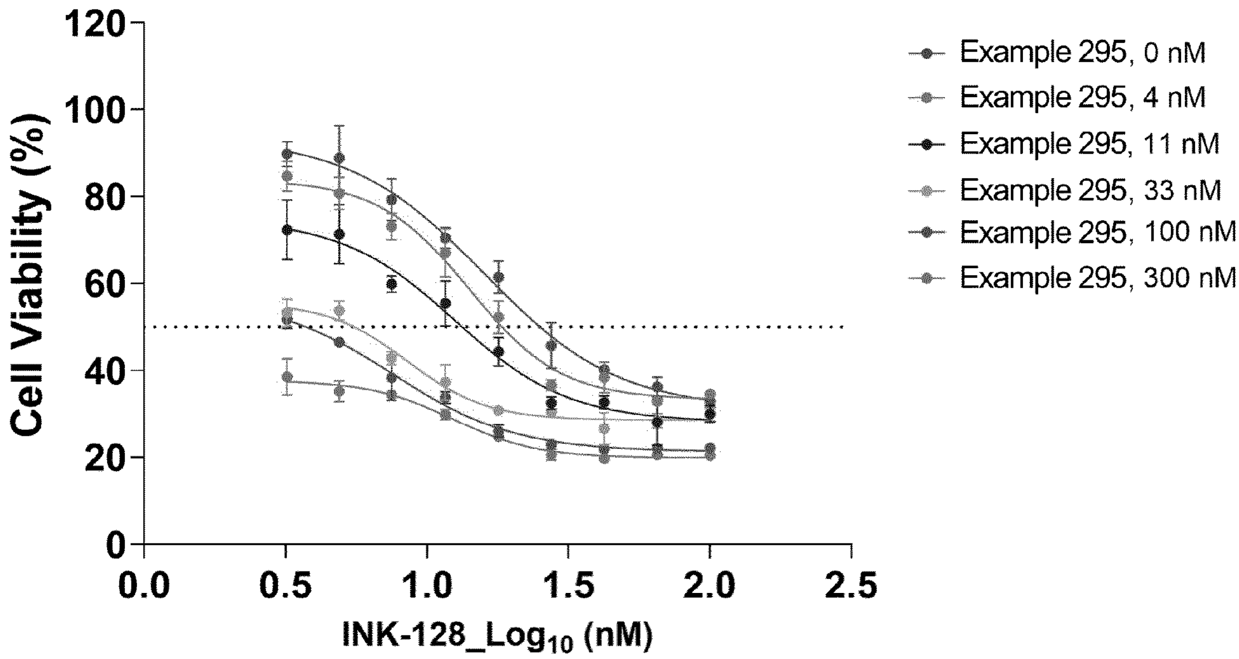
120
100
80
60
40
20
0
Cell Viability (%)
0.0
0.5
1.0
1.5
2.0
2.5
INK-128_Log$_{10}$ (nM)
Example 295, 0 nM
Example 295, 4 nM
Example 295, 11 nM
Example 295, 33 nM
Example 295, 100 nM
Example 295, 300 nM

【FIG. 2】

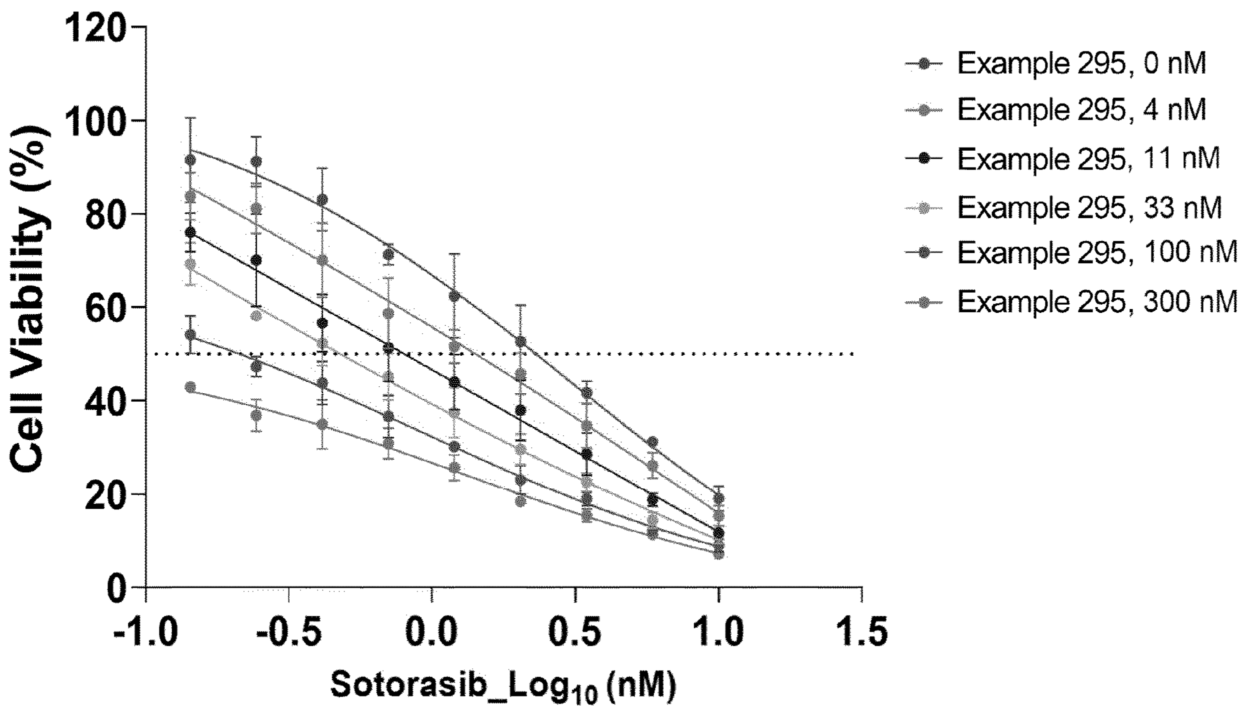
120
100
80
60
40
20
0
Cell Viability (%)
-1.0
-0.5
0.0
0.5
1.0
1.5
Sotorasib_Log$_{10}$ (nM)
Example 295, 0 nM
Example 295, 4 nM
Example 295, 11 nM
Example 295, 33 nM
Example 295, 100 nM
Example 295, 300 nM

【FIG. 3】

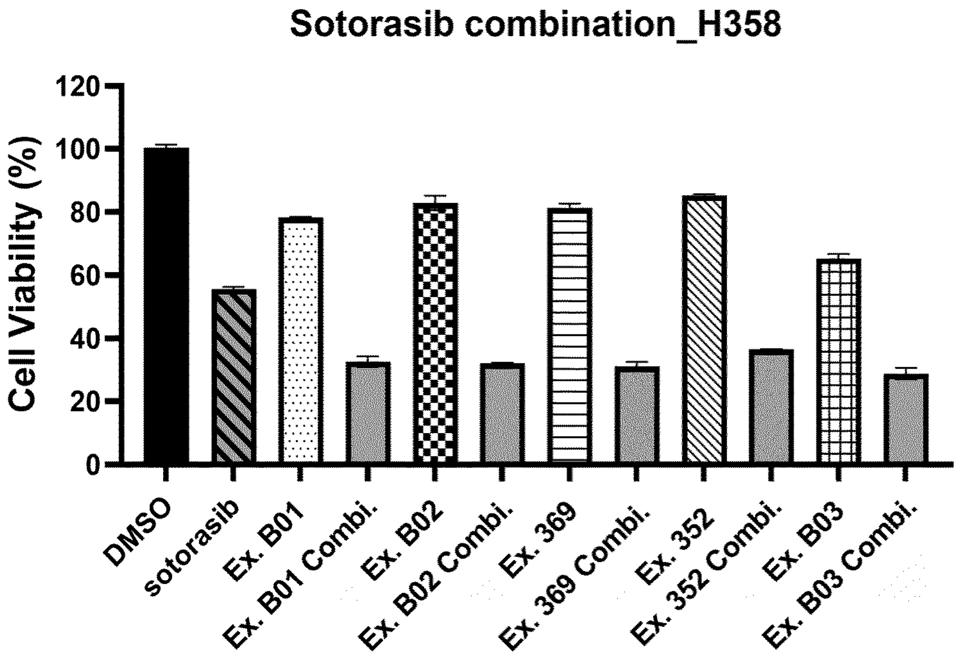
Sotorasib combination_H358
Cell Viability (%)
120
100
80
60
40
20
0
DMSO
sotorasib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 4】

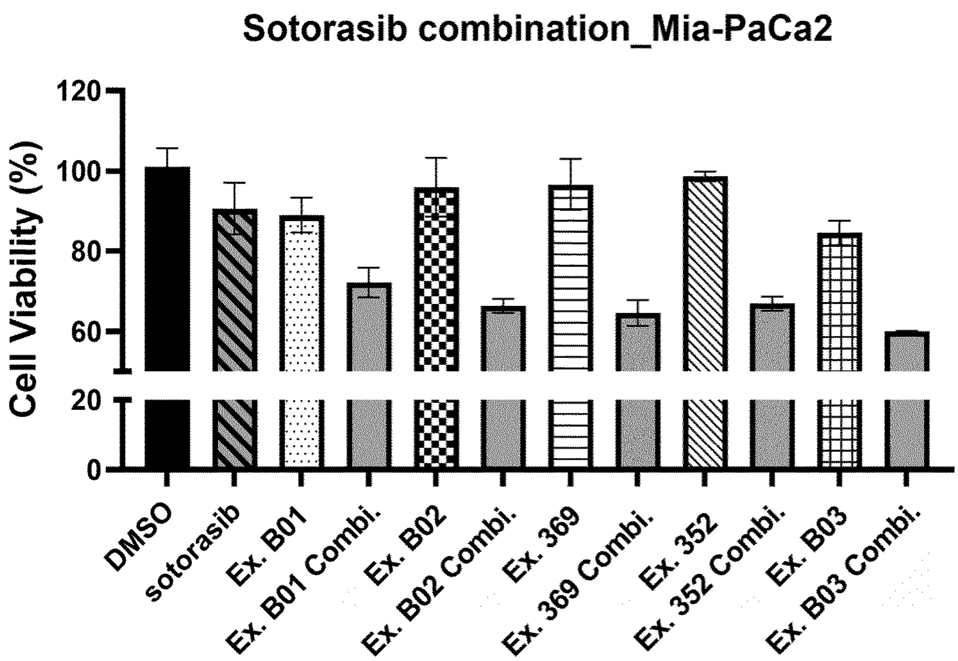
Sotorasib combination_Mia-PaCa2
Cell Viability (%)
120
100
80
60
20
0
DMSO
sotorasib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 5】

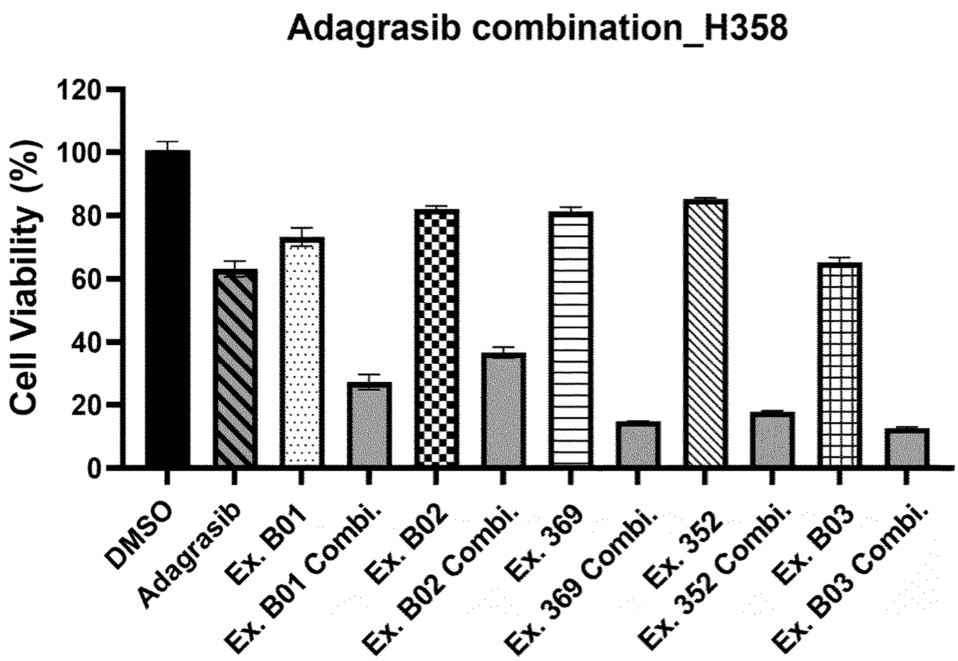
Adagrasib combination_H358
Cell Viability (%)
120
100
80
60
40
20
0
DMSO
Adagrasib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 6】

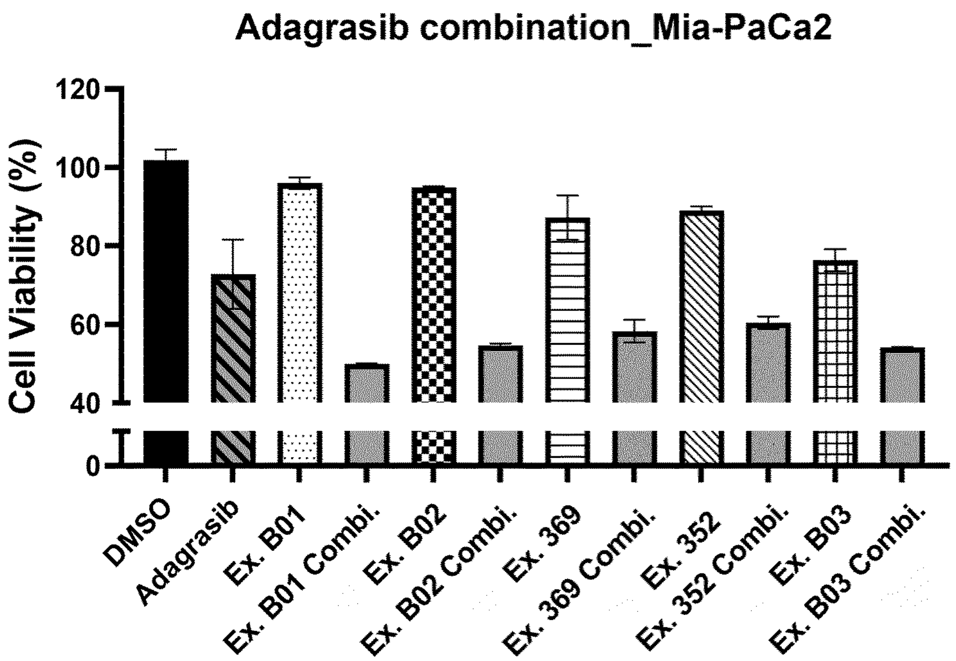
Adagrasib combination_Mia-PaCa2
Cell Viability (%)
120
100
80
60
40
0
DMSO
Adagrasib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 7】

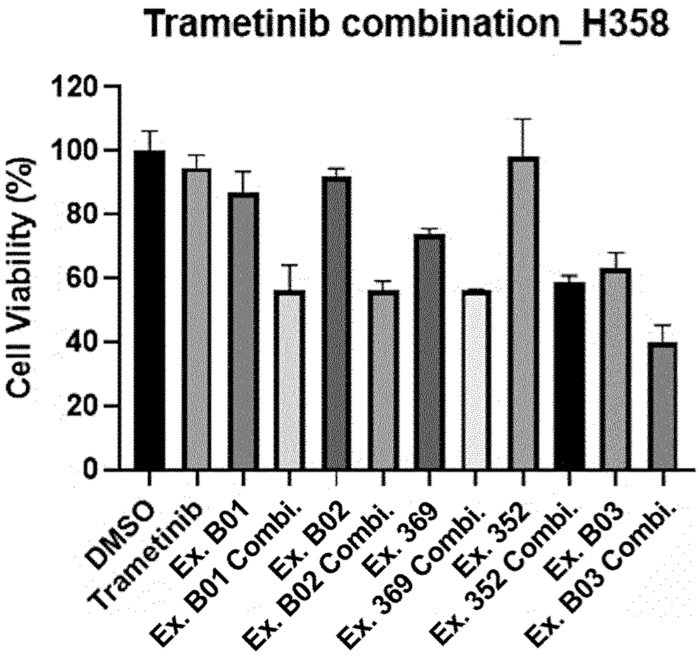
Trametinib combination_H358
Cell Viability (%)
120
100
80
60
40
20
0
DMSO
Trametinib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 8】

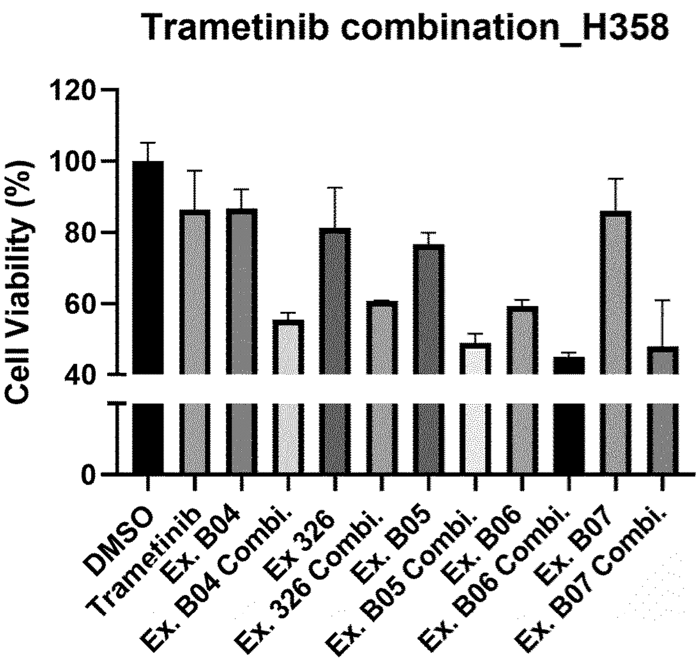
Trametinib combination_H358
Cell Viability (%)
120
100
80
60
40
0
DMSO
Trametinib
Ex. B04
Ex. B04 Combi.
Ex 326
Ex. 326 Combi.
Ex. B05
Ex. B05 Combi.
Ex. B06
Ex. B06 Combi.
Ex. B07
Ex. B07 Combi.

【FIG. 9】

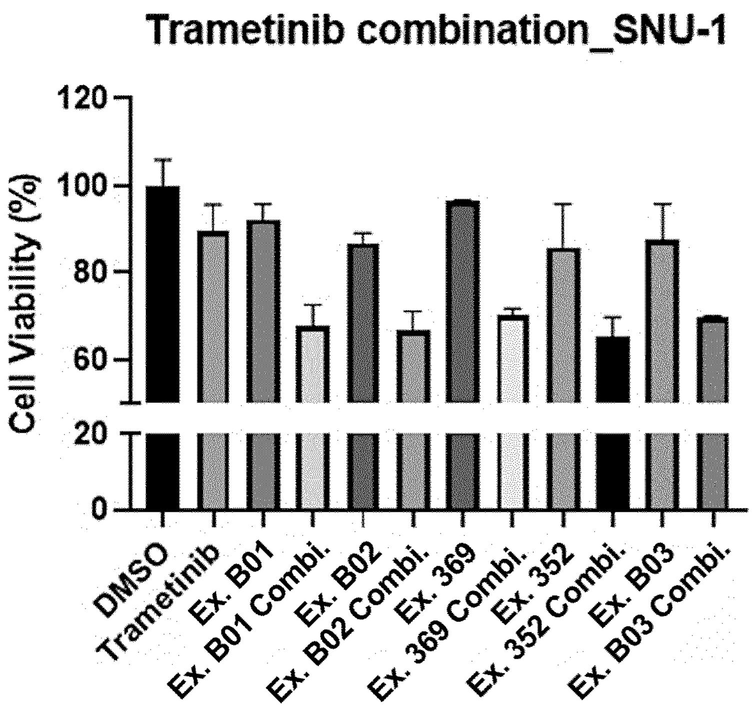
Trametinib combination_SNU-1
Cell Viability (%)
120
100
80
60
20
0
DMSO
Trametinib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 10】

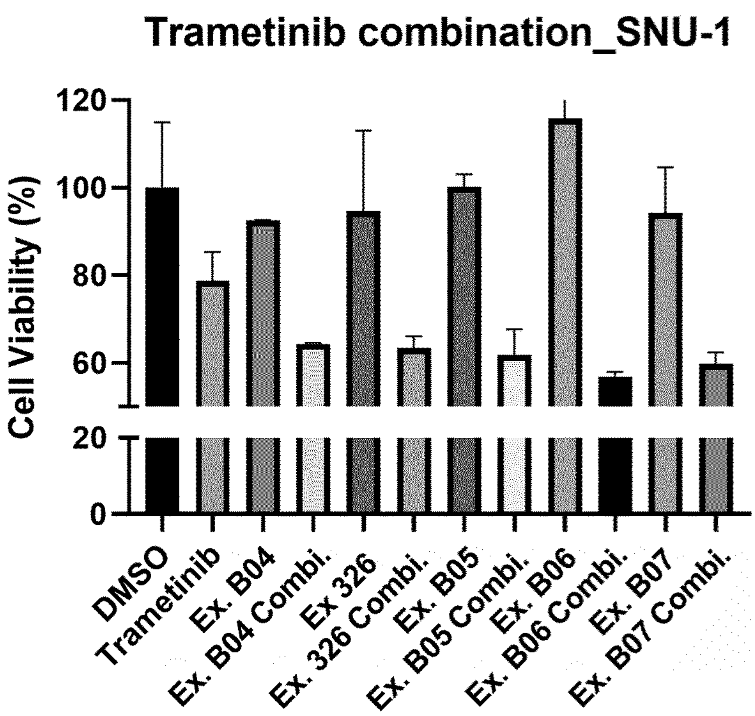
Trametinib combination_SNU-1
Cell Viability (%)
120
100
80
60
20
0
DMSO
Trametinib
Ex. B04
Ex. B04 Combi.
Ex 326
Ex. 326 Combi.
Ex. B05
Ex. B05 Combi.
Ex. B06
Ex. B06 Combi.
Ex. B07
Ex. B07 Combi.

【FIG. 11】

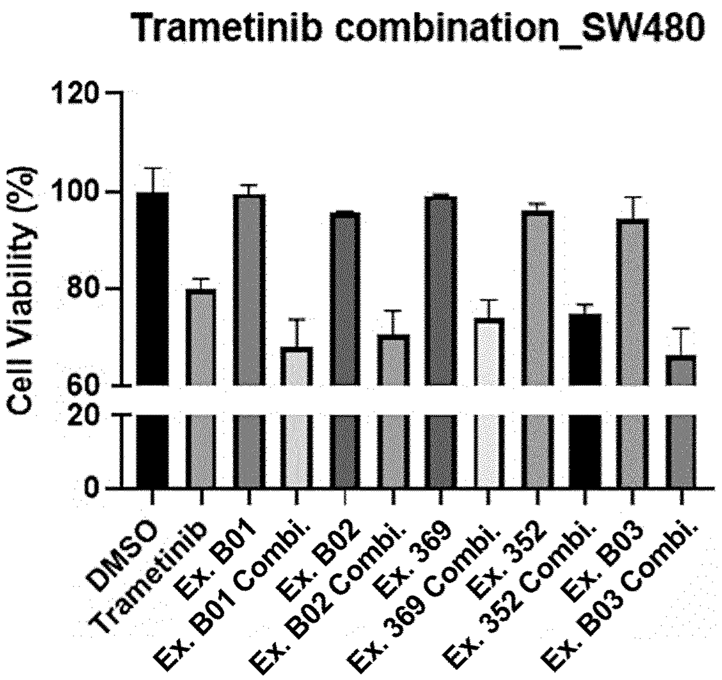

Trametinib combination_SW480
Cell Viability (%)
120
100
80
60
20
0
DMSO
Trametinib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.


【FIG. 12】

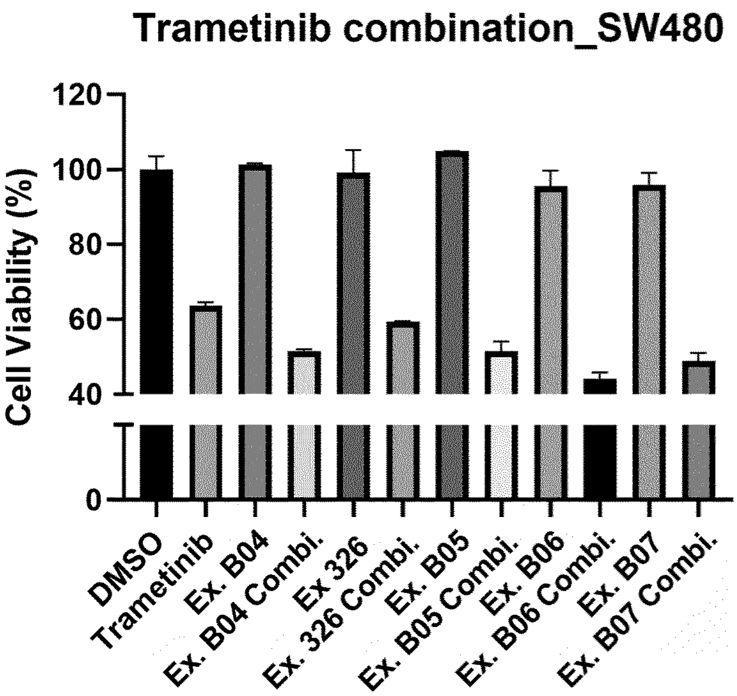

Trametinib combination_SW480
Cell Viability (%)
120
100
80
60
40
0
DMSO
Trametinib
Ex. B04
Ex. B04 Combi.
Ex 326
Ex. 326 Combi.
Ex. B05
Ex. B05 Combi.
Ex. B06
Ex. B06 Combi.
Ex. B07
Ex. B07 Combi.

【FIG. 13】

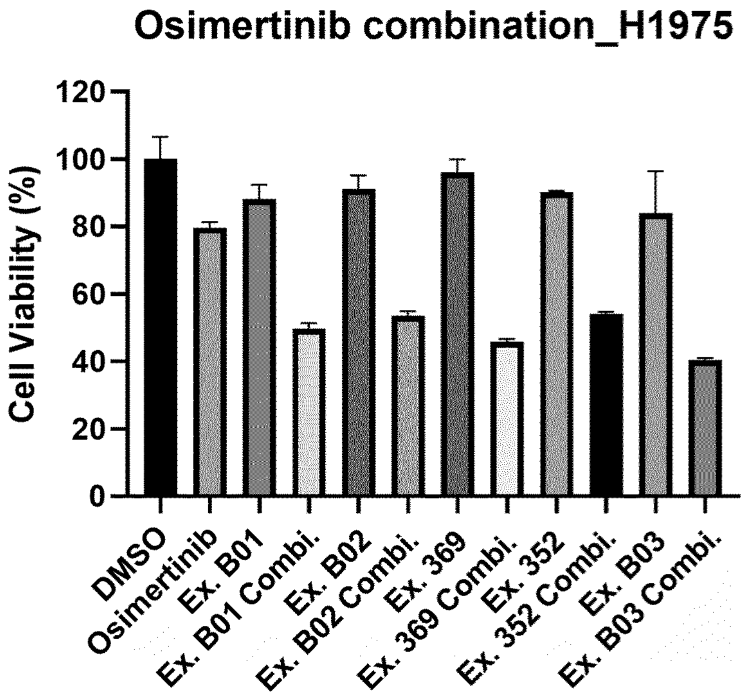
Osimertinib combination_H1975
Cell Viability (%)
0
20
40
60
80
100
120
DMSO
Osimertinib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 14】

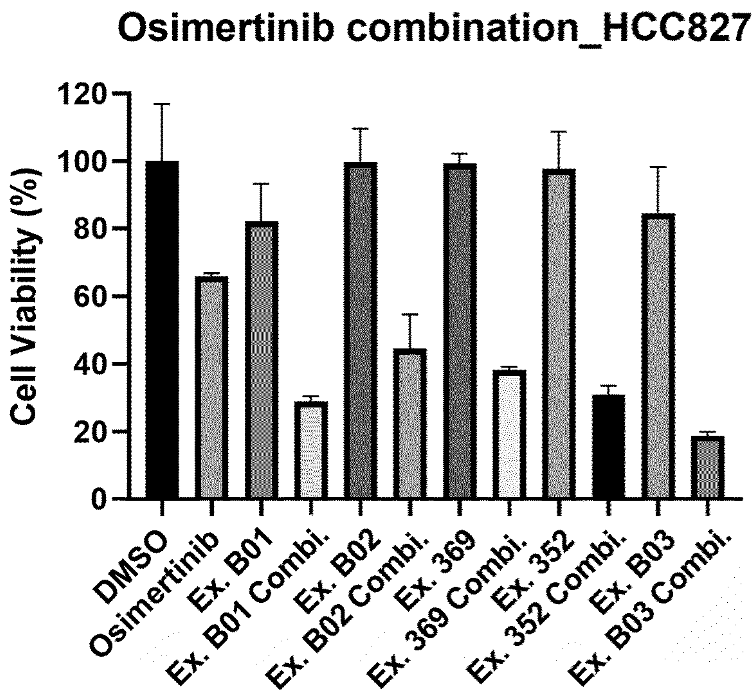
Osimertinib combination_HCC827
Cell Viability (%)
0
20
40
60
80
100
120
DMSO
Osimertinib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 15】

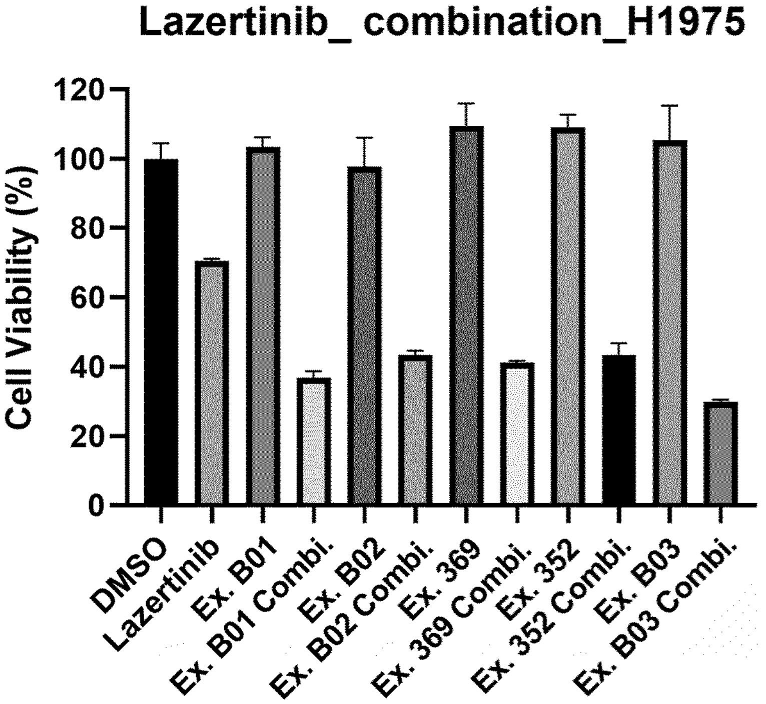
Lazertinib_ combination_H1975
Cell Viability (%)
120
100
80
60
40
20
0
DMSO
Lazertinib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 16】

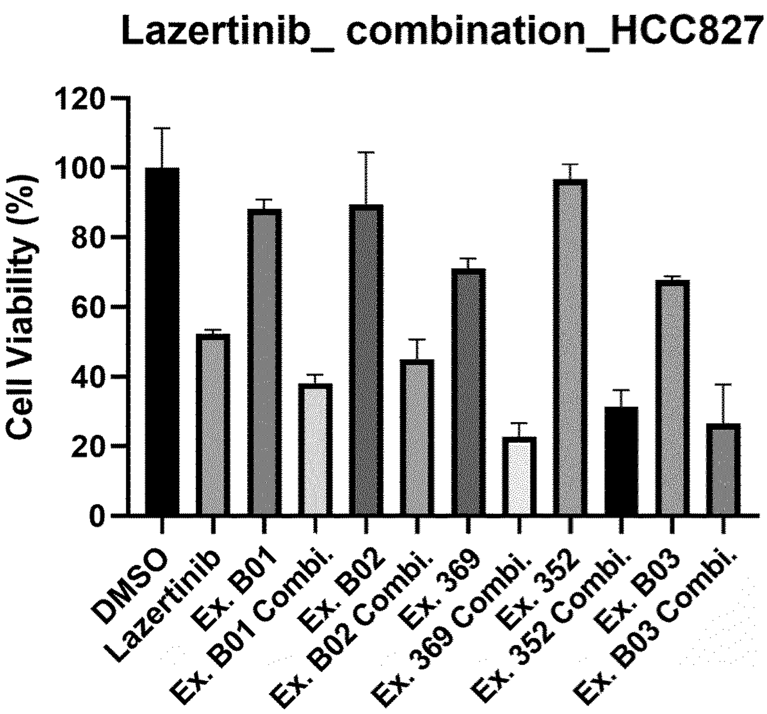
Lazertinib_ combination_HCC827
Cell Viability (%)
120
100
80
60
40
20
0
DMSO
Lazertinib
Ex. B01
Ex. B01 Combi.
Ex. B02
Ex. B02 Combi.
Ex. 369
Ex. 369 Combi.
Ex. 352
Ex. 352 Combi.
Ex. B03
Ex. B03 Combi.

【FIG. 17】

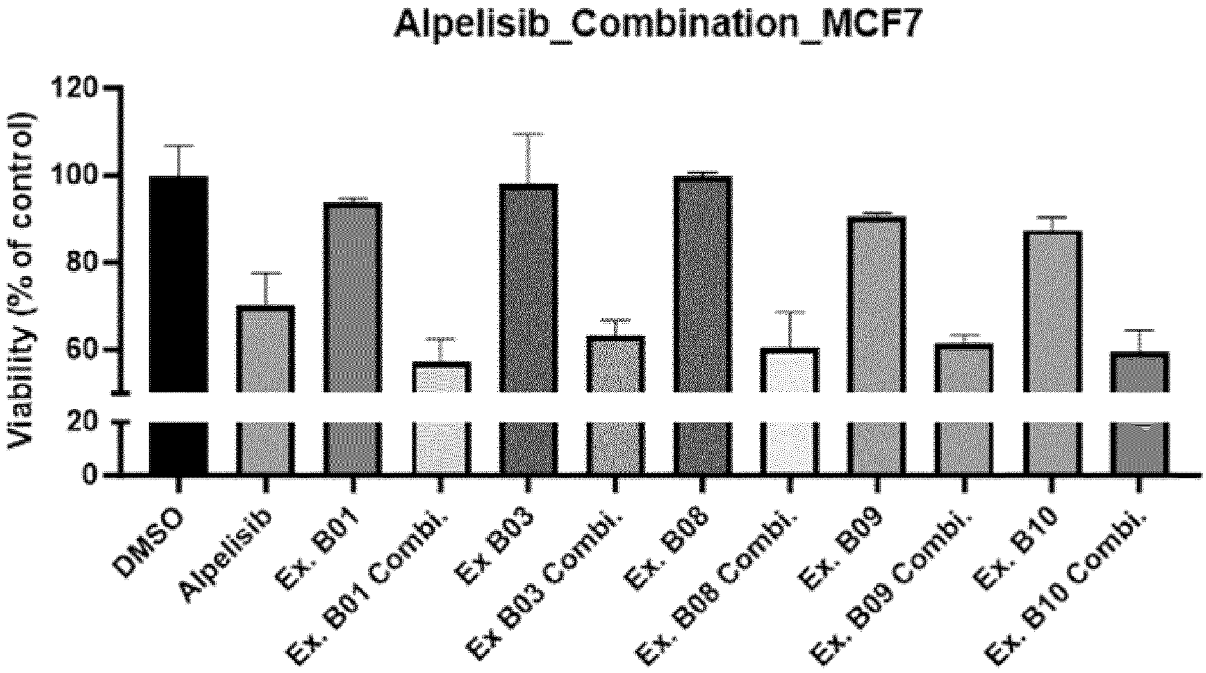
Alpelisib_Combination_MCF7
Viability (% of control)
120
100
80
60
20
0
DMSO
Alpelisib
Ex. B01
Ex. B01 Combi.
Ex B03
Ex B03 Combi.
Ex. B08
Ex. B08 Combi.
Ex. B09
Ex. B09 Combi.
Ex. B10
Ex. B10 Combi.

【FIG. 18】

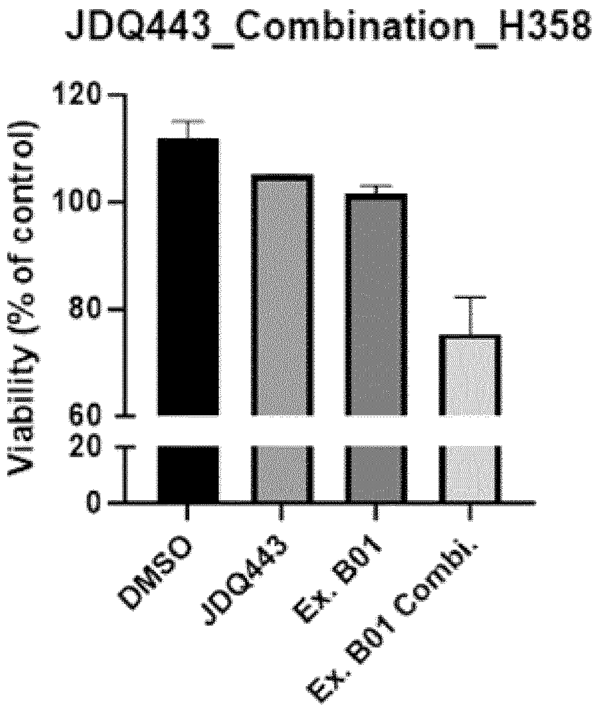
JDQ443_Combination_H358
Viability (% of control)
120
100
80
60
20
0
DMSO
JDQ443
Ex. B01
Ex. B01 Combi.

【FIG. 19】

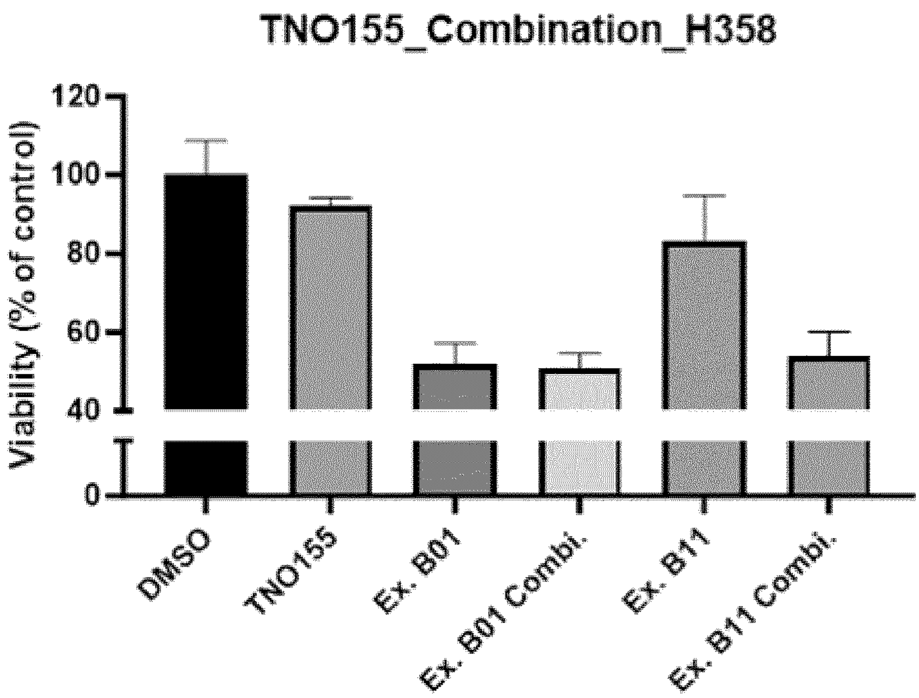
TNO155_Combination_H358
Viability (% of control)
120
100
80
60
40
0
DMSO
TNO155
Ex. B01
Ex. B01 Combi.
Ex. B11
Ex. B11 Combi.

【FIG. 20】

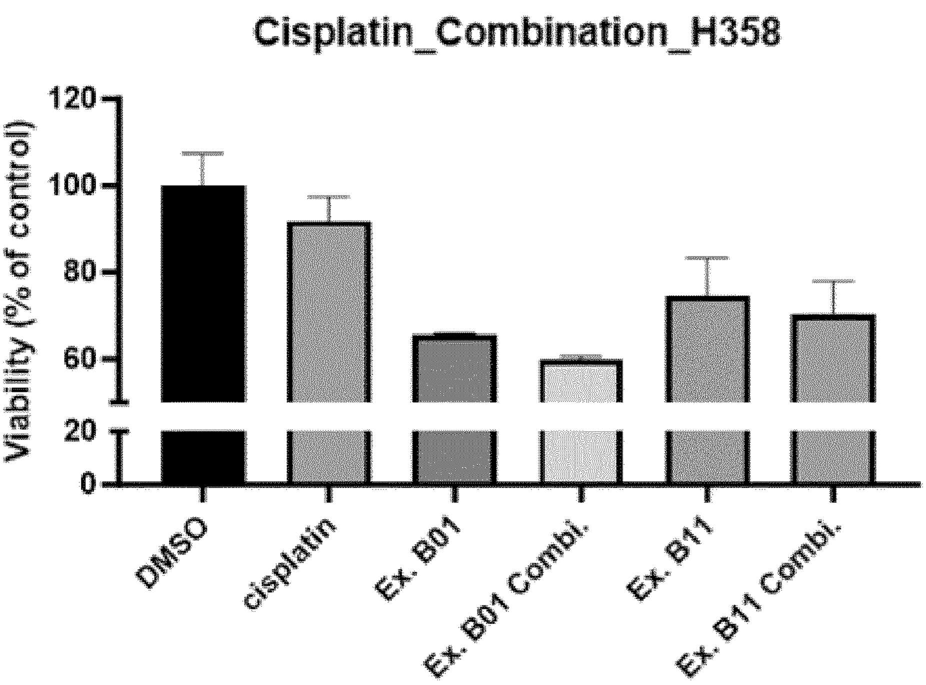
Cisplatin_Combination_H358
Viability (% of control)
120
100
80
60
20
0
DMSO
cisplatin
Ex. B01
Ex. B01 Combi.
Ex. B11
Ex. B11 Combi.

【FIG. 21】

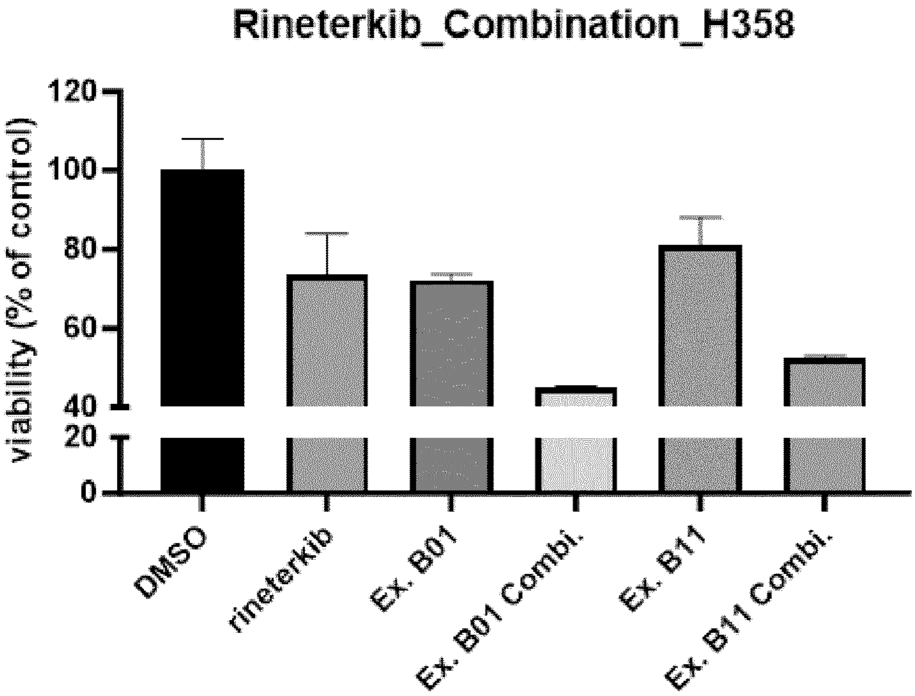
Rineterkib_Combination_H358
viability (% of control)
120
100
80
60
40
20
0
DMSO
rineterkib
Ex. B01
Ex. B01 Combi.
Ex. B11
Ex. B11 Combi.

【FIG. 22】

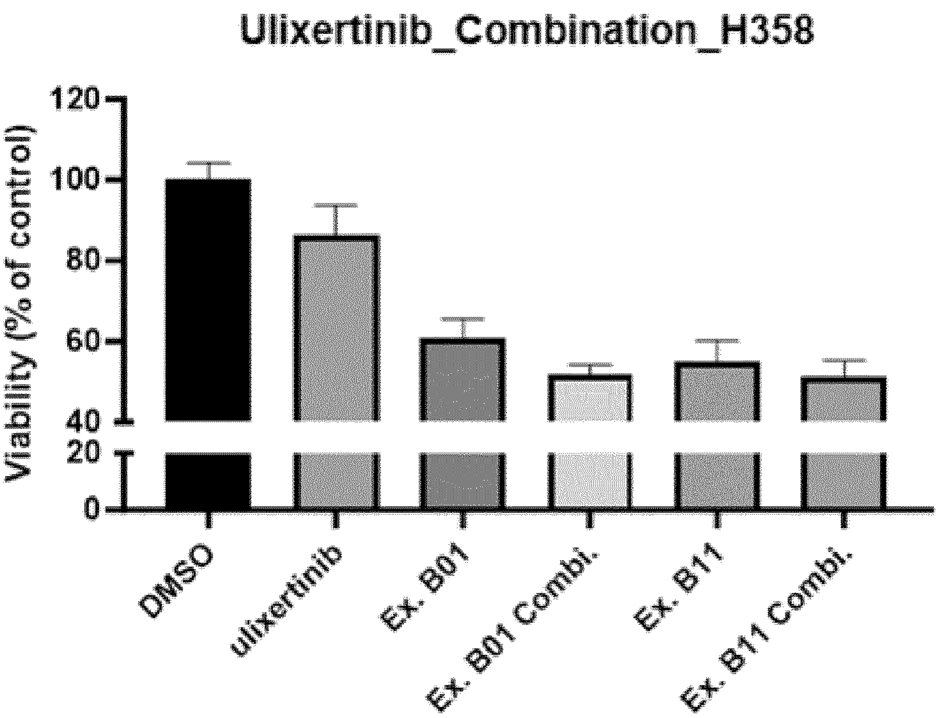
Ulixertinib_Combination_H358
Viability (% of control)
120
100
80
60
40
20
0
DMSO
ulixertinib
Ex. B01
Ex. B01 Combi.
Ex. B11
Ex. B11 Combi.

【FIG. 23】

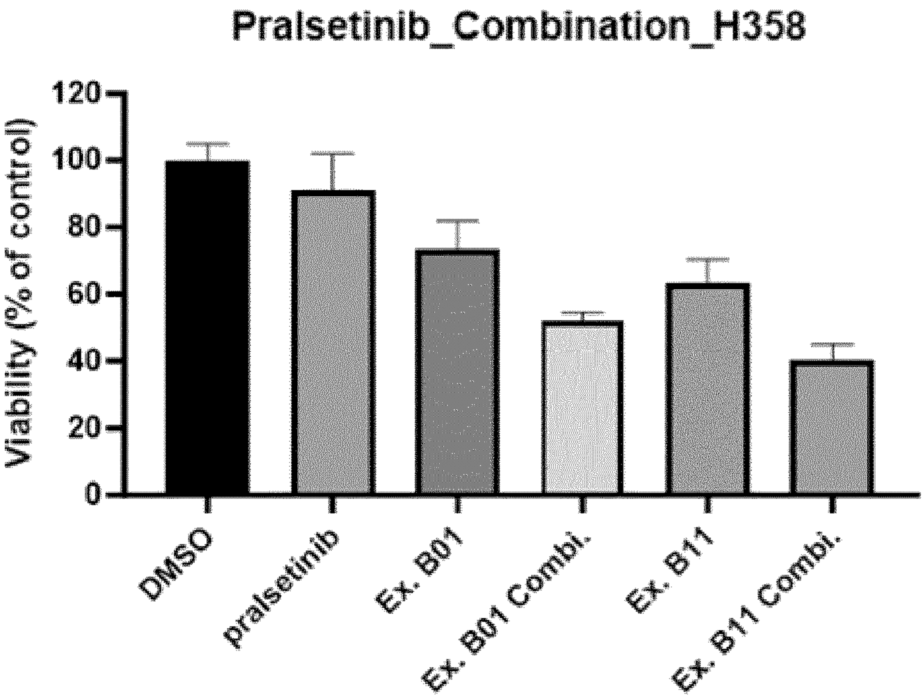
Pralsetinib_Combination_H358
Viability (% of control)
120
100
80
60
40
20
0
DMSO
pralsetinib
Ex. B01
Ex. B01 Combi.
Ex. B11
Ex. B11 Combi.

【FIG. 24】

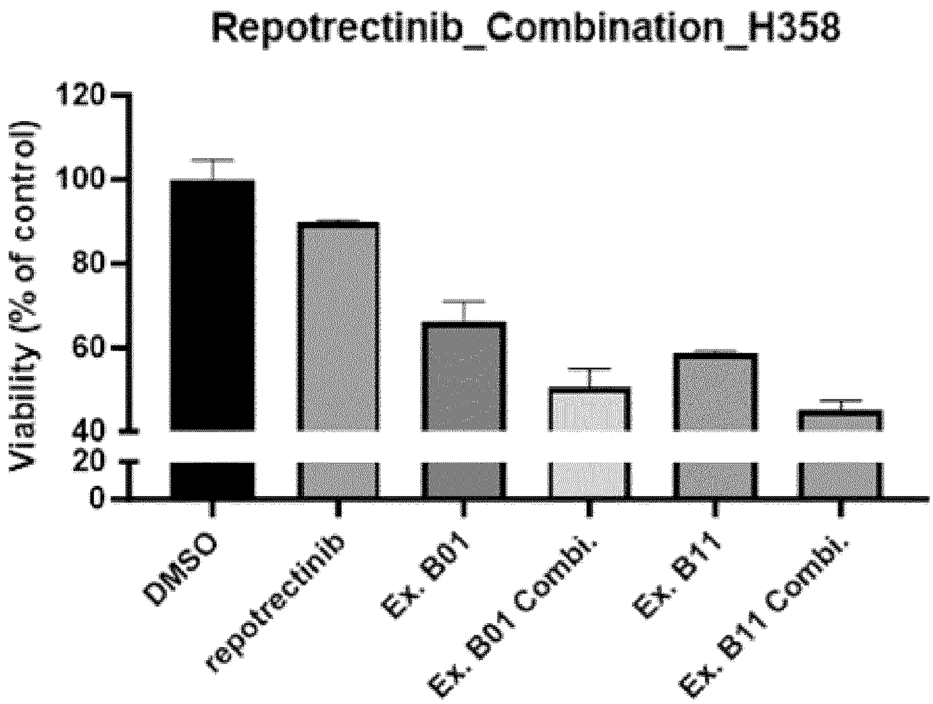
Repotrectinib_Combination_H358
Viability (% of control)
120
100
80
60
40
20
0
DMSO
repotrectinib
Ex. B01
Ex. B01 Combi.
Ex. B11
Ex. B11 Combi.

【FIG. 25】

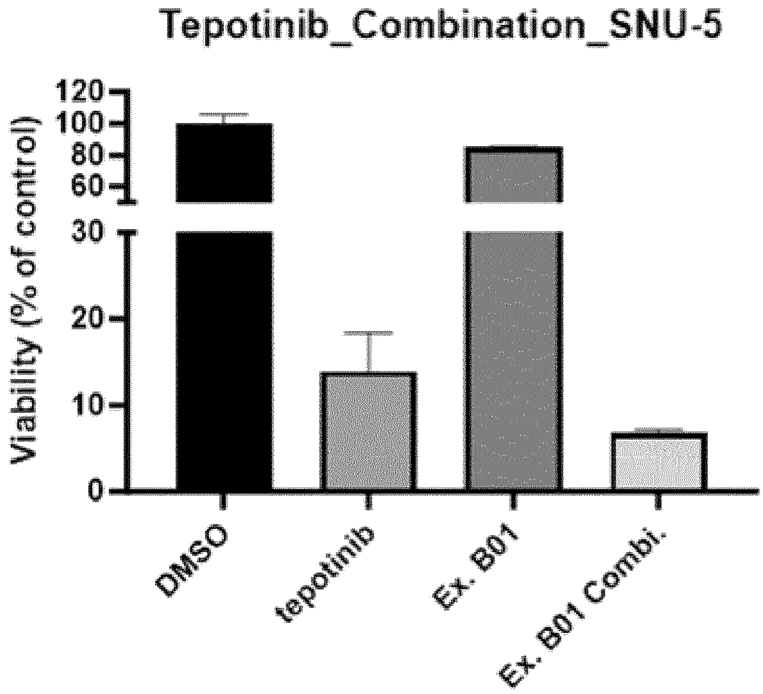
Tepotinib_Combination_SNU-5
Viability (% of control)
120
100
80
60
30
20
10
0
DMSO
tepotinib
Ex. B01
Ex. B01 Combi.

【FIG. 26】

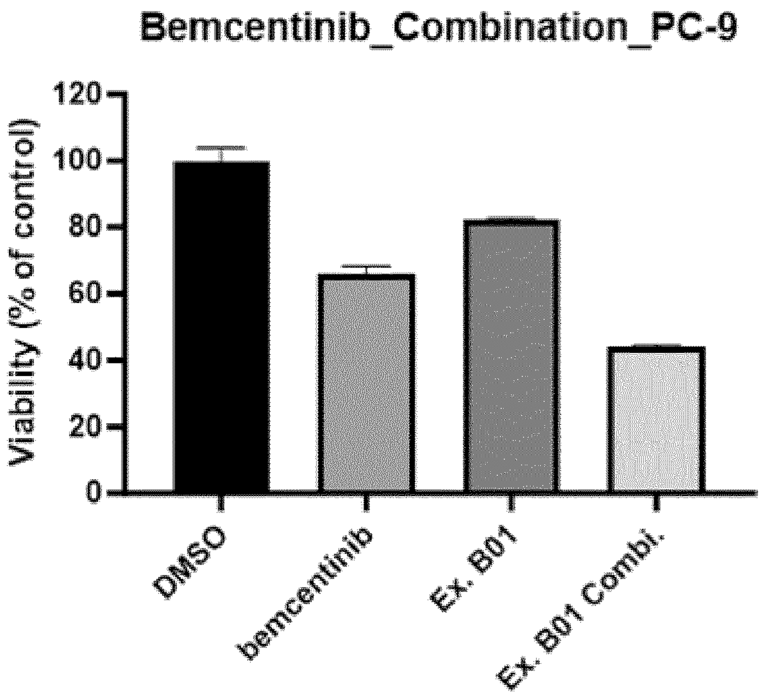
Bemcentinib_Combination_PC-9
Viability (% of control)
120
100
80
60
40
20
0
DMSO
bemcentinib
Ex. B01
Ex. B01 Combi.

【FIG. 27】

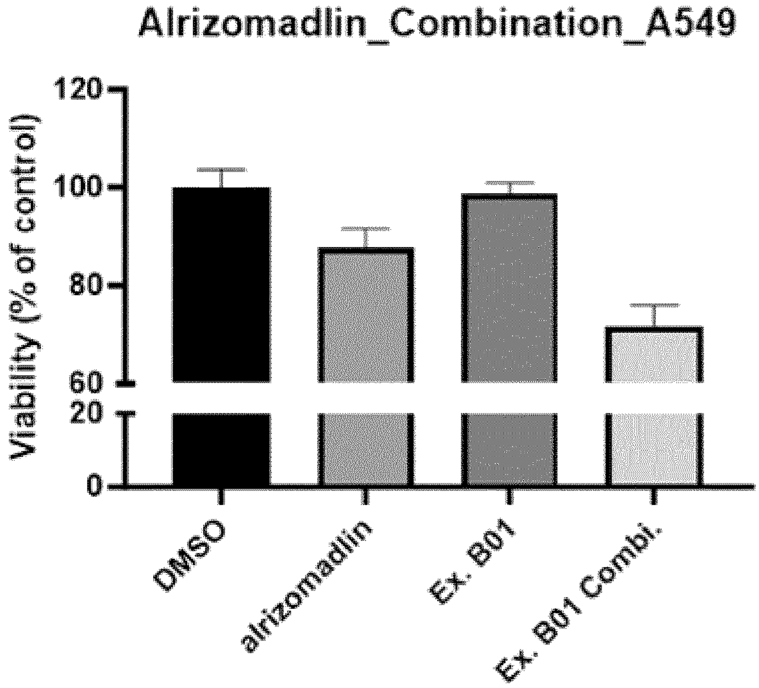
Alrizomadlin_Combination_A549
Viability (% of control)
120
100
80
60
20
0
DMSO
alrizomadlin
Ex. B01
Ex. B01 Combi.

【FIG. 28】

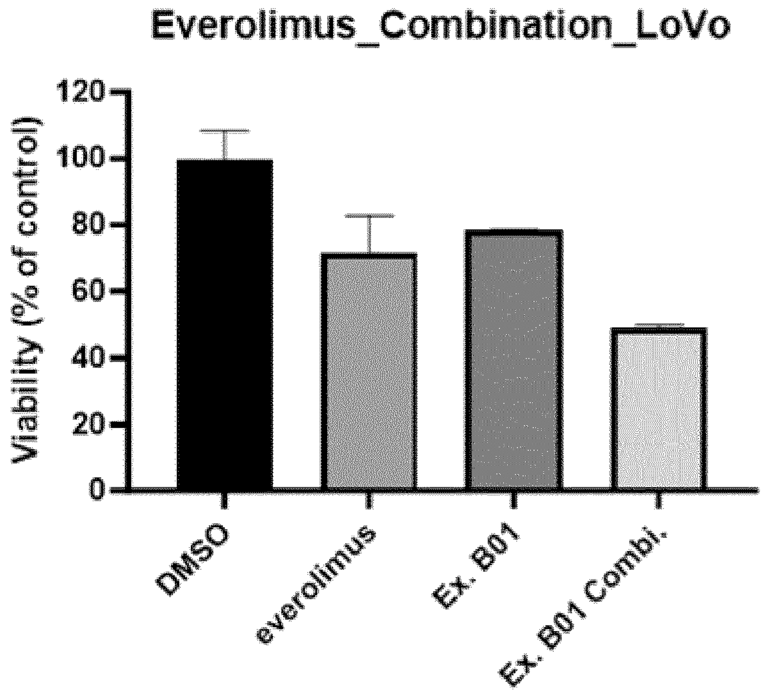
Everolimus_Combination_LoVo
Viability (% of control)
120
100
80
60
40
20
0
DMSO
everolimus
Ex. B01
Ex. B01 Combi.

【FIG. 29】

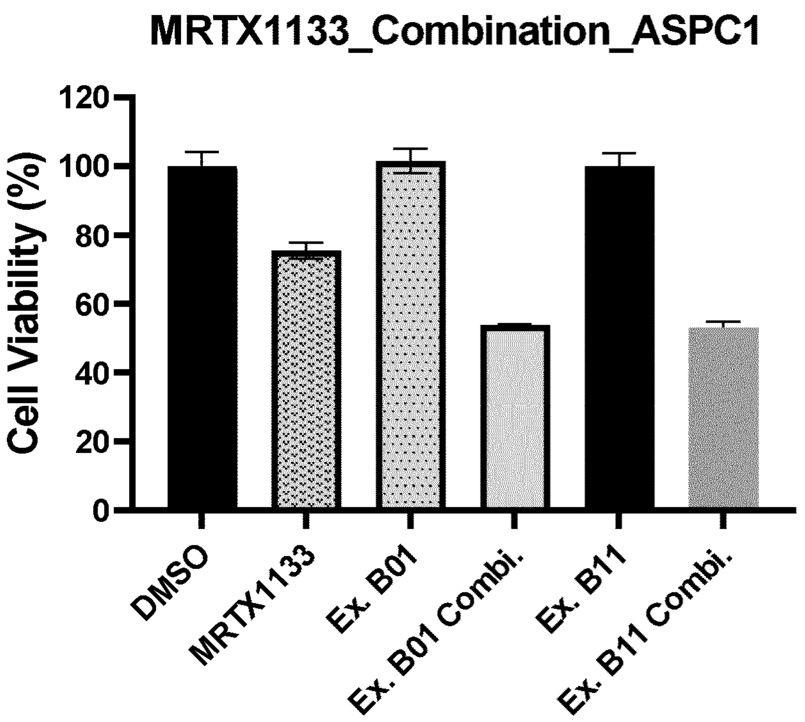


【FIG. 30a】

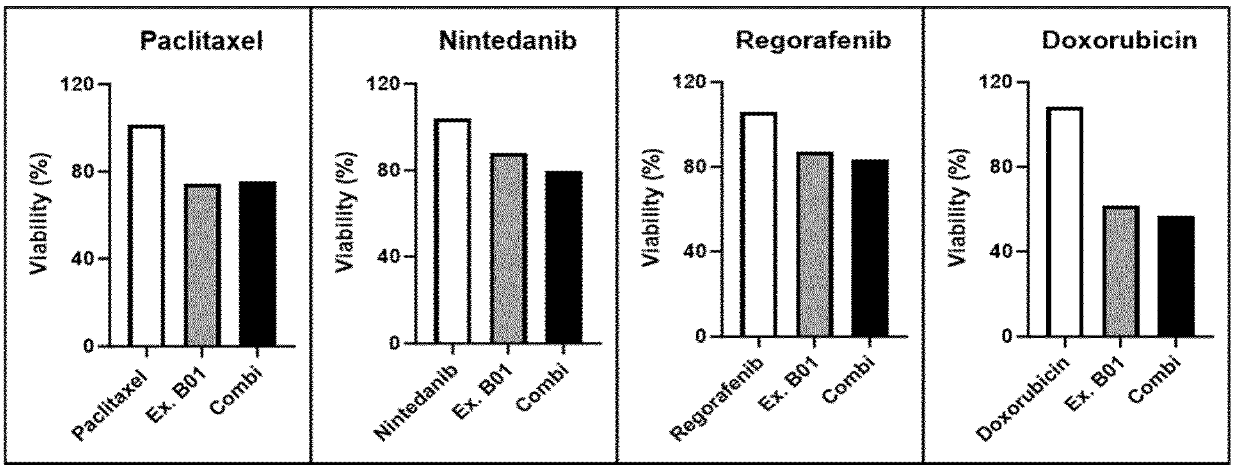

【FIG. 30b】

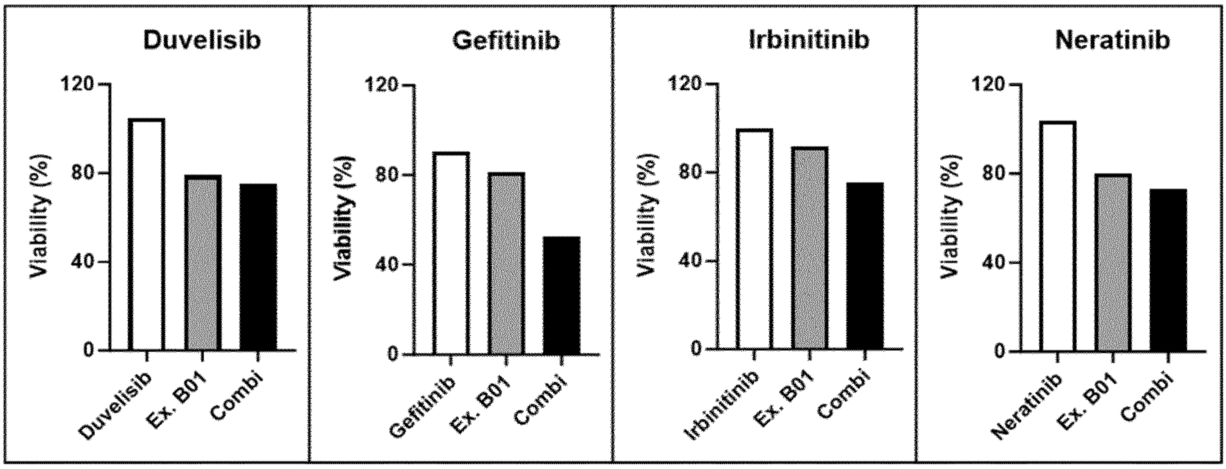


【FIG. 30c】

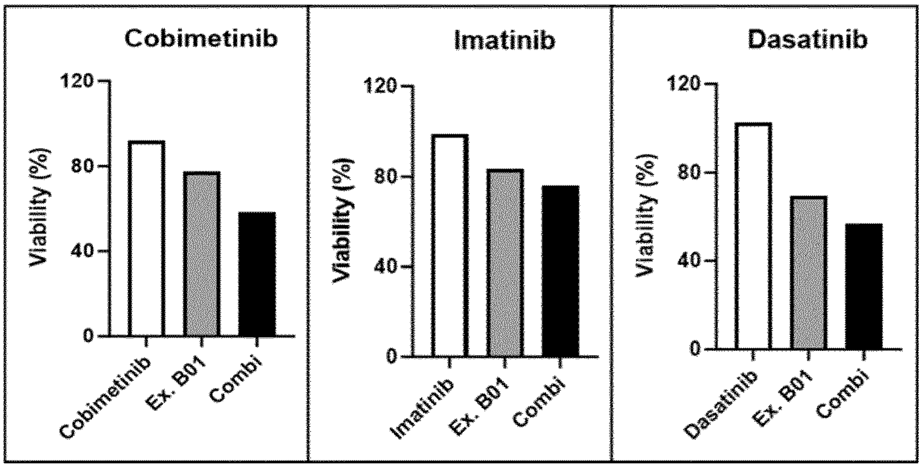


【FIG. 31a】

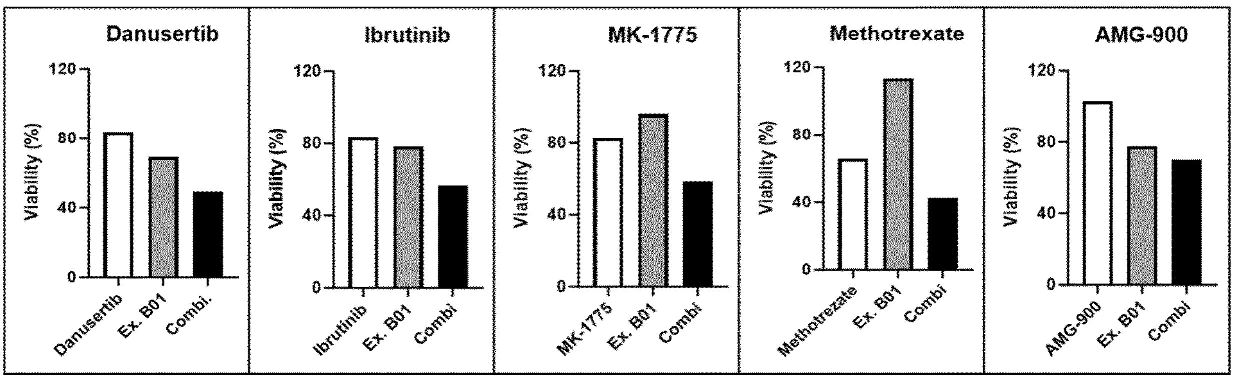

【FIG. 31b】

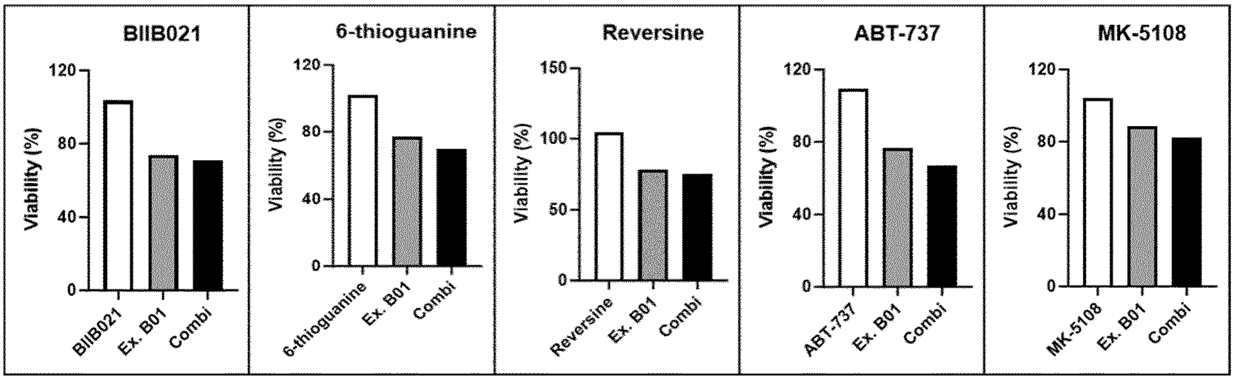


【FIG. 31c】

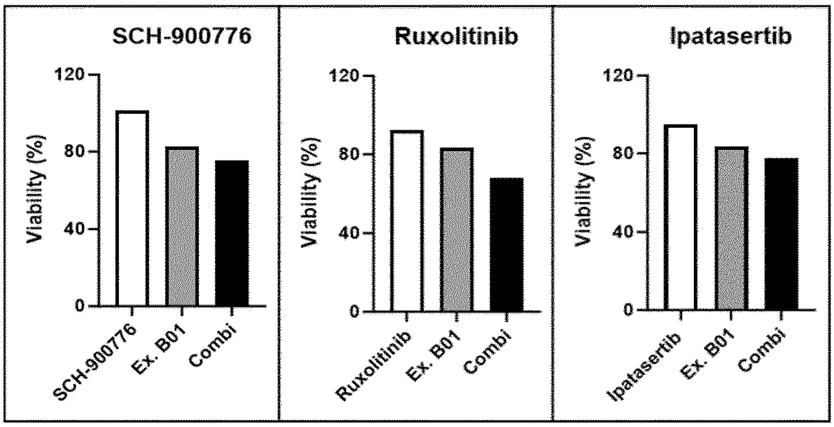

## INTERNATIONAL SEARCH REPORT

International application No.
**PCT/KR2024/095358**

### A. CLASSIFICATION OF SUBJECT MATTER

**C07D 237/24**(2006.01)i; **A61K 31/50**(2006.01)i; **A61K 31/501**(2006.01)i; **A61K 31/5377**(2006.01)i; **A61P 35/00**(2006.01)i; **C07D 405/04**(2006.01)i; **C07D 401/04**(2006.01)i; **C07D 409/04**(2006.01)i; **C07D 401/10**(2006.01)i; **C07D 417/10**(2006.01)i

According to International Patent Classification (IPC) or to both national classification and IPC

### B. FIELDS SEARCHED

Minimum documentation searched (classification system followed by classification symbols)

C07D 237/24(2006.01); A61K 31/496(2006.01); A61K 31/50(2006.01); C07C 317/32(2006.01); C07C 317/34(2006.01); C07D 211/90(2006.01); C07D 237/14(2006.01); C07D 401/12(2006.01); C07D 401/14(2006.01); C07D 413/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus) & keywords: SOS1(Son of Sevenless 1), 암(cancer), 피리다진(pyridazine), 피라진(pyrazine), 항암제(anti-cancer agent), 병용 요법(combination therapy)

### C. DOCUMENTS CONSIDERED TO BE RELEVANT

| Category* | Citation of document, with indication, where appropriate, of the relevant passages | Relevant to claim No. |
|---|---|---|
| X | WO 2014-031928 A2 (JONES, P. et al.) 27 February 2014 (2014-02-27)<br>See abstract; paragraphs [0072]-[0074], [0229], [0231]-[0232], [0235] and [0238]; and example 93. | 1-4,6,11-15,25,41-51 |
| A | | 5,7-10,16-24,26-40 |
| A | KR 10-2015-0013563 A (CHUGAI SEIYAKU KABUSHIKIKAISHA) 05 February 2015 (2015-02-05)<br>See entire document. | 1-51 |
| A | US 2005-0176775 A1 (DEVADAS, B. et al.) 11 August 2005 (2005-08-11)<br>See entire document. | 1-51 |
| A | CN 102558147 A (JIANGSU SIMCERE PHARMACEUTICAL RESEARCH CO., LIMITED) 11 July 2012 (2012-07-11)<br>See entire document. | 1-51 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 June 2024** | **04 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/095358**

**C. DOCUMENTS CONSIDERED TO BE RELEVANT**

| Category* | Citation of document, with indication, where appropriate, of the relevant passages | Relevant to claim No. |
|---|---|---|
| A | WO 2015-022546 A1 (KALVISTA PHARMACEUTICALS LIMITED) 19 February 2015 (2015-02-19)<br>See entire document. | 1-51 |
| PX | KR 10-2023-0026288 A (KANAPH THERAPEUTICS INC. et al.) 24 February 2023 (2023-02-24)<br>See claims 1-48; and paragraphs [0520] and [0526]. | 1-51 |

Form PCT/ISA/210 (second sheet) (July 2022)

**INTERNATIONAL SEARCH REPORT**
**Information on patent family members**

International application No.
**PCT/KR2024/095358**

| Patent document cited in search report | Publication date (day/month/year) | Patent family member(s) | Publication date (day/month/year) |
|---|---|---|---|
| WO 2014-031928 A2 | 27 February 2014 | EP 2888256 A2 | 01 July 2015 |
| | | EP 2888256 A4 | 17 February 2016 |
| | | US 2014-0073634 A1 | 13 March 2014 |
| | | WO 2014-031928 A3 | 22 May 2014 |
| KR 10-2015-0013563 A | 05 February 2015 | CN 104379560 A | 25 February 2015 |
| | | EP 2842939 A1 | 04 March 2015 |
| | | EP 2842939 A4 | 11 November 2015 |
| | | EP 2842939 B1 | 30 May 2018 |
| | | JP 2015-161851 A1 | 24 December 2015 |
| | | JP 6177768 B2 | 09 August 2017 |
| | | US 2015-0152047 A1 | 04 June 2015 |
| | | US 9695118 B2 | 04 July 2017 |
| | | WO 2013-161851 A1 | 31 October 2013 |
| US 2005-0176775 A1 | 11 August 2005 | TW 200517109 A | 01 June 2005 |
| | | WO 2005-018557 A2 | 03 March 2005 |
| | | WO 2005-018557 A3 | 04 August 2005 |
| CN 102558147 A | 11 July 2012 | CN 102558147 B | 17 September 2014 |
| WO 2015-022546 A1 | 19 February 2015 | CN 105683179 A | 15 June 2016 |
| | | CN 105683179 B | 26 October 2018 |
| | | EP 3033336 A1 | 22 June 2016 |
| | | EP 3033336 B1 | 30 May 2018 |
| | | JP 2016-530262 A | 29 September 2016 |
| | | JP 6527147 B2 | 05 June 2019 |
| | | US 10221161 B2 | 05 March 2019 |
| | | US 2016-0168123 A1 | 16 June 2016 |
| KR 10-2023-0026288 A | 24 February 2023 | AU 2024-330732 A1 | 15 February 2024 |
| | | TW 202328076 A | 16 July 2023 |
| | | WO 2023-022497 A1 | 23 February 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Med. Chem.*, 2021, vol. 64 (10), 6569-6580
- *Current Opinion in Chemical Biology*, 2021, vol. 62, 109-118